# EUROPEAN PATENT SPECIFICATION

(11) **EP 4 486 744 B1**
(45) Date of publication and mention of the grant of the patent: **25.11.2026**
(21) Application number: 23714133.8
(22) Date of filing: 27.02.2023
(51) Int. Cl.: C07D 471/04, C07D 487/04, C07D 519/00, A61K 31/437, A61P 25/00

(54) **(4-BENZO[D]OXAZOL-2-YL)-6,7-DIHYDRO-1H-IMIDAZO[4,5-C]PYRIDINE-5(4H)-YL)METHANONE DERIVATIVES AS MUTANT PAH STABILIZERS FOR THE TREATMENT OF PHENYLKETONURIA**
(4-BENZO[D]OXAZOL-2-YL)-6,7-DIHYDRO-1H-IMIDAZO[4,5-C]PYRIDINE-5(4H)-YL)METHANON-DERIVATE ALS STABILISATOREN VON PAH MUTANTEN ZUR BEHANDLUNG VON PHENYLKETONURIA
DÉRIVÉS DE (4-BENZO[D]OXAZOL-2-YL)-6,7-DIHYDRO-1H-IMIDAZO[4,5-C]PYRIDINE-5(4H)-YL)MÉTHANONE EN TANT QUE STABILISATUERS DE PAH MUTANTES POUR LE TRAITEMENT DE PHENYLKETONURIA

(30) Priority: 28.02.2022 US 202263314678 P
(43) Date of publication of application: 08.01.2025
(60) Divisional application: 26208983.2
(73) Proprietor: Agios Pharmaceuticals, Inc., Cambridge, MA 02139 (US)
(72) Inventor: DE PASCALIS, Lucrezia, Cambridge, MA 02139 (US); KING, Sandra, Cambridge, MA 02139 (US); KONTEATIS, Zenon D., Cambridge, MA 02139 (US); SHOOK, Brian C., Cambridge, MA 02139 (US)
(74) Representative: Potter Clarkson
(86) International application number: PCT/US2023/013986
(87) International publication number: WO 2023/164233

(56) References cited:
- WO-A1-2019/105886
- WO-A2-2009/000552

## Description

### TECHNICAL FIELD

This application pertains to compounds that stabilize phenylalanine hydroxylase (PAH) mutations, pharmaceutical compositions comprising those compounds, and those compounds for use in methods for treating phenylketonuria.

### BACKGROUND

Phenylketonuria (PKU) is an autosomal recessive disorder affecting approximately 1:10,000 people worldwide (approx. 1:15,000-1:20,000 in the U.S.). The number of patients varies globally depending on region. PKU arises in patients who have mutations in the gene encoding the phenylalanine hydroxylase (PAH) enzyme responsible for converting phenylalanine to tyrosine. PAH is a tetrameric enzyme expressed in the liver requiring BH4 cofactor for activity. Reduction or loss of PAH activity results in toxic accumulation of phenylalanine (Phe) in the blood and brain. High levels of Phe damage brain white matter and interfere with neurotransmitter production. If untreated, high levels of Phe can result in mental retardation and decreased IQ in children and neurocognitive and psychiatric issues in adults, such as executive function deficits (for example, difficulty with attention, memory, flexible thinking, and organization/time management), psychological issues (for example, depression, anxiety, and mood swings), psychiatric and/or behavioral issues (for example, attention deficit hyperactivity disorder, self-harm, schizophrenia, agoraphobia, and agitation) and neurological abnormalities (for example, spasticity, tremor, gait disturbances, and seizures).

PKU phenotypes can vary from mild hyperphenylalaninemia (HPA) to more severe phenotypes that result in untreated blood Phe concentrations exceeding 1200 µM. American medical guidelines currently recommend maintaining blood Phe concentration in the range of 120 to 360 µM in both adults and children under the age of 12 years. European medical guidelines currently recommend maintaining blood Phe concentration below 360 µM in children under the age of 12 years and in pregnant women and below 600 µM in non-pregnant patients older than 12 years.

A standard of care for treating PKU is a Phe-restricted diet that severely limits the intake of natural protein. Such diets are very strict diets and challenging to adhere to. Two medications are currently approved for treating PKU, each having its own challenges. Kuvan (sapropterin dihydrochloride) is a synthetic BH4 cofactor approved in 2007 for use in infants to adults. Kuvan is not effective for all PKU patients, and the current guidelines suggest response testing in patients unless the patient is known to have two null mutations. Pegvaliase is an enzyme substitution therapy approved in 2018 for adults with a blood Phe concentration greater than 600µM, despite prior management with available treatment options. Pegvaliase typically involves injection of a purified PEGylated form of phenylalanine ammonia lyase that reduces Phe by converting it to ammonia and transcinnamic acid instead of tyrosine. One of the main complications with enzyme substitution therapy is the attainment and maintenance of therapeutically effective amounts of protein in vivo due to rapid degradation or inactivation of the infused protein. A current approach to overcome this problem is to perform numerous costly high dose injections.

Pharmaceutical agents that enable patients to increase their intake of natural protein are desired.

WO 2009/000662 A2 relates to isoxazole-imidazole derivatives having affinity and selectivity for GABA A α5 receptor binding site, their manufacture, pharmaceutical compositions containing them and their use as medicaments. WO 2019/105886 A1 relates to compounds which may inhibit salt-inducible kinases ('SIK' kinases).

### SUMMARY

In some aspects, the disclosure provides compounds of Formula I: 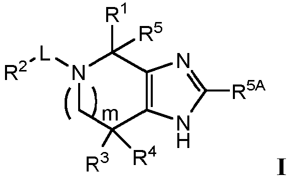 or a pharmaceutically acceptable salt thereof, wherein:
m is 0, 1 or 2;
R¹ is 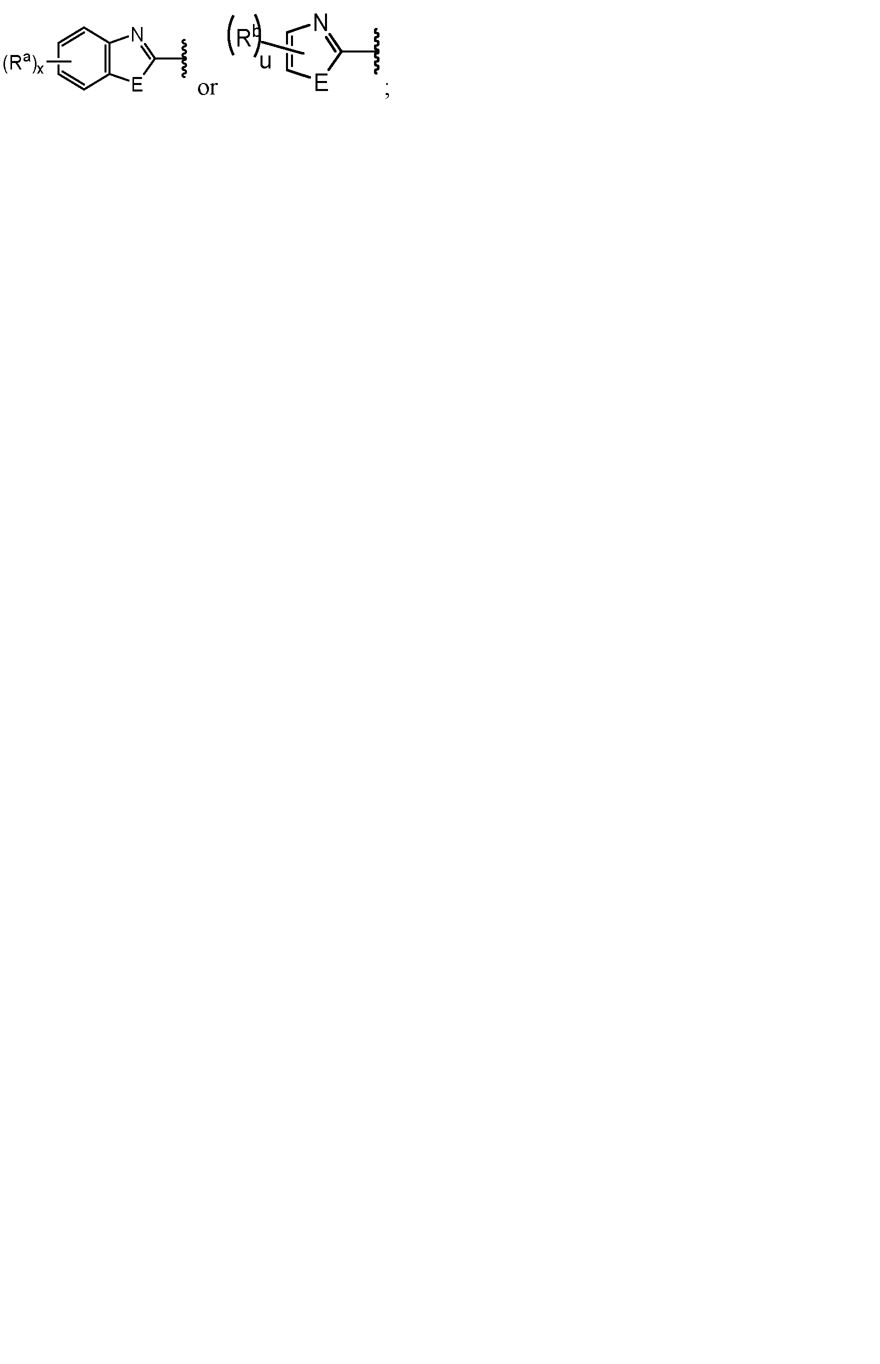
   E is O;
   u is 0 to 2;
   x is 0 to 4;
   each R^{a} independently is halo, C₁₋₆alkyl, C₃₋₆cycloalkyl, C₁₋₆haloalkyl, C₁₋₆alkoxy or C₁₋₆haloalkoxy;
   each R^{b} is independently optionally substituted aryl, C₁₋₆alkyl, C₃₋₆cycloalkyl, or halo;
R² is C₁₋₄alkyl, optionally substituted C₃₋₈cycloalkyl, optionally substituted heterocyclyl, optionally substituted aryl, or optionally substituted heteroaryl;
R³ is H or C₁₋₆alkyl;
R⁴ is H or C₁₋₆alkyl;
or R³ and R⁴, together with the atom to which they are attached, form a C₃₋₆cycloalkyl;
R⁵ is H or D;
R^{5A} is H or D;
L is a bond, carbonyl, optionally substituted C₁₋₆alkylene, optionally substituted C₁₋₆alkylenecarbonyl, optionally substituted C₂₋₆alkenylenecarbonyl, optionally substituted C₁₋₆haloalkylenecarbonyl, or optionally substituted - C(O)NR^{c}(C₁₋₆alkylene)-, wherein the carbon atom of the carbonyl group is connected to N in Formula I; and
R^{c} is H or C₁₋₆alkyl.

In other aspects, the disclosure provides compounds of Formula **I-A, I-B, I-C, I-D, I-E, I-F, I-G, I-H, I-I, I-J, I-K, I-L,** or **I-M,** or a pharmaceutically acceptable salt thereof:

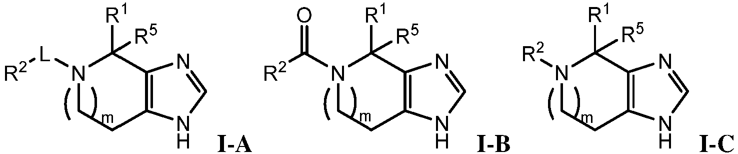

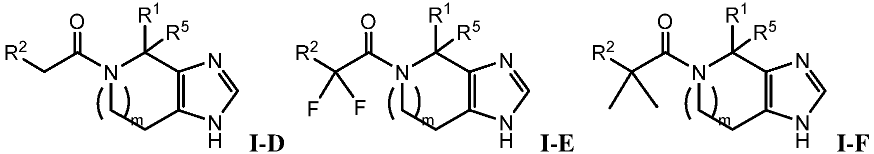

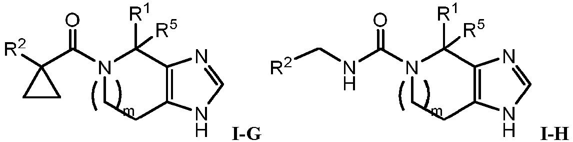

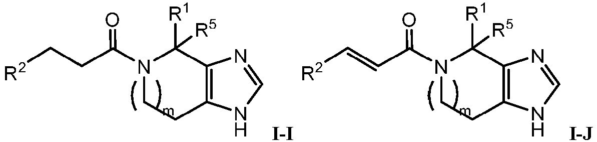

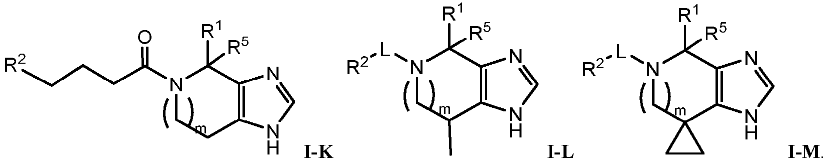

Stereoisomers and mixtures of stereoisomers of the compounds of Formula **I, I-A, I-B, I-C, I-D, I-E, I-F, I-G, I-H, I-I, I-J, I-K, I-L,** and **I-M,** and the pharmaceutical salts thereof, are also described

In further aspects, the disclosure provides pharmaceutical compositions comprising one or more compound described herein or a pharmaceutically acceptable salt thereof, and a pharmaceutically acceptable excipient.

In yet other aspects, the disclosure provides compounds, or pharmaceutically acceptable salts thereof, for use in methods for stabilizing a mutant PAH protein, comprising contacting the protein with one or more compound as described herein or a pharmaceutically acceptable salt thereof. In some embodiments, the mutant PAH protein contains at least one R408W, R261Q, R243Q, Y414C, L48S, A403V, I65T, R241C, L348V, R408Q, or V388M mutation. In other embodiments, the mutant PAH protein contains at least one R408W, Y414C, I65T, F39L, R408Q, L348V, R261Q, A300S, or L48S mutation.

In still further aspects, the disclosure provides compounds, or pharmaceutically acceptable salts thereof, for use in methods for reducing phenylalanine levels in a subject suffering from phenylketonuria comprising administering a therapeutically effective amount of one or more compound as described herein or a pharmaceutically acceptable salt thereof.

### DETAILED DESCRIPTION OF ILLUSTRATIVE EMBODIMENTS

### Definitions

Unless otherwise defined, all technical and scientific terms used herein have the same meaning as commonly understood by one of ordinary skill in the art to which this disclosure pertains. Any references relating to methods of treatment/treating refer to the compounds, pharmaceutically acceptable salts thereof, and pharmaceutical compositions of the invention for use in those methods.

As used in structures herein, " 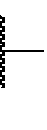 " indicates the point of attachment of the particular depicted structure or substituent group to the appropriate atom(s) in the remainder of the molecule.

The articles "a" and "an" as used herein and in the appended claims are used herein to refer to one or to more than one (*e.g.,* to at least one) of the grammatical object of the article unless the context clearly indicates otherwise. By way of example, "an element" means one element or more than one element.

"Pharmaceutically acceptable" means approved or approvable by a regulatory agency of the Federal or a state government or the corresponding agency in a country other than the United States, or that is listed in the U.S. Pharmacopoeia or other generally recognized pharmacopoeia for use in animals, *e.g.*, in humans.

"Pharmaceutically acceptable salt" refers to a salt of a compound of the disclosure that is pharmaceutically acceptable and that possesses the desired pharmacological activity of the parent compound. In particular, such salts are non-toxic and may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, fumarate, tartrate, mesylate, acetate, maleate, oxalate and the like.

A "pharmaceutically acceptable excipient" refers to a substance that is non-toxic, biologically tolerable, and otherwise biologically suitable for administration to a subject, such as an inert substance, added to a pharmacological composition or otherwise used as a vehicle, carrier, or diluent to facilitate administration of an agent and that is compatible therewith.

The term "alkyl," when used alone or as part of a substituent group, refers to a straight- or branched-chain hydrocarbon group having from 1 to 12 carbon atoms ("C₁₋₁₂"), for example 1 to 6 carbons atoms ("C₁₋₆"), in the group. Examples of alkyl groups include methyl (C₁), ethyl (C₂), propyl (C₃) (*e.g*., n-propyl, isopropyl), butyl (C₄) (*e.g*., n-butyl, tert-butyl, sec-butyl, iso-butyl), pentyl (C₅) (*e.g*., n-pentyl, 3-pentyl, amyl, neopentyl, 3-methyl-2-butanyl, tertiary amyl), hexyl (C₆) (*e.g*., n-hexyl), heptyl (C₇) (*e.g*., n-heptyl), octyl (C₈) (*e.g.,* n-octyl), and the like. In some embodiments, the alkyl group is a C₁₋₆alkyl; in other embodiments, it is a C₁₋₄alkyl; and in other embodiments, it is a C₁₋₃alkyl.

The term "alkylene," when used alone or as part of a substituent group, refers to an alkyl diradical, *i.e.,* a straight- or branched-chain hydrocarbon group that is attached to two other groups. For example, one embodiment of a C₂alkylene is the diradical - CH₂CH₂-. In some embodiments, the alkylene group is C₁₋₆alkylene; in other embodiments, it is C₁₋₄alkylene.

When a range of carbon atoms is used herein, for example, C₁₋₆, all ranges, as well as individual numbers of carbon atoms are encompassed. For example, "C₁₋₃" includes C₁₋₃, C₁₋₂, C₂₋₃, C₁, C₂, and C₃.

The term "cycloalkyl" when used alone or as part of a substituent group refers to cyclic-containing, non-aromatic hydrocarbon groups having from 3 to 10 carbon atoms ("C₃₋₁₀"), for example from 3 to 7 carbon atoms ("C₃₋₇"). Examples of cycloalkyl groups include cyclopropyl (C₃), cyclobutyl (C₄), cyclopentyl (C₅), cyclohexyl (C₆), cycloheptyl (C₇), and the like. In some embodiments, the cycloalkyl group is a C₃₋₄cycloalkyl; in other embodiments, it is a C₃₋₆cycloalkyl; and in other embodiments, it is C₃₋₈cycloalkyl. The cycloalkyl may be unsubstituted or substituted. In some embodiments, the cycloalkyl is substituted with two substituents. In further embodiments, the cycloalkyl is substituted with one substituent. In yet other embodiments, the cycloalkyl is substituted with three substituents. In still further embodiments, the cycloalkyl is unsubstituted.

The term "aryl" when used alone or as part of a substituent group also refers to a mono- or bicyclic- aromatic hydrocarbon ring structure having 6 or 10 carbon atoms in the ring. Examples of aryl groups include phenyl, indenyl, naphthyl, 1,2,3,4-tetrahydronaphthyl, and the like. The aryl may be unsubstituted or substituted. In other embodiments, the optionally substituted phenyl has four substituents. In further embodiments, the optionally substituted phenyl has three substituents. In yet other embodiments, the optionally substituted phenyl has two substituents. In still further embodiments, the optionally substituted phenyl has one substituent. In other embodiments, the optionally substituted phenyl is unsubstituted.

As used herein, the term "alkenyl" refers to a straight- or branched-chain group having from 2 to 12 carbon atoms ("C₂₋₁₂") in the group, wherein the group includes at least one carbon-carbon double bond. Examples of alkenyl groups include vinyl (-CH=CH₂; C₂alkenyl), allyl (-CH₂-CH=CH₂; C₃alkenyl), propenyl (-CH=CHCH₃; C₃alkenyl), isopropenyl (-C(CH₃)=CH₂; C₃alkenyl), butenyl (-CH=CHCH₂CH₃; C₄alkenyl), sec-butenyl (-C(CH₃)=CHCH₃; C₄alkenyl), iso-butenyl (-CH=C(CH₃)₂; C₄alkenyl), 2-butenyl (-CH₂CH=CHCH₃; C₄alkyl), pentenyl (-CH=CHCH₂CH₂CH₃; C₅alkenyl), and the like. In some embodiments, the alkenyl group is a C₂₋₆ alkenyl group; in other embodiments, it is C₂₋₄alkenyl.

As used herein, the term "alkynyl" refers to a straight- or branched-chain group having from 2 to 12 carbon atoms ("C₂₋₁₂") in the group, and wherein the group includes at least one carbon-carbon triple bond. Examples of alkynyl groups include ethynyl (-C≡CH; C₂alkynyl), propargyl (-CH₂-C≡CH; C₃alkynyl), propynyl (-C≡CCH₃; C₃alkynyl), butynyl (-C≡CCH₂CH₃; C₄alkynyl), pentynyl (-C≡CCH₂CH₂CH₃; C₅alkynyl), and the like. In some embodiments, the alkynyl group is a C₂₋₆alkynyl group; in other embodiments, it is C₂₋₄alkynyl.

The term "carbonyl" as used by itself or as part of another group refers to C(O) or C(=O).

The term "alkylcarbonyl" as used by itself or as part of another group refers to an alkyl group as defined above wherein at least one carbon is bonded to an oxo group. For example, one embodiment of an C₃alkylcarbonyl is -CH₂C(O)CH₃. In some embodiments, the alkylcarbonyl group is a C₁₋₆alkylcarbonyl group.

The term "alkenylenecarbonyl" as used by itself or as part of another group refers to an -C(O)-(alkenylene) group, where alkenylene refers to an alkylene diradical, *i.e., a* straight- or branched-chain hydrocarbon group containing at least one carbon-carbon double bond that is attached to two other groups. For example, one embodiment of a -C(O)-C₂alkenylene is -C(O)-CH=CH-. In some embodiments, the alkenylene group of the alkenylenecarbonyl is a C₂₋₆alkenylene group; in other embodiments, the alkenylene group is C₂₋₄alkenylene.

The term "halo" or "halogen," as used by itself or as part of another group refers to a fluorine, chlorine, bromine, or iodine atom.

As used herein, the term "haloalkyl" refers to an alkyl group wherein one or more of the hydrogen atoms has been replaced with one or more halogen atoms which may be the same or different. In some embodiments, the alkyl is substituted by at least one halogen. In other embodiments, the alkyl is substituted by one, two, or three F and/or Cl. Examples of haloalkyl groups include fluoromethyl (CH₂F), 1-fluoroethyl (CH(CH₃)F), 2-fluoroethyl, difluoromethyl (CHF₂), trifluoromethyl (CF₃), pentafluoroethyl, 1,1-difluoroethyl (C(CH₃)F₂), 2,2-difluoroethyl (CH₂CHF₂), 2,2,2-trifluoroethyl (CH₂CF₃), 2-fluoropropan-2-yl (C(CH₃)₂F), 3,3,3-trifluoropropyl, 4,4,4-trifluorobutyl, trichloromethyl and the like. In some embodiments, the haloalkyl group is a C₁₋₆haloalkyl; in other embodiments, it is a C₁₋₄haloalkyl; and in other embodiments, it is a C₁₋₃haloalkyl.

The term "haloalkylenecarbonyl" as used by itself or as part of another group refers to a -C(O)-(haloalkylene) group, where the haloalkylene refers to a haloalkyl diradical. For example, one embodiment of a -C(O)-C₁haloalkylene is -C(O)-CF₂-. In some embodiments, the haloalkylene group is a C₁₋₆haloalkylene; in other embodiments, it is a C₁₋₄haloalkylene; and in other embodiments, it is a C₁₋₃haloalkylene.

The term "cyanoalkyl" as used by itself or as part of another group refers to an alkyl as defined herein that is substituted by one or more CN. In some embodiments, the alkyl is substituted by at least one CN. In other embodiments, the alkyl is substituted by one, two, or three CN. In further embodiments, the cyanoalkyl group is a C₁₋₆cyanoalkyl. In yet other embodiments, the cyanoalkyl is a C₁₋₄cyanoalkyl. Examples of cyanoalkyl groups include CH₂CN, CH₂CH₂CN, CH(CN)CH₃, CH₂CH₂CH₂CN, C(CH₃)₂CN, CH₂CH(CN)CH₃, CH(CN)CH₂CH₃, and the like.

The term "hydroxyalkyl" as used by itself or as part of another group refers to an alkyl group as defined herein wherein one or more of the hydrogen atoms has been replaced with one or more hydroxyl (*i.e.,* -OH). In some embodiments, the hydroxyalkyl contains one OH. In other embodiments, the hydroxyalkyl contains two OH. In further embodiments, the hydroxyalkyl contains three OH. Examples of hydroxyalkyl groups include hydroxymethyl, hydroxyethyl (*e.g*., 1-hydroxyethyl, 2-hydroxyethyl), 1,2-dihydroxyethyl, hydroxypropyl (*e.g*., 2-hydroxypropyl, 3-hydroxypropyl), hydroxybutyl (*e.g*., 3-hydroxybutyl, 4-hydroxybutyl), 2-hydroxy-1-methylpropyl, 1,3-dihydroxyprop-2-yl, and the like. In some embodiments, the hydroxyalkyl group is C₁₋₆hydoxyalkyl; in other embodiments, it is C₁₋₄hydroxyalkyl; and in other embodiments, it is C₁₋₃hydroxyalkyl.

The term "cycloalkylsulfonyl" as used by itself or as part of another group refers to a cycloalkyl as defined herein that is bound to a sulfonyl, *i.e.,* -SO₂-, and the sulfonyl group forms the point of attachment to the remainder of the molecule. In some embodiments, the cycloalkylsulfonyl is a C₃₋₈cycloalkylsulfonyl; in other embodiments, it is a C₃₋₆cycloalkylsulfonyl. Examples of cycloalkylsulfonyl groups include -SO₂-cyclopropyl, -SO₂-cyclobutyl, -SO₂-cyclopentyl, and the like.

The term "alkylsulfonyl" as used by itself or as part of another group refers to an alkyl as defined herein that is bound to a sulfonyl, *i.e.,* -SO₂-, and the sulfonyl group forms the point of attachment to the remainder of the molecule. In some embodiments, the alkylsulfonyl is C₁₋₆alkylsulfonyl; in other embodiments, it is a C₁₋₄alkylsulfonyl. Examples of alkylsulfonyl groups include -SO₂CH₃, -SO₂CH₂CH₃, and the like.

The term "alkylsulfonyl(alkylene)" as used by itself or as part of another group refers to an alkylene group as defined herein that is bound to the sulfonyl of an alkylsulfonyl group as defined herein. Examples of alkylsulfonyl(alkylene) groups include - C(CH₃)₂SO₂CH₃, -CH₂SO₂CH₃, -CH(CH₃)SO₂CH₃, and the like.

The term "alkoxy" as used by itself or as part of another group refers to an oxygen radical attached to an alkyl group by a single bond. Examples of alkoxyl groups include methoxy (OCH₃), ethoxy (OCH₂CH₃), propoxy (*e.g.,* -OⁿPr, -OⁱPr), or butoxy (*e.g., -* OⁿBu, -OⁱBu, -O^{s}Bu, -O^{t}Bu), and the like. In other embodiments, the alkoxy group is a C₁₋₆alkoxy. In further embodiments, the alkoxy group is a C₁₋₄alkoxy.

The term "alkoxy(alkylene)" as used by itself or as part of another group refers to an alkylene group as defined herein that is bound to an alkoxy group as defined herein. Examples of alkoxy(alkylene) groups include -CH₂OCH₃, -CH₂CH₂OCH₃, and the like.

The term "haloalkoxy" as used by itself or as part of another group refers to an oxygen radical attached to a haloalkyl group by a single bond, wherein haloalkyl is defined above. Examples of haloalkoxy groups include fluoromethoxy (OCH₂F), 2-fluoroethoxy, difluoromethoxy (OCHF₂), trifluoromethoxy (OCF₃), pentafluoroethoxy, 1,1-difluoroethoxy (OC(CH₃)F₂), 2,2-difluoroethoxy, 2,2,2-trifluoroethoxy (OCH₂CF₃), 3,3,3-trifluoropropoxy, 4,4,4-trifluorobutoxy, trichloromethoxy groups, and the like. In some embodiments, the haloalkoxy group is a C₁₋₆haloalkoxy; in other embodiments, it is C₁₋₄haloalkoxy; and in other embodiments, it is C₁₋₃haloalkoxy.

The term "haloalkoxy(alkylene)" as used by itself or as part of another group refers to an alkylene group as defined herein that is bound to an haloalkoxy group as defined herein. Examples of haloalkoxy(alkylene) groups include -CH₂OCF₃, and the like.

The term "heteroaryl" when used alone or as part of a substituent group refers to a mono- or bicyclic- aromatic ring structure including carbon atoms as well as up to four heteroatoms that are each independently nitrogen, oxygen, or sulfur. Heteroaryl rings can include a total of 5, 6, 9, or 10 ring atoms. In some embodiments, heteroaryl rings are characterized by the number of ring atoms in the heteroaryl group. For example, a 6-membered heteroaryl group refers to a heteroaryl group having 6 ring atoms in the group. Similarly, a 5-membered heteroaryl group refers to a heteroaryl group having 5 ring atoms in the group. The heteroaryl moiety can be unsubstituted, or one or more of the carbon atoms or nitrogen atoms in the ring can be substituted. Examples of heteroaryl groups include thienyl, benzo[b]thienyl, furanyl, benzofuryl, pyranyl, thiophenyl, isobenzofuranyl, benzoxazinyl, chromenyl, xanthenyl, 2H pyrrolyl, pyrrolyl, imidazolyl, pyrazolyl, pyridyl, pyrazinyl, pyrimidinyl, pyridazinyl, isoindolyl, 3H-indolyl, indolyl, indazolyl, purinyl, isoquinolyl, quinolyl, quinoxalyl, phthalazinyl, naphthyridinyl, cinnolinyl, triazolyl, tetrazolyl, thiadiazolyl, oxadiazolyl, quinazolinyl, pteridinyl, pyrimidinyl, thiazolyl, isothiazolyl, oxazolyl, isoxazolyl, furazanyl, pyrazolo[1,5-a]pyridinyl, benzoisothiazolyl, imidazol[1,5-a]pyridinyl, pyrrolo[1,2]pyridazinyl, benzo[d]thiazolyl, benzo[d]imidazolyl, benzo[d]oxazolyl, benzoisoxazolyl, isothiazolyl, tetrahydropyrazolo[1,5-a]pyridinyl and the like. In some embodiments, the heteroaryl is thienyl (*e.g.,* thien-2-yl and thien-3-yl), furyl (*e.g*., 2-furanyl, 3-furanyl, 4-furanyl), pyrrolyl (*e.g*., pyrrol-2-yl, pyrrol-3-yl), imidazolyl (*e.g*., imidazol-2-yl, imidazol-4-yl), pyrazolyl (*e.g*., pyrazol-1-yl, pyrazol-3-yl, pyrazol-4-yl, pyrazol-5-yl), pyridyl (*e.g*., pyridin-1-yl, pyridin-2-yl, pyridin-3-yl, and pyridin-4-yl), pyrimidinyl (*e.g*., pyrimidin-2-yl, pyrimidin-4-yl, and pyrimidin-5-yl), thiazolyl (*e.g*., thiazol-2-yl, thiazol-4-yl, and thiazol-5-yl), isothiazolyl (*e.g*., isothiazol-3-yl, isothiazol-4-yl, and isothiazol-5-yl), oxazolyl (*e.g*., oxazol-2-yl, oxazol-4-yl, and oxazol-5-yl), isoxazolyl (*e.g.,* isoxazol-3-yl, isoxazol-4-yl, and isoxazol-5-yl), pyrazinyl (*e.g*., pyrazin-2-yl, pyrazin-3-yl, pyrazin-5-yl, pyrazin-6-yl), triazolyl (*e.g*., 1,2,4-triazol-1-yl, 1,2,4-triazol-3-yl, 1,2,4-triazol-5-yl), thiadiazolyl (*e.g*., 1,2,3-thiadiazolyl, 1,2,4-thiadiazolyl, 1,2,5-thidiazolyl, 1,3,4-thiadiazolyl), oxadiazolyl (*e.g*., 1,2,3-oxadiazolyl, 1,2,4-oxadiazolyl, 1,2,5-oxadiazolyl, 1,3,4-oxadiazolyl), indazolyl (*e.g*., indazol-3-yl), pyrazolo[1,5-a]pyridinyl (*e.g*., pyrazolo[1,5-a]pyridin-3-yl), imidazol[1,5-a]pyridinyl (*e.g*., imidazol[1,5-a]pyridin-1-yl), pyrrolo[1,2]pyridazinyl (*e.g*., pyrrolo[1,2]pyridazin-5-yl, pyrrolo[1,2]pyridazin-6-yl), benzo[d]thiazolyl (*e.g*., benzo[d]thiazol-3-yl, benzo[d]thiazol-2-yl), benzo[d]imidazolyl (*e.g.,* benzo[d]imidazol-2-yl), benzo[d]oxazolyl (*e.g*., benzo[d]oxazol-2-yl), benzo[d]isoxazolyl (*e.g*., benzo[d]isoxazol-3-yl), benzo[d]isothiazolyl (*e.g*., benzo[d]isothiazol-3-yl), benzo[c]isoxazolyl (*e.g*., benzo[c]isoxazol-3-yl), quinolinyl (*e.g*., quinolin-3-yl), and pyridazinyl (*e.g*., pyridazin-3-yl, pyridazin-4-yl). The term "heteroaryl" also includes N-oxides. The heteroaryl may be unsubstituted or substituted. In some embodiments, the heteroaryl is substituted with two substituents. In further embodiments, the heteroaryl is substituted with one substituent. In yet other embodiments, the heteroaryl is substituted with three substituents. In still further embodiments, the heteroaryl is unsubstituted. Substitution may occur on any available carbon or heteroatom (*e.g*., nitrogen), or both, as permitted by substituent valency.

In some embodiments, the heteroaryl is a 5- or 6-membered heteroaryl. In some embodiments, the heteroaryl is a 5-membered heteroaryl, *i.e.,* the heteroaryl is a monocyclic aromatic ring system having 5 ring atoms wherein at least one carbon atom of the ring is replaced with a heteroatom independently selected from nitrogen, oxygen, and sulfur. Examples of 5-membered heteroaryl groups include thienyl, furyl, pyrrolyl, oxazolyl, pyrazolyl, imidazolyl, thiazolyl, isothiazolyl, and isoxazolyl and the like. In other embodiments, the heteroaryl is a 6-membered heteroaryl, *e.g.,* the heteroaryl is a monocyclic aromatic ring system having 6 ring atoms wherein at least one carbon atom of the ring is replaced with a nitrogen atom. Examples of 6-membered heteroaryl groups include pyridyl, pyrazinyl, pyrimidinyl, pyridazinyl and the like.

The term "heterocyclyl" as used by itself or as part of another group refers to non-aromatic, saturated or partially unsaturated, *e.g*., containing one or two double bonds, cyclic groups containing one, two, or three rings having from three to fourteen ring members, *i.e.,* a 3-14-membered heterocyclyl, wherein at least one carbon atom of one of the rings is replaced with a heteroatom. Each heteroatom is independently selected from oxygen, sulfur, including sulfoxide and sulfone, and/or nitrogen atoms, which can be oxidized or quaternized. The term "heterocyclyl" also includes groups wherein a ring -CH₂- is replaced with a -C(=O)-. The term "heterocyclyl" also includes groups having fused optionally substituted aryl groups, *e.g*., indolinyl or chroman-4-yl and groups having fused optionally substituted cycloalkyl groups, *e.g*., 6-azaspiro[2.5]octanyl. In some embodiments, the heterocyclyl group is a C₄₋₆heterocyclyl, *i.e.,* a 4-, 5- or 6-membered cyclic group, containing one ring and one or two oxygen and/or nitrogen atoms. In other embodiments, the heterocyclyl is a C₄₋₆heterocyclyl containing one ring and one nitrogen atom. The heterocyclyl can be optionally linked to the rest of the molecule through any available carbon or heteroatom that results in a stable structure. Examples of heterocyclyl groups include azetidinyl, dioxanyl, tetrahydropyranyl, pyrrolidinyl, piperidinyl, morpholinyl, piperazinyl, pyrrolidinyl, indolinyl, oxetanyl, tetrahydrofuranyl, tetrahydrothiophenyl, azepanyl, aziridinyl, dioxolanyl, imidazolidinyl, pyrazolidinyl, thianyl, dithianyl, thiomorpholinyl, oxazepanyl, oxiranyl, tetrahydropyranyl, and the like. In some embodiments, the heterocyclyl group includes azetidinyl, pyrrolidinyl, piperidinyl, piperazinyl, morpholinyl, and 6-azaspiro[2.5]octanyl. The heterocyclyl may be unsubstituted or substituted. In some embodiments, the heterocyclyl is substituted with two substituents. In further embodiments, the heterocyclyl is substituted with one substituent. In yet other embodiments, the heterocyclyl is substituted with three substituents. In still further embodiments, the heterocyclyl is unsubstituted.

The term "(heterocyclyl)alkylene" as used by itself or part of another group refers to an alkylene group as defined herein that is bound to a heterocyclyl group as defined herein.

The term "optionally substituted," as used herein to describe a chemical moiety defined herein, means that the moiety may, but is not required to be, substituted with one or more suitable functional groups or other substituents as provided herein. For example, a substituent may be optionally substituted with one or more of: halo, cyano, -NO₂, -N₃, - OH, -SH, C₁₋₆alkyl, C₃₋₈cycloalkyl, C₃₋₈cycloalkenyl, C₂₋₆alkenyl, C₂₋₆alkynyl, C₁₋₆haloalkyl, C₁₋₆alkoxy, C₁₋₆alkoxy(alkylene), C₁₋₆haloalkoxy, C₁₋₆haloalkoxy(alkylene), C₁₋₆alkylcarbonyl, C₁₋₆cyanoalkyl, C₁₋₆hydoxyalkyl, C₁₋₆alkylenethio, (CR^{v}R^{×})ₚNR^{y}R^{z} (wherein R^{v} and R^{x} are, independently, H or C₁₋₆alkyl; R^{y} and R^{z} are independently H, C₁₋₆alkyl, C₃₋₆cycloalkyl, C₁₋₆hydroxyalkyl, C₁₋₆haloalkyl, C₁₋₆alkoxy(alkylene), or C(O)OC₁₋₆alkyl, and p is 0, 1, 2, or 3), -C(O)NH₂, -C(O)NHC₁₋₆alkyl, -C(O)N(C₁₋₆alkyl)₂, -C(O)NHC₃₋₆cycloalkyl, - C(O)N(C₃₋₆cycloalkyl)₂, -COOH, -C₁₋₆alkyleneCOOH, -C₃₋₆cycloalkylCOOH, -C₁₋₆alkyleneCONH₂, C₃₋₆cycloalkylCONH₂, -C₁₋₆alkyleneCONHC₁₋₆alkyl, -C₁₋₆alkyleneCON(C₁₋₆alkyl)₂, -C(O)OC₁₋₆alkyl, -NHCO(C₁₋₆alkyl), -N(C₁₋₆alkyl)C(O)(C₁₋₆alkyl), -S(O)C₁₋₆alkyl, - S(O)C₃₋₆cycloalkyl, C₁₋₆alkylsulfonyl, C₃₋₈cycloalkylsulfonyl, C₁₋₆alkylsulfonyl(alkylene), oxo (=O), 3-7-membered heterocyclyl, heterocyclyl(alkylene), aryl, aryl(alkylene), or heteroaryl groups. In some embodiments, the C₁₋₆alkyl group in any of the substituent groups in this paragraph is a C₁₋₄alkyl; in other embodiments it is C₁₋₃alkyl. In some embodiments, the C₁₋₆alkylene group in any of the substituent groups in this paragraph is a C₁₋₄alkylene. In some embodiments, the C₁₋₆haloalkyl substituent is a C₁₋₄haloalkyl; in other embodiments, it is C₁₋₃haloalkyl. In some embodiments, the C₃₋₆cycloalkyl substituent is a C₃₋₄cycloalkyl substituent. In some embodiments, the C₁₋₆alkoxy substituent is a C₁₋₃alkoxy; in other embodiments, it is C₁₋₄alkoxy. In some embodiments, the C₁₋₆haloalkoxy substituent is a C₁₋₃haloalkoxy; in other embodiments, it is C₁₋₄haloalkoxy.

The term "nitrogen protecting group" refers to a moiety that is attached to a nitrogen atom to prevent reaction at that nitrogen atom. Nitrogen protecting groups will be known by those skilled in the art and include those described in Wuts, P.G., Greene's Protective Groups in Organic Synthesis. Wiley; 5th edition (October 27, 2014).

Recitation of ranges of values herein are intended to serve as a shorthand method of referring individually to each separate value falling within the range, unless otherwise indicated herein, and each separate value is incorporated into the specification as if it were individually recited herein.

The term "about" when used in combination with a numeric value or range of values means the value or range of values may deviate to an extent deemed reasonable to one of ordinary skill in the art.

Compounds described herein can comprise one or more asymmetric centers, and thus can exist in various stereoisomeric forms, *e.g*., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including supercritical fluid chromatography (SFC), chiral high-pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, E.L. Stereochemistry of Carbon Compounds (McGraw-Hill, NY, 1962); and Wilen, S.H. Tables of Resolving Agents and Optical Resolutions p. 268 (E.L. Eliel, Ed., Univ. of Notre Dame Press, Notre Dame, IN 1972).

Exemplary compounds of the disclosure including a chiral center may be depicted herein as having particular stereochemistries, but for which absolute stereochemistry has not been obtained. Absolute configurations can be obtained using methods known in the art.

As used herein, the term "stereoisomers" refers to compounds which have identical chemical constitution and connectivity, but differ with regard to the arrangement of the atoms or groups in space, e.g., enantiomers or diastereomers.

When the stereochemical configuration at a chiral center in a compound having one or more chiral centers is depicted by its chemical name (e.g., where the configuration is indicated in the chemical name by "R" or "S") or structure (e.g., the configuration is indicated by dashed or wedge bonds), the enrichment of the indicated configuration relative to the opposite configuration is greater than 50%, 60%, 70%, 80%, 90%, 99% or 99.9%. "Enrichment of the indicated configuration relative to the opposite configuration" is a mole percent and is determined by dividing the number of compounds with the indicated stereochemical configuration at the chiral center(s) by the total number of all of the compounds with the same or opposite stereochemical configuration in a mixture.

When a disclosed compound is named or depicted by structure without indicating stereochemistry, it is understood that the name or the structure encompasses one of the possible stereoisomers or geometric isomers free of the others, or a mixture of the encompassed stereoisomers or geometric isomers.

It will be understood that certain compounds disclosed herein may exist in tautomeric forms. Such forms are included as part of the present disclosure. Thus, when a compound herein is represented by a structural formula or designated by a chemical name herein, all tautomeric forms which may exist for the compound are encompassed by the structural formula.

When the compounds described herein contain olefinic double bonds or other centers of geometric asymmetry, and unless specified otherwise, it is intended that they include both E and Z geometric isomers.

In some embodiments, the compounds described herein are isotopically enriched compounds, e.g., an isotopologue. The term "isotopically enriched" refers to an atom having an isotopic composition other than the naturally abundant isotopic composition of that atom. "Isotopically enriched" may also refer to a compound containing at least one atom having an isotopic composition other than the natural isotopic composition of that atom. In an isotopologue, "isotopic enrichment" refers to the percentage of incorporation of an amount of a specific isotope of a given atom in a molecule in the place of that atom's natural isotopic composition. For example, deuterium enrichment of 1% at a given position means that 1% of the molecules in a given sample contain deuterium at the specified position. Because the naturally occurring distribution of deuterium is about 0.0156%, deuterium enrichment at any position in a compound synthesized using non-enriched starting materials is about 0.0156%. In one embodiment, one or more hydrogen atoms on a described compound may be replaced by deuterium.

Thus, as used herein, and unless otherwise indicated, the term "isotopic enrichment factor" refers to the ratio between the isotopic composition and the natural isotopic composition of a specified isotope.

With regard to the compounds provided herein, when a particular atom's position is designated as having deuterium or "D" or "²H", it is understood that the abundance of deuterium at that position is substantially greater than the natural abundance of deuterium, which is about 0.015%. A position designated as having deuterium typically has a minimum isotopic enrichment factor of, in particular embodiments, at least 1000 (15% deuterium incorporation), at least 2000 (30% deuterium incorporation), at least 3000 (45% deuterium incorporation), at least 3500 (52.5% deuterium incorporation), at least 4000 (60% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation) at each designated deuterium atom. The isotopic enrichment and isotopic enrichment factor of the compounds provided herein can be determined using conventional analytical methods known to one of ordinary skill in the art, including mass spectrometry and nuclear magnetic resonance spectroscopy.

The term "patient" or "subject" is used throughout the specification to describe an animal, preferably a human or a domesticated animal, to whom treatment, including prophylactic treatment, with the compounds or compositions according to the present disclosure is provided. For treatment of those conditions or disease states which are specific for a specific animal such as a human patient, the term patient refers to that specific animal, including a domesticated animal such as a dog or cat or a farm animal such as a horse, cow, sheep, etc. In general, in the present disclosure, the term patient refers to a human patient unless otherwise stated or implied from the context of the use of the term.

The terms "therapeutically effective amount" or "effective amount" means an amount or dose of a compound of the disclosure (or a pharmaceutically acceptable salt thereof) sufficient to generally bring about the desired therapeutic benefit in subjects in need of such treatment for the designated disease or disorder. Further, a therapeutically effective amount with respect to a compound of the disclosure means that amount of therapeutic agent alone, or in combination with other therapies, that provides a therapeutic benefit in the treatment or prevention of a disease.

"Treating" or "treatment" of any disease or disorder refers, in one embodiment, to ameliorating the disease or disorder (e.g., arresting or reducing the development of the disease or at least one of the clinical symptoms thereof). In another embodiment "treating" or "treatment" refers to ameliorating at least one physical parameter, which may not be discernible by the subject. In yet another embodiment, "treating" or "treatment" refers to modulating the disease or disorder, either physically (e.g., stabilization of a discernible symptom), physiologically (e.g., stabilization of a physical parameter), or both. In yet another embodiment, "treating" or "treatment" refers to delaying the onset of the disease or disorder.

The terms "prevent," "preventing," and "prevention" refer to the prevention of the onset, recurrence, or spread of the disease in a subject resulting from the administration of a prophylactic or therapeutic agent.

### The Compounds

The present disclosure provides compounds of Formula I or pharmaceutically acceptable salts thereof:

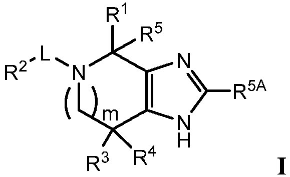

In Formula **I,** m is 0, 1, or 2. In other embodiments, m is 1 or 2. In some embodiments, m is 1. In other embodiments, m is 2. In yet other embodiments, m is 0.

In formula **I,** R¹ is 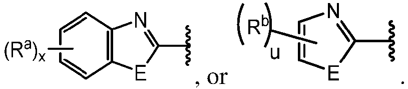 In some embodiments, R¹ is 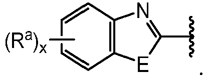 In other embodiments, R¹ is

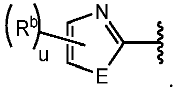

In the structures for R¹, u is 0 to 2. In some embodiments, u is 0. In other embodiments, u is 1. In further embodiments, u is 2.

In the structures for R¹, x is 0 to 4. In some embodiments, x is 0. In other embodiments, x is 1. In further embodiments, x is 2. In yet other embodiments, x is 3. In still further embodiments, x is 4.

In the structures for R¹, E is O.

In the structures for R¹, each R^{a} is independently halo, C₁₋₆alkyl, C₃₋₆cycloalkyl, C₁₋₆haloalkyl, C₁₋₆alkoxy or C₁₋₆haloalkoxy. In some embodiments, R^{a} is halo such as F, Cl, Br, or I. In other embodiments, R^{a} is F, Br, or Cl. In still other embodiments, R^{a} is F. In further embodiments, R^{a} is Br. In yet other embodiments, R^{a} is Cl. In still further embodiments, R^{a} is I. In other embodiments, R^{a} is C₁₋₆alkyl such as methyl, ethyl, propyl, butyl, pentyl, or hexyl. In yet other embodiments, R^{a} is methyl. In further embodiments, R^{a} is C₃₋₆cycloalkyl such as cyclopropyl, cyclobutyl, cyclopentyl, or cyclohexyl. In still further embodiments, R^{a} is cyclopropyl. In yet other embodiments, R^{a} is C₁₋₆haloalkyl such as CF₃, CHF₂, CH₂F, CH₂CF₃, C(CH₃)₂F, or C(CH₃)F₂. In still other embodiments, R^{a} is CF₃ or CHF₂. In yet other embodiments, R^{a} is CF₃. In further embodiments, R^{a} is C₁₋₆alkoxy such as methoxy, ethoxy, propoxy, butoxy, pentoxy, or hexoxy. In yet other embodiments, R^{a} is methoxy or ethoxy. In still further embodiments, R^{a} is methoxy. In other embodiments, R^{a} is C₁₋₆haloalkoxy such as OCF₃ or OCH₂CF₃. In yet other embodiments, R^{a} is OCF₃. In still other embodiments, one R^{a} is halo such as Br, Cl, or F or C₁₋₆alkyl such as methyl, and the second R^{a} is halo such as Br, Cl, or F, C₁₋₆alkoxy such as methoxy, or C₁₋₆alkyl such as methyl.

In the structures for R¹, each R^{b} is independently optionally substituted aryl, C₁₋₆alkyl, C₃₋₆cycloalkyl, or halo. In some embodiments, R^{b} is halo such as F, Cl, Br, or I. In other embodiments, R^{b} is F, Br, or Cl. In still other embodiments, R^{b} is F. In yet other embodiments, R^{b} is Br. In still other embodiments, R^{b} is Cl. In yet other embodiments, R^{b} is I. In other embodiments, R^{b} is C₁₋₆alkyl such as methyl, ethyl, propyl, butyl, pentyl, or hexyl. In yet other embodiments, R^{b} is methyl. In further embodiments, R^{b} is C₃₋₆cycloalkyl such as cyclopropyl, cyclobutyl, cyclopentyl, or cyclohexyl. In still further embodiments, R^{b} is cyclopropyl. In further embodiments, R^{b} is optionally substituted aryl such as phenyl. In some embodiments, R^{b} is aryl, substituted with one or more of halo, C₁₋₆alkyl, C₁₋₆haloalkyl, C₁₋₆alkoxy, or C₃₋₈cycloalkyl. In other embodiments, R^{b} is unsubstituted phenyl.

In further embodiments, R¹ is 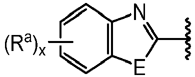 and x is 0 or 1 such as 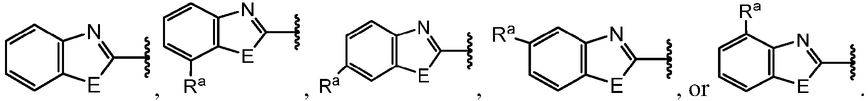 In other embodiments, R¹ is 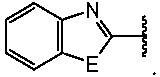 In yet other embodiments, R¹ is 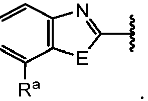 In still other embodiments, R¹ is 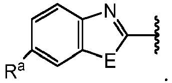 In yet other embodiments, R¹ is 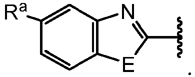 In still other embodiments, R¹ is

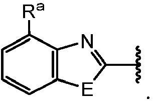

In further embodiments, R¹ is 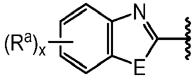 and x is 2 such as 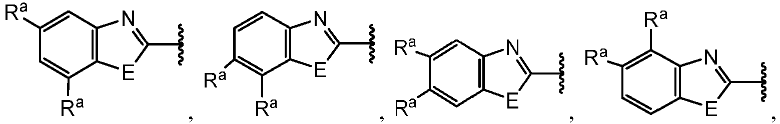 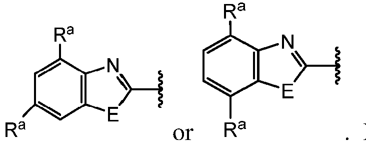 In other embodiments, R¹ is 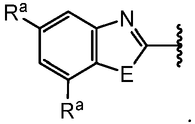 In yet other embodiments, R¹ is 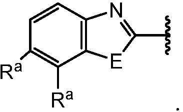 In still other embodiments, R¹ is 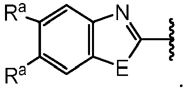 In yet other embodiments, R¹ is 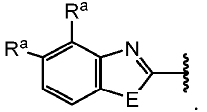 In still other embodiments, R¹ is 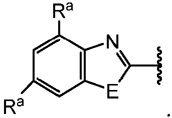 In yet other embodiments, R¹ is

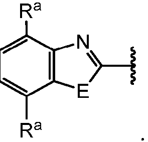

In further embodiments, R¹ is 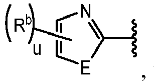 wherein R^{b} is phenyl or halo and u is 1. In some embodiments, R^{b} is phenyl. In other embodiments, R^{b} is halo.

In Formula **I,** R² is C₁₋₄alkyl, optionally substituted C₃₋₈cycloalkyl, optionally substituted heterocyclyl, optionally substituted aryl, or optionally substituted heteroaryl. In further embodiments, R² is optionally substituted C₃₋₈cycloalkyl, optionally substituted heterocyclyl, optionally substituted aryl, or optionally substituted heteroaryl. In still further embodiments, R² is unsubstituted C₃₋₈cycloalkyl, unsubstituted heterocyclyl, unsubstituted aryl, or unsubstituted heteroaryl. In other embodiments, R² is substituted C₃₋₈cycloalkyl, substituted heterocyclyl, substituted aryl, or substituted heteroaryl. In some embodiments, R² is C₁₋₄alkyl such as methyl, ethyl, propyl, butyl, or tert-butyl. In other embodiments, R² is optionally substituted C₃₋₈cycloalkyl such as cyclopropyl, cyclobutyl, cyclopentyl, cyclohexyl, cycloheptyl, or cyclooctyl. In further embodiments, R² is optionally substituted heterocyclyl such as azetidinyl. In still other embodiments, R² is optionally substituted aryl such as phenyl. In yet further embodiments, R² is optionally substituted heteroaryl such as pyridinyl, pyrazolyl, oxazolyl, isoxazolyl, thiazolyl, isothiazolyl, triazolyl, oxadiazolyl, thiadiazolyl, furanyl, thiophenyl, pyrimidinyl, pyrazinyl, indazolyl, pyrazolo[1,5-a]pyridinyl, imidazol[1,5-a]pyridinyl, pyrrolo[1,2-b]pyridazinyl, benzo[d]thiazolyl, benzo[d]isothiazolyl, benzo[d]imidazolyl, benzo[d]oxazolyl, or benzo[d]isoxazolyl.

In Formula **I,** R³ is H or C₁₋₆alkyl. In some embodiments, R³ is H. In other embodiments, R³ is C₁₋₆alkyl such as methyl, ethyl, propyl, butyl, pentyl, or hexyl. In further embodiments, R³ is methyl. In yet other embodiments, R³ is ethyl. In still further embodiments, R³ is propyl. In other embodiments, R³ is butyl. In further embodiments, R³ is pentyl. In yet other embodiments, R³ is hexyl.

In Formula **I,** R⁴ is H or C₁₋₆alkyl. In some embodiments, R⁴ is H. In other embodiments, R⁴ is C₁₋₆alkyl such as methyl, ethyl, propyl, butyl, pentyl, or hexyl. In further embodiments, R⁴ is methyl. In yet other embodiments, R⁴ is ethyl. In still further embodiments, R⁴ is propyl. In other embodiments, R⁴ is butyl. In further embodiments, R⁴ is pentyl. In yet other embodiments, R⁴ is hexyl.

Alternatively, R³ and R⁴, together with the atom to which they are attached, form a C₃₋₆cycloalkyl. In some embodiments, R³ and R⁴ together form a cyclopropyl. In other embodiments, R³ and R⁴ together form a cyclobutyl. In further embodiments, R³ and R⁴ together form a cyclopentyl. In yet other embodiments, R³ and R⁴ together form a cyclohexyl.

In some embodiments, both R³ and R⁴ are H. In other embodiments, R³ is methyl and R⁴ is H. In still other embodiments, both R³ and R⁴ are methyl.

In Formula **I,** R⁵ is H or D. In some embodiments, R⁵ is H. In further embodiments, R⁵ is D.

In Formula **I,** R^{5A} is H or D. In some embodiments, R^{5A} is H. In further embodiments, R^{5A} is D.

In Formula **I,** L is a bond, carbonyl, optionally substituted C₁₋₆alkylene, optionally substituted C₁₋₆alkylenecarbonyl, optionally substituted C₂₋₆alkenylenecarbonyl, optionally substituted C₁₋₆haloalkylenecarbonyl, or optionally substituted -C(O)NR^{c}(C₁₋₆alkylene)-, wherein the carbon atom of the carbonyl group is connected to N in Formula **I.** In some embodiments, L is a bond. In other embodiments, L is carbonyl (wherein the carbon atom of the carbonyl group is connected to N in Formula **I**). In further embodiments, L is optionally substituted C₁₋₆alkylene such as methylene, ethylene, propylene, butylene, pentylene, or hexylene. In other embodiments, L is C₁alkylene. In still other embodiments, L is C₂alkylene. In further embodiments, L is C₃alkylene. In yet other embodiments, L is C₄alkylene. In still further embodiments, L is C₅alkylene. In other embodiments, L is C₆alkylene. In yet other embodiments, L is optionally substituted C₁₋₆alkylenecarbonyl (wherein the carbon atom of the carbonyl group is connected to N in Formula **I**). In further embodiments, L is -C₁alkylene-C(O)-. In other embodiments, L is -C₂alkylene-C(O)-. In further embodiments, L is -C₃alkylene-C(O)-. In yet other embodiments, L is -C₄alkylene-C(O)-. In still further embodiments, L is -C₅alkylene-C(O)-. In other embodiments, L is - C₆alkylene-C(O)-. In still further embodiments, L is optionally substituted C₂₋₆alkenylenecarbonyl (wherein the carbon atom of the carbonyl group is connected to N in Formula **I**). In other embodiments, L is -C₂alkenylene-C(O)-. In further embodiments, L is - C₃alkenylene-C(O)-. In yet other embodiments, L is -C₄alkenylene-C(O)-. In still further embodiments, L is -C₅alkenylene-C(O)-. In other embodiments, L is -C₆alkenylene-C(O)-. In other embodiments, L is optionally substituted C₁₋₆haloalkylenecarbonyl (wherein the carbon atom of the carbonyl group is connected to N in Formula **I**). In yet other embodiments, L is - C₁haloalkylene-C(O)-. In other embodiments, L is -C₂haloalkylene-C(O)-. In further embodiments, L is -C₃haloalkylene-C(O)-. In yet other embodiments, L is -C₄haloalkylene-C(O)-. In still further embodiments, L is -C₅haloalkylene-C(O)-. In other embodiments, L is - C₆haloalkylene-C(O)-. In further embodiments, L is -C(O)NR^{c}(C₁₋₆alkylene)- (wherein the carbon atom of the carbonyl group is connected to N in Formula **I**). In yet other embodiments, L is -C(O)NR^{c}C₁alkylene-. In other embodiments, L is -C(O)NR^{c}C₂alkylene-. In further embodiments, L is -C(O)NR^{c}C₃alkylene-. In yet other embodiments, L is - C(O)NR^{c}C₄alkylene-. In still further embodiments, L is -C(O)NR^{c}C₅alkylene-. In other embodiments, L is -C(O)NR^{c}C₆alkylene-.

In the structures for L, R^{c} is H or C₁₋₆alkyl. In some embodiments, R^{c} is H. In other embodiments, R^{c} is C₁₋₆alkyl, such as methyl, ethyl, propyl, butyl, pentyl, or hexyl. In further embodiments, R^{c} is methyl. In yet other embodiments, R^{c} is ethyl. In still further embodiments, R^{c} is propyl. In other embodiments, R^{c} is butyl. In further embodiments, R^{c} is pentyl. In yet other embodiments, R^{c} is hexyl.

In some embodiments, L is a bond, -C(O)-, -C(O)CH₂-, -C(O)CH₂CH₂-, -C(O)CH₂CH₂CH₂-, -C(O)CF₂-, -C(O)CHF-, -C(O)C(CH₃)₂-, -C(O)CH=CH-, 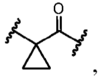 -C(O)NHCH₂-, -CH₂-, or -CH₂CH₂-. In yet other embodiments, L is a bond, -C(O)-, -C(O)CH₂-, -C(O)CH₂CH₂-, or

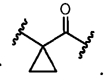

In some embodiments, R² is cyclopentyl, cyclobutyl, cyclopropyl, azetidinyl, phenyl, pyrazolyl, oxazolyl, thiazolyl, triazolyl, oxadiazolyl, pyridinyl, pyrimidinyl, pyrazolo[1,5-a]pyridinyl, indazolyl, thiadiazolyl, imidazol[1,5-a]pyridinyl, pyrrolo[1,2]pyridazinyl, thiophenyl, isoxazolyl, isothiazolyl, benzo[d]thiazolyl, benzo[d]imidazolyl, benzo[d]oxazolyl, benzo[d]isoxazolyl, benzo[d]isothiazolyl, furanyl, or pyrazinyl, each of which is optionally substituted.

In further embodiments, R² is cyclopentyl, cyclobutyl, cyclopropyl, cyclohexyl, azetidinyl, phenyl, pyrazolyl, oxazolyl, thiazolyl, triazolyl, oxadiazolyl, pyridinyl, pyrimidinyl, pyrazolo[1,5-a]pyridinyl, indazolyl, thiadiazolyl, imidazol[1,5-a]pyridinyl, pyrrolo[1,2]pyridazinyl, thiophenyl, isoxazolyl, isothiazolyl, benzo[d]thiazolyl, benzo[d]imidazolyl, benzo[d]oxazolyl, benzo[d]isoxazolyl, benzo[c]isoxazolyl, benzo[d]isothiazolyl, furanyl, pyrazinyl or quinolinyl, each of which is optionally substituted.

In other embodiments, R² is cyclopentyl, cyclobutyl, cyclopropyl, azetidinyl, phenyl, pyrazolyl, oxazolyl, thiazolyl, triazolyl, oxadiazolyl, pyridinyl, pyrimidinyl, pyrazolo[1,5-a]pyridinyl, indazolyl, thiadiazolyl, imidazol[1,5-a]pyridinyl, pyrrolo[1,2]pyridazinyl, thiophenyl, isoxazolyl, isothiazolyl, benzo[d]thiazolyl, benzo[d]imidazolyl, benzo[d]oxazolyl, benzo[d]isoxazolyl, benzo[d]isothiazolyl, furanyl, or pyrazinyl, each of which is unsubstituted.

In yet other embodiments, R² is cyclopentyl, cyclobutyl, cyclopropyl, cyclohexyl, azetidinyl, phenyl, pyrazolyl, oxazolyl, thiazolyl, triazolyl, oxadiazolyl, pyridinyl, pyrimidinyl, pyrazolo[1,5-a]pyridinyl, indazolyl, thiadiazolyl, imidazol[1,5-a]pyridinyl, pyrrolo[1,2]pyridazinyl, thiophenyl, isoxazolyl, isothiazolyl, benzo[d]thiazolyl, benzo[d]imidazolyl, benzo[d]oxazolyl, benzo[d]isoxazolyl, benzo[c]isoxazolyl, benzo[d]isothiazolyl, furanyl, pyrazinyl or quinolinyl, each of which is unsubstituted.

In further embodiments, R² is optionally substituted cyclopentyl. In further embodiments, R² is optionally substituted cyclobutyl. In still other embodiments, R² is optionally substituted cyclopropyl. In yet other embodiments, R² is optionally substituted cyclohexyl. In yet further embodiments, R² is optionally substituted azetidinyl. In other embodiments, R² is optionally substituted phenyl. In further embodiments, R² is optionally substituted pyrazolyl. In still other embodiments, R² is optionally substituted oxazolyl. In yet further embodiments, R² is optionally substituted thiazolyl. In other embodiments, R² is optionally substituted triazolyl. In further embodiments, R² is optionally substituted oxadiazolyl. In still other embodiments, R² is optionally substituted pyridinyl. In yet further embodiments, R² is optionally substituted pyrimidinyl. In other embodiments, R² is optionally substituted pyrazolo[1,5-a]pyridinyl. In further embodiments, R² is optionally substituted indazolyl. In yet other embodiments, R² is optionally substituted thiadiazolyl. In other embodiments, R² is optionally substituted imidazol[1,5-a]pyridinyl. In further embodiments, R² is optionally substituted pyrrolo[1,2]pyridazinyl. In still other embodiments, R² is optionally substituted thiophenyl. In yet further embodiments, R² is optionally substituted isoxazolyl. In further embodiments, R² is optionally substituted isothiazolyl. In other embodiments, R² is optionally substituted benzo[d]thiazolyl. In further embodiments, R² is optionally substituted benzo[d]imidazolyl. In still other embodiments, R² is optionally substituted benzo[d]oxazolyl. In yet further embodiments, R² is benzo[d]isoxazolyl. In yet other embodiments, R² is optionally substituted benzo[c]isoxazolyl. In still further embodiments, R² is optionally substituted benzo[d]isothiazolyl. In other embodiments, R² is optionally substituted furanyl. In yet other embodiments, R² is optionally substituted pyrazinyl. In still further embodiments, R² is optionally substituted quinolinyl.

In other embodiments, R² is azetidin-1-yl, azetidin-3-yl, pyrazol-1-yl, pyrazol-3-yl, pyrazol-4-yl, pyrazol-5-yl, 1,2,4-triazol-1-yl, 1,2,4-triazol-3-yl, 1,2,4-triazol-5-yl, 1,2,3-triazol-5-yl, 1,3,4-oxadiazol-2-yl, 1,2,4-oxadiazol-3-yl, 1,2,4-oxadiazol-5-yl, pyrazolo[1,5-a]pyridin-3-yl, indazol-3-yl, thiazol-2-yl, thiazol-4-yl, thiazol-5-yl, oxazol-2-yl, oxazol-4-yl, oxazol-5-yl, 1,3,4-thiadiazol-2-yl, pyridin-1-yl, pyridin-2-yl, pyridin-3-yl, pyridin-4-yl, pyrimidin-2-yl, imidazol[1,5-a]pyridin-1-yl, pyrrolo[1,2]pyridazin-5-yl, pyrrolo[1,2]pyridazin-6-yl, thiophen-2-yl, isoxazol-3-yl, isoxazol-4-yl, isoxazol-5-yl, benzo[d]thiazol-2-yl, benzo[d]thiazol-3-yl, benzo[d]imidazol-2-yl, benzo[d]oxazol-2-yl, benzo[d]isoxazol-3-yl, benzo[d]isothiazol-3-yl, furan-3-yl, isothiazol-3-yl, isothiazol-4-yl, isothiazol-5-yl, or pyrazin-2-yl, each of which is optionally substituted.

In yet other embodiments, R² is azetidin-1-yl, azetidin-3-yl, pyrazol-1-yl, pyrazol-3-yl, pyrazol-4-yl, pyrazol-5-yl, 1,2,4-triazol-1-yl, 1,2,4-triazol-3-yl, 1,2,4-triazol-5-yl, 1,2,3-triazol-5-yl, 1,3,4-oxadiazol-2-yl, 1,2,4-oxadiazol-3-yl, 1,2,4-oxadiazol-5-yl, pyrazolo[1,5-a]pyridin-3-yl, indazol-3-yl, thiazol-2-yl, thiazol-4-yl, thiazol-5-yl, oxazol-2-yl, oxazol-4-yl, oxazol-5-yl, 1,3,4-thiadiazol-2-yl, pyridin-1-yl, pyridin-2-yl, pyridin-3-yl, pyridin-4-yl, pyrimidin-2-yl, imidazol[1,5-a]pyridin-1-yl, pyrrolo[1,2]pyridazin-5-yl, pyrrolo[1,2]pyridazin-6-yl, thiophen-2-yl, isoxazol-3-yl, isoxazol-4-yl, isoxazol-5-yl, benzo[d]thiazol-2-yl, benzo[d]thiazol-3-yl, benzo[d]imidazol-2-yl, benzo[d]oxazol-2-yl, benzo[d]isoxazol-3-yl, benzo[d]isothiazol-3-yl, furan-3-yl, isothiazol-3-yl, isothiazol-4-yl, isothiazol-5-yl, pyrazin-2-yl, benzo[c]isoxazole-3-yl, or quinolin-3-yl, each of which is optionally substituted.

In further embodiments, R² is C₃₋₈cycloalkyl, heterocyclyl, aryl, or heteroaryl, each of which is substituted with one or more of C₁₋₆alkyl, optionally substituted C₂₋₆alkenyl, halo, CN, C₁₋₆cyanoalkyl, C₁₋₆haloalkyl, OH, optionally substituted C₃₋₈cycloalkyl, optionally substituted C₃₋₈cycloalkyl(alkylene), optionally substituted C₃₋₈cycloalkenyl, optionally substituted aryl, optionally substituted aryl(alkylene), optionally substituted heteroaryl, optionally substituted heterocyclyl, optionally substituted heterocyclyl(alkylene), C₁₋₆hydroxyalkyl, C₁₋₆haloalkoxy, C₁₋₆haloalkoxy(alkylene), C₁₋₆alkoxy, C₁₋₆alkoxy(alkylene), C₁₋₆deuteratedalkoxy(alkylene), C₁₋₆alkylcarbonyl, C₃₋₈cycloalkylsulfonyl, C₁₋₆alkylsulfonyl, C₁₋₆alkylsulfonyl(alkylene), (CR^{v}R^{x})ₚNR^{y}R^{z} (wherein R^{v} and R^{x} are, independently, H or C₁₋₆alkyl; R^{y} and R^{z}, are independently, H, C₁₋₆alkyl, C₃₋₆cycloalkyl, C₁₋₆hydroxyalkyl, C₁₋₆haloalkyl, C₁₋₆alkoxy(alkylene), or C(O)OC₁₋₆alkyl and p is 0, 1, 2, or 3), or C(O)NR^{y2}R^{z2} (wherein R^{y2} and R^{z2}, are independently, H, C₁₋₆alkyl, or C₃₋₆cycloalkyl). In other embodiments, R² is optionally substituted with C₁₋₆alkyl, such as methyl, ethyl, propyl, isopropyl, or tert-butyl. In yet further embodiments, R² is optionally substituted with C₂₋₆alkenyl such as CH=CH₂, CH=CHC(CH₃)₂OH, or CH=CH-cyclopropyl. In further embodiments, R² is optionally substituted with halo such as Br, Cl, or F. In yet other embodiments, R² is optionally substituted with CN. In still further embodiments, R² is optionally substituted with C₁₋₆cyanoalkyl such as C(CH₃)₂CN. In other embodiments, R² is optionally substituted with C₁₋₆haloalkyl, such as CF₃, CHF₂, CH₂F, CH(CH₃)F, CH₂CF₃, C(CH₃)₂F, C(CH₃)F₂, or CH₂CHF₂. In further embodiments, R² is optionally substituted with OH. In still other embodiments, R² is optionally substituted with optionally substituted C₃₋₈cycloalkyl, such as optionally substituted cyclopropyl, optionally substituted cyclobutyl, optionally substituted cyclopentyl, or optionally substituted cyclohexyl. In further embodiments, R² is optionally substituted with optionally substituted C₃₋₈cycloalkyl(alkylene) such as optionally substituted cyclopropyl(alkylene) or optionally substituted cyclobutyl(alkylene). In other embodiments, R² is optionally substituted with optionally substituted C₃₋₈cycloalkenyl, such as optionally substituted cyclohexenyl. In yet further embodiments, R² is optionally substituted with optionally substituted aryl such as optionally substituted phenyl. In further embodiments, R² optionally substituted with optionally substituted aryl(alkylene) such as optionally substituted benzyl. In other embodiments, R² is optionally substituted with optionally substituted heteroaryl, such optionally substituted pyrazolyl, optionally substituted imidazolyl, optionally substituted pyridinyl, optionally substituted pyrimidinyl, or optionally substituted pyrazinyl. In yet other embodiments, R² is optionally substituted with optionally substituted heteroaryl, such optionally substituted pyrazolyl, optionally substituted imidazolyl, optionally substituted pyridinyl, optionally substituted pyrimidinyl, optionally substituted pyrazinyl, or optionally substituted pyridazinyl. In further embodiments, R² is optionally substituted with optionally substituted heterocyclyl such as optionally substituted azetidinyl, optionally substituted piperidinyl, optionally substituted piperazinyl, optionally substituted pyrrolidinyl, optionally substituted morpholinyl, or optionally substituted 6-azaspiro[2.5]octan-6-yl. In yet further embodiments, R² is optionally substituted with optionally substituted morpholinyl(alkylene), optionally substituted piperidinyl(alkylene), optionally substituted piperazinyl(alkylene), or optionally substituted azetidinyl(alkylene). In still other embodiments, R² is optionally substituted with C₁₋₆hydroxyalkyl such as C(CH₃)₂OH, CH(CH₃)OH, C(CH₃)₂CH₂OH, CH₂C(CH₃)₂OH, or CH(CH₂CH₃)OH. In yet further embodiments, R² is optionally substituted with C₁₋₆haloalkoxy such as OCF₃, OCH₂CF₃, or OCH₂CH₂CF₃. In still further embodiments, R² is optionally substituted with C₁₋₆haloalkoxy(alkylene) such as CH₂OCF₃. In other embodiments, R² is optionally substituted with C₁₋₆alkoxy, such as methoxy or ethoxy. In yet other embodiments, R² is optionally substituted with C₁₋₆alkoxy(alkylene), such as C(CH₃)₂OCH₃, CH₂OCH₃, or (CH₂)₂OCH₃. In further embodiments, R² is optionally substituted with C₁₋₆deuteratedalkoxy(alkylene) such as CH₂OCD₃. In further embodiments, R² is optionally substituted with C₁₋₆alkylcarbonyl, such as C(=O)CH₃ or CH₂C(=O)CH₃. In yet other embodiments, R² is optionally substituted with C₃₋₈cycloalkylsulfonyl such as cyclopropylsulfonyl, cyclobutylsulfonyl, or cyclopentylsulfonyl. In other embodiments, R² is optionally substituted with C₁₋₆alkylsulfonyl such as methylsulfonyl, ethylsulfonyl, or propylsulfonyl. In yet other embodiments, R² is optionally substituted with C₁₋₆alkylsulfonyl(alkylene), such as C(CH₃)₂SO₂CH₃. In still further embodiments, R² is optionally substituted with (CR^{v}R^{x})ₚNR^{y}R^{z}, wherein R^{v}, R^{x}, R^{y}, R^{z}, and p are defined above, such as NH₂, NHcyclopropyl, NHCH₃, N(CH₃)₂, CH₂N(CH₃)₂, (CH₂)₂N(CH₃)₂, CH₂N(CH₃)(CH₂CH₃), C(CH₃)₂NH(CH₃), C(CH₃)₂N(CH₃)₂, CH₂N(CH₃)cyclobutyl, or CH₂N(CH₃)(C(O)Otert-butyl). In some embodiments, p is 0. In other embodiments, p is 1. In further embodiments, p is 2. In still other embodiments, p is 3. In some embodiments, R^{v} and R^{x} are, independently, hydrogen or methyl. In other embodiments, R^{y} and R^{z} are, independently, hydrogen, methyl, ethyl, cyclopropyl, cyclobutyl, C(O)Omethyl, C(O)Oethyl, C(O)Opropyl, or C(O)Otert-butyl. In other embodiments, R² is C(O)NR^{y2}R^{z2}, wherein R^{y2} and R^{z2} are defined above, such as C(O)N(CH₃)₂ or C(O)NHcyclopropyl. In yet other embodiments, R^{y2} and R^{z2} are, independently, hydrogen, methyl, or cyclopropyl. In further embodiments, R² is substituted with one or more of methyl, ethyl, propyl, isopropyl, tert-butyl, CH=CH₂, CH=CHC(CH₃)₂OH, CH=CH-cyclopropyl, Br, Cl, F, CN, C(CH₃)₂CN, CF₃, CHF₂, CH₂F, CH(CH₃)F, CH₂CF₃, C(CH₃)₂F, C(CH₃)F₂, CH₂CHF₂, OH, optionally substituted cyclopropyl, optionally substituted cyclobutyl, optionally substituted cyclopentyl, optionally substituted cyclohexyl, optionally substituted cyclohexenyl, optionally substituted cyclopropyl(alkylene), optionally substituted cyclobutyl(alkylene), optionally substituted phenyl, optionally substituted benzyl, optionally substituted pyrazolyl, optionally substituted imidazolyl, optionally substituted pyridinyl, optionally substituted pyrimidinyl, optionally substituted pyrazinyl, optionally substituted azetidinyl, optionally substituted piperidinyl, optionally substituted piperazinyl, optionally substituted pyrrolidinyl, optionally substituted morpholinyl, optionally substituted 6-azaspiro[2.5]octan-6-yl, optionally substituted morpholinyl(alkylene), optionally substituted piperidinyl(alkylene), optionally substituted piperazinyl(alkylene), or optionally substituted azetidinyl(alkylene), C(CH₃)₂OH, CH(CH₃)OH, C(CH₃)₂CH₂OH, CH₂C(CH₃)₂OH, CH(CH₂CH₃)OH, OCF₃, OCH₂CF₃, OCH₂CH₂CF₃, CH₂OCF₃, methoxy, ethoxy, C(CH₃)₂OCH₃, CH₂OCH₃, (CH₂)₂OCH₃, CH₂OCD₃, C(=O)CH₃, CH₂C(=O)CH₃, cyclopropylsulfonyl, cyclobutylsulfonyl, cyclopentylsulfonyl, C(CH₃)₂SO₂CH₃, NH₂, NHcyclopropyl, NHCH₃, N(CH₃)₂, CH₂N(CH₃)₂, (CH₂)₂N(CH₃)₂, CH₂N(CH₃)(CH₂CH₃), C(CH₃)₂NH(CH₃), C(CH₃)₂N(CH₃)₂, CH₂N(CH₃)cyclobutyl, CH₂N(CH₃)(C(O)Otert-butyl), C(O)N(CH₃)₂, or C(O)NHcyclopropyl.

In yet further embodiments, R² is 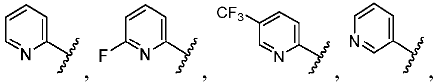 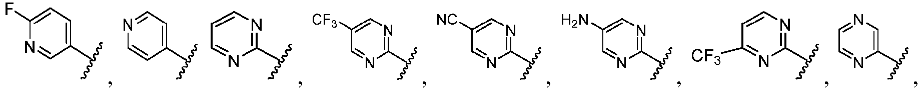 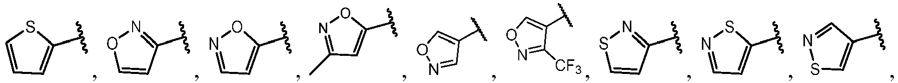 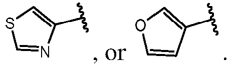 In other embodiments, R² is 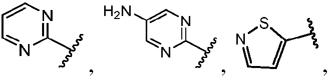 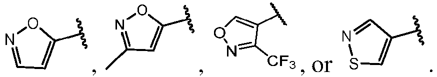 In yet other embodiments, R² is

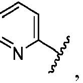

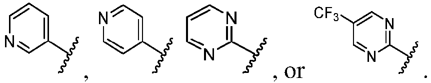

In some embodiments, R² is heterocyclyl, optionally substituted with one or more of halo, C₁₋₆haloalkyl, or optionally substituted heteroaryl. In still other embodiments, R² is 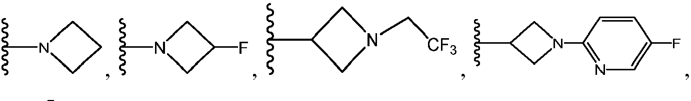 or 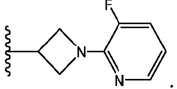 In yet other embodiments, R² is heterocyclyl, substituted with one or more of halo, C₁₋₆haloalkyl, or optionally substituted heteroaryl. In further embodiments, R² is 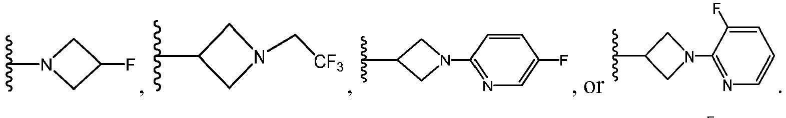 In still further embodiments, R² is

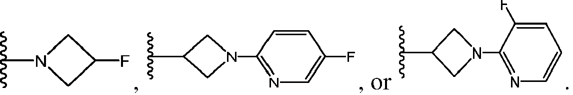

In some embodiments, R² is C₃₋₈cycloalkyl, optionally substituted with one or more of halo, C₁₋₆alkyl, C₁₋₆haloalkyl, or OH. In yet other embodiments, R² is unsubstituted cyclopropyl, unsubstituted cyclobutyl, unsubstituted cyclopentyl, unsubstituted cyclohexyl, 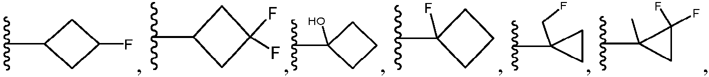 or 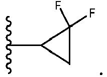 In further embodiments, R² is C₃₋₈cycloalkyl, substituted with one or more of halo, C₁₋₆alkyl, C₁₋₆haloalkyl, or OH. In still further embodiments, R² is 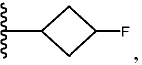 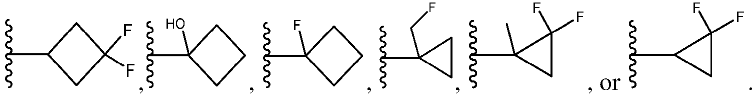 In other embodiments, R² is 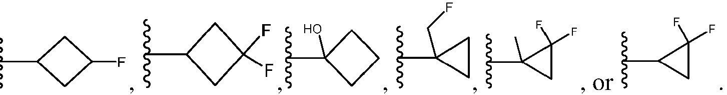 In yet other embodiments, R² is unsubstituted cyclopropyl, 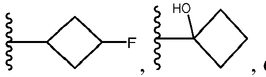 or

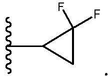

In some embodiments, R² is aryl, optionally substituted with one or more of halo or C₁₋₆alkoxy. In other embodiments, R² is unsubstituted phenyl, 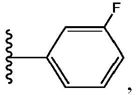 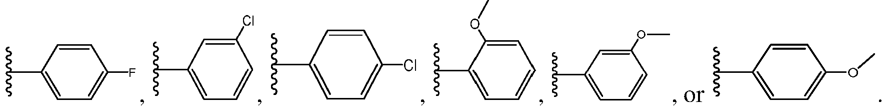 In yet other embodiments, R² is aryl, substituted with one or more of halo or C₁₋₆alkoxy. In further embodiments, R² is 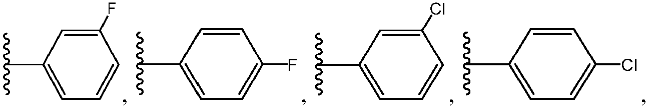 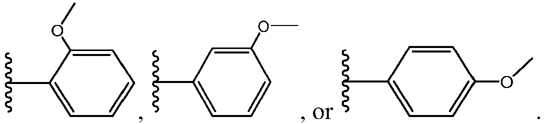 In other embodiments, R² is

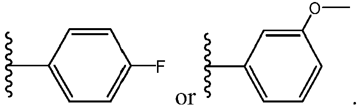

In some embodiments, R² is optionally substituted pyridinyl, optionally substituted pyrimidinyl, or optionally substituted pyrazinyl. In other embodiments, the pyridinyl, pyrimidinyl, or pyrazinyl group is substituted with one or more of halo, C₁₋₆haloalkyl, cyano, or NR^{y}R^{z}, wherein R^{y} and R^{z} are independently H or C₁₋₆alkyl.

In still further embodiments, R² is heteroaryl, substituted with one or more of C₁₋₆alkyl, C₁₋₆cyanoalkyl, C₁₋₆haloalkyl, or C₁₋₆hydroxyalkyl. In other embodiments, R² is heteroaryl substituted with one or more of C₁₋₆alkyl. In yet other embodiments, R² is heteroaryl substituted with one or more of C₁₋₆cyanoalkyl. In further embodiments, R² is heteroaryl substituted with one or more of C₁₋₆haloalkyl. In still further embodiments, R² is heteroaryl, substituted with one or more of C₁₋₆fluoroalkyl. In yet other embodiments, R² is heteroaryl substituted with one or more of C₁₋₆hydroxyalkyl. In other embodiments, R² is heteroaryl, substituted with one or more of methyl, ethyl, isopropyl, tert-butyl, C(CH₃)₂CN, CH(CH₃)OH, C(CH₃)₂OH, C(CH₃)₂CH₂OH, CH(CH₂CH₃)OH, CH₂C(CH₃)₂OH, CH₂F, CHF₂, CF₃, CH₂CF₃, CH₂CHF₂, CH(CH₃)F, C(CH₃)F₂, or C(CH₃)₂F. In yet other embodiments, R² is heteroaryl, substituted with methyl. In still other embodiments, R² is heteroaryl, substituted with ethyl. In yet other embodiments, R² is heteroaryl, substituted with isopropyl. In other embodiments, R² is heteroaryl, substituted with tert-butyl. In yet other embodiments, R² is heteroaryl, substituted with C(CH₃)₂CN. In still further embodiments, R² is heteroaryl, substituted with CH(CH₃)OH. In other embodiments, R² is heteroaryl, substituted with C(CH₃)₂OH. In yet other embodiments, R² is heteroaryl, substituted with C(CH₃)₂CH₂OH. In still other embodiments, R² is heteroaryl, substituted with CH(CH₂CH₃)OH. In further embodiments, R² is heteroaryl, substituted with CH₂C(CH₃)₂OH. In further embodiments, R² is heteroaryl, substituted with CHF₂. In yet other embodiments, R² is heteroaryl, substituted with CH₂F. In still other embodiments, R² is heteroaryl, substituted with CF₃. In further embodiments, R² is heteroaryl, substituted with CH₂CF₃. In yet other embodiments, R² is heteroaryl, substituted with CH₂CHF₂. In further embodiments, R² is heteroaryl, substituted with C(CH₃)F₂. In still other embodiments, R² is heteroaryl, substituted with CH(CH₃)F. In other embodiments, R² is heteroaryl, substituted with C(CH₃)₂F.

In other embodiments, R² is heteroaryl, substituted with C₃₋₈cycloalkyl, wherein the C₃₋₈cycloalkyl itself is optionally substituted with one or more of halo, OH, C₁₋₆haloalkyl, C₁₋₆alkyl or C₁₋₆alkoxy. In yet other embodiments, R² is heteroaryl, substituted with an unsubstituted C₃₋₈cycloalkyl, such as cyclopropyl, cyclobutyl, cyclopentyl, or cyclohexyl. In further embodiments, R² is heteroaryl, substituted with cyclopropyl or cyclobutyl. In yet other embodiments, R² is heteroaryl, substituted with C₃₋₈cycloalkyl, such as cyclopropyl, cyclobutyl, cyclopentyl, or cyclohexyl, wherein the C₃₋₈cycloalkyl itself is substituted with one or more of halo, OH, C₁₋₆haloalkyl, C₁₋₆alkyl or C₁₋₆alkoxy. In some embodiments, the substituted C₃₋₈cycloalkyl is substituted with OH. In still further embodiments, the substituted C₃₋₈cycloalkyl is substituted with one or more of halo, such as F, Cl, or Br. In other embodiments, the substituted C₃₋₈cycloalkyl is substituted with C₁₋₆alkyl, such as methyl, ethyl, or propyl. In further embodiments, the substituted C₃₋₈cycloalkyl is substituted with C₁₋₆haloalkyl, such as CF₃, CH₂CF₃, or CHF₂. In still other embodiments, the substituted C₃₋₈cycloalkyl is substituted with C₁₋₆alkoxy, such as methoxy, ethoxy, or propoxy. In further embodiments, the substituted C₃₋₈cycloalkyl is cyclopropyl or cyclobutyl, each of which is substituted with one or more of F, OH, or methyl. In other embodiments, R² is heteroaryl, substituted with

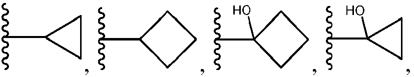

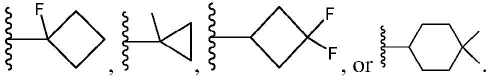

In other embodiments, R² is heteroaryl, substituted with aryl, wherein the aryl itself is optionally substituted with one or more of halo, C₁₋₆haloalkyl, C₁₋₆alkyl, or C₃₋₈cycloalkyl. In yet other embodiments, R² is heteroaryl, substituted with an unsubstituted phenyl. In further embodiments, R² is heteroaryl, substituted with aryl, such as phenyl, wherein the aryl itself is substituted with one or more of halo, C₁₋₆haloalkyl, C₁₋₆alkyl, or C₃₋₈cycloalkyl. In some embodiments, the substituted aryl is substituted with one or more of halo, such as F, Cl, or Br. In yet other embodiments, the substituted aryl is substituted with C₁₋₆haloalkyl, such as CF₃, CH₂CF₃, or CHF₂. In still further embodiments, the substituted aryl is substituted with C₁₋₆alkyl, such as methyl, ethyl, or propyl. In other embodiments, the substituted aryl is substituted with C₃₋₈cycloalkyl, such as cyclopropyl, cyclobutyl, or cyclopentyl. In yet other embodiments, the substituted aryl is phenyl that is substituted with one or more of F, methyl, or CF₃. In further embodiments, R² is heteroaryl, substituted with 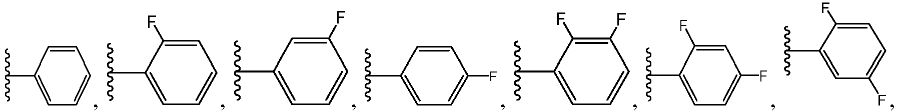 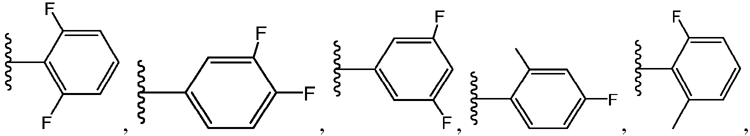 or 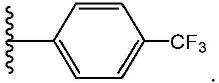 In still further embodiments, R² is heteroaryl, , substituted with 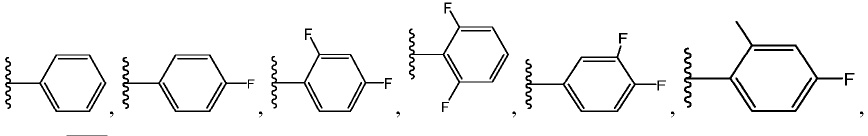 or

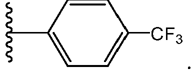

In still further embodiments, R² is heteroaryl, substituted with an optionally substituted heteroaryl. In some embodiments, the optionally substituted heteroaryl is optionally substituted pyridinyl, optionally substituted pyrazinyl, optionally substituted pyrazolyl, optionally substituted imidazolyl, or optionally substituted pyrimidinyl. In yet other embodiments, the optional substitution on the heteroaryl is one or more of halo, C₁₋₆haloalkyl, C₁₋₆alkyl, C₁₋₆alkoxy, C₁₋₆haloalkoxy, C₃₋₈cycloalkyl or C₃₋₈cycloalkylsulfonyl. In other embodiments, the optionally substituted heteroaryl is substituted with one or more of halo, such as F, Cl, or Br. In still other embodiments, the optionally substituted heteroaryl is substituted with one or more of C₁₋₆haloalkyl, such as CF₃, CH₂CF₃, CH₂CHF₂, or CHF₂. In other embodiments, the optionally substituted heteroaryl is substituted with one or more of C₁₋₆alkyl, such as methyl, ethyl, propyl, or isopropyl. In still other embodiments, the optionally substituted heteroaryl is substituted with one or more of C₁₋₆alkoxy, such as methoxy, ethoxy, or propoxy. In other embodiments, the optionally substituted heteroaryl is substituted with one or more of C₁₋₆haloalkoxy, such as OCF₃. In yet other embodiments, the optionally substituted heteroaryl is substituted with one or more of C₃₋₈cycloalkyl, such as cyclopropyl, cyclobutyl, or cyclopentyl. In other embodiments, the optionally substituted heteroaryl is substituted with one or more of C₃₋₈cycloalkylsulfonyl, such as cyclopropylsulfonyl, cyclobutylsulfonyl, or cyclopentylsulfonyl. In still other embodiments, the optionally substituted heteroaryl is substituted with one or more of F, CF₃, CH₂CF₃, CH₂CHF₂, CHF₂, methyl, isopropyl, methoxy, OCF₃, cyclopropyl, cyclobutyl, or cyclopropylsulfonyl.

In yet other embodiments, R² is heteroaryl, substituted with pyrazol-1-yl, pyrazol-3-yl, pyrazol-4-yl, pyrazol-5-yl, pyridin-2-yl, pyridin-3-yl, pyridin-4-yl, pyrimidin-2-yl, pyrimidin-4-yl, pyrimidin-5-yl, pyrazin-2-yl, pyrazin-3-yl, pyrazin-4-yl, pyrazin-5-yl, imidazol-4-yl, or imidazol-5-yl, each of which can be optionally substituted. In further embodiments, R² is substituted with pyrazol-1-yl, pyrazol-3-yl, pyrazol-4-yl, pyrazol-5-yl, pyridin-2-yl, pyridin-3-yl, pyridin-4-yl, pyrimidin-2-yl, pyrimidin-4-yl, pyrimidin-5-yl, pyrazin-2-yl, pyrazin-3-yl, pyrazin-4-yl, pyrazin-5-yl, imidazol-4-yl, imidazol-5-yl, pyridazin-3-yl, pyridazin-4-yl, each of which is optionally substituted. In still further embodiments, R² is substituted with optionally substituted pyrazol-1-yl. In other embodiments, R² is substituted with optionally substituted pyrazol-3-yl. In further embodiments, R² is substituted with optionally substituted pyrazol-4-yl. In yet further embodiments, R² is substituted with optionally substituted pyridin-2-yl. In other embodiments, R² is substituted with optionally substituted pyridin-3-yl. In further embodiments, R² is substituted with optionally substituted pyridin-4-yl. In further embodiments, R² is substituted with optionally substituted imidazol-4-yl. In yet other embodiments, R² is substituted with optionally substituted imidazol-5-yl. In further embodiments, R² is substituted with optionally substituted pyrimidin-2-yl. In other embodiments, R² is substituted with optionally substituted pyrimidin-4-yl. In still further embodiments, R² is substituted with optionally substituted pyrimidin-5-yl. In yet other embodiments, R² is substituted with optionally substituted pyrazin-2-yl. In other embodiments, R² is substituted with optionally substituted pyridazin-3-yl. In further embodiments, R² is substituted with optionally substituted pyridazin-4-yl,

In some embodiments, R² is heteroaryl, substituted with 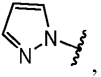 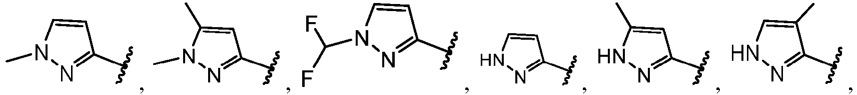 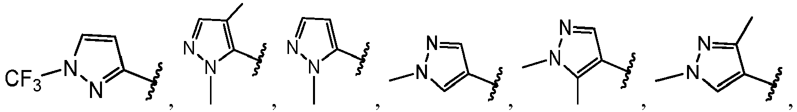 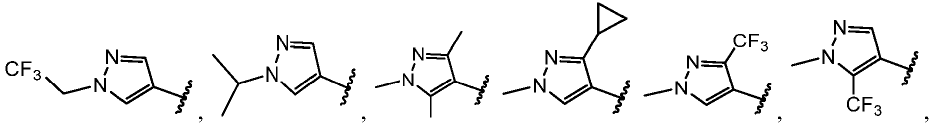 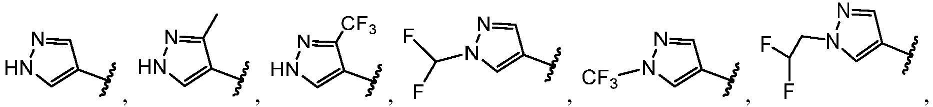 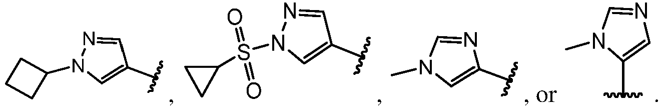 In still further embodiments, R² is heteroaryl, substituted with 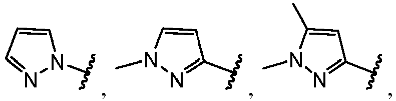 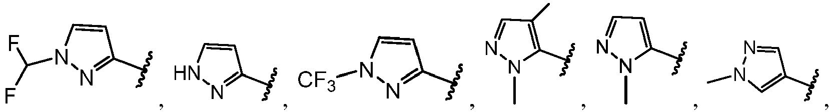 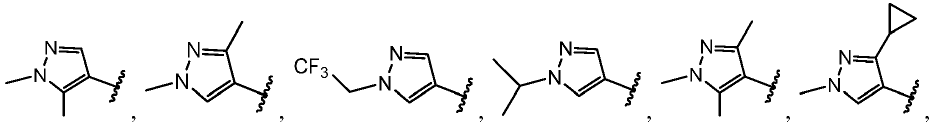 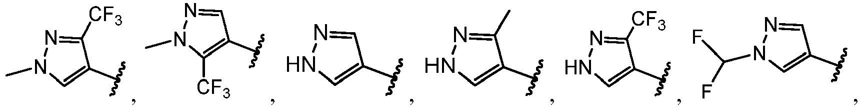 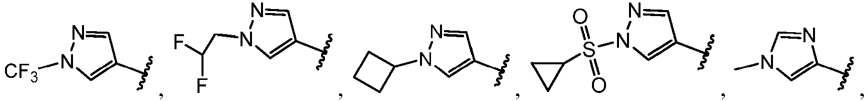 or 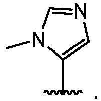 In still further embodiments, R² is heteroaryl, substituted with

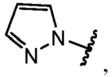

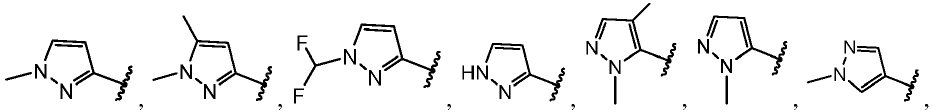

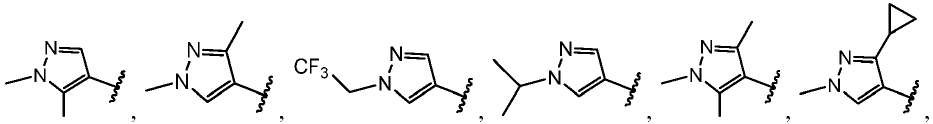

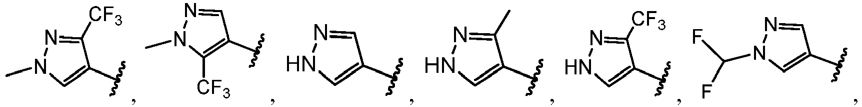

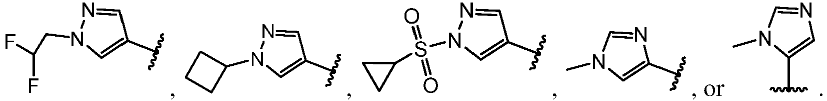

In some embodiments, R² is heteroaryl, substituted with 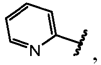 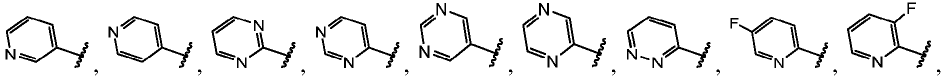 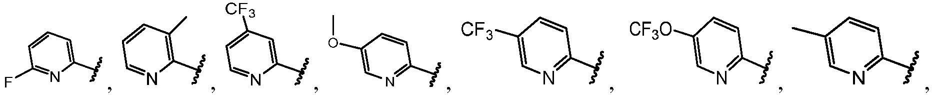 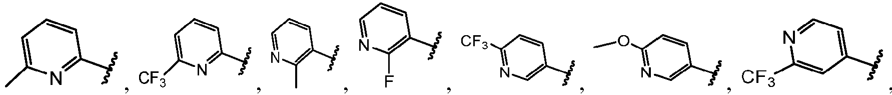 or 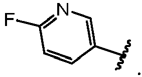 In other embodiments, R² is heteroaryl, substituted with 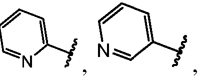 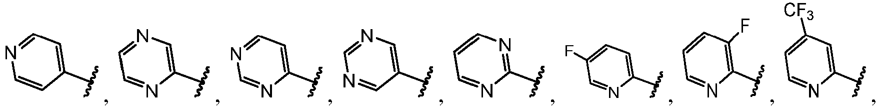 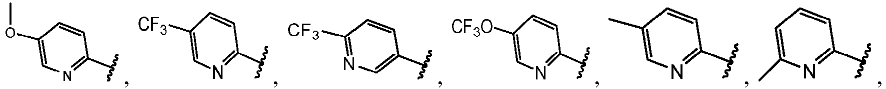 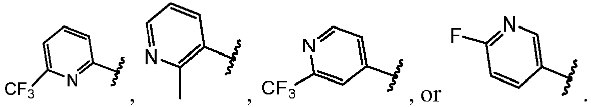 In still other embodiments, R² is heteroaryl, substituted with

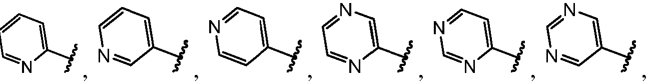

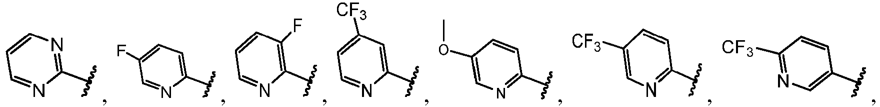

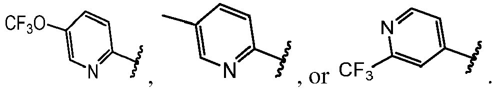

In other embodiments, R² is heteroaryl, substituted with heterocyclyl or heterocyclyl(alkylene), wherein the heterocyclyl and heterocyclyl(alkylene) groups themselves are each optionally substituted with one or more of halo, OH, C₁₋₆haloalkyl, C₁₋₆alkyl, C₁₋₆hydroxyalkyl, C₁₋₆alkoxy, C(O)O(C₁₋₆alkyl), or C₃₋₈cycloalkyl. In yet other embodiments, R² is heteroaryl, substituted with an unsubstituted heterocyclyl group such as azetidinyl, pyrrolidinyl, piperidinyl, piperazinyl, morpholinyl, or 6-azaspiro[2.5]octan-6-yl or unsubstituted heterocyclyl(alkylene) group, such as azetidinyl(alkylene), pyrrolidinyl(alkylene), piperidinyl(alkylene), piperazinyl(alkylene), or morpholinyl(alkylene). In further embodiments, R² is heteroaryl, substituted with heterocyclyl, such as azetidinyl, pyrrolidinyl, piperidinyl, piperazinyl, morpholinyl, or 6-azaspiro[2.5]octan-6-yl or heterocyclyl(alkylene), such as azetidinyl(alkylene), pyrrolidinyl(alkylene), piperidinyl(alkylene), piperazinyl(alkylene), or morpholinyl(alkylene), wherein the heterocyclyl and heterocyclyl(alkylene) groups themselves are each substituted with one or more of halo, OH, C₁₋₆haloalkyl, C₁₋₆alkyl, C₁₋₆hydroxyalkyl, C₁₋₆alkoxy, C(O)O(C₁₋₆alkyl), or C₃₋₈cycloalkyl. In further embodiments, the substituted heterocyclyl or substituted heterocyclyl(alkylene) is substituted with one or more of halo, such as F, Cl, or Br. In yet further embodiments, the substituted heterocyclyl or substituted heterocyclyl(alkylene) is substituted with one or more of OH. In yet other embodiments, the substituted heterocyclyl or substituted heterocyclyl(alkylene) is substituted with one or more of C₁₋₆haloalkyl, such as CF₃, CH₂CF₃, or CHF₂. In still further embodiments, the substituted heterocyclyl or substituted heterocyclyl(alkylene) is substituted with one or more of C₁₋₆alkyl, such as methyl, ethyl, or propyl. In yet further embodiments, the substituted heterocyclyl or substituted heterocyclyl(alkylene) is substituted with one or more of C₁₋₆hydroxyalkyl such as C(CH₃)₂OH, or CH(CH₃)OH. In other embodiments, the substituted heterocyclyl or substituted heterocyclyl(alkylene) is substituted with one or more of C₁₋₆alkoxy, such as methoxy, ethoxy, or propoxy. In yet other embodiments, the substituted heterocyclyl or substituted heterocyclyl(alkylene) is substituted with one or more of C(O)O(C₁₋₆alkyl), such as C(O)Omethyl, C(O)Oethyl, C(O)Opropyl, or C(O)Otert-butyl. In further embodiments, the substituted heterocyclyl or substituted heterocyclyl(alkylene) is substituted with one or more of C₃₋₈cycloalkyl, such as cyclopropyl, cyclobutyl, or cyclopentyl. In yet other embodiments, the substituted heterocyclyl or substituted heterocyclyl(alkylene) is substituted with one or more of F, OH, methyl, C(CH₃)₂OH, C(O)Otert-butyl, or cyclopropyl. In yet other embodiments where R² is heteroaryl, the R² is substituted with 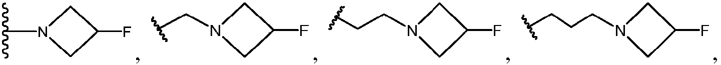 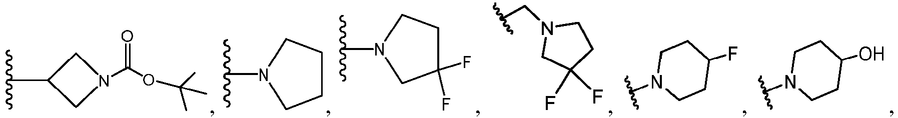 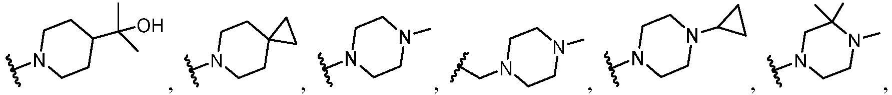 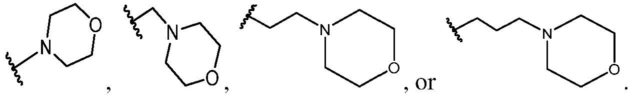 In still further embodiments where R² is heteroaryl, the R² is substituted with

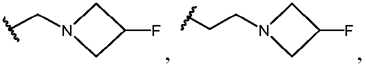

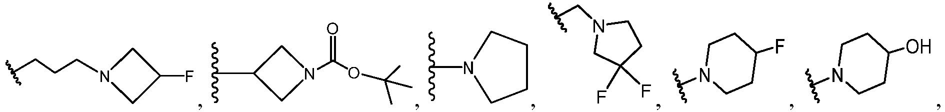

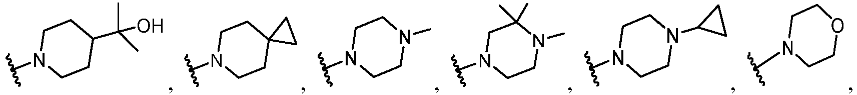

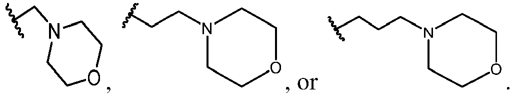

In further embodiments, R² is 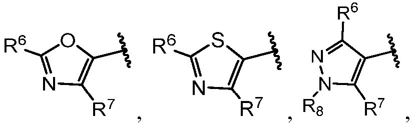 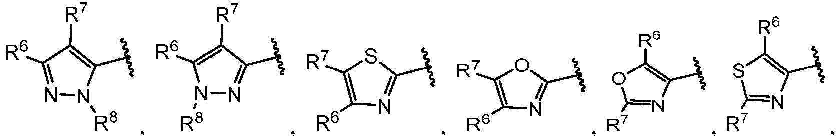 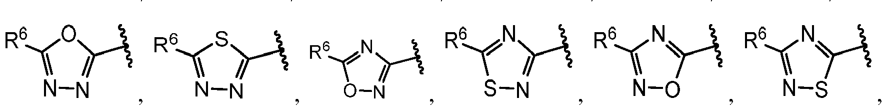 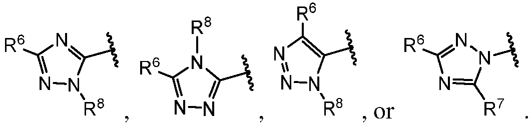 In yet further embodiments, R² is 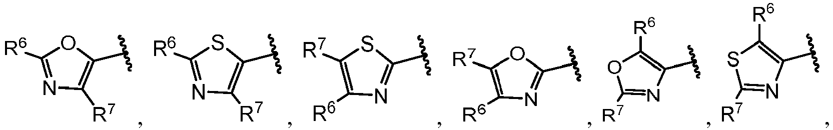 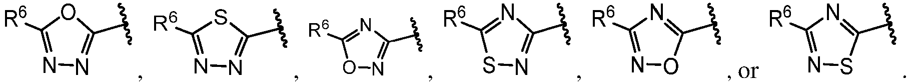 In still further embodiments, R² is, 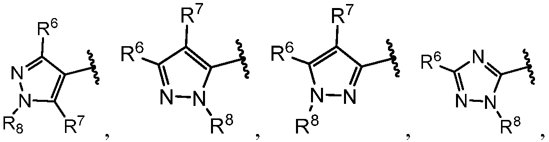 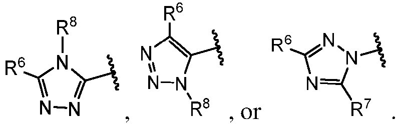 In other embodiments, R² is 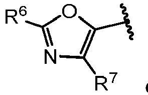 or 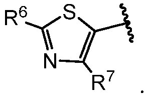 In further embodiments, R² is 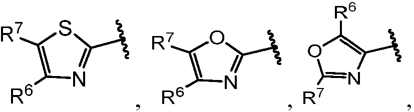 or 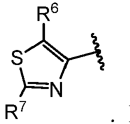 In yet further embodiments, R² is 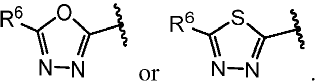 In still further embodiments, R² is 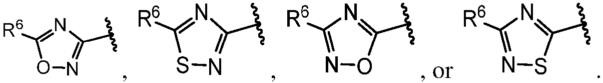 In yet other embodiments, R² is 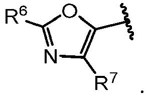 In other embodiments, R² is 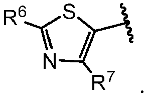 In further embodiments, R² is 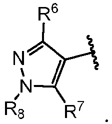 In yet other embodiments, R² is 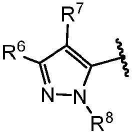 . In still further embodiments, R² is 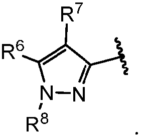 In other embodiments, R² is 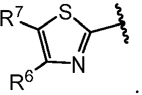 In further embodiments, R² is 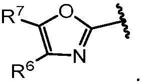 In still other embodiments, R² is 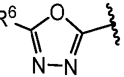 . In yet further embodiments, R² is 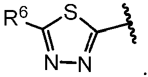 In other embodiments, R² is 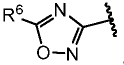 . In further embodiments, R² is 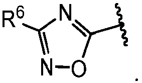 In yet other embodiments, R² is 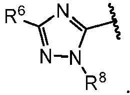 In still further embodiments, R² is 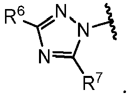 In other embodiments, R² is 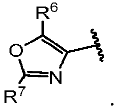 In other embodiments, R² is 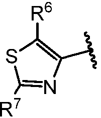 . In further embodiments, R² is 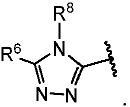 In yet other embodiments, R² is

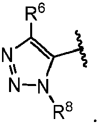

In these structures for R², R⁶ and R⁷ are each independently, H, CN, C₁₋₆alkyl, C₁₋₆haloalkyl, C₁₋₆cyanoalkyl, C₁₋₆hydroxyalkyl, C₁₋₆alkoxy, C₁₋₆alkoxy(alkylene), C₁₋₆haloalkoxy, C₁₋₆haloalkoxy(alkylene), C₁₋₆deuteratedalkoxy(alkylene), halo, (CR^{v}R^{x})ₚNR^{y}R^{z}, C(O)NR^{y2}R^{z2}, C₁₋₆alkylcarbonyl, optionally substituted C₃₋₈cycloalkyl, optionally substituted heterocyclyl, optionally substituted aryl, optionally substituted heteroaryl, optionally substituted C₂₋₆alkenyl, optionally substituted C₃₋₈cycloalkenyl, optionally substituted (C₃₋₈cycloalkyl)alkylene, optionally substituted (aryl)alkylene, optionally substituted (heterocyclyl)alkylene, or C₁₋₆alkylsulfonyl; R⁸ is H, C₁₋₆alkyl, C₁₋₆haloalkyl, C₁₋₆alkoxy(alkylene), C₁₋₆alkylcarbonyl, C₁₋₆hydroxyalkyl, (CR^{v}R^{x})ₚNR^{y}R^{z}, optionally substituted C₃₋₈cycloalkyl, optionally substituted aryl, optionally substituted heteroaryl, or optionally substituted (C₃₋₈cycloalkyl)alkylene; R^{v} and R^{x} are, independently, H or C₁₋₆alkyl; R^{y} and R^{z} are, independently, H, C₁₋₆alkyl, C₁₋₆alkoxy(alkylene), or C₃₋₆cycloalkyl; R^{y2} and R^{z2} are, independently, H, C₁₋₆alkyl, or C₃₋₆cycloalkyl; and p is 0, 1, 2, or 3. In some embodiments, R⁶, R⁷, and R⁸ are H.

In other embodiments, R⁶ is C₁₋₆alkyl, C₁₋₆haloalkyl, C₁₋₆cyanoalkyl, or C₁₋₆hydroxyalkyl. In further embodiments, R⁶ is C₁₋₆alkyl such as methyl, ethyl, isopropyl or tert-butyl. In still further embodiments, R⁶ is C₁₋₆haloalkyl such as CF₃, CHF₂, CH₂F, CH₂CF₃, CH(CH₃)F, C(CH₃)F₂, or C(CH₃)₂F. In further embodiments, R⁶ is C₁₋₆cyanoalkyl such as C(CH₃)₂CN. In other embodiments, R⁶ is C₁₋₆hydroxyalkyl such as C(CH₃)₂OH, CH(CH₃)OH, CH(CH₂CH₃)OH, CH₂C(CH₃)₂OH, or C(CH₃)₂CH₂OH. In further embodiments, R⁶ is methyl, ethyl, isopropyl, tert-butyl, CF₃, CHF₂, CH₂F, CH(CH₃)F, C(CH₃)F₂, C(CH₃)₂F, C(CH₃)₂CN, C(CH₃)₂OH, CH(CH₃)OH, CH(CH₂CH₃)OH, CH₂C(CH₃)₂OH, or C(CH₃)₂CH₂OH.

In other embodiments, R⁶ is C₃₋₆cycloalkyl optionally substituted with one or more of halo, OH, C₁₋₆haloalkyl, C₁₋₆alkyl, or C₁₋₆alkoxy. In further embodiments, R⁶ is cyclopropyl, cyclobutyl, cyclopentyl or cyclohexyl, optionally substituted with one or more of halo, OH, C₁₋₆haloalkyl, C₁₋₆alkyl, or C₁₋₆alkoxy. In still further embodiments, R⁶ is unsubstituted cyclopropyl, cyclobutyl, cyclopentyl or cyclohexyl. In other embodiments, R⁶ is C₃₋₆cycloalkyl substituted with one or more halo such as F, Cl, or Br. In yet other embodiments, R⁶ is C₃₋₆cycloalkyl substituted with one or more OH. In still further embodiments, R⁶ is C₃₋₆cycloalkyl substituted with one or more C₁₋₆haloalkyl such as CF₃, CH₂CF₃, or CHF₂. In further embodiments, R⁶ is C₃₋₆cycloalkyl substituted with one or more C₁₋₆alkyl such as methyl, ethyl, or propyl. In still further embodiments, R⁶ is C₃₋₆cycloalkyl substituted with C₁₋₆alkoxy, such as one or more methoxy, ethoxy, or propoxy. In other embodiments, R⁶ is cyclopropyl, cyclobutyl, or cyclohexyl, each of which is optionally substituted with one or more of F, OH, or methyl. In other embodiments, R⁶ is

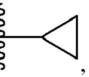

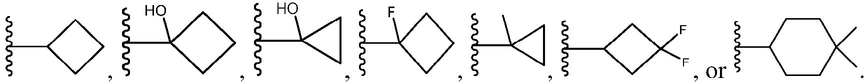

In further embodiments, R⁶ is aryl optionally substituted with one or more of halo, C₁₋₆haloalkyl, C₁₋₆alkyl or C₃₋₆cycloalkyl. In other embodiments, R⁶ is phenyl optionally substituted with one or more of halo, C₁₋₆haloalkyl, C₁₋₆alkyl or C₃₋₆cycloalkyl. In still other embodiments, R⁶ is aryl optionally substituted with one or more of halo such as F, Cl, or Br. In yet other embodiments, R⁶ is aryl optionally substituted with one or more of C₁₋₆haloalkyl such as CF₃, CH₂CF₃, or CHF₂. In other embodiments, R⁶ is aryl optionally substituted with one or more of C₁₋₆alkyl such as methyl, ethyl, or propyl. In yet other embodiments, R⁶ is aryl optionally substituted with one or more of C₃₋₆cycloalkyl such as cyclopropyl, cyclobutyl, or cyclopentyl. In other embodiments, R⁶ is aryl (*e.g.,* phenyl) optionally substituted with F, methyl, or CF₃. In other embodiments, R⁶ is 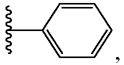 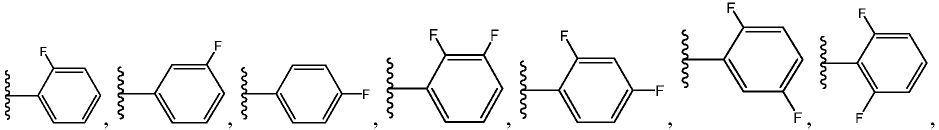 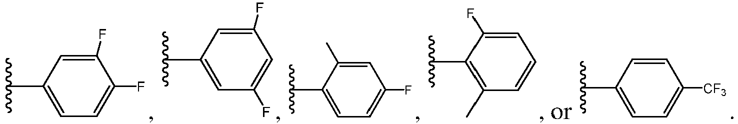 In yet other embodiments, R⁶ is

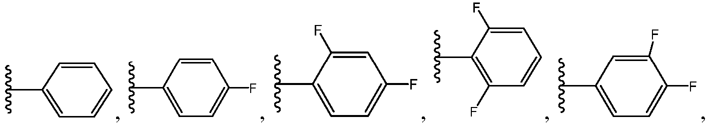

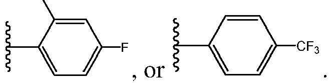

In yet other embodiments, R⁶ is heteroaryl, optionally substituted with one or more of halo, C₁₋₆haloalkyl, C₁₋₆alkyl, C₁₋₆alkoxy, C₁₋₆haloalkoxy, C₃₋₆cycloalkyl, or C₃₋₆cycloalkylsulfonyl. In still other embodiments, R⁶ is pyridinyl, pyrazolyl, pyrazinyl, imidazolyl, or pyrimidinyl, each of which is optionally substituted. In still other embodiments, R⁶ is pyridinyl, pyrazolyl, pyrazinyl, pyridazinyl, imidazolyl, or pyrimidinyl, each of which is optionally substituted. In further embodiments, R⁶ is optionally substituted pyrazinyl such as pyrazin-2-yl, pyrazin-3-yl, pyrazin-4-yl, or pyrazin-5-yl. In other embodiments, R⁶ is optionally substituted pyrazolyl such as pyrazol-1-yl, pyrazol-3-yl, pyrazol-4-yl, or pyrazol-5-yl. In still further embodiments, R⁶ is optionally substituted pyridinyl such as pyridin-2-yl, pyridin-3-yl, or pyridin-4-yl. In other embodiments, R⁶ is optionally substituted pyridazinyl such as pyridazin-3-yl or pyridazin-4-yl. In yet other embodiments, R⁶ is optionally substituted imidazolyl such as imidazol-4-yl or imidazol-5-yl. In further embodiments, R⁶ is optionally substituted pyrimidinyl such as pyridimidin-2-yl, pyrimidin-4-yl, or pyrimidin-5-yl. In other embodiments, R⁶ is pyrazin-2-yl, pyrazin-3-yl, pyrazin-4-yl, pyrazin-5-yl, pyrazol-1-yl, pyrazol-3-yl, pyrazol-4-yl, pyrazol-5-yl, pyridin-2-yl, pyridin-3-yl, pyridin-4-yl, imidazol-4-yl, imidazol-5-yl, pyrimidin-2-yl, pyrimidin-4-yl, or pyrimidin-5-yl, each of which is optionally substituted. In yet other embodiments, R⁶ is pyrazin-2-yl, pyrazin-3-yl, pyrazin-4-yl, pyrazin-5-yl, pyrazol-1-yl, pyrazol-3-yl, pyrazol-4-yl, pyrazol-5-yl, pyridin-2-yl, pyridin-3-yl, pyridin-4-yl, imidazol-4-yl, imidazol-5-yl, pyrimidin-2-yl, pyrimidin-4-yl, pyrimidin-5-yl, pyridazin-3-yl or pyridazin-4-yl, each of which is optionally substituted. In further embodiments, R⁶ is unsubstituted pyridin-2-yl, unsubstituted pyridin-3-yl, unsubstituted pyridin-4-yl, unsubstituted pyrimidin-2-yl, unsubstituted pyrimidin-4-yl, unsubstituted pyrimidin-5-yl, or unsubstituted pyrazin-2-yl. In further embodiments, R⁶ is unsubstituted pyridin-2-yl, unsubstituted pyridin-3-yl, unsubstituted pyridin-4-yl, unsubstituted pyrimidin-2-yl, unsubstituted pyrimidin-4-yl, unsubstituted pyrimidin-5-yl, unsubstituted pyrazin-2-yl, or unsubstituted pyridazin-3-yl. In other embodiments, R⁶ is optionally substituted with one or more halo such as F, Cl, or Br. In yet other embodiments, R⁶ is optionally substituted with one or more C₁₋₆haloalkyl such as CF₃, CH₂CF₃, or CHF₂. In other embodiments, R⁶ is optionally substituted with one or more C₁₋₆alkyl such as methyl, ethyl, propyl, or isopropyl. In still other embodiments, R⁶ is optionally substituted with one or more C₁₋₆alkoxy such as methoxy, ethoxy, or propoxy. In yet further embodiments, R⁶ is optionally substituted with one or more C₁₋₆haloalkoxy such as OCF₃. In further embodiments, R⁶ is optionally substituted with one or more C₃₋₆cycloalkyl such as cyclopropyl, cyclobutyl, or cyclopentyl. In yet further embodiments, R⁶ is optionally substituted with one or more C₃₋₆cycloalkylsulfonyl such as cyclopropylsulfonyl, cyclobutylsulfonyl, or cyclopentylsulfonyl. In yet other embodiments, R⁶ is heteroaryl optionally substituted with one or more of F, CF₃, CH₂CF₃, CH₂CHF₂, CHF₂, methyl, isopropyl, methoxy, OCF₃, cyclopropyl, cyclobutyl, or cyclopropylsulfonyl.

In other embodiments, R⁶ is a five-membered heteroaryl, optionally substituted with one or more of CF₃, CH₂CF₃, CH₂CHF₂, CHF₂, methyl, isopropyl, cyclopropyl, cyclobutyl, or cyclopropylsulfonyl. In further embodiments, R⁶ is 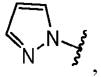 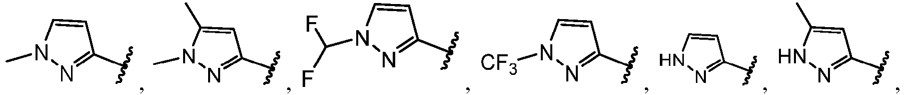 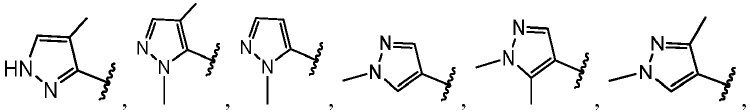 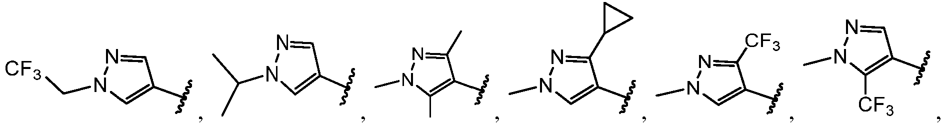 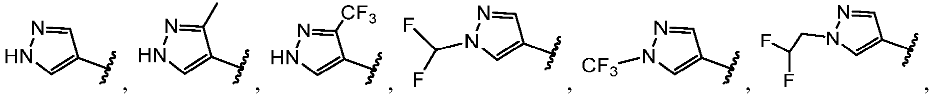 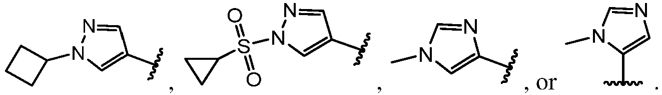 In still further embodiments, R⁶ is 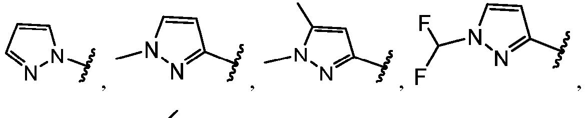 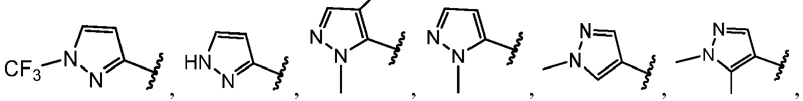 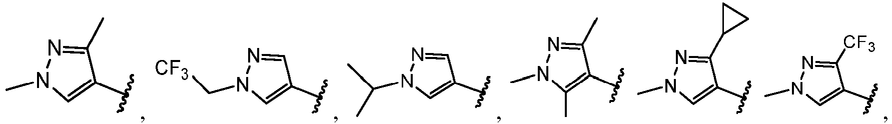 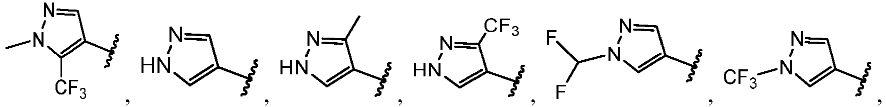 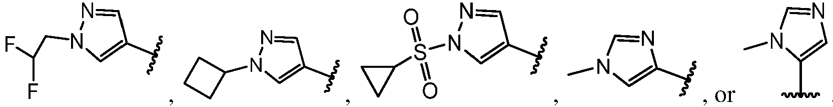 . In other embodiments, R⁶ is 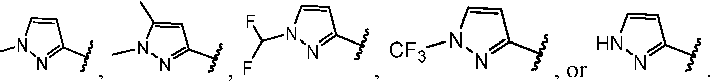 In still further embodiments, R⁶ is 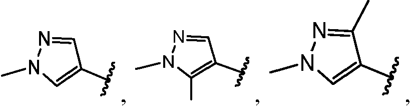 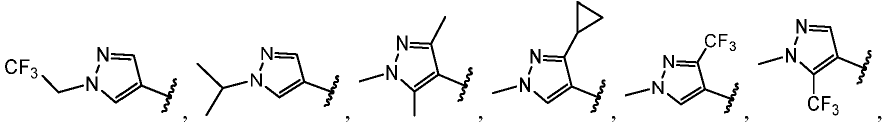 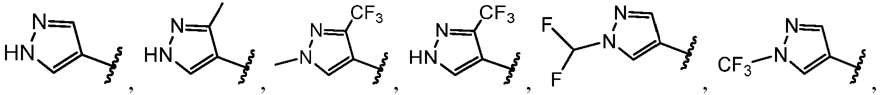 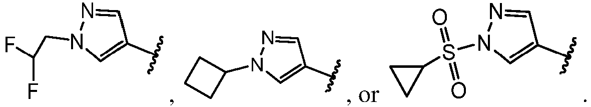 In yet further embodiments, R⁶ is 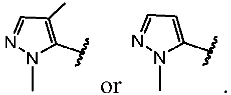 In further embodiments, R⁶ is 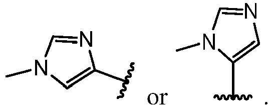 In yet further embodiments, R⁶ is

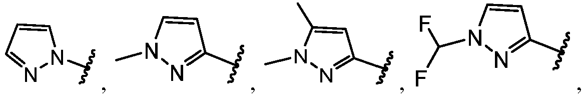

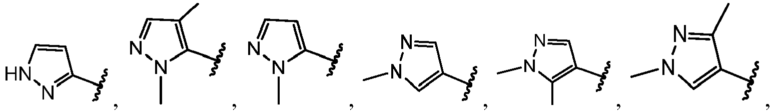

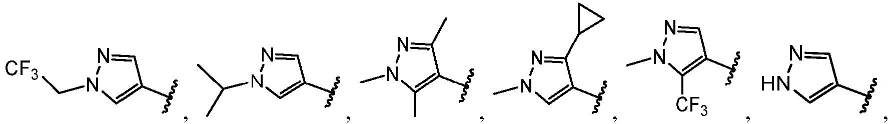

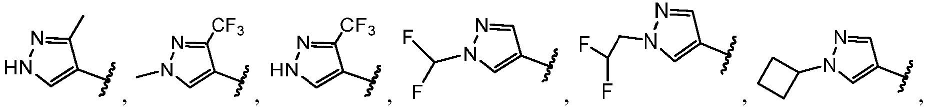

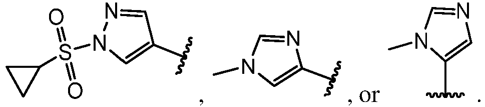

In other embodiments, R⁶ is a six-membered heteroaryl, optionally substituted with one or more of F, CF₃, methoxy, OCF₃, or methyl. In still other embodiments, R⁶ is 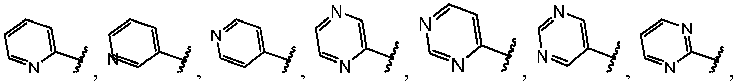 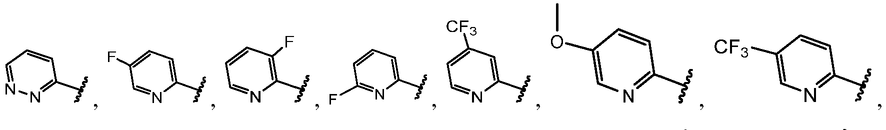 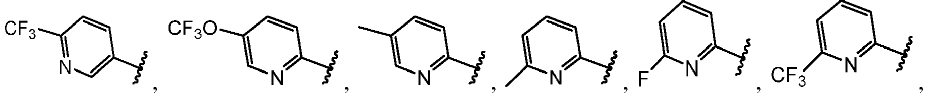 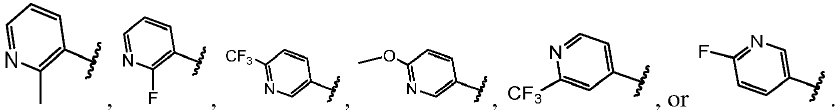 In still further embodiments, R⁶ is 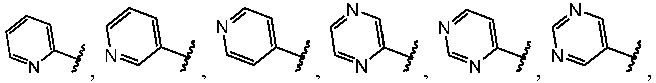 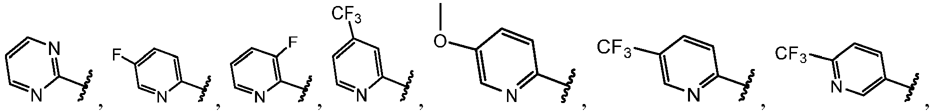 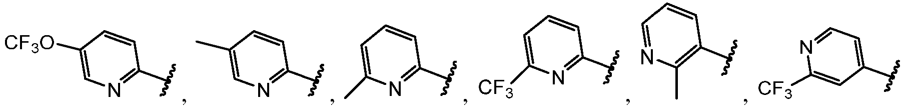 , or 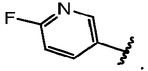 In yet other embodiments, R⁶ is

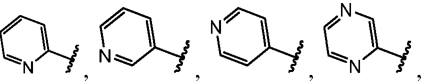

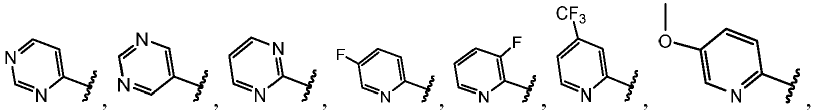

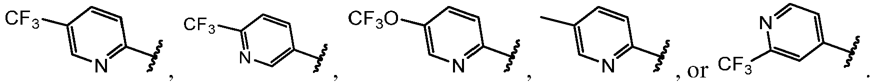

In yet other embodiments, R⁶ is heterocyclyl or heterocyclyl(alkylene), each optionally substituted with one or more of halo, OH, C₁₋₆haloalkyl, C₁₋₆alkyl, C₁₋₆hydroxyalkyl, C₁₋₆alkoxy, C(O)O(C₁₋₆alkyl), or C₃₋₆cycloalkyl. In further embodiments, R⁶ is optionally substituted azetidinyl, optionally substituted pyrrolidinyl, optionally substituted piperidinyl, optionally substituted piperazinyl, or optionally substituted morpholinyl. In other embodiments, R⁶ is optionally substituted azetidinyl. In yet other embodiments, R⁶ is optionally substituted pyrrolidinyl. In further embodiments, R⁶ is optionally substituted piperidinyl. In yet other embodiments, R⁶ is optionally substituted piperazinyl. In still further embodiments, R⁶ is optionally substituted morpholinyl. In other embodiments, R⁶ is optionally substituted morpholinyl(alkylene), optionally substituted piperidinyl(alkylene), optionally substituted piperazinyl(alkylene), or optionally substituted azetidinyl(alkylene). In further embodiments, R⁶ is unsubstituted azetidinyl, pyrrolidinyl, piperidinyl, piperazinyl, or morpholinyl. In other embodiments, R⁶ is unsubstituted morpholinyl(alkylene), piperidinyl(alkylene), piperazinyl(alkylene), or azetidinyl(alkylene). In yet other embodiments, R⁶ is unsubstituted morpholinyl or morpholinyl(alkylene). In other embodiments, the heterocyclyl and heterocyclyl(alkylene) groups are optionally substituted with one or more halo such as F, Cl, or Br. In yet other embodiments, the heterocyclyl and heterocyclyl(alkylene) groups are optionally substituted with one or more OH. In further embodiments, the heterocyclyl and heterocyclyl(alkylene) groups are optionally substituted with one or more C₁₋₆haloalkyl such as CF₃, CH₂CF₃, or CHF₂. In still further embodiments, the heterocyclyl and heterocyclyl(alkylene) groups are optionally substituted with one or more C₁₋₆alkyl such as methyl, ethyl, or propyl. In further embodiments, the heterocyclyl and heterocyclyl(alkylene) groups are optionally substituted with one or more C₁₋₆hydroxyalkyl such as C(CH₃)₂OH. In yet further embodiments, the heterocyclyl and heterocyclyl(alkylene) groups are optionally substituted with one or more C₁₋₆alkoxy, such as methoxy, ethoxy, or propoxy. In other embodiments, the heterocyclyl and heterocyclyl(alkylene) groups are optionally substituted with one or more C(O)O(C₁₋₆alkyl), such as C(O)Omethyl, C(O)Oethyl, C(O)Opropyl, or C(O)Otert-butyl. In still further embodiments, the heterocyclyl and heterocyclyl(alkylene) groups are optionally substituted with one or more C₃₋₆cycloalkyl such as one or more cyclopropyl, cyclobutyl, or cyclopentyl. In yet other embodiments, R⁶ is heterocyclyl and heterocyclyl(alkylene), each optionally substituted with one or more of F, OH, C(CH₃)₂(OH), methyl, C(O)O(tert-butyl), or cyclopropyl.

In further embodiments, R⁶ is 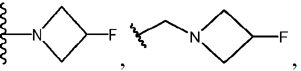 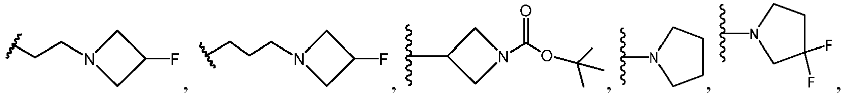 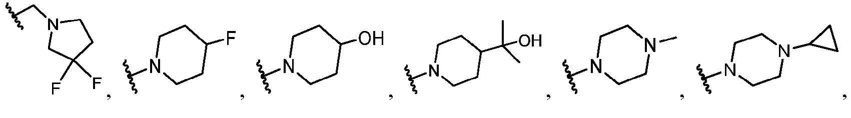 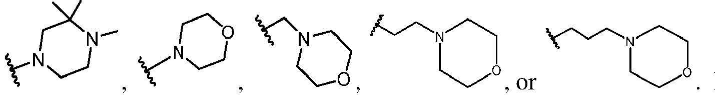 In yet other embodiments, R⁶ is 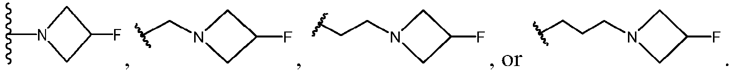 In further embodiments, R⁶ is 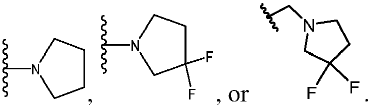 In still further embodiments, R⁶ is 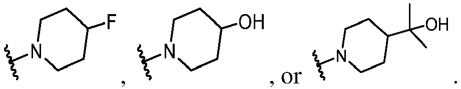 In yet other embodiments, R⁶ is 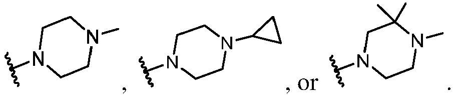 In other embodiments, R⁶ is 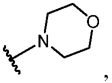 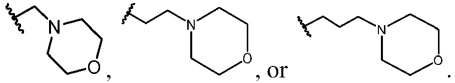 In yet further embodiments, R⁶ is

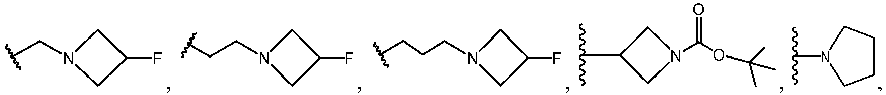

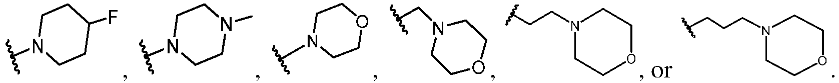

In further embodiments, R⁶ is H, CN, C₁₋₆alkoxy, C₁₋₆alkoxy(alkylene), C₁₋₆haloalkoxy, C₁₋₆haloalkoxy(alkylene), C₁₋₆deuteratedalkoxy(alkylene), halo, (CR^{v}R^{x})ₚNR^{y}R^{z}, C(O)NR^{y2}R^{z2}, C₁₋₆alkylcarbonyl, optionally substituted C₂₋₆alkenyl, optionally substituted C₃₋₆cycloalkenyl, optionally substituted (C₃₋₆cycloalkyl)alkylene, optionally substituted (aryl)alkylene, or C₁₋₆alkylsulfonyl. In other embodiments, R⁶ is H. In further embodiments, R⁶ is CN. In still other embodiments, R⁶ is C₁₋₆alkoxy such as methoxy. In yet other embodiments, R⁶ is C₁₋₆alkoxy(alkylene) such as CH₂OCH₃, C(CH₃)₂OCH₃, or (CH₂)₂OCH₃. In still other embodiments, R⁶ is C₁₋₆haloalkoxy such as OCF₃, OCHF₂, OCH₂F, or O(CH₂)₂CF₃. In other embodiments, R⁶ is C₁₋₆haloalkoxy(alkylene) such as CH₂OCF₃. In yet other embodiments, R⁶ is C₁₋₆deuteratedalkoxy(alkylene) such as CH₂OCD₃. In further embodiments, R⁶ is halo such as F, Br, or Cl. In yet further embodiments, R⁶ is (CR^{v}R^{x})ₚNR^{y}R^{z} such as NH₂, N(CH₃)₂, NHCH₂CF₃, NHCH₂CH₂OCH₃, NH(cyclopropyl), CH₂N(CH₃)₂, (CH₂)₂N(CH₃)₂, C(CH₃)₂NHCH₃, C(CH₃)₂N(CH₃)₂, CH₂NH(cyclopropyl), or CH₂CH₂NH(cyclopropyl). In other embodiments, R⁶ is C(O)NR^{y2}R^{z2} such as C(O)N(CH₃)₂ or C(O)NH(cyclopropyl). In yet other embodiments, R⁶ is C₁₋₆alkylcarbonyl such as C(O)CH₃. In further embodiments, R⁶ is optionally substituted C₂₋₆alkenyl such as CH=CH₂, CH=CH-cyclopropyl, or CH=CHC(CH₃)₂OH. In yet other embodiments, R⁶ is optionally substituted C₃₋₆cycloalkenyl such as 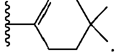 In still other embodiments, R⁶ is optionally substituted (C₃₋₆cycloalkyl)alkylene such as CH₂-cyclopropyl, CH₂CH₂-cyclopropyl or C(CH₃)OH-cyclopropyl. In further embodiments, R⁶ is optionally substituted (aryl)alkylene such as benzyl. In other embodiments, C₁₋₆alkylsulfonyl(alkylene) such as C(CH₃)₂SO₂CH₃. In yet other embodiments, R⁶ is H, CH₂OCH₃, C(CH₃)₂OCH₃, (CH₂)₂OCH₃, O(CH₂)₂CF₃, CH₂OCF₃, CH₂OCD₃, Br, Cl, NH₂, NH(cyclopropyl), CH₂N(CH₃)₂, (CH₂)₂N(CH₃)₂, C(CH₃)₂NHCH₃, C(CH₃)₂N(CH₃)₂, CH=CHC(CH₃)₂OH, CH=CH-cyclopropyl, 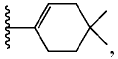 CH₂-cyclopropyl, CH₂CH₂-cyclopropyl, C(CH₃)OH-cyclopropyl, benzyl, or C(CH₃)₂SO₂CH₃.

In some embodiments, R⁷ is H, CN, C₁₋₆alkyl, C₁₋₆haloalkyl, halo, C₃₋₈cycloalkyl, aryl, or heteroaryl. In yet other embodiments, R⁷ is H. In still further embodiments, R⁷ is CN. In other embodiments, R⁷ is C₁₋₆alkyl, such as methyl, ethyl, or propyl. In further embodiments, R⁷ is methyl. In yet other embodiments, R⁷ is C₁₋₆haloalkyl, such as CHF₂, CH₂F, C(CH₃)F₂, CH₂CHF₂, or CF₃. In further embodiments, R⁷ is halo, such as F, Br, or Cl. In yet other embodiments, R⁷ is Br or Cl. In still further embodiments, R⁷ is C₃₋₆cycloalkyl, such as cyclopropyl, cyclobutyl, or cyclopentyl. In other embodiments, R⁷ is cyclopropyl. In further embodiments, R⁷ is aryl. In yet other embodiments, R⁷ is phenyl. In still further embodiments, R⁷ is heteroaryl. In other embodiments, R⁷ is pyridinyl. In further embodiments, R⁷ is H, CN, methyl, CF₃, CH₂F, CHF₂, CF₂(CH₃), CH₂CHF₂, Br, Cl, cyclopropyl, phenyl, or pyridinyl.

In some embodiments of R², R⁸ is H. In other embodiments, R⁸ is C₁₋₆alkyl such as methyl, ethyl, propyl, isopropyl, or tert-butyl. In further embodiments, R⁸ is C₁₋₆haloalkyl such as CF₃, CHF₂, CH₂F, CH₂CF₃, CH₂CHF₂, C(CH₃)₂F, or C(CH₃)F₂. In still further embodiments, R⁸ is C₁₋₆alkoxy(alkylene) such as CH₂CH₂OCH₃. In other embodiments, R⁸ is C₁₋₆alkylcarbonyl such as CH₂C(=O)CH₃. In still other embodiments, R⁸ is C₁₋₆hydroxyalkyl such as CH₂C(CH₃)₂OH. In further embodiments, R⁸ is (CR^{v}R^{x})ₚNR^{y}R^{z} such as (CH₂)₂N(CH₃)₂. In yet other embodiments, R⁸ is optionally substituted C₃₋₈cycloalkyl such as optionally substituted cyclopropyl, optionally substituted cyclobutyl, optionally substituted cyclopentyl, or optionally substituted cyclohexyl. In other embodiments, R⁸ is optionally substituted aryl such as optionally substituted phenyl. In further embodiments, R⁸ is optionally substituted heteroaryl such as optionally substituted pyridinyl. In yet other embodiments, R⁸ is optionally substituted (C₃₋₈cycloalkyl)alkylene such as 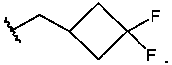 In still further embodiments, R⁸ is H, methyl, ethyl, isopropyl, tert-butyl, CHF₂, CH₂CF₃, CH₂CHF₂, CF₃, CH₂C(=O)CH₃, CH₂C(CH₃)₂OH, CH₂CH₂OCH₃, (CH₂)₂N(CH₃)₂, cyclopropyl, phenyl, 4-fluorophenyl, 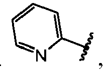 CH₂-cyclopropyl or

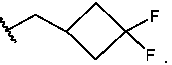

In some embodiments, one or both of R^{v} and R^{x} are H. In other embodiments, one or both of R^{v} and R^{x} are C₁₋₆alkyl such as methyl, ethyl, propyl, or butyl. In yet other embodiments, one or both of R^{v} and R^{x} are methyl. In some embodiments, one or both of R^{y} and R^{z} are H. In other embodiments, one or both of R^{y} and R^{z} are C₁₋₆alkyl such as methyl, ethyl, propyl, or butyl. In yet other embodiments, one or both of R^{y} and R^{z} are C₁₋₆alkoxy(alkylene) such as CH₂OCH₃. In further embodiments, one or both of R^{y} and R^{z} are C₃₋₆cycloalkyl such as cyclopropyl, cyclobutyl, cyclopentyl, or cyclohexyl. In some embodiments, one or both of R^{y2} and R^{z2} are H. In other embodiments, one or both of R^{y2} and R^{z2} are C₁₋₆alkyl such as methyl, ethyl, propyl, or butyl. In further embodiments, one or both of R^{y2} and R^{z2} are C₃₋₆cycloalkyl such as cyclopropyl, cyclobutyl, cyclopentyl, or cyclohexyl. In further embodiments, p is 0. In other embodiments, p is 1. In still further embodiments, p is 2. In yet other embodiments, p is 3.

In some embodiments, R² is 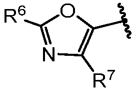 wherein: R⁶ is H, C₁₋₆alkyl, C₁₋₆haloalkyl, C₁₋₆cyanoalkyl, C₁₋₆hydroxyalkyl, C₁₋₆alkoxy, C₁₋₆alkoxy(alkylene), C₁₋₆haloalkoxy, C₁₋₆haloalkoxy(alkylene), C₁₋₆deuteratedalkoxy(alkylene), (CR^{v}R^{x})ₚNR^{y}R^{z}, optionally substituted C₃₋₆cycloalkyl, optionally substituted aryl, optionally substituted heterocyclyl, optionally substituted heteroaryl, optionally substituted C₂₋₆alkenyl, optionally substituted (C₃₋₈cycloalkyl)alkylene, optionally substituted (heterocyclyl)alkylene, or C₁₋₆alkylsulfonyl(alkylene); R⁷ is H, CN, C₁₋₆alkyl, C₁₋₆haloalkyl, halo, C₃₋₆cycloalkyl, aryl, or heteroaryl; R^{v} and R^{x} are, independently, H or C₁₋₆alkyl; R^{y} and R^{z} are, independently, H, C₁₋₆alkyl, or C₃₋₆cycloalkyl; and p is 0, 1, 2, or 3. In further embodiments, R⁶ is H, C₁₋₆alkyl, C₁₋₆haloalkyl, C₁₋₆cyanoalkyl, C₁₋₆hydroxyalkyl, C₁₋₆alkoxy, C₁₋₆alkoxy(alkylene), C₁₋₆haloalkoxy, C₁₋₆haloalkoxy(alkylene), C₁₋₆deuteratedalkoxy(alkylene), (CR^{v}R^{x})ₚNR^{y}R^{z}, optionally substituted C₃₋₆cycloalkyl, optionally substituted heterocyclyl, or optionally substituted heteroaryl; R⁷ is H, C₁₋₆alkyl, C₁₋₆haloalkyl, halo, or C₃₋₆cycloalkyl; R^{v} and R^{x} are, independently, H or C₁₋₆alkyl; R^{y} and R^{z} are, independently, H, C₁₋₆alkyl, or C₃₋₆cycloalkyl; and p is 0, 1, 2, or 3. In some embodiments, the optionally substituted heteroaryl is substituted with one or more of C₁₋₆alkyl such as methyl, C₁₋₆haloalkyl such as CF₃, halo such as F, or C₁₋₆alkoxy such as methoxy. In other embodiments, the optionally substituted C₃₋₆cycloalkyl is substituted with one or more OH. In yet other embodiments, the optionally substituted heterocyclyl is substituted with one or more of halo such as F or C₁₋₆alkyl such as methyl. In other embodiments, R⁶ is H. In further embodiments, R⁶ is C₁₋₆alkyl. In still further embodiments, R⁶ is methyl, isopropyl, or tert-butyl. In yet other embodiments, R⁶ is C₁₋₆haloalkyl. In still further embodiments, R⁶ is CF₂H, C(CH₃)₂F, CH(CH₃)F, or CF₃. In other embodiments, R⁶ is C₁₋₆cyanoalkyl. In further embodiments, R⁶ is C(CH₃)₂CN. In yet other embodiments, R⁶ is C₁₋₆hydroxyalkyl. In still further embodiments, R⁶ is C(CH₃)₂OH, CH₂C(CH₃)₂OH, CH(CH₂CH₃)OH, C(CH₃)₂(CH₂OH), or CH(CH₃)OH. In other embodiments, R⁶ is C₁₋₆alkoxy, such as methoxy. In yet other embodiments, R⁶ is C₁₋₆alkoxy(alkylene). In further embodiments, R⁶ is CH₂OCH₃, C(CH₃)₂OCH₃, or (CH₂)₂OCH₃. In still other embodiments, R⁶ is C₁₋₆haloalkoxy. In yet other embodiments, R⁶ is O(CH₂)₂CF₃. In other embodiments, R⁶ is C₁₋₆haloalkoxy(alkylene) In further embodiments, R⁶ is CH₂OCF₃. In yet other embodiments, R⁶ is C₁₋₆deuteratedalkoxy(alkylene). In still other embodiments, R⁶ is CH₂OCD₃. In further embodiments, R⁶ is (CR^{v}R^{x})ₚNR^{y}R^{z}. In yet other embodiments, R⁶ is NH₂, CH₂N(CH₃)₂, (CH₂)₂N(CH₃)₂, C(CH₃)₂NHCH₃, C(CH₃)₂N(CH₃)₂, or NHcyclopropyl. In still other embodiments, R⁶ is optionally substituted C₃₋₆cycloalkyl. In further embodiments, R⁶ is 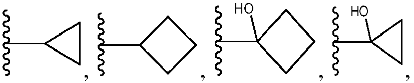 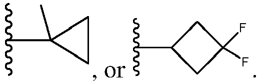 In still further embodiments, , R⁶ is 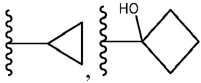 , or 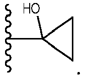 In other embodiments, R⁶ is optionally substituted aryl. In further embodiments, R⁶ is phenyl, 4-fluorophenyl, 3-fluorophenyl, 2-fluorophenyl, or 2,6-difluorophenyl. In other embodiments, R⁶ is optionally substituted heterocyclyl. In further embodiments, R⁶ is 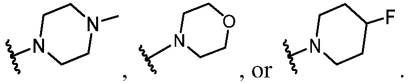 In other embodiments, R⁶ is optionally substituted heteroaryl. In yet other embodiments, R⁶ is optionally substituted pyridinyl, optionally substituted pyrazolyl, optionally substituted pyrazinyl, optionally substituted pyrimidinyl, or optionally substituted imidazolyl. In further embodiments, R⁶ is 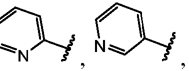 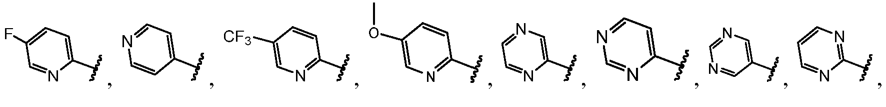 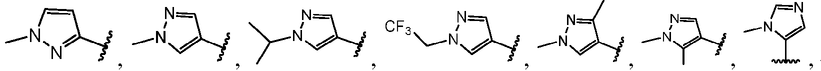 or 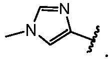 In still further embodiments, R⁶ is 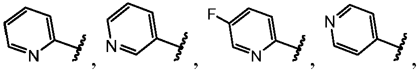 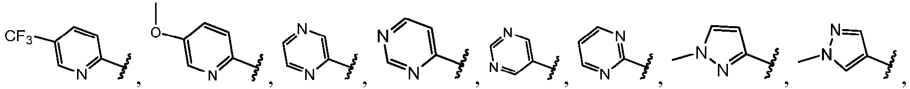 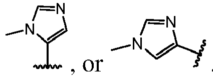 . In other embodiments, R⁶ is optionally substituted C₂₋₆alkenyl. In further embodiments, R⁶ is CH=CH₂, CH=CHC(CH₃)₂OH, or CH=CH-cyclopropyl. In other embodiments, R⁶ is optionally substituted (C₃₋₈cycloalkyl)alkylene. In further embodiments, R⁶ is CH₂-cyclopropyl, CH₂CH₂-cyclopropyl or C(CH₃)OH-cyclopropyl. In other embodiments, R⁶ is optionally substituted (heterocyclyl)alkylene. In further embodiments, R⁶ is 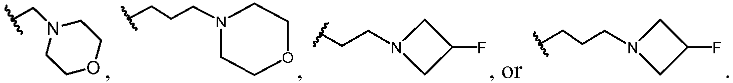 In other embodiments, C₁₋₆alkylsulfonyl(alkylene). In further embodiments, R⁶ is C(CH₃)₂SO₂CH₃. In other embodiments, R⁶ is H, methyl, isopropyl, tert-butyl, C(CH₃)₂F, CH(CH₃)F, CF₃, C(CH₃)₂CN, C(CH₃)₂OH, CH₂C(CH₃)₂OH, CH(CH₂CH₃)OH, C(CH₃)₂(CH₂OH), CH(CH₃)OH, CH₂OCH₃, C(CH₃)₂OCH₃, (CH₂)₂OCH₃, O(CH₂)₂CF₃, CH₂OCF₃, CH₂OCD₃, NH₂, CH₂N(CH₃)₂, (CH₂)₂N(CH₃)₂, C(CH₃)₂NHCH₃, C(CH₃)₂N(CH₃)₂, NHcyclopropyl, 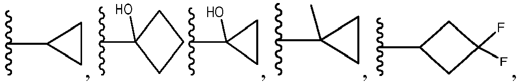 phenyl, 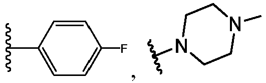 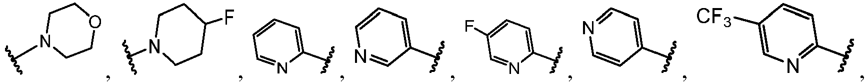 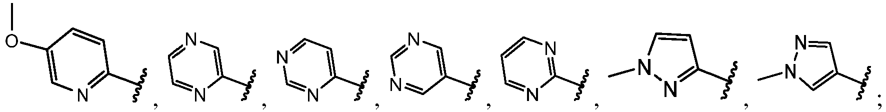 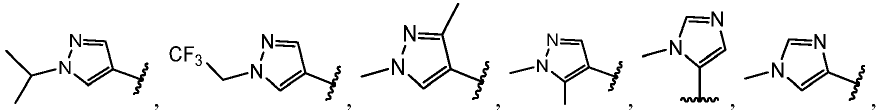 CH=CHC(CH₃)₂OH, CH=CH-cyclopropyl, CH₂-cyclopropyl, CH₂CH₂-cyclopropyl, C(CH₃)OH-cyclopropyl, 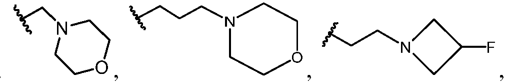 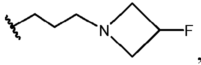 or C(CH₃)₂SO₂CH₃. In yet other embodiments, R⁶ is H, tert-butyl, C(CH₃)₂F, C(CH₃)₂CN, C(CH₃)₂OH, C(CH₃)₂(CH₂OH), CH(CH₃)OH, CH₂OCH₃, CH₂OCF₃, CH₂OCD₃, C(CH₃)₂NHCH₃, C(CH₃)₂N(CH₃)₂, 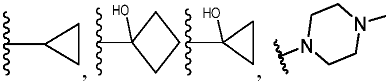 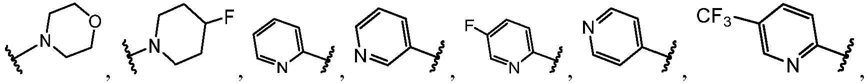 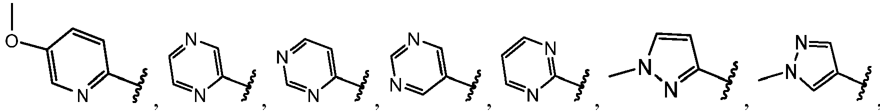 , or 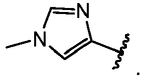 In yet other embodiments, R⁷ is H. In still further embodiments, R⁷ is CN. In other embodiments, R⁷ is C₁₋₆alkyl. In further embodiments, R⁷ is methyl. In yet other embodiments, R⁷ is C₁₋₆haloalkyl. In still further embodiments, R⁷ is CHF₂, CH₂F, C(CH₃)F₂, CH₂CHF₂, or CF₃. In further embodiments, R⁷ is halo. In yet other embodiments, R⁷ is Br or Cl. In still further embodiments, R⁷ is C₃₋₆cycloalkyl. In other embodiments, R⁷ is cyclopropyl. In further embodiments, R⁷ is aryl. In yet other embodiments, R⁷ is phenyl. In still further embodiments, R⁷ is heteroaryl. In other embodiments, R⁷ is pyridinyl. In yet other embodiments, R⁷ is H, CN, methyl, CHF₂, CH₂F, C(CH₃)F₂, CH₂CHF₂, CF₃, Br, Cl, cyclopropyl, phenyl, or pyridinyl. In still other embodiments, R⁷ is H, methyl, CHF₂, CH₂F, C(CH₃)F₂, CH₂CHF₂, CF₃, Br, Cl, or cyclopropyl.

In other embodiments, R² is 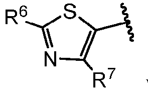 wherein: R⁶ is H, C₁₋₆alkyl, C₁₋₆haloalkyl, C₁₋₆hydroxyalkyl, C₁₋₆alkoxy, C₁₋₆alkoxy(alkylene), optionally substituted C₃₋₆cycloalkyl, (CR^{v}R^{x})ₚNR^{y}R^{z}, optionally substituted aryl, optionally substituted aryl(alkylene), optionally substituted heteroaryl, optionally substituted C₃₋₈cycloalkenyl, or optionally substituted heterocyclyl; R⁷ is H, C₁₋₆alkyl, C₁₋₆haloalkyl, halo, or C₃₋₆cycloalkyl; R^{v} and R^{x} are, independently, H or C₁₋₆alkyl; R^{y} and R^{z} are, independently, H, C₁₋₆alkyl, or C₃₋₆cycloalkyl; and p is 0, 1, 2, or 3. In yet other embodiments, R⁶ is H, C₁₋₆alkyl, C₁₋₆haloalkyl, or optionally substituted heteroaryl; and R⁷ is H. In further embodiments, R⁶ is H. In further embodiments, R⁶ is C₁₋₆alkyl. In still further embodiments, R⁶ is methyl, isopropyl, or tert-butyl. In yet other embodiments, R⁶ is C₁₋₆haloalkyl. In still further embodiments, R⁶ is CHF₂, C(CH₃)F₂, C(CH₃)₂F, or CF₃. In yet other embodiments, R⁶ is C₁₋₆hydroxyalkyl. In still further embodiments, R⁶ is C(CH₃)₂OH. In other embodiments, R⁶ is C₁₋₆alkoxy. In still other embodiments, R⁶ is methoxy. In yet other embodiments, R⁶ is C₁₋₆alkoxy(alkylene). In further embodiments, R⁶ is CH₂OCH₃. In other embodiments, R⁶ is optionally substituted C₃₋₆cycloalkyl. In yet further embodiments, R⁶ is 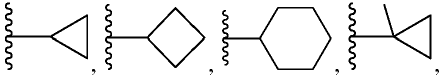 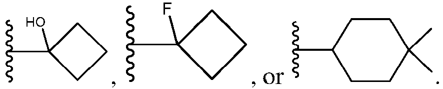 In further embodiments, R⁶ is (CR^{v}R^{x})ₚNR^{y}R^{z}. In yet other embodiments, R⁶ is NHcyclopropyl. In other embodiments, R⁶ is optionally substituted aryl. In further embodiments, R⁶ is phenyl, 3-fluorophenyl, 4-fluorophenyl, or 2,6-difluorophenyl. In other embodiments, R⁶ is optionally substituted aryl(alkylene). In further embodiments, R⁶ is benzyl. In other embodiments, R⁶ is optionally substituted heteroaryl. In yet other embodiments, R⁶ is optionally substituted pyridinyl or optionally substituted pyrazolyl. In further embodiments, R⁶ is 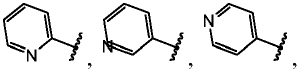 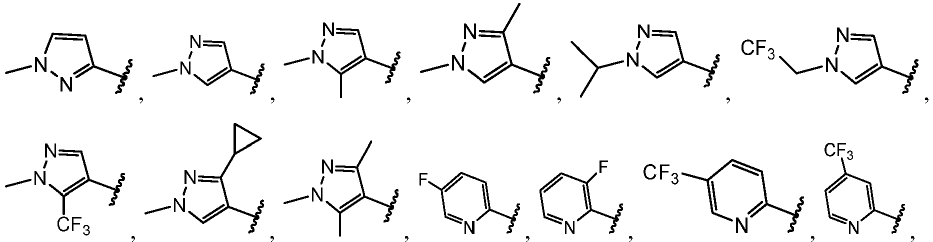 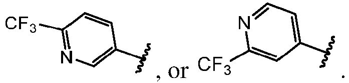 In still further embodiments, R⁶ is 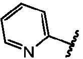 or 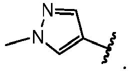 In other embodiments, R⁶ is optionally substituted C₃₋₈cycloalkenyl. In further embodiments, R⁶ is 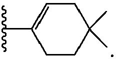 In other embodiments, R⁶ is optionally substituted heterocyclyl. In further embodiments, R⁶ is 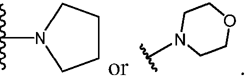 In other embodiments, R⁶ is H, methyl, tert-butyl, CF₃, CHF₂, C(CH₃)₂F, C(CH₃)F₂, C(CH₃)₂OH, CH₂OCH₃, 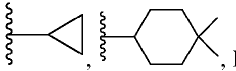 NH-cyclopropyl, phenyl, 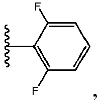 benzyl, 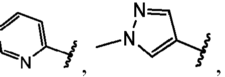 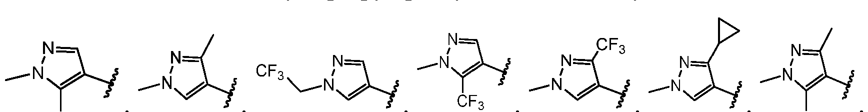 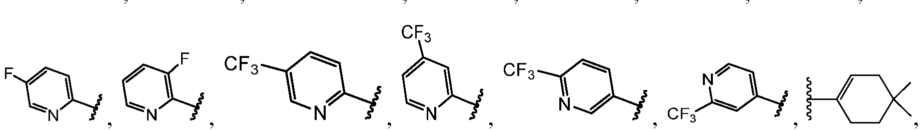 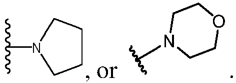 In further embodiments, R⁶ is H, tert-butyl, C(CH₃)F₂, 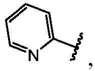 or 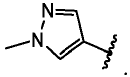 In yet other embodiments, R⁷ is H. In other embodiments, R⁷ is C₁₋₆alkyl. In further embodiments, R⁷ is methyl. In yet other embodiments, R⁷ is C₁₋₆haloalkyl. In still further embodiments, R⁷ is CF₂H or CF₃. In further embodiments, R⁷ is halo. In yet other embodiments, R⁷ is Br or Cl. In still further embodiments, R⁷ is C₃₋₆cycloalkyl. In other embodiments, R⁷ is cyclopropyl. In still further embodiments, R⁷ is H, methyl, CF₂H, CF₃, Br, Cl, or cyclopropyl.

In further embodiments, R² is 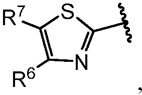 wherein R⁶ and R⁷ are independently H, C₁₋₆alkyl, C₁₋₆haloalkyl, halo, optionally substituted C₃₋₆cycloalkyl, or optionally substituted aryl. In other embodiments, R⁶ and R⁷ are each H. In further embodiments, one of R⁶ or R⁷ is C₁₋₆alkyl such as methyl. In yet other embodiments, one of R⁶ or R⁷ is C₁₋₆haloalkyl such as CF₃. In still other embodiments, one of R⁶ or R⁷ is halo such as Br or Cl. In yet further embodiments, one of R⁶ or R⁷ is optionally substituted C₃₋₆cycloalkyl such as unsubstituted cyclopropyl. In other embodiments, one of R⁶ or R⁷ is optionally substituted aryl such as unsubstituted phenyl. In yet other embodiments, R⁶ is methyl, CF₃, Cl, cyclopropyl, or phenyl and R⁷ is H. In still other embodiments, R⁷ is methyl, CF₃, Cl, cyclopropyl, or phenyl and R⁶ is H. In still further embodiments, R⁷ is Cl, and R⁶ is H.

In yet further embodiments, R² is 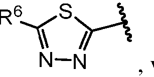 wherein R⁶ is H, C₁₋₆alkyl, halo, or optionally substituted aryl. In other embodiments, R⁶ is H. In further embodiments, R⁶ is C₁₋₆alkyl, such as methyl, ethyl, isopropyl, or tert-butyl. In other embodiments, R⁶ is methyl or ethyl. In other embodiments, R⁶ is halo, such as F, Br, or Cl. In further embodiments, R⁶ is Br. In other embodiments, R⁶ is optionally substituted aryl. In further embodiments, R⁶ is phenyl. In yet other embodiments, R⁶ is H, methyl, ethyl, Br, or phenyl.

In yet other embodiments, R² is 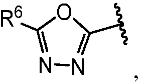 wherein R⁶ is H, C₁₋₆alkyl, C₁₋₆haloalkyl, C₁₋₆hydroxyalkyl, (CR^{v}R^{x})ₚNR^{y}R^{z}, C₁₋₆haloalkoxy, optionally substituted C₃₋₆cycloalkyl, optionally substituted aryl, optionally substituted heterocyclyl, or optionally substituted heteroaryl; R^{v} and R^{x} are, independently, H or C₁₋₆alkyl; R^{y} and R^{z} are, independently, H, C₁₋₆alkyl, C₁₋₆alkoxy(alkylene), or C₃₋₆cycloalkyl; and p is 0, 1, 2, or 3. In further embodiments, R⁶ is H, C₁₋₆alkyl, C₁₋₆haloalkyl, C₁₋₆hydroxyalkyl, optionally substituted C₃₋₆cycloalkyl, optionally substituted aryl, or optionally substituted heteroaryl. In some embodiments, the optionally substituted heteroaryl is substituted with one or more of C₁₋₆alkyl such as methyl, ethyl, or isopropyl, C₁₋₆haloalkyl such as CF₃, CH₂CF₃, or CHF₂, halo such as F, C₁₋₆alkoxy such as methoxy, ethoxy, or propoxy, C₃₋₆cycloalkyl such as cyclopropyl or cyclobutyl; or C3-6cycloalkylsulfonyl such as cyclopropylsulfonyl, cyclobutylsulfonyl, or cyclopentylsulfonyl. In other embodiments, the optionally substituted aryl is substituted with one or more of halo such as F, C₁₋₆alkyl such as methyl, or C₁₋₆haloalkyl such as CF₃. In some embodiments, R⁶ is H. In further embodiments, R⁶ is C₁₋₆alkyl. In other embodiments, R⁶ is methyl, ethyl, isopropyl, or tert-butyl. In other embodiments, R⁶ is C₁₋₆haloalkyl. In further embodiments, R⁶ is CHF₂, CF₃, or C(CH₃)₂F. In yet other embodiments, R⁶ is C₁₋₆hydroxyalkyl. In still further embodiments, R⁶ is C(CH₃)₂OH or CH(CH₃)OH. In other embodiments, R⁶ is (CR^{v}R^{x})ₚNR^{y}R^{z}. In further embodiments, R⁶ is NH₂, N(CH₃)₂, NHCH₂CF₃, NHCH₂CH₂OCH₃, NH(cyclopropyl), CH₂N(CH₃)₂, CH₂NH(cyclopropyl), or CH₂CH₂NH(cyclopropyl). In still further embodiments, R⁶ is C₁₋₆haloalkoxy such as OCF₃, OCHF₂, OCH₂F, or OCH₂CH₂CF₃. In yet other embodiments, R⁶ is optionally substituted C₃₋₆cycloalkyl. In further embodiments, R⁶ is unsubstituted C₃₋₆cycloalkyl. In still further embodiments, R⁶ is 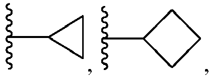 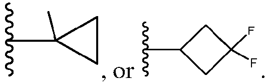 In still other embodiments, R⁶ is 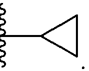 In other embodiments, R⁶ is optionally substituted aryl. In further embodiments, R⁶ is 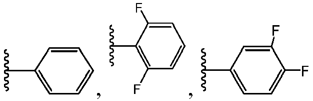 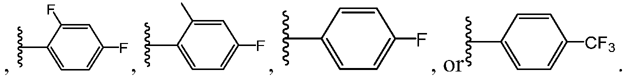 In still other embodiments, R⁶ is optionally substituted heterocyclyl. In yet further embodiments, R⁶ is 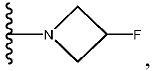 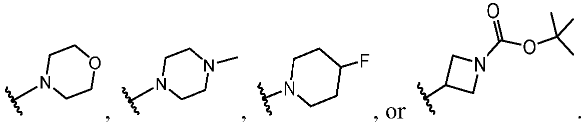 In other embodiments, R⁶ is optionally substituted heteroaryl such as optionally substituted pyrazolyl, optionally substituted imidazolyl, optionally substituted pyridinyl, or optionally substituted pyrazinyl. In yet other embodiments, R⁶ is 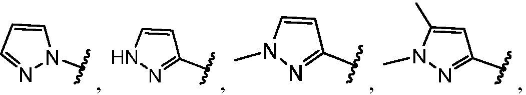 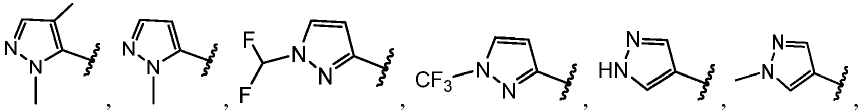 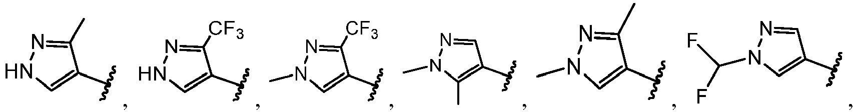 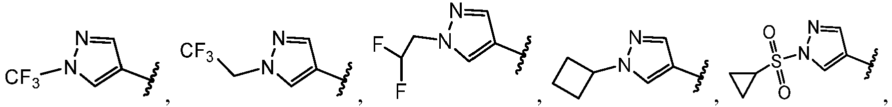 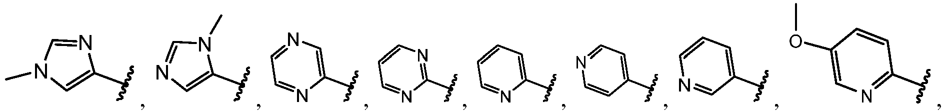 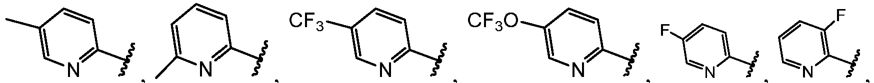 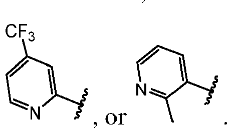 In still other embodiments, R⁶ is 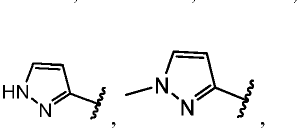 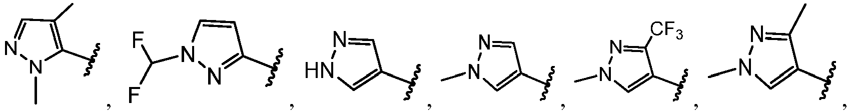 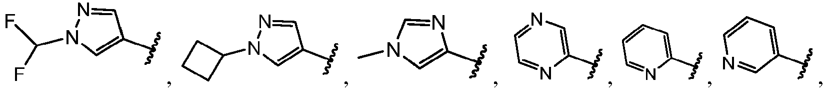 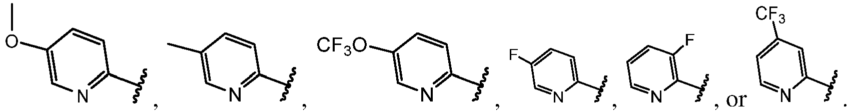 In further embodiments, R⁶ is methyl, tert-butyl, C(CH₃)₂F, C(CH₃)₂OH, N(CH₃)₂, NH(cyclopropyl), OCH₂CH₂CF₃, 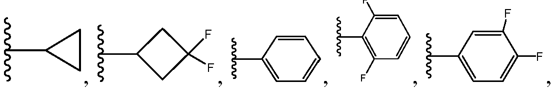 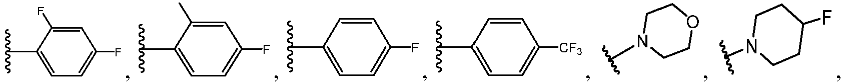 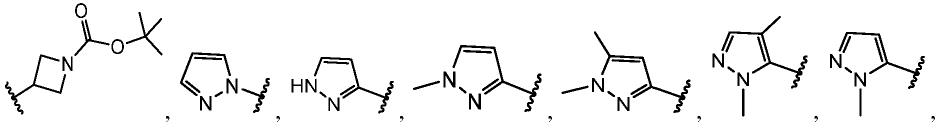 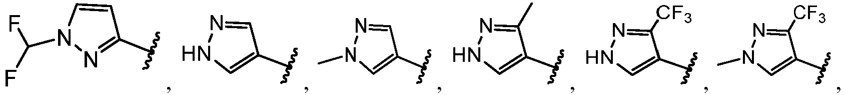 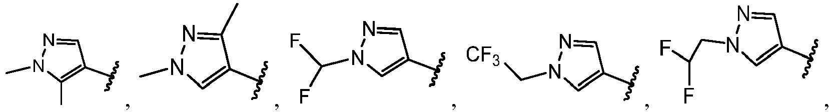 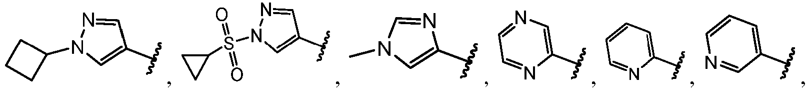 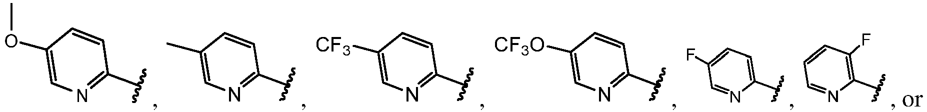 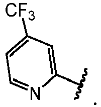 In still further embodiments, R⁶ is tert-butyl, C(CH₃)₂F, CF₃, C(CH₃)₂OH,

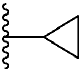

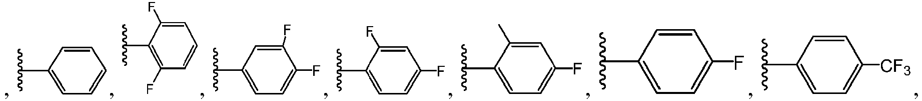

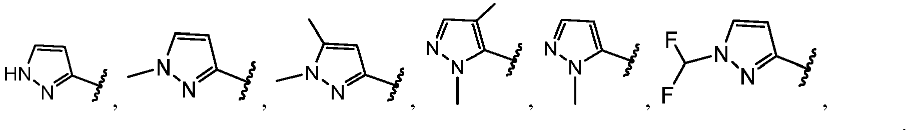

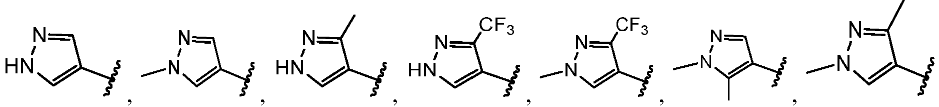

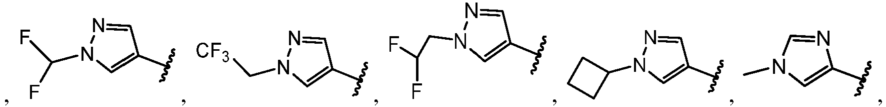

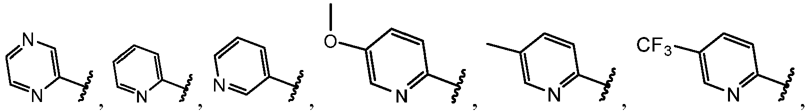

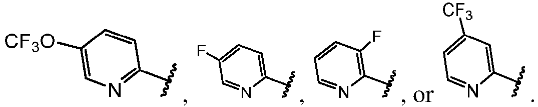

In still further embodiments, R² is 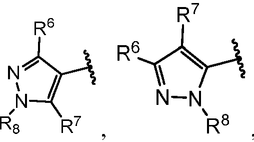 , or 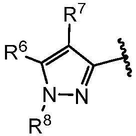 wherein: R⁶ and R⁷ are, independently, H, halo, C₁₋₆alkyl, C₁₋₆haloalkyl, (CR^{v}R^{x})ₚNR^{y}R^{z}, or C₃₋₆cycloalkyl; R⁸ is H, C₁₋₆alkyl, C₁₋₆haloalkyl, C₁₋₆alkylcarbonyl, C₁₋₆hydroxyalkyl, C₁₋₆alkoxy(alkylene), (CR^{v}R^{x})ₚNR^{y}R^{z}, C₃₋₆cycloalkyl, optionally substituted aryl, heteroaryl, or optionally substituted C₃₋₈cycloalkyl(alkylene); R^{v} and R^{x} are, independently, H or C₁₋₆alkyl; R^{y} and R^{z} are, independently, H, C₁₋₆alkyl, or C₃₋₆cycloalkyl; and p is 0, 1, 2, or 3. In other embodiments, R⁶ and R⁷ are, independently, H, halo, C₁₋₆alkyl, or C₁₋₆haloalkyl; R⁸ is H, C₁₋₆alkyl, C₁₋₆haloalkyl, C₃₋₆cycloalkyl, or heteroaryl. In some embodiments, one or both of R⁶ and R⁷ are H. In still other embodiments, one of R⁶ or R⁷ is halo. In further embodiments, one of R⁶ or R⁷ is Cl or Br. In yet other embodiments, one of R⁶ or R⁷ is C₁₋₆alkyl. In still further embodiments, one of R⁶ or R⁷ is methyl. In other embodiments, one of R⁶ or R⁷ is C₁₋₆haloalkyl. In further embodiments, one of R⁶ or R⁷ is CHF₂, CH₂CF₃, CH₂F, or CF₃. In yet other embodiments, , one of R⁶ or R⁷ is CHF₂. In other embodiments, one of R⁶ or R⁷ is (CR^{v}R^{x})ₚNR^{y}R^{z}. In further embodiments, one of R⁶ or R⁷ is NH₂. In other embodiments, one of R⁶ or R⁷ is C₃₋₆cycloalkyl. In further embodiments, one or both of R⁶ or R⁷ are cyclopropyl or cyclobutyl. In yet other embodiments, R⁶ and R⁷ are each independently H, Cl, Br, methyl, CHF₂, CFH₂, CF₃, NH₂, or cyclobutyl. In further embodiments, R⁶ and R⁷ are each independently H, Cl, methyl, or CHF₂. In some embodiments, R⁸ is H. In other embodiments, R⁸ is C₁₋₆alkyl. In further embodiments, R⁸ is methyl, ethyl, isopropyl, or tert-butyl. In other embodiments, R⁸ is methyl or isopropyl. In still other embodiments, R⁸ is C₁₋₆haloalkyl. In yet further embodiments, R⁸ is CHF₂, CH₂CF₃, CF₂H, CH₂CHF₂, or CF₃. In other embodiments, R⁸ is C₁₋₆alkylcarbonyl. In further embodiments, R⁸ is C(=O)CH₃ or CH₂C(=O)CH₃. In other embodiments, R⁸ is C₁₋₆hydroxyalkyl. In further embodiments, R⁸ is CH₂C(CH₃)₂OH. In other embodiments, R⁸ is C₁₋₆alkoxy(alkylene). In further embodiments, R⁸ is CH₂CH₂OCH₃. In other embodiments, R⁸ is (CR^{v}R^{x})ₚNR^{y}R^{z}. In further embodiments, R⁸ is (CH₂)₂N(CH₃)₂. In other embodiments, R⁸ is C₃₋₆cycloalkyl. In further embodiments, R⁸ is cyclopropyl. In yet other embodiments, R⁸ is optionally substituted aryl. In still further embodiments, R⁸ is phenyl or 4-fluorophenyl. In other embodiments, R⁸ is heteroaryl. In further embodiments, R⁸ is pyridinyl. In other embodiments, R⁸ is substituted C₃₋₈cycloalkyl(alkylene). In further embodiments, R⁸ is CH₂-cyclopropyl or 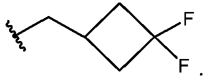 In still other embodiments, R⁸ is H, methyl, isopropyl, tert-butyl, CHF₂, CH₂CF₃, CH₂CHF₂, CF₃, CH₂C(=O)CH₃, CH₂C(CH₃)₂OH, CH₂CH₂OCH₃, (CH₂)₂N(CH₃)₂, cyclopropyl, phenyl, 4-fluorophenyl, 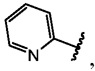 CH₂-cyclopropyl or 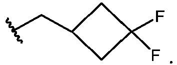 In further embodiments, R⁸ is methyl, isopropyl, CHF₂, CH₂CF₃, CH₂CHF₂, CF₃, cyclopropyl, or pyridinyl.

In further embodiments, R² is 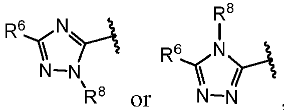 wherein R⁶ is H, C₁₋₆alkyl, halo, C₁₋₆haloalkyl, C₃₋₆cycloalkyl, or C₁₋₆cyanoalkyl; and R⁸ is H, C₁₋₆alkyl, C₁₋₆haloalkyl, or C₃₋₆cycloalkyl. In other embodiments, R⁶ is H, C₁₋₆alkyl, halo, C₁₋₆haloalkyl, or C₃₋₆cycloalkyl; and R⁸ is H or C₁₋₆alkyl. In some embodiments, R⁶ is H. In still other embodiments, R⁶ is C₁₋₆alkyl such as methyl. In yet other embodiments, R⁶ is halo such as Cl or Br. In other embodiments, R⁶ is C₁₋₆haloalkyl such as CHF₂ or CF₃. In yet other embodiments, R⁶ is C₃₋₆cycloalkyl such as cyclopropyl. In other embodiments, R⁶ is C₁₋₆cyanoalkyl such as C(CH₃)₂CN. In still further embodiments, R⁶ is H, methyl, Cl, Br, CHF₂, CF₃, cyclopropyl, or C(CH₃)₂CN. In some embodiments, R⁸ is H. In other embodiments, R⁸ is C₁₋₆alkyl such as methyl, ethyl, or isopropyl. In yet other embodiments, R⁸ is C₁₋₆haloalkyl, such as CF₃ or CHF₂. In other embodiments, R⁸ is C₃₋₆cycloalkyl such as cyclopropyl. In yet other embodiments, R⁸ is methyl, ethyl, isopropyl, or cyclopropyl.

In yet other embodiments, R² is 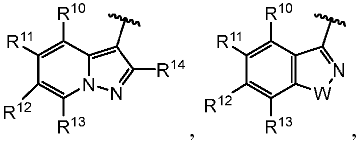 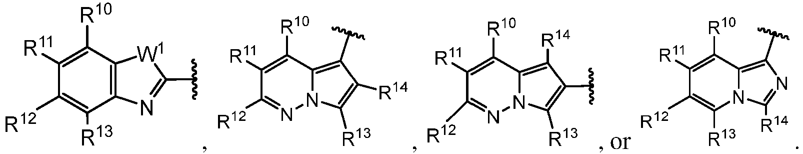 In these structures, W is S or NR¹⁵; W¹ is S, O, or NR¹⁵; R¹⁰, R¹¹, R¹², R¹³, and R¹⁴ are each independently H, C₁₋₆alkyl, C₁₋₆haloalkyl, C₁₋₆alkoxy, C₁₋₆alkoxy(alkylene), C₁₋₆hydroxyalkyl, C₁₋₆haloalkoxy, C₁₋₆haloalkoxy(alkylene), C₂₋₆alkenyl, CN, halo, (CR^{v}R^{x})ₚNR^{y}R^{z}, C(O)NR^{y2}R^{z2}, optionally substituted C₃₋₈cycloalkyl, optionally substituted heterocyclyl, optionally substituted heterocyclyl(alkylene), optionally substituted aryl, or optionally substituted heteroaryl; R^{v} and R^{x} are, independently, H or C₁₋₆alkyl; R^{y} and R^{z} are, independently, H, C₁₋₆alkyl, C₃₋₆cycloalkyl, C₁₋₆hydroxyalkyl, C₁₋₆haloalkyl, C₁₋₆alkoxy(alkylene), or C(O)OC₁₋₆alkyl; R^{y2} and R^{z2}, are independently, H, C₁₋₆alkyl, or C₃₋₆cycloalkyl; p is 0, 1, 2, or 3; and R¹⁵ is H or C₁₋₆alkyl.

In some embodiments, W is S. In other embodiments, W is NR¹⁵. In some embodiments, W¹ is S. In other embodiments, W¹ is O. In further embodiments, W¹ is NR¹⁵. In some embodiments, R¹⁰, R¹¹, R¹², R¹³, and R¹⁴ are each H. In some embodiments, R¹⁰, R¹¹, R¹², and R¹³ are each H. In other embodiments, at least one of R¹⁰, R¹¹, R¹², R¹³, and R¹⁴ is C₁₋₆alkyl. In further embodiments, at least one of R¹⁰, R¹¹, R¹², R¹³, and R¹⁴ is C₁₋₆haloalkyl. In yet other embodiments, at least one of R¹⁰, R¹¹, R¹², R¹³, and R¹⁴ is C₁₋₆alkoxy. In still other embodiments, at least one of R¹⁰, R¹¹, R¹², R¹³, and R¹⁴ is C₁₋₆alkoxy(alkylene). In further embodiments, at least one of R¹⁰, R¹¹, R¹², R¹³, and R¹⁴ is C₁₋₆hydroxyalkyl. In other embodiments, at least one of R¹⁰, R¹¹, R¹², R¹³, and R¹⁴ is C₁₋₆haloalkoxy. In still other embodiments, at least one of R¹⁰, R¹¹, R¹², R¹³, and R¹⁴ is C₁₋₆haloalkoxy(alkylene). In yet other embodiments, at least one of R¹⁰, R¹¹, R¹², R¹³, and R¹⁴ is C₂₋₆alkenyl. In further embodiments, at least one of R¹⁰, R¹¹, R¹², R¹³, and R¹⁴ is CN. In yet other embodiments, at least one of R¹⁰, R¹¹, R¹², R¹³, and R¹⁴ is halo. In still further embodiments, at least one of R¹⁰, R¹¹, R¹², R¹³, and R¹⁴ is (CR^{v}R^{x})ₚNR^{y}R^{z}. In other embodiments, at least one of R¹⁰, R¹¹, R¹², R¹³, and R¹⁴ is C(O)NR^{y2}R^{z2}. In further embodiments, at least one of R¹⁰, R¹¹, R¹², R¹³, and R¹⁴ is optionally substituted C₃₋₈cycloalkyl. In yet other embodiments, at least one of R¹⁰, R¹¹, R¹², R¹³, and R¹⁴ is optionally substituted heterocyclyl. In still other embodiments, at least one of R¹⁰, R¹¹, R¹², R¹³, and R¹⁴ is optionally substituted heterocyclyl(alkylene). In still further embodiments, at least one of R¹⁰, R¹¹, R¹², R¹³, and R¹⁴ is optionally substituted aryl. In other embodiments, at least one of R¹⁰, R¹¹, R¹², R¹³, and R¹⁴ is optionally substituted heteroaryl. In some embodiments, one or both of R^{v} and R^{x} are H. In other embodiments, one or both of R^{v} and R^{x} are C₁₋₆alkyl. In some embodiments, one or both of R^{y} and R^{z} are H. In other embodiments, one or both of R^{y} and R^{z} are C₁₋₆alkyl. In further embodiments, one or both of R^{y} and R^{z} are C₃₋₆cycloalkyl. In yet other embodiments, one or both of R^{y} and R^{z} are C₁₋₆hydroxyalkyl. In still further embodiments, one or both of R^{y} and R² are C₁₋₆haloalkyl. In yet other embodiments, one or both of R^{y} and R^{z} are C₁₋₆alkoxy(alkylene). In further embodiments, one or both of R^{y} and R^{z} are C(O)OC₁₋₆alkyl. In some embodiments, one or both of R^{y2} and R^{z2} are H. In other embodiments, one or both of R^{y2} and R^{z2} are C₁₋₆alkyl. In further embodiments, one or both of R^{y2} and R^{z2} are C₃₋₆cycloalkyl. In some embodiments, p is 0. In other embodiments, p is 1. In further embodiments, p is 2. In yet other embodiments, p is 3. In some embodiments, R¹⁵ is H. In other embodiments, R¹⁵ is C₁₋₆alkyl.

In further embodiments, R² is 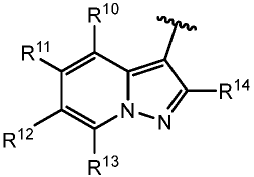 wherein R¹⁰, R¹¹, R¹², R¹³, and R¹⁴ are each independently H, C₁₋₆alkyl, C₁₋₆haloalkyl, C₃₋₆cycloalkyl, C₁₋₆alkoxy, C₁₋₆alkoxy(alkylene), C₁₋₆hydroxyalkyl, CN, halo, C₂₋₆alkenyl, optionally substituted aryl, optionally substituted heterocyclyl, optionally substituted heterocyclyl(alkylene), optionally substituted heteroaryl, or (CR^{v}R^{x})ₚNR^{y}R^{z}. In other embodiments, R¹⁰, R¹¹, R¹², R¹³, and R¹⁴ are each independently H, C₁₋₆alkyl, C₁₋₆haloalkyl, C₁₋₆alkoxy, C₁₋₆alkoxy(alkylene), optionally substituted heterocyclyl, optionally substituted heterocyclyl(alkylene), optionally substituted heteroaryl, or (CR^{v}R^{x})ₚNR^{y}R^{z}. In some embodiments, R¹⁰, R¹¹, R¹², R¹³, and R¹⁴ are H. In other embodiments, at least one of R¹⁰, R¹¹, R¹², R¹³, and R¹⁴ is C₁₋₆alkyl such as methyl or ethyl. In yet other embodiments, at least one of R¹⁰, R¹¹, R¹², R¹³, and R¹⁴ is C₁₋₆alkyl such as methyl. In further embodiments, at least one of R¹⁰, R¹¹, R¹², R¹³, and R¹⁴ is C₁₋₆haloalkyl such as CF₃ or C(CH₃)₂F. In yet further embodiments, at least one of R¹⁰, R¹¹, R¹², R¹³, and R¹⁴ is C₃₋₆cycloalkyl such as cyclopropyl. In yet other embodiments, at least one of R¹⁰, R¹¹, R¹², R¹³, and R¹⁴ is C₁₋₆alkoxy such as methoxy. In other embodiments, at least one of R¹⁰, R¹¹, R¹², R¹³, and R¹⁴ is C₁₋₆alkoxy(alkylene) such as CH₂OCH₃ or (CH₂)₂OCH₃. In still further embodiments, at least one of R¹⁰, R¹¹, R¹², R¹³, and R¹⁴ is C₁₋₆hydroxyalkyl such as C(CH₃)₂OH. In further embodiments, at least one of R¹⁰, R¹¹, R¹², R¹³, and R¹⁴ is CN. In yet other embodiments, at least one of R¹⁰, R¹¹, R¹², R¹³, and R¹⁴ is halo such as F, Br, or Cl. In yet other embodiments, at least one of R¹⁰, R¹¹, R¹², R¹³, and R¹⁴ is C₂₋₆alkenyl such as CH=CH₂. In still further embodiments, at least one of R¹⁰, R¹¹, R¹², R¹³, and R¹⁴ is optionally substituted aryl such as unsubstituted phenyl. In yet other embodiments, at least one of R¹⁰, R¹¹, R¹², R¹³, and R¹⁴ is optionally substituted heterocyclyl such as 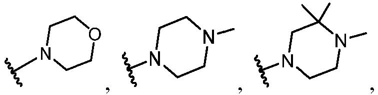 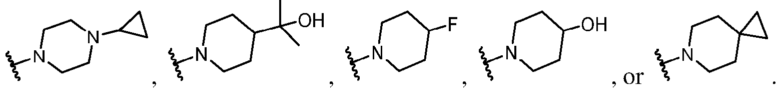 In further embodiments, at least one of R¹⁰, R¹¹, R¹², R¹³, and R¹⁴ is optionally substituted heterocyclyl(alkylene) such as 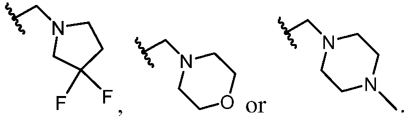 In other embodiments, at least one of R¹⁰, R¹¹, R¹², R¹³, and R¹⁴ is optionally substituted heteroaryl such as optionally substituted pyridinyl, optionally substituted pyrazinyl, optionally substituted pyrimidinyl, or optionally substituted pyrazolyl. In yet other embodiments, at least one of R¹⁰, R¹¹, R¹², R¹³, and R¹⁴ is 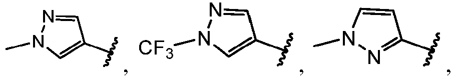 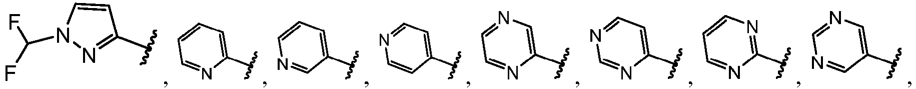 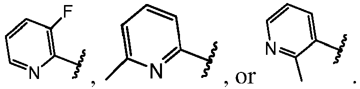 In still other embodiments, at least one of R¹⁰, R¹¹, R¹², R¹³, and R¹⁴ is unsubstituted pyridinyl or 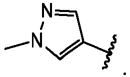 In still further embodiments, at least one of R¹⁰, R¹¹, R¹², R¹³, and R¹⁴ is (CR^{v}R^{x})ₚNR^{y}R^{z} such as NH₂, NH(CH₃), N(CH₃)₂, CH₂N(CH₃)₂, or CH₂CH₂N(CH₃)₂. In further embodiments, R¹⁰, R¹¹, R¹², R¹³, and R¹⁴ each are independently H, methyl, ethyl, C(CH₃)₂F, C(CH₃)₂OH, cyclopropyl, methoxy, CH₂OCH₃, (CH₂)₂OCH₃, Br, F, Cl, CN, CH=CH₂, phenyl, 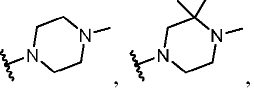 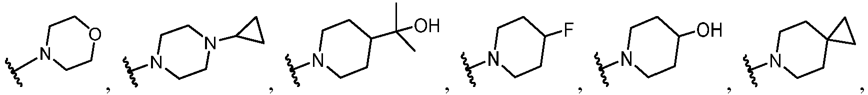 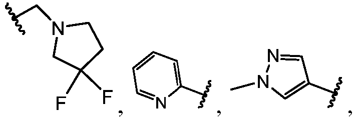 NH₂, NH(CH₃), N(CH₃)₂, CH₂N(CH₃)₂, or CH₂CH₂N(CH₃)₂. In yet other embodiments, R¹⁰, R¹¹, R¹², R¹³, and R¹⁴ each are independently H, methyl, C(CH₃)₂F, CF₃, methoxy, CH₂OCH₃, (CH₂)₂OCH₃, Br, F, Cl, 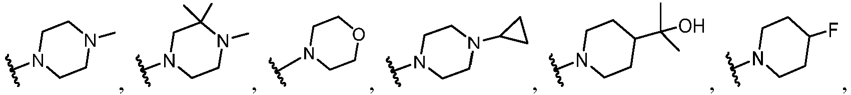 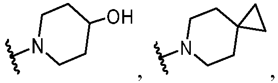 pyridinyl, 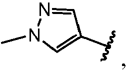 NH₂, NH(CH₃), N(CH₃)₂, CH₂N(CH₃)₂, or CH₂CH₂N(CH₃)₂. In other embodiments, R¹⁰, R¹³, and R¹⁴ are each hydrogen. In further embodiments, R¹⁰, R¹³, and R¹⁴ are each hydrogen; and R¹¹ is halo. In yet further embodiments, R¹⁰, R¹¹, R¹³, and R¹⁴ are each hydrogen.

In further embodiments, at least one of R¹⁰, R¹¹, R¹², R¹³, and R¹⁴ is substituted heterocyclyl or substituted heterocyclyl(alkylene), substituted with one or more of halo such as F, Cl, or Br; C₁₋₆haloalkyl such as CF₃, CH₂CF₃, or CHF₂; C₁₋₆alkyl such as methyl, ethyl, or propyl; OH; C₁₋₆hydroxyalkyl such as C(CH₃)₂OH, C₁₋₆alkoxy such as methoxy, ethoxy, or propoxy; or C₃₋₆cycloalkyl such as cyclopropyl, cyclobutyl, or cyclopentyl. In other embodiments, the substituted heterocyclyl or substituted heterocyclyl(alkylene) is substituted with one or more methyl, OH, F, C(CH₃)₂OH, or cyclopropyl.

In further embodiments, at least one of R¹⁰, R¹¹, R¹², R¹³, and R¹⁴ is substituted heteroaryl, substituted with one or more of halo such as F, Cl, or Br; C₁₋₆haloalkyl such as CF₃, CH₂CF₃, or CHF₂; C₁₋₆alkyl such as methyl, ethyl, propyl, or isopropyl; C₁₋₆alkoxy such as methoxy, ethoxy, or propoxy; C₁₋₆haloalkoxy such as OCF₃; C₃₋₆cycloalkyl such as cyclopropyl, cyclobutyl, or cyclopentyl; or C₃₋₆cycloalkylsulfonyl such as cyclopropylsulfonyl, cyclobutylsulfonyl, or cyclopentylsulfonyl. In still further embodiments, at least one of R¹⁰, R¹¹, R¹², R¹³, and R¹⁴ is substituted heteroaryl, substituted with one or more C₁₋₆alkyl such as methyl, ethyl, propyl, or isopropyl. In yet other embodiments, the substituted heteroaryl is substituted with methyl.

In other embodiments, R² is 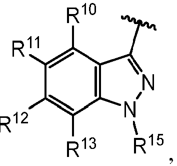 wherein R¹⁰, R¹¹, R¹², and R¹³ are independently H, C₁₋₆alkyl, or halo and R¹⁵ is H or C₁₋₆alkyl. In some embodiments, R¹⁰, R¹¹, R¹², and R¹³ are each H. In other embodiments, at least one of R¹⁰, R¹¹, R¹², and R¹³ is C₁₋₆alkyl. In further embodiments, at least one of R¹⁰, R¹¹, R¹², and R¹³ is halo. In yet other embodiments, at least one of R¹⁰, R¹¹, R¹², and R¹³ is Br. In some embodiments, R¹⁵ is H. In other embodiments, R¹⁵ is C₁₋₆alkyl. In further embodiments, R¹⁵ is methyl. In yet other embodiments, R¹⁵ is H or methyl.

In further embodiments, R² is 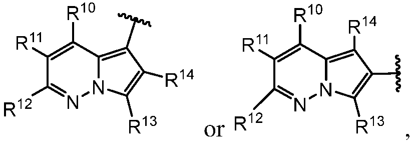 wherein R¹⁰, R¹¹, R¹², R¹³, and R¹⁴ are independently H, halo, or C₁₋₆alkyl. In some embodiments, R² is 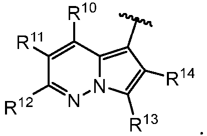 In other embodiments, R² is 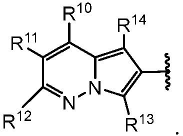 In some embodiments, R¹⁰, R¹¹, R¹², R¹³, and R¹⁴ are each H. In other embodiments, at least one of R¹⁰, R¹¹, R¹², R¹³, and R¹⁴ is halo. In further embodiments, at least one of R¹⁰, R¹¹, R¹², R¹³, and R¹⁴ is C₁₋₆alkyl.

In yet other embodiments, R² is 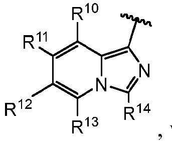 wherein R¹⁰, R¹¹, R¹², R¹³ and R¹⁴ are independently H, halo, or C₁₋₆alkyl. In some embodiments, R¹⁰, R¹¹, R¹², R¹³, and R¹⁴ are each H. In other embodiments, at least one of R¹⁰, R¹¹, R¹², R¹³, and R¹⁴ is halo. In further embodiments, at least one of R¹⁰, R¹¹, R¹², R¹³, and R¹⁴ is Br. In yet other embodiments, at least one of R¹⁰, R¹¹, R¹², R¹³, and R¹⁴ is C₁₋₆alkyl.

In still further embodiments, R² is 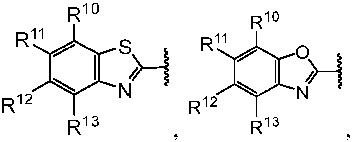 or 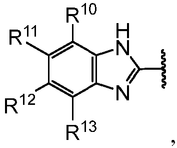 wherein R¹⁰, R¹¹, R¹², and R¹³ are independently H, C₁₋₆alkyl, or halo. In some embodiments, R² is 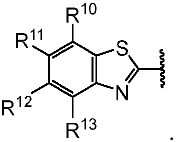 In other embodiments, R² is 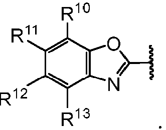 In further embodiments, R² is 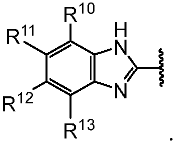 In some embodiments, R¹⁰, R¹¹, R¹², and R¹³ are each H. In other embodiments, at least one of R¹⁰, R¹¹, R¹², and R¹³ is halo. In further embodiments, at least one of R¹⁰, R¹¹, R¹², and R¹³ is F. In yet other embodiments, at least one of R¹⁰, R¹¹, R¹², and R¹³ is C₁₋₆alkyl. In still further embodiments, at least one of R¹⁰, R¹¹, R¹², and R¹³ is methyl. In yet other embodiments, R¹⁰, R¹¹, R¹², and R¹³ are independently H, methyl, or F.

In other embodiments, R² is 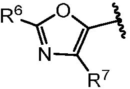 wherein R⁶ is defined herein and R⁷ is H. In yet other embodiments, R² is 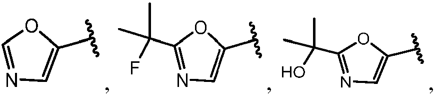 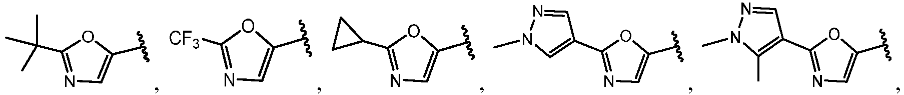 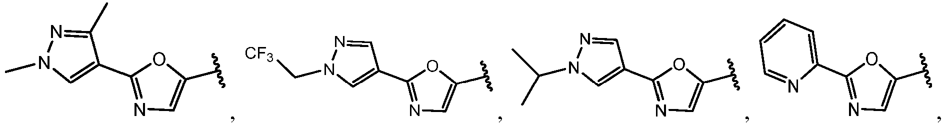 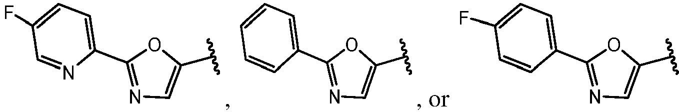 . In further embodiments, R² is 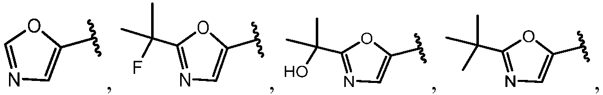 or

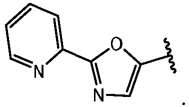

In further embodiments, R² is 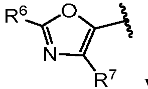 wherein R⁶ is defined herein and R⁷ is CH₃. In other embodiments, R² is 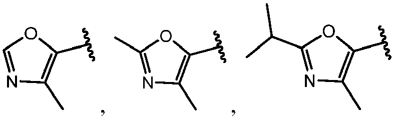 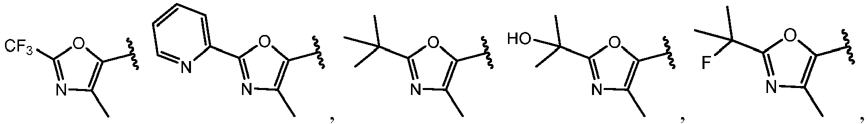 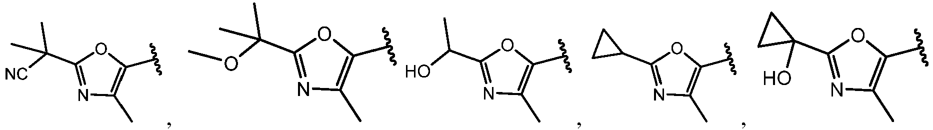 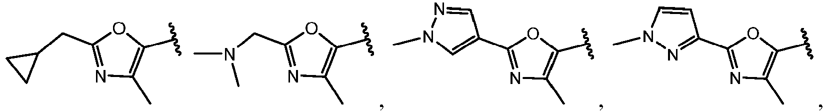 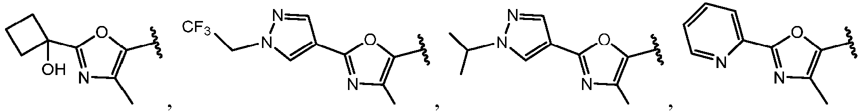 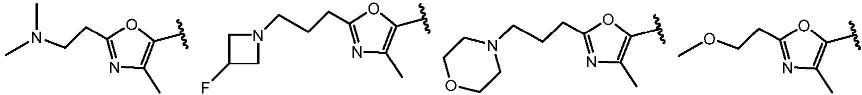 or 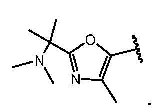 In yet other embodiments, R² is

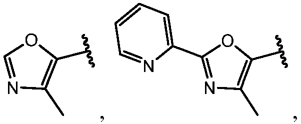

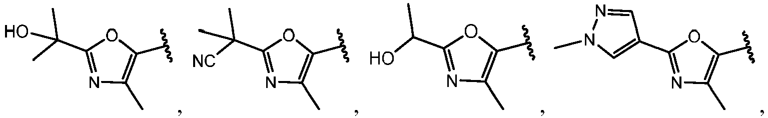

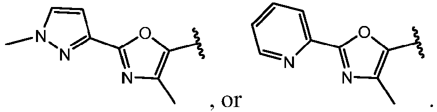

In yet other embodiments, R² is 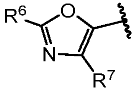 wherein R⁶ is defined herein and R⁷ is cyclopropyl. In further embodiments, R² is 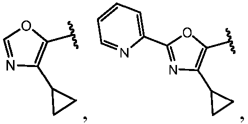 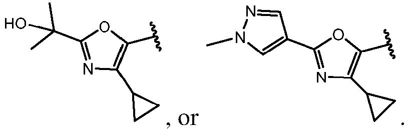 In still further embodiments, R² is

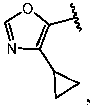

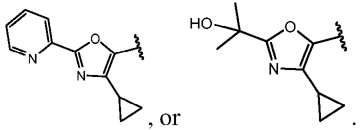

In still further embodiments, R² is 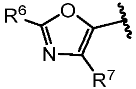 wherein R⁶ is defined herein and R⁷ is CHF₂. In other embodiments, R² is 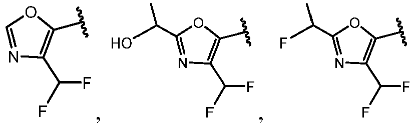 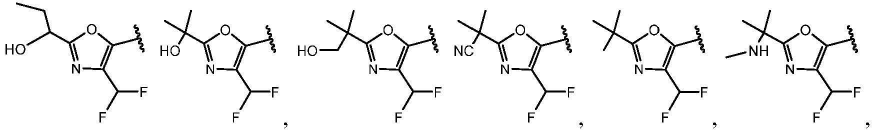 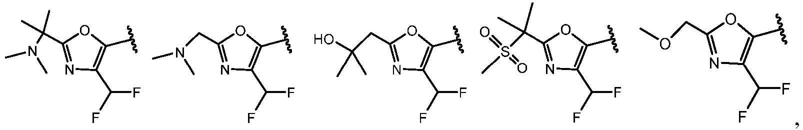 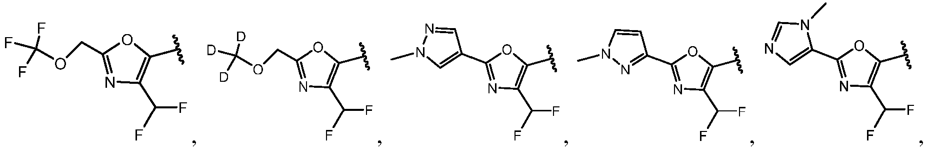 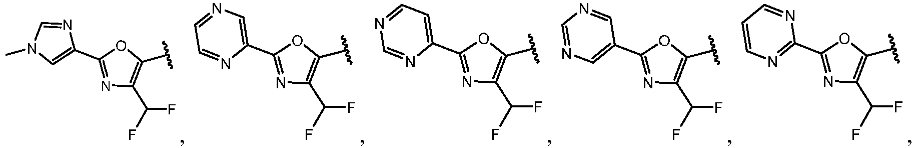 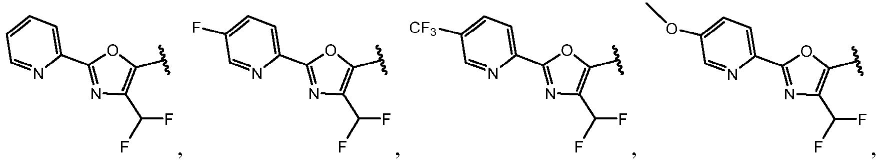 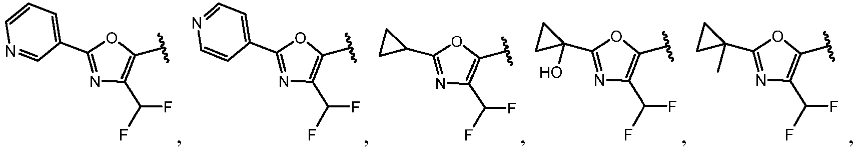 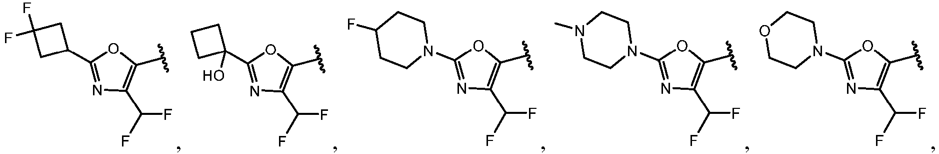 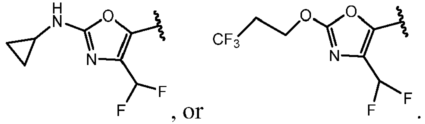 In yet other embodiments, R² is 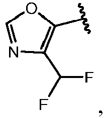 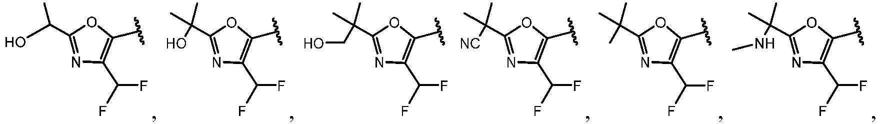 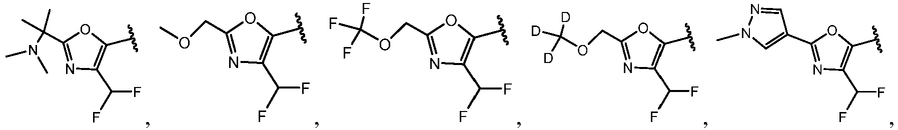 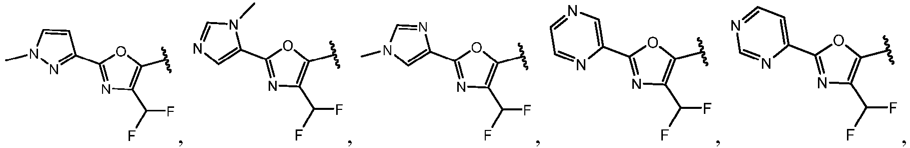 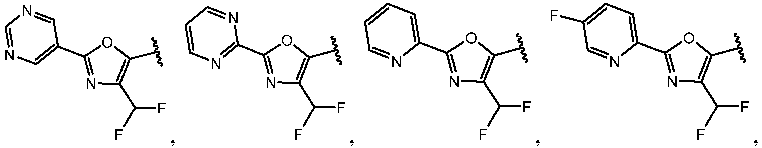 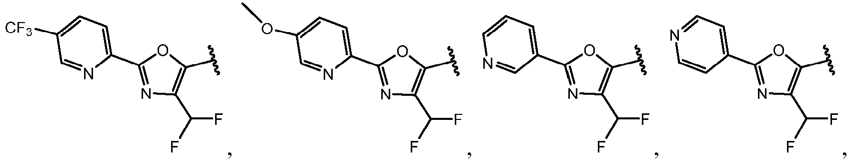 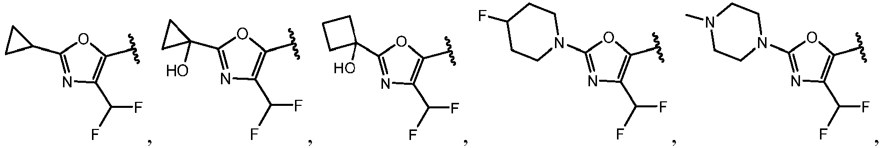 or

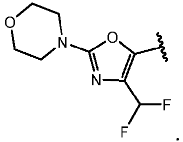

In other embodiments, R² is 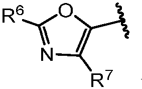 wherein R⁶ is defined herein and R⁷ is CF₃. In still other embodiments, R² is 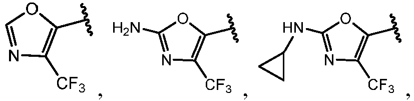 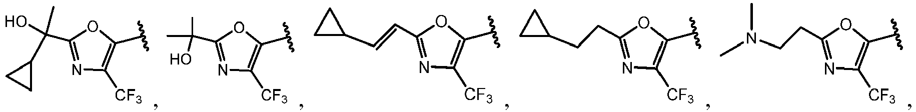 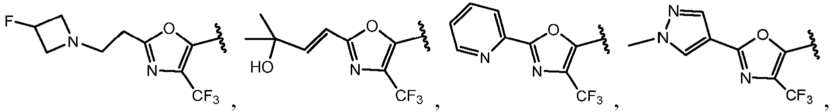 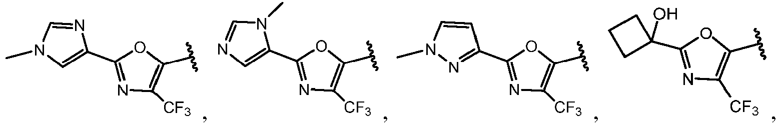 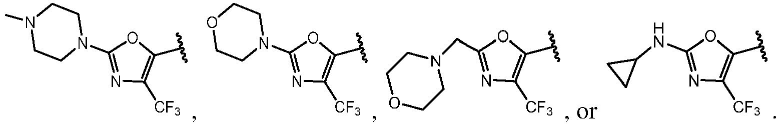 In yet other embodiments, R² is

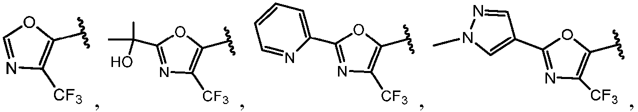

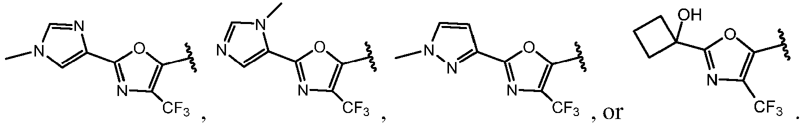

In other embodiments, R² is 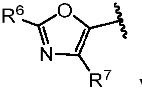 wherein R⁶ is defined herein and R⁷ is CHF₂, C(CH)₂F, or CH₂CHF₂. In further embodiments, R² is

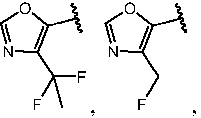

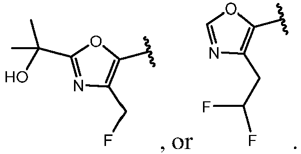

In further embodiments, R² is 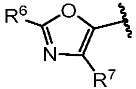 wherein R⁶ is defined herein and R⁷ is CN. In still further embodiments, R² is 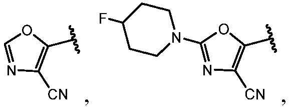 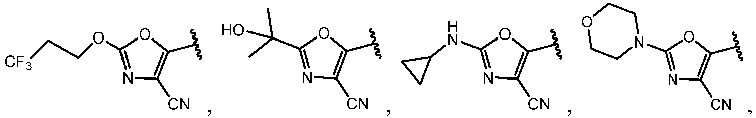 or

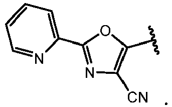

In yet other embodiments, R² is 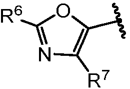 wherein R⁶ is defined herein and R⁷ is Br or Cl. In further embodiments, R² is 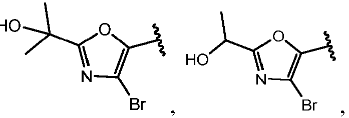 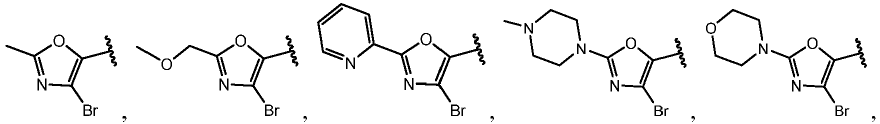 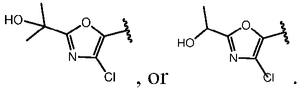 In still further embodiments, R² is

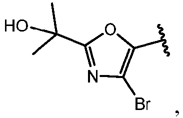

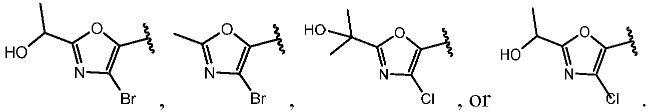

In still further embodiments, R² is 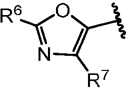 wherein R⁶ is defined herein and R⁷ is phenyl or pyridinyl. In other embodiments, R² is 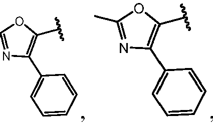 or

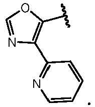

In further embodiments, R² is 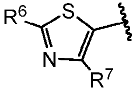 wherein R⁶ is defined herein and R⁷ is H. In other embodiments, R² is 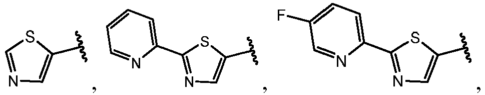 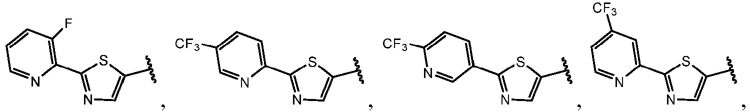 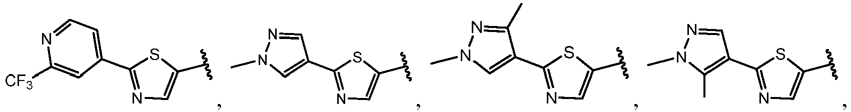 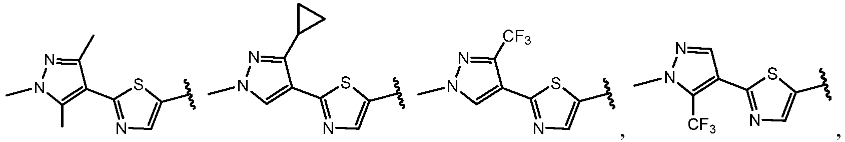 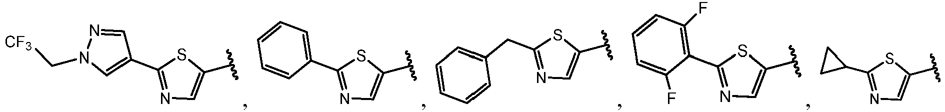 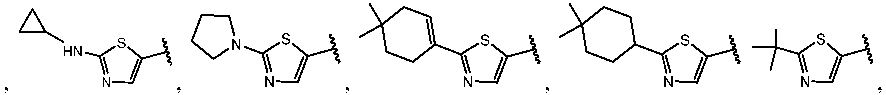 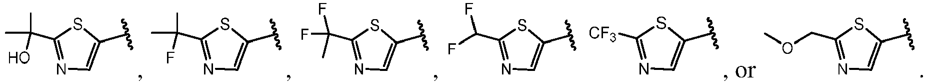 In yet other embodiments, R² is 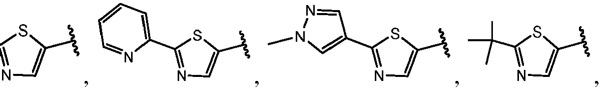 or

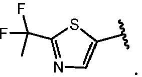

In still other embodiments, R² is 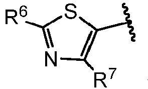 wherein R⁶ is defined herein and R⁷ is CF₃, cyclopropyl, or CH₃. In yet other embodiments, R² is

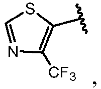

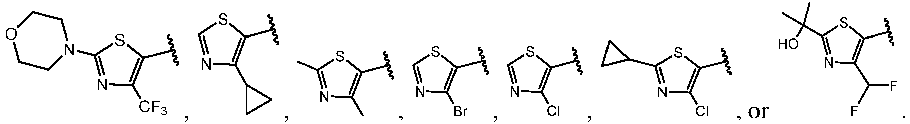

In yet further embodiments, R² is 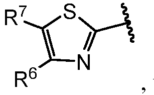 wherein R⁶ and R⁷ are defined herein. In other embodiments, R² is 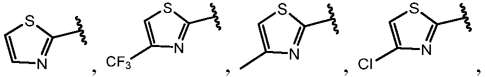 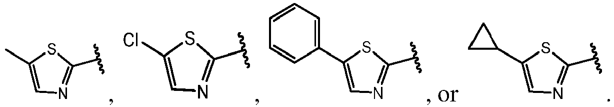 In yet other embodiments, R² is

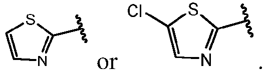

In other embodiments, R² is 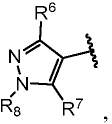 wherein R⁶-R⁸ are defined herein. In further embodiments, R² is 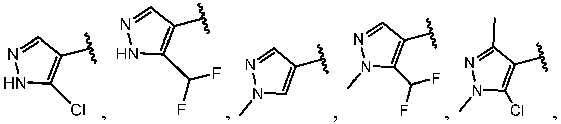 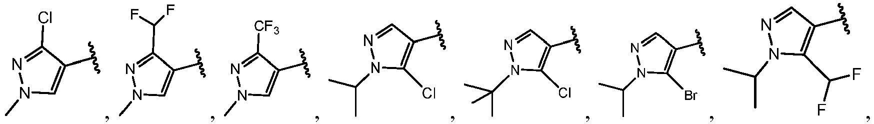 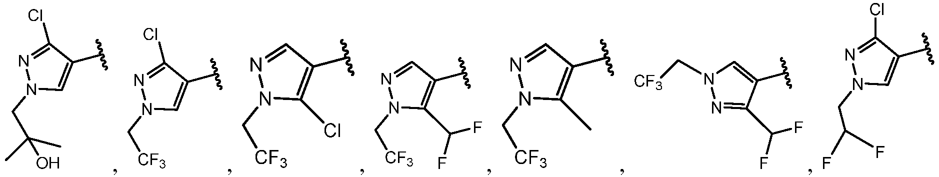 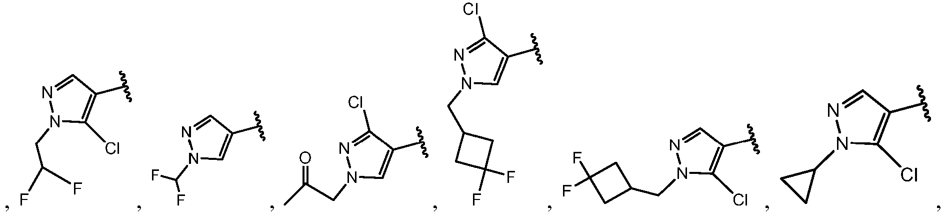 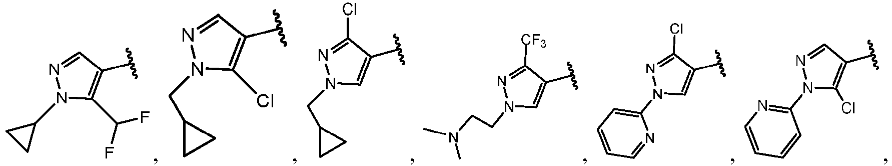 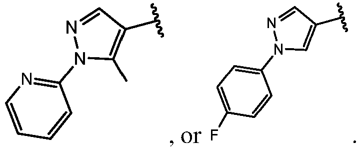 In still further embodiments, R² is

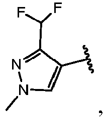

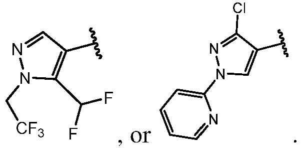

In further embodiments, R² is 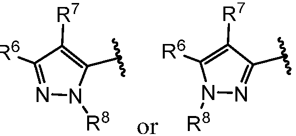 wherein R⁶-R⁸ are defined herein. In yet other embodiments, R² is 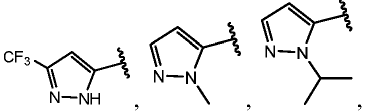 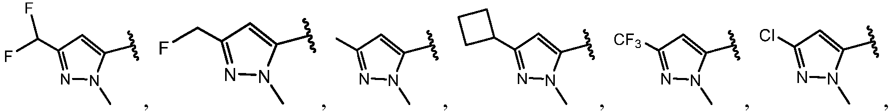 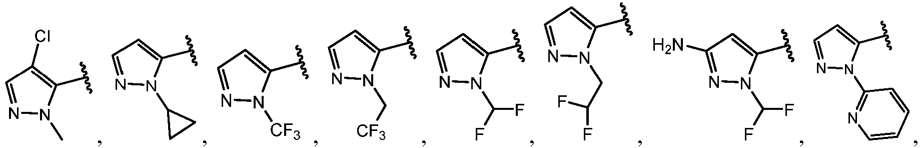 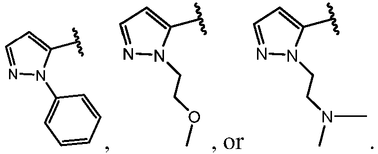 In further embodiments, R² is 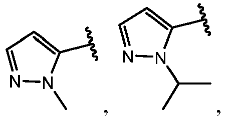 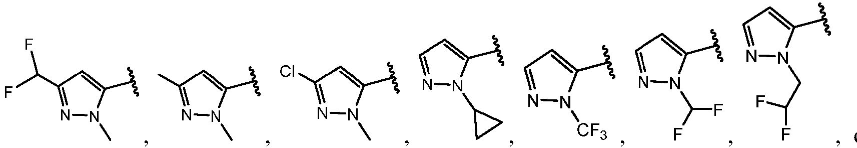 or

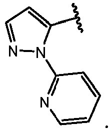

In further embodiments, R² is 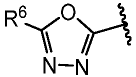 wherein R⁶ is optionally substituted heterocyclyl. In yet other embodiments, R² is

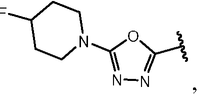

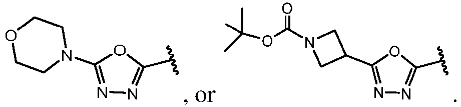

In further embodiments, R² is 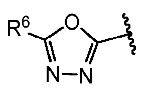 wherein R⁶ is optionally substituted heteroaryl. In yet other embodiments, R² is 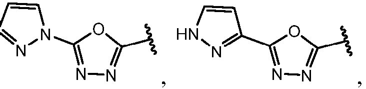 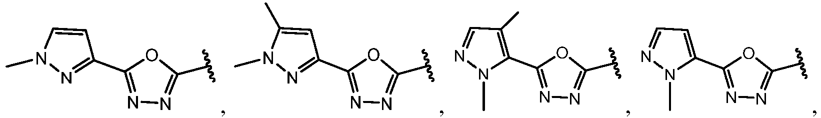 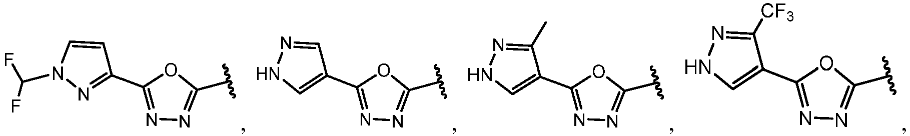 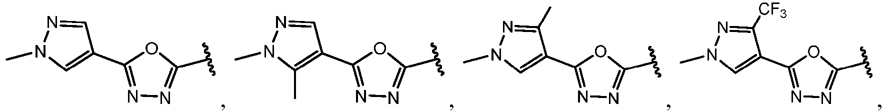 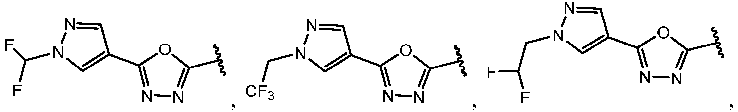 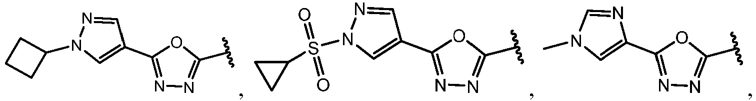 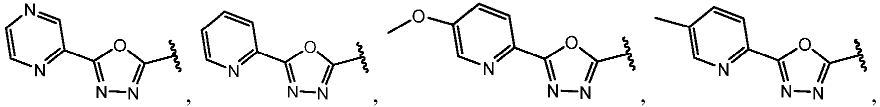 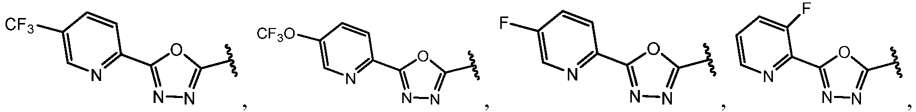 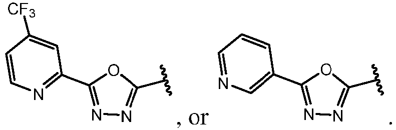 In further embodiments, R² is

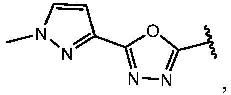

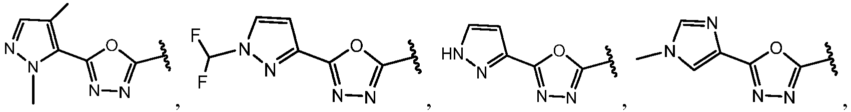

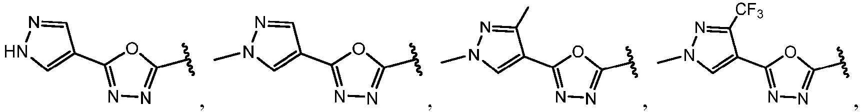

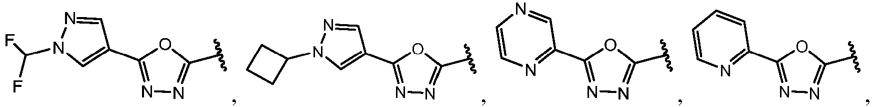

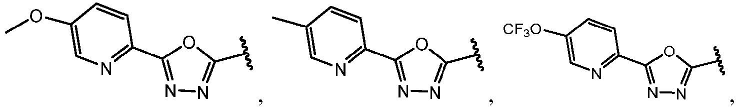

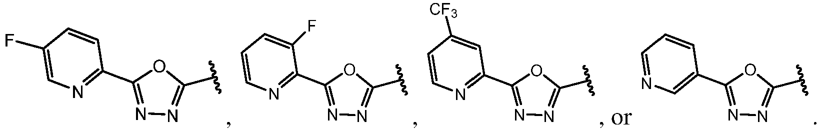

In further embodiments, R² is 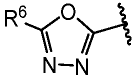 wherein R⁶ is optionally substituted aryl. In yet other embodiments, R² is 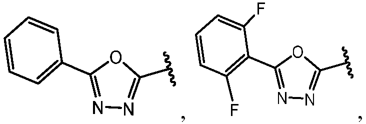 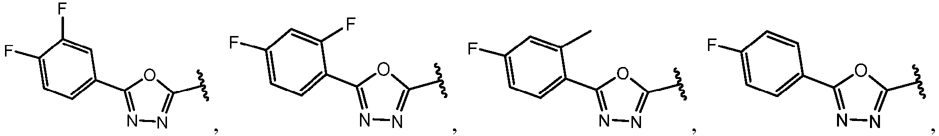 or

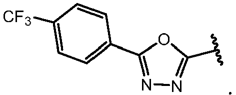

In further embodiments, R² is 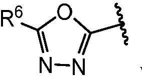 wherein R⁶ is C₁₋₆alkyl. In yet other embodiments, R² is 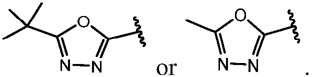 In still further embodiments, R² is

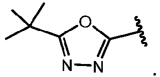

In further embodiments, R² is 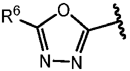 wherein R⁶ is C₁₋₆haloalkyl or C₁₋₆hydroxyalkyl. In yet other embodiments, R² is 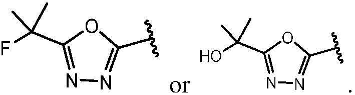 In still further embodiments, R² is

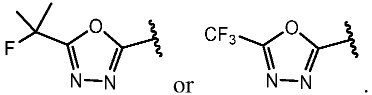

In further embodiments, R² is 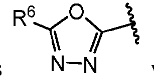 wherein R⁶ is optionally substituted C₃₋₈cycloalkyl. In yet other embodiments, R² is 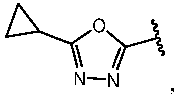 or 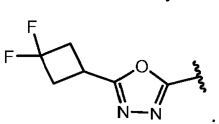 In still further embodiments, R² is

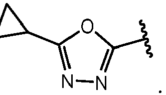

In further embodiments R² is 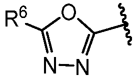 wherein R⁶ is (CR^{v}R^{x})ₚNR^{y}R^{z}, and p, R^{v}, R^{x}, R^{y}, and R^{z} are defined herein. In yet other embodiments, R² is 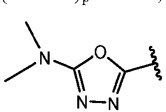 or

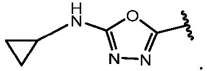

In other embodiments, R² is 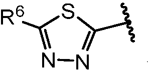 wherein R⁶ is H, *i.e.,*

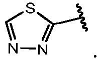

In further embodiments, R² is 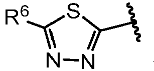 wherein R⁶ is C₁₋₆alkyl. In yet other embodiments, R² is

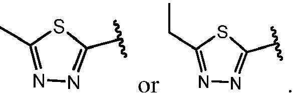

In further embodiments, R² is 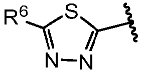 wherein R⁶ is halo. In yet other embodiments, R² is

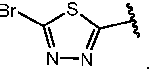

In further embodiments, R² is 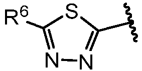 wherein R⁶ is aryl. In yet other embodiments, R² is

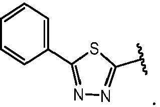

In further embodiments, R² is 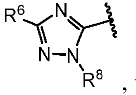 wherein R⁸ is C₁₋₆alkyl and R⁶ is defined herein. In yet other embodiments, R² is 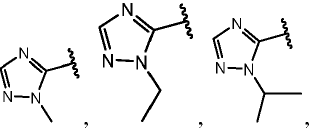 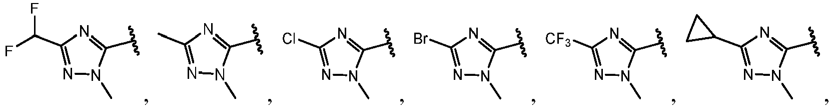 or 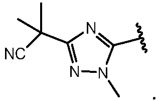 In still further embodiments, R² is

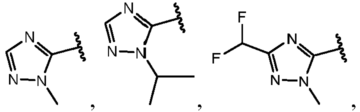

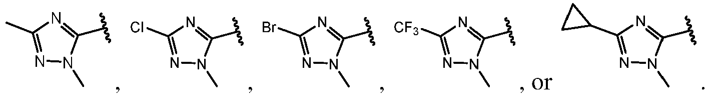

In further embodiments, R² is 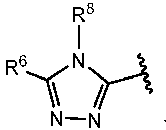 wherein R⁶ and R⁸ are defined herein. In yet other embodiments, R² is

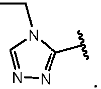

In further embodiments, R² is 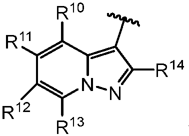 wherein R¹⁰-R¹⁴ are defined herein. In yet other embodiments, R² is 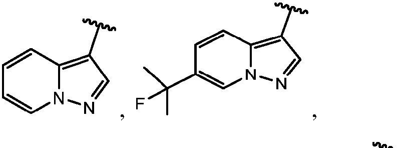 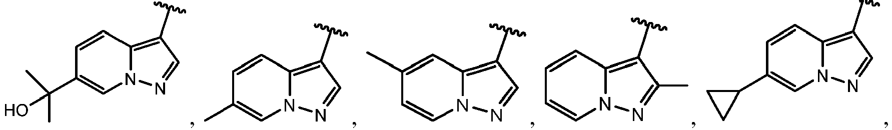 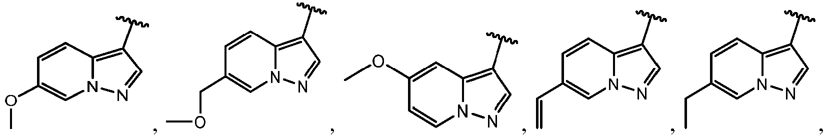 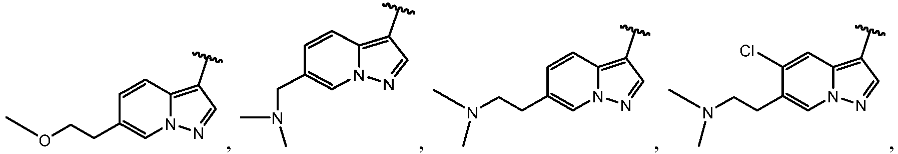 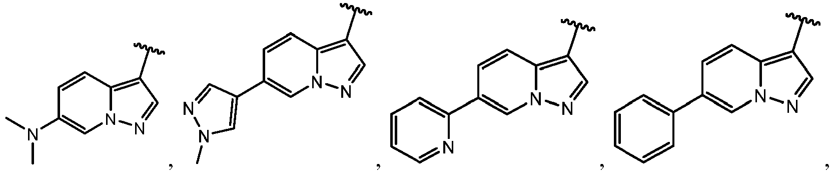 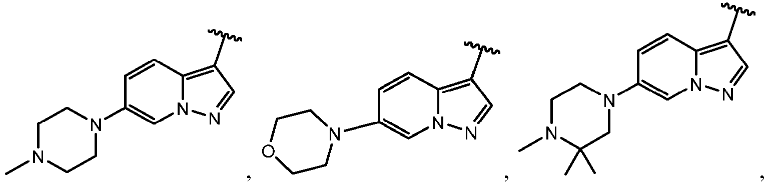 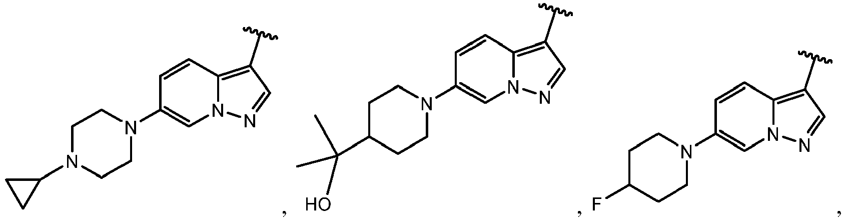 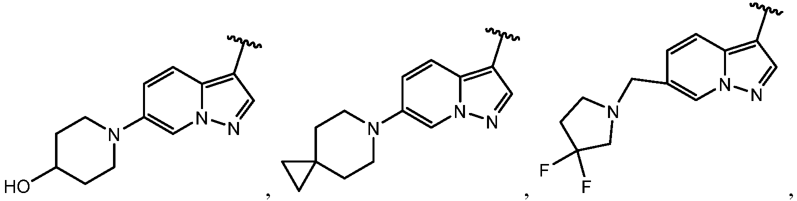 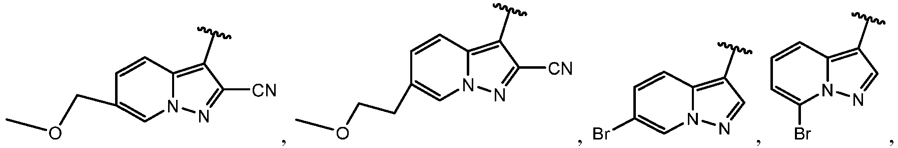 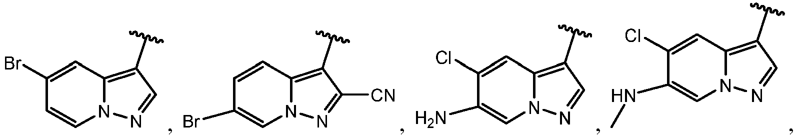 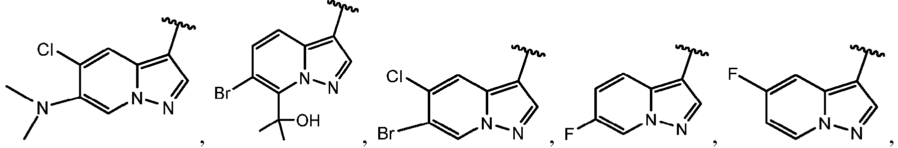 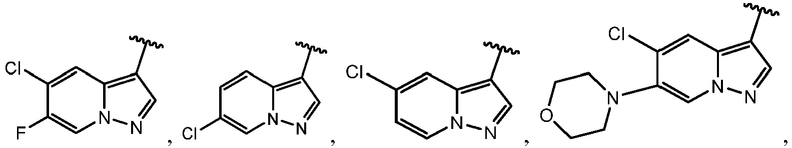 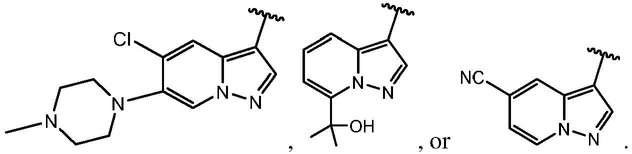 In still further embodiments, R² is

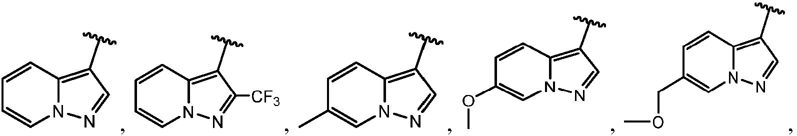

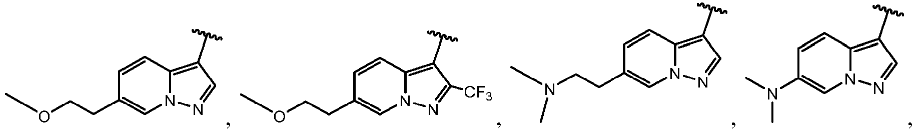

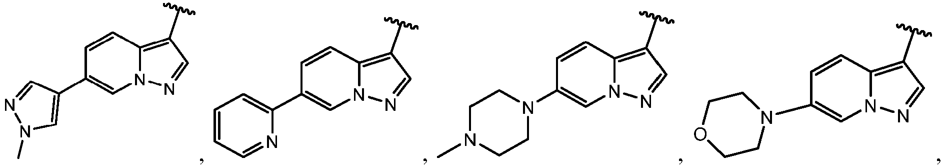

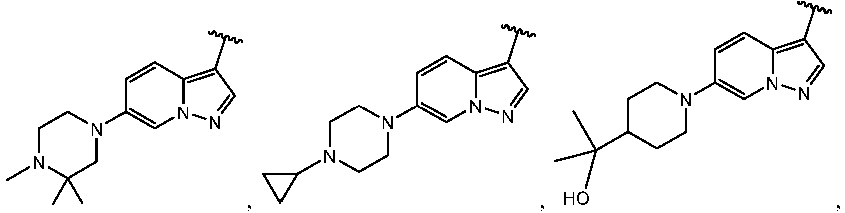

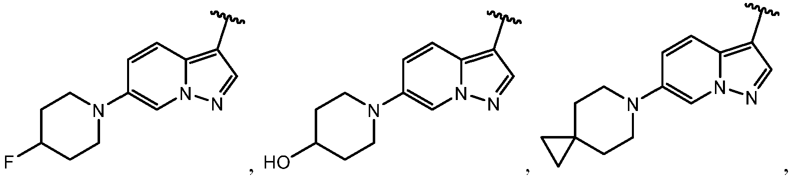

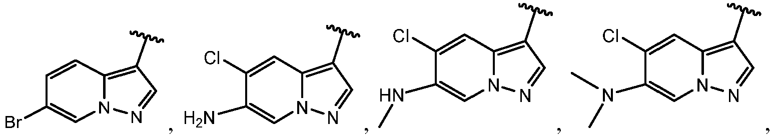

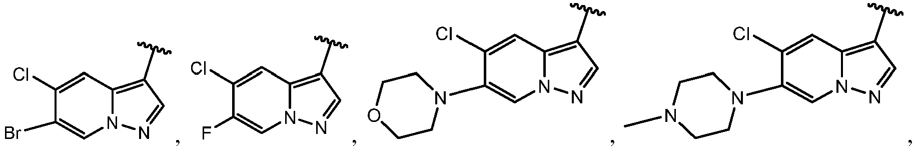

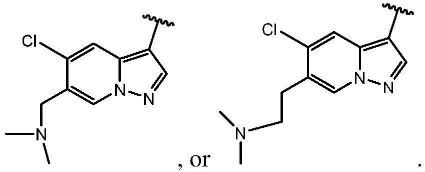

In further embodiments, R² is 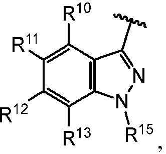 wherein R¹³ is H and R¹⁰, R¹¹, R¹², and R¹⁵ are defined herein. In yet other embodiments, R² is 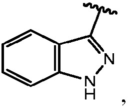 or

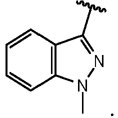

In further embodiments, R² is 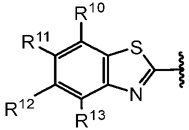 wherein R¹⁰-R¹³ are defined herein. In yet other embodiments, R² is 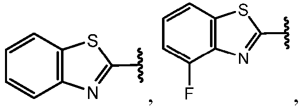 or

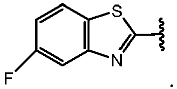

In some embodiments, the compound of Formula I is of Formula I-A or a pharmaceutically acceptable salt thereof: 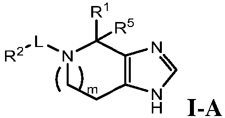 such as 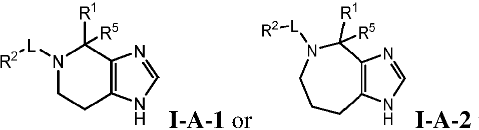 wherein R¹, R², R⁵, L, and m are defined herein. In other embodiments, the compound is 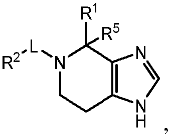 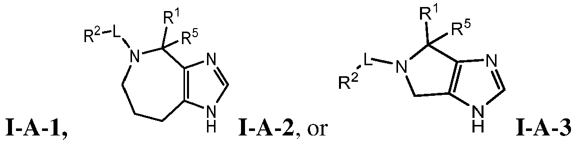 , or a pharmaceutically acceptable salt thereof. In some embodiments, the compound is 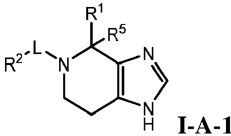 or a pharmaceutically acceptable salt thereof. In other embodiments, the compound is 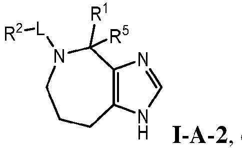 or a pharmaceutically acceptable salt thereof. In yet other embodiments, the compound is 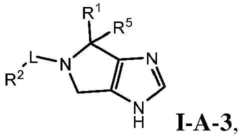 or a pharmaceutically acceptable salt thereof.

In other embodiments, the compound of Formula I is of Formula I-B or a pharmaceutically acceptable salt thereof: 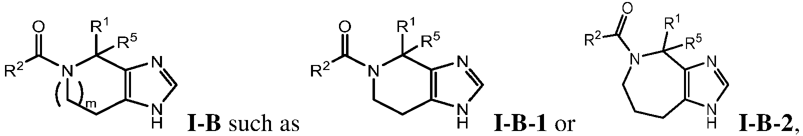 wherein R¹, R², R⁵, and m are defined herein. In other embodiments, the compound is 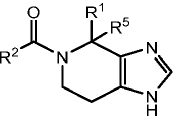 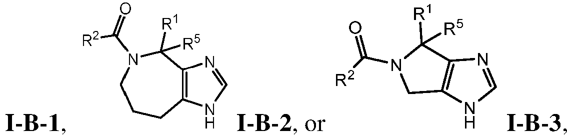 or a pharmaceutically acceptable salt thereof. In some embodiments, the compound is 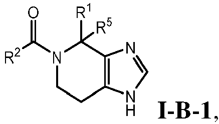 or a pharmaceutically acceptable salt thereof. In other embodiments, the compound is 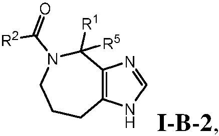 or a pharmaceutically acceptable salt thereof. In yet other embodiments, the compound is 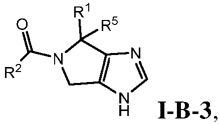 or a pharmaceutically acceptable salt thereof.

In further embodiments, the compound of Formula I is of Formula I-C or a pharmaceutically acceptable salt thereof: 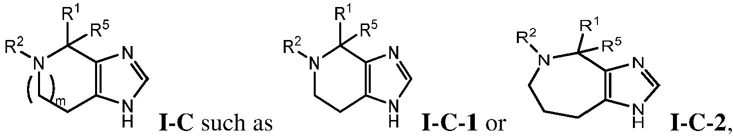 wherein R¹, R², R⁵, and m are defined herein. In other embodiments, the compound is 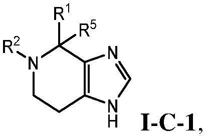 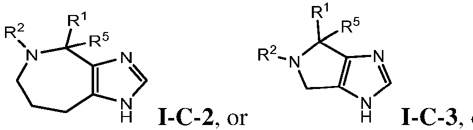 or a pharmaceutically acceptable salt thereof. In some embodiments, the compound is 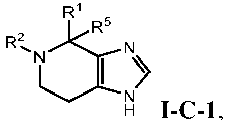 or a pharmaceutically acceptable salt thereof. In other embodiments, the compound is 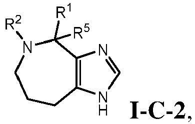 or a pharmaceutically acceptable salt thereof. In yet other embodiments, the compound is 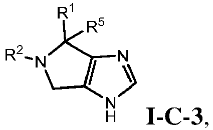 or a pharmaceutically acceptable salt thereof.

In still other embodiments, the compound of Formula I is of Formula I-D or a pharmaceutically acceptable salt thereof: 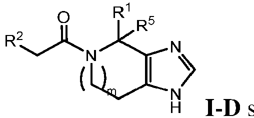 such as 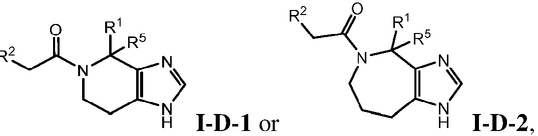 wherein R¹, R², R⁵, and m are defined herein. In other embodiments, the compound is 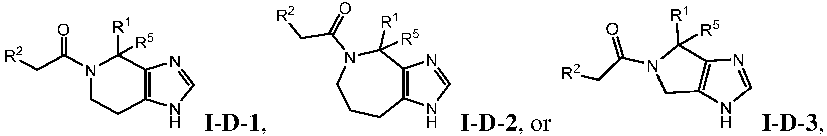 or a pharmaceutically acceptable salt thereof. In some embodiments, the compound is 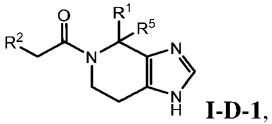 or a pharmaceutically acceptable salt thereof. In other embodiments, the compound is 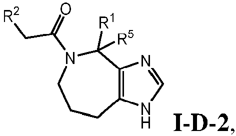 or a pharmaceutically acceptable salt thereof. In yet other embodiments, the compound is 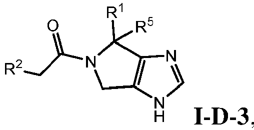 or a pharmaceutically acceptable salt thereof.

In still further embodiments, the compound of Formula I is of Formula I-E or a pharmaceutically acceptable salt thereof: 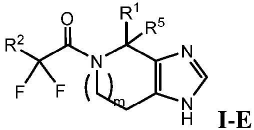 such as 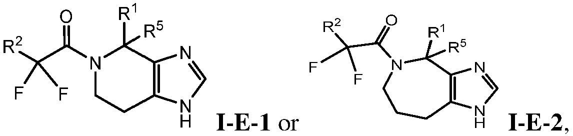 wherein R¹, R², R⁵, and m are defined herein. In other embodiments, the compound is 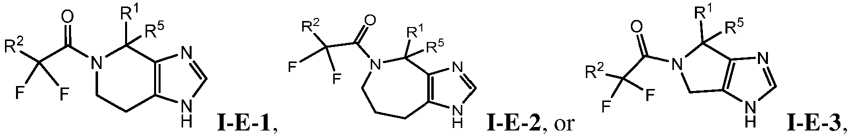 or a pharmaceutically acceptable salt thereof. In some embodiments, the compound is 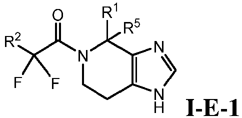 , or a pharmaceutically acceptable salt thereof. In other embodiments, the compound is 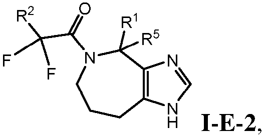 or a pharmaceutically acceptable salt thereof. In yet other embodiments, the compound is 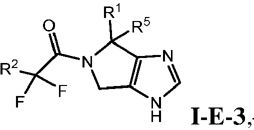 or a pharmaceutically acceptable salt thereof.

In further embodiments, the compound of Formula I is of Formula I-F or a pharmaceutically acceptable salt thereof: 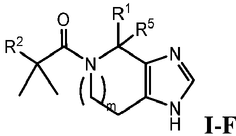 such as 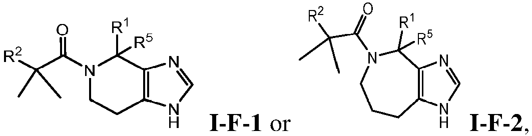 wherein R¹, R², R⁵, and m are defined herein. In other embodiments, the compound is 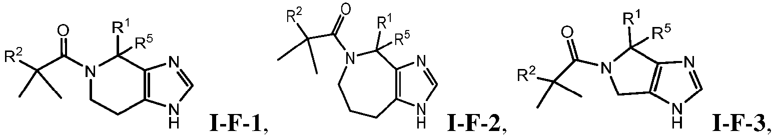 or a pharmaceutically acceptable salt thereof. In some embodiments, the compound is 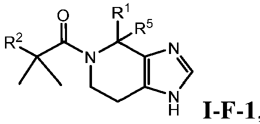 or a pharmaceutically acceptable salt thereof. In other embodiments, the compound is 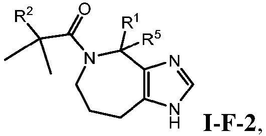 or a pharmaceutically acceptable salt thereof. In yet other embodiments, the compound is 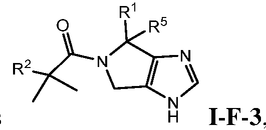 or a pharmaceutically acceptable salt thereof.

In further embodiments, the compound of Formula I is of Formula I-G or a pharmaceutically acceptable salt thereof: 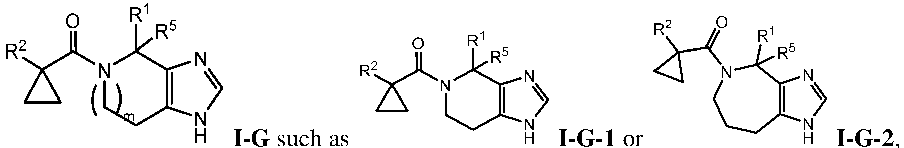 wherein R¹, R², R⁵, and m are defined herein. In other embodiments, the compound is 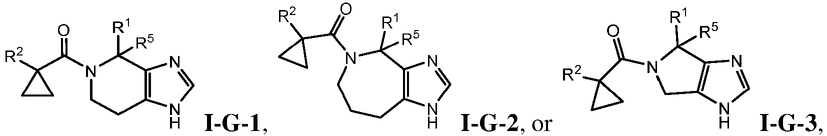 or a pharmaceutically acceptable salt thereof. In some embodiments, the compound is 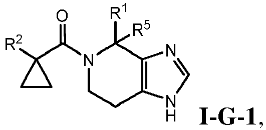 or a pharmaceutically acceptable salt thereof. In other embodiments, the compound is 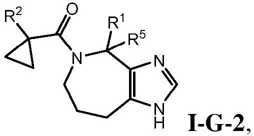 or a pharmaceutically acceptable salt thereof. In yet other embodiments, the compound is 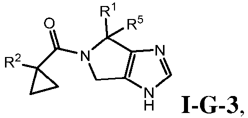 or a pharmaceutically acceptable salt thereof.

In yet other embodiments, the compound of Formula I is of Formula I-H or a pharmaceutically acceptable salt thereof: 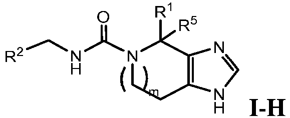 such as 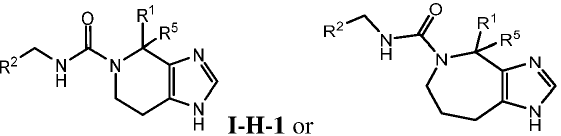 **I-H-2,** wherein R¹, R², R⁵, and m are defined herein. In other embodiments, the compound is 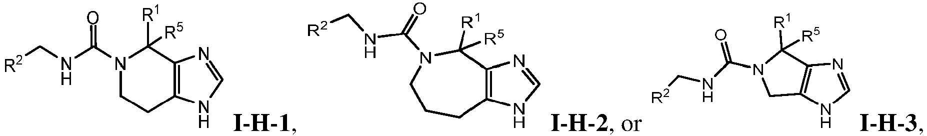 or a pharmaceutically acceptable salt thereof. In some embodiments, the compound is 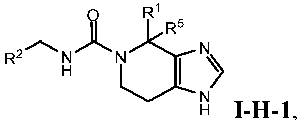 or a pharmaceutically acceptable salt thereof. In other embodiments, the compound is 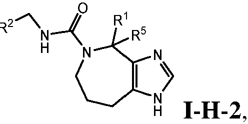 or a pharmaceutically acceptable salt thereof. In yet other embodiments, the compound is 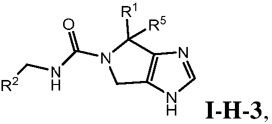 or a pharmaceutically acceptable salt thereof.

In still further embodiments, the compound of Formula I is of Formula I-I or a pharmaceutically acceptable salt thereof: 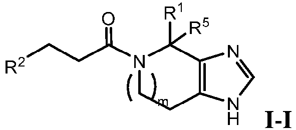 such as 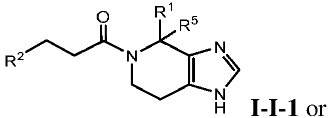 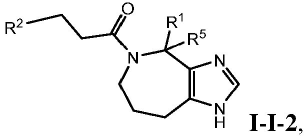 wherein R¹, R², R⁵, and m are defined herein. In other embodiments, the compound is 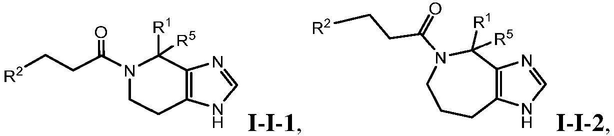 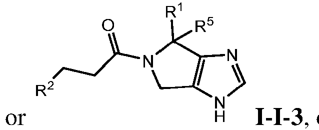 or a pharmaceutically acceptable salt thereof. In some embodiments, the compound is 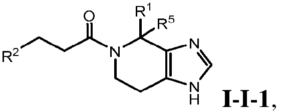 or a pharmaceutically acceptable salt thereof. In other embodiments, the compound is 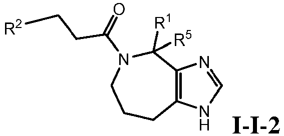 or a pharmaceutically acceptable salt thereof. In yet other embodiments, the compound is 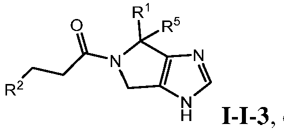 or a pharmaceutically acceptable salt thereof.

In further embodiments, the compound of Formula I is of Formula I-J or a pharmaceutically acceptable salt thereof: 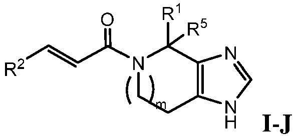 such as 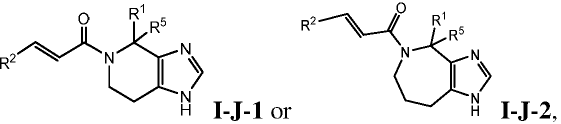 wherein R¹, R², R⁵, and m are defined herein. In other embodiments, the compound is 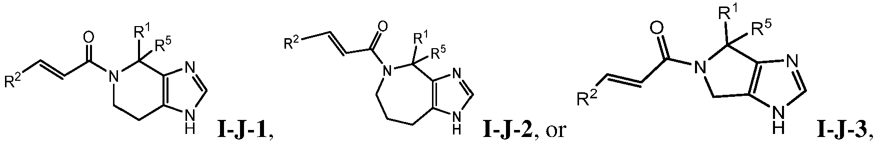 or a pharmaceutically acceptable thereof. In some embodiments, the compound is 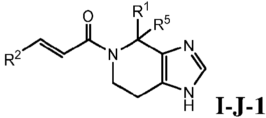 , or a pharmaceutically acceptable salt thereof. In other embodiments, the compound is 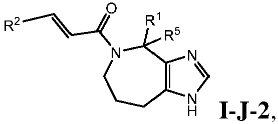 or a pharmaceutically acceptable salt thereof. In yet other embodiments, the compound is 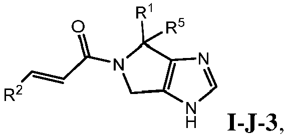 or a pharmaceutically acceptable salt thereof.

In further embodiments, the compound of Formula I is of Formula I-K or a pharmaceutically acceptable salt thereof: 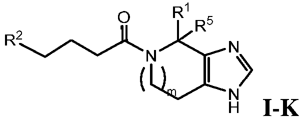 such as 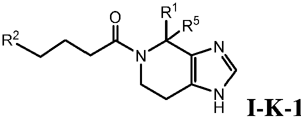 or 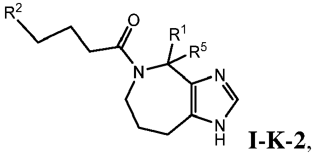 wherein R¹, R², R⁵, and m are defined herein. In other embodiments, the compound is 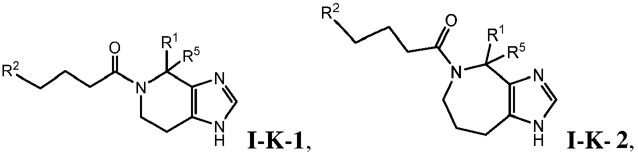 or 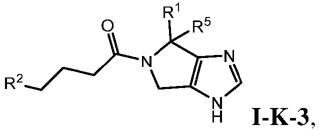 or a pharmaceutically acceptable salt thereof. In some embodiments, the compound is 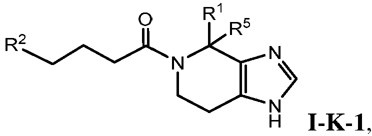 or a pharmaceutically acceptable salt thereof. In other embodiments, the compound is 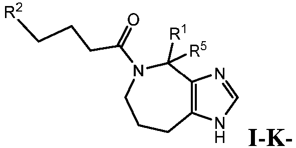 **2,** or a pharmaceutically acceptable salt thereof. In yet other embodiments, the compound is 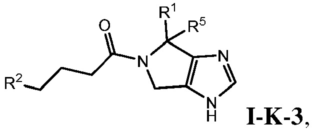 or a pharmaceutically acceptable salt thereof.

In still other embodiments, the compound of Formula **I** is of Formula **I-L** or a pharmaceutically acceptable salt thereof: 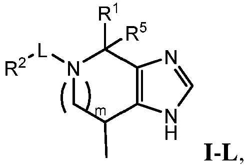 wherein R¹, R², R⁵, L, and m are defined herein. In further embodiments, the compound is 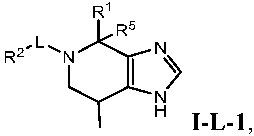 or a pharmaceutically acceptable salt thereof.

In yet further embodiments, the compound of Formula **I** is of Formula **I-M** or a pharmaceutically acceptable salt thereof: 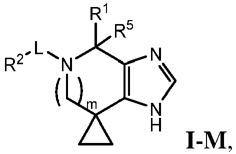 wherein R¹, R², R⁵, L, and m are defined herein. In other embodiments, the compound is 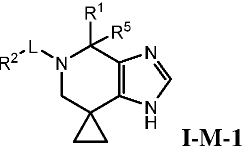 or a pharmaceutically acceptable salt thereof.

In some embodiments, compounds of the disclosure are any one or more of the compounds of Tables 1S, 1R, and 2, and their pharmaceutically acceptable salts and/or isotopologues. Compounds having the Formula **I** are further disclosed in the Exemplification and are included in the present disclosure. Pharmaceutically acceptable salts thereof as well as the neutral forms are included.

The disclosure further provides R-enantiomers, S-enantiomers, or racemic mixtures of any of the compounds described herein. In some embodiments, the compound is an S-enantiomer. In other embodiments, the compound is the R-enantiomer. In further embodiments, the compound is racemic. In some embodiments, the compound is a single enantiomer, and the R¹ moiety is in an alpha (α) configuration. In other embodiments, the compound is a single enantiomer, and the R¹ moiety is in an beta (β) configuration.

In another embodiment, the compounds of the disclosure may be enantiomerically enriched, *e.g.,* the enantiomeric excess or "ee" of the compound is greater than about 5% as measured by chiral HPLC. In some embodiments, the ee is greater than about 10%. In other embodiments, the ee is greater than about 20%. In further embodiments, the ee is greater than about 30%. In yet other embodiments, the ee is greater than about 40%. In still further embodiments, the ee is greater than about 50%. In other embodiments, the ee is greater than about 60%. In further embodiments, the ee is greater than about 70%. In still other embodiments, the ee is greater than about 80%. In yet further embodiments, the ee is greater than about 85%. In other embodiments, the ee is greater than about 90%. In further embodiments, the ee is greater than about 91%. In yet other embodiments, the ee is greater than about 92%. In still further embodiments, the ee is greater than about 93%. In other embodiments, the ee is greater than about 94%. In further embodiments, the ee is greater than about 95%. In still other embodiments, the ee is greater than about 96%. In yet further embodiments, the ee is greater than about 97%. In other embodiments, the ee is greater than about 98%. In further embodiments, the ee is greater than about 99%.

The present disclosure encompasses the preparation and use of salts of compounds of the disclosure. Salts of compounds of the disclosure can be prepared during the final isolation and purification of the compounds or separately by reacting the compound with an acid or base as appropriate.

### Treatment Methods

Compounds of the disclosure have several uses as described herein. In some embodiments, compounds of the disclosure are useful in methods for stabilizing mutant PAH proteins. These methods comprise contacting the protein with one or more compounds described herein or a pharmaceutically acceptable salt thereof. The compounds of the disclosure can provide for better Phe control for patients whose disease is not well-managed on diet alone and lessen the severity of a patient's phenylketonuria. Thus, patients administered a compound of the disclosure will have a better quality of life, *e.g.,* a more normal lifestyle and/or none or fewer dietary restrictions, as compared with phenylketonuria patients who have not been administered a compound of the disclosure. In some embodiments, patients administered a compound of the disclosure may experience increases in executive function, decreases in anxiety symptoms, and/or decreases in attention deficit hyperactivity disorder symptoms.

The term "mutant PAH gene" as used herein refers to the full DNA sequence of PAH that differs in one or more ways from the canonically accepted sequence ("the basis gene") that is published in any one of a variety of curated databases. As one example, the sequence described by GenBank Accession number NG_008690.2 describes the basis gene.

The term "mutant PAH protein" as used herein refers to a PAH protein that contains at least one mutation in the amino acid sequence relative to that encoded by the reference. The reference human PAH protein is described by Genbank Accession number NP_000268 and contains 452 amino acids. PAH protein mutations can be identified using methods known in the art. In some embodiments, the mutant PAH protein contains at least one R408W, R261Q, R243Q, Y414C, L48S, A403V, I65T, R241C, L348V, R408Q, or V388M, or L348V mutation. In yet other embodiments, the mutant PAH protein contains at least one R408W mutation. In further embodiments, the mutant PAH protein contains at least two R408W mutations. In further embodiments, the mutant PAH protein contains at least one R261Q mutation. In yet other embodiments, the mutant PAH protein contains at least one R243Q mutation. In yet other embodiments, the mutant PAH protein contains at least one Y414C mutation. In still further embodiments, the mutant PAH protein contains at least one L48S mutation. In other embodiments, the mutant PAH protein contains at least one A403V mutation. In further embodiments, the mutant PAH protein contains at least one I65T mutation. In yet further embodiments, the mutant PAH protein contains at least one R241C mutation. In yet other embodiments, the mutant PAH protein contains at least one L348V mutation. In further embodiments, the mutant PAH protein contains at least one R408Q mutation. In other embodiments, the mutant PAH protein contains at least one V388M mutation. In other embodiments, the mutant PAH protein contains at least one F39L mutation. In still further embodiments, the mutant PAH protein contains at least one A300S mutation. In yet further embodiments, the mutant PAH protein contains at least one L48S mutation.

In other embodiments, the disclosure provides compounds, or pharmaceutically acceptable salts thereof, for use in methods for stabilizing the activity of mutant phenylalanine hydroxylase (PAH) proteins as compared to wild type PAH. Such methods include contacting phenylalanine hydroxylase with one or more compounds described herein, or a pharmaceutically acceptable salt thereof. The term "stabilizing" as used herein refers to modulating the activity or quantity of a PAH enzyme so that it catalyzes hydroxylation of the aromatic side-chain of phenylalanine at a rate that is more similar to the PAH catalysis rate of a control population having wild type PAH, *i.e.,* without a mutant PAH gene mutation, as compared to the baseline PAH catalysis rate. In some aspects, the term "stabilizing" refers to modulating the activity of a subject's PAH so that it catalyzes hydroxylation of the aromatic side-chain of phenylalanine at a flux more similar to the PAH catalytic flux of a control subject population without a mutant PAH gene mutation. In some embodiments, "stabilizing" activity of PAH includes increasing levels of the enzyme PAH as compared to baseline. By increasing the buildup of stabilized active PAH protein, a subject's toxic Phe levels can be reduced as compared to the subject's baseline levels of dietary Phe prior to administration of a compound of the disclosure or a pharmaceutical composition comprising compounds of the disclosure.

In some embodiments, the disclosure provides compounds, or pharmaceutically acceptable salts thereof, for use in methods for reducing blood phenylalanine concentrations in a subject suffering from phenylketonuria to a concentration less than or equal to about 600µM. In other embodiments, the blood Phe concentration is reduced to a concentration less than or equal to about 360 µM. In other embodiments, the disclosure provides methods for reducing blood Phe concentrations as compared to untreated baseline. In some embodiments, a subject's blood Phe concentration as compared to untreated baseline is reduced by a percentage including but not limited at least about 10%, at least about 20%, at least about 30%, at least about 40%, at least about 50%, at least about 60%, at least about 70%, at least about 80%, or at least about 90%. In other embodiments, a subject's blood Phe concentration as compared to untreated baseline is reduced by at least about 10%. In further embodiments, a subject's blood Phe concentration as compared to untreated baseline is reduced by at least about 20%. In yet other embodiments, a subject's blood Phe concentration as compared to untreated baseline is reduced by at least about 30%. In still further embodiments, a subject's blood Phe concentration as compared to untreated baseline is reduced by at least about 40%. In other embodiments, a subject's blood Phe concentration as compared to untreated baseline is reduced by at least about 50%. In further embodiments, a subject's blood Phe concentration as compared to untreated baseline is reduced by at least about 60%. In yet other embodiments, a subject's blood Phe concentration as compared to untreated baseline is reduced by at least about 70%. In still further embodiments, a subject's blood Phe concentration as compared to untreated baseline is reduced by at least about 80%. In other embodiments, a subject's blood Phe concentration as compared to untreated baseline is reduced by at least about 90%. A subject's Phe concentration can be determined by blood tests and methods for measuring such levels are known in the art. In some embodiments, the reduction in Phe concentration achieved using compounds of the disclosure is obtained in conjunction with the subject actively managing their dietary Phe intake. In other embodiments, the reduction in Phe concentration is obtained in conjunction with the subject maintaining a Phe-restricted diet.

In some embodiments, a subject is treated with compounds of the disclosure, or a pharmaceutical composition comprising compounds of the disclosure. The compound is administered in an amount sufficient for stabilizing the PAH protein, or for reducing blood phenylalanine concentration in a subject, or combinations thereof in the subject.

In further embodiments, the subject is a human patient, such as is a human adult over 18 years old in need of treatment. In yet further embodiments, the human patient is a human child less than 18 years old. In yet other embodiments, the human patient is a human child less than 12 years old. In still further embodiments, the human patient is a human child between 12 years and 18 years old. In any of the embodiments, the subject has phenylketonuria (PKU), optionally classic PKU or severe PKU. In some embodiments, the subject has a blood Phe concentration greater than about 600 µM prior to administration of a compound of the disclosure or a pharmaceutical composition comprising compounds of the disclosure. In other embodiments, the subject's blood Phe concentration prior to administration is greater than about 700 µM. In further embodiments, the subject's blood Phe concentration prior to administration is greater than about 800 µM. In still further embodiments, the subject's blood Phe concentration prior to administration is greater than about 900 µM. In yet other embodiments, the subject's blood Phe concentration prior to administration is greater than about 1000 µM. In further embodiments, the subject's blood Phe concentration prior to administration is greater than about 1100 µM. In other embodiments, the subject's blood Phe concentration prior to administration is greater than about 1200 µM.

The present methods also encompass administering an additional therapeutic agent to the subject in addition to the compounds of the disclosure. In some embodiments, the additional therapeutic agent is selected from drugs known as useful in a stabilizing mutant PAH protein and/or reducing blood Phe concentrations. The additional therapeutic agent is different from the compounds of the disclosure. In some embodiments, the additional therapeutic agent is sapropterin or sepiapterin. In other embodiments, the additional therapeutic agent is a nutritional supplement. Nutritional supplements that may be used include those that contain amino acids and other nutrients. In further embodiments, the nutritional supplement contains large neutral amino acids such as leucine, tyrosine, tryptophan, methionine, histidine, isoleucine, valine, threonine. In other embodiments, the nutritional supplement contains tyrosine. In further embodiments, the nutritional supplement contains casein glycomacropeptide, *i.e.,* a milk peptide naturally free of Phe in its pure form. In other embodiments, the additional therapeutic agent is an enzyme substrate or enzyme co-factor. In yet other embodiments, the enzyme substrate or co-factor is tetrahydrobiopterin. In other embodiments, the additional therapeutic agent is a biopterin analogue. In further embodiments, the additional therapeutic agent is a biotherapeutic, synthetic biotic, microbiota or probiotic. In yet other embodiments, the biotherapeutic, synthetic biotic, microbiota or probiotic contains a genetically modified phenylalanine ammonia lyase (PAL) gene, such as, for example, E. coli Nissle PAL. Examples of genetically modified E. coli Nissle PAL biotherapetuics include SYNB1934 and SYNB 1618, and the like. In still further embodiments, the additional therapeutic agent is an inhibitor of an amino acid transporter. In some embodiments, the amino acid transporter is B⁰AT1 (also referred to as SLC6A19), and the additional therapeutic agent is a SLC6A19 inhibitor. Examples of SLC6A19 inhibitors include nimesulide, benztropine, NSC63912, NSC22789, cinromide, CB3, E62, JNT-517, and the like.

Compounds of the disclosure and the additional therapeutic agents can be administered simultaneously or sequentially to achieve the desired effect. In addition, the compounds of the disclosure and additional therapeutic agent can be administered in a single composition or two separate compositions.

The additional therapeutic agent is administered in an amount to provide its desired therapeutic effect. The effective dosage range for each additional therapeutic agent is known in the art, and the additional therapeutic agent is administered to an individual in need thereof within such established ranges.

Compounds of the disclosure and the additional therapeutic agents can be administered together as a single-unit dose or separately as multi-unit doses, wherein the compounds of the disclosure are administered before the additional therapeutic agent or vice versa. One or more doses of the compounds of the disclosure and/or one or more dose of the additional therapeutic agents can be administered.

The compounds of the disclosure may also be administered sequentially or concurrently with non-pharmacological techniques. In some embodiments, the patient uses non-pharmacological techniques to maintain lower Phe levels. In other embodiments, the non-pharmacological technique is administering a diet that is low in Phe. One skilled in the art would be able to determine what type of diet to maintain appropriate levels of Phe. In some embodiments, a phenylamine diet containing about 200 to about 500 mg/day (patients 10 years or younger) of Phe or less than about 600 mg/day (patients over 10 years of age). In other embodiments, the diet may include restricting or eliminating one or more foods that are high in Phe, such as soybeans, egg whites, shrimp, chicken breast, spirulina, watercress, fish, nuts, crayfish, lobster, tuna, turkey, legumes, and low-fat cottage cheese.

An example of a dose is in the range of from about 0.001 to about 100 mg of compound per kg of subject's body weight per day, in single or divided dosage units (e.g., BID, TID, QID). For a 70-kg human, a suitable dosage amount is from about 0.05 to about 7 g/day.

In some embodiments, the therapeutically effective amount of one or more compounds described herein is an amount that is effective in stabilizing a mutant PAH protein described herein. In other embodiments, the therapeutically effective amount of one or more compounds described herein is an amount that is effective in reducing blood phenylalanine concentrations.

Unless otherwise noted, the amounts of the compounds described herein are set forth on a free base basis. That is, the amounts indicate that amount of the compound administered, exclusive of, for example, solvent or counterions (such as in pharmaceutically acceptable salts).

### Pharmaceutical Compositions

The disclosure also provides pharmaceutical compositions comprising compounds of the disclosure and a pharmaceutically acceptable carrier or excipient.

The methods of the present disclosure can be accomplished by administering compounds of the disclosure as the neat compound or as a pharmaceutical composition. Administration of a pharmaceutical composition, or neat compound of the disclosure, can be performed at any time period as determined by the attending physician. Typically, the pharmaceutical compositions contain no toxic, carcinogenic, or mutagenic compounds that would cause an adverse reaction when administered.

Pharmaceutical compositions include those wherein compounds of the disclosure are administered in an effective amount to achieve its intended purpose. The exact formulation, route of administration, and dosage is determined by an individual physician.

Compounds of the disclosure can be administered by any suitable route, *e.g.,* by oral, buccal, inhalation, sublingual, rectal, vaginal, intracisternal or intrathecal through lumbar puncture, transurethral, nasal, percutaneous, *i.e.,* transdermal, or parenteral (including intravenous, intramuscular, subcutaneous, intracoronary, intradermal, intramammary, intraperitoneal, intraarticular, intrathecal, retrobulbar, intrapulmonary injection and/or surgical implantation at a particular site) administration. Parenteral administration can be accomplished using a needle and syringe or using a high-pressure technique.

The above-mentioned additional therapeutically active agents, one or more of which can be used in combination with compounds of the disclosure, are prepared and administered as described in the art.

Compounds of the disclosure may be administered in admixture with pharmaceutical carriers selected with regard to the intended route of administration and standard pharmaceutical practice. Pharmaceutical compositions for use in accordance with the present disclosure are formulated in a conventional manner using one or more physiologically acceptable carriers comprising excipients and/or auxiliaries that facilitate processing of compounds of the disclosure.

Administration of the compounds or pharmaceutical compositions of the disclosure can be effected by any method that enables delivery of the compounds to the site of action. These methods include oral routes, intraduodenal routes, parenteral injection (including intravenous, intraarterial, subcutaneous, intramuscular, intravascular, intraperitoneal or infusion), topical (e.g. transdermal application), rectal administration, via local delivery by catheter or stent or through inhalation. Compounds can also be administered intraadiposally or intrathecally.

The amount of the compound administered will be dependent on the subject being treated, the severity of the disorder or condition, the rate of administration, the disposition of the compound and the discretion of the prescribing physician. The desired dose can be administered in a single dose, or as multiple doses administered at appropriate intervals, e.g., as one, two, three, four or more subdoses per day. In some embodiments, the compounds disclosed herein are effective over a wide dosage range. For example, in the treatment of adult humans, dosage forms containing from about 0.01 to 2000 mg of a compound disclosed herein per day are examples of dosage forms that may be used. The exact dosage will depend upon the route of administration, the form in which the compound is administered, the subject to be treated, the body weight of the subject to be treated, and the preference and experience of the attending physician. In some instances, dosage levels below the lower limit of the aforesaid range may be more than adequate, while in other cases still larger doses may be employed without causing any harmful side effect, e.g. by dividing such larger doses into several small doses for administration throughout the day.

In some embodiments, a compound of the disclosure is administered in a single dose.

Typically, such administration will be by a solid oral dosage form such as tablet or capsule. However, other routes may be used as appropriate. A single dose of a compound may also be used for treatment of an acute condition.

In some embodiments, a compound of the disclosure may be administered in multiple doses. Dosing may be about once, twice, three times, four times, five times, six times, or more than six times per day. In another embodiment, a compound described herein and another therapeutic agent are administered together about once per day to about 6 times per day. Administration of the compounds disclosed herein may continue as long as necessary. In some embodiments, a compound is administered chronically on an ongoing basis, e.g., for the treatment of chronic effects.

An effective amount of a compound of the disclosure may be administered in either single or multiple doses by any of the accepted modes of administration of therapeutic agents having similar utilities, including rectal, buccal, intranasal and transdermal routes, by intra-arterial injection, intravenously, intraperitoneally, parenterally, intramuscularly, subcutaneously, orally, topically, or as an inhalant.

The pharmaceutical composition may, for example, be in a form suitable for oral administration as a tablet, capsule, pill, powder, sustained release formulations, solution, suspension, for parenteral injection as a sterile solution, suspension or emulsion, for topical administration as an ointment or cream or for rectal administration as a suppository. The pharmaceutical composition may be in unit dosage forms suitable for single administration of precise dosages. The pharmaceutical composition will include one or more conventional pharmaceutical carriers or excipients and a compound disclosed herein as an active ingredient. In addition, it may include other medicinal or pharmaceutical agents, carriers, adjuvants, etc.

Exemplary parenteral administration forms include solutions or suspensions of the compound of the disclosure in sterile aqueous solutions, for example, aqueous propylene glycol or dextrose solutions. Such dosage forms can be suitably buffered, if desired.

### Pharmaceutical compositions for oral administration

In some embodiments, the disclosure provides a pharmaceutical composition for oral administration containing a compound of the disclosure and pharmaceutical excipients suitable for oral administration.

In some embodiments, the disclosure provides a solid pharmaceutical composition for oral administration containing: (i) an effective amount of a compound of the disclosure; optionally (ii) an effective amount of a second therapeutic agent; and (iii) a pharmaceutical excipient suitable for oral administration. In some embodiments, the composition further contains: (iv) an effective amount of a third therapeutic agent.

In some embodiments, the pharmaceutical composition may be a pharmaceutical composition suitable for oral consumption. Pharmaceutical compositions containing a compound of the disclosure suitable for oral administration can be presented as discrete dosage forms, such as capsules, cachets, or tablets, or liquids or aerosol sprays each containing a predetermined amount of an active ingredient as a powder or in granules, a solution, or a suspension in an aqueous or non-aqueous liquid, an oil-in-water emulsion, or a water-in-oil liquid emulsion. Such dosage forms can be prepared by any of the methods of pharmacy, but all methods include the step of bringing the compound of the disclosure into association with the carrier, which constitutes one or more necessary ingredients. In general, the compositions are prepared by uniformly and intimately admixing the compound of the disclosure with liquid carriers or finely divided solid carriers or both, and then, if necessary, shaping the product into the desired presentation. For example, a tablet can be prepared by compression or molding, optionally with one or more accessory ingredients. Compressed tablets can be prepared by compressing in a suitable machine the active ingredient in a free-flowing form such as powder or granules, optionally mixed with an excipient such as, but not limited to, a binder, a lubricant, an inert diluent, and/or a surface active or dispersing agent. Molded tablets can be made by molding in a suitable machine a mixture of the powdered compound moistened with an inert liquid diluent.

This disclosure further encompasses anhydrous pharmaceutical compositions and dosage forms comprising an active ingredient, since water can facilitate the degradation of some compounds. For example, water may be added (e.g., 5%) in the pharmaceutical arts as a means of simulating long-term storage in order to determine characteristics such as shelf-life or the stability of formulations over time. Anhydrous pharmaceutical compositions and dosage forms containing a compound of the disclosure can be prepared using anhydrous or low moisture containing ingredients and low moisture or low humidity conditions. Pharmaceutical compositions and dosage forms containing a compound of the disclosure which contain lactose can be made anhydrous if substantial contact with moisture and/or humidity during manufacturing, packaging, and/or storage is expected. An anhydrous pharmaceutical composition may be prepared and stored such that its anhydrous nature is maintained. Accordingly, anhydrous compositions may be packaged using materials known to prevent exposure to water such that they can be included in suitable formulary kits. Examples of suitable packaging include, but are not limited to, hermetically sealed foils, plastic or the like, unit dose containers, blister packs, and strip packs.

The compound of the disclosure can be combined in an intimate admixture with a pharmaceutical carrier according to conventional pharmaceutical compounding techniques. The carrier can take a wide variety of forms depending on the form of preparation desired for administration. In preparing the compositions for an oral dosage form, any of the usual pharmaceutical media can be employed as carriers, such as, for example, water, glycols, oils, alcohols, flavoring agents, preservatives, coloring agents, and the like in the case of oral liquid preparations (such as suspensions, solutions, and elixirs) or aerosols; or carriers such as starches, sugars, micro-crystalline cellulose, diluents, granulating agents, lubricants, binders, and disintegrating agents can be used in the case of oral solid preparations, in some embodiments without employing the use of lactose. For example, suitable carriers include powders, capsules, and tablets, with the solid oral preparations. If desired, tablets can be coated by standard aqueous or nonaqueous techniques.

Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, colloidal silicon dioxide, microcrystalline cellulose, and mixtures thereof.

Examples of suitable fillers for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof.

Disintegrants may be used in the compositions of the disclosure to provide tablets that disintegrate when exposed to an aqueous environment. Too much of a disintegrant may produce tablets which may disintegrate in the bottle. Too little may be insufficient for disintegration to occur and may thus alter the rate and extent of release of the active ingredient(s) from the dosage form. Thus, a sufficient amount of disintegrant that is neither too little nor too much to detrimentally alter the release of the active ingredient(s) may be used to form the dosage forms of the compounds disclosed herein. The amount of disintegrant used may vary based upon the type of formulation and mode of administration, and may be readily discernible to those of ordinary skill in the art. About 0.5 to about 15 weight percent of disintegrant, or about 1 to about 5 weight percent of disintegrant, may be used in the pharmaceutical composition. Disintegrants that can be used to form pharmaceutical compositions and dosage forms of the disclosure include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums or mixtures thereof.

Lubricants which can be used to form pharmaceutical compositions and dosage forms of the disclosure include, but are not limited to, calcium stearate, magnesium stearate, sodium stearyl fumarate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, or mixtures thereof. Additional lubricants include, for example, a syloid silica gel, a coagulated aerosol of synthetic silica, or mixtures thereof. A lubricant can optionally be added, in an amount of less than about 2 weight percent of the pharmaceutical composition.

When aqueous suspensions and/or elixirs are desired for oral administration, the active ingredient therein may be combined with various sweetening or flavoring agents, coloring matter or dyes and, if so desired, emulsifying and/or suspending agents, together with such diluents as water, ethanol, propylene glycol, glycerin and various combinations thereof.

The tablets can be uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Surfactants which can be used to form pharmaceutical compositions and dosage forms of the disclosure include, but are not limited to, hydrophilic surfactants, lipophilic surfactants, and mixtures thereof. That is, a mixture of hydrophilic surfactants may be employed, a mixture of lipophilic surfactants may be employed, or a mixture of at least one hydrophilic surfactant and at least one lipophilic surfactant may be employed.

A suitable hydrophilic surfactant may generally have an HLB value of at least 10, while suitable lipophilic surfactants may generally have an HLB value of or less than about 10. An empirical parameter used to characterize the relative hydrophilicity and hydrophobicity of non-ionic amphiphilic compounds is the hydrophilic-lipophilic balance (" HLB" value). Surfactants with lower HLB values are more lipophilic or hydrophobic, and have greater solubility in oils, while surfactants with higher HLB values are more hydrophilic, and have greater solubility in aqueous solutions.

Hydrophilic surfactants are generally considered to be those compounds having an HLB value greater than about 10, as well as anionic, cationic, or zwitterionic compounds for which the HLB scale is not generally applicable. Similarly, lipophilic (*i.e.,* hydrophobic) surfactants are compounds having an HLB value equal to or less than about 10. However, HLB value of a surfactant is merely a rough guide generally used to enable formulation of industrial, pharmaceutical, and cosmetic emulsions.

Hydrophilic surfactants may be either ionic or non-ionic. Suitable ionic surfactants include, but are not limited to, alkylammonium salts; fusidic acid salts; fatty acid derivatives of amino acids, oligopeptides, and polypeptides; glyceride derivatives of amino acids, oligopeptides, and polypeptides; lecithins and hydrogenated lecithins; lysolecithins and hydrogenated lysolecithins; phospholipids and derivatives thereof; lysophospholipids and derivatives thereof; carnitine fatty acid ester salts; salts of alkylsulfates; fatty acid salts; sodium docusate; acyl lactylates; mono- and di-acetylated tartaric acid esters of mono- and di-glycerides; succinylated mono- and di-glycerides; citric acid esters of mono- and di-glycerides; and mixtures thereof.

Within the aforementioned group, ionic surfactants include, by way of example: lecithins, lysolecithin, phospholipids, lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Ionic surfactants may be the ionized forms of lecithin, lysolecithin, phosphatidylcholine, phosphatidylethanolamine, phosphatidylglycerol, phosphatidic acid, phosphatidylserine, lysophosphatidylcholine, lysophosphatidylethanolamine, lysophosphatidylglycerol, lysophosphatidic acid, lysophosphatidylserine, PEG-phosphatidylethanolamine, PVP-phosphatidylethanolamine, lactylic esters of fatty acids, stearoyl-2-lactylate, stearoyl lactylate, succinylated monoglycerides, mono/diacetylated tartaric acid esters of mono/diglycerides, citric acid esters of mono/diglycerides, cholylsarcosine, caproate, caprylate, caprate, laurate, myristate, palmitate, oleate, ricinoleate, linoleate, linolenate, stearate, lauryl sulfate, teracecyl sulfate, docusate, lauroyl carnitines, palmitoyl carnitines, myristoyl carnitines, and salts and mixtures thereof.

Hydrophilic non-ionic surfactants may include, but are not limited to, alkylglucosides; alkylmaltosides; alkylthioglucosides; lauryl macrogolglycerides; polyoxyalkylene alkyl ethers such as polyethylene glycol alkyl ethers; polyoxyalkylene alkylphenols such as polyethylene glycol alkyl phenols; polyoxyalkylene alkyl phenol fatty acid esters such as polyethylene glycol fatty acids monoesters and polyethylene glycol fatty acids diesters; polyethylene glycol glycerol fatty acid esters; polyglycerol fatty acid esters; polyoxyalkylene sorbitan fatty acid esters such as polyethylene glycol sorbitan fatty acid esters; hydrophilic transesterification products of a polyol with at least one member of the group consisting of glycerides, vegetable oils, hydrogenated vegetable oils, fatty acids, and sterols; polyoxyethylene sterols, derivatives, and analogues thereof; polyoxyethylated vitamins and derivatives thereof; polyoxyethylene-polyoxypropylene block copolymers; and mixtures thereof; polyethylene glycol sorbitan fatty acid esters and hydrophilic transesterification products of a polyol with at least one member of the group consisting of triglycerides, vegetable oils, and hydrogenated vegetable oils. The polyol may be glycerol, ethylene glycol, polyethylene glycol, sorbitol, propylene glycol, pentaerythritol, or a saccharide.

Other hydrophilic-non-ionic surfactants include, without limitation, PEG-10 laurate, PEG-12 laurate, PEG-20 laurate, PEG-32 laurate, PEG-32 dilaurate, PEG-12 oleate, PEG-15 oleate, PEG-20 oleate, PEG-20 dioleate, PEG-32 oleate, PEG-200 oleate, PEG-400 oleate, PEG-15 stearate, PEG-32 distearate, PEG-40 stearate, PEG-100 stearate, PEG-20 dilaurate, PEG-25 glyceryl trioleate, PEG-32 dioleate, PEG-20 glyceryl laurate, PEG-30 glyceryl laurate, PEG-20 glyceryl stearate, PEG-20 glyceryl oleate, PEG-30 glyceryl oleate, PEG-30 glyceryl laurate, PEG-40 glyceryl laurate, PEG-40 palm kernel oil, PEG-50 hydrogenated castor oil, PEG-40 castor oil, PEG-35 castor oil, PEG-60 castor oil, PEG-40 hydrogenated castor oil, PEG-60 hydrogenated castor oil, PEG-60 corn oil, PEG-6 caprate/caprylate glycerides, PEG-8 caprate/caprylate glycerides, polyglyceryl-10 laurate, PEG-30 cholesterol, PEG-25 phyto sterol, PEG-30 soya sterol, PEG-20 trioleate, PEG-40 sorbitan oleate, PEG-80 sorbitan laurate, polysorbate 20, polysorbate 80, POE-9 lauryl ether, POE-23 lauryl ether, POE-10 oleyl ether, POE-20 oleyl ether, POE-20 stearyl ether, tocopheryl PEG-1000 succinate, PEG-24 cholesterol, polyglyceryl-10-oleate, Tween 40, Tween 60, sucrose monostearate, sucrose mono laurate, sucrose monopalmitate, PEG 10-100 nonyl phenol series, PEG 15-100 octyl phenol series, and poloxamers.

Suitable lipophilic surfactants include, by way of example only: fatty alcohols; glycerol fatty acid esters; acetylated glycerol fatty acid esters; lower alcohol fatty acids esters; propylene glycol fatty acid esters; sorbitan fatty acid esters; polyethylene glycol sorbitan fatty acid esters; sterols and sterol derivatives; polyoxyethylated sterols and sterol derivatives; polyethylene glycol alkyl ethers; sugar esters; sugar ethers; lactic acid derivatives of mono- and di-glycerides; hydrophobic transesterification products of a polyol with at least one member of the group consisting of glycerides, vegetable oils, hydrogenated vegetable oils, fatty acids and sterols; oil-soluble vitamins/vitamin derivatives; and mixtures thereof. Within this group, preferred lipophilic surfactants include glycerol fatty acid esters, propylene glycol fatty acid esters, and mixtures thereof, or are hydrophobic transesterification products of a polyol with at least one member of the group consisting of vegetable oils, hydrogenated vegetable oils, and triglycerides.

In one embodiment, the composition may include a solubilizer to ensure good solubilization and/or dissolution of the compound of the disclosure and to minimize precipitation of the compound of the disclosure. This can be important for compositions for non-oral use, e.g., compositions for injection. A solubilizer may also be added to increase the solubility of the hydrophilic drug and/or other components, such as surfactants, or to maintain the composition as a stable or homogeneous solution or dispersion.

Examples of suitable solubilizers include, but are not limited to, the following: alcohols and polyols, such as ethanol, isopropanol, butanol, benzyl alcohol, ethylene glycol, propylene glycol, butanediols and isomers thereof, glycerol, pentaerythritol, sorbitol, mannitol, transcutol, dimethyl isosorbide, polyethylene glycol (PEG), polypropylene glycol, polyvinylalcohol, hydroxypropyl methylcellulose and other cellulose derivatives, cyclodextrins and cyclodextrin derivatives; ethers of polyethylene glycols having an average molecular weight of about 200 to about 6000, such as tetrahydrofurfuryl alcohol PEG ether (glycofurol) or methoxy PEG; polyethylene glycol 660 12-hydroxystearate; amides and other nitrogen-containing compounds such as 2-pyrrolidone, 2-piperidone, ε-caprolactam, N-alkylpyrrolidone, N-hydroxyalkylpyrrolidone, N-alkylpiperidone, N-alkylcaprolactam, dimethylacetamide and polyvinylpyrrolidone; esters such as ethyl propionate, tributylcitrate, acetyl triethylcitrate, acetyl tributyl citrate, triethylcitrate, ethyl oleate, ethyl caprylate, ethyl butyrate, triacetin, propylene glycol monoacetate, propylene glycol diacetate, ε-caprolactone and isomers thereof, δ-valerolactone and isomers thereof, β-butyrolactone and isomers thereof; and other solubilizers known in the art, such as dimethyl acetamide, dimethyl isosorbide, N-methyl pyrrolidones, monooctanoin, diethylene glycol monoethyl ether, and water.

Mixtures of solubilizers may also be used. Examples include, but not limited to, triacetin, triethylcitrate, ethyl oleate, ethyl caprylate, dimethylacetamide, N-methylpyrrolidone, N-hydroxyethylpyrrolidone, polyvinylpyrrolidone, hydroxypropyl methylcellulose, hydroxypropyl cyclodextrins, ethanol, transcutol, propylene glycol, and dimethyl isosorbide. Particularly preferred solubilizers include sorbitol, glycerol, triacetin, ethyl alcohol, PEG having an average molecular weight of about 100 to about 8000 g/mole, glycofurol and propylene glycol.

The amount of solubilizer that can be included is not particularly limited. The amount of a given solubilizer may be limited to a bioacceptable amount, which may be readily determined by one of skill in the art. In some circumstances, it may be advantageous to include amounts of solubilizers far in excess of bioacceptable amounts, for example to maximize the concentration of the drug, with excess solubilizer removed prior to providing the composition to a subject using conventional techniques, such as distillation or evaporation. Thus, if present, the solubilizer can be in a weight ratio of less than about 10%, less than about 25%, less than about 50%, about 100%, or up to less than about 200% by weight, based on the combined weight of the drug, and other excipients. If desired, very small amounts of solubilizer may also be used, such as less than about 5%, less than about 2%, less than about 1% or even less. Typically, the solubilizer may be present in an amount of less than about 1% to about 100%, more typically less than about 5% to less than about 25% by weight.

The composition can further include one or more pharmaceutically acceptable additives and excipients. Such additives and excipients include, without limitation, detackifiers, anti-foaming agents, buffering agents, polymers, antioxidants, preservatives, chelating agents, viscomodulators, tonicifiers, flavorants, colorants, odorants, opacifiers, suspending agents, binders, fillers, plasticizers, lubricants, and mixtures thereof.

### Pharmaceutical compositions for injection

In some embodiments, the disclosure provides a pharmaceutical composition for injection containing a compound described herein and pharmaceutical excipients suitable for injection. Components and amounts of agents in the compositions are as described herein.

The forms in which the compositions of the disclosure may be incorporated for administration by injection include aqueous or oil suspensions or emulsions. Such compositions may comprise sesame oil, corn oil, cottonseed oil, peanut oil, elixirs containing mannitol or dextrose, sterile water, and similar pharmaceutical vehicles.

Aqueous solutions in saline are also conventionally used for injection. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, for the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like.

Sterile injectable solutions are prepared by incorporating the compound of the disclosure in the required amount in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, certain desirable methods of preparation are vacuum-drying and freeze-drying techniques which yield a powder of the active ingredient plus any additional desired ingredient from a previously sterile-filtered solution thereof.

### Other pharmaceutical compositions

Pharmaceutical compositions may also be prepared from compositions described herein and one or more pharmaceutically acceptable excipients suitable for topical, sublingual, buccal, rectal, intraosseous, intraocular, intranasal, epidural, or intraspinal administration. Preparations for such pharmaceutical compositions are well-known in the art. See, e.g., Anderson, Philip O.; Knoben, James E.; Troutman, William G, eds., Handbook of Clinical Drug Data, Tenth Edition, McGraw-Hill, 2002; Pratt and Taylor, eds., Principles of Drug Action, Third Edition, Churchill Livingston, New York, 1990; Katzung, ed., Basic and Clinical Pharmacology, Ninth Edition, McGraw Hill, 2004; Goodman and Gilman, eds., The Pharmacological Basis of Therapeutics, Tenth Edition, McGraw Hill, 2001 ; Remington's Pharmaceutical Sciences, 20th Ed., Lippincott Williams & Wilkins., 2000; Martindale, The Extra Pharmacopoeia, Thirty-Second Edition (The Pharmaceutical Press, London, 1999).

### Synthesis of Compounds of the Disclosure

Compounds of the disclosure can be prepared by methods described in the General Schemes, procedures, and Examples set forth within, and by related methods known in the art. For example, Compounds of Formula I can be prepared by the general methods shown in General Schemes 1, 1A, 2, 2A, and 3-11.

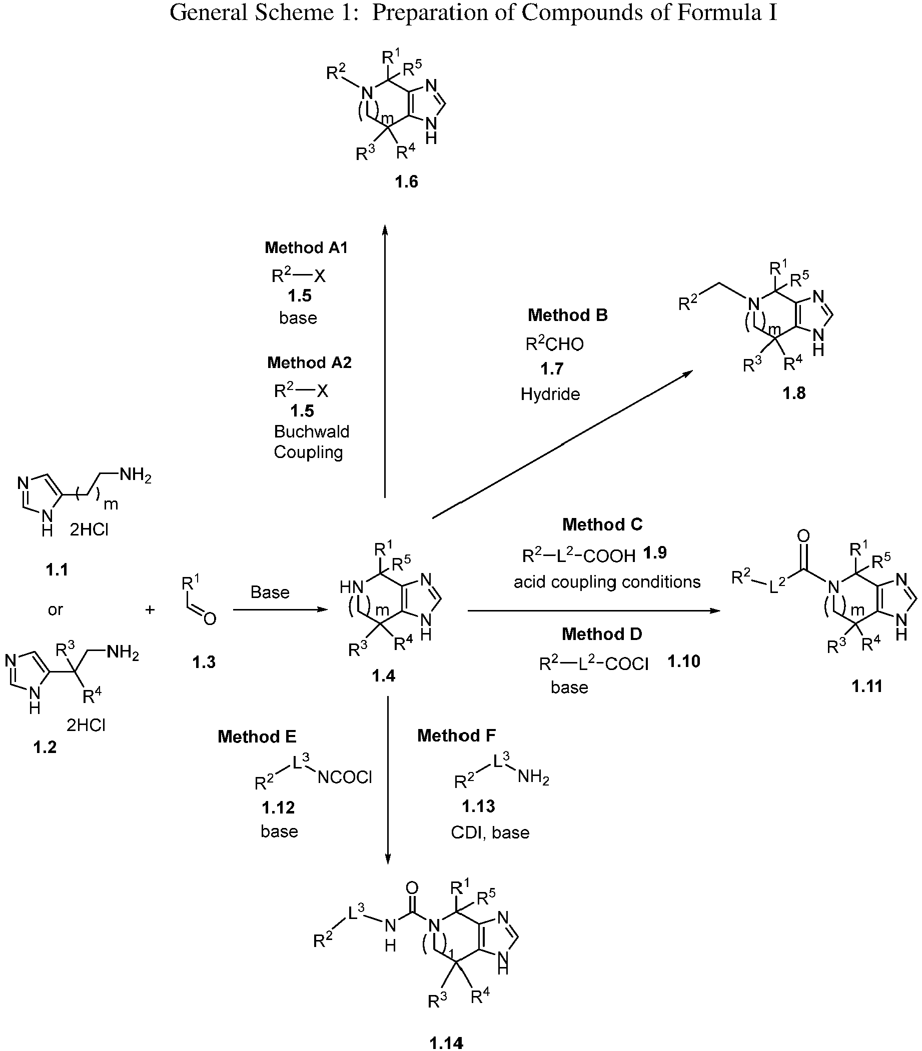

Compounds of Formula I were obtained through the reactions depicted in General Scheme 1. An amine of formulas 1.1 or 1.2 and an aldehyde of formula 1.3 were first reacted under Pictet-Spengler reaction conditions to afford the core amine of formula 1.4. To the extent that an imine by-product is formed during the Pictet-Spengler reaction, the imine by-product can be converted to the core amine of formula 1.4 by reaction with sodium borohydride in an alcoholic solvent (for example, methanol or ethanol). Various L and R² groups were then installed by using either a nucleophilic substitution reaction (Method A1), a Buchwald or other similar cross-coupling reaction (Method A2), a reductive animation reaction (Method B), or amide coupling reactions (Methods C, D, E, and F). Under Method A1, a R² aryl or heteroaryl halide of formula 1.5 (wherein X is Br, Cl, or F) is coupled to the core amine of formula 1.4 using a nucleophilic substitution reaction under basic conditions, such as DIPEA, to afford compounds of formula 1.6. Under Method A2, a R² aryl or heteroaryl halide of formula 1.5 (wherein X is Br, Cl, or I) is coupled to the core amine of formula 1.4 using Buchwald coupling or cross-coupling conditions known in the art, such as using a palladium catalyst (for example, CPhos-Pd-G3, Pd(OAc)₂, Pd(dppf)Cl₂), and a base such as Cs₂CO₃ to afford compounds of formula 1.6. Under Method B, a R² aryl or heteroaryl aldehyde of formula 1.7 is coupled to the core amine of formula 1.4 under reductive animation reaction conditions using a hydride such as sodium triacetoxyborohydride to afford compounds of formula 1.8. Under Method C, a R² carboxylic acid of formula 1.9 or a basic salt (*i.e.,* Li, K, or Na) thereof is coupled to core amine of formula 1.4 using acid coupling conditions known in the art, such as using one of the following reagents-HOBt, EDCI, HATU, T3P-along with a base, such as DIPEA (Hunig's base), pyridine, or TEA, to afford compounds of formula 1.11, wherein L² is a bond, optionally substituted C₁₋₆alkylene, optionally substituted C₂₋₆alkenylene, or optionally substituted C₁₋₆haloalkylene. Alternatively, under Method D, a R² acid chloride of formula 1.10 is coupled to the core amine of formula 1.4 under basic conditions to afford compounds of formula 1.11, wherein L² is a bond, optionally C₁₋₆alkylene, optionally substituted C₂₋₆alkenylene, or optionally substituted C₁₋₆haloalkylene. Under Method E, a R² acid chloride of formula 1.12 is coupled to the core amine of formula 1.4 under basic conditions to afford compounds of formula 1.14, wherein L³ is a bond or optionally substituted C₁₋₆alkylene. Under Method F, an R² amine of formula 1.13 is coupled to the core amine of formula 1.4 and carbonyldiimidazole under basic conditions to afford compounds of formula 1.14, wherein L³ is a bond or optionally substituted C₁₋₆alkylene.

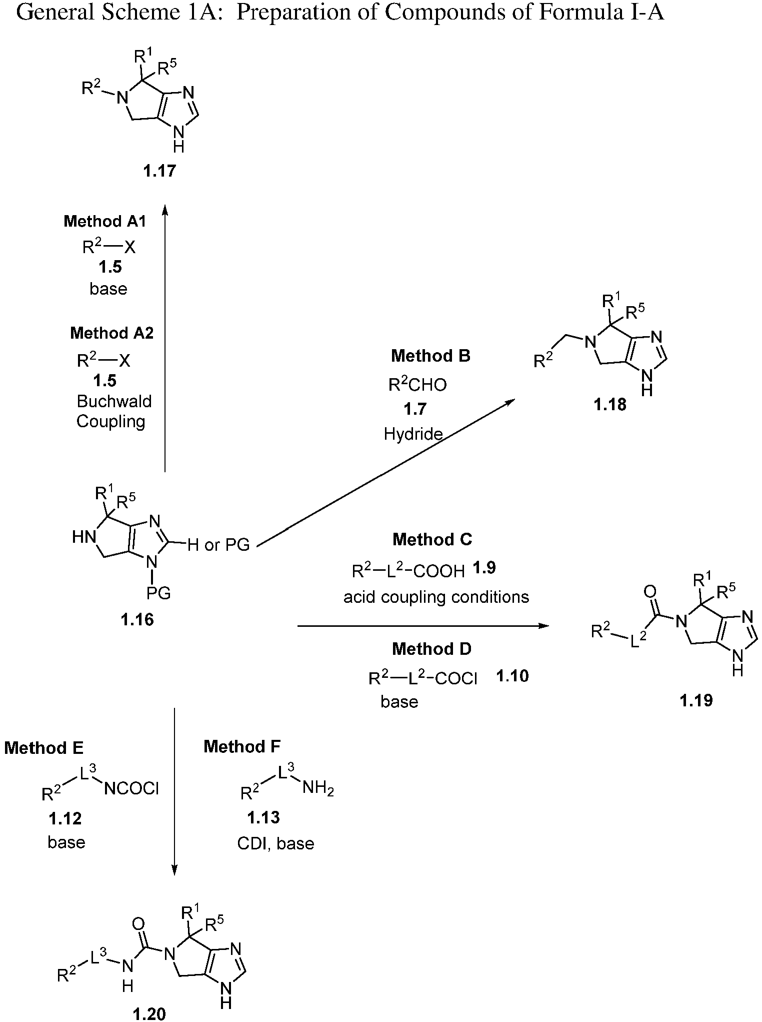

Compounds of Formula I-A wherein m is 0 and R³ and R⁴ are each hydrogen can be obtained through the reactions depicted in General Scheme 1A. Starting with an amine of formula 1.16, prepared as described in General Scheme 2A, various L and R² groups can be installed by using either a nucleophilic substitution reaction (Method A1), a Buchwald or other similar cross-coupling reaction (Method A2), a reductive animation reaction (Method B), or amide coupling reactions (Methods C, D, E, and F). Under Method A1, a R² aryl or heteroaryl halide of formula 1.5 (wherein X is Br, Cl, or F) is coupled to the core amine of formula 1.16 using a nucleophilic substitution reaction under basic conditions, such as DIPEA. Under Method A2, a R² aryl or heteroaryl halide of formula 1.5 (wherein X is Br, Cl, or I) is coupled to the core amine of formula 1.16 using Buchwald coupling or cross-coupling conditions known in the art, such as using a palladium catalyst (for example, CPhos-Pd-G3, Pd(OAc)₂, Pd(dppf)Cl₂)), and a base such as Cs₂CO₃. Under Method B, a R² aryl or heteroaryl aldehyde of formula 1.7 is coupled to the core amine of formula 1.16 under reductive animation reaction conditions using a hydride such as sodium triacetoxyborohydride. Under Method C, a R² carboxylic acid of formula 1.9 or a basic salt (*i.e.,* Li, K, or Na) thereof is coupled to core amine of formula 1.16 using acid coupling conditions known in the art, such as using one of the following reagents-HOBt, EDCI, HATU, T3P-along with a base, such as DIPEA (Hunig's base) or TEA, to afford compounds of formula 1.19 (after any remaining protecting groups are removed), wherein L² is a bond, optionally substituted C₁₋₆alkylene, optionally substituted C₂₋₆alkenylene, or optionally substituted C₁₋₆haloalkylene. Alternatively, under Method D, a R² acid chloride of formula 1.10 is coupled to the core amine of formula 1.16 under basic conditions to afford compounds of formula 1.19 (after any remaining protecting groups are removed), wherein L² is a bond, optionally C₁₋₆alkylene, optionally substituted C₂₋₆alkenylene, or optionally substituted C₁₋₆haloalkylene. Under Method E, a R² acid chloride of formula 1.12 is coupled to the core amine of formula 1.16 under basic conditions to afford compounds of formula 1.20 (after any remaining protecting groups are removed), wherein L³ is a bond or optionally substituted C₁₋₆alkylene. Under Method F, an R² amine of formula 1.13 is coupled to the core amine of formula 1.16 and carbonyldiimidazole under basic conditions to afford compounds of formula 1.20 (after any remaining protecting groups are removed), wherein L³ is a bond or optionally substituted C₁₋₆alkylene. After the coupling reactions in Methods A1, A2, B, C, D, E, or F are completed, any remaining protecting groups (PG) on the imidazole ring or R² group can be removed under suitable conditions to afford the depicted products in General Scheme IA (compounds of formulas 1.17, 1.18, 1.19, and 1.20). For example, the TIPS protecting group can be removed via reaction with tetrabutylammonium fluoride and the amine t-boc protecting group can be removed under acidic conditions.

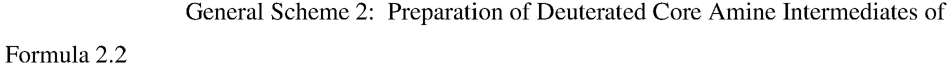

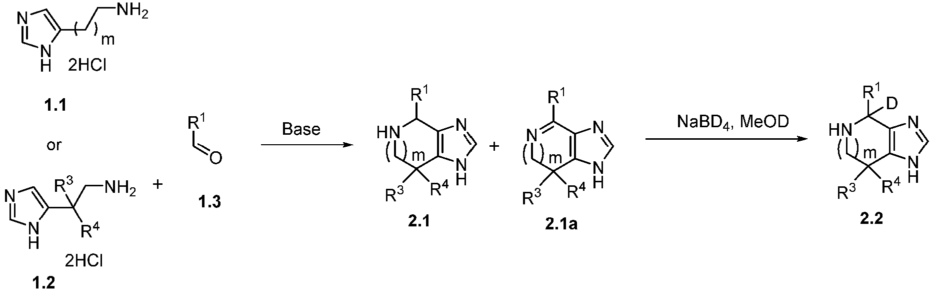

Compounds of Formula I wherein R⁵ is D are prepared in accordance with General Scheme 2. An amine of formula 1.1 or 1.2 and an aldehyde of formula 1.3 are reacted under Pictet-Spengler reaction conditions to afford compounds of formula 2.1 or mixtures of compounds of formula 2.1 and the imine by-product of formula 2.1a. Compounds of formula 2.1 or mixtures of compounds of formulas 2.1 and 2.1a are then reacted with sodium borodeuteride in deuterated methanol to afford deuterated core amine of formula 2.2. Deuterated core amine intermediates of formula 2.2 can then be further coupled to various L and R² groups via methods A1, A2, B, C, D, E, and F as described in General Scheme 1 to afford Compounds of Formula I, wherein R⁵ is D.

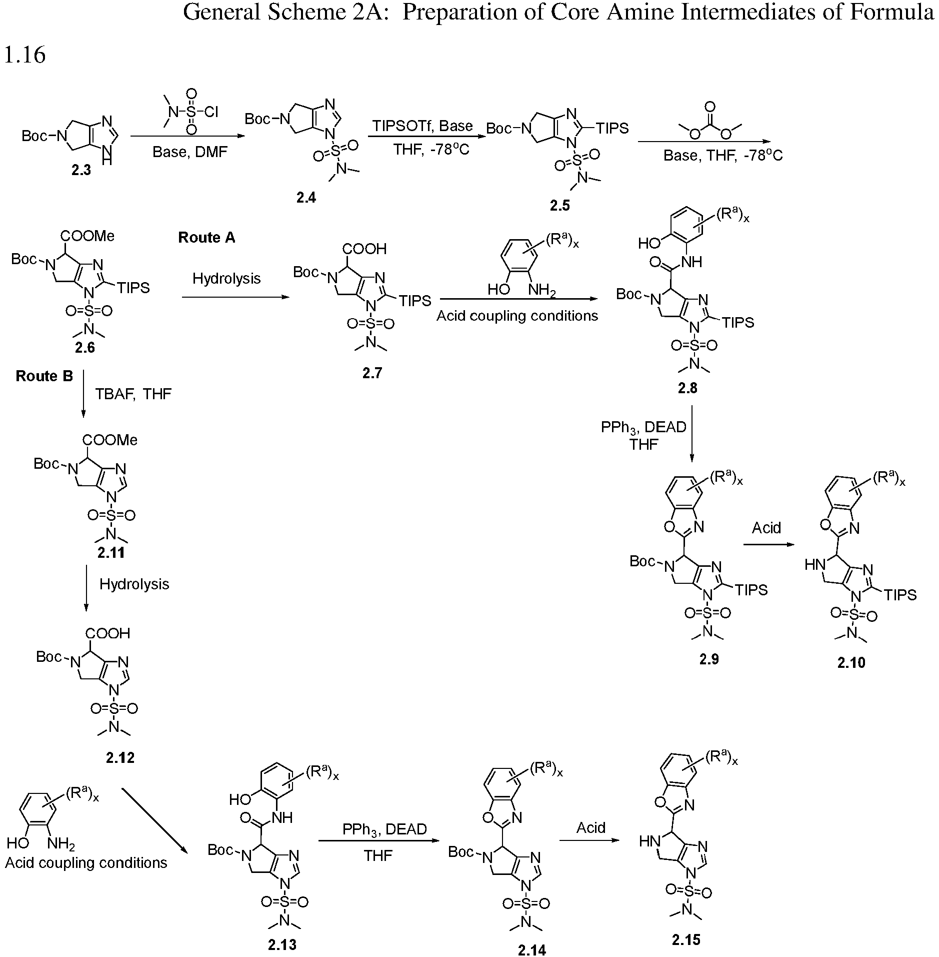

Intermediates of formula 1.16 were prepared in accordance with General Scheme 2A. Starting with tert-butyl 4,6-dihydro-1H-pyrrolo[3,4-d]imidazole-5-carboxylate (2.3), the imidazole amine was protected with a N,N-dimethylsulfamoyl protecting group or another suitable nitrogen protecting group in the presence of a strong base such as LDA and NaH to afford compound 2.4. The acidic proton on the imidazole ring was then protected with a triisopropylsilyl (TIPS) group or other suitable silyl protecting group in the presence of a strong base such as LDA, t-BuLi, or n-BuLi to afford compound 2.5. A methyl ester (or alternatively a C₂-C₄alkyl ester) was installed by reacting dimethyl carbonate and a strong base such as LDA to afford methyl ester 2.6. Under Route A, the methyl ester in compound 2.6 was then hydrolyzed with a basic hydroxide such as LiOH or NaOH to afford carboxylic acid 2.7. The carboxylic acid in compound 2.7 was then reacted with 2-aminophenol (or alternatively a substituted 2-aminophenol) using acid coupling conditions known in the art, such as using one of the following reagents-HOBt, EDCI, HATU, T3P-along with a base, such as DIPEA (Hunig's base) or TEA, to afford compounds of formula 2.8. A compound of formula 2.8 was cyclized using Mitsunobu reaction conditions known in the art, such as PPh₃ and DIAD or DEAD, to form the benzoxazole R¹ group in compounds of formula 2.9. The Boc protecting group was then removed in a compound of formula 2.9 under acidic conditions such as HCl or TFA to afford the amine of formula 2.10. Alternatively, under Route B, the TIPS protecting group can be removed from compound 2.6 prior to hydrolysis of the methyl ester. Amine intermediates of formula 2.10 and 2.15 can then be further coupled to various L and R² groups via methods A1, A2, B, C, D, E, and F as described in General Scheme 1A to afford Compounds of Formula IA, wherein m is 0 and R³ and R⁴ are each hydrogen.

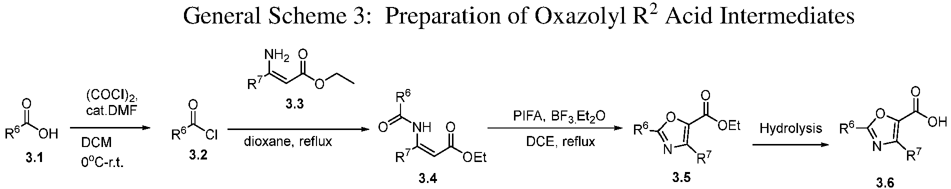

Oxazolyl R² carboxylic acid intermediates containing substitutions at the R⁶ and/or R⁷ positions were prepared in accordance with General Scheme 3. A carboxylic acid of formula 3.1 was reacted with oxalyl chloride and catalytic N,N-dimethylformamide to afford acid chloride of formula 3.2. Acid chloride of formula 3.2 was then reacted with an enamine of formula 3.3 to afford an enamide of formula 3.4. An enamide of formula 3.4 then underwent hypervalent iodine-mediated cyclization after reaction with [bis(trifluoroacetoxy)iodo]benzene and boron trifluoride diethyl etherate to afford oxazole of formula 3.5. Hydrolysis of the ester of the oxazole formula 3.5 with a base such as LiOH, KOH, or NaOH in THF/water afforded compounds of formula 3.6. Alternatively, the basic salt (*i.e.,* Li, K, or Na) of the carboxylic acid of formula 3.6 may be obtained after the hydrolysis reaction by isolating the product at a basic pH. The carboxylic acid or basic salt thereof can then be used without further purification in the coupling reactions described in General Schemes 1 and 1A, Method C.

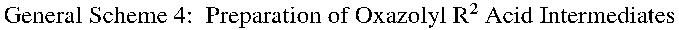

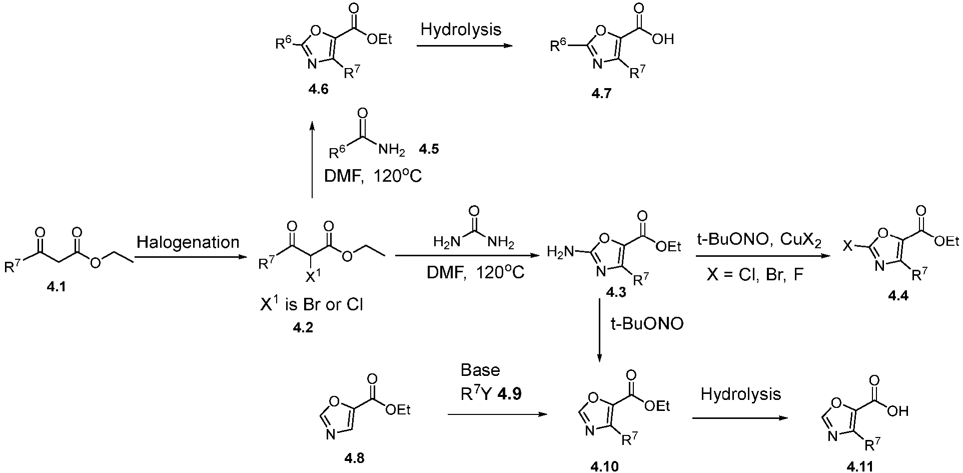

Oxazolyl R² carboxylic acid intermediates containing substitutions at the R⁶ and/or R⁷ positions were prepared in accordance with General Scheme 4. A β-keto ester of formula 4.1 was halogenated with a chlorinating agent such as SOCl₂ or brominating agent such as NBS to yield compounds of formula 4.2 (wherein X¹ is Br or Cl). Compounds of formula 4.2 were reacted with urea to afford amine oxazole compounds of formula 4.3. In addition, compounds of formula 4.2 were reacted with amides of formula 4.5 to afford oxazole compounds of formula 4.6. The amine in compounds of formula 4.3 was subjected to Sandmeyer reaction conditions to afford compounds of formula 4.4 (wherein X is Cl, Br, or F) and compounds of formula 4.10. Alternatively, compounds of formula 4.10 may be obtained by deprotonating a compound of formula 4.8 with a base such as LiHMDS followed by reaction in a nucleophilic substitution reaction with reagents of formula 4.9 (wherein Y is a suitable leaving group such as Br, Cl, mesylate, or tosylate) to afford compounds of formula 4.10. Hydrolysis of the ester of compounds of formulas 4.6 and 4.10 with a base such as LiOH, KOH, or NaOH in THF/water affords compounds of formulas 4.7 and 4.11. Alternatively, the basic salt (*i.e.,* Li, K, or Na) of the carboxylic acid of formulas 4.7 and 4.11 may be obtained after the hydrolysis reaction by isolating the product at a basic pH. The carboxylic acid or basic salt thereof can then be used without further purification in the coupling reactions described in General Schemes 1 and 1A, Method C.

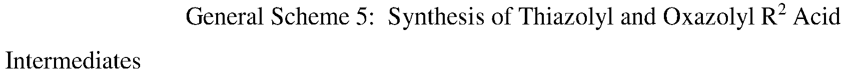

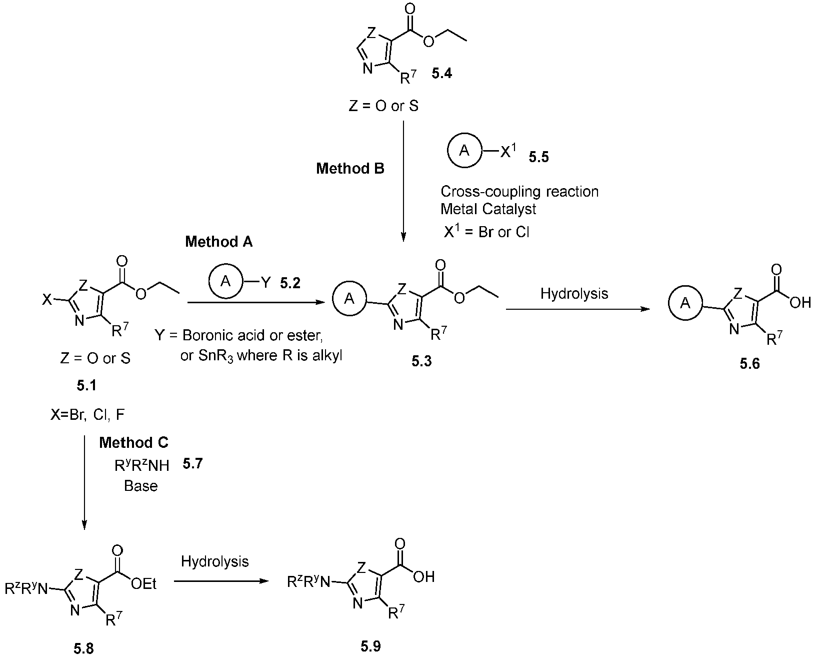

Oxazolyl and thiazolyl R² carboxylic acid intermediates containing substitutions at the R⁶ and/or R⁷ positions were prepared in accordance with General Scheme 5. Under Method A, a halide of formula 5.1 was reacted with a heteroaryl or aryl of formula 5.2 in either a Suzuki coupling (where Y is a boronic acid or ester) or a Stille coupling (where Y is SnR₃) to afford compounds of formula 5.3. Under Method B, compounds of formula 5.3 were obtained by reaction of a compound of formula 5.4 with a heteroaryl or aryl halide compound of formula 5.5 in a cross-coupling reaction using a metal catalyst such as a Buchwald catalyst or Ullman catalyst. Under Method C, a halide of formula 5.1 may also be reacted with an amine of formula 5.7 or a heterocyclic amine such as piperidine, morpholine, piperazine, azetidine, and pyrrolidine, and a base such as TEA in a displacement reaction to afford compounds of formula 5.8. Hydrolysis of the ester of compounds of formulas 5.3 and 5.8 with a base such as LiOH, KOH, or NaOH in THF/water affords compounds of formulas 5.6 and 5.9. Alternatively, the basic salt (*i.e.,* Li, K, or Na) of the carboxylic acid of formulas 5.6 and 5.9 may be obtained after the hydrolysis reaction by isolating the product at a basic pH. The carboxylic acid or basic salt thereof can then be used without further purification in the coupling reactions described in General Schemes 1 and 1A, Method C.

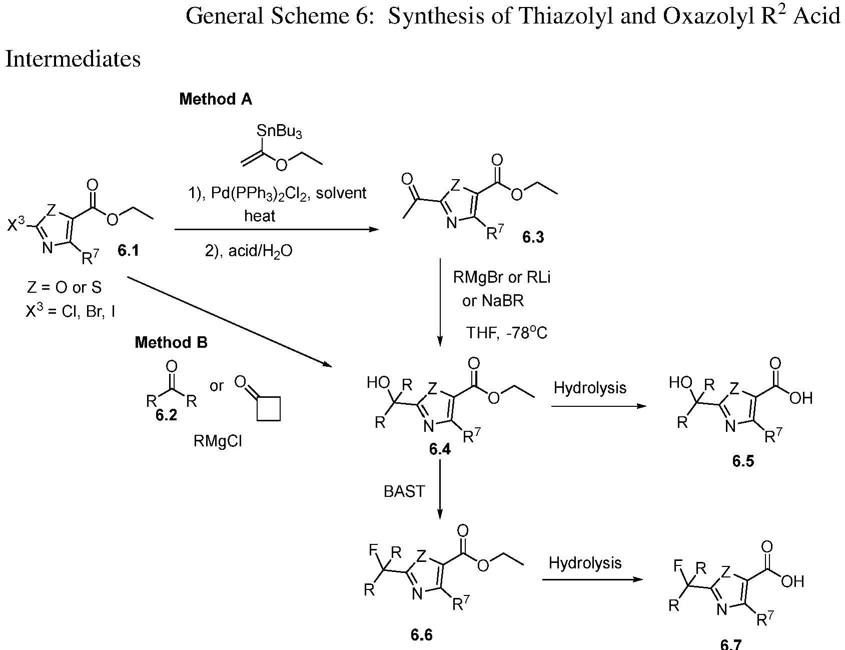

Oxazolyl and thiazolyl R² carboxylic acid intermediates containing hydroxyalkyl and haloalkyl substitutions were prepared in accordance with General Scheme 6. Under Method A, a halide of formula 6.1 was reacted with tributyl(1-ethoxyvinyl)stannane in a Stille coupling, followed by hydrolysis with acid/water such as TFA to afford compounds of formula 6.3. An alkyl group ("R") is then added to the ketone in compounds of formula 6.3 using RMgBr or RLi or through reduction of the ketone with NaBR to afford compounds of formula 6.4. Under Method B, compounds of formula 6.4 may also be obtained starting with a compound of formula 6.1 through a Grignard addition reaction with a ketone of formula 6.2 (wherein R is an alkyl) or cyclobutanone and RMgCl such as iPrMgCl. The hydroxyl substituent in compounds of formula 6.4 was converted to a fluorine substituent using a fluorinating agent such as BAST to afford compounds of formula 6.6. Hydrolysis of the ester of compounds of formulas 6.4 and 6.6 with a base such as LiOH, KOH, or NaOH in THF/water affords compounds of formulas 6.5 and 6.7. Alternatively, the basic salt (*i.e.,* Li, K, or Na) of the carboxylic acid of formulas 6.5 and 6.7 may be obtained after the hydrolysis reaction by isolating the product at a basic pH. The carboxylic acid or basic salt thereof can then be used without further purification in the coupling reactions described in General Schemes 1 and 1A, Method C.

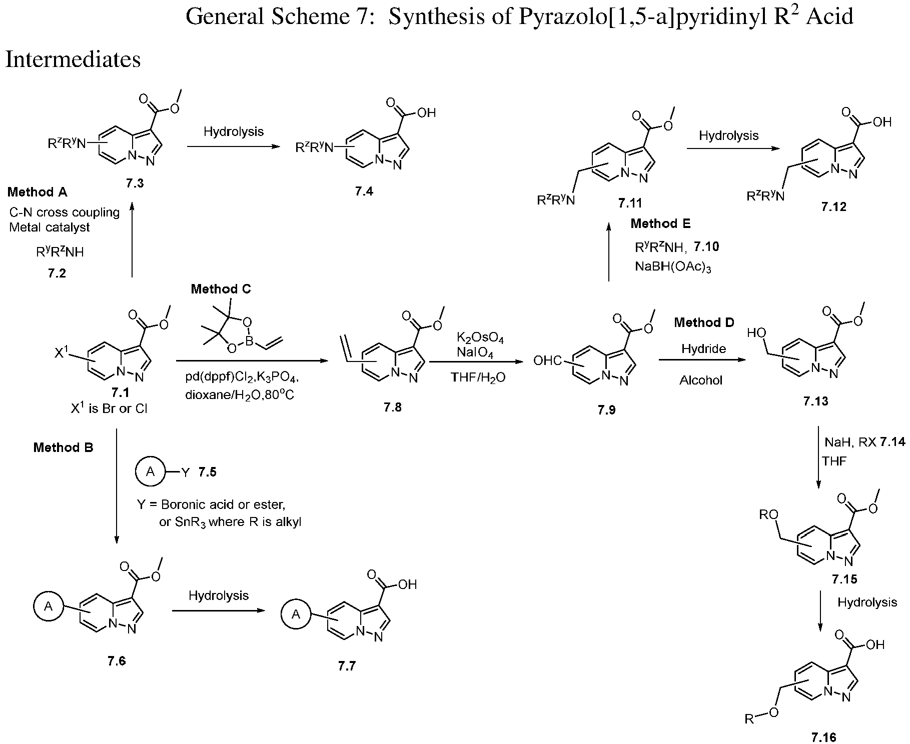

Substituted pyrazolo[1,5-a]pyridinyl R² carboxylic acid intermediates were prepared in accordance with General Scheme 7. Under Method A, a halide of formula 7.1 (wherein X¹ is Br or Cl) was reacted in a C-N cross-coupling reaction using a metal catalyst such as a Buchwald catalyst or Ullman catalyst with an amine of formula 7.2 or a heterocyclic amine such as piperidine, morpholine, piperazine, azetidine, and pyrrolidine to afford compounds of formula 7.3. Under Method B, a halide of formula 7.1 (wherein X¹ is Br or Cl) may also be reacted with a heteroaryl or aryl compound of formula 7.5 in either a Suzuki coupling (where Y is a boronic acid or ester) or a Stille coupling (where Y is SnR₃) to afford compounds of formula 7.6. Under Method C, a halide of formula 7.1 (wherein X¹ is Br or Cl) was reacted with 4,4,5,5-tetramethyl-2-vinyl-1,3,2-dioxaborolane in a Suzuki coupling to afford compounds of formula 7.8. The olefin in compounds of formula 7.8 then underwent oxidative cleavage to an aldehyde to afford compounds of formula 7.9. Under Method D, the aldehyde in compounds of formula 7.9 was reduced to an alcohol with a hydride such as NaBH₄ in an alcoholic solvent such as methanol or ethanol to afford formula 7.13. The alcohol in compounds of formula 7.13 was alkylated with sodium hydride and an alkyl ("R") halide of formula 7.14 (wherein X is Cl, Br, or F) to afford compounds of formula 7.15. Under Method E, a compound of formula 7.9 was reacted with an amine of formula 7.10 under reductive animation conditions to afford compounds of formula 7.11. Hydrolysis of the ester of compounds of formulas 7.3, 7.6, 7.11, and 7.15 with a base such as LiOH, KOH, or NaOH in THF/water affords compounds of formulas 7.4, 7.7, 7.12, and 7.16. Alternatively, the basic salt (*i.e.,* Li, K, or Na) of the carboxylic acid of formulas 7.4, 7.7, 7.12, and 7.16 may be obtained after the hydrolysis reaction by isolating the product at a basic pH. The carboxylic acid or basic salt thereof can then be used without further purification in the coupling reactions described in General Schemes 1 and 1A, Method C.

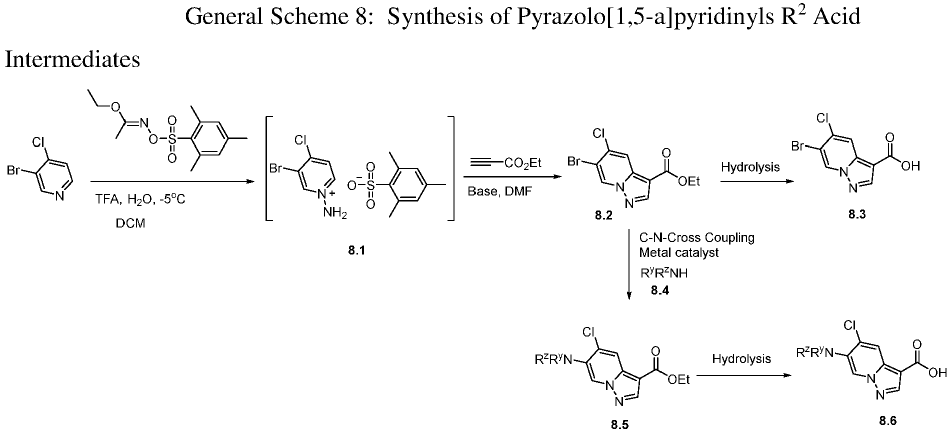

Di-substituted pyrazolo[1,5-a]pyridinyl R² carboxylic acid intermediates were prepared in accordance with General Scheme 8. Starting reagent 3-bromo-4-chloropyridine was reacted with ethyl (E)-N-((mesitylsulfonyl)oxy)acetimidate to afford the N-aminopyridinium salt 8.1. A cycloaddition reaction of the N-aminopyridinium salt 8.1 with ethyl propiolate and base such as K₂CO₃ afforded ethyl 6-bromo-5-chloropyrazolo[1,5-a]pyridine-3-carboxylate 8.2. Compound 8.2 was reacted in a C-N cross-coupling reaction using a metal catalyst such as a Buchwald catalyst or Ullman catalyst with an amine of formula 8.4 or a heterocyclic amine such as piperidine, morpholine, piperazine, azetidine, and pyrrolidine to afford compounds of formula 8.5. Hydrolysis of the ester of compounds of formulas 8.2 and 8.5 with a base such as LiOH, KOH, or NaOH in THF/water affords compounds of formulas 8.3 and 8.6. Alternatively, the basic salt (*i.e.,* Li, K, or Na) of the carboxylic acid of formulas 8.3 and 8.6 may be obtained after the hydrolysis reaction by isolating the product at a basic pH. The carboxylic acid or basic salt thereof can then be used without further purification in the coupling reactions described in General Schemes 1 and 1A, Method C.

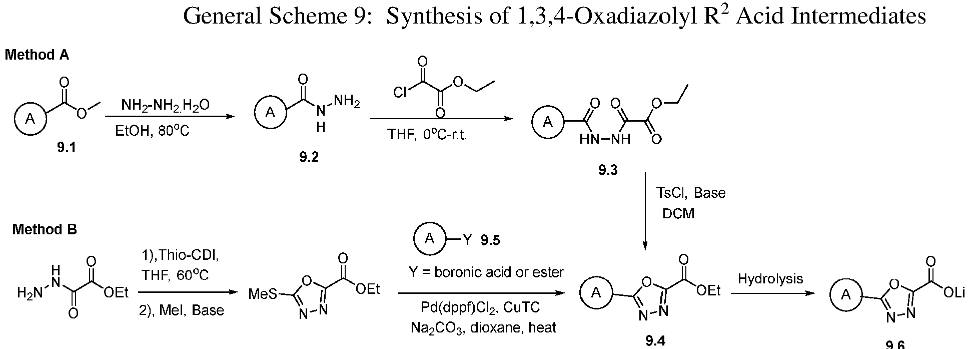

Heteroaryl and aryl-substituted oxadiazolyl R² carboxylic acid intermediates were prepared in accordance with General Scheme 9. Under Method A, heteroaryl and aryl esters of formula 9.1 were reacted with hydrazine hydrate to afford hydrazines of formula 9.2. Compounds of formula 9.2 were then reacted with ethyl-2-chloro-2-oxoacetate to afford compounds of formula 9.3, which subsequently underwent intramolecular cyclization with p-toluenesulfonyl chloride and a base such as TEA to afford the 1,3,4-oxadiazole of formula 9.4. Alternatively, compounds of formula 9.4 can be made using Method B. Under Method B, ethyl 2-hydrazineyl-2-oxoacetate was reacted with 1,1'-thiocarbonyldiimidazole to form the 1,3,4-oxadiazole, which was then alkylated with methyl iodide and base such as TEA to afford ethyl 5-(methylthio)-1,3,4-oxadiazole-2-carboxylate. Ethyl 5-(methylthio)-1,3,4-oxadiazole-2-carboxylate was reacted with a heteroaryl or aryl boronic acid or ester of formula 9.5 in a desulfitative C-C cross coupling reaction (also known as Liebeskind-Srogl cross-coupling) to afford compounds of formula 9.4. Hydrolysis of the ester of compounds of formula 9.4 with a base such as LiOH, KOH, or NaOH in THF/water affords compounds of formula 9.6. Alternatively, the basic salt (*i.e.,* Li, K, or Na) of the carboxylic acid of formula 9.6 may be obtained after the hydrolysis reaction by isolating the product at a basic pH. The carboxylic acid or basic salt thereof can then be used without further purification in the coupling reactions described in General Schemes 1 and 1A, Method C.

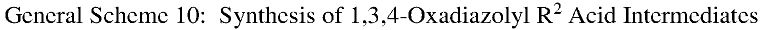

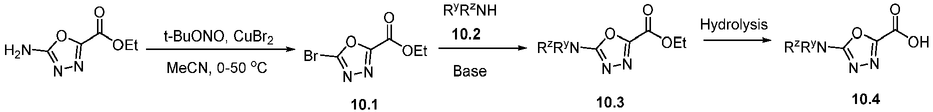

Substituted 1,3,4-oxadiazolyl R² carboxylic acid intermediates were prepared in accordance with General Scheme 10. Starting reagent ethyl 5-amino-1,3,4-oxadiazole-2-carboxylate was subjected to Sandmeyer reaction conditions to afford ethyl 5-bromo-1,3,4-oxadiazole-2-carboxylate (Compound 10.1). The bromo in compound 10.1 was reacted with an amine of formula 10.2 or a heterocyclic amine such as piperidine, morpholine, piperazine, azetidine, and pyrrolidine, and a base such as TEA to afford compounds of formula 10.3. Hydrolysis of the ester of compounds of formula 10.3 with a base such as LiOH, KOH, or NaOH in THF/water afforded compounds of formula 10.4. Alternatively, the basic salt (*i.e.,* Li, K, or Na) of the carboxylic acid of formula 10.4 may be obtained after the hydrolysis reaction by isolating the product at a basic pH. The carboxylic acid or basic salt thereof can then be used without further purification in the coupling reactions described in General Schemes 1 and 1A, Method C.

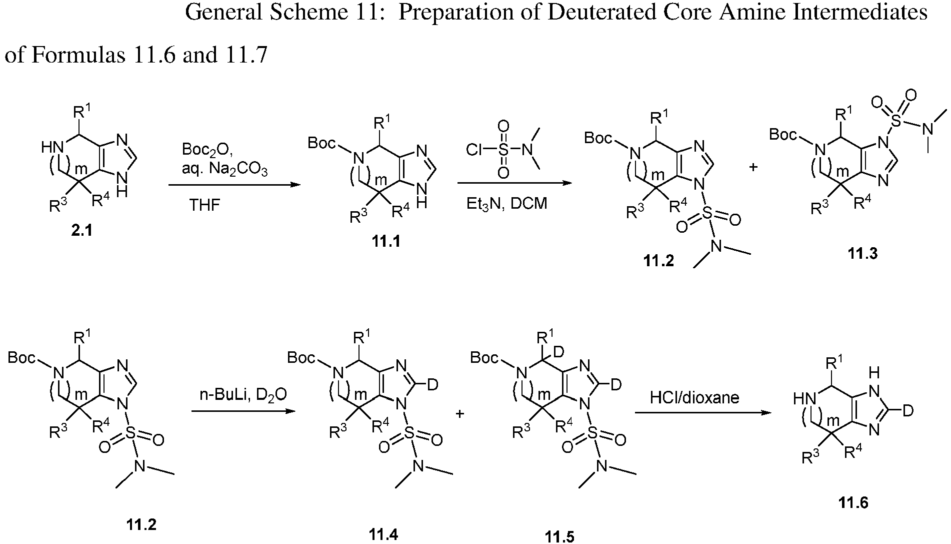

Compounds of Formula I wherein R^{5A} is D are prepared in accordance with General Scheme 11. An amine of formula 2.1, prepared according to General Scheme 2, is protected with a BOC protecting group or other suitable nitrogen protecting group to afford compounds of formula 11.1. The amine in formula 11.1 is then protected with a dimethylsulfamoyl protecting group to afford a mixture of compounds of formula 11.2 and 11.3, which are separated during purification. Deuterium is then incorporated at the R^{5A} position by deprotonating compounds of formula 11.2 with butyllithium and then adding D₂O to afford compounds of formulas 11.4 and 11.5. Deprotection of the nitrogen protecting groups with acid afforded deuterated core amine intermediates of formulas 11.6. Deuterated core amine intermediates of formulas 11.6 can be further coupled to various L and R² groups via methods A1, A2, B, C, D, E, and F as described in General Scheme 1 to afford Compounds of Formula I, wherein R^{5A} is D.

### Examples

In some embodiments, the disclosure provides specific examples of Formula I, and their pharmaceutically acceptable salts and/or isotopologues, as set forth in Table 1S below.

| **Table 1S: Benzo[d]oxazoles** | | |
|---|---|---|
| **Ex. #** | **Structure** | **Name** |
| 1 | 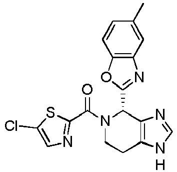 | (S)-(5-chlorothiazol-2-yl)(4-(5-methylbenzo[d]oxazol-2-yl)-6,7-dihydro-1H-imidazo[4,5-c]pyridin-5(4H)-yl)methanone |
| 3 | 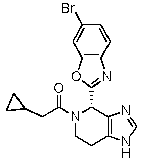 | (S)-1-(4-(6-bromobenzo[d]oxazol-2-yl)-6,7-dihydro-1H-imidazo[4,5-c]pyridin-5(4H)-yl)-2-cyclopropylethanone |
| 5 | 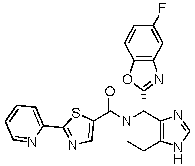 | (S)-(4-(5-fluorobenzo[d]oxazol-2-yl)-6,7-dihydro-1H-imidazo[4,5-c]pyridin-5(4H)-yl)(2-(pyridin-2-yl)thiazol-5-yl)methanone |
| 7 | 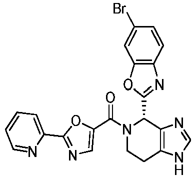 | (S)-(4-(6-bromobenzo[d]oxazol-2-yl)-6,7-dihydro-1H-imidazo[4,5-c]pyridin-5(4H)-yl)(2-(pyridin-2-yl)oxazol-5-yl)methanone |
| 9 | 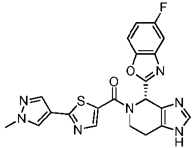 | (S)-(4-(5-fluorobenzo[d]oxazol-2-yl)-6,7-dihydro-1H-imidazo[4,5-c]pyridin-5(4H)-yl)(2-(1-methyl-1H-pyrazol-4-yl)thiazol-5-yl)methanone |
| 11 | 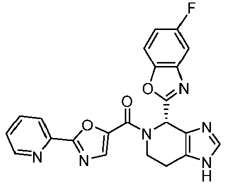 | (S)-(4-(5-fluorobenzo[d]oxazol-2-yl)-6,7-dihydro-1H-imidazo[4,5-c]pyridin-5(4H)-yl)(2-(pyridin-2-yl)oxazol-5-yl)methanone |
| 13 | 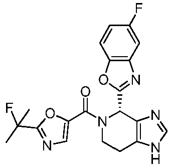 | (S)-(4-(5-fluorobenzo[d]oxazol-2-yl)-6,7-dihydro-1H-imidazo[4,5-c]pyridin-5(4H)-yl)(2-(2-fluoropropan-2-yl)oxazol-5-yl)methanone |
| 15 | 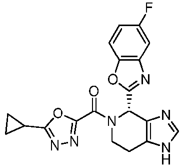 | (S)-(5-cyclopropyl-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 17 | 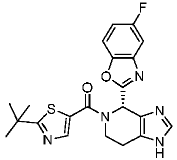 | (S)-(2-(tert-butyl)thiazol-5-yl)(4-(5-fluorobenzo[d]oxazol-2-yl)-6,7-dihydro-1H-imidazo[4,5-c]pyridin-5(4H)-yl)methanone |
| 19 | 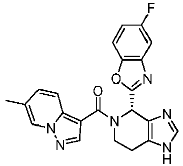 | (S)-(4-(5-fluorobenzo[d]oxazol-2-yl)-6,7-dihydro-1H-imidazo[4,5-c]pyridin-5(4H)-yl)(6-methylpyrazolo[1,5-a]pyridin-3-yl)methanone |
| 21 | 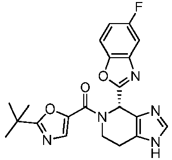 | (S)-(2-(tert-butyl)oxazol-5-yl)(4-(5-fluorobenzo[d]oxazol-2-yl)-6,7-dihydro-1H-imidazo[4,5-c]pyridin-5(4H)-yl)methanone |
| 23 | 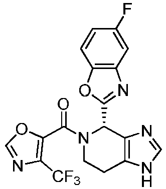 | (S)-(4-(5-fluorobenzo[d]oxazol-2-yl)-6,7-dihydro-1H-imidazo[4,5-c]pyridin-5(4H)-yl)(4-(trifluoromethyl)oxazol-5-yl)methanone |
| 25 | 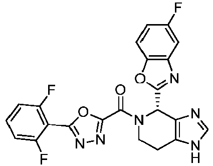 | (S)-(5-(2,6-difluorophenyl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 27 | 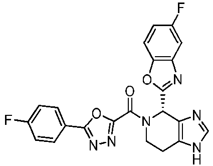 | (S)-(4-(5-fluorobenzo[d]oxazol-2-yl)-6,7-dihydro-1H-imidazo[4,5-c]pyridin-5(4H)-yl)(5-(4-fluorophenyl)-1,3,4-oxadiazol-2-yl)methanone |
| 29 | 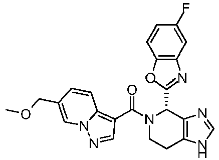 | (S)-(4-(5-fluorobenzo[d]oxazol-2-yl)-6,7-dihydro-1H-imidazo[4,5-c]pyridin-5(4H)-yl)(6-(methoxymethyl)pyrazolo[1,5-a]pyridin-3-yl)methanone |
| 31 | 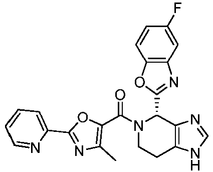 | (S)-(4-(5-fluorobenzo[d]oxazol-2-yl)-6,7-dihydro-1H-imidazo[4,5-c]pyridin-5(4H)-yl)(4-methyl-2-(pyridin-2-yl)oxazol-5-yl)methanone |
| 33 | 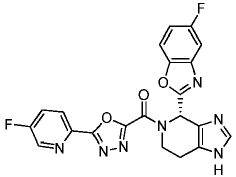 | (S)-(4-(5-fluorobenzo[d]oxazol-2-yl)-6,7-dihydro-1H-imidazo[4,5-c]pyridin-5(4H)-yl)(5-(5-fluoropyridin-2-yl)-1,3,4-oxadiazol-2-yl)methanone |
| 35 | 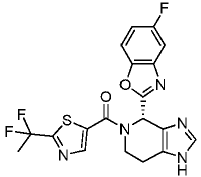 | (S)-(2-(1,1-difluoroethyl)thiazol-5-yl)(4-(5-fluorobenzo[d]oxazol-2-yl)-6,7-dihydro-1H-imidazo[4,5-c]pyridin-5(4H)-yl)methanone |
| 37 | 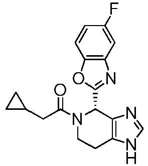 | (S)-2-cyclopropyl-1-(4-(5-fluorobenzo[d]oxazol-2-yl)-6,7-dihydro-1H-imidazo[4,5-c]pyridin-5(4H)-yl)ethanone |
| 39 | 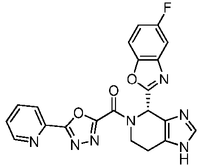 | (S)-(4-(5-fluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 41 | 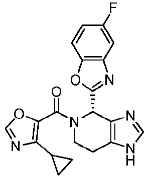 | (S)-(4-cyclopropyloxazol-5-yl)(4-(5-fluorobenzo[d]oxazol-2-yl)-6,7-dihydro-1H-imidazo[4,5-c]pyridin-5(4H)-yl)methanone |
| 43 | 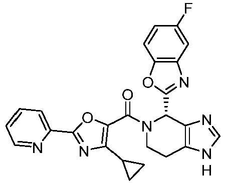 | (S)-(4-cyclopropyl-2-(pyridin-2-yl)oxazol-5-yl)(4-(5-fluorobenzo[d]oxazol-2-yl)-6,7-dihydro-1H-imidazo[4,5-c]pyridin-5(4H)-yl)methanone |
| 45 | 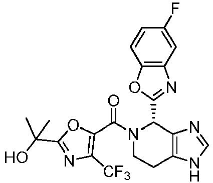 | (S)-(4-(5-fluorobenzo[d]oxazol-2-yl)-6,7-dihydro-1H-imidazo[4,5-c]pyridin-5(4H)-yl)(2-(2-hydroxypropan-2-yl)-4-(trifluoromethyl)oxazol-5-yl)methanone |
| 47 | 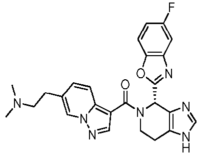 | (S)-(6-(2-(dimethylamino)ethyl)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 49 | 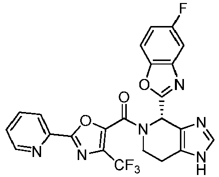 | (S)-(4-(5-fluorobenzo[d]oxazol-2-yl)-6,7-dihydro-1H-imidazo[4,5-c]pyridin-5(4H)-yl)(2-(pyridin-2-yl)-4-(trifluoromethyl)oxazol-5-yl)methanone |
| 51 | 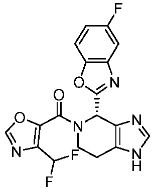 | (S)-(4-(difluoromethyl)oxazol-5-yl)(4-(5-fluorobenzo[d]oxazol-2-yl)-6,7-dihydro-1H-imidazo[4,5-c]pyridin-5(4H)-yl)methanone |
| 53 | 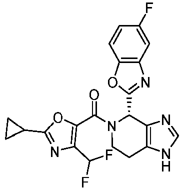 | (S)-(2-cyclopropyl-4-(difluoromethyl)oxazol-5-yl)(4-(5-fluorobenzo[d]oxazol-2-yl)-6,7-dihydro-1H-imidazo[4,5-c]pyridin-5(4H)-yl)methanone |
| 55 | 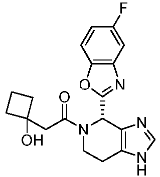 | (S)-1-(4-(5-fluorobenzo[d]oxazol-2-yl)-6,7-dihydro-1H-imidazo[4,5-c]pyridin-5(4H)-yl)-2-(1-hydroxycyclobutyl)ethanone |
| 57 | 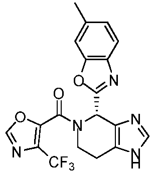 | (S)-(4-(6-methylbenzo[d]oxazol-2-yl)-6,7-dihydro-1H-imidazo[4,5-c]pyridin-5(4H)-yl)(4-(trifluoromethyl)oxazol-5-yl)methanone |
| 59 | 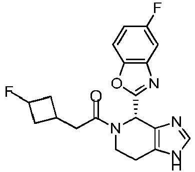 | (S)-1-(4-(5-fluorobenzo[d]oxazol-2-yl)-6,7-dihydro-1H-imidazo[4,5-c]pyridin-5(4H)-yl)-2-(3-fluorocyclobutyl)ethanone |
| 61 | 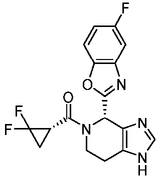 | ((S)-2,2-difluorocyclopropyl)((S)-4-(5-fluorobenzo[d]oxazol-2-yl)-6,7-dihydro-1H-imidazo[4,5-c]pyridin-5(4H)-yl)methanone |
| 63 | 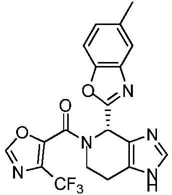 | (S)-(4-(5-methylbenzo[d]oxazol-2-yl)-6,7-dihydro-1H-imidazo[4,5-c]pyridin-5(4H)-yl)(4-(trifluoromethyl)oxazol-5-yl)methanone |
| 65 | 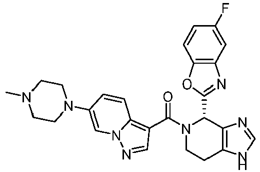 | (S)-(4-(5-fluorobenzo[d]oxazol-2-yl)-6,7-dihydro-1H-imidazo[4,5-c]pyridin-5(4H)-yl)(6-(4-methylpiperazin-1-yl)pyrazolo[1,5-a]pyridin-3-yl)methanone |
| 67 | 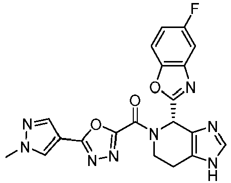 | (S)-(4-(5-fluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 69 | 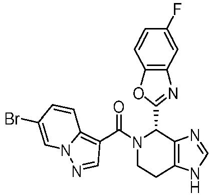 | (S)-(6-bromopyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 71 | 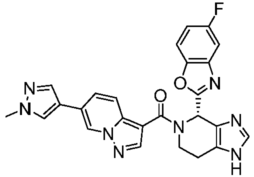 | (S)-(4-(5-fluorobenzo[d]oxazol-2-yl)-6,7-dihydro-1H-imidazo[4,5-c]pyridin-5(4H)-yl)(6-(1-methyl-1H-pyrazol-4-yl)pyrazolo[1,5-a]pyridin-3-yl)methanone |
| 73 | 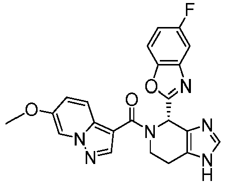 | (S)-(4-(5-fluorobenzo[d]oxazol-2-yl)-6,7-dihydro-1H-imidazo[4,5-c]pyridin-5(4H)-yl)(6-methoxypyrazolo[1,5-a]pyridin-3-yl)methanone |
| 75 | 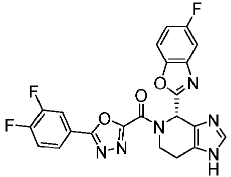 | (S)-(5-(3,4-difluorophenyl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 77 | 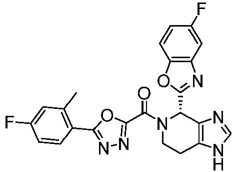 | (S)-(5-(4-fluoro-2-methylphenyl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 79 | 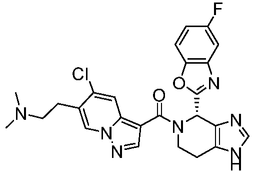 | (S)-(5-chloro-6-(2-(dimethylamino)ethyl)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 81 | 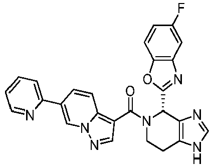 | (S)-(4-(5-fluorobenzo[d]oxazol-2-yl)-6,7-dihydro-1H-imidazo[4,5-c]pyridin-5(4H)-yl)(6-(pyridin-2-yl)pyrazolo[1,5-a]pyridin-3-yl)methanone |
| 84 | 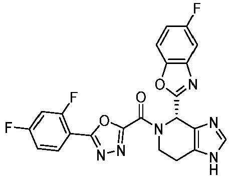 | (S)-(5-(2,4-difluorophenyl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 85 | 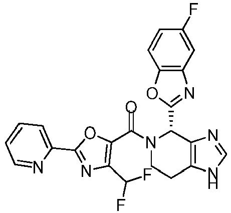 | (S)-(4-(difluoromethyl)-2-(pyridin-2-yl)oxazol-5-yl)(4-(5-fluorobenzo[d]oxazol-2-yl)-6,7-dihydro-1H-imidazo[4,5-c]pyridin-5(4H)-yl)methanone |
| 88 | 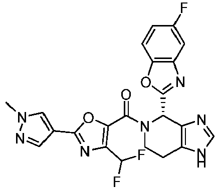 | (S)-(4-(difluoromethyl)-2-(1-methyl-1H-pyrazol-4-yl)oxazol-5-yl)(4-(5-fluorobenzo[d]oxazol-2-yl)-6,7-dihydro-1H-imidazo[4,5-c]pyridin-5(4H)-yl)methanone |
| 90 | 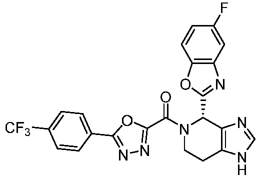 | (S)-(4-(5-fluorobenzo[d]oxazol-2-yl)-6,7-dihydro-1H-imidazo[4,5-c]pyridin-5(4H)-yl)(5-(4-(trifluoromethyl)phenyl)-1,3,4-oxadiazol-2-yl)methanone |
| 91 | 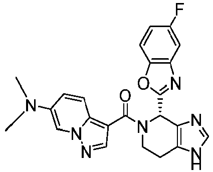 | (S)-(6-(dimethylamino)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 93 | 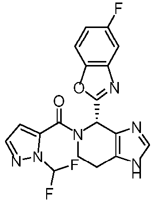 | (S)-(1-(difluoromethyl)- 1H-pyrazol-5-yl)(4-(5-fluorobenzo[d]oxazol-2-yl)-6,7-dihydro-1H-imidazo[4,5-c]pyridin-5(4H)-yl)methanone |
| 95 | 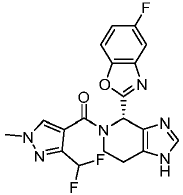 | (S)-(3-(difluoromethyl)-1-methyl-1H-pyrazol-4-yl)(4-(5-fluorobenzo[d]oxazol-2-yl)-6,7-dihydro-1H-imidazo[4,5-c]pyridin-5(4H)-yl)methanone |
| 97 | 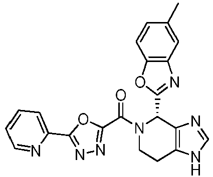 | (S)-(4-(5-methylbenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 99 | 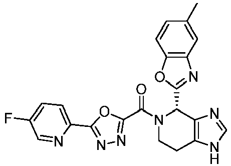 | (S)-(5-(5-fluoropyridin-2-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 101 | 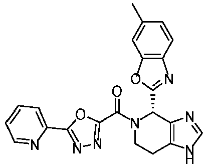 | (S)-(4-(6-methylbenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 103 | 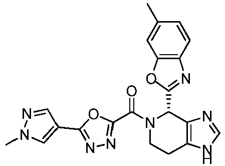 | (S)-(5-(1-methyl-1H-pyrazol-4-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 106 | 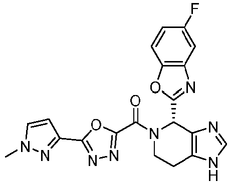 | (S)-(4-(5-fluorobenzo[d]oxazol-2-yl)-6,7-dihydro-1H-imidazo[4,5-c]pyridin-5(4H)-yl)(5-(1-methyl-1H-pyrazol-3-yl)-1,3,4-oxadiazol-2-yl)methanone |
| 107 | 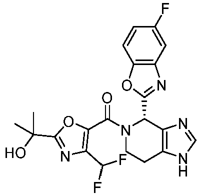 | (S)-(4-(difluoromethyl)-2-(2-hydroxypropan-2-yl)oxazol-5-yl)(4-(5-fluorobenzo[d]oxazol-2-yl)-6,7-dihydro-1H-imidazo[4,5-c]pyridin-5(4H)-yl)methanone |
| 110 | 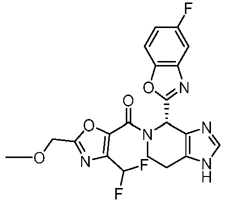 | (S)-(4-(difluoromethyl)-2-(methoxymethyl)oxazol-5-yl)(4-(5-fluorobenzo[d]oxazol-2-yl)-6,7-dihydro-1H-imidazo[4,5-c]pyridin-5(4H)-yl)methanone |
| 111 | 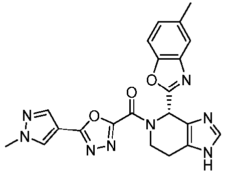 | (S)-(5-(1-methyl-1H-pyrazol-4-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 113 | 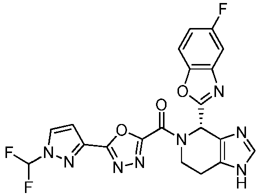 | (S)-(5-(1-(difluoromethyl)-1H-pyrazol-3-yl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 115 | 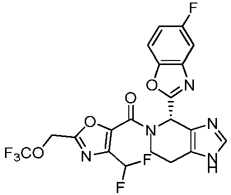 | (S)-(4-(difluoromethyl)-2-((trifluoromethoxy)methyl)oxazol-5-yl)(4-(5-fluorobenzo[d]oxazol-2-yl)-6,7-dihydro-1H-imidazo[4,5-c]pyridin-5(4H)-yl)methanone |
| 118 | 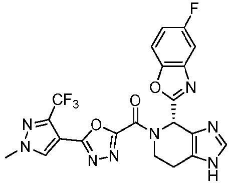 | (S)-(4-(5-fluorobenzo[d]oxazol-2-yl)-6,7-dihydro-1H-imidazo[4,5-c]pyridin-5(4H)-yl)(5-(1-methyl-3-(trifluoromethyl)-1H-pyrazol-4-yl)-1,3,4-oxadiazol-2-yl)methanone |
| 119 | 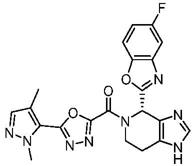 | (S)-(5-(1,4-dimethyl-1H-pyrazol-5-yl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 122 | 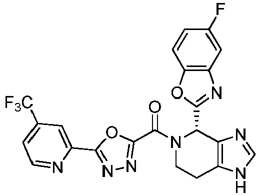 | [(4S)-4-(5-fluoro-1,3-benzoxazol-2-yl)-1,4,6,7-tetrahydroimidazo[4,5-c]pyridin-5-yl]-[5-[4-(trifluoromethyl)-2-pyridyl]-1,3,4-oxadiazol-2-yl]methanone |
| 124 | 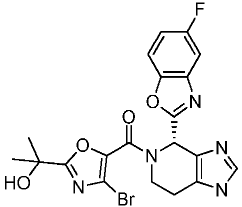 | (S)-(4-bromo-2-(2-hydroxypropan-2-yl)oxazol-5-yl)(4-(5-fluorobenzo[d]oxazol-2-yl)-6,7-dihydro-1H-imidazo[4,5-c]pyridin-5(4H)-yl)methanone |
| 126 | 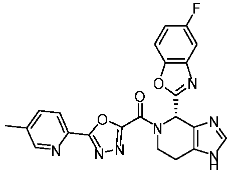 | (S)-(4-(5-fluorobenzo[d]oxazol-2-yl)-6,7-dihydro-1H-imidazo[4,5-c]pyridin-5(4H)-yl)(5-(5-methylpyridin-2-yl)-1,3,4-oxadiazol-2-yl)methanone |
| 127 | 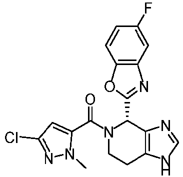 | (S)-(3-chloro-1-methyl-1H-pyrazol-5-yl)(4-(5-fluorobenzo[d]oxazol-2-yl)-6,7-dihydro-1H-imidazo[4,5-c]pyridin-5(4H)-yl)methanone |
| 129 | 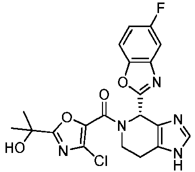 | (S)-(4-chloro-2-(2-hydroxypropan-2-yl)oxazol-5-yl)(4-(5-fluorobenzo[d]oxazol-2-yl)-6,7-dihydro-1H-imidazo[4,5-c]pyridin-5(4H)-yl)methanone |
| 131 | 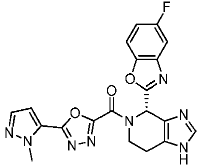 | (S)-(4-(5-fluorobenzo[d]oxazol-2-yl)-6,7-dihydro-1H-imidazo[4,5-c]pyridin-5(4H)-yl)(5-(1-methyl-1H-pyrazol-5-yl)-1,3,4-oxadiazol-2-yl)methanone |
| 133 | 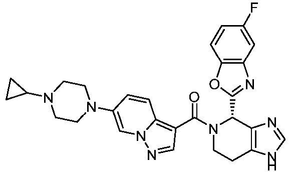 | (S)-(6-(4-cyclopropylpiperazin-1-yl)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 135 | 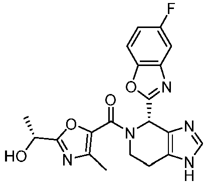 | ((S)-4-(5-fluorobenzo[d]oxazol-2-yl)-6,7-dihydro-1H-imidazo[4,5-c]pyridin-5(4H)-yl)(2-((R)-1-hydroxyethyl)-4-methyloxazol-5-yl)methanone |
| 137 | 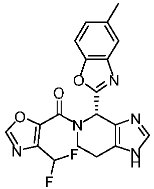 | (S)-(4-(difluoromethyl)oxazol-5-yl)(4-(5-methylbenzo[d]oxazol-2-yl)-6,7-dihydro-1H-imidazo[4,5-c]pyridin-5(4H)-yl)methanone |
| 139 | 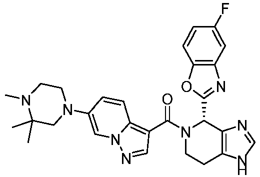 | (S)-(4-(5-fluorobenzo[d]oxazol-2-yl)-6,7-dihydro-1H-imidazo[4,5-c]pyridin-5(4H)-yl)(6-(3,3,4-trimethylpiperazin-1-yl)pyrazolo[1,5-a]pyridin-3-yl)methanone |
| 141 | 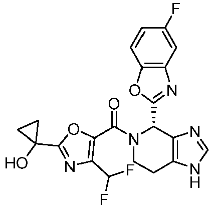 | (S)-(4-(difluoromethyl)-2-(1-hydroxycyclopropyl)oxazol-5-yl)(4-(5-fluorobenzo[d]oxazol-2-yl)-6,7-dihydro-1H-imidazo[4,5-c]pyridin-5(4H)-yl)methanone |
| 143 | 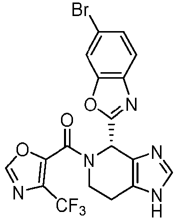 | (S)-(4-(6-bromobenzo[d]oxazol-2-yl)-6,7-dihydro-1H-imidazo[4,5-c]pyridin-5(4H)-yl)(4-(trifluoromethyl)oxazol-5-yl)methanone |
| 145 | 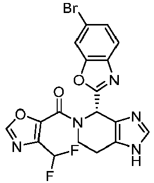 | (S)-(4-(6-bromobenzo[d]oxazol-2-yl)-6,7-dihydro-1H-imidazo[4,5-c]pyridin-5(4H)-yl)(4-(difluoromethyl)oxazol-5-yl)methanone |
| 147 | 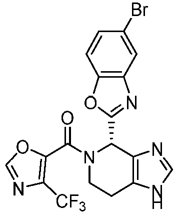 | (S)-(4-(5-bromobenzo[d]oxazol-2-yl)-6,7-dihydro-1H-imidazo[4,5-c]pyridin-5(4H)-yl)(4-(trifluoromethyl)oxazol-5-yl)methanone |
| 149 | 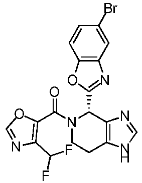 | (S)-(4-(5-bromobenzo[d]oxazol-2-yl)-6,7-dihydro-1H-imidazo[4,5-c]pyridin-5(4H)-yl)(4-(difluoromethyl)oxazol-5-yl)methanone |
| 151 | 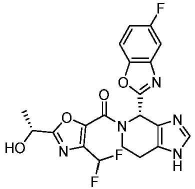 | (4-(difluoromethyl)-2-((R)-1-hydroxyethyl)oxazol-5-yl)((S)-4-(5-fluorobenzo[d]oxazol-2-yl)-6,7-dihydro-1H-imidazo[4,5-c]pyridin-5(4H)-yl)methanone |
| 153 | 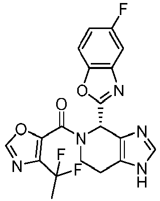 | (S)-(4-(1,1-difluoroethyl)oxazol-5-yl)(4-(5-fluorobenzo[d]oxazol-2-yl)-6,7-dihydro-1H-imidazo[4,5-c]pyridin-5(4H)-yl)methanone |
| 155 | 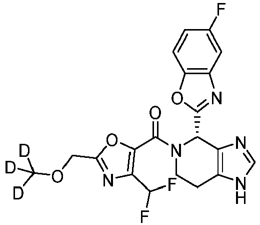 | (S)-(4-(difluoromethyl)-2-((methoxy-d3)methyl)oxazol-5-yl)(4-(5-fluorobenzo[d]oxazol-2-yl)-1,4,6,7-tetrahydro-5H-imidazo[4,5-c]pyridin-5-yl)methanone |
| 157 | 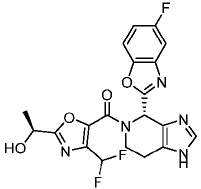 | (4-(difluoromethyl)-2-((S)-1-hydroxyethyl)oxazol-5-yl)((S)-4-(5-fluorobenzo[d]oxazol-2-yl)-6,7-dihydro-1H-imidazo[4,5-c]pyridin-5(4H)-yl)methanone |
| 159 | 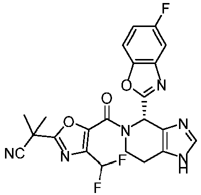 | (S)-2-(4-(difluoromethyl)-5-(4-(5-fluorobenzo[d]oxazol-2-yl)-4,5,6,7-tetrahydro-1H-imidazo[4,5-c]pyridine-5-carbonyl)oxazol-2-yl)-2-methylpropanenitrile |
| 161 | 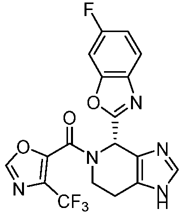 | (S)-(4-(6-fluorobenzo[d]oxazol-2-yl)-6,7-dihydro-1H-imidazo[4,5-c]pyridin-5(4H)-yl)(4-(trifluoromethyl)oxazol-5-yl)methanone |
| 163 | 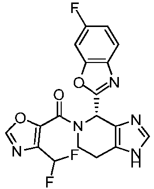 | (S)-(4-(difluoromethyl)oxazol-5-yl)(4-(6-fluorobenzo[d]oxazol-2-yl)-6,7-dihydro-1H-imidazo[4,5-c]pyridin-5(4H)-yl)methanone |
| 165 | 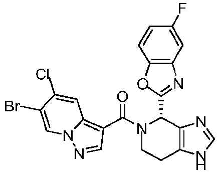 | (S)-(6-bromo-5-chloropyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 167 | 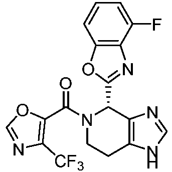 | (S)-(4-(4-fluorobenzo[d]oxazol-2-yl)-6,7-dihydro-1H-imidazo[4,5-c]pyridin-5(4H)-yl)(4-(trifluoromethyl)oxazol-5-yl)methanone |
| 169 | 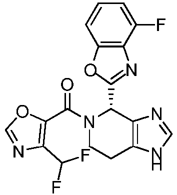 | (S)-(4-(difluoromethyl)oxazol-5-yl)(4-(4-fluorobenzo[d]oxazol-2-yl)-6,7-dihydro-1H-imidazo[4,5-c]pyridin-5(4H)-yl)methanone |
| 171 | 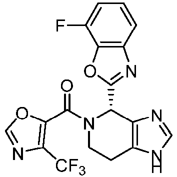 | (S)-(4-(7-fluorobenzo[d]oxazol-2-yl)-6,7-dihydro-1H-imidazo[4,5-c]pyridin-5(4H)-yl)(4-(trifluoromethyl)oxazol-5-yl)methanone |
| 173 | 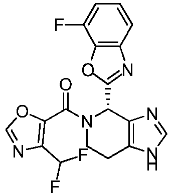 | (S)-(4-(difluoromethyl)oxazol-5-yl)(4-(7-fluorobenzo[d]oxazol-2-yl)-6,7-dihydro-1H-imidazo[4,5-c]pyridin-5(4H)-yl)methanone |
| 175 | 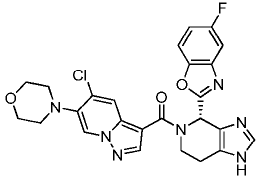 | (S)-(5-chloro-6-morpholinopyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 177 | 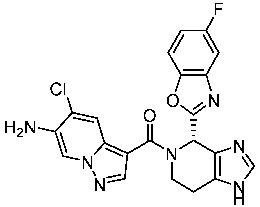 | (S)-(6-amino-5-chloropyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 179 | 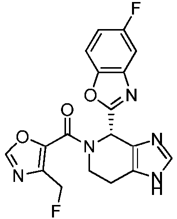 | (S)-(4-(5-fluorobenzo[d]oxazol-2-yl)-6,7-dihydro-1H-imidazo[4,5-c]pyridin-5(4H)-yl)(4-(fluoromethyl)oxazol-5-yl)methanone |
| 181 | 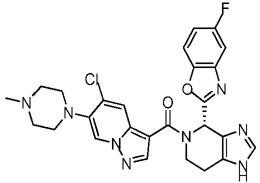 | (S)-(5-chloro-6-(4-methylpiperazin-1-yl)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 183 | 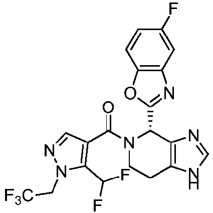 | (S)-(5-(difluoromethyl)-1-(2,2,2-trifluoroethyl)-1H-pyrazol-4-yl)(4-(5-fluorobenzo[d]oxazol-2-yl)-6,7-dihydro-1H-imidazo[4,5-c]pyridin-5(4H)-yl)methanone |
| 185 | 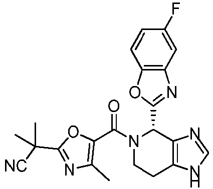 | (S)-2-(5-(4-(5-fluorobenzo[d]oxazol-2-yl)-4,5,6,7-tetrahydro-1H-imidazo[4,5-c]pyridine-5-carbonyl)-4-methyloxazol-2-yl)-2-methylpropanenitrile |
| 187 | 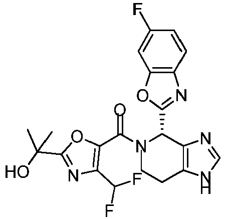 | (S)-(4-(difluoromethyl)-2-(2-hydroxypropan-2-yl)oxazol-5-yl)(4-(6-fluorobenzo[d]oxazol-2-yl)-6,7-dihydro-1H-imidazo[4,5-c]pyridin-5(4H)-yl)methanone |
| 189 | 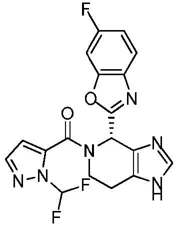 | (S)-(1-(difluoromethyl)- 1H-pyrazol-5-yl)(4-(6-fluorobenzo[d]oxazol-2-yl)-6,7-dihydro-1H-imidazo[4,5-c]pyridin-5(4H)-yl)methanone |
| 191 | 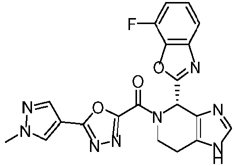 | (S)-(4-(7-fluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 193 | 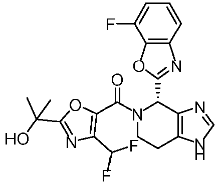 | (S)-(4-(difluoromethyl)-2-(2-hydroxypropan-2-yl)oxazol-5-yl)(4-(7-fluorobenzo[d]oxazol-2-yl)-6,7-dihydro-1H-imidazo[4,5-c]pyridin-5(4H)-yl)methanone |
| 195 | 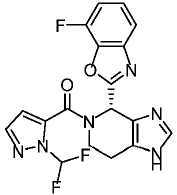 | (S)-(1-(difluoromethyl)- 1H-pyrazol-5-yl)(4-(7-fluorobenzo[d]oxazol-2-yl)-6,7-dihydro-1H-imidazo[4,5-c]pyridin-5(4H)-yl)methanone |
| 197 | 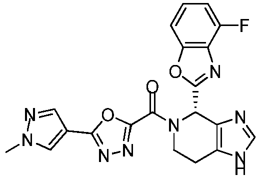 | (S)-(4-(4-fluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 199 | 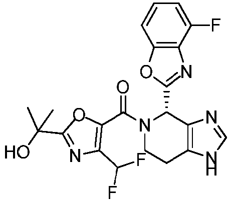 | (S)-(4-(difluoromethyl)-2-(2-hydroxypropan-2-yl)oxazol-5-yl)(4-(4-fluorobenzo[d]oxazol-2-yl)-6,7-dihydro-1H-imidazo[4,5-c]pyridin-5(4H)-yl)methanone |
| 201 | 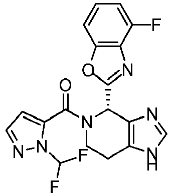 | (S)-(1-(difluoromethyl)-1H-pyrazol-5-yl)(4-(4-fluorobenzo[d]oxazol-2-yl)-6,7-dihydro-1H-imidazo[4,5-c]pyridin-5(4H)-yl)methanone |
| 203 | 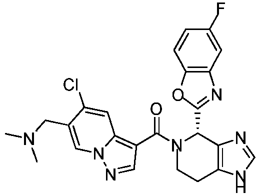 | (S)-(5-chloro-6-((dimethylamino)methyl)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanonee |
| 205 | 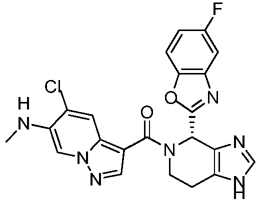 | (S)-(5-chloro-6-(methylamino)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 207 | 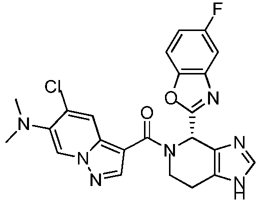 | (S)-(5-chloro-6-(dimethylamino)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 210 | 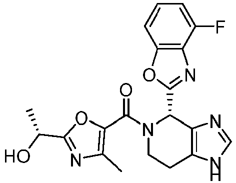 | ((S)-4-(4-fluorobenzo[d]oxazol-2-yl)-6,7-dihydro-1H-imidazo[4,5-c]pyridin-5(4H)-yl)(2-((R)-1-hydroxyethyl)-4-methyloxazol-5-yl)methanone |
| 211 | 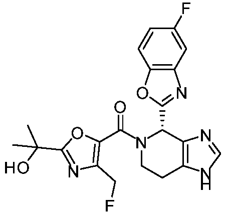 | (S)-(4-(5-fluorobenzo[d]oxazol-2-yl)-6,7-dihydro-1H-imidazo[4,5-c]pyridin-5(4H)-yl)(4-(fluoromethyl)-2-(2-hydroxypropan-2-yl)oxazol-5-yl)methanone |
| 214 | 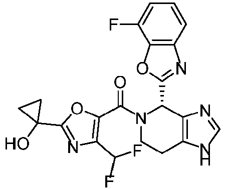 | (S)-(4-(difluoromethyl)-2-(1-hydroxycyclopropyl)oxazol-5-yl)(4-(7-fluorobenzo[d]oxazol-2-yl)-6,7-dihydro-1H-imidazo[4,5-c]pyridin-5(4H)-yl)methanone |
| 215 | 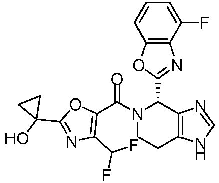 | (S)-(4-(difluoromethyl)-2-(1-hydroxycyclopropyl)oxazol-5-yl)(4-(4-fluorobenzo[d]oxazol-2-yl)-6,7-dihydro-1H-imidazo[4,5-c]pyridin-5(4H)-yl)methanone |
| 217 | 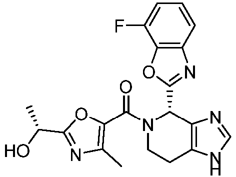 | ((S)-4-(7-fluorobenzo[d]oxazol-2-yl)-6,7-dihydro-1H-imidazo[4,5-c]pyridin-5(4H)-yl)(2-((R)-1-hydroxyethyl)-4-methyloxazol-5-yl)methanone |
| 219 | 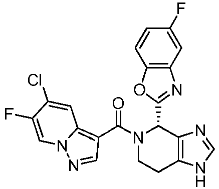 | (S)-(5-chloro-6-fluoropyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 221 | 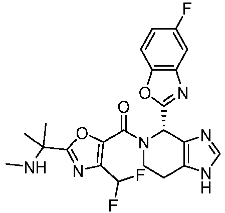 | (S)-(4-(difluoromethyl)-2-(2-(methylamino)propan-2-yl)oxazol-5-yl)(4-(5-fluorobenzo[d]oxazol-2-yl)-6,7-dihydro-1H-imidazo[4,5-c]pyridin-5(4H)-yl)methanone |
| 225 | 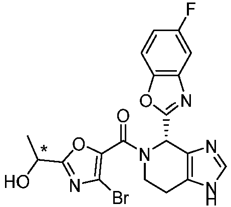 | (4-bromo-2-(1-hydroxyethyl)oxazol-5-yl)((S)-4-(5-fluorobenzo[d]oxazol-2-yl)-6,7-dihydro-1H-imidazo[4,5-c]pyridin-5(4H)-yl)methanone |
| | | Diastereomer #1 (Rt=4.793 min) |
| 226 | 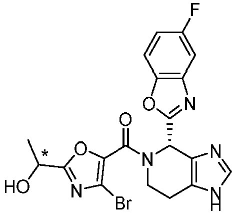 | (4-bromo-2-(1-hydroxyethyl)oxazol-5-yl)((S)-4-(5-fluorobenzo[d]oxazol-2-yl)-6,7-dihydro-1H-imidazo[4,5-c]pyridin-5(4H)-yl)methanone |
| | | Diastereomer #2 (Rt=5.231 min) |
| 227 | 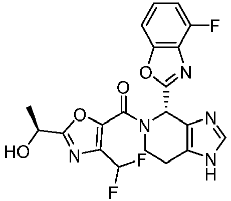 | (4-(difluoromethyl)-2-((S)-1-hydroxyethyl)oxazol-5-yl)((S)-4-(4-fluorobenzo[d]oxazol-2-yl)-6,7-dihydro-1H-imidazo[4,5-c]pyridin-5(4H)-yl)methanone |
| 229 | 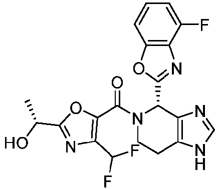 | (4-(difluoromethyl)-2-((R)-1-hydroxyethyl)oxazol-5-yl)((S)-4-(4-fluorobenzo[d]oxazol-2-yl)-6,7-dihydro-1H-imidazo[4,5-c]pyridin-5(4H)-yl)methanone |
| 231 | 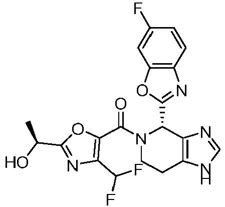 | (4-(difluoromethyl)-2-((S)-1-hydroxyethyl)oxazol-5-yl)((S)-4-(6-fluorobenzo[d]oxazol-2-yl)-6,7-dihydro-1H-imidazo[4,5-c]pyridin-5(4H)-yl)methanone |
| 235 | 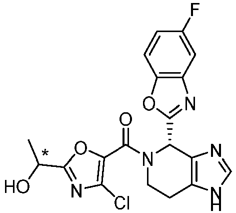 | (4-chloro-2-(1-hydroxyethyl)oxazol-5-yl)((S)-4-(5-fluorobenzo[d]oxazol-2-yl)-6,7-dihydro-1H-imidazo[4,5-c]pyridin-5(4H)-yl)methanone |
| | | Diastereomer #1 (Rt=4.258 min) |
| 236 | 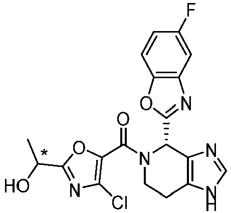 | (4-chloro-2-(1-hydroxyethyl)oxazol-5-yl)((S)-4-(5-fluorobenzo[d]oxazol-2-yl)-6,7-dihydro-1H-imidazo[4,5-c]pyridin-5(4H)-yl)methanone |
| | | Diastereomer #2 (Rt=4.417 min) |
| 237 | 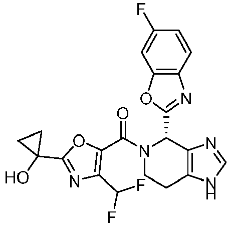 | (S)-(4-(difluoromethyl)-2-(1-hydroxycyclopropyl)oxazol-5-yl)(4-(6-fluorobenzo[d]oxazol-2-yl)-6,7-dihydro-1H-imidazo[4,5-c]pyridin-5(4H)-yl)methanone |
| 239 | 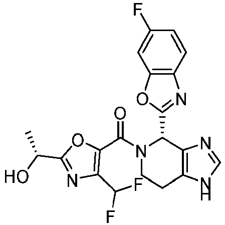 | (4-(difluoromethyl)-2-((R)-1-hydroxyethyl)oxazol-5-yl)((S)-4-(6-fluorobenzo[d]oxazol-2-yl)-6,7-dihydro-1H-imidazo[4,5-c]pyridin-5(4H)-yl)methanone |
| 241 | 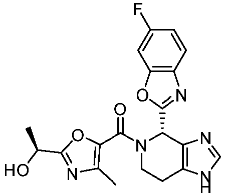 | ((S)-4-(6-fluorobenzo[d]oxazol-2-yl)-6,7-dihydro-1H-imidazo[4,5-c]pyridin-5(4H)-yl)(2-((S)-1-hydroxyethyl)-4-methyloxazol-5-yl)methanone |
| 243 | 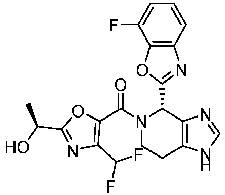 | (4-(difluoromethyl)-2-((S)-1-hydroxyethyl)oxazol-5-yl)((S)-4-(7-fluorobenzo[d]oxazol-2-yl)-6,7-dihydro-1H-imidazo[4,5-c]pyridin-5(4H)-yl)methanone |
| 245 | 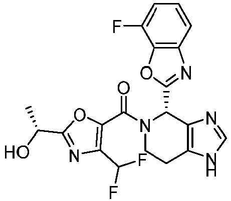 | (4-(difluoromethyl)-2-((R)-1-hydroxyethyl)oxazol-5-yl)((S)-4-(7-fluorobenzo[d]oxazol-2-yl)-6,7-dihydro-1H-imidazo[4,5-c]pyridin-5(4H)-yl)methanone |
| 247 | 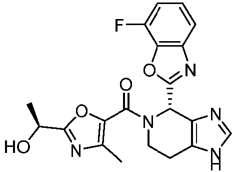 | ((S)-4-(7-fluorobenzo[d]oxazol-2-yl)-6,7-dihydro-1H-imidazo[4,5-c]pyridin-5(4H)-yl)(2-((S)-1-hydroxyethyl)-4-methyloxazol-5-yl)methanone |
| 249 | 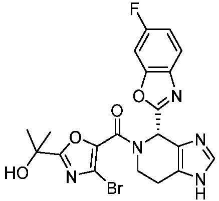 | (S)-(4-bromo-2-(2-hydroxypropan-2-yl)oxazol-5-yl)(4-(6-fluorobenzo[d]oxazol-2-yl)-6,7-dihydro-1H-imidazo[4,5-c]pyridin-5(4H)-yl)methanone |
| 252 | 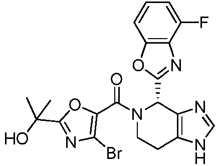 | (S)-(4-bromo-2-(2-hydroxypropan-2-yl)oxazol-5-yl)(4-(4-fluorobenzo[d]oxazol-2-yl)-6,7-dihydro-1H-imidazo[4,5-c]pyridin-5(4H)-yl)methanone |
| 253 | 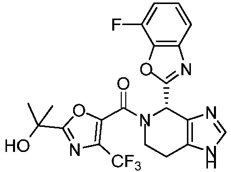 | (S)-(4-(7-fluorobenzo[d]oxazol-2-yl)-6,7-dihydro-1H-imidazo[4,5-c]pyridin-5(4H)-yl)(2-(2-hydroxypropan-2-yl)-4-(trifluoromethyl)oxazol-5-yl)methanone |
| 255 | 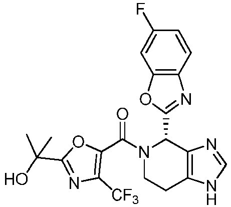 | (S)-(4-(6-fluorobenzo[d]oxazol-2-yl)-6,7-dihydro-1H-imidazo[4,5-c]pyridin-5(4H)-yl)(2-(2-hydroxypropan-2-yl)-4-(trifluoromethyl)oxazol-5-yl)methanone |
| 257 | 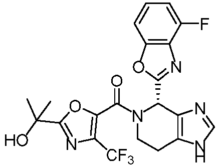 | (S)-(4-(4-fluorobenzo[d]oxazol-2-yl)-6,7-dihydro-1H-imidazo[4,5-c]pyridin-5(4H)-yl)(2-(2-hydroxypropan-2-yl)-4-(trifluoromethyl)oxazol-5-yl)methanone |
| 259 | 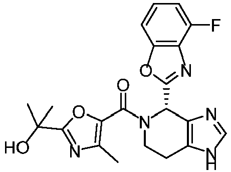 | (S)-(4-(4-fluorobenzo[d]oxazol-2-yl)-6,7-dihydro-1H-imidazo[4,5-c]pyridin-5(4H)-yl)(2-(2-hydroxypropan-2-yl)-4-methyloxazol-5-yl)methanone |
| 261 | 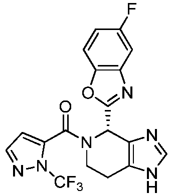 | (S)-(4-(5-fluorobenzo[d]oxazol-2-yl)-6,7-dihydro-1H-imidazo[4,5-c]pyridin-5(4H)-yl)(1-(trifluoromethyl)-1H-pyrazol-5-yl)methanone |
| 263 | 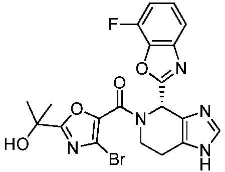 | (S)-(4-bromo-2-(2-hydroxypropan-2-yl)oxazol-5-yl)(4-(7-fluorobenzo[d]oxazol-2-yl)-6,7-dihydro-1H-imidazo[4,5-c]pyridin-5(4H)-yl)methanone |
| 265 | 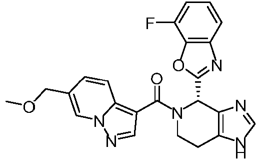 | (S)-(4-(7-fluorobenzo[d]oxazol-2-yl)-6,7-dihydro-1H-imidazo[4,5-c]pyridin-5(4H)-yl)(6-(methoxymethyl)pyrazolo[1,5-a]pyridin-3-yl)methanone |
| 268 | 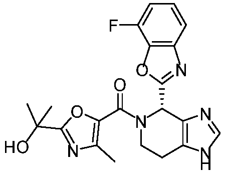 | (S)-(4-(7-fluorobenzo[d]oxazol-2-yl)-6,7-dihydro-1H-imidazo[4,5-c]pyridin-5(4H)-yl)(2-(2-hydroxypropan-2-yl)-4-methyloxazol-5-yl)methanone |
| 269 | 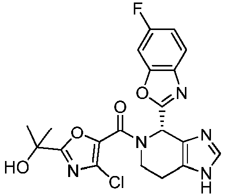 | (S)-(4-chloro-2-(2-hydroxypropan-2-yl)oxazol-5-yl)(4-(6-fluorobenzo[d]oxazol-2-yl)-6,7-dihydro-1H-imidazo[4,5-c]pyridin-5(4H)-yl)methanone |
| 272 | 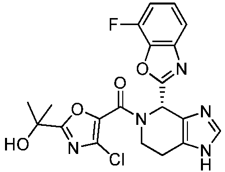 | (S)-(4-chloro-2-(2-hydroxypropan-2-yl)oxazol-5-yl)(4-(7-fluorobenzo[d]oxazol-2-yl)-6,7-dihydro-1H-imidazo[4,5-c]pyridin-5(4H)-yl)methanone |
| 274 | 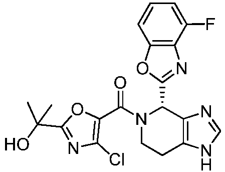 | (S)-(4-chloro-2-(2-hydroxypropan-2-yl)oxazol-5-yl)(4-(4-fluorobenzo[d]oxazol-2-yl)-6,7-dihydro-1H-imidazo[4,5-c]pyridin-5(4H)-yl)methanone |
| 275 | 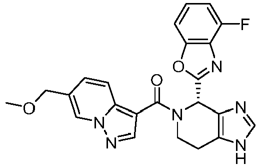 | (S)-(4-(4-fluorobenzo[d]oxazol-2-yl)-6,7-dihydro-1H-imidazo[4,5-c]pyridin-5(4H)-yl)(6-(methoxymethyl)pyrazolo[1,5-a]pyridin-3-yl)methanone |
| 277 | 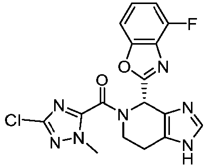 | (S)-(3-chloro-1-methyl-1H-1,2,4-triazol-5-yl)(4-(4-fluorobenzo[d]oxazol-2-yl)-6,7-dihydro-1H-imidazo[4,5-c]pyridin-5(4H)-yl)methanone |
| 279 | 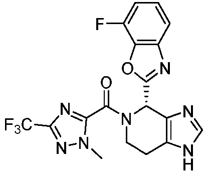 | (S)-(4-(7-fluorobenzo[d]oxazol-2-yl)-6,7-dihydro-1H-imidazo[4,5-c]pyridin-5(4H)-yl)(1-methyl-3-(trifluoromethyl)-1H-1,2,4-triazol-5-yl)methanone |
| 281 | 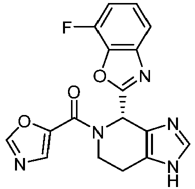 | (S)-(4-(7-fluorobenzo[d]oxazol-2-yl)-6,7-dihydro-1H-imidazo[4,5-c]pyridin-5(4H)-yl)(oxazol-5-yl)methanone |
| 283 | 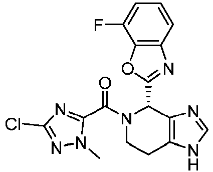 | (S)-(3-chloro-1-methyl-1H-1,2,4-triazol-5-yl)(4-(7-fluorobenzo[d]oxazol-2-yl)-6,7-dihydro-1H-imidazo[4,5-c]pyridin-5(4H)-yl)methanone |
| 285 | 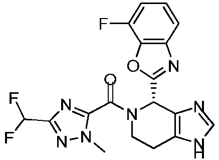 | (S)-(3-(difluoromethyl)-1-methyl-1H-1,2,4-triazol-5-yl)(4-(7-fluorobenzo[d]oxazol-2-yl)-6,7-dihydro-1H-imidazo[4,5-c]pyridin-5(4H)-yl)methanone |
| 287 | 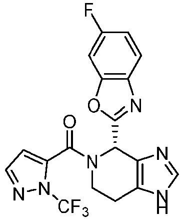 | (S)-(4-(6-fluorobenzo[d]oxazol-2-yl)-6,7-dihydro-1H-imidazo[4,5-c]pyridin-5(4H)-yl)(1-(trifluoromethyl)-1H-pyrazol-5-yl)methanone |
| 289 | 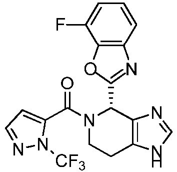 | (S)-(4-(7-fluorobenzo[d]oxazol-2-yl)-6,7-dihydro-1H-imidazo[4,5-c]pyridin-5(4H)-yl)(1-(trifluoromethyl)-1H-pyrazol-5-yl)methanone |
| 291 | 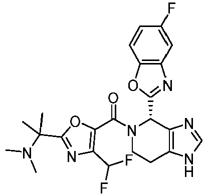 | (S)-(4-(difluoromethyl)-2-(2-(dimethylamino)propan-2-yl)oxazol-5-yl)(4-(5-fluorobenzo[d]oxazol-2-yl)-6,7-dihydro-1H-imidazo[4,5-c]pyridin-5(4H)-yl)methanone |
| 293 | 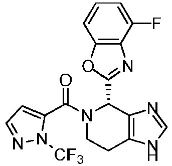 | (S)-(4-(4-fluorobenzo[d]oxazol-2-yl)-6,7-dihydro-1H-imidazo[4,5-c]pyridin-5(4H)-yl)(1-(trifluoromethyl)-1H-pyrazol-5-yl)methanone |
| 295 | 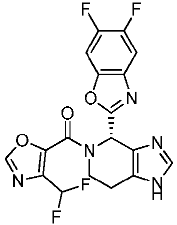 | (S)-(4-(5,6-difluorobenzo[d]oxazol-2-yl)-6,7-dihydro-1H-imidazo[4,5-c]pyridin-5(4H)-yl)(4-(difluoromethyl)oxazol-5-yl)methanone |
| 297 | 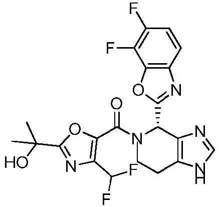 | (S)-(4-(6,7-difluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 299 | 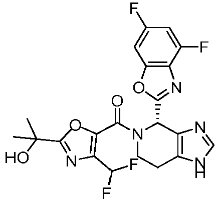 | (S)-(4-(4,6-difluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 301 | 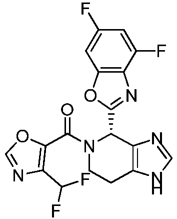 | (S)-(4-(4,6-difluorobenzo[d]oxazol-2-yl)-6,7-dihydro-1H-imidazo[4,5-c]pyridin-5(4H)-yl)(4-(difluoromethyl)oxazol-5-yl)methanone |
| 303 | 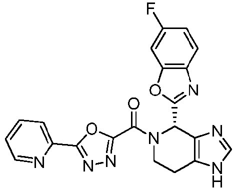 | (S)-(4-(6-fluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 305 | 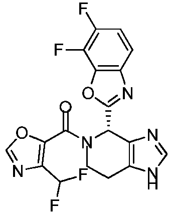 | (S)-(4-(6,7-difluorobenzo[d]oxazol-2-yl)-6,7-dihydro-1H-imidazo[4,5-c]pyridin-5(4H)-yl)(4-(difluoromethyl)oxazol-5-yl)methanone |
| 307 | 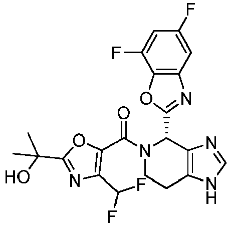 | (S)-(4-(5,7-difluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 309 | 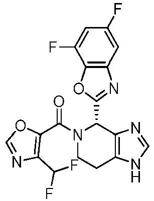 | (S)-(4-(5,7-difluorobenzo[d]oxazol-2-yl)-6,7-dihydro-1H-imidazo[4,5-c]pyridin-5(4H)-yl)(4-(difluoromethyl)oxazol-5-yl)methanone |
| 311 | 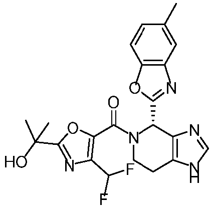 | (S)-(4-(difluoromethyl)-2-(2-hydroxypropan-2-yl)oxazol-5-yl)(4-(5-methylbenzo[d]oxazol-2-yl)-6,7-dihydro-1H-imidazo[4,5-c]pyridin-5(4H)-yl)methanone |
| 313 | 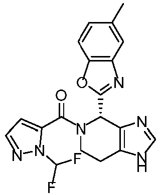 | (S)-(1-(difluoromethyl)- 1H-pyrazol-5-yl)(4-(5-methylbenzo[d]oxazol-2-yl)-6,7-dihydro-1H-imidazo[4,5-c]pyridin-5(4H)-yl)methanone |
| 315 | 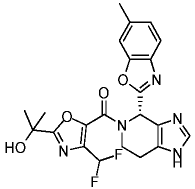 | (S)-(4-(difluoromethyl)-2-(2-hydroxypropan-2-yl)oxazol-5-yl)(4-(6-methylbenzo[d]oxazol-2-yl)-6,7-dihydro-1H-imidazo[4,5-c]pyridin-5(4H)-yl)methanone |
| 317 | 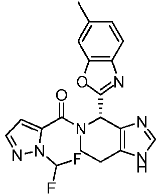 | (S)-(1-(difluoromethyl)- 1H-pyrazol-5-yl)(4-(6-methylbenzo[d]oxazol-2-yl)-6,7-dihydro-1H-imidazo[4,5-c]pyridin-5(4H)-yl)methanone |
| 319 | 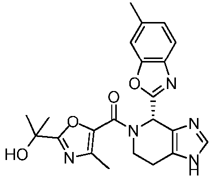 | (S)-(2-(2-hydroxypropan-2-yl)-4-methyloxazol-5-yl)(4-(6-methylbenzo[d]oxazol-2-yl)-6,7-dihydro-1H-imidazo[4,5-c]pyridin-5(4H)-yl)methanone |
| 321 | 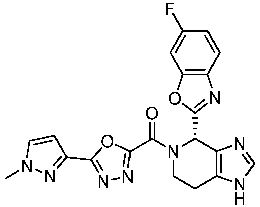 | (S)-(4-(6-fluorobenzo[d]oxazol-2-yl)-6,7-dihydro-1H-imidazo[4,5-c]pyridin-5(4H)-yl)(5-(1-methyl-1H-pyrazol-3-yl)-1,3,4-oxadiazol-2-yl)methanone |
| 323 | 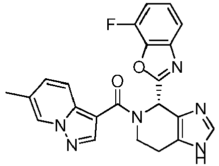 | (S)-(4-(7-fluorobenzo[d]oxazol-2-yl)-6,7-dihydro-1H-imidazo[4,5-c]pyridin-5(4H)-yl)(6-methylpyrazolo[1,5-a]pyridin-3-yl)methanone |
| 325 | 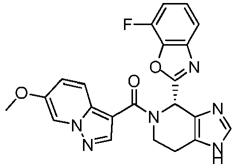 | (S)-(4-(7-fluorobenzo[d]oxazol-2-yl)-6,7-dihydro-1H-imidazo[4,5-c]pyridin-5(4H)-yl)(6-methoxypyrazolo[1,5-a]pyridin-3-yl)methanone |
| 327 | 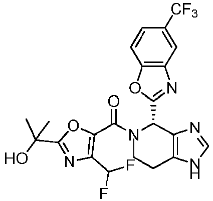 | (S)-(4-(difluoromethyl)-2-(2-hydroxypropan-2-yl)oxazol-5-yl)(4-(5-(trifluoromethyl)benzo[d]oxazol-2-yl)-6,7-dihydro-1H-imidazo[4,5-c]pyridin-5(4H)-yl)methanone |
| 329 | 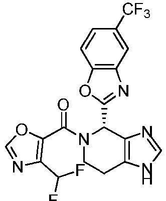 | (S)-(4-(difluoromethyl)oxazol-5-yl)(4-(5-(trifluoromethyl)benzo[d]oxazol-2-yl)-6,7-dihydro-1H-imidazo[4,5-c]pyridin-5(4H)-yl)methanone |
| 331 | 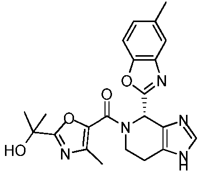 | (S)-(2-(2-hydroxypropan-2-yl)-4-methyloxazol-5-yl)(4-(5-methylbenzo[d]oxazol-2-yl)-6,7-dihydro-1H-imidazo[4,5-c]pyridin-5(4H)-yl)methanone |
| 333 | 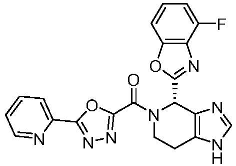 | (S)-(4-(4-fluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 335 | 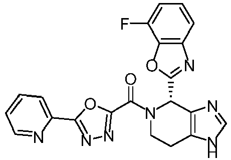 | (S)-(4-(7-fluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 337 | 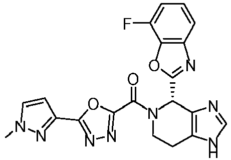 | (S)-(4-(7-fluorobenzo[d]oxazol-2-yl)-6,7-dihydro-1H-imidazo[4,5-c]pyridin-5(4H)-yl)(5-(1-methyl-1H-pyrazol-3-yl)-1,3,4-oxadiazol-2-yl)methanone |
| 339 | 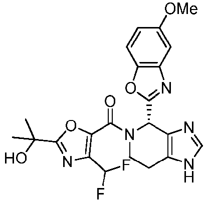 | (S)-(4-(difluoromethyl)-2-(2-hydroxypropan-2-yl)oxazol-5-yl)(4-(5-methoxybenzo[d]oxazol-2-yl)-6,7-dihydro-1H-imidazo[4,5-c]pyridin-5(4H)-yl)methanone |
| 341 | 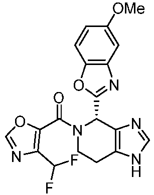 | (S)-(4-(difluoromethyl)oxazol-5-yl)(4-(5-methoxybenzo[d]oxazol-2-yl)-6,7-dihydro-1H-imidazo[4,5-c]pyridin-5(4H)-yl)methanone |
| 343 | 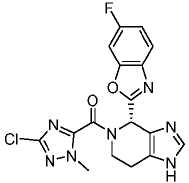 | (S)-(3-chloro-1-methyl-1H-1,2,4-triazol-5-yl)(4-(6-fluorobenzo[d]oxazol-2-yl)-6,7-dihydro-1H-imidazo[4,5-c]pyridin-5(4H)-yl)methanone |
| 345 | 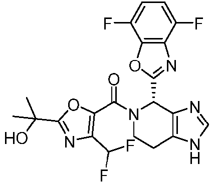 | (S)-(4-(4,7-difluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 347 | 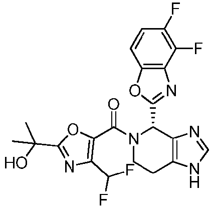 | (S)-(4-(4,5-difluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 350 | 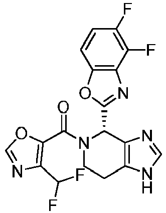 | (S)-(4-(4,5-difluorobenzo[d]oxazol-2-yl)-6,7-dihydro-1H-imidazo[4,5-c]pyridin-5(4H)-yl)(4-(difluoromethyl)oxazol-5-yl)methanone |
| 351 | 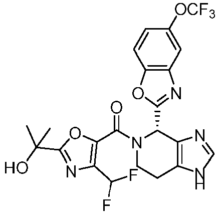 | (S)-(4-(difluoromethyl)-2-(2-hydroxypropan-2-yl)oxazol-5-yl)(4-(5-(trifluoromethoxy)benzo[d]oxazol-2-yl)-6,7-dihydro-1H-imidazo[4,5-c]pyridin-5(4H)-yl)methanone |
| 354 | 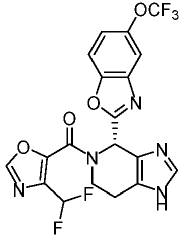 | (S)-(4-(difluoromethyl)oxazol-5-yl)(4-(5-(trifluoromethoxy)benzo[d]oxazol-2-yl)-6,7-dihydro-1H-imidazo[4,5-c]pyridin-5(4H)-yl)methanone |
| 355 | 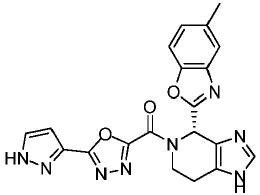 | (S)-(5-(1H-pyrazol-3-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 357 | 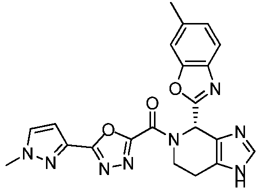 | (S)-(5-(1-methyl-1H-pyrazol-3-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 359 | 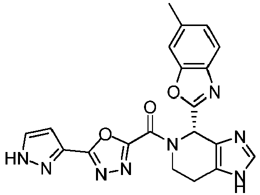 | (S)-(5-(1H-pyrazol-3-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 361 | 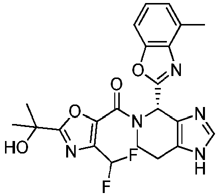 | (S)-(4-(difluoromethyl)-2-(2-hydroxypropan-2-yl)oxazol-5-yl)(4-(4-methylbenzo[d]oxazol-2-yl)-6,7-dihydro-1H-imidazo[4,5-c]pyridin-5(4H)-yl)methanone |
| 363 | 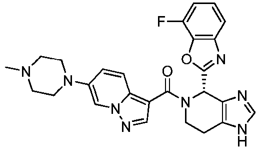 | (S)-(4-(7-fluorobenzo[d]oxazol-2-yl)-6,7-dihydro-1H-imidazo[4,5-c]pyridin-5(4H)-yl)(6-(4-methylpiperazin-1-yl)pyrazolo[1,5-a]pyridin-3-yl)methanone |
| 365 | 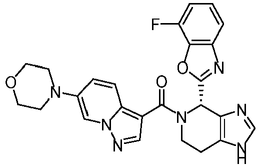 | (S)-(4-(7-fluorobenzo[d]oxazol-2-yl)-6,7-dihydro-1H-imidazo[4,5-c]pyridin-5(4H)-yl)(6-morpholinopyrazolo[1,5-a]pyridin-3-yl)methanone |
| 368 | 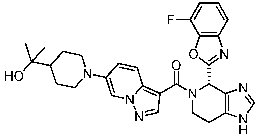 | (S)-(4-(7-fluorobenzo[d]oxazol-2-yl)-6,7-dihydro-1H-imidazo[4,5-c]pyridin-5(4H)-yl)(6-(4-(2-hydroxypropan-2-yl)piperidin-1-yl)pyrazolo[1,5-a]pyridin-3-yl)methanone |
| 369 | 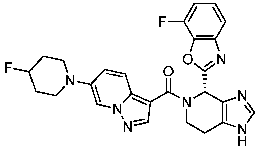 | (S)-(4-(7-fluorobenzo[d]oxazol-2-yl)-6,7-dihydro-1H-imidazo[4,5-c]pyridin-5(4H)-yl)(6-(4-fluoropiperidin-1-yl)pyrazolo[1,5-a]pyridin-3-yl)methanone |
| 371 | 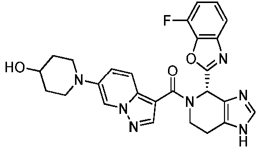 | (S)-(4-(7-fluorobenzo[d]oxazol-2-yl)-6,7-dihydro-1H-imidazo[4,5-c]pyridin-5(4H)-yl)(6-(4-hydroxypiperidin-1-yl)pyrazolo[1,5-a]pyridin-3-yl)methanone |
| 373 | 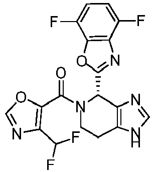 | (S)-(4-(4,7-difluorobenzo[d]oxazol-2-yl)-6,7-dihydro-1H-imidazo[4,5-c]pyridin-5(4H)-yl)(4-(difluoromethyl)oxazol-5-yl)methanone |
| 375 | 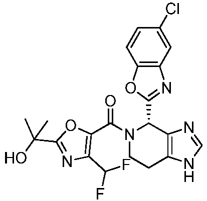 | (S)-(4-(5-chlorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 378 | 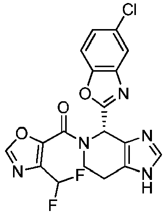 | (S)-(4-(5-chlorobenzo[d]oxazol-2-yl)-6,7-dihydro-1H-imidazo[4,5-c]pyridin-5(4H)-yl)(4-(difluoromethyl)oxazol-5-yl)methanone |
| 379 | 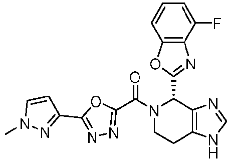 | (S)-(4-(4-fluorobenzo[d]oxazol-2-yl)-1,4,6,7-tetrahydro-5H-imidazo[4,5-c]pyridin-5-yl)(5-(1-methyl-1H-pyrazol-3-yl)-1,3,4-oxadiazol-2-yl)methanone |
| 382 | 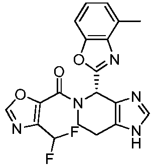 | (S)-(4-(difluoromethyl)oxazol-5-yl)(4-(4-methylbenzo[d]oxazol-2-yl)-6,7-dihydro-1H-imidazo[4,5-c]pyridin-5(4H)-yl)methanone |
| 383 | 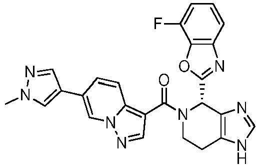 | (S)-(4-(7-fluorobenzo[d]oxazol-2-yl)-6,7-dihydro-1H-imidazo[4,5-c]pyridin-5(4H)-yl)(6-(1-methyl-1H-pyrazol-4-yl)pyrazolo[1,5-a]pyridin-3-yl)methanone |
| 385 | 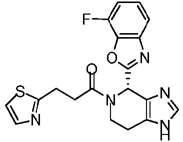 | 1-[(4S)-4-(7-fluoro-1,3-benzoxazol-2-yl)-1,4,6,7-tetrahydroimidazo[4,5-c]pyridin-5-yl]-3-thiazol-2-yl-propan-1-one |
| 387 | 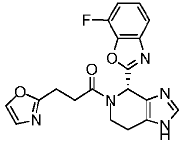 | (S)-1-(4-(7-fluorobenzo[d]oxazol-2-yl)-6,7-dihydro-1H-imidazo[4,5-c]pyridin-5(4H)-yl)-3-(oxazol-2-yl)propan-1-one |
| 389 | 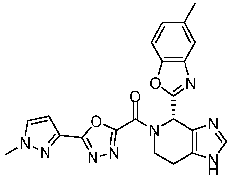 | (S)-(5-(1-methyl-1H-pyrazol-3-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 392 | 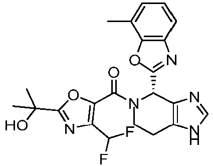 | (S)-(4-(difluoromethyl)-2-(2-hydroxypropan-2-yl)oxazol-5-yl)(4-(7-methylbenzo[d]oxazol-2-yl)-6,7-dihydro-1H-imidazo[4,5-c]pyridin-5(4H)-yl)methanone |
| 393 | 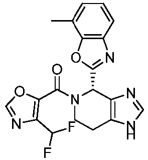 | (S)-(4-(difluoromethyl)oxazol-5-yl)(4-(7-methylbenzo[d]oxazol-2-yl)-6,7-dihydro-1H-imidazo[4,5-c]pyridin-5(4H)-yl)methanone |
| 395 | 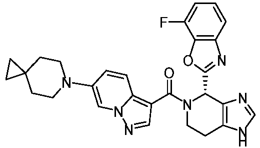 | (S)-(6-(6-azaspiro[2.5]octan-6-yl)pyrazolo[1,5-a]pyridin-3-yl)(4-(7-fluorobenzo[d]oxazol-2-yl)-6,7-dihydro-1H-imidazo[4,5-c]pyridin-5(4H)-yl)methanone |
| 397 | 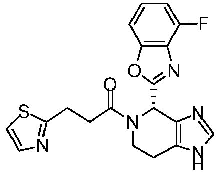 | (S)-1-(4-(4-fluorobenzo[d]oxazol-2-yl)-6,7-dihydro-1H-imidazo[4,5-c]pyridin-5(4H)-yl)-3-(thiazol-2-yl)propan-1-one |
| 399 | 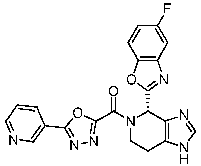 | (S)-(4-(5-fluorobenzo[d]oxazol-2-yl)-6,7-dihydro-1H-imidazo[4,5-c]pyridin-5(4H)-yl)(5-(pyridin-3-yl)-1,3,4-oxadiazol-2-yl)methanone |
| 401 | 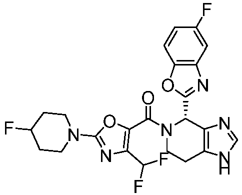 | (S)-(4-(difluoromethyl)-2-(4-fluoropiperidin-1-yl)oxazol-5-yl)(4-(5-fluorobenzo[d]oxazol-2-yl)-6,7-dihydro-1H-imidazo[4,5-c]pyridin-5(4H)-yl)methanone |
| 403 | 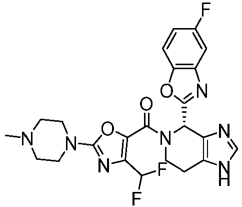 | (S)-(4-(difluoromethyl)-2-(4-methylpiperazin-1-yl)oxazol-5-yl)(4-(5-fluorobenzo[d]oxazol-2-yl)-6,7-dihydro-1H-imidazo[4,5-c]pyridin-5(4H)-yl)methanone |
| 406 | 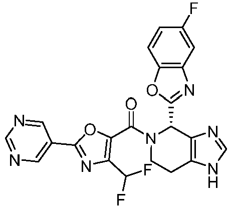 | (S)-(4-(difluoromethyl)-2-(pyrimidin-5-yl)oxazol-5-yl)(4-(5-fluorobenzo[d]oxazol-2-yl)-6,7-dihydro-1H-imidazo[4,5-c]pyridin-5(4H)-yl)methanone |
| 407 | 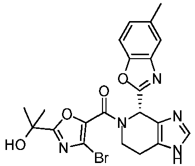 | (S)-(4-bromo-2-(2-hydroxypropan-2-yl)oxazol-5-yl)(4-(5-methylbenzo[d]oxazol-2-yl)-6,7-dihydro-1H-imidazo[4,5-c]pyridin-5(4H)-yl)methanone |
| 409 | 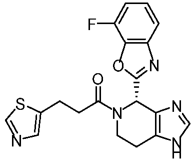 | (S)-1-(4-(7-fluorobenzo[d]oxazol-2-yl)-6,7-dihydro-1H-imidazo[4,5-c]pyridin-5(4H)-yl)-3-(thiazol-5-yl)propan-1-one |
| 411 | 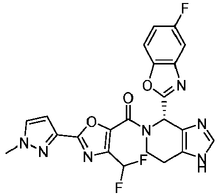 | (S)-(4-(difluoromethyl)-2-(1-methyl-1H-pyrazol-3-yl)oxazol-5-yl)(4-(5-fluorobenzo[d]oxazol-2-yl)-6,7-dihydro-1H-imidazo[4,5-c]pyridin-5(4H)-yl)methanone |
| 413 | 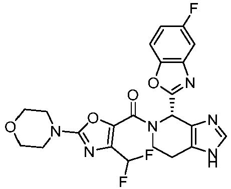 | (S)-(4-(difluoromethyl)-2-morpholinooxazol-5-yl)(4-(5-fluorobenzo[d]oxazol-2-yl)-6,7-dihydro-1H-imidazo[4,5-c]pyridin-5(4H)-yl)methanone |
| 415 | 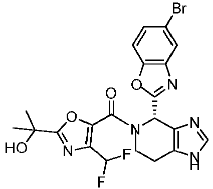 | [(4S)-4-(5-bromo-1,3-benzoxazol-2-yl)-1,4,6,7-tetrahydroimidazo[4,5-c]pyridin-5-yl]-[4-(difluoromethyl)-2-(1-hydroxy-1-methyl-ethyl)oxazol-5-yl]methanone |
| 418 | 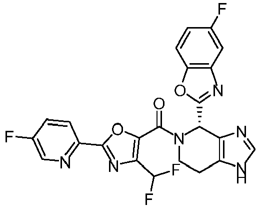 | (S)-(4-(difluoromethyl)-2-(5-fluoropyridin-2-yl)oxazol-5-yl)(4-(5-fluorobenzo[d]oxazol-2-yl)-6,7-dihydro-1H-imidazo[4,5-c]pyridin-5(4H)-yl)methanone |
| 420 | 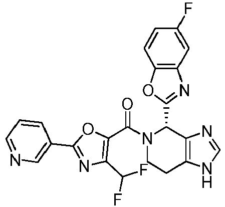 | (S)-(4-(difluoromethyl)-2-(pyridin-3-yl)oxazol-5-yl)(4-(5-fluorobenzo[d]oxazol-2-yl)-6,7-dihydro-1H-imidazo[4,5-c]pyridin-5(4H)-yl)methanone |
| 421 | 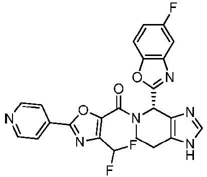 | (S)-(4-(difluoromethyl)-2-(pyridin-4-yl)oxazol-5-yl)(4-(5-fluorobenzo[d]oxazol-2-yl)-6,7-dihydro-1H-imidazo[4,5-c]pyridin-5(4H)-yl)methanone |
| 423 | 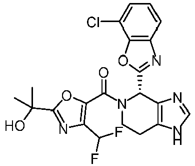 | (S)-(4-(7-chlorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 425 | 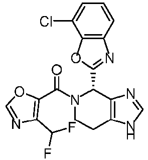 | (S)-(4-(7-chlorobenzo[d]oxazol-2-yl)-6,7-dihydro-1H-imidazo[4,5-c]pyridin-5(4H)-yl)(4-(difluoromethyl)oxazol-5-yl)methanone |
| 427 | 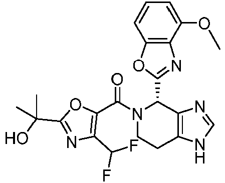 | (S)-(4-(difluoromethyl)-2-(2-hydroxypropan-2-yl)oxazol-5-yl)(4-(4-methoxybenzo[d]oxazol-2-yl)-6,7-dihydro-1H-imidazo[4,5-c]pyridin-5(4H)-yl)methanone |
| 429 | 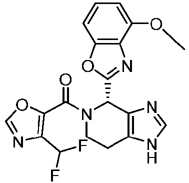 | (S)-(4-(difluoromethyl)oxazol-5-yl)(4-(4-methoxybenzo[d]oxazol-2-yl)-6,7-dihydro-1H-imidazo[4,5-c]pyridin-5(4H)-yl)methanone |
| 431 | 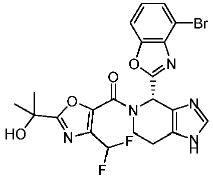 | (S)-(4-(4-bromobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 433 | 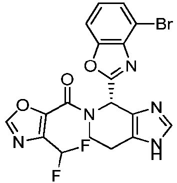 | (S)-(4-(4-bromobenzo[d]oxazol-2-yl)-6,7-dihydro-1H-imidazo[4,5-c]pyridin-5(4H)-yl)(4-(difluoromethyl)oxazol-5-yl)methanone |
| 435 | 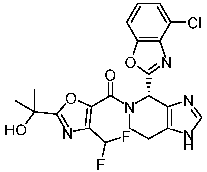 | [(4S)-4-(4-chloro-1,3-benzoxazol-2-yl)-1,4,6,7-tetrahydroimidazo[4,5-c]pyridin-5-yl]-[4-(difluoromethyl)-2-(1-hydroxy-1-methyl-ethyl)oxazol-5-yl]methanone |
| 437 | 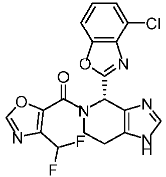 | [(4S)-4-(4-chloro-1,3-benzoxazol-2-yl)-1,4,6,7-tetrahydroimidazo[4,5-c]pyridin-5-yl]-[4-(difluoromethyl)oxazol-5-yl]methanone |
| 439 | 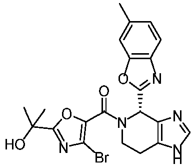 | (S)-(4-bromo-2-(2-hydroxypropan-2-yl)oxazol-5-yl)(4-(6-methylbenzo[d]oxazol-2-yl)-6,7-dihydro-1H-imidazo[4,5-c]pyridin-5(4H)-yl)methanone |
| 442 | 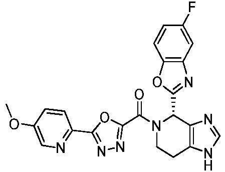 | (S)-(4-(5-fluorobenzo[d]oxazol-2-yl)-6,7-dihydro-1H-imidazo[4,5-c]pyridin-5(4H)-yl)(5-(5-methoxypyridin-2-yl)-1,3,4-oxadiazol-2-yl)methanone |
| 444 | 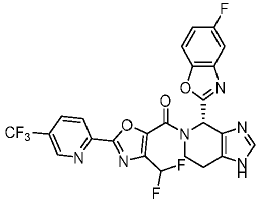 | (S)-(4-(difluoromethyl)-2-(5-(trifluoromethyl)pyridin-2-yl)oxazol-5-yl)(4-(5-fluorobenzo[d]oxazol-2-yl)-6,7-dihydro-1H-imidazo[4,5-c]pyridin-5(4H)-yl)methanone |
| 445 | 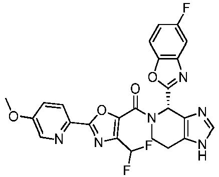 | (S)-(4-(difluoromethyl)-2-(5-methoxypyridin-2-yl)oxazol-5-yl)(4-(5-fluorobenzo[d]oxazol-2-yl)-6,7-dihydro-1H-imidazo[4,5-c]pyridin-5(4H)-yl)methanone |
| 447 | 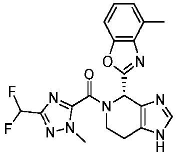 | (S)-(3-(difluoromethyl)-1-methyl-1H-1,2,4-triazol-5-yl)(4-(4-methylbenzo[d]oxazol-2-yl)-6,7-dihydro-1H-imidazo[4,5-c]pyridin-5(4H)-yl)methanone |
| 449 | 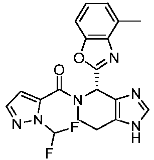 | (S)-(1-(difluoromethyl)-1H-pyrazol-5-yl)(4-(4-methylbenzo[d]oxazol-2-yl)-6,7-dihydro-1H-imidazo[4,5-c]pyridin-5(4H)-yl)methanone |
| 451 | 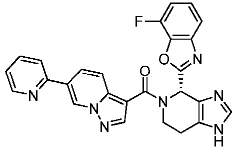 | (S)-(4-(7-fluorobenzo[d]oxazol-2-yl)-6,7-dihydro-1H-imidazo[4,5-c]pyridin-5(4H)-yl)(6-(pyridin-2-yl)pyrazolo[1,5-a]pyridin-3-yl)methanone |
| 453 | 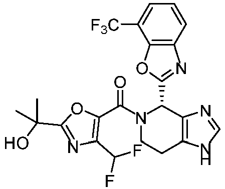 | (S)-(4-(difluoromethyl)-2-(2-hydroxypropan-2-yl)oxazol-5-yl)(4-(7-(trifluoromethyl)benzo[d]oxazol-2-yl)-6,7-dihydro-1H-imidazo[4,5-c]pyridin-5(4H)-yl)methanone |
| 455 | 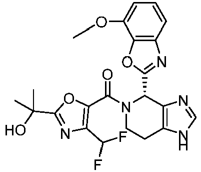 | (S)-(4-(difluoromethyl)-2-(2-hydroxypropan-2-yl)oxazol-5-yl)(4-(7-methoxybenzo[d]oxazol-2-yl)-6,7-dihydro-1H-imidazo[4,5-c]pyridin-5(4H)-yl)methanone |
| 457 | 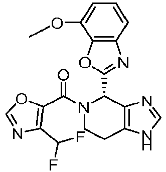 | (S)-(4-(difluoromethyl)oxazol-5-yl)(4-(7-methoxybenzo[d]oxazol-2-yl)-6,7-dihydro-1H-imidazo[4,5-c]pyridin-5(4H)-yl)methanone |
| 460 | 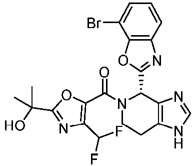 | (S)-(4-(7-bromobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 461 | 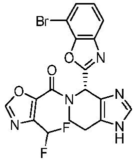 | (S)-(4-(7-bromobenzo[d]oxazol-2-yl)-6,7-dihydro-1H-imidazo[4,5-c]pyridin-5(4H)-yl)(4-(difluoromethyl)oxazol-5-yl)methanone |
| 463 | 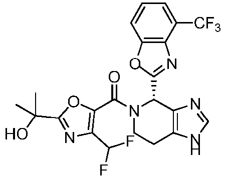 | (S)-(4-(difluoromethyl)-2-(2-hydroxypropan-2-yl)oxazol-5-yl)(4-(4-(trifluoromethyl)benzo[d]oxazol-2-yl)-6,7-dihydro-1H-imidazo[4,5-c]pyridin-5(4H)-yl)methanone |
| 465 | 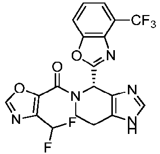 | (S)-(4-(difluoromethyl)oxazol-5-yl)(4-(4-(trifluoromethyl)benzo[d]oxazol-2-yl)-6,7-dihydro-1H-imidazo[4,5-c]pyridin-5(4H)-yl)methanone |
| 467 | 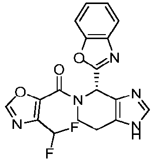 | (S)-(4-(benzo[d]oxazol-2-yl)-6,7-dihydro-1H-imidazo[4,5-c]pyridin-5(4H)-yl)(4-(difluoromethyl)oxazol-5-yl)methanone |
| 470 | 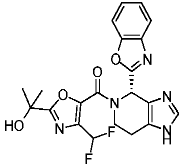 | (S)-(4-(benzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 471 | 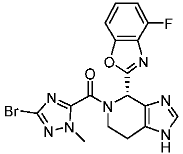 | (S)-(3-bromo-1-methyl-1H-1,2,4-triazol-5-yl)(4-(4-fluorobenzo[d]oxazol-2-yl)-6,7-dihydro-1H-imidazo[4,5-c]pyridin-5(4H)-yl)methanone |
| 473 | 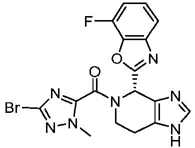 | (S)-(3-bromo-1-methyl-1H-1,2,4-triazol-5-yl)(4-(7-fluorobenzo[d]oxazol-2-yl)-6,7-dihydro-1H-imidazo[4,5-c]pyridin-5(4H)-yl)methanone |
| 475 | 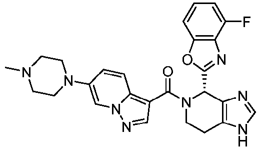 | (S)-(4-(4-fluorobenzo[d]oxazol-2-yl)-6,7-dihydro-1H-imidazo[4,5-c]pyridin-5(4H)-yl)(6-(4-methylpiperazin-1-yl)pyrazolo[1,5-a]pyridin-3-yl)methanone |
| 477 | 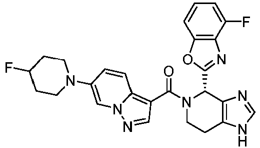 | (S)-(4-(4-fluorobenzo[d]oxazol-2-yl)-6,7-dihydro-1H-imidazo[4,5-c]pyridin-5(4H)-yl)(6-(4-fluoropiperidin-1-yl)pyrazolo[1,5-a]pyridin-3-yl)methanone |
| 480 | 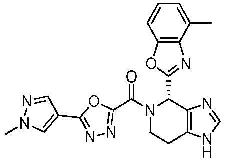 | (S)-(5-(1-methyl-1H-pyrazol-4-yl)-1,3,4-oxadiazol-2-yl)(4-(4-methylbenzo[d]oxazol-2-yl)-6,7-dihydro-1H-imidazo[4,5-c]pyridin-5(4H)-yl)methanone |
| 482 | 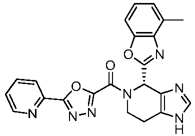 | (S)-(4-(4-methylbenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 483 | 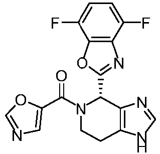 | (S)-(4-(4,7-difluorobenzo[d]oxazol-2-yl)-6,7-dihydro-1H-imidazo[4,5-c]pyridin-5(4H)-yl)(oxazol-5-yl)methanone |
| 486 | 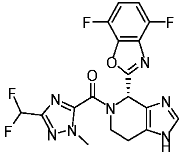 | (S)-(4-(4,7-difluorobenzo[d]oxazol-2-yl)-6,7-dihydro-1H-imidazo[4,5-c]pyridin-5(4H)-yl)(3-(difluoromethyl)-1-methyl-1H-1,2,4-triazol-5-yl)methanone |
| 487 | 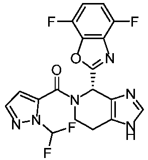 | (S)-(4-(4,7-difluorobenzo[d]oxazol-2-yl)-6,7-dihydro-1H-imidazo[4,5-c]pyridin-5(4H)-yl)(1-(difluoromethyl)-1H-pyrazol-5-yl)methanone |
| 489 | 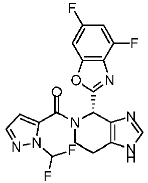 | (S)-(4-(4,6-difluorobenzo[d]oxazol-2-yl)-6,7-dihydro-1H-imidazo[4,5-c]pyridin-5(4H)-yl)(1-(difluoromethyl)-1H-pyrazol-5-yl)methanone |
| 491 | 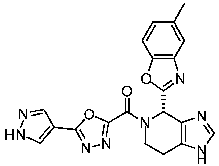 | (S)-(5-(1H-pyrazol-4-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 493 | 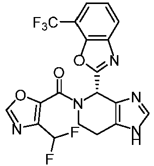 | (S)-(4-(difluoromethyl)oxazol-5-yl)(4-(7-(trifluoromethyl)benzo[d]oxazol-2-yl)-6,7-dihydro-1H-imidazo[4,5-c]pyridin-5(4H)-yl)methanone |
| 495 | 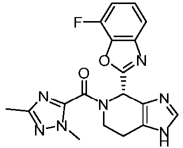 | (S)-(1,3-dimethyl-1H-1,2,4-triazol-5-yl)(4-(7-fluorobenzo[d]oxazol-2-yl)-6,7-dihydro-1H-imidazo[4,5-c]pyridin-5(4H)-yl)methanone |
| 497 | 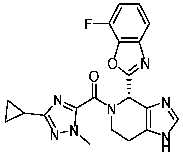 | (S)-(3-cyclopropyl-1-methyl-1H-1,2,4-triazol-5-yl)(4-(7-fluorobenzo[d]oxazol-2-yl)-6,7-dihydro-1H-imidazo[4,5-c]pyridin-5(4H)-yl)methanone |
| 499 | 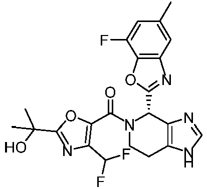 | (S)-(4-(difluoromethyl)-2-(2-hydroxypropan-2-yl)oxazol-5-yl)(4-(7-fluoro-5-methylbenzo[d]oxazol-2-yl)-6,7-dihydro-1H-imidazo[4,5-c]pyridin-5(4H)-yl)methanone |
| 501 | 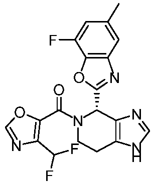 | (S)-(4-(difluoromethyl)oxazol-5-yl)(4-(7-fluoro-5-methylbenzo[d]oxazol-2-yl)-6,7-dihydro-1H-imidazo[4,5-c]pyridin-5(4H)-yl)methanone |
| 504 | 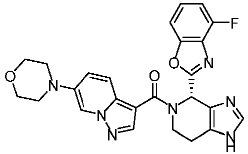 | (S)-(4-(4-fluorobenzo[d]oxazol-2-yl)-6,7-dihydro-1H-imidazo[4,5-c]pyridin-5(4H)-yl)(6-morpholinopyrazolo[1,5-a]pyridin-3-yl)methanone |
| 505 | 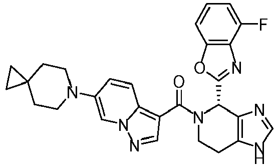 | (S)-(6-(6-azaspiro[2.5]octan-6-yl)pyrazolo[1,5-a]pyridin-3-yl)(4-(4-fluorobenzo[d]oxazol-2-yl)-6,7-dihydro-1H-imidazo[4,5-c]pyridin-5(4H)-yl)methanone |
| 507 | 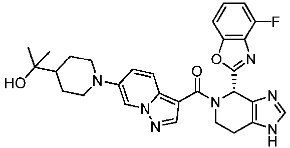 | (S)-(4-(4-fluorobenzo[d]oxazol-2-yl)-6,7-dihydro-1H-imidazo[4,5-c]pyridin-5(4H)-yl)(6-(4-(2-hydroxypropan-2-yl)piperidin-1-yl)pyrazolo[1,5-a]pyridin-3-yl)methanone |
| 509 | 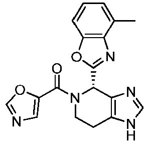 | (S)-(4-(4-methylbenzo[d]oxazol-2-yl)-6,7-dihydro-1H-imidazo[4,5-c]pyridin-5(4H)-yl)(oxazol-5-yl)methanone |
| 511 | 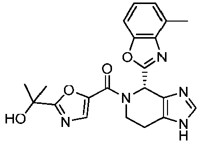 | (S)-(2-(2-hydroxypropan-2-yl)oxazol-5-yl)(4-(4-methylbenzo[d]oxazol-2-yl)-6,7-dihydro-1H-imidazo[4,5-c]pyridin-5(4H)-yl)methanone |
| 513 | 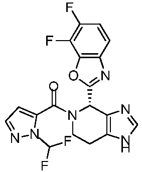 | (S)-(4-(6,7-difluorobenzo[d]oxazol-2-yl)-6,7-dihydro-1H-imidazo[4,5-c]pyridin-5(4H)-yl)(1-(difluoromethyl)-1H-pyrazol-5-yl)methanone |
| 515 | 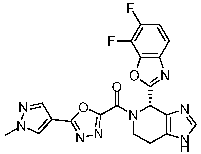 | (S)-(4-(6,7-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 518 | 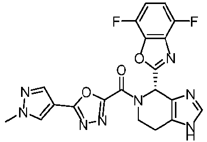 | (S)-(4-(4,7-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 520 | 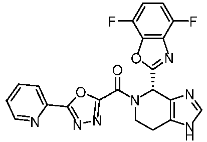 | (S)-(4-(4,7-difluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 522 | 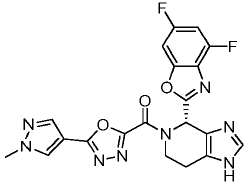 | (S)-(4-(4,6-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 523 | 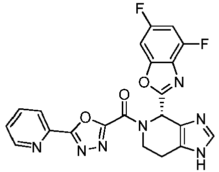 | (S)-(4-(4,6-difluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 526 | 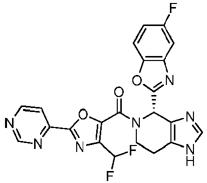 | (S)-(4-(difluoromethyl)-2-(pyrimidin-4-yl)oxazol-5-yl)(4-(5-fluorobenzo[d]oxazol-2-yl)-6,7-dihydro-1H-imidazo[4,5-c]pyridin-5(4H)-yl)methanone |
| 527 | 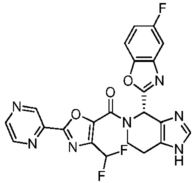 | (S)-(4-(difluoromethyl)-2-(pyrazin-2-yl)oxazol-5-yl)(4-(5-fluorobenzo[d]oxazol-2-yl)-6,7-dihydro-1H-imidazo[4,5-c]pyridin-5(4H)-yl)methanone |
| 529 | 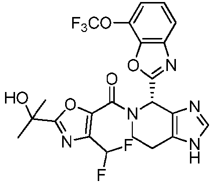 | (S)-(4-(difluoromethyl)-2-(2-hydroxypropan-2-yl)oxazol-5-yl)(4-(7-(trifluoromethoxy)benzo[d]oxazol-2-yl)-6,7-dihydro-1H-imidazo[4,5-c]pyridin-5(4H)-yl)methanone |
| 532 | 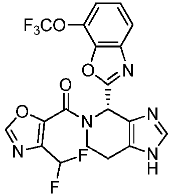 | (S)-(4-(difluoromethyl)oxazol-5-yl)(4-(7-(trifluoromethoxy)benzo[d]oxazol-2-yl)-6,7-dihydro-1H-imidazo[4,5-c]pyridin-5(4H)-yl)methanone |
| 533 | 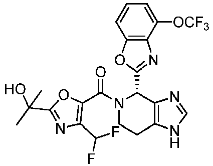 | (S)-(4-(difluoromethyl)-2-(2-hydroxypropan-2-yl)oxazol-5-yl)(4-(4-(trifluoromethoxy)benzo[d]oxazol-2-yl)-6,7-dihydro-1H-imidazo[4,5-c]pyridin-5(4H)-yl)methanone |
| 536 | 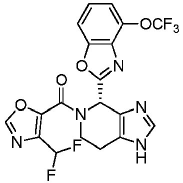 | (S)-(4-(difluoromethyl)oxazol-5-yl)(4-(4-(trifluoromethoxy)benzo[d]oxazol-2-yl)-6,7-dihydro-1H-imidazo[4,5-c]pyridin-5(4H)-yl)methanone |
| 537 | 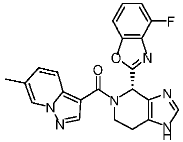 | (S)-(4-(4-fluorobenzo[d]oxazol-2-yl)-6,7-dihydro-1H-imidazo[4,5-c]pyridin-5(4H)-yl)(6-methylpyrazolo[1,5-a]pyridin-3-yl)methanone |
| 539 | 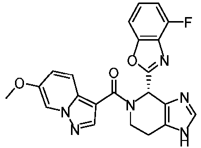 | (S)-(4-(4-fluorobenzo[d]oxazol-2-yl)-6,7-dihydro-1H-imidazo[4,5-c]pyridin-5(4H)-yl)(6-methoxypyrazolo[1,5-a]pyridin-3-yl)methanone |
| 541 | 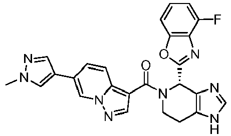 | (S)-(4-(4-fluorobenzo[d]oxazol-2-yl)-6,7-dihydro-1H-imidazo[4,5-c]pyridin-5(4H)-yl)(6-(1-methyl-1H-pyrazol-4-yl)pyrazolo[1,5-a]pyridin-3-yl)methanone |
| 543 | 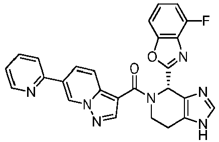 | (S)-(4-(4-fluorobenzo[d]oxazol-2-yl)-6,7-dihydro-1H-imidazo[4,5-c]pyridin-5(4H)-yl)(6-(pyridin-2-yl)pyrazolo[1,5-a]pyridin-3-yl)methanone |
| 546 | 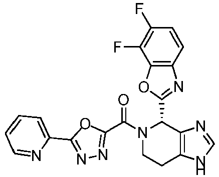 | (S)-(4-(6,7-difluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 547 | 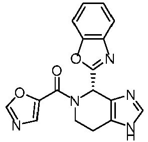 | (S)-(4-(benzo[d]oxazol-2-yl)-6,7-dihydro-1H-imidazo[4,5-c]pyridin-5(4H)-yl)(oxazol-5-yl)methanone |
| 549 | 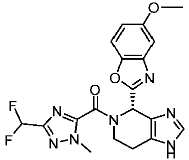 | (S)-(3-(difluoromethyl)-1-methyl-1H-1,2,4-triazol-5-yl)(4-(5-methoxybenzo[d]oxazol-2-yl)-6,7-dihydro-1H-imidazo[4,5-c]pyridin-5(4H)-yl)methanone |
| 551 | 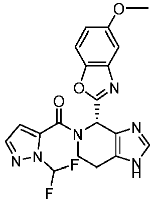 | (S)-(1-(difluoromethyl)- 1H-pyrazol-5-yl)(4-(5-methoxybenzo[d]oxazol-2-yl)-6,7-dihydro-1H-imidazo[4,5-c]pyridin-5(4H)-yl)methanone |
| 553 | 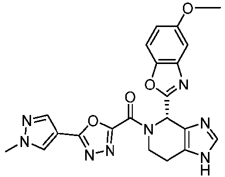 | (S)-(4-(5-methoxybenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 555 | 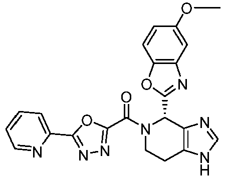 | (S)-(4-(5-methoxybenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 557 | 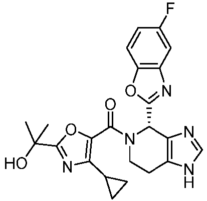 | (S)-(4-cyclopropyl-2-(2-hydroxypropan-2-yl)oxazol-5-yl)(4-(5-fluorobenzo[d]oxazol-2-yl)-6,7-dihydro-1H-imidazo[4,5-c]pyridin-5(4H)-yl)methanone |
| 560 | 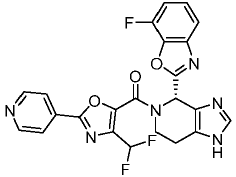 | (S)-(4-(difluoromethyl)-2-(pyridin-4-yl)oxazol-5-yl)(4-(7-fluorobenzo[d]oxazol-2-yl)-6,7-dihydro-1H-imidazo[4,5-c]pyridin-5(4H)-yl)methanone |
| 561 | 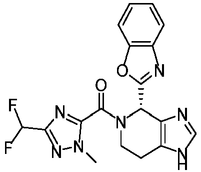 | (S)-(4-(benzo[d]oxazol-2-yl)-6,7-dihydro-1H-imidazo[4,5-c]pyridin-5(4H)-yl)(3-(difluoromethyl)-1-methyl-1H-1,2,4-triazol-5-yl)methanone |
| 563 | 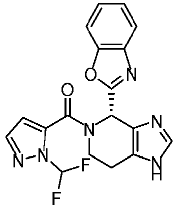 | (S)-(4-(benzo[d]oxazol-2-yl)-6,7-dihydro-1H-imidazo[4,5-c]pyridin-5(4H)-yl)(1-(difluoromethyl)-1H-pyrazol-5-yl)methanone |
| 565 | 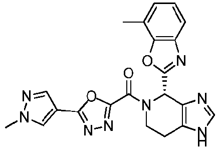 | (S)-(5-(1-methyl-1H-pyrazol-4-yl)-1,3,4-oxadiazol-2-yl)(4-(7-methylbenzo[d]oxazol-2-yl)-6,7-dihydro-1H-imidazo[4,5-c]pyridin-5(4H)-yl)methanone |
| 567 | 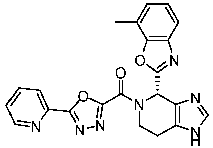 | (S)-(4-(7-methylbenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 569 | 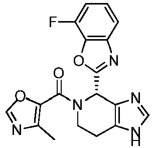 | (S)-(4-(7-fluorobenzo[d]oxazol-2-yl)-6,7-dihydro-1H-imidazo[4,5-c]pyridin-5(4H)-yl)(4-methyloxazol-5-yl)methanone |
| 571 | 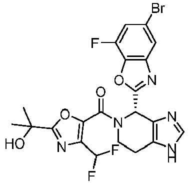 | (S)-(4-(5-bromo-7-fluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 573 | 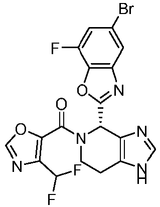 | (S)-(4-(5-bromo-7-fluorobenzo[d]oxazol-2-yl)-6,7-dihydro-1H-imidazo[4,5-c]pyridin-5(4H)-yl)(4-(difluoromethyl)oxazol-5-yl)methanone |
| 575 | 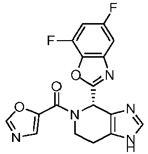 | (S)-(4-(5,7-difluorobenzo[d]oxazol-2-yl)-6,7-dihydro-1H-imidazo[4,5-c]pyridin-5(4H)-yl)(oxazol-5-yl)methanone |
| 577 | 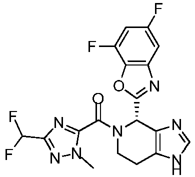 | (S)-(4-(5,7-difluorobenzo[d]oxazol-2-yl)-6,7-dihydro-1H-imidazo[4,5-c]pyridin-5(4H)-yl)(3-(difluoromethyl)-1-methyl-1H-1,2,4-triazol-5-yl)methanone |
| 580 | 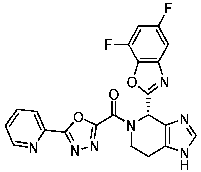 | (S)-(4-(5,7-difluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 581 | 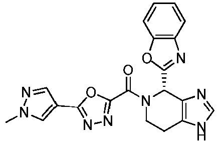 | (S)-(4-(benzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 583 | 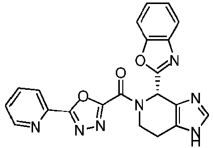 | (S)-(4-(benzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 585 | 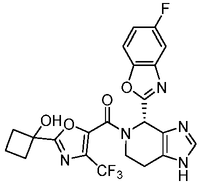 | (S)-(4-(5-fluorobenzo[d]oxazol-2-yl)-6,7-dihydro-1H-imidazo[4,5-c]pyridin-5(4H)-yl)(2-(1-hydroxycyclobutyl)-4-(trifluoromethyl)oxazol-5-yl)methanone |
| 587 | 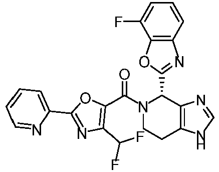 | (S)-(4-(difluoromethyl)-2-(pyridin-2-yl)oxazol-5-yl)(4-(7-fluorobenzo[d]oxazol-2-yl)-6,7-dihydro-1H-imidazo[4,5-c]pyridin-5(4H)-yl)methanone |
| 590 | 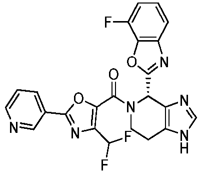 | (S)-(4-(difluoromethyl)-2-(pyridin-3-yl)oxazol-5-yl)(4-(7-fluorobenzo[d]oxazol-2-yl)-6,7-dihydro-1H-imidazo[4,5-c]pyridin-5(4H)-yl)methanone |
| 591 | 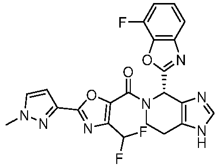 | (S)-(4-(difluoromethyl)-2-(1-methyl-1H-pyrazol-3-yl)oxazol-5-yl)(4-(7-fluorobenzo[d]oxazol-2-yl)-6,7-dihydro-1H-imidazo[4,5-c]pyridin-5(4H)-yl)methanone |
| 594 | 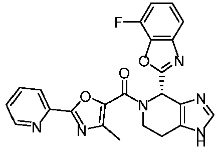 | (S)-(4-(7-fluorobenzo[d]oxazol-2-yl)-6,7-dihydro-1H-imidazo[4,5-c]pyridin-5(4H)-yl)(4-methyl-2-(pyridin-2-yl)oxazol-5-yl)methanone |
| 596 | 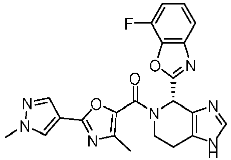 | (S)-(4-(7-fluorobenzo[d]oxazol-2-yl)-6,7-dihydro-1H-imidazo[4,5-c]pyridin-5(4H)-yl)(4-methyl-2-(1-methyl-1H-pyrazol-4-yl)oxazol-5-yl)methanone |
| 597 | 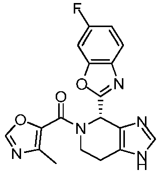 | (S)-(4-(6-fluorobenzo[d]oxazol-2-yl)-6,7-dihydro-1H-imidazo[4,5-c]pyridin-5(4H)-yl)(4-methyloxazol-5-yl)methanone |
| 599 | 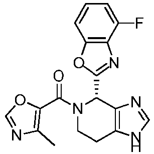 | (S)-(4-(4-fluorobenzo[d]oxazol-2-yl)-6,7-dihydro-1H-imidazo[4,5-c]pyridin-5(4H)-yl)(4-methyloxazol-5-yl)methanone |
| 601 | 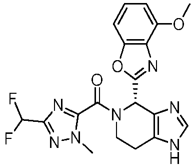 | (S)-(3-(difluoromethyl)-1-methyl-1H-1,2,4-triazol-5-yl)(4-(4-methoxybenzo[d]oxazol-2-yl)-6,7-dihydro-1H-imidazo[4,5-c]pyridin-5(4H)-yl)methanone |
| 603 | 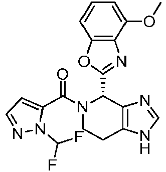 | (S)-(1-(difluoromethyl)-1H-pyrazol-5-yl)(4-(4-methoxybenzo[d]oxazol-2-yl)-6,7-dihydro-1H-imidazo[4,5-c]pyridin-5(4H)-yl)methanone |
| 605 | 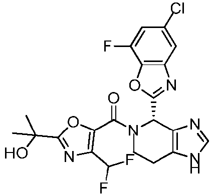 | (S)-(4-(5-chloro-7-fluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 608 | 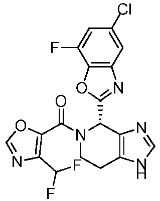 | (S)-(4-(5-chloro-7-fluorobenzo[d]oxazol-2-yl)-6,7-dihydro-1H-imidazo[4,5-c]pyridin-5(4H)-yl)(4-(difluoromethyl)oxazol-5-yl)methanone |
| 609 | 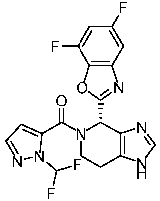 | (S)-(4-(5,7-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 611 | 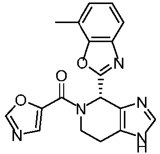 | (S)-(4-(7-methylbenzo[d]oxazol-2-yl)-6,7-dihydro-1H-imidazo[4,5-c]pyridin-5(4H)-yl)(oxazol-5-yl)methanone |
| 613 | 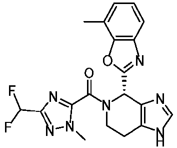 | (S)-(3-(difluoromethyl)-1-methyl-1H-1,2,4-triazol-5-yl)(4-(7-methylbenzo[d]oxazol-2-yl)-6,7-dihydro-1H-imidazo[4,5-c]pyridin-5(4H)-yl)methanone |
| 616 | 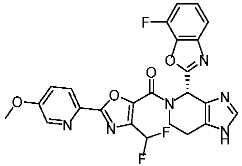 | (S)-(4-(difluoromethyl)-2-(5-methoxypyridin-2-yl)oxazol-5-yl)(4-(7-fluorobenzo[d]oxazol-2-yl)-6,7-dihydro-1H-imidazo[4,5-c]pyridin-5(4H)-yl)methanone |
| 617 | 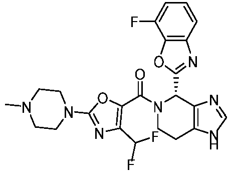 | (S)-(4-(difluoromethyl)-2-(4-methylpiperazin-1-yl)oxazol-5-yl)(4-(7-fluorobenzo[d]oxazol-2-yl)-6,7-dihydro-1H-imidazo[4,5-c]pyridin-5(4H)-yl)methanone |
| 620 | 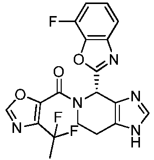 | (S)-(4-(1,1-difluoroethyl)oxazol-5-yl)(4-(7-fluorobenzo[d]oxazol-2-yl)-6,7-dihydro-1H-imidazo[4,5-c]pyridin-5(4H)-yl)methanone |
| 621 | 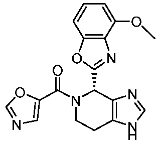 | (S)-(4-(4-methoxybenzo[d]oxazol-2-yl)-6,7-dihydro-1H-imidazo[4,5-c]pyridin-5(4H)-yl)(oxazol-5-yl)methanone |
| 624 | 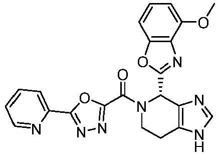 | (S)-(4-(4-methoxybenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 626 | 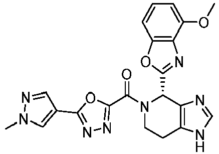 | (S)-(4-(4-methoxybenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 628 | 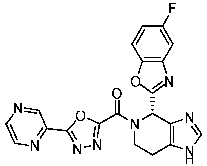 | (S)-(4-(5-fluorobenzo[d]oxazol-2-yl)-6,7-dihydro-1H-imidazo[4,5-c]pyridin-5(4H)-yl)(5-(pyrazin-2-yl)-1,3,4-oxadiazol-2-yl)methanone |
| 629 | 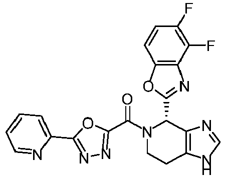 | (S)-(4-(4,5-difluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 632 | 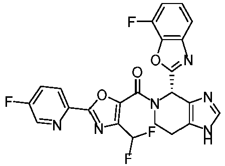 | (S)-(4-(difluoromethyl)-2-(5-fluoropyridin-2-yl)oxazol-5-yl)(4-(7-fluorobenzo[d]oxazol-2-yl)-6,7-dihydro-1H-imidazo[4,5-c]pyridin-5(4H)-yl)methanone |
| 633 | 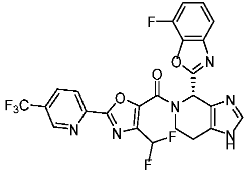 | (S)-(4-(difluoromethyl)-2-(5-(trifluoromethyl)pyridin-2-yl)oxazol-5-yl)(4-(7-fluorobenzo[d]oxazol-2-yl)-6,7-dihydro-1H-imidazo[4,5-c]pyridin-5(4H)-yl)methanone |
| 636 | 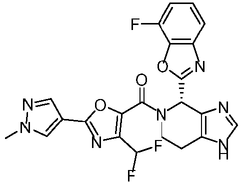 | (S)-(4-(difluoromethyl)-2-(1-methyl-1H-pyrazol-4-yl)oxazol-5-yl)(4-(7-fluorobenzo[d]oxazol-2-yl)-6,7-dihydro-1H-imidazo[4,5-c]pyridin-5(4H)-yl)methanone |
| 638 | 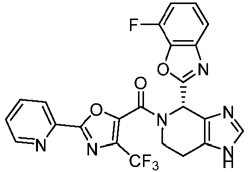 | (S)-(4-(7-fluorobenzo[d]oxazol-2-yl)-6,7-dihydro-1H-imidazo[4,5-c]pyridin-5(4H)-yl)(2-(pyridin-2-yl)-4-(trifluoromethyl)oxazol-5-yl)methanone |
| 639 | 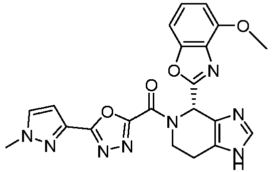 | (S)-(4-(4-methoxybenzo[d]oxazol-2-yl)-6,7-dihydro-1H-imidazo[4,5-c]pyridin-5(4H)-yl)(5-(1-methyl-1H-pyrazol-3-yl)-1,3,4-oxadiazol-2-yl)methanone |
| 641 | 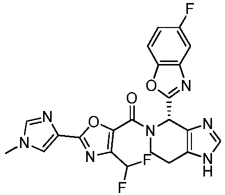 | (S)-(4-(difluoromethyl)-2-(1-methyl-1H-imidazol-4-yl)oxazol-5-yl)(4-(5-fluorobenzo[d]oxazol-2-yl)-6,7-dihydro-1H-imidazo[4,5-c]pyridin-5(4H)-yl)methanone |
| 643 | 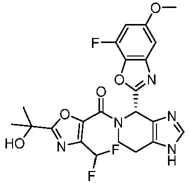 | (S)-(4-(difluoromethyl)-2-(2-hydroxypropan-2-yl)oxazol-5-yl)(4-(7-fluoro-5-methoxybenzo[d]oxazol-2-yl)-6,7-dihydro-1H-imidazo[4,5-c]pyridin-5(4H)-yl)methanone |
| 645 | 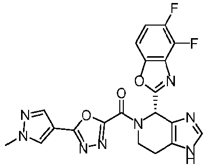 | (S)-(4-(4,5-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 648 | 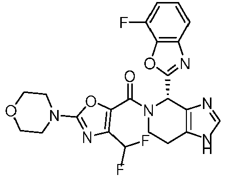 | (S)-(4-(difluoromethyl)-2-morpholinooxazol-5-yl)(4-(7-fluorobenzo[d]oxazol-2-yl)-6,7-dihydro-1H-imidazo[4,5-c]pyridin-5(4H)-yl)methanone |
| 649 | 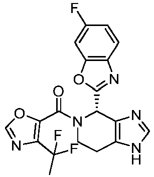 | (S)-(4-(1,1-difluoroethyl)oxazol-5-yl)(4-(6-fluorobenzo[d]oxazol-2-yl)-6,7-dihydro-1H-imidazo[4,5-c]pyridin-5(4H)-yl)methanone |
| 651 | 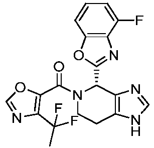 | (S)-(4-(1,1-difluoroethyl)oxazol-5-yl)(4-(4-fluorobenzo[d]oxazol-2-yl)-6,7-dihydro-1H-imidazo[4,5-c]pyridin-5(4H)-yl)methanone |
| 653 | 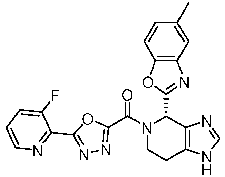 | (S)-(5-(3-fluoropyridin-2-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 655 | 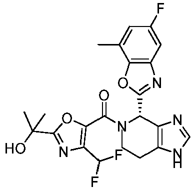 | (S)-(4-(difluoromethyl)-2-(2-hydroxypropan-2-yl)oxazol-5-yl)(4-(5-fluoro-7-methylbenzo[d]oxazol-2-yl)-6,7-dihydro-1H-imidazo[4,5-c]pyridin-5(4H)-yl)methanone |
| 657 | 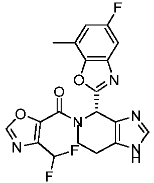 | (S)-(4-(difluoromethyl)oxazol-5-yl)(4-(5-fluoro-7-methylbenzo[d]oxazol-2-yl)-6,7-dihydro-1H-imidazo[4,5-c]pyridin-5(4H)-yl)methanone |
| 659 | 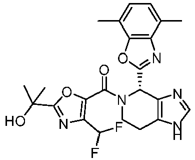 | (S)-(4-(difluoromethyl)-2-(2-hydroxypropan-2-yl)oxazol-5-yl)(4-(4,7-dimethylbenzo[d]oxazol-2-yl)-6,7-dihydro-1H-imidazo[4,5-c]pyridin-5(4H)-yl)methanone |
| 661 | 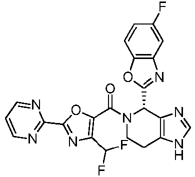 | (S)-(4-(difluoromethyl)-2-(pyrimidin-2-yl)oxazol-5-yl)(4-(5-fluorobenzo[d]oxazol-2-yl)-6,7-dihydro-1H-imidazo[4,5-c]pyridin-5(4H)-yl)methanone |
| 663 | 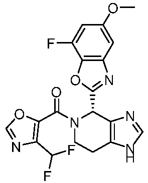 | (S)-(4-(difluoromethyl)oxazol-5-yl)(4-(7-fluoro-5-methoxybenzo[d]oxazol-2-yl)-6,7-dihydro-1H-imidazo[4,5-c]pyridin-5(4H)-yl)methanone |
| 665 | 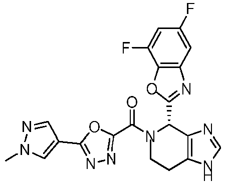 | (S)-(4-(5,7-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 667 | 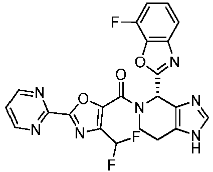 | (S)-(4-(difluoromethyl)-2-(pyrimidin-2-yl)oxazol-5-yl)(4-(7-fluorobenzo[d]oxazol-2-yl)-6,7-dihydro-1H-imidazo[4,5-c]pyridin-5(4H)-yl)methanone |
| 669 | 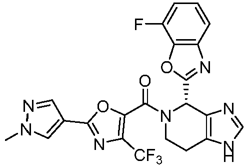 | (S)-(4-(7-fluorobenzo[d]oxazol-2-yl)-6,7-dihydro-1H-imidazo[4,5-c]pyridin-5(4H)-yl)(2-(1-methyl-1H-pyrazol-4-yl)-4-(trifluoromethyl)oxazol-5-yl)methanone |
| 671 | 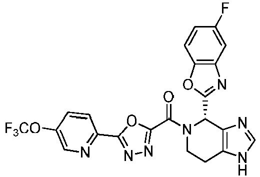 | (S)-(4-(5-fluorobenzo[d]oxazol-2-yl)-6,7-dihydro-1H-imidazo[4,5-c]pyridin-5(4H)-yl)(5-(5-(trifluoromethoxy)pyridin-2-yl)-1,3,4-oxadiazol-2-yl)methanone |
| 673 | 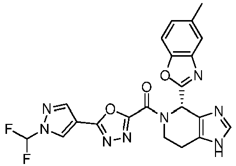 | (S)-(5-(1-(difluoromethyl)-1H-pyrazol-4-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 676 | 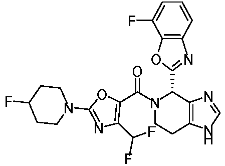 | (S)-(4-(difluoromethyl)-2-(4-fluoropiperidin-1-yl)oxazol-5-yl)(4-(7-fluorobenzo[d]oxazol-2-yl)-6,7-dihydro-1H-imidazo[4,5-c]pyridin-5(4H)-yl)methanone |
| 677 | 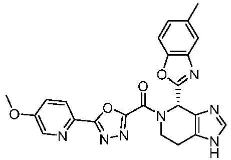 | (S)-(5-(5-methoxypyridin-2-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 679 | 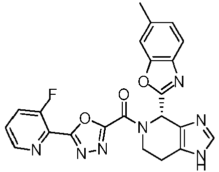 | (S)-(5-(3-fluoropyridin-2-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 681 | 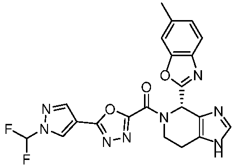 | (S)-(5-(1-(difluoromethyl)-1H-pyrazol-4-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 683 | 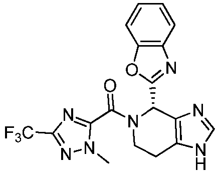 | (S)-(4-(benzo[d]oxazol-2-yl)-6,7-dihydro-1H-imidazo[4,5-c]pyridin-5(4H)-yl)(1-methyl-3-(trifluoromethyl)-1H-1,2,4-triazol-5-yl)methanone |
| 685 | 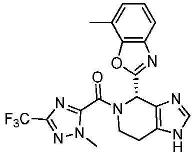 | (S)-(1-methyl-3-(trifluoromethyl)-1H-1,2,4-triazol-5-yl)(4-(7-methylbenzo[d]oxazol-2-yl)-6,7-dihydro-1H-imidazo[4,5-c]pyridin-5(4H)-yl)methanone |
| 687 | 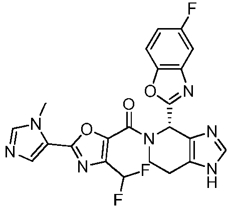 | (S)-(4-(difluoromethyl)-2-(1-methyl-1H-imidazol-5-yl)oxazol-5-yl)(4-(5-fluorobenzo[d]oxazol-2-yl)-6,7-dihydro-1H-imidazo[4,5-c]pyridin-5(4H)-yl)methanone |
| 689 | 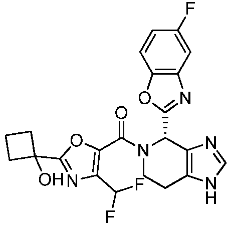 | (S)-(4-(difluoromethyl)-2-(1-hydroxycyclobutyl)oxazol-5-yl)(4-(5-fluorobenzo[d]oxazol-2-yl)-6,7-dihydro-1H-imidazo[4,5-c]pyridin-5(4H)-yl)methanone |
| 692 | 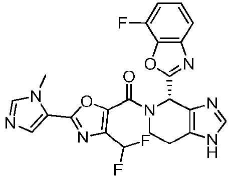 | (S)-(4-(difluoromethyl)-2-(1-methyl-1H-imidazol-5-yl)oxazol-5-yl)(4-(7-fluorobenzo[d]oxazol-2-yl)-6,7-dihydro-1H-imidazo[4,5-c]pyridin-5(4H)-yl)methanone |
| 693 | 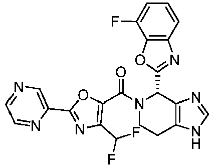 | (S)-(4-(difluoromethyl)-2-(pyrazin-2-yl)oxazol-5-yl)(4-(7-fluorobenzo[d]oxazol-2-yl)-6,7-dihydro-1H-imidazo[4,5-c]pyridin-5(4H)-yl)methanone |
| 695 | 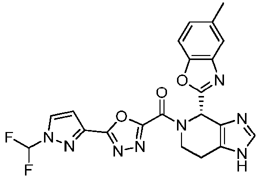 | (S)-(5-(1-(difluoromethyl)-1H-pyrazol-3-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 697 | 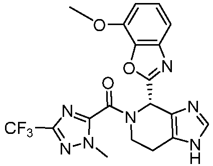 | (S)-(4-(7-methoxybenzo[d]oxazol-2-yl)-6,7-dihydro-1H-imidazo[4,5-c]pyridin-5(4H)-yl)(1-methyl-3-(trifluoromethyl)-1H-1,2,4-triazol-5-yl)methanone |
| 699 | 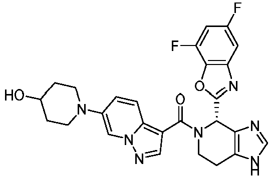 | (S)-(4-(5,7-difluorobenzo[d]oxazol-2-yl)-6,7-dihydro-1H-imidazo[4,5-c]pyridin-5(4H)-yl)(6-(4-hydroxypiperidin-1-yl)pyrazolo[1,5-a]pyridin-3-yl)methanone |
| 701 | 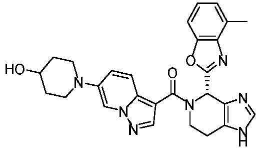 | (S)-(6-(4-hydroxypiperidin-1-yl)pyrazolo[1,5-a]pyridin-3-yl)(4-(4-methylbenzo[d]oxazol-2-yl)-6,7-dihydro-1H-imidazo[4,5-c]pyridin-5(4H)-yl)methanone |
| 703 | 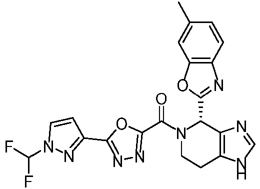 | (S)-(5-(1-(difluoromethyl)-1H-pyrazol-3-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 705 | 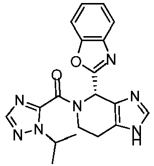 | (S)-(4-(benzo[d]oxazol-2-yl)-6,7-dihydro-1H-imidazo[4,5-c]pyridin-5(4H)-yl)(1-isopropyl-1H-1,2,4-triazol-5-yl)methanone |
| 707 | 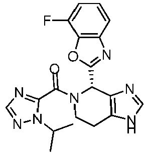 | (S)-(4-(7-fluorobenzo[d]oxazol-2-yl)-6,7-dihydro-1H-imidazo[4,5-c]pyridin-5(4H)-yl)(1-isopropyl-1H-1,2,4-triazol-5-yl)methanone |
| 710 | 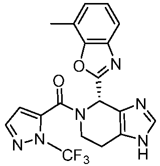 | (S)-(4-(7-methylbenzo[d]oxazol-2-yl)-6,7-dihydro-1H-imidazo[4,5-c]pyridin-5(4H)-yl)(1-(trifluoromethyl)-1H-pyrazol-5-yl)methanone |
| 711 | 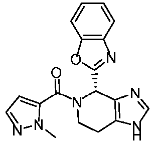 | (S)-(4-(benzo[d]oxazol-2-yl)-6,7-dihydro-1H-imidazo[4,5-c]pyridin-5(4H)-yl)(1-methyl-1H-pyrazol-5-yl)methanone |
| 713 | 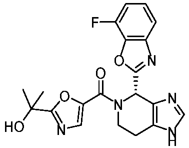 | (S)-(4-(7-fluorobenzo[d]oxazol-2-yl)-6,7-dihydro-1H-imidazo[4,5-c]pyridin-5(4H)-yl)(2-(2-hydroxypropan-2-yl)oxazol-5-yl)methanone |
| 715 | 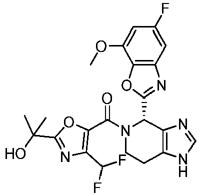 | (S)-(4-(difluoromethyl)-2-(2-hydroxypropan-2-yl)oxazol-5-yl)(4-(5-fluoro-7-methoxybenzo[d]oxazol-2-yl)-6,7-dihydro-1H-imidazo[4,5-c]pyridin-5(4H)-yl)methanone |
| 718 | 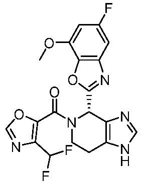 | (S)-(4-(difluoromethyl)oxazol-5-yl)(4-(5-fluoro-7-methoxybenzo[d]oxazol-2-yl)-6,7-dihydro-1H-imidazo[4,5-c]pyridin-5(4H)-yl)methanone |
| 719 | 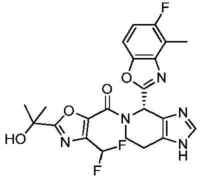 | (S)-(4-(difluoromethyl)-2-(2-hydroxypropan-2-yl)oxazol-5-yl)(4-(5-fluoro-4-methylbenzo[d]oxazol-2-yl)-6,7-dihydro-1H-imidazo[4,5-c]pyridin-5(4H)-yl)methanone |
| 722 | 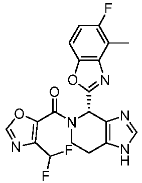 | (S)-(4-(difluoromethyl)oxazol-5-yl)(4-(5-fluoro-4-methylbenzo[d]oxazol-2-yl)-6,7-dihydro-1H-imidazo[4,5-c]pyridin-5(4H)-yl)methanone |
| 723 | 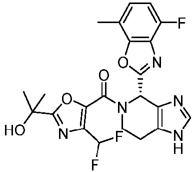 | (S)-(4-(difluoromethyl)-2-(2-hydroxypropan-2-yl)oxazol-5-yl)(4-(4-fluoro-7-methylbenzo[d]oxazol-2-yl)-6,7-dihydro-1H-imidazo[4,5-c]pyridin-5(4H)-yl)methanone |
| 725 | 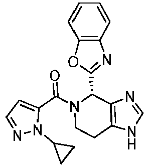 | (S)-(4-(benzo[d]oxazol-2-yl)-6,7-dihydro-1H-imidazo[4,5-c]pyridin-5(4H)-yl)(1-cyclopropyl-1H-pyrazol-5-yl)methanone |
| 727 | 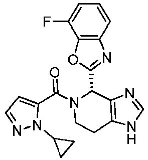 | (S)-(1-cyclopropyl-1H-pyrazol-5-yl)(4-(7-fluorobenzo[d]oxazol-2-yl)-6,7-dihydro-1H-imidazo[4,5-c]pyridin-5(4H)-yl)methanone |
| 729 | 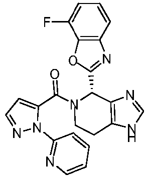 | (S)-(4-(7-fluorobenzo[d]oxazol-2-yl)-6,7-dihydro-1H-imidazo[4,5-c]pyridin-5(4H)-yl)(1-(pyridin-2-yl)-1H-pyrazol-5-yl)methanone |
| 731 | 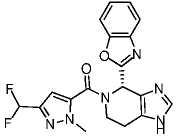 | (S)-(4-(benzo[d]oxazol-2-yl)-6,7-dihydro-1H-imidazo[4,5-c]pyridin-5(4H)-yl)(3-(difluoromethyl)-1-methyl-1H-pyrazol-5-yl)methanone |
| 733 | 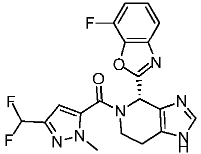 | (S)-(3-(difluoromethyl)-1-methyl-1H-pyrazol-5-yl)(4-(7-fluorobenzo[d]oxazol-2-yl)-6,7-dihydro-1H-imidazo[4,5-c]pyridin-5(4H)-yl)methanone |
| 735 | 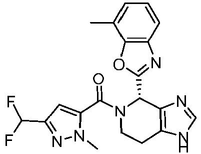 | (S)-(3-(difluoromethyl)-1-methyl-1H-pyrazol-5-yl)(4-(7-fluorobenzo[d]oxazol-2-yl)-6,7-dihydro-1H-imidazo[4,5-c]pyridin-5(4H)-yl)methanone |
| 737 | 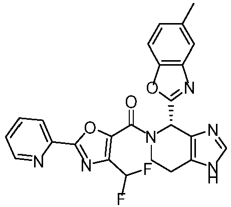 | (S)-(4-(difluoromethyl)-2-(pyridin-2-yl)oxazol-5-yl)(4-(5-methylbenzo[d]oxazol-2-yl)-6,7-dihydro-1H-imidazo[4,5-c]pyridin-5(4H)-yl)methanone |
| 740 | 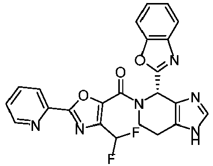 | (S)-(4-(benzo[d]oxazol-2-yl)-6,7-dihydro-1H-imidazo[4,5-c]pyridin-5(4H)-yl)(4-(difluoromethyl)-2-(pyridin-2-yl)oxazol-5-yl)methanone |
| 742 | 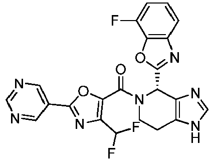 | (S)-(4-(difluoromethyl)-2-(pyrimidin-5-yl)oxazol-5-yl)(4-(7-fluorobenzo[d]oxazol-2-yl)-6,7-dihydro-1H-imidazo[4,5-c]pyridin-5(4H)-yl)methanone |
| 743 | 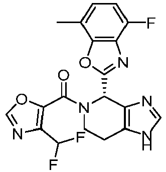 | (S)-(4-(difluoromethyl)oxazol-5-yl)(4-(4-fluoro-7-methylbenzo[d]oxazol-2-yl)-6,7-dihydro-1H-imidazo[4,5-c]pyridin-5(4H)-yl)methanone |
| 745 | 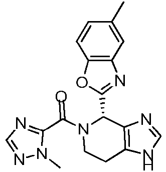 | (S)-(1-methyl-1H-1,2,4-triazol-5-yl)(4-(5-methylbenzo[d]oxazol-2-yl)-6,7-dihydro-1H-imidazo[4,5-c]pyridin-5(4H)-yl)methanone |
| 747 | 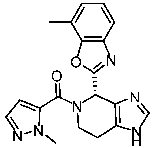 | (S)-(1-methyl-1H-pyrazol-5-yl)(4-(7-methylbenzo[d]oxazol-2-yl)-6,7-dihydro-1H-imidazo[4,5-c]pyridin-5(4H)-yl)methanone |
| 749 | 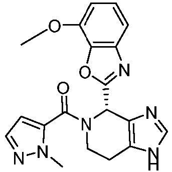 | (S)-(4-(7-methoxybenzo[d]oxazol-2-yl)-6,7-dihydro-1H-imidazo[4,5-c]pyridin-5(4H)-yl)(1-methyl-1H-pyrazol-5-yl)methanone |
| 752 | 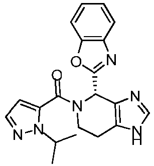 | (S)-(4-(benzo[d]oxazol-2-yl)-6,7-dihydro-1H-imidazo[4,5-c]pyridin-5(4H)-yl)(1-isopropyl-1H-pyrazol-5-yl)methanone |
| 753 | 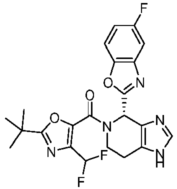 | (S)-(2-(tert-butyl)-4-(difluoromethyl)oxazol-5-yl)(4-(5-fluorobenzo[d]oxazol-2-yl)-6,7-dihydro-1H-imidazo[4,5-c]pyridin-5(4H)-yl)methanone |
| 755 | 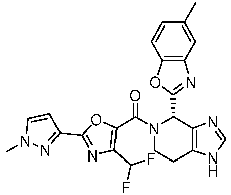 | (S)-(4-(difluoromethyl)-2-(1-methyl-1H-pyrazol-3-yl)oxazol-5-yl)(4-(5-methylbenzo[d]oxazol-2-yl)-6,7-dihydro-1H-imidazo[4,5-c]pyridin-5(4H)-yl)methanone |
| 758 | 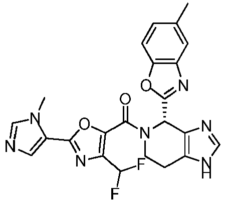 | (S)-(4-(difluoromethyl)-2-(1-methyl-1H-imidazol-5-yl)oxazol-5-yl)(4-(5-methylbenzo[d]oxazol-2-yl)-6,7-dihydro-1H-imidazo[4,5-c]pyridin-5(4H)-yl)methanone |
| 760 | 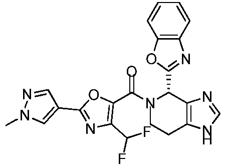 | (S)-(4-(benzo[d]oxazol-2-yl)-6,7-dihydro-1H-imidazo[4,5-c]pyridin-5(4H)-yl)(4-(difluoromethyl)-2-(1-methyl-1H-pyrazol-4-yl)oxazol-5-yl)methanone |
| 761 | 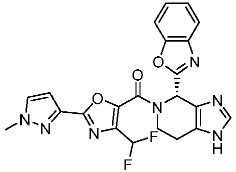 | (S)-(4-(benzo[d]oxazol-2-yl)-6,7-dihydro-1H-imidazo[4,5-c]pyridin-5(4H)-yl)(4-(difluoromethyl)-2-(1-methyl-1H-pyrazol-3-yl)oxazol-5-yl)methanone |
| 764 | 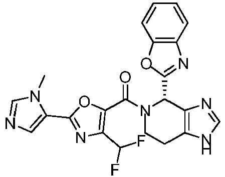 | (S)-(4-(benzo[d]oxazol-2-yl)-6,7-dihydro-1H-imidazo[4,5-c]pyridin-5(4H)-yl)(4-(difluoromethyl)-2-(1-methyl-1H-imidazol-5-yl)oxazol-5-yl)methanone |
| 765 | 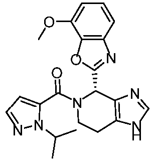 | (S)-(1-isopropyl-1H-pyrazol-5-yl)(4-(7-methoxybenzo[d]oxazol-2-yl)-6,7-dihydro-1H-imidazo[4,5-c]pyridin-5(4H)-yl)methanone |
| 767 | 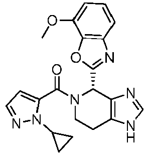 | (S)-(1-cyclopropyl-1H-pyrazol-5-yl)(4-(7-methoxybenzo[d]oxazol-2-yl)-6,7-dihydro-1H-imidazo[4,5-c]pyridin-5(4H)-yl)methanone |
| 769 | 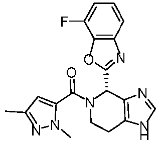 | (S)-(1,3-dimethyl-1H-pyrazol-5-yl)(4-(7-fluorobenzo[d]oxazol-2-yl)-6,7-dihydro-1H-imidazo[4,5-c]pyridin-5(4H)-yl)methanone |
| 771 | 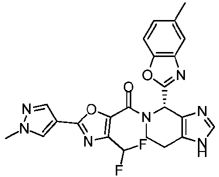 | (S)-(4-(difluoromethyl)-2-(1-methyl-1H-pyrazol-4-yl)oxazol-5-yl)(4-(5-methylbenzo[d]oxazol-2-yl)-6,7-dihydro-1H-imidazo[4,5-c]pyridin-5(4H)-yl)methanone |
| 773 | 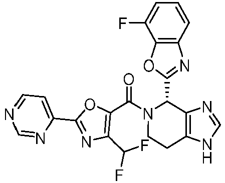 | (S)-(4-(difluoromethyl)-2-(pyrimidin-4-yl)oxazol-5-yl)(4-(7-fluorobenzo[d]oxazol-2-yl)-6,7-dihydro-1H-imidazo[4,5-c]pyridin-5(4H)-yl)methanone |
| 775 | 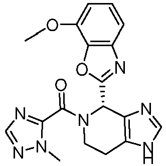 | (S)-(4-(7-methoxybenzo[d]oxazol-2-yl)-6,7-dihydro-1H-imidazo[4,5-c]pyridin-5(4H)-yl)(1-methyl-1H-1,2,4-triazol-5-yl)methanone |
| 777 | 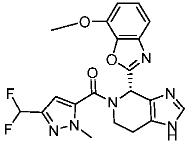 | (S)-(3-(difluoromethyl)-1-methyl-1H-pyrazol-5-yl)(4-(7-methoxybenzo[d]oxazol-2-yl)-6,7-dihydro-1H-imidazo[4,5-c]pyridin-5(4H)-yl)methanone |
| 779 | 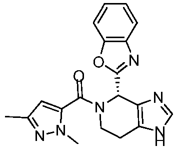 | (S)-(4-(benzo[d]oxazol-2-yl)-6,7-dihydro-1H-imidazo[4,5-c]pyridin-5(4H)-yl)(1,3-dimethyl-1H-pyrazol-5-yl)methanone |
| 781 | 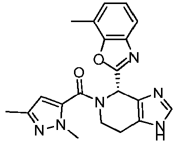 | (S)-(1,3-dimethyl-1H-pyrazol-5-yl)(4-(7-methylbenzo[d]oxazol-2-yl)-6,7-dihydro-1H-imidazo[4,5-c]pyridin-5(4H)-yl)methanone |
| 784 | 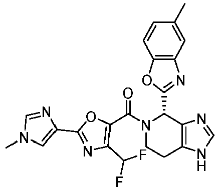 | (S)-(4-(difluoromethyl)-2-(1-methyl-1H-imidazol-4-yl)oxazol-5-yl)(4-(5-methylbenzo[d]oxazol-2-yl)-6,7-dihydro-1H-imidazo[4,5-c]pyridin-5(4H)-yl)methanone |
| 786 | 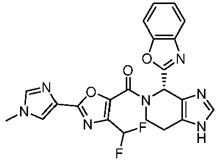 | (S)-(4-(benzo[d]oxazol-2-yl)-6,7-dihydro-1H-imidazo[4,5-c]pyridin-5(4H)-yl)(4-(difluoromethyl)-2-(1-methyl-1H-imidazol-4-yl)oxazol-5-yl)methanone |
| 787 | 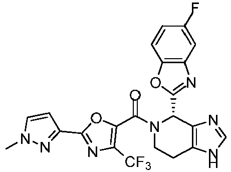 | (S)-(4-(5-fluorobenzo[d]oxazol-2-yl)-6,7-dihydro-1H-imidazo[4,5-c]pyridin-5(4H)-yl)(2-(1-methyl-1H-pyrazol-3-yl)-4-(trifluoromethyl)oxazol-5-yl)methanone |
| 790 | 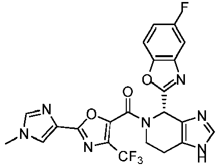 | (S)-(4-(5-fluorobenzo[d]oxazol-2-yl)-6,7-dihydro-1H-imidazo[4,5-c]pyridin-5(4H)-yl)(2-(1-methyl-1H-imidazol-4-yl)-4-(trifluoromethyl)oxazol-5-yl)methanone |
| 791 | 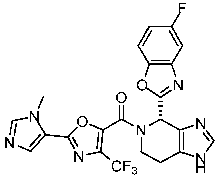 | (S)-(4-(5-fluorobenzo[d]oxazol-2-yl)-6,7-dihydro-1H-imidazo[4,5-c]pyridin-5(4H)-yl)(2-(1-methyl-1H-imidazol-5-yl)-4-(trifluoromethyl)oxazol-5-yl)methanone |
| 793 | 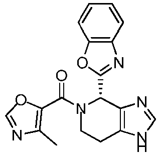 | (S)-(4-(benzo[d]oxazol-2-yl)-6,7-dihydro-1H-imidazo[4,5-c]pyridin-5(4H)-yl)(4-methyloxazol-5-yl)methanone |
| 795 | 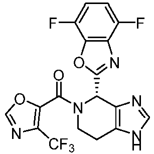 | (S)-(4-(4,7-difluorobenzo[d]oxazol-2-yl)-6,7-dihydro-1H-imidazo[4,5-c]pyridin-5(4H)-yl)(4-(trifluoromethyl)oxazol-5-yl)methanone |
| 797 | 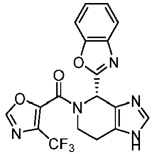 | (S)-(4-(benzo[d]oxazol-2-yl)-6,7-dihydro-1H-imidazo[4,5-c]pyridin-5(4H)-yl)(4-(trifluoromethyl)oxazol-5-yl)methanone |
| 800 | 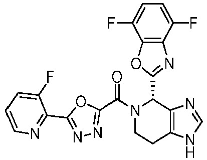 | (S)-(4-(4,7-difluorobenzo[d]oxazol-2-yl)-6,7-dihydro-1H-imidazo[4,5-c]pyridin-5(4H)-yl)(5-(3-fluoropyridin-2-yl)-1,3,4-oxadiazol-2-yl)methanone |
| 801 | 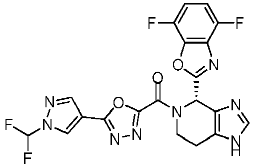 | (S)-(4-(4,7-difluorobenzo[d]oxazol-2-yl)-6,7-dihydro-1H-imidazo[4,5-c]pyridin-5(4H)-yl)(5-(1-(difluoromethyl)-1H-pyrazol-4-yl)-1,3,4-oxadiazol-2-yl)methanone |
| 803 | 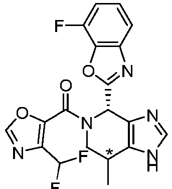 | (4-(difluoromethyl)oxazol-5-yl)((4S)-4-(7-fluorobenzo[d]oxazol-2-yl)-7-methyl-6,7-dihydro-1H-imidazo[4,5-c]pyridin-5(4H)-yl)methanone |
| | | Diastereomer #1 (Rt=0.949 min) |
| 804 | 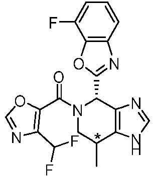 | (4-(difluoromethyl)oxazol-5-yl)((4S)-4-(7-fluorobenzo[d]oxazol-2-yl)-7-methyl-6,7-dihydro-1H-imidazo[4,5-c]pyridin-5(4H)-yl)methanone |
| | | Diastereomer #1 (Rt=1.175 min) |
| 807 | 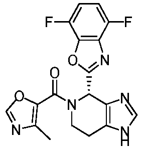 | (S)-(4-(4,7-difluorobenzo[d]oxazol-2-yl)-6,7-dihydro-1H-imidazo[4,5-c]pyridin-5(4H)-yl)(4-methyloxazol-5-yl)methanone |
| 811 | 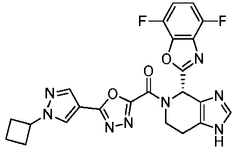 | (S)-(5-(1-cyclobutyl-1H-pyrazol-4-yl)-1,3,4-oxadiazol-2-yl)(4-(4,7-difluorobenzo[d]oxazol-2-yl)-6,7-dihydro-1H-imidazo[4,5-c]pyridin-5(4H)-yl)methanone |
| 813 | 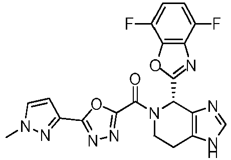 | (S)-(3-(difluoromethyl)-1-methyl-1H-1,2,4-triazol-5-yl)(4-(4-methylbenzo[d]oxazol-2-yl)-6,7-dihydro-1H-imidazo[4,5-c]pyridin-5(4H)-yl)methanone |
| 817 | 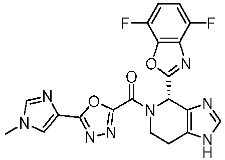 | (S)-(4-(4,7-difluorobenzo[d]oxazol-2-yl)-6,7-dihydro-1H-imidazo[4,5-c]pyridin-5(4H)-yl)(5-(1-methyl-1H-imidazol-4-yl)-1,3,4-oxadiazol-2-yl)methanone |
| 819 | 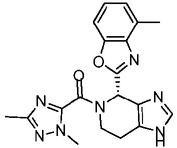 | (S)-(1,3-dimethyl-1H-1,2,4-triazol-5-yl)(4-(4-methylbenzo[d]oxazol-2-yl)-6,7-dihydro-1H-imidazo[4,5-c]pyridin-5(4H)-yl)methanone |
| 821 | 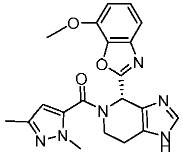 | (S)-(1,3-dimethyl-1H-pyrazol-5-yl)(4-(7-methoxybenzo[d]oxazol-2-yl)-6,7-dihydro-1H-imidazo[4,5-c]pyridin-5(4H)-yl)methanone |
| 824 | 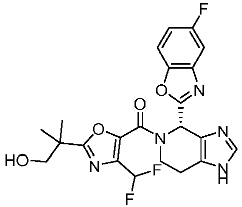 | (S)-(4-(difluoromethyl)-2-(1-hydroxy-2-methylpropan-2-yl)oxazol-5-yl)(4-(5-fluorobenzo[d]oxazol-2-yl)-6,7-dihydro-1H-imidazo[4,5-c]pyridin-5(4H)-yl)methanone |
| 825 | 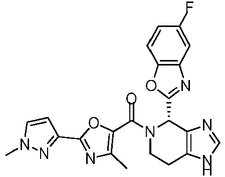 | (S)-(4-(5-fluorobenzo[d]oxazol-2-yl)-6,7-dihydro-1H-imidazo[4,5-c]pyridin-5(4H)-yl)(4-methyl-2-(1-methyl-1H-pyrazol-3-yl)oxazol-5-yl)methanone |
| 827 | 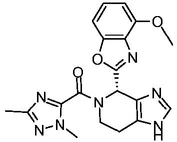 | (S)-(1,3-dimethyl-1H-1,2,4-triazol-5-yl)(4-(4-methoxybenzo[d]oxazol-2-yl)-6,7-dihydro-1H-imidazo[4,5-c]pyridin-5(4H)-yl)methanone |
| 830 | 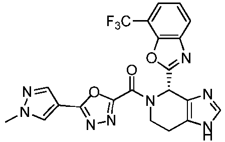 | (S)-(5-(1-methyl-1H-pyrazol-4-yl)-1,3,4-oxadiazol-2-yl)(4-(7-(trifluoromethyl)benzo[d]oxazol-2-yl)-6,7-dihydro-1H-imidazo[4,5-c]pyridin-5(4H)-yl)methanone |
| 832 | 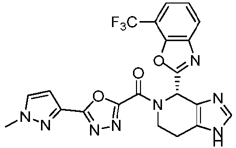 | (S)-(5-(1-methyl-1H-pyrazol-3-yl)-1,3,4-oxadiazol-2-yl)(4-(7-(trifluoromethyl)benzo[d]oxazol-2-yl)-6,7-dihydro-1H-imidazo[4,5-c]pyridin-5(4H)-yl)methanone |
| 833 | 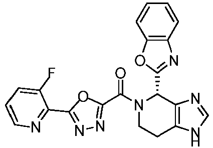 | (S)-(4-(benzo[d]oxazol-2-yl)-6,7-dihydro-1H-imidazo[4,5-c]pyridin-5(4H)-yl)(5-(3-fluoropyridin-2-yl)-1,3,4-oxadiazol-2-yl)methanone |
| 835 | 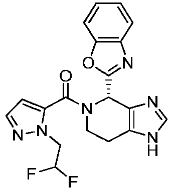 | (S)-(4-(benzo[d]oxazol-2-yl)-6,7-dihydro-1H-imidazo[4,5-c]pyridin-5(4H)-yl)(1-(2,2-difluoroethyl)-1H-pyrazol-5-yl)methanone |
| 838 | 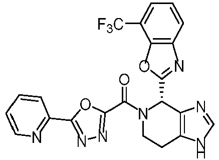 | (S)-(5-(pyridin-2-yl)-1,3,4-oxadiazol-2-yl)(4-(7-(trifluoromethyl)benzo[d]oxazol-2-yl)-6,7-dihydro-1H-imidazo[4,5-c]pyridin-5(4H)-yl)methanone |
| 840 | 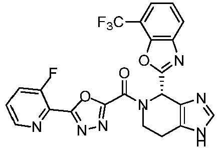 | (S)-(5-(3-fluoropyridin-2-yl)-1,3,4-oxadiazol-2-yl)(4-(7-(trifluoromethyl)benzo[d]oxazol-2-yl)-6,7-dihydro-1H-imidazo[4,5-c]pyridin-5(4H)-yl)methanone |
| 841 | 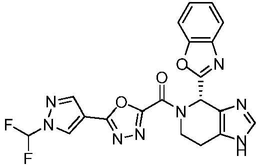 | (S)-(4-(benzo[d]oxazol-2-yl)-6,7-dihydro-1H-imidazo[4,5-c]pyridin-5(4H)-yl)(5-(1-(difluoromethyl)-1H-pyrazol-4-yl)-1,3,4-oxadiazol-2-yl)methanone |
| 843 | 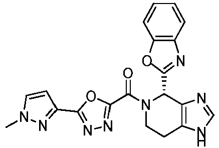 | (S)-(4-(benzo[d]oxazol-2-yl)-6,7-dihydro-1H-imidazo[4,5-c]pyridin-5(4H)-yl)(5-(1-methyl-1H-pyrazol-3-yl)-1,3,4-oxadiazol-2-yl)methanone |
| 846 | 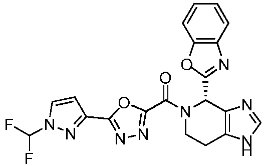 | (S)-(4-(benzo[d]oxazol-2-yl)-6,7-dihydro-1H-imidazo[4,5-c]pyridin-5(4H)-yl)(5-(1-(difluoromethyl)-1H-pyrazol-3-yl)-1,3,4-oxadiazol-2-yl)methanone |
| 847 | 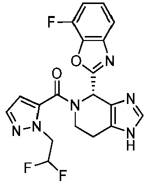 | (S)-(1-(2,2-difluoroethyl)-1H-pyrazol-5-yl)(4-(7-fluorobenzo[d]oxazol-2-yl)-6,7-dihydro-1H-imidazo[4,5-c]pyridin-5(4H)-yl)methanone |
| 850 | 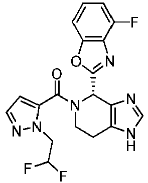 | (S)-(1-(2,2-difluoroethyl)-1H-pyrazol-5-yl)(4-(4-fluorobenzo[d]oxazol-2-yl)-6,7-dihydro-1H-imidazo[4,5-c]pyridin-5(4H)-yl)methanone |
| 852 | 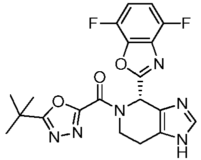 | (S)-(5-(tert-butyl)-1,3,4-oxadiazol-2-yl)(4-(4,7-difluorobenzo[d]oxazol-2-yl)-6,7-dihydro-1H-imidazo[4,5-c]pyridin-5(4H)-yl)methanone |
| 854 | 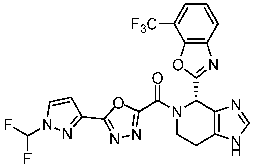 | (S)-(5-(1-(difluoromethyl)-1H-pyrazol-3-yl)-1,3,4-oxadiazol-2-yl)(4-(7-(trifluoromethyl)benzo[d]oxazol-2-yl)-6,7-dihydro-1H-imidazo[4,5-c]pyridin-5(4H)-yl)methanone |
| 855 | 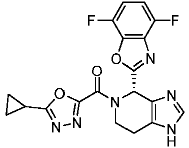 | (S)-(5-cyclopropyl-1,3,4-oxadiazol-2-yl)(4-(4,7-difluorobenzo[d]oxazol-2-yl)-6,7-dihydro-1H-imidazo[4,5-c]pyridin-5(4H)-yl)methanone |
| 858 | 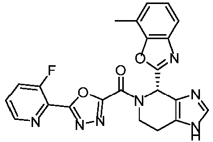 | (S)-(5-(3-fluoropyridin-2-yl)-1,3,4-oxadiazol-2-yl)(4-(7-methylbenzo[d]oxazol-2-yl)-6,7-dihydro-1H-imidazo[4,5-c]pyridin-5(4H)-yl)methanone |
| 860 | 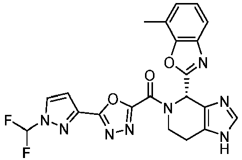 | (S)-(5-(1-(difluoromethyl)-1H-pyrazol-3-yl)-1,3,4-oxadiazol-2-yl)(4-(7-methylbenzo[d]oxazol-2-yl)-6,7-dihydro-1H-imidazo[4,5-c]pyridin-5(4H)-yl)methanone |
| 862 | 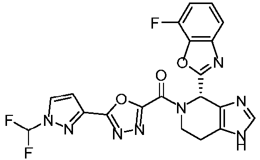 | (S)-(5-(1-(difluoromethyl)-1H-pyrazol-3-yl)-1,3,4-oxadiazol-2-yl)(4-(7-fluorobenzo[d]oxazol-2-yl)-6,7-dihydro-1H-imidazo[4,5-c]pyridin-5(4H)-yl)methanone |
| 863 | 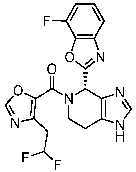 | (S)-(4-(2,2-difluoroethyl)oxazol-5-yl)(4-(7-fluorobenzo[d]oxazol-2-yl)-6,7-dihydro-1H-imidazo[4,5-c]pyridin-5(4H)-yl)methanone |
| 865 | 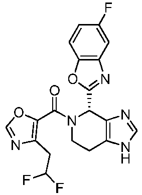 | (S)-(4-(2,2-difluoroethyl)oxazol-5-yl)(4-(5-fluorobenzo[d]oxazol-2-yl)-6,7-dihydro-1H-imidazo[4,5-c]pyridin-5(4H)-yl)methanone |
| 868 | 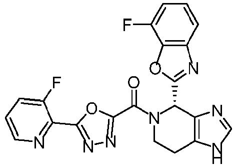 | (S)-(4-(7-fluorobenzo[d]oxazol-2-yl)-6,7-dihydro-1H-imidazo[4,5-c]pyridin-5(4H)-yl)(5-(3-fluoropyridin-2-yl)-1,3,4-oxadiazol-2-yl)methanone |
| 869 | 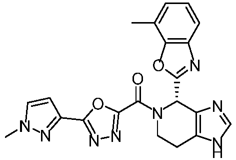 | (S)-(5-(1-methyl-1H-pyrazol-3-yl)-1,3,4-oxadiazol-2-yl)(4-(7-methylbenzo[d]oxazol-2-yl)-6,7-dihydro-1H-imidazo[4,5-c]pyridin-5(4H)-yl)methanone |
| 872 | 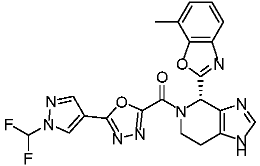 | (S)-(5-(1-(difluoromethyl)-1H-pyrazol-4-yl)-1,3,4-oxadiazol-2-yl)(4-(7-methylbenzo[d]oxazol-2-yl)-6,7-dihydro-1H-imidazo[4,5-c]pyridin-5(4H)-yl)methanone |
| 873 | 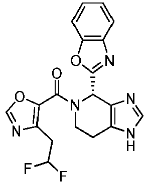 | (S)-(4-(benzo[d]oxazol-2-yl)-6,7-dihydro-1H-imidazo[4,5-c]pyridin-5(4H)-yl)(4-(2,2-difluoroethyl)oxazol-5-yl)methanone |
| 875 | 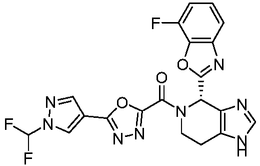 | (S)-(5-(1-(difluoromethyl)-1H-pyrazol-4-yl)-1,3,4-oxadiazol-2-yl)(4-(7-fluorobenzo[d]oxazol-2-yl)-6,7-dihydro-1H-imidazo[4,5-c]pyridin-5(4H)-yl)methanone |
| 877 | 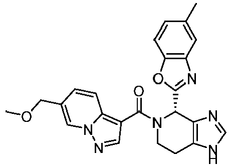 | (S)-(6-(methoxymethyl)pyrazolo[1,5-a]pyridin-3-yl)(4-(5-methylbenzo[d]oxazol-2-yl)-6,7-dihydro-1H-imidazo[4,5-c]pyridin-5(4H)-yl)methanone |
| 879 | 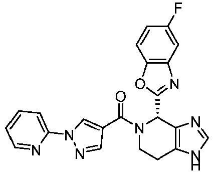 | (S)-(4-(5-fluorobenzo[d]oxazol-2-yl)-6,7-dihydro-1H-imidazo[4,5-c]pyridin-5(4H)-yl)(1-(pyridin-2-yl)-1H-pyrazol-4-yl)methanone |
| 881 | 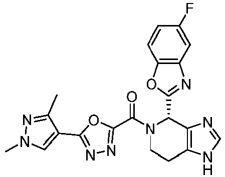 | (S)-(5-(1,3-dimethyl-1H-pyrazol-4-yl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 884 | 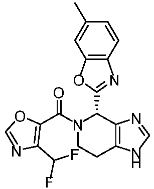 | (S)-(4-(difluoromethyl)oxazol-5-yl)(4-(6-methylbenzo[d]oxazol-2-yl)-6,7-dihydro-1H-imidazo[4,5-c]pyridin-5(4H)-yl)methanone |
| 885 | 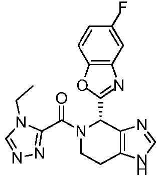 | (S)-(4-ethyl-4H-1,2,4-triazol-3-yl)(4-(5-fluorobenzo[d]oxazol-2-yl)-6,7-dihydro-1H-imidazo[4,5-c]pyridin-5(4H)-yl)methanone |
| 887 | 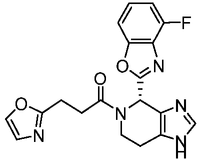 | (S)-1-(4-(4-fluorobenzo[d]oxazol-2-yl)-6,7-dihydro-1H-imidazo[4,5-c]pyridin-5(4H)-yl)-3-(oxazol-2-yl)propan-1-one |
| 890 | 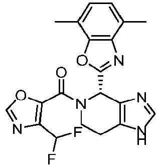 | (S)-(4-(difluoromethyl)oxazol-5-yl)(4-(4,7-dimethylbenzo[d]oxazol-2-yl)-6,7-dihydro-1H-imidazo[4,5-c]pyridin-5(4H)-yl)methanone |
| 892 | 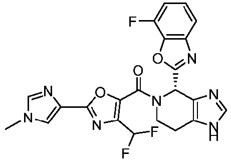 | (S)-(4-(difluoromethyl)-2-(1-methyl-1H-imidazol-4-yl)oxazol-5-yl)(4-(7-fluorobenzo[d]oxazol-2-yl)-1,4,6,7-tetrahydro-5H-imidazo[4,5-c]pyridin-5-yl)methanone |
| 893 | 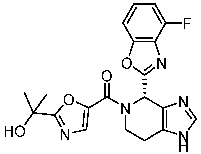 | (S)-(4-(4-fluorobenzo[d]oxazol-2-yl)-6,7-dihydro-1H-imidazo[4,5-c]pyridin-5(4H)-yl)(2-(2-hydroxypropan-2-yl)oxazol-5-yl)methanone |
| 895 | 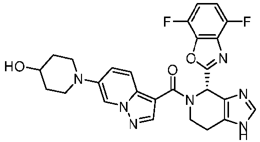 | (S)-(4-(4,7-difluorobenzo[d]oxazol-2-yl)-6,7-dihydro-1H-imidazo[4,5-c]pyridin-5(4H)-yl)(6-(4-hydroxypiperidin-1-yl)pyrazolo[1,5-a]pyridin-3-yl)methanone |
| 898 | 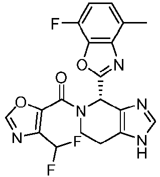 | (S)-(4-(difluoromethyl)oxazol-5-yl)(4-(7-fluoro-4-methylbenzo[d]oxazol-2-yl)-6,7-dihydro-1H-imidazo[4,5-c]pyridin-5(4H)-yl)methanone |
| 899 | 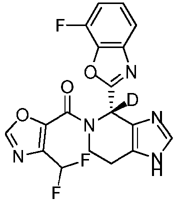 | (S)-(4-(difluoromethyl)oxazol-5-yl)(4-deuterium-4-(7-fluorobenzo[d]oxazol-2-yl)-6,7-dihydro-1H-imidazo[4,5-c]pyridin-5(4H)-yl)methanone |
| 901 | 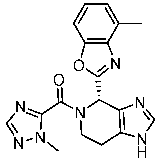 | (S)-(1-methyl-1H-1,2,4-triazol-5-yl)(4-(4-methylbenzo[d]oxazol-2-yl)-6,7-dihydro-1H-imidazo[4,5-c]pyridin-5(4H)-yl)methanone |
| 903 | 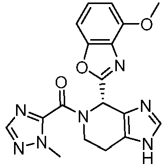 | (S)-(4-(4-methoxybenzo[d]oxazol-2-yl)-6,7-dihydro-1H-imidazo[4,5-c]pyridin-5(4H)-yl)(1-methyl-1H-1,2,4-triazol-5-yl)methanone |
| 905 | 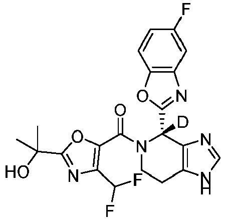 | (S)-(4-(deuterium)-2-(2-hydroxypropan-2-yl)oxazol-5-yl)(4-fluoro-4-(5-fluorobenzo[d]oxazol-2-yl)-6,7-dihydro-1H-imidazo[4,5-c]pyridin-5(4H)-yl)methanone |
| 907 | 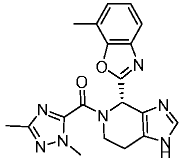 | (S)-(1,3-dimethyl-1H-1,2,4-triazol-5-yl)(4-(7-methylbenzo[d]oxazol-2-yl)-6,7-dihydro-1H-imidazo[4,5-c]pyridin-5(4H)-yl)methanone |
| 909 | 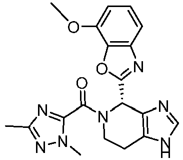 | (S)-(1,3-dimethyl-1H-1,2,4-triazol-5-yl)(4-(7-methoxybenzo[d]oxazol-2-yl)-6,7-dihydro-1H-imidazo[4,5-c]pyridin-5(4H)-yl)methanone |
| 911 | 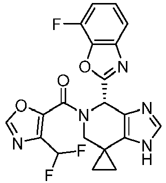 | (S)-(4-(difluoromethyl)oxazol-5-yl)(4'-(7-fluorobenzo[d]oxazol-2-yl)spiro[cyclopropane-1,7'-imidazo[4,5-c]pyridin]-5'(1'H,4'H,6'H)-yl)methanone |
| 913 | 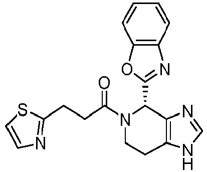 | (S)-1-(4-(benzo[d]oxazol-2-yl)-6,7-dihydro-1H-imidazo[4,5-c]pyridin-5(4H)-yl)-3-(thiazol-2-yl)propan-1-one |
| 915 | 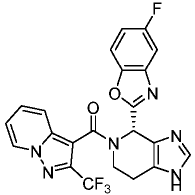 | (S)-(4-(5-fluorobenzo[d]oxazol-2-yl)-6,7-dihydro-1H-imidazo[4,5-c]pyridin-5(4H)-yl)(2-(trifluoromethyl)pyrazolo[1,5-a]pyridin-3-yl)methanone |
| 917 | 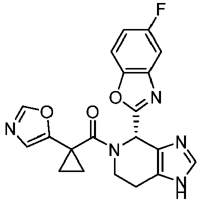 | (S)-(4-(5-fluorobenzo[d]oxazol-2-yl)-6,7-dihydro-1H-imidazo[4,5-c]pyridin-5(4H)-yl)(1-(oxazol-5-yl)cyclopropyl)methanone |
| 919 | 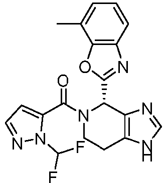 | (S)-(1-(difluoromethyl)- 1H-pyrazol-5-yl)(4-(7-methylbenzo[d]oxazol-2-yl)-6,7-dihydro-1H-imidazo[4,5-c]pyridin-5(4H)-yl)methanone |

In some embodiments, the disclosure provides specific examples of Formula I, and their pharmaceutically acceptable salts and/or isotopologues, as set forth in Table 1R below.

| **Table 1R: Benzo[d]oxazoles** | |
|---|---|
| **Ex. #** | **Name** |
| 2 | (R)-(5-chlorothiazol-2-yl)(4-(5-methylbenzo[d]oxazol-2-yl)-6,7-dihydro-1H-imidazo[4,5-c]pyridin-5(4H)-yl)methanone |
| 4 | (R)-1-(4-(6-bromobenzo[d]oxazol-2-yl)-6,7-dihydro-1H-imidazo[4,5-c]pyridin-5(4H)-yl)-2-cyclopropylethanone |
| 6 | (R)-(4-(5-fluorobenzo[d]oxazol-2-yl)-6,7-dihydro- 1H-imidazo[4,5-c]pyridin-5(4H)-yl)(2-(pyridin-2-yl)thiazol-5-yl)methanone |
| 8 | (R)-(4-(6-bromobenzo[d]oxazol-2-yl)-6,7-dihydro- 1H-imidazo[4,5-c]pyridin-5(4H)-yl)(2-(pyridin-2-yl)oxazol-5-yl)methanone |
| 10 | (R)-(4-(5-fluorobenzo[d]oxazol-2-yl)-6,7-dihydro- 1H-imidazo[4,5-c]pyridin-5(4H)-yl)(2-(1-methyl-1H-pyrazol-4-yl)thiazol-5-yl)methanone |
| 12 | (R)-(4-(5-fluorobenzo[d]oxazol-2-yl)-6,7-dihydro- 1H-imidazo[4,5-c]pyridin-5(4H)-yl)(2-(pyridin-2-yl)oxazol-5-yl)methanone |
| 14 | (R)-(4-(5-fluorobenzo[d]oxazol-2-yl)-6,7-dihydro- 1H-imidazo[4,5-c]pyridin-5(4H)-yl)(2-(2-fluoropropan-2-yl)oxazol-5-yl)methanone |
| 16 | (R)-(5-cyclopropyl-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 18 | (R)-(2-(tert-butyl)thiazol-5-yl)(4-(5-fluorobenzo[d]oxazol-2-yl)-6,7-dihydro-1H-imidazo[4,5-c]pyridin-5(4H)-yl)methanone |
| 20 | (R)-(4-(5-fluorobenzo[d]oxazol-2-yl)-6,7-dihydro- 1H-imidazo[4,5-c]pyridin-5(4H)-yl)(6-methylpyrazolo[1,5-a]pyridin-3-yl)methanone |
| 22 | (R)-(2-(tert-butyl)oxazol-5-yl)(4-(5-fluorobenzo[d]oxazol-2-yl)-6,7-dihydro-1H-imidazo[4,5-c]pyridin-5(4H)-yl)methanone |
| 24 | ((R)-(4-(5-fluorobenzo[d]oxazol-2-yl)-6,7-dihydro- 1H-imidazo[4,5-c]pyridin-5(4H)-yl)(4-(trifluoromethyl)oxazol-5-yl)methanone |
| 26 | (R)-(5-(2,6-difluorophenyl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 28 | (R)-(4-(5-fluorobenzo[d]oxazol-2-yl)-6,7-dihydro-1H-imidazo[4,5-c]pyridin-5(4H)-yl)(5-(4-fluorophenyl)-1,3,4-oxadiazol-2-yl)methanone |
| 30 | (R)-(4-(5-fluorobenzo[d]oxazol-2-yl)-6,7-dihydro-1H-imidazo[4,5-c]pyridin-5(4H)-yl)(6-(methoxymethyl)pyrazolo[1,5-a]pyridin-3-yl)methanone |
| 32 | (R)-(4-(5-fluorobenzo[d]oxazol-2-yl)-6,7-dihydro-1H-imidazo[4,5-c]pyridin-5(4H)-yl)(4-methyl-2-(pyridin-2-yl)oxazol-5-yl)methanone |
| 34 | (R)-(4-(5-fluorobenzo[d]oxazol-2-yl)-6,7-dihydro-1H-imidazo[4,5-c]pyridin-5(4H)-yl)(5-(5-fluoropyridin-2-yl)-1,3,4-oxadiazol-2-yl)methanone |
| 36 | (R)-(2-(1,1-difluoroethyl)thiazol-5-yl)(4-(5-fluorobenzo[d]oxazol-2-yl)-6,7-dihydro-1H-imidazo[4,5-c]pyridin-5(4H)-yl)methanone |
| 38 | (R)-2-cyclopropyl-1-(4-(5-fluorobenzo[d]oxazol-2-yl)-6,7-dihydro-1H-imidazo[4,5-c]pyridin-5(4H)-yl)ethanone |
| 40 | (R)-(4-(5-fluorobenzo[d]oxazol-2-yl)-6,7-dihydro- 1H-imidazo[4,5-c]pyridin-5(4H)-yl)(5-(pyridin-2-yl)-1,3,4-oxadiazol-2-yl)methanone |
| 42 | (R)-(4-cyclopropyloxazol-5-yl)(4-(5-fluorobenzo[d]oxazol-2-yl)-6,7-dihydro-1H-imidazo[4,5-c]pyridin-5(4H)-yl)methanone |
| 44 | (R)-(4-cyclopropyl-2-(pyridin-2-yl)oxazol-5-yl)(4-(5-fluorobenzo[d]oxazol-2-yl)-6,7-dihydro-1H-imidazo[4,5-c]pyridin-5(4H)-yl)methanone |
| 46 | (R)-(4-(5-fluorobenzo[d]oxazol-2-yl)-6,7-dihydro-1H-imidazo[4,5-c]pyridin-5(4H)-yl)(2-(2-hydroxypropan-2-yl)-4-(trifluoromethyl)oxazol-5-yl)methanone |
| 48 | (R)-(6-(2-(dimethylamino)ethyl)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 50 | (R)-(4-(5-fluorobenzo[d]oxazol-2-yl)-6,7-dihydro-1H-imidazo[4,5-c]pyridin-5(4H)-yl)(2-(pyridin-2-yl)-4-(trifluoromethyl)oxazol-5-yl)methanone |
| 52 | (R)-(4-(difluoromethyl)oxazol-5-yl)(4-(5-fluorobenzo[d]oxazol-2-yl)-6,7-dihydro-1H-imidazo[4,5-c]pyridin-5(4H)-yl)methanone |
| 54 | (R)-(2-cyclopropyl-4-(difluoromethyl)oxazol-5-yl)(4-(5-fluorobenzo[d]oxazol-2-yl)-6,7-dihydro-1H-imidazo[4,5-c]pyridin-5(4H)-yl)methanone |
| 56 | (R)-1-(4-(5-fluorobenzo[d]oxazol-2-yl)-6,7-dihydro-1H-imidazo[4,5-c]pyridin-5(4H)-yl)-2-(1-hydroxycyclobutyl)ethanone |
| 58 | (R)-(4-(6-methylbenzo[d]oxazol-2-yl)-6,7-dihydro- 1H-imidazo[4,5-c]pyridin-5(4H)-yl)(4-(trifluoromethyl)oxazol-5-yl)methanone |
| 60 | (R)-1-(4-(5-fluorobenzo[d]oxazol-2-yl)-6,7-dihydro-1H-imidazo[4,5-c]pyridin-5(4H)-yl)-2-(3-fluorocyclobutyl)ethanone |
| 62 | ((S)-2,2-difluorocyclopropyl)((R)-4-(5-fluorobenzo[d]oxazol-2-yl)-6,7-dihydro- 1H-imidazo[4,5-c]pyridin-5(4H)-yl)methanone |
| 64 | (R)-(4-(5-methylbenzo[d]oxazol-2-yl)-6,7-dihydro- 1H-imidazo[4,5-c]pyridin-5(4H)-yl)(4-(trifluoromethyl)oxazol-5-yl)methanone |
| 66 | (R)-(4-(5-fluorobenzo[d]oxazol-2-yl)-6,7-dihydro- 1H-imidazo[4,5-c]pyridin-5(4H)-yl)(6-(4-methylpiperazin-1-yl)pyrazolo[1,5-a]pyridin-3-yl)methanone |
| 68 | (R)-(4-(5-fluorobenzo[d]oxazol-2-yl)-6,7-dihydro- 1H-imidazo[4,5-c]pyridin-5(4H)-yl)(5-(1-methyl-1H-pyrazol-4-yl)-1,3,4-oxadiazol-2-yl)methanone |
| 70 | (R)-(6-bromopyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 72 | (R)-(4-(5-fluorobenzo[d]oxazol-2-yl)-6,7-dihydro- 1H-imidazo[4,5-c]pyridin-5(4H)-yl)(6-(1-methyl-1H-pyrazol-4-yl)pyrazolo[1,5-a]pyridin-3-yl)methanone |
| 74 | (R)-(4-(5-fluorobenzo[d]oxazol-2-yl)-6,7-dihydro- 1H-imidazo[4,5-c]pyridin-5(4H)-yl)(6-methoxypyrazolo[1,5-a]pyridin-3-yl)methanone |
| 76 | (R)-(5-(3,4-difluorophenyl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 78 | (R)-(5-(4-fluoro-2-methylphenyl)- 1,3,4-oxadiazol-2-yl)(4-(5-fluorobenzo[d]oxazol-2-yl)-6,7-dihydro-1H-imidazo[4,5-c]pyridin-5(4H)-yl)methanone |
| 80 | (R)-(5-chloro-6-(2-(dimethylamino)ethyl)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 82 | (R)-(4-(5-fluorobenzo[d]oxazol-2-yl)-6,7-dihydro- 1H-imidazo[4,5-c]pyridin-5(4H)-yl)(6-(pyridin-2-yl)pyrazolo[1,5-a]pyridin-3-yl)methanone |
| 83 | (R)-(5-(2,4-difluorophenyl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 86 | (R)-(4-(difluoromethyl)-2-(pyridin-2-yl)oxazol-5-yl)(4-(5-fluorobenzo[d]oxazol-2-yl)-6,7-dihydro-1H-imidazo[4,5-c]pyridin-5(4H)-yl)methanone |
| 87 | (R)-(4-(difluoromethyl)-2-(1-methyl-1H-pyrazol-4-yl)oxazol-5-yl)(4-(5-fluorobenzo[d]oxazol-2-yl)-6,7-dihydro-1H-imidazo[4,5-c]pyridin-5(4H)-yl)methanone |
| 89 | (R)-(4-(5-fluorobenzo[d]oxazol-2-yl)-6,7-dihydro-1H-imidazo[4,5-c]pyridin-5(4H)-yl)(5-(4-(trifluoromethyl)phenyl)-1,3,4-oxadiazol-2-yl)methanone |
| 92 | (R)-(6-(dimethylamino)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 94 | (R)-(1-(difluoromethyl)-1H-pyrazol-5-yl)(4-(5-fluorobenzo[d]oxazol-2-yl)-6,7-dihydro-1H-imidazo[4,5-c]pyridin-5(4H)-yl)methanone |
| 96 | (R)-(3-(difluoromethyl)-1-methyl-1H-pyrazol-4-yl)(4-(5-fluorobenzo[d]oxazol-2-yl)-6,7-dihydro-1H-imidazo[4,5-c]pyridin-5(4H)-yl)methanone |
| 98 | (R)-(4-(5-methylbenzo[d]oxazol-2-yl)-6,7-dihydro- 1H-imidazo[4,5-c]pyridin-5(4H)-yl)(5-(pyridin-2-yl)-1,3,4-oxadiazol-2-yl)methanone |
| 100 | (R)-(5-(5-fluoropyridin-2-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 102 | (R)-(4-(6-methylbenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 104 | (R)-(5-(1-methyl-1H-pyrazol-4-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 105 | (R)-(4-(5-fluorobenzo[d]oxazol-2-yl)-6,7-dihydro-1H-imidazo[4,5-c]pyridin-5(4H)-yl)(5-(1-methyl-1H-pyrazol-3-yl)-1,3,4-oxadiazol-2-yl)methanone |
| 108 | (R)-(4-(difluoromethyl)-2-(2-hydroxypropan-2-yl)oxazol-5-yl)(4-(5-fluorobenzo[d]oxazol-2-yl)-6,7-dihydro-1H-imidazo[4,5-c]pyridin-5(4H)-yl)methanone |
| 109 | (R)-(4-(difluoromethyl)-2-(methoxymethyl)oxazol-5-yl)(4-(5-fluorobenzo[d]oxazol-2-yl)-6,7-dihydro-1H-imidazo[4,5-c]pyridin-5(4H)-yl)methanone |
| 112 | (R)-(5-(1-methyl-1H-pyrazol-4-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 114 | (R)-(5-(1-(difluoromethyl)-1H-pyrazol-3-yl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 116 | (R)-(4-(difluoromethyl)-2-((trifluoromethoxy)methyl)oxazol-5-yl)(4-(5-fluorobenzo[d]oxazol-2-yl)-6,7-dihydro-1H-imidazo[4,5-c]pyridin-5(4H)-yl)methanone |
| 117 | (R)-(4-(5-fluorobenzo[d]oxazol-2-yl)-6,7-dihydro-1H-imidazo[4,5-c]pyridin-5(4H)-yl)(5-(1-methyl-3-(trifluoromethyl)-1H-pyrazol-4-yl)-1,3,4-oxadiazol-2-yl)methanone |
| 120 | (R)-(5-(1,4-dimethyl-1H-pyrazol-5-yl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 121 | [(4R)-4-(5-fluoro-1,3-benzoxazol-2-yl)-1,4,6,7-tetrahydroimidazo[4,5-c]pyridin-5-yl]-[5-[4-(trifluoromethyl)-2-pyridyl]-1,3,4-oxadiazol-2-yl]methanone |
| 123 | (R)-(4-bromo-2-(2-hydroxypropan-2-yl)oxazol-5-yl)(4-(5-fluorobenzo[d]oxazol-2-yl)-6,7-dihydro-1H-imidazo[4,5-c]pyridin-5(4H)-yl)methanone |
| 125 | (R)-(4-(5-fluorobenzo[d]oxazol-2-yl)-6,7-dihydro-1H-imidazo[4,5-c]pyridin-5(4H)-yl)(5-(5-methylpyridin-2-yl)-1,3,4-oxadiazol-2-yl)methanone |
| 128 | (R)-(3-chloro-1-methyl-1H-pyrazol-5-yl)(4-(5-fluorobenzo[d]oxazol-2-yl)-6,7-dihydro-1H-imidazo[4,5-c]pyridin-5(4H)-yl)methanone |
| 130 | (R)-(4-chloro-2-(2-hydroxypropan-2-yl)oxazol-5-yl)(4-(5-fluorobenzo[d]oxazol-2-yl)-6,7-dihydro-1H-imidazo[4,5-c]pyridin-5(4H)-yl)methanone |
| 132 | (R)-(4-(5-fluorobenzo[d]oxazol-2-yl)-6,7-dihydro-1H-imidazo[4,5-c]pyridin-5(4H)-yl)(5-(1-methyl-1H-pyrazol-5-yl)-1,3,4-oxadiazol-2-yl)methanone |
| 134 | (R)-(6-(4-cyclopropylpiperazin-1-yl)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 136 | ((R)-4-(5-fluorobenzo[d]oxazol-2-yl)-6,7-dihydro-1H-imidazo[4,5-c]pyridin-5(4H)-yl)(2-((R)-1-hydroxyethyl)-4-methyloxazol-5-yl)methanone |
| 138 | (R)-(4-(difluoromethyl)oxazol-5-yl)(4-(5-methylbenzo[d]oxazol-2-yl)-6,7-dihydro-1H-imidazo[4,5-c]pyridin-5(4H)-yl)methanone |
| 140 | (R)-(4-(5-fluorobenzo[d]oxazol-2-yl)-6,7-dihydro-1H-imidazo[4,5-c]pyridin-5(4H)-yl)(6-(3,3,4-trimethylpiperazin-1-yl)pyrazolo[1,5-a]pyridin-3-yl)methanone |
| 142 | (R)-(4-(difluoromethyl)-2-(1-hydroxycyclopropyl)oxazol-5-yl)(4-(5-fluorobenzo[d]oxazol-2-yl)-6,7-dihydro-1H-imidazo[4,5-c]pyridin-5(4H)-yl)methanone |
| 144 | (R)-(4-(6-bromobenzo[d]oxazol-2-yl)-6,7-dihydro- 1H-imidazo[4,5-c]pyridin-5(4H)-yl)(4-(trifluoromethyl)oxazol-5-yl)methanone |
| 146 | (R)-(4-(6-bromobenzo[d]oxazol-2-yl)-6,7-dihydro- 1H-imidazo[4,5-c]pyridin-5(4H)-yl)(4-(difluoromethyl)oxazol-5-yl)methanone |
| 148 | (R)-(4-(5-bromobenzo[d]oxazol-2-yl)-6,7-dihydro- 1H-imidazo[4,5-c]pyridin-5(4H)-yl)(4-(trifluoromethyl)oxazol-5-yl)methanone |
| 150 | (R)-(4-(5-bromobenzo[d]oxazol-2-yl)-6,7-dihydro- 1H-imidazo[4,5-c]pyridin-5(4H)-yl)(4-(difluoromethyl)oxazol-5-yl)methanone |
| 152 | (4-(difluoromethyl)-2-((R)-1-hydroxyethyl)oxazol-5-yl)((R)-4-(5-fluorobenzo[d]oxazol-2-yl)-6,7-dihydro-1H-imidazo[4,5-c]pyridin-5(4H)-yl)methanone |
| 154 | (R)-(4-(1,1-difluoroethyl)oxazol-5-yl)(4-(5-fluorobenzo[d]oxazol-2-yl)-6,7-dihydro-1H-imidazo[4,5-c]pyridin-5(4H)-yl)methanone |
| 156 | (R)-(4-(difluoromethyl)-2-((methoxy-d3)methyl)oxazol-5-yl)(4-(5-fluorobenzo[d]oxazol-2-yl)-1,4,6,7-tetrahydro-5H-imidazo[4,5-c]pyridin-5-yl)methanone |
| 158 | (4-(difluoromethyl)-2-((S)-1-hydroxyethyl)oxazol-5-yl)((R)-4-(5-fluorobenzo[d]oxazol-2-yl)-6,7-dihydro-1H-imidazo[4,5-c]pyridin-5(4H)-yl)methanone |
| 160 | (R)-2-(4-(difluoromethyl)-5-(4-(5-fluorobenzo[d]oxazol-2-yl)-4,5,6,7-tetrahydro-1H-imidazo[4,5-c]pyridine-5-carbonyl)oxazol-2-yl)-2-methylpropanenitrile |
| 162 | (R)-(4-(6-fluorobenzo[d]oxazol-2-yl)-6,7-dihydro- 1H-imidazo[4,5-c]pyridin-5(4H)-yl)(4-(trifluoromethyl)oxazol-5-yl)methanone |
| 164 | (R)-(4-(difluoromethyl)oxazol-5-yl)(4-(6-fluorobenzo[d]oxazol-2-yl)-6,7-dihydro-1H-imidazo[4,5-c]pyridin-5(4H)-yl)methanone |
| 166 | (R)-(6-bromo-5-chloropyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 168 | (R)-(4-(4-fluorobenzo[d]oxazol-2-yl)-6,7-dihydro-1H-imidazo[4,5-c]pyridin-5(4H)-yl)(4-(trifluoromethyl)oxazol-5-yl)methanone |
| 170 | (R)-(4-(difluoromethyl)oxazol-5-yl)(4-(4-fluorobenzo[d]oxazol-2-yl)-6,7-dihydro-1H-imidazo[4,5-c]pyridin-5(4H)-yl)methanone |
| 172 | (R)-(4-(7-fluorobenzo[d]oxazol-2-yl)-6,7-dihydro-1H-imidazo[4,5-c]pyridin-5(4H)-yl)(4-(trifluoromethyl)oxazol-5-yl)methanone |
| 174 | (R)-(4-(difluoromethyl)oxazol-5-yl)(4-(7-fluorobenzo[d]oxazol-2-yl)-6,7-dihydro-1H-imidazo[4,5-c]pyridin-5(4H)-yl)methanone |
| 176 | (R)-(5-chloro-6-morpholinopyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 178 | (R)-(6-amino-5-chloropyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 180 | (R)-(4-(5-fluorobenzo[d]oxazol-2-yl)-6,7-dihydro- 1H-imidazo[4,5-c]pyridin-5(4H)-yl)(4-(fluoromethyl)oxazol-5-yl)methanone |
| 182 | (R)-(5-chloro-6-(4-methylpiperazin-1-yl)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 184 | (R)-(5-(difluoromethyl)-1-(2,2,2-trifluoroethyl)-1H-pyrazol-4-yl)(4-(5-fluorobenzo[d]oxazol-2-yl)-6,7-dihydro-1H-imidazo[4,5-c]pyridin-5(4H)-yl)methanone |
| 186 | (R)-2-(5-(4-(5-fluorobenzo[d]oxazol-2-yl)-4,5,6,7-tetrahydro-1H-imidazo[4,5-c]pyridine-5-carbonyl)-4-methyloxazol-2-yl)-2-methylpropanenitrile |
| 188 | (R)-(4-(difluoromethyl)-2-(2-hydroxypropan-2-yl)oxazol-5-yl)(4-(6-fluorobenzo[d]oxazol-2-yl)-6,7-dihydro-1H-imidazo[4,5-c]pyridin-5(4H)-yl)methanone |
| 190 | (R)-(1-(difluoromethyl)-1H-pyrazol-5-yl)(4-(6-fluorobenzo[d]oxazol-2-yl)-6,7-dihydro-1H-imidazo[4,5-c]pyridin-5(4H)-yl)methanone |
| 192 | (R)-(4-(7-fluorobenzo[d]oxazol-2-yl)-6,7-dihydro- 1H-imidazo[4,5-c]pyridin-5(4H)-yl)(5-(1-methyl-1H-pyrazol-4-yl)-1,3,4-oxadiazol-2-yl)methanone |
| 194 | (R)-(4-(difluoromethyl)-2-(2-hydroxypropan-2-yl)oxazol-5-yl)(4-(7-fluorobenzo[d]oxazol-2-yl)-6,7-dihydro-1H-imidazo[4,5-c]pyridin-5(4H)-yl)methanone |
| 196 | (R)-(1-(difluoromethyl)-1H-pyrazol-5-yl)(4-(7-fluorobenzo[d]oxazol-2-yl)-6,7-dihydro-1H-imidazo[4,5-c]pyridin-5(4H)-yl)methanone |
| 198 | (R)-(4-(4-fluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 200 | (R)-(4-(difluoromethyl)-2-(2-hydroxypropan-2-yl)oxazol-5-yl)(4-(4-fluorobenzo[d]oxazol-2-yl)-6,7-dihydro-1H-imidazo[4,5-c]pyridin-5(4H)-yl)methanone |
| 202 | (R)-(1-(difluoromethyl)-1H-pyrazol-5-yl)(4-(4-fluorobenzo[d]oxazol-2-yl)-6,7-dihydro-1H-imidazo[4,5-c]pyridin-5(4H)-yl)methanone |
| 204 | (R)-(5-chloro-6-((dimethylamino)methyl)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanonee |
| 206 | (R)-(5-chloro-6-(methylamino)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 208 | (R)-(5-chloro-6-(dimethylamino)pyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 209 | ((R)-4-(4-fluorobenzo[d]oxazol-2-yl)-6,7-dihydro-1H-imidazo[4,5-c]pyridin-5(4H)-yl)(2-((R)-1-hydroxyethyl)-4-methyloxazol-5-yl)methanone |
| 212 | (R)-(4-(5-fluorobenzo[d]oxazol-2-yl)-6,7-dihydro- 1H-imidazo[4,5-c]pyridin-5(4H)-yl)(4-(fluoromethyl)-2-(2-hydroxypropan-2-yl)oxazol-5-yl)methanone |
| 213 | (R)-(4-(difluoromethyl)-2-(1-hydroxycyclopropyl)oxazol-5-yl)(4-(7-fluorobenzo[d]oxazol-2-yl)-6,7-dihydro-1H-imidazo[4,5-c]pyridin-5(4H)-yl)methanone |
| 216 | (R)-(4-(difluoromethyl)-2-(1-hydroxycyclopropyl)oxazol-5-yl)(4-(4-fluorobenzo[d]oxazol-2-yl)-6,7-dihydro-1H-imidazo[4,5-c]pyridin-5(4H)-yl)methanone |
| 218 | ((R)-4-(7-fluorobenzo[d]oxazol-2-yl)-6,7-dihydro-1H-imidazo[4,5-c]pyridin-5(4H)-yl)(2-((R)-1-hydroxyethyl)-4-methyloxazol-5-yl)methanone |
| 220 | (R)-(5-chloro-6-fluoropyrazolo[1,5-a]pyridin-3-yl)(4-(5-fluorobenzo[d]oxazol-2-yl)-6,7-dihydro-1H-imidazo[4,5-c]pyridin-5(4H)-yl)methanone |
| 222 | (R)-(4-(difluoromethyl)-2-(2-(methylamino)propan-2-yl)oxazol-5-yl)(4-(5-fluorobenzo[d]oxazol-2-yl)-6,7-dihydro-1H-imidazo[4,5-c]pyridin-5(4H)-yl)methanone |
| 223 | (4-bromo-2-(1-hydroxyethyl)oxazol-5-yl)((R)-4-(5-fluorobenzo[d]oxazol-2-yl)-6,7-dihydro-1H-imidazo[4,5-c]pyridin-5(4H)-yl)methanone Diastereomer #1 (Rt=5.902 min) |
| 224 | (4-bromo-2-(1-hydroxyethyl)oxazol-5-yl)((R)-4-(5-fluorobenzo[d]oxazol-2-yl)-6,7-dihydro-1H-imidazo[4,5-c]pyridin-5(4H)-yl)methanone |
| | Diastereomer #2 (Rt=6.092 min) |
| 228 | (4-(difluoromethyl)-2-((S)-1-hydroxyethyl)oxazol-5-yl)((R)-4-(4-fluorobenzo[d]oxazol-2-yl)-6,7-dihydro-1H-imidazo[4,5-c]pyridin-5(4H)-yl)methanone |
| 230 | (4-(difluoromethyl)-2-((R)-1-hydroxyethyl)oxazol-5-yl)((R)-4-(4-fluorobenzo[d]oxazol-2-yl)-6,7-dihydro-1H-imidazo[4,5-c]pyridin-5(4H)-yl)methanone |
| 232 | (4-(difluoromethyl)-2-((S)-1-hydroxyethyl)oxazol-5-yl)((R)-4-(6-fluorobenzo[d]oxazol-2-yl)-6,7-dihydro-1H-imidazo[4,5-c]pyridin-5(4H)-yl)methanone |
| 233 | (4-chloro-2-(1-hydroxyethyl)oxazol-5-yl)((R)-4-(5-fluorobenzo[d]oxazol-2-yl)-6,7-dihydro-1H-imidazo[4,5-c]pyridin-5(4H)-yl)methanone |
| | Diastereomer #1 (Rt=5.433 min) |
| 234 | (4-chloro-2-(1-hydroxyethyl)oxazol-5-yl)((R)-4-(5-fluorobenzo[d]oxazol-2-yl)-6,7-dihydro-1H-imidazo[4,5-c]pyridin-5(4H)-yl)methanone |
| | Diastereomer #2 (Rt=5.625 min) |
| 238 | (R)-(4-(difluoromethyl)-2-(1-hydroxycyclopropyl)oxazol-5-yl)(4-(6-fluorobenzo[d]oxazol-2-yl)-6,7-dihydro-1H-imidazo[4,5-c]pyridin-5(4H)-yl)methanone |
| 240 | (4-(difluoromethyl)-2-((R)-1-hydroxyethyl)oxazol-5-yl)((R)-4-(6-fluorobenzo[d]oxazol-2-yl)-6,7-dihydro-1H-imidazo[4,5-c]pyridin-5(4H)-yl)methanone |
| 242 | ((R)-4-(6-fluorobenzo[d]oxazol-2-yl)-6,7-dihydro- 1H-imidazo[4,5-c]pyridin-5(4H)-yl)(2-((S)-1-hydroxyethyl)-4-methyloxazol-5-yl)methanone |
| 244 | (4-(difluoromethyl)-2-((S)-1-hydroxyethyl)oxazol-5-yl)((R)-4-(7-fluorobenzo[d]oxazol-2-yl)-6,7-dihydro-1H-imidazo[4,5-c]pyridin-5(4H)-yl)methanone |
| 246 | (4-(difluoromethyl)-2-((R)-1-hydroxyethyl)oxazol-5-yl)((R)-4-(7-fluorobenzo[d]oxazol-2-yl)-6,7-dihydro-1H-imidazo[4,5-c]pyridin-5(4H)-yl)methanone |
| 248 | ((R)-4-(7-fluorobenzo[d]oxazol-2-yl)-6,7-dihydro-1H-imidazo[4,5-c]pyridin-5(4H)-yl)(2-((S)-1-hydroxyethyl)-4-methyloxazol-5-yl)methanone |
| 250 | (R)-(4-bromo-2-(2-hydroxypropan-2-yl)oxazol-5-yl)(4-(6-fluorobenzo[d]oxazol-2-yl)-6,7-dihydro-1H-imidazo[4,5-c]pyridin-5(4H)-yl)methanone |
| 251 | (R)-(4-bromo-2-(2-hydroxypropan-2-yl)oxazol-5-yl)(4-(4-fluorobenzo[d]oxazol-2-yl)-6,7-dihydro-1H-imidazo[4,5-c]pyridin-5(4H)-yl)methanone |
| 254 | (R)-(4-(7-fluorobenzo[d]oxazol-2-yl)-6,7-dihydro-1H-imidazo[4,5-c]pyridin-5(4H)-yl)(2-(2-hydroxypropan-2-yl)-4-(trifluoromethyl)oxazol-5-yl)methanone |
| 256 | (R)-(4-(6-fluorobenzo[d]oxazol-2-yl)-6,7-dihydro-1H-imidazo[4,5-c]pyridin-5(4H)-yl)(2-(2-hydroxypropan-2-yl)-4-(trifluoromethyl)oxazol-5-yl)methanone |
| 258 | (R)-(4-(4-fluorobenzo[d]oxazol-2-yl)-6,7-dihydro-1H-imidazo[4,5-c]pyridin-5(4H)-yl)(2-(2-hydroxypropan-2-yl)-4-(trifluoromethyl)oxazol-5-yl)methanone |
| 260 | (R)-(4-(4-fluorobenzo[d]oxazol-2-yl)-6,7-dihydro-1H-imidazo[4,5-c]pyridin-5(4H)-yl)(2-(2-hydroxypropan-2-yl)-4-methyloxazol-5-yl)methanone |
| 262 | (R)-(4-(5-fluorobenzo[d]oxazol-2-yl)-6,7-dihydro-1H-imidazo[4,5-c]pyridin-5(4H)-yl)(1-(trifluoromethyl)-1H-pyrazol-5-yl)methanone |
| 264 | (R)-(4-bromo-2-(2-hydroxypropan-2-yl)oxazol-5-yl)(4-(7-fluorobenzo[d]oxazol-2-yl)-6,7-dihydro-1H-imidazo[4,5-c]pyridin-5(4H)-yl)methanone |
| 266 | (R)-(4-(7-fluorobenzo[d]oxazol-2-yl)-6,7-dihydro-1H-imidazo[4,5-c]pyridin-5(4H)-yl)(6-(methoxymethyl)pyrazolo[1,5-a]pyridin-3-yl)methanone |
| 267 | (R)-(4-(7-fluorobenzo[d]oxazol-2-yl)-6,7-dihydro-1H-imidazo[4,5-c]pyridin-5(4H)-yl)(2-(2-hydroxypropan-2-yl)-4-methyloxazol-5-yl)methanone |
| 270 | (R)-(4-chloro-2-(2-hydroxypropan-2-yl)oxazol-5-yl)(4-(6-fluorobenzo[d]oxazol-2-yl)-6,7-dihydro-1H-imidazo[4,5-c]pyridin-5(4H)-yl)methanone |
| 271 | (R)-(4-chloro-2-(2-hydroxypropan-2-yl)oxazol-5-yl)(4-(7-fluorobenzo[d]oxazol-2-yl)-6,7-dihydro-1H-imidazo[4,5-c]pyridin-5(4H)-yl)methanone |
| 273 | (R)-(4-chloro-2-(2-hydroxypropan-2-yl)oxazol-5-yl)(4-(4-fluorobenzo[d]oxazol-2-yl)-6,7-dihydro-1H-imidazo[4,5-c]pyridin-5(4H)-yl)methanone |
| 276 | (R)-(4-(4-fluorobenzo[d]oxazol-2-yl)-6,7-dihydro-1H-imidazo[4,5-c]pyridin-5(4H)-yl)(6-(methoxymethyl)pyrazolo[1,5-a]pyridin-3-yl)methanone |
| 278 | (R)-(3-chloro-1-methyl-1H-1,2,4-triazol-5-yl)(4-(4-fluorobenzo[d]oxazol-2-yl)-6,7-dihydro-1H-imidazo[4,5-c]pyridin-5(4H)-yl)methanone |
| 280 | (R)-(4-(7-fluorobenzo[d]oxazol-2-yl)-6,7-dihydro-1H-imidazo[4,5-c]pyridin-5(4H)-yl)(1-methyl-3-(trifluoromethyl)-1H-1,2,4-triazol-5-yl)methanone |
| 282 | (R)-(4-(7-fluorobenzo[d]oxazol-2-yl)-6,7-dihydro-1H-imidazo[4,5-c]pyridin-5(4H)-yl)(oxazol-5-yl)methanone |
| 284 | (R)-(3-chloro-1-methyl-1H-1,2,4-triazol-5-yl)(4-(7-fluorobenzo[d]oxazol-2-yl)-6,7-dihydro-1H-imidazo[4,5-c]pyridin-5(4H)-yl)methanone |
| 286 | (R)-(3-(difluoromethyl)-1-methyl-1H-1,2,4-triazol-5-yl)(4-(7-fluorobenzo[d]oxazol-2-yl)-6,7-dihydro-1H-imidazo[4,5-c]pyridin-5(4H)-yl)methanone |
| 288 | (R)-(4-(6-fluorobenzo[d]oxazol-2-yl)-6,7-dihydro-1H-imidazo[4,5-c]pyridin-5(4H)-yl)(1-(trifluoromethyl)-1H-pyrazol-5-yl)methanone |
| 290 | (R)-(4-(7-fluorobenzo[d]oxazol-2-yl)-6,7-dihydro-1H-imidazo[4,5-c]pyridin-5(4H)-yl)(1-(trifluoromethyl)-1H-pyrazol-5-yl)methanone |
| 292 | (R)-(4-(difluoromethyl)-2-(2-(dimethylamino)propan-2-yl)oxazol-5-yl)(4-(5-fluorobenzo[d]oxazol-2-yl)-6,7-dihydro-1H-imidazo[4,5-c]pyridin-5(4H)-yl)methanone |
| 294 | (R)-(4-(4-fluorobenzo[d]oxazol-2-yl)-6,7-dihydro-1H-imidazo[4,5-c]pyridin-5(4H)-yl)(1-(trifluoromethyl)-1H-pyrazol-5-yl)methanone |
| 296 | (R)-(4-(5,6-difluorobenzo[d]oxazol-2-yl)-6,7-dihydro-1H-imidazo[4,5-c]pyridin-5(4H)-yl)(4-(difluoromethyl)oxazol-5-yl)methanone |
| 298 | (R)-(4-(6,7-difluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 300 | (R)-(4-(4,6-difluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 302 | (R)-(4-(4,6-difluorobenzo[d]oxazol-2-yl)-6,7-dihydro-1H-imidazo[4,5-c]pyridin-5(4H)-yl)(4-(difluoromethyl)oxazol-5-yl)methanone |
| 304 | (R)-(4-(6-fluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 306 | (R)-(4-(6,7-difluorobenzo[d]oxazol-2-yl)-6,7-dihydro-1H-imidazo[4,5-c]pyridin-5(4H)-yl)(4-(difluoromethyl)oxazol-5-yl)methanone |
| 308 | (R)-(4-(5,7-difluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 310 | (R)-(4-(5,7-difluorobenzo[d]oxazol-2-yl)-6,7-dihydro-1H-imidazo[4,5-c]pyridin-5(4H)-yl)(4-(difluoromethyl)oxazol-5-yl)methanone |
| 312 | (R)-(4-(difluoromethyl)-2-(2-hydroxypropan-2-yl)oxazol-5-yl)(4-(5-methylbenzo[d]oxazol-2-yl)-6,7-dihydro-1H-imidazo[4,5-c]pyridin-5(4H)-yl)methanone |
| 314 | (R)-(1-(difluoromethyl)-1H-pyrazol-5-yl)(4-(5-methylbenzo[d]oxazol-2-yl)-6,7-dihydro-1H-imidazo[4,5-c]pyridin-5(4H)-yl)methanone |
| 316 | (R)-(4-(difluoromethyl)-2-(2-hydroxypropan-2-yl)oxazol-5-yl)(4-(6-methylbenzo[d]oxazol-2-yl)-6,7-dihydro-1H-imidazo[4,5-c]pyridin-5(4H)-yl)methanone |
| 318 | (R)-(1-(difluoromethyl)-1H-pyrazol-5-yl)(4-(6-methylbenzo[d]oxazol-2-yl)-6,7-dihydro-1H-imidazo[4,5-c]pyridin-5(4H)-yl)methanone |
| 320 | (R)-(2-(2-hydroxypropan-2-yl)-4-methyloxazol-5-yl)(4-(6-methylbenzo[d]oxazol-2-yl)-6,7-dihydro-1H-imidazo[4,5-c]pyridin-5(4H)-yl)methanone |
| 322 | (R)-(4-(6-fluorobenzo[d]oxazol-2-yl)-6,7-dihydro-1H-imidazo[4,5-c]pyridin-5(4H)-yl)(5-(1-methyl-1H-pyrazol-3-yl)-1,3,4-oxadiazol-2-yl)methanone |
| 324 | (R)-(4-(7-fluorobenzo[d]oxazol-2-yl)-6,7-dihydro-1H-imidazo[4,5-c]pyridin-5(4H)-yl)(6-methylpyrazolo[1,5-a]pyridin-3-yl)methanone |
| 326 | (R)-(4-(7-fluorobenzo[d]oxazol-2-yl)-6,7-dihydro-1H-imidazo[4,5-c]pyridin-5(4H)-yl)(6-methoxypyrazolo[1,5-a]pyridin-3-yl)methanone |
| 328 | (R)-(4-(difluoromethyl)-2-(2-hydroxypropan-2-yl)oxazol-5-yl)(4-(5-(trifluoromethyl)benzo[d]oxazol-2-yl)-6,7-dihydro-1H-imidazo[4,5-c]pyridin-5(4H)-yl)methanone |
| 330 | (R)-(4-(difluoromethyl)oxazol-5-yl)(4-(5-(trifluoromethyl)benzo[d]oxazol-2-yl)-6,7-dihydro-1H-imidazo[4,5-c]pyridin-5(4H)-yl)methanone |
| 332 | (R)-(2-(2-hydroxypropan-2-yl)-4-methyloxazol-5-yl)(4-(5-methylbenzo[d]oxazol-2-yl)-6,7-dihydro-1H-imidazo[4,5-c]pyridin-5(4H)-yl)methanone |
| 334 | (R)-(4-(4-fluorobenzo[d]oxazol-2-yl)-6,7-dihydro- 1H-imidazo[4,5-c]pyridin-5(4H)-yl)(5-(pyridin-2-yl)-1,3,4-oxadiazol-2-yl)methanone |
| 336 | (R)-(4-(7-fluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 338 | (R)-(4-(7-fluorobenzo[d]oxazol-2-yl)-6,7-dihydro-1H-imidazo[4,5-c]pyridin-5(4H)-yl)(5-(1-methyl-1H-pyrazol-3-yl)-1,3,4-oxadiazol-2-yl)methanone |
| 340 | (R)-(4-(difluoromethyl)-2-(2-hydroxypropan-2-yl)oxazol-5-yl)(4-(5-methoxybenzo[d]oxazol-2-yl)-6,7-dihydro-1H-imidazo[4,5-c]pyridin-5(4H)-yl)methanone |
| 342 | (R)-(4-(difluoromethyl)oxazol-5-yl)(4-(5-methoxybenzo[d]oxazol-2-yl)-6,7-dihydro-1H-imidazo[4,5-c]pyridin-5(4H)-yl)methanone |
| 344 | (R)-(3-chloro-1-methyl-1H-1,2,4-triazol-5-yl)(4-(6-fluorobenzo[d]oxazol-2-yl)-6,7-dihydro-1H-imidazo[4,5-c]pyridin-5(4H)-yl)methanone |
| 346 | (R)-(4-(4,7-difluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 348 | (R)-(4-(4,5-difluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 349 | (R)-(4-(4,5-difluorobenzo[d]oxazol-2-yl)-6,7-dihydro-1H-imidazo[4,5-c]pyridin-5(4H)-yl)(4-(difluoromethyl)oxazol-5-yl)methanone |
| 352 | (R)-(4-(difluoromethyl)-2-(2-hydroxypropan-2-yl)oxazol-5-yl)(4-(5-(trifluoromethoxy)benzo[d]oxazol-2-yl)-6,7-dihydro- 1H-imidazo[4,5-c]pyridin-5(4H)-vl)methanone |
| 353 | (R)-(4-(difluoromethyl)oxazol-5-yl)(4-(5-(trifluoromethoxy)benzo[d]oxazol-2-yl)-6,7-dihydro-1H-imidazo[4,5-c]pyridin-5(4H)-yl)methanone |
| 356 | (R)-(5-(1H-pyrazol-3-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 358 | (R)-(5-(1-methyl-1H-pyrazol-3-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 360 | (R)-(5-(1H-pyrazol-3-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 362 | (R)-(4-(difluoromethyl)-2-(2-hydroxypropan-2-yl)oxazol-5-yl)(4-(4-methylbenzo[d]oxazol-2-yl)-6,7-dihydro-1H-imidazo[4,5-c]pyridin-5(4H)-yl)methanone |
| 364 | (R)-(4-(7-fluorobenzo[d]oxazol-2-yl)-6,7-dihydro-1H-imidazo[4,5-c]pyridin-5(4H)-yl)(6-(4-methylpiperazin-1-yl)pyrazolo[1,5-a]pyridin-3-yl)methanone |
| 366 | (R)-(4-(7-fluorobenzo[d]oxazol-2-yl)-6,7-dihydro-1H-imidazo[4,5-c]pyridin-5(4H)-yl)(6-morpholinopyrazolo[1,5-a]pyridin-3-yl)methanone |
| 367 | (R)-(4-(7-fluorobenzo[d]oxazol-2-yl)-6,7-dihydro-1H-imidazo[4,5-c]pyridin-5(4H)-yl)(6-(4-(2-hydroxypropan-2-yl)piperidin-1-yl)pyrazolo[1,5-a]pyridin-3-yl)methanone |
| 370 | (R)-(4-(7-fluorobenzo[d]oxazol-2-yl)-6,7-dihydro- 1H-imidazo[4,5-c]pyridin-5(4H)-yl)(6-(4-fluoropiperidin-1-yl)pyrazolo[1,5-a]pyridin-3-yl)methanone |
| 372 | (R)-(4-(7-fluorobenzo[d]oxazol-2-yl)-6,7-dihydro- 1H-imidazo[4,5-c]pyridin-5(4H)-yl)(6-(4-hydroxypiperidin-1-yl)pyrazolo[1,5-a]pyridin-3-yl)methanone |
| 374 | (R)-(4-(4,7-difluorobenzo[d]oxazol-2-yl)-6,7-dihydro-1H-imidazo[4,5-c]pyridin-5(4H)-yl)(4-(difluoromethyl)oxazol-5-yl)methanone |
| 376 | (R)-(4-(5-chlorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 377 | (R)-(4-(5-chlorobenzo[d]oxazol-2-yl)-6,7-dihydro-1H-imidazo[4,5-c]pyridin-5(4H)-yl)(4-(difluoromethyl)oxazol-5-yl)methanone |
| 380 | (R)-(4-(4-fluorobenzo[d]oxazol-2-yl)-1,4,6,7-tetrahydro-5H-imidazo[4,5-c]pyridin-5-yl)(5-(1-methyl-1H-pyrazol-3-yl)-1,3,4-oxadiazol-2-yl)methanone |
| 381 | (R)-(4-(difluoromethyl)oxazol-5-yl)(4-(4-methylbenzo[d]oxazol-2-yl)-6,7-dihydro-1H-imidazo[4,5-c]pyridin-5(4H)-yl)methanone |
| 384 | (R)-(4-(7-fluorobenzo[d]oxazol-2-yl)-6,7-dihydro-1H-imidazo[4,5-c]pyridin-5(4H)-yl)(6-(1-methyl-1H-pyrazol-4-yl)pyrazolo[1,5-a]pyridin-3-yl)methanone |
| 386 | 1-[(4R)-4-(7-fluoro-1,3-benzoxazol-2-yl)-1,4,6,7-tetrahydroimidazo[4,5-c]pyridin-5-yl]-3-thiazol-2-yl-propan-1-one |
| 388 | (R)-1-(4-(7-fluorobenzo[d]oxazol-2-yl)-6,7-dihydro-1H-imidazo[4,5-c]pyridin-5(4H)-yl)-3-(oxazol-2-yl)propan-1-one |
| 390 | (R)-(5-(1-methyl-1H-pyrazol-3-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 391 | (R)-(4-(difluoromethyl)-2-(2-hydroxypropan-2-yl)oxazol-5-yl)(4-(7-methylbenzo[d]oxazol-2-yl)-6,7-dihydro-1H-imidazo[4,5-c]pyridin-5(4H)-yl)methanone |
| 394 | (R)-(4-(difluoromethyl)oxazol-5-yl)(4-(7-methylbenzo[d]oxazol-2-yl)-6,7-dihydro-1H-imidazo[4,5-c]pyridin-5(4H)-yl)methanone |
| 396 | (R)-(6-(6-azaspiro[2.5]octan-6-yl)pyrazolo[1,5-a]pyridin-3-yl)(4-(7-fluorobenzo[d]oxazol-2-yl)-6,7-dihydro-1H-imidazo[4,5-c]pyridin-5(4H)-yl)methanone |
| 398 | (R)-1-(4-(4-fluorobenzo[d]oxazol-2-yl)-6,7-dihydro-1H-imidazo[4,5-c]pyridin-5(4H)-yl)-3-(thiazol-2-yl)propan-1-one |
| 400 | (R)-(4-(5-fluorobenzo[d]oxazol-2-yl)-6,7-dihydro- 1H-imidazo[4,5-c]pyridin-5(4H)-yl)(5-(pyridin-3-yl)-1,3,4-oxadiazol-2-yl)methanone |
| 402 | (R)-(4-(difluoromethyl)-2-(4-fluoropiperidin-1-yl)oxazol-5-yl)(4-(5-fluorobenzo[d]oxazol-2-yl)-6,7-dihydro-1H-imidazo[4,5-c]pyridin-5(4H)-yl)methanone |
| 404 | (R)-(4-(difluoromethyl)-2-(4-methylpiperazin-1-yl)oxazol-5-yl)(4-(5-fluorobenzo[d]oxazol-2-yl)-6,7-dihydro-1H-imidazo[4,5-c]pyridin-5(4H)-yl)methanone |
| 405 | (R)-(4-(difluoromethyl)-2-(pyrimidin-5-yl)oxazol-5-yl)(4-(5-fluorobenzo[d]oxazol-2-yl)-6,7-dihydro-1H-imidazo[4,5-c]pyridin-5(4H)-yl)methanone |
| 408 | (R)-(4-bromo-2-(2-hydroxypropan-2-yl)oxazol-5-yl)(4-(5-methylbenzo[d]oxazol-2-yl)-6,7-dihydro-1H-imidazo[4,5-c]pyridin-5(4H)-yl)methanone |
| 410 | (R)-1-(4-(7-fluorobenzo[d]oxazol-2-yl)-6,7-dihydro-1H-imidazo[4,5-c]pyridin-5(4H)-yl)-3-(thiazol-5-yl)propan-1-one |
| 412 | (R)-(4-(difluoromethyl)-2-(1-methyl-1H-pyrazol-3-yl)oxazol-5-yl)(4-(5-fluorobenzo[d]oxazol-2-yl)-6,7-dihydro-1H-imidazo[4,5-c]pyridin-5(4H)-yl)methanone |
| 414 | (R)-(4-(difluoromethyl)-2-morpholinooxazol-5-yl)(4-(5-fluorobenzo[d]oxazol-2-yl)-6,7-dihydro-1H-imidazo[4,5-c]pyridin-5(4H)-yl)methanone |
| 416 | [(4R)-4-(5-bromo-1,3-benzoxazol-2-yl)-1,4,6,7-tetrahydroimidazo[4,5-c]pyridin-5-yl]-[4-(difluoromethyl)-2-(1-hydroxy-1-methyl-ethyl)oxazol-5-yl]methanone |
| 417 | (R)-(4-(difluoromethyl)-2-(5-fluoropyridin-2-yl)oxazol-5-yl)(4-(5-fluorobenzo[d]oxazol-2-yl)-6,7-dihydro-1H-imidazo[4,5-c]pyridin-5(4H)-yl)methanone |
| 419 | (R)-(4-(difluoromethyl)-2-(pyridin-3-yl)oxazol-5-yl)(4-(5-fluorobenzo[d]oxazol-2-yl)-6,7-dihydro-1H-imidazo[4,5-c]pyridin-5(4H)-yl)methanone |
| 422 | (R)-(4-(difluoromethyl)-2-(pyridin-4-yl)oxazol-5-yl)(4-(5-fluorobenzo[d]oxazol-2-yl)-6,7-dihydro-1H-imidazo[4,5-c]pyridin-5(4H)-yl)methanone |
| 424 | (R)-(4-(7-chlorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 426 | (R)-(4-(7-chlorobenzo[d]oxazol-2-yl)-6,7-dihydro-1H-imidazo[4,5-c]pyridin-5(4H)-yl)(4-(difluoromethyl)oxazol-5-yl)methanone |
| 428 | (R)-(4-(difluoromethyl)-2-(2-hydroxypropan-2-yl)oxazol-5-yl)(4-(4-methoxybenzo[d]oxazol-2-yl)-6,7-dihydro-1H-imidazo[4,5-c]pyridin-5(4H)-yl)methanone |
| 430 | (R)-(4-(difluoromethyl)oxazol-5-yl)(4-(4-methoxybenzo[d]oxazol-2-yl)-6,7-dihydro-1H-imidazo[4,5-c]pyridin-5(4H)-yl)methanone |
| 432 | (R)-(4-(4-bromobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 434 | (R)-(4-(4-bromobenzo[d]oxazol-2-yl)-6,7-dihydro-1H-imidazo[4,5-c]pyridin-5(4H)-yl)(4-(difluoromethyl)oxazol-5-yl)methanone |
| 436 | [(4R)-4-(4-chloro-1,3-benzoxazol-2-yl)-1,4,6,7-tetrahydroimidazo[4,5-c]pyridin-5-yl]-[4-(difluoromethyl)-2-(1-hydroxy-1-methyl-ethyl)oxazol-5-yl]methanone |
| 438 | [(4R)-4-(4-chloro-1,3-benzoxazol-2-yl)-1,4,6,7-tetrahydroimidazo[4,5-c]pyridin-5-yl]-[4-(difluoromethyl)oxazol-5-yl]methanone |
| 440 | (R)-(4-bromo-2-(2-hydroxypropan-2-yl)oxazol-5-yl)(4-(6-methylbenzo[d]oxazol-2-yl)-6,7-dihydro-1H-imidazo[4,5-c]pyridin-5(4H)-yl)methanone |
| 441 | (R)-(4-(5-fluorobenzo[d]oxazol-2-yl)-6,7-dihydro- 1H-imidazo[4,5-c]pyridin-5(4H)-yl)(5-(5-methoxypyridin-2-yl)-1,3,4-oxadiazol-2-yl)methanone |
| 443 | (R)-(4-(difluoromethyl)-2-(5-(trifluoromethyl)pyridin-2-yl)oxazol-5-yl)(4-(5-fluorobenzo[d]oxazol-2-yl)-6,7-dihydro-1H-imidazo[4,5-c]pyridin-5(4H)-yl)methanone |
| 446 | (R)-(4-(difluoromethyl)-2-(5-methoxypyridin-2-yl)oxazol-5-yl)(4-(5-fluorobenzo[d]oxazol-2-yl)-6,7-dihydro-1H-imidazo[4,5-c]pyridin-5(4H)-yl)methanone |
| 448 | (R)-(3-(difluoromethyl)-1-methyl-1H-1,2,4-triazol-5-yl)(4-(4-methylbenzo[d]oxazol-2-yl)-6,7-dihydro-1H-imidazo[4,5-c]pyridin-5(4H)-yl)methanone |
| 450 | (R)-(1-(difluoromethyl)-1H-pyrazol-5-yl)(4-(4-methylbenzo[d]oxazol-2-yl)-6,7-dihydro-1H-imidazo[4,5-c]pyridin-5(4H)-yl)methanone |
| 452 | (R)-(4-(7-fluorobenzo[d]oxazol-2-yl)-6,7-dihydro- 1H-imidazo[4,5-c]pyridin-5(4H)-yl)(6-(pyridin-2-yl)pyrazolo[1,5-a]pyridin-3-yl)methanone |
| 454 | (R)-(4-(difluoromethyl)-2-(2-hydroxypropan-2-yl)oxazol-5-yl)(4-(7-(trifluoromethyl)benzo[d]oxazol-2-yl)-6,7-dihydro-1H-imidazo[4,5-c]pyridin-5(4H)-vl)methanone |
| 456 | (R)-(4-(difluoromethyl)-2-(2-hydroxypropan-2-yl)oxazol-5-yl)(4-(7-methoxybenzo[d]oxazol-2-yl)-6,7-dihydro-1H-imidazo[4,5-c]pyridin-5(4H)-yl)methanone |
| 458 | (R)-(4-(difluoromethyl)oxazol-5-yl)(4-(7-methoxybenzo[d]oxazol-2-yl)-6,7-dihydro-1H-imidazo[4,5-c]pyridin-5(4H)-yl)methanone |
| 459 | (R)-(4-(7-bromobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 462 | (R)-(4-(7-bromobenzo[d]oxazol-2-yl)-6,7-dihydro- 1H-imidazo[4,5-c]pyridin-5(4H)-yl)(4-(difluoromethyl)oxazol-5-yl)methanone |
| 464 | (R)-(4-(difluoromethyl)-2-(2-hydroxypropan-2-yl)oxazol-5-yl)(4-(4-(trifluoromethyl)benzo[d]oxazol-2-yl)-6,7-dihydro- 1H-imidazo[4,5-c]pyridin-5(4H)-vl)methanone |
| 466 | (R)-(4-(difluoromethyl)oxazol-5-yl)(4-(4-(trifluoromethyl)benzo[d]oxazol-2-yl)-6,7-dihydro-1H-imidazo[4,5-c]pyridin-5(4H)-yl)methanone |
| 468 | (R)-(4-(benzo[d]oxazol-2-yl)-6,7-dihydro-1H-imidazo[4,5-c]pyridin-5(4H)-yl)(4-(difluoromethyl)oxazol-5-yl)methanone |
| 469 | (R)-(4-(benzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 472 | (R)-(3-bromo-1-methyl-1H-1,2,4-triazol-5-yl)(4-(4-fluorobenzo[d]oxazol-2-yl)-6,7-dihydro-1H-imidazo[4,5-c]pyridin-5(4H)-yl)methanone |
| 474 | (R)-(3-bromo-1-methyl-1H-1,2,4-triazol-5-yl)(4-(7-fluorobenzo[d]oxazol-2-yl)-6,7-dihydro-1H-imidazo[4,5-c]pyridin-5(4H)-yl)methanone |
| 476 | (R)-(4-(4-fluorobenzo[d]oxazol-2-yl)-6,7-dihydro-1H-imidazo[4,5-c]pyridin-5(4H)-yl)(6-(4-methylpiperazin-1-yl)pyrazolo[1,5-a]pyridin-3-yl)methanone |
| 478 | (R)-(4-(4-fluorobenzo[d]oxazol-2-yl)-6,7-dihydro- 1H-imidazo[4,5-c]pyridin-5(4H)-yl)(6-(4-fluoropiperidin-1-yl)pyrazolo[1,5-a]pyridin-3-yl)methanone |
| 479 | (R)-(5-(1-methyl-1H-pyrazol-4-yl)-1,3,4-oxadiazol-2-yl)(4-(4-methylbenzo[d]oxazol-2-yl)-6,7-dihydro-1H-imidazo[4,5-c]pyridin-5(4H)-yl)methanone |
| 481 | (R)-(4-(4-methylbenzo[d]oxazol-2-yl)-6,7-dihydro- 1H-imidazo[4,5-c]pyridin-5(4H)-yl)(5-(pyridin-2-yl)-1,3,4-oxadiazol-2-yl)methanone |
| 484 | (R)-(4-(4,7-difluorobenzo[d]oxazol-2-yl)-6,7-dihydro-1H-imidazo[4,5-c]pyridin-5(4H)-yl)(oxazol-5-yl)methanone |
| 485 | (R)-(4-(4,7-difluorobenzo[d]oxazol-2-yl)-6,7-dihydro-1H-imidazo[4,5-c]pyridin-5(4H)-yl)(3-(difluoromethyl)-1-methyl-1H-1,2,4-triazol-5-yl)methanone |
| 488 | (R)-(4-(4,7-difluorobenzo[d]oxazol-2-yl)-6,7-dihydro-1H-imidazo[4,5-c]pyridin-5(4H)-yl)(1-(difluoromethyl)-1H-pyrazol-5-yl)methanone |
| 490 | (R)-(4-(4,6-difluorobenzo[d]oxazol-2-yl)-6,7-dihydro-1H-imidazo[4,5-c]pyridin-5(4H)-yl)(1-(difluoromethyl)-1H-pyrazol-5-yl)methanone |
| 492 | (R)-(5-(1H-pyrazol-4-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 494 | (R)-(4-(difluoromethyl)oxazol-5-yl)(4-(7-(trifluoromethyl)benzo[d]oxazol-2-yl)-6,7-dihydro-1H-imidazo[4,5-c]pyridin-5(4H)-yl)methanone |
| 496 | (R)-(1,3-dimethyl-1H-1,2,4-triazol-5-yl)(4-(7-fluorobenzo[d]oxazol-2-yl)-6,7-dihydro-1H-imidazo[4,5-c]pyridin-5(4H)-yl)methanone |
| 498 | (R)-(3-cyclopropyl-1-methyl-1H-1,2,4-triazol-5-yl)(4-(7-fluorobenzo[d]oxazol-2-yl)-6,7-dihydro-1H-imidazo[4,5-c]pyridin-5(4H)-yl)methanone |
| 500 | (R)-(4-(difluoromethyl)-2-(2-hydroxypropan-2-yl)oxazol-5-yl)(4-(7-fluoro-5-methylbenzo[d]oxazol-2-yl)-6,7-dihydro-1H-imidazo[4,5-c]pyridin-5(4H)-yl)methanone |
| 502 | (R)-(4-(difluoromethyl)oxazol-5-yl)(4-(7-fluoro-5-methylbenzo[d]oxazol-2-yl)-6,7-dihydro-1H-imidazo[4,5-c]pyridin-5(4H)-yl)methanone |
| 503 | (R)-(4-(4-fluorobenzo[d]oxazol-2-yl)-6,7-dihydro- 1H-imidazo[4,5-c]pyridin-5(4H)-yl)(6-morpholinopyrazolo[1,5-a]pyridin-3-yl)methanone |
| 506 | (R)-(6-(6-azaspiro[2.5]octan-6-yl)pyrazolo[1,5-a]pyridin-3-yl)(4-(4-fluorobenzo[d]oxazol-2-yl)-6,7-dihydro-1H-imidazo[4,5-c]pyridin-5(4H)-yl)methanone |
| 508 | (R)-(4-(4-fluorobenzo[d]oxazol-2-yl)-6,7-dihydro-1H-imidazo[4,5-c]pyridin-5(4H)-yl)(6-(4-(2-hydroxypropan-2-yl)piperidin- 1-yl)pyrazolo[1,5-a]pyridin-3-yl)methanone |
| 510 | (R)-(4-(4-methylbenzo[d]oxazol-2-yl)-6,7-dihydro-1H-imidazo[4,5-c]pyridin-5(4H)-yl)(oxazol-5-yl)methanone |
| 512 | (R)-(2-(2-hydroxypropan-2-yl)oxazol-5-yl)(4-(4-methylbenzo[d]oxazol-2-yl)-6,7-dihydro-1H-imidazo[4,5-c]pyridin-5(4H)-yl)methanone |
| 514 | (R)-(4-(6,7-difluorobenzo[d]oxazol-2-yl)-6,7-dihydro-1H-imidazo[4,5-c]pyridin-5(4H)-yl)(1-(difluoromethyl)-1H-pyrazol-5-yl)methanone |
| 516 | (R)-(4-(6,7-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 517 | (R)-(4-(4,7-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 519 | (R)-(4-(4,7-difluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 521 | (R)-(4-(4,6-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 524 | (R)-(4-(4,6-difluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 525 | (R)-(4-(difluoromethyl)-2-(pyrimidin-4-yl)oxazol-5-yl)(4-(5-fluorobenzo[d]oxazol-2-yl)-6,7-dihydro-1H-imidazo[4,5-c]pyridin-5(4H)-yl)methanone |
| 528 | (R)-(4-(difluoromethyl)-2-(pyrazin-2-yl)oxazol-5-yl)(4-(5-fluorobenzo[d]oxazol-2-yl)-6,7-dihydro-1H-imidazo[4,5-c]pyridin-5(4H)-yl)methanone |
| 530 | (R)-(4-(difluoromethyl)-2-(2-hydroxypropan-2-yl)oxazol-5-yl)(4-(7-(trifluoromethoxy)benzo[d]oxazol-2-yl)-6,7-dihydro-1H-imidazo[4,5-c]pyridin-5(4H)-vl)methanone |
| 531 | (R)-(4-(difluoromethyl)oxazol-5-yl)(4-(7-(trifluoromethoxy)benzo[d]oxazol-2-yl)-6,7-dihydro-1H-imidazo[4,5-c]pyridin-5(4H)-yl)methanone |
| 534 | (R)-(4-(difluoromethyl)-2-(2-hydroxypropan-2-yl)oxazol-5-yl)(4-(4-(trifluoromethoxy)benzo[d]oxazol-2-yl)-6,7-dihydro-1H-imidazo[4,5-c]pyridin-5(4H)-yl)methanone |
| 535 | (R)-(4-(difluoromethyl)oxazol-5-yl)(4-(4-(trifluoromethoxy)benzo[d]oxazol-2-yl)-6,7-dihydro-1H-imidazo[4,5-c]pyridin-5(4H)-yl)methanone |
| 538 | (R)-(4-(4-fluorobenzo[d]oxazol-2-yl)-6,7-dihydro- 1H-imidazo[4,5-c]pyridin-5(4H)-yl)(6-methylpyrazolo[1,5-a]pyridin-3-yl)methanone |
| 540 | (R)-(4-(4-fluorobenzo[d]oxazol-2-yl)-6,7-dihydro-1H-imidazo[4,5-c]pyridin-5(4H)-yl)(6-methoxypyrazolo[1,5-a]pyridin-3-yl)methanone |
| 542 | (R)-(4-(4-fluorobenzo[d]oxazol-2-yl)-6,7-dihydro-1H-imidazo[4,5-c]pyridin-5(4H)-yl)(6-(1-methyl-1H-pyrazol-4-yl)pyrazolo[1,5-a]pyridin-3-yl)methanone |
| 544 | (R)-(4-(4-fluorobenzo[d]oxazol-2-yl)-6,7-dihydro-1H-imidazo[4,5-c]pyridin-5(4H)-yl)(6-(pyridin-2-yl)pyrazolo[1,5-a]pyridin-3-yl)methanone |
| 545 | (R)-(4-(6,7-difluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 548 | (R)-(4-(benzo[d]oxazol-2-yl)-6,7-dihydro-1H-imidazo[4,5-c]pyridin-5(4H)-yl)(oxazol-5-yl)methanone |
| 550 | (R)-(3-(difluoromethyl)-1-methyl-1H-1,2,4-triazol-5-yl)(4-(5-methoxybenzo[d]oxazol-2-yl)-6,7-dihydro-1H-imidazo[4,5-c]pyridin-5(4H)-yl)methanone |
| 552 | (R)-(1-(difluoromethyl)-1H-pyrazol-5-yl)(4-(5-methoxybenzo[d]oxazol-2-yl)-6,7-dihydro-1H-imidazo[4,5-c]pyridin-5(4H)-yl)methanone |
| 554 | (R)-(4-(5-methoxybenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 556 | (R)-(4-(5-methoxybenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 558 | (R)-(4-cyclopropyl-2-(2-hydroxypropan-2-yl)oxazol-5-yl)(4-(5-fluorobenzo[d]oxazol-2-yl)-6,7-dihydro-1H-imidazo[4,5-c]pyridin-5(4H)-yl)methanone |
| 559 | (R)-(4-(difluoromethyl)-2-(pyridin-4-yl)oxazol-5-yl)(4-(7-fluorobenzo[d]oxazol-2-yl)-6,7-dihydro-1H-imidazo[4,5-c]pyridin-5(4H)-yl)methanone |
| 562 | (R)-(4-(benzo[d]oxazol-2-yl)-6,7-dihydro-1H-imidazo[4,5-c]pyridin-5(4H)-yl)(3-(difluoromethyl)-1-methyl-1H-1,2,4-triazol-5-yl)methanone |
| 564 | (R)-(4-(benzo[d]oxazol-2-yl)-6,7-dihydro-1H-imidazo[4,5-c]pyridin-5(4H)-yl)(1-(difluoromethyl)-1H-pyrazol-5-yl)methanone |
| 566 | (R)-(5-(1-methyl-1H-pyrazol-4-yl)-1,3,4-oxadiazol-2-yl)(4-(7-methylbenzo[d]oxazol-2-yl)-6,7-dihydro-1H-imidazo[4,5-c]pyridin-5(4H)-yl)methanone |
| 568 | (R)-(4-(7-methylbenzo[d]oxazol-2-yl)-6,7-dihydro- 1H-imidazo[4,5-c]pyridin-5(4H)-yl)(5-(pyridin-2-yl)-1,3,4-oxadiazol-2-yl)methanone |
| 570 | (R)-(4-(7-fluorobenzo[d]oxazol-2-yl)-6,7-dihydro- 1H-imidazo[4,5-c]pyridin-5(4H)-yl)(4-methyloxazol-5-yl)methanone |
| 572 | (R)-(4-(5-bromo-7-fluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 574 | (R)-(4-(5-bromo-7-fluorobenzo[d]oxazol-2-yl)-6,7-dihydro-1H-imidazo[4,5-c]pyridin-5(4H)-yl)(4-(difluoromethyl)oxazol-5-yl)methanone |
| 576 | (R)-(4-(5,7-difluorobenzo[d]oxazol-2-yl)-6,7-dihydro-1H-imidazo[4,5-c]pyridin-5(4H)-yl)(oxazol-5-yl)methanone |
| 578 | (R)-(4-(5,7-difluorobenzo[d]oxazol-2-yl)-6,7-dihydro-1H-imidazo[4,5-c]pyridin-5(4H)-yl)(3-(difluoromethyl)-1-methyl-1H-1,2,4-triazol-5-yl)methanone |
| 579 | (R)-(4-(5,7-difluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 582 | (R)-(4-(benzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 584 | (R)-(4-(benzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 586 | (R)-(4-(5-fluorobenzo[d]oxazol-2-yl)-6,7-dihydro-1H-imidazo[4,5-c]pyridin-5(4H)-yl)(2-(1-hydroxycyclobutyl)-4-(trifluoromethyl)oxazol-5-yl)methanone |
| 588 | (R)-(4-(difluoromethyl)-2-(pyridin-2-yl)oxazol-5-yl)(4-(7-fluorobenzo[d]oxazol-2-yl)-6,7-dihydro-1H-imidazo[4,5-c]pyridin-5(4H)-yl)methanone |
| 589 | (R)-(4-(difluoromethyl)-2-(pyridin-3-yl)oxazol-5-yl)(4-(7-fluorobenzo[d]oxazol-2-yl)-6,7-dihydro-1H-imidazo[4,5-c]pyridin-5(4H)-yl)methanone |
| 592 | (R)-(4-(difluoromethyl)-2-(1-methyl-1H-pyrazol-3-yl)oxazol-5-yl)(4-(7-fluorobenzo[d]oxazol-2-yl)-6,7-dihydro-1H-imidazo[4,5-c]pyridin-5(4H)-yl)methanone |
| 593 | (R)-(4-(7-fluorobenzo[d]oxazol-2-yl)-6,7-dihydro-1H-imidazo[4,5-c]pyridin-5(4H)-yl)(4-methyl-2-(pyridin-2-yl)oxazol-5-yl)methanone |
| 595 | (R)-(4-(7-fluorobenzo[d]oxazol-2-yl)-6,7-dihydro-1H-imidazo[4,5-c]pyridin-5(4H)-yl)(4-methyl-2-(1-methyl-1H-pyrazol-4-yl)oxazol-5-yl)methanone |
| 598 | (R)-(4-(6-fluorobenzo[d]oxazol-2-yl)-6,7-dihydro- 1H-imidazo[4,5-c]pyridin-5(4H)-yl)(4-methyloxazol-5-yl)methanone |
| 600 | (R)-(4-(4-fluorobenzo[d]oxazol-2-yl)-6,7-dihydro-1H-imidazo[4,5-c]pyridin-5(4H)-yl)(4-methyloxazol-5-yl)methanone |
| 602 | (R)-(3-(difluoromethyl)-1-methyl-1H-1,2,4-triazol-5-yl)(4-(4-methoxybenzo[d]oxazol-2-yl)-6,7-dihydro-1H-imidazo[4,5-c]pyridin-5(4H)-yl)methanone |
| 604 | (R)-(1-(difluoromethyl)-1H-pyrazol-5-yl)(4-(4-methoxybenzo[d]oxazol-2-yl)-6,7-dihydro-1H-imidazo[4,5-c]pyridin-5(4H)-yl)methanone |
| 606 | (R)-(4-(5-chloro-7-fluorobenzo[d]oxazol-2-yl)-6,7-dihydro-1H-imidazo[4,5-c]pyridin-5(4H)-yl)(4-(difluoromethyl)-2-(2-hydroxypropan-2-yl)oxazol-5-yl)methanone |
| 607 | (R)-(4-(5-chloro-7-fluorobenzo[d]oxazol-2-yl)-6,7-dihydro-1H-imidazo[4,5-c]pyridin-5(4H)-yl)(4-(difluoromethyl)oxazol-5-yl)methanone |
| 610 | (R)-(4-(5,7-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 612 | (R)-(4-(7-methylbenzo[d]oxazol-2-yl)-6,7-dihydro- 1H-imidazo[4,5-c]pyridin-5(4H)-yl)(oxazol-5-yl)methanone |
| 614 | (R)-(3-(difluoromethyl)-1-methyl-1H-1,2,4-triazol-5-yl)(4-(7-methylbenzo[d]oxazol-2-yl)-6,7-dihydro-1H-imidazo[4,5-c]pyridin-5(4H)-yl)methanone |
| 615 | (R)-(4-(difluoromethyl)-2-(5-methoxypyridin-2-yl)oxazol-5-yl)(4-(7-fluorobenzo[d]oxazol-2-yl)-6,7-dihydro-1H-imidazo[4,5-c]pyridin-5(4H)-yl)methanone |
| 618 | (R)-(4-(difluoromethyl)-2-(4-methylpiperazin-1-yl)oxazol-5-yl)(4-(7-fluorobenzo[d]oxazol-2-yl)-6,7-dihydro-1H-imidazo[4,5-c]pyridin-5(4H)-yl)methanone |
| 619 | (R)-(4-(1,1-difluoroethyl)oxazol-5-yl)(4-(7-fluorobenzo[d]oxazol-2-yl)-6,7-dihydro-1H-imidazo[4,5-c]pyridin-5(4H)-yl)methanone |
| 622 | (R)-(4-(4-methoxybenzo[d]oxazol-2-yl)-6,7-dihydro-1H-imidazo[4,5-c]pyridin-5(4H)-yl)(oxazol-5-yl)methanone |
| 623 | (R)-(4-(4-methoxybenzo[d]oxazol-2-yl)-6,5-dihydro-1H-imidazo[4,5-c]pyridin-5(4H)-yl)(5-(pyridin-2-yl)-1,3,4-oxadiazol-2-yl)methanone |
| 625 | (R)-(4-(4-methoxybenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 627 | (R)-(4-(5-fluorobenzo[d]oxazol-2-yl)-6,7dihydro-1H-imidazo[4,5-c]pyridin-5(4H)-yl)(5-(pyrazin-2-yl)-1,3,4-oxadiazol-2-yl)methanone |
| 630 | (R)-(4-(4,5-difluorobenzo[d]oxazol-2-yl)-6,7-dihydro-1H-imidazo[4,5-c]pyridin-5(4H)-yl)(5-(pyridin-2-yl)-1,3,4-oxadiazol-2-yl)methanone |
| 631 | (R)-(4-(difluoromethyl)-2-(5-fluoropyridin-2-yl)oxazol-5-yl)(4-(7-fluorobenzo[d]oxazol-2-yl)-6,7-dihydro-1H-imidazo[4,5-c]pyridin-5(4H)-yl)methanone |
| 634 | (R)-(4-(difluoromethyl)-2-(5-(trifluoromethyl)pyridin-2-yl)oxazol-5-yl)(4-(7-fluorobenzo[d]oxazol-2-yl)-6,7-dihydro-1H-imidazo[4,5-c]pyridin-5(4H)-yl)methanone |
| 635 | (R)-(4-(difluoromethyl)-2-(1-methyl-1H-pyrazol-4-yl)oxazol-5-yl)(4-(7-fluorobenzo[d]oxazol-2-yl)-6,7-dihydro-1H-imidazo[4,5-c]pyridin-5(4H)-yl)methanone |
| 637 | (R)-(4-(7-fluorobenzo[d]oxazol-2-yl)-6,7-dihydro-1H-imidazo[4,5-c]pyridin-5(4H)-yl)(2-(pyridin-2-yl)-4-(trifluoromethyl)oxazol-5-yl)methanone |
| 640 | (R)-(4-(4-methoxybenzo[d]oxazol-2-yl)-6,7-dihydro-1H-imidazo[4,5-c]pyridin-5(4H)-yl)(5-(1-methyl-1H-pyrazol-3-yl)-1,3,4-oxadiazol-2-yl)methanone |
| 642 | (R)-(4-(difluoromethyl)-2-(1-methyl-1H-imidazol-4-yl)oxazol-5-yl)(4-(5-fluorobenzo[d]oxazol-2-yl)-6,7-dihydro-1H-imidazo[4,5-c]pyridin-5(4H)-yl)methanone |
| 644 | (R)-(4-(difluoromethyl)-2-(2-hydroxypropan-2-yl)oxazol-5-yl)(4-(7-fluoro-5-methoxybenzo[d]oxazol-2-yl)-6,7-dihydro-1H-imidazo[4,5-c]pyridin-5(4H)-yl)methanone |
| 646 | (R)-(4-(4,5-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 647 | (R)-(4-(difluoromethyl)-2-morpholinooxazol-5-yl)(4-(7-fluorobenzo[d]oxazol-2-yl)-6,7-dihydro-1H-imidazo[4,5-c]pyridin-5(4H)-yl)methanone |
| 650 | (R)-(4-(1,1-difluoroethyl)oxazol-5-yl)(4-(6-fluorobenzo[d]oxazol-2-yl)-6,7-dihydro-1H-imidazo[4,5-c]pyridin-5(4H)-yl)methanone |
| 652 | (R)-(4-(1,1-difluoroethyl)oxazol-5-yl)(4-(4-fluorobenzo[d]oxazol-2-yl)-6,7-dihydro-1H-imidazo[4,5-c]pyridin-5(4H)-yl)methanone |
| 654 | (R)-(5-(3-fluoropyridin-2-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 656 | (R)-(4-(difluoromethyl)-2-(2-hydroxypropan-2-yl)oxazol-5-yl)(4-(5-fluoro-7-methylbenzo[d]oxazol-2-yl)-6,7-dihydro-1H-imidazo[4,5-c]pyridin-5(4H)-yl)methanone |
| 658 | (R)-(4-(difluoromethyl)oxazol-5-yl)(4-(5-fluoro-7-methylbenzo[d]oxazol-2-yl)-6,7-dihydro-1H-imidazo[4,5-c]pyridin-5(4H)-yl)methanone |
| 660 | (R)-(4-(difluoromethyl)-2-(2-hydroxypropan-2-yl)oxazol-5-yl)(4-(4,7-dimethylbenzo[d]oxazol-2-yl)-6,7-dihydro-1H-imidazo[4,5-c]pyridin-5(4H)-yl)methanone |
| 662 | (R)-(4-(difluoromethyl)-2-(pyrimidin-2-yl)oxazol-5-yl)(4-(5-fluorobenzo[d]oxazol-2-yl)-6,7-dihydro-1H-imidazo[4,5-c]pyridin-5(4H)-yl)methanone |
| 664 | (R)-(4-(difluoromethyl)oxazol-5-yl)(4-(7-fluoro-5-methoxybenzo[d]oxazol-2-yl)-6,7-dihydro-1H-imidazo[4,5-c]pyridin-5(4H)-yl)methanone |
| 666 | (R)-(4-(5,7-difluorobenzo[d]oxazol-2-yl)-6,7-dihydro-1H-imidazo[4,5-c]pyridin-5(4H)-yl)(5-(1-methyl-1H-pyrazol-4-yl)-1,3,4-oxadiazol-2-yl)methanone |
| 668 | (R)-(4-(difluoromethyl)-2-(pyrimidin-2-yl)oxazol-5-yl)(4-(7-fluorobenzo[d]oxazol-2-yl)-6,7-dihydro-1H-imidazo[4,5-c]pyridin-5(4H)-yl)methanone |
| 670 | (R)-(4-(7-fluorobenzo[d]oxazol-2-yl)-6,7-dihydro- 1H-imidazo[4,5-c]pyridin-5(4H)-yl)(2-(1-methyl-1H-pyrazol-4-yl)-4-(trifluoromethyl)oxazol-5-yl)methanone |
| 672 | (R)-(4-(5-fluorobenzo[d]oxazol-2-yl)-6,7-dihydro- 1H-imidazo[4,5-c]pyridin-5(4H)-yl)(5-(5-(trifluoromethoxy)pyridin-2-yl)-1,3,4-oxadiazol-2-yl)methanone |
| 674 | (R)-(5-(1-(difluoromethyl)-1H-pyrazol-4-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 675 | (R)-(4-(difluoromethyl)-2-(4-fluoropiperidin-1-yl)oxazol-5-yl)(4-(7-fluorobenzo[d]oxazol-2-yl)-6,7-dihydro-1H-imidazo[4,5-c]pyridin-5(4H)-yl)methanone |
| 678 | (R)-(5-(5-methoxypyridin-2-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 680 | (R)-(5-(3-fluoropyridin-2-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 682 | (R)-(5-(1-(difluoromethyl)-1H-pyrazol-4-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 684 | (R)-(4-(benzo[d]oxazol-2-yl)-6,7-dihydro-1H-imidazo[4,5-c]pyridin-5(4H)-yl)(1-methyl-3-(trifluoromethyl)-1H-1,2,4-triazol-5-yl)methanone |
| 686 | (R)-(1-methyl-3-(trifluoromethyl)-1H-1,2,4-triazol-5-yl)(4-(7-methylbenzo[d]oxazol-2-yl)-6,7-dihydro-1H-imidazo[4,5-c]pyridin-5(4H)-yl)methanone |
| 688 | (R)-(4-(difluoromethyl)-2-(1-methyl-1H-imidazol-5-yl)oxazol-5-yl)(4-(5-fluorobenzo[d]oxazol-2-yl)-6,7-dihydro-1H-imidazo[4,5-c]pyridin-5(4H)-yl)methanone |
| 690 | (R)-(4-(difluoromethyl)-2-(1-hydroxycyclobutyl)oxazol-5-yl)(4-(5-fluorobenzo[d]oxazol-2-yl)-6,7-dihydro-1H-imidazo[4,5-c]pyridin-5(4H)-yl)methanone |
| 691 | (R)-(4-(difluoromethyl)-2-(1-methyl-1H-imidazol-5-yl)oxazol-5-yl)(4-(7-fluorobenzo[d]oxazol-2-yl)-6,7-dihydro-1H-imidazo[4,5-c]pyridin-5(4H)-yl)methanone |
| 694 | (R)-(4-(difluoromethyl)-2-(pyrazin-2-yl)oxazol-5-yl)(4-(7-fluorobenzo[d]oxazol-2-yl)-6,7-dihydro-1H-imidazo[4,5-c]pyridin-5(4H)-yl)methanone |
| 696 | (R)-(5-(1-(difluoromethyl)-1H-pyrazol-3-yl)-1,3,4-oxadiazol-2-yl)(4-(5-methylbenzo[d]oxazol-2-yl)-6,7-dihydro-1H-imidazo[4,5-c]pyridin-5(4H)-yl)methanone |
| 698 | (R)-(4-(7-methoxybenzo[d]oxazol-2-yl)-6,7-dihydro- 1H-imidazo[4,5-c]pyridin-5(4H)-yl)(1-methyl-3-(trifluoromethyl)-1H-1,2,4-triazol-5-yl)methanone |
| 700 | (R)-(4-(5,7-difluorobenzo[d]oxazol-2-yl)-6,7-dihydro-1H-imidazo[4,5-c]pyridin-5(4H)-yl)(6-(4-hydroxypiperidin-1-yl)pyrazolo[1,5-a]pyridin-3-yl)methanone |
| 702 | (R)-(6-(4-hydroxypiperidin-1-yl)pyrazolo[1,5-a]pyridin-3-yl)(4-(4-methylbenzo[d]oxazol-2-yl)-6,7-dihydro-1H-imidazo[4,5-c]pyridin-5(4H)-yl)methanone |
| 704 | (R)-(5-(1-(difluoromethyl)-1H-pyrazol-3-yl)-1,3,4-oxadiazol-2-yl)(4-(6-methylbenzo[d]oxazol-2-yl)-6,7-dihydro-1H-imidazo[4,5-c]pyridin-5(4H)-yl)methanone |
| 706 | (R)-(4-(benzo[d]oxazol-2-yl)-6,7-dihydro-1H-imidazo[4,5-c]pyridin-5(4H)-yl)(1-isopropyl-1H-1,2,4-triazol-5-yl)methanone |
| 708 | (R)-(4-(7-fluorobenzo[d]oxazol-2-yl)-6,7-dihydro-1H-imidazo[4,5-c]pyridin-5(4H)-yl)(1-isopropyl-1H-1,2,4-triazol-5-yl)methanone |
| 709 | (R)-(4-(7-methylbenzo[d]oxazol-2-yl)-6,7-dihydro-1H-imidazo[4,5-c]pyridin-5(4H)-yl)(1-(trifluoromethyl)-1H-pyrazol-5-yl)methanone |
| 712 | (R)-(4-(benzo[d]oxazol-2-yl)-6,7-dihydro-1H-imidazo[4,5-c]pyridin-5(4H)-yl)(1-methyl-1H-pyrazol-5-yl)methanone |
| 714 | (R)-(4-(7-fluorobenzo[d]oxazol-2-yl)-6,7-dihydro-1H-imidazo[4,5-c]pyridin-5(4H)-yl)(2-(2-hydroxypropan-2-yl)oxazol-5-yl)methanone |
| 716 | (R)-(4-(difluoromethyl)-2-(2-hydroxypropan-2-yl)oxazol-5-yl)(4-(5-fluoro-7-methoxybenzo[d]oxazol-2-yl)-6,7-dihydro-1H-imidazo[4,5-c]pyridin-5(4H)-yl)methanone |
| 717 | (R)-(4-(difluoromethyl)oxazol-5-yl)(4-(5-fluoro-7-methoxybenzo[d]oxazol-2-yl)-6,7-dihydro-1H-imidazo[4,5-c]pyridin-5(4H)-yl)methanone |
| 720 | (R)-(4-(difluoromethyl)-2-(2-hydroxypropan-2-yl)oxazol-5-yl)(4-(5-fluoro-4-methylbenzo[d]oxazol-2-yl)-6,7-dihydro-1H-imidazo[4,5-c]pyridin-5(4H)-yl)methanone |
| 721 | (R)-(4-(difluoromethyl)oxazol-5-yl)(4-(5-fluoro-4-methylbenzo[d]oxazol-2-yl)-6,7-dihydro-1H-imidazo[4,5-c]pyridin-5(4H)-yl)methanone |
| 724 | (R)-(4-(difluoromethyl)-2-(2-hydroxypropan-2-yl)oxazol-5-yl)(4-(4-fluoro-7-methylbenzo[d]oxazol-2-yl)-6,7-dihydro-1H-imidazo[4,5-c]pyridin-5(4H)-yl)methanone |
| 726 | (R)-(4-(benzo[d]oxazol-2-yl)-6,7-dihydro-1H-imidazo[4,5-c]pyridin-5(4H)-yl)(1-cyclopropyl-1H-pyrazol-5-yl)methanone |
| 728 | (R)-(1-cyclopropyl-1H-pyrazol-5-yl)(4-(7-fluorobenzo[d]oxazol-2-yl)-6,7-dihydro-1H-imidazo[4,5-c]pyridin-5(4H)-yl)methanone |
| 730 | (R)-(4-(7-fluorobenzo[d]oxazol-2-yl)-6,7-dihydro-1H-imidazo[4,5-c]pyridin-5(4H)-yl)(1-(pyridin-2-yl)-1H-pyrazol-5-yl)methanone |
| 732 | (R)-(4-(benzo[d]oxazol-2-yl)-6,7-dihydro-1H-imidazo[4,5-c]pyridin-5(4H)-yl)(3-(difluoromethyl)-1-methyl-1H-pyrazol-5-yl)methanone |
| 734 | (R)-(3-(difluoromethyl)-1-methyl-1H-pyrazol-5-yl)(4-(7-fluorobenzo[d]oxazol-2-yl)-6,7-dihydro-1H-imidazo[4,5-c]pyridin-5(4H)-yl)methanone |
| 736 | (R)-(3-(difluoromethyl)-1-methyl-1H-pyrazol-5-yl)(4-(7-methylbenzo[d]oxazol-2-yl)-6,7-dihydro-1H-imidazo[4,5-c]pyridin-5(4H)-yl)methanone |
| 738 | (R)-(4-(difluoromethyl)-2-(pyridin-2-yl)oxazol-5-yl)(4-(5-methylbenzo[d]oxazol-2-yl)-6,7-dihydro-1H-imidazo[4,5-c]pyridin-5(4H)-yl)methanone |
| 739 | (R)-(4-(benzo[d]oxazol-2-yl)-6,7-dihydro-1H-imidazo[4,5-c]pyridin-5(4H)-yl)(4-(difluoromethyl)-2-(pyridin-2-yl)oxazol-5-yl)methanone |
| 741 | (R)-(4-(difluoromethyl)-2-(pyrimidin-5-yl)oxazol-5-yl)(4-(7-fluorobenzo[d]oxazol-2-yl)-6,7-dihydro-1H-imidazo[4,5-c]pyridin-5(4H)-yl)methanone |
| 744 | (R)-(4-(difluoromethyl)oxazol-5-yl)(4-(4-fluoro-7-methylbenzo[d]oxazol-2-yl)-6,7-dihydro-1H-imidazo[4,5-c]pyridin-5(4H)-yl)methanone |
| 746 | (R)-(1-methyl-1H-1,2,4-triazol-5-yl)(4-(5-methylbenzo[d]oxazol-2-yl)-6,7-dihydro-1H-imidazo[4,5-c]pyridin-5(4H)-yl)methanone |
| 748 | (R)-(1-methyl-1H-pyrazol-5-yl)(4-(7-methylbenzo[d]oxazol-2-yl)-6,7-dihydro-1H-imidazo[4,5-c]pyridin-5(4H)-yl)methanone |
| 750 | (R)-(4-(7-methoxybenzo[d]oxazol-2-yl)-6,7-dihydro-1H-imidazo[4,5-c]pyridin-5(4H)-yl)(1-methyl-1H-pyrazol-5-yl)methanone |
| 751 | (R)-(4-(benzo[d]oxazol-2-yl)-6,7-dihydro-1H-imidazo[4,5-c]pyridin-5(4H)-yl)(1-isopropyl-1H-pyrazol-5-yl)methanone |
| 754 | (R)-(2-(tert-butyl)-4-(difluoromethyl)oxazol-5-yl)(4-(5-fluorobenzo[d]oxazol-2-yl)-6,7-dihydro-1H-imidazo[4,5-c]pyridin-5(4H)-yl)methanone |
| 756 | (R)-(4-(difluoromethyl)-2-(1-methyl-1H-pyrazol-3-yl)oxazol-5-yl)(4-(5-methylbenzo[d]oxazol-2-yl)-6,7-dihydro-1H-imidazo[4,5-c]pyridin-5(4H)-yl)methanone |
| 757 | (R)-(4-(difluoromethyl)-2-(1-methyl-1H-imidazol-5-yl)oxazol-5-yl)(4-(5-methylbenzo[d]oxazol-2-yl)-6,7-dihydro-1H-imidazo[4,5-c]pyridin-5(4H)-yl)methanone |
| 759 | (R)-(4-(benzo[d]oxazol-2-yl)-6,7-dihydro-1H-imidazo[4,5-c]pyridin-5(4H)-yl)(4-(difluoromethyl)-2-(1-methyl-1H-pyrazol-4-yl)oxazol-5-yl)methanone |
| 762 | (R)-(4-(benzo[d]oxazol-2-yl)-6,7-dihydro-1H-imidazo[4,5-c]pyridin-5(4H)-yl)(4-(difluoromethyl)-2-(1-methyl-1H-pyrazol-3-yl)oxazol-5-yl)methanone |
| 763 | (R)-(4-(benzo[d]oxazol-2-yl)-6,7-dihydro-1H-imidazo[4,5-c]pyridin-5(4H)-yl)(4-(difluoromethyl)-2-(1-methyl-1H-imidazol-5-yl)oxazol-5-yl)methanone |
| 766 | (R)-(1-isopropyl-1H-pyrazol-5-yl)(4-(7-methoxybenzo[d]oxazol-2-yl)-6,7-dihydro-1H-imidazo[4,5-c]pyridin-5(4H)-yl)methanone |
| 768 | (R)-(1-cyclopropyl-1H-pyrazol-5-yl)(4-(7-methoxybenzo[d]oxazol-2-yl)-6,7-dihydro-1H-imidazo[4,5-c]pyridin-5(4H)-yl)methanone |
| 770 | (R)-(1,3-dimethyl- 1H-pyrazol-5-yl)(4-(7-fluorobenzo[d]oxazol-2-yl)-6,7-dihydro-1H-imidazo[4,5-c]pyridin-5(4H)-yl)methanone |
| 772 | (R)-(4-(difluoromethyl)-2-(1-methyl-1H-pyrazol-4-yl)oxazol-5-yl)(4-(5-methylbenzo[d]oxazol-2-yl)-6,7-dihydro-1H-imidazo[4,5-c]pyridin-5(4H)-yl)methanone |
| 774 | (R)-(4-(difluoromethyl)-2-(pyrimidin-4-yl)oxazol-5-yl)(4-(7-fluorobenzo[d]oxazol-2-yl)-6,7-dihydro-1H-imidazo[4,5-c]pyridin-5(4H)-yl)methanone |
| 776 | (R)-(4-(7-methoxybenzo[d]oxazol-2-yl)-6,7-dihydro- 1H-imidazo[4,5-c]pyridin-5(4H)-yl)(1-methyl-1H-1,2,4-triazol-5-yl)methanone |
| 778 | (R)-(3-(difluoromethyl)-1-methyl-1H-pyrazol-5-yl)(4-(7-methoxybenzo[d]oxazol-2-yl)-6,7-dihydro-1H-imidazo[4,5-c]pyridin-5(4H)-yl)methanone |
| 780 | (R)-(4-(benzo[d]oxazol-2-yl)-6,7-dihydro-1H-imidazo[4,5-c]pyridin-5(4H)-yl)(1,3-dimethyl-1H-pyrazol-5-yl)methanone |
| 782 | (R)-(1,3-dimethyl-1H-pyrazol-5-yl)(4-(7-methylbenzo[d]oxazol-2-yl)-6,7-dihydro-1H-imidazo[4,5-c]pyridin-5(4H)-yl)methanone |
| 783 | (R)-(4-(difluoromethyl)-2-(1-methyl-1H-imidazol-4-yl)oxazol-5-yl)(4-(5-methylbenzo[d]oxazol-2-yl)-6,7-dihydro-1H-imidazo[4,5-c]pyridin-5(4H)-yl)methanone |
| 785 | (R)-(4-(benzo[d]oxazol-2-yl)-6,7-dihydro-1H-imidazo[4,5-c]pyridin-5(4H)-yl)(4-(difluoromethyl)-2-(1-methyl-1H-imidazol-4-yl)oxazol-5-yl)methanone |
| 788 | (R)-(4-(5-fluorobenzo[d]oxazol-2-yl)-6,7-dihydro- 1H-imidazo[4,5-c]pyridin-5(4H)-yl)(2-(1-methyl-1H-pyrazol-3-yl)-4-(trifluoromethyl)oxazol-5-yl)methanone |
| 789 | (R)-(4-(5-fluorobenzo[d]oxazol-2-yl)-6,7-dihydro-1H-imidazo[4,5-c]pyridin-5(4H)-yl)(2-(1-methyl-1H-imidazol-4-yl)-4-(trifluoromethyl)oxazol-5-yl)methanone |
| 792 | (R)-(4-(5-fluorobenzo[d]oxazol-2-yl)-6,7-dihydro-1H-imidazo[4,5-c]pyridin-5(4H)-yl)(2-(1-methyl-1H-imidazol-5-yl)-4-(trifluoromethyl)oxazol-5-yl)methanone |
| 794 | (R)-(4-(benzo[d]oxazol-2-yl)-6,7-dihydro-1H-imidazo[4,5-c]pyridin-5(4H)-yl)(4-methyloxazol-5-yl)methanone |
| 796 | (R)-(4-(4,7-difluorobenzo[d]oxazol-2-yl)-6,7-dihydro-1H-imidazo[4,5-c]pyridin-5(4H)-yl)(4-(trifluoromethyl)oxazol-5-yl)methanone |
| 798 | (R)-(4-(benzo[d]oxazol-2-yl)-6,7-dihydro-1H-imidazo[4,5-c]pyridin-5(4H)-yl)(4-(trifluoromethyl)oxazol-5-yl)methanone |
| 799 | (R)-(4-(4,7-difluorobenzo[d]oxazol-2-yl)-6,7-dihydro-1H-imidazo[4,5-c]pyridin-5(4H)-yl)(5-(3-fluoropyridin-2-yl)-1,3,4-oxadiazol-2-yl)methanone |
| 802 | (R)-(4-(4,7-difluorobenzo[d]oxazol-2-yl)-6,7-dihydro-1H-imidazo[4,5-c]pyridin-5(4H)-yl)(5-(1-(difluoromethyl)-1H-pyrazol-4-yl)-1,3,4-oxadiazol-2-yl)methanone |
| 805 | (4-(difluoromethyl)oxazol-5-yl)((4R)-4-(7-fluorobenzo[d]oxazol-2-yl)-7-methyl-6,7-dihydro-1H-imidazo[4,5-c]pyridin-5(4H)-yl)methanone |
| | Diastereomer #2 (Rt=1.335 min) |
| 806 | (4-(difluoromethyl)oxazol-5-yl)((4R)-4-(7-fluorobenzo[d]oxazol-2-yl)-7-methyl-6,7-dihydro-1H-imidazo[4,5-c]pyridin-5(4H)-yl)methanone |
| | Diastereomer #2 (Rt=1.213 min) |
| 808 | (R)-(4-(4,7-difluorobenzo[d]oxazol-2-yl)-6,7-dihydro-1H-imidazo[4,5-c]pyridin-5(4H)-yl)(4-methyloxazol-5-yl)methanone |
| 809 | (R)-(4-(4,7-difluorobenzo[d]oxazol-2-yl)-6,7-dihydro-1H-imidazo[4,5-c]pyridin-5(4H)-yl)(5-(2-fluoropropan-2-yl)-1,3,4-oxadiazol-2-yl)methanone |
| 810 | (R)-(4-(4,7-difluorobenzo[d]oxazol-2-yl)-6,7-dihydro-1H-imidazo[4,5-c]pyridin-5(4H)-yl)(5-(2-fluoropropan-2-yl)-1,3,4-oxadiazol-2-yl)methanone |
| 812 | (R)-(5-(1-cyclobutyl-1H-pyrazol-4-yl)-1,3,4-oxadiazol-2-yl)(4-(4,7-difluorobenzo[d]oxazol-2-yl)-6,7-dihydro-1H-imidazo[4,5-c]pyridin-5(4H)-yl)methanone |
| 814 | (R)-(3-(difluoromethyl)-1-methyl-1H-1,2,4-triazol-5-yl)(4-(4-methylbenzo[d]oxazol-2-yl)-6,7-dihydro-1H-imidazo[4,5-c]pyridin-5(4H)-yl)methanone |
| 815 | (R)-(4-(4,7-difluorobenzo[d]oxazol-2-yl)-6,7-dihydro-1H-imidazo[4,5-c]pyridin-5(4H)-yl)(5-(1-(difluoromethyl)-1H-pyrazol-3-yl)-1,3,4-oxadiazol-2-yl)methanone |
| 816 | (R)-(4-(4,7-difluorobenzo[d]oxazol-2-yl)-6,7-dihydro-1H-imidazo[4,5-c]pyridin-5(4H)-yl)(5-(1-(difluoromethyl)-1H-pyrazol-3-yl)-1,3,4-oxadiazol-2-yl)methanone |
| 818 | (R)-(4-(4,7-difluorobenzo[d]oxazol-2-yl)-6,7-dihydro-1H-imidazo[4,5-c]pyridin-5(4H)-yl)(5-(1-methyl-1H-imidazol-4-yl)-1,3,4-oxadiazol-2-yl)methanone |
| 820 | (R)-(1,3-dimethyl-1H-1,2,4-triazol-5-yl)(4-(4-methylbenzo[d]oxazol-2-yl)-6,7-dihydro-1H-imidazo[4,5-c]pyridin-5(4H)-yl)methanone |
| 822 | (R)-(1,3-dimethyl-1H-pyrazol-5-yl)(4-(7-methoxybenzo[d]oxazol-2-yl)-6,7-dihydro-1H-imidazo[4,5-c]pyridin-5(4H)-yl)methanone |
| 823 | (R)-(4-(difluoromethyl)-2-(1-hydroxy-2-methylpropan-2-yl)oxazol-5-yl)(4-(5-fluorobenzo[d]oxazol-2-yl)-6,7-dihydro-1H-imidazo[4,5-c]pyridin-5(4H)-yl)methanone |
| 826 | (R)-(4-(5-fluorobenzo[d]oxazol-2-yl)-6,7-dihydro- 1H-imidazo[4,5-c]pyridin-5(4H)-yl)(4-methyl-2-(1-methyl-1H-pyrazol-3-yl)oxazol-5-yl)methanone |
| 828 | (R)-(1,3-dimethyl-1H-1,2,4-triazol-5-yl)(4-(4-methoxybenzo[d]oxazol-2-yl)-6,7-dihydro-1H-imidazo[4,5-c]pyridin-5(4H)-yl)methanone |
| 829 | (R)-(5-(1-methyl-1H-pyrazol-4-yl)-1,3,4-oxadiazol-2-yl)(4-(7-(trifluoromethyl)benzo[d]oxazol-2-yl)-6,7-dihydro- 1H-imidazo[4,5-c]pyridin-5(4H)-vl)methanone |
| 831 | (R)-(5-(1-methyl-1H-pyrazol-3-yl)-1,3,4-oxadiazol-2-yl)(4-(7-(trifluoromethyl)benzo[d]oxazol-2-yl)-6,7-dihydro-1H-imidazo[4,5-c]pyridin-5(4H)-yl)methanone |
| 834 | (R)-(4-(benzo[d]oxazol-2-yl)-6,7-dihydro-1H-imidazo[4,5-c]pyridin-5(4H)-yl)(5-(3-fluoropyridin-2-yl)-1,3,4-oxadiazol-2-yl)methanone |
| 836 | (R)-(4-(benzo[d]oxazol-2-yl)-6,7-dihydro-1H-imidazo[4,5-c]pyridin-5(4H)-yl)(1-(2,2-difluoroethyl)-1H-pyrazol-5-yl)methanone |
| 837 | (R)-(5-(pyridin-2-yl)-1,3,4-oxadiazol-2-yl)(4-(7-(trifluoromethyl)benzo[d]oxazol-2-yl)-6,7-dihydro-1H-imidazo[4,5-c]pyridin-5(4H)-yl)methanone |
| 839 | (R)-(5-(3-fluoropyridin-2-yl)-1,3,4-oxadiazol-2-yl)(4-(7-(trifluoromethyl)benzo[d]oxazol-2-yl)-6,7-dihydro-1H-imidazo[4,5-c]pyridin-5(4H)-yl)methanone |
| 842 | (R)-(4-(benzo[d]oxazol-2-yl)-6,7-dihydro-1H-imidazo[4,5-c]pyridin-5(4H)-yl)(5-(1-(difluoromethyl)-1H-pyrazol-4-yl)-1,3,4-oxadiazol-2-yl)methanone |
| 844 | (R)-(4-(benzo[d]oxazol-2-yl)-6,7-dihydro-1H-imidazo[4,5-c]pyridin-5(4H)-yl)(5-(1-methyl-1H-pyrazol-3-yl)-1,3,4-oxadiazol-2-yl)methanone |
| 845 | (R)-(4-(benzo[d]oxazol-2-yl)-6,7-dihydro-1H-imidazo[4,5-c]pyridin-5(4H)-yl)(5-(1-(difluoromethyl)-1H-pyrazol-3-yl)-1,3,4-oxadiazol-2-yl)methanone |
| 848 | (R)-(1-(2,2-difluoroethyl)-1H-pyrazol-5-yl)(4-(7-fluorobenzo[d]oxazol-2-yl)-6,7-dihydro-1H-imidazo[4,5-c]pyridin-5(4H)-yl)methanone |
| 849 | (R)-(1-(2,2-difluoroethyl)-1H-pyrazol-5-yl)(4-(4-fluorobenzo[d]oxazol-2-yl)-6,7-dihydro-1H-imidazo[4,5-c]pyridin-5(4H)-yl)methanone |
| 851 | (R)-(5-(tert-butyl)-1,3,4-oxadiazol-2-yl)(4-(4,7-difluorobenzo[d]oxazol-2-yl)-6,7-dihydro-1H-imidazo[4,5-c]pyridin-5(4H)-yl)methanone |
| 853 | (R)-(5-(1-(difluoromethyl)-1H-pyrazol-3-yl)-1,3,4-oxadiazol-2-yl)(4-(7-(trifluoromethyl)benzo[d]oxazol-2-yl)-6,7-dihydro-1H-imidazo[4,5-c]pyridin-5(4H)-yl)methanone |
| 856 | (R)-(5-cyclopropyl-1,3,4-oxadiazol-2-yl)(4-(4,7-difluorobenzo[d]oxazol-2-yl)-6,7-dihydro-1H-imidazo[4,5-c]pyridin-5(4H)-yl)methanone |
| 857 | (R)-(5-(3-fluoropyridin-2-yl)-1,3,4-oxadiazol-2-yl)(4-(7-methylbenzo[d]oxazol-2-yl)-6,7-dihydro-1H-imidazo[4,5-c]pyridin-5(4H)-yl)methanone |
| 859 | (R)-(5-(1-(difluoromethyl)-1H-pyrazol-3-yl)-1,3,4-oxadiazol-2-yl)(4-(7-methylbenzo[d]oxazol-2-yl)-6,7-dihydro-1H-imidazo[4,5-c]pyridin-5(4H)-yl)methanone |
| 861 | (R)-(5-(1-(difluoromethyl)-1H-pyrazol-3-yl)-1,3,4-oxadiazol-2-yl)(4-(7-fluorobenzo[d]oxazol-2-yl)-6,7-dihydro-1H-imidazo[4,5-c]pyridin-5(4H)-yl)methanone |
| 864 | (R)-(4-(2,2-difluoroethyl)oxazol-5-yl)(4-(7-fluorobenzo[d]oxazol-2-yl)-6,7-dihydro-1H-imidazo[4,5-c]pyridin-5(4H)-yl)methanone |
| 866 | (R)-(4-(2,2-difluoroethyl)oxazol-5-yl)(4-(5-fluorobenzo[d]oxazol-2-yl)-6,7-dihydro-1H-imidazo[4,5-c]pyridin-5(4H)-yl)methanone |
| 867 | (R)-(4-(7-fluorobenzo[d]oxazol-2-yl)-6,7-dihydro-1H-imidazo[4,5-c]pyridin-5(4H)-yl)(5-(3-fluoropyridin-2-yl)-1,3,4-oxadiazol-2-yl)methanone |
| 870 | (R)-(5-(1-methyl-1H-pyrazol-3-yl)-1,3,4-oxadiazol-2-yl)(4-(7-methylbenzo[d]oxazol-2-yl)-6,7-dihydro-1H-imidazo[4,5-c]pyridin-5(4H)-yl)methanone |
| 871 | (R)-(5-(1-(difluoromethyl)-1H-pyrazol-4-yl)-1,3,4-oxadiazol-2-yl)(4-(7-methylbenzo[d]oxazol-2-yl)-6,7-dihydro-1H-imidazo[4,5-c]pyridin-5(4H)-yl)methanone |
| 874 | (R)-(4-(benzo[d]oxazol-2-yl)-6,7-dihydro-1H-imidazo[4,5-c]pyridin-5(4H)-yl)(4-(2,2-difluoroethyl)oxazol-5-yl)methanone |
| 876 | (R)-(5-(1-(difluoromethyl)-1H-pyrazol-4-yl)-1,3,4-oxadiazol-2-yl)(4-(7-fluorobenzo[d]oxazol-2-yl)-6,7-dihydro-1H-imidazo[4,5-c]pyridin-5(4H)-yl)methanone |
| 878 | (R)-(6-(methoxymethyl)pyrazolo[1,5-a]pyridin-3-yl)(4-(5-methylbenzo[d]oxazol-2-yl)-6,7-dihydro-1H-imidazo[4,5-c]pyridin-5(4H)-yl)methanone |
| 880 | (R)-(4-(5-fluorobenzo[d]oxazol-2-yl)-6,7-dihydro- 1H-imidazo[4,5-c]pyridin-5(4H)-yl)(1-(pyridin-2-yl)-1H-pyrazol-4-yl)methanone |
| 882 | (R)-(5-(1,3-dimethyl-1H-pyrazol-4-yl)-1,3,4-oxadiazol-2-yl)(4-(5-fluorobenzo[d]oxazol-2-yl)-6,7-dihydro-1H-imidazo[4,5-c]pyridin-5(4H)-yl)methanone |
| 883 | (R)-(4-(difluoromethyl)oxazol-5-yl)(4-(6-methylbenzo[d]oxazol-2-yl)-6,7-dihydro-1H-imidazo[4,5-c]pyridin-5(4H)-yl)methanone |
| 886 | (R)-(4-ethyl-4H-1,2,4-triazol-3-yl)(4-(5-fluorobenzo[d]oxazol-2-yl)-6,7-dihydro-1H-imidazo[4,5-c]pyridin-5(4H)-yl)methanone |
| 888 | (R)-1-(4-(4-fluorobenzo[d]oxazol-2-yl)-6,7-dihydro-1H-imidazo[4,5-c]pyridin-5(4H)-yl)-3-(oxazol-2-yl)propan-1-one |
| 889 | (R)-(4-(difluoromethyl)oxazol-5-yl)(4-(4,7-dimethylbenzo[d]oxazol-2-yl)-6,7-dihydro-1H-imidazo[4,5-c]pyridin-5(4H)-yl)methanone |
| 891 | (R)-(4-(difluoromethyl)-2-(1-methyl-1H-imidazol-4-yl)oxazol-5-yl)(4-(7-fluorobenzo[d]oxazol-2-yl)-1,4,6,7-tetrahydro-5H-imidazo[4,5-c]pyridin-5-yl)methanone |
| 894 | (R)-(4-(4-fluorobenzo[d]oxazol-2-yl)-6,7-dihydro-1H-imidazo[4,5-c]pyridin-5(4H)-yl)(2-(2-hydroxypropan-2-yl)oxazol-5-yl)methanone |
| 896 | (R)-(4-(4,7-difluorobenzo[d]oxazol-2-yl)-6,7-dihydro-1H-imidazo[4,5-c]pyridin-5(4H)-yl)(6-(4-hydroxypiperidin-1-yl)pyrazolo[1,5-a]pyridin-3-yl)methanone |
| 897 | (R)-(4-(difluoromethyl)oxazol-5-yl)(4-(7-fluoro-4-methylbenzo[d]oxazol-2-yl)-6,7-dihydro-1H-imidazo[4,5-c]pyridin-5(4H)-yl)methanone |
| 900 | (R)-(4-(difluoromethyl)oxazol-5-yl)(4-deuterium-4-(7-fluorobenzo[d]oxazol-2-yl)-6,7-dihydro-1H-imidazo[4,5-c]pyridin-5(4H)-yl)methanone |
| 902 | (R)-(1-methyl-1H-1,2,4-triazol-5-yl)(4-(4-methylbenzo[d]oxazol-2-yl)-6,7-dihydro-1H-imidazo[4,5-c]pyridin-5(4H)-yl)methanone |
| 904 | (R)-(4-(4-methoxybenzo[d]oxazol-2-yl)-6,7-dihydro-1H-imidazo[4,5-c]pyridin-5(4H)-yl)(1-methyl-1H-1,2,4-triazol-5-yl)methanone |
| 906 | (R)-(4-(deuterium)-2-(2-hydroxypropan-2-yl)oxazol-5-yl)(4-fluoro-4-(5-fluorobenzo[d]oxazol-2-yl)-6,7-dihydro-1H-imidazo[4,5-c]pyridin-5(4H)-yl)methanone |
| 908 | (R)-(1,3-dimethyl-1H-1,2,4-triazol-5-yl)(4-(7-methylbenzo[d]oxazol-2-yl)-6,7-dihydro-1H-imidazo[4,5-c]pyridin-5(4H)-yl)methanone |
| 910 | (R)-(1,3-dimethyl-1H-1,2,4-triazol-5-yl)(4-(7-methoxybenzo[d]oxazol-2-yl)-6,7-dihydro-1H-imidazo[4,5-c]pyridin-5(4H)-yl)methanone |
| 912 | (R)-(4-(difluoromethyl)oxazol-5-yl)(4'-(7-fluorobenzo[d]oxazol-2-yl)spiro[cyclopropane-1,7'-imidazo[4,5-c]pyridin]-5'(1H,4'H,6H)-yl)methanone |
| 914 | (R)-1-(4-(benzo[d]oxazol-2-yl)-6,7-dihydro-1H-imidazo[4,5-c]pyridin-5(4H)-yl)-3-(thiazol-2-yl)propan-1-one |
| 916 | (R)-(4-(5-fluorobenzo[d]oxazol-2-yl)-6,7-dihydro- 1H-imidazo[4,5-c]pyridin-5(4H)-yl)(2-(trifluoromethyl)pyrazolo[1,5-a]pyridin-3-yl)methanone |
| 918 | (R)-(4-(5-fluorobenzo[d]oxazol-2-yl)-6,7-dihydro- 1H-imidazo[4,5-c]pyridin-5(4H)-yl)(1-(oxazol-5-yl)cyclopropyl)methanone |
| 920 | (R)-(1-(difluoromethyl)-1H-pyrazol-5-yl)(4-(7-methylbenzo[d]oxazol-2-yl)-6,7-dihydro-1H-imidazo[4,5-c]pyridin-5(4H)-yl)methanone |

In some embodiments, the disclosure provides specific examples of Formula I, and their pharmaceutically acceptable salts and/or isotopologues, as set forth in Table 2 below.

| **Table 2** | | |
|---|---|---|
| **Ex. #** | **Structure** | **Name** |
| 921 | 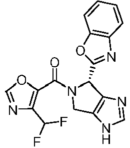 | (S)-(4-(benzo[d]oxazol-2-yl)-4,6-dihydropyrrolo[3,4-d]imidazol-5(1H)-yl)(4-(difluoromethyl)oxazol-5-yl)methanone |
| 922 | 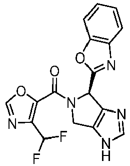 | (R)-(4-(benzo[d]oxazol-2-yl)-4,6-dihydropyrrolo[3,4-d]imidazol-5(1H)-yl)(4-(difluoromethyl)oxazol-5-yl)methanone |
| 923 | 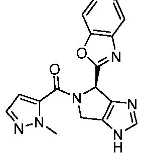 | (R)-(4-(benzo[d]oxazol-2-yl)-4,6-dihydropyrrolo[3,4-d]imidazol-5(1H)-yl)(1-methyl-1H-pyrazol-5-yl)methanone |
| 924 | 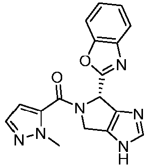 | (S)-(4-(benzo[d]oxazol-2-yl)-4,6-dihydropyrrolo[3,4-d]imidazol-5(1H)-yl)(1-methyl-1H-pyrazol-5-yl)methanone |
| 925 | 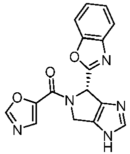 | (S)-(4-(benzo[d]oxazol-2-yl)-4,6-dihydropyrrolo[3,4-d]imidazol-5(1H)-yl)(oxazol-5-yl)methanone |
| 926 | 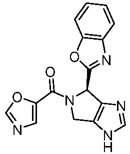 | (R)-(4-(benzo[d]oxazol-2-yl)-4,6-dihydropyrrolo[3,4-d]imidazol-5(1H)-yl)(oxazol-5-yl)methanone |
| 927 | 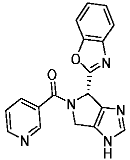 | (S)-(4-(benzo[d]oxazol-2-yl)-4,6-dihydropyrrolo[3,4-d]imidazol-5(1H)-yl)(pyridin-3-yl)methanone |
| 928 | 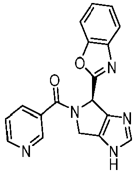 | (R)-(4-(benzo[d]oxazol-2-yl)-4,6-dihydropyrrolo[3,4-d]imidazol-5(1H)-yl)(pyridin-3-yl)methanone |
| 929 | 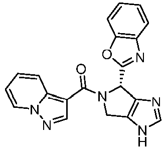 | (S)-(4-(benzo[d]oxazol-2-yl)-4,6-dihydropyrrolo[3,4-d]imidazol-5(1H)-yl)(pyrazolo[1,5-a]pyridin-3-yl)methanone |
| 930 | 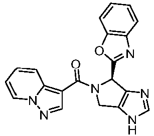 | (R)-(4-(benzo[d]oxazol-2-yl)-4,6-dihydropyrrolo[3,4-d]imidazol-5(1H)-yl)(pyrazolo[1,5-a]pyridin-3-yl)methanone |
| 931 | 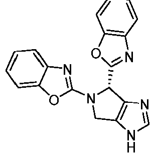 | (S)-2,2'-(4,6-dihydropyrrolo[3,4-d]imidazole-4,5(1H)-diyl)bis(benzo[d]oxazole) |
| 932 | 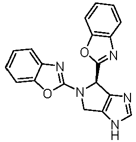 | (R)-2,2'-(4,6-dihydropyrrolo[3,4-d]imidazole-4,5(1H)-diyl)bis(benzo[d]oxazole) |
| 933 | 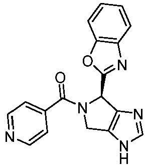 | (R)-(4-(benzo[d]oxazol-2-yl)-4,6-dihydropyrrolo[3,4-d]imidazol-5(1H)-yl)(pyridin-4-yl)methanone |
| 934 | 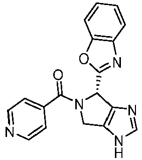 | (S)-(4-(benzo[d]oxazol-2-yl)-4,6-dihydropyrrolo[3,4-d]imidazol-5(1H)-yl)(pyridin-4-yl)methanone |
| 935 | 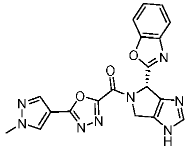 | (S)-(4-(benzo[d]oxazol-2-yl)-4,6-dihydropyrrolo[3,4-d]imidazol-5(1H)-yl)(5-(1-methyl-1H-pyrazol-4-yl)-1,3,4-oxadiazol-2-yl)methanone |
| 936 | 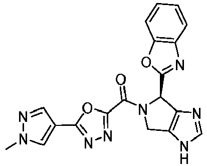 | (R)-(4-(benzo[d]oxazol-2-yl)-4,6-dihydropyrrolo[3,4-d]imidazol-5(1H)-yl)(5-(1-methyl-1H-pyrazol-4-yl)-1,3,4-oxadiazol-2-yl)methanone |
| 937 | 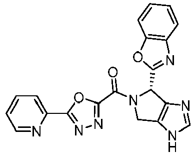 | (S)-(4-(benzo[d]oxazol-2-yl)-4,6-dihydropyrrolo[3,4-d]imidazol-5(1H)-yl)(5-(pyridin-2-yl)-1,3,4-oxadiazol-2-yl)methanone |
| 938 | 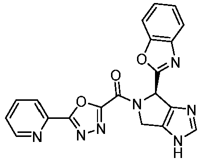 | (R)-(4-(benzo[d]oxazol-2-yl)-4,6-dihydropyrrolo[3,4-d]imidazol-5(1H)-yl)(5-(pyridin-2-yl)-1,3,4-oxadiazol-2-yl)methanone |
| 939 | 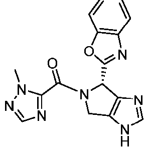 | (S)-(4-(benzo[d]oxazol-2-yl)-4,6-dihydropyrrolo[3,4-d]imidazol-5(1H)-yl)(1-methyl-1H-1,2,4-triazol-5-yl)methanone |
| 940 | 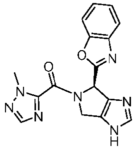 | (R)-(4-(benzo[d]oxazol-2-yl)-4,6-dihydropyrrolo[3,4-d]imidazol-5(1H)-yl)(1-methyl-1H-1,2,4-triazol-5-yl)methanone |
| 941 | 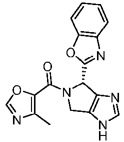 | (S)-(4-(benzo[d]oxazol-2-yl)-4,6-dihydropyrrolo[3,4-d]imidazol-5(1H)-yl)(4-methyloxazol-5-yl)methanone |
| 942 | 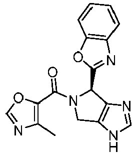 | (R)-(4-(benzo[d]oxazol-2-yl)-4,6-dihydropyrrolo[3,4-d]imidazol-5(1H)-yl)(4-methyloxazol-5-yl)methanone |
| 943 | 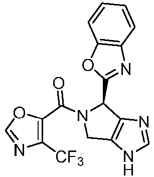 | (R)-(4-(benzo[d]oxazol-2-yl)-4,6-dihydropyrrolo[3,4-d]imidazol-5(1H)-yl)(4-(trifluoromethyl)oxazol-5-yl)methanone |
| 944 | 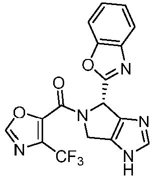 | (S)-(4-(benzo[d]oxazol-2-yl)-4,6-dihydropyrrolo[3,4-d]imidazol-5(1H)-yl)(4-(trifluoromethyl)oxazol-5-yl)methanone |
| 945 | 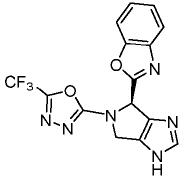 | (R)-2-(5-(5-(trifluoromethyl)-1,3,4-oxadiazol-2-yl)-1,4,5,6-tetrahydropyrrolo[3,4-d]imidazol-4-yl)benzo[d]oxazole |
| 946 | 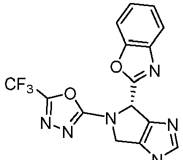 | (S)-2-(5-(5-(trifluoromethyl)-1,3,4-oxadiazol-2-yl)-1,4,5,6-tetrahydropyrrolo[3,4-d]imidazol-4-yl)benzo[d]oxazole |
| 947 | 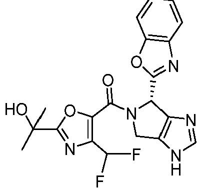 | (S)-(4-(benzo[d]oxazol-2-yl)-4,6-dihydropyrrolo[3,4-d]imidazol-5(1H)-yl)(4-(difluoromethyl)-2-(2-hydroxypropan-2-yl)oxazol-5-yl)methanone |
| 948 | 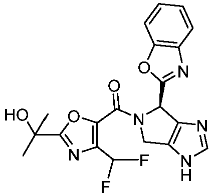 | (R)-(4-(benzo[d]oxazol-2-yl)-4,6-dihydropyrrolo[3,4-d]imidazol-5(1H)-yl)(4-(difluoromethyl)-2-(2-hydroxypropan-2-yl)oxazol-5-yl)methanone |
| 949 | 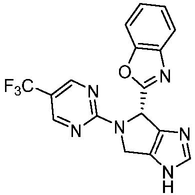 | (S)-2-(5-(5-(trifluoromethyl)pyrimidin-2-yl)-1,4,5,6-tetrahydropyrrolo[3,4-d]imidazol-4-yl)benzo[d]oxazole |
| 950 | 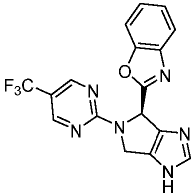 | (R)-2-(5-(5-(trifluoromethyl)pyrimidin-2-yl)-1,4,5,6-tetrahydropyrrolo[3,4-d]imidazol-4-yl)benzo[d]oxazole |
| 951 | 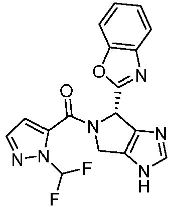 | (S)-(4-(benzo[d]oxazol-2-yl)-4,6-dihydropyrrolo[3,4-d]imidazol-5(1H)-yl)(1-(difluoromethyl)-1H-pyrazol-5-yl)methanone |
| 952 | 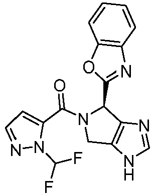 | (R)-(4-(benzo[d]oxazol-2-yl)-4,6-dihydropyrrolo[3,4-d]imidazol-5(1H)-yl)(1-(difluoromethyl)-1H-pyrazol-5-yl)methanone |
| 953 | 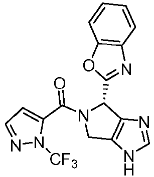 | (S)-(4-(benzo[d]oxazol-2-yl)-4,6-dihydropyrrolo[3,4-d]imidazol-5(1H)-yl)(1-(trifluoromethyl)-1H-pyrazol-5-yl)methanone |
| 954 | 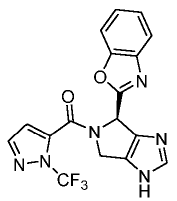 | (R)-(4-(benzo[d]oxazol-2-yl)-4,6-dihydropyrrolo[3,4-d]imidazol-5(1H)-yl)(1-(trifluoromethyl)-1H-pyrazol-5-yl)methanone |

In further embodiments, the compound of Formula I is one or more of the compounds in Table 2 that is the S-enantiomer, or a pharmaceutically acceptable salt thereof.

In still further embodiments, the compound of Formula I is one or more of Examples 5, 23, 29, 33, 39, 47, 51, 67, 93, 107, 124, 141, 157, 161, 163, 167, 169, 171, 173, 187, 191, 193, 195, 197, 199, 201, 217, 227, 229, 231, 239, 243, 245, 247, 252, 259, 263, 269, 272, 277, 281, 283, 285, 301, 305, 309, 373, 467, 483, 486, 487, 518, 561, 563, 569, 599, and 683, or a pharmaceutically acceptable salt thereof. In yet other embodiments, the compound of Formula I is one or more of Examples 5, 23, 29, 39, 51, 67, 93, 107, 124, 141, 157, 161, 163, 167, 169, 171, 173, 187, 191, 193, 195, 197, 199, 201, 217, 227, 229, 243, 245, 247, 252, 259, 263, 269, 272, 277, 281, 283, 285, 301, 305, 309, 373, 467, 483, 486, 487, 518, 561, 563, 569, 599, and 683, or a pharmaceutically acceptable salt thereof.

In other embodiments, the compound of Formula I is a racemate of one or more of the compounds in Table 1S, or a pharmaceutically acceptable salt thereof.

Abbreviations and terms list:

| | |
|---|---|
| aq | aqueous |
| min | minute(s) |
| hrs | hours |
| mL | milliliter |
| µL | microliter |
| mmol | millimole(s) |
| µmol | micromole(s) |
| mol | mole(s) |
| M | molar |
| eq | equivalents |
| LCMS | Liquid chromatography mass spectrometry |
| NMR | nuclear magnetic resonance |
| TLC | thin layer chromatography |
| HPLC | high-performance liquid chromatography |
| SFC | supercritical fluid chromatography |
| sat | saturated |
| °C | degrees Celsius |
| rt | room temperature |
| N₂ | nitrogen gas |
| Hz | Hertz |
| δ | chemical shift |
| s | singlet |
| d | doublet |
| t | triplet |
| q | quartet |
| m | multiplet |
| br | broad |
| dd | doublet of doublets |
| ddd | doublet of doublet or doublets |
| td | triplet of doublets |
| dt | doublet of triplets |
| CDCl₃ | chloroform-*d* |
| CD₃OD | methanol-*d₄* |
| DMSO-d₆ | dimethyl sulfoxide-*d₆* |

Solvents and reagents:

| | |
|---|---|
| DCE | 1,2-dichloroethane |
| DCM | dichloromethane |
| DMAc | dimethylacetamide |
| DME | dimethoxyethane |
| DMF | dimethylformamide |
| EtOH | ethyl alcohol |
| EtOAc | ethyl acetate |
| i-PrOH or IPA | isopropanol |
| MeOH | methyl alcohol |
| MeCN or ACN | acetonitrile |
| NMP | 1-methyl-2-pyrrolidone |
| PE | petroleum ether |
| TBME | tert-butyl methyl ether |
| H₂O | water |
| THF | tetrahydrofuran |
| HOAc | acetic acid |
| HCl | hydrochloric acid |
| H₂SO₄ | sulfuric acid |
| FA | formic acid |
| TFA | trifluoroacetic acid |
| TsOH | p-toluenesulfonic acid |
| TosCl or TsCl | p-toluenesulfonyl chloride |
| NH₄Cl | ammonium chloride |
| KOH | Potassium hydroxide |
| LiOH | lithium hydroxide |
| K₂CO₃ | potassium carbonate |
| Na₂CO₃ | sodium carbonate |
| Cs₂CO₃ | cesium carbonate |
| Na₂SO₄ | sodium sulfate |
| NaHCO₃ | sodium bicarbonate |
| Na₂S₂O₃ | sodium thiosulfate |
| Et₃N or TEA | triethylamine |
| DIPEA | N,N-diisopropylethylamine |
| Py | pyridine |
| KOAc | potassium acetate |
| KF | potassium fluoride |
| CsF | cesium fluoride |
| TBAF | tetrabutylammonium fluoride |
| NH₃ | ammonia |
| LDA | lithium diisopropylamine |
| t-BuLi | tert-butyllithium |
| n-BuLi | n-butyllithium |
| LiHMDS | lithium bis(trimethylsilyl)amide |
| MeMgBr | methylmagnesium bromide |
| iPrMgCl.LiC1 | isopropylmagnesium chloride lithium chloride complex |
| PFIB | perfluoroisobutene |
| BuOK | potassium t-butoxide |
| CAN | cerium ammonium nitrate |
| DIBAL-H | diisobutylaluminium hydride |
| NaOMe | sodium methoxide |
| NaH | sodium hydride |
| DMP | Dess-Martin periodinane |
| MnO₂ | manganese(IV) oxide |
| IBX | 2-iodoxybenzoic acid |
| t-BuONO | tert-butyl nitrite |
| PIDA | (diacetoxyiodo)benzene |
| NFSI | N-fluorodi(benzenesulfonyl)amine |
| NBS | N-bromosuccinimide |
| NCS | N-chlorosuccinimide |
| MeI | methyl iodide |
| LiBH₄ | lithium borohydride |
| NaBH₄ | sodium borohydride |
| NaBH(OAc)₃ | Sodium triacetoxyborohydride |
| EDCI | 1-ethyl-3-(3-dimethylaminopropyl)carbodiimide |
| HOBt | 1-hydroxybenzotriazole |
| HATU | 1-[Bis(dimethylamino)methylene]-1H-1,2,3-triazolo[4,5-b]pyridinium 3-oxid hexafluorophosphate |
| T3P | Propanephosphonic acid anhydride |
| CDI | carbonyldiimidazole |
| Thio-CDI | 1,1'-Thiocarbonyldiimidazole |
| DPPA | diphenylphosphoryl azide |
| BCl₃ | boron trichloride |
| Boc₂O | di-tert-butyl dicarbonate |
| DHP | Dihydropyran |
| Pd/C | palladium on carbon |
| Pd(PPh₃)₂Cl₂ | bis(triphenylphosphine)palladium(II) dichloride |
| Pd₂(dba)₃ | tris(dibenzylideneacetone)dipalladium |
| Pd(dppf)Cl₂ | (1,1'-Bis(diphenylphosphino)ferrocene)palladium(II) dichloride |
| Pd(OAc)₂ | palladium(II) acetate |
| Xantphos | 4,5-Bis(diphenylphosphino)-9,9-dimethylxanthene |
| t-BuXPhos-Pd-G3 | [(2-Di-tert-butylphosphino-2',4',6'-triisopropyl-1,1'-biphenyl)-2-(2'-amino-1,1'-biphenyl)] palladium(II) methanesulfonate |
| CPhos-Pd-G3 | [(2-Dicyclohexylphosphine-2',6'-bis(N,N-dimethylamino)-1,1'-biphenyl)-2-(2'-amino-1,1'-biphenyl)] palladium(II) methanesulfonate |
| PPh₃ | triphenylphosphine |
| DEAD | diethyl azodicarboxylate |
| DIAD | diisopropyl azodicarboxylate |
| Pin₂B₂ | bis(pinacolato)diborane |
| CuI | copper iodide |
| CuBr₂ | copper (II) bromide |
| Zn(CN)₂ | zinc cyanide |
| H₂ | hydrogen gas |
| SiO₂ | silica |
| TFAA | Trifluoroacetic anhydride |
| K₂OsO₄.2H₂O | potassium osmate(VI) dihydrate |
| NaIO₄ | sodium periodate |

### General Experimental

In the following examples, the reagents and solvents were purchased from commercial sources (such as Alfa, Acros, AstaTech, CombiBlocks, Enamine, Sigma Aldrich, TCI, PharmaBock, Bide Pharmatech Ltd., Accela ChemBio, Aladdin, Shanghai Haohong Pharmaceutical Co., Ltd, Amkchem, Beijing Ouhe Technology Co., Ltd, Haoyuan Chemexpress Co., Ltd, Hualun, Coolpharm, Scochem, Titan and WuXi LabNetwork, and used without further purification unless otherwise specified. Flash chromatography was performed on a CombiFlashRf 150 (ISCO) via column with silica gel particles of 200-300 mesh. HPLC was performed on an Agilent 1100 Liquid Chromatography (Agilent, USA) and a Shimadzu LC 20/20A (Shimadzu, Japan). Supercritical fluid chromatography was performed on a Waters Prep SFC 150 AP /80Q /200 / 350 system (Waters, USA). Analytical and preparative thin layer chromatography plates (TLC) were HSGF 254 (0.15-0.2mm thickness, Shanghai Anbang Company, China). Nuclear magnetic resonance (NMR) spectra were obtained on a Brucker AV-400 NMR (Bruker, Switzerland). Chemical shifts were reported in parts per million (ppm, δ) downfield from tetramethylsilane. Mass spectra were given with electrospray ionization (ESI) from a Waters LCT TOF Mass Spectrometer (Waters, USA). LC-MS was performed on an Agilent Prime-6125B/ Agilent LC1260-MS6150/ Agilent LC1260-MS6125B/ Agilent LC1200-MS6110 (Agilent, USA) and a Shimadzu LC20-MS2020. Microwave reactions were run on an Initiator 2.5 Microwave Synthesizer (Biotage, Sweden).

### Intermediate (1-(1H-imidazol-5-yl)cyclopropyl)methanamine hydrochloride

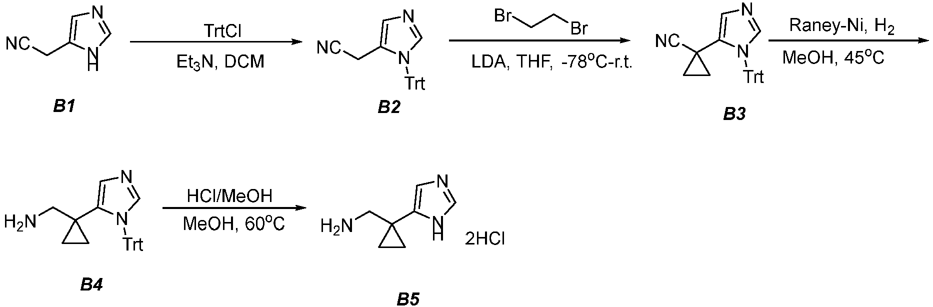

### Step 1: Preparation of 2-(3-tritylimidazol-4-yl)acetonitrile (B2)

B2 was prepared starting with 2-(1H-imidazol-5-yl)acetonitrile (B1) in accordance with literature procedures. See, e.g., WO2008/003766 (page 19).

### Step 2: Preparation of 1-(3-tritylimidazol-4-yl)cyclopropanecarbonitrile (B3)

To a solution of 2-(3-tritylimidazol-4-yl)acetonitrile (B2) (10 g, 28.6 mmol) in THF (200 mL) was added LDA (2 M, 42.9 mL, 3 eq) dropwise at -78°C. After addition was complete, the reaction mixture was stirred at -20°C to -10°C for 1 hr. The reaction mixture was then cooled to -78°C, and 1,2-dibromoethane (10.75 g, 57.2 mmol, 4.32 mL, 2 eq) was added dropwise at -78°C. After the addition was complete, the reaction mixture was warmed to rt slowly and stirred for another 2 hrs. Reaction progress was tracked using TLC (PE:EtOAc=1:1). The reaction mixture was quenched with sat. NH₄Cl solution (200 mL) and stirred at rt for 0.5 hr. The aqueous portion was extracted with EtOAc (100 mL x 3), and the combined organic layers were dried over Na₂SO₄, filtered, and concentrated to dryness. The residue (combined with two other reactions performed using 10 g of B2) was purified by flash silica gel chromatography (ISCO^{®}; 120 g SepaFlash^{®} Silica Flash Column, eluent of 0~40% EtOAc/PE, gradient @ 40 mL/min) to give B3 (23.4 g, 72% yield). ¹H NMR (400 MHz, DMSO-*d₆*) δ 7.30-7.52 (m, 10 H), 7.09 (dd, 6 H), 6.84 (d, 1 H), 1.51-1.66 (m, 2 H), 1.29-1.46 (m, 2 H).

### Step 3: Preparation of 1-(3-tritylimidazol-4-yl)cyclopropyl]methanamine (B4)

A mixture of 1-(3-tritylimidazol-4-yl)cyclopropanecarbonitrile (B3) (19 g, 50.6 mmol), Raney-Ni (4.60 g, 53.7 mmol, 1.06 eq), NH₃.H₂O (591 mg, 5.06 mmol, 650 µL, 30% purity, 0.1 eq) in MeOH (200 mL) was degassed and purged with H₂ 3 times, and then the reaction mixture was stirred at 45°C for 16 hrs under H₂ (45 psi) atmosphere. Reaction progress was tracked using TLC (DCM:MeOH=10:1). The reaction mixture was filtered, and additional Raney-Ni (4.60 g, 53.7 mmol, 1.06 eq) was added. The reaction mixture was stirred at 45°C under H₂ (45 psi) atmosphere for another 24 hrs. The reaction mixture was filtered through a pad of Celite, and the filtrate was concentrated to dryness to give B4 (18 g), which was used without further purification. ¹H NMR (400 MHz, DMSO-*d₆*) δ 7.33-7.44 (m, 9 H), 7.18-7.25 (m, 1 H), 7.04-7.13 (m, 6 H), 6.64-6.71 (m, 1 H), 3.17 (br s, 2 H), 2.58-2.74 (m, 2 H), 0.60-0.82 (m, 4 H).

### Step 4: Preparation of [1-(1H-imidazol-5-yl)cyclopropyl]methanamine hydrochloride (B5)

To a solution of [1-(3-tritylimidazol-4-yl)cyclopropyl]methanamine (B4) (18 g, 47.4 mmol) in MeOH (100 mL) was added HCl/MeOH (4 M, 100 mL, 8.4 eq). The reaction mixture was stirred at 60°C for 16 hrs. Reaction progress was tracked using LC-MS. The reaction mixture was concentrated to dryness, and the residue was triturated with EtOAc(40 mL) and stirred for 15 min. The precipitate was collected by filtration and then dried in vacuo to give B5 (8.5 g, 2HCl salt). ¹H NMR (400 MHz, DMSO-*d₆*) δ 14.73 (br s, 1 H), 9.04 (s, 1 H), 8.27 (br s, 2 H), 7.51 (s, 1 H), 2.98-3.22 (m, 2 H), 0.90-1.23 (m, 4 H).

### Intermediate 2-(1H-imidazol-5-yl)-2-methylpropan-1-amine hydrochloride

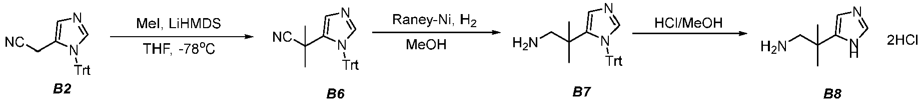

### Step 1: Preparation of (2-methyl-2-(3-tritylimidazol-4-yl)propanenitrile (B6)

To a solution of 2-(3-tritylimidazol-4-yl)acetonitrile (B2) (12 g, 34.3 mmol) in THF (400 mL) was drop-wise added LiHMDS (1 M, 96.2 mL, 2.8 eq) at -78°C. The reaction mixture was stirred at -78°C for 15 min, and then MeI was added (14.62 g, 103 mmol, 6.41 mL, 3 eq). The reaction mixture was stirred at -78°C for 1 hr, and then warmed to rt for 18 hrs. Reaction progress was tracked using TLC (PE:EtOAc = 1:1). The reaction mixture was cooled to 0°C, quenched by the addition of sat. NH₄Cl solution (15 mL) and then H₂O (60 mL) was added. The aqueous portion was extracted with EtOAc (60 mL x 3). The combined organic layers were washed with brine (60 mL), dried over Na₂SO₄, and concentrated to dryness. The residue (combined with the residue from another reaction performed using 2 g of B2) was purified by flash silica gel chromatography (ISCO^{®}; 80 g SepaFlash^{®} Silica Flash Column, eluent of 0~80% EtOAc/PE, gradient @ 60 mL/min) to give B6 (14 g, 92% yield). ¹H NMR (400 MHz, CDCl₃) δ 7.41 (d, 1 H), 7.32-7.39 (m, 9 H), 7.08-7.16 (m, 6 H), 6.81 (d, 1 H), 1.69 (s, 6 H).

### Step 2: Preparation of (2-methyl-2-(3-tritylimidazol-4-yl)propan-1-amine (B7)

To a solution of 2-methyl-2-(3-tritylimidazol-4-yl)propanenitrile (B6) (14 g, 37.1 mmol) in MeOH (350 mL) was added Raney-Ni (2 g, 23.3 mmol, 0.63 eq) under N₂. The reaction mixture was degassed under vacuum and purged with H₂ several times. The reaction mixture was stirred under H₂ (50 psi) at 40°C for 36 hrs. Reaction progress was tracked using TLC (PE:EtOAc = 1:1). The reaction mixture was filtered through of pad of celite, and the filtrate was concentrated to dryness to afford B7 (12 g, 85% yield). ¹H NMR (400 MHz, CDCl₃) δ 7.38 (s, 1 H), 7.29-7.36 (m, 9 H), 7.10-7.18 (m, 6 H), 6.55 (s, 1 H), 3.49 (br s, 1 H), 2.74 (s, 2 H), 1.20 (s, 6 H); LCMS: m/z 382.2 [M+H]⁺.

### Step 3: Preparation of (2-(1H-imidazol-5-yl)-2-methyl-propan-1-amine (B8)

To a solution of 2-methyl-2-(3-tritylimidazol-4-yl)propan-1-amine (B7) (14 g, 36.7 mmol) in MeOH (150 mL) was added HCl/MeOH (4 M, 150 mL, 16.4 eq), and the reaction mixture was stirred at 50°C for 18 hrs. Reaction progress was tracked using TLC (EtOAc). The reaction mixture was concentrated to dryness, and 200 mL of EtOAc was added. The mixture was stirred at 60°C for 1 hr. The precipitate was collected by filtration and rinsed with EtOAc, and then dried in vacuo to give B8 (7.0 g, 90% yield, 2HCl salt). ¹H NMR (400 MHz, CD₃OD) δ 8.96 (s, 1 H), 7.52 (s, 1 H), 3.20-3.27 (m, 2 H), 1.49 (s, 6 H).

### Intermediate 2-(1H-imidazol-5-yl)propan-1-amine hydrochloride

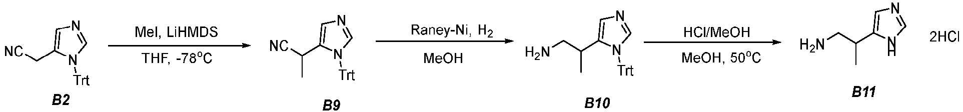

### Step 1: Preparation of 2-(3-tritylimidazol-4-yl)propanenitrile (B9)

To a solution of 2-(3-tritylimidazol-4-yl)acetonitrile (B2) (15 g, 42.9 mmol) in THF (120 mL) was added LiHMDS (1 M, 51.5 mL, 1.2 eq) dropwise at -78°C. After addition, the reaction mixture was stirred for 0.5 hr, and then MeI (10.97 g, 77.3 mmol, 4.81 mL, 1.8 eq) was added dropwise at -78°C. The reaction mixture was stirred at -78°C for 2 hrs. Reaction progress was tracked using TLC (PE:EtOAc=2:1). The reaction mixture was quenched by the addition of sat. NH₄Cl solution (120 mL) and stirred for 15 min. The aqueous portion was extracted with EtOAc (400 mL), and the organic layer washed by water (100 mL) and brine (60 mL), dried over Na₂SO₄, filtered, and concentrated to dryness. The residue (combined with the residue from another reaction performed using 15 g of B2) was purified by column chromatography on silica gel (PE:EtOAc=20:1 to 2:1) to give B9 (33.5 g, 75% yield). ¹H NMR (400 MHz, DMSO-*d*₆) δ 7.38-7.45 (m, 10 H), 7.06-7.13 (m, 6 H), 6.82-6.92 (m, 1 H), 4.12-4.23 (m, 1 H), 1.48 (d, 3 H).

### Step 2: Preparation of 2-(3-tritylimidazol-4-yl)propan-1-amine (B10)

A mixture of 2-(3-tritylimidazol-4-yl)propanenitrile (B9) (16.5 g, 45.4 mmol), Raney-Ni (4.08 g, 47.7 mmol, 1.05 eq), NH₃.H₂O (910 mg, 7.79 mmol, 1 mL, 30% purity) in MeOH (300 mL) was degassed under vacuum and purged with H₂ 3 times. The reaction mixture was stirred at 45°C for 16 hrs under H₂ (45 psi) atmosphere. Reaction progress was tracked using TLC (PE:EtOAc=2:1). The reaction mixture was filtered through a pad of Celite and the filtrate was concentrated to dryness to afford B10 (33 g), which was used without further purification. ¹H NMR (400 MHz, CD₃OD) δ 7.36-7.44 (m, 10 H), 7.13-7.20 (m, 6 H), 6.72 (br s, 1 H), 2.57-2.92 (m, 3 H), 1.21 (br d, 3 H).

### Step 3: Preparation of 2-(1H-imidazol-5-yl)propan-1-amine (B11)

To a solution of 2-(3-tritylimidazol-4-yl)propan-1-amine (B10) (23 g, 62.6 mmol) in MeOH (100 mL) was added HCl/MeOH (4 M, 100 mL). The mixture was stirred at rt for 12 hrs. Reaction progress was tracked using TLC (EtOAc). The reaction mixture (combined with the reaction mixture from another reaction performed using 23 g of B10) was concentrated to dryness. H₂O (40 mL) was added to the residue, and the aqueous layer washed with EtOAc (50 mL). The aqueous layer was lyophilized to give B11 (21 g, 2HCl). ¹H NMR (400 MHz, CD₃OD) δ 8.96 (d, 1 H), 7.56 (s, 1 H), 3.43-3.51 (m, 1 H), 3.17-3.32 (m, 2 H), 1.29-1.53 (m, 3 H).

### Intermediate 2-[(2R)-2-(1H-imidazol-5-yl)propyl]isoindoline-1,3-dione and 2-[(2S)-2-(1H-imidazol-5-yl)propyl]isoindoline-1,3-dione

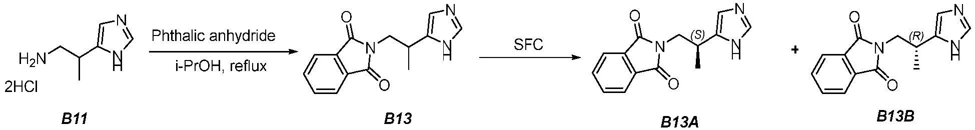

### Step 1: Preparation of 2-[2-(1H-imidazol-5-yl)propyl]isoindoline-1,3-dione (B13)

To a solution of 2-(1H-imidazol-5-yl)propan-1-amine (B11) (10 g, 50.5 mmol, 2HCl) and phthalic anhydride (7.85 g, 53.0 mmol, 1.05 eq) in i-PrOH (400 mL) was added Et₃N (15.32 g, 151 mmol, 21.1 mL, 3 eq). The reaction mixture was stirred at 100°C for 18 hrs under N₂ atmosphere. After cooling, the reaction mixture was concentrated to dryness. Water (200 mL) was then added, and the mixture was stirred at rt for 10 mins. The precipitate was collected by filtration, rinsed with water (100 mL), and dried in vacuo to afford B13 (13 g), which was used without further purification. ¹H NMR (400 MHz, DMSO-*d*₆) δ 7.71-7.89 (m, 4 H), 7.49 (s, 1 H), 6.79 (s, 1 H), 3.70-3.76 (m, 1 H), 3.60-3.65 (m, 1 H), 3.15-3.23 (m, 1 H), 1.16 (d, 3 H).

### Step 2: SFC Separation

Compound 2-[2-(1H-imidazol-5-yl)propyl]isoindoline-1,3-dione (13 g) was separated by SFC separation (column: DAICEL CHIRALPAK AD(250mm*50mm,10um);mobile phase: [0.1%NH₃H₂O ETOH];B%: 40%-40%,min) to afford Enantiomer 1 (4.8 g, 37% yield, Rt = 3.953 min) and Enantiomer 2 (5.2 g, 40% yield, Rt = 4.652 min).

Enantiomer 1: 2-[(2R)-2-(1H-imidazol-5-yl)propyl]isoindoline-1,3-dione (B13A). ¹H NMR (400 MHz, CD₃OD) δ 7.73-7.89 (m, 4 H), 7.56 (d, 1 H), 6.82 (s, 1 H), 3.84-3.93 (m, 1 H), 3.70-3.82 (m, 1 H), 3.36-3.39 (m, 1 H), 1.31 (d, 3 H); SFC: 98.4% ee.

Enantiomer 2: 2-[(2S)-2-(1H-imidazol-5-yl)propyl]isoindoline-1,3-dione (B13B). ¹H NMR (400 MHz, CD₃OD) δ 7.77-7.88 (m, 4 H), 7.55 (d, 1 H), 6.82 (s, 1 H), 3.84-3.97 (m, 1 H), 3.72-3.82 (m, 1 H), 3.36-3.42 (m, 1 H), 1.31 (d, 3 H); SFC: 99.1% ee.

### Preparation of Intermediate 6-(1,3-benzoxazol-2-yl)-N,N-dimethyl-2-triisopropylsilyl-5,6-dihydro-4H-pyrrolo[3,4-d]imidazole-3-sulfonamide

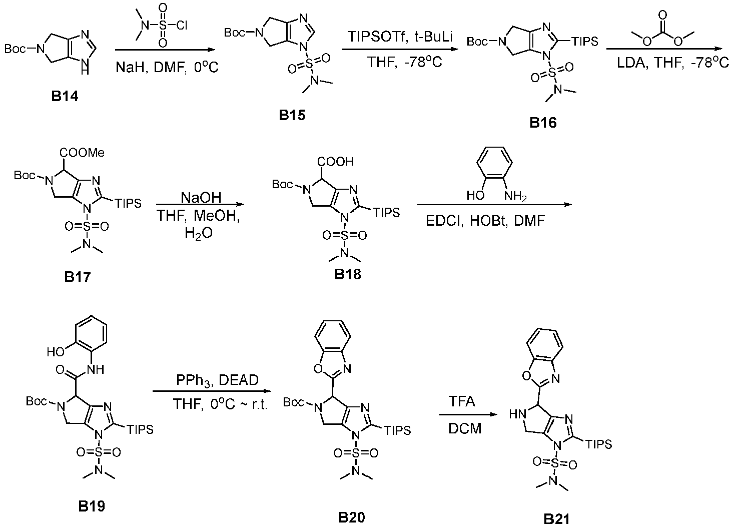

### Step 1: Preparation of tert-butyl 3-(dimethylsulfamoyl)-4,6-dihydropyrrolo[3,4-d]imidazole- 5-carboxylate (B15)

To a solution of tert-butyl 4,6-dihydro-1H-pyrrolo[3,4-d]imidazole-5-carboxylate (B14) (4.5 g, 21.5 mmol) in DMF (60 mL) was added NaH (1.72 g, 43.0 mmol, 60% dispersion in mineral oil, 2 eq) at 0°C. The reaction mixture was stirred at 0°C for 30 min, then N,N-dimethylsulfamoyl chloride (4.63 g, 32.3 mmol, 3.46 mL) was added to the reaction mixture at 0°C, and the reaction mixture was stirred at rt for 1 hr. Reaction progress was tracked using TLC (DCM:EtOAc=1:1). Sat. aq. NH₄Cl (80 mL) was added to the reaction mixture, and the aqueous portion extracted with DCM (100 mL). The organic layer was washed by water (80 mL) and brine (80 mL), dried over Na₂SO₄, filtered, and concentrated to dryness. The residue was purified by column chromatography (SiO₂, DCM/EtOAc=1/0 to 1/1) to give B15 (5.6 g, 82.3% yield). ¹H NMR (400 MHz, CDCl₃) δ 7.70 (s, 1 H), 4.46-4.64 (m, 2 H), 4.36 (dt, 2 H), 2.83 (d, 6 H), 1.44 (s, 9 H).

### Step 2: Preparation of tert-butyl 3-(dimethylsulfamoyl)-2-triisopropylsilyl-4,6-dihydropyrrolo[3,4-d]imidazole-5-carboxylate (B16)

To a solution of tert-butyl 3-(dimethylsulfamoyl)-4,6-dihydropyrrolo[3,4-d]imidazole-5- carboxylate (B15) (9.2 g, 29.1 mmol) in THF (120 mL) was added t-BuLi (1.3 M in pentane, 26.8 mL) at -78°C, and the reaction mixture was stirred at -78°C for 1 hr. Then 2-[diisopropyl(trifluoromethoxysulfonyl)silyl]propane (10.69 g, 34.9 mmol, 40.6 uL) in THF(10 mL) was added to the reaction mixture dropwise at -78°C, and the mixture was stirred at -78°C for 1 hr. Reaction progress was tracked using TLC (PE:EtOAc=2:1). Sat. aq. NH₄Cl solution (300 mL) was added, and the aqueous portion extracted with DCM (200 mL). The organic layer was washed by water (100 mL) and brine (100 mL), dried over Na₂SO₄, filtered, and concentrated to dryness. The residue was purified by column chromatography (SiO₂, PE/EtOAc=1/0 to 5/1) to give B16 (7.4 g, 53.8% yield). ¹H NMR (400 MHz, CDCl₃) δ 4.39-4.66 (m, 4 H), 2.89-3.04 (m, 6 H), 1.66 (quin, 3 H), 1.52 (s, 9 H), 1.17 (d, 18 H)

### Step 3: Preparation of 5-(tert-butyl) 4-methyl 3-(N,N-dimethylsulfamoyl)-2-(triisopropylsilyl)-4,6-dihydropyrrolo[3,4-d]imidazole-4,5(3H)-dicarboxylate (B17)

To a solution of dimethyl carbonate (610 mg, 6.77 mmol, 570 uL) and tert-butyl 3-(dimethylsulfamoyl)-2-triisopropylsilyl-4,6-dihydropyrrolo[3,4-d]imidazole-5-carboxylate (B16) (2 g, 4.23 mmol) in THF (40 mL) was added LDA (2 M, 3.60 mL) at - 78°C, and the reaction mixture was stirred at -78°C for 1 hr. Reaction progress was tracked using LCMS. Sat. aq. NH₄Cl solution (100 mL) was added, and the aqueous portion extracted with EtOAc (200 mL). The organic layer was washed by sat. aq. Na₂CO₃ (150 mL) and brine (150 mL), dried over Na₂SO₄, filtered, and concentrated to dryness. The residue was purified prep. HPLC (column: Xtimate C18 150*40mm*IOum;mobile phase: [water(NH₃H₂O+NH₄HCO₃)-ACN];B%: 85%-100%,7min) to give B17 (1 g, 44.1% yield). ¹H NMR (400 MHz, CDCl₃) δ 5.20-5.36 (m, 1 H), 4.56-4.66 (m, 1 H), 4.41-4.54 (m, 1 H), 3.78 (d, 3 H), 2.91 (d, 6 H), 1.63-1.75 (m, 3 H), 1.41-1.55 (m, 9 H), 1.16 (d, 18 H).

### Step 4: Preparation of 5-tert-butoxycarbonyl-3-(dimethylsulfamoyl)-2-triisopropylsilyl-4,6- dihydropyrrolo[3,4-d]imidazole-6-carboxylic acid (B18)

To a solution of 5-(tert-butyl) 4-methyl 3-(N,N-dimethylsulfamoyl)-2-(triisopropylsilyl)-4,6-dihydropyrrolo[3,4-d]imidazole-4,5(3H)-dicarboxylate (B17) (420 mg, 791 µmol) in THF (4 mL), MeOH (3 mL) and H₂O (2 mL) was added NaOH (47.5 mg, 1.19 mmol), and the reaction mixture was stirred at rt for 24 hrs. The reaction mixture was concentrated in vacuum to remove the organic solvents, and H₂O (10 mL) was added. Reaction progress was tracked using LCMS. The aqueous layer was adjusted to pH ~7 by the addition of 0.5 M HCl. The reaction mixture was concentrated to dryness to give B18 (400 mg, 95.2% yield). ¹H NMR (400 MHz, CDCl₃) δ 5.14-5.58 (m, 1 H), 4.49 (br s, 2 H), 2.83-2.99 (m, 6 H), 1.58-1.71 (m, 3 H), 1.46-1.56 (m, 9 H), 1.16 (d, 18 H).

### Step 5: Preparation of tert-butyl 3-(dimethylsulfamoyl)-6-[(2-hydroxyphenyl)carbamoyl]-2-triisopropylsilyl-4,6-dihydropyrrolo[3,4-d]imidazole-5-carboxylate (B19)

To a solution of 2-aminophenol (50.7 mg, 464 µmol) in DMF (3 mL) was added EDCI (96.5 mg, 503 µmol), HOBt (68.0 mg, 503 µmol) and 5-tert-butoxycarbonyl-3-(dimethylsulfamoyl)-2-triisopropylsilyl-4,6-dihydropyrrolo[3,4-d]imidazole-6-carboxylic acid (B18) (200 mg, 387 µmol), and the reaction mixture was stirred at rt for 1 hr. Reaction progress was tracked using LCMS. EtOAc was added to the reaction mixture (30 mL), and the organic layer was washed by water (10 mL) and brine (10 mL), dried over Na₂SO₄, filtered, and concentrated to dryness. The residue was purified by column chromatography (SiO₂, PE/EtOAc=1/0 to 1/1) to give B19 (110 mg, 46.8% yield). ¹H NMR (400 MHz, CDCl₃) δ 9.15 (s, 1 H), 8.61 (s, 1 H), 6.73-7.14 (m, 4 H), 5.29-5.65 (m, 1 H), 4.41-4.64 (m, 2 H), 2.74-2.90 (m, 6 H), 1.54-1.59 (m, 3 H), 1.46 (s, 9 H), 1.05-1.09 (m, 18 H).

### Step 6: Preparation of tert-butyl 6-(1,3-benzoxazol-2-yl)-3-(dimethylsulfamoyl)-2-triisopropylsilyl-4,6-dihydropyrrolo[3,4-d] imidazole-5-carboxylate (B20)

To a solution of tert-butyl 3-(dimethylsulfamoyl)-6-[(2-hydroxyphenyl)carbamoyl]- 2-triisopropylsilyl-4,6-dihydropyrrolo[3,4-d]imidazole-5-carboxylate (B19) (100 mg, 165 µmol) in THF (1 mL) was added PPh₃ (86.3 mg, 329 µmol), DEAD (57.3 mg, 329 µmol, 59.8 µL) at 0°C, and the reaction mixture was stirred at rt for 12 hrs. Reaction progress was tracked using LCMS. DCM was added (30 mL), and the organic layer washed by water (10 mL) and brine (10 mL), dried over Na₂SO₄, filtered, and concentrated to dryness. The residue was purified by prep. HPLC (column: Phenomenex C18 75*30mm*3um; mobile phase: [water (NH₃H₂O+NH₄HCO₃)-ACN]; B%: 70%-100%,7min) to give B20 (90 mg, 92.8% yield). ¹H NMR (400 MHz, CDCl₃) δ 7.68-7.79 (m, 1 H), 7.48-7.60 (m, 1 H), 7.32-7.43 (m, 2 H), 6.07-6.38 (m, 1 H), 4.52-4.87 (m, 2 H), 2.39-2.74 (m, 6 H), 1.61-1.67 (m, 3 H), 1.25-1.52 (m, 9 H), 1.17 (dd, 18 H).

### Step 7: Preparation of 6-(1,3-benzoxazol-2-yl)-N,N-dimethyl-2-triisopropylsilyl-5,6-dihydro-4H-pyrrolo[3,4-d]imidazole-3-sulfonamide (B21)

To a solution of tert-butyl 6-(1,3-benzoxazol-2-yl)-3-(dimethylsulfamoyl)-2-triisopropylsilyl- 4,6-dihydropyrrolo[3,4-d]imidazole-5-carboxylate (B20) (50 mg, 84.8 µmol) in DCM (2 mL) was added TFA (308 mg, 2.70 mmol, 0.2 mL), and the reaction mixture was stirred at rt for 1 hr. Reaction progress was tracked using LCMS. Water (10 mL)was added, and the aqueous layer was adjusted to pH ~8 by the addition of sat. aq. NaHCO₃ solution. The suspension was extracted with DCM/MeOH (50/5 mL) 3 times. The combined organic layer was dried over Na₂SO₄, filtered, and concentrated to dryness to afford B21 (41 mg, 100% yield). LC-MS: m/z 490.2 [M+H]⁺.

### Intermediate 1,3-benzoxazole-2-carbaldehyde

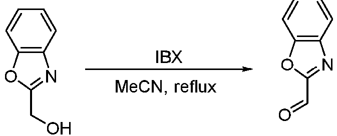

To a solution of 1,3-benzoxazol-2-ylmethanol (5.5 g, 36.9 mmol) in MeCN (170 mL) was added IBX (13.42 g, 47.9 mmol, 1.3 eq) in portions at rt. The mixture was stirred at 80°C for 2 hrs. Reaction progress was tracked using TLC (PE:EtOAc= 1:1). The reaction mixture was filtered, and the filtrate was concentrated to dryness to afford 1,3-benzoxazole-2-carbaldehyde (5.7 g), which was used without further purification. ¹H NMR (400 MHz, CDCl₃) δ 10.03 (s, 1 H), 7.98 (d, 1 H), 7.72 (d, 1 H), 7.62 (td, 1 H), 7.50-7.56 (m, 1 H).

### Intermediate 5-methyl-1,3-benzoxazole-2-carbaldehyde

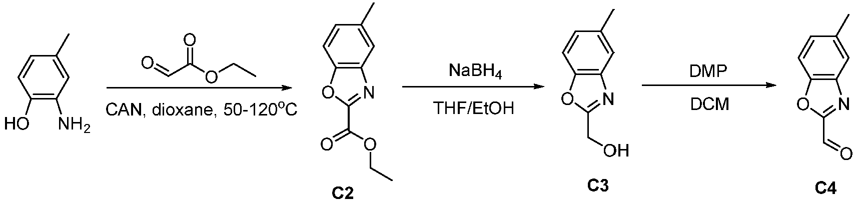

### Step 1: Preparation of ethyl 5-methyl-1,3-benzoxazole-2-carboxylate (C2)

To a solution of 2-amino-4-methyl-phenol (5 g, 40.6 mmol) in dioxane (120 mL) was added ethyl 2-oxoacetate (16.58 g, 81.2 mmol, 2 eq) in two portion per hour. The reaction mixture was heated at 50°C for 2 hrs, CAN (24.48 g, 44.7 mmol, 22.3 mL, 1.1 eq) was then added, and the reaction mixture was stirred at 120°C for 6 hrs. Reaction progress was tracked using TLC (PE:EtOAc=5:1). The reaction mixture was filtered, and the filtrate was concentrated to dryness. The residue was purified by column chromatography on silica gel (PE:EtOAc=20:1 to 5:1) to give C2 (1.6 g, 19% yield). ¹H NMR (400 MHz, DMSO-*d*₆) δ 7.65-7.77 (m, 2 H), 7.39 (dd, 1 H), 4.39 (q, 2 H), 2.43 (s, 3 H), 1.29-1.37 (m, 3 H).

### Step 2: Preparation of (5-methyl-1,3-benzoxazol-2-yl)methanol (C3)

To a solution of ethyl 5-methyl-1,3-benzoxazole-2-carboxylate (C2) (2.5 g, 12.2 mmol) and THF (40 mL) and EtOH (4 mL) was added NaBH₄ (2.30 g, 60.9 mmol, 5 eq) in portions at 0°C. The reaction mixture was stirred at rt for 4 hrs. Reaction progress was checked using TLC (PE:EtOAc=1:1). The reaction mixture was quenched with sat. NH₄Cl solution (20 mL), and the aqueous portion was extracted with EtOAc (100 mL). The organic layer was washed with water (30 mL) and brine (30 mL), dried over Na₂SO₄, filtered, and concentrated to dryness. The residue was purified by column chromatography on silica gel (PE:EtOAc=20:1 to 1:1) to give C3 (600 mg, 24% yield). LCMS: m/z 164.2 [M+H]⁺.

### Step 3: Preparation of 5-methyl-1,3-benzoxazole-2-carbaldehyde (C4)

To a solution of (5-methyl-1,3-benzoxazol-2-yl)methanol (C3) (400 mg, 2.45 mmol) in DCM (15 mL) was added DMP (2.08 g, 4.90 mmol, 1.5 mL, 2 eq). The mixture was stirred at rt for 2 hrs. Reaction progress was tracked using TLC (PE:EtOAc=1:1). DCM (60 mL) was added, and the organic layer washed with sat. NaHCO₃ solution (20 mL) and brine (20 mL), dried over Na₂SO₄, filtered, and concentrated to dryness to afford C4 (300 mg), which was used without further purification.

### Intermediate 5-fluorobenzo[d]oxazole-2-carbaldehyde

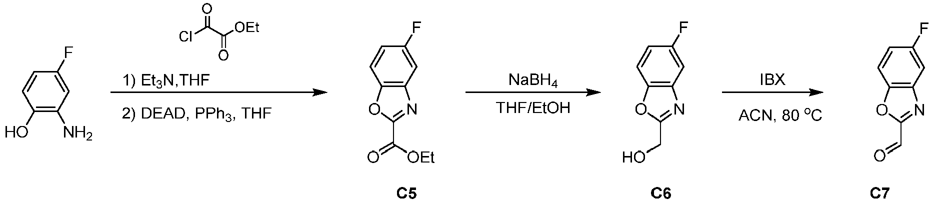

### Step 1: Preparation of ethyl 5-fluorobenzo[d]oxazole-2-carboxylate (C5)

To a solution of 2-amino-4-fluorophenol (120 g, 944 mmol) in THF (360 mL) was added Et₃N (191 g, 1.89 mol, 2 eq) and ethyl 2-chloro-2-oxoacetate (142 g, 1.04 mol, 1.1 eq). The reaction mixture was stirred at rt for 1 hr. PPh₃ (384 g, 1.46 mol, 1.55 eq) in THF (360 mL) and DEAD (247 g, 1.42 mol, 1.5 eq) was added to the reaction mixture dropwise at 0°C. The reaction mixture was stirred at rt for 1 hr. Reaction progress was tracked using TLC (PE/EtOAc=1:1). The reaction mixture was filtered, and MTBE (700 mL) was added to the filtrate. The organic layer was washed with H₂O (700 mL) and brine (400 mL), dried over Na₂SO₄, filtered, and concentrated to dryness. The residue was purified by column chromatography (SiO₂, PE/EtOAc=100/1 to 3/1) to give C5 (145 g, 73% yield). ¹H NMR (400 MHz DMSO-*d*₆) δ 7.91-7.95 (m, 1H), 7.79-7.82 (m, 1H), 7.47-7.79 (m, 1H),4.41-4.46 (m, 2H),1.31-1.38 (m, 3H).

### Step 2: Preparation of 5-fluorobenzo[d]oxazol-2-yl)methanol (C6)

To a solution of ethyl 5-fluorobenzo[d]oxazole-2-carboxylate (C5) (145 g, 693 mmol) in THF (430 mL) and EtOH (430 mL) was added NaBH₄ (78.7 g, 2.08 mol, 3 eq) in portions at 0°C. The mixture was stirred at rt for 2 hrs. Reaction progress was tracked using TLC (PE/EtOAc=1/1). DCM (600 mL) was added, and the organic layer washed with H₂O (500 mL) and brine (300 mL), dried over Na₂SO₄, filtered, and concentrated to dryness. The residue was purified by column chromatography (SiO₂, PE/EtOAc=30/1 to 1/1) to compound C6 (75.5 g, 65% yield). ¹H NMR (400 MHz DMSO-*d*₆) δ 7.75-7.78 (m, 1H), 7.60-7.63 (m, 1H), 7.24-7.30 (m, 1H), 5.89-5.92(t, 1H), 4.69-4.71(d, 2H).

### Step 3: Preparation of 5-fluorobenzo[d]oxazole-2-carbaldehyde (C7)

To a solution of 5-fluorobenzo[d]oxazol-2-yl)methanol (C6) (75.5 g, 452 mmol) in ACN (450 mL) was added IBX (164 g, 587 mmol, 1.3 eq) at rt. The reaction mixture was stirred at 80°C for 2 hrs. Reaction progress was tracked using TLC (PE/EtOAc=1/1). The reaction mixture was filtered, and the filter cake was washed with DCM (200 mL x 2). The filtrate was concentrated to dryness, then triturated with PE (200 mL) at rt for 30 min, and filtered to give a filter cake. The filter cake was dissolved in DCM (200 mL) and the organic layer washed with 1 M K₂CO₃ solution (150 mL), dried over Na₂SO₄, filtered, and concentrated to dryness to give C7 (52.0 g, 70% yield). ¹H NMR (400 MHz DMSO-*d*₆) δ 9.91 (s, 1H),7.92-8.00 (m, 2H), 7.53-7.55 (m, 1H).

### Intermediates 2-amino-3-(trifluoromethoxy)phenol and 4-(trifluoromethoxy)benzo[d]oxazole-2-carbaldehyde

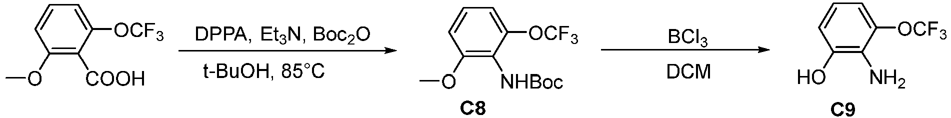

### Step 1: Preparation of tert-butyl N-[2-methoxy-6-(trifluoromethoxy)phenyl]carbamate (C8)

To a solution of 2-methoxy-6-(trifluoromethoxy)benzoic acid (1 g, 4.23 mmol) in t-BuOH (3 mL) was added DPPA (1.52 g, 5.51 mmol, 1.2 mL, 1.3 eq), Boc₂O (2.77 g, 12.7 mmol, 2.9 mL, 3 eq) and TEA (643 mg, 6.35 mmol, 884 µL, 1.5 eq), the mixture was stirred at 85°C for 3 hrs. EtOAc (220 mL) was added, and the organic layer washed with water (200 mL) and brine (150 mL), dried over Na₂SO₄, and concentrated to dryness. The residue was purified by column chromatography (SiO₂, PE/EtOAc=1/0 to 10/1) to give C8 (470 mg, 36% yield). ¹H NMR (400 MHz, CDCl₃) δ 7.20 (t, 1 H), 6.92 (dt, 1 H), 6.85 (dd, 1 H), 5.92 (br s, 1 H), 3.73-3.96 (m, 3 H), 1.50 (s, 9 H).

### Step 2: Preparation of 2-amino-3-(trifluoromethoxy)phenol (C9)

To a solution of tert-butyl N-[2-methoxy-6-(trifluoromethoxy)phenyl]carbamate (C8) (1 g, 3.25 mmol) in DCM (10 mL) was added BCl₃ (1 M, 9.8 mL, 3 eq), the reaction mixture was stirred at rt for 12 hrs. The reaction mixture was adjusted to pH ~9 by the addition of sat. NaHCO₃ solution, and the aqueous portion extracted with DCM (30 mL x 3). The combined organic layer was dried over Na₂SO₄, filtered, and concentrated to dryness. The residue was purified by column chromatography (SiO₂, PE/EtOAc=1/0 to 5/1) to give C9 (400 mg, 64% yield). ¹H NMR (400 MHz, DMSO-*d₆*) δ 9.66 (s, 1 H), 6.56-6.74 (m, 2 H), 6.37-6.51 (m, 1 H), 4.64 (br s, 2 H).

C9 was then used to prepare 4-(trifluoromethoxy)benzo[d]oxazole-2-carbaldehyde following the procedure described above for C7.

### Intermediate 4-fluoro-7-methyl-1,3-benzoxazole-2-carbaldehyde

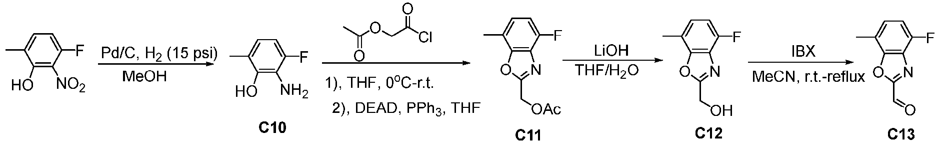

### Step 1: Preparation of 2-amino-3-fluoro-6-methyl-phenol (C10)

To a solution of 3-fluoro-6-methyl-2-nitro-phenol (1 g, 5.84 mmol, 1 eq) in MeOH (100 mL) was added Pd/C (250 mg, 584 µmol, 20% purity, 0.1 eq), the mixture was degassed in vacuum and purged with H₂ for 3 times, and then the reaction mixture was stirred at rt for 2 hrs under H₂ (15 psi). Reaction progress was tracked using TLC (PE:EtOAc=10:1) and LCMS. The reaction mixture was filtered through a pad of Celite, and the filtrate was concentrated to give C10 (880 mg), which was used without further purification. ¹H NMR (400 MHz, CDCl₃) δ 6.45-6.64 (m, 2 H), 3.83 (br s, 2 H), 2.20 (s, 3 H); LCMS: m/z 142.1 [M+H]⁺.

### Step 2: Preparation of (4-fluoro-7-methyl-1,3-benzoxazol-2-yl)methyl acetate (C11)

To a solution of 2-amino-3-fluoro-6-methyl-phenol (C10) (880 mg, 6.23 mmol) in THF (15 mL) was added (2-chloro-2-oxo-ethyl) acetate (970 mg, 7.11 mmol, 764 µL, 1.1 eq) dropwise at 0 °C. The reaction mixture was stirred at rt for 1 hr. Reaction progress was tracked using TLC (PE:EtOAc=5:1). Water (100 mL) was added, and the aqueous portion extracted with EtOAc (100 mL x 3). The combined organic layer was dried over Na₂SO₄, filtered, and concentrated to dryness. The residue (1.7 g) was dissolved in THF (20 mL), PPh₃ (2.77 g, 10.6 mmol, 1.5 eq) was added, and then DEAD (1.84 g, 10.6 mmol, 1.9 mL, 1.5 eq) was added dropwise at 0°C. The reaction mixture was stirred at rt for 14 hrs. Reaction progress was tracked using LCMS. Water (60 mL) was added, and the aqueous portion extracted with EtOAc (60 mL x 3). The combined organic phase was concentrated to dryness. The residue was purified by flash silica gel chromatography (ISCO^{®}; 20 g SepaFlash^{®} Silica Flash Column, Eluent of 0~20% EtOAc/PE gradient @ 35 mL/min) to give C11 (1.1 g, 68% yield). ¹H NMR (400 MHz, CDCl₃) δ 7.11 (dd, 1 H), 6.92-7.04 (m, 1 H), 5.35 (s, 2 H), 2.49 (s, 3 H), 2.22 (s, 3 H); LCMS: m/z 224.1 [M+H]⁺.

### Step 3: Preparation of (4-fluoro-7-methyl-1,3-benzoxazol-2-yl)methanol (C12)

To a solution of (4-fluoro-7-methyl-1,3-benzoxazol-2-yl)methyl acetate (C11) (1.1 g, 4.93 mmol) in THF (10 mL) and H₂O (3 mL) was added LiOH.H₂O (248 mg, 5.91 mmol, 1.2 eq), and the reaction mixture was stirred at rt for 1 hr. Reaction progress was tracked using LCMS. EtOAc (60 mL) was added, and the organic layer washed with water (20 mL) and brine (20 mL), dried over Na₂SO₄, filtered, and concentrated to dryness to afford C12 (1.2 g), which was used without further purification. LCMS: m/z 182.1 [M+H]⁺.

### Step 4: Preparation of 4-fluoro-7-methyl-1,3-benzoxazole-2-carbaldehyde (C13)

To a solution of (4-fluoro-7-methyl-1,3-benzoxazol-2-yl)methanol (C12) (580 mg, 3.20 mmol) in CH₃CN (30 mL) was added IBX (1.17 g, 4.16 mmol, 1.3 eq) at rt. The reaction mixture was stirred at 80°C for 3 hrs. Reaction progress was tracked using LCMS. The reaction mixture was filtered, and filtrate was concentrated to dryness to afford C13 (650 mg, 90% yield), which was used without further purification. ¹HNMR (400 MHz, CDCl₃) δ 9.99 (s, 1 H), 7.31 (dd, 1 H), 7.10 (t, 1 H), 2.55 (s, 3 H); LCMS: m/z 180.1 [M+H]⁺.

### Intermediate 5-(trifluoromethyl)benzo[d]oxazole-2-carbaldehyde

Intermediate 5-(trifluoromethyl)benzo[d]oxazole-2-carbaldehyde was prepared starting from 2-amino-4-(trimethyl)phenol following the procedure for steps 2, 3, and 4 above for C13. In the hydrolysis step 3, K₂CO₃ and MeOH was used as the base and solvent instead of LiOH and THF/H₂O.

### Intermediate 5-fluoro-7-methoxy-1,3-benzoxazole-2-carbaldehyde

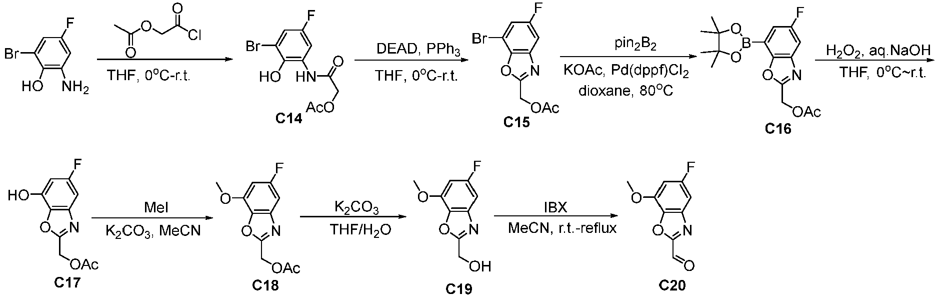

### Step 1: Preparation of [2-(3-bromo-5-fluoro-2-hydroxy-anilino)-2-oxoethyl] acetate (C14)

To a solution of 2-amino-6-bromo-4-fluoro-phenol (3.56 g, 17.3 mmol) in THF (70 mL) was added (2-chloro-2-oxo-ethyl) acetate (2.71 g, 19.9 mmol, 2.1 mL, 1.15 eq) dropwise at 0°C. The reaction mixture was stirred at rt for 1 hr. Reaction progress was tracked using LCMS. H₂O (200 mL) was added, and the aqueous portion extracted with EtOAc (100 mL x 3). The organic layer was dried over Na₂SO₄, filtered, and concentrated to dryness to afford C14 (5.29 g), which was used without further purification. LCMS: m/z 306.0 [M+H]⁺.

### Step 2: Preparation of (7-bromo-5-fluoro-1,3-benzoxazol-2-yl) methyl acetate (C15)

To a solution of [2-(3-bromo-5-fluoro-2-hydroxy-anilino)-2-oxo-ethyl] acetate (C14) (5.29 g, 17.3 mmol) and PPh₃ (6.80 g, 25.9 mmol, 1.5 eq) in THF (70 mL) was added DEAD (4.51 g, 25.9 mmol, 4.7 mL, 1.5 eq) dropwise at 0°C. The reaction mixture was stirred at rt for 2 hrs. Reaction progress was tracked using TLC (PE:EtOAc=2: 1). Water (200 mL) was added, and the aqueous portion extracted with EtOAc (100 mL x 3). The combined organic phase was concentrated to dryness. The residue was purified by flash silica gel chromatography (ISCO^{®}; 80 g SepaFlash^{®} Silica Flash Column, Eluent of 0~100% EtOAc/PE gradient @ 35 mL/min) to give C15 (2.42 g, 49% yield). ¹H NMR (400 MHz, CDCl₃) δ 7.30-7.42 (m, 2 H), 5.35 (s, 2 H), 2.23 (s, 3 H).

### Step 3: Preparation of [5-fluoro-7-(4,4,5,5-tetramethyl-1,3,2-dioxaborolan-2-yl)-1,3-benzoxazol-2-yl] methyl acetate (C16)

To a solution of (7-bromo-5-fluoro-1,3-benzoxazol-2-yl)methyl acetate (C15) (2.42 g, 8.40 mmol) in dioxane (100 mL) was added Pin₂B₂ (2.56 g, 10.1 mmol, 1.2 eq) KOAc (1.65 g, 16.8 mmol, 2 eq) and Pd(dppf)Cl₂ (615 mg, 840 µmol, 0.1 eq). The mixture was stirred at 80°C for 16 hrs under N₂. Reaction progress was tracked using LCMS. Water (200 mL) was added, and the aqueous portion extracted with EtOAc (100 mL x 3). The combined organic phase was concentrated to dryness. The residue was purified by flash silica gel chromatography (ISCO^{®}; 40 g SepaFlash^{®} Silica Flash Column, Eluent of 0~100% EtOAc/PE gradient @ 35 mL/min) to give C16 (2.46 g, 87% yield). ¹H NMR (400 MHz, CDCl₃) δ 7.46-7.53 (m, 2 H), 5.38 (s, 2 H), 2.23 (s, 3 H), 1.40 (s, 12 H); LCMS: m/z 336.2 [M+H]⁺.

### Step 4: Preparation of (5-fluoro-7-hydroxy-1,3-benzoxazol-2-yl)methyl acetate (C17)

To a solution of [5-fluoro-7-(4,4,5,5-tetramethyl-1,3,2-dioxaborolan-2-yl)-1,3-benzoxazol-2-yl]methyl acetate (C16) (2.46 g, 7.34 mmol) in THF (40 mL) was added H₂O₂ (1.25 g, 11.0 mmol, 1.1 mL, 30% purity, 1.5 eq) and NaOH (1 M, 7.7 mL, 1.05 eq) at 0°C. The mixture was stirred at rt for 3 hrs. Reaction progress was tracked using LCMS. Water (100 mL) was added, and the aqueous portion extracted with EtOAc (50 mL x 3). The combined organic phase was concentrated to dryness. The residue was purified by flash silica gel chromatography (ISCO^{®}; 20 g SepaFlash^{®} Silica Flash Column, Eluent of 0~100% EtOAc/PE gradient @ 35 mL/min) to give C17 (1.65 g, 100% yield). ¹H NMR (400 MHz, CDCl₃) δ 7.38-8.11 (m, 1 H), 6.99 (dd, 1 H), 6.69 (dd, 1 H), 5.32 (s, 2 H), 2.19 (s, 3 H); LCMS: m/z 226.1 [M+H]⁺.

### Step 5: Preparation of (5-fluoro-7-methoxy-1,3-benzoxazol-2-yl) methyl acetate (C18)

To a solution of (5-fluoro-7-hydroxy-1,3-benzoxazol-2-yl)methyl acetate (C17) (1.65 g, 7.34 mmol) in MeCN (30 mL) was added K₂CO₃ (2.13 g, 15.4 mmol, 2.1 eq) and MeI (1.15 g, 8.07 mmol, 503 µL, 1.1 eq) at 0°C. The reaction mixture was stirred at rt for 16 hrs. Reaction progress was tracked using LCMS. Water (50 mL) was added, and the aqueous portion extracted with EtOAc (30 mL x 3). The combined organic phase was concentrated to dryness. The residue was purified by flash silica gel chromatography (ISCO^{®}; 20 g SepaFlash^{®} Silica Flash Column, Eluent of 0~100% EtOAc/PE gradient @ 35 mL/min) to give C18 (1.14 g, 65% yield). ¹H NMR (400 MHz, CDCl₃) δ 7.03 (dd, 1 H), 6.66 (dd, 1 H), 5.31 (s, 2 H), 4.01 (s, 3 H), 2.20 (s, 3 H); LCMS: m/z 240.1 [M+H]⁺.

### Step 6: Preparation of (5-fluoro-7-methoxy-1,3-benzoxazol-2-yl)methanol (C19)

To a solution of (5-fluoro-7-methoxy-1,3-benzoxazol-2-yl)methyl acetate (C18) (1.14 g, 4.77 mmol) in MeOH (20 mL) was added K₂CO₃ (725 mg, 5.24 mmol, 1.1 eq) at 0°C. The mixture was stirred at rt for 2 hrs. Reaction progress was tracked using LCMS. The reaction mixture was concentrated to dryness. Water (15 mL) was added, and the aqueous portion extracted with EtOAc (10 mL x 3). The combined organic phase was dried over Na₂SO₄, filtered, and concentrated to dryness to give C19 (910 mg), which was used without further purification. ¹H NMR (400 MHz, CDCl₃) δ 7.02 (dd, 1 H), 6.65 (dd, 1 H), 4.92 (s, 2 H), 4.01 (s, 3 H); LCMS: m/z 198.1 [M+H]⁺.

### Step 7: Preparation of 5-fluoro-7-methoxy-1,3-benzoxazole-2-carbaldehyde (C20)

To a solution of (5-fluoro-7-methoxy-1,3-benzoxazol-2-yl)methanol (C19) (910 mg, 4.62 mmol) in MeCN (20 mL) was added IBX (1.68 g, 6.00 mmol, 1.3 eq), and then the mixture was stirred at 80°C for 2 hr. Reaction progress was tracked using LCMS. The reaction mixture was filtered, and the filtrate was concentrated to dryness to afford C20 (960 mg), which was used without further purification. ¹H NMR (400 MHz, CDCl₃) δ 9.98 (s, 1 H), 7.20 (dd, 1 H), 6.83 (dd, 1 H), 4.06 (s, 3 H); LCMS: m/z 196.1 [M+H]⁺.

### Intermediate 7-fluoro-5-methoxy-1,3-benzoxazole-2-carbaldehyde

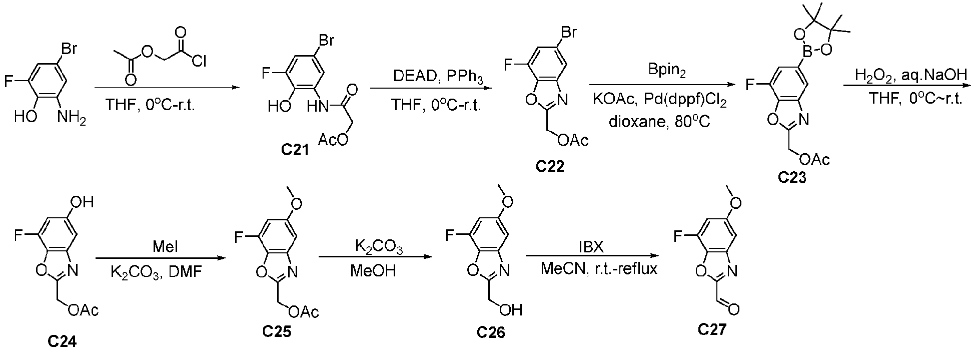

### Step 1: Preparation of [2-(5-bromo-3-fluoro-2-hydroxy-anilino)-2-oxoethyl] acetate (C21)

To a solution of 2-amino-4-bromo-6-fluoro-phenol (6.4 g, 31.1 mmol) in THF (80 mL) was added (2-chloro-2-oxo-ethyl) acetate (4.84 g, 35.4 mmol, 3.8 mL, 1.14 eq) dropwise at 0 °C. The reaction mixture was stirred at rt for 1 hr. Reaction progress was checked using TLC (PE:EtOAc=3:1). The reaction mixture was concentrated to dryness. Water (100 mL) was added, and the aqueous portion extracted with EtOAc (150 mL x 2). The combined organic layers were washed with brine (100 mL), dried over Na₂SO₄, filtered, and concentrated to dryness to afford C21 (9.4 g), which was used without purification.

### Step 2: Preparation of (5-bromo-7-fluoro-1,3-benzoxazol-2-yl)methyl acetate (C22)

To a solution of [2-(5-bromo-3-fluoro-2-hydroxy-anilino)-2-oxo-ethyl] acetate (C21) (9.4 g, 30.7 mmol) and PPh₃ (12.08 g, 46.1 mmol, 1.5 eq) in THF (100 mL) was added DEAD (8.02 g, 46.1 mmol, 8.4 mL, 1.5 eq) dropwise at 0 °C. The reaction mixture was stirred at rt for 12 hrs. Reaction progress was checked using TLC (PE:EtOAc= 2:1). The reaction mixture was concentrated to dryness. Water (300 mL) was added, and the aqueous portion extracted with EtOAc (300 mL x 2). The combined organic layers were washed with brine (300 mL), dried over Na₂SO₄, filtered, and concentrated to dryness. The residue was purified by silica gel chromatography (eluent PE:EtOAc 20:1 to 10: 1) to afford C22 (7.5 g, 85% yield). ¹H NMR (400 MHz, DMSO-*d₆*) δ 7.94 (d, 1 H), 7.75 (dd, 1 H), 5.41 (s, 2 H), 2.16 (s, 3 H).

### Step 3: Preparation of [7-fluoro-5-(4,4,5,5-tetramethyl-1,3,2-dioxaborolan-2-yl)-1,3-benzoxazol-2-yl]methyl acetate (C23)

A mixture of (5-bromo-7-fluoro-1,3-benzoxazol-2-yl)methyl acetate (1 g, 3.47 mmol) (C22), Pin₂B₂ (970 mg, 3.82 mmol, 1.1 eq), Pd(dppf)Cl₂ (127 mg, 174 µmol, 0.05 eq) and KOAc (681 mg, 6.94 mmol, 2 eq) in dioxane (10 mL) was degassed and purged with N₂ for 3 times. The reaction mixture was stirred at 80°C for 24 hrs under N₂ atmosphere. Reaction progress was checked using TLC (PE:EtOAc=3:1). The reaction mixture was filtered and concentrated to dryness. The residue was purified by silica gel chromatography (eluent PE:EtOAc from 50:1 to 10:1) to afford C23 (1 g, 86% yield). ¹H NMR (400 MHz, DMSO-*d*₆) δ 7.82 (s, 1 H), 7.50 (d, 1 H), 5.41 (s, 2 H), 2.16 (s, 3 H), 1.32 (s, 12 H).

### Step 4: Preparation of (7-fluoro-5-hydroxy-1,3-benzoxazol-2-yl)methyl acetate (C24)

To a solution of [7-fluoro-5-(4,4,5,5-tetramethyl-1,3,2-dioxaborolan-2-yl)-1,3-benzoxazol-2-yl]methyl acetate (C23) (1 g, 2.98 mmol) in THF (20 mL) was added H₂O₂ (507 mg, 4.48 mmol, 430 µL, 30% purity, 1.5 eq) and NaOH (1 M, 3.1 mL, 1.05 eq). The reaction mixture was stirred at rt for 3 hrs. Reaction progress was checked using TLC (PE:EtOAc=3:1). EtOAc (60 mL) was added, and the organic portion washed with sat. Na₂S₂O₃ solution (20 mL) and brine (20 mL), dried over Na₂SO₄, filtered, and concentrated to dryness. The residue was purified by flash silica gel chromatography (ISCO^{®}; 24 g SepaFlash^{®} Silica Flash Column, Eluent of 0~20% EtOAc/PE gradient @ 35 mL/min) to give C24 (0.78 g). ¹H NMR (400 MHz, DMSO-*d*₆) δ 9.98 (s, 1 H), 6.90 (br s, 1 H), 6.77 (br d, 1 H), 5.33 (s, 2 H), 2.14 (s, 3 H); LCMS: m/z 225.9 [M+H]⁺.

### Step 5: Preparation of (7-fluoro-5-methoxy-1,3-benzoxazol-2-yl)methyl acetate (C25)

To a solution of (7-fluoro-5-hydroxy-1,3-benzoxazol-2-yl)methyl acetate (C24) (2.3 g, 10.2 mmol) and K₂CO₃ (2.96 g, 21.5 mmol, 2.1 eq) in DMF (2 mL) was added CH₃I (1.59 g, 11.2 mmol, 699 µL, 1.1 eq). The mixture was stirred at rt for 6 hrs. Reaction progress was tracked using TLC (PE:EtOAc=2:1). The reaction mixture was concentrated to dryness. Water (10 mL) was added, and the aqueous portion extracted with EtOAc (30 mL). The organic layer was washed with brine (10 mL), dried over Na₂SO₄, filtered, and concentrated to dryness. The residue was purified by flash silica gel chromatography (ISCO^{®}; 24 g SepaFlash^{®} Silica Flash Column, Eluent of 0~20% EtOAc/PE gradient @ 35 mL/min) to give C25 (1.3 g, 53% yield). ¹H NMR (400 MHz, DMSO-*d*₆) δ 7.20 (d, 1 H), 7.04 (dd, 1 H), 5.36 (s, 2 H), 3.82 (s, 3 H), 2.15 (s, 3 H).

### Step 6: Preparation of (7-fluoro-5-methoxy-1,3-benzoxazol-2-yl)methanol (C26)

To a solution of (7-fluoro-5-methoxy-1,3-benzoxazol-2-yl)methyl acetate (C25) (1.4 g, 5.85 mmol) in MeOH (15 mL) was added K₂CO₃ (971 mg, 7.02 mmol, 1.2 eq). The mixture was stirred at rt for 3 hrs. Reaction progress was tracked using TLC (PE:EtOAc=2:1). The reaction mixture was concentrated to dryness. Water (20 mL) was added, and the aqueous portion extracted with EtOAc (30 mL x 2). The combined organic layers were washed with brine (30 mL), dried over Na₂SO₄, filtered, and concentrated to dryness. The residue was purified by flash silica gel chromatography (ISCO^{®}; 24 g SepaFlash^{®} Silica Flash Column, Eluent of 0~20% EtOAc/PE gradient @ 35 mL/min) to give C26 (1.1 g, 95% yield). ¹H NMR (400 MHz, DMSO-*d*₆) δ 7.15 (d, 1 H), 6.99 (dd, 1 H), 5.94 (t, 1 H), 4.70 (d, 2 H), 3.81 (s, 3 H).

### Step 7: Preparation of 7-fluoro-5-methoxy-1,3-benzoxazole-2-carbaldehyde (C27)

To a solution of (7-fluoro-5-methoxy-1,3-benzoxazol-2-yl)methanol (C27) (1.1 g, 5.58 mmol) in MeCN (20 mL) was added IBX (2.07 g, 7.25 mmol, 98% purity, 1.3 eq) at rt. The mixture was stirred at 80°C for 3 hrs. Reaction progress was tracked using TLC (PE:EtOAc=3:1). The reaction mixture was filtered, and the filtrate was concentrated to dryness to afford C27 (1 g), which was used without further purification. ¹H NMR (400 MHz, DMSO-*d*₆) δ 9.89 (s, 1 H), 7.41 (d, 1 H), 7.28 (dd, 1 H), 3.87 (s, 3 H).

### Intermediate 4-(difluoromethyl)oxazole-5-carboxylic acid according to General Scheme 4

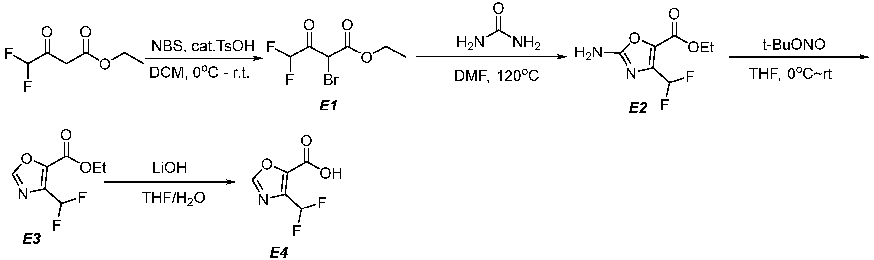

### Step 1: Preparation of ethyl 2-bromo-4,4-difluoro-3-oxo-butanoate (E1)

To a solution of ethyl 4,4-difluoro-3-oxo-butanoate (20 g, 120 mmol) in DCM (240 mL) was added TsOH (4.15 g, 24.1 mmol, 0.2 eq) and NBS (22.50 g, 126 mmol, 1.05 eq) in portions at 0°C. After addition was complete, the reaction mixture was stirred at rt for 1 hr. The reaction progress was checked using TLC (PE:EtOAc=5:1). DCM was added (100 mL), and the organic portion was washed with sat. Na₂CO₃ solution (100 mL) and brine (150 mL), dried over Na₂SO₄, filtered, and concentrated to dryness to afford E1 (34 g), which was used without further purification. ¹H NMR (400 MHz, DMSO-*d*₆) δ 5.81-6.16 (m, 1 H), 4.34-4.41 (m, 1 H), 4.12-4.20 (m, 2 H), 1.17-1.23 (m, 3 H).

### Step 2: Preparation of ethyl 2-amino-4-(difluoromethyl) oxazole-5-carboxylate (E2)

A mixture of E1 (37 g, 151 mmol) and urea (45.34 g, 755 mmol, 40.5 mL, 5 eq) in DMF (30 mL) was stirred at 120°C for 12 hrs. The reaction progress was checked using LC-MS. The reaction mixture was cooled to rt and poured into 100 mL of water. The reaction mixture was stirred for 15 min at 0°C, and the precipitate was collected by filtration, rinsed with water (50 mL), dried in vacuo to give E2 (15.5 g, 63% yield for steps 1 and 2). LC-MS: m/z 207.2 (M+H)⁺; ¹H NMR (400 MHz, DMSO-*d*₆) δ 7.86 (s, 2 H), 6.84-7.44 (m, 1 H), 4.27 (q, 2 H), 1.28 (t, 3 H).

### Step 3: Preparation of ethyl 4-(difluoromethyl)oxazole-5-carboxylate (E3)

To a solution of ethyl 2-amino-4-(difluoromethyl)oxazole-5-carboxylate (20 g, 97.0 mmol) in THF (300 mL) was added t-BuONO (30.01 g, 291 mmol, 34.6 mL, 3 eq) dropwise at 0°C, the reaction mixture was stirred at rt for 12 hrs. DCM (300 mL) was added, and the organic portion was washed with water (200 mL) and brine (200 mL), dried over Na₂SO₄, filtered, and concentrated to dryness. The residue was purified by column chromatography (SiO₂, PE/EtOAc=1/0 to 10/1) to give E3 (12.7 g, 69% yield). ¹H NMR (400 MHz, CDCl₃) δ 8.05 (s, 1 H), 6.93-7.27 (m, 1 H), 4.44 (q, 2 H), 1.40 (t, 3 H).

### Step 5: Preparation of 4-(difluoromethyl)oxazole-5-carboxylic acid (E4)

To a solution of ethyl 4-(difluoromethyl)oxazole-5-carboxylate (12.7 g, 66.5 mmol) in THF (100 mL) and H₂O (20 mL) was added LiOH.H₂O (3.07 g, 73.1 mmol, 1.1 eq). The reaction mixture was stirred at rt for 1 hr and then concentrated in vacuo to remove THF. Water (80 mL) was added, and the aqueous portion extracted with TBME (50 mL). The aqueous layer was then adjusted to pH~6 by the addition of 0.5 *M* HCl. The aqueous layer was concentrated to dryness to give E4 (11 g), which was used without further purification. ¹H NMR (400 MHz, DMSO-*d*₆) δ 8.30 (s, 1 H), 7.34-7.76 (m, 1 H).

### Intermediate ethyl 2-bromo-4-(difluoromethyl)oxazole-5-carboxylate according to General Scheme 4

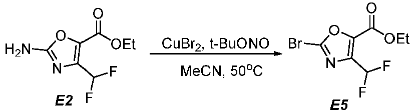

### Preparation of ethyl 2-bromo-4-(difluoromethyl)oxazole-5-carboxylate (E5)

To a solution of ethyl 2-amino-4-(difluoromethyl)oxazole-5-carboxylate (E2) (25 g, 121 mmol) in MeCN (200 mL) was added CuBr₂ (40.63 g, 182 mmol, 8.5 mL, 1.5 eq) at 0°C. The mixture turned dark green and further stirred for 15 min at rt. t-BuONO (18.76 g, 182 mmol, 21.6 mL, 1.5 eq) was added at 0°C. The reaction was stirred at rt for 2 hrs, and then was heated to 50°C and stirred for 12 hrs. Reaction progress was tracked using TLC (EtOAc:PE=5:1). The reaction mixture was filtered. DCM (200 mL) was added to the filtrate, and the organic layer was washed by water (100 mL) and brine (100 mL), dried over Na₂SO₄, filtered, and concentrated to dryness. The residue was purified by flash silica gel chromatography (ISCO^{®}; 120 g SepaFlash^{®} Silica Flash Column, Eluent of 0~20% EtOAc/PE gradient @ 100 mL/min) to afford E5 (19 g, 58% yield). ¹H NMR (400 MHz, CDCl₃) δ 7.02 (t, 1 H), 4.39 (q, 2 H), 1.35 (t, 3 H).

### Intermediate 4-(difluoromethyl)-2-(1-hydroxy-1-methyl-ethyl)oxazole-5-carboxylic acid according to General Scheme 6, Method A

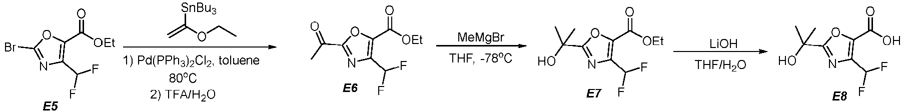

### Step 1: Preparation of ethyl 2-acetyl-4-(difluoromethyl)oxazole-5-carboxylate (E6)

To a solution of ethyl 2-bromo-4-(difluoromethyl)oxazole-5-carboxylate (E5) (10 g, 37.0 mmol) in toluene (150 mL) was added Pd(PPh₃)₂Cl₂ (2.60 g, 3.70 mmol, 0.1 eq) and tributyl(1-ethoxyvinyl)stannane (17.39 g, 48.1 mmol, 16.3 mL, 1.3 eq). The mixture was stirred at 85°C for 12 hrs under N₂ atmosphere. The reaction progress was checked using TLC (PE:EtOAc). EtOAc (100 mL) was added to the reaction mixture, and the organic portion washed with sat. KF solution (150 mL) and brine (100 mL), dried over Na₂SO₄, filtered, and concentrated to dryness. The residue was dissolved in THF (60 mL) and treated with HCl (4 M, 60 mL), and then was stirred at 40°C for 12 hrs. The reaction progress was checked by TLC (Petroleum ether:EtOAc=10:1). EtOAc (100 mL) was added to the reaction mixture, and the organic portion washed with water (50 mL) and brine (50 mL), dried over Na₂SO₄, filtered, and concentrated to dryness. The residue was purified by flash silica gel chromatography (ISCO^{®}; 40 g SepaFlash^{®} Silica Flash Column, eluent of 0~20% EtOAc/PE gradient @ 40 mL/min) to give E6 (7.1 g, 82% yield). ¹H NMR (400 MHz, CDCl₃) δ 7.00-7.36 (m, 1 H), 4.48 (q, 2 H), 2.66-2.86 (m, 3 H), 1.43 (t, 3 H).

### Step 2: Preparation of ethyl 4-(difluoromethyl)-2-(1-hydroxy-1-methylethyl)oxazole-5-carboxylate (E7)

To a solution of E6 (2.5 g, 10.7 mmol) in THF (120 mL) was added MeMgBr (3 M, 7.15 mL, 2 eq) at -78°C. The mixture was stirred at -78°C for 1 hr. The reaction progress was checked by TLC (PE:EtOAc=2:1). The reaction mixture was quenched by addition of sat. NH₄Cl solution (50 mL) and then water (100 mL) was added. The aqueous portion was extracted with EtOAc (100 mL x 3). The combined organic layers were dried over Na₂SO₄, filtered, and concentrated to dryness. The residue was purified by flash silica gel chromatography (ISCO^{®}; 40 g SepaFlash^{®} Silica Flash Column, eluent of 0-12% EtOAc/PE gradient @ 40 mL/min) to give E7 (3.2 g, 60% yield). ¹H NMR (400 MHz, CDCl₃) δ 6.94-7.29 (m, 1 H), 4.46 (q, 2 H), 2.86 (s, 1 H), 1.72 (s, 6 H), 1.44 (t, 3 H). The starting material E6 (300 mg) was also recovered.

### Step 3: Preparation of 4-(difluoromethyl)-2-(1-hydroxy-1-methylethyl)oxazole-5-carboxylic acid (E8)

To a solution of E7 (3.9 g, 15.7 mmol) in THF (40 mL) and H₂O (40 mL) was added LiOH.H₂O (690 mg, 16.4 mmol, 1.05 eq). The mixture was stirred at rt for 1 hr. The reaction progress was checked using TLC (PE:EtOAc=5:1). The reaction mixture was concentrated to remove THF. The aqueous mixture was then adjusted to pH~3 by the addition of 1M HCl, and then lyophilized in vacuo to give E8 (4 g), which was used without further purification. ¹H NMR (400 MHz, DMSO-*d*₆) δ 7.35-7.71 (m, 1 H), 5.68 (br s, 1 H), 1.50 (s, 6 H).

### Intermediate 2-(1-hydroxycyclobutyl)-4-(trifluoromethyl)oxazole-5-carboxylic acid

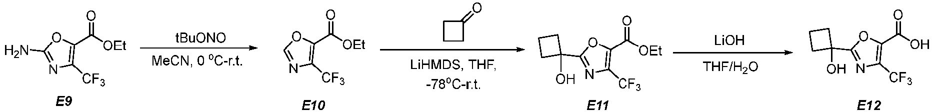

### Step 1: Preparation of ethyl 4-(trifluoromethyl)oxazole-5-carboxylate (E10)

To a solution of ethyl 2-amino-4-(trifluoromethyl)oxazole-5-carboxylate (2.00 g, 8.92 mmol) in THF (60 mL) was added t-BuONO (1.84 g, 17.9 mmol, 2.12 mL, 2 eq). The reaction mixture was stirred at 55°C for 24 hrs. EtOAc (120 mL) was added to the reaction mixture, and the organic portion washed with water (100 mL) and brine (100 mL), dried over Na₂SO₄, filtered, and concentrated to dryness. The residue was purified by column chromatography (SiO₂, PE/EtOAc=1/0 to 8/1) to give E10 (1.2 g, 64% yield). ¹H NMR (400 MHz, CDCl₃) δ 7.96 (s, 1 H), 4.39 (q, 2 H), 1.34 (t, 3 H).

### Step 2: Preparation of ethyl 2-(1-hydroxycyclobutyl)-4-(trifluoromethyl)oxazole-5-carboxylate (E11)

To a solution of ethyl 4-(trifluoromethyl)oxazole-5-carboxylate (1.2 g, 5.74 mmol) in THF (30 mL) was added LiHMDS (1 M, 8.61 mL, 1.5 eq) at -78°C, and the reaction mixture was stirred at -78°C for 0.5 hr. Cyclobutanone (1.21 g, 17.2 mmol, 1.29 mL, 3 eq) in THF (5 mL) was added to the reaction mixture, and the mixture was stirred at - 78°C for 2 hrs under N₂ atmosphere. The reaction mixture was quenched by the addition of sat. NH₄Cl solution (30 mL), and the aqueous portion extracted with EtOAc (100 mL). The organic layer washed by water (70 mL) and brine (70 mL), dried over Na₂SO₄, filtered, and concentrated to dryness. The residue was purified by column chromatography (SiO₂, PE/EtOAc=1/0 to 10/1) to give E11 (600 mg, 37% yield). ¹H NMR (400 MHz, CDCl₃) δ 4.29 (q, 2 H), 2.78-2.94 (m, 1 H), 2.52-2.65 (m, 2 H), 2.24-2.38 (m, 2 H), 1.78-1.98 (m, 3 H), 1.26 (t, 3 H).

### Step 3: Preparation of 2-(1-hydroxycyclobutyl)-4-(trifluoromethyl)oxazole-5-carboxylic acid (E12)

To a solution of ethyl 2-(1-hydroxycyclobutyl)-4-(trifluoromethyl)oxazole-5-carboxylate (600 mg, 2.15 mmol) in H₂O (2 mL) and THF (10 mL) was added LiOH.H₂O (99.2 mg, 2.36 mmol, 1.1 eq) at 0°C. The reaction mixture was stirred at rt for 2 hrs and concentrated in vacuo to remove THF. Water (10 mL) was added, and the aqueous portion extracted with TBME (30 mL). The aqueous layer was then adjusted to pH~6 by the addition of 0.5 *M* HCl and concentrated to dryness to give E12 (540 mg), which was used without further purification. ¹H NMR (400 MHz, CD₃OD) δ 2.54-2.67 (m, 2 H), 2.19-2.34 (m, 2 H), 1.81-1.95 (m, 1 H), 1.66-1.80 (m, 1 H).

### Intermediate ethyl 4-bromo-2-(2-hydroxypropan-2-yl)oxazole-5-carboxylate according to General Scheme 6

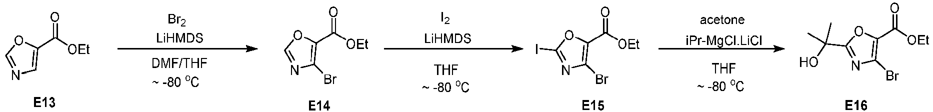

### Step 1: Preparation of Ethyl 4-bromooxazole-5-carboxylate (E14)

Ethyl oxazole-5-carboxylate (E13) (500 mg, 3.54 mmol) was added in THF (2.50 mL) and DMF (2.50 mL) at approximately 10°C. The reaction mixture was cooled to - 80°C and LiHMDS (1 M, 4.61 mL, 1.30 eq) was added dropwise at approximately -80°C. The reaction mixture was stirred at approximately -80°C for 0.5 hr. Br₂ (736 mg, 4.61 mmol, 1.3 eq) was then added dropwise at approximately -80°C. The reaction mixture was stirred at approximately -80°C for 0.5 hr. The reaction progress was checked using LC-MS. The reaction mixture was combined with 19 other reactions performed using 500 mg of E13. The combined reaction mixture was poured into sat. citric acid at approximately -10°C. The aqueous portion was extracted with EtOAc (50 mL x 3). The combined organic layer was washed with brine (50 mL), dried with Na₂SO₄, filtered, and concentrated to dryness. The residue was purified by column chromatography (SiO₂, PE/EtOAc=1/0 to 0/1) to give E14 (3.00 g, 19% yield). ¹H NMR (400 MHz, DMSO-*d₆*) δ 8.70 (s, 1 H), 4.29-4.34 (m, 2 H), 1.29 (t, 3 H); LC-MS: m/z 219.9 & 221.9 (M+H)⁺.

### Step 2: Preparation of Ethyl 4-bromo-2-iodooxazole-5-carboxylate (E15)

Ethyl 4-bromooxazole-5-carboxylate (E14) (3.15 g, 14.3 mmol, 1.00 eq) was added in THF (15.0 mL) at rt. LiHMDS (1 M, 17.2 mL, 1.20 eq) was added dropwise at approximately -80°C. I₂ (5.45 g, 21.5 mmol, 1.50 eq) in THF (15.0 mL) was then added dropwise at approximately -80°C. The reaction mixture was stirred at approximately -80°C for 1 hr. The reaction progress was checked using TLC (PE/EtOAc). The reaction mixture was poured into saturated citric acid (30 mL) at approximately -10°C. The aqueous phase was extracted with ethyl acetate (30 mL x 3). The combined organic layer was washed with brine (30 mL), dried with Na₂SO₄, filtered, and concentrated to dryness. The residue was purified by column chromatography (SiO₂, PE/EtOAc=1/0 to 0/1) to afford E15 (1.10 g, 22% yield). ¹H NMR (400 MHz, DMSO-*d₆*) δ: 4.29-4.35 (m, 2H), 1.30 (t, 3H). LC-MS: m/z 345.8 & 347.9 (M+H)⁺.

### Step 3: Preparation of Ethyl 4-bromo-2-(2-hydroxypropan-2-yl)oxazole-5-carboxylate (E16)

Ethyl 4-bromo-2-iodooxazole-5-carboxylate (E15) (1.10 g, 3.18 mmol) was added in THF (10 mL) at approximately 10°C under N₂. The reaction mixture was degassed under vacuum and purged with N₂ three times. The mixture was cooled to -80°C, and iPr-MgCl.LiCl (1.3 M, 2.45 mL, 1 eq) was added dropwise at approximately -80°C. The reaction mixture was stirred at approximately -80°C for 0.5 hr, and then acetone (222 mg, 3.82 mmol, 1.20 eq) was added dropwise at approximately -80°C. The mixture was stirred at approximately -80°C for 0.5 hr. The reaction progress was checked using TLC (PE/EtOAc=5/1). The reaction mixture was poured into sat. citric acid solution (5 mL) at approximately -10°C. The aqueous portion was extracted with ethyl acetate (5 mL x 3). The combined organic layer was washed with brine (5 mL), dried with Na₂SO₄, filtered, and concentrated to dryness. The residue was purified by column chromatography (SiO₂, PE/EtOAc=1/0 to 0/1) and further purified by prep-HPLC (column: Phenomenex luna C18 80 x 40 mm x 3 um; mobile phase: [water (0.04% HCl)-ACN]; B%: 18%-45%, 7 min) to afford E16 (140 mg, 16% yield). ¹H NMR (400 MHz, DMSO-*d₆*) δ: 5.93 (s, 1H), 4.30 - 4.37 (m, 2H), 1.50 (s, 6H), 1.32 (t, 3H). LC-MS: m/z 277.9 & 279.9 (M+H)⁺.

### Intermediate ethyl 4-chloro-2-(2-hydroxypropan-2-yl)oxazole-5-carboxylate according to General Scheme 6

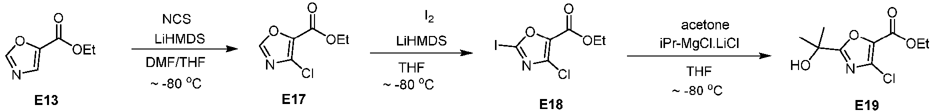

### Step 1: Preparation of Ethyl 4-chlorooxazole-5-carboxylate (E17)

Ethyl oxazole-5-carboxylate (E13) (10.0 g, 70.9 mmol) was added in DMF (50 mL) at approximately 10°C. The reaction mixture was cooled to -80°C, and LiHMDS (1 M, 92.1 mL, 1.3 eq) was added dropwise at approximately -80°C. The reaction mixture was stirred at approximately -80°C for 0.5 hr, and NCS (12.3 g, 92.1 mmol, 1.3 eq) in THF (50 mL) was then added dropwise at approximately -80°C. The mixture was stirred at approximately -80°C for 0.5 hr. The reaction progress was checked using TLC (PE/EtOAc=5/1). The reaction mixture was combined for workup with seven other reactions each performed using 10 g of E13. The combined reaction mixture was poured into sat. citric acid solution (100 mL) at approximately -10°C. The aqueous portion was extracted with ethyl acetate (100 mL x 3). The combined organic layer was washed with brine (50 mL), dried with Na₂SO₄, filtered, and concentrated to dryness. The residue was purified by column chromatography (SiO₂, PE/EtOAc=1/0 to 0/1) to afford E17 (18.0 g, 18% yield). ¹H NMR (400 MHz, CDCl₃) δ 7.94 (s, 1 H), 4.44 (q, 2 H), 1.42 (t, 3 H); LC-MS: m/z 176.0 (M+H)⁺.

### Step 2: Preparation of Ethyl 4-chloro-2-iodooxazole-5-carboxylate (E18)

Three reactions were carried out in parallel. Ethyl 4-chlorooxazole-5-carboxylate (E17) (6.00 g, 34.2 mmol) was dissolved in THF (30 mL) at rt. LiHMDS (1 M, 41.0 mL, 1.2 eq) was added dropwise at approximately -80°C, followed by the dropwise addition of I₂ (13.0 g, 51.3 mmol, 1.5 eq) in THF (30 mL) at approximately -80°C. The reaction mixture was stirred at approximately -80°C for 1 hr. The reaction progress was checked using TLC (PE/EtOAc=5/1). The three parallel reactions were combined together for workup. The combined reaction mixture was poured into sat. citric acid solution (100 mL) at approximately -5°C. The aqueous portion was extracted with ethyl acetate (100 mL x 3). The combined organic layer was washed with brine (50 mL), dried with Na₂SO₄, filtered, and concentrated to dryness. The residue was purified by column chromatography (SiO₂, PE/EtOAc=1/0 to 0/1) to afford E18 (10.0 g, 32% yield). ¹H NMR (400 MHz, DMSO-*d₆*) δ 4.20 - 4.27 (m, 2 H), 1.21 (t, 3 H); LC-MS: m/z 301.9 (M+H)⁺.

### Step 3: Preparation of Ethyl 4-chloro-2-(2-hydroxypropan-2-yl)oxazole-5-carboxylate (E19)

Two reactions were carried out in parallel. Compound E18 (5.00 g, 16.6 mmol) was added in THF (50 mL) at approximately 10°C under N₂. The suspension was degassed under vacuum and purged with N₂ three times. The mixture was cooled to -80°C, and iPr-MgCl.LiCl (1.3 M, 12.8 mL, 1 eq) was added dropwise at approximately -80°C. The mixture was stirred at approximately -80°C for 0.5 hr, and acetone (1.16 g, 19.9 mmol, 1.2 eq) was then added dropwise at approximately -80°C. The mixture was stirred at approximately -80°C for 0.5 hr. The reaction progress was checked using TLC (PE/EtOAc=5/1). The two parallel reactions were combined together for workup. The combined reaction mixture was poured into sat. citric acid solution (50 mL) at approximately -10°C. The aqueous portion was extracted with ethyl acetate (50 mL x 3). The combined organic layer was washed with brine (50 mL), dried with Na₂SO₄, filtered, and concentrated to dryness. The residue was purified by column chromatography (SiO₂, PE/EtOAc=1/0 to 0/1) and further purified by prep-HPLC (column: Phenomenex luna C18 (250 x 70 mm, 15 um); mobile phase: [water (0.05% HCl)-ACN]; B%: 20%-50%, 23 min) to afford E19 (1.80 g, 23% yield). ¹H NMR (400 MHz, DMSO-*d₆*) δ: 5.92 (s, 1H), 4.32 - 4.37 (m, 2H), 1.51 (s, 6H), 1.31 (t, 3H); LC-MS: m/z 234.0 (M+H)⁺.

### Intermediate 4-(difluoromethyl)-2-(2-cyanopropan-2-yl)oxazole-5-carboxylic acid

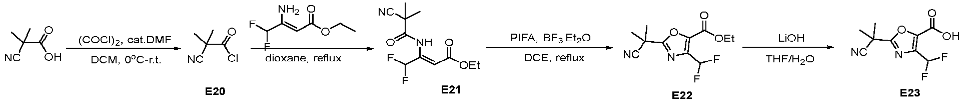

### Step 1: Preparation of 2-cyano-2-methyl-propanoyl chloride (E20)

To a solution of 2-cyano-2-methyl-propanoic acid (2 g, 17.7 mmol) and DMF (129 mg, 1.77 mmol, 136 µL, 0.1 eq) in DCM (20 mL) was added oxalyl chloride (2.69 g, 21.2 mmol, 1.86 mL, 1.2 eq) dropwise at 0°C, and then the reaction mixture was stirred at rt for 1 hr. The reaction mixture was concentrated to dryness to afford E20 (2.5 g), which was used without further purification.

### Step 2: Preparation of ethyl (Z)-3-[(2-cyano-2-methyl-propanoyl)amino]-4,4-difluoro-but-2-enoate (E21)

To a solution of ethyl (Z)-3-amino-4,4-difluoro-but-2-enoate (2 g, 12.1 mmol) in dioxane (20 mL) was added a solution of 2-cyano-2-methyl-propanoyl chloride (E20) (2.39 g, 18.2 mmol, 1.5 eq) in dioxane (20 mL) at rt. The reaction mixture was stirred at 110°C for 12 hrs. The reaction progress was checked using TLC (PE/Ethyl acetate=5/1). The reaction mixture was concentrated to dryness, and the residue was purified by flash silica gel chromatography (ISCO^{®}; 40 g SepaFlash^{®} Silica Flash Column, eluent of 0~7% EtOAc/PE gradient @ 40 mL/min) to give E21 (2.4 g, 76% yield). ¹H NMR (400 MHz, DMSO-*d*₆) δ 10.90 (s, 1 H), 6.95-7.27 (m, 1 H), 5.81 (s, 1 H), 4.12-4.29 (m, 2 H), 1.51-1.69 (m, 6 H), 1.20 (t, 3 H); LCMS: m/z 259.1 [M+H]⁺.

### Step 3: Preparation of ethyl 2-(1-cyano-1-methyl-ethyl)-4-(difluoromethyl)oxazole-5-carboxylate (E22)

To a solution of ethyl (Z)-3-[(2-cyano-2-methyl-propanoyl)amino]-4,4-difluoro-but-2-enoate (E21) (2.4 g, 9.22 mmol) in DCE (20 mL) was added BF₃.Et₂O (2.62 g, 18.4 mmol, 2.28 mL, 2 eq) and PIDA (4.16 g, 12.9 mmol, 1.4 eq) at rt. The mixture was stirred at 90 °C for 18 hrs. The reaction progress was checked by TLC (PE/EtOAc). The reaction mixture was concentrated to dryness. DCM (100 mL) was added to the residue, and the organic layer washed with sat. Na₂CO₃ solution (10 mL) and brine (50 mL), dried over Na₂SO₄, filtered, and concentrated to dryness. The residue was purified by flash silica gel chromatography (ISCO^{®}; 12 g SepaFlash^{®} Silica Flash Column, eluent of 0~7% EtOAc/PE gradient @ 30 mL/min) to give E22 (0.25 g, 11% yield). ¹H NMR (400 MHz, DMSO-*d*₆) δ 7.03-7.55 (m, 1 H), 4.35 (q, 2 H), 1.77 (s, 6 H), 1.29 (t, 3 H).

### Step 4: Preparation of 2-(1-cyano-1-methyl-ethyl)-4-(difluoromethyl)oxazole-5-carboxylic acid (E23)

To a solution of ethyl 2-(1-cyano-1-methyl-ethyl)-4-(difluoromethyl)oxazole-5-carboxylate (E22) (0.25 g, 968 µmol) in THF (3 mL) and H₂O (1 mL) was added LiOH.H₂O (48.8 mg, 1.16 mmol, 1.2 eq). The mixture was stirred at rt for 18 hrs. Reaction completeness was checked using TLC (PE:EtOAc=5:1). The reaction mixture was concentrated under reduced pressure to remove THF. The aqueous portion was adjusted with 2 *M* HCl to pH ~7, and then lyophilized under vacuo to give E23 (0.22 g, 99% yield). ¹H NMR (400 MHz, DMSO-*d*₆) δ 7.23-7.74 (m, 1 H), 1.73 (s, 6 H).

### Intermediate 4-(difluoromethyl)-2-(pyrimidin-2-yl)oxazole-5-carboxylic acid according to General Scheme 5, Method B

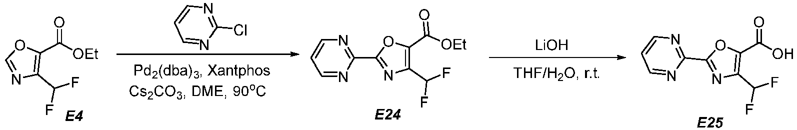

### Step 1: Preparation of ethyl 4-(difluoromethyl)-2-pyrimidin-2-yl-oxazole-5-carboxylate (E24)

To a solution of ethyl 4-(difluoromethyl)oxazole-5-carboxylate (E4) (1 g, 5.23 mmol), Pd₂(dba)₃ (240 mg, 262 µmol, 0.05 eq), (5-diphenylphosphanyl-9,9-dimethyl-xanthen-4-yl)-diphenyl-phosphane (151 mg, 262 µmol, 0.05 eq) and Cs₂CO₃ (3.41 g, 10.5 mmol, 2 eq) in 1,2-dimethoxyethane (3 mL) was added 2-chloropyrimidine (599 mg, 5.23 mmol) under N₂ atmosphere. The reaction mixture was stirred at 90°C for 12 hrs. DCM (50 mL) was added to the reaction mixture, and the organic portion washed by water (40 mL) and brine (40 mL), dried over Na₂SO₄, filtered, and concentrated to dryness. The residue was purified by column chromatography (SiO₂, PE/EtOAc=1/0 to 1/1) to give E24 (400 mg, 25% yield). ¹H NMR (400 MHz, CDCl₃) δ 9.02 (br d, 2 H), 7.52 (t, 1 H), 7.38 (s, 1 H), 7.24 (s, 1 H), 7.11 (s, 1 H), 4.54 (q, 2 H), 1.48 (t, 3 H).

### Step 2: Preparation of 4-(difluoromethyl)-2-pyrimidin-2-yl-oxazole-5-carboxylic acid (E25)

To a solution of ethyl 4-(difluoromethyl)-2-pyrimidin-2-yl-oxazole-5-carboxylate (E24) (450 mg, 1.67 mmol) in THF (2 mL) and H₂O (2 mL) was added LiOH.H₂O (77.2 mg, 1.84 mmol, 1.1 eq). The reaction mixture was stirred at rt for 1 hr. The reaction mixture was concentrated to remove THF, and H₂O (10 mL) was added. The aqueous layer was extracted with TBME (30 mL). The aqueous layer was then adjusted to pH~6 with 0.5 *M* HCl and extracted with DCM/MeOH (30/3 mL) 3 times. The combined DCM/MeOH organic layer was dried over Na₂SO₄, filtered, and concentrated to dryness to afford E25 (350 mg), which was used without further purification. ¹H NMR (400 MHz, DMSO-*d*₆) δ 9.06 (d, 2 H), 7.75 (t, 1 H), 7.20-7.57 (m, 1 H).

### Intermediate 2-(1-hydroxy-1-methyl-ethyl)-4-methyl-oxazole-5-carboxylic acid according to General Scheme 4 and General Scheme 6, Method A

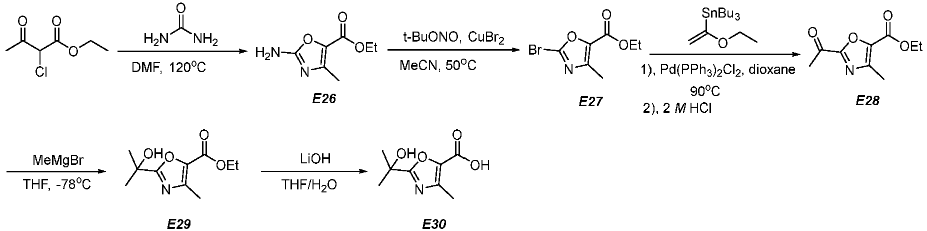

### Step 1: Preparation of ethyl 2-amino-4-methyl-oxazole-5-carboxylate (E26)

A mixture of ethyl 2-chloro-3-oxo-butanoate (55 g, 334 mmol, 46.2 mL) and urea (100.34 g, 1.67 mol, 89.6 mL, 5 eq) in DMF (100 mL) was stirred at 120°C for 12 hours. Reaction progress was checked using TLC (PE:EtOAc=5:1). The reaction mixture was cooled to rt, poured into H₂O (400 ml), and stirred at 0°C for 30 min. The solid was collected by filtration, rinsed with water (30 mL), and then dried in vacuo to give E26 (22.2 g, 39% yield). ¹H NMR (400 MHz, CD₃OD) δ 4.29 (q, 2 H), 2.32 (s, 3 H), 1.35 (t, 3 H).

### Step 2: Preparation of ethyl 2-bromo-4-methyl-oxazole-5-carboxylate (E27)

To a solution of E26 (11.1 g, 65.2 mmol) in MeCN (120 mL) was added CuBr₂ (21.85 g, 97.9 mmol, 4.58 mL, 1.5 eq) at 0°C. The mixture turned dark green and was further stirred for 15 min at rt. t-BuONO (10.09 g, 97.9 mmol, 11.6 mL, 1.5 eq) was added. The reaction was stirred at rt for 2 hrs and then heated at 50°C for 4 hrs. Reaction progress was checked using TLC (Petroleum ether:EtOAc=3:1). The reaction mixture was concentrated to dryness. The residue was purified by flash silica gel chromatography (ISCO^{®}; 120 g SepaFlash^{®} Silica Flash Column, eluent of 0~5% EtOAc/PE gradient @ 100 mL/min) to give E27 (8.5 g, 56% yield). ¹H NMR (400 MHz, CDCl₃) δ 4.25-4.35 (m, 2 H), 2.45 (s, 3 H), 1.32 (t, 3 H).

### Step 3: Preparation of ethyl 2-acetyl-4-methyloxazole-5-carboxylate (E28)

A mixture of E27 (9.5 g, 40.6 mmol), tributyl(1-ethoxyvinyl) stannane (17.59 g, 48.7 mmol, 16.4 mL, 1.2 eq) and Pd(PPh₃)₂Cl₂ (2.85 g, 4.06 mmol, 0.1 eq) in toluene (200 mL) was stirred at 90°C for 12 hrs under N₂ atmosphere. Reaction progress was checked using TLC (Petroleum ether:EtOAc=5:1). EtOAc (300 ml) was added, followed by sat. KF solution (500 ml). The resulting mixture was stirred at rt for 40 min, the mixture filtered, and the filtrate separated. The organic layer was washed with brine (100 mL), dried over Na₂SO₄, filtered, and concentrated to dryness. The residue was dissolved in THF (300 ml), and 4 N HCl (300 ml) was added. The mixture was stirred at rt for 12 hrs. LCMS indicated the desired mass was detected. The reaction mixture was extracted with DCM (100 mL x 3). The combined organic layer was dried over Na₂SO₄, filtered, and concentrated to dryness. The residue was purified by flash silica gel chromatography (ISCO^{®}; 24 g SepaFlash^{®} Silica Flash Column, eluent of 0~10% EtOAc/PE gradient @ 35mL/min) to give E28 (6.8 g, 85% yield). ¹H NMR (400 MHz, CDCl₃) δ 4.35 (q, 2 H), 2.62 (s, 3 H), 2.48 (s, 3 H), 1.34 (t, 3 H); LCMS: m/z 198.1 (M+H)⁺.

### Step 4: Preparation of ethyl 2-(2-hydroxypropan-2-yl)-4-methyloxazole-5-carboxylate (E29)

To a solution of E28 (2.5 g, 12.7 mmol) in THF (25 mL) was added MeMgBr (3 M, 12.7 mL, 3 eq) dropwise under N₂ at -78°C. The reaction was stirred at -78°C for 1.5 hr. Reaction progress was checked using TLC (Petroleum ether:EtOAc=5:1, by UV). The reaction was quenched by the addition of sat. NH₄Cl solution (30 mL) slowly and then the organic portion extracted with EtOAc (30 mL x 3). The combined organic phase was washed with brine (30 mL), dried over Na₂SO₄, filtered, and concentrated to dryness. The residue was purified by column chromatography (SiO₂, Petroleum ether/EtOAc=1/0 to 1/1) to give E29 (1.7 g, 63% yield). ¹H NMR (400 MHz, CDCl₃) δ 4.39 (q, 2 H), 2.47 (s, 3 H), 1.67 (s, 6 H), 1.40 (t, 3 H).

Alternatively, E29 can be prepared by reacting 2-hydroxy-2-methylpropanamide with ethyl 2-chloro-3-oxo-butanoate in a neat reaction at 150°C for 6 hrs.

### Step 5: Preparation of ethyl 2-(2-hydroxypropan-2-yl)-4-methyloxazole-5-carboxylic acid (E30)

To a solution of E29 (3.4 g, 16.0 mmol) in THF (30 mL) and H₂O (15 mL) was added LiOH.H₂O (803 mg, 19.1 mmol, 1.2 eq). The reaction was stirred at rt for 1.5 hr. Reaction progress was tracked using LCMS. The reaction mixture was adjusted to pH 7 with HCl (1 M) and concentrated to dryness to afford E30 (4 g), which was used without further purification. ¹H NMR (400 MHz, DMSO-*d₆*) δ 5.67 (s, 1 H), 2.30 (s, 2 H), 1.44 (s, 6 H); LCMS: m/z 186.1 (M+H)⁺.

### Intermediate 4-cyclopropyl-2-(2-hydroxypropan-2-yl)oxazole-5-carboxylic acid according to General Scheme 4

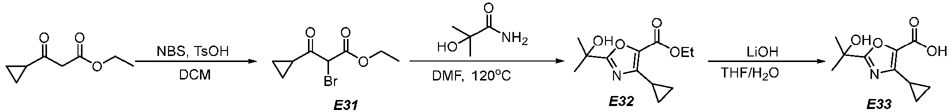

### Step 1: Preparation of ethyl 2-bromo-3-cyclopropyl-3-oxo-propanoate (E31)

To a solution of ethyl 3-cyclopropyl-3-oxo-propanoate (8 g, 51.2 mmol) in DCM (100 mL) was added NBS (9.12 g, 51.2 mmol) and TsOH.H₂O (1.95 g, 10.2 mmol, 0.2 eq). The reaction mixture was stirred at rt for 2 hrs. Reaction progress was tracked using TLC (PE:EtOAc=10:1). The reaction mixture was concentrated to dryness. EtOAc (120 mL) was added to the residue, and the mixture filtered. The filtrate was washed with sat. NaHCO₃ solution (2 × 100 mL) and water (100 mL), dried over Na₂SO₄, filtered, and concentrated to dryness. The residue was purified by column chromatography (SiO₂, PE/EtOAc=1:0 to 10:1) to give E31 (12 g, 100% yield). ¹H NMR (400 MHz, DMSO-*d*₆) δ 5.71 (s, 1 H), 4.18 (q, 2 H), 2.14-2.28 (m, 1 H), 1.18 (t, 3 H), 1.01-1.08 (m, 2 H), 0.89-0.96 (m, 2 H); LCMS: m/z 235.0 [M+H]⁺.

### Step 2: Preparation of ethyl 4-cyclopropyl-2-(1-hydroxy-1-methylethyl)oxazole-5-carboxylate (E32)

To a solution of ethyl 2-bromo-3-cyclopropyl-3-oxo-propanoate (E31) (1 g, 4.25 mmol) in DMF (2 mL) was added 2-hydroxy-2-methyl-propanamide (2.19 g, 21.3 mmol, 5 eq). The mixture was stirred at 110°C for 40 hrs. Reaction progress was tracked using LC-MS. The reaction mixture was adjusted to pH~8 by addition of sat. aq. NaHCO₃ at 0°C. H₂O (50 mL) was added, and the aqueous portion extracted with EtOAc (3 x 50 mL). The combined organic layers were dried over Na₂SO₄, filtered, and concentrated to dryness. The residue was purified by column chromatography (SiO₂, PE/EtOAc=1:0 to 2:1) to give E32 (0.15 g, 14% yield). ¹H NMR (400 MHz, DMSO-*d*₆) δ 5.70 (s, 1 H), 4.29 (q, 2 H), 2.40-2.45 (m, 1 H), 1.43 (s, 6 H), 1.27 (t, 3 H), 0.94-1.04 (m, 2 H), 0.79-0.92 (m, 2 H); LCMS: m/z 240.0 [M+H]⁺.

### Step 3: Preparation of 4-cyclopropyl-2-(1-hydroxy-1-methyl-ethyl)oxazole-5-carboxylic acid (E33)

To a solution of ethyl 4-cyclopropyl-2-(1-hydroxy-1-methyl-ethyl)oxazole-5-carboxylate (E32) (590 mg, 2.47 mmol) in THF (4 mL) and H₂O (4 mL) was added LiOH.H₂O (114 mg, 2.71 mmol, 1.1 eq). The reaction mixture was stirred at rt for 16 hrs. Reaction progress was tracked using LC-MS. The reaction mixture was concentrated to remove THF. The aqueous portion was adjusted to pH~7 by the addition of HCl (1 M) and then lyophilized to give E33 (550 mg, 90% yield). ¹H NMR (400 MHz, DMSO-*d*₆) δ 2.70-2.84 (m, 1 H), 1.39 (s, 6 H), 0.62-0.81 (m, 4 H); LCMS: m/z 212.0 [M+H]⁺.

### Intermediate 4-cyanooxazole-5-carboxylic acid

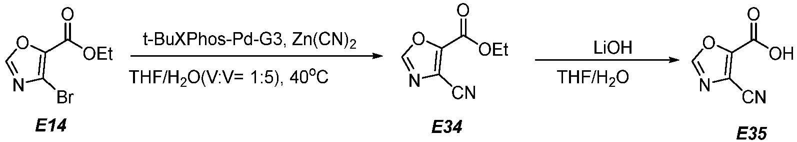

### Step 1: Preparation of ethyl 4-cyanooxazole-5-carboxylate (E34)

To a solution of ethyl 4-bromooxazole-5-carboxylate (E14) (10 g, 45.5 mmol) in THF (30 mL) and H₂O (150 mL) was added Zn(CN)₂ (3.74 g, 31.8 mmol, 2.02 mL, 0.7 eq) and t-BuXPhos-Pd-G3 (1.81 g, 2.27 mmol, 0.05 eq). The reaction mixture was degassed under vacuum and purged with N₂ 3 times, and then the mixture was stirred at 40°C for 16 hrs under N₂. Reaction progress was tracked using TLC (PE:EtOAc=5:1). EtOAc(300 mL) was added to the reaction mixture, and the organic layer washed with H₂O (50 mL) and brine (50 mL), dried over Na₂SO₄, filtered, and concentrated to dryness. The residue was purified by column chromatography (SiO₂, PE/EtOAc=100/1 to 10/1) to give E34 (7.5 g, 99% yield). ¹H NMR (400 MHz, DMSO-*d₆*) δ 8.89 (s, 1 H), 4.37 (q, 2 H), 1.30 (t, 3 H).

### Step 2: Preparation of (4-cyanooxazole-5-carbonyl)oxylithium (E35)

To a solution of E34 (5 g, 30.1 mmol) in THF (40 mL) and H₂O (80 mL) was added LiOH.H₂O (1.33 g, 31.6 mmol, 1.05 eq). The mixture was stirred at rt for 2 hrs. Reaction progress was tracked using TLC (PE:EtOAc=5:1). The reaction mixture was concentrated in vacuo to remove most of the THF and then lyophilized in vacuo to give E35 (4.5 g), which was used without further purification. ¹H NMR (400 MHz, DMSO-*d₆*) δ 8.47 (s, 1 H).

### Intermediate [5-[1-(2,2,2-trifluoroethyl)pyrazol-4-yl]-1,3,4-oxadiazole-2-carbonyl]oxylithium according to General Scheme 9

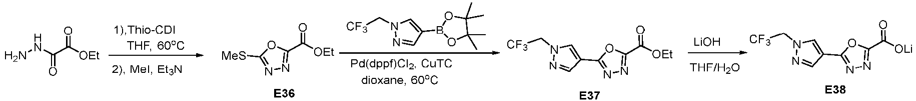

### Step 1: Preparation of ethyl 5-methylsulfanyl-1,3,4-oxadiazole-2-carboxylate (E36)

A mixture of ethyl 2-hydrazino-2-oxo-acetate (16 g, 121 mmol) and di(1H-imidazol-1-yl)methanethione (25.90 g, 145 mmol, 1.2 eq) in THF (300 mL) was stirred at rt for 12 hrs, and then heated to 75°C for 4 hrs. After cooling to rt, K₂CO₃ (50.21 g, 363 mmol, 3 eq) and CH₃I (85.95 g, 606 mmol, 37.7 mL, 5 eq) were added. The resulting mixture was stirred at rt for 2 hrs. Reaction progress was tracked using TLC (PE:EtOAc=1:1). The reaction mixture was combined with another reaction performed with 8.5 g of ethyl 2-hydrazino-2-oxo-acetate and quenched by the addition of H₂O (100 mL). DCM (500 mL) was added, and the organic layer was separated and washed with brine (2 x 100 mL), dried over Na₂SO₄, filtered, and concentrated to dryness. The residue was purified by flash silica gel chromatography (Eluent of 0~25% PE:EtOAc @ 40 mL/min) to give E36 (25 g). ¹H NMR (400 MHz, CDCl₃) δ 4.43 (q, 2 H), 2.72 (s, 3 H), 1.38 (t, 3 H).

### Step 2: Preparation of ethyl 5-[1-(2,2,2-trifluoroethyl)pyrazol-4-yl]-1,3,4-oxadiazole-2-carboxylate (E37)

A mixture of E36 (233 mg, 1.24 mmol), 4-(4,4,5,5-tetramethyl-1,3,2-dioxaborolan-2-yl)-1-(2,2,2-trifluoroethyl)pyrazole (410 mg, 1.49 mmol, 1.2 eq), Na₂CO₃ (394 mg, 3.71 mmol, 3 eq), thiophene-2-carbonyloxycopper (472 mg, 2.48 mmol, 2 eq) and Pd(dppf)Cl₂ (181 mg, 248 µmol, 0.2 eq) in dioxane (9 mL) was degassed and purged with N₂ 3 times, and then the reaction mixture was stirred at 75°C for 16 hrs under N₂. Reaction progress was tracked using TLC (PE:EtOAc=2:1). EtOAc (100 mL) was added to the reaction mixture, and the organic layer washed with water (30 mL) and brine (30 mL), dried over Na₂SO₄, filtered, and concentrated to dryness. The residue was purified by column chromatography (SiO₂, PE/EtOAc=1/0 to 2/1) to give E37 (140 mg, 39% yield). ¹H NMR (400 MHz, CD₃OD) δ 8.61 (s, 1 H), 8.22 (s, 1 H), 5.12 (q, 2 H), 4.51 (q, 2 H), 1.44 (t, 3 H); LCMS: m/z 291.1 (M+H)⁺.

### Step 3: Preparation of [5-[1-(2,2,2-trifluoroethyl)pyrazol-4-yl]-1,3,4-oxadiazole-2-carbonyl]oxylithium (E38)

To a solution of E37 (140 mg, 482 µmol) in THF (1.5 mL) was added LiOH.H₂O (22.3 mg, 531 µmol, 1.1 eq) in H₂O (3 mL). The reaction mixture was stirred at rt for 2 hrs. Reaction progress was tracked using TLC (DCM:MeOH). The reaction mixture was concentrated in vacuo to remove most of the THF and then lyophilized in vacuo to give E38 (115 mg), which was used without further purification. ¹H NMR (400 MHz, DMSO-*d₆*) δ 8.58 (s, 1 H), 8.14 (s, 1 H), 5.27 (q, 2 H).

Alternatively, ethyl 5-[1-(2,2,2-trifluoroethyl)pyrazol-4-yl]-1,3,4-oxadiazole-2-carboxylate (E37) can be prepared accordingly to the following scheme.

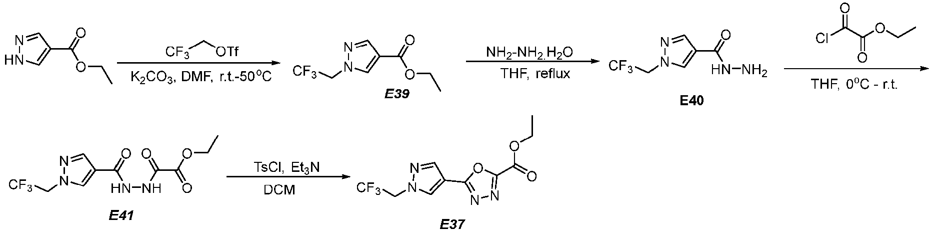

### Step A: Preparation of ethyl 1-(2,2,2-trifluoroethyl)pyrazole-4-carboxylate (E39)

To a solution of ethyl 1H-pyrazole-4-carboxylate (10 g, 71.4 mmol) and K₂CO₃ (19.72 g, 142 mmol, 2 eq) in DMF (45 mL) was added 2,2,2-trifluoroethyl trifluoromethanesulfonate (21.53 g, 92.8 mmol, 1.3 eq) dropwise, and then the resulting mixture was stirred at 50°C for 6 hrs. Reaction progress was tracked using TLC (PE:EtOAc=1:1). EtOAc (300 mL) was added to the reaction mixture, and the organic layer washed with H₂O (100 mL) and brine (2 x 100 mL), dried over Na₂SO₄, filtered, and concentrated to dryness. The residue was purified by flash silica gel chromatography (ISCO^{®}; 40g SepaFlash^{®} Silica Flash Column, eluent of 0-12% PE/EtOAc @ 60 mL/min) to give E39 (16 g). ¹H NMR (400 MHz, CDCl₃) δ 8.01 (d, 2 H), 4.73 (q, 2 H), 4.32 (q, 2 H), 1.36 (t, 3 H).

### Step B: Preparation of 1-(2,2,2-trifluoroethyl)pyrazole-4-carbohydrazide (E40)

To a solution of E39 (16 g, 72.0 mmol) in THF (100 mL) was added NH₂NH₂.H2O (7.73 g, 151 mmol, 7.5 mL, 98% purity, 2.1 eq), and then the reaction mixture was stirred at 80°C for 15 hrs. Reaction progress was tracked using TLC (PE:EtOAc=1:1). The reaction mixture was concentrated to dryness. The residue was triturated with TBME (50 mL) and stirred for 15 min. The precipitate was collected by filtration, and then dried in vacuo to give E40 (13 g). ¹H NMR (400 MHz, CD₃OD) δ 8.19 (s, 1 H), 7.93 (s, 1 H), 4.98 (q, 2 H).

### Step C: Preparation of ethyl N-[[1-(2,2,2-trifluoroethyl)pyrazole-4-carbonyl]amino] carbamate (E41)

To a solution of E40 (3 g, 14.4 mmol) in THF (70 mL) was added ethyl 2-chloro-2-oxo-acetate (2.36 g, 17.3 mmol, 1.9 mL, 1.2 eq) dropwise at 0°C, and then the reaction mixture was stirred at rt for 3 hrs. The precipitate was collected by filtration and rinsed with TBME (30 mL), and then dried in vacuo to give E41 (1.5 g, 37% yield). ¹H NMR (400 MHz, CDCl₃) δ 9.75 (br s, 1 H), 9.06 (br s, 1 H), 8.25 (s, 1 H), 7.99 (s, 1 H), 4.76 (q, 2 H), 4.42 (q, 2 H), 1.42 (t, 3 H).

### Step D: Preparation of ethyl 5-[1-(2,2,2-trifluoroethyl)pyrazol-4-yl]-1,3,4-oxadiazole-2-carboxylate (E37)

To a solution of E41 (1.5 g, 5.35 mmol) and Et₃N (271 mg, 2.68 mmol, 373 µL, 0.5 eq) in DCM (30 mL) was added TsCl (491 mg, 6.96 mmol, 1.3 eq) in portions at 0°C, and then the resulting mixture was stirred at rt for 18 hrs. Reaction progress was tracked using TLC (PE:EtOAc=1:1). DCM (100 mL) was added to the reaction mixture, and the organic layer washed with sat. Na₂CO₃ solution (60 mL) and brine (60 mL), dried over Na₂SO₄, filtered, and concentrated to dryness. The residue was purified by flash silica gel chromatography (ISCO^{®}; 40g SepaFlash^{®} Silica Flash Column, Eluent of 0-16% PE/EtOAc @ 40 mL/min) to give E37 (0.65 g, 42% yield). ¹H NMR (400 MHz, CDCl₃) δ 8.16-8.29 (m, 2 H), 4.80 (q, 2 H), 4.53 (q, 2 H), 1.47 (t, 3 H).

### Intermediate lithium 5-morpholino-1,3,4-oxadiazole-2-carboxylate according to General Scheme 10

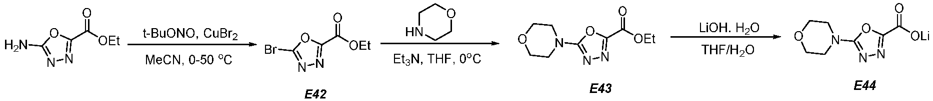

### Step 1: Preparation of ethyl 5-bromo-1, 3, 4-oxadiazole-2-carboxylate (E42)

To a solution of ethyl 5-amino-1,3,4-oxadiazole-2-carboxylate (5 g, 31.8 mmol) in MeCN (60 mL) was added CuBr₂ (10.66 g, 47.7 mmol, 2.2 mL, 1.5 eq) at 0°C. The reaction mixture turned dark green and was stirred for 15 min at rt. t-BuONO (4.92 g, 47.7 mmol, 5.7 mL, 1.5 eq) was added at 0 °C, and the reaction mixture was stirred at rt for 2 hrs, then heated at 50°C for another 12 hrs. The reaction progress was checked using TLC (PE/EtOAc=1/1). The reaction mixture was filtered and concentrated to dryness. The residue was purified by flash silica gel chromatography (ISCO^{®}; 40 g SepaFlash^{®} Silica Flash Column, eluent of 0~50% EtOAc/PE gradient @ 40mL/min) to give E42 (4.5 g, 64% yield). ¹H NMR (400 MHz, CDCl₃) δ 4.52 (q, 2 H), 1.45 (t, 3 H).

### Step 2: Preparation of ethyl 5-morpholino-1,3,4-oxadiazole-2-carboxylate (E43)

To a solution of ethyl 5-bromo-1,3,4-oxadiazole-2-carboxylate (E42) (1 g, 4.52 mmol) and morpholine (473 mg, 5.43 mmol, 478 µL, 1.2 eq) in THF (40 mL) was added DIPEA (1.17 g, 9.05 mmol, 1.6 mL, 2 eq) at 0°C. The mixture was stirred at rt for 1 hr. The reaction progress was checked using TLC (PE/EtOAc=1/1). EtOAc (100 mL) was added to the reaction mixture, and the organic portion washed with water (30 mL) and brine (30 mL x 2), dried over Na₂SO₄, filtered, and concentrated to dryness. The residue was purified by flash silica gel chromatography (ISCO^{®}; 12 g SepaFlash^{®} Silica Flash Column, Eluent of 0~30% EtOAc/PE gradient @ 35 mL/min) to give E43 (800 mg, 78% yield). ¹H NMR (400 MHz, DMSO-*d*₆) δ 4.37 (q, 2 H), 3.68-3.74 (m, 4 H), 3.48-3.52 (m, 4 H), 1.31 (t, 3 H).

### Step 3: Preparation of (5-morpholino-1, 3, 4-oxadiazole-2-carbonyl) oxylithium (E44)

To a solution of ethyl 5-morpholino-1, 3, 4-oxadiazole-2-carboxylate (E43) (800 mg, 3.52 mmol) in THF (8 mL) and H₂O (12 mL) was added LiOH.H₂O (162 mg, 3.87 mmol, 1.1 eq). The reaction mixture was stirred at rt for 12 hrs. The reaction progress was checked using TLC (PE/EtOAc=1/1). The reaction mixture was concentrated to dryness to afford E44 (600 mg), which was used without further purification. ¹H NMR (400 MHz, DMSO-*d*₆) δ 3.62-3.74 (m, 4 H), 3.36 (br d, 4 H).

### Intermediate [5-(5-fluoro-2-pyridyl)-1,3,4-oxadiazole-2-carbonyl]oxylithium according to General Scheme 9, Method A

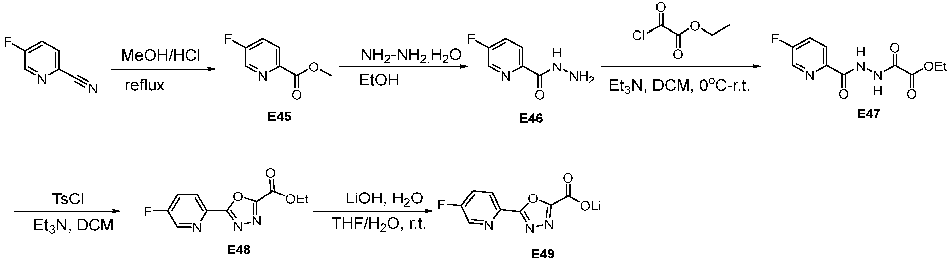

### Step 1: Preparation of methyl 5-fluoropyridine-2-carboxylate (E45)

A solution of 5-fluoropyridine-2-carbonitrile (15 g, 123 mmol) in HCl/MeOH (4 M, 180 mL, 5.9 eq) was stirred at 60°C for 12 hrs. The reaction mixture was concentrated in vacuo, and the residue was dissolved in EtOAc (150 mL), washed with sat. NaHCO₃ solution (50 mL) and brine (20 mL). The organic layer was dried over Na₂SO₄, filtered, and concentrated to dryness. The residue was purified by flash silica gel chromatography (ISCO^{®}; 80 g SepaFlash^{®} Silica Flash Column, Eluent of 0~25% EtOAc/PE gradient @ 60 mL/min) to give E45 (15.24 g, 80% yield). ¹H NMR (400 MHz, CDCl₃) δ 8.58 (d, 1 H), 8.20 (dd, 1 H), 7.53 (ddd, 1 H), 4.00 (s, 3 H).

### Step 2: Preparation of 5-fluoropyridine-2-carbohydrazide (E46)

A mixture of methyl 5-fluoropyridine-2-carboxylate (E45) (16 g, 103 mmol), NH₂NH₂.H₂O (11.06 g, 217 mmol, 10.7 mL, 98% purity, 2.1 eq) in EtOH (70 mL) was degassed and purged with N₂ for 3 times, and then the reaction mixture was stirred at rt for 3 hrs under N₂ atmosphere. The reaction mixture was concentrated to dryness to give E46 (15 g, 94% yield). ¹H NMR (400 MHz, DMSO-*d*₆) δ 9.89 (br s, 1 H), 8.61 (d, 1 H), 8.07 (dd, 1 H), 7.88 (td, 1 H), 4.57 (br s, 2 H).

### Step 3: Preparation of ethyl 2-[2-(5-fluoropyridine-2-carbonyl)hydrazino]-2-oxo-acetate (E47)

To a mixture of 5-fluoropyridine-2-carbohydrazide (E46) (13.5 g, 87.0 mmol) and TEA (17.61 g, 174 mmol, 24.2 mL, 2 eq) in DCM (500 mL) was added ethyl 2-chloro-2-oxo-acetate (15.45 g, 113 mmol, 12.7 mL, 1.3 eq) over a period of 10 min at 0°C. The reaction mixture was stirred for 2 hrs at rt. H₂O (100 mL) was added to the reaction mixture, and the aqueous portion extracted with DCM (300 mL x 3). The combined organic layer was washed with brine (100 mL), dried over Na₂SO₄, filtered, and concentrated to dryness. The residue was purified by flash silica gel chromatography (ISCO^{®}; 80 g SepaFlash^{®} Silica Flash Column, Eluent of 0~2.5% EtOAc/PE gradient @ 60 mL/min) to give E47 (16 g, 53% yield). ¹H NMR (400 MHz, CDCl₃) δ 10.03-10.15 (m, 1 H), 9.65 (br d, 1 H), 8.35-8.42 (m, 1 H), 8.11-8.18 (m, 1 H), 7.51 (td, 2 H), 4.35 (q, 1 H), 1.34 (t, 3 H); LCMS: m/z 256.2 [M+H]⁺.

### Step 4: Preparation of ethyl 5-(5-fluoro-2-pyridyl)-1,3,4-oxadiazole-2-carboxylate (E48)

To a solution of ethyl 2-[2-(5-fluoropyridine-2-carbonyl)hydrazino]-2-oxoacetate (E47) (16 g, 62.7 mmol) in DCM (350 mL) was added TEA (8.25 g, 81.5 mmol, 11.3 mL, 1.3 eq) and TosCl (5.98 g, 31.4 mmol, 0.5 eq) in 3 portions at 0°C. The mixture was stirred at rt for 3 hrs. Sat. NaHCO₃ solution (200 mL) was added, and the aqueous portion extracted with DCM (400 mL x 3). The combined organic layer was washed with brine (200 mL), dried over Na₂SO₄, filtered, and concentrated to dryness. The residue was purified by flash silica gel chromatography (ISCO^{®}; 120 g SepaFlash^{®} Silica Flash Column, Eluent of 0~2.5% MeOH/DCM@ 85 mL/min) to give E48 (8.2 g, 49% yield). ¹H NMR (400 MHz, CDCl₃) δ 8.71 (d, 1 H), 8.39 (dd, 1 H), 7.66 (ddd, 1 H), 4.59 (q, 2 H), 1.51 (t, 3 H); LCMS: m/z 238.2 [M+H]⁺.

### Step 5: Preparation of [5-(5-fluoro-2-pyridyl)-1,3,4-oxadiazole-2-carbonyl]oxylithium (E49)

To a solution of ethyl 5-(5-fluoro-2-pyridyl)-1,3,4-oxadiazole-2-carboxylate (E48) (12 g, 50.6 mmol) in THF (140 mL) and H₂O (180 mL) was added LiOH.H₂O (2.23 g, 53.1 mmol, 1.05 eq). The mixture was stirred at rt for 2 hrs. The reaction mixture was concentrated in vacuo to remove most of THF, and the aqueous portion was lyophilized in vacuo to give E49 (11.5 g), which was used without further purification. ¹H NMR (400 MHz, DMSO-*d*₆) δ 8.79 (d, 1 H), 8.24 (dd, 1 H), 7.99 (td, 1 H).

### Intermediate 5-(difluoromethyl)-1-(2,2,2-trifluoroethyl)pyrazole-4-carboxylic acid

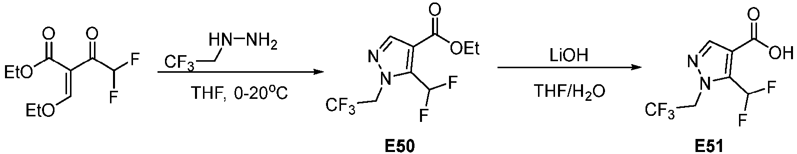

### Step 1: Preparation of ethyl 5-(difluoromethyl)-1-(2,2,2-trifluoroethyl)pyrazole-4-carboxylate (E50)

To a solution of (Z)-ethyl 2-(ethoxymethylene)-4,4-difluoro-3-oxobutanoate (1 g, 4.52 mmol) in THF (8 mL) was added 2,2,2-trifluoroethylhydrazine (670 mg, 5.88 mmol, 1.3 eq) dropwise at 0°C. The reaction mixture was stirred at 0°C for 1 hr, and then stirred at rt for 16 hrs. Reaction progress was checked using LCMS. The reaction mixture was concentrated to dryness, and the residue was purified by flash silica gel chromatography (Eluent: 0~5% EtOAc/PE gradient) to give E50 (850 mg, 69% yield). ¹H NMR (400 MHz, CDCl₃) δ 8.02 (s, 1 H), 7.38-7.75 (m, 1 H), 4.95 (q, 2 H), 4.37 (q, 2 H), 1.40 (t, 3 H); LCMS: m/z 273.1 [M+H]⁺.

### Step 2: Preparation of 5-(difluoromethyl)-1-(2,2,2-trifluoroethyl)pyrazole-4-carboxylic acid (E51)

To a solution of ethyl 5-(difluoromethyl)-1-(2,2,2-trifluoroethyl)pyrazole-4-carboxylate (E50) (910 mg, 3.34 mmol) in THF (5 mL) and H₂O (5 mL) was added LiOH.H₂O (224 mg, 5.35 mmol, 1.6 eq). The mixture was stirred at rt for 2 hrs. The reaction progress was checked using TLC (PE:EtOAc=2:1). The reaction mixture was concentrated to remove THF. The aqueous portion was adjusted to pH~6 by the addition of HCl (1 *M).* The resulting precipitate was collected by filtration and then dried in vacuo to give E51 (816 mg, 100% yield). ¹H NMR (400 MHz, DMSO-*d*₆) δ 8.08 (s, 1 H), 7.45-7.78 (m, 1 H), 5.30 (q, 2 H).

### Intermediate ethyl 2-(2-pyridyl)pyrazole-3-carboxylate

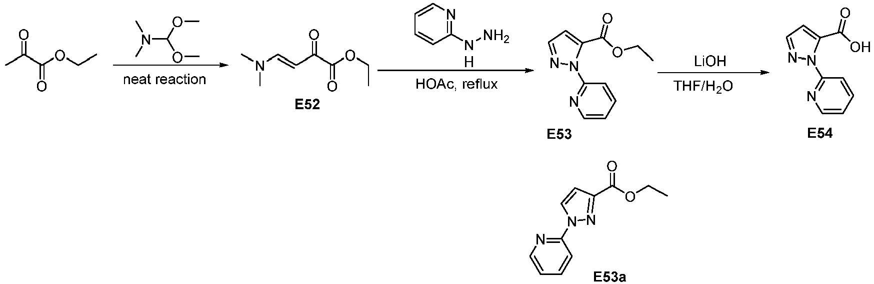

### Step 1: Preparation of ethyl (E)-4-(dimethylamino)-2-oxo-but-3-enoate (E52)

Ethyl 2-oxopropanoate (2 g, 17.2 mmol, 1.9 mL) and 1,1-dimethoxy-N,N-dimethyl-methanamine (2.09 g, 17.6 mmol, 2.3 mL, 1.02 eq) were stirred at rt for 12 hrs. The reaction progress was checked using TLC (PE:EtOAc=10:1). The reaction mixture was concentrated to dryness to give E52 (2.4 g), which was used without further purification.

### Step 2: Preparation of ethyl 2-(2-pyridyl)pyrazole-3-carboxylate (E53)

A mixture of ethyl (E)-4-(dimethylamino)-2-oxo-but-3-enoate (E52) (2.4 g, 14.0 mmol) and 2-pyridylhydrazine (1.53 g, 14.0 mmol) in HOAc (100 mL) was stirred at 110°C for 12 hrs. The reaction progress was checked using TLC (PE:EtOAc=10:1). The reaction mixture was adjusted to pH ~9 by the addition of sat. Na₂CO₃ solution. The aqueous portion was extracted with EtOAc (300 mL). The organic layer was dried over Na₂SO₄, filtered, and concentrated to dryness. The residue was purified by column chromatography (SiO₂, PE/EtOAc=100/1 to 50/1) to give E53 (1.03 g, 34% yield). ¹H NMR (400 MHz, DMSO-*d*₆) δ 8.48 (dt, 1 H), 8.05 (td, 1 H), 7.83-7.96 (m, 1 H), 7.76 (d, 1 H), 7.49 (ddd, 1 H), 6.96-7.05 (m, 1 H), 4.21 (q, 2 H), 1.05-1.23 (m, 3 H); LCMS: m/z 218.1 [M+H]⁺.

Ethyl 1-(2-pyridyl)pyrazole-3-carboxylate (E53a) was also obtained (190 mg, 6% yield). ¹H NMR (400 MHz, DMSO-*d*₆) δ 8.70-8.81 (m, 1 H), 8.54 (dd, 1 H), 7.92-8.13 (m, 2 H), 7.47 (ddd, 1 H), 7.02 (d, 1 H), 4.35 (q, 2 H), 1.34 (t, 3 H). The regiochemistry of E53 and E53a were confirmed by HSQC and HMBC NMR analysis.

### Step 3: Preparation of 2-(2-pyridyl)pyrazole-3-carboxylic acid (E54)

To a solution of ethyl 2-(2-pyridyl)pyrazole-3-carboxylate (E53) (1 g, 4.60 mmol) in THF (10 mL) and H₂O (10 mL) was added LiOH.H₂O (386 mg, 9.21 mmol, 2 eq), and the reaction mixture was stirred at rt for 12 hrs. The reaction progress was checked using TLC (PE/EtOAc=1/1). The reaction mixture was concentrated under reduced pressure to remove THF. The aqueous portion was adjusted to pH~7 by the addition of 2 M HCl and lyophilized in vacuo to give (E54) (1.2 g), which was used without further purification. ¹H NMR (400 MHz, DMSO-*d*₆) δ 8.36-8.44 (m, 1 H), 7.88 (td, 1 H), 7.44-7.53 (m, 2 H), 7.35 (ddd, 1 H), 6.44 (d, 1 H).

### Intermediate [5-(difluoromethyl)-2-methyl-1,2,4-triazole-3-carbonyl]oxylithium

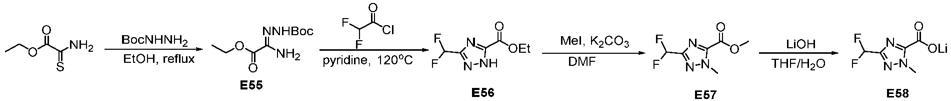

### Step 1: Preparation of tert-butyl (Z)-2-(1-amino-2-ethoxy-2-oxoethylidene)hydrazine-1-carboxylate (E55)

Tert-butyl (Z)-2-(1-amino-2-ethoxy-2-oxoethylidene)hydrazine-1-carboxylate was prepared from ethyl 2-amino-2-thioxoacetate following the procedure in Bioorg. Med. Chem., 26 (2016) 3223-3225. The reaction was heated to reflux.

### Step 2: Preparation of ethyl 3-(difluoromethyl)-1H-1,2,4-triazole-5-carboxylate (E56)

Reagent 2,2-difluoroacetyl chloride was prepared by adding oxalyl dichloride (18.90 g, 149 mmol, 13.0 mL, 1.1 eq) dropwise at 0°C to a solution of 2,2-difluoroacetic acid (13 g, 135 mmol, 8.5 mL) and DMF (989 mg, 13.5 mmol, 1.0 mL, 0.1 eq) in DCM (80 mL). The reaction mixture was stirred at rt for 1 hr, and the solution was used without further purification.

To a solution of tert-butyl (Z)-2-(1-amino-2-ethoxy-2-oxoethylidene)hydrazine-1-carboxylate (E55) (13 g, 56.2 mmol) in pyridine (90 mL) was added 2,2-difluoroacetyl chloride (15.47 g, 135 mmol, 2.4 eq). The reaction mixture was heated to 120°C and stirred for 12 hrs. Reaction progress was checked using LCMS. The reaction mixture was concentrated to dryness. DCM (200 mL) was added to the residue, and the organic portion washed with 1 *M* HCl (30 mL) and brine (60 mL), dried over Na₂SO₄, filtered, and concentrated to dryness. The residue was purified by column chromatography on silica gel (PE:EtOAc=20:1 to 2:1) to give E56 (8.87 g, 83% yield). ¹H NMR (400 MHz, DMSO-*d*₆) δ 7.01-7.32 (m, 1 H), 4.36 (q, 2 H), 1.30 (t, 3 H); LCMS: m/z 192.0 [M+H]⁺.

### Step 3: Preparation of ethyl 5-(difluoromethyl)-2-methyl-1,2,4-triazole-3-carboxylate (E57)

To a solution of ethyl 3-(difluoromethyl)-1H-1,2,4-triazole-5-carboxylate (E56) (10 g, 52.3 mmol) in DMF (80 mL) was added MeI (22.28 g, 157 mmol, 9.8 mL, 3 eq) and K₂CO₃ (21.69 g, 157 mmol, 3 eq). The mixture was stirred at rt for 12 hrs. Reaction progress was checked using TLC (PE:EtOAc=1:1). The reaction mixture was filtered, and the precipitate was rinsed with EtOAc (150 mL). The filtrate was washed with water (30 mL) and brine (30 mL), dried over Na₂SO₄, filtered, and concentrated to dryness. The residue was purified by column chromatography on silica gel (PE:EtOAc=20:1 to 1:1) to give E57 (4.46 g, 42% yield). ¹H NMR (400 MHz, DMSO-*d*₆) δ 6.99-7.34 (m, 1 H), 4.39 (q, 2 H), 4.11-4.22 (m, 3 H), 1.35 (t, 3 H).

### Step 4: Preparation of [5-(difluoromethyl)-2-methyl-1,2,4-triazole-3-carbonyl]oxylithium (E58)

To a solution of ethyl 5-(difluoromethyl)-2-methyl-1,2,4-triazole-3-carboxylate (E57) (2.35 g, 11.5 mmol) in THF (40 mL) and H₂O (10 mL) was added LiOH.H₂O (505 mg, 12.0 mmol, 1.05 eq). The reaction mixture was stirred at rt for 1 hr. Reaction progress was checked using TLC (PE:EtOAc=2:1). The reaction mixture was concentrated in vacuo to remove THF. H₂O (20 mL) was added, and the aqueous portion extracted with TBME (20 mL), and then lyophilized in vacuo to give E58 (2.15 g), which was used without further purification. ¹H NMR (400 MHz, DMSO-*d*₆) δ 6.75-7.21 (m, 1 H), 4.09 (s, 3 H).

### Intermediate ethyl 3-(1-hydroxy-1-methyl-ethyl)-1,2,4-oxadiazole-5-carboxylate

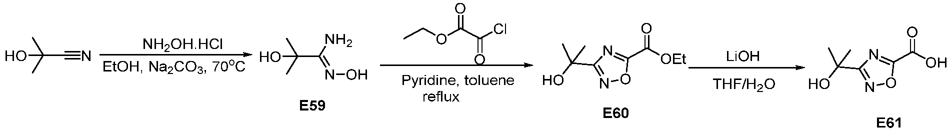

### Step 1: Preparation of N',2-dihydroxy-2-methyl-propanamidine (E59)

To a solution of 2-hydroxy-2-methyl-propanenitrile (2.14 g, 25.2 mmol, 2.3 mL) in EtOH (20 mL) was added hydroxylamino hydrochloride (3.49 g, 50.3 mmol, 2 eq) and Na₂CO₃ (5.33 g, 50.3 mmol, 2 eq), and then the reaction was stirred at 70°C for 16 hrs. The reaction mixture was concentrated to dryness. EtOAc (200 mL) was added to the residue, and the organic portion washed twice with water (50 mL) and brine (50 mL), dried over Na₂SO₄, filtered, and concentrated to dryness to afford E59 (1 g), which was used without further purification. ¹H NMR (400 MHz, DMSO-*d*₆) δ 10.11 (s, 1 H), 8.86 (s, 1 H), 5.03 (br s, 1 H), 1.70 (s, 3 H), 1.66 (s, 3 H).

### Step 2: Preparation of ethyl 3-(1-hydroxy-1-methyl-ethyl)-1,2,4-oxadiazole-5-carboxylate (E60)

To a solution of N',2-dihydroxy-2-methyl-propanamidine (E59) (520 mg, 4.40 mmol) and pyridine (696 mg, 8.80 mmol, 711 µL, 2 eq) in toluene (15 mL) was added ethyl 2-chloro-2-oxo-acetate (601 mg, 4.40 mmol, 493 µL) at 0°C. The reaction mixture was stirred at rt for 1 hr, and then stirred at 100°C for 15 hrs. Reaction progress was checked using LCMS. The reaction mixture was concentrated to dryness. The residue was purified by column chromatography (SiO₂, PE/EtOAc=1/0 to 2/1) and further purified by prep-HPLC (column: Boston Uni C18 40*150*5um;mobile phase: [water(0.225%FA)-ACN];B%: 16%-46%,7.7min) to give E60 (250 mg, 28% yield). ¹H NMR (400 MHz, CDCl₃) δ 4.52 (q, 2 H), 2.65 (br s, 1 H), 1.67 (s, 6 H), 1.45 (t, 3 H); LCMS: m/z 201.1 [M+H]⁺.

### Step 3: Preparation of ethyl 3-(1-hydroxy-1-methyl-ethyl)-1,2,4-oxadiazole-5-carboxylate (E61)

To a solution of ethyl 3-(1-hydroxy-1-methyl-ethyl)-1,2,4-oxadiazole-5-carboxylate (E60) (200 mg, 999 µmol) in THF (5 mL) and H₂O (5 mL) was added LiOH.H₂O (41.9 mg, 999 µmol), and then the reaction mixture was stirred at rt for 1 hr. The solvent was removed in vacuo, and the aqueous portion was lyophilized in vacuo to give E61 (170 mg), which was used without further purification.

### Intermediates E62, E63, E64, E65, E66, and E67

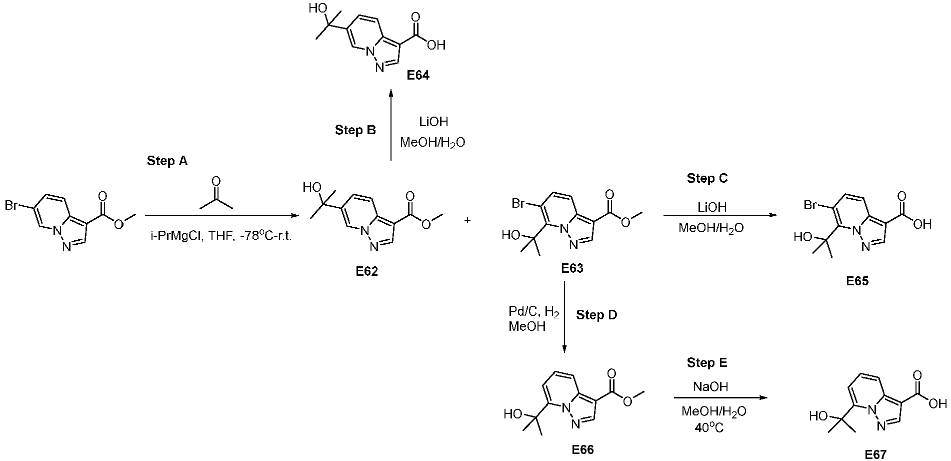

### Step A: Preparation of methyl 6-(2-hydroxypropan-2-yl)pyrazolo[1,5-a]pyridine-3-carboxylate (E62) and methyl 6-bromo-7-(2-hydroxypropan-2-yl)pyrazolo[1,5-a]pyridine-3-carboxylate (E63)

To a solution of methyl 6-bromopyrazolo[1,5-a]pyridine-3-carboxylate (1 g, 3.92 mmol) in THF (10 mL) was added i-PrMgCl-LiCl (1.3 M, 6.03 mL, 2 eq) at -78°C. The reaction mixture was stirred at -78°C for 15 min, then acetone (1.37 g, 23.5 mmol, 1.7 mL, 6 eq) was added dropwise to the reaction mixture. The reaction mixture was stirred at - 78°C for 1 hr. Reaction progress was checked using LCMS. The reaction mixture was dropwise added to 10 mL of aq. NH₄Cl solution. Water (30 mL) was added, and the aqueous portion extracted with EtOAc (25 mL x 2). The combined organic layers were washed with brine (20 mL), dried over Na₂SO₄, filtered, and concentrated to dryness. The residue was purified by column chromatography (SiO₂, PE/EtOAc=1/0 to 20:1) to give E62 (320 mg, 34% yield) and E63 (300 mg, 23% yield).

E62: ¹H NMR (400 MHz, CD₃OD) δ 8.40-8.54 (m, 1 H), 8.14 (dd, 1 H), 7.57 (dd, 1 H), 7.27 (dd, 1 H), 3.88-4.03 (m, 3 H), 1.77-1.92 (m, 6 H); LCMS: m/z 235.2 [M+H]⁺.

E63: ¹H NMR (400 MHz, CD₃OD) δ 8.46 (s, 1 H), 8.02 (d, 1 H), 7.76 (d, 1 H), 3.93 (s, 3 H), 1.95 (s, 6 H); LCMS: m/z 313.0 [M+H]⁺.

### Step B: Preparation of 6-(2-hydroxypropan-2-yl)pyrazolo[1,5-a]pyridine-3-carboxylic acid (E64)

To a solution of methyl 6-(1-hydroxy-1-methyl-ethyl)pyrazolo[1,5-a]pyridine-3-carboxylate (E62) (500 mg, 2.13 mmol) in MeOH (3 mL) and H₂O (1 mL) was added LiOH.H₂O (116 mg, 2.77 mmol, 1.3 eq). The reaction mixture was stirred at 40°C for 12 hrs. Reaction progress was checked using LCMS. The reaction mixture was concentrated in vacuo to remove MeOH, and water (50 mL) was added. The aqueous portion was extracted with TBME (30 mL), and the aqueous layer was then adjusted to pH ~6 by the addition of 0.5 *M* HCl. The resulting white suspension was extracted with DCM/MeOH (100/10 mL) 3 times. The combined DCM/MeOH organic layer was dried over Na₂SO₄, filtered, and concentrated to dryness to afford E64 (420 mg, 89% yield). LCMS: m/z 221.1 [M+H]⁺.

### Step C: Preparation of 6-bromo-7-(2-hydroxypropan-2-yl)pyrazolo[1,5-a]pyridine-3-carboxylic acid (E65)

To a solution of methyl 6-bromo-7-(2-hydroxypropan-2-yl)pyrazolo[1,5-a]pyridine-3-carboxylate (E63) (120 mg, 383 µmol) in MeOH (2 mL) and H₂O (1 mL) was added LiOH.H₂O (17.69 mg, 422 µmol, 1.1 eq), the mixture was stirred at 40°C for 12 hr. Reaction progress was checked using LCMS. The reaction mixture was concentrated in vacuo to remove MeOH, and water (10 mL) was added. The aqueous portion was extracted with TBME (30 mL) and was then adjusted to pH ~6 by the addition of 0.5 *M* HCl. The resulting white suspension was extracted with DCM/MeOH (30/3 mL) 3 times. The combined DCM/MeOH organic layer was dried over Na₂SO₄, filtered, and concentrated to dryness to afford E65 (100 mg, 87% yield). ¹H NMR (400 MHz, CD₃OD) δ 8.44 (s, 1 H), 8.07 (d, 1 H), 7.74 (d, 1 H), 1.96 (s, 6 H); LCMS: m/z 301.1 [M+H]⁺.

### Step D: Preparation of methyl 7-(2-hydroxypropan-2-yl)pyrazolo[1,5-a]pyridine-3-carboxylate (E66)

To a solution of methyl 6-bromo-7-(2-hydroxypropan-2-yl)pyrazolo[1,5-a]pyridine-3-carboxylate (E63) (450 mg, 1.44 mmol) in MeOH (10 mL) was added Pd/C (100 mg, 10% purity) under N₂. The resulting mixture was degassed under vacuum and purged with H₂ 3 times, and then the mixture was stirred at rt for 1 hr under H₂ (15 psi). Reaction progress was checked using LCMS. The reaction mixture was filtered through a pad of Celite, and the filtrate was concentrated in vacuo to afford E66 (350 mg), which was used without further purification. LCMS: m/z 234.9 [M+H]⁺.

### Step E: Preparation of 7-(2-hydroxypropan-2-yl)pyrazolo[1,5-a]pyridine-3-carboxylic acid (E67)

To a solution of methyl 7-(2-hydroxypropan-2-yl)pyrazolo[1,5-a]pyridine-3-carboxylate (E66) (350 mg, 1.49 mmol) in MeOH (5 mL) and H₂O (1 mL) was added NaOH (89.64 mg, 2.24 mmol, 1.5 eq), the mixture was stirred at 40°C for 12 hrs. Reaction progress was checked using LCMS. The reaction mixture was concentrated in vacuo to remove MeOH, and water (10 mL) was added. The aqueous portion was extracted with TBME (30 mL), and then adjusted to pH ~6 by the addition of 0.5 *M* HCl. The resulting white suspension was extracted with DCM/MeOH (30/3 mL) 3 times. The combined DCM/MeOH organic layer was dried over Na₂SO₄, filtered, and concentrated to dryness to afford E67 (320 mg), which was used without further purification. LCMS: m/z 220.9 [M+H]⁺.

### Intermediate E72 according to General Scheme 7, Methods C and D

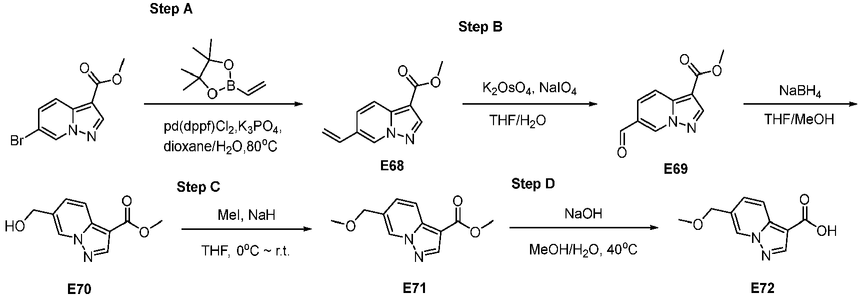

### Step A: Preparation of methyl 6-vinylpyrazolo[1,5-a]pyridine-3-carboxylate (E68)

A mixture of 4,4,5,5-tetramethyl-2-vinyl-1,3,2-dioxaborolane (8.15 g, 52.9 mmol, 8.98 mL), methyl 6-bromopyrazolo[1,5-a]pyridine-3-carboxylate (9 g, 35.3 mmol), K₃PO₄ (22.47 g, 106 mmol) and Pd(dppf)Cl₂ (1.29 g, 1.76 mmol) in dioxane (80 mL) and H₂O (40 mL) was stirred at 80°C for 12 hrs under N₂. Reaction progress was checked using LCMS. The reaction mixture was combined with another 1 g batch reaction, and DCM (200 mL) was added. The organic layer was washed with brine (2 x 80 mL), dried over Na₂SO₄, filtered, and concentrated to dryness. The residue was purified by column chromatography (SiO₂, PE/EtOAc=1/0 to 4/1) to give E68 ( 6.4 g, 80.2% yield). ¹H NMR (400 MHz, CDCl₃) δ 8.37 (s, 1 H), 8.30 (s, 1 H), 8.03 (d, 1 H), 7.52 (dd, 1 H), 6.61 (dd, 1 H), 5.76 (d, 1 H), 5.34 (d, 1 H), 3.84 (s, 3 H); LCMS: m/z 203.1 [M+H]⁺.

### Step B: Preparation of methyl 6-formylpyrazolo[1,5-a]pyridine-3-carboxylate (E69)

To a solution of methyl 6-vinylpyrazolo[1,5-a]pyridine-3-carboxylate (E68) (6.4 g, 31.7 mmol) in THF (80 mL) and H₂O (80 mL) was added K₂OsO₄.2H₂O (583 mg, 1.58 mmol) and NaIO₄ (16.92 g, 79.1 mmol, 4.38 mL) at 0°C, and then the reaction mixture was stirred at rt for 12 hrs. Reaction progress was checked using LCMS. DCM (200 mL) was added to the reaction mixture, and the organic layer was washed with brine (2 x 80 mL), dried over Na₂SO₄, filtered, and concentrated to dryness. The residue was purified by column chromatography (SiO₂, DCM/EtOAc=1/0 to 6/1) to give E69 (5.9 g, 91.3% yield). ¹H NMR (400 MHz, CDCl₃) δ 9.94 (s, 1 H), 8.90-8.98 (m, 1 H), 8.48 (s, 1 H), 8.18 (d, 1 H), 7.80 (dd, 1 H), 3.88 (s, 3 H); LCMS: m/z 204.9 [M+H]⁺.

### Step C: Preparation of methyl 6-(hydroxymethyl)pyrazolo[1,5-a]pyridine-3-carboxylate (E70)

To a solution of methyl 6-formylpyrazolo[1,5-a]pyridine-3-carboxylate (E69) (2.4 g, 11.8 mmol) in THF (30 mL) and MeOH (30 mL) was added NaBH₄ (1.78 g, 47.0 mmol) at 0°C, and then the reaction mixture was stirred at rt for 1 hr. Reaction progress was checked using LCMS. The reaction mixture was quenched by the addition of sat. aq. NH₄Cl (50 mL), and the aqueous portion extracted with EtOAc (100 mL). The organic layer was washed with water (50 mL) and brine (40 mL), dried over Na₂SO₄, filtered, and concentrated to dryness to give E70 (2.3 g, 94.9% yield). ¹H NMR (400 MHz, CDCl₃) δ 8.54 (s, 1 H), 8.38 (s, 1 H), 8.11 (d, 1 H), 7.41 (dd, 1 H), 4.78 (s, 2 H), 3.93 (s, 3 H); LCMS: m/z 207.0 [M+H]⁺.

### Step D: Preparation of methyl 6-(methoxymethyl)pyrazolo[1,5-a]pyridine-3-carboxylate (E71)

To a solution of methyl 6-(hydroxymethyl)pyrazolo[1,5-a]pyridine-3-carboxylate (E70) (2.2 g, 10.7 mmol) in THF (50 mL) was added NaH (512 mg, 12.8 mmol, 60% purity) at 0°C. The reaction mixture was stirred at 0°C for 15 min, and then MeI (1.82 g, 12.8 mmol, 797 pL) was added to the reaction mixture. The reaction mixture was stirred at rt for 1 hr, and reaction progress was checked using LCMS. The reaction mixture was quenched by the addition of sat. aq. NH₄Cl (40 mL), and the aqueous portion extracted with DCM (2 x 60 mL). The combined organic layer was washed by water (30 mL) and brine (30 mL), dried over Na₂SO₄, filtered, and concentrated to dryness. The residue was purified by column chromatography (SiO₂, PE/EtOAc=1/0 to 5/1) to give E71 (1.8 g, 76.6% yield). ¹H NMR (400 MHz, CDCl₃) δ 8.52 (s, 1 H), 8.41 (s, 1 H), 8.16 (d, 1 H), 7.42 (dd, 1 H), 4.53 (s, 2 H), 3.94 (s, 3 H), 3.46 (s, 3 H); LCMS: m/z 221.1 [M+H]⁺.

### Step E: Preparation of 6-(methoxymethyl)pyrazolo[1,5-a]pyridine-3-carboxylic acid (E72)

To a solution of methyl 6-(methoxymethyl)pyrazolo[1,5-a]pyridine-3-carboxylate (E71) (1.8 g, 8.17 mmol) in MeOH (40 mL) and H₂O (40 mL) was added NaOH (719 mg, 18.0 mmol), and the reaction mixture was stirred at 40°C for 12 hrs. Reaction progress was checked using LCMS. The reaction mixture was concentrated to remove MeOH, and then H₂O (10 mL) was added. The pH of the aqueous mixture was adjusted to ~6 by the addition of 0.5 M HCl, and the aqueous mixture was filtered. The precipitate was dried in vacuo to give E72 (1.6 g, 94.9% yield). ¹H NMR (400 MHz, CDCl₃) δ 8.57 (s, 1 H), 8.45-8.51 (m, 1 H), 8.21 (d, 1 H), 7.48 (dd, 1 H), 4.55 (s, 2 H), 3.48 (s, 3 H); LCMS: m/z 206.7 [M+H]⁺.

### Intermediate E78 according to General Scheme 9, Method A

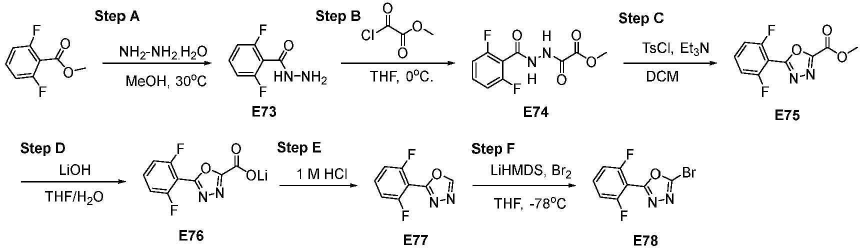

### Step A: Preparation of 2,6-difluorobenzohydrazide (E73)

To a solution of methyl 2,6-difluorobenzoate (25 g, 145 mmol) in MeOH (250 mL) was added NH₂NH₂.H₂O (18.18 g, 363 mmol, 17.7 mL), and the reaction mixture was stirred at 60°C for 12 hrs. Reaction progress was checked using TLC. Additional NH₂NH₂.H₂O (7.27 g, 145 mmol, 7.05 mL) was added, and the reaction mixture was stirred at 60°C for another 3 hrs. The reaction mixture was concentrated in vacuo to give E73 (25 g), which was used without further purification. ¹H NMR (400 MHz, CDCl₃) δ 7.37 (s, 1 H), 6.93 (t, 2 H), 3.45-3.89 (m, 2 H).

### Step B: Preparation of methyl 2-[2-(2,6-difluorobenzoyl)hydrazino]-2-oxo-acetate (E74)

To a solution of 2,6-difluorobenzohydrazide (E73) (25 g, 145 mmol) in THF (250 mL) was added methyl 2-chloro-2-oxo-acetate (19.57 g, 160 mmol, 14.7 mL) at 0 °C, and the reaction mixture was stirred at 0°C for 1 hr. Reaction progress was checked using TLC. The reaction mixture was filtered. The precipitate was washed with MTBE (800 mL), and then dried in vacuo to give E74 (37 g). ¹H NMR (400 MHz, DMSO-*d*₆) δ 7.59 (ddd, 2 H), 7.21 (t, 1 H), 3.83 (s, 2 H).

### Step C: Preparation of methyl 5-(2,6-difluorophenyl)-1,3,4-oxadiazole-2 - carboxylate (E75)

To a solution of methyl 2-[2-(2,6-difluorobenzoyl)hydrazino]-2-oxo-acetate (E74) (15 g, 58.1 mmol) in DCM (250 mL) was added Et₃N (23.52 g, 232 mmol, 32.4 mL) and TosCl (14.40 g, 75.5 mmol) in portions at 0°C. The reaction mixture was allowed to warm to rt and stirred at rt for 12 hrs. Reaction progress was checked using TLC. DCM (100 mL) was added to the reaction mixture, and the organic layer was washed with sat. aq. NaHCO₃ (100 mL) and water (100 mL), dried over Na₂SO₄, filtered, and concentrated to dryness. The residue was purified by column chromatography (SiO₂, PE/EtOAc=1/0 to 1/1) to give E75 (4.3 g, 30.8% yield). ¹H NMR (400 MHz, CDCl₃) δ 7.61 (tt, 1 H), 7.14 (t, 2 H), 4.10 (s, 3 H).

### Step D: Preparation of 5-(2,6-difluorophenyl)-1,3,4-oxadiazole-2-carboxylic acid (E76)

To a solution of methyl 5-(2,6-difluorophenyl)-1,3,4-oxadiazole-2-carboxylate (E75) (300 mg, 1.25 mmol) in H₂O (2 mL) and THF (4 mL) was added LiOH.H₂O (52.4 mg, 1.25 mmol). The reaction mixture was stirred at rt for 1 hr. Reaction progress was checked using LCMS. The reaction mixture was concentrated in vacuo to give E76 (300 mg), which was used without further purification. LCMS: m/z 226.8 [M+H]⁺

### Step E: Preparation of 2-(2,6-difluorophenyl)-1,3,4-oxadiazole (E77)

To a solution of 5-(2,6-difluorophenyl)-1,3,4-oxadiazole-2-carboxylic acid (E76) (4 g, 17.7 mmol) in H₂O (20 mL) was added HCl (1 M, 35.4 mL), and the reaction mixture was stirred at rt for 1 hr. Reaction progress was checked using LCMS and TLC. DCM (60 mL) was added to the reaction mixture, and the organic layer washed by water (20 mL), dried over Na₂SO₄, filtered, and concentrated to dryness. The residue was purified by column chromatography (SiO₂, PE/EtOAc=1/0 to 1/1) to give E77 (1.5 g, 46.6% yield). ¹H NMR (400 MHz, CDCl₃) δ 8.63 (s, 1 H), 7.57 (tt, 1 H), 7.12 (t, 2 H); LCMS: m/z 182.8 [M+H]⁺.

### Step F: Preparation of 2-bromo-5-(2,6-difluorophenyl)-1,3,4-oxadiazole (E78)

To a solution of 2-(2,6-difluorophenyl)-1,3,4-oxadiazole (E77) (1.5 g, 8.24 mmol) in THF (50 mL) was added LiHMDS (1 M, 16.5 mL) dropwise at -78°C. The reaction mixture was stirred at -78°C for 30 min, then Br₂ (2.63 g, 16.5 mmol, 849 µL) was added to the reaction mixture at -78°C dropwise. The reaction mixture was stirred at -78°C for 2 hrs. Reaction progress was checked using TLC. Sat. aq. NH₄Cl (100 mL) was added to the reaction mixture, and the aqueous portion extracted with DCM (3 x 50 mL). The combined organic layer was washed with brine (40 mL), dried over Na₂SO₄, filtered and concentrated to dryness. The residue was purified by column chromatography (SiO₂, PE/EtOAc=1/0 to 3/1) to give E78 (1.6 g, 54.2% yield). ¹H NMR (400 MHz, DMSO-*d*₆) δ 7.79 (tt, 1 H), 7.41 (t, 2 H); LCMS: m/z 341.2 [M+H]⁺.

### Preparation of Examples 107 and 108 according to General Scheme 1, Method C

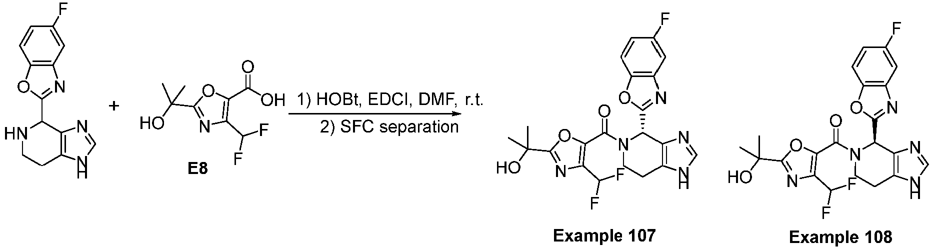

### Step 1: Preparation of [4-(difluoromethyl)-2-(2-hydroxypropan-2-yl)oxazol-5-yl]-[4-(5-fluoro-1,3-benzoxazol-2-yl)-1,4,6,7-tetrahydroimidazo[4,5-c]pyridin-5-yl]methanone

To a solution of 5-fluoro-2-(4,5,6,7-tetrahydro-1H-imidazo[4,5-c]pyridin-4-yl)-1,3-benzoxazole (4.00 g, 15.5 mmol) and 4-(difluoromethyl)-2-(1-hydroxy-1-methylethyl) oxazole-5-carboxylic acid (E8) (3.77 g, 17.0 mmol, 1.1 eq) in DMF (80 mL) was added HOBt (2.72 g, 20.1 mmol, 1.3 eq) and EDCI (3.86 g, 20.1 mmol, 1.3 eq). The reaction mixture was stirred at rt for 16 hrs. Reaction progress was tracked using LCMS. DCM (300 mL) was added, and the organic portion washed with sat. NaHCO₃ solution (50 mL) and brine (50 mL), dried over Na₂SO₄, filtered, and concentrated to dryness. The residue was purified by flash silica gel chromatography (ISCO^{®}; 80 g SepaFlash^{®} Silica Flash Column, Eluent of 0~5% DCM:MeOH @ 35 mL/min) to give compound [4-(difluoromethyl)-2-(2-hydroxypropan-2-yl)oxazol-5-yl]-[4-(5-fluoro-1,3-benzoxazol-2-yl)-1,4,6,7-tetrahydroimidazo[4,5-c]pyridin-5-yl]methanone (4.4 g, 60% yield). LCMS: m/z 462.1 [M+H]⁺.

### Step 2: SFC Separation

[4-(difluoromethyl)-2-(2-hydroxypropan-2-yl)oxazol-5-yl]-[4-(5-fluoro-1,3-benzoxazol-2-yl)-1,4,6,7-tetrahydroimidazo[4,5-c]pyridin-5-yl]methanone (4.4 g) underwent SFC separation (column: DAICEL CHIRALPAK IC(250mm*50mm,10um);mobile phase: [Neu-IPA];B%: 40%-40%,min) to afford Enantiomer 1 (1.75 g, 39.6%, Rt = 3.174 min) and Enantiomer 2 (1.79 g, 39% yield, Rt=3.769 min).

Enantiomer 1 (Example 107): (S)-(4-(difluoromethyl)-2-(2-hydroxypropan-2-yl)oxazol-5-yl)(4-(5-fluorobenzo[d]oxazol-2-yl)-6,7-dihydro-1H-imidazo[4,5-c]pyridin-5(4H)-yl)methanone. ¹H NMR (400 MHz, CD₃OD) δ 7.57-7.78 (m, 2 H), 7.35-7.53 (m, 1 H), 6.88-7.31 (m, 2 H), 6.47-6.82 (m, 1 H), 4.59 (br d, 1 H), 4.44-4.64 (m, 1 H), 3.96 (br s, 0.7 H), 3.63 (br s, 0.3 H), 2.77-3.27 (m, 2 H), 1.47-1.73 (m, 6 H); LCMS: m/z 462.1 [M+H]⁺; SFC: 98.1% ee.

Enantiomer 2 (Example 108): (R)-(4-(difluoromethyl)-2-(2-hydroxypropan-2-yl)oxazol-5-yl)(4-(5-fluorobenzo[d]oxazol-2-yl)-6,7-dihydro-1H-imidazo[4,5-c]pyridin-5(4H)-yl)methanone. ¹H NMR (400 MHz, CD₃OD) δ 7.57-7.85 (m, 2 H), 7.35-7.54 (m, 1 H), 6.91-7.31 (m, 2 H), 6.59-6.87 (m, 1 H), 4.57 (br s, 1 H), 3.96 (br s, 0.7 H), 3.59 (br s, 0.3 H), 2.74-3.19 (m, 2 H), 1.42-1.72 (m, 6 H); LCMS: m/z 462.1 [M+H]⁺; SFC: 93.9% ee.

### Preparation of Examples 925 and 926 according to General Scheme 1A, Method C

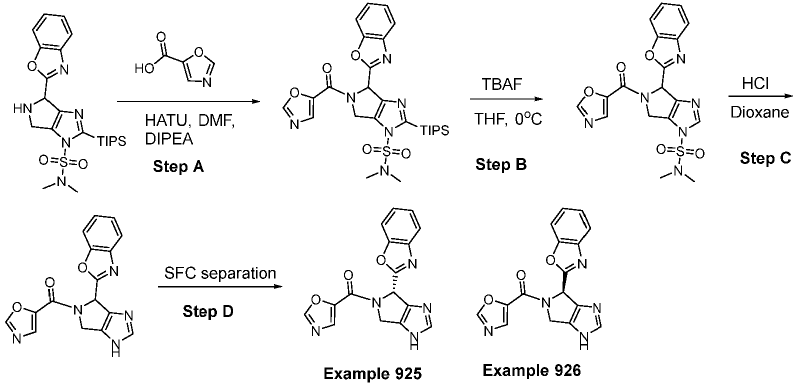

### Step A: Preparation of 6-(benzo[d]oxazol-2-yl)-N,N-dimethyl-5-(oxazole-5-carbonyl)-5,6-dihydropyrrolo[3,4-d]imidazole-1(4H)-sulfonamide

Oxazole-5-carboxylicacid (39.6 mg, 350 µmol) was dissolved in DMF (1.5 mL), and then HATU (145 mg, 382 µmol) and Diisopropylethylamine (82.3 mg, 110 µL, 637 µmol) were added. 6-(benzo[d]oxazol-2-yl)-N,N-dimethyl-2-(triisopropylsilyl)-5,6-dihydropyrrolo[3,4-d]imidazole-1(4H)-sulfonamide (156 mg, 319 µmol) was then added. The reaction mixture was stirred at rt overnight. DCM (30 mL) was added, and the organic layer was washed by water (10 mL) and brine (10 mL), dried over Na₂SO₄, filtered, and concentrated to dryness. The residue was purified by column chromatography (SiO₂, Heptane/EtOAc=1/0 to 0/1) to give 6-(benzo[d]oxazol-2-yl)-N,N-dimethyl-5-(oxazole-5-carbonyl)-5,6-dihydropyrrolo[3,4-d]imidazole-1(4H)-sulfonamide (110 mg, 59.1% yield). LC-MS: m/z 585.1 [M+H]⁺.

### Step B: Preparation of 6-(benzo[d]oxazol-2-yl)-N,N-dimethyl-5-(oxazole-5-carbonyl)-5,6-dihydropyrrolo[3,4-d]imidazole-1(4H)-sulfonamide

6-(benzo[d]oxazol-2-yl)-N,N-dimethyl-5-(oxazole-5-carbonyl)-2-(triisopropylsilyl)-5,6-dihydropyrrolo[3,4-d]imidazole-1(4H)-sulfonamide (110 mg, 188 µmol) was dissolved in THF (2.6 mL) and cooled to 0°C. Tetrabutylammonium fluoride (98.4 mg, 376 µL, 1M in THF, 376 µmol) was then added, and the reaction mixture was stirred at 0°C for 1 hr. DCM was added (30 mL), and the organic layer washed by water (10 mL) and brine (10 mL), dried over Na₂SO₄, filtered, and concentrated to dryness. The residue was purified by prep. HPLC (column: XSelect CSH C18 5um, 19x100mm; mobile phase: 30-36% MeCN in H₂O [with 0.1% formic acid]) to give 6-(benzo[d]oxazol-2-yl)-N,N-dimethyl-5-(oxazole-5-carbonyl)-5,6-dihydropyrrolo[3,4-d]imidazole-1(4H)-sulfonamide (20 mg, 25% yield). ¹H NMR (400 MHz, CDCl₃) δ 8.03 (s, 1H), 7.87 (s, 1H), 7.79 (s, 1H), 7.67-7.65 (d, 1H), 7.56-7.54 (d, 1H), 7.37-7.29 (m, 2H), 6.68 (s, 1H), 5.32-5.28 (dd, 1H), 5.09-5.06 (d, 1H), 2.66 (s, 6H); LC-MS: m/z 429.1 [M+H]⁺.

### Step C: Preparation of (6-(benzo[d]oxazol-2-yl)-4,6-dihydropyrrolo[3,4-d]imidazol-5(1H)-yl)(oxazol-5-yl)methanone

6-(benzo[d]oxazol-2-yl)-N,N-dimethyl-5-(oxazole-5-carbonyl)-5,6-dihydropyrrolo[3,4-d]imidazole-1(4H)-sulfonamide (19 mg, 44 µmol) was dissolved in 1,4-dioxane (0.49 mL) and then hydrogen chloride (49 mg, 0.33 mL, 4M in dioxane, 1.3 mmol) was added. The reaction mixture was stirred at rt for 3 hrs, and then sat. aq. Na₂CO₃ was added until the pH of the aqueous layer was ~8. The aqueous layer was extracted with DCM (~10 mL) three times. The combined organic layer was dried over Na₂SO₄, filtered, and concentrated to dryness. The residue was purified by HPLC (column: XSelect CSH C18 5um, 19x100mm, gradient: 13-17% MeCN in H₂O [with 0.1% formic acid]) to give (6-(benzo[d]oxazol-2-yl)-4,6-dihydropyrrolo[3,4-d]imidazol-5(1H)-yl)(oxazol-5-yl)methanone (9 mg, 60% yield). ¹H NMR (400 MHz, CD₃OD) δ 8.45 (s, 1H), 7.91 (s, 1H), 7.72 (s, 1H), 7.66-7.59 (m, 2H), 7.41-7.35 (m, 2H), 6.46 (s, 1H), 5.36 (s, 1H), 5.23 (s, 1H); LC-MS: m/z 322.0 [M+H]⁺.

### Step D: SFC separation

(6-(benzo[d]oxazol-2-yl)-4,6-dihydropyrrolo[3,4-d]imidazol-5(1H)-yl)(oxazol-5-yl)methanone (11 mg, 34 µmol) was separated into its enantiomers by SFC (column: ColumnTek Enantiocel A2S-5 250 (L) x 20 (ID) mm; mobile phase: 25% EtOH with CO₂) to give Enantiomer 1 (5 mg, Rt = 4.617 min) and Enantiomer 2 (5 mg, Rt = 6.066 min).

Enantiomer 1 (Example 925): (S)-(4-(benzo[d]oxazol-2-yl)-4,6-dihydropyrrolo[3,4-d]imidazol-5(1H)-yl)(oxazol-5-yl)methanone. ¹H NMR (400 MHz, CD₃OD) δ 8.45 (s, 1H), 7.91 (s, 1H), 7.72 (s, 1H), 7.65-7.59 (m, 2H), 7.42-7.35 (m, 2H), 6.47 (s, 1H), 5.38-5.35 (d, 1H), 5.23-5.20 (d, 1H); LC-MS: 332.1 (M+H)⁺; SFC: 99.5% ee.

Enantiomer 2 (Example 926): (R)-(4-(benzo[d]oxazol-2-yl)-4,6-dihydropyrrolo[3,4-d]imidazol-5(1H)-yl)(oxazol-5-yl)methanone. ¹H NMR (400 MHz, CD₃OD) δ 8.44 (s, 1H), 7.91 (s, 1H), 7.72 (s, 1H), 7.65-7.59 (m, 2H), 7.41-7.34 (m, 2H), 6.47 (s, 1H), 5.37-5.34 (d, 1H), 5.23-5.20 (d, 1H); LC-MS: 332.1 (M+H)⁺; SFC: 96.8% ee.

### Preparation of Examples 931 and 932 According to General Scheme 1A, Method B

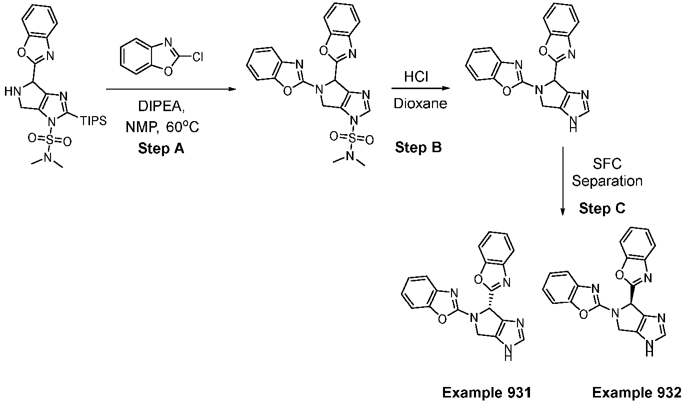

### Step A: Preparation of 5,6-bis(benzo[d]oxazol-2-yl)-N,N-dimethyl-5,6-dihydropyrrolo[3,4-d]imidazole-1(4H)-sulfonamide

6-(1,3-benzoxazol-2-yl)-N,N-dimethyl-2-triisopropylsilyl-5,6-dihydro-4H-pyrrolo[3,4-d]imidazole-3-sulfonamide (66 mg, 0.13 mmol) was dissolved in NMP (1.3 mL), and then 2-chlorobenzo[d]oxazole (25 mg, 18 µL, 0.16 mmol) and diisopropylethylamine (26 mg, 35 µL, 0.20 mmol) were added. The reaction mixture was stirred overnight at 60°C. The reaction was allowed to cool and DCM (30 mL) was added. The organic layer was washed by water (10 mL) and brine (10 mL), dried over Na₂SO₄, filtered, and concentrated to dryness. The residue was purified by column chromatography (SiO₂, Heptane/EtOAc=1/0 to 0/1) to give 5,6-bis(benzo[d]oxazol-2-yl)-N,N-dimethyl-5,6-dihydropyrrolo[3,4-d]imidazole-1(4H)-sulfonamide (44 mg, 72% yield). ¹H NMR (400 MHz, CDCl₃) δ 7.89 (s, 1H), 7.73-7.71 (m, 1H), 7.54-7.52 (m, 1H), 7.40-7.38 (m, 1H), 7.36-7.32 (m, 2H), 7.31-7.28 (m, 1H), 7.17-7.13 (td, 1H), 7.05-7.01 (td, 1H), 6.61-6.59 (m, 1H), 5.09-5.05 (dd, 1H), 4.96-4.92 (dd, 1H), 2.70 (s, 6H); LC-MS: m/z 451.0 [M+H]⁺.

### Step B: Preparation of 2,2'-(4,6-dihydropyrrolo[3,4-d]imidazole-4,5(1H)-diyl)bis(benzo[d]oxazole)

5,6-bis(benzo[d]oxazol-2-yl)-N,N-dimethyl-5,6-dihydropyrrolo[3,4-d]imidazole-1(4H)-sulfonamide (44 mg, 98 µmol) was dissolved in dioxane (0.98 mL), and then hydrogen chloride (0.11 g, 0.73 mL, 4M in dioxane, 2.9 mmol) was added. The reaction mixture was stirred at rt for 3 hrs. Sat. aq. Na₂CO₃ was added until the pH was ~8. The aqueous layer was extracted with DCM (~10 mL) three times. The combined organic layer was dried over Na₂SO₄, filtered, and concentrated to dryness. The residue was purified by HPLC (column: XSelect CSH C18 5um, 30x100mm, gradient: 28-32% MeCN in H₂O [with 0.1% formic acid]) to give 2,2'-(4,6-dihydropyrrolo[3,4-d]imidazole-4,5(1H)-diyl)bis(benzo[d]oxazole) (15 mg, 45% yield). ¹H NMR (400 MHz, CD₃OD) δ 7.72 (s, 1H), 7.68-7.66 (m, 1H), 7.62-7.60 (m, 1H), 7.42-7.36 (m, 2H), 7.32-7.29 (m, 2H), 7.18-7.14 (td, 1H), 7.07-7.03 (td, 1H), 6.45 (s, 1H), 5.11-5.07 (dd, 1H), 5.01-4.98 (d, 1H); LC-MS: m/z 344.0 [M+H]⁺.

### Step C: SFC separation

2,2'-(4,6-dihydropyrrolo[3,4-d]imidazole-4,5(1H)-diyl)bis(benzo[d]oxazole) (19 mg, 55 umol) was separated into its enantiomers by SFC (column: ColumnTek Enantiocel C4-5, 250 (L) x 30 (ID) mm; mobile phase: 40% EtOH (0.1% DEA) with CO₂ to give Enantiomer 1 (8.5 mg, Rt = 7.070 min) and Enantiomer 2 (8.6 mg, Rt = 9.800 min).

Enantiomer 1 (Example 931): (S)-2,2'-(4,6-dihydropyrrolo[3,4-d]imidazole-4,5(1H)-diyl)bis(benzo[d]oxazole). ¹H NMR (400 MHz, CD₃OD) δ 7.72 (s, 1H), 7.67-7.65 (m, 1H), 7.62-7.59 (dd, 1H), 7.41-7.36 (m, 2H), 7.34-7.29 (m, 2H), 7.18-7.14 (td, 1H), 7.07-7.03 (td, 1H), 6.45 (s, 1H), 5.11-5.07 (dd, 1H), 5.01-4.98 (d, 1H); LC-MS: 344.1 (M+H)⁺; SFC: >99% ee.

Enantiomer 2 (Example 932): (R)-2,2'-(4,6-dihydropyrrolo[3,4-d]imidazole-4,5(1H)-diyl)bis(benzo[d]oxazole). ¹H NMR (400 MHz, CD₃OD) δ 7.72 (s, 1H), 7.67-7.65 (m, 1H), 7.61-7.59 (m, 1H), 7.41-7.35 (m, 2H), 7.34-7.29 (m, 2H), 7.18-7.14 (td, 1H), 7.07-7.02 (td, 1H), 6.45-6.44 (d, 1H), 5.10-5.06 (dd, 1H), 5.00-4.97 (dd, 1H). LC-MS: 344.1 (M+H)⁺; SFC: >99% ee.

The compounds of Tables 1S, 1R, and 2 were characterized using proton NMR and LCMS and the enantiomeric excess determined. See, Table 3.

| **Table 3** | | | |
|---|---|---|---|
| **Ex. #** | **¹H NMR (400 MHz)** | **LCMS m/z** | **% ee** |
| 1 | (CD₃OD) δ 8.12 (br s, 0.5 H), 7.75-7.91 (m, 1 H), 7.66 (s, 1 H), 7.32-7.53 (m, 2 H), 7.10-7.28 (m, 1 H), 6.77 (br s, 0.5 H), 5.78 (br dd, 0.5 H), 4.98 (br d, 0.5 H), 3.81 (br s, 0.5 H), 3.46 (br s, 0.5 H), 2.85-3.18 (m, 1 H), 2.79 (br d, 1 H), 2.43 (br s, 3 H) | 399.9 [M+H]⁺ | 83.9% |
| 2 | (CD₃OD) δ 8.12 (br s, 0.5 H), 7.76-7.92 (m, 1 H), 7.66 (s, 1 H), 7.35-7.51 (m, 2 H), 7.22 (br d, 1 H), 6.78 (br s, 0.5 H), 5.61-5.88 (m, 0.5 H), 4.98 (br s, 0.5 H), 3.80 (br s, 0.5 H), 3.46 (br s, 0.5 H), 2.85-3.17 (m, 1 H), 2.79 (br d, 1 H), 2.43 (br s, 3 H) | 399.9 [M+H]⁺ | 89.1% |
| 3 | (CD₃OD) δ 7.88 (d, 1 H), 7.51-7.71 (m, 3 H), 6.81 (br s, 0.7 H), 6.35 (br s, 0.3 H), 4.95-5.03 (m, 0.4 H), 4.33 (dd, 0.6 H), 3.80 (br s, 1 H), 2.95 (br s, 1 H), 2.71-2.86 (m, 1 H), 2.42-2.66 (m, 2 H), 1.08 (br d, 1 H), 0.50-0.61 (m, 2 H), 0.24 (br d, 2 H) | 403.0 [M+H]⁺ | 93.2% |
| 4 | (CD₃OD) δ 7.88 (d, 1 H), 7.50-7.72 (m, 3 H), 6.82 (br s, 0.7 H), 6.36 (br s, 0.3 H), 4.95-5.04 (m, 0.3 H), 4.33 (dd, 0.7 H), 3.80 (br s, 1 H), 2.95 (br s, 1 H), 2.68-2.86 (m, 1 H), 2.41-2.66 (m, 2 H), 1.08 (br d, 1 H), 0.49-0.65 (m, 2 H), 0.24 (br d, 2 H) | 402.9 [M+H]⁺ | 96.8% |
| 5 | (CD₃OD) δ 8.63 (d, 1 H), 8.35 (br s, 1 H), 8.25 (d, 1 H), 7.97 (td, 1 H), 7.62-7.79 (m, 2 H), 7.39-7.57 (m, 2 H), 7.23 (td, 1 H), 6.74 (br s, 1 H), 4.63 (s, 1 H), 3.96 (s, 1 H), 3.14 (br d, 1 H), 2.87 (br d, 1 H) | 447.1 [M+H]⁺ | 95.9% |
| 6 | (CD₃OD) δ 8.63 (d, 1 H), 8.35 (br s, 1 H), 8.24 (d, 1 H), 7.97 (td, 1 H), 7.59-7.80 (m, 2 H), 7.38-7.56 (m, 2 H), 7.22 (td, 1 H), 6.77 (br s, 1 H), 4.63 (br s, 1 H), 3.96 (br s, 1 H), 3.11 (br d, 1 H), 2.77-2.96 (m, 1 H) | 447.0 [M+H]⁺ | 94.6% |
| 7 | (DMSO-d₆) δ 12.23 (br s, 1 H), 8.76 (br s, 1 H), 8.12-8.26 (m, 2 H), 7.95-8.11 (m, 2 H), 7.73 (br d, 1 H), 7.55-7.65 (m, 3 H), 6.62 (br s, 1 H), 4.66 (br s, 1 H), 3.94 (br s, 1 H), 3.11 (br s, 1 H), 2.83 (br d, 1 H) | 491.0 [M+H]⁺ | 99.2% |
| 8 | (DMSO-d₆) δ 12.22 (br s, 1 H), 8.76 (br s, 1 H), 8.13-8.25 (m, 2 H), 7.97-8.12 (m, 2 H), 7.73 (br d, 1 H), 7.56-7.64 (m, 3 H), 6.60 (br s, 1 H), 4.66 (br s, 1 H), 3.95 (br s, 1 H), 3.11 (br s, 1 H), 2.83 (br d, 1 H) | 491.0 [M+H]⁺ | 99.0% |
| 9 | (CD₃OD) δ 8.26 (s, 1 H), 8.22 (br s, 1 H), 8.01 (s, 1 H), 7.73 (s, 1 H), 7.68 (dd, 1 H), 7.46 (dd, 1 H), 7.23 (td, 1 H), 6.73 (br s, 1 H), 4.68 (br s, 1 H), 3.98 (s, 4 H), 3.03-3.20 (m, 1 H), 2.80-2.92 (m, 1 H) | 450.1 [M+H]⁺ | 97.3% |
| 10 | (CD₃OD) δ 8.26 (s, 1 H), 8.22 (br s, 1 H), 8.01 (s, 1 H), 7.73 (s, 1 H), 7.68 (dd, 1 H), 7.40-7.50 (m, 1 H), 7.23 (td, 1 H), 6.73 (br s, 1 H), 4.67 (br s, 1 H), 3.98 (s, 4 H), 3.07-3.17 (m, 1 H), 2.87 (br dd, 1 H) | 450.2 [M+H]⁺ | 94.3% |
| 11 | (CD₃OD) δ 8.69 (br s, 1 H), 8.23 (br s, 1 H), 7.99 (br s, 2 H), 7.70 (s, 1 H), 7.64 (dd, 1 H), 7.56 (br s, 1 H), 7.42 (br s, 1 H), 7.19 (br t, 1 H), 6.80 (s, 1 H), 4.74-4.85 (m, 1 H), 3.98 (br s, 1 H), 3.20 (br s, 1 H), 2.88 (br d, 1 H) | 431.2 [M+H]⁺ | 97.2% |
| 12 | (CD₃OD) δ 8.54-8.79 (m, 1 H), 8.22 (br s, 1 H), 7.98 (br s, 2 H), 7.69 (s, 1 H), 7.64 (br dd, 1 H), 7.56 (br s, 1 H), 7.42 (br s, 1 H), 7.19 (br t, 1 H), 6.80 (s, 1 H), 4.75-4.86 (m, 1 H), 3.99 (br s, 1 H), 3.20 (br s, 1 H), 2.88 (br d, 1 H) | 431.2 [M+H]⁺ | 88.1% |
| 13 | (CD₃OD) δ 7.83 (br s, 1 H), 7.60-7.76 (m, 2 H), 7.44 (br d, 1 H), 7.21 (td, 1 H), 6.74 (br s, 1 H), 4.54-4.82 (m, 1 H), 3.98 (br s, 1 H), 2.71-3.23 (m, 2 H), 1.84 (br d, 6 H) | 414.2 [M+H]⁺ | 91.5% |
| 14 | (CD₃OD) δ 7.83 (br s, 1 H), 7.61-7.75 (m, 2 H), 7.44 (br d, 1 H), 7.21 (td, 1 H), 6.73 (br s, 1 H), 4.67 (br s, 1 H), 3.99 (br s, 1 H), 2.72-3.24 (m, 2 H), 1.65-1.98 (m, 6 H) | 414.2 [M+H]⁺ | 95.5% |
| 15 | (CD₃OD) δ 7.67 (s, 1 H), 7.58-7.65 (m, 1 H), 7.36-7.41 (m, 1 H), 7.12-7.25 (m, 1 H), 6.78 (br s, 1 H), 5.21 (dd, 0.7 H), 5.04 (dd, 0.3 H), 3.91 (br s, 0.7 H), 3.58 (br s, 0.3 H), 3.11 (br s, 0.8 H), 2.92-3.02 (m, 0.2 H), 2.84 (br d, 1 H), 2.18-2.39 (m, 1 H), 1.15-1.28 (m, 4 H) | 395.1 [M+H]⁺ | 96.3% |
| 16 | (CD₃OD) δ 7.67 (s, 1 H), 7.57-7.65 (m, 1 H), 7.39 (br dd, 1 H), 7.12-7.22 (m, 1 H), 6.80 (br s, 1 H), 5.21 (dd, 0.7 H), 5.04 (dd, 0.3 H), 3.89 (br s, 0.6 H), 3.58 (br s, 0.4 H), 3.06-3.20 (m, 0.8 H), 2.94 (br s, 0.2 H), 2.84 (br d, 1 H), 2.18-2.36 (m, 1 H), 1.15-1.30 (m, 4 H) | 395.1 [M+H]⁺ | 86.1% |
| 17 | (CD₃OD) δ 8.15 (br s, 1 H), 7.66-7.74 (m, 2 H), 7.45 (br d, 1 H), 7.22 (td, 1 H), 6.70 (br s, 1 H), 4.63 (s, 1 H), 3.95 (br s, 1 H), 3.00-3.19 (m, 1 H), 2.85 (br d, 1 H), 1.49 (s, 9 H) | 426.0 [M+H]⁺ | 98.4% |
| 18 | (CD₃OD) δ 8.15 (br s, 1 H), 7.66-7.74 (m, 2 H), 7.45 (br d, 1 H), 7.23 (t, 1 H), 6.68 (br s, 1 H), 4.64 (s, 1 H), 3.93 (s, 1 H), 3.03-3.20 (m, 1 H), 2.87 (br s, 1 H), 1.49 (s, 9 H) | 426.1 [M+H]⁺ | 97.2% |
| 19 | (CD₃OD) δ 8.45 (s, 1 H), 8.34 (s, 1 H), 7.84 (d, 1 H), 7.59-7.70 (m, 2 H), 7.37-7.47 (m, 1 H), 7.32 (d, 1 H), 7.12-7.22 (m, 1 H), 6.73 (s, 1 H), 4.62-4.77 (m, 1 H), 3.88 (br s, 1 H), 3.02-3.15 (m, 1 H), 2.76-2.88 (m, 1 H), 2.37 (s, 3 H) | 417.1 [M+H]⁺ | 99.3% |
| 20 | (CD₃OD) δ 8.45 (s, 1 H), 8.34 (s, 1 H), 7.84 (d, 1 H), 7.57-7.69 (m, 2 H), 7.38-7.45 (m, 1 H), 7.32 (d, 1 H), 7.17 (td, 1 H), 6.73 (br s, 1 H), 4.63-4.76 (m, 1 H), 3.87 (br s, 1 H), 3.01-3.15 (m, 1 H), 2.75-2.87 (m, 1 H), 2.37 (s, 3 H) | 417.1 [M+H] | 98.7% |
| 21 | (CD₃OD) δ 7.63-7.80 (m, 3 H), 7.44 (br d, 1 H), 7.16-7.28 (m, 1 H), 6.71 (br s, 1 H), 4.65-4.80 (m, 1 H), 3.57-4.11 (m, 1 H), 3.00-3.14 (m, 1 H), 2.90 (br s, 1 H), 1.44 (br s, 9 H) | 410.0 [M+H]⁺ | 96.8% |
| 22 | (CD₃OD) δ 7.62-7.79 (m, 3 H), 7.44 (br d, 1 H), 7.22 (td, 1 H), 6.73 (br s, 1 H), 4.70 (br s, 1 H), 3.56-4.10 (m, 1 H), 3.11 (br s, 1 H), 2.90 (br s, 1 H), 1.44 (br s, 9 H) | 410.1 [M+H]⁺ | 97.2% |
| 23 | (CD₃OD) δ 8.40-8.51 (m, 1 H), 7.55-7.73 (m, 2 H), 7.40 (dd, 1 H), 7.19 (td, 1 H), 6.79 (br s, 0.8 H), 6.03-6.20 (m, 0.2 H), 3.81-4.12 (m, 1.8 H), 3.57 (br s, 0.2 H), 2.73-3.03 (m, 2 H) | 422.1 [M+H]⁺ | 91.0% |
| 24 | (CD₃OD) δ 8.37-8.52 (m, 1 H), 7.54-7.72 (m, 2 H), 7.40 (dd, 1 H), 7.18 (td, 1 H), 6.79 (br s, 0.8 H), 6.10 (br s, 0.2 H), 3.84-4.11 (m, 1.7 H), 3.58 (br d, 0.3 H), 2.70-3.06 (m, 2 H) | 422.1 [M+H]⁺ | 92.3% |
| 25 | (CD₃OD) δ 7.64-7.78 (m, 3 H), 7.40-7.54 (m, 1 H), 7.17-7.34 (m, 3 H), 6.89 (br s, 1 H), 5.25 (dd, 0.7 H), 5.14 (dd, 0.3 H), 4.00 (br s, 0.7 H), 3.60-3.78 (m, 0.3 H), 3.20-3.31 (m, 0.7 H), 2.97-3.10 (m, 0.3 H), 2.92 (br d, 1 H) | 467.0 [M+H]⁺ | 90.6% |
| 26 | (CD₃OD) δ 7.61-7.81 (m, 3 H), 7.41-7.57 (m, 1 H), 7.16-7.35 (m, 3 H), 6.89 (br s, 1 H), 5.25 (dd, 0.7 H), 5.14 (dd, 0.3H), 4.00 (br s, 0.7H), 3.56-3.76 (m, 0.3 H), 3.19-3.30 (m, 0.7 H), 2.98-3.10 (m, 0.3 H), 2.92 (br d, 1 H) | 467.0 [M+H]⁺ | 83.6% |
| 27 | (CD₃OD) δ 8.08-8.24 (m, 2 H), 7.68-7.72 (m, 1 H), 7.59-7.67 (m, 1 H), 7.56 (s, 0.3 H), 7.30-7.43 (m, 3 H), 7.15-7.22 (m, 1 H), 6.87 (s, 0.7 H), 5.32 (dd, 0.7 H), 5.10 (dd, 0.3 H), 3.88-4.02 (m, 0.7 H), 3.64 (br s, 0.3 H), 3.13-3.24 (m, 0.6 H), 2.97 (br d, 0.4 H), 2.79-2.93 (m, 1 H) | 449.0 [M+H]⁺ | 96.8% |
| 28 | (CD₃OD) δ 8.06-8.24 (m, 2 H), 7.67-7.71 (m, 1 H), 7.67-7.71 (m, 1 H), 7.59-7.67 (m, 0.3 H), 7.25-7.44 (m, 3 H), 7.14-7.22 (m, 1 H), 6.87 (s, 0.7 H), 5.31 (dd, 0.7 H), 5.10 (br dd, 0.3 H), 3.90-4.00 (m, 0.7 H), 3.58-3.68 (m, 0.3 H), 2.79-3.25 (m, 2 H) | 449.0 [M+H]⁺ | 99.4% |
| 29 | (CD₃OD) δ 8.58 (s, 1 H), 8.41 (br s, 1 H), 7.92 (d, 1 H), 7.63-7.73 (m, 2 H), 7.38-7.48 (m, 2 H), 7.21 (td, 1 H), 6.77 (br s, 1 H), 4.67-4.80 (m, 1 H), 3.90 (br d, 1 H), 3.05-3.20 (m, 1 H), 2.80-2.95 (m, 3 H), 2.63-2.76 (m, 2 H), 2.38 (s, 6 H) | 474.1 [M+H]⁺ | 100% |
| 30 | (CD₃OD) δ 8.61 (s, 1 H), 8.42 (br s, 1 H), 7.93 (d,l H), 7.61-7.75 (m, 2 H), 7.44 (td, 2 H), 7.21 (td, 1 H), 6.77 (br s, 1 H), 4.74 (br d, 1 H), 3.91 (br s, 1 H), 3.05-3.21 (m, 1 H), 2.78-3.04 (m, 5 H), 2.51 (s, 6 H) | 474.1 [M+H]⁺ | 88.4% |
| 31 | (CD₃OD) δ 8.71 (br s, 1 H), 8.20 (br s, 0.7 H), 8.00 (br s, 1.3 H), 7.67 (dt, 2 H), 7.56 (br s, 1 H), 7.43 (br d, 1 H), 7.21 (td, 1 H), 6.75 (br s, 1 H), 4.64-4.87 (m, 1 H), 3.57-4.09 (m, 1 H), 3.26 (br s, 1 H), 2.87 (br d, 1 H), 2.48 (s, 3 H) | 445.0 [M+H]⁺ | 89.8% |
| 32 | (CD₃OD) δ 8.67 (br s, 1 H), 8.17 (br s, 0.4 H), 7.97 (br s, 1.6 H), 7.60-7.71 (m, 2 H), 7.53 (br s, 1 H), 7.40 (br d, 1 H), 7.14-7.22 (m, 1 H), 6.72 (br s, 1 H), 4.62-4.78 (m, 1 H), 3.96 (br s, 1 H), 3.22 (br s, 1 H), 2.85 (br d, 1 H), 2.46 (s, 3 H) | 445.0 [M+H]⁺ | 90.4% |
| 33 | (CD₃OD) δ 8.64-8.73 (m, 1 H), 8.30-8.42 (m, 1 H), 7.82-7.95 (m, 1 H), 7.62-7.76 (m, 2 H), 7.55 (s, 0.3 H), 7.36-7.46 (m, 1 H), 7.12-7.27 (m, 1 H), 6.90 (s, 0.7 H), 5.28 (dd, 0.7 H), 5.07-5.18 (m, 0.3 H), 3.93-4.09 (m, 0.7 H), 3.61-3.73 (m, 0.3 H), 3.17-3.28 (m, 0.7 H), 3.02 (td, 0.3 H), 2.84-2.95 (m, 1 H) | 449.9 [M+H]⁺ | 100% |
| 34 | (CD₃OD) δ 8.66-8.75 (m, 1 H), 8.31-8.42 (m, 1 H), 7.83-7.94 (m, 1 H), 7.62-7.78 (m, 2 H), 7.55 (s, 0.3 H), 7.38-7.45 (m, 1 H), 7.15-7.27 (m, 1 H), 6.91 (s, 0.7 H), 5.28 (dd, 0.7 H), 5.13 (br dd, 0.3 H), 3.92-4.06 (m, 0.7 H), 3.62-3.72 (m, 0.3 H), 3.18-3.28 (m, 0.7 H), 2.98-3.06 (m, 0.3 H), 2.84-2.95 (m, 1 H) | 449.9 [M+H]⁺ | 97.6% |
| 35 | (CD₃OD) δ 8.28 (br s, 1 H), 7.59-7.71 (m, 2 H), 7.41 (br d, 1 H), 7.19 (td, 1 H), 6.73 (br s, 1 H), 4.47 (br s, 1 H), 3.98 (br s, 1 H), 3.07 (br s, 1 H), 2.81 (br d, 1 H), 2.11 (t, 3 H) | 434.1 [M+H]⁺ | 85.8% |
| 36 | (CD₃OD) δ 8.28 (br s, 1 H), 7.58-7.71 (m, 2 H), 7.42 (br d, 1 H), 7.19 (td, 1 H), 6.72 (br s, 1 H), 4.47 (br s, 1 H), 3.97 (br s, 1 H), 3.07 (br s, 1 H), 2.81 (br d, 1 H), 2.11 (t, 3 H) | 434.1 [M+H]⁺ | 88.1% |
| 37 | (CD₃OD) δ 7.41-7.55 (m, 2 H), 7.15-7.32 (m, 1 H), 6.94-7.07 (m, 1 H), 6.65 (s, 0.8 H), 6.19 (s, 0.2 H), 4.82 (br d, 0.3 H), 4.14 (br dd, 0.7 H), 3.51-3.70 (m, 0.8 H), 3.02-3.11 (m, 0.2 H), 2.53-2.84 (m, 2 H), 2.29-2.46 (m, 2 H), 0.84-0.98 (m, 1 H), 0.32-0.45 (m, 2 H), 0.04-0.13 (m, 2 H) | 341.1 [M+H]⁺ | 95.9% |
| 38 | (CD₃OD) δ 7.60-7.69 (m, 2 H), 7.34-7.47 (m, 1 H), 7.18 (td, 1 H), 6.82 (s, 0.8 H), 6.35 (s, 0.2 H), 4.31 (br dd, 1 H), 3.66-3.88 (m, 1 H), 2.72-2.98 (m, 2 H), 2.40-2.65 (m, 2 H), 1.07 (br d, 1 H), 0.49-0.62 (m, 2 H), 0.23 (br d, 2 H) | 341.0 [M+H]⁺ | 92.0% |
| 39 | (CD₃OD) δ 8.69-8.78 (m, 1 H), 8.22-8.32 (m, 1 H), 8.00-8.10 (m, 1 H), 7.66-7.73 (m, 1 H), 7.58-7.66 (m, 2 H), 7.52 (br s, 0.3 H), 7.32-7.44 (m, 1 H), 7.09-7.24 (m, 1 H), 6.87 (s, 0.7 H), 5.04-5.32 (m, 1 H), 3.54-4.05 (m, 1 H), 2.94-3.26 (m, 1 H), 2.81-2.94 (m, 1 H) | 432.1 [M+H]⁺ | 97.1% |
| 40 | (CD₃OD) δ 8.69-8.77 (m, 1 H), 8.21-8.31 (m, 1 H), 8.00-8.09 (m, 1 H), 7.67-7.73 (m, 1 H), 7.58-7.66 (m, 2 H), 7.52 (br s, 0.3 H), 7.34-7.43 (m, 1 H), 7.10-7.22 (m, 1 H), 6.87 (s, 0.7 H), 5.05-5.32 (m, 1 H), 3.55-4.03 (m, 1 H), 2.93-3.25 (m, 1 H), 2.79-2.92 (m, 1 H) | 432.1 [M+H]⁺ | 98.4% |
| 41 | (CD₃OD) δ 8.12 (s, 1 H), 7.59-7.68 (m, 2 H), 7.39 (dd, 1 H), 7.17 (td, 1 H), 6.64 (br s, 1 H), 4.76-4.83 (m, 1 H), 3.89 (br s, 1 H), 3.06 (br s, 1 H), 2.82 (br d, 1 H), 2.38 (br d, 1 H), 0.93-0.98 (m, 4 H) | 394.0 [M+H]⁺ | 95.4% |
| 42 | (CD₃OD) δ 8.13 (s, 1 H), 7.60-7.69 (m, 2 H), 7.39 (dd, 1 H), 7.17 (td, 1 H), 6.67 (br s, 1 H), 4.79-4.84 (m, 1 H), 3.87 (br s, 1 H), 3.05 (br s, 1 H), 2.82 (br d, 1 H), 2.37 (quin, 1 H), 0.93-0.98 (m, 4 H) | 394.0 [M+H]⁺ | 98.3% |
| 43 | (CD₃OD) δ 8.67 (br s, 1 H), 8.17 (br s, 1 H), 7.99 (br s, 1 H), 7.65-7.75 (m, 2 H), 7.56 (br s, 1 H), 7.45 (dd, 1 H), 7.23 (td, 1 H), 6.78 (br s, 1 H), 4.71-4.87 (m, 1 H), 3.73-4.16 (m, 1 H), 3.22 (br s, 1 H), 2.90 (br d, 1 H), 2.38-2.55 (m, 1 H), 1.01-1.16 (m, 4 H) | 471.1 [M+H]⁺ | 92.7% |
| 44 | (CD₃OD) δ 8.67 (br s, 1 H), 7.87-8.27 (m, 2 H), 7.66-7.74 (m, 2H), 7.56 (br s, 1 H), 7.45 (dd, 1 H), 7.23 (td, 1 H), 6.78 (br s, 1 H), 4.63-4.65 (m, 1 H), 3.99 (s, 1 H), 3.23 (br s, 1 H), 2.90 (br d, 1 H), 2.38-2.59 (m, 1 H), 1.00-1.15 (m, 4 H) | 471.2 [M+H]⁺ | 91.5% |
| 45 | (CD₃OD) δ 7.53-7.73 (m, 2 H), 7.40 (dd, 1 H), 7.18 (td, 1 H), 5.87-6.84 (m, 1 H), 3.53-4.07 (m, 2 H), 2.67-3.16 (m, 2 H), 1.50-1.69 (m, 6 H) | 480.0 [M+H]⁺ | 85.3% |
| 46 | (CD₃OD) δ 7.57-7.69 (m, 2 H), 7.40 (dd, 1 H), 7.18 (td, 1 H), 6.01-6.87 (m, 1 H), 3.49-4.07 (m, 2 H), 2.72-3.13 (m, 2 H), 1.51-1.71 (m, 6 H) | 480.0 [M+H]⁺ | 85.7% |
| 47 | (CD₃OD) δ 8.55 (s, 1 H), 8.37 (br s, 1 H), 7.89 (d, 1 H), 7.61-7.67 (m, 2 H), 7.36-7.44 (m, 2 H), 7.18 (td, 1 H), 6.72 (br s, 1 H), 4.70 (br d, 1 H), 3.72-4.09 (m, 1 H), 3.10 (br s, 1 H), 2.78-2.93 (m, 3 H), 2.62-2.72 (m, 2 H), 2.34 (s, 6 H) | 474.2 [M+H]⁺ | 83.9% |
| 48 | (CD₃OD) δ 8.54 (s, 1 H), 8.37 (br s, 1 H), 7.88 (d, 1 H), 7.61-7.68 (m, 2 H), 7.35-7.44 (m, 2 H), 7.18 (td, 1 H), 6.73 (br s, 1 H), 4.70 (br d, 1 H), 3.76-4.02 (m, 1 H), 3.04-3.16 (m, 1 H), 2.77-2.91 (m, 3 H), 2.60-2.69 (m, 2 H), 2.31 (s, 6 H) | 474.2 [M+H]⁺ | 95.7% |
| 49 | (CD₃OD) δ 8.71 (br d, 0.7 H), 8.46 (br s, 0.3 H), 8.28 (d, 0.7 H), 8.00-8.12 (m, 1 H), 7.92 (br s, 0.3 H), 7.48-7.78 (m, 3 H), 7.35-7.47 (m, 1 H), 7.20 (td, 1 H), 6.20-6.92 (m, 1 H), 4.92-5.08 (m, 0.3 H), 4.22 (br dd, 0.7 H), 3.60-4.07 (m, 1 H), 2.95-3.16 (m, 1 H), 2.82 (br d, 1 H) | 499.0 [M+H]⁺ | 97.2% |
| 50 | (CD₃OD) δ 8.71 (br d, 0.8 H), 8.46 (br s, 0.2 H), 8.28 (d, 0.7 H), 8.04 (br t, 1 H), 7.93 (br s, 0.3 H), 7.48-7.78 (m, 3.3 H), 7.42 (br d, 1.2 H), 7.20 (td, 1 H), 6.24-6.92 (m, 0.5 H), 4.94-5.10 (m, 0.3 H), 4.22 (br dd, 0.7 H), 3.59-4.07 (m, 1 H), 2.93-3.17 (m, 1 H), 2.84 (br s, 1 H) | 499.0 [M+H]⁺ | 77.6% |
| 51 | (CD₃OD) δ 8.47 (br s, 1 H), 7.63-7.73 (m, 2 H), 7.43 (dd, 1 H), 6.99-7.29 (m, 2 H), 6.60-6.89 (m, 1 H), 4.45-4.66 (m, 1 H), 3.49-4.05 (m, 1 H), 2.96-3.20 (m, 1 H), 2.86 (br d, 1 H) | 403.9 [M+H]⁺ | 90.8% |
| 52 | (CD₃OD) δ 8.47 (br s, 1 H), 7.63-7.73 (m, 2 H), 7.38-7.63 (m, 1 H), 6.96-7.30 (m, 2 H), 6.77 (br s, 1 H), 4.56 (br d, 1 H), 3.46-4.04 (m, 1 H), 3.11 (br s, 1 H), 2.86 (br d, 1 H) | 404.0 [M+H]⁺ | 82.8% |
| 53 | (CD₃OD) δ 7.64-7.75 (m, 2 H), 7.44 (br d, 1 H), 6.93-7.28 (m, 2 H), 6.71 (br s, 1 H), 4.53-4.61 (m, 1 H), 3.54-4.04 (m, 1 H), 3.07 (br s, 1 H), 2.88 (br s, 1 H), 2.26 (br s, 1 H), 1.21 (br s, 4 H) | 444.2 [M+H]⁺ | 95.0% |
| 54 | (CD₃OD) δ 7.52-7.61 (m, 2 H), 7.32 (br d, 1 H), 6.81-7.16 (m, 2 H), 6.60 (br s, 1 H), 4.37-4.48 (m, 1 H), 3.46-3.92 (m, 1 H), 2.94 (br s, 1 H), 2.75 (br d, 1 H), 2.14 (br s, 1 H), 1.09 (br s, 4 H) | 444.1 [M+H]⁺ | 70.9% |
| 55 | (CD₃OD) δ 7.60-7.70 (m, 2 H), 7.34-7.48 (m, 1 H), 7.16-7.24 (m, 1 H), 6.61-6.92 (m, 1 H), 4.99 (br dd, 0.3 H), 4.47 (dd, 0.7 H), 3.42-3.88 (m, 1 H), 2.68-3.21 (m, 4 H), 2.03-2.34 (m, 4 H), 1.57-1.86 (m, 2 H) | 371.2 [M+H]⁺ | 98.6% |
| 56 | (CD₃OD) δ 7.62-7.68 (m, 2 H), 7.38-7.47 (m, 1 H), 7.16-7.24 (m, 1 H), 6.58-6.94 (m, 1 H), 4.99 (br dd, 0.3 H), 4.47 (dd, 0.7 H), 3.73-3.83 (m, 1 H), 2.64-3.17 (m, 4 H), 2.03-2.30 (m, 4 H), 1.61-1.85 (m, 2 H) | 371.2 [M+H]⁺ | 92.4% |
| 57 | (CD₃OD) δ 8.44-8.51 (m, 1 H), 7.69 (br s, 1 H), 7.55 (d, 1 H), 7.38-7.48 (m, 1 H), 7.22 (d, 1 H), 6.81 (br d, 0.6 H), 6.06 (br s, 0.2 H), 4.93 (br d, 0.7 H), 3.80-4.06 (m, 1 H), 3.43-3.68 (m, 0.4 H), 2.95 (br dd, 1 H), 2.72-2.89 (m, 1 H), 2.49 (s, 3 H) | 418.1 [M+H]⁺ | 93.1% |
| 58 | (CD₃OD) δ 8.43-8.51 (m, 1 H), 7.69 (s, 1 H), 7.54 (d, 1 H), 7.38-7.47 (m, 1 H), 7.22 (d, 1 H), 6.82 (br s, 1 H), 6.07 (br s, 0.4 H), 4.89-5.05 (m, 0.1 H), 3.86-3.97 (m, 0.8 H), 3.85-4.03 (m, 1 H), 3.45-3.62 (m, 0.3 H), 2.91-3.03 (m, 1 H), 2.76-2.90 (m, 1 H), 2.49 (s, 3 H) | 418.2 [M+H]⁺ | 92.5% |
| 59 | (CD₃OD) δ 7.48-7.60 (m, 2 H), 7.24-7.36 (m, 1 H), 7.02-7.14 (m, 1 H), 6.67 (s, 0.8 H), 6.28 (s, 0.2 H), 5.05-5.15 (m, 0.5 H), 4.90-5.01 (m, 0.5 H), 4.63-4.72 (m, 0.3 H), 4.21 (dd, 0.7 H), 3.59-3.71 (m, 0.8 H), 3.01-3.15 (m, 0.2 H), 2.56-2.90 (m, 5 H), 1.97-2.50 (m, 4 H) | 373.2 [M+H]⁺ | 96.5% |
| 60 | (CD₃OD) δ 7.55-7.69 (m, 2 H), 7.28-7.47 (m, 1 H), 7.06-7.23 (m, 1 H), 6.75 (br s, 0.7 H), 6.35 (br s, 0.3 H), 5.18 (br t, 0.5 H), 4.96-5.10 (m, 0.5 H), 4.29 (br dd, 1 H), 3.74 (br s, 1 H), 2.65-3.00 (m, 5 H), 2.00-2.48 (m, 4 H) | 373.2 [M+H]⁺ | 99.0% |
| 61 | (CD₃OD) δ 7.56-7.73 (m, 2 H), 7.31-7.49 (m, 1 H), 7.11-7.26 (m, 1 H), 6.87 (s, 0.8 H), 6.67 (s, 0.2 H), 4.96 (br dd, 0.3 H), 4.44-4.54 (m, 0.7 H), 3.80-3.94 (m, 0.7 H), 3.36-3.45 (m, 0.3 H), 3.10-3.25 (m, 1 H), 2.73-2.99 (m, 2 H), 1.97-2.20 (m, 1 H), 1.69-1.94 (m, 1 H) | 363.0 [M+H]⁺ | 99.4% |
| 62 | (CD₃OD) δ 7.56-7.75 (m, 2 H), 7.35-7.51 (m, 1 H), 7.12-7.25 (m, 1 H), 6.71 (s, 0.7 H), 6.45 (s, 0.3 H), 4.91-4.97 (m, 0.3 H), 4.50 (dd, 0.7 H), 3.82-4.04 (m, 0.7 H), 3.11-3.29 (m, 1.3 H), 2.93-3.10 (m, 0.7 H), 2.68-2.91 (m, 1.3 H), 2.01-2.30 (m, 1 H), 1.73-1.99 (m, 1 H) | 363.0 [M+H]⁺ | 99.2% |
| 63 | (CD₃OD) δ 8.35-8.60 (m, 1 H), 7.70 (s, 1 H), 7.35-7.57 (m, 2 H), 7.25 (d, 1 H), 6.83 (s, 0.8 H), 6.09 (br s, 0.2 H), 4.95 (br d, 0.2 H), 3.84-4.11 (m, 1.6 H), 3.58 (br d, 0.2 H), 2.76-3.06 (m, 2 H), 2.46 (s, 3 H) | 418.0 [M+H]⁺ | 100% |
| 64 | (CD₃OD) δ 8.39-8.57 (m, 1 H), 7.71 (s, 1 H), 7.40-7.58 (m, 2 H), 7.25 (d, 1 H), 6.83 (s, 0.8 H), 6.09 (br s, 0.2 H), 4.93-5.00 (m, 0.3 H), 3.97-4.05 (m, 0.7 H), 3.84-3.95 (m, 0.8 H), 3.57 (br s, 0.2 H), 2.77-3.02 (m, 2 H), 2.46 (s, 3 H) | 418.0 [M+H]⁺ | 98.8% |
| 65 | (CD₃OD) δ 8.32 (br s, 1 H), 8.12 (d, 1 H), 7.85 (d, 1 H), 7.63-7.71 (m, 2 H), 7.39-7.49 (m, 2 H), 7.20 (td, 1 H), 6.77 (br s, 1 H), 4.75 (br dd, 1 H), 3.92 (br s, 1 H), 3.18-3.26 (m, 4 H), 3.06-3.17 (m, 1 H), 2.85 (br dd, 3.0 Hz, 1 H), 2.61-2.73 (m, 4 H), 2.39 (s, 3 H) | 501.1 [M+H]⁺ | 86.6% |
| 66 | (CD₃OD) δ 8.33 (br s, 1 H), 8.13 (d, 1 H), 7.86 (d, 1 H), 7.61-7.73 (m, 2 H), 7.39-7.49 (m, 2 H), 7.21 (td, 1 H), 6.77 (br s, 1 H), 4.75 (br dd, 1 H), 3.92 (br s, 1 H), 3.19-3.27 (m, 4 H), 3.06-3.17 (m, 1 H), 2.85 (br dd, 1 H), 2.66-2.75 (m, 4 H), 2.42 (s, 3 H) | 501.1 [M+H]⁺ | 85.6% |
| 67 | (CD₃OD) δ 8.30-8.42 (m, 1 H), 7.99-8.14 (m, 1 H), 7.49-7.73 (m, 2.3 H), 7.32-7.45 (m, 1 H), 7.10-7.27 (m, 1 H), 6.85 (br s, 0.7 H), 5.30 (br dd, 0.8 H), 5.08 (br dd, 0.2 H), 3.83-4.06 (m, 4 H), 3.61 (br s, 0.4 H), 3.10-3.23 (m, 0.6 H), 2.76-3.03 (m, 1 H) | 435.0 [M+H]⁺ | 100% |
| 68 | (CD₃OD) δ 8.28-8.43 (m, 1 H), 7.97-8.14 (m, 1 H), 7.51-7.72 (m, 2.3 H), 7.31-7.46 (m, 1 H), 7.11-7.28 (m, 1 H), 6.85 (br s, 0.7 H), 5.30 (br dd, 0.7 H), 5.08 (br dd, 0.3 H), 3.85-4.03 (m, 4 H), 3.59 (br d, 0.3 H), 3.10-3.22 (m, 0.7 H), 2.78-3.01 (m, 1 H) | 435.0 [M+H]⁺ | 82.8% |
| 69 | (CD₃OD) δ 8.82 (dd, 1 H), 8.32 (br s, 1 H), 7.79 (d, 1 H), 7.52-7.59 (m, 2 H), 7.39-7.52 (m, 1 H), 7.26-7.39 (m, 1 H), 7.09 (td, 1 H), 6.65 (br s, 1 H), 4.60 (br d, 1 H), 3.80 (br s, 1 H), 2.92-3.10 (m, 1 H), 2.73 (dd, 1 H) | 482.9 [M+H]⁺ | 96.7% |
| 70 | (CD₃OD) δ 8.78-8.82 (m, 1 H), 8.31 (br s, 1 H), 7.78 (d, 1 H), 7.52-7.59 (m, 2 H), 7.42 (d, 1 H), 7.26-7.38 (m, 1 H), 7.08 (td, 1 H), 6.65 (br s, 1 H), 4.59 (br d, 1 H), 3.81 (br s, 1 H), 2.93-3.08 (m, 1 H), 2.73 (dd, 1 H) | 482.9 [M+H]⁺ | 94.1% |
| 71 | (CD₃OD) δ 8.78 (s, 1 H), 8.31 (br s, 1 H), 7.99 (s, 1 H), 7.79-7.89 (m, 2 H), 7.51-7.63 (m, 3 H), 7.34 (dd, 1 H), 7.10 (td, 1 H), 6.67 (br s, 1 H), 4.64 (br d, 1 H), 3.85 (s, 4 H), 2.93-3.09 (m, 1 H), 2.74 (br dd, 1 H) | 483.0 [M+H]⁺ | 94.5% |
| 72 | (CD₃OD) δ 8.77 (s, 1 H), 8.30 (br s, 1 H), 7.99 (s, 1 H), 7.77-7.91 (m, 2 H), 7.51-7.63 (m, 3 H), 7.34 (dd, 1 H), 7.10 (td, 1 H), 6.67 (br s, 1 H), 4.64 (br d, 1 H), 3.85 (s, 4 H), 2.95-3.10 (m, 1 H), 2.74 (br dd, 1 H) | 483.0 [M+H]⁺ | 91.1% |
| 73 | (CD₃OD) δ 8.15-8.28 (m, 2 H), 7.75 (d, 1 H), 7.52-7.59 (m, 2 H), 7.32 (dd, 1 H), 7.06-7.15 (m, 2 H), 6.65 (br s, 1 H), 4.63 (br dd, 1 H), 3.78 (s, 4 H), 2.94-3.07 (m, 1 H), 2.73 (br dd, 1 H) | 433.0 [M+H]⁺ | 92.5% |
| 74 | (CD₃OD) δ 8.16-8.27 (m, 2 H), 7.75 (d, 1 H), 7.52-7.59 (m, 2 H), 7.32 (dd, 1 H), 7.00-7.16 (m, 2 H), 6.65 (br s, 1 H), 4.63 (br dd, 1 H), 3.78 (s, 4 H), 2.96-3.06 (m, 1 H), 2.73 (br dd, 1 H) | 433.0 [M+H]⁺ | 86.6% |
| 75 | (CD₃OD) δ 7.94-8.19 (m, 2 H), 7.63-7.76 (m, 2 H), 7.50-7.61 (m, 1.4 H), 7.38-7.48 (m, 1 H), 7.15-7.29 (m, 1 H), 6.90 (br s, 0.6 H), 5.35 (dd, 0.7 H), 5.14 (dd, 0.3 H), 4.00 (br s, 0.7 H), 3.68 (br s, 0.3 H), 3.18-3.28 (m, 0.7 H), 3.01 (br d, 0.3 H), 2.92 (br d, 1 H) | 467.1 [M+H]⁺ | 100% |
| 76 | (CD₃OD) δ 8.11 (br s, 2 H), 7.72-7.82 (m, 1 H), 7.64-7.71 (m, 1 H), 7.51-7.63 (m, 1.3 H), 7.40-7.49 (m, 1 H), 7.17-7.29 (m, 1 H), 6.92 (s, 0.7 H), 5.36 (dd, 0.7 H), 5.15 (dd, 0.3 H), 4.01 (br t, 0.7 H), 3.62-3.77 (m, 0.3 H), 3.14-3.31 (m, 0.7 H), 2.98-3.12 (m, 0.3 H), 2.81-2.98 (m, 1 H) | 467.1 [M+H]⁺ | 83.2% |
| 77 | (DMSO-d₆) δ 12.25 (br s, 1 H), 7.89-8.05 (m, 1 H), 7.83 (dd, 1 H), 7.58-7.71 (m, 2 H), 7.23-7.43 (m, 3 H), 6.61-7.20 (m, 1 H), 4.82-5.17 (m, 1 H), 3.59-4.07 (m, 1 H), 2.79-3.12 (m, 2 H), 2.60-2.69 (m, 3 H) | 463.1 [M+H]⁺ | 65.4% |
| 78 | (DMSO-d₆) δ 12.18-12.29 (m, 1 H), 7.91-8.04 (m, 1 H), 7.83 (dd, 1 H), 7.60-7.71 (m, 2 H), 7.25-7.43 (m, 3 H), 6.63-7.19 (m, 1 H), 4.86-5.16 (m, 1 H), 3.65-4.05 (m, 1 H), 2.82-3.11 (m, 2 H), 2.59-2.70 (m, 3 H) | 463.1 [M+H]⁺ | 76.6% |
| 79 | (CD₃OD) δ 8.69 (s, 1 H), 8.43 (br s, 1 H), 8.05 (s, 1 H), 7.58-7.76 (m, 2 H), 7.32-7.53 (m, 1 H), 7.20 (td, 1 H), 6.61-6.85 (m, 1 H), 4.54-4.79 (m, 1 H), 3.89 (br s, 1 H), 3.06-3.20 (m, 1 H), 2.96-3.03 (m, 2 H), 2.84 (br dd, 1 H), 2.72 (dd, 2 H), 2.41 (s, 6 H) | 508.1 [M+H]⁺ | 95.7% |
| 80 | (CD₃OD) δ 8.70 (s, 1 H), 8.44 (br s, 1 H), 8.05 (s, 1 H), 7.61-7.77 (m, 2 H), 7.43 (dd, 1 H), 7.20 (td, 1 H), 6.74 (br s, 1 H), 4.72 (br d, 1 H), 3.90 (br s, 1 H), 3.06-3.20 (m, 1 H), 2.96-3.06 (m, 2 H), 2.84 (br dd, 1 H), 2.73 (dd, 2 H), 2.42 (s, 6 H)) | 508.0 [M+H]⁺ | 97.8% |
| 81 | (CD₃OD) δ 9.34 (s, 1 H), 8.69 (d, 1 H), 8.52 (s, 1 H), 8.06-8.17 (m, 2 H), 7.91-8.05 (m, 3 H), 7.69 (dd, 1 H), 7.38-7.52 (m, 2 H), 7.23 (td, 1 H), 6.90 (s, 1 H), 4.78 (br dd, 1 H), 3.91 (br s, 1 H), 3.10-3.24 (m, 1 H), 2.90 (br dd, 1 H) | 480.4 [M+H]⁺ | 96.0% |
| 82 | (CD₃OD) δ 9.35 (s, 1 H), 8.70 (d, 1 H), 8.53 (s, 1 H), 8.06-8.28 (m, 3 H), 7.91-8.04 (m, 2 H), 7.70 (dd, 1 H), 7.36-7.55 (m, 2 H), 7.24 (td, 1 H), 6.93 (s, 1 H), 4.79 (br dd, 1 H), 3.90 (br s, 1 H), 3.12-3.26 (m, 1 H), 2.83-2.98 (m, 1 H) | 480.4 [M+H]⁺ | 98.7% |
| 83 | (CD₃OD) δ 8.03-8.26 (m, 1 H), 7.57-7.78 (m, 2 H), 7.49 (br s, 0.2 H), 7.35-7.43 (m, 1 H), 7.13-7.32 (m, 3 H), 6.86 (br s, 0.7 H), 5.25 (br dd, 0.7 H), 5.10 (br dd, 0.4 H), 3.87-4.05 (m, 0.8 H), 3.63 (br s, 0.4 H), 2.79-3.27 (m, 2 H) | 467.0 [M+H]⁺ | 98.4% |
| 84 | (CD₃OD) δ 8.06-8.33 (m, 1 H), 7.57-7.73 (m, 2 H), 7.50 (br s, 0.3 H), 7.35-7.44 (m, 1 H), 7.13-7.33 (m, 3 H), 6.87 (br s, 0.7 H), 5.26 (br dd, 0.7 H), 5.10 (br dd, 0.4 H), 3.95 (br t, 0.8 H), 3.62 (br d, 0.4 H), 2.78-3.27 (m, 2 H) | 467.0 [M+H]⁺ | 100% |
| 85 | (CD₃OD) δ 8.42-8.82 (m, 1 H), 7.86-8.39 (m, 2 H), 7.65-7.77 (m, 2 H), 7.40-7.64 (m, 2 H), 7.05-7.38 (m, 2 H), 6.82 (s, 1 H), 5.00 (br s, 0.3 H), 4.76 (br d, 0.7 H), 3.57-4.09 (m, 1 H), 2.97-3.30 (m, 1 H), 2.91 (br d, 1 H) | 481.0 [M+H]⁺ | 98.9% |
| 86 | (CD₃OD) δ 8.43-8.81 (m, 1 H), 7.87-8.38 (m, 2 H), 7.65-7.77 (m, 2 H), 7.40-7.65 (m, 2 H), 7.02-7.38 (m, 2 H), 6.82 (br s, 1 H), 5.01 (br s, 0.3 H), 4.76 (br d, 0.7 H), 3.56-4.10 (m, 1 H), 2.97-3.29 (m, 1 H), 2.91 (br d, 1 H) | 481.0 [M+H]⁺ | 96.9% |
| 87 | (CD₃OD) δ 7.84-8.47 (m, 2 H), 7.58-7.77 (m, 2 H), 6.95-7.53 (m, 3 H), 6.77 (s, 1 H), 4.71 (br s, 1 H), 3.98 (br s, 4 H), 2.63-3.23 (m, 2 H) | 484.1 [M+H]⁺ | 99.2% |
| 88 | (CD₃OD) δ 7.86-8.60 (m, 2 H), 7.60-7.85 (m, 2 H), 6.95-7.58 (m, 3 H), 6.77 (s, 1 H), 4.52-4.80 (m, 1 H), 3.98 (br s, 4 H), 2.76-3.26 (m, 2 H) | 484.1 [M+H]⁺ | 98.8% |
| 89 | (CD₃OD) δ 8.16-8.37 (m, 2 H), 7.66-7.95 (m, 2 H), 7.43-7.65 (m, 2.4 H), 7.22-7.37 (m, 1 H), 6.91-7.20 (m, 1 H), 6.80 (s, 0.6 H), 4.98-5.33 (m, 1 H), 3.47-3.95 (m, 1 H), 2.70-3.14 (m, 2 H) | 498.9 [M+H]⁺ | 99.1% |
| 90 | (CD₃OD) δ 8.21-8.42 (m, 2 H), 7.81-7.98 (m, 2 H), 7.50-7.75 (m, 2.4 H), 7.34-7.48 (m, 1 H), 7.09-7.24 (m, 1 H), 6.88 (s, 0.6 H), 5.06-5.37 (m, 1 H), 3.57-4.02 (m, 1 H), 2.69-3.23 (m, 2 H) | 499.0 [M+H]⁺ | 99.1% |
| 91 | (CD₃OD) δ 8.26 (br s, 1 H), 7.94 (d, 1 H), 7.83 (d, 1 H), 7.63-7.69 (m, 2 H), 7.41 (ddd, 2 H), 7.20 (td, 1 H), 6.76 (br s, 1 H), 4.75 (br dd, 1 H), 3.89 (br s, 1 H), 3.07-3.16 (m, 1 H), 2.96 (s, 6 H), 2.83 (br dd, 1 H) | 446.2 [M+H]⁺ | 90.1% |
| 92 | (CD₃OD) δ 8.26 (br s, 1 H), 7.93 (s, 1 H), 7.82 (d, 1 H), 7.63-7.68 (m, 2 H), 7.36-7.44 (m, 2 H), 7.19 (td, 1 H), 6.75 (s, 1 H), 4.74 (br dd, 1 H), 3.89 (br s, 1 H), 3.06-3.16 (m, 1 H), 2.95 (s, 6 H), 2.79-2.87 (m, 1 H) | 446.2 [M+H]⁺ | 91.6% |
| 93 | (CD₃OD) δ 7.58-7.88 (m, 4 H), 7.39-7.51 (m, 1 H), 7.20 (td, 1 H), 6.23-6.92 (m, 2 H), 4.28 (br d, 1 H), 3.93 (br s, 1 H), 2.94-3.11 (m, 1 H), 2.80 (br d, 1 H) | 403.0 [M+H]⁺ | 98.6% |
| 94 | (CD₃OD) δ 7.56-7.88 (m, 4 H), 7.39-7.53 (m, 1 H), 7.21 (td, 1 H), 6.25-6.95 (m, 2 H), 4.17-4.38 (m, 1 H), 3.93 (br s, 1 H), 2.95-3.09 (m, 1 H), 2.80 (br d, 1 H) | 403.0 [M+H]⁺ | 97.7% |
| 95 | (CD₃OD) δ 8.06 (br s, 1 H), 7.55-7.73 (m, 2 H), 7.42 (br d, 1 H), 7.19 (td, 1 H), 6.64-7.05 (m, 2 H), 4.12-4.39 (m, 1 H), 3.98 (s, 3 H), 3.63-3.91 (m, 1 H), 2.88-3.07 (m, 1 H), 2.76 (br dd, 1 H) | 417.1 [M+H]⁺ | 97.1% |
| 96 | (CD₃OD) δ 7.90-8.05 (m, 2 H), 7.71 (s, 1 H), 7.36-7.58 (m, 2 H), 6.69-7.12 (m, 2 H), 4.16 (br d, 1 H), 3.97 (s, 3 H), 3.45-3.81 (m, 1 H), 2.87-3.05 (m, 1 H), 2.74 (br dd, 1 H) | 417.1 [M+H]⁺ | 96.2% |
| 97 | (CD₃OD) δ 8.75-8.81 (m, 1 H), 8.27-8.35 (m, 1 H), 8.05-8.13 (m, 1 H), 7.63-7.76 (m, 2 H), 7.45-7.59 (m, 2 H), 7.20-7.32 (m, 1 H), 6.92 (s, 1 H), 5.27 (dd, 0.7 H), 5.12 (dd, 0.3 H), 3.93-4.03 (m, 0.7 H), 3.57-3.72 (m, 0.3 H), 2.81-3.29 (m, 2 H), 2.44-2.49 (m, 3 H) | 428.2 [M+H]⁺ | 100% |
| 98 | (CD₃OD) δ 8.69-8.80 (m, 1 H), 8.27-8.34 (m, 1 H), 8.02-8.12 (m, 1 H), 7.63-7.76 (m, 2 H), 7.45-7.59 (m, 2 H), 7.20-7.30 (m, 1 H), 6.92 (s, 1 H), 5.27 (dd, 0.7 H), 5.12 (dd, 0.3 H), 3.92-4.03 (m, 0.7 H), 3.64 (td, 0.3 H), 2.85-3.29 (m, 2 H), 2.41-2.49 (m, 3 H) | 428.2 [M+H]⁺ | 98.3% |
| 99 | (CD₃OD) δ 8.67-8.73 (m, 1 H), 8.33-8.41 (m, 1 H), 7.85-7.93 (m, 1 H), 7.70-7.75 (m, 1 H), 7.45-7.58 (m, 2 H), 7.21-7.30 (m, 1 H), 6.91 (s, 1 H), 5.28 (dd, 0.7 H), 5.12 (dd, 0.3 H), 3.86-4.05 (m, 0.7 H), 3.56-3.71 (m, 0.3 H), 2.83-3.29 (m, 2 H), 2.41-2.50 (m, 3 H) | 446.2 [M+H]⁺ | 100% |
| 100 | (CD₃OD) δ 8.56-8.76 (m, 1 H), 8.21-8.45 (m, 1 H), 7.80-7.96 (m, 1 H), 7.73 (br s, 1 H), 7.36-7.60 (m, 2 H), 7.19-7.31 (m, 1 H), 6.91 (br s, 1 H), 5.20-5.33 (m, 0.7 H), 5.12 (br d, 0.3 H), 3.97 (br t, 0.7 H), 3.58-3.73 (m, 0.3 H), 2.83-3.28 (m, 2 H), 2.43-2.55 (m, 3 H) | 446.2 [M+H]⁺ | 98.5% |
| 101 | (CD₃OD) δ 8.70-8.78 (m, 1 H), 8.22-8.30 (m, 1 H), 8.01-8.09 (m, 1 H), 7.39-7.73 (m, 4 H), 7.14-7.22 (m, 1 H), 6.89 (s, 1 H), 5.24 (dd, 0.7 H), 5.09 (br dd, 0.3 H), 3.95 (ddd, 0.7 H), 3.55-3.66 (m, 0.3 H), 3.15-3.26 (m, 0.7 H), 2.95-3.06 (m, 0.3 H), 2.80-2.93 (m, 1 H), 2.43-2.49 (m, 3 H) | 428.1 [M+H]⁺ | 97.2% |
| 102 | (CD₃OD) δ 8.72-8.78 (m, 1 H), 8.24-8.31 (m, 1 H), 8.02-8.10 (m, 1 H), 7.40-7.73 (m, 4 H), 7.16-7.23 (m, 1 H), 6.90 (s, 1 H), 5.25 (dd, 0.7 H), 5.09 (br dd, 0.3 H), 3.95 (ddd, 0.7 H), 3.56-3.66 (m, 0.3 H), 3.16-3.27 (m, 0.7 H), 2.96-3.07 (m, 0.3 H), 2.82-2.93 (m, 1 H), 2.43-2.50 (m, 3 H) | 428.1 [M+H]⁺ | 97.0% |
| 103 | (CD₃OD) δ 8.30-8.51 (m, 1 H), 8.04-8.19 (m, 1 H), 7.72 (s, 1 H), 7.43-7.65 (m, 2.4 H), 7.18-7.29 (m, 1 H), 6.89 (br s, 0.6 H), 5.32 (dd, 0.7 H), 5.10 (dd, 0.3 H), 3.91-4.08 (m, 3.8 H), 3.61 (br s, 0.2 H), 3.15-3.25 (m, 0.6 H), 2.86-3.07 (m, 1.4 H), 2.47-2.54 (m, 3 H) | 431.1 [M+H]⁺ | 100% |
| 104 | (CD₃OD) δ 8.35-8.47 (m, 1 H), 8.06-8.16 (m, 1 H), 7.72 (s, 1 H), 7.44-7.64 (m, 2.4 H), 7.20-7.27 (m, 1 H), 6.89 (br s, 0.6 H), 5.32 (dd, 0.7 H), 5.10 (dd, 0.3 H), 3.90-4.04 (m, 3.8 H), 3.61 (br s, 0.3 H), 3.16-3.25 (m, 0.6 H), 2.84-3.06 (m, 1.4 H), 2.46-2.54 (m, 3 H) | 431.1 [M+H]⁺ | 99.3% |
| 105 | (CD₃OD) δ 7.79-7.89 (m, 1 H), 7.50-7.78 (m, 2.3 H), 7.36-7.49 (m, 1 H), 7.10-7.28 (m, 1 H), 6.79-7.06 (m, 1.7 H), 5.31 (dd, 0.7 H), 5.13 (dd, 0.3 H), 3.84-4.12 (m, 3.7 H), 3.49-3.75 (m, 0.3 H), 2.81-3.26 (m, 2 H) | 435.1 [M+H]⁺ | 96.4% |
| 106 | (CD₃OD) δ 7.78-7.90 (m, 1 H), 7.48-7.77 (m, 2.3 H), 7.36-7.46 (m, 1 H), 7.11-7.26 (m, 1 H), 6.76-7.06 (m, 1.7 H), 5.31 (dd, 0.7 H), 5.13 (dd, 0.3 H), 3.89-4.11 (m, 3.7 H), 3.65 (br s, 0.3 H), 2.71-3.26 (m, 2 H) | 435.2 [M+H]⁺ | 97.4% |
| 107 | Data provided above | | |
| 108 | Data provided above | | |
| 109 | (CD₃OD) δ 7.54-7.76 (m, 2 H), 7.42 (dd, 1 H), 7.17-7.28 (m, 1.3 H), 6.56-7.16 (m, 1.7 H), 4.67 (br s, 2 H), 4.55 (br s, 1 H), 3.94 (br s, 1 H), 3.34-3.69 (m, 3 H), 3.12 (br d, 1 H), 2.85 (br d, 1 H) | 448.1 [M+H]⁺ | 97.8% |
| 110 | (CD₃OD) δ 7.58-7.75 (m, 2 H), 7.41 (dd, 1 H), 7.16-7.26 (m, 1.3 H), 6.57-7.15 (m, 1.7 H), 4.67 (br s, 2 H), 4.56 (br d, 1 H), 3.94 (br s, 1 H), 3.49 (br s, 3 H), 3.12 (br d, 1 H), 2.76-2.92 (m, 1 H) | 448.1 [M+H]⁺ | 96.2% |
| 111 | (CD₃OD) δ 8.27-8.42 (m, 1 H), 8.01-8.11 (m, 1 H), 7.68 (s, 1 H), 7.39-7.59 (m, 2.3 H), 7.12-7.32 (m, 1 H), 6.85 (s, 0.7 H), 5.29 (br dd, 0.7 H), 5.06 (br dd, 0.3 H), 3.86-4.01 (m, 3.8 H), 3.53-3.62 (m, 0.2 H), 2.75-3.21 (m, 2 H), 2.37-2.50 (m, 3 H) | 431.1 [M+H]⁺ | 100% |
| 112 | (CD₃OD) δ 8.31-8.41 (m, 1 H), 8.02-8.13 (m, 1 H), 7.68 (s, 1 H), 7.37-7.60 (m, 2.3 H), 7.12-7.27 (m, 1 H), 6.85 (br s, 0.7 H), 5.29 (br dd, 0.7 H), 5.06 (br dd, 0.3 H), 3.87-4.00 (m, 3.8 H), 3.58 (br d, 0.2 H), 2.78-3.23 (m, 2 H), 2.36-2.48 (m, 3 H) | 431.1 [M+H]⁺ | 99.3% |
| 113 | (CD₃OD) δ 8.31-8.37 (m, 1 H), 7.62-7.86 (m, 3 H), 7.38-7.57 (m, 1.5 H), 7.15-7.27 (m, 2 H), 6.90 (br s, 0.5 H), 5.30 (dd, 0.7 H), 5.05-5.19 (m, 0.3 H), 3.99 (br t, 0.7 H), 3.66 (br s, 0.3 H), 2.97-3.28 (m, 1 H), 2.81-2.96 (m, 1 H) | 471.2 [M+H]⁺ | 91.0% |
| 114 | (CD₃OD) δ 8.29-8.39 (m, 1 H), 7.61-7.87 (m, 3 H), 7.38-7.59 (m, 1.5 H), 7.14-7.28 (m, 2 H), 6.90 (br s, 0.5 H), 5.30 (dd, 0.7 H), 5.13 (dd, 0.3 H), 3.99 (br t, 0.7 H), 3.66 (br s, 0.3 H), 2.97-3.28 (m, 1 H), 2.74-2.96 (m, 1 H) | 471.2 [M+H]⁺ | 92.7% |
| 115 | (CD₃OD) δ 7.62-7.75 (m, 2 H), 7.43 (br d, 1 H), 7.09-7.36 (m, 2 H), 6.94-7.04 (m, 0.3 H), 6.53-6.85 (m, 0.7 H), 5.15-5.47 (m, 2 H), 4.96-5.12 (m, 0.3 H), 4.47-4.61 (m, 0.7 H), 3.97 (br s, 0.7 H), 3.44-3.81 (m, 0.3 H), 3.11 (br s, 1 H), 2.87 (br d, 1 H) | 502.2 [M+H]⁺ | 87.8% |
| 116 | (CD₃OD) δ 7.60-7.78 (m, 2 H), 7.40-7.47 (m, 1 H), 7.06-7.31 (m, 2 H), 6.93-7.02 (m, 0.3 H), 6.51-6.87 (m, 0.7 H), 5.14-5.49 (m, 2 H), 4.94-5.12 (m, 0.3 H), 4.53 (br d, 0.7 H), 3.98 (br s, 0.7 H), 3.47-3.81 (m, 0.3 H), 3.13 (br d, 1 H), 2.87 (br d, 1 H) | 502.1 [M+H]⁺ | 87.4% |
| 117 | (CD₃OD) δ 8.48-8.68 (m, 1 H), 7.61-7.81 (m, 2 H), 7.37-7.59 (m, 1.3 H), 7.16-7.30 (m, 1 H), 6.89 (s, 0.7 H), 5.05-5.33 (m, 1 H), 4.04-4.11 (m, 3 H), 3.98 (br t, 0.7 H), 3.57-3.75 (m, 0.3 H), 2.77-3.26 (m, 2 H) | 503.2 [M+H]⁺ | 99.7% |
| 118 | (CD₃OD) δ 8.54-8.63 (m, 1 H), 7.63-7.81 (m, 2 H), 7.37-7.60 (m, 1.3 H), 7.13-7.32 (m, 1 H), 6.69-7.07 (m, 0.7 H), 5.27 (dd, 0.7 H), 5.11 (br d, 0.3 H), 4.05-4.11 (m, 3 H), 3.98 (br s, 0.7 H), 3.65 (s, 0.3 H), 3.14-3.26 (m, 1 H), 2.91 (br d, 1 H) | 503.2 [M+H]⁺ | 99.1% |
| 119 | (CD₃OD) δ 7.61-7.76 (m, 2 H), 7.37-7.56 (m, 2.3 H), 7.13-7.28 (m, 1 H), 6.88 (br s, 0.7 H), 5.32 (dd, 0.7 H), 5.02-5.17 (m, 0.3 H), 4.14-4.29 (m, 3 H), 3.88-4.09 (m, 0.7 H), 3.65 (br s, 0.3 H), 2.82-3.26 (m, 2 H), 2.22-2.53 (m, 3 H) | 449.1 [M+H]⁺ | 100% |
| 120 | (CD₃OD) δ 7.59-7.83 (m, 2 H), 7.35-7.55 (m, 2 H), 7.14-7.26 (m, 1 H), 6.88 (s, 1 H), 5.32 (br dd, 0.7 H), 5.12 (br dd, 0.3 H), 4.11-4.29 (m, 3 H), 3.78-4.08 (m, 0.7 H), 3.57-3.72 (m, 0.3 H), 2.80-3.27 (m, 2 H), 2.25-2.45 (m, 3 H) | 449.1 [M+H]⁺ | 89.7% |
| 121 | (CD₃OD) δ 8.98-9.08 (m, 1 H), 8.48-8.60 (m, 1 H), 7.90-8.04 (m, 1 H), 7.61-7.77 (m, 2 H), 7.54 (s, 0.3 H), 7.36-7.48 (m, 1 H), 7.13-7.27 (m, 1 H), 6.91 (s, 0.7 H), 5.24-5.38 (m, 0.7 H), 5.13 (dd, 0.3 H), 4.00 (ddd, 0.7 H), 3.58-3.77 (m, 0.3 H), 3.18-3.26 (m, 0.7 H), 2.97-3.08 (m, 0.3 H), 2.82-2.95 (m, 1 H) | 500.1 [M+H]⁺ | 85.6% |
| 122 | (CD₃OD) δ 8.99-9.06 (m, 1 H), 8.50-8.59 (m, 1 H), 7.92-8.00 (m, 1 H), 7.62-7.77 (m, 2 H), 7.54 (s, 0.3 H), 7.38-7.45 (m, 1 H), 7.15-7.25 (m, 1 H), 6.91 (s, 0.6 H), 5.29 (dd, 0.7 H), 5.13 (dd, 0.3 H), 3.93-4.06 (m, 0.7 H), 3.67 (td, 0.3 H), 3.18-3.27 (m, 0.7 H), 2.98-3.07 (m, 0.3 H), 2.84-2.95 (m, 1 H) | 500.0 **[M+H]+** | 86.4% |
| 123 | (CD₃OD) δ 7.62-7.75 (m, 2 H), 7.43 (dd, 1 H), 7.21 (td, 1 H), 6.39-6.84 (m, 1 H), 4.39 (br s, 1 H), 3.58-4.09 (m, 1 H), 3.18 (br s, 1 H), 2.87 (br d, 1 H), 1.48-1.73 (m, 6 H) | 490.1 [M+H]⁺ | 97.4% |
| 124 | (CD₃OD) δ 7.63-7.74 (m, 2 H), 7.44 (dd, 1 H), 7.22 (td, 1 H), 6.33-6.84 (m, 1 H), 4.39 (br s, 1 H), 3.62-4.11 (m, 1 H), 3.15 (br s, 1 H), 2.87 (br d, 1 H), 1.66 (br s, 6 H) | 492.2 [M+H]⁺ | 94.5% |
| 125 | (CD₃OD) δ 8.58-8.66 (m, 1 H), 8.13-8.29 (m, 1 H), 7.92 (br d, 1 H), 7.63-7.79 (m, 2 H), 7.39-7.60 (m, 1.3 H), 7.14-7.28 (m, 1 H), 6.91 (br s, 0.7 H), 5.07-5.34 (m, 1 H), 4.00 (br s, 0.7 H), 3.67 (br s, 0.3 H), 3.24 (br t, 0.7 H), 3.02 (br d, 0.3 H), 2.91 (br d, 1 H), 2.49 (br s, 3 H) | 446.1 [M+H]⁺ | 98.3% |
| 126 | (CD₃OD) δ 8.58-8.66 (m, 1 H), 8.13-8.29 (m, 1 H), 7.92 (br d, 1 H), 7.63-7.79 (m, 2 H), 7.39-7.60 (m, 1.3 H), 7.14-7.28 (m, 1 H), 6.91 (br s, 0.7 H), 5.07-5.34 (m, 1 H), 4.00 (br s, 0.7 H), 3.67 (br s, 0.3 H), 3.24 (br t, 0.7 H), 3.02 (br d,0.3 H), 2.91 (br d, 1 H), 2.49 (br s, 3 H) | 446.1 [M+H]⁺ | 91.3% |
| 127 | (CD₃OD) δ 7.58-7.76 (m, 2 H), 7.42 (br d, 1 H), 7.20 (td, 1 H), 6.79 (br s, 0.7 H), 6.63 (s, 1.3 H), 4.30 (br d, 1 H), 3.88 (br s, 4 H), 2.94-3.09 (m, 1 H), 2.80 (br d, 1 H) | 401.0 [M+H]⁺ | 96.7% |
| 128 | (CD₃OD) δ 7.58-7.75 (m, 2 H), 7.42 (br d, 1 H), 7.20 (td, 1 H), 6.79 (br s, 0.7 H), 6.63 (s, 1.3 H), 4.30 (br d, 1 H), 3.88 (br s, 4 H), 2.94-3.10 (m, 1 H), 2.79 (br d, 1 H) | 401.0 [M+H]⁺ | 93.7% |
| 129 | (CD₃OD) δ 7.64-7.73 (m, 2 H), 7.43 (dd, 1 H), 7.21 (td, 1 H), 6.43-6.81 (m, 1 H), 4.33-4.55 (m, 1 H), 3.54-4.14 (m, 1 H), 3.16 (br s, 1 H), 2.88 (br d, 1 H), 1.47-1.73 (m, 6 H) | 446.2 [M+H]⁺ | 91.4% |
| 130 | (CD₃OD) δ 7.64-7.73 (m, 2 H), 7.43 (dd, 1 H), 7.21 (td, 1 H), 6.38-6.83 (m, 1 H), 4.43 (br s, 1 H), 3.49-4.11 (m, 1 H), 2.96-3.26 (m, 1 H), 2.88 (br d, 1 H), 1.44-1.75 (m, 6 H) | 446.2 [M+H]⁺ | 88.1% |
| 131 | (CD₃OD) δ 7.62-7.79 (m, 3.2 H), 7.37-7.62 (m, 1.3 H), 7.14-7.37 (m, 1 H), 7.01-7.13 (m, 1 H), 6.90 (br s, 0.5 H), 5.33 (dd, 0.8 H), 5.12 (br d, 0.2 H), 4.27-4.38 (m, 3 H), 4.00 (br s, 0.7 H), 3.68 (br s, 0.3 H), 2.79-3.25 (m, 2 H) | 435.30 [M+H]⁺ | 99.1% |
| 132 | (CD₃OD) δ 7.63-7.84 (m, 3.3 H), 7.35-7.62 (m, 1.2 H), 7.15-7.30 (m, 1 H), 7.02-7.14 (m, 1 H), 6.90 (br s, 0.5 H), 5.33 (dd, 0.7 H), 5.14 (br dd, 0.3 H), 4.25-4.36 (m, 3 H), 4.00 (br s, 0.7 H), 3.69 (br s, 0.3 H), 3.10-3.29 (m, 1 H), 2.86-3.01 (m, 1 H) | 435.30 [M+H]⁺ | 98.3% |
| 133 | (CD₃OD) δ 8.30 (br s, 1 H), 8.08 (d, 1 H), 7.83 (d, 1 H), 7.63-7.68 (m, 2 H), 7.43 (ddd, 2 H), 7.19 (td, 1 H), 6.75 (br s, 1 H), 4.73 (br dd, 1 H), 3.90 (br s, 1 H), 3.09-3.18 (m, 5 H), 2.81-2.86 (m, 5 H), 1.72-1.78 (m, 1 H), 0.51-0.56 (m, 2 H), 0.44-0.49 (m, 2 H) | 527.3 [M+H]⁺ | 100% |
| 134 | (CD₃OD) δ 8.30 (br s, 1 H), 8.08 (d, 1 H), 7.83 (d, 1 H), 7.62-7.68 (m, 2 H), 7.41-7.46 (m, 2 H), 7.19 (td, 1 H), 6.75 (br s, 1 H), 4.73 (br dd, 1 H), 3.80-3.99 (m, 1 H), 3.90 (br s, 1 H), 3.07-3.17 (m, 5 H), 2.80-2.86 (m, 5 H), 1.72-1.78 (m, 1 H), 0.51-0.57 (m, 2 H), 0.44-0.49 (m, 2 H) | 527.3 [M+H]⁺ | 99.5% |
| 135 | (CD₃OD) δ 7.61-7.76 (m, 2 H), 7.43 (dd, 1 H), 7.21 (td, 1 H), 6.70 (br s, 1 H), 4.79-4.87 (m, 1 H), 4.63 (s, 1 H), 3.92 (br s, 1 H), 2.75-3.22 (m, 1 H), 2.75-3.22 (m, 1 H), 2.23-2.50 (m, 3 H), 1.31-1.75 (m, 3 H) | 412.3 [M+H]⁺ | 88.4% |
| 136 | (CD₃OD) δ 7.60-7.75 (m, 2 H), 7.43 (dd, 1 H), 7.21 (td, 1 H), 6.71 (br s, 1 H), 4.81-4.87 (m, 1 H), 4.63 (s, 1 H), 3.91 (br s, 1 H), 2.73-3.23 (m, 2 H), 2.39 (s, 3 H), 1.58 (br s, 3 H) | 412.3 [M+H]⁺ | 91.4% |
| 137 | (CD₃OD) δ 8.48 (br s, 1 H), 8.16 (br s, 1 H), 7.43-7.62 (m, 2 H), 7.23-7.37 (m, 1.4 H), 6.70-7.21 (m, 1.6 H), 4.59 (br s, 1 H), 3.87 (br s, 1 H), 3.15 (br s, 1 H), 2.91 (br d, 1 H), 2.48 (s, 3 H) | 400.3 [M+H]⁺ | 96.8% |
| 138 | (CD₃OD) δ 8.47 (br s, 1 H), 8.08 (br s, 1 H), 7.45-7.62 (m, 2 H), 7.24-7.36 (m, 1.4 H), 6.67-7.21 (m, 1.6 H), 4.58 (br s, 1 H), 3.87 (br s, 1 H), 3.15 (br s, 1 H), 2.90 (br d, 1 H), 2.47 (s, 3 H) | 400.30 [M+H]⁺ | 96.0% |
| 139 | (CD₃OD) δ 8.28 (br s, 1 H), 8.06 (d, 1 H), 7.81 (d, 1 H), 7.59-7.67 (m, 2 H), 7.37-7.44 (m, 2 H), 7.17 (td, 1 H), 6.71 (br s, 1 H), 4.71 (br d, 1 H), 3.88 (br s, 1 H), 3.12-3.19 (m, 2 H), 3.09 (br s, 1 H), 2.91 (s, 2 H), 2.81 (br d, 1 H), 2.71-2.78 (m, 2 H), 2.29 (s, 3 H), 1.16 (s, 6 H) | 265.2 [1/2M+H]⁺ | 98.7% |
| 140 | (CD₃OD) δ 8.28 (br s, 1 H), 8.06 (d, 1 H), 7.81 (d, 1 H), 7.61-7.67 (m, 2 H), 7.37-7.43 (m, 2 H), 7.17 (td, 1 H), 6.71 (br s, 1 H), 4.67-4.76 (m, 1 H), 3.88 (br s, 1 H), 3.13-3.19 (m, 2 H), 3.09 (br s, 1 H), 2.91 (s, 2 H), 2.81 (br d, 1 H), 2.71-2.78 (m, 2 H), 2.29 (s, 3 H), 1.16 (s, 6 H) | 265.3 [1/2M+H]⁺ | 98.4% |
| 141 | (CD₃OD) δ 7.77 (s, 1 H), 7.67 (dd, 1 H), 7.43 (dd, 1 H), 7.22 (td, 1 H), 6.90-7.17 (m, 1 H), 6.76 (br s, 1 H), 4.64 (br s, 1 H), 3.94 (br s, 1 H), 3.07-3.21 (m, 1 H), 2.83-2.95 (m, 1 H), 1.29-1.50 (m, 4 H) | 460.20 [M+H]⁺ | 96.1% |
| 142 | (CD₃OD) δ 7.73 (s, 1 H), 7.67 (dd, 1 H), 7.43 (dd, 1 H), 7.22 (td, 1 H), 6.95-7.15 (m, 1 H), 6.75 (br s, 1 H), 4.61 (br s, 1 H), 3.94 (br s, 1 H), 3.06-3.23 (m, 1 H), 2.87 (br d, 1 H), 1.30-1.53 (m, 4 H) | 460.20 [M+H]⁺ | 96.1% |
| 143 | (CD₃OD) δ 8.43-8.58 (m, 1 H), 7.82-7.98 (m, 1 H), 7.73 (s, 1 H), 7.52-7.67 (m, 2 H), 6.83 (s, 0.7 H), 6.14 (br s, 0.3 H), 3.86-4.13 (m, 1.7 H), 3.60 (br d, 0.3 H), 2.79-3.07 (m, 2 H) | 482.1 [M+H]⁺ | 98.5% |
| 144 | (CD₃OD) δ 8.40-8.56 (m, 1 H), 7.83-7.96 (m, 1 H), 7.72 (s, 1 H), 7.50-7.65 (m, 2 H), 6.83 (s, 0.8 H), 6.14 (br s, 0.2 H), 3.85-4.13 (m, 1.7 H), 3.61 (br s, 0.3 H), 2.77-3.06 (m, 2 H) | 484.2 [M+H]⁺ | 96.7% |
| 145 | (CD₃OD) δ 8.47 (br s, 1 H), 7.91 (d, 1 H), 7.71 (s, 1 H), 7.53-7.67 (m, 2 H), 7.27 (s, 0.4 H), 7.14 (s, 0.6 H), 7.01 (s, 0.2 H), 6.76 (br s, 0.8 H), 4.55 (br s, 1 H), 3.95 (br s, 1 H), 3.12 (br s, 1 H), 2.87 (br d, 1 H) | 466.1 [M+H]⁺ | 97.8% |
| 146 | (CD₃OD) δ 8.47 (br s, 1 H), 7.91 (d, 1 H), 7.71 (s, 1 H), 7.52-7.65 (m, 2 H), 6.97-7.32 (m, 1 H), 6.76 (br s, 1 H), 4.56 (br d, 1 H), 3.95 (br s, 1 H), 3.13 (br d, 1 H), 2.86 (br d, 1 H) | 466.1 [M+H]⁺ | 95.3% |
| 147 | (CD₃OD) δ 8.40-8.54 (m, 1 H), 7.86 (s, 1 H), 7.49-7.74 (m, 3 H), 6.82 (br s, 0.8 H), 6.13 (br s, 0.2 H), 4.95 (br s, 0.3 H), 3.87-4.08 (m, 1.5 H), 3.58 (br s, 0.2 H), 2.74-3.05 (m, 2 H) | 482.2 [M+H]⁺ | 96.4% |
| 148 | (CD₃OD) δ 8.39-8.55 (m, 1 H), 7.86 (s, 1 H), 7.49-7.75 (m, 3 H), 6.82 (br s, 0.8 H), 6.13 (br s, 0.2 H), 4.96 (br d, 0.3 H), 3.88-4.06 (m, 1.5 H), 3.58 (br s, 0.2 H), 2.75-3.05 (m, 2 H) | 482.2 [M+H]⁺ | 95.4% |
| 149 | (CD₃OD) δ 8.45 (br s, 1 H), 7.85 (s, 1 H), 7.50-7.74 (m, 3 H), 6.93-7.32 (m, 1 H), 6.74 (br s, 1 H), 4.46-4.58 (m, 1 H), 3.50-4.06 (m, 1 H), 2.75-3.19 (m, 2 H) | 464.1 [M+H]⁺ | 91.3% |
| 150 | (CD₃OD) δ 8.45 (br s, 1 H), 7.85 (s, 1 H), 7.48-7.74 (m, 3 H), 6.90-7.32 (m, 1 H), 6.75 (br s, 1 H), 4.54 (br d, 1 H), 3.50-4.02 (m, 1 H), 2.71-3.19 (m, 2 H) | 466.1 [M+H]⁺ | 76.2% |
| 151 | (CD₃OD) δ 7.54-7.79 (m, 2 H), 7.39 (br dd, 1 H), 7.18 (td, 1 H), 6.57-7.11 (m, 2 H), 4.96 (br s, 1 H), 4.55 (br d, 1 H), 3.49-4.05 (m, 1 H), 2.72-3.22 (m, 2 H), 1.39-1.71 (m, 3 H) | 448.2 [M+H]⁺ | 97.1% |
| 152 | (CD₃OD) δ 7.52-7.74 (m, 2 H), 7.39 (dd, 1 H), 7.15-7.26 (m, 1 H), 6.57-7.11 (m, 2 H), 4.97 (br s, 1 H), 4.57 (br d, 1 H), 3.45-4.11 (m, 1 H), 2.76-3.18 (m, 2 H), 1.35-1.77 (m, 3 H) | 448.3 [M+H]⁺ | 100% |
| 153 | (CD₃OD) δ 8.40 (s, 1 H), 7.59-7.81 (m, 2 H), 7.44 (dd, 1 H), 7.22 (td, 1 H), 6.84 (br s, 0.8 H), 6.06 (br s, 0.2 H), 4.98 (br s, 0.4 H), 3.87-4.04 (m, 1.5 H), 3.62 (br s, 0.1 H), 2.97 (br d, 1 H), 2.74-2.92 (m, 1 H), 1.99 (t, 3 H) | 418.2 [M+H]⁺ | 92.5% |
| 154 | (CD₃OD) δ 8.32-8.47 (m, 1 H), 7.59-7.76 (m, 2 H), 7.44 (dd, 1 H), 7.22 (td, 1 H), 6.84 (br s, 0.8 H), 6.05 (br s, 0.2 H), 4.97-5.04 (m, 0.4 H), 3.88-4.03 (m, 1.5 H), 3.62 (br s, 0.1 H), 2.92-3.05 (m, 1 H), 2.74-2.91 (m, 1 H), 1.99 (t, 3 H) | 418.2 [M+H]⁺ | 96.6% |
| 155 | (CD₃OD) δ 7.62-7.73 (m, 2 H), 7.43 (dd, 1 H), 6.97-7.28 (m, 2 H), 6.76 (br s, 1 H), 4.51-4.77 (m, 3 H), 3.59-4.06 (m, 1 H), 2.97-3.19 (m, 1 H), 2.79-2.92 (m, 1 H) | 451.2 [M+H]⁺ | 98.5% |
| 156 | (CD₃OD) δ 7.51-7.62 (m, 2 H), 7.31 (dd, 1 H), 6.86-7.17 (m, 2 H), 6.65 (br s, 1 H), 4.38-4.60 (m, 3 H), 3.44-3.92 (m, 1 H), 3.00 (br s, 1 H), 2.75 (br d, 1 H) | 451.3 [M+H]⁺ | 98.6% |
| 157 | (CD₃OD) δ 7.58-7.71 (m, 2 H), 7.39 (dd, 1 H), 6.89-7.27 (m, 2 H), 6.71 (br s, 1 H), 4.93-4.99 (m, 1 H), 4.51-4.57 (m, 1 H), 3.92 (br s, 1 H), 2.71-3.19 (m, 2 H), 1.39-1.70 (m, 3 H) | 448.2 [M+H]⁺ | 98.4% |
| 158 | (CD₃OD) δ 7.55-7.72 (m, 2 H), 7.39 (dd, 1 H), 6.90-7.25 (m, 2 H), 6.71 (br s, 1 H), 4.96 (br s, 1 H), 4.46-4.58 (m, 1 H), 3.91 (br s, 1 H), 2.77-3.18 (m, 2 H), 1.38-1.74 (m, 3 H) | 448.2 [M+H]⁺ | 100% |
| 159 | (CD₃OD) δ 7.66 (dt, 2 H), 7.42 (br d, 1 H), 7.15-7.27 (m, 1 H), 6.95-7.15 (m, 1 H), 6.54-6.78 (m, 1 H), 4.54 (br d, 1 H), 3.56-4.08 (m, 1 H), 2.76-3.24 (m, 2 H), 1.71-1.95 (m, 6 H) | 471.3 [M+H]⁺ | 95.7% |
| 160 | (CD₃OD) δ 7.66 (dt, 2 H), 7.42 (br d, 1 H), 6.95-7.28 (m, 2 H), 6.54-6.79 (m, 1 H), 4.54 (br d, 1 H), 3.56-4.07 (m, 1 H), 2.76-3.25 (m, 2 H), 1.71-1.94 (m, 6 H) | 471.2 [M+H]⁺ | 96.0% |
| 161 | (CD₃OD) δ 8.31-8.44 (m, 1 H), 7.54-7.67 (m, 2 H), 7.29-7.46 (m, 1 H), 7.08 (ddd, 1 H), 6.72 (s, 0.8 H), 6.01 (br s, 0.2 H), 3.74-4.02 (m, 1.8 H), 3.42-3.59 (m, 0.2 H), 2.83-2.93 (m, 1 H), 2.64-2.78 (m, 1 H) | 422.0 [M+H]⁺ | 100% |
| 162 | (CD₃OD) δ 8.32-8.42 (m, 1 H), 7.54-7.68 (m, 2 H), 7.28-7.41 (m, 1 H), 7.03-7.17 (m, 1 H), 6.72 (s, 0.8 H), 6.01 (br s, 0.2 H), 3.74-4.00 (m, 1.8 H), 3.48 (br t, 0.2 H), 2.84-2.96 (m, 1 H), 2.66-2.81 (m, 1 H) | 422.0 [M+H]⁺ | 99.2% |
| 163 | (CD₃OD) δ 8.32 (s, 0.8 H), 7.52-7.63 (m, 2 H), 7.36 (br d, 1 H), 6.98-7.18 (m, 2 H), 6.87 (s, 0.2 H), 6.65 (br s, 1 H), 4.43-4.52 (m, 1 H), 3.82 (br s, 0.8 H), 3.50 (br s, 0.2 H), 2.86-3.07 (m, 1 H), 2.74 (br d, 1 H) | 404.0 [M+H]⁺ | 98.5% |
| 164 | (CD₃OD) δ 8.33 (s, 0.8 H), 7.53-7.62 (m, 2 H), 7.36 (dd, 1 H), 6.98-7.21 (m, 2 H), 6.88 (s, 1 H), 6.65 (br s, 0.2 H), 4.43 (br s, 1 H), 3.82 (br s, 1 H), 3.00 (br d, 1 H), 2.67-2.80 (m, 1 H) | 404.0 [M+H]⁺ | 96.4% |
| 165 | (DMSO-d₆) δ 12.14 (br s, 1 H), 9.44 (s, 1 H), 8.53 (br s, 1 H), 8.00-8.14 (m, 1 H), 7.77 (br dd, 1 H), 7.51-7.65 (m, 2 H), 7.25 (br t, 1 H), 6.45 (br s, 1 H), 4.52 (br s, 1 H), 3.90 (br s, 1 H), 2.92-3.17 (m, 1 H), 2.75 (br d, 1 H) | 517.1 [M+H]⁺ | 98.0% |
| 166 | (DMSO-d₆) δ 12.14 (br s, 1 H), 9.45 (s, 1 H), 8.51-8.57 (m, 1 H), 8.08 (s, 1 H), 7.72-7.81 (m, 1 H), 7.50-7.65 (m, 2 H), 7.25 (td, 1 H), 6.45 (s, 1 H), 4.37-4.65 (m, 1 H), 3.91 (br s, 1 H), 2.98-3.16 (m, 1 H), 2.75 (br d, 1 H) | 517.1 [M+H]⁺ | 99.5% |
| 167 | (CD₃OD) δ 8.29-8.53 (m, 1 H), 7.60 (s, 1 H), 7.25-7.45 (m, 2 H), 6.93-7.13 (m, 1 H), 6.73 (s, 0.8 H), 6.04 (br s, 0.2 H), 3.73-4.01 (m, 2 H), 2.59-2.99 (m, 2 H) | 422.2 [M+H]⁺ | 98.7% |
| 168 | (CD₃OD) δ 8.25-8.47 (m, 1 H), 7.50-7.68 (m, 1 H), 7.22-7.45 (m, 2 H), 6.96-7.10 (m, 1 H), 6.73 (s, 0.8 H), 6.04 (br s, 0.2H), 3.77-4.06 (m, 2 H), 2.54-3.00 (m, 2 H) | 422.2 [M+H]⁺ | 99.1% |
| 169 | (CD₃OD) δ 8.46 (br s, 1 H), 7.71 (s, 1 H), 7.49-7.54 (m, 1 H), 7.43 (td, 1 H), 7.28 (s, 0.2 H), 7.10-7.21 (m, 1.6 H), 7.01 (s, 0.2 H), 6.78 (br s, 1 H), 4.47-4.58 (m, 1 H), 3.97 (br d, 1 H), 2.79-3.24 (m, 2 H) | 404.0 [M+H]⁺ | 97.7% |
| 170 | (CD₃OD) δ 8.46 (br s, 1 H), 7.76 (s, 1 H), 7.49-7.54 (m, 1 H), 7.43 (td, 1 H), 7.28 (s, 0.2 H), 7.12-7.19 (m, 1.6 H), 7.01 (s, 0.2 H), 6.80 (br s, 1 H), 4.56 (br s, 1 H), 3.96 (br s, 1 H), 2.87-3.20 (m, 2 H) | 404.0 [M+H]⁺ | 96.7% |
| 171 | (CD₃OD) δ 8.43-8.53 (m, 1 H), 7.70 (s, 1 H), 7.52 (d, 1 H), 7.37 (td, 1 H), 7.23 (ddd, 1 H), 6.85 (br s, 0.7 H), 6.17 (br s, 0.2 H), 4.97 (br d, 0.1 H), 3.88-4.08 (m, 1.6 H), 3.55-3.68 (m, 0.3 H), 2.93-3.05 (m, 1 H), 2.77-2.92 (m, 1 H) | 422.0 [M+H]⁺ | 98.0% |
| 172 | (CD₃OD) δ 8.43-8.53 (m, 1 H), 7.70 (s, 1 H), 7.51 (d, 1 H), 7.37 (td, 1 H), 7.18-7.27 (m, 1 H), 6.85 (br s, 0.7 H), 6.18 (br s, 0.3 H), 4.95-5.02 (m, 0.3 H), 3.86-4.12 (m, 1.6 H), 3.55-3.66 (m, 0.2 H), 2.77-3.04 (m, 2 H) | 422.0 [M+H]⁺ | 94.8% |
| 173 | (CD₃OD) δ 8.45 (br s, 1 H), 7.69 (s, 1 H), 7.51 (d, 1 H), 7.37 (td, 1 H), 7.20-7.26 (m, 1 H), 6.98-7.15 (m, 1 H), 6.61-6.88 (m, 1 H), 4.56 (br d, 1 H), 3.97 (br s, 1 H), 3.10 (br s, 1 H), 2.86 (br d, 1 H) | 404.0 [M+H]⁺ | 94.3% |
| 174 | (CD₃OD) δ 8.47 (br s, 1 H), 7.71 (s, 1 H), 7.53 (d, 1 H), 7.39 (td, 1 H), 7.22-7.29 (m, 1 H), 6.99-7.17 (m, 1 H), 6.66-6.91 (m, 1 H), 4.58 (br d, 1 H), 3.99 (br s, 1 H), 3.13 (br s, 1 H), 2.88 (br d, 1 H) | 404.0 [M+H]⁺ | 90.8% |
| 175 | (CD₃OD) δ 8.34-8.41 (m, 2 H), 8.02 (s, 1 H), 7.60-7.68 (m, 2 H), 7.41 (dd, 1 H), 7.17 (td, 1 H), 6.72 (br s, 1 H), 4.70 (br d, 1 H), 3.79-3.89 (m, 5 H), 3.01-3.11 (m, 5 H), 2.82 (br d, 1 H) | 522.30 [M+H]⁺ | 95.6% |
| 176 | (CD₃OD) δ 8.38 (s, 2 H), 8.01 (s, 1 H), 7.60-7.67 (m, 2 H), 7.41 (dd, 1 H), 7.17 (td, 1 H), 6.72 (br s, 1 H), 4.70 (br d, 1 H), 3.79-3.88 (m, 5 H), 2.97-3.10 (m, 5 H), 2.82 (br d, 1 H) | 522.20 [M+H]⁺ | 96.0% |
| 177 | (CD₃OD) δ 8.24 (br s, 1 H), 8.15 (s, 1 H), 7.94 (s, 1 H), 7.56-7.71 (m, 2 H), 7.40 (dd, 1 H), 7.17 (td, 1 H), 6.72 (br s, 1 H), 4.72 (br dd, 1 H), 3.85 (br s, 1 H), 3.01-3.17 (m, 1 H), 2.81 (br dd, 1 H) | 452.2 [M+H]⁺ | 99.2% |
| 178 | (CD₃OD) δ 8.09-8.32 (m, 2 H), 7.95 (s, 1 H), 7.57-7.70 (m, 2 H), 7.41 (dd, 1 H), 7.17 (td, 1 H), 6.72 (br s, 1 H), 4.72 (br dd, 1 H), 3.85 (br s, 1 H), 3.03-3.15 (m, 1 H), 2.73-2.92 (m, 1 H) | 452.3 [M+H]⁺ | 99.3% |
| 179 | (CD₃OD) δ 8.39 (br s, 1 H), 7.60-7.72 (m, 2 H), 7.41 (dd, 1 H), 7.20 (td, 1 H), 6.54-6.82 (m, 1 H), 5.36-5.64 (m, 2 H), 4.54 (br s, 1 H), 3.94 (br s, 1 H), 3.09 (br s, 1 H), 2.84 (br d, 1 H) | 386.0 [M+H]⁺ | 98.9% |
| 180 | (CD₃OD) δ 8.39 (br s, 1 H), 7.59-7.74 (m, 2 H), 7.41 (dd, 1 H), 7.20 (td, 1 H), 6.73 (br s, 1 H), 5.38-5.65 (m, 2 H), 4.39-4.58 (m, 1 H), 3.94 (br s, 1 H), 3.11 (br d, 1 H), 2.84 (br d, 1 H) | 386.0 [M+H]⁺ | 96.4% |
| 181 | (CD₃OD) δ 8.37-8.48 (m, 2 H), 8.06 (s, 1 H), 7.65-7.72 (m, 2 H), 7.45 (dd, 1 H), 7.22 (td, 1 H), 6.76 (br s, 1 H), 4.75 (br d, 1 H), 3.91 (br s, 1 H), 3.06-3.23 (m, 5 H), 2.63-2.89 (m, 5 H), 2.41 (s, 3 H) | 535.4 [M+H]⁺ | 95.3% |
| 182 | (CD₃OD) δ 8.37-8.48 (m, 2 H), 8.06 (s, 1 H), 7.61-7.74 (m, 2 H), 7.45 (dd, 1 H), 7.22 (td, 1 H), 6.76 (br s, 1 H), 4.76 (br d, 1 H), 3.91 (br s, 1 H), 3.15 (br s, 5 H), 2.64-2.93 (m, 5 H), 2.40 (s, 3 H) | 535.3 [M+H]⁺ | 97.4% |
| 183 | (CD₃OD) δ 7.97 (br s, 1 H), 7.54-7.76 (m, 2 H), 7.26-7.52 (m, 1.3 H), 6.66-7.25 (m, 2.5 H), 6.26 (br s, 0.2 H), 5.13 (q, 2 H), 4.25 (br s, 1 H), 3.92 (br s, 1 H), 2.95-3.08 (m, 1 H), 2.79 (dd, 1 H) | 485.0 [M+H]⁺ | 96.7% |
| 184 | (CD₃OD) δ 7.97 (br s, 1 H), 7.55-7.77 (m, 2 H), 7.30-7.50 (m, 1.2 H), 6.63-7.24 (m, 2.5 H), 6.24 (br s, 0.3 H), 5.05-5.23 (m, 2 H), 4.26 (br s, 1 H), 3.92 (br s, 1 H), 2.92-3.08 (m, 1 H), 2.79 (br dd, 1 H) | 485.0 [M+H]⁺ | 95.1% |
| 185 | (CD₃OD) δ 7.60-7.73 (m, 2 H), 7.41 (dd, 1 H), 7.19 (td, 1 H), 6.67 (br s, 1 H), 4.51 (br s, 1 H), 3.93 (br s, 1 H), 2.73-3.22 (m, 2 H), 2.25-2.58 (m, 3 H), 1.83 (br s, 6 H) | 435.0 [M+H]⁺ | 98.0% |
| 186 | (CD₃OD) δ 7.67 (br s, 2 H), 7.41 (br s, 1 H), 7.19 (br s, 1 H), 6.66 (br s, 1 H), 4.51 (br s, 1 H), 3.46-4.13 (m, 1 H), 2.68-3.23 (m, 2 H), 2.38 (br s, 3 H), 1.83 (br s, 6 H) | 435.0 [M+H]⁺ | 99.2% |
| 187 | (CD₃OD) δ 7.64-7.77 (m, 2 H), 7.50 (dd, 1 H), 6.94-7.33 (m, 2 H), 6.77 (br s, 1 H), 4.56 (br s, 1 H), 3.54-4.06 (m, 1 H), 2.73-3.26 (m, 2 H), 1.54-1.80 (m, 6 H) | 462.2 [M+H]⁺ | 98.9% |
| 188 | (CD₃OD) δ 7.63-7.74 (m, 2 H), 7.50 (dd, 1 H), 6.92-7.32 (m, 2 H), 6.77 (br s, 1 H), 4.50-4.61 (m, 1 H), 3.54-4.07 (m, 1 H), 2.83-3.24 (m, 2 H), 1.52-1.75 (m, 6 H) | 462.2 [M+H]⁺ | 97.7% |
| 189 | (CD₃OD) δ 7.61-7.90 (m, 3.8 H), 7.50 (br d, 1.2 H), 7.20 (td, 1 H), 6.71-7.03 (m, 1.8 H), 6.32 (br s, 0.2 H), 4.29 (b d, 1 H), 3.93 (br s, 0.8 H), 3.50 (br s, 0.2 H), 2.97-3.09 (m, 1 H), 2.82 (br d, 1 H) | 403.2 [M+H]⁺ | 99.1% |
| 190 | (CD₃OD) δ 7.61-7.89 (m, 3.8 H), 7.50 (br d, 1.2 H), 7.20 (td, 1 H), 6.74-7.02 (m, 1.7 H), 6.32 (br s, 0.3 H), 4.29 (br d, 1 H), 3.92 (br s, 0.7 H), 3.50 (br s, 0.3 H), 2.98-3.10 (m, 1 H), 2.82 (br d, 1 H) | 403.2 [M+H]⁺ | 97.3% |
| 191 | (CD₃OD) δ 8.37-8.43 (m, 1 H), 8.07-8.14 (m, 1 H), 7.73 (br s, 1 H), 7.60 (s, 0.3 H), 7.49-7.55 (m, 1 H), 7.33-7.41 (m, 1 H), 7.19-7.27 (m, 1 H), 6.92 (s, 0.7 H), 5.06-5.40 (m, 1 H), 4.00-4.04 (m, 3 H), 3.95 (br d, 0.6 H), 3.67 (td, 0.3 H), 2.97-3.29 (m, 1 H), 2.81-2.96 (m, 1 H) | 435.30 [M+H]⁺ | 98.0% |
| 192 | (CD₃OD) δ 8.37-8.43 (m, 1 H), 8.07-8.14 (m, 1 H), 7.73 (br s, 1 H), 7.60 (s, 0.3 H), 7.49-7.55 (m, 1 H), 7.33-7.41 (m, 1 H), 7.19-7.27 (m, 1 H), 6.92 (s, 0.7 H), 5.06-5.40 (m, 1 H), 4.00-4.04 (m, 3 H), 3.95 (br d, 0.7 H), 3.67 (td, 0.3 H), 2.97-3.29 (m, 1 H), 2.81-2.96 (m, 1 H) | 435.30 [M+H]⁺ | 95.6% |
| 193 | (CD₃OD) δ 7.71 (s, 1 H), 7.53 (d, 1 H), 7.38 (td, 1 H), 6.94-7.31 (m, 2 H), 6.79 (br s, 1 H), 4.48-4.62 (m, 1 H), 3.99 (br s, 0.7 H), 3.67 (br s, 0.3 H), 2.74-3.22 (m, 2 H), 1.48-1.77 (m, 6 H) | 462.30 [M+H]⁺ | 97.4% |
| 194 | (CD₃OD) δ 7.70 (s, 1 H), 7.53 (d, 1 H), 7.39 (td, 1 H), 6.92-7.32 (m, 2 H), 6.59-6.88 (m, 1 H), 4.61 (br s, 1 H), 3.99 (br s, 0.7 H), 3.67 (br s, 0.3 H), 2.75-3.26 (m, 2 H), 1.48-1.80 (m, 6 H) | 462.20 [M+H]⁺ | 98.0% |
| 195 | (CD₃OD) δ 7.59-7.89 (m, 3 H), 7.46-7.57 (m, 1 H), 7.37 (td, 1 H), 7.18-7.27 (m, 1 H), 6.86 (br s, 2 H), 4.28 (br d, 1 H), 3.94 (br s, 0.7 H), 3.50 (br d, 0.3 H), 2.97-3.10 (m, 1 H), 2.74-2.88 (m, 1 H) | 403.2 [M+H]⁺ | 97.7% |
| 196 | (CD₃OD) δ 7.60-7.86 (m, 3 H), 7.45-7.57 (m, 1 H), 7.37 (td, 1 H), 7.18-7.26 (m, 1 H), 6.26-6.95 (m, 2 H), 4.28 (br d, 1 H), 3.94 (br s, 0.7 H), 3.42-3.61 (m, 0.3 H), 2.95-3.10 (m, 1 H), 2.82 (br dd, 1 H) | 403.2 [M+H]⁺ | 97.4% |
| 197 | (CD₃OD) δ 8.27-8.48 (m, 1 H), 8.00-8.19 (m, 1 H), 7.66-7.78 (m, 1 H), 7.34-7.63 (m, 2.3 H), 7.03-7.26 (m, 1 H), 6.87 (br s, 0.7 H), 5.05-5.41 (m, 1 H), 3.59-4.06 (m, 4 H), 2.83-3.26 (m, 2 H) | 435.2 [M+H]⁺ | 100% |
| 198 | (CD₃OD) δ 8.18-8.46 (m, 1 H), 7.87-8.10 (m, 1 H), 7.55-7.75 (m, 1 H), 7.23-7.52 (m, 2.3 H), 6.95-7.15 (m, 1 H), 6.77 (s, 0.7 H), 4.92-5.28 (m, 1 H), 3.49-4.01 (m, 4 H), 2.68-3.16 (m, 2 H) | 435.2 [M+H]⁺ | 99.3% |
| 199 | (CD₃OD) δ 7.69 (s, 1 H), 7.35-7.55 (m, 2 H), 6.90-7.32 (m, 2 H), 6.51-6.84 (m, 1 H), 4.57 (br d, 1 H), 3.51-4.07 (m, 1 H), 2.80-3.25 (m, 2 H), 1.49-1.76 (m, 6 H) | 462.1 [M+H]⁺ | 99.7% |
| 200 | (CD₃OD) δ 7.72 (s, 1 H), 7.36-7.54 (m, 2 H), 6.92-7.28 (m, 2 H), 6.59-6.84 (m, 1 H), 4.46-4.67 (m, 1 H), 3.50-4.07 (m, 1 H), 2.81-3.23 (m, 2 H), 1.48-1.75 (m, 6 H) | 462.1 [M+H]⁺ | 98.0% |
| 201 | (CD₃OD) δ 7.60-7.90 (m, 2.7 H), 7.35-7.57 (m, 2.3 H), 7.14 (t, 1 H), 6.19-6.99 (m, 2 H), 4.20-4.39 (m, 1 H), 3.42-4.05 (m, 1 H), 2.95-3.11 (m, 1 H), 2.81 (br d, 1 H) | 403.2 [M+H]⁺ | 97.3% |
| 202 | (CD₃OD) δ 7.58-7.88 (m, 2.7 H), 7.36-7.56 (m, 2.3 H), 7.14 (t, 1 H), 6.21-6.99 (m, 2 H), 4.30 (br d, 1 H), 3.42-4.02 (m, 1 H), 2.96-3.09 (m, 1 H), 2.71-2.90 (m, 1 H) | 403.2 [M+H]⁺ | 98.2% |
| 203 | (CD₃OD) δ 8.73 (s, 1 H), 8.47 (br s, 1 H), 8.07 (s, 1 H), 7.61-7.86 (m, 2 H), 7.16-7.57 (m, 2 H), 6.77 (br s, 1 H), 4.73 (br d, 1 H), 3.80-4.02 (m, 1 H), 3.65 (s, 2 H), 2.78-3.22 (m, 2 H), 2.37 (s, 6 H) | 494.3 [M+H]⁺ | 99.5% |
| 204 | (CD₃OD) δ 8.72 (s, 1 H), 8.47 (br s, 1 H), 8.07 (s, 1 H), 7.61-7.86 (m, 2 H), 7.45 (dd, 1 H), 7.21 (td, 1 H), 6.76 (br s, 1 H), 4.73 (br d, 1 H), 3.92 (br s, 1 H), 3.65 (s, 2 H), 2.79-3.23 (m, 2 H), 2.37 (s, 6 H) | 494.2 [M+H]⁺ | 99.7% |
| 205 | (CD₃OD) δ 8.22 (br s, 1 H), 7.91 (s, 1 H), 7.80 (s, 1 H), 7.60-7.66 (m, 2 H), 7.39 (br d, 1 H), 7.11-7.20 (m, 1 H), 6.72 (s, 1 H), 4.69-4.75 (m, 1 H), 3.85 (br s, 1 H), 3.04-3.14 (m, 1 H), 2.82 (s, 4 H) | 466.3 [M+H]⁺ | 98.3% |
| 206 | (CD₃OD) δ 8.23 (br s, 1 H), 7.93 (s, 1 H), 7.82 (s, 1 H), 7.61-7.66 (m, 2 H), 7.40 (dd, 1 H), 7.17 (td, 1 H), 6.72 (s, 1 H), 4.73 (br dd, 1 H), 3.86 (br s, 1 H), 3.04-3.14 (m, 1 H), 2.78-2.87 (m, 4 H) | 466.2 [M+H]⁺ | 98.2% |
| 207 | (CD₃OD) δ 8.24-8.38 (m, 2 H), 7.95 (s, 1 H), 7.56-7.67 (m, 2 H), 7.38 (dd, 1 H), 7.14 (td, 1 H), 6.72 (br s, 1 H), 4.69 (br dd, 1 H), 3.86 (br s, 1 H), 3.03-3.14 (m, 1 H), 2.76-2.84 (m, 7 H) | 480.3 [M+H]⁺ | 99.8% |
| 208 | (CD₃OD) δ 8.32 (s, 2 H), 7.97 (s, 1 H), 7.60-7.66 (m, 2 H), 7.40 (dd, 1 H), 7.16 (td, 1 H), 6.72 (br s, 1 H), 4.70 (br dd, 1 H), 3.85 (br s, 1 H), 3.03-3.14 (m, 1 H), 2.80-2.82 (m, 7 H) | 480.2 [M+H]⁺ | 99.6% |
| 209 | (CD₃OD) δ 7.68 (s, 1 H), 7.29-7.54 (m, 2 H), 7.14 (dd, 1 H), 6.69 (br s, 1 H), 4.61 (s, 2 H), 3.59-4.09 (m, 1 H), 2.75-3.21 (m, 2 H), 2.38 (s, 3 H), 1.28-1.73 (m, 3 H) | 412.2 [M+H]⁺ | 99.0% |
| 210 | (CD₃OD) δ 7.70 (s, 1 H), 7.34-7.57 (m, 2 H), 7.16 (dd, 1 H), 6.71 (br s, 1 H), 4.62 (s, 2 H), 3.54-4.12 (m, 1 H), 2.75-3.23 (m, 2 H), 2.29-2.55 (m, 3 H), 1.30-1.74 (m, 3 H) | 412.2 [M+H]⁺ | 96.9% |
| 211 | (CD₃OD) δ 7.60-7.71 (m, 2 H), 7.42 (dd, 1 H), 7.20 (td, 1 H), 6.72 (br s, 1 H), 5.34-5.60 (m, 2 H), 4.55 (br s, 1 H), 3.94 (br s, 0.7 H), 3.64 (br s, 0.3 H), 3.13 (br s, 1 H), 2.85 (br d, 1 H), 1.42-1.75 (m, 6 H) | 444.0 [M+H]⁺ | 98.4% |
| 212 | (CD₃OD) δ 7.61-7.71 (m, 2 H), 7.42 (br d, 1 H), 7.20 (td, 1 H), 6.70 (br s, 1 H), 5.36-5.61 (m, 2 H), 4.55 (br s, 1 H), 3.67-4.05 (m, 1 H), 3.14 (br s, 1 H), 2.85 (br d, 1 H), 1.48-1.75 (m, 6 H) | 444.0 [M+H]⁺ | 99.7% |
| 213 | (CD₃OD) δ 7.68 (s, 1 H), 7.51 (d, 1 H), 7.37 (td, 1 H), 7.18-7.26 (m, 1 H), 6.94-7.13 (m, 1 H), 6.76 (br s, 1 H), 4.89-4.97 (m, 1 H), 4.70 (br d, 1 H), 3.96 (br s, 0.7 H), 3.64 (br s, 0.3 H), 3.13 (br s, 1 H), 2.74-2.94 (m, 1 H), 1.27-1.50 (m, 4 H) | 460.1 [M+H]⁺ | 96.3% |
| 214 | (CD₃OD) δ 7.68 (s, 1 H), 7.51 (d, 1 H), 7.37 (td, 1 H), 7.20-7.26 (m, 1 H), 7.10 (br s, 1 H), 6.97 (br s, 1 H), 6.75 (br s, 1 H), 4.89-4.91 (m, 1 H), 4.60 (s, 1 H), 3.55-4.04 (m, 1 H), 3.00-3.21 (m, 1 H), 2.87 (br d, 1 H), 1.26-1.49 (m, 4 H) | 460.2 [M+H]⁺ | 97.9% |
| 215 | (CD₃OD) δ 7.58 (s, 1 H), 7.27-7.48 (m, 2 H), 6.79-7.23 (m, 2 H), 6.62 (br s, 1 H), 4.52-4.63 (m, 1 H), 3.37-4.03 (m, 1 H), 2.51-3.15 (m, 2 H), 0.97-1.56 (m, 4 H) | 460.2 [M+H]⁺ | 96.0% |
| 216 | (CD₃OD) δ 7.58 (s, 1 H), 7.27-7.46 (m, 2 H), 6.82-7.24 (m, 2 H), 6.62 (br s, 1 H), 4.50-4.65 (m, 1 H), 3.41-4.07 (m, 1 H), 2.51-3.15 (m, 2 H), 1.04-1.55 (m, 4 H) | 460.2 [M+H]⁺ | 85.3% |
| 217 | (CD₃OD) δ 7.68 (s, 1 H), 7.49 (d, 1 H), 7.34 (td, 1 H), 7.15-7.25 (m, 1 H), 6.71 (br s, 1 H), 4.78-4.87 (m, 1 H), 4.59 (br s, 1 H), 3.92 (br dd, 1 H), 3.10 (br s, 1 H), 2.79-2.89 (m, 1 H), 2.37 (s, 3 H), 1.56 (br s, 3 H) | 412.1 [M+H]⁺ | 99.0% |
| 218 | (CD₃OD) δ 7.68 (s, 1 H), 7.50 (d, 1 H), 7.35 (td, 1 H), 7.15-7.24 (m, 1 H), 6.72 (br s, 1 H), 4.95-5.01 (m, 1 H), 4.60 (br s, 1 H), 3.73-4.02 (m, 1 H), 3.11 (br s, 1 H), 2.85 (br d, 1 H), 2.38 (s, 3 H), 1.56 (br s, 3 H) | 412.1 [M+H]⁺ | 97.9% |
| 219 | (CD₃OD) δ 8.97 (d, 1 H), 8.49 (br s, 1 H), 8.16 (d, 1 H), 7.64-7.73 (m, 2 H), 7.45 (dd, 1 H), 7.21 (td, 1 H), 6.76 (br s, 1 H), 4.73 (br d, 1 H), 3.91 (br s, 1 H), 3.08-3.19 (m, 1 H), 2.86 (br dd, 1 H) | 455.2 [M+H]⁺ | 83.2% |
| 220 | (CD₃OD) δ 8.97 (d, 1 H), 8.49 (br s, 1 H), 8.17 (d, 1 H), 7.65-7.72 (m, 2 H), 7.45 (dd, 1 H), 7.21 (td, 1 H), 6.76 (br s, 1 H), 4.73 (br d, 1 H), 3.91 (br s, 1 H), 3.05-3.24 (m, 1 H), 2.86 (br dd, 1 H) | 455.2 [M+H]⁺ | 87.5% |
| 221 | (CD₃OD) δ 7.56 (dt, 2 H), 7.33 (br s, 1 H), 7.11 (td, 1 H), 6.50-7.06 (m, 2 H), 4.45 (br s, 1 H), 3.46-3.95 (m, 1 H), 2.68-3.12 (m, 2 H), 1.96-2.25 (m, 3 H), 1.30-1.65 (m, 6 H) | 475.30 [M+H]⁺ | 97.4% |
| 222 | (CD₃OD) δ 7.56 (dt, 2 H), 7.33 (br s, 1 H), 7.11 (td, 1 H), 6.47-7.06 (m, 2 H), 4.46 (br s, 1 H), 3.46-3.97 (m, 1 H), 2.64-3.11 (m, 2 H), 1.98-2.24 (m, 3 H), 1.29-1.57 (m, 6 H) | 475.30 [M+H]⁺ | 97.4% |
| 223 | (CD₃OD) δ 7.50-7.64 (m, 2 H), 7.31 (dd, 1 H), 7.10 (td, 1 H), 6.28-6.82 (m, 1 H), 4.78-4.91 (m, 1 H), 4.26 (br s, 1 H), 3.86 (br s, 1 H), 3.01-3.10 (m, 1 H), 2.75 (br d, 1 H), 1.32-1.59 (m, 3 H) | 478.1 [M+H]⁺ | 91.4% |
| 224 | (CD₃OD) δ 7.49-7.64 (m, 2 H), 7.31 (dd, 1 H), 7.09 (td, 1 H), 6.18-6.77 (m, 1 H), 4.81-4.91 (m, 1 H), 4.28 (br s, 1 H), 3.86 (br s, 1 H), 3.03 (br s, 1 H), 2.75 (br d, 1 H), 1.47 (br s, 3 H) | 476.1 [M+H]⁺ | 94.4% |
| 225 | (CD₃OD) δ 7.51-7.62 (m, 2 H), 7.31 (dd, 1 H), 7.09 (td, 1 H), 6.24-6.67 (m, 1 H), 4.86-5.02 (m, 1 H), 4.29 (br s, 1 H), 3.87 (br s, 1 H), 3.03 (br s, 1 H), 2.71-2.81 (m, 1 H), 1.48 (br s, 3 H) | 476.1 [M+H]⁺ | 90.1% |
| 226 | (CD₃OD) δ 7.51-7.67 (m, 2 H), 7.31 (dd, 1 H), 7.09 (td, 1 H), 6.28-6.71 (m, 1 H), 4.87-5.04 (m, 1 H), 4.27 (br s, 1 H), 3.86 (br s, 1 H), 3.03 (br s, 1 H), 2.71-2.79 (m, 1 H), 1.32-1.58 (m, 3 H) | 476.1 [M+H]⁺ | 92.0% |
| 227 | (CD₃OD) δ 7.73 (s, 1 H), 7.49-7.56 (m, 1 H), 7.43 (td, 1 H), 6.98-7.31 (m, 2 H), 6.78 (br s, 1 H), 5.01 (br s, 1.4 H), 4.61 (br d, 0.6 H), 3.78-4.07 (m, 1 H), 2.81-3.25 (m, 2 H), 1.44-1.74 (m, 3 H) | 448.20 [M+H]⁺ | 98.4% |
| 228 | (CD₃OD) δ 7.74 (s, 1 H), 7.48-7.55 (m, 1 H), 7.43 (td, 1 H), 6.97-7.31 (m, 2 H), 6.78 (br s, 1 H), 5.00 (br s, 1.4 H), 4.61 (br d, 0.6 H), 3.86-4.07 (m, 1 H), 2.84-3.26 (m, 2 H), 1.43-1.76 (m, 3 H) | 448.20 [M+H]⁺ | 99.5% |
| 229 | (CD₃OD) δ 7.71 (s, 1 H), 7.37-7.56 (m, 2 H), 6.97-7.29 (m, 2 H), 6.77 (br s, 1 H), 5.00 (br s, 1 H), 4.60 (br d, 1 H), 3.63-4.07 (m, 1 H), 3.15 (br s, 1 H), 2.88 (br d, 1 H), 1.44-1.69 (m, 3 H) | 448.1 [M+H]⁺ | 95.6% |
| 230 | (CD₃OD) δ 7.71 (s, 1 H), 7.39-7.54 (m, 2 H), 6.95-7.29 (m, 2 H), 6.77 (br s, 1 H), 5.01 (br s, 1 H), 4.61 (br d, 1 H), 3.54-4.04 (m, 1 H), 3.00-3.24 (m, 1 H), 2.88 (br d, 1 H), 1.43-1.71 (m, 3 H) | 448.1 [M+H]⁺ | 92.1% |
| 231 | (CD₃OD) δ 7.65-7.74 (m, 2 H), 7.48 (dd, 1 H), 6.94-7.32 (m, 2 H), 6.77 (br s, 1 H), 5.01 (br s, 1 H), 4.60 (br s, 1 H), 3.47-4.01 (m, 1 H), 2.96-3.22 (m, 1 H), 2.87 (br d, 1 H), 1.38-1.73 (m, 3 H) | 448.1 [M+H]⁺ | 98.5% |
| 232 | (CD₃OD) δ 7.65-7.74 (m, 2 H), 7.46-7.51 (m, 1 H), 6.95-7.29 (m, 2 H), 6.77 (br s, 1 H), 5.00 (br s, 1 H), 4.60 (br s, 1 H), 3.51-4.09 (m, 1 H), 2.95-3.27 (m, 1 H), 2.87 (br d, 1 H), 1.40-1.72 (m, 3 H) | 448.1 [M+H]⁺ | 95.6% |
| 233 | (CD₃OD) δ 7.61-7.73 (m, 2 H), 7.41 (dd, 1 H), 7.19 (td, 1 H), 6.41-6.77 (m, 1 H), 4.68-4.74 (m, 1 H), 4.44 (br s, 1 H), 3.86-4.03 (m, 0.7 H), 3.62 (br s, 0.3 H), 3.13 (br s, 1 H), 2.86 (br d, 1 H), 1.57 (br s, 3 H) | 432.1 [M+H]⁺ | 91.3% |
| 234 | (CD₃OD) δ 7.74 (s, 1 H), 7.65 (dd, 1 H), 7.41 (dd, 1 H), 7.20 (td, 1 H), 6.45-6.80 (m, 1 H), 4.55-4.66 (m, 1 H), 4.44 (br s, 1 H), 3.86-4.04 (m, 0.7 H), 3.61 (br d, 0.3 H), 2.97-3.23 (m, 1 H), 2.86 (br d, 1 H), 1.37-1.66 (m, 3 H) | 432.1 [M+H]⁺ | 92.4% |
| 235 | (CD₃OD) δ 7.60-7.71 (m, 2 H), 7.41 (br d, 1 H), 7.19 (td, 1 H), 6.70 (br s, 1 H), 4.59 (br s, 1 H), 4.27-4.52 (m, 1 H), 3.97 (br s, 1 H), 3.13 (br s, 1 H), 2.85 (br d, 1 H), 1.57 (br s, 3 H) | 432.2 [M+H]⁺ | 92.2% |
| 236 | (CD₃OD) δ 7.61-7.72 (m, 2 H), 7.41 (dd, 1 H), 7.19 (td, 1 H), 6.45-6.77 (m, 1 H), 4.59 (br s, 1 H), 4.44 (br s, 1 H), 3.97 (br s, 0.7 H), 3.63 (br s, 0.3 H), 3.13 (br s, 1 H), 2.85 (br d, 1 H), 1.58 (br s, 3 H) | 432.1 [M+H]⁺ | 92.3% |
| 237 | (CD₃OD) δ 7.60-7.78 (m, 2 H), 7.48 (dd, 1 H), 6.87-7.30 (m, 2 H), 6.70 (br s, 1 H), 4.96-5.18 (m, 1 H), 3.51-4.03 (m, 1 H), 2.73-3.22 (m, 2 H), 1.15-1.52 (m, 4 H) | 460.2 [M+H]⁺ | 97.5% |
| 238 | (CD₃OD) δ 7.58-7.77 (m, 2 H), 7.48 (dd, 1 H), 6.90-7.29 (m, 2 H), 6.71 (br s, 1 H), 4.94-5.01 (m, 1 H), 3.44-4.04 (m, 1 H), 2.72-3.20 (m, 2 H), 1.12-1.49 (m, 4 H) | 460.2 [M+H]⁺ | 93.9% |
| 239 | (CD₃OD) δ 7.56-7.81 (m, 2 H), 7.47 (dd, 1 H), 6.90-7.32 (m, 2 H), 6.74 (br s, 1 H), 4.98 (br s, 1.6 H), 4.52-4.57 (m, 0.4 H), 3.52-4.06 (m, 1 H), 2.77-3.23 (m, 2 H), 1.38-1.78 (m, 3 H) | 448.3 [M+H]⁺ | 93.9% |
| 240 | (CD₃OD) δ 7.60-7.77 (m, 2 H), 7.47 (dd, 1 H), 6.87-7.32 (m, 2 H), 6.74 (br s, 1 H), 4.98 (br s, 1.6 H), 4.43-4.56 (m, 0.4 H), 3.51-4.04 (m, 1 H), 2.75-3.21 (m, 2 H), 1.40-1.74 (m, 3 H) | 448.2 [M+H]⁺ | 91.7% |
| 241 | (CD₃OD) δ 7.58-7.76 (m, 2 H), 7.47 (dd, 1 H), 7.17 (td, 1 H), 6.69 (br s, 1 H), 4.87-4.98 (m, 2 H), 3.86 (br s, 1 H), 3.09 (br s, 1 H), 2.75-2.93 (m, 1 H), 2.37 (s, 3 H), 1.55 (br s, 3 H) | 412.2 [M+H]⁺ | 99.1% |
| 242 | (CD₃OD) δ 7.57-7.76 (m, 2 H), 7.47 (dd, 1 H), 7.17 (td, 1 H), 6.69 (br s, 1 H), 4.88-5.10 (m, 2 H), 3.56-4.04 (m, 1 H), 3.09 (br s, 1 H), 2.74-2.90 (m, 1 H), 2.28-2.47 (m, 3 H), 1.35-1.65 (m, 3 H) | 412.2 [M+H]⁺ | 98.3% |
| 243 | (CD₃OD) δ 7.69 (s, 1 H), 7.51 (d, 1 H), 7.37 (td, 1 H), 6.95-7.27 (m, 2 H), 6.76 (br s, 1 H), 4.99 (br s, 1 H), 4.52-4.60 (m, 1 H), 3.51-4.07 (m, 1 H), 2.82-3.20 (m, 2 H), 1.47-1.69 (m, 3 H) | 448.2 [M+H]⁺ | 86.5% |
| 244 | (CD₃OD) δ 7.69 (s, 1 H), 7.52 (d, 1 H), 7.37 (td, 1 H), 7.19-7.27 (m, 1 H), 6.73-7.14 (m, 1 H), 4.98 (br s, 1 H), 4.57 (br s, 1 H), 3.60-4.02 (m, 1 H), 2.81-3.23 (m, 2 H), 1.46-1.67 (m, 3 H) | 448.2 [M+H]⁺ | 93.0% |
| 245 | (CD₃OD) δ 7.69 (s, 1 H), 7.52 (d, 1 H), 7.37 (td, 1 H), 7.23 (dd, 1 H), 6.94-7.13 (m, 1 H), 6.76 (br s, 1 H), 4.96-4.98 (m, 1 H), 4.60-4.65 (m, 1 H), 3.93 (br dd, 1 H), 3.13 (br s, 1 H), 2.87 (br d, 1 H), 1.44-1.69 (m, 3 H) | 448.2 [M+H]⁺ | 89.2% |
| 246 | (CD₃OD) δ 7.69 (s, 1 H), 7.51 (d, 1 H), 7.37 (td, 1 H), 7.19-7.26 (m, 1 H), 6.94-7.15 (m, 1 H), 6.77 (br s, 1 H), 4.99 (br s, 1 H), 4.53-4.60 (m, 1 H), 3.54-4.08 (m, 1 H), 2.81-3.24 (m, 2 H), 1.46-1.70 (m, 3 H) | 448.2 [M+H]⁺ | 94.6% |
| 247 | (CD₃OD) δ 7.68 (s, 1 H), 7.51 (d, 1 H), 7.36 (td, 1 H), 7.22 (dd, 1 H), 6.72 (br s, 1 H), 4.92-4.95 (m, 1 H), 4.60-4.62 (m, 1 H), 3.91 (br s, 1 H), 3.11 (br s, 1 H), 2.85 (br d, 1 H), 2.37 (s, 3 H), 1.55 (br s, 3 H) | 412.2 [M+H]⁺ | 99.7% |
| 248 | (CD₃OD) δ 7.67 (s, 1 H), 7.51 (d, 1 H), 7.36 (td, 1 H), 7.22 (dd, 1 H), 6.71 (br s, 1 H), 4.94-5.01 (m, 1 H), 4.60-4.69 (m, 1 H), 3.91 (br s, 1 H), 3.10 (br s, 1 H), 2.85 (br d, 1 H), 2.37 (s, 3 H), 1.56 (br s, 3 H) | 412.2 [M+H]⁺ | 99.2% |
| 249 | (CD₃OD) δ 7.66-7.75 (m, 2 H), 7.46-7.54 (m, 1 H), 7.20 (td, 1 H), 6.32-6.82 (m, 1 H), 4.39 (br s, 1 H), 3.48-4.09 (m, 1 H), 2.98-3.27 (m, 1 H), 2.87 (br d, 1 H), 1.65 (br s, 6 H) | 492.2 [M+H]⁺ | 96.4% |
| 250 | (CD₃OD) δ 7.66-7.74 (m, 2 H), 7.49 (dd, 1 H), 7.20 (td, 1 H), 6.05-6.88 (m, 1 H), 4.38 (br s, 1 H), 3.53-4.08 (m, 1 H), 2.96-3.23 (m, 1 H), 2.87 (br d, 1 H), 1.49-1.74 (m, 6 H) | 490.2 [M+H]⁺ | 97.2% |
| 251 | (CD₃OD) δ 7.70 (s, 1 H), 7.33-7.56 (m, 2 H), 6.99-7.24 (m, 1 H), 6.33-6.80 (m, 1 H), 4.27-4.58 (m, 1 H), 3.54-4.15 (m, 1 H), 2.69-3.28 (m, 2 H), 1.66 (br s, 6 H) | 490.20 [M+H]⁺ | 96.7% |
| 252 | (CD₃OD) δ 7.70 (s, 1 H), 7.30-7.57 (m, 2 H), 7.06-7.26 (m, 1 H), 6.20-6.84 (m, 1 H), 4.28-4.53 (m, 1 H), 3.55-4.14 (m, 1 H), 2.59-3.29 (m, 2 H), 1.42-1.83 (m, 6 H) | 490.20 [M+H]⁺ | 96.9% |
| 253 | (CD₃OD) δ 7.67-7.73 (m, 1 H), 7.52 (dd, 1 H), 7.38 (td, 1 H), 7.20-7.27 (m, 1 H), 6.84 (s, 0.7 H), 6.22 (br s, 0.3 H), 3.89-4.12 (m, 2 H), 2.93-3.11 (m, 1 H), 2.80-2.91 (m, 1 H), 1.52-1.71 (m, 6 H) | 480.2 [M+H]⁺ | 99.6% |
| 254 | (CD₃OD) δ 7.67-7.74 (m, 1 H), 7.52 (dd, 1 H), 7.38 (td, 1 H), 7.24 (dd, 1 H), 6.84 (s, 0.7 H), 6.22 (br s, 0.3 H), 4.02-4.12 (m, 1 H), 3.89-4.00 (m, 0.7 H), 3.57-3.73 (m, 0.3 H), 2.92-3.11 (m, 1 H), 2.79-2.91 (m, 1 H), 1.52-1.71 (m, 6 H) | 480.2 [M+H]⁺ | 98.3% |
| 255 | (CD₃OD) δ 7.60-7.83 (m, 2 H), 7.40-7.55 (m, 1 H), 7.21 (td, 1 H), 6.80 (br s, 0.7 H), 6.14 (br s, 0.3 H), 4.99 (br s, 0.4 H), 4.02-4.15 (m, 0.6 H), 3.99 (br s, 0.7 H), 3.66 (br s, 0.3 H), 3.05 (br s, 1 H), 2.87 (br s, 1 H), 1.54-1.71 (m, 6 H) | 480.2 [M+H]⁺ | 100% |
| 256 | (CD₃OD) δ 7.63-7.81 (m, 2 H), 7.42-7.56 (m, 1 H), 7.21 (td, 1 H), 6.82 (br s, 0.7 H), 6.16 (br s, 0.3 H), 4.95 (br s, 0.4 H), 4.01-4.12 (m, 0.6 H), 3.95 (br s, 0.7 H), 3.64 (br s, 0.3 H), 3.03 (br d, 1 H), 2.85 (br d, 1 H), 1.54-1.71 (m, 6 H) | 480.2 [M+H]⁺ | 98.7% |
| 257 | (CD₃OD) δ 7.55-7.64 (m, 1 H), 7.27-7.47 (m, 2 H), 7.05 (t, 1 H), 6.05-6.80 (m, 1 H), 4.88 (br s, 0.2 H), 3.82-4.01 (m, 1.5 H), 3.48-3.65 (m, 0.3 H), 2.70-3.01 (m, 2 H), 1.40-1.61 (m, 6 H) | 480.2 [M+H]⁺ | 96.9% |
| 258 | (CD₃OD) δ 7.55-7.64 (m, 1 H), 7.29-7.45 (m, 2 H), 7.01-7.13 (m, 1 H), 6.00-6.80 (m, 1 H), 4.86 (br d, 0.2 H), 3.81-4.01 (m, 1.5 H), 3.56 (br s, 0.3 H), 2.86-2.99 (m, 1 H), 2.70-2.81 (m, 1 H), 1.41-1.61 (m, 6 H) | 480.2 [M+H]⁺ | 97.5% |
| 259 | (CD₃OD) δ 7.70 (s, 1 H), 7.49-7.55 (m, 1 H), 7.43 (td, 1 H), 7.08-7.24 (m, 1 H), 6.71 (br s, 1 H), 4.61 (br s, 1 H), 3.95 (br s, 1 H), 3.15 (br s, 1 H), 2.87 (br d, 1 H), 2.40 (s, 3 H), 1.64 (br s, 6 H) | 426.2 [M+H]⁺ | 99.3% |
| 260 | (CD₃OD) δ 7.69 (s, 1 H), 7.49-7.54 (m, 1 H), 7.43 (td, 1 H), 7.12-7.20 (m, 1 H), 6.71 (br s, 1 H), 4.61 (s, 1 H), 3.94 (br s, 1 H), 3.15 (br s, 1 H), 2.84-2.94 (m, 1 H), 2.40 (s, 3 H), 1.64 (br s, 6 H) | 426.2 [M+H]⁺ | 98.9% |
| 261 | (CD₃OD) δ 7.80-7.96 (m, 1 H), 7.50-7.76 (m, 2 H), 7.41 (dd, 1 H), 7.19 (td, 1 H), 6.73-6.93 (m, 1.7 H), 6.07 (s, 0.3 H), 3.78-4.08 (m, 1.6 H), 3.42-3.58 (m, 0.4 H), 2.41-3.08 (m, 2 H) | 421.0 [M+H]⁺ | 100% |
| 262 | (CD₃OD) δ 7.79-7.99 (m, 1 H), 7.54-7.76 (m, 2 H), 7.41 (dd, 1 H), 7.19 (td, 1 H), 6.75-6.93 (m, 1.8 H), 6.07 (s, 0.2 H), 3.79-4.06 (m, 1.7 H), 3.41-3.58 (m, 0.3 H), 2.64-2.99 (m, 2 H) | 421.0 [M+H]⁺ | 100% |
| 263 | (CD₃OD) δ 7.66 (s, 1 H), 7.50 (d, 1 H), 7.35 (td, 1 H), 7.16-7.26 (m, 1 H), 6.38-6.91 (m, 1 H), 4.37 (br s, 1 H), 3.98 (br s, 0.7 H), 3.62 (br s, 0.3 H), 3.15 (br s, 1 H), 2.85 (br d, 1 H), 1.62 (br s, 6 H) | 490.0 [M+H]⁺ | 97.3% |
| 264 | (CD₃OD) δ 7.67 (s, 1 H), 7.50 (d, 1 H), 7.35 (td, 1 H), 7.13-7.26 (m, 1 H), 6.34-6.83 (m, 1 H), 4.23-4.52 (m, 1 H), 3.51-4.13 (m, 1 H), 2.79-3.21 (m, 2 H), 1.62 (br s, 6 H) | 490.0 [M+H]⁺ | 91.8% |
| 265 | (CD₃OD) δ 8.62 (s, 1 H), 8.42 (br s, 1 H), 7.93 (br d, 1 H), 7.68 (s, 1 H), 7.53 (br d, 1 H), 7.32-7.48 (m, 2 H), 7.18-7.29 (m, 1 H), 6.80 (br s, 1 H), 4.73 (br d, 1.3 H), 4.53 (s, 2 H), 3.94 (br s, 0.7 H), 3.43 (s, 3 H), 3.06-3.21 (m, 1 H), 2.86 (br d, 1 H) | 447.20 [M+H]⁺ | 94.5% |
| 266 | (CD₃OD) δ 8.62 (s, 1 H), 8.42 (br s, 1 H), 7.94 (d, 1 H), 7.69 (br s, 1 H), 7.52 (brd, 1 H), 7.32-7.46 (m, 2 H), 7.22 (dd, 1 H), 6.79 (br s, 1 H), 4.72 (br d, 1 H), 4.53 (s, 2 H), 3.92 (br s, 1 H), 3.43 (s, 3 H), 3.05-3.21 (m, 1 H), 2.86 (br d, 1 H) | 447.20 [M+H]⁺ | 97.1% |
| 267 | (CD₃OD) δ 7.69 (s, 1 H), 7.53 (d, 1 H), 7.38 (td, 1 H), 7.20-7.29 (m, 1 H), 6.73 (br s, 1 H), 4.48-4.64 (m, 1 H), 3.94 (dt, 1 H), 2.79-3.24 (m, 2 H), 2.40 (s, 3 H), 1.64 (br s, 6 H) | 426.2 [M+H]⁺ | 100% |
| 268 | (CD₃OD) δ 7.70 (s, 1 H), 7.53 (d, 1 H), 7.38 (td, 1 H), 7.20-7.29 (m, 1 H), 6.73 (br s, 1 H), 4.55-4.64 (m, 1 H), 3.44-4.07 (m, 1 H), 2.79-3.26 (m, 2 H), 2.39 (s, 3 H), 1.40-1.80 (m, 6 H) | 426.2 [M+H]⁺ | 100% |
| 269 | (CD₃OD) δ 7.62-7.81 (m, 2 H), 7.49 (dd, 1 H), 7.20 (td, 1 H), 6.38-6.88 (m, 1 H), 4.43 (br s, 0.6 H), 3.97 (br s, 1 H), 3.66 (br s, 0.4 H), 3.15 (br s, 1 H), 2.87 (br d, 1 H), 1.65 (br s, 6 H) | 446.2 [M+H]⁺ | 100% |
| 270 | (CD₃OD) δ 7.62-7.90 (m, 2 H), 7.49 (dd, 1 H), 7.20 (td, 1 H), 6.41-6.88 (m, 1 H), 4.45 (br s, 0.5 H), 3.97 (br s, 1 H), 3.66 (br s, 0.5 H), 3.15 (br s, 1 H), 2.87 (br d, 1 H), 1.65 (br s, 6 H) | 446.2 [M+H]⁺ | 100% |
| 271 | (CD₃OD) δ 7.70 (s, 1 H), 7.53 (br d, 1 H), 7.39 (td, 1 H), 7.17-7.29 (m, 1 H), 6.74 (br s, 1 H), 4.26-4.52 (m, 0.4 H), 4.00 (br s, 1 H), 3.66 (br s, 0.6 H), 3.16 (br s, 1 H), 2.79-2.97 (m, 1 H), 1.64 (br s, 6 H) | 446.2 [M+H]⁺ | 99.3% |
| 272 | (CD₃OD) δ 7.70 (br s, 1 H), 7.53 (br d, 1 H), 7.39 (br s, 1 H), 7.18-7.34 (m, 1 H), 6.76 (br s, 1 H), 4.47 (br s, 0.9 H), 4.01 (br s, 1 H), 3.46-3.82 (m, 0.1 H), 3.17 (br s, 1 H), 2.91 (br s, 1 H), 1.65 (br s, 6 H) | 446.2 [M+H]⁺ | 99.1% |
| 273 | (CD₃OD) δ 7.70 (s, 1 H), 7.49-7.59 (m, 1 H), 7.43 (td, 1 H), 7.16 (t, 1 H), 6.73 (br s, 1 H), 4.47 (br s, 0.5 H), 4.02 (br s, 1 H), 3.68 (br s, 0.5 H), 3.15 (br s, 1 H), 2.89 (br d, 1 H), 1.45-1.83 (m, 6 H) | 446.2 [M+H]⁺ | 99.7% |
| 274 | (CD₃OD) δ 7.70 (s, 1 H), 7.49-7.60 (m, 1 H), 7.43 (td, 1 H), 7.08-7.24 (m, 1 H), 6.73 (br s, 1 H), 4.47 (br s, 0.5 H), 4.02 (br s, 1 H), 3.68 (br s, 0.5 H), 3.15 (br s, 1 H), 2.89 (br d, 1 H), 1.65 (br s, 6 H) | 446.2 [M+H]⁺ | 99.7% |
| 275 | (CD₃OD) δ 8.53 (s, 1 H), 8.32 (s, 1 H), 7.85 (d, 1 H), 7.62 (s, 1 H), 7.38-7.44 (m, 1 H), 7.28-7.36 (m, 2 H), 7.01-7.09 (m, 1 H), 6.70 (s, 1 H), 4.62 (br dd, 1 H), 4.43 (s, 2 H), 3.84 (br s, 1 H), 3.33 (s, 3 H), 2.69-3.10 (m, 2 H) | 447.30 [M+H]⁺ | 99.2% |
| 276 | (CD₃OD) δ 8.52 (s, 1 H), 8.32 (s, 1 H), 7.85 (d, 1 H), 7.57 (s, 1 H), 7.38-7.43 (m, 1 H), 7.27-7.35 (m, 2 H), 6.99-7.09 (m, 1 H), 6.68 (br s, 1 H), 4.62 (br dd, 1 H), 4.43 (s, 2 H), 3.85 (br s, 1 H), 3.33 (s, 3 H), 2.69-3.10 (m, 2 H) | 447.20 [M+H]⁺ | 99.3% |
| 277 | (CD₃OD) δ 7.71 (s, 1 H), 7.36-7.58 (m, 2 H), 7.08-7.23 (m, 1.3 H), 6.84 (s, 0.7 H), 5.08 (dd, 0.3 H), 4.77 (dd, 0.7 H), 4.02-4.11 (m, 3 H), 3.80-3.96 (m, 0.7 H), 3.45-3.59 (m, 0.3 H), 2.79-3.26 (m, 2 H) | 402.10 [M+H]⁺ | 100% |
| 278 | (CD₃OD) δ 7.59 (s, 1 H), 7.24-7.44 (m, 2 H), 6.96-7.10 (m, 1.3 H), 6.71 (br s, 0.7 H), 4.96 (dd, 0.3 H), 4.65 (br dd, 0.7 H), 3.91-3.99 (m, 3 H), 3.76 (br d, 0.7 H), 3.40 (br d, 0.3 H), 2.64-3.15 (m, 2 H) | 402.20 [M+H]⁺ | 98.4% |
| 279 | (CD₃OD) δ 7.67-7.76 (m, 1 H), 7.48-7.58 (m, 1 H), 7.33-7.44 (m, 1 H), 7.25 (t, 1 H), 6.81-7.12 (m, 1 H), 5.12 (dd, 0.3 H), 4.77 (dd, 0.7 H), 4.14-4.20 (m, 3 H), 3.55-3.99 (m, 1 H), 2.96-3.28 (m, 1 H), 2.79-2.94 (m, 1 H) | 436.20 [M+H]⁺ | 99.6% |
| 280 | (CD₃OD) δ 7.65-7.77 (m, 1 H), 7.46-7.59 (m, 1 H), 7.32-7.43 (m, 1 H), 7.18-7.30 (m, 1 H), 6.91 (s, 1 H), 5.11 (dd, 0.3 H), 4.77 (dd, 0.7 H), 4.06-4.21 (m, 3 H), 3.56-3.97 (m, 1 H), 2.96-3.27 (m, 1 H), 2.80-2.94 (m, 1 H) | 436.2 [M+H]⁺ | 99.0% |
| 281 | (CD₃OD) δ 8.39 (s, 1 H), 7.83 (br s, 1 H), 7.66 (s, 1 H), 7.49 (br d, 1 H), 7.34 (td, 1 H), 7.11-7.25 (m, 1 H), 6.76 - 6.89 (m, 1 H), 4.69 (br s, 1 H), 3.96 (br s, 1 H), 3.08 (br s, 1 H), 2.85 (br d, 1 H) | 354.2 [M+H]⁺ | 98.6% |
| 282 | (CD₃OD) δ 8.39 (s, 1 H), 7.83 (br s, 1 H), 7.66 (s, 1 H), 7.49 (br d, 1 H), 7.34 (td, 1 H), 7.16-7.25 (m, 1 H), 6.76 (br s, 1 H), 4.68 (br s, 1 H), 3.94 (br s, 1 H), 3.07 (br s, 1 H), 2.85 (br d, 1 H) | 354.2 [M+H]⁺ | 99.1% |
| 283 | (CD₃OD) δ 7.71 (s, 1 H), 7.49-7.60 (m, 1 H), 7.33-7.46 (m, 1 H), 6.79-7.29 (m, 2 H), 5.08 (dd, 0.3 H), 4.78 (dd, 0.7 H), 4.03-4.09 (m, 3 H), 3.83-3.97 (m, 0.7 H), 3.46-3.60 (m, 0.3 H), 2.93-3.27 (m, 1 H), 2.78-2.90 (m, 1 H) | 402.1 [M+H]⁺ | 100% |
| 284 | (CD₃OD) δ 7.71 (s, 1 H), 7.49-7.56 (m, 1 H), 7.32-7.44 (m, 1 H), 6.74-7.31 (m, 2 H), 5.08 (dd, 0.3 H), 4.78 (dd, 0.7 H), 4.02-4.08 (m, 3 H), 3.89 (br t, 0.7 H), 3.45-3.63 (m, 0.3 H), 2.94-3.26 (m, 1 H), 2.77-2.92 (m, 1 H) | 402.1 [M+H]⁺ | 99.6% |
| 285 | (CD₃OD) δ 7.60 (s, 1 H), 7.35-7.45 (m, 1 H), 7.21-7.31 (m, 1 H), 7.09-7.16 (m, 1 H), 6.77-7.08 (m, 1 H), 6.47-6.76 (m, 1 H), 4.99 (dd, 0.3 H), 4.66 (dd, 0.7 H), 3.97-4.05 (m, 3 H), 3.36-3.83 (m, 1 H), 2.83-3.16 (m, 1 H), 2.62-2.80 (m, 1 H) | 418.3 [M+H]⁺ | 97.8% |
| 286 | (CD₃OD) δ 7.57-7.62 (m, 1 H), 7.36-7.43 (m, 1 H), 7.20-7.30 (m, 1 H), 7.08-7.16 (m, 1 H), 6.77-7.08 (m, 1 H), 6.47-6.77 (m, 1 H), 4.99 (dd, 0.3 H), 4.66 (dd, 0.7 H), 3.96-4.05 (m, 3 H), 3.34-3.83 (m, 1 H), 2.82-3.17 (m, 1 H), 2.65-2.80 (m, 1 H) | 418.4 [M+H]⁺ | 93.6% |
| 287 | (CD₃OD) δ 7.90 (s, 1 H), 7.64-7.75 (m, 2 H), 7.40-7.53 (m, 1 H), 7.19 (td, 1 H), 6.79-6.94 (m, 1.8 H), 6.07 (s, 0.2 H), 4.93-4.99 (m, 0.4 H), 3.84-4.00 (m, 1.4 H), 3.45-3.58 (m, 0.2 H), 2.71-3.02 (m, 2 H) | 421.0 [M+H]⁺ | 99.1% |
| 288 | (CD₃OD) δ 7.90 (s, 1 H), 7.64-7.76 (m, 2 H), 7.38-7.57 (m, 1 H), 7.10-7.31 (m, 1 H), 6.73-6.98 (m, 1.8 H), 6.07 (br s, 0.2 H), 4.96 (br d, 0.4 H), 3.87-4.02 (m, 1.4 H), 3.45-3.57 (m, 0.2 H), 2.70-3.04 (m, 2 H) | 421.0 [M+H]⁺ | 98.5% |
| 289 | (CD₃OD) δ 7.90 (s, 1 H), 7.63-7.78 (m, 1 H), 7.53 (br d, 1 H), 7.38 (td, 1 H), 7.11-7.31 (m, 1 H), 6.77-6.98 (m, 1.8 H), 6.04-6.25 (m, 0.2 H), 4.97 (br d, 0.5 H), 3.97-4.03 (m, 0.5 H), 3.86-3.96 (m, 0.7 H), 3.43-3.62 (m, 0.3 H), 2.72-3.07 (m, 2 H) | 421.0 [M+H] | 99.3% |
| 290 | (CD₃OD) δ 7.90 (s, 1 H), 7.64-7.79 (m, 1 H), 7.53 (br d, 1 H), 7.38 (td, 1 H), 7.18-7.31 (m, 1 H), 6.75-6.99 (m, 1.8 H), 6.13 (s, 0.2 H), 4.97 (br s, 0.3 H), 3.97-4.04 (m, 0.7 H), 3.85-3.96 (m, 0.7 H), 3.47-3.58 (m, 0.3 H), 2.73-3.02 (m, 2 H) | 421.0 [M+H] | 97.7% |
| 291 | (CD₃OD) δ 7.63-7.76 (m, 2 H), 7.44 (dd, 1 H), 6.59-7.31 (m, 3 H), 4.98 (br s, 0.4 H), 4.57 (br s, 0.6 H), 3.52-4.09 (m, 1 H), 2.76-3.24 (m, 2 H), 2.03-2.42 (m, 6 H), 1.39-1.76 (m, 6 H) | 489.10 [M+H]⁺ | 100% |
| 292 | (CD₃OD) δ 7.51-7.64 (m, 2 H), 7.31 (br d, 1 H), 6.41-7.22 (m, 3 H), 4.83-4.90 (m, 0.4 H), 4.46 (br s, 0.6 H), 3.40-3.96 (m, 1 H), 2.66-3.14 (m, 2 H), 1.92-2.31 (m, 6 H), 1.27-1.67 (m, 6 H) | 489.20 [M+H]⁺ | 98.0% |
| 293 | (CD₃OD) δ 7.90 (s, 1 H), 7.61-7.78 (m, 1 H), 7.36-7.59 (m, 2 H), 7.16 (t, 1 H), 6.73-6.98 (m, 1.8 H), 6.10 (br s, 0.2 H), 4.98 (br d, 0.2 H), 3.87-4.06 (m, 1.5 H), 3.45-3.63 (m, 0.3 H), 2.72-3.03 (m, 2 H) | 421.0 [M+H]⁺ | 99.1% |
| 294 | (CD₃OD) δ 7.90 (s, 1 H), 7.63-7.78 (m, 1 H), 7.35-7.59 (m, 2 H), 7.15 (t, 1 H), 6.87 (br d, 1.8 H), 6.10 (br s, 0.2 H), 4.99 (br dd, 0.2 H), 3.87-4.07 (m, 1.5 H), 3.45-3.63 (m, 0.3 H), 2.73-3.04 (m, 2 H) | 421.1 [M+H]⁺ | 99.2% |
| 295 | (CD₃OD) δ 8.47 (br s, 1 H), 7.55-7.87 (m, 3 H), 6.91-7.32 (m, 1 H), 6.76 (br s, 1 H), 4.54 (br s, 1 H), 3.68-4.02 (m, 1 H), 2.78-3.12 (m, 2 H) | 422.0 [M+H]⁺ | 93.5% |
| 296 | (CD₃OD) δ 8.47 (br s, 1 H), 7.58-7.74 (m, 3 H), 6.95-7.33 (m, 1 H), 6.76 (br s, 1 H), 4.57 (br s, 1 H), 3.66-4.07 (m, 1 H), 3.13 (br d, 1 H), 2.86 (br d, 1 H) | 422.0 [M+H]⁺ | 94.0% |
| 297 | (CD₃OD) δ 7.69 (s, 1 H), 7.49 (br dd, 1 H), 7.29-7.42 (m, 1 H), 6.93-7.27 (m, 1 H), 6.76 (br s, 1 H), 4.50-4.59 (m, 1 H), 3.54-4.06 (m, 1 H), 2.76-3.26 (m, 2 H), 1.49-1.76 (m, 6 H) | 480.1 [M+H] | 88.4% |
| 298 | (CD₃OD) δ 7.69 (s, 1 H), 7.49 (br dd, 1 H), 7.34 (ddd, 1 H), 6.91-7.28 (m, 1 H), 6.76 (br s, 1 H), 4.46-4.58 (m, 1 H), 3.53-4.06 (m, 1 H), 2.76-3.24 (m, 2 H), 1.51-1.78 (m, 6 H) | 480.1 [M+H] | 94.8% |
| 299 | (CD₃OD) δ 7.69 (s, 1 H), 7.38 (br d, 1 H), 7.24 (br s, 0.2 H), 7.03-7.14 (m, 1.6 H), 6.97 (br s, 0.2 H), 6.73 (br s, 1 H), 4.55 (br s, 1 H), 3.95 (br s, 0.7 H), 3.64 (br s, 0.3 H), 2.94-3.24 (m, 1 H), 2.87 (br d, 1 H), 1.51-1.74 (m, 6 H) | 480.1 [M+H]⁺ | 97.7% |
| 300 | (CD₃OD) δ 7.69 (s, 1 H), 7.38 (br d, 1 H), 7.24 (br s, 0.2 H), 7.03-7.13 (m, 1.6 H), 6.97 (br s, 0.2 H), 6.73 (br s, 1 H), 4.48-4.58 (m, 1 H), 3.95 (br s, 0.7 H), 3.64 (br s, 0.3 H), 2.93-3.23 (m, 1 H), 2.87 (br d, 1 H), 1.50-1.76 (m, 6 H) | 480.1 [M+H]⁺ | 98.5% |
| 301 | (CD₃OD) δ 8.46 (br s, 1 H), 7.69 (s, 1 H), 7.38 (br d, 1 H), 7.26 (s, 0.2 H), 6.96-7.15 (m, 1.8 H), 6.75 (br s, 1 H), 4.56 (br d, 1 H), 3.93 (br d, 1 H), 3.11 (br s, 1 H), 2.86 (br d, 1 H) | 422.0 [M+H]⁺ | 100% |
| 302 | (CD₃OD) δ 8.46 (br s, 1 H), 7.69 (s, 1 H), 7.38 (br d, 1 H), 7.26 (s, 0.2 H), 6.97-7.15 (m, 1.9 H), 6.74 (br s, 1 H), 4.55 (br d, 1 H), 3.94 (br s, 1 H), 3.11 (br s, 1 H), 2.85 (br d, 1 H) | 422.0 [M+H]⁺ | 99.1% |
| 303 | (CD₃OD) δ 8.76-8.81 (m, 1 H), 8.28-8.35 (m, 1 H), 8.06-8.13 (m, 1 H), 7.65-7.75 (m, 3 H), 7.43-7.58 (m, 1 H), 7.12-7.29 (m, 1 H), 6.93 (s, 1 H), 5.10-5.34 (m, 1 H), 3.89-4.07 (m, 0.7 H), 3.61-3.74 (m, 0.3 H), 3.00-3.29 (m, 1 H), 2.83-2.97 (m, 1 H) | 432.1 [M+H]⁺ | 100% |
| 304 | (CD₃OD) δ 8.76-8.81 (m, 1 H), 8.28-8.35 (m, 1 H), 8.06-8.13 (m, 1 H), 7.65-7.75 (m, 3 H), 7.47-7.59 (m, 1 H), 7.16-7.24 (m, 1 H), 6.92 (br s, 1 H), 5.10-5.40 (m, 1 H), 3.99 (br t, 0.7 H), 3.66 (br s, 0.3 H), 3.00-3.30 (m, 1 H), 2.81-2.97 (m, 1 H) | 432.1 [M+H]⁺ | 97.7% |
| 305 | (CD₃OD) δ 8.45 (s, 1 H), 7.69 (s, 1 H), 7.49 (ddd, 1 H), 7.33 (ddd, 1 H), 6.91-7.28 (m, 1 H), 6.77 (br s, 1 H), 4.56 (br d, 1 H), 3.56-4.02 (m, 1 H), 2.82-3.16 (m, 2 H) | 422.1 [M+H]⁺ | 99.5% |
| 306 | (CD₃OD) δ 8.45 (s, 1 H), 7.69 (s, 1 H), 7.49 (ddd, 1 H), 7.33 (ddd, 1 H), 6.94-7.27 (m, 1 H), 6.77 (br s, 1 H), 4.44-4.56 (m, 1 H), 3.52-4.03 (m, 1 H), 2.80-3.18 (m, 2 H) | 422.0 [M+H]⁺ | 99.2% |
| 307 | (CD₃OD) δ 7.70 (s, 1 H), 7.33 (br d, 1 H), 6.95-7.29 (m, 2 H), 6.76 (br s, 1 H), 4.96-5.00 (m, 0.5 H), 4.50-4.61 (m, 0.5 H), 3.53-4.12 (m, 1 H), 3.15 (br s, 1 H), 2.91 (br s, 1 H), 1.47-1.83 (m, 6 H) | 480.1 [M+H]⁺ | 94.0% |
| 308 | (CD₃OD) δ 7.70 (s, 1 H), 7.33 (br d, 1 H), 6.96-7.30 (m, 2 H), 6.75 (br s, 1 H), 4.96-5.03 (m, 0.6 H), 4.52-4.61 (m, 0.4 H), 3.54-4.14 (m, 0.8 H), 2.95-3.24 (m, 1.2 H), 2.92 (br s, 1 H), 1.53-1.79 (m, 6 H) | 480.1 [M+H]⁺ | 97.6% |
| 309 | (CD₃OD) δ 8.47 (br s, 1 H), 7.71 (s, 1 H), 7.33 (br d, 1 H), 6.97-7.29 (m, 2 H), 6.78 (br s, 1 H), 4.95-5.04 (m, 0.5 H), 4.50-4.60 (m, 0.5 H), 3.51-4.11 (m, 1 H), 3.12 (br s, 1 H), 2.88 (br d, 1 H) | 422.1 [M+H]⁺ | 98.0% |
| 310 | (CD₃OD) δ 8.47 (br s, 1 H), 7.71 (s, 1 H), 7.33 (br d, 1 H), 6.95-7.29 (m, 2 H), 6.78 (br s, 1 H), 4.96-5.07 (m, 0.4 H), 4.48-4.60 (m, 0.6 H), 3.53-4.11 (m, 1 H), 3.12 (br s, 1 H), 2.88 (br d, 1 H) | 422.1 [M+H]⁺ | 98.8% |
| 311 | (CD₃OD) δ 7.69 (br s, 1 H), 7.42-7.57 (m, 2 H), 7.25 (d, 1 H), 6.94-7.17 (m, 1 H), 6.75 (br s, 1 H), 4.47-4.66 (m, 1 H), 3.92 (br s, 1 H), 2.74-3.15 (m, 2 H), 2.46 (s, 3 H), 1.49-1.72 (m, 6 H) | 458.1 [M+H]⁺ | 98.0% |
| 312 | (CD₃OD) δ 7.69 (s, 1 H), 7.44-7.56 (m, 2 H), 7.25 (d, 1.2 H), 7.11 (br s, 0.4 H), 6.98 (br s, 0.2 H), 6.55-6.84 (m, 0.8 H), 4.40-4.67 (m, 1 H), 3.93 (br s, 1 H), 2.65-3.15 (m, 2 H), 2.46 (s, 3 H), 1.41-1.77 (m, 6 H) | 458.1 [M+H]⁺ | 89.9% |
| 313 | (CD₃OD) δ 7.77-7.85 (m, 1 H), 7.61-7.73 (m, 2 H), 7.46-7.54 (m, 1 H), 7.25 (d, 1 H), 6.73-6.95 (m, 2 H), 5.98-6.46 (m, 1 H), 4.28 (br d, 1 H), 3.90 (br s, 1 H), 2.93-3.12 (m, 1 H), 2.80 (br d, 1 H), 2.46 (s, 3 H) | 399.1 [M+H]⁺ | 96.8% |
| 314 | (CD₃OD) δ 7.78-7.88 (m, 1 H), 7.61-7.72 (m, 2 H), 7.46-7.57 (m, 2 H), 7.26 (d, 1 H), 6.87 (br s, 2 H), 4.27 (br s, 1 H), 3.91 (br s, 1 H), 3.02 (br d, 1 H), 2.81 (br d, 1 H), 2.47 (s, 3 H) | 399.1 [M+H]⁺ | 92.5% |
| 315 | (CD₃OD) δ 7.68 (s, 1 H), 7.54 (d, 1 H), 7.46 (s, 1 H), 6.95-7.27 (m, 2 H), 6.54-6.86 (m, 1 H), 4.55 (br d, 1 H), 3.54-3.97 (m, 1 H), 2.78-3.22 (m, 2 H), 2.48 (s, 3 H), 1.51-1.73 (m, 6 H) | 458.1 [M+H] | 99.6% |
| 316 | (CD₃OD) δ 7.68 (s, 1 H), 7.53 (d, 1 H), 7.45 (s, 1 H), 6.94-7.30 (m, 2 H), 6.57-6.82 (m, 1 H), 4.54 (br d, 1 H), 3.52-4.00 (m, 1 H), 2.79-3.22 (m, 2 H), 2.48 (s, 3 H), 1.51-1.72 (m, 6 H) | 458.1 [M+H] | 98.8% |
| 317 | (CD₃OD) δ 7.75-7.88 (m, 1.2 H), 7.61-7.74 (m, 1.5 H), 7.40-7.61 (m, 2.3 H), 7.23 (d, 1 H), 6.84 (br d, 1.7 H), 6.28 (br s, 0.3 H), 4.27 (br d, 1 H), 3.90 (br s, 1 H), 2.96-3.09 (m, 1 H), 2.74-2.88 (m, 1 H), 2.49 (s, 3 H) | 399.1 [M+H] | 99.7% |
| 318 | (CD₃OD) δ 7.75-7.88 (m, 1.2 H), 7.61-7.74 (m, 1.5 H), 7.39-7.59 (m, 2.3 H), 7.22 (d, 1 H), 6.84 (br d, 1.7 H), 6.28 (br s, 0.3 H), 4.27 (br d, 1 H), 3.42-3.97 (m, 1 H), 2.95-3.08 (m, 1 H), 2.74-2.85 (m, 1 H), 2.49 (s, 3 H) | 399.1 [M+H] | 99.2% |
| 319 | (CD₃OD) δ 7.67 (s, 1 H), 7.53 (d, 1 H), 7.45 (s, 1 H), 7.21 (d, 1 H), 6.70 (br s, 1 H), 4.44-4.59 (m, 1 H), 3.52-3.99 (m, 1 H), 2.75-3.19 (m, 2 H), 2.48 (s, 3 H), 2.37 (s, 3 H), 1.62 (br s, 6 H) | 422.1 [M+H] | 98.9% |
| 320 | (CD₃OD) δ 7.67 (s, 1 H), 7.54 (d, 1 H), 7.45 (s, 1 H), 7.21 (d, 1 H), 6.70 (br s, 1 H), 4.34-4.57 (m, 1 H), 3.52-3.99 (m, 1 H), 3.13 (br s, 1 H), 2.83 (br d, 1 H), 2.48 (s, 3 H), 2.37 (s, 3 H), 1.61 (br s, 6 H) | 422.1 [M+H] | 91.4% |
| 321 | (CD₃OD) δ 7.66-7.75 (m, 1 H), 7.53-7.64 (m, 2 H), 7.46 (br s, 0.3 H), 7.30-7.41 (m, 1 H), 7.03-7.12 (m, 1 H), 6.84-6.95 (m, 1 H), 6.79 (br s, 0.7 H), 4.95-5.22 (m, 1 H), 3.79-3.98 (m, 3.7 H), 3.44-3.58 (m, 0.3 H), 2.85-3.17 (m, 1 H), 2.65-2.83 (m, 1 H) | 435.1 [M+H]⁺ | 99.6% |
| 322 | (CD₃OD) δ 7.65-7.76 (m, 1 H), 7.53-7.63 (m, 2 H), 7.46 (br s, 0.3 H), 7.33-7.40 (m, 1 H), 7.01-7.13 (m, 1 H), 6.84-6.91 (m, 1 H), 6.78 (br s, 0.7 H), 4.95-5.21 (m, 1 H), 3.78-3.95 (m, 3.7 H), 3.44-3.58 (m, 0.3 H), 2.85-3.16 (m, 1 H), 2.70-2.84 (m, 1 H) | 435.1 [M+H]⁺ | 98.2% |
| 323 | (CD₃OD) δ 8.34-8.51 (m, 2 H), 7.87 (d, 1 H), 7.67 (s, 1 H), 7.53 (d, 1 H), 7.32-7.41 (m, 2 H), 7.18-7.28 (m, 1 H), 6.79 (br s, 1 H), 4.72 (br s, 1 H), 3.93 (br s, 1 H), 3.03-3.21 (m, 1 H), 2.86 (br d, 1 H), 2.40 (s, 3 H) | 417.1 [M+H]⁺ | 99.8% |
| 324 | (CD₃OD) δ 8.33-8.51 (m, 2 H), 7.87 (d, 1 H), 7.67 (s, 1 H), 7.54 (d, 1 H), 7.31-7.44 (m, 2 H), 7.16-7.28 (m, 1 H), 6.79 (br s, 1 H), 4.72 (br s, 1 H), 3.94 (br s, 1 H), 3.05-3.20 (m, 1 H), 2.86 (br d, 1 H), 2.40 (s, 3 H) | 417.1 [M+H]⁺ | 96.5% |
| 325 | (CD₃OD) δ 8.27-8.38 (m, 2 H), 7.86 (d, 1 H), 7.67 (s, 1 H), 7.53 (d, 1 H), 7.37 (td, 1 H), 7.17-7.27 (m, 2 H), 6.79 (s, 1 H), 4.74 (br dd, 1 H), 3.89 (s, 4 H), 3.05-3.18 (m, 1 H), 2.85 (br dd, 1 H) | 433.1 [M+H]⁺ | 99.5% |
| 326 | (CD₃OD) δ 8.27-8.39 (m, 2 H), 7.86 (d, 1 H), 7.67 (s, 1 H), 7.53 (d, 1 H), 7.37 (td, 1 H), 7.17-7.26 (m, 2 H), 6.79 (s, 1 H), 4.74 (br dd, 1 H), 3.89 (s, 4 H), 3.05-3.18 (m, 1 H), 2.85 (dd, 1 H) | 433.1 [M+H]⁺ | 99.3% |
| 327 | (CD₃OD) δ 8.02 (br s, 1 H), 7.86 (br d, 1 H), 7.64-7.80 (m, 2 H), 6.92-7.32 (m, 1 H), 6.77 (br s, 1 H), 4.48 (s, 1 H), 3.97 (br s, 1 H), 3.17 (br s, 1 H), 2.86 (br s, 1 H), 1.39-1.75 (m, 6 H) | 512.1 [M+H]⁺ | 97.4% |
| 328 | (CD₃OD) δ 8.02 (br s, 1 H), 7.86 (d, 1 H), 7.65-7.78 (m, 2 H), 6.91-7.30 (m, 1 H), 6.77 (br s, 1 H), 4.92-5.03 (m, 0.6 H), 4.57 (br s, 0.4 H), 3.53-4.08 (m, 1 H), 2.95-3.25 (m, 1 H), 2.88 (br d, 1 H), 1.40-1.80 (m, 6 H) | 512.1 [M+H]⁺ | 97.1% |
| 329 | (CD₃OD) δ 8.46 (br s, 1 H), 8.02 (s, 1 H), 7.85 (d, 1 H), 7.63-7.78 (m, 2 H), 6.94-7.29 (m, 1 H), 6.79 (br s, 1 H), 4.57 (br d, 1 H), 3.96 (br s, 0.8 H), 3.60 (br s, 0.2 H), 3.11 (br s, 1 H), 2.86 (br d, 1 H) | 454.0 [M+H]⁺ | 96.5% |
| 330 | (CD₃OD) δ 8.46 (br s, 1 H), 8.02 (s, 1 H), 7.86 (d, 1 H), 7.65-7.78 (m, 2 H), 6.95-7.29 (m, 1 H), 6.80 (br s, 1 H), 4.59 (br s, 1 H), 3.96 (br s, 0.8 H), 3.62 (br d, 0.2 H), 3.11 (br s, 1 H), 2.86 (br d, 1 H) | 454.0 [M+H]⁺ | 97.1% |
| 331 | (CD₃OD) δ 7.67 (s, 1 H), 7.44-7.56 (m, 2 H), 7.24 (d, 1 H), 6.70 (br s, 1 H), 4.41-4.73 (m, 1 H), 3.42-4.02 (m, 1 H), 2.77-3.21 (m, 2 H), 2.45 (s, 3 H), 2.37 (s, 3 H), 1.62 (br s, 6 H) | 422.1 [M+H]⁺ | 96.9% |
| 332 | (CD₃OD) δ 7.69 (s, 1 H), 7.46-7.58 (m, 2 H), 7.26 (d, 1 H), 6.72 (br s, 1 H), 4.48-4.69 (m, 1 H), 3.92 (br s, 1 H), 2.77-3.21 (m, 2 H), 2.47 (s, 3 H), 2.39 (s, 3 H), 1.37-1.77 (m, 6 H) | 422.1 [M+H]⁺ | 92.2% |
| 333 | (CD₃OD) δ 8.73-8.84 (m, 1 H), 8.26-8.38 (m, 1 H), 8.00-8.17 (m, 1 H), 7.63-7.79 (m, 2 H), 7.38-7.59 (m, 2.3 H), 7.08-7.22 (m, 1 H), 6.93 (br s, 0.7 H), 5.12-5.42 (m, 1 H), 3.62-4.13 (m, 1 H), 2.79-3.27 (m, 2 H) | 432.0 [M+H]⁺ | 95.1% |
| 334 | (CD₃OD) δ 8.59-8.77 (m, 1 H), 8.12-8.29 (m, 1 H), 7.90-8.04 (m, 1 H), 7.52-7.67 (m, 2 H), 7.24-7.47 (m, 2.3 H), 6.95-7.14 (m, 1 H), 6.81 (br s, 0.7 H), 5.01-5.28 (m, 1 H), 3.51-4.02 (m, 1 H), 2.74-3.17 (m, 2 H) | 432.1 [M+H]⁺ | 94.1% |
| 335 | (CD₃OD) δ 8.71-8.80 (m, 1 H), 8.25-8.35 (m, 1 H), 8.03-8.12 (m, 1 H), 7.62-7.75 (m, 2 H), 7.47-7.58 (m, 1 H), 7.32-7.41 (m, 1 H), 7.17-7.27 (m, 1 H), 6.93 (br s, 1 H), 5.28 (dd, 0.7 H), 5.14 (dd, 0.3 H), 4.01 (br s, 0.7H), 3.62-3.79 (m, 0.3 H), 2.85-3.27 (m, 2 H) | 432.1 [M+H]⁺ | 97.5% |
| 336 | (CD₃OD) δ 8.72-8.80 (m, 1 H), 8.26-8.34 (m, 1 H), 8.03-8.12 (m, 1 H), 7.62-7.74 (m, 2 H), 7.46-7.58 (m, 1 H), 7.33-7.41 (m, 1 H), 7.18-7.27 (m, 1 H), 6.93 (br s, 1 H), 5.28 (dd, 0.7 H), 5.14 (dd, 0.3 H), 4.01 (br s, 0.7 H), 3.70 (br s, 0.3 H), 2.87-3.26 (m, 2 H) | 432.1 [M+H]⁺ | 88.4% |
| 337 | (CD₃OD) δ 7.78-7.83 (m, 1 H), 7.67-7.73 (m, 1 H), 7.49-7.54 (m, 1 H), 7.32-7.41 (m, 1 H), 7.18-7.27 (m, 1 H), 6.85-7.03 (m, 2 H), 5.30 (dd, 0.7 H), 5.12 (br dd, 0.3 H), 4.00-4.03 (m, 3 H), 3.93-3.99 (m, 0.4 H), 3.67 (br s, 0.3 H), 2.83-3.27 (m, 2.3 H) | 435.1 [M+H]⁺ | 95.2% |
| 338 | (CD₃OD) δ 7.63-7.84 (m, 2 H), 7.52 (br d, 1 H), 7.33-7.41 (m, 1 H), 7.18-7.27 (m, 1 H), 6.87-7.02 (m, 2 H), 5.30 (br dd, 0.7 H), 5.12 (br d, 0.3 H), 4.00-4.03 (m, 3 H), 3.97 (s, 0.3 H), 3.57-3.86 (m, 0.7 H), 2.88-3.24 (m, 2 H) | 435.1 [M+H]⁺ | 98.9% |
| 339 | (CD₃OD) δ 7.71 (s, 1 H), 7.54 (d, 1 H), 7.10-7.32 (m, 2 H), 6.93-7.06 (m, 1 H), 6.46-6.84 (m, 1 H), 4.57 (br d, 0.7 H), 3.85 (s, 4 H), 3.53-3.70 (m, 0.3 H), 2.96-3.24 (m, 1 H), 2.88 (br d, 1 H), 1.47-1.75 (m, 6 H) | 474.1 [M+H]⁺ | 100% |
| 340 | (CD₃OD) δ 7.70 (s, 1 H), 7.54 (d, 1 H), 7.09-7.33 (m, 2 H), 6.92-7.05 (m, 1 H), 6.49-6.85 (m, 1 H), 4.57 (br d, 0.7 H), 3.78-4.06 (m, 4 H), 3.51-3.72 (m, 0.3 H), 2.96-3.24 (m, 1 H), 2.88 (br d, 1 H), 1.49-1.74 (m, 6 H) | 474.1 [M+H]⁺ | 98.1% |
| 341 | (CD₃OD) δ 8.46 (br s, 1 H), 7.71 (s, 1 H), 7.54 (d, 1 H), 7.10-7.37 (m, 2 H), 7.02 (dd, 1 H), 6.77 (br s, 1 H), 4.55 (br d, 0.7 H), 3.79-4.08 (m, 4 H), 3.54-3.69 (m, 0.3 H), 3.11 (br s, 1 H), 2.86 (br d, 1 H) | 416.0 [M+H]⁺ | 98.5% |
| 342 | (CD₃OD) δ 8.34 (br s, 1 H), 7.58 (s, 1 H), 7.41 (d, 1 H), 6.98-7.18 (m, 2 H), 6.90 (dd, 1 H), 6.42-6.76 (m, 1 H), 4.43 (br d, 0.7 H), 3.68-3.88 (m, 4 H), 3.34-3.60 (m, 0.3 H), 3.00 (br d, 1 H), 2.65-2.80 (m, 1 H) | 416.0 [M+H]⁺ | 96.6% |
| 343 | (CD₃OD) δ 7.49-7.66 (m, 2 H), 7.31-7.41 (m, 1 H), 6.94-7.17 (m, 1.3 H), 6.70 (br s, 0.7 H), 4.95 (br dd, 0.3 H), 4.64 (dd, 0.7 H), 3.87-3.99 (m, 3 H), 3.74 (br s, 0.7 H), 3.38 (br d, 0.3 H), 2.56-3.14 (m, 2 H) | 402.1 [M+H]⁺ | 100% |
| 344 | (CD₃OD) δ 7.70 (br s, 1 H), 7.23-7.27 (m, 1 H), 7.00-7.18 (m, 2 H), 6.75 (br s, 1 H), 5.02 (br s, 0.4 H), 4.60 (br s, 0.6 H), 3.62-4.19 (m, 1 H), 2.75-3.25 (m, 2 H), 1.48-1.88 (m, 6 H) | 480.3 [M+H]⁺ | 96.1% |
| 345 | (CD₃OD) δ 7.70 (br s, 1 H), 7.23-7.27 (m, 1 H), 7.00-7.18 (m, 2 H), 6.75 (br s, 1 H), 5.02 (br s, 0.4 H), 4.60 (br s, 0.6 H), 3.62-4.19 (m, 1 H), 2.75-3.25 (m, 2 H), 1.48-1.88 (m, 6 H) | 480.3 [M+H]⁺ | 96.1% |
| 346 | (CD₃OD) δ 7.58 (br s, 1 H), 7.11-7.15 (m, 1 H), 6.87-7.04 (m, 2 H), 6.75 (br s, 1 H), 4.81-4.95 (m, 0.4 H), 4.39-4.50 (m, 0.6 H), 3.48-4.04 (m, 1 H), 2.72-3.15 (m, 2 H), 1.41-1.69 (m, 6 H) | 480.2 [M+H]⁺ | 96.3% |
| 347 | (CD₃OD) δ 7.71 (s, 1 H), 7.50 (dd, 1 H), 7.32-7.46 (m, 1 H), 6.93-7.32 (m, 1 H), 6.73 (br s, 1 H), 4.95-5.01 (m, 0.5 H), 4.58 (br s, 0.5 H), 3.58-4.09 (m, 1 H), 3.17 (br s, 1 H), 2.89 (br d, 1 H), 1.51-1.79 (m, 6 H) | 480.2 [M+H]⁺ | 99.2% |
| 348 | (CD₃OD) δ 7.71 (s, 1 H), 7.51 (br d, 1 H), 7.34-7.46 (m, 1 H), 6.94-7.33 (m, 1 H), 6.74 (br s, 1 H), 4.96-5.01 (m, 0.5 H), 4.59 (br s, 0.5 H), 3.56-4.13 (m, 1 H), 3.16 (br d, 1 H), 2.90 (br d, 1 H), 1.49-1.74 (m, 6 H) | 480.2 [M+H]⁺ | 98.5% |
| 349 | (CD₃OD) δ 8.47 (br s, 1 H), 7.71 (s, 1 H), 7.50 (br d, 1 H), 7.33-7.45 (m, 1 H), 6.94-7.32 (m, 1 H), 6.76 (br s, 1 H), 4.58 (br d, 1 H), 3.54-4.14 (m, 1 H), 3.12 (br s, 1 H), 2.88 (br d, 1 H) | 422.1 [M+H]⁺ | 96.5% |
| 350 | (CD₃OD) δ 8.47 (br s, 1 H), 7.72 (s, 1 H), 7.50 (br d, 1 H), 7.32-7.44 (m, 1 H), 6.98-7.32 (m, 1 H), 6.76 (br s, 1 H), 4.59 (br d, 1 H), 3.53-4.11 (m, 1 H), 3.13 (br s, 1 H), 2.88 (br d, 1 H) | 422.2 [M+H]⁺ | 97.4% |
| 351 | (CD₃OD) δ 7.75 (d, 1 H), 7.61-7.71 (m, 2 H), 7.37 (dd, 1 H), 6.95-7.27 (m, 1 H), 6.76 (br s, 1 H), 4.59 (br s, 1 H), 3.53-4.07 (m, 1 H), 2.78-3.22 (m, 2 H), 1.51-1.72 (m, 6 H) | 528.2 [M+H]⁺ | 98.3% |
| 352 | (CD₃OD) δ 7.75 (d, 1 H), 7.59-7.72 (m, 2 H), 7.37 (br d, 1 H), 6.92-7.27 (m, 1 H), 6.73 (br s, 1 H), 4.48-4.59 (m, 1 H), 3.59-4.04 (m, 1 H), 2.74-3.24 (m, 2 H), 1.49-1.75 (m, 6 H) | 528.1 [M+H]⁺ | 97.6% |
| 353 | (CD₃OD) δ 8.45 (br s, 1 H), 7.75 (d, 1 H), 7.59-7.71 (m, 2 H), 7.37 (br d, 1 H), 6.96-7.28 (m, 1 H), 6.75 (br s, 1 H), 4.42-4.58 (m, 1 H), 3.96 (br s, 1 H), 2.83-3.17 (m, 2 H) | 470.1 [M+H]⁺ | 97.7% |
| 354 | (CD₃OD) δ 8.45 (br s, 1 H), 7.75 (d, 1 H), 7.60-7.71 (m, 2 H), 7.37 (dd, 1 H), 6.96-7.29 (m, 1 H), 6.74 (br s, 1 H), 4.46-4.59 (m, 1 H), 3.56-4.05 (m, 1 H), 2.79-3.18 (m, 2 H) | 470.2 [M+H]⁺ | 95.7% |
| 355 | (CD₃OD) δ 7.88-7.93 (m, 1 H), 7.72 (s, 1 H), 7.46-7.56 (m, 2 H), 7.21-7.30 (m, 1 H), 7.01-7.09 (m, 1 H), 6.91 (br s, 1 H), 5.06-5.34 (m, 1 H), 3.90-4.05 (m, 0.7 H), 3.62 (br s, 0.3 H), 2.85-3.27 (m, 2 H), 2.42-2.53 (m, 3 H) | 417.2 [M+H]⁺ | 90.7% |
| 356 | (CD₃OD) δ 7.88-7.94 (m, 1 H), 7.73 (s, 1 H), 7.47-7.55 (m, 2 H), 7.22-7.29 (m, 1 H), 7.01-7.09 (m, 1 H), 6.91 (br s, 1 H), 5.08-5.31 (m, 1 H), 3.96 (br t, 0.7 H), 3.63 (br d, 0.3 H), 2.86-3.28 (m, 2 H), 2.42-2.54 (m, 3 H) | 417.2 [M+H]⁺ | 98.7% |
| 357 | (CD₃OD) δ 7.77-7.85 (m, 1 H), 7.70 (s, 1 H), 7.40-7.61 (m, 2.4 H), 7.13-7.28 (m, 1 H), 6.82-7.05 (m, 1.6 H), 5.04-5.32 (m, 1 H), 4.00-4.07 (m, 3 H), 3.52-3.99 (m, 1 H), 2.81-3.26 (m, 2 H), 2.44-2.52 (m, 3 H) | 431.3 [M+H]⁺ | 94.7% |
| 358 | (CD₃OD) δ 7.77-7.87 (m, 1 H), 7.70 (s, 1 H), 7.40-7.63 (m, 2.3 H), 7.14-7.28 (m, 1 H), 6.80-7.08 (m, 1.7 H), 5.05-5.31 (m, 1 H), 3.99-4.05 (m, 3 H), 3.53-3.98 (m, 1 H), 2.82-3.25 (m, 2 H), 2.40-2.53 (m, 3 H) | 431.3 [M+H]⁺ | 97.8% |
| 359 | (CD₃OD) δ 7.91 (br s, 1 H), 7.72 (s, 1 H), 7.44-7.59 (m, 2 H), 7.18-7.29 (m, 1 H), 7.00-7.10 (m, 1 H), 6.91 (br s, 1 H), 5.07-5.31 (m, 1 H), 3.54-4.00 (m, 1 H), 2.82-3.25 (m, 2 H), 2.46-2.54 (m, 3 H) | 417.4 [M+H]⁺ | 100% |
| 360 | (CD₃OD) δ 7.91 (br d, 1 H), 7.74 (s, 1 H), 7.45-7.61 (m, 2 H), 7.18-7.29 (m, 1 H), 7.02-7.10 (m, 1 H), 6.92 (s, 1 H), 5.05-5.35 (m, 1 H), 3.55-4.02 (m, 1 H), 2.85-3.27 (m, 2 H), 2.46-2.53 (m, 3 H) | 417.4 [M+H]⁺ | 100% |
| 361 | (CD₃OD) δ 7.70 (br s, 1 H), 7.45 (br d, 1 H), 6.96-7.38 (m, 3 H), 6.53-6.91 (m, 1 H), 4.59 (br d, 1 H), 3.59-4.17 (m, 1 H), 2.95-3.23 (m, 1 H), 2.91 (br s, 1 H), 2.58 (s, 3 H), 1.55-1.75 (m, 6 H) | 458.3 [M+H]⁺ | 100% |
| 362 | (CD₃OD) δ 7.70 (br s, 1 H), 7.45 (br d, 1 H), 6.96-7.38 (m, 3 H), 6.79 (br s, 1 H), 4.57 (br s, 1 H), 3.57-4.17 (m, 1 H), 2.95-3.28 (m, 1 H), 2.91 (br s, 1 H), 2.57 (s, 3 H), 1.50-1.73 (m, 6 H) | 458.2 [M+H]⁺ | 100% |
| 363 | (CD₃OD) δ 8.33 (br s, 1 H), 8.12 (d, 1 H), 7.85 (d, 1 H), 7.69 (s, 1 H), 7.54 (d, 1 H), 7.45 (dd, 1 H), 7.38 (td, 1 H), 7.19-7.28 (m, 1 H), 6.80 (br s, 1 H), 4.76 (br dd, 1 H), 3.92 (br d, 1 H), 3.07-3.25 (m, 5 H), 2.87 (br d, 1 H), 2.63-2.71 (m, 4 H), 2.38 (s, 3 H) | 501.4 [M+H]⁺ | 97.2% |
| 364 | (CD₃OD) δ 8.33 (br s, 1 H), 8.13 (d, 1 H), 7.86 (d, 1 H), 7.69 (s, 1 H), 7.55 (d, 1 H), 7.46 (dd, 1 H), 7.39 (td, 1 H), 7.24 (dd, 1 H), 6.80 (br s, 1 H), 4.76 (br dd, 1 H), 3.74-4.04 (m, 1 H), 3.08-3.24 (m, 5 H), 2.80-2.93 (m, 1 H), 2.64-2.70 (m, 4 H), 2.39 (s, 3 H) | 501.4 [M+H]⁺ | 98.5% |
| 365 | (CDCl₃) δ 8.31 (br s, 1 H), 8.09 (s, 1 H), 7.83 (br d, 1 H), 7.66 (br s, 1 H), 7.31-7.55 (m, 3 H), 7.16-7.29 (m, 1 H), 6.77 (br s, 1 H), 4.70-4.80 (m, 1 H), 3.85 (br d, 5 H), 3.13 (br d, 5 H), 2.84 (br d, 1 H) | 488.3 [M+H]⁺ | 100% |
| 366 | (CD₃OD) δ 8.34 (br s, 1 H), 8.12 (s, 1 H), 7.87 (d, 1 H), 7.69 (s, 1 H), 7.55 (d, 1 H), 7.35-7.49 (m, 2 H), 7.20-7.28 (m, 1 H), 6.79 (br s, 1 H), 4.76 (br dd, 1 H), 3.79-4.02 (m, 5 H), 3.13-3.22 (m, 5 H), 2.87 (br d, 1 H) | 488.3 [M+H]⁺ | 99.1% |
| 367 | (CD₃OD) δ 8.32 (br s, 1 H), 8.10 (s, 1 H), 7.84 (d, 1 H), 7.69 (s, 1 H), 7.55 (d, 1 H), 7.46 (dd, 1 H), 7.39 (td, 1 H), 7.20-7.31 (m, 1 H), 6.79 (br s, 1 H), 4.76 (br dd, 1 H), 3.94 (br s, 1 H), 3.74 (br d, 2 H), 3.13 (br d, 1 H), 2.87 (br d, 1 H), 2.66 (br t, 2 H), 1.94 (br d, 2 H), 1.39-1.62 (m, 3 H), 1.21 (s, 6 H) | 544.3 [M+H]⁺ | 100% |
| 368 | (CD₃OD) δ 8.32 (br s, 1 H), 8.10 (s, 1 H), 7.84 (d, 1 H), 7.69 (s, 1 H), 7.55 (d, 1 H), 7.46 (dd, 1 H), 7.39 (td, 1 H), 7.18-7.31 (m, 1 H), 6.80 (br s, 1 H), 4.76 (br dd, 1 H), 3.95 (br s, 1 H), 3.74 (br d, 2 H), 3.03-3.22 (m, 1 H), 2.87 (br d, 1 H), 2.65 (br t, 2 H), 1.94 (br d, 2 H), 1.41-1.64 (m, 3 H), 1.21 (s, 6 H) | 544.3 [M+H]⁺ | 99.4% |
| 369 | (CD₃OD) δ 8.33 (br s, 1 H), 8.14-8.16 (m, 1 H), 7.85 (d, 1 H), 7.69 (s, 1 H), 7.55 (d, 1 H), 7.36-7.49 (m, 2 H), 7.24 (t, 1 H), 6.79 (br s, 1 H), 4.68-4.82 (m, 1.5 H), 3.65-4.16 (m, 1 H), 3.34-3.38 (m, 1 H), 3.27-3.31 (m, 1 H), 3.06-3.23 (m, 3 H), 2.87 (br d, 1 H), 1.91-2.20 (m, 4.5 H) | 504.3 [M+H]⁺ | 98.1% |
| 370 | (CD₃OD) δ 8.33 (br s, 1 H), 8.15 (s, 1 H), 7.85 (d, 1 H), 7.69 (s, 1 H), 7.55 (d, 1 H), 7.36-7.49 (m, 2 H), 7.25 (t, 1 H), 6.78 (br s, 1 H), 4.76 (br d, 1 H), 3.80-4.08 (m, 1 H), 3.35-3.39 (m, 1 H), 3.27-3.31 (m, 1 H), 3.08-3.23 (m, 4 H), 2.87 (br d, 1 H), 1.87-2.21 (m, 5 H)) | 504.3 [M+H]⁺ | 92.7% |
| 371 | (CD₃OD) δ 8.32 (br s, 1 H), 8.12 (s, 1 H), 7.84 (d, 1 H), 7.69 (s, 1 H), 7.55 (d, 1 H), 7.46 (br d, 1 H), 7.39 (td, 1 H), 7.17-7.30 (m, 1 H), 6.78 (br s, 1 H), 4.76 (br dd, 1 H), 3.95 (br d, 1 H), 3.70-3.86 (m, 1 H), 3.45-3.58 (m, 2 H), 3.12 (br d, 1 H), 2.80-2.97 (m, 3 H), 2.03 (br dd, 2 H), 1.61-1.82 (m, 2 H) | 502.2 [M+H]⁺ | 98.6% |
| 372 | (CD₃OD) δ 8.32 (br s, 1 H), 8.12 (s, 1 H), 7.84 (d, 1 H), 7.69 (s, 1 H), 7.55 (d, 1 H), 7.46 (br d, 1 H), 7.39 (td, 1 H), 7.16-7.31 (m, 1 H), 6.78 (br s, 1 H), 4.76 (br dd, 1 H), 3.96 (br s, 1 H), 3.75-3.85 (m, 1 H), 3.46-3.62 (m, 2 H), 3.12 (br d, 1 H), 2.78-3.00 (m, 3 H), 2.03 (br d, 2 H), 1.62-1.82 (m, 2 H) | 502.2 [M+H]⁺ | 96.5% |
| 373 | (CD₃OD) δ 8.45 (br s, 1 H), 7.69 (s, 1 H), 7.23 (td, 1.3 H), 7.08-7.17 (m, 1.7 H), 6.72-7.00 (m, 1 H), 4.45-4.65 (m, 1 H), 3.60-4.08 (m, 1 H), 2.82-3.18 (m, 2 H) | 422.1 [M+H]⁺ | 99.4% |
| 374 | (CD₃OD) δ 8.45 (br s, 1 H), 7.69 (s, 1 H), 7.09-7.28 (m, 3 H), 6.74-7.01 (m, 1 H), 4.58 (br d, 1 H), 3.59-4.10 (m, 1 H), 2.81-3.18 (m, 2 H) | 422.2 [M+H]⁺ | 97.6% |
| 375 | (CD₃OD) δ 7.62-7.82 (m, 3 H), 7.45 (dd, 1 H), 6.93-7.33 (m, 1 H), 6.76 (br s, 1 H), 4.59 (br d, 1 H), 3.96 (br s, 0.7 H), 3.62 (br s, 0.3 H), 2.96-3.26 (m, 1 H), 2.88 (br d, 1 H), 1.50-1.75 (m, 6 H) | 478.3 [M+H]⁺ | 92.9% |
| 376 | (CD₃OD) δ 7.61-7.78 (m, 3 H), 7.45 (dd, 1 H), 6.95-7.35 (m, 1 H), 6.77 (br s, 1 H), 4.59 (br d, 1 H), 3.96 (br s, 0.7 H), 3.53-3.72 (m, 0.3 H), 2.94-3.24 (m, 1 H), 2.88 (br d, 1 H), 1.50-1.74 (m, 6 H) | 478.3 [M+H]⁺ | 85.5% |
| 377 | (CD₃OD) δ 8.47 (br s, 1 H), 7.69-7.79 (m, 2 H), 7.66 (d, 1 H), 7.44 (dd, 1 H), 6.96-7.35 (m, 1 H), 6.77 (br s, 1 H), 4.57 (br d, 1 H), 3.52-4.07 (m, 1 H), 3.12 (br s, 1 H), 2.86 (br d, 1 H) | 420.3 [M+H]⁺ | 97.8% |
| 378 | (CD₃OD) δ 8.47 (br s, 1 H), 7.71 (s, 2 H), 7.66 (d, 1 H), 7.39-7.49 (m, 1 H), 6.95-7.31 (m, 1 H), 6.78 (br s, 1 H), 4.57 (br d, 1 H), 3.52-4.10 (m, 1 H), 3.12 (br s, 1 H), 2.87 (br d, 1 H) | 420.3 [M+H]⁺ | 98.2% |
| 379 | (CD₃OD) δ 7.78-7.83 (m, 1 H), 7.68-7.73 (m, 1 H), 7.35-7.57 (m, 2.4 H), 7.10-7.19 (m, 1 H), 6.95-7.01 (m, 1 H), 6.88 (br s, 0.6 H), 5.30 (dd, 0.7 H), 5.12 (dd, 0.3 H), 4.00-4.04 (m, 3 H), 3.93-3.99 (m, 0.7 H), 3.68 (br s, 0.3 H), 2.83-3.27 (m, 2 H) | 435.30 [M+H]⁺ | 94.5% |
| 380 | (CD₃OD) δ 7.77-7.84 (m, 1 H), 7.71 (br s, 1 H), 7.37-7.56 (m, 2.4 H), 7.09-7.19 (m, 1 H), 6.94-7.02 (m, 1 H), 6.88 (br s, 0.6 H), 5.29 (dd, 0.7 H), 5.12 (dd, 0.3 H), 3.99-4.05 (m, 3 H), 3.90-3.98 (m, 0.7 H), 3.68 (br s, 0.3 H), 2.85-3.27 (m, 2 H) | 435.20 [M+H]⁺ | 97.5% |
| 381 | (CD₃OD) δ 8.47 (br s, 1 H), 7.70 (s, 1 H), 7.44 (br d, 1 H), 6.99-7.36 (m, 3 H), 6.80 (br s, 1 H), 4.56 (br d, 1 H), 3.53-4.16 (m, 1 H), 3.12 (br s, 1 H), 2.88 (br d, 1 H), 2.57 (s, 3 H) | 400.2 [M+H]⁺ | 100% |
| 382 | (CD₃OD) δ 8.47 (br s, 1 H), 7.70 (s, 1 H), 7.45 (br d, 1 H), 6.99-7.39 (m, 3 H), 6.81 (br s, 1 H), 4.55 (br s, 1 H), 3.53-4.18 (m, 1 H), 3.12 (br s, 1 H), 2.88 (br d, 1 H), 2.57 (s, 3 H) | 400.3 [M+H]⁺ | 99.8% |
| 383 | (CD₃OD) δ 8.91 (s, 1 H), 8.44 (br s, 1 H), 8.12 (s, 1 H), 7.94-8.02 (m, 2 H), 7.67-7.79 (m, 2 H), 7.56 (br d, 1 H), 7.35-7.45 (m, 1 H), 7.19-7.31 (m, 1 H), 6.83 (br s, 1 H), 4.77 (br d, 1 H), 3.97 (s, 4 H), 3.15 (br s, 1 H), 2.89 (br d, 1 H) | 483.4 [M+H]⁺ | 98.4% |
| 384 | (CD₃OD) δ 8.91 (s, 1 H), 8.45 (br s, 1 H), 8.18 (br s, 1 H), 8.12 (s, 1 H), 8.00 (d, 1 H), 7.95 (s, 1 H), 7.72 (d, 1 H), 7.56 (br d, 1 H), 7.36-7.46 (m, 1 H), 7.23-7.33 (m, 1 H), 6.97 (br s, 1 H), 4.72-4.83 (m, 1 H), 3.97 (s, 4 H), 3.17 (br d, 1 H), 2.92 (br d, 1 H) | 483.3 [M+H]⁺ | 85.1% |
| 385 | (CD₃OD) δ 7.48-7.68 (m, 3 H), 7.33-7.43 (m, 2 H), 7.19-7.27 (m, 1 H), 6.45-6.93 (m, 1 H), 4.33-4.64 (m, 1 H), 3.67-3.89 (m, 1 H), 3.39-3.44 (m, 2 H), 3.12-3.23 (m, 2 H), 2.91-3.05 (m, 1 H), 2.80 (br d, 1 H) | 398.20 [M+H]⁺ | 100% |
| 386 | (CD₃OD) δ 7.48-7.68 (m, 3 H), 7.34-7.42 (m, 2 H), 7.19-7.27 (m, 1 H), 6.45-6.90 (m, 1 H), 4.33-4.65 (m, 1 H), 3.80 (br s, 1 H), 3.39-3.44 (m, 2 H), 3.11-3.23 (m, 2 H), 2.76-3.01 (m, 2 H) | 398.20 [M+H]⁺ | 99.5% |
| 387 | (CD₃OD) δ 7.77 (s, 1 H), 7.64-7.71 (m, 1 H), 7.45-7.56 (m, 1 H), 7.29-7.40 (m, 1 H), 7.16-7.26 (m, 1 H), 7.03 (s, 1 H), 6.49-6.96 (m, 1 H), 4.94 (br dd, 0.6 H), 4.37 (dd, 0.7 H), 3.81 (br t, 0.7 H), 3.07-3.29 (m, 4 H), 2.70-3.06 (m, 2 H) | 382.3 [M+H]⁺ | 100% |
| 388 | (CD₃OD) δ 7.77 (s, 1 H), 7.63-7.72 (m, 1 H), 7.45-7.56 (m, 1 H), 7.31-7.40 (m, 1 H), 7.15-7.27 (m, 1 H), 6.94-7.07 (m, 1 H), 6.41-6.88 (m, 1 H), 4.90-4.99 (m, 0.6 H), 4.37 (dd, 0.7 H), 3.81 (br t, 0.7 H), 3.09-3.29 (m, 4 H), 2.70-3.06 (m, 2 H) | 382.2 [M+H]⁺ | 100% |
| 389 | (CD₃OD) δ 7.77-7.85 (m, 1 H), 7.70 (s, 1 H), 7.42-7.59 (m, 2 H), 7.15-7.29 (m, 1 H), 6.81-7.02 (m, 2 H), 5.27 (dd, 0.7 H), 5.09 (br dd, 0.3 H), 3.99-4.06 (m, 3 H), 3.94 (br t, 0.8 H), 3.59 (br t, 0.2 H), 3.11-3.26 (m, 0.8 H), 2.95-3.05 (m, 0.2 H), 2.79-2.93 (m, 1 H), 2.37-2.50 (m, 3 H) | 431.1 [M+H]⁺ | 99.7% |
| 390 | (CD₃OD) δ 7.77-7.84 (m, 1 H), 7.70 (s, 1 H), 7.41-7.61 (m, 2 H), 7.17-7.27 (m, 1 H), 6.83-7.01 (m, 2 H), 5.27 (dd, 0.7 H), 5.09 (dd, 0.3 H), 4.00-4.05 (m, 3 H), 3.84-3.98 (m, 0.7 H), 3.60 (br s, 0.3 H), 3.11-3.26 (m, 0.7 H), 2.94-3.06 (m, 0.3 H), 2.88 (br d, 1 H), 2.35-2.52 (m, 3 H) | 431.1 [M+H]⁺ | 92.6% |
| 391 | (CD₃OD) δ 7.69 (s, 1 H), 7.48 (d, 1 H), 7.18-7.32 (m, 2 H), 6.92-7.16 (m, 1 H), 6.76 (br s, 1 H), 4.92-5.01 (m, 0.2 H), 4.42-4.66 (m, 0.8 H), 3.94 (br d, 0.7 H), 3.62 (br s, 0.3 H), 2.94-3.23 (m, 1 H), 2.87 (br d, 1 H), 2.55 (s, 3 H), 1.41-1.77 (m, 6 H) | 458.1 [M+H]⁺ | 99.1% |
| 392 | (CD₃OD) δ 7.69 (s, 1 H), 7.48 (d, 1 H), 7.19-7.32 (m, 2 H), 6.91-7.16 (m, 1 H), 6.74 (br s, 1 H), 4.92-5.01 (m, 0.3 H), 4.49-4.62 (m, 0.7 H), 3.97 (br s, 0.6 H), 3.53-3.72 (m, 0.3 H), 2.94-3.23 (m, 1 H), 2.88 (br s, 1 H), 2.55 (s, 3 H), 1.40-1.79 (m, 6 H) | 458.1 [M+H]⁺ | 99.5% |
| 393 | (CD₃OD) δ 8.45 (s, 1 H), 7.69 (s, 1 H), 7.48 (d, 1 H), 7.16-7.33 (m, 2 H), 6.94-7.14 (m, 1 H), 6.78 (br s, 1 H), 4.54 (br d, 1 H), 3.95 (br s, 0.8 H), 3.45-3.69 (m, 0.2 H), 3.10 (br s, 1 H), 2.76-2.92 (m, 1 H), 2.54 (s, 3 H) | 400.2 [M+H]⁺ | 99.2% |
| 394 | (CD₃OD) δ 8.45 (s, 1 H), 7.69 (s, 1 H), 7.48 (d, 1 H), 7.16-7.33 (m, 2 H), 6.93-7.15 (m, 1 H), 6.78 (br s, 1 H), 4.52 (br s, 1 H), 3.95 (br s, 0.7 H), 3.48-3.67 (m, 0.3 H), 3.10 (br s, 1 H), 2.86 (br d, 1 H), 2.55 (s, 3 H) | 400.2 [M+H]⁺ | 99.3% |
| 395 | (CD₃OD) δ 8.33 (br s, 1 H), 8.12 (s, 1 H), 7.84 (d, 1 H), 7.69 (s, 1 H), 7.35-7.60 (m, 3 H), 7.20-7.31 (m, 1 H), 6.81 (br s, 1 H), 4.76 (br dd, 1 H), 3.95 (br s, 1 H), 3.20-3.27 (m, 4 H), 3.07-3.19 (m, 1 H), 2.80-2.95 (m, 1 H), 1.57-1.63 (m, 4 H), 0.41 (s, 4 H) | 512.3 [M+H]⁺ | 100% |
| 396 | (CD₃OD) δ 8.33 (br s, 1 H), 8.12 (d, 1 H), 7.84 (d, 1 H), 7.70 (d, 1 H), 7.35-7.58 (m, 3 H), 7.20-7.29 (m, 1 H), 6.81 (s, 1 H), 4.77 (br dd, 1 H), 3.94 (br s, 1 H), 3.20-3.25 (m, 4 H), 3.09-3.19 (m, 1 H), 2.87 (dd, 1 H), 1.56-1.65 (m, 4 H), 0.41 (s, 4 H) | 512.2 [M+H]⁺ | 97.4% |
| 397 | (CD₃OD) δ 7.64-7.70 (m, 2 H), 7.47-7.50 (m, 1 H), 7.37-7.44 (m, 2 H), 7.11-7.19 (m, 1 H), 6.83 (s, 0.8 H), 6.48 (br s, 0.2 H), 4.37 (br dd, 1 H), 3.81 (br t, 1 H), 3.39-3.45 (m, 2 H), 3.13-3.20 (m, 2 H), 2.91-3.01 (m, 1 H), 2.80 (br dd, 1 H) | 398.20 [M+H]⁺ | 100% |
| 398 | (CD₃OD) δ 7.46-7.70 (m, 1 H), 7.46-7.70 (m, 2 H), 7.35-7.45 (m, 2 H), 7.11-7.19 (m, 1 H), 6.42-6.88 (m, 1 H), 4.34-4.64 (m, 1 H), 3.82 (br s, 1 H), 3.40-3.45 (m, 2 H), 3.12-3.24 (m, 2 H), 2.89-3.04 (m, 1 H), 2.80 (br d, 1 H) | 398.20 [M+H] | 99.8% |
| 399 | (CD₃OD) δ 9.29-9.38 (m, 1 H), 8.80-8.89 (m, 1 H), 8.52-8.63 (m, 1 H), 7.53-7.81 (m, 3.5 H), 7.44 (br d, 1 H), 7.13-7.30 (m, 1 H), 6.90 (br s, 0.5 H), 5.11-5.44 (m, 1 H), 3.59-4.14 (m, 1 H), 3.00-3.29 (m, 1 H), 2.93 (br d, 1 H) | 432.2 [M+H]⁺ | 100% |
| 400 | (CD₃OD) δ 9.27-9.37 (m, 1 H), 8.79-8.84 (m, 1 H), 8.41-8.65 (m, 1 H), 7.50-7.77 (m, 3.5 H), 7.40-7.47 (m, 1 H), 7.17-7.26 (m, 1 H), 6.90 (br s, 0.5 H), 5.10-5.41 (m, 1 H), 3.62-4.10 (m, 1 H), 2.99-3.29 (m, 1 H), 2.93 (br d, 1 H) | 432.3 [M+H]⁺ | 99.3% |
| 401 | (CD₃OD) δ 7.61-7.74 (m, 2 H), 7.44 (br d, 1 H), 6.97-7.30 (m, 2 H), 6.67 (br s, 1 H), 4.81 (br s, 1.3 H), 3.65 (br s, 5.7 H), 3.04 (br s, 1 H), 2.85 (br d, 1 H), 1.89 (br s, 4 H) | 505.3 [M+H]⁺ | 96.8% |
| 402 | (CD₃OD) δ 7.65-7.72 (m, 2 H), 7.44 (br d, 1 H), 6.96-7.28 (m, 2 H), 6.67 (br s, 1 H), 4.82 (br s, 1.3 H), 3.66 (br s, 5.7 H), 3.05 (br s, 1 H), 2.85 (br d, 1 H), 1.89 (br s, 4 H) | 505.3 [M+H]⁺ | 95.5% |
| 403 | (CD₃OD) δ 7.64-7.71 (m, 2 H), 7.45 (br d, 1 H), 6.94-7.28 (m, 2 H), 6.69 (br s, 1 H), 4.77 (br s, 1 H), 3.53-3.88 (m, 5 H), 3.05 (br s, 1 H), 2.85 (br d, 1 H), 2.53 (br s, 4 H), 2.36 (s, 3 H) | 502.3 [M+H]⁺ | 97.4% |
| 404 | (CD₃OD) δ 7.65-7.74 (m, 2 H), 7.45 (br d, 1 H), 6.96-7.29 (m, 2 H), 6.68 (br s, 1 H), 4.78 (br s, 1 H), 3.51-3.90 (m, 5 H), 3.04 (br s, 1 H), 2.85 (br d, 1 H), 2.53 (br s, 4 H), 2.35 (s, 3 H) | 502.3 [M+H]⁺ | 96.1% |
| 405 | (CD₃OD) δ 9.31-9.54 (m, 3 H), 7.70 (br d, 2 H), 7.32-7.60 (m, 1 H), 7.01-7.30 (m, 2 H), 6.81 (br s, 1 H), 4.96-5.03 (m, 0.4 H), 4.73 (br d, 0.6 H), 3.72-4.07 (m, 1 H), 3.01-3.30 (m, 1 H), 2.95 (br d, 1 H) | 482.2 [M+H]⁺ | 88.0% |
| 406 | (CD₃OD) δ 9.30-9.56 (m, 3 H), 7.70 (br d, 2 H), 7.33-7.60 (m, 1 H), 6.99-7.29 (m, 2 H), 6.81 (br s, 1 H), 4.95-5.06 (m, 0.4 H), 4.74 (br d, 0.6 H), 3.73-4.10 (m, 1 H), 3.01-3.29 (m, 1 H), 2.95 (br d, 1 H) | 482.3 [M+H]⁺ | 98.0% |
| 407 | (CD₃OD) δ 7.69 (s, 1 H), 7.42-7.56 (m, 2 H), 7.24 (d, 1 H), 6.34-6.80 (m, 1 H), 4.36 (br s, 1 H), 3.51-4.03 (m, 1 H), 2.92-3.22 (m, 1 H), 2.85 (br d, 1 H), 2.45 (s, 3 H), 1.63 (br s, 6 H) | 486.2 [M+H]⁺ | 99.3% |
| 408 | (CD₃OD) δ 7.68 (s, 1 H), 7.40-7.56 (m, 2 H), 7.24 (br d, 1 H), 6.29-6.80 (m, 1 H), 4.36 (br s, 1 H), 3.52-4.06 (m, 1 H), 3.13 (br s, 1 H), 2.85 (br d, 1 H), 2.45 (s, 3 H), 1.63 (br s, 6 H) | 486.1 [M+H]⁺ | 97.8% |
| 409 | (CD₃OD) δ 8.59-8.71 (m, 1 H), 7.50-7.62 (m, 2 H), 7.35-7.47 (m, 1 H), 7.20-7.29 (m, 1 H), 7.05-7.16 (m, 1 H), 6.23-6.82 (m, 1 H), 4.87 (br dd, 0.2 H), 4.22 (dd, 0.8 H), 3.66 (br t, 0.8 H), 3.17 (br t, 2.2 H), 2.62-3.04 (m, 4 H) | 398.1 [M+H]⁺ | 100% |
| 410 | (CD₃OD) δ 8.60-8.72 (m, 1 H), 7.51-7.61 (m, 2 H), 7.34-7.46 (m, 1 H), 7.20-7.29 (m, 1 H), 7.04-7.19 (m, 1 H), 6.21-6.93 (m, 1 H), 4.87 (br dd, 0.2 H), 4.22 (br dd, 0.8 H), 3.66 (br t, 0.8 H), 3.17 (br t, 2.2 H), 2.61-3.09 (m, 4 H) | 398.1 [M+H]⁺ | 92.3% |
| 411 | (CD₃OD) δ 7.64-7.88 (m, 3 H), 7.44 (br d, 1 H), 6.72-7.37 (m, 4 H), 4.93-5.07 (m, 0.4 H), 4.74 (br s, 0.6 H), 3.75-4.13 (m, 4 H), 3.10-3.28 (m, 1 H), 2.90 (br d, 1 H) | 484.2 [M+H]⁺ | 97.5% |
| 412 | (CD₃OD) δ 7.63-7.88 (m, 3 H), 7.44 (br d, 1 H), 6.74-7.35 (m, 4 H), 4.91-5.09 (m, 0.4 H), 4.75 (br s, 0.6 H), 3.57-4.11 (m, 4 H), 3.08-3.30 (m, 1 H), 2.90 (br d, 1 H) | 484.2 [M+H]⁺ | 97.2% |
| 413 | (CD₃OD) δ 7.64-7.70 (m, 2 H), 7.44 (dd, 1 H), 6.97-7.29 (m, 2 H), 6.68 (br s, 1 H), 4.77 (br s, 1 H), 3.46-3.87 (m, 9 H), 3.05 (br s, 1 H), 2.85 (br d, 1 H) | 489.1 [M+H]⁺ | 95.9% |
| 414 | (CD₃OD) δ 7.63-7.72 (m, 2 H), 7.44 (dd, 1 H), 6.97-7.29 (m, 2 H), 6.70 (br s, 1 H), 4.78 (br s, 1 H), 3.48-3.88 (m, 9 H), 3.05 (br s, 1 H), 2.84 (br d, 1 H) | 489.1 [M+H]⁺ | 98.3% |
| 415 | (CD₃OD) δ 7.88 (s, 1 H), 7.70 (br s, 1 H), 7.56-7.64 (m, 2 H), 6.97-7.28 (m, 1 H), 6.74 (br s, 1 H), 4.59 (br d, 1 H), 3.58-4.05 (m, 1 H), 2.81-3.22 (m, 2 H), 1.52-1.73 (m, 6 H) | 522.10 [M+H]⁺ | 94.5% |
| 416 | (CD₃OD) δ 7.87 (s, 1 H), 7.70 (br s, 1 H), 7.56-7.64 (m, 2 H), 6.95-7.30 (m, 1 H), 6.74 (br s, 1 H), 4.59 (br d, 1 H), 3.57-4.05 (m, 1 H), 2.78-3.25 (m, 2 H), 1.51-1.74 (m, 6 H) | 524.20 [M+H] | 79.2% |
| 417 | (CD₃OD) δ 8.18-8.74 (m, 2 H), 7.64-7.96 (m, 3 H), 7.05-7.52 (m, 3 H), 6.81 (br s, 1 H), 5.02 (br s, 0.3 H), 4.75 (br d, 0.7 H), 3.62-4.10 (m, 1 H), 3.15 (br s, 1 H), 2.91 (br d, 1 H) | 499.2 [M+H]⁺ | 98.7% |
| 418 | (CD₃OD) δ 8.05-8.64 (m, 2 H), 7.50-7.82 (m, 3 H), 6.93-7.39 (m, 3 H), 6.69 (s, 1 H), 4.86-4.95 (m, 0.3 H), 4.64 (br d, 0.7 H), 3.51-4.04 (m, 1 H), 2.89-3.16 (m, 1 H), 2.79 (br d, 1 H) | 499.2 [M+H]⁺ | 96.8% |
| 419 | (CD₃OD) δ 9.30 (br s, 1 H), 8.76 (br s, 1 H), 8.38-8.58 (m, 1 H), 7.40-7.78 (m, 4 H), 7.04-7.38 (m, 2 H), 6.79 (br s, 1 H), 4.74 (br s, 1 H), 3.63-4.16 (m, 1 H), 3.14 (br s, 1 H), 2.92 (br s, 1 H) | 481.3 [M+H]⁺ | 96.7% |
| 420 | (CD₃OD) δ 9.18 (s, 1 H), 8.65 (br s, 1 H), 8.15-8.49 (m, 1 H), 7.27-7.69 (m, 4 H), 6.91-7.26 (m, 2 H), 6.70 (br s, 1 H), 4.64 (br s, 1 H), 3.57-4.00 (m, 1 H), 2.89-3.17 (m, 1 H), 2.83 (br d, 1 H) | 481.3 [M+H]⁺ | 96.9% |
| 421 | (CD₃OD) δ 8.61-8.90 (m, 2 H), 7.88-8.22 (m, 2 H), 7.70 (br s, 2 H), 7.32-7.58 (m, 1 H), 7.01-7.30 (m, 2 H), 6.80 (br s, 1 H), 4.96-5.08 (m, 0.5 H), 4.72 (br d, 0.5 H), 3.56-4.20 (m, 1 H), 3.25 (br s, 1 H), 2.94 (br s, 1 H) | 481.3 [M+H]⁺ | 100% |
| 422 | (CD₃OD) δ 8.59-8.89 (m, 2 H), 7.86-8.20 (m, 2 H), 7.71 (br d, 2 H), 7.31-7.58 (m, 1 H), 6.98-7.30 (m, 2 H), 6.81 (br s, 1 H), 4.99 (br s, 0.5 H), 4.72 (br d, 0.5 H), 3.67-4.15 (m, 1 H), 3.24 (br s, 1 H), 2.95 (br d, 1 H) | 481.3 [M+H]⁺ | 99.0% |
| 423 | (CD₃OD) δ 7.59-7.73 (m, 2 H), 7.42-7.50 (m, 1 H), 7.32-7.41 (m, 1 H), 6.93-7.28 (m, 1 H), 6.76 (br s, 1 H), 4.47-4.61 (m, 1 H), 3.56-4.12 (m, 1 H), 3.13 (br s, 1 H), 2.87 (br s, 1 H), 1.46-1.78 (m, 6 H) | 478.2 [M+H]⁺ | 96.9% |
| 424 | (CD₃OD) δ 7.57-7.75 (m, 2 H), 7.42-7.49 (m, 1 H), 7.33-7.41 (m, 1 H), 6.92-7.28 (m, 1 H), 6.76 (br s, 1 H), 4.56 (br s, 1 H), 3.54-4.12 (m, 1 H), 2.95-3.24 (m, 1 H), 2.87 (br s, 1 H), 1.50-1.74 (m, 6 H) | 478.2 [M+H]⁺ | 100% |
| 425 | (CD₃OD) δ 8.45 (s, 1 H), 7.58-7.74 (m, 2 H), 7.31-7.49 (m, 2 H), 6.94-7.29 (m, 1 H), 6.80 (br s, 1 H), 4.54 (br s, 1 H), 3.52-4.10 (m, 1 H), 3.10 (br s, 1 H), 2.87 (br d, 1 H) | 420.2 [M+H]⁺ | 98.8% |
| 426 | (CD₃OD) δ 8.45 (s, 1 H), 7.58-7.76 (m, 2 H), 7.43-7.50 (m, 1 H), 7.33-7.42 (m, 1 H), 6.95-7.29 (m, 1 H), 6.79 (br s, 1 H), 4.54 (br s, 1 H), 3.51-4.09 (m, 1 H), 3.12 (br d, 1 H), 2.87 (br d, 1 H) | 420.2 [M+H]⁺ | 98.5% |
| 427 | (CD₃OD) δ 7.70 (s, 1 H), 7.32-7.44 (m, 1 H), 6.98-7.31 (m, 2 H), 6.94 (d, 1 H), 6.55-6.85 (m, 1 H), 4.56 (br d, 1 H), 4.00 (s, 3.8 H), 3.65 (br s, 0.2 H), 2.95-3.24 (m, 1 H), 2.87 (br d, 1 H), 1.51-1.76 (m, 6 H) | 474.3 [M+H]⁺ | 99.2% |
| 428 | (CD₃OD) δ 7.70 (br s, 1 H), 7.33-7.43 (m, 1 H), 6.99-7.31 (m, 2 H), 6.94 (d, 1 H), 6.75 (br s, 1 H), 4.57 (br d, 1 H), 4.01 (s, 3.8 H), 3.65 (br s, 0.2 H), 2.94-3.25 (m, 1 H), 2.88 (br d, 1 H), 1.49-1.73 (m, 6 H) | 474.3 [M+H]⁺ | 98.9% |
| 429 | (CD₃OD) δ 8.46 (br s, 1 H), 7.70 (s, 1 H), 7.33-7.42 (m, 1 H), 7.00-7.32 (m, 2 H), 6.94 (d, 1 H), 6.76 (br s, 1 H), 4.55 (br d, 1 H), 4.00 (s, 3.9 H), 3.61 (br s, 0.1 H), 3.12 (br s, 1 H), 2.86 (br d, 1 H) | 416.3 [M+H]⁺ | 99.7% |
| 430 | (CD₃OD) δ 8.46 (br s, 1 H), 7.70 (s, 1 H), 7.34-7.45 (m, 1 H), 7.00-7.32 (m, 2 H), 6.94 (d, 1 H), 6.76 (br s, 1 H), 4.55 (br d, 1 H), 4.00 (s, 3.9 H), 3.62 (br s, 0.1 H), 3.12 (br s, 1 H), 2.86 (br d, 1 H) | 416.3 [M+H]⁺ | 99.3% |
| 431 | (CD₃OD) δ 7.62-7.72 (m, 2 H), 7.57 (d, 1 H), 7.30-7.39 (m, 1 H), 6.95-7.29 (m, 1 H), 6.72 (br s, 1 H), 4.52-4.60 (m, 1 H), 3.63-4.13 (m, 1 H), 2.81-3.24 (m, 2 H), 1.52-1.71 (m, 6 H) | 524.2 [M+H]⁺ | 98.7% |
| 432 | (CD₃OD) δ 7.61-7.76 (m, 2 H), 7.57 (d, 1 H), 7.30-7.39 (m, 1 H), 6.94-7.29 (m, 1 H), 6.74 (br s, 1 H), 4.60 (s, 1 H), 3.62-4.12 (m, 1 H), 2.82-3.21 (m, 2 H), 1.51-1.72 (m, 6 H) | 522.2 [M+H]⁺ | 98.8% |
| 433 | (CD₃OD) δ 8.45 (br s, 1 H), 7.61-7.72 (m, 2 H), 7.57 (d, 1 H), 7.30-7.38 (m, 1 H), 6.97-7.29 (m, 1 H), 6.76 (br s, 1 H), 4.51-4.60 (m, 1 H), 3.54-4.15 (m, 1 H), 2.82-3.18 (m, 2 H) | 466.1 [M+H]⁺ | 99.3% |
| 434 | (CD₃OD) δ 8.45 (br s, 1 H), 7.62-7.72 (m, 2 H), 7.56 (d, 1 H), 7.29-7.38 (m, 1 H), 6.94-7.28 (m, 1 H), 6.76 (br s, 1 H), 4.60 (s, 1 H), 3.57-4.10 (m, 1 H), 2.81-3.19 (m, 2 H) | 466.1 [M+H]⁺ | 99.1% |
| 435 | (CD₃OD) δ 7.71 (s, 1 H), 7.63 (br d, 1 H), 7.39-7.45 (m, 2 H), 7.00-7.29 (m, 1 H), 6.60-6.83 (m, 1 H), 4.53-4.68 (m, 1 H), 3.65-4.08 (m, 1 H), 3.17 (br d, 1 H), 2.90 (br d, 1 H), 1.54-1.73 (m, 6 H) | 478.30 [M+H] | 98.9% |
| 436 | (CD₃OD) δ 7.71 (s, 1 H), 7.63 (br d, 1 H), 7.40-7.44 (m, 2 H), 7.01-7.28 (m, 1 H), 6.65-6.80 (m, 1 H), 4.57-4.64 (m, 1 H), 3.68-4.06 (m, 1 H), 3.17 (br d, 1 H), 2.90 (br d, 1 H), 1.55-1.72 (m, 6 H) | 478.30 [M+H] | 98.5% |
| 437 | (CD₃OD) δ 8.47 (br s, 1 H), 7.71 (s, 1 H), 7.63 (dd, 1 H), 7.39-7.45 (m, 2 H), 7.02-7.30 (m, 1 H), 6.79 (br s, 1 H), 4.51-4.66 (m, 1 H), 4.02 (br s, 1 H), 3.13 (br s, 1 H), 2.88 (br d, 1 H) | 420.20 [M+H]⁺ | 99.6% |
| 438 | (CD₃OD) δ 8.47 (br s, 1 H), 7.71 (s, 1 H), 7.63 (dd, 1 H), 7.40-7.44 (m, 2 H), 7.02-7.30 (m, 1 H), 6.79 (br s, 1 H), 4.54-4.67 (m, 1 H), 4.01 (br s, 1 H), 3.13 (br s, 1 H), 2.89 (br d, 1 H) | 420.20 [M+H] | 99.6% |
| 439 | (CD₃OD) δ 7.68 (s, 1 H), 7.54 (d, 1 H), 7.45 (s, 1 H), 7.21 (d, 1 H), 6.34-6.84 (m, 1 H), 4.35 (br s, 1 H), 3.50-4.05 (m, 1 H), 2.79-3.25 (m, 2 H), 2.48 (s, 3 H), 1.48-1.71 (m, 6 H) | 486.2 [M+H]⁺ | 99.5% |
| 440 | (CD₃OD) δ 7.68 (s, 1 H), 7.54 (d, 1 H), 7.46 (s, 1 H), 7.22 (d, 1 H), 6.34-6.83 (m, 1 H), 4.35 (br s, 1 H), 3.52-4.03 (m, 1 H), 2.78-3.25 (m, 2 H), 2.48 (s, 3 H), 1.64 (br s, 6 H) | 486.2 [M+H]⁺ | 99.2% |
| 441 | (CD₃OD) δ 8.35-8.54 (m, 1 H), 8.15-8.33 (m, 1 H), 7.51-7.82 (m, 3.4 H), 7.34-7.48 (m, 1 H), 7.14-7.28 (m, 1 H), 6.76-7.04 (m, 0.6 H), 5.30 (br dd, 0.8 H), 5.14 (br dd, 0.2 H), 3.90-4.11 (m, 3.8 H), 3.66 (br s, 0.2 H), 2.77-3.27 (m, 2 H) | 462.3 [M+H]⁺ | 100% |
| 442 | (CD₃OD) δ 8.37-8.54 (m, 1 H), 8.15-8.33 (m, 1 H), 7.51-7.80 (m, 3.3 H), 7.33-7.48 (m, 1 H), 7.12-7.33 (m, 1 H), 6.89 (br s, 0.7 H), 5.10-5.36 (m, 1 H), 3.87-4.14 (m, 3.7 H), 3.66 (br s, 0.3 H), 2.74-3.27 (m, 2 H) | 462.3 [M+H]⁺ | 98.4% |
| 443 | (CD₃OD) δ 8.65-9.19 (m, 1 H), 8.25-8.58 (m, 2 H), 7.65-7.78 (m, 2 H), 6.68-7.56 (m, 4 H), 5.02 (br s, 0.2 H), 4.76 (br d, 0.8 H), 3.68-4.13 (m, 1 H), 3.01-3.30 (m, 1 H), 2.92 (br d, 1 H) | 549.3 [M+H]⁺ | 100% |
| 444 | (CD₃OD) δ 8.52-9.02 (m, 1 H), 8.14-8.42 (m, 2 H), 7.54-7.63 (m, 2 H), 6.66-7.40 (m, 4 H), 4.89 (br s, 0.2 H), 4.64 (br d, 0.8 H), 3.51-3.97 (m, 1 H), 2.89-3.18 (m, 1 H), 2.80 (br d, 1 H) | 549.3 [M+H]⁺ | 99.3% |
| 445 | (CD₃OD) δ 8.01-8.55 (m, 2 H), 7.00-7.96 (m, 6 H), 6.79 (br s, 1 H), 4.78 (br s, 1 H), 3.99 (br s, 4 H), 2.64-3.27 (m, 2 H) | 511.1 [M+H]⁺ | 97.4% |
| 446 | (CD₃OD) δ 7.95-8.59 (m, 2 H), 7.15-7.91 (m, 5.6 H), 6.40-7.11 (m, 1.4 H), 4.78 (br s, 1 H), 3.99 (br s, 4 H), 2.69-3.27 (m, 2 H) | 511.3 [M+H]⁺ | 96.6% |
| 447 | (CD₃OD) δ 7.67-7.75 (m, 1 H), 7.36-7.48 (m, 1 H), 7.24-7.33 (m, 1 H), 7.12-7.22 (m, 1 H), 6.88-7.03 (m, 1 H), 6.64-6.88 (m, 1 H), 5.09 (dd, 0.3 H), 4.74 (dd, 0.7 H), 4.12 (s, 3 H), 3.87-4.00 (m, 0.7 H), 3.46-3.58 (m, 0.3 H), 2.95-3.29 (m, 1 H), 2.77-2.92 (m, 1 H), 2.49-2.60 (m, 3 H) | 414.1 [M+H]⁺ | 100% |
| 448 | (CD₃OD) δ 7.67-7.73 (m, 1 H), 7.37-7.48 (m, 1 H), 7.24-7.33 (m, 1 H), 7.19 (d, 1 H), 6.89-7.16 (m, 1 H), 6.65-6.88 (m, 1 H), 5.09 (dd, 0.3 H), 4.74 (dd, 0.7 H), 4.12 (s, 3 H), 3.94 (br t, 0.7 H), 3.46-3.58 (m, 0.3 H), 2.95-3.28 (m, 1 H), 2.78-2.92 (m, 1 H), 2.52-2.59 (m, 3 H) | 414.1 [M+H]⁺ | 99.2% |
| 449 | (CD₃OD) δ 7.85 (br s, 1 H), 7.50-7.76 (m, 2 H), 7.40-7.50 (m, 1 H), 7.31 (t, 1 H), 7.20 (d, 1 H), 6.72-7.06 (m, 1.8 H), 6.33 (br s, 0.2 H), 4.94-5.04 (m, 0.3 H), 4.27 (br d, 0.7 H), 3.43-4.10 (m, 1 H), 2.94-3.12 (m, 1 H), 2.73-2.88 (m, 1 H), 2.58 (s, 3 H) | 399.1 [M+H]⁺ | 97.9% |
| 450 | (CD₃OD) δ 7.85 (br s, 1 H), 7.50-7.75 (m, 2 H), 7.45 (br d, 1 H), 7.30 (t, 1 H), 7.20 (d, 1 H), 6.75-7.04 (m, 1.8 H), 6.21-6.47 (m, 0.2 H), 4.93 (br s, 0.3 H), 4.27 (br d, 0.7 H), 3.45-4.07 (m, 1 H), 2.93-3.10 (m, 1 H), 2.82 (br d, 1 H), 2.58 (s, 3 H) | 399.1 [M+H]⁺ | 98.9% |
| 451 | (CD₃OD) δ 9.35 (s, 1 H), 8.70 (d, 1 H), 8.53 (br s, 1 H), 8.05-8.21 (m, 2 H), 7.89-8.04 (m, 2 H), 7.71 (s, 1 H), 7.57 (d, 1 H), 7.36-7.51 (m, 2 H), 7.19-7.33 (m, 1 H), 6.84 (br s, 1 H), 4.77 (br d, 1 H), 3.80-4.19 (m, 1 H), 3.16 (br d, 1 H), 2.90 (br d, 1 H) | 480.1 [M+H]⁺ | 95.5% |
| 452 | (CD₃OD) δ 9.35 (s, 1 H), 8.70 (d, 1 H), 8.53 (br s, 1 H), 8.06-8.20 (m, 2 H), 7.91-8.05 (m, 2 H), 7.71 (s, 1 H), 7.57 (d, 1 H), 7.33-7.50 (m, 2 H), 7.19-7.31 (m, 1 H), 6.84 (br s, 1 H), 4.78 (br d, 1 H), 3.98 (br s, 1 H), 3.11-3.25 (m, 1 H), 2.90 (br d, 1 H) | 480.2 [M+H]⁺ | 95.3% |
| 453 | (CD₃OD) δ 7.97 (d, 1 H), 7.66-7.77 (m, 2 H), 7.52-7.60 (m, 1 H), 6.91-7.28 (m, 1 H), 6.81 (br s, 1 H), 4.55 (br s, 1 H), 3.54-4.07 (m, 1 H), 2.85-3.25 (m, 2 H), 1.48-1.71 (m, 6 H) | 512.3 [M+H]⁺ | 96.0% |
| 454 | (CD₃OD) δ 7.97 (d, 1 H), 7.65-7.75 (m, 2 H), 7.52-7.60 (m, 1 H), 6.91-7.26 (m, 1 H), 6.79 (br s, 1 H), 4.47-4.67 (m, 1 H), 3.55-4.11 (m, 1 H), 2.83-3.25 (m, 2 H), 1.49-1.72 (m, 6 H) | 512.3 [M+H]⁺ | 95.6% |
| 455 | (CD₃OD) δ 7.70 (s, 1 H), 6.96-7.39 (m, 4 H), 6.62-6.84 (m, 1 H), 4.77-4.86 (m, 0.3 H), 4.57 (br d, 0.7 H), 4.02 (s, 3.7 H), 3.62 (br s, 0.3 H), 2.99-3.23 (m, 1 H), 2.89 (br d, 1 H), 1.53-1.78 (m, 6 H) | 474.1 [M+H]⁺ | 97.4% |
| 456 | (CD₃OD) δ 7.70 (s, 1 H), 6.98-7.37 (m, 4 H), 6.62-6.84 (m, 1 H), 4.81-4.86 (m, 0.3 H), 4.57 (br d, 0.7 H), 4.02 (s, 3.7 H), 3.62 (br s, 0.3 H), 3.00-3.22 (m, 1 H), 2.89 (br d, 1 H), 1.53-1.76 (m, 6 H) | 474.1 [M+H]⁺ | 95.4% |
| 457 | (CD₃OD) δ 8.46 (s, 1 H), 7.70 (s, 1 H), 6.97-7.36 (m, 4 H), 6.81 (br s, 1 H), 4.55 (br d, 1 H), 4.02 (s, 3.8 H), 3.59 (br s, 0.2 H), 3.12 (br s, 1 H), 2.87 (br d, 1 H) | 416.1 [M+H]⁺ | 99.6% |
| 458 | (CD₃OD) δ 8.46 (s, 1 H), 7.70 (s, 1 H), 6.98-7.35 (m, 4 H), 6.81 (br s, 1 H), 4.55 (br d, 1 H), 4.02 (s, 3.8 H), 3.61 (br s, 0.2 H), 3.11 (br s, 1 H), 2.87 (br d, 1 H) | 416.1 [M+H]⁺ | 99.4% |
| 459 | (CD₃OD) δ 7.66-7.76 (m, 2 H), 7.61 (d, 1 H), 7.23-7.40 (m, 1 H), 6.92-7.21 (m, 1 H), 6.78 (br s, 1 H), 4.63 (s, 1 H), 3.60-4.12 (m, 1 H), 2.81-3.27 (m, 2 H), 1.50-1.79 (m, 6 H) | 522.0 [M+H]⁺ | 98.2% |
| 460 | (CD₃OD) δ 7.63-7.79 (m, 2 H), 7.59 (d, 1 H), 7.32 (t, 1 H), 6.93-7.26 (m, 1 H), 6.76 (br s, 1 H), 4.58 (br d, 1 H), 3.54-4.13 (m, 1 H), 2.90 (br s, 2 H), 1.68 (br s, 6 H) | 522.0 [M+H]⁺ | 97.1% |
| 461 | (CD₃OD) δ 8.45 (s, 1 H), 7.49-7.72 (m, 3 H), 7.31 (t, 1 H), 6.95-7.27 (m, 1 H), 6.80 (br s, 1 H), 4.39-4.73 (m, 1 H), 3.48-4.10 (m, 1 H), 2.67-3.20 (m, 2 H) | 466.2 [M+H]⁺ | 96.3% |
| 462 | (CD₃OD) δ 8.45 (s, 1 H), 7.52-7.74 (m, 3 H), 7.31 (t, 1 H), 6.92-7.26 (m, 1 H), 6.80 (br s, 1 H), 3.84-4.66 (m, 1 H), 3.43-4.69 (m, 1 H), 2.80-3.17 (m, 2 H) | 464.1 [M+H]⁺ | 93.5% |
| 463 | (CD₃OD) δ 7.93 (d, 1 H), 7.63-7.74 (m, 2 H), 7.52-7.62 (m, 1 H), 6.93-7.28 (m, 1 H), 6.75 (br s, 1 H), 4.91-5.04 (m, 1 H), 3.58-4.23 (m, 1 H), 3.14 (br s, 1 H), 2.90 (br s, 1 H), 1.43-1.78 (m, 6 H) | 512.1 [M+H]⁺ | 98.0% |
| 464 | (CD₃OD) δ 7.94 (d, 1 H), 7.64-7.74 (m, 2 H), 7.52-7.62 (m, 1 H), 6.92-7.29 (m, 1 H), 6.78 (br s, 1 H), 4.58 (br s, 1 H), 3.60-4.20 (m, 1 H), 2.97-3.23 (m, 1 H), 2.89 (br d, 1 H), 1.50-1.78 (m, 6 H) | 512.4 [M+H]⁺ | 100% |
| 465 | (CD₃OD) δ 8.45 (br s, 1 H), 7.93 (br d, 1 H), 7.64-7.75 (m, 2 H), 7.50-7.63 (m, 1 H), 6.93-7.28 (m, 1 H), 6.80 (br s, 1 H), 4.61 (br s, 1 H), 4.05 (br s, 1 H), 3.10 (br s, 1 H), 2.87 (br d, 1 H) | 454.2 [M+H]⁺ | 99.9% |
| 466 | (CD₃OD) δ 8.45 (br s, 1 H), 7.93 (d, 1 H), 7.63-7.72 (m, 2 H), 7.50-7.61 (m, 1 H), 6.93-7.28 (m, 1 H), 6.77 (br s, 1 H), 4.42-4.59 (m, 1 H), 3.59-4.20 (m, 1 H), 3.09 (br s, 1 H), 2.87 (br d, 1 H) | 454.1 [M+H]⁺ | 97.7% |
| 467 | (CD₃OD) δ 8.45 (br s, 1 H), 7.62-7.71 (m, 3 H), 7.35-7.45 (m, 2 H), 6.93-7.30 (m, 1 H), 6.78 (br s, 1 H), 4.54 (br d, 1 H), 3.51-4.02 (m, 1 H), 2.81-3.16 (m, 2 H) | 386.2 [M+H]⁺ | 98.2% |
| 468 | (CD₃OD) δ 8.45 (br s, 1 H), 7.62-7.71 (m, 3 H), 7.35-7.45 (m, 2 H), 6.92-7.32 (m, 1 H), 6.78 (br s, 1 H), 4.44-4.64 (m, 1 H), 3.53-4.04 (m, 1 H), 2.82-3.15 (m, 2 H) | 386.2 [M+H]⁺ | 98.4% |
| 469 | (CD₃OD) δ 7.57-7.76 (m, 3 H), 7.41 (qd, 2 H), 6.93-7.30 (m, 1 H), 6.75 (br s, 1 H), 4.56 (br d, 1 H), 3.52-4.04 (m, 1 H), 2.76-3.19 (m, 2 H), 1.48-1.74 (m, 6 H) | 444.3 [M+H]⁺ | 99.4% |
| 470 | (CD₃OD) δ 7.58-7.78 (m, 3 H), 7.33-7.49 (m, 2 H), 6.92-7.31 (m, 1 H), 6.75 (br s, 1 H), 4.56 (br d, 1 H), 3.53-4.04 (m, 1 H), 2.79-3.20 (m, 2 H), 1.46-1.73 (m, 6 H) | 444.3 [M+H]⁺ | 98.0% |
| 471 | (CD₃OD) δ 7.70 (s, 1 H), 7.34-7.56 (m, 2 H), 7.08-7.19 (m, 1 H), 6.81 (br s, 1 H), 5.06 (br dd, 0.3 H), 4.74 (dd, 0.7 H), 4.00-4.15 (m, 3 H), 3.88 (br s, 0.6 H), 3.50 (br d, 0.4 H), 3.10-3.25 (m, 0.7 H), 2.89-3.04 (m, 0.3 H), 2.73-2.89 (m, 1 H) | 446.0 [M+H]⁺ | 96.6% |
| 472 | (CD₃OD) δ 7.70 (s, 1 H), 7.34-7.56 (m, 2 H), 7.02-7.28 (m, 1 H), 6.81 (br s, 1 H), 5.06 (br dd, 0.3 H), 4.74 (dd, 0.7 H), 3.99-4.17 (m, 3 H), 3.88 (br s, 0.6 H), 3.50 (br d, 0.4 H), 3.11-3.26 (m, 0.7 H), 2.91-3.04 (m, 0.3 H), 2.74-2.89 (m, 1 H) | 446.0 [M+H]⁺ | 94.1% |
| 473 | (CD₃OD) δ 7.69 (s, 1 H), 7.45-7.58 (m, 1 H), 7.29-7.42 (m, 1 H), 7.18-7.27 (m, 1 H), 6.78-7.16 (m, 1 H), 5.06 (br dd, 0.3 H), 4.75 (dd, 0.7H), 3.99-4.13 (m, 3 H), 3.87 (br t, 0.7 H), 3.43-3.57 (m, 0.3 H), 3.10-3.24 (m, 0.7 H), 2.92-3.04 (m, 0.3 H), 2.74-2.90 (m, 1 H) | 448.1 [M+H]⁺ | 99.2% |
| 474 | (CD₃OD) δ 7.69 (s, 1 H), 7.45-7.58 (m, 1 H), 7.30-7.42 (m, 1 H), 7.17-7.28 (m, 1 H), 6.77-7.16 (m, 1 H), 5.06 (br dd, 0.3 H), 4.75 (dd, 0.7 H), 3.99-4.10 (m, 3 H), 3.40-3.95 (m, 1 H), 2.69-3.24 (m, 2 H) | 446.1 [M+H]⁺ | 99.3% |
| 475 | (CD₃OD) δ 8.33 (br s, 1 H), 8.12 (s, 1 H), 7.86 (d, 1 H), 7.69 (s, 1 H), 7.50-7.55 (m, 1 H), 7.38-7.48 (m, 2 H), 7.11-7.20 (m, 1 H), 6.79 (br s, 1 H), 4.76 (br dd, 1 H), 3.95 (br s, 1 H), 3.07-3.27 (m, 5 H), 2.86 (br d, 1 H), 2.59-2.73 (m, 4 H), 2.39 (s, 3 H) | 501.50 [M+H]⁺ | 95.4% |
| 476 | (CD₃OD) δ 8.32 (br s, 1 H), 8.11 (s, 1 H), 7.86 (d, 1 H), 7.69 (s, 1 H), 7.49-7.54 (m, 1 H), 7.36-7.48 (m, 2 H), 7.16 (t, 1 H), 6.79 (br s, 1 H), 4.75 (br dd, 1 H), 3.96 (br s, 1 H), 3.07-3.25 (m, 5 H), 2.80-2.93 (m, 1 H), 2.59-2.73 (m, 4 H), 2.38 (s, 3 H) | 501.50 [M+H]⁺ | 99.5% |
| 477 | (CD₃OD) δ 8.28 (br s, 1 H), 8.09 (d, 1 H), 7.80 (d, 1 H), 7.65 (s, 1 H), 7.44-7.53 (m, 1 H), 7.30-7.42 (m, 2 H), 7.07-7.18 (m, 1 H), 6.75 (br s, 1 H), 4.81-4.85 (m, 0.5 H), 4.72 (dt, 1.5 H), 3.91 (br s, 1 H), 3.25 (br s, 1 H), 3.03-3.18 (m, 3.5 H), 2.82 (br dd, 1 H), 1.81-2.17 (m, 4.5 H) | 504.40 [M+H]⁺ | 100% |
| 478 | (CD₃OD) δ 8.28 (br s, 1 H), 8.09 (d, 1 H), 7.81 (d, 1 H), 7.65 (s, 1 H), 7.45-7.50 (m, 1 H), 7.35-7.43 (m, 2 H), 7.08-7.16 (m, 1 H), 6.75 (br s, 1 H), 4.81-4.86 (m, 0.4 H), 4.72 (dt, 1.6 H), 3.92 (br s, 1 H), 3.26 (br s, 1 H), 3.03-3.18 (m, 3.4 H), 2.82 (br dd, 1 H), 1.88-2.13 (m, 4.6 H) | 504.30 [M+H]⁺ | 99.6% |
| 479 | (CD₃OD) δ 8.30-8.52 (m, 1 H), 8.02-8.20 (m, 1 H), 7.72 (s, 1 H), 7.60 (s, 0.3 H), 7.39-7.51 (m, 1 H), 7.24-7.37 (m, 1 H), 7.11-7.24 (m, 1 H), 6.91 (s, 0.7 H), 5.33 (dd, 0.7 H), 5.11 (br dd, 0.3 H), 3.97-4.06 (m, 3.7 H), 3.63-3.74 (m, 0.3 H), 3.16-3.27 (m, 0.7 H), 2.97-3.08 (m, 0.3 H), 2.84-2.96 (m, 1 H), 2.49-2.68 (m, 3 H) | 431.1 [M+H]⁺ | 100% |
| 480 | (CD₃OD) δ 8.18-8.42 (m, 1 H), 7.89-8.11 (m, 1 H), 7.60 (s, 1 H), 7.48 (br s, 0.3 H), 7.25-7.39 (m, 1 H), 7.12-7.24 (m, 1 H), 7.01-7.11 (m, 1 H), 6.79 (s, 0.7 H), 5.21 (dd, 0.7 H), 4.99 (br dd, 0.3 H), 3.82-3.99 (m, 3.7 H), 3.50-3.65 (m, 0.3 H), 3.02-3.16 (m, 0.7 H), 2.85-2.95 (m, 0.3 H), 2.71-2.84 (m, 1 H), 2.37-2.49 (m, 3 H) | 431.1 [M+H]⁺ | 100% |
| 481 | (CD₃OD) δ 8.56-8.95 (m, 1 H), 8.23-8.45 (m, 1 H), 8.01-8.19 (m, 1 H), 7.62-7.82 (m, 2 H), 7.56 (br s, 0.3 H), 7.39-7.49 (m, 1 H), 7.24-7.36 (m, 1 H), 7.12-7.24 (m, 1 H), 6.94 (s, 0.7 H), 5.28 (dd, 0.7 H), 5.14 (br dd, 0.3 H), 3.99-4.14 (m, 0.7 H), 3.64-3.77 (m, 0.3 H), 3.20-3.31 (m, 0.7 H), 2.99-3.11 (m, 0.3 H), 2.83-2.98 (m, 1 H), 2.49-2.67 (m, 3 H) | 428.1 [M+H]⁺ | 99.8% |
| 482 | (CD₃OD) δ 8.58-8.75 (m, 1 H), 8.11-8.29 (m, 1 H), 7.87-8.05 (m, 1 H), 7.52-7.65 (m, 2 H), 7.44 (br s, 0.3 H), 7.27-7.39 (m, 1 H), 7.12-7.24 (m, 1 H), 7.00-7.12 (m, 1 H), 6.82 (s, 0.7 H), 5.16 (dd, 0.7 H), 5.01 (dd, 0.3 H), 3.86-4.03 (m, 0.7 H), 3.50-3.70 (m, 0.3 H), 3.07-3.18 (m, 0.7 H), 2.87-2.98 (m, 0.3 H), 2.72-2.86 (m, 1 H), 2.36-2.51 (m, 3 H) | 428.1 [M+H]⁺ | 99.3% |
| 483 | (CD₃OD) δ 8.41 (s, 1 H), 7.86 (br s, 1 H), 7.69 (s, 1 H), 7.19-7.27 (m, 1 H), 7.13 (td, 1 H), 6.77 (br s, 1 H), 4.71 (br s, 1 H), 3.58-4.13 (m, 1 H), 2.81-3.19 (m, 2 H) | 372.0 [M+H]⁺ | 99.8% |
| 484 | (CD₃OD) δ 8.41 (s, 1 H), 7.86 (br s, 1 H), 7.69 (s, 1 H), 7.19-7.26 (m, 1 H), 7.13 (td, 1 H), 6.77 (br s, 1 H), 4.72 (br s, 1 H), 3.99 (br s, 1 H), 3.10 (br s, 1 H), 2.88 (br d, 1 H) | 372.0 [M+H]⁺ | 98.0% |
| 485 | (CD₃OD) δ 7.66-7.73 (m, 1 H), 7.08-7.28 (m, 2.3 H), 6.60-7.00 (m, 1.7 H), 5.10 (dd, 0.3 H), 4.77 (dd, 0.7 H), 4.09-4.15 (m, 3 H), 3.90 (br t, 0.7 H), 3.51-3.62 (m, 0.3 H), 2.79-3.26 (m, 2 H) | 436.1 [M+H]⁺ | 100% |
| 486 | (CD₃OD) δ 7.67-7.73 (m, 1 H), 7.06-7.30 (m, 2.3 H), 6.60-7.00 (m, 1.7 H), 5.10 (dd, 0.3 H), 4.77 (dd, 0.7 H), 4.10-4.15 (m, 3 H), 3.81-3.98 (m, 0.7 H), 3.52-3.65 (m, 0.3 H), 2.79-3.27 (m, 2 H) | 436.1 [M+H]⁺ | 98.5% |
| 487 | (CD₃OD) δ 7.47-7.86 (m, 3 H), 7.08-7.30 (m, 2 H), 6.26-6.99 (m, 2 H), 4.30 (br d, 1 H), 3.97 (br s, 1 H), 2.95-3.10 (m, 1 H), 2.82 (br dd, 1 H) | 421.3 [M+H]⁺ | 98.3% |
| 488 | (CD₃OD) δ 7.48-7.86 (m, 3 H), 7.06-7.30 (m, 2 H), 6.26-6.97 (m, 2 H), 4.30 (br d, 1 H), 3.46-4.08 (m, 1 H), 2.95-3.11 (m, 1 H), 2.82 (br dd, 1 H) | 421.3 [M+H]⁺ | 98.2% |
| 489 | (CD₃OD) δ 7.75-7.87 (m, 1 H), 7.47-7.74 (m, 2 H), 7.38 (br d, 1 H), 7.09 (br t, 1 H), 6.70-6.97 (m, 2 H), 4.29 (br d, 1 H), 3.93 (br s, 1 H), 2.92-3.13 (m, 1 H), 2.81 (br d, 1 H) | 421.2 [M+H]⁺ | 99.3% |
| 490 | (CD₃OD) δ 7.76-7.88 (m, 1 H), 7.46-7.74 (m, 2 H), 7.39 (br d, 1 H), 7.09 (br t, 1 H), 6.70-6.99 (m, 2 H), 4.28 (br d, 1 H), 3.93 (br s, 1 H), 2.94-3.12 (m, 1 H), 2.81 (br d, 1 H) | 421.2 [M+H]⁺ | 98.9% |
| 491 | (CD₃OD) δ 8.45 (br s, 1 H), 8.18 (br s, 1 H), 7.56-7.73 (m, 1 H), 7.43-7.54 (m, 2 H), 7.16-7.27 (m, 1 H), 6.88 (s, 1 H), 5.31 (dd, 0.7 H), 5.08 (br dd, 0.3 H), 3.84-4.03 (m, 0.7 H), 3.60 (td, 4.2 Hz, 0.3H), 3.11-3.26 (m, 0.7 H), 2.95-3.07 (m, 0.3 H), 2.76-2.93 (m, 1 H), 2.38-2.49 (m, 3 H) | 417.3 [M+H]⁺ | 100% |
| 492 | (CD₃OD) δ 8.34-8.53 (m, 1 H), 8.07-8.26 (m, 1 H), 7.57-7.72 (m, 1 H), 7.42-7.54 (m, 2 H), 7.19-7.28 (m, 1 H), 6.89 (s, 1 H), 5.31 (br dd, 0.7 H), 5.09 (br dd, 0.3 H), 3.88-3.99 (m, 0.7 H), 3.54-3.65 (m, 0.3 H), 3.13-3.25 (m, 0.7 H), 2.95-3.05 (m, 0.3 H), 2.80-2.93 (m, 1 H), 2.38-2.49 (m, 3 H) | 417.3 [M+H]⁺ | 99.2% |
| 493 | (CD₃OD) δ 8.45 (s, 1 H), 7.97 (d, 1 H), 7.64-7.76 (m, 2 H), 7.50-7.62 (m, 1 H), 6.93-7.26 (m, 1 H), 6.80 (br s, 1 H), 4.40-4.64 (m, 1 H), 3.45-4.13 (m, 1 H), 2.77-3.21 (m, 2 H) | 454.20 [M+H]⁺ | 91.7% |
| 494 | (CD₃OD) δ 8.45 (s, 1 H), 7.97 (d, 1 H), 7.65-7.75 (m, 2 H), 7.52-7.59 (m, 1 H), 6.94-7.26 (m, 1 H), 6.81 (br s, 1 H), 4.53 (br s, 1 H), 3.52-4.08 (m, 1 H), 2.75-3.20 (m, 2 H) | 454.20 [M+H]⁺ | 87.4% |
| 495 | (CD₃OD) δ 7.71 (s, 1 H), 7.48-7.58 (m, 1 H), 7.33-7.45 (m, 1 H), 6.88-7.31 (m, 2 H), 5.08 (dd, 0.3 H), 4.70 (dd, 0.7 H), 3.95-4.05 (m, 3 H), 3.83-3.94 (m, 0.7 H), 3.48 (td, 0.3 H), 2.94-3.25 (m, 1 H), 2.76-2.91 (m, 1 H), 2.29-2.44 (m, 3 H) | 382.3 [M+H]⁺ | 100% |
| 496 | (CD₃OD) δ 7.71 (s, 1 H), 7.48-7.61 (m, 1 H), 7.32-7.45 (m, 1 H), 6.81-7.30 (m, 2 H), 5.08 (br dd, 0.3 H), 4.70 (dd, 0.7 H), 3.95-4.05 (m, 3 H), 3.81-3.95 (m, 0.7 H), 3.42-3.56 (m, 0.3 H), 2.93-3.25 (m, 1 H), 2.75-2.91 (m, 1 H), 2.29-2.45 (m, 3 H) | 382.2 [M+H]⁺ | 99.5% |
| 497 | (CD₃OD) δ 7.70 (d, 1 H), 7.52 (t, 1 H), 7.32-7.42 (m, 1 H), 7.18-7.31 (m, 1 H), 6.81-7.12 (m, 1 H), 5.09 (br dd, 0.4 H), 4.67 (br dd, 0.6 H), 3.94-4.04 (m, 3 H), 3.55-3.91 (m, 1 H), 2.92-3.23 (m, 1 H), 2.75-2.90 (m, 1 H), 1.90-2.13 (m, 1 H), 0.50-1.09 (m, 4 H) | 408.3 [M+H]⁺ | 100% |
| 498 | (CD₃OD) δ 7.70 (d, 1 H), 7.52 (t, 1 H), 7.34-7.42 (m, 1 H), 7.19-7.29 (m, 1 H), 6.86-7.06 (m, 1 H), 5.09 (br dd, 0.4 H), 4.67 (br dd, 0.6 H), 3.94-4.04 (m, 3 H), 3.56-3.92 (m, 1 H), 2.93-3.22 (m, 1 H), 2.73-2.91 (m, 1 H), 1.91-2.10 (m, 1 H), 0.55-1.09 (m, 4 H) | 408.3 [M+H]⁺ | 98.9% |
| 499 | (CD₃OD) δ 7.70 (s, 1 H), 6.95-7.39 (m, 3 H), 6.61-6.82 (m, 1 H), 4.59 (br d, 1 H), 3.61-4.05 (m, 1 H), 2.97-3.23 (m, 1 H), 2.89 (br d, 1 H), 2.47 (s, 3 H), 1.54-1.75 (m, 6 H) | 476.3 [M+H]⁺ | 98.2% |
| 500 | (CD₃OD) δ 7.70 (s, 1 H), 6.94-7.39 (m, 3 H), 6.77 (br s, 1 H), 4.59 (br d, 1 H), 3.62-4.07 (m, 1 H), 3.00-3.26 (m, 1 H), 2.89 (br d, 1 H), 2.47 (s, 3 H), 1.50-1.76 (m, 6 H) | 476.3 [M+H]⁺ | 97.8% |
| 501 | (CD₃OD) δ 8.46 (br s, 1 H), 7.70 (s, 1 H), 6.97-7.36 (m, 3 H), 6.78 (br s, 1 H), 4.55 (br s, 1 H), 3.58-4.11 (m, 1 H), 3.11 (br s, 1 H), 2.87 (br d, 1 H), 2.46 (s, 3 H) | 418.2 [M+H]⁺ | 99.1% |
| 502 | (CD₃OD) δ 8.46 (br s, 1 H), 7.70 (s, 1 H), 6.98-7.36 (m, 3 H), 6.78 (br s, 1 H), 4.56 (br d, 1 H), 3.57-4.05 (m, 1 H), 3.11 (br s, 1 H), 2.87 (br d, 1 H), 2.46 (s, 3 H) | 418.2 [M+H]⁺ | 98.5% |
| 503 | (DMSO-d₆) δ 12.19 (s, 1 H), 8.36 (s, 1 H), 8.23 (s, 1 H), 7.80 (d, 1 H), 7.41-7.69 (m, 4 H), 7.24-7.33 (m, 1 H), 6.51 (s, 1 H), 4.61 (br d, 1 H), 3.94 (br s, 1 H), 3.73-3.78 (m, 4 H), 3.12 (br d, 5 H), 2.81 (br d, 1 H) | 488.4 [M+H]⁺ | 100% |
| 504 | (DMSO-d₆) δ 12.19 (s, 1 H), 8.36 (s, 1 H), 8.23 (s, 1 H), 7.80 (d, 1 H), 7.40-7.71 (m, 4 H), 7.24-7.32 (m, 1 H), 6.51 (s, 1 H), 4.61 (br d, 1 H), 3.94 (br s, 1 H), 3.72-3.80 (m, 4 H), 3.08-3.15 (m, 5 H), 2.81 (br d, 1 H) | 488.4 [M+H]⁺ | 100% |
| 505 | (CD₃OD) δ 8.33 (br s, 1 H), 8.12 (d, 1 H), 7.85 (d, 1 H), 7.70 (s, 1 H), 7.39-7.55 (m, 3 H), 7.13-7.20 (m, 1 H), 6.80 (s, 1 H), 4.76 (br dd, 1 H), 3.95 (br s, 1 H), 3.08-3.26 (m, 5 H), 2.87 (br dd, 1 H), 1.57-1.63 (m, 4 H), 0.41 (s, 4 H) | 512.3 [M+H]⁺ | 98.8% |
| 506 | (CD₃OD) δ 8.32 (br s, 1 H), 8.12 (d, 1 H), 7.85 (d, 1 H), 7.70 (s, 1 H), 7.39-7.55 (m, 3 H), 7.12-7.23 (m, 1 H), 6.80 (s, 1 H), 4.76 (br dd, 1 H), 3.95 (br s, 1 H), 3.07-3.27 (m, 5 H), 2.87 (br dd, 1 H), 1.57-1.65 (m, 4 H), 0.41 (s, 4 H) | 512.3 [M+H]⁺ | 99.3% |
| 507 | (CD₃OD) δ 8.32 (br s, 1 H), 8.09 (s, 1 H), 7.84 (d, 1 H), 7.76 (s, 1 H), 7.49-7.58 (m, 1 H), 7.39-7.48 (m, 2 H), 7.16 (t, 1 H), 6.81 (s, 1 H), 4.76 (br dd, 1 H), 3.93 (br s, 1 H), 3.73 (br d, 2 H), 3.05-3.20 (m, 1 H), 2.80-2.96 (m, 1 H), 2.65 (br t, 2 H), 1.94 (br d, 2 H), 1.42-1.61 (m, 3 H), 1.21 (s, 6 H) | 544.4 [M+H]⁺ | 99.3% |
| 508 | (CD₃OD) δ 8.32 (br s, 1 H), 8.10 (s, 1 H), 7.85 (d, 1 H), 7.71 (s, 1 H), 7.50-7.59 (m, 1 H), 7.38-7.49 (m, 2 H), 7.12-7.21 (m, 1 H), 6.80 (s, 1 H), 4.76 (br dd, 1 H), 3.86-4.05 (m, 1 H), 3.74 (br d, 2 H), 3.13 (br s, 1 H), 2.81-2.90 (m, 1 H), 2.59-2.71 (m, 2 H), 1.94 (br d, 2 H), 1.39-1.62 (m, 3 H), 1.21 (s, 6 H) | 544.4 [M+H]⁺ | 98.6% |
| 509 | (CD₃OD) δ 8.40 (s, 1 H), 7.76-7.99 (m, 1 H), 7.67 (s, 1 H), 7.42 (d, 1 H), 7.27 (t, 1 H), 7.16 (br d, 1 H), 6.77 (br s, 1 H), 4.57-4.77 (m, 1 H), 3.52-4.20 (m, 1 H), 3.09 (br s, 1 H), 2.86 (br d, 1 H), 2.55 (s, 3 H) | 350.3 [M+H]⁺ | 99.6% |
| 510 | (CD₃OD) δ 8.42 (s, 1 H), 7.75-8.13 (m, 1 H), 7.70 (s, 1 H), 7.44 (d, 1 H), 7.29 (t, 1 H), 7.18 (br d, 1 H), 6.79 (br s, 1 H), 4.71 (br s, 1 H), 3.52-4.25 (m, 1 H), 3.11 (br s, 1 H), 2.88 (br d, 1 H), 2.57 (s, 3 H) | 350.2 [M+H]⁺ | 99.7% |
| 511 | (CD₃OD) δ 7.75 (br s, 1 H), 7.67 (s, 1 H), 7.42 (d, 1 H), 7.28 (t, 1 H), 7.17 (br d, 1 H), 6.76 (br s, 1 H), 4.62 (s, 1 H), 3.53-4.08 (m, 1 H), 3.13 (br s, 1 H), 2.87 (br d, 1 H), 2.56 (s, 3 H), 1.64 (br s, 6 H) | 408.3 [M+H]⁺ | 98.5% |
| 512 | (CD₃OD) δ 7.61-7.91 (m, 2 H), 7.42 (d, 1 H), 7.28 (t, 1 H), 7.17 (br d, 1 H), 6.76 (br s, 1 H), 4.72 (br s, 1 H), 3.55-4.15 (m, 1 H), 3.13 (br s, 1 H), 2.87 (br d, 1 H), 2.56 (s, 3 H), 1.64 (br s, 6 H) | 408.3 [M+H]⁺ | 85.5% |
| 513 | (CD₃OD) δ 7.46-7.87 (m, 4 H), 7.28-7.42 (m, 1 H), 6.85 (br d, 1.8 H), 6.33 (br s, 0.2 H), 4.29 (br d, 1 H), 3.43-4.07 (m, 1 H), 2.91-3.14 (m, 1 H), 2.81 (br dd, 1 H) | 421.2 [M+H]⁺ | 99.1% |
| 514 | (CD₃OD) δ 7.45-7.89 (m, 4 H), 7.34 (dt, 1 H), 6.85 (br d, 1.8 H), 6.33 (br s, 0.2 H), 4.29 (br d, 1 H), 3.43-4.04 (m, 1 H), 2.97-3.10 (m, 1 H), 2.81 (br dd, 1 H) | 421.2 [M+H]⁺ | 99.1% |
| 515 | (CD₃OD) δ 8.32-8.47 (m, 1 H), 7.95-8.17 (m, 1 H), 7.71 (s, 1 H), 7.25-7.64 (m, 2.4 H), 6.88 (br s, 0.6 H), 5.34 (dd, 0.7 H), 5.11 (br dd, 0.3 H), 3.93-4.09 (m, 3.7 H), 3.66 (br s, 0.3 H), 2.80-3.24 (m, 2 H) | 453.3 [M+H]⁺ | 96.5% |
| 516 | (CD₃OD) δ 8.32-8.45 (m, 1 H), 7.99-8.19 (m, 1 H), 7.66-7.75 (m, 1 H), 7.19-7.63 (m, 2.4 H), 6.89 (br s, 0.6 H), 5.34 (dd, 0.7 H), 5.11 (br dd, 0.3 H), 3.91-4.05 (m, 3.8 H), 3.66 (br s, 0.2 H), 2.83-3.22 (m, 2 H) | 453.3 [M+H]⁺ | 97.8% |
| 517 | (CD₃OD) δ 8.36-8.42 (m, 1 H), 8.05-8.12 (m, 1 H), 7.44-7.80 (m, 1.4 H), 7.08-7.28 (m, 2 H), 6.88 (br s, 0.6 H), 5.09-5.38 (m, 1 H), 3.69-4.03 (m, 4 H), 2.88-3.24 (m, 2 H) | 453.30 [M+H]⁺ | 93.8% |
| 518 | (CD₃OD) δ 8.36-8.43 (m, 1 H), 8.05-8.13 (m, 1 H), 7.45-7.76 (m, 1.3 H), 7.07-7.29 (m, 2 H), 6.90 (br s, 0.7 H), 5.09-5.39 (m, 1 H), 3.69-4.03 (m, 4 H), 2.87-3.24 (m, 2 H) | 453.40 [M+H]⁺ | 96.3% |
| 519 | (CD₃OD) δ 8.70-8.81 (m, 1 H), 8.26-8.35 (m, 1 H), 8.03-8.13 (m, 1 H), 7.49-7.75 (m, 2.3 H), 7.08-7.28 (m, 2 H), 6.93 (br s, 0.7 H), 5.11-5.35 (m, 1 H), 3.69-4.10 (m, 1 H), 2.89-3.25 (m, 2 H) | 450.20 [M+H]⁺ | 97.0% |
| 520 | (CD₃OD) δ 8.72-8.80 (m, 1 H), 8.26-8.34 (m, 1 H), 8.03-8.12 (m, 1 H), 7.51-7.73 (m, 2.3 H), 7.09-7.28 (m, 2 H), 6.93 (s, 0.7 H), 5.11-5.35 (m, 1 H), 3.70-4.08 (m, 1 H), 3.12-3.28 (m, 1 H), 2.85-2.97 (m, 1 H) | 450.30 [M+H]⁺ | 96.5% |
| 521 | (CD₃OD) δ 8.34-8.42 (m, 1 H), 8.03-8.12 (m, 1 H), 7.67-7.75 (m, 1 H), 7.57 (s, 0.3 H), 7.31-7.42 (m, 1 H), 7.00-7.11 (m, 1 H), 6.86 (s, 0.7 H), 5.33 (dd, 0.7 H), 5.10 (br dd, 0.3 H), 3.98-4.04 (m, 3 H), 3.86-3.97 (m, 0.6 H), 3.56-3.72 (m, 0.4 H), 3.10-3.26 (m, 0.8 H), 2.94-3.04 (m, 0.2 H), 2.81-2.93 (m, 1 H) | 453.2 [M+H]⁺ | 98.3% |
| 522 | (CD₃OD) δ 8.35-8.42 (m, 1 H), 8.03-8.12 (m, 1 H), 7.66-7.74 (m, 1 H), 7.57 (s, 0.3 H), 7.31-7.44 (m, 1 H), 6.98-7.12 (m, 1 H), 6.86 (s, 0.7 H), 5.33 (dd, 0.7 H), 5.10 (dd, 0.3 H), 3.91-4.02 (m, 3.8 H), 3.61-3.70 (m, 0.2 H), 3.13-3.25 (m, 0.7 H), 2.94-3.05 (m, 0.3 H), 2.80-2.93 (m, 1 H) | 453.3 [M+H]⁺ | 99.1% |
| 523 | (CD₃OD) δ 8.72-8.81 (m, 1 H), 8.25-8.34 (m, 1 H), 8.01-8.13 (m, 1 H), 7.60-7.75 (m, 2 H), 7.53 (br s, 0.3 H), 7.33-7.45 (m, 1 H), 6.99-7.12 (m, 1 H), 6.89 (s, 0.7 H), 5.28 (dd, 0.7 H), 5.13 (br dd, 0.3 H), 3.99 (br t, 0.7 H), 3.58-3.76 (m, 0.3 H), 3.16-3.28 (m, 0.7 H), 2.97-3.07 (m, 0.3 H), 2.82-2.95 (m, 1 H) | 450.2 [M+H]⁺ | 100% |
| 524 | (CD₃OD) δ 8.70-8.81 (m, 1 H), 8.25-8.34 (m, 1 H), 8.02-8.13 (m, 1 H), 7.61-7.75 (m, 2 H), 7.52 (br s, 0.3 H), 7.33-7.44 (m, 1 H), 7.01-7.13 (m, 1 H), 6.89 (br s, 0.7 H), 5.28 (br dd, 0.7 H), 5.13 (br dd, 0.3 H), 3.99 (br t, 0.7 H), 3.69 (br s, 0.3 H), 3.17-3.27 (m, 0.7 H), 2.97-3.07 (m, 0.3 H), 2.81-2.95 (m, 1 H) | 450.2 [M+H]⁺ | 97.6% |
| 525 | (CD₃OD) δ 9.38 (br s, 0.7 H), 9.07 (br d, 1.3 H), 8.10-8.40 (m, 1 H), 7.71 (br d, 2 H), 7.45 (br d, 1 H), 7.05-7.40 (m, 2 H), 6.82 (br s, 1 H), 5.03 (br s, 0.3 H), 4.73 (br d, 0.7 H), 4.02 (br s, 0.7 H), 3.72 (br s, 0.3 H), 3.21-3.31 (m, 0.7 H), 3.04 (br s, 0.3 H), 2.92 (br d, 1 H) | 482.2 [M+H]⁺ | 99.2% |
| 526 | (CD₃OD) δ 9.39 (br s, 0.7 H), 9.07 (br s, 1.3 H), 8.08-8.46 (m, 1 H), 7.72 (br d, 2 H), 7.45 (br d, 1 H), 7.04-7.41 (m, 2 H), 6.82 (br s, 1 H), 5.03 (br s, 0.3 H), 4.73 (br d, 0.7 H), 3.88-4.12 (m, 0.7 H), 3.72 (br s, 0.3 H), 3.20-3.30 (m, 0.6 H), 2.99-3.12 (m, 0.4 H), 2.81-2.99 (m, 1 H) | 482.2 [M+H]⁺ | 98.1% |
| 527 | (CD₃OD) δ 9.29-9.54 (m, 1 H), 8.45-8.89 (m, 2 H), 7.59-7.79 (m, 2 H), 7.07-7.51 (m, 3 H), 6.81 (br s, 1 H), 5.01 (br s, 0.3 H), 4.75 (br d, 0.7 H), 3.67-4.11 (m, 1 H), 2.98-3.29 (m, 1 H), 2.92 (br d, 1 H) | 482.3 [M+H]⁺ | 99.5% |
| 528 | (CD₃OD) δ 9.31-9.51 (m, 1 H), 8.41-8.89 (m, 2 H), 7.58-7.77 (m, 2 H), 7.05-7.52 (m, 3 H), 6.81 (s, 1 H), 5.00 (br s, 0.4 H), 4.75 (br d, 0.6 H), 3.65-4.11 (m, 1 H), 2.99-3.30 (m, 1 H), 2.92 (br d, 1 H) | 482.2 [M+H]⁺ | 90.2% |
| 529 | (CD₃OD) δ 7.67-7.79 (m, 2 H), 7.38-7.53 (m, 2 H), 6.93-7.31 (m, 1 H), 6.81 (br s, 1 H), 4.93-5.05 (m, 0.4 H), 4.57 (br s, 0.6 H), 3.52-4.10 (m, 1 H), 2.97-3.26 (m, 1 H), 2.90 (br d, 1 H), 1.50-1.82 (m, 6 H) | 528.3 [M+H]⁺ | 98.7% |
| 530 | (CD₃OD) δ 7.68-7.79 (m, 2 H), 7.39-7.55 (m, 2 H), 6.93-7.31 (m, 1 H), 6.82 (br s, 1 H), 4.94-5.07 (m, 0.3 H), 4.59 (br d, 0.7 H), 3.54-4.07 (m, 1 H), 2.98-3.24 (m, 1 H), 2.90 (br d, 1 H), 1.43-1.77 (m, 6 H) | 528.3 [M+H]⁺ | 97.8% |
| 531 | (CD₃OD) δ 8.36-8.54 (m, 1 H), 7.69-7.76 (m, 2 H), 7.38-7.55 (m, 2 H), 6.97-7.29 (m, 1 H), 6.83 (br s, 1 H), 4.59 (br d, 1 H), 3.53-4.07 (m, 1 H), 3.15 (br s, 1 H), 2.88 (br d, 1 H) | 470.3 [M+H]⁺ | 81.4% |
| 532 | (CD₃OD) δ 8.47 (br s, 1 H), 7.68-7.76 (m, 2 H), 7.39-7.52 (m, 2 H), 6.97-7.33 (m, 1 H), 6.82 (br s, 1 H), 4.55 (br s, 1 H), 3.51-4.11 (m, 1 H), 3.12 (br s, 1 H), 2.89 (br d, 1 H) | 470.3 [M+H]⁺ | 85.3% |
| 533 | (CD₃OD) δ 7.63-7.84 (m, 2 H), 7.51 (t, 1 H), 7.36 (br d, 1 H), 6.95-7.31 (m, 1 H), 6.79 (br s, 1 H), 4.94-5.09 (m, 0.4 H), 4.62 (br s, 0.6 H), 3.54-4.14 (m, 1 H), 2.97-3.26 (m, 1 H), 2.90 (br d, 1 H), 1.48-1.79 (m, 6 H) | 528.2 [M+H]⁺ | 98.0% |
| 534 | (CD₃OD) δ 7.63-7.82 (m, 2 H), 7.52 (t, 1 H), 7.36 (d, 1 H), 6.97-7.31 (m, 1 H), 6.78 (br s, 1 H), 4.94-5.06 (m, 0.4 H), 4.60 (br d, 0.6 H), 3.57-4.13 (m, 1 H), 2.98-3.30 (m, 1 H), 2.90 (br d, 1 H), 1.49-1.75 (m, 6 H) | 528.2 [M+H]⁺ | 98.3% |
| 535 | (CD₃OD) δ 8.47 (br s, 1 H), 7.66-7.79 (m, 2 H), 7.51 (t, 1 H), 7.35 (d, 1 H), 6.97-7.30 (m, 1 H), 6.80 (br s, 1 H), 4.95-5.10 (m, 0.2 H), 4.57 (br s, 0.8 H), 3.54-4.14 (m, 1 H), 3.12 (br s, 1 H), 2.88 (br d, 1 H) | 470.1 [M+H]⁺ | 97.3% |
| 536 | (CD₃OD) δ 8.47 (br s, 1 H), 7.66-7.81 (m, 2 H), 7.51 (t, 1 H), 7.36 (br d, 1 H), 6.97-7.31 (m, 1 H), 6.80 (br s, 1 H), 4.94-5.13 (m, 0.2 H), 4.58 (br d, 0.8 H), 3.54-4.15 (m, 1 H), 3.12 (br s, 1 H), 2.88 (br d, 1 H) | 470.2 [M+H]⁺ | 92.2% |
| 537 | (CD₃OD) δ 8.32-8.49 (m, 2 H), 7.87 (d, 1 H), 7.67 (s, 1 H), 7.48-7.53 (m, 1 H), 7.41 (td, 1 H), 7.33 (d, 1 H), 7.11-7.18 (m, 1 H), 6.77 (br s, 1 H), 4.72 (br dd, 1 H), 3.94 (br s, 1 H), 3.05-3.18 (m, 1 H), 2.84 (br dd, 1 H), 2.39 (s, 3 H) | 417.40 [M+H]⁺ | 99.5% |
| 538 | (CD₃OD) δ 8.32-8.49 (m, 2 H), 7.87 (d, 1 H), 7.67 (s, 1 H), 7.47-7.53 (m, 1 H), 7.41 (td, 1 H), 7.33 (d, 1 H), 7.14 (t, 1 H), 6.77 (br s, 1 H), 4.72 (br dd, 1 H), 3.94 (br s, 1 H), 3.04-3.18 (m, 1 H), 2.84 (br d, 1 H), 2.39 (s, 3 H) | 417.40 [M+H]⁺ | 98.3% |
| 539 | (CD₃OD) δ 8.28-8.36 (m, 2 H), 7.86 (d, 1 H), 7.67 (s, 1 H), 7.47-7.52 (m, 1 H), 7.41 (td, 1 H), 7.23 (dd, 1 H), 7.10-7.18 (m, 1 H), 6.77 (br s, 1 H), 4.74 (br dd, 1 H), 3.89 (s, 4 H), 3.06-3.17 (m, 1 H), 2.84 (br dd, 1 H) | 433.30 [M+H]⁺ | 97.9% |
| 540 | (CD₃OD) δ 8.27-8.37 (m, 2 H), 7.86 (d, 1 H), 7.67 (s, 1 H), 7.47-7.52 (m, 1 H), 7.40 (td, 1 H), 7.22 (dd, 1 H), 7.10-7.17 (m, 1 H), 6.77 (s, 1 H), 4.73 (br dd, 1 H), 3.88 (s, 4 H), 3.06-3.17 (m, 1 H), 2.84 (br dd, 1 H) | 433.30 [M+H]⁺ | 97.2% |
| 541 | (CD₃OD) δ 8.88 (s, 1 H), 8.42 (br s, 1 H), 8.10 (s, 1 H), 7.90-8.03 (m, 2 H), 7.63-7.77 (m, 2 H), 7.50-7.57 (m, 1 H), 7.43 (td, 1 H), 7.10-7.22 (m, 1 H), 6.81 (s, 1 H), 4.69-4.80 (m, 1 H), 3.97 (s, 4 H), 3.07-3.20 (m, 1 H), 2.87 (br dd, 1 H) | 483.4 [M+H]⁺ | 100% |
| 542 | (CD₃OD) δ 8.89 (s, 1 H), 8.43 (br s, 1 H), 8.11 (s, 1 H), 7.92-8.04 (m, 2 H), 7.64-7.77 (m, 2 H), 7.50-7.56 (m, 1 H), 7.43 (td, 1 H), 7.09-7.23 (m, 1 H), 6.81 (br s, 1 H), 4.76 (br d, 1 H), 3.97 (s, 4 H), 3.07-3.22 (m, 1 H), 2.80-2.94 (m, 1 H) | 483.5 [M+H]⁺ | 99.8% |
| 543 | (CD₃OD) δ 9.35 (s, 1 H), 8.70 (br d, 1 H), 8.53 (br s, 1 H), 8.05-8.21 (m, 2 H), 7.91-8.04 (m, 2 H), 7.72 (s, 1 H), 7.54 (d, 1 H), 7.39-7.49 (m, 2 H), 7.12-7.27 (m, 1 H), 6.83 (br s, 1 H), 4.72-4.83 (m, 1 H), 3.79-4.19 (m, 1 H), 3.06-3.23 (m, 1 H), 2.89 (br d, 1 H) | 480.3 [M+H]⁺ | 100% |
| 544 | (CD₃OD) δ 9.35 (s, 1 H), 8.70 (br d, 1 H), 8.53 (br s, 1 H), 8.07-8.21 (m, 2 H), 7.91-8.06 (m, 2 H), 7.75 (s, 1 H), 7.52-7.63 (m, 1 H), 7.38-7.50 (m, 2 H), 7.18 (t, 1 H), 6.84 (br s, 1 H), 4.78 (br d, 1 H), 4.02 (s, 1 H), 3.16 (br s, 1 H), 2.89 (br d, 1 H) | 480.3 [M+H]⁺ | 100% |
| 545 | (CD₃OD) δ 8.71-8.81 (m, 1 H), 8.24-8.35 (m, 1 H), 8.02-8.13 (m, 1 H), 7.69-7.76 (m, 1 H), 7.61-7.69 (m, 1 H), 7.43-7.58 (m, 1.4 H), 7.25-7.39 (m, 1 H), 6.92 (s, 0.6 H), 5.28 (dd, 0.7 H), 5.13 (dd, 0.3 H), 3.93-4.05 (m, 0.7 H), 3.69 (td, 0.3 H), 2.87-3.25 (m, 2 H) | 450.3 [M+H]⁺ | 100% |
| 546 | (CD₃OD) δ 8.72-8.81 (m, 1 H), 8.24-8.36 (m, 1 H), 8.03-8.14 (m, 1 H), 7.69-7.75 (m, 1 H), 7.61-7.68 (m, 1 H), 7.43-7.57 (m, 1.3 H), 7.27-7.41 (m, 1 H), 6.92 (s, 0.7 H), 5.28 (dd, 0.7 H), 5.13 (dd, 0.3 H), 3.95-4.05 (m, 0.7 H), 3.64-3.75 (m, 0.3 H), 2.84-3.25 (m, 2 H) | 450.2 [M+H]⁺ | 98.5% |
| 547 | (CD₃OD) δ 8.31 (s, 1 H), 7.75 (br s, 1 H), 7.53-7.60 (m, 3 H), 7.26-7.34 (m, 2 H), 6.68 (br s, 1 H), 4.59 (br s, 1 H), 3.83 (br s, 1 H), 2.99 (br s, 1 H), 2.76 (br d, 1 H) | 336.2 [M+H]⁺ | 99.3% |
| 548 | (CD₃OD) δ 8.43 (s, 1 H), 7.87 (br s, 1 H), 7.65-7.72 (m, 3 H), 7.39-7.45 (m, 2 H), 6.80 (br s, 1 H), 4.71 (br s, 1 H), 3.96 (br s, 1 H), 3.13 (br d, 1 H), 2.88 (br d, 1 H) | 336.2 [M+H]⁺ | 99.1% |
| 549 | (CD₃OD) δ 7.69 (s, 1 H), 7.45-7.56 (m, 1 H), 7.14-7.24 (m, 1.3 H), 6.62-7.08 (m, 2.7 H), 5.07 (dd, 0.3 H), 4.73 (dd, 0.7 H), 4.07-4.14 (m, 3 H), 3.80-3.90 (m, 3.7 H), 3.47 (td, 0.3 H), 2.92-3.28 (m, 1 H), 2.76-2.89 (m, 1 H) | 430.2 [M+H]⁺ | 100% |
| 550 | (CD₃OD) δ 7.69 (s, 1 H), 7.42-7.58 (m, 1 H), 7.12-7.24 (m, 1.3 H), 6.62-7.06 (m, 2.7 H), 5.07 (dd, 0.3 H), 4.73 (dd, 0.7 H), 4.06-4.16 (m, 3 H), 3.80-3.92 (m, 3.7 H), 3.47 (td, 0.3 H), 3.14-3.26 (m, 0.7 H), 2.76-3.05 (m, 1.3 H) | 430.2 [M+H]⁺ | 99.1% |
| 551 | (CD₃OD) δ 7.43-7.95 (m, 4 H), 7.19 (br s, 1 H), 7.01 (dd, 1 H), 6.20-6.91 (m, 2 H), 4.92-4.99 (m, 0.3 H), 4.28 (br d, 0.7 H), 3.46-3.99 (m, 4 H), 2.95-3.11 (m, 1 H), 2.74-2.87 (m, 1 H) | 415.2 [M+H]⁺ | 97.1% |
| 552 | (CD₃OD) δ 7.60-7.92 (m, 4 H), 7.21 (br s, 1 H), 6.11-7.09 (m, 3 H), 4.94-5.07 (m, 0.3 H), 4.30 (br d, 0.7 H), 3.47-4.04 (m, 4 H), 2.99-3.11 (m, 1 H), 2.82 (br d, 1 H) | 415.3 [M+H]⁺ | 96.1% |
| 553 | (CD₃OD) δ 8.32-8.45 (m, 1 H), 8.02-8.17 (m, 1 H), 7.72 (s, 1 H), 7.45-7.64 (m, 1 H), 7.14-7.23 (m, 1 H), 6.95-7.08 (m, 1 H), 6.88 (s, 1 H), 5.04-5.36 (m, 1 H), 3.90-4.04 (m, 3.8 H), 3.81-3.87 (m, 3 H), 3.56-3.67 (m, 0.2 H), 2.96-3.25 (m, 1 H), 2.80-2.94 (m, 1 H) | 447.3 [M+H]⁺ | 100% |
| 554 | (CD₃OD) δ 8.27-8.47 (m, 1 H), 8.01-8.18 (m, 1 H), 7.72 (s, 1 H), 7.42-7.63 (m, 1 H), 7.13-7.24 (m, 1 H), 6.96-7.05 (m, 1 H), 6.88 (s, 1 H), 5.04-5.38 (m, 1 H), 3.89-4.04 (m, 3.8 H), 3.76-3.88 (m, 3 H), 3.51-3.66 (m, 0.2 H), 2.95-3.26 (m, 1 H), 2.79-2.93 (m, 1 H) | 447.3 [M+H]⁺ | 99.5% |
| 555 | (CD₃OD) δ 8.74-8.81 (m, 1 H), 8.27-8.35 (m, 1 H), 8.05-8.13 (m, 1 H), 7.64-7.75 (m, 2 H), 7.42-7.59 (m, 1 H), 7.13-7.25 (m, 1 H), 6.96-7.06 (m, 1 H), 6.91 (s, 1 H), 5.04-5.34 (m, 1 H), 3.92-4.04 (m, 0.7 H), 3.77-3.90 (m, 3 H), 3.57-3.69 (m, 0.3 H), 2.97-3.29 (m, 1 H), 2.82-2.96 (m, 1 H) | 444.2 [M+H]⁺ | 100% |
| 556 | (CD₃OD) δ 8.73-8.82 (m, 1 H), 8.25-8.35 (m, 1 H), 7.99-8.19 (m, 1 H), 7.63-7.78 (m, 2 H), 7.43-7.59 (m, 1 H), 7.11-7.24 (m, 1 H), 6.96-7.06 (m, 1 H), 6.91 (s, 1 H), 5.06-5.34 (m, 1 H), 3.93-4.05 (m, 0.7 H), 3.76-3.89 (m, 3 H), 3.64 (td, 0.3 H), 2.99-3.28 (m, 1 H), 2.85-2.95 (m, 1 H) | 444.2 [M+H]⁺ | 94.9% |
| 557 | (CD₃OD) δ 7.59-7.77 (m, 2 H), 7.43 (dd, 1 H), 7.21 (td, 1 H), 6.69 (br s, 1 H), 4.62 (br s, 1 H), 3.53-4.09 (m, 1 H), 3.12 (br s, 1 H), 2.85 (br d, 1 H), 2.31-2.47 (m, 1 H), 1.39-1.73 (m, 6 H), 0.92-1.04 (m, 4 H) | 452.3 [M+H]⁺ | 98.4% |
| 558 | (CD₃OD) δ 7.64-7.72 (m, 2 H), 7.43 (dd, 1 H), 7.21 (td, 1 H), 6.69 (br s, 1 H), 4.65 (br s, 1 H), 3.94 (br d, 1 H), 3.12 (br s, 1 H), 2.85 (br d, 1 H), 2.31-2.51 (m, 1 H), 1.58 (br s, 6 H), 0.92-1.04 (m, 4 H) | 452.4 [M+H]⁺ | 96.6% |
| 559 | (CD₃OD) δ 8.63-8.91 (m, 2 H), 8.11 (br s, 2 H), 7.51-7.82 (m, 2 H), 7.02-7.48 (m, 3 H), 6.86 (s, 1 H), 4.97 (br s, 0.5 H), 4.73 (br d, 0.5 H), 3.74-4.13 (m, 1 H), 3.03-3.30 (m, 1 H), 2.80-3.01 (m, 1 H) | 481.3 [M+H]⁺ | 96.5% |
| 560 | (CD₃OD) δ 8.67-8.85 (m, 2 H), 8.11 (br s, 2 H), 7.51-7.78 (m, 2 H), 7.03-7.48 (m, 3 H), 6.85 (s, 1 H), 4.97 (br s, 0.4 H), 4.73 (br d, 0.6 H), 3.72-4.15 (m, 1 H), 3.03-3.30 (m, 1 H), 2.97 (br d, 1 H) | 481.4 [M+H]⁺ | 98.1% |
| 561 | (CD₃OD) δ 7.58-7.74 (m, 3 H), 7.33-7.46 (m, 2 H), 6.60-7.22 (m, 2 H), 5.08 (dd, 0.3 H), 4.75 (dd, 0.7 H), 4.07-4.15 (m, 3 H), 3.81-3.92 (m, 0.7 H), 3.49 (td, 0.3 H), 3.15-3.26 (m, 0.7 H), 2.94-3.04 (m, 0.3 H), 2.76-2.90 (m, 1 H) | 400.3 [M+H]⁺ | 98.5% |
| 562 | (CD₃OD) δ 7.57-7.84 (m, 3 H), 7.30-7.52 (m, 2 H), 6.60-7.25 (m, 2 H), 5.10 (br dd, 0.3 H), 4.76 (dd, 0.7 H), 4.07-4.19 (m, 3 H), 3.79-3.95 (m, 0.7 H), 3.44-3.56 (m, 0.3 H), 3.05-3.28 (m, 1 H), 2.74-2.95 (m, 1 H) | 400.3 [M+H]⁺ | 95.3% |
| 563 | (CD₃OD) δ 7.60-7.86 (m, 5 H), 7.34-7.54 (m, 2 H), 6.86 (br s, 2 H), 3.82-4.62 (m, 1.5 H), 3.48 (br s, 0.2 H), 2.93-3.10 (m, 1 H), 2.81 (br d, 1 H) | 385.3 [M+H]⁺ | 96.0% |
| 564 | (CD₃OD) δ 7.49-7.86 (m, 5 H), 7.41 (quin, 2 H), 6.86 (br s, 2 H), 4.91-5.01 (m, 0.5 H), 3.47-4.64 (m, 1.7 H), 2.97-3.09 (m, 1 H), 2.81 (br d, 1 H) | 385.3 [M+H]⁺ | 98.5% |
| 565 | (CD₃OD) δ 8.33-8.44 (m, 1 H), 8.02-8.13 (m, 1 H), 7.71 (s, 1 H), 7.58 (br s, 0.3 H), 7.41-7.52 (m, 1 H), 7.15-7.32 (m, 2 H), 6.90 (s, 0.7 H), 5.32 (dd, 0.7 H), 5.10 (br dd, 0.3 H), 3.92-4.04 (m, 3.8 H), 3.62 (br t, 0.2 H), 3.10-3.26 (m, 0.8 H), 2.96-3.06 (m, 0.3 H), 2.53 (br s, 1 H), 2.45-2.60 (m, 3 H) | 431.1 [M+H]⁺ | 99.4% |
| 566 | (CD₃OD) δ 8.32-8.43 (m, 1 H), 8.03-8.13 (m, 1 H), 7.71 (s, 1 H), 7.57 (br s, 0.3 H), 7.43-7.52 (m, 1 H), 7.15-7.31 (m, 2 H), 6.90 (s, 0.7 H), 5.32 (dd, 0.7 H), 5.10 (br dd, 0.3 H), 3.93-4.02 (m, 3.8H), 3.61 (br d, 0.3 H), 3.11-3.26 (m, 0.8 H), 2.99 (br d, 0.2 H), 2.90 (br d, 1 H), 2.48-2.59 (m, 3 H) | 431.1 [M+H]⁺ | 98.4% |
| 567 | (CD₃OD) δ 8.67-8.86 (m, 1 H), 8.22-8.36 (m, 1 H), 8.01-8.14 (m, 1 H), 7.60-7.77 (m, 2 H), 7.41-7.56 (m, 1 H), 7.15-7.32 (m, 2 H), 6.93 (s, 1 H), 5.26 (dd, 0.7 H), 5.12 (dd, 0.3 H), 3.93-4.06 (m, 0.7 H), 3.58-3.75 (m, 0.3 H), 3.17-3.29 (m, 0.7 H), 2.97-3.08 (m, 0.3 H), 2.81-2.95 (m, 1 H), 2.47-2.59 (m, 3 H) | 428.4 [M+H]⁺ | 100% |
| 568 | (CD₃OD) δ 8.70-8.81 (m, 1 H), 8.19-8.35 (m, 1 H), 8.00-8.14 (m, 1 H), 7.60-7.75 (m, 2 H), 7.41-7.57 (m, 1 H), 7.14-7.32 (m, 2 H), 6.86-6.97 (m, 1 H), 5.26 (dd, 0.7 H), 5.04-5.16 (m, 0.3 H), 3.90-4.08 (m, 0.7 H), 3.57-3.74 (m, 0.3 H), 2.99-3.27 (m, 1 H), 2.82-2.97 (m, 1 H), 2.43-2.59 (m, 3 H) | 428.4 [M+H]⁺ | 100% |
| 569 | (CD₃OD) δ 8.26 (s, 1 H), 7.68 (s, 1 H), 7.51 (d, 1 H), 7.36 (td, 1 H), 7.22 (dd, 1 H), 6.73 (br s, 1 H), 4.61 (br s, 1 H), 3.93 (br s, 1 H), 3.08 (br s, 1 H), 2.74-2.92 (m, 1 H), 2.39 (s, 3 H) | 367.9 [M+H]⁺ | 100% |
| 570 | (CD₃OD) δ 8.26 (s, 1 H), 7.68 (s, 1 H), 7.51 (d, 1 H), 7.36 (td, 1 H), 7.22 (dd, 1 H), 6.73 (br s, 1 H), 4.57 (br s, 1 H), 3.74-4.08 (m, 1 H), 3.08 (br s, 1 H), 2.84 (br dd, 1 H), 2.36-2.42 (m, 3 H) | 368.1 [M+H]⁺ | 99.2% |
| 571 | (CD₃OD) δ 7.70 (br d, 2 H), 7.48 (d, 1 H), 6.94-7.28 (m, 1 H), 6.73 (br s, 1 H), 4.91-5.02 (m, 0.3 H), 4.57 (br d, 0.7 H), 3.55-4.05 (m, 1 H), 2.81-3.21 (m, 2 H), 1.50-1.71 (m, 6 H) | 540.1 [M+H]⁺ | 95.0% |
| 572 | (CD₃OD) δ 7.70 (br d, 2 H), 7.48 (d, 1 H), 6.93-7.28 (m, 1 H), 6.73 (br s, 1 H), 4.90-5.05 (m, 0.3 H), 4.45-4.67 (m, 0.7 H), 3.55-4.09 (m, 1 H), 2.77-3.23 (m, 2 H), 1.50-1.72 (m, 6 H) | 540.0 [M+H]⁺ | 96.2% |
| 573 | (CD₃OD) δ 8.45 (br s, 1 H), 7.70 (br d, 2 H), 7.48 (d, 1 H), 6.96-7.27 (m, 1 H), 6.74 (br s, 1 H), 4.39-4.69 (m, 1 H), 3.52-4.09 (m, 1 H), 3.10 (br s, 1 H), 2.86 (br d, 1 H) | 482.0 [M+H]⁺ | 92.1% |
| 574 | (CD₃OD) δ 8.45 (br s, 1 H), 7.70 (br d, 2 H), 7.48 (d, 1 H), 6.96-7.27 (m, 1 H), 6.74 (br s, 1 H), 4.56 (br d, 1 H), 3.56-4.05 (m, 1 H), 3.10 (br s, 1 H), 2.86 (br d, 1 H) | 484.0 [M+H]⁺ | 93.6% |
| 575 | (CD₃OD) δ 8.41 (s, 1 H), 7.85 (br s, 1 H), 7.68 (s, 1 H), 7.31 (br d, 1 H), 7.15 (td, 1 H), 6.76 (br s, 1 H), 4.59-4.82 (m, 1 H), 3.97 (br s, 1 H), 2.99-3.25 (m, 1 H), 2.87 (br d, 1 H) | 372.1 [M+H]⁺ | 98.8% |
| 576 | (CD₃OD) δ 8.41 (s, 1 H), 7.85 (br s, 1 H), 7.69 (s, 1 H), 7.31 (br d, 1 H), 7.15 (td, 1 H), 6.76 (br s, 1 H), 4.58-4.80 (m, 1 H), 3.68-4.12 (m, 1 H), 3.10 (br s, 1 H), 2.87 (br d, 1 H) | 372.2 [M+H]⁺ | 98.5% |
| 577 | (CD₃OD) δ 7.67-7.75 (m, 1 H), 7.28-7.37 (m, 1 H), 7.08-7.22 (m, 1.3 H), 6.61-7.03 (m, 1.7 H), 5.06-5.16 (m, 0.3 H), 4.79 (dd, 0.7 H), 4.10-4.16 (m, 3 H), 3.91 (s, 0.7 H), 3.58 (br s, 0.3 H), 2.95-3.27 (m, 1 H), 2.78-2.92 (m, 1 H) | 436.0 [M+H]⁺ | 100% |
| 578 | (CD₃OD) δ 7.71 (br s, 1 H), 7.27-7.40 (m, 1 H), 7.07-7.24 (m, 1.3 H), 6.58-7.04 (m, 1.7 H), 5.11 (br dd, 0.3 H), 4.79 (br dd, 0.7 H), 4.05-4.21 (m, 3 H), 3.91 (br s, 0.7 H), 3.58 (br s, 0.3 H), 2.95-3.27 (m, 1 H), 2.77-2.94 (m, 1 H) | 436.0 [M+H]⁺ | 99.3% |
| 579 | (CD₃OD) δ 8.71-8.84 (m, 1 H), 8.21-8.37 (m, 1 H), 7.99-8.15 (m, 1 H), 7.61-7.80 (m, 2 H), 7.54 (s, 0.3 H), 7.27-7.38 (m, 1 H), 7.10-7.24 (m, 1 H), 6.93 (s, 0.7 H), 5.08-5.36 (m, 1 H), 3.63-4.10 (m, 1 H), 2.98-3.29 (m, 1 H), 2.84-2.97 (m, 1 H) | 450.1 [M+H]⁺ | 100% |
| 580 | (CD₃OD) δ 8.73-8.81 (m, 1 H), 8.26-8.35 (m, 1 H), 8.05-8.13 (m, 1 H), 7.60-7.82 (m, 2 H), 7.54 (s, 0.3 H), 7.26-7.38 (m, 1 H), 7.06-7.24 (m, 1 H), 6.92 (s, 0.7 H), 5.08-5.36 (m, 1 H), 3.61-4.09 (m, 1 H), 2.99-3.29 (m, 1 H), 2.84-2.97 (m, 1 H) | 449.9 [M+H]⁺ | 99.2% |
| 581 | (CD₃OD) δ 8.37-8.45 (m, 1 H), 8.07-8.14 (m, 1 H), 7.65-7.74 (m, 3 H), 7.62-7.64 (m, 0.3 H), 7.38-7.47 (m, 2 H), 6.92 (br s, 0.7 H), 5.08-5.38 (m, 1 H), 4.00-4.03 (m, 3 H), 3.92-3.99 (m, 0.6 H), 3.46-3.72 (m, 0.4 H), 3.13-3.28 (m, 1 H), 2.85-2.96 (m, 1 H) | 417.0 [M+H]⁺ | 100% |
| 582 | (CD₃OD) δ 8.35-8.44 (m, 1 H), 8.07-8.15 (m, 1 H), 7.65-7.74 (m, 3 H), 7.62-7.64 (m, 0.3 H), 7.36-7.48 (m, 2 H), 6.92 (s, 0.7 H), 5.07-5.39 (m, 1 H), 4.00-4.04 (m, 3 H), 3.91-3.98 (m, 0.6 H), 3.43-3.72 (m, 0.4 H), 3.13-3.28 (m, 1 H), 2.84-2.95 (m, 1 H) | 416.9 [M+H]⁺ | 99.0% |
| 583 | (CD₃OD) δ 8.62-8.71 (m, 1 H), 8.14-8.24 (m, 1 H), 7.92-8.02 (m, 1 H), 7.60-7.62 (m, 1 H), 7.49-7.59 (m, 3 H), 7.47 (s, 0.3 H), 7.26-7.36 (m, 2 H), 6.83 (s, 0.7 H), 4.98-5.19 (m, 1 H), 3.50-3.92 (m, 1 H), 3.07-3.18 (m, 1 H), 2.73-2.85 (m, 1 H) | 414.1 [M+H]⁺ | 100% |
| 584 | (CD₃OD) δ 8.74-8.83 (m, 1 H), 8.27-8.35 (m, 1 H), 8.05-8.14 (m, 1 H), 7.73 (br d, 1 H), 7.62-7.71 (m, 3 H), 7.59 (br s, 0.3 H), 7.36-7.49 (m, 2 H), 6.95 (s, 0.7 H), 5.10-5.33 (m, 1 H), 3.60-4.07 (m, 1 H), 3.19-3.31 (m, 1 H), 2.92 (br d, 1 H) | 414.2 [M+H]⁺ | 99.3% |
| 585 | (CD₃OD) δ 7.59-7.80 (m, 2 H), 7.44 (dd, 1 H), 7.22 (td, 1 H), 6.82 (s, 0.7 H), 6.20 (br s, 0.3 H), 4.98 (br d, 0.4 H), 4.04-4.16 (m, 0.6 H), 3.88-4.02 (m, 0.7 H), 3.65 (br s, 0.3 H), 2.94-3.10 (m, 1 H), 2.56-2.93 (m, 3 H), 2.24-2.52 (m, 2 H), 1.64-2.12 (m, 2 H) | 492.2 [M+H]⁺ | 99.0% |
| 586 | (CD₃OD) δ 7.53-7.93 (m, 2 H), 7.44 (br d, 1 H), 7.13-7.33 (m, 1 H), 6.82 (br s, 0.7 H), 6.20 (br s, 0.3 H), 4.97 (br s, 0.4 H), 4.03-4.21 (m, 0.6 H), 3.88-4.03 (m, 0.7 H), 3.65 (br s, 0.3 H), 3.02 (br d, 1 H), 2.57-2.94 (m, 3 H), 2.21-2.54 (m, 2 H), 1.66-2.15 (m, 2 H) | 492.1 [M+H]⁺ | 95.5% |
| 587 | (CD₃OD) δ 8.47-8.81 (m, 1 H), 8.16-8.39 (m, 1 H), 7.91-8.13 (m, 1 H), 7.52-7.77 (m, 3 H), 7.04-7.45 (m, 3 H), 6.86 (br s, 1 H), 5.02 (br s, 0.3 H), 4.77 (br d, 0.7 H), 3.70-4.09 (m, 1 H), 3.02-3.30 (m, 1 H), 2.93 (br d, 1 H) | 481.2 [M+H]⁺ | 92.7% |
| 588 | (CD₃OD) δ 8.43-8.82 (m, 1 H), 8.18-8.38 (m, 1 H), 7.89-8.17 (m, 1 H), 7.50-7.77 (m, 3 H), 7.05-7.44 (m, 3 H), 6.86 (br s, 1 H), 5.01 (br s, 0.3 H), 4.76 (br d, 0.7 H), 3.69-4.16 (m, 1 H), 3.04-3.29 (m, 1 H), 2.93 (br d, 1 H) | 481.1 [M+H]⁺ | 93.3% |
| 589 | (CD₃OD) δ 9.20 (br s, 1 H), 8.65 (br s, 1 H), 8.43 (br d, 1 H), 7.36-7.68 (m, 3 H), 7.05-7.35 (m, 3 H), 6.63-7.02 (m, 1 H), 4.82-4.93 (m, 0.5 H), 4.64 (br d, 0.5 H), 3.58-4.02 (m, 1 H), 2.90-3.17 (m, 1 H), 2.85 (br d, 1 H) | 481.2 [M+H]⁺ | 99.7% |
| 590 | (CD₃OD) δ 9.20 (br s, 1 H), 8.65 (br s, 1 H), 8.43 (br d, 1 H), 7.36-7.67 (m, 3 H), 7.03-7.35 (m, 3 H), 6.63-7.02 (m, 1 H), 4.82-4.96 (m, 0.5 H), 4.64 (br d, 0.5 H), 3.51-4.03 (m, 1 H), 2.90-3.18 (m, 1 H), 2.85 (br d, 1 H) | 481.2 [M+H]⁺ | 99.2% |
| 591 | (CD₃OD) δ 7.61-7.95 (m, 2 H), 7.54 (d, 1 H), 7.39 (td, 1 H), 7.04-7.36 (m, 2 H), 6.77-7.03 (m, 2 H), 4.94-5.11 (m, 0.4 H), 4.75 (br s, 0.6 H), 3.63-4.18 (m, 4 H), 3.24 (br s, 0.7 H), 3.02 (br s, 0.3 H), 2.92 (br d, 1 H) | 484.1 [M+H]⁺ | 97.7% |
| 592 | (CD₃OD) δ 7.65-7.89 (m, 2 H), 7.54 (d, 1 H), 7.39 (td, 1 H), 7.04-7.35 (m, 2 H), 6.77-7.04 (m, 2 H), 4.95-5.10 (m, 0.3 H), 4.76 (br s, 0.7 H), 3.65-4.20 (m, 4 H), 3.00-3.29 (m, 1 H), 2.79-2.99 (m, 1 H) | 484.2 [M+H]⁺ | 94.8% |
| 593 | (CD₃OD) δ 8.69 (br s, 1 H), 7.89-8.23 (m, 2 H), 7.68 (s, 1 H), 7.52 (br d, 2 H), 7.37 (td, 1 H), 7.18-7.28 (m, 1 H), 6.77 (br s, 1 H), 4.74 (br s, 1 H), 3.98 (br s, 1 H), 3.03-3.29 (m, 1 H), 2.88 (br d, 1 H), 2.48 (s, 3 H) | 445.3 [M+H]⁺ | 99.2% |
| 594 | (CD₃OD) δ 8.69 (br s, 1 H), 7.88-8.26 (m, 2 H), 7.68 (br s, 1 H), 7.52 (br d, 2 H), 7.32-7.43 (m, 1 H), 7.14-7.29 (m, 1 H), 6.78 (br s, 1 H), 4.56-4.86 (m, 1 H), 3.69-4.15 (m, 1 H), 3.09-3.28 (m, 1 H), 2.89 (br d, 1 H), 2.48 (s, 3 H) | 445.1 [M+H]⁺ | 99.2% |
| 595 | (CD₃OD) δ 8.22 (br s, 1 H), 7.97 (br s, 1 H), 7.67 (s, 1 H), 7.54 (br d, 1 H), 7.33-7.43 (m, 1 H), 7.19-7.30 (m, 1 H), 6.74 (br s, 1 H), 4.75 (br s, 1 H), 3.97 (s, 4 H), 3.11 (br s, 1 H), 2.88 (br d, 1 H), 2.42 (s, 3 H) | 448.0 [M+H]⁺ | 99.1% |
| 596 | (CD₃OD) δ 8.22 (br s, 1 H), 7.97 (br s, 1 H), 7.67 (s, 1 H), 7.54 (br d, 1 H), 7.35-7.41 (m, 1 H), 7.24 (br t, 1 H), 6.74 (br s, 1 H), 4.76 (br s, 1 H), 3.96 (s, 4 H), 3.12 (br d, 1 H), 2.88 (br d, 1 H), 2.42 (s, 3 H) | 448.1 [M+H]⁺ | 99.3% |
| 597 | (CD₃OD) δ 8.25 (s, 1 H), 7.62-7.70 (m, 2 H), 7.47 (dd, 1 H), 7.17 (td, 1 H), 6.70 (br s, 1 H), 4.54 (br s, 1 H), 3.88 (br s, 1 H), 3.06 (br s, 1 H), 2.83 (br d, 1 H), 2.39 (s, 3 H) | 368.1 [M+H]⁺ | 100% |
| 598 | (CD₃OD) δ 8.25 (s, 1 H), 7.61-7.72 (m, 2 H), 7.47 (dd, 1 H), 7.17 (td, 1 H), 6.70 (br s, 1 H), 4.55 (br s, 1 H), 3.87 (br s, 1 H), 3.07 (br s, 1 H), 2.83 (br d, 1 H), 2.39 (s, 3 H) | 368.2 [M+H]⁺ | 99.8% |
| 599 | (CD₃OD) δ 8.25 (s, 1 H), 7.68 (s, 1 H), 7.46-7.53 (m, 1 H), 7.41 (td, 1 H), 7.09-7.18 (m, 1 H), 6.71 (br s, 1 H), 4.57 (br s, 1 H), 3.92 (br s, 1 H), 3.08 (br s, 1 H), 2.84 (br d, 1 H), 2.39 (s, 3 H) | 368.2 [M+H]⁺ | 100% |
| 600 | (CD₃OD) δ 8.25 (s, 1 H), 7.68 (s, 1 H), 7.46-7.52 (m, 1 H), 7.40 (td, 1 H), 7.06-7.18 (m, 1 H), 6.70 (br s, 1 H), 4.57 (br s, 1 H), 3.92 (br s, 1 H), 3.07 (br s, 1 H), 2.74-2.92 (m, 1 H), 2.39 (s, 3 H) | 368.2 [M+H]⁺ | 99.8% |
| 601 | (CD₃OD) δ 7.71 (s, 1 H), 7.30-7.44 (m, 1 H), 7.12-7.29 (m, 1.3 H), 6.62-7.04 (m, 2.7 H), 5.08 (dd, 0.3 H), 4.74 (dd, 0.7 H), 4.09-4.19 (m, 3 H), 3.96-4.06 (m, 3 H), 3.83-3.94 (m, 0.7 H), 3.42-3.55 (m, 0.3 H), 3.17-3.28 (m, 0.7 H), 2.94-3.06 (m, 0.3 H), 2.76-2.92 (m, 1 H) | 430.1 [M+H]⁺ | 99.5% |
| 602 | (CD₃OD) δ 7.70 (s, 1 H), 7.30-7.42 (m, 1 H), 7.12-7.30 (m, 1.3 H), 6.63-7.05 (m, 2.7 H), 5.08 (dd, 0.2 H), 4.74 (dd, 0.8 H), 4.09-4.21 (m, 3 H), 3.97-4.07 (m, 3 H), 3.82-3.95 (m, 0.7 H), 3.49 (td, 0.3 H), 3.18-3.28 (m, 0.7 H), 2.93-3.07 (m, 0.3 H), 2.72-2.92 (m, 1 H) | 430.2 [M+H]⁺ | 97.7% |
| 603 | (CD₃OD) δ 7.49-8.06 (m, 3 H), 7.32-7.43 (m, 1 H), 7.25 (br d, 1 H), 6.74-7.01 (m, 2.8 H), 6.31 (br s, 0.2 H), 4.91-5.07 (m, 0.3 H), 4.31 (br d, 0.7 H), 4.02 (s, 3.8 H), 3.50 (br s, 0.2 H), 2.97-3.12 (m, 1 H), 2.82 (br d, 1 H) | 415.1 [M+H]⁺ | 100% |
| 604 | (CD₃OD) δ 7.49-8.07 (m, 3 H), 7.33-7.44 (m, 1 H), 7.25 (br d, 1 H), 6.75-7.05 (m, 2.8 H), 6.31 (br s, 0.2 H), 4.92-5.03 (m, 0.3 H), 4.31 (br d, 0.7 H), 4.02 (s, 3.8 H), 3.50 (br s, 0.2 H), 2.97-3.12 (m, 1 H), 2.81 (br d, 1 H) | 415.1 [M+H]⁺ | 100% |
| 605 | (CD₃OD) δ 7.69 (s, 1 H), 7.58 (s, 1 H), 7.36 (dd, 1 H), 6.92-7.27 (m, 1 H), 6.75 (br s, 1 H), 4.50-4.70 (m, 1 H), 3.97 (br s, 0.7 H), 3.64 (br s, 0.3 H), 3.15 (br d, 1 H), 2.88 (br d, 1 H), 1.50-1.71 (m, 6 H) | 496.0 [M+H]⁺ | 99.0% |
| 606 | (CD₃OD) δ 7.69 (s, 1 H), 7.58 (s, 1 H), 7.36 (dd, 1 H), 6.91-7.27 (m, 1 H), 6.75 (br s, 1 H), 4.50-4.69 (m, 1 H), 3.97 (br s, 0.7 H), 3.65 (br s, 0.3 H), 3.15 (br d, 1 H), 2.88 (br d, 1 H), 1.51-1.71 (m, 6 H) | 496.0 [M+H]⁺ | 96.5% |
| 607 | (CD₃OD) δ 8.48 (br s, 1 H), 7.72 (s, 1 H), 7.59 (s, 1 H), 7.37 (br d, 1 H), 6.97-7.31 (m, 1 H), 6.79 (br s, 1 H), 4.58 (br d, 1 H), 3.98 (br s, 1 H), 2.98-3.22 (m, 1 H), 2.88 (br d, 1 H) | 437.9 [M+H]⁺ | 98.0% |
| 608 | (CD₃OD) δ 8.47 (br s, 1 H), 7.70 (br d, 1 H), 7.59 (br d, 1 H), 7.37 (br t, 1 H), 6.94-7.30 (m, 1 H), 6.77 (br s, 1 H), 4.48-4.69 (m, 1 H), 3.96 (br s, 1 H), 3.12 (br s, 1 H), 2.87 (br s, 1 H) | 437.9 [M+H]⁺ | 96.4% |
| 609 | (CD₃OD) δ 7.61-7.88 (m, 2.7H), 7.29-7.54 (m, 1.3 H), 7.18 (br t, 1 H), 6.79-6.99 (m, 2 H), 4.30 (br d, 1 H), 3.96 (br s, 1 H), 2.98-3.12 (m, 1 H), 2.84 (br d, 1 H) | 421.0 [M+H]⁺ | 99.2% |
| 610 | (CD₃OD) δ 7.62-7.89 (m, 3 H), 7.28-7.53 (m, 1 H), 7.18 (br t, 1 H), 6.78-6.97 (m, 2 H), 4.31 (br d, 1 H), 3.96 (br s, 1 H), 2.98-3.11 (m, 1 H), 2.84 (br d, 1 H) | 420.9 [M+H]⁺ | 98.7% |
| 611 | (CD₃OD) δ 8.43 (s, 1 H), 7.87 (br s, 1 H), 7.71 (s, 1 H), 7.50 (br d, 1 H), 7.22-7.31 (m, 2 H), 6.80 (br s, 1 H), 4.57-4.77 (m, 1 H), 3.61-4.03 (m, 1 H), 3.13 (br d, 1 H), 2.88 (br d, 1 H), 2.57 (s, 3 H) | 350.1 [M+H]⁺ | 98.9% |
| 612 | (CD₃OD) δ 8.43 (s, 1 H), 7.87 (br s, 1 H), 7.71 (s, 1 H), 7.50 (br d, 1 H), 7.22-7.31 (m, 2 H), 6.80 (br s, 1 H), 4.56-4.79 (m, 1 H), 3.99 (br s, 1 H), 3.12 (br s, 1 H), 2.88 (br d, 1 H), 2.57 (s, 3 H) | 350.2 [M+H]⁺ | 98.3% |
| 613 | (CD₃OD) δ 7.69-7.75 (m, 1 H), 7.46-7.52 (m, 1 H), 7.20-7.31 (m, 2.4 H), 7.01 (s, 0.2 H), 6.87-6.93 (m, 1 H), 6.65-6.80 (m, 0.4 H), 5.10 (dd, 0.3 H), 4.76 (dd, 0.7 H), 4.12 (s, 3 H), 3.84-3.95 (m, 0.7 H), 3.50-3.62 (m, 0.3 H), 3.17-3.28 (m, 0.7 H), 2.96-3.06 (m, 0.3 H), 2.79-2.92 (m, 1 H), 2.50-2.59 (m, 3 H) | 414.0 [M+H]⁺ | 100% |
| 614 | (CD₃OD) δ 7.69-7.74 (m, 1 H), 7.45-7.53 (m, 1 H), 7.19-7.32 (m, 2.4 H), 7.01 (s, 0.2 H), 6.89 (d, 1 H), 6.65-6.81 (m, 0.4 H), 5.10 (dd, 0.3 H), 4.76 (dd, 0.7 H), 4.12 (s, 3 H), 3.86-3.94 (m, 0.7 H), 3.55 (td, 0.3 H), 3.18-3.28 (m, 0.7 H), 2.96-3.07 (m, 0.3 H), 2.79-2.92 (m, 1 H), 2.49-2.59 (m, 3 H) | 414.0 [M+H]⁺ | 99.5% |
| 615 | (CD₃OD) δ 8.09-8.46 (m, 2 H), 7.72 (br s, 1 H), 7.45-7.63 (m, 2 H), 7.05-7.43 (m, 3 H), 6.85 (s, 1 H), 4.97-5.07 (m, 0.4 H), 4.79 (br s, 0.6 H), 3.98 (br s, 4 H), 3.01-3.30 (m, 1 H), 2.92 (br d, 1 H) | 510.9 [M+H]⁺ | 99.8% |
| 616 | (CD₃OD) δ 8.07-8.47 (m, 2 H), 7.72 (br s, 1 H), 7.45-7.62 (m, 2 H), 7.05-7.42 (m, 3 H), 6.84 (s, 1 H), 4.95-5.07 (m, 0.3 H), 4.78 (br d, 0.7 H), 3.98 (br s, 4 H), 2.99-3.29 (m, 1 H), 2.92 (br d, 1 H) | 511.0 [M+H]⁺ | 99.4% |
| 617 | (CD₃OD) δ 7.67 (s, 1 H), 7.53 (d, 1 H), 7.38 (td, 1 H), 7.20-7.29 (m, 1 H), 6.91-7.12 (m, 1 H), 6.71 (s, 1 H), 4.57-4.84 (m, 1 H), 3.63 (br s, 5 H), 2.95-3.19 (m, 1 H), 2.84 (br d, 1 H), 2.53 (br s, 4 H), 2.35 (s, 3 H) | 502.2 [M+H]⁺ | 98.7% |
| 618 | (CD₃OD) δ 7.67 (s, 1 H), 7.53 (d, 1 H), 7.33-7.43 (m, 1 H), 7.20-7.28 (m, 1 H), 6.68-7.11 (m, 2 H), 4.56-4.81 (m, 1 H), 3.63 (br s, 5 H), 2.99-3.18 (m, 1 H), 2.84 (br d, 1 H), 2.54 (br s, 4 H), 2.35 (s, 3 H) | 502.2 [M+H]⁺ | 98.2% |
| 619 | (CD₃OD) δ 8.39 (s, 1 H), 7.65-7.80 (m, 1 H), 7.54 (d, 1 H), 7.39 (td, 1 H), 7.21-7.31 (m, 1 H), 6.88 (s, 0.8 H), 6.11 (br s, 0.2 H), 5.02 (br d, 0.2 H), 3.87-4.09 (m, 1.6 H), 3.63 (br s, 0.2 H), 2.94-3.05 (m, 1 H), 2.75-2.92 (m, 1 H), 1.99 (t, 3 H) | 418.0 [M+H]⁺ | 98.0% |
| 620 | (CD₃OD) δ 8.39 (s, 1 H), 7.67-7.78 (m, 1 H), 7.54 (d, 1 H), 7.39 (td, 1 H), 7.17-7.31 (m, 1 H), 6.88 (s, 0.8 H), 6.11 (br s, 0.2 H), 5.01 (br d, 0.2 H), 3.87-4.07 (m, 1.6 H), 3.63 (br s, 0.2 H), 2.94-3.08 (m, 1 H), 2.75-2.93 (m, 1 H), 1.99 (t, 3 H) | 417.9 [M+H]⁺ | 95.8% |
| 621 | (CD₃OD) δ 8.42 (s, 1 H), 7.87 (br s, 1 H), 7.75 (s, 1 H), 7.33-7.41 (m, 1 H), 7.24 (d, 1 H), 6.94 (d, 1 H), 6.78 (br s, 1 H), 4.70 (br s, 1 H), 4.01 (s, 4 H), 3.00-3.18 (m, 1 H), 2.81-2.92 (m, 1 H) | 366.0 [M+H]⁺ | 98.7% |
| 622 | (CD₃OD) δ 8.42 (s, 1 H), 7.87 (br s, 1 H), 7.72 (s, 1 H), 7.32-7.41 (m, 1 H), 7.24 (d, 1 H), 6.94 (d, 1 H), 6.77 (br s, 1 H), 4.70 (br s, 1 H), 4.00 (s, 4 H), 3.13 (br d, 1 H), 2.87 (br d, 1 H) | 366.0 [M+H]⁺ | 98.3% |
| 623 | (CD₃OD) δ 8.67-8.91 (m, 1 H), 8.26-8.37 (m, 1 H), 8.01-8.18 (m, 1 H), 7.70-7.78 (m, 1 H), 7.63-7.70 (m, 1 H), 7.50-7.61 (m, 0.3 H), 7.30-7.43 (m, 1 H), 7.19-7.29 (m, 1 H), 6.85-6.99 (m, 1.7 H), 5.06-5.33 (m, 1 H), 3.89-4.06 (m, 3.8 H), 3.60-3.75 (m, 0.2 H), 3.18-3.30 (m, 0.8 H), 2.97-3.09 (m, 0.2 H), 2.83-2.96 (m, 1 H) | 444.0 [M+H]⁺ | 95.4% |
| 624 | (CD₃OD) δ 8.55-8.81 (m, 1 H), 8.12-8.29 (m, 1 H), 7.90-8.05 (m, 1 H), 7.58-7.64 (m, 1 H), 7.50-7.58 (m, 1 H), 7.44 (br s, 0.3 H), 7.17-7.30 (m, 1 H), 7.04-7.17 (m, 1 H), 6.67-6.90 (m, 1.7 H), 4.94-5.22 (m, 1 H), 3.78-3.99 (m, 3.7 H), 3.46-3.64 (m, 0.3 H), 3.06-3.18 (m, 0.7 H), 2.86-2.99 (m, 0.3 H), 2.71-2.85 (m, 1 H) | 444.0 [M+H]⁺ | 94.2% |
| 625 | (CD₃OD) δ 8.26-8.57 (m, 1 H), 8.06-8.18 (m, 1 H), 7.72 (s, 1 H), 7.57 (br s, 0.3 H), 7.31-7.42 (m, 1 H), 7.18-7.30 (m, 1 H), 6.81-7.01 (m, 1.7 H), 5.03-5.42 (m, 1 H), 3.89-4.09 (m, 6.8 H), 3.65 (br s, 0.2 H), 3.12-3.27 (m, 0.7 H), 2.96-3.07 (m, 0.3 H), 2.90 (br d, 1 H) | 447.0 [M+H]⁺ | 99.6% |
| 626 | (CD₃OD) δ 8.17-8.43 (m, 1 H), 7.90-8.07 (m, 1 H), 7.54-7.67 (m, 1 H), 7.46 (s, 0.3 H), 7.18-7.31 (m, 1 H), 7.02-7.17 (m, 1 H), 6.66-6.91 (m, 1.7 H), 4.93-5.27 (m, 1 H), 3.78-3.96 (m, 6.8 H), 3.45-3.61 (m, 0.2 H), 3.01-3.15 (m, 0.7 H), 2.83-2.95 (m, 0.3 H), 2.68-2.82 (m, 1 H) | 447.0 [M+H]⁺ | 98.7% |
| 627 | (CD₃OD) δ 9.40-9.53 (m, 1 H), 8.78-8.92 (m, 2 H), 7.60-7.79 (m, 2 H), 7.40-7.58 (m, 1.3 H), 7.15-7.29 (m, 1 H), 6.92 (br s, 0.7 H), 5.29 (dd, 0.7 H), 5.15 (dd, 0.3 H), 4.02 (br t, 0.7 H), 3.69 (br s, 0.3 H), 3.14-3.30 (m, 1 H), 2.86-3.10 (m, 1 H) | 433.0 [M+H]⁺ | 99.1% |
| 628 | (CD₃OD) δ 9.40-9.52 (m, 1 H), 8.80-8.90 (m, 2 H), 7.64-7.77 (m, 2 H), 7.38-7.56 (m, 1.3 H), 7.16-7.27 (m, 1 H), 6.92 (br s, 0.7 H), 5.29 (dd, 0.7 H), 5.15 (dd, 0.3 H), 4.01 (br t, 0.7 H), 3.62-3.75 (m, 0.3 H), 3.14-3.30 (m, 1 H), 2.86-3.09 (m, 1 H) | 432.9 [M+H]⁺ | 97.5% |
| 629 | (CD₃OD) δ 8.74-8.85 (m, 1 H), 8.27-8.38 (m, 1 H), 8.05-8.16 (m, 1 H), 7.60-7.77 (m, 2 H), 7.32-7.55 (m, 2.4 H), 6.91 (s, 0.6 H), 5.10-5.37 (m, 1 H), 3.66-4.11 (m, 1 H), 3.21-3.27 (m, 0.5 H), 2.86-3.11 (m, 1.5 H) | 450.1 [M+H]⁺ | 98.9% |
| 630 | (CD₃OD) δ 8.75-8.83 (m, 1 H), 8.26-8.38 (m, 1 H), 8.04-8.16 (m, 1 H), 7.62-7.81 (m, 2 H), 7.33-7.56 (m, 2.4 H), 6.91 (s, 0.6 H), 5.11-5.37 (m, 1 H), 3.67-4.11 (m, 1 H), 3.20-3.29 (m, 0.6 H), 2.87-3.10 (m, 1.4 H) | 450.1 [M+H]⁺ | 96.6% |
| 631 | (CD₃OD) δ 8.23-8.75 (m, 2 H), 7.52-7.95 (m, 3 H), 7.02-7.46 (m, 3 H), 6.85 (br s, 1 H), 5.01 (br s, 0.4 H), 4.77 (br d, 0.6 H), 3.70-4.14 (m, 1 H), 3.00-3.31 (m, 1 H), 2.93 (br d, 1 H) | 499.1 [M+H]⁺ | 99.4% |
| 632 | (CD₃OD) δ 8.08-8.66 (m, 2 H), 7.39-7.85 (m, 3 H), 6.91-7.33 (m, 3 H), 6.72 (br s, 1 H), 4.86-4.96 (m, 0.4 H), 4.65 (br d, 0.6 H), 3.55-4.00 (m, 1 H), 2.87-3.18 (m, 1 H), 2.80 (br d, 1 H) | 499.1 [M+H]⁺ | 98.8% |
| 633 | (CD₃OD) δ 8.62-9.17 (m, 1 H), 8.25-8.56 (m, 2 H), 7.73 (br s, 1 H), 7.55 (br d, 1 H), 7.07-7.46 (m, 3 H), 6.86 (br s, 1 H), 5.02 (br s, 0.4 H), 4.77 (br d, 0.6 H), 3.76-4.09 (m, 1 H), 3.02-3.30 (m, 1 H), 2.94 (br d, 1 H) | 549.1 [M+H]⁺ | 99.0% |
| 634 | (CD₃OD) δ 8.65-9.18 (m, 1 H), 8.26-8.55 (m, 2 H), 7.73 (br s, 1 H), 7.54 (br d, 1 H), 7.06-7.45 (m, 3 H), 6.86 (br s, 1 H), 5.02 (br s, 0.4 H), 4.77 (br d, 0.6 H), 3.77-4.13 (m, 1 H), 3.01-3.31 (m, 1 H), 2.94 (br d, 1 H) | 549.1 [M+H]⁺ | 99.0% |
| 635 | (CD₃OD) δ 8.35 (br s, 1 H), 7.87-8.18 (m, 1 H), 7.70 (br s, 1 H), 7.54 (br s, 1 H), 7.38 (br s, 1 H), 7.00-7.33 (m, 2 H), 6.81 (br s, 1 H), 4.73 (br s, 1 H), 3.69-4.07 (m, 4 H), 2.82-3.26 (m, 2 H) | 484.2 [M+H]⁺ | 99.1% |
| 636 | (CD₃OD) δ 8.35 (br s, 1 H), 8.08 (br s, 1 H), 7.69 (br s, 1 H), 7.54 (br s, 1 H), 7.38 (br s, 1 H), 6.97-7.34 (m, 2 H), 6.81 (br s, 1 H), 4.57-4.84 (m, 1 H), 3.47-4.07 (m, 4 H), 2.83-3.27 (m, 2 H) | 484.1 [M+H]⁺ | 98.9% |
| 637 | (CD₃OD) δ 8.45-8.78 (m, 1 H), 7.90-8.34 (m, 2 H), 7.52-7.78 (m, 3 H), 7.41 (td, 1 H), 7.22-7.31 (m, 1 H), 6.91 (s, 1 H), 4.99-5.09 (m, 0.2 H), 4.28 (br dd, 0.8 H), 3.95-4.07 (m, 0.7 H), 3.68-3.80 (m, 0.3 H), 2.83-3.19 (m, 2 H) | 499.0 [M+H]⁺ | 98.7% |
| 638 | (CD₃OD) δ 8.34-8.66 (m, 1 H), 7.82-8.24 (m, 2 H), 7.41-7.64 (m, 3 H), 7.29 (td, 1 H), 7.10-7.19 (m, 1 H), 6.25-6.85 (m, 1 H), 4.88-4.98 (m, 0.2 H), 4.16 (br dd, 0.8 H), 3.83-3.96 (m, 0.7 H), 3.56-3.71 (m, 0.3 H), 2.72-3.06 (m, 2 H) | 499.0 [M+H]⁺ | 97.0% |
| 639 | (CD₃OD) δ 7.78-7.86 (m, 1 H), 7.72 (s, 1 H), 7.58 (br s, 0.5 H), 7.30-7.42 (m, 1 H), 7.18-7.28 (m, 1 H), 6.85-7.03 (m, 2.5 H), 5.29 (br dd, 0.7 H), 5.10 (br d, 0.3 H), 3.91-4.10 (m, 6.7 H), 3.54-3.76 (m, 0.3 H), 3.14-3.28 (m, 1 H), 2.84-3.06 (m, 1 H) | 447.0 [M+H]⁺ | 100% |
| 640 | (CD₃OD) δ 7.83 (br s, 1 H), 7.72 (s, 1 H), 7.58 (br s, 0.3 H), 7.30-7.41 (m, 1 H), 7.17-7.29 (m, 1 H), 6.83-7.05 (m, 2.7 H), 5.29 (dd, 0.7 H), 5.05-5.15 (m, 0.3 H), 3.93-4.07 (m, 6.9 H), 3.64 (br s, 0.1 H), 3.13-3.28 (m, 1 H), 2.83-3.07 (m, 1 H) | 447.0 [M+H]⁺ | 100% |
| 641 | (CD₃OD) δ 7.61-7.82 (m, 2 H), 7.38-7.52 (m, 2 H), 7.02-7.37 (m, 3 H), 6.81 (br s, 1 H), 4.77 (br d, 1 H), 4.19 (br s, 3 H), 3.99 (br s, 1 H), 3.17-3.30 (m, 1 H), 2.89 (br d, 1 H) | 484.1 [M+H]⁺ | 86.5% |
| 642 | (CD₃OD) δ 7.46-7.67 (m, 2 H), 7.27-7.40 (m, 2 H), 6.91-7.25 (m, 3 H), 6.69 (br s, 1 H), 4.65 (br d, 1 H), 3.95-4.16 (m, 3 H), 3.87 (br s, 1 H), 3.06-3.18 (m, 1 H), 2.77 (br d, 1 H) | 484.1 [M+H]⁺ | 96.6% |
| 643 | (CD₃OD) δ 7.68 (s, 1 H), 6.95-7.29 (m, 2 H), 6.85 (dd, 1 H), 6.59-6.81 (m, 1 H), 4.57 (br d, 1 H), 3.88-4.06 (m, 1 H), 3.83 (s, 3 H), 2.98-3.26 (m, 1 H), 2.87 (br d, 1 H), 1.48-1.77 (m, 6 H) | 492.2 [M+H]⁺ | 99.0% |
| 644 | (CD₃OD) δ 7.69 (s, 1 H), 6.95-7.35 (m, 2 H), 6.86 (dd, 1 H), 6.74 (br s, 1 H), 4.57 (br d, 1 H), 3.89-4.08 (m, 1 H), 3.83 (s, 3 H), 2.94-3.24 (m, 1 H), 2.87 (br d, 1 H), 1.50-1.74 (m, 6 H) | 492.2 [M+H]⁺ | 98.4% |
| 645 | (CD₃OD) δ 8.31-8.49 (m, 1 H), 7.99-8.21 (m, 1 H), 7.67-7.79 (m, 1 H), 6.79-7.60 (m, 3 H), 5.08-5.43 (m, 1 H), 3.63-4.17 (m, 4 H), 3.02-3.31 (m, 1 H), 2.92 (br d, 1 H) | 453.1 [M+H]⁺ | 100% |
| 646 | (CD₃OD) δ 8.39-8.49 (m, 1 H), 8.01-8.19 (m, 1 H), 7.70-7.80 (m, 1 H), 6.80-7.62 (m, 3 H), 5.10-5.46 (m, 1 H), 3.67-4.05 (m, 4 H), 3.05-3.28 (m, 1 H), 2.86-3.00 (m, 1 H) | 453.1 [M+H]⁺ | 98.8% |
| 647 | (CD₃OD) δ 7.69 (s, 1 H), 7.54 (d, 1 H), 7.40 (td, 1 H), 7.22-7.30 (m, 1 H), 6.97-7.14 (m, 1 H), 6.73 (s, 1 H), 4.76 (br s, 1 H), 3.78 (br s, 5 H), 3.61 (br s, 4 H), 2.99-3.17 (m, 1 H), 2.86 (br d, 1 H) | 489.1 [M+H]⁺ | 94.6% |
| 648 | (CD₃OD) δ 7.74 (s, 1 H), 7.54 (d, 1 H), 7.40 (td, 1 H), 7.21-7.33 (m, 1 H), 6.96-7.15 (m, 1 H), 6.75 (s, 1 H), 4.80 (br s, 1 H), 3.78 (br s, 5 H), 3.61 (br s, 4 H), 2.96-3.19 (m, 1 H), 2.86 (br d, 1 H) | 489.2 [M+H]⁺ | 97.7% |
| 649 | (CD₃OD) δ 8.32-8.49 (m, 1 H), 7.63-7.77 (m, 2 H), 7.40-7.55 (m, 1 H), 7.21 (td, 1 H), 6.85 (s, 0.8 H), 6.06 (br s, 0.2 H), 5.00 (br dd, 0.2 H), 3.84-4.07 (m, 1.6 H), 3.55-3.69 (m, 0.2 H), 2.91-3.06 (m, 1 H), 2.74-2.90 (m, 1 H), 1.99 (t, 3 H) | 418.1 [M+H]⁺ | 99.0% |
| 650 | (CD₃OD) δ 8.26-8.50 (m, 1 H), 7.60-7.80 (m, 2 H), 7.41-7.56 (m, 1 H), 7.21 (td, 1 H), 6.85 (s, 0.8 H), 6.06 (br s, 0.2 H), 4.97-5.03 (m, 0.2 H), 3.98 (br d, 1.6 H), 3.60 (br s, 0.2 H), 2.92-3.06 (m, 1 H), 2.71-2.91 (m, 1 H), 1.99 (t, 3 H) | 418.1 [M+H]⁺ | 99.0% |
| 651 | (CD₃OD) δ 8.23-8.35 (m, 1 H), 7.54-7.67 (m, 1 H), 7.23-7.46 (m, 2 H), 7.05 (t, 1 H), 6.74 (s, 0.8 H), 5.96 (br s, 0.2 H), 4.89 (br dd, 0.2 H), 3.87 (br d, 1.6 H), 3.44-3.59 (m, 0.2 H), 2.80-2.93 (m, 1 H), 2.62-2.80 (m, 1 H), 1.87 (t, 3 H) | 418.1 [M+H]⁺ | 97.4% |
| 652 | (CD₃OD) δ 8.30-8.46 (m, 1 H), 7.63-7.80 (m, 1 H), 7.35-7.57 (m, 2 H), 7.17 (t, 1 H), 6.86 (br s, 0.8 H), 6.08 (br s, 0.2 H), 4.99-5.07 (m, 0.2 H), 3.84-4.11 (m, 1.6 H), 3.55-3.72 (m, 0.2 H), 2.98 (br dd, 1 H), 2.73-2.92 (m, 1 H), 1.91-2.09 (m, 3 H) | 418.1 [M+H]⁺ | 98.8% |
| 653 | (CD₃OD) δ 8.54-8.73 (m, 1 H), 7.87-8.04 (m, 1 H), 7.66-7.84 (m, 2 H), 7.42-7.57 (m, 2 H), 7.17-7.32 (m, 1 H), 6.89 (br s, 1 H), 5.06-5.29 (m, 1 H), 3.57-4.12 (m, 1 H), 3.23 (br s, 1 H), 2.90 (br d, 1 H), 2.38-2.49 (m, 3 H) | 446.1 [M+H]⁺ | 99.3% |
| 654 | (CD₃OD) δ 8.45-8.77 (m, 1 H), 7.86-8.02 (m, 1 H), 7.65-7.81 (m, 2 H), 7.39-7.58 (m, 2 H), 7.14-7.30 (m, 1 H), 6.91 (s, 1 H), 5.05-5.28 (m, 1 H), 3.51-4.04 (m, 1 H), 2.83-3.28 (m, 2 H), 2.38-2.49 (m, 3 H) | 446.1 [M+H]⁺ | 93.9% |
| 655 | (CD₃OD) δ 7.69 (s, 1 H), 6.95-7.25 (m, 3 H), 6.70 (br s, 1 H), 4.92-5.02 (m, 0.5 H), 4.57 (br s, 0.5 H), 3.55-4.06 (m, 1 H), 2.81-3.22 (m, 2 H), 2.55 (s, 3 H), 1.50-1.72 (m, 6 H) | 476.2 [M+H]⁺ | 98.6% |
| 656 | (CD₃OD) δ 7.69 (s, 1 H), 6.94-7.25 (m, 3 H), 6.72 (br s, 1 H), 4.94-5.03 (m, 0.5 H), 4.56 (br d, 0.5 H), 3.55-4.07 (m, 1 H), 2.80-3.19 (m, 2 H), 2.55 (s, 3 H), 1.50-1.71 (m, 6 H) | 476.2 [M+H]⁺ | 98.2% |
| 657 | (CD₃OD) δ 8.45 (s, 1 H), 7.69 (s, 1 H), 6.98-7.25 (m, 3 H), 6.75 (br s, 1 H), 4.53 (br s, 1 H), 3.50-4.02 (m, 1 H), 2.82-3.13 (m, 2 H), 2.55 (s, 3 H) | 418.1 [M+H]⁺ | 98.0% |
| 658 | (CD₃OD) δ 8.45 (s, 1 H), 7.70 (s, 1 H), 6.98-7.26 (m, 3 H), 6.75 (br s, 1 H), 4.47-4.63 (m, 1 H), 3.50-4.04 (m, 1 H), 2.82-3.15 (m, 2 H), 2.55 (s, 3 H) | 418.1 [M+H]⁺ | 99.1% |
| 659 | (CD₃OD) δ 7.68 (s, 1 H), 6.62-7.28 (m, 4 H), 4.49-4.66 (m, 1 H), 4.04 (br s, 1 H), 2.80-3.19 (m, 2 H), 2.45-2.55 (m, 6 H), 1.51-1.73 (m, 6 H) | 472.2 [M+H]⁺ | 99.8% |
| 660 | (CD₃OD) δ 7.68 (br s, 1 H), 6.68-7.27 (m, 4 H), 4.49-4.64 (m, 1 H), 3.62-4.15 (m, 1 H), 2.76-3.22 (m, 2 H), 2.46-2.53 (m, 6 H), 1.53-1.70 (m, 6 H) | 472.2 [M+H]⁺ | 99.3% |
| 661 | (CD₃OD) δ 8.62-9.14 (m, 2 H), 7.50-7.84 (m, 3 H), 7.44 (dd, 1 H), 7.06-7.40 (m, 2 H), 6.81 (br s, 1 H), 5.05 (br s, 0.3 H), 4.76 (br d, 0.7 H), 4.01 (br s, 0.7 H), 3.69 (br s, 0.3 H), 3.21-3.32 (m, 0.7 H), 3.02 (br s, 0.3 H), 2.91 (br d, 1 H) | 482.1 [M+H]⁺ | 90.7% |
| 662 | (CD₃OD) δ 8.61-9.19 (m, 2 H), 7.51-7.83 (m, 3 H), 7.44 (dd, 1 H), 7.06-7.41 (m, 2 H), 6.82 (br s, 1 H), 5.05 (br s, 0.3 H), 4.76 (br d, 0.7 H), 3.56-4.14 (m, 1 H), 3.22-3.32 (m, 0.7 H), 3.00 (br s, 0.3 H), 2.80-2.97 (m, 1 H) | 482.2 [M+H]⁺ | 88.8% |
| 663 | (CD₃OD) δ 8.45 (br s, 1 H), 7.68 (s, 1 H), 6.96-7.32 (m, 2 H), 6.61-6.89 (m, 2 H), 4.53 (br s, 1 H), 3.82-4.04 (m, 4 H), 2.73-3.18 (m, 2 H) | 434.1 [M+H]⁺ | 99.2% |
| 664 | (CD₃OD) δ 8.45 (br s, 1 H), 7.68 (s, 1 H), 6.98-7.27 (m, 2 H), 6.69-6.89 (m, 2 H), 4.49-4.61 (m, 1 H), 3.83 (s, 4 H), 2.79-3.16 (m, 2 H) | 434.1 [M+H]⁺ | 96.5% |
| 665 | (CD₃OD) δ 8.34-8.47 (m, 1 H), 8.05-8.16 (m, 1 H), 7.67-7.77 (m, 1 H), 7.56 (br s, 0.4 H), 7.27-7.39 (m, 1 H), 7.11-7.23 (m, 1 H), 6.89 (br s, 0.6 H), 5.36 (dd, 0.7 H), 5.13 (dd, 0.3 H), 3.90-4.11 (m, 3.7 H), 3.69 (br s, 0.3 H), 3.13-3.27 (m, 1 H), 2.84-3.07 (m, 1 H) | 453.1 [M+H]⁺ | 100% |
| 666 | (CD₃OD) δ 8.35-8.47 (m, 1 H), 8.05-8.16 (m, 1 H), 7.72 (s, 1 H), 7.56 (br s, 0.3 H), 7.33 (br d, 1 H), 7.10-7.24 (m, 1 H), 6.89 (br s, 0.7 H), 5.36 (dd, 0.7 H), 5.13 (dd, 0.3 H), 3.90-4.10 (m, 3.7 H), 3.70 (br s, 0.3 H), 3.13-3.26 (m, 1 H), 2.84-3.07 (m, 1 H) | 453.1 [M+H]⁺ | 98.2% |
| 667 | (CD₃OD) δ 8.57-9.21 (m, 2 H), 7.50-7.83 (m, 3 H), 7.06-7.44 (m, 3 H), 6.86 (br s, 1 H), 5.06 (br s, 0.4 H), 4.77 (br d, 0.6 H), 3.66-4.19 (m, 1 H), 3.21-3.32 (m, 0.7 H), 3.03 (br s, 0.3 H), 2.93 (br d, 1 H) | 482.1 [M+H]⁺ | 97.3% |
| 668 | (CD₃OD) δ 8.58-9.27 (m, 2 H), 7.49-7.84 (m, 3 H), 7.05-7.46 (m, 3 H), 6.85 (br s, 1 H), 5.05 (br s, 0.3 H), 4.77 (br d, 0.7 H), 3.64-4.21 (m, 1 H), 3.19-3.32 (m, 0.7 H), 3.00-3.11 (m, 0.3 H), 2.93 (br d, 1 H) | 482.1 [M+H]⁺ | 88.6% |
| 669 | (CD₃OD) δ 8.37 (s, 0.7 H), 8.16 (br s, 0.3 H), 8.09 (s, 0.6 H), 7.91 (br s, 0.4 H), 7.67-7.74 (m, 1 H), 7.55 (br d, 1 H), 7.40 (br d, 1 H), 7.22-7.31 (m, 1 H), 6.87 (br s, 0.7 H), 6.40 (br s, 0.3 H), 4.92-5.04 (m, 1 H), 4.24 (br d, 0.7 H), 3.96-4.01 (m, 3 H), 3.74 (br s, 0.3 H), 3.00-3.15 (m, 1 H), 2.85-2.94 (m, 1 H) | 502.2 [M+H]⁺ | 100% |
| 670 | (CD₃OD) δ 8.37 (s, 0.7 H), 8.16 (br s, 0.3 H), 8.09 (s, 0.6 H), 7.91 (br s, 0.4 H), 7.73 (br s, 1 H), 7.55 (br d, 1 H), 7.40 (br d, 1 H), 7.22-7.32 (m, 1 H), 6.87 (br s, 0.7 H), 6.40 (br s, 0.3 H), 4.91-5.04 (m, 1 H), 4.24 (br d, 0.7 H), 3.96-4.01 (m, 3 H), 3.73 (br s, 0.3 H), 3.00-3.15 (m, 1 H), 2.83-2.96 (m, 1 H) | 502.2 [M+H]⁺ | 100% |
| 671 | (CD₃OD) δ 8.70-8.90 (m, 1 H), 8.31-8.55 (m, 1 H), 8.02-8.17 (m, 1 H), 7.71-7.82 (m, 1 H), 7.63-7.71 (m, 1 H), 7.55 (s, 0.3 H), 7.37-7.50 (m, 1 H), 7.14-7.30 (m, 1 H), 6.92 (s, 0.7 H), 5.09-5.36 (m, 1 H), 3.92-4.10 (m, 0.7 H), 3.68 (br dd, 0.3 H), 3.18-3.29 (m, 0.8 H), 2.98-3.09 (m, 0.2 H), 2.82-2.97 (m, 1 H) | 516.2 [M+H]⁺ | 100% |
| 672 | (CD₃OD) δ 8.58-8.75 (m, 1 H), 8.19-8.46 (m, 1 H), 7.88-8.06 (m, 1 H), 7.59-7.66 (m, 1 H), 7.51-7.59 (m, 1 H), 7.43 (s, 0.3 H), 7.28-7.38 (m, 1 H), 7.04-7.14 (m, 1 H), 6.80 (s, 0.7 H), 4.97-5.24 (m, 1 H), 3.81-3.96 (m, 0.7 H), 3.56 (td, 0.3 H), 3.05-3.17 (m, 0.8 H), 2.86-2.98 (m, 0.2 H), 2.73-2.86 (m, 1 H) | 516.2 [M+H]⁺ | 99.0% |
| 673 | (CD₃OD) δ 8.75-9.06 (m, 1 H), 8.09-8.59 (m, 1 H), 7.56-7.82 (m, 2 H), 7.40-7.54 (m, 2 H), 7.17-7.31 (m, 1 H), 6.89 (s, 1 H), 5.03-5.39 (m, 1 H), 3.54-4.04 (m, 1 H), 2.81-2.94 (m, 2 H), 2.39-2.57 (m, 3 H) | 467.1 [M+H]⁺ | 100% |
| 674 | (CD₃OD) δ 8.80-9.00 (m, 1 H), 8.20-8.42 (m, 1 H), 7.56-7.84 (m, 2 H), 7.44-7.54 (m, 2 H), 7.14-7.37 (m, 1 H), 6.88 (br s, 1 H), 5.03-5.41 (m, 1 H), 3.53-4.11 (m, 1 H), 2.79-3.27 (m, 2 H), 2.42-2.48 (m, 3 H) | 467.1 [M+H]⁺ | 98.8% |
| 675 | (CD₃OD) δ 7.69 (s, 1 H), 7.55 (d, 1 H), 7.40 (td, 1 H), 7.23-7.31 (m, 1 H), 6.94-7.14 (m, 1 H), 6.72 (br s, 1 H), 4.91-4.99 (m, 1 H), 4.82 (br s, 1 H), 3.62-3.94 (m, 5 H), 2.80-3.13 (m, 2 H), 1.90 (br s, 4 H) | 505.2 [M+H]⁺ | 91.4% |
| 676 | (CD₃OD) δ 7.69 (s, 1 H), 7.55 (d, 1 H), 7.40 (td, 1 H), 7.22-7.30 (m, 1 H), 6.96-7.14 (m, 1 H), 6.73 (br s, 1 H), 4.93 (br s, 1 H), 4.82 (br s, 1 H), 3.63-3.90 (m, 5 H), 2.80-3.14 (m, 2 H), 1.83-2.08 (m, 4 H) | 505.2 [M+H]⁺ | 86.6% |
| 677 | (DMSO-d₆) δ 12.11-12.29 (m, 1 H), 8.42-8.62 (m, 1 H), 8.09-8.28 (m, 1 H), 7.59-7.68 (m, 3 H), 7.48-7.58 (m, 1 H), 7.16-7.30 (m, 1 H), 6.65 (s, 1 H), 4.71-5.21 (m, 1 H), 3.93-3.95 (m, 3 H), 3.41-3.79 (m, 1 H), 2.80-3.14 (m, 2 H), 2.38-2.45 (m, 3 H) | 458.1 [M+H]⁺ | 99.4% |
| 678 | (DMSO-d₆) δ 12.01-12.33 (m, 1 H), 8.40-8.57 (m, 1 H), 8.09-8.29 (m, 1 H), 7.60-7.68 (m, 3 H), 7.44-7.59 (m, 1 H), 7.17-7.26 (m, 1 H), 6.58-6.99 (m, 1 H), 4.92-5.19 (m, 1 H), 3.93-3.96 (m, 3 H), 3.42-3.79 (m, 1 H), 2.81-3.14 (m, 2 H), 2.38-2.44 (m, 3 H) | 458.2 [M+H]⁺ | 98.3% |
| 679 | (CD₃OD) δ 8.59-8.69 (m, 1 H), 7.85-7.98 (m, 1 H), 7.71-7.78 (m, 2 H), 7.37-7.59 (m, 2 H), 7.17-7.26 (m, 1 H), 6.89 (br s, 1 H), 5.02-5.24 (m, 1 H), 3.97 (br s, 1 H), 2.83-3.25 (m, 2 H), 2.42-2.55 (m, 3 H) | 446.1 [M+H]⁺ | 93.0% |
| 680 | (CD₃OD) δ 8.63-8.69 (m, 1 H), 7.94 (q, 1 H), 7.68-7.81 (m, 2 H), 7.44-7.60 (m, 2 H), 7.16-7.26 (m, 1 H), 6.93 (s, 1 H), 5.03-5.27 (m, 1 H), 3.46-4.04 (m, 2 H), 2.84-3.30 (m, 2 H), 2.40-2.56 (m, 3 H) | 446.2 [M+H]⁺ | 95.3% |
| 681 | (CD₃OD) δ 8.82-8.96 (m, 1 H), 8.29-8.40 (m, 1 H), 7.34-7.87 (m, 5 H), 7.16-7.24 (m, 1 H), 6.87 (br s, 1 H), 5.05-5.37 (m, 1 H), 3.45-4.07 (m, 1 H), 2.81-3.25 (m, 2 H), 2.45-2.58 (m, 3 H) | 467.2 [M+H]⁺ | 100% |
| 682 | (CD₃OD) δ 8.86-8.96 (m, 1 H), 8.26-8.38 (m, 1 H), 7.41-7.83 (m, 5 H), 7.10-7.28 (m, 1 H), 6.88 (br s, 1 H), 5.03-5.36 (m, 1 H), 3.44-4.05 (m, 1 H), 2.72-3.24 (m, 2 H), 2.44-2.54 (m, 3 H) | 467.2 [M+H]⁺ | 99.5% |
| 683 | (CD₃OD) δ 7.59-7.78 (m, 3 H), 7.30-7.49 (m, 2 H), 6.79-7.24 (m, 1 H), 5.11 (br dd, 0.3 H), 4.76 (br dd, 0.7 H), 4.17 (s, 3 H), 3.52-3.98 (m, 1 H), 2.96-3.28 (m, 1 H), 2.84 (br d, 1 H) | 418.1 [M+H]⁺ | 100% |
| 684 | (CD₃OD) δ 7.48-7.63 (m, 3 H), 7.23-7.38 (m, 2 H), 6.69-7.05 (m, 1 H), 4.99 (dd, 0.3 H), 4.64 (dd, 0.7 H), 4.02-4.08 (m, 3 H), 3.37-3.86 (m, 1 H), 2.81-3.15 (m, 1 H), 2.63-2.80 (m, 1 H) | 418.1 [M+H]⁺ | 100% |
| 685 | (CD₃OD) δ 7.68-7.76 (m, 1 H), 7.45-7.54 (m, 1 H), 7.20-7.32 (m, 2 H), 6.82-7.15 (m, 1 H), 5.10 (br dd, 0.3 H), 4.76 (dd, 0.7 H), 4.17 (s, 3 H), 3.91 (br t, 0.7 H), 3.61 (br t, 0.3 H), 2.95-3.29 (m, 1 H), 2.75-2.93 (m, 1 H), 2.49-2.62 (m, 3 H) | 432.2 [M+H]⁺ | 100% |
| 686 | (CD₃OD) δ 7.57-7.64 (m, 1 H), 7.34-7.41 (m, 1 H), 7.08-7.20 (m, 2 H), 6.60-7.05 (m, 1 H), 4.98 (dd, 0.3 H), 4.64 (dd, 0.7 H), 4.05 (s, 3 H), 3.75-3.83 (m, 0.7 H), 3.49 (td, 0.3 H), 2.84-3.16 (m, 1 H), 2.67-2.81 (m, 1 H), 2.37-2.49 (m, 3 H) | 432.2 [M+H]⁺ | 98.7% |
| 687 | (CD₃OD) δ 7.56-8.10 (m, 4 H), 7.45 (br s, 1 H), 6.98-7.38 (m, 2 H), 6.78 (br s, 1 H), 4.92-5.08 (m, 0.4 H), 4.69 (br s, 0.6 H), 4.11 (br s, 3.7 H), 3.73 (br s, 0.3 H), 3.16 (br s, 1 H), 2.91 (br s, 1 H) | 484.2 [M+H]⁺ | 98.8% |
| 688 | (CD₃OD) δ 7.57-8.11 (m, 4 H), 7.45 (br s, 1 H), 6.98-7.38 (m, 2 H), 6.78 (br s, 1 H), 4.93-5.09 (m, 0.4 H), 4.70 (br s, 0.6 H), 4.11 (br s, 3.7 H), 3.75 (br s, 0.3 H), 3.02-3.28 (m, 1 H), 2.90 (br s, 1 H) | 484.2 [M+H]⁺ | 98.5% |
| 689 | (CD₃OD) δ 7.61-7.76 (m, 2 H), 7.43 (dd, 1 H), 6.96-7.32 (m, 2 H), 6.75 (br s, 1 H), 4.94-5.12 (m, 0.5 H), 4.58 (br s, 0.5 H), 3.57-4.07 (m, 1 H), 2.94-3.25 (m, 1 H), 2.87 (br d, 1 H), 2.53-2.81 (m, 2 H), 2.18-2.51 (m, 2 H), 1.54-2.11 (m, 2 H) | 474.2 [M+H]⁺ | 99.2% |
| 690 | (CD₃OD) δ 7.59-7.80 (m, 2 H), 7.43 (dd, 1 H), 6.94-7.31 (m, 2 H), 6.75 (br s, 1 H), 4.93-5.12 (m, 0.4 H), 4.58 (br s, 0.6 H), 3.52-4.11 (m, 1 H), 2.94-3.23 (m, 1 H), 2.87 (br d, 1 H), 2.53-2.81 (m, 2 H), 2.12-2.51 (m, 2 H), 1.49-2.10 (m, 2 H) | 474.2 [M+H]⁺ | 100% |
| 691 | (CD₃OD) δ 7.64-8.13 (m, 3 H), 7.56 (br s, 1 H), 7.40 (br d, 1 H), 7.01-7.35 (m, 2 H), 6.82 (s, 1 H), 4.93-5.08 (m, 0.5 H), 4.71 (br s, 0.5 H), 4.11 (br s, 3.7 H), 3.76 (br s, 0.3 H), 3.15 (br s, 1 H), 2.92 (br s, 1 H) | 484.1 [M+H]⁺ | 100% |
| 692 | (CD₃OD) δ 7.64-8.15 (m, 3 H), 7.56 (br s, 1 H), 7.40 (br d, 1 H), 7.01-7.35 (m, 2 H), 6.82 (s, 1 H), 4.91-5.12 (m, 0.5 H), 4.72 (br s, 0.5 H), 4.11 (br s, 3.8 H), 3.77 (br s, 0.2 H), 3.02-3.27 (m, 1 H), 2.92 (br s, 1 H) | 484.2 [M+H]⁺ | 99.5% |
| 693 | (CD₃OD) δ 9.47 (br s, 1 H), 8.36-8.95 (m, 2 H), 7.72 (br s, 1 H), 7.55 (br d, 1 H), 7.05-7.47 (m, 3 H), 6.85 (br s, 1 H), 5.04 (br s, 0.3 H), 4.76 (br d, 0.7 H), 4.05 (br s, 0.7 H), 3.78 (br s, 0.3 H), 3.28 (br s, 0.8 H), 3.03 (br s, 0.2 H), 2.94 (br d, 1 H) | 482.1 [M+H]⁺ | 99.7% |
| 694 | (CD₃OD) δ 9.46 (br s, 1 H), 8.39-8.92 (m, 2 H), 7.72 (br s, 1 H), 7.55 (br d, 1 H), 7.04-7.46 (m, 3 H), 6.85 (br s, 1 H), 5.02 (br s, 0.3 H), 4.77 (br d, 0.7 H), 4.05 (br s, 0.6 H), 3.79 (br s, 0.4 H), 3.28 (br s, 0.7 H), 3.01-3.13 (m, 0.3 H), 2.93 (br d, 1 H) | 482.1 [M+H]⁺ | 99.4% |
| 695 | (CD₃OD) δ 8.25-8.43 (m, 1 H), 7.45-7.84 (m, 4 H), 7.15-7.31 (m, 2 H), 6.89 (br s, 1 H), 5.04-5.35 (m, 1 H), 3.43-4.07 (m, 1 H), 2.79-3.28 (m, 2 H), 2.39-2.52 (m, 3 H) | 467.2 [M+H]+ | 81.1% |
| 696 | (CD₃OD) δ 8.16-8.50 (m, 1 H), 7.45-7.84 (m, 4 H), 7.11-7.34 (m, 2 H), 6.88 (br s, 1 H), 5.03-5.34 (m, 1 H), 3.51-4.04 (m, 1 H), 2.78-3.28 (m, 2 H), 2.39-2.51 (m, 3 H) | 467.2 [M+H]+ | 81.1% |
| 697 | (CD₃OD) δ 7.71 (s, 1 H), 7.22-7.36 (m, 2 H), 6.98-7.11 (m, 1.3 H), 6.90 (s, 0.7 H), 5.07 (dd, 0.3 H), 4.74 (dd, 0.7 H), 4.12-4.22 (m, 3 H), 3.98-4.07 (m, 3 H), 3.80-3.94 (m, 0.7 H), 3.40-3.54 (m, 0.3 H), 2.95-3.29 (m, 1 H), 2.77-2.93 (m, 1 H) | 448.2 [M+H]⁺ | 99.1% |
| 698 | (CD₃OD) δ 7.71 (s, 1 H), 7.21-7.37 (m, 2 H), 6.98-7.15 (m, 1.3 H), 6.90 (br s, 0.7 H), 5.07 (dd, 0.3 H), 4.74 (dd, 0.7 H), 4.12-4.21 (m, 3 H), 3.99-4.05 (m, 3 H), 3.88 (br t, 0.7 H), 3.42-3.54 (m, 0.3 H), 2.95-3.28 (m, 1 H), 2.79-2.93 (m, 1 H) | 448.2 [M+H]⁺ | 98.3% |
| 699 | (CD₃OD) δ 8.31 (br s, 1 H), 8.11 (s, 1 H), 7.83 (d, 1 H), 7.69 (s, 1 H), 7.46 (dd, 1 H), 7.34 (br d, 1 H), 7.16 (td, 1 H), 6.77 (s, 1 H), 4.75 (br dd, 1 H), 3.75-4.09 (m, 2 H), 3.48-3.61 (m, 2 H), 3.06-3.23 (m, 1 H), 2.80-2.97 (m, 3 H), 2.03 (br d, 2 H), 1.59-1.81 (m, 2 H) | 520.2 [M+H]⁺ | 98.2% |
| 700 | (CD₃OD) δ 8.30 (br s, 1 H), 8.11 (s, 1 H), 7.83 (d, 1 H), 7.69 (s, 1 H), 7.46 (dd, 1 H), 7.34 (br d, 1 H), 7.16 (td, 1 H), 6.77 (s, 1 H), 4.75 (br dd, 1 H), 3.72-4.04 (m, 2 H), 3.46-3.60 (m, 2 H), 3.07-3.20 (m, 1 H), 2.80-3.01 (m, 3 H), 2.03 (br d, 2 H), 1.61-1.82 (m, 2 H) | 520.2 [M+H]⁺ | 94.3% |
| 701 | (CD₃OD) δ 8.35 (br s, 1 H), 8.12 (s, 1 H), 7.85 (d, 1 H), 7.69 (s, 1 H), 7.45 (d, 2 H), 7.30 (t, 1 H), 7.20 (d, 1 H), 6.80 (br s, 1 H), 4.76 (br d, 1 H), 3.94 (br s, 1 H), 3.76-3.86 (m, 1 H), 3.46-3.61 (m, 2 H), 3.05-3.19 (m, 1 H), 2.80-3.00 (m, 3 H), 2.59 (s, 3 H), 1.97-2.10 (m, 2 H), 1.63-1.82 (m, 2 H) | 498.3 [M+H]⁺ | 100% |
| 702 | (CD₃OD) δ 8.35 (br s, 1 H), 8.11 (s, 1 H), 7.85 (d, 1 H), 7.69 (s, 1 H), 7.45 (d, 2 H), 7.30 (t, 1 H), 7.19 (d, 1 H), 6.80 (br s, 1 H), 4.71-4.82 (m, 1 H), 3.95 (br s, 1 H), 3.80 (tt, 1 H), 3.48-3.57 (m, 2 H), 3.02-3.21 (m, 1 H), 2.82-2.97 (m, 3 H), 2.59 (s, 3 H), 1.98-2.06 (m, 2 H), 1.63-1.81 (m, 2 H) | 498.3 [M+H]⁺ | 100% |
| 703 | (CD₃OD) δ 8.28-8.35 (m, 1 H), 7.62-7.83 (m, 2 H), 7.39-7.57 (m, 3 H), 7.16-7.25 (m, 2 H), 6.90 (s, 1 H), 5.02-5.30 (m, 1 H), 3.87-4.01 (m, 1 H), 3.44-3.66 (m, 1 H), 2.78-3.25 (m, 2 H), 2.44-2.52 (m, 3 H) | 467.2 [M+H]⁺ | 100% |
| 704 | (CD₃OD) δ 8.29-8.36 (m, 1 H), 7.63-7.83 (m, 2 H), 7.41-7.59 (m, 3 H), 7.16-7.24 (m, 2 H), 6.90 (s, 1 H), 5.04-5.32 (m, 1 H), 3.86-4.03 (m, 1 H), 3.42-3.65 (m, 1 H), 2.80-3.25 (m, 2 H), 2.45-2.51 (m, 3 H) | 467.2 [M+H]⁺ | 99.1% |
| 705 | (CD₃OD) δ 7.96-8.09 (m, 1 H), 7.64-7.72 (m, 2.7 H), 7.51-7.62 (m, 0.3 H), 7.30-7.47 (m, 2 H), 6.83-6.99 (m, 1 H), 4.92-5.13 (m, 1.3 H), 4.37 (dd, 0.7 H), 3.78-3.91 (m, 0.7 H), 3.47 (td, 0.3 H), 2.93-3.21 (m, 1 H), 2.71-2.91 (m, 1 H), 1.38-1.52 (m, 6 H) | 378.2 [M+H]⁺ | 99.7% |
| 706 | (CD₃OD) δ 7.98-8.07 (m, 1 H), 7.62-7.73 (m, 2.8 H), 7.53-7.59 (m, 0.2 H), 7.32-7.46 (m, 2 H), 6.84-6.98 (m, 1 H), 4.92-5.14 (m, 1.4 H), 4.37 (dd, 0.6 H), 3.78-3.91 (m, 0.7 H), 3.47 (td, 0.3 H), 2.92-3.21 (m, 1 H), 2.73-2.91 (m, 1 H), 1.37-1.52 (m, 6 H) | 378.2 [M+H]⁺ | 98.1% |
| 707 | (CD₃OD) δ 7.96-8.09 (m, 1 H), 7.70 (s, 1 H), 7.43-7.56 (m, 1 H), 7.31-7.40 (m, 1 H), 7.15-7.27 (m, 1 H), 6.86-7.06 (m, 1 H), 4.92-5.12 (m, 1.4 H), 4.38 (dd, 0.6 H), 3.78-3.92 (m, 0.7 H), 3.48 (td, 0.3 H), 2.95-3.20 (m, 1 H), 2.73-2.94 (m, 1 H), 1.42-1.53 (m, 6 H) | 396.2 [M+H]⁺ | 100% |
| 708 | (CD₃OD) δ 7.96-8.10 (m, 1 H), 7.70 (s, 1 H), 7.44-7.58 (m, 1 H), 7.28-7.43 (m, 1 H), 7.12-7.27 (m, 1 H), 6.84-7.07 (m, 1 H), 4.97-5.12 (m, 1.3 H), 4.38 (dd, 0.7 H), 3.85 (br t, 0.7 H), 3.41-3.54 (m, 0.3 H), 2.96-3.20 (m, 1 H), 2.74-2.91 (m, 1 H), 1.42-1.53 (m, 6 H) | 396.2 [M+H]⁺ | 99.1% |
| 709 | (CD₃OD) δ 7.90-7.94 (m, 1 H), 7.69-7.75 (m, 1 H), 7.51 (br d, 1 H), 7.23-7.32 (m, 2 H), 6.75-6.98 (m, 1.8 H), 6.11 (s, 0.2 H), 5.00 (br dd, 0.3 H), 3.83-4.05 (m, 1.5 H), 3.46-3.63 (m, 0.2 H), 2.88-3.05 (m, 1 H), 2.71-2.87 (m, 1 H), 2.49-2.60 (m, 3 H) | 417.2 [M+H]⁺ | 98.5% |
| 710 | (CD₃OD) δ 7.90-7.94 (m, 1 H), 7.69-7.75 (m, 1 H), 7.51 (br d, 1 H), 7.23-7.32 (m, 2 H), 6.76-7.00 (m, 1.8 H), 6.11 (s, 0.2 H), 5.00 (br dd, 0.3 H), 3.81-4.06 (m, 1.5 H), 3.48-3.61 (m, 0.2 H), 2.88-3.10 (m, 1 H), 2.72-2.86 (m, 1 H), 2.52-2.59 (m, 3 H) | 417.2 [M+H]⁺ | 96.9% |
| 711 | (CD₃OD) δ 7.65-7.76 (m, 3 H), 7.56 (s, 1 H), 7.37-7.48 (m, 2 H), 6.40-6.94 (m, 2 H), 4.34 (br s, 1 H), 3.95 (br s, 4 H), 2.98-3.08 (m, 1 H), 2.75-2.89 (m, 1 H) | 349.2 [M+H]⁺ | 100% |
| 712 | (CD₃OD) δ 7.63-7.73 (m, 3 H), 7.56 (s, 1 H), 7.37-7.47 (m, 2 H), 6.42-6.93 (m, 2 H), 4.34 (br s, 1 H), 3.95 (s, 4 H), 2.99-3.08 (m, 1 H), 2.76-2.86 (m, 1 H) | 349.2 [M+H]⁺ | 99.2% |
| 713 | (CD₃OD) δ 7.62-7.85 (m, 2 H), 7.53 (br d, 1 H), 7.39 (td, 1 H), 7.18-7.30 (m, 1 H), 6.70-6.86 (m, 1 H), 4.75 (br s, 1 H), 3.98 (br s, 1 H), 3.15 (br s, 1 H), 2.89 (br d, 1 H), 1.65 (br s, 6 H) | 412.2 [M+H]⁺ | 93.3% |
| 714 | (CD₃OD) δ 7.64-7.87 (m, 2 H), 7.53 (br d, 1 H), 7.39 (td, 1 H), 7.18-7.30 (m, 1 H), 6.80 (br s, 1 H), 4.74 (br s, 1 H), 3.98 (br s, 1 H), 3.15 (br s, 1 H), 2.89 (br d, 1 H), 1.65 (br s, 6 H) | 412.1 [M+H]⁺ | 98.8% |
| 715 | (CD₃OD) δ 7.67 (s, 1 H), 6.94-7.25 (m, 2 H), 6.58-6.89 (m, 2 H), 4.90-5.02 (m, 0.5 H), 4.55 (m, 0.5 H), 4.00 (s, 3 H), 3.53-3.98 (m, 1 H), 2.82-3.21 (m, 2 H), 1.54-1.72 (m, 6 H) | 492.2 [M+H]⁺ | 98.6% |
| 716 | (CD₃OD) δ 7.67 (s, 1 H), 6.95-7.24 (m, 2 H), 6.55-6.89 (m, 2 H), 4.90-4.98 (m, 0.5 H), 4.55 (m, 0.5 H), 4.00 (s, 3 H), 3.45-3.98 (m, 1 H), 2.80-3.20 (m, 2 H), 1.54-1.72 (m, 6 H) | 492.2 [M+H]+ | 94.0% |
| 717 | (CD₃OD) δ 8.44 (s, 1 H), 7.68 (s, 1 H), 6.94-7.26 (m, 2 H), 6.60-6.88 (m, 2 H), 4.90-4.96 (m, 0.5 H), 4.53 (m, 0.5 H), 4.00 (s, 4 H), 2.82-3.12 (m, 2 H) | 434.1 [M+H]⁺ | 98.8% |
| 718 | (CD₃OD) δ 8.44 (s, 1 H), 7.68 (s, 1 H), 6.93-7.26 (m, 2 H), 6.62-6.88 (m, 2 H), 4.89-5.01 (m, 0.5 H), 4.53 (m, 0.5 H), 4.00 (s, 4 H), 2.81-3.12 (m, 2 H) | 434.1 [M+H]+ | 93.5% |
| 719 | (CD₃OD) δ 7.67 (s, 1 H), 7.43 (dd, 1 H), 6.97-7.24 (m, 2 H), 6.74 (br s, 1 H), 4.90-5.01 (m, 0.5 H), 4.48-4.56 (m, 0.5 H), 3.58-4.10 (m, 1 H), 2.82-3.21 (m, 2 H), 2.46 (d, 3 H), 1.53-1.72 (m, 6 H) | 476.2 [M+H]⁺ | 98.0% |
| 720 | (CD₃OD) δ 7.67 (s, 1 H), 7.43 (dd, 1 H), 6.97-7.25 (m, 2 H), 6.46-6.89 (m, 1 H), 4.90-5.05 (m, 0.5 H), 4.51-4.58 (m, 0.5 H), 3.57-4.06 (m, 1 H), 2.80-3.16 (m, 2 H), 2.46 (d, 3 H), 1.52-1.71 (m, 6 H) | 476.2 [M+H]+ | 95.9% |
| 721 | (CD₃OD) δ 8.44 (br s, 1 H), 7.68 (s, 1 H), 7.43 (dd, 1 H), 6.99-7.26 (m, 2 H), 6.76 (br s, 1 H), 4.46-4.59 (m, 1 H), 3.99 (br s, 1 H), 2.82-3.10 (m, 2 H), 2.45 (d, 3 H) | 418.1 [M+H]⁺ | 97.8% |
| 722 | (CD₃OD) δ 8.44 (br s, 1 H), 7.68 (s, 1 H), 7.43 (dd, 1 H), 6.99-7.26 (m, 2 H), 6.76 (br s, 1 H), 4.57 (br d, 1 H), 4.00 (br s, 1 H), 2.82-3.12 (m, 2 H), 2.45 (d, 3 H) | 418.1 [M+H]+ | 98.1% |
| 723 | (CD₃OD) δ 7.69 (s, 1 H), 6.95-7.29 (m, 3 H), 6.57-6.81 (m, 1 H), 4.46-4.72 (m, 1 H), 3.52-4.09 (m, 1 H), 2.80-3.25 (m, 2 H), 2.51 (s, 3 H), 1.48-1.73 (m, 6 H) | 476.2 [M+H]⁺ | 99.3% |
| 724 | (CD₃OD) δ 7.69 (s, 1 H), 6.92-7.32 (m, 3 H), 6.75 (br s, 1 H), 4.60 (s, 1 H), 3.59-4.15 (m, 1 H), 2.79-3.23 (m, 2 H), 2.51 (s, 3 H), 1.48-1.75 (m, 6 H) | 476.2 [M+H]⁺ | 98.8% |
| 725 | (CD₃OD) δ 7.61-7.81 (m, 3 H), 7.52 (s, 1 H), 7.38-7.49 (m, 2 H), 6.93 (br s, 0.7 H), 6.59 (s, 1 H), 6.22 (br s, 0.3 H), 5.02 (br s, 0.2 H), 4.08-4.25 (m, 0.8 H), 3.92 (br s, 0.7 H), 3.47-3.74 (m, 1.3 H), 3.00 (br s, 1 H), 2.72-2.91 (m, 1 H), 0.80-1.19 (m, 4 H) | 375.2 [M+H]⁺ | 99.7% |
| 726 | (CD₃OD) δ 7.61-7.86 (m, 3 H), 7.53 (d, 1 H), 7.36-7.49 (m, 2 H), 6.93 (br s, 0.7 H), 6.59 (s, 1 H), 6.22 (br s, 0.3 H), 5.01 (br s, 0.3 H), 4.07-4.21 (m, 0.7 H), 3.94 (br d, 0.7 H), 3.46-3.75 (m, 1.3 H), 3.00 (br s, 1 H), 2.69-2.91 (m, 1 H), 0.78-1.17 (m, 4 H) | 375.2 [M+H]⁺ | 99.7% |
| 727 | (CD₃OD) δ 7.72 (s, 1 H), 7.49-7.61 (m, 2 H), 7.40 (td, 1 H), 7.21-7.31 (m, 1 H), 6.92 (br s, 0.7 H), 6.60 (d, 1 H), 6.14-6.42 (m, 0.3 H), 4.95-5.13 (m, 0.3 H), 4.16 (br s, 0.7 H), 3.92 (br s, 0.7 H), 3.44-3.79 (m, 1.3 H), 2.93-3.12 (m, 1 H), 2.83 (br s, 1 H), 1.09-1.22 (m, 1 H), 0.83-1.09 (m, 3 H) | 393.2 [M+H]⁺ | 99.2% |
| 728 | (CD₃OD) δ 7.72 (s, 1 H), 7.50-7.63 (m, 2 H), 7.40 (td, 1 H), 7.21-7.31 (m, 1 H), 6.91 (br s, 0.7 H), 6.60 (d, 1 H), 6.15-6.41 (m, 0.3 H), 5.01 (br s, 0.3 H), 4.17 (br d, 0.7 H), 3.92 (br s, 0.7 H), 3.42-3.80 (m, 1.3 H), 3.01 (br s, 1 H), 2.82 (br d, 1 H), 0.82-1.20 (m, 4 H) | 393.2 [M+H]⁺ | 99.2% |
| 729 | (CD₃OD) δ 7.75-7.96 (m, 3 H), 7.66-7.74 (m, 1 H), 7.56-7.64 (m, 1.6 H), 7.41-7.48 (m, 1 H), 7.27-7.36 (m, 1 H), 7.09-7.23 (m, 1 H), 6.94-7.01 (m, 1 H), 6.71-6.77 (m, 1 H), 6.02 (s, 0.4 H), 5.07 (dd, 0.3 H), 3.62-4.00 (m, 1.7 H), 2.66-3.16 (m, 2 H) | 430.2 [M+H]⁺ | 99.1% |
| 730 | (CD₃OD) δ 7.63-7.83 (m, 3 H), 7.53-7.61 (m, 1 H), 7.43-7.52 (m, 1.6 H), 7.29-7.37 (m, 1 H), 7.14-7.25 (m, 1 H), 6.97-7.10 (m, 1 H), 6.82-6.89 (m, 1 H), 6.58-6.65 (m, 1 H), 5.83-5.93 (m, 0.4 H), 4.95 (br dd, 0.3 H), 3.51-3.86 (m, 1.7 H), 2.54-3.04 (m, 2 H) | 430.2 [M+H]⁺ | 98.0% |
| 731 | (CD₃OD) δ 7.61-7.75 (m, 3 H), 7.34-7.47 (m, 2 H), 6.57-6.95 (m, 3 H), 4.29 (br s, 1 H), 3.96 (br s, 4 H), 2.94-3.11 (m, 1 H), 2.81 (br d, 1 H) | 399.2 [M+H]⁺ | 100% |
| 732 | (CD₃OD) δ 7.58-7.78 (m, 3 H), 7.29-7.51 (m, 2 H), 6.58-7.00 (m, 3 H), 4.31 (br d, 1 H), 3.96 (br s, 4 H), 3.03 (br t, 1 H), 2.81 (br d, 1 H) | 399.2 [M+H]⁺ | 99.0% |
| 733 | (CD₃OD) δ 7.70 (s, 1 H), 7.53 (br d, 1 H), 7.37 (td, 1 H), 7.15-7.29 (m, 1 H), 6.57-6.95 (m, 3 H), 4.30 (br s, 1 H), 3.97 (s, 4 H), 3.04 (br t, 1 H), 2.82 (br d, 1 H) | 417.2 [M+H]⁺ | 100% |
| 734 | (CD₃OD) δ 7.70 (s, 1 H), 7.53 (br d, 1 H), 7.38 (td, 1 H), 7.18-7.28 (m, 1 H), 6.60-6.95 (m, 3 H), 4.30 (br s, 1 H), 3.97 (s, 4 H), 2.94-3.14 (m, 1 H), 2.83 (br d, 1 H) | 417.2 [M+H]⁺ | 99.4% |
| 735 | (CD₃OD) δ 7.70 (s, 1 H), 7.49 (br d, 1 H), 7.16-7.34 (m, 2 H), 6.80-6.94 (m, 2 H), 6.61-6.79 (m, 1 H), 4.30 (br s, 1 H), 3.96 (br s, 4 H), 2.95-3.11 (m, 1 H), 2.82 (br d, 1 H), 2.56 (s, 3 H) | 413.2 [M+H]⁺ | 99.3% |
| 736 | (CD₃OD) δ 7.70 (s, 1 H), 7.49 (br d, 1 H), 7.17-7.32 (m, 2 H), 6.81-6.95 (m, 2 H), 6.62-6.80 (m, 1 H), 4.28 (br s, 1 H), 3.96 (br s, 4 H), 3.02 (br d, 1 H), 2.82 (br d, 1 H), 2.56 (s, 3 H) | 413.2 [M+H]⁺ | 92.8% |
| 737 | (CD₃OD) δ 8.47-8.84 (m, 1 H), 7.87-8.39 (m, 2 H), 7.60-7.77 (m, 2 H), 7.48-7.58 (m, 2 H), 7.06-7.42 (m, 2 H), 6.83 (br s, 1 H), 4.74 (br d, 1 H), 3.64-4.13 (m, 1 H), 3.15 (s, 1 H), 2.91 (br d, 1 H), 2.48 (s, 3 H) | 477.2 [M+H]⁺ | 99.3% |
| 738 | (CD₃OD) δ 8.47-8.85 (m, 1 H), 7.87-8.38 (m, 2 H), 7.58-7.76 (m, 2 H), 7.47-7.57 (m, 2 H), 7.07-7.37 (m, 2 H), 6.83 (br s, 1 H), 4.75 (br d, 1 H), 3.66-4.09 (m, 1 H), 3.15 (br s, 1 H), 2.91 (br d, 1 H), 2.48 (s, 3 H) | 477.2 [M+H]⁺ | 97.5% |
| 739 | (CD₃OD) δ 8.43-8.92 (m, 1 H), 7.88-8.40 (m, 2 H), 7.52-7.78 (m, 4 H), 7.05-7.50 (m, 3 H), 6.86 (br s, 1 H), 4.76 (br d, 1 H), 3.64-4.07 (m, 1 H), 2.99-3.31 (m, 1 H), 2.92 (br d, 1 H) | 463.2 [M+H]⁺ | 97.1% |
| 740 | (CD₃OD) δ 8.32-8.73 (m, 1 H), 7.74-8.25 (m, 2 H), 7.40-7.66 (m, 4 H), 6.93-7.39 (m, 3 H), 6.74 (br s, 1 H), 4.63 (br d, 1 H), 3.53-3.97 (m, 1 H), 2.89-3.18 (m, 1 H), 2.80 (br d, 1 H) | 463.2 [M+H]⁺ | 97.8% |
| 741 | (CD₃OD) δ 9.42-9.57 (m, 2 H), 9.36 (s, 1 H), 7.49-7.78 (m, 2 H), 7.41 (br s, 1.4 H), 7.17-7.32 (m, 1.6 H), 6.81-7.15 (m, 1 H), 4.94-5.04 (m, 0.5 H), 4.75 (br d, 0.5 H), 3.69-4.19 (m, 1 H), 2.78-3.29 (m, 2 H) | 482.2 [M+H]⁺ | 99.5% |
| 742 | (CD₃OD) δ 9.49 (br s, 2 H), 9.36 (s, 1 H), 7.50-7.79 (m, 2 H), 7.41 (br s, 1.4 H), 7.16-7.31 (m, 1.6 H), 6.77-7.15 (m, 1 H), 4.95-5.06 (m, 0.5 H), 4.74 (br d, 0.5 H), 3.72-4.15 (m, 1 H), 2.84-3.28 (m, 2 H) | 482.1 [M+H]⁺ | 99.3% |
| 743 | (CD₃OD) δ 8.45 (s, 1 H), 7.70 (br s, 1 H), 6.94-7.31 (m, 3 H), 6.76 (br s, 1 H), 4.58 (br d, 1 H), 3.98 (br s, 1 H), 2.77-3.19 (m, 2 H), 2.51 (br s, 3 H) | 418.2 [M+H]⁺ | 98.7% |
| 744 | (CD₃OD) δ 8.45 (s, 1 H), 7.69 (s, 1 H), 6.97-7.30 (m, 3 H), 6.76 (br s, 1 H), 4.59 (s, 1 H), 3.99 (br s, 1 H), 2.79-3.21 (m, 2 H), 2.51 (s, 3 H) | 418.1 [M+H]⁺ | 99.0% |
| 745 | (CD₃OD) δ 7.93-8.07 (m, 1 H), 7.71 (s, 1 H), 7.43-7.56 (m, 2 H), 7.16-7.29 (m, 1.3 H), 6.88 (s, 0.7 H), 5.09 (dd, 0.3 H), 4.71 (dd, 0.7 H), 4.04-4.11 (m, 3 H), 3.79-3.89 (m, 0.7 H), 3.48 (td, 0.3 H), 3.14-3.24 (m, 0.7 H), 2.93-3.05 (m, 0.3 H), 2.75-2.90 (m, 1 H), 2.43-2.50 (m, 3 H) | 364.2 [M+H]⁺ | 98.8% |
| 746 | (CD₃OD) δ 7.92-8.08 (m, 1 H), 7.71 (s, 1 H), 7.43-7.57 (m, 2 H), 7.16-7.31 (m, 1.3 H), 6.88 (s, 0.7 H), 5.09 (dd, 0.3 H), 4.71 (br dd, 0.7 H), 4.02-4.13 (m, 3 H), 3.78-3.92 (m, 0.7 H), 3.48 (td, 0.3 H), 3.15-3.25 (m, 0.7 H), 2.94-3.05 (m, 0.3 H), 2.75-2.90 (m, 1 H), 2.42-2.50 (m, 3 H) | 364.2 [M+H]⁺ | 98.0% |
| 747 | (CD₃OD) δ 7.66-7.75 (m, 1 H), 7.45-7.60 (m, 2 H), 7.18-7.32 (m, 2 H), 6.66 (s, 2 H), 4.23-4.45 (m, 1 H), 3.94 (br s, 4 H), 2.83 (br s, 2 H), 2.56 (br s, 3 H) | 363.2 [M+H]⁺ | 99.3% |
| 748 | (CD₃OD) δ 7.70 (s, 1 H), 7.44-7.59 (m, 2 H), 7.19-7.31 (m, 2 H), 6.66 (s, 2 H), 4.21-4.50 (m, 1 H), 3.94 (br s, 4 H), 2.75-3.11 (m, 2 H), 2.56 (br s, 3 H) | 363.2 [M+H]⁺ | 98.2% |
| 749 | (CD₃OD) δ 7.68 (s, 1 H), 7.55 (d, 1 H), 7.21-7.36 (m, 2 H), 7.01 (d, 1 H), 6.15-6.92 (m, 2 H), 4.17-4.46 (m, 1 H), 3.43-4.06 (m, 7 H), 2.77-3.05 (m, 2 H) | 379.2 [M+H]⁺ | 98.2% |
| 750 | (CD₃OD) δ 7.68 (s, 1 H), 7.54 (d, 1 H), 7.21-7.36 (m, 2 H), 7.01 (d, 1 H), 6.29-6.91 (m, 2 H), 4.24-4.41 (m, 1 H), 3.50-4.05 (m, 7 H), 2.77-3.05 (m, 2 H) | 379.2 [M+H]⁺ | 98.4% |
| 751 | (CD₃OD) δ 7.50-7.78 (m, 4 H), 7.29-7.48 (m, 2 H), 6.86 (br s, 1 H), 6.56 (s, 1 H), 4.48-4.85 (m, 2 H), 3.80-4.35 (m, 1 H), 2.70-3.07 (m, 2 H), 1.46 (br s, 6 H) | 377.2 [M+H] ⁺ | 99.6% |
| 752 | (CD₃OD) δ 7.53-7.73 (m, 4 H), 7.33-7.48 (m, 2 H), 6.86 (br s, 1 H), 6.56 (s, 1 H), 4.40-4.86 (m, 2 H), 4.22 (br d, 1 H), 3.88 (br s, 1 H), 3.63-4.04 (m, 1 H), 2.69-3.05 (m, 2 H), 1.46 (br s, 6 H) | 377.2 [M+H]⁺ | 99.3% |
| 753 | (CD₃OD) δ 7.66 (dd, 2 H), 7.43 (br s, 1 H), 6.53-7.26 (m, 3 H), 4.93-5.02 (m, 1 H), 4.52 (br s, 1 H), 3.59-4.03 (m, 1 H), 2.81-3.18 (m, 2 H), 1.23-1.50 (m, 9 H) | 460.2 [M+H]⁺ | 98.8% |
| 754 | (CD₃OD) δ 7.66 (dt, 2 H), 7.43 (br s, 1 H), 6.55-7.29 (m, 3 H), 4.91-4.94 (m, 1 H), 4.53 (br s, 1 H), 3.59-4.02 (m, 1 H), 2.74-3.18 (m, 2 H), 1.24-1.53 (m, 9 H) | 460.2 [M+H]⁺ | 85.0% |
| 755 | (CD₃OD) δ 7.63-7.81 (m, 2 H), 7.46-7.52 (m, 2 H), 7.04-7.32 (m, 2 H), 6.68-7.02 (m, 2 H), 4.91-5.03 (m, 0.5 H), 4.70 (m, 0.5 H), 3.59-4.04 (m, 4 H), 2.83-3.24 (m, 2 H), 2.45 (s, 3 H) | 480.2 [M+H]⁺ | 98.5% |
| 756 | (CD₃OD) δ 7.62-7.82 (m, 2 H), 7.46-7.53 (m, 2 H), 7.05-7.32 (m, 2 H), 6.66-7.04 (m, 2 H), 4.96 (br s, 0.5 H), 4.70 (m, 0.5 H), 3.65-4.06 (m, 4 H), 2.83-3.25 (m, 2 H), 2.45 (s, 3 H) | 480.2 [M+H]+ | 98.2% |
| 757 | (CD₃OD) δ 7.37-8.12 (m, 5 H), 6.73-7.36 (m, 3 H), 4.72 (br s, 1 H), 3.60-4.16 (m, 4 H), 2.86-3.24 (m, 2 H), 2.40-2.51 (m, 3 H) | 480.2 [M+H]⁺ | 99.3% |
| 758 | (CD₃OD) δ 7.37-8.05 (m, 5 H), 6.64-7.36 (m, 3 H), 4.70 (br s, 1 H), 3.64-4.15 (m, 4 H), 2.82-3.25 (m, 2 H), 2.39-2.54 (m, 3 H) | 480.2 [M+H]⁺ | 98.8% |
| 759 | (CD₃OD) δ 7.86-8.45 (m, 2 H), 7.63-7.83 (m, 3 H), 6.99-7.55 (m, 3 H), 6.81 (s, 1 H), 4.73 (br s, 1 H), 3.99 (br s, 4 H), 2.79-3.28 (m, 2 H) | 466.2 [M+H]⁺ | 98.5% |
| 760 | (CD₃OD) δ 7.88-8.41 (m, 2 H), 7.64-7.74 (m, 3 H), 6.99-7.48 (m, 3 H), 6.80 (s, 1 H), 4.71 (br s, 1 H), 3.97 (br d, 4 H), 2.82-3.26 (m, 2 H) | 466.2 [M+H]⁺ | 98.8% |
| 761 | (CD₃OD) δ 7.63-7.81 (m, 4 H), 7.36-7.45 (m, 2 H), 7.04-7.32 (m, 1 H), 6.68-7.03 (m, 2 H), 4.97 (br s, 0.5 H), 4.71 (m, 0.5 H), 3.62-4.08 (m, 4 H), 2.79-3.28 (m, 2 H) | 466.2 [M+H]⁺ | 99.0% |
| 762 | (CD₃OD) δ 7.59-7.80 (m, 4 H), 7.37-7.45 (m, 2 H), 7.05-7.32 (m, 1 H), 6.67-7.01 (m, 2 H), 4.97 (br s, 0.5 H), 4.73 (br s, 0.5 H), 3.66-4.08 (m, 4 H), 2.79-3.29 (m, 2 H) | 466.2 [M+H]+ | 97.4% |
| 763 | (CD₃OD) δ 7.59-7.96 (m, 5 H), 7.42 (br t, 2 H), 6.98-7.35 (m, 1 H), 6.78 (br s, 1 H), 4.67 (br s, 1 H), 3.88-4.18 (m, 3.7 H), 3.71 (br s, 0.3 H), 2.86-3.25 (m, 2 H) | 466.2 [M+H]⁺ | 98.4% |
| 764 | (CD₃OD) δ 7.59-7.99 (m, 5 H), 7.42 (br t, 2 H), 7.01-7.36 (m, 1 H), 6.78 (br s, 1 H), 4.68 (br s, 1 H), 3.89-4.19 (m, 3.7 H), 3.75 (br s, 0.3 H), 2.78-3.24 (m, 2 H) | 466.2 [M+H]⁺ | 98.3% |
| 765 | (CD₃OD) δ 7.68 (s, 1 H), 7.58 (d, 1 H), 7.28 (dt, 2 H), 7.00 (dd, 1 H), 6.88 (br s, 1 H), 6.55 (d, 1 H), 4.75 (br s, 2 H), 4.18 (br d, 0.5 H), 4.01 (s, 3 H), 3.82 (br s, 0.5 H), 2.74-3.05 (m, 2 H), 1.46 (br s, 6 H) | 407.2 [M+H]⁺ | 99.8% |
| 766 | (CD₃OD) δ 7.68 (s, 1 H), 7.58 (d, 1 H), 7.16-7.38 (m, 2 H), 7.01 (d, 1 H), 6.88 (br s, 1 H), 6.55 (d, 1 H), 4.75 (br s, 2 H), 4.19 (br s, 0.5 H), 4.01 (s, 3 H), 3.82 (br s, 0.5 H), 2.70-3.10 (m, 2 H), 1.46 (br s, 6 H) | 407.2 [M+H]⁺ | 99.7% |
| 767 | (CD₃OD) δ 7.71 (s, 1 H), 7.53 (d, 1 H), 7.22-7.40 (m, 2 H), 7.03 (d, 1 H), 6.94 (br s, 0.7 H), 6.58 (br s, 1 H), 6.24 (br s, 0.3 H), 4.96-5.05 (m, 0.3 H), 4.12 (br d, 0.7 H), 4.02 (s, 3 H), 3.81-3.95 (m, 0.7 H), 3.69 (br s, 1 H), 3.50 (br s, 0.3 H), 2.99 (br s, 1 H), 2.72-2.92 (m, 1 H), 0.80-1.20 (m, 4 H) | 405.2 [M+H]⁺ | 99.7% |
| 768 | (CD₃OD) δ 7.71 (s, 1 H), 7.53 (d, 1 H), 7.23-7.41 (m, 2 H), 7.03 (d, 1 H), 6.94 (br s, 0.7 H), 6.58 (br s, 1 H), 6.24 (br s, 0.3 H), 4.96-5.10 (m, 0.4 H), 4.12 (br d, 0.6 H), 4.02 (s, 3 H), 3.88 (br s, 0.7 H), 3.69 (br s, 1 H), 3.50 (br s, 0.3 H), 2.99 (br s, 1 H), 2.69-2.92 (m, 1 H), 0.80-1.18 (m, 4 H) | 405.2 [M+H]⁺ | 99.7% |
| 769 | (CD₃OD) δ 7.83 (s, 1 H), 7.54 (br d, 1 H), 7.40 (td, 1 H), 7.10-7.31 (m, 1 H), 6.89 (br s, 1 H), 6.46 (s, 1 H), 4.38 (br s, 1 H), 3.88 (s, 4 H), 2.80-3.12 (m, 2 H), 2.29 (s, 3 H) | 381.2 [M+H]⁺ | 89.4% |
| 770 | (CD₃OD) δ 7.63 (s, 1 H), 7.42 (br d, 1 H), 7.27 (td, 1 H), 7.13 (t, 1 H), 6.74 (br s, 1 H), 6.33 (s, 1 H), 4.25 (br s, 1 H), 3.75 (s, 4 H), 2.61-3.04 (m, 2 H), 2.17 (s, 3 H) | 381.2 [M+H]⁺ | 95.7% |
| 771 | (CD₃OD) δ 8.27-8.48 (m, 0.5 H), 7.86-8.24 (m, 1.5 H), 7.45-7.82 (m, 3 H), 7.01-7.37 (m, 2 H), 6.78 (br s, 1 H), 4.72 (br s, 1 H), 3.61-4.14 (m, 4 H), 2.79-3.27 (m, 2 H), 2.41-2.54 (m, 3 H) | 480.2 [M+H]⁺ | 99.9% |
| 772 | (CD₃OD) δ 8.38 (br s, 0.5 H), 7.83-8.23 (m, 1.5 H), 7.43-7.77 (m, 3 H), 6.99-7.38 (m, 2 H), 6.78 (s, 1 H), 4.60-4.85 (m, 1 H), 4.00 (br s, 4 H), 2.81-3.28 (m, 2 H), 2.48 (s, 3 H) | 480.2 [M+H]⁺ | 99.4% |
| 773 | (CD₃OD) δ 9.38 (br s, 0.7 H), 8.91-9.17 (m, 1.3 H), 8.17-8.38 (m, 1 H), 7.64-7.84 (m, 1 H), 7.48-7.62 (m, 1 H), 7.05-7.47 (m, 3 H), 6.86 (br s, 1 H), 5.03 (br s, 0.2 H), 4.75 (br d, 0.8 H), 4.05 (br s, 0.6 H), 3.80 (br d, 0.4 H), 3.22-3.31 (m, 0.6 H), 3.05 (br s, 0.4 H), 2.94 (br d, 1 H) | 482.2 [M+H]⁺ | 99.6% |
| 774 | (CD₃OD) δ 9.26 (br s, 0.7 H), 8.78-9.06 (m, 1.3 H), 8.05-8.28 (m, 1 H), 7.61 (br s, 1 H), 7.36-7.50 (m, 1 H), 6.92-7.35 (m, 3 H), 6.74 (br s, 1 H), 4.89 (br d, 0.2 H), 4.63 (br d, 0.8 H), 3.92 (br s, 0.6 H), 3.67 (br s, 0.4 H), 3.07-3.19 (m, 0.6 H), 2.92 (br s, 0.4 H), 2.82 (br d, 1 H) | 482.2 [M+H]⁺ | 98.5% |
| 775 | (CD₃OD) δ 7.96-8.03 (m, 1 H), 7.67-7.73 (m, 1 H), 7.20-7.33 (m, 2 H), 6.97-7.02 (m, 1 H), 5.05 (br dd, 0.4 H), 4.68 (dd, 0.6 H), 3.92-4.14 (m, 6 H), 3.79-3.88 (m, 0.6 H), 3.36-3.45 (m, 0.4 H), 2.74-3.23 (m, 3 H) | 380.2 [M+H]⁺ | 97.0% |
| 776 | (CD₃OD) δ 7.92-8.07 (m, 1 H), 7.65-7.79 (m, 1 H), 7.19-7.34 (m, 2 H), 6.94-7.04 (m, 1 H), 5.05 (dd, 0.4 H), 4.68 (dd, 0.6 H), 3.96-4.10 (m, 6 H), 3.75-3.90 (m, 0.6 H), 3.35-3.49 (m, 0.4 H), 2.73-3.25 (m, 3 H) | 380.1 [M+H]⁺ | 94.7% |
| 777 | (CD₃OD) δ 7.68 (s, 1 H), 7.20-7.34 (m, 2 H), 7.00 (d, 1 H), 6.81-6.94 (m, 2 H), 6.60-6.79 (m, 0.8 H), 6.39 (br s, 0.2 H), 4.18-4.37 (m, 1 H), 3.98 (d, 7 H), 2.95-3.09 (m, 1 H), 2.72-2.87 (m, 1 H) | 429.2 [M+H]⁺ | 99.5% |
| 778 | (CD₃OD) δ 7.69 (s, 1 H), 7.21-7.34 (m, 2 H), 7.00 (d, 1 H), 6.80-6.94 (m, 2 H), 6.59-6.79 (m, 0.8 H), 6.39 (br s, 0.2 H), 4.27 (br s, 1 H), 3.98 (d, 7 H), 2.92-3.10 (m, 1 H), 2.81 (br d, 1 H) | 429.2 [M+H]⁺ | 98.3% |
| 779 | (CD₃OD) δ 7.61-7.79 (m, 3 H), 7.36-7.53 (m, 2 H), 6.85 (br s, 1 H), 6.44 (s, 1 H), 4.25-4.69 (m, 1.5 H), 3.87 (br s, 3.8 H), 2.78-3.12 (m, 2 H), 2.29 (br s, 3 H) | 363.2 [M+H]⁺ | 99.3% |
| 780 | (CD₃OD) δ 7.64-7.79 (m, 3 H), 7.34-7.51 (m, 2 H), 6.85 (br s, 1 H), 6.44 (s, 1 H), 4.27-4.69 (m, 1.3 H), 3.87 (br s, 3.7 H), 2.73-3.13 (m, 2 H), 2.29 (br s, 3 H) | 363.2 [M+H]⁺ | 98.2% |
| 781 | (CD₃OD) δ 7.60 (s, 1 H), 7.38 (br d, 1 H), 6.95-7.24 (m, 2 H), 6.74 (br s, 1 H), 6.33 (s, 1 H), 4.16-4.60 (m, 1 H), 3.63-3.93 (m, 4 H), 2.62-2.99 (m, 2 H), 2.45 (s, 3 H), 2.17 (s, 3 H) | 377.2 [M+H]⁺ | 97.8% |
| 782 | (CD₃OD) δ 7.72 (s, 1 H), 7.50 (br d, 1 H), 7.15-7.35 (m, 2 H), 6.86 (br s, 1 H), 6.45 (s, 1.1 H), 4.37 (br d, 1 H), 3.87 (br s, 4 H), 2.77-3.10 (m, 2 H), 2.57 (s, 3 H), 2.29 (s, 3 H) | 377.2 [M+H]⁺ | 96.4% |
| 783 | (CD₃OD) δ 7.75-8.02 (m, 1 H), 7.69 (s, 1 H), 7.43-7.55 (m, 2 H), 7.00-7.32 (m, 2 H), 6.79 (br s, 1 H), 4.77 (br s, 2 H), 3.64-4.08 (m, 4 H), 2.78-3.28 (m, 2 H), 2.45 (s, 3 H) | 480.2 [M+H]+ | 95.8% |
| 784 | (CD₃OD) δ 7.78-8.02 (m, 1 H), 7.69 (s, 1 H), 7.44-7.54 (m, 2 H), 7.02-7.33 (m, 2 H), 6.79 (s, 1 H), 4.76 (br s, 2 H), 3.58-4.07 (m, 4 H), 2.80-3.27 (m, 2 H), 2.45 (s, 3 H) | 480.2 [M+H]⁺ | 97.9% |
| 785 | (CD₃OD) δ 7.77-8.02 (m, 1 H), 7.63-7.75 (m, 3 H), 7.36-7.46 (m, 2 H), 6.98-7.34 (m, 1 H), 6.82 (s, 1 H), 4.78 (br s, 2 H), 3.64-4.09 (m, 4 H), 2.81-3.29 (m, 2 H) | 466.2 [M+H]⁺ | 99.1% |
| 786 | (CD₃OD) δ 7.79-8.01 (m, 1 H), 7.59-7.75 (m, 3 H), 7.41 (qd, 2 H), 7.01-7.33 (m, 1 H), 6.81 (br s, 1 H), 4.78 (br s, 2 H), 3.70-4.09 (m, 4 H), 2.78-3.28 (m, 2 H) | 466.2 [M+H]⁺ | 98.3% |
| 787 | (CD₃OD) δ 7.55-7.82 (m, 3 H), 7.42 (dd, 1 H), 7.20 (td, 1 H), 6.67-6.99 (m, 1.7 H), 6.30 (br s, 0.3 H), 4.98 (br d, 0.4 H), 4.20 (br dd, 0.6 H), 3.79-4.05 (m, 3.7 H), 3.63-3.74 (m, 0.3 H), 2.77-3.16 (m, 2 H) | 502.2 [M+H]⁺ | 99.1% |
| 788 | (CD₃OD) δ 7.56-7.81 (m, 3 H), 7.42 (dd, 1 H), 7.20 (td, 1 H), 6.68-7.02 (m, 1.7 H), 6.29 (br s, 0.3 H), 4.99 (br d, 0.3 H), 4.20 (br dd, 0.7 H), 3.79-4.06 (m, 3.8 H), 3.60-3.73 (m, 0.2 H), 2.78-3.14 (m, 2 H) | 502.2 [M+H]⁺ | 99.2% |
| 789 | (CD₃OD) δ 7.97 (s, 1 H), 7.81 (s, 1 H), 7.56-7.77 (m, 2 H), 7.43 (dd, 1 H), 7.22 (td, 1 H), 6.82 (br s, 0.6 H), 6.26 (br s, 0.1 H), 4.19 (br dd, 1 H), 3.95 (br t, 0.8 H), 3.71-3.87 (m, 3 H), 3.55-3.69 (m, 0.2 H), 2.79-3.17 (m, 2 H) | 502.2 [M+H]⁺ | 98.7% |
| 790 | (CD₃OD) δ 7.97 (s, 1 H), 7.80 (s, 1 H), 7.56-7.76 (m, 2 H), 7.43 (dd, 1 H), 7.22 (td, 1 H), 6.82 (s, 0.6 H), 6.27 (br s, 0.1 H), 4.19 (br dd, 1 H), 3.95 (br t, 0.8 H), 3.72-3.86 (m, 3 H), 3.64 (br s, 0.2 H), 2.80-3.17 (m, 2 H) | 502.2 [M+H]⁺ | 99.2% |
| 791 | (CD₃OD) δ 7.78-7.99 (m, 2 H), 7.59-7.75 (m, 2 H), 7.39-7.52 (m, 1 H), 7.14-7.29 (m, 1 H), 6.27-6.90 (m, 1 H), 4.24 (br dd, 0.6 H), 3.90-4.12 (m, 4 H), 3.71 (br d, 0.4 H), 2.94-3.11 (m, 1 H), 2.79-2.91 (m, 1 H) | 502.2 [M+H]⁺ | 100% |
| 792 | (CD₃OD) δ 7.78-7.97 (m, 2 H), 7.59-7.75 (m, 2 H), 7.38-7.51 (m, 1 H), 7.16-7.28 (m, 1 H), 6.31-6.85 (m, 1 H), 4.24 (br dd, 0.6 H), 3.89-4.13 (m, 4 H), 3.69 (br s, 0.4 H), 2.94-3.12 (m, 1 H), 2.78-2.92 (m, 1 H) | 502.2 [M+H]⁺ | 99.4% |
| 793 | (CD₃OD) δ 8.25 (s, 1 H), 7.58-7.75 (m, 3 H), 7.27-7.51 (m, 2 H), 6.73 (br s, 1 H), 4.20-4.59 (m, 1 H), 3.88 (br s, 1 H), 2.77-3.16 (m, 2 H), 2.39 (s, 3 H) | 350.2 [M+H]⁺ | 100% |
| 794 | (CD₃OD) δ 8.27 (s, 1 H), 7.58-7.76 (m, 3 H), 7.30-7.51 (m, 2 H), 6.75 (br s, 1 H), 4.49-4.60 (m, 1 H), 3.75-4.02 (m, 1 H), 2.80-3.18 (m, 2 H), 2.41 (s, 3 H) | 350.1 [M+H]⁺ | 99.5% |
| 795 | (CD₃OD) δ 8.52 (s, 1 H), 7.74 (s, 1 H), 7.10-7.31 (m, 2 H), 6.86 (s, 1 H), 4.97-5.06 (m, 0.2 H), 4.05-4.14 (m, 0.8 H), 3.92-4.04 (m, 0.9 H), 3.64 (br s, 0.1 H), 2.83-3.11 (m, 2 H) | 440.1 [M+H]⁺ | 98.0% |
| 796 | (CD₃OD) δ 8.27-8.77 (m, 1 H), 7.79 (s, 1 H), 7.06-7.37 (m, 2 H), 6.13-7.02 (m, 1 H), 4.99 (br s, 0.3 H), 4.05-4.16 (m, 0.8 H), 3.89-4.03 (m, 0.8 H), 3.63 (br s, 0.2 H), 2.82-3.07 (m, 2 H) | 440.2 [M+H]⁺ | 96.2% |
| 797 | (CD₃OD) δ 8.41-8.62 (m, 1 H), 7.60-7.79 (m, 3 H), 7.36-7.51 (m, 2 H), 6.87 (br s, 1 H), 4.99 (br d, 0.3 H), 3.85-4.10 (m, 1.5 H), 2.70-3.12 (m, 2 H) | 404.1 [M+H]⁺ | 99.3% |
| 798 | (CD₃OD) δ 8.50 (s, 1 H), 7.58-7.84 (m, 3 H), 7.30-7.51 (m, 2 H), 6.87 (br s, 1 H), 4.92-5.06 (m, 0.3 H), 3.82-4.15 (m, 1.7 H), 2.77-3.08 (m, 2 H) | 404.1 [M+H]⁺ | 98.7% |
| 799 | (CD₃OD) δ 8.55-8.74 (m, 1 H), 7.89-8.00 (m, 1 H), 7.83-7.89 (m, 1 H), 7.73-7.81 (m, 1 H), 7.56 (s, 0.3 H), 7.21-7.31 (m, 1 H), 7.12-7.20 (m, 1 H), 6.99 (s, 0.7 H), 5.13-5.34 (m, 1 H), 3.67-4.09 (m, 1 H), 2.86-3.29 (m, 2 H) | 468.2 [M+H]⁺ | 99.4% |
| 800 | (CD₃OD) δ 8.76-8.80 (m, 1 H), 8.63-8.71 (m, 1 H), 7.91-8.02 (m, 1 H), 7.74-7.83 (m, 1 H), 7.25-7.36 (m, 2 H), 7.15-7.25 (m, 1 H), 5.13-5.48 (m, 1 H), 3.56-3.95 (m, 1 H), 3.04-3.17 (m, 1 H), 2.89-3.04 (m, 1 H) | 468.2 [M+H]⁺ | 97.6% |
| 801 | (CD₃OD) δ 8.93 (d, 1 H), 8.34-8.39 (m, 1 H), 7.63-7.86 (m, 2 H), 7.44-7.61 (m, 0.5 H), 7.11-7.29 (m, 2 H), 6.92 (br s, 0.5 H), 5.08-5.43 (m, 1 H), 3.64-4.13 (m, 1 H), 2.86-3.28 (m, 2 H) | 489.1 [M+H]⁺ | 96.0% |
| 802 | (CD₃OD) δ 8.91-8.96 (m, 1 H), 8.34-8.39 (m, 1 H), 7.80 (t, 1 H), 7.46-7.70 (m, 1 H), 7.12-7.29 (m, 2 H), 6.95 (s, 1 H), 5.07-5.42 (m, 1 H), 3.66-4.10 (m, 1 H), 2.84-3.28 (m, 2 H) | 489.2 [M+H]⁺ | 96.5% |
| 803 | (CD₃OD) δ 8.47 (br s, 1 H), 7.71 (s, 1 H), 7.54 (d, 1 H), 7.39 (td, 1 H), 6.65-7.31 (m, 3 H), 4.50 (br d, 1 H), 3.38-3.77 (m, 1 H), 3.23 (br s, 1 H), 1.36 (d, 3 H) | 418.1 [M+H]⁺ | 96.7% |
| 804 | (CD₃OD) δ 8.49 (br s, 1 H), 7.70 (s, 1 H), 7.52 (d, 1 H), 7.38 (td, 1 H), 6.63-7.30 (m, 3 H), 4.73-4.84 (m, 0.3 H), 4.40 (br d, 0.7 H), 3.62-4.24 (m, 1 H), 3.19 (br s, 1 H), 1.36 (br d, 3 H) | 418.2 [M+H]⁺ | 100% |
| 805 | (CD₃OD) δ 8.49 (br s, 1 H), 7.70 (s, 1 H), 7.52 (d, 1 H), 7.38 (td, 1 H), 6.78-7.29 (m, 3 H), 4.79-4.85 (m, 0.3 H), 4.41 (br d, 0.7 H), 3.67-4.21 (m, 1 H), 3.20 (br s, 1 H), 1.36 (br d, 3 H) | 418.2 [M+H]⁺ | 99.0% |
| 806 | (CD₃OD) δ 8.47 (br s, 1 H), 7.71 (s, 1 H), 7.54 (d, 1 H), 7.39 (td, 1 H), 6.76-7.30 (m, 3 H), 4.50 (br d, 1 H), 3.57 (br s, 1 H), 3.24 (br s, 1 H), 1.36 (d, 3 H) | 418.2 [M+H]⁺ | 97.2% |
| 807 | (CD₃OD) δ 8.28 (s, 1 H), 7.70 (s, 1 H), 7.04-7.37 (m, 2 H), 6.71 (br s, 1 H), 4.61 (br s, 1 H), 3.98 (br s, 1 H), 2.79-3.22 (m, 2 H), 2.42 (s, 3 H) | 386.2 [M+H]⁺ | 99.7% |
| 808 | (CD₃OD) δ 8.28 (s, 1 H), 7.69 (s, 1 H), 7.09-7.31 (m, 2 H), 6.71 (br s, 1 H), 4.61 (br s, 1 H), 3.97 (br s, 1 H), 2.76-3.19 (m, 2 H), 2.42 (s, 3 H) | 386.2 [M+H]⁺ | 99.3% |
| 809 | (CD₃OD) δ 7.67-7.76 (m, 1 H), 7.46 (br s, 0.3 H), 7.21-7.30 (m, 1 H), 7.09-7.20 (m, 1 H), 6.91 (br s, 0.7 H), 5.05-5.32 (m, 1 H), 3.65-4.07 (m, 1 H), 2.84-3.27 (m, 2 H), 1.81-1.97 (m, 6 H) | 433.2 [M+H]⁺ | 95.6% |
| 810 | (CD₃OD) δ 7.64-7.79 (m, 1 H), 7.46 (br s, 0.3 H), 7.21-7.31 (m, 1 H), 7.08-7.19 (m, 1 H), 6.91 (br s, 0.7 H), 5.08-5.35 (m, 1 H), 3.63-4.06 (m, 1 H), 2.85-3.27 (m, 2 H), 1.80-1.96 (m, 6 H) | 433.2 [M+H]⁺ | 91.0% |
| 811 | (CD₃OD) δ 8.42-8.54 (m, 1 H), 8.04-8.22 (m, 1 H), 7.70-7.80 (m, 1 H), 7.53-7.64 (m, 0.3 H), 7.10-7.31 (m, 2 H), 6.93 (s, 0.7 H), 5.08-5.44 (m, 1 H), 4.94-5.04 (m, 1 H), 3.65-4.07 (m, 1 H), 2.85-3.28 (m, 2 H), 2.44-2.71 (m, 4 H), 1.88-2.02 (m, 2 H) | 493.2 [M+H]⁺ | 100% |
| 812 | (CD₃OD) δ 8.45-8.53 (m, 1 H), 8.09-8.21 (m, 1 H), 7.69-7.76 (m, 1 H), 7.59 (s, 0.3 H), 7.08-7.30 (m, 2 H), 6.92 (s, 0.7 H), 5.10-5.43 (m, 1 H), 4.93-5.03 (m, 1 H), 3.67-4.09 (m, 1 H), 2.86-3.28 (m, 2 H), 2.45-2.71 (m, 4 H), 1.86-2.01 (m, 2 H) | 493.2 [M+H]⁺ | 96.5% |
| 813 | (CD₃OD) δ 7.80-7.86 (m, 2 H), 7.60 (s, 0.2 H), 7.12-7.33 (m, 2 H), 6.91-7.07 (m, 1.8 H), 5.10-5.43 (m, 1 H), 3.97-4.06 (m, 3.7 H), 3.72 (td, 0.3 H), 2.77-3.30 (m, 2 H) | 453.2 [M+H]⁺ | 93.0% |
| 814 | (CD₃OD) δ 7.80-7.87 (m, 2 H), 7.61 (s, 0.2 H), 7.09-7.32 (m, 2 H), 6.87-7.05 (m, 1.8 H), 5.08-5.41 (m, 1 H), 3.93-4.09 (m, 3.8 H), 3.65-3.77 (m, 0.2 H), 2.82-3.28 (m, 2 H) | 453.2 [M+H]⁺ | 86.1% |
| 815 | (CD₃OD) δ 8.30-8.38 (m, 1 H), 7.48-7.89 (m, 2 H), 7.08-7.33 (m, 3 H), 6.93 (br s, 1 H), 5.10-5.40 (m, 1 H), 3.65-4.15 (m, 1 H), 2.83-3.29 (m, 2 H) | 489.2 [M+H]⁺ | 100% |
| 816 | (CD₃OD) δ 8.31-8.38 (m, 1 H), 7.49-7.87 (m, 2 H), 7.08-7.34 (m, 3 H), 6.94 (s, 1 H), 5.10-5.40 (m, 1 H), 3.68-4.15 (m, 1 H), 2.85-3.29 (m, 2 H) | 489.2 [M+H]⁺ | 99.3% |
| 817 | (CD₃OD) δ 7.87 (d, 1 H), 7.71-7.77 (m, 1 H), 7.55-7.65 (m, 1 H), 7.41 (br s, 0.3 H), 7.08-7.19 (m, 1 H), 6.96-7.08 (m, 1 H), 6.79 (br s, 0.7 H), 4.97-5.30 (m, 1 H), 3.82-4.06 (m, 4 H), 2.75-3.16 (m, 2 H) | 453.2 [M+H]⁺ | 97.3% |
| 818 | (CD₃OD) δ 7.84-7.89 (m, 1 H), 7.70-7.78 (m, 1 H), 7.55-7.66 (m, 1 H), 7.43 (br s, 0.3 H), 7.09-7.19 (m, 1 H), 6.96-7.07 (m, 1 H), 6.80 (br s, 0.7 H), 4.94-5.34 (m, 1 H), 3.57-4.05 (m, 4 H), 2.73-3.16 (m, 2 H) | 453.2 [M+H]⁺ | 95.5% |
| 819 | (CD₃OD) δ 7.68-7.76 (m, 1 H), 7.37-7.51 (m, 1 H), 7.25-7.35 (m, 1 H), 6.82-7.21 (m, 2 H), 5.07 (br dd, 0.4 H), 4.62-4.72 (m, 0.6 H), 3.46-4.05 (m, 4 H), 2.93-3.24 (m, 1 H), 2.76-2.92 (m, 1 H), 2.51-2.63 (m, 3 H), 2.31-2.43 (m, 3 H) | 378.2 [M+H]⁺ | 99.9% |
| 820 | (CD₃OD) δ 7.71 (br d, 1 H), 7.37-7.48 (m, 1 H), 7.25-7.35 (m, 1 H), 6.87-7.22 (m, 2 H), 5.07 (br dd, 0.4 H), 4.65 (dd, 0.6 H), 3.46-4.01 (m, 4 H), 2.94-3.25 (m, 1 H), 2.77-2.94 (m, 1 H), 2.53-2.59 (m, 3 H), 2.31-2.43 (m, 3 H) | 378.2 [M+H]⁺ | 99.7% |
| 821 | (CD₃OD) δ 7.68 (s, 1 H), 7.15-7.40 (m, 2 H), 7.01 (d, 1 H), 6.84 (br s, 1 H), 6.42 (s, 1 H), 4.33 (br s, 0.6 H), 4.01 (s, 3 H), 3.87-3.93 (m, 0.4 H), 3.85 (s, 3 H), 2.70-3.09 (m, 2 H), 2.27 (s, 3 H) | 393.2 [M+H]⁺ | 98.2% |
| 822 | (CD₃OD) δ 7.70 (s, 1 H), 7.18-7.40 (m, 2 H), 7.03 (br d, 1 H), 6.86 (br s, 1 H), 6.44 (s, 1 H), 4.34 (br s, 0.6 H), 4.03 (s, 3 H), 3.89-3.97 (m, 0.4 H), 3.87 (s, 3 H), 2.78-3.10 (m, 2 H), 2.29 (s, 3 H) | 393.2 [M+H]⁺ | 96.7% |
| 823 | (CD₃OD) δ 7.60-7.79 (m, 2 H), 7.45 (br s, 1 H), 6.91-7.34 (m, 2 H), 6.75 (br s, 1 H), 4.61 (br s, 1 H), 3.56-4.00 (m, 3 H), 2.98-3.28 (m, 1 H), 2.89 (br d, 1 H), 1.11-1.55 (m, 6 H) | 476.2 [M+H]⁺ | 95.3% |
| 824 | (CD₃OD) δ 7.65-7.84 (m, 2 H), 7.44 (br d, 1 H), 6.95-7.34 (m, 2 H), 6.76 (br s, 1 H), 4.61 (br s, 1 H), 3.58-4.03 (m, 3 H), 2.99-3.25 (m, 1 H), 2.90 (br d, 1 H), 1.18-1.56 (m, 6 H) | 476.2 [M+H]⁺ | 93.8% |
| 825 | (CD₃OD) δ 7.60-7.78 (m, 3 H), 7.43 (dd, 1 H), 7.20 (td, 1 H), 6.54-6.99 (m, 2 H), 4.52-4.77 (m, 1 H), 3.66-4.07 (m, 4 H), 2.82-3.25 (m, 2 H), 2.44 (s, 3 H) | 448.2 [M+H]⁺ | 95.2% |
| 826 | (CD₃OD) δ 7.64-7.76 (m, 3 H), 7.42 (dd, 1 H), 7.20 (td, 1 H), 6.58-6.94 (m, 2 H), 4.60 (s, 1 H), 3.79-4.09 (m, 4 H), 2.83-3.24 (m, 2 H), 2.44 (s, 3 H) | 448.2 [M+H]⁺ | 96.1% |
| 827 | (CD₃OD) δ 7.66-7.75 (m, 1 H), 6.82-7.40 (m, 4 H), 5.05 (br dd, 0.2 H), 4.66 (br dd, 0.8 H), 3.94-4.03 (m, 6 H), 3.40-3.93 (m, 1 H), 2.92-3.24 (m, 1 H), 2.71-2.91 (m, 1 H), 2.30-2.42 (m, 3 H) | 394.2 [M+H]⁺ | 100% |
| 828 | (CD₃OD) δ 7.66-7.75 (m, 1 H), 6.79-7.41 (m, 4 H), 5.05 (br dd, 0.2 H), 4.66 (dd, 0.8 H), 3.95-4.03 (m, 6 H), 3.39-3.92 (m, 1 H), 2.91-3.24 (m, 1 H), 2.74-2.91 (m, 1 H), 2.29-2.44 (m, 3 H) | 394.2 [M+H]⁺ | 99.7% |
| 829 | (CD₃OD) δ 8.34-8.44 (m, 1 H), 8.05-8.14 (m, 1 H), 7.91-8.01 (m, 1 H), 7.67-7.75 (m, 2 H), 7.50-7.61 (m, 1 H), 6.96 (s, 1 H), 5.33 (dd, 0.6 H), 5.10 (dd, 0.4 H), 3.95-4.06 (m, 3.7 H), 3.61 (td, 0.3 H), 3.11-3.27 (m, 0.8 H), 2.83-3.07 (m, 1.2 H) | 485.2 [M+H]⁺ | 98.8% |
| 830 | (CD₃OD) δ 8.35-8.43 (m, 1 H), 8.04-8.13 (m, 1 H), 7.92-8.01 (m, 1 H), 7.66-7.74 (m, 2 H), 7.50-7.60 (m, 1 H), 6.95 (s, 1 H), 5.33 (dd, 0.6 H), 5.10 (dd, 0.4 H), 3.96-4.06 (m, 3.7 H), 3.53-3.69 (m, 0.3 H), 3.14-3.26 (m, 0.8 H), 2.80-3.08 (m, 1.2 H) | 485.2 [M+H]⁺ | 99.0% |
| 831 | (CD₃OD) δ 7.93-8.01 (m, 1 H), 7.78-7.84 (m, 1 H), 7.65-7.75 (m, 2.4 H), 7.49-7.60 (m, 1 H), 6.94-7.02 (m, 1.6 H), 5.29 (dd, 0.7 H), 5.11 (dd, 0.3 H), 3.97-4.07 (m, 3.7 H), 3.62 (td, 0.3 H), 3.17-3.28 (m, 0.7 H), 2.84-3.09 (m, 1.3 H) | 485.2 [M+H]⁺ | 99.5% |
| 832 | (CD₃OD) δ 7.92-8.00 (m, 1 H), 7.77-7.83 (m, 1 H), 7.65-7.75 (m, 2.4 H), 7.50-7.59 (m, 1 H), 6.92-7.02 (m, 1.6 H), 5.29 (dd, 0.7 H), 5.11 (dd, 0.3 H), 3.95-4.07 (m, 3.7 H), 3.62 (td, 0.3 H), 3.15-3.28 (m, 0.7 H), 2.83-3.08 (m, 1.3 H) | 485.2 [M+H]⁺ | 96.7% |
| 833 | (CD₃OD) δ 8.55-8.70 (m, 1 H), 7.87-7.98 (m, 1 H), 7.62-7.78 (m, 4 H), 7.34-7.54 (m, 2.3 H), 6.93 (s, 0.7 H), 5.08-5.26 (m, 1 H), 3.58-4.10 (m, 1 H), 2.84-3.29 (m, 2 H) | 432.2 [M+H]⁺ | 98.9% |
| 834 | (CD₃OD) δ 8.55-8.70 (m, 1 H), 7.86-7.98 (m, 1 H), 7.62-7.80 (m, 4 H), 7.33-7.57 (m, 2.3 H), 6.93 (s, 0.7 H), 5.07-5.29 (m, 1 H), 3.56-4.06 (m, 1 H), 2.83-3.29 (m, 2 H) | 432.2 [M+H]⁺ | 90.4% |
| 835 | (CD₃OD) δ 7.57-7.82 (m, 4 H), 7.42 (br t, 2 H), 6.63-6.98 (m, 2 H), 5.93-6.55 (m, 1 H), 4.77 (br d, 2 H), 4.41 (br s, 1 H), 3.43-3.98 (m, 1 H), 2.77-3.14 (m, 2 H) | 399.2 [M+H]⁺ | 99.7% |
| 836 | (CD₃OD) δ 7.59-7.87 (m, 4 H), 7.35-7.52 (m, 2 H), 6.68-7.02 (m, 2 H), 6.01-6.47 (m, 1 H), 4.77 (br d, 2 H), 4.41 (br s, 1 H), 3.45-4.00 (m, 1 H), 2.72-3.19 (m, 2 H) | 399.2 [M+H]⁺ | 99.1% |
| 837 | (CD₃OD) δ 8.66-8.87 (m, 1 H), 8.28-8.37 (m, 1 H), 8.06-8.16 (m, 1 H), 7.95-8.03 (m, 1 H), 7.51-7.79 (m, 4.4 H), 6.99 (br s, 0.6 H), 5.30 (dd, 0.7 H), 5.15 (br dd, 0.3 H), 3.59-4.17 (m, 1 H), 2.87-3.30 (m, 2 H) | 482.2 [M+H]⁺ | 99.3% |
| 838 | (CD₃OD) δ 8.72-8.80 (m, 1 H), 8.27-8.32 (m, 1 H), 8.05-8.11 (m, 1 H), 7.92-8.00 (m, 1 H), 7.51-7.76 (m, 4.6 H), 6.98 (s, 0.4 H), 5.27 (br dd, 0.7 H), 5.10-5.15 (m, 0.3 H), 4.03 (br t, 0.7 H), 3.65 (br s, 0.3 H), 2.86-3.26 (m, 2 H) | 482.2 [M+H]⁺ | 98.4% |
| 839 | (CD₃OD) δ 8.57-8.68 (m, 1 H), 7.86-8.03 (m, 2 H), 7.65-7.79 (m, 3 H), 7.49-7.63 (m, 1.3 H), 6.97 (br s, 0.7 H), 5.07-5.29 (m, 1 H), 3.57-4.13 (m, 1 H), 2.85-3.27 (m, 2 H) | 500.2 [M+H]⁺ | 92.5% |
| 840 | (CD₃OD) δ 8.60-8.69 (m, 1 H), 7.89-8.03 (m, 2 H), 7.68-7.81 (m, 3 H), 7.50-7.63 (m, 1.3 H), 6.99 (br s, 0.7 H), 5.09-5.31 (m, 1 H), 3.56-4.18 (m, 1 H), 2.84-3.29 (m, 2 H) | 500.2 [M+H]⁺ | 90.3% |
| 841 | (CD₃OD) δ 8.83-8.96 (m, 1 H), 8.27-8.38 (m, 1 H), 7.35-7.80 (m, 6.5 H), 6.90 (br s, 0.5 H), 5.07-5.38 (m, 1 H), 3.58-4.05 (m, 1 H), 2.83-3.26 (m, 2 H) | 453.2 [M+H]+ | 100% |
| 842 | (CD₃OD) δ 8.76-9.02 (m, 1 H), 8.19-8.48 (m, 1 H), 7.34-7.80 (m, 6.5 H), 6.91 (s, 0.5 H), 5.04-5.40 (m, 1 H), 3.55-4.04 (m, 1 H), 2.83-3.26 (m, 2 H) | 453.2 [M+H]⁺ | 99.3% |
| 843 | (CD₃OD) δ 7.78-7.84 (m, 1 H), 7.61-7.73 (m, 3.2 H), 7.33-7.47 (m, 2 H), 6.80-7.08 (m, 1.8 H), 5.07-5.32 (m, 1 H), 3.82-4.15 (m, 4 H), 2.76-3.27 (m, 2 H) | 417.2 [M+H]⁺ | 100% |
| 844 | (CD₃OD) δ 7.77-7.84 (m, 1 H), 7.56-7.74 (m, 3.5 H), 7.32-7.48 (m, 2 H), 6.95-7.04 (m, 1 H), 6.91 (br s, 0.5 H), 5.03-5.38 (m, 1 H), 4.00-4.04 (m, 3 H), 3.57-3.99 (m, 1 H), 2.84-3.26 (m, 2 H) | 417.2 [M+H]⁺ | 99.1% |
| 845 | (CD₃OD) δ 8.33 (d, 1 H), 7.34-7.87 (m, 6.5 H), 7.02-7.24 (m, 1 H), 6.91 (br s, 0.5 H), 5.04-5.37 (m, 1 H), 3.56-4.05 (m, 1 H), 2.80-3.27 (m, 2 H) | 453.2 [M+H]⁺ | 100% |
| 846 | (CD₃OD) δ 8.27-8.37 (m, 1 H), 7.34-7.84 (m, 6.5 H), 7.11-7.28 (m, 1 H), 6.92 (br s, 0.5 H), 5.05-5.36 (m, 1 H), 3.57-4.07 (m, 1 H), 2.82-3.27 (m, 2 H) | 453.2 [M+H]⁺ | 98.0% |
| 847 | (CD₃OD) δ 7.64-7.80 (m, 2 H), 7.53 (br d, 1 H), 7.39 (br d, 1 H), 7.24 (br t, 1 H), 6.72-6.94 (m, 1 H), 6.63-7.01 (m, 1 H), 5.93-6.56 (m, 1 H), 4.75 (br d, 2 H), 4.42 (br s, 1 H), 3.42-4.08 (m, 1 H), 2.74-3.12 (m, 2 H) | 417.2 [M+H]⁺ | 99.6% |
| 848 | (CD₃OD) δ 7.50-7.79 (m, 3 H), 7.11-7.44 (m, 2 H), 6.65-6.98 (m, 2 H), 5.86-6.54 (m, 1 H), 4.75 (br d, 2 H), 4.41 (br s, 1 H), 3.47-4.03 (m, 1 H), 2.72-3.12 (m, 2 H) | 417.2 [M+H]⁺ | 93.9% |
| 849 | (CD₃OD) δ 7.58-7.76 (m, 2 H), 7.48-7.54 (m, 1 H), 7.38-7.48 (m, 1 H), 7.17 (br t, 1 H), 6.66-6.93 (m, 2 H), 5.96-6.43 (m, 1 H), 4.75 (br s, 2 H), 4.42 (br s, 1 H), 3.45-4.07 (m, 1 H), 2.74-3.12 (m, 2 H) | 417.2 [M+H]⁺ | 100% |
| 850 | (CD₃OD) δ 7.59-7.77 (m, 2 H), 7.48-7.54 (m, 1 H), 7.37-7.47 (m, 1 H), 7.16 (br t, 1 H), 6.60-6.96 (m, 2 H), 5.95-6.50 (m, 1 H), 4.75 (br s, 2 H), 4.42 (br s, 1 H), 3.45-3.98 (m, 1 H), 2.73-3.13 (m, 2 H) | 417.2 [M+H]⁺ | 99.3% |
| 851 | (CD₃OD) δ 7.68-7.75 (m, 1 H), 7.45 (br s, 0.3 H), 7.20-7.30 (m, 1 H), 7.10-7.20 (m, 1 H), 6.89 (br s, 0.7 H), 5.05-5.34 (m, 1 H), 3.64-4.08 (m, 1 H), 2.86-3.26 (m, 2 H), 1.50 (s, 6 H), 1.46 (s, 3 H) | 429.2 [M+H]⁺ | 98.7% |
| 852 | (CD₃OD) δ 7.56-7.64 (m, 1 H), 7.25-7.40 (m, 0.4 H), 7.08-7.18 (m, 1 H), 6.99-7.07 (m, 1 H), 6.78 (br s, 0.6 H), 4.95-5.22 (m, 1 H), 3.51-3.94 (m, 1 H), 2.72-3.13 (m, 2 H), 1.38 (s, 6 H), 1.34 (s, 3 H) | 429.2 [M+H]⁺ | 99.1% |
| 853 | (CD₃OD) δ 8.29-8.37 (m, 1 H), 7.89-8.03 (m, 1 H), 7.48-7.86 (m, 4.5 H), 7.16-7.26 (m, 1 H), 6.97 (s, 0.5 H), 5.07-5.35 (m, 1 H), 3.94-4.12 (m, 0.7 H), 3.57-3.72 (m, 0.3 H), 2.84-3.28 (m, 2 H) | 521.2 [M+H]⁺ | 94.8% |
| 854 | (CD₃OD) δ 8.31-8.36 (m, 1 H), 7.92-8.04 (m, 1 H), 7.48-7.85 (m, 4.5 H), 7.18-7.27 (m, 1 H), 6.96 (br s, 0.5 H), 5.04-5.37 (m, 1 H), 4.05 (br s, 0.7 H), 3.66 (br s, 0.3 H), 2.82-3.29 (m, 2 H) | 521.2 [M+H]⁺ | 78.2% |
| 855 | (CD₃OD) δ 7.69-7.73 (m, 1 H), 7.49 (br s, 0.3 H), 7.02-7.33 (m, 2 H), 6.88 (s, 0.7 H), 5.04-5.32 (m, 1 H), 3.59-4.05 (m, 1 H), 2.98-3.24 (m, 1 H), 2.78-2.94 (m, 1 H), 2.26-2.41 (m, 1 H), 1.18-1.32 (m, 4 H) | 413.2 [M+H]⁺ | 97.9% |
| 856 | (CD₃OD) δ 7.66-7.78 (m, 1 H), 7.48 (br s, 0.3 H), 7.10-7.32 (m, 2 H), 6.88 (br s, 0.7 H), 5.06-5.36 (m, 1 H), 3.60-4.06 (m, 1 H), 2.82-3.24 (m, 2 H), 2.23-2.44 (m, 1 H), 1.14-1.38 (m, 4 H) | 413.2 [M+H]⁺ | 92.4% |
| 857 | (CD₃OD) δ 8.40-8.91 (m, 1 H), 7.87-8.04 (m, 1 H), 7.70-7.80 (m, 2 H), 7.46-7.54 (m, 1 H), 7.19-7.39 (m, 3 H), 6.64-7.14 (m, 1 H), 5.24 (dd, 1 H), 4.02 (br t, 0.6 H), 3.69-3.90 (m, 0.5 H), 3.17-3.31 (m, 0.8 H), 2.85-3.02 (m, 1.2 H), 2.50-2.62 (m, 3 H) | 446.2 [M+H]⁺ | 100% |
| 858 | (CD₃OD) δ 8.36-8.80 (m, 1 H), 7.83-8.04 (m, 1 H), 7.69-7.82 (m, 2 H), 7.44-7.59 (m, 1.3 H), 7.19-7.37 (m, 2.7 H), 6.76-7.04 (m, 1 H), 5.24 (dd, 1 H), 4.02 (br t, 0.7 H), 3.72-3.88 (m, 0.4 H), 3.11-3.28 (m, 1 H), 2.84-2.97 (m, 1 H), 2.51-2.60 (m, 3 H) | 446.2 [M+H]⁺ | 99.1% |
| 859 | (CD₃OD) δ 8.06-8.61 (m, 1 H), 7.61-7.94 (m, 2 H), 6.85-7.61 (m, 5 H), 5.29 (br dd, 1 H), 3.53-4.43 (m, 1 H), 2.77-3.24 (m, 2 H), 2.34-2.68 (m, 3 H) | 467.2 [M+H]⁺ | 98.7% |
| 860 | (CD₃OD) δ 8.21-8.57 (m, 1 H), 7.58-8.00 (m, 2 H), 6.89-7.58 (m, 5 H), 5.29 (br dd, 1 H), 3.53-4.20 (m, 1 H), 2.87-3.26 (m, 2 H), 2.42-2.68 (m, 3 H) | 467.1 [M+H]⁺ | 98.7% |
| 861 | (CD₃OD) δ 8.35 (d, 1 H), 7.69-7.87 (m, 1.6 H), 7.31-7.69 (m, 3 H), 7.15-7.31 (m, 2 H), 6.94 (br s, 0.4 H), 5.04-5.35 (m, 1 H), 3.60-4.16 (m, 1 H), 2.90-3.26 (m, 2 H) | 471.1 [M+H]⁺ | 100% |
| 862 | (CD₃OD) δ 8.09-8.33 (m, 1 H), 7.57-7.73 (m, 1.6 H), 7.17-7.57 (m, 3 H), 6.99-7.17 (m, 2 H), 6.82 (br s, 0.4 H), 4.99-5.30 (m, 1 H), 3.49-4.01 (m, 1 H), 2.77-3.17 (m, 2 H) | 471.1 [M+H]⁺ | 95.1% |
| 863 | (CD₃OD) δ 8.34 (s, 1 H), 7.70 (s, 1 H), 7.53 (d, 1 H), 7.38 (td, 1 H), 7.19-7.31 (m, 1 H), 6.78 (br s, 1 H), 6.00-6.42 (m, 1 H), 4.95-5.18 (m, 1 H), 3.95 (br s, 1 H), 3.41 (td, 2 H), 3.12 (br d, 1 H), 2.87 (br d, 1 H) | 418.1 [M+H]⁺ | 99.3% |
| 864 | (CD₃OD) δ 8.34 (s, 1 H), 7.70 (s, 1 H), 7.53 (d, 1 H), 7.38 (td, 1 H), 7.14-7.30 (m, 1 H), 6.78 (br s, 1 H), 6.02-6.42 (m, 1 H), 4.95-5.09 (m, 1 H), 3.65-4.13 (m, 1 H), 3.41 (td, 2 H), 3.12 (br d, 1 H), 2.87 (br d, 1 H) | 418.1 [M+H]⁺ | 98.5% |
| 865 | (CD₃OD) δ 8.34 (br s, 1 H), 7.59-7.84 (m, 2 H), 7.42 (dd, 1 H), 7.21 (td, 1 H), 6.75 (br s, 1 H), 5.99-6.49 (m, 1 H), 4.50-4.61 (m, 1 H), 3.92 (br s, 1 H), 3.41 (td, 2 H), 3.11 (br d, 1 H), 2.85 (br d, 1 H) | 418.1 [M+H]⁺ | 98.4% |
| 866 | (CD₃OD) δ 8.34 (br s, 1 H), 7.60-7.81 (m, 2 H), 7.42 (dd, 1 H), 7.21 (td, 1 H), 6.75 (br s, 1 H), 6.01-6.45 (m, 1 H), 4.46-4.61 (m, 1 H), 3.93 (br s, 1 H), 3.41 (td, 2 H), 3.09 (br s, 1 H), 2.85 (br d, 1 H) | 418.1 [M+H]⁺ | 98.2% |
| 867 | (CD₃OD) δ 8.55-8.74 (m, 1 H), 7.87-8.00 (m, 1 H), 7.69-7.81 (m, 2 H), 7.48-7.62 (m, 1 H), 7.33-7.44 (m, 1 H), 7.12-7.32 (m, 1 H), 6.95 (br s, 1 H), 5.12-5.32 (m, 1 H), 4.03 (br s, 0.7 H), 3.72 (br s, 0.3 H), 3.02-3.29 (m, 1 H), 2.94 (br d, 1 H) | 450.1 [M+H]⁺ | 99.0% |
| 868 | (CD₃OD) δ 8.38-8.72 (m, 1 H), 7.75-7.96 (m, 1 H), 7.54-7.72 (m, 2 H), 7.35-7.51 (m, 1 H), 7.19-7.34 (m, 1 H), 7.05-7.19 (m, 1 H), 6.84 (s, 1 H), 4.96-5.24 (m, 1 H), 3.79-3.98 (m, 0.7 H), 3.53-3.68 (m, 0.3 H), 2.96-3.19 (m, 1 H), 2.76-2.91 (m, 1 H) | 450.1 [M+H]⁺ | 96.8% |
| 869 | (CD₃OD) δ 7.79-7.90 (m, 1 H), 7.73 (s, 1 H), 7.38-7.65 (m, 1.3 H), 7.17-7.38 (m, 2 H), 6.85-7.16 (m, 1.7 H), 5.12-5.34 (m, 1.4 H), 3.96-4.08 (m, 3.6 H), 2.89-3.30 (m, 2 H), 2.50-2.61 (m, 3 H) | 431.1 [M+H]⁺ | 100% |
| 870 | (CD₃OD) δ 7.71 (d, 1 H), 7.61 (s, 1 H), 7.29-7.49 (m, 1.3 H), 7.05-7.22 (m, 2 H), 6.65-6.95 (m, 1.7 H), 4.89-5.27 (m, 1.2 H), 3.81-3.96 (m, 3.8 H), 2.74-3.18 (m, 2 H), 2.36-2.52 (m, 3 H) | 431.2 [M+H]⁺ | 98.5% |
| 871 | (CD₃OD) δ 8.94 (s, 1 H), 8.14-8.67 (m, 1 H), 7.57-7.84 (m, 2 H), 7.40-7.54 (m, 1.3 H), 7.13-7.37 (m, 2 H), 6.93 (br s, 0.7 H), 5.33 (dd, 0.7 H), 5.12 (br dd, 0.3 H), 3.91-4.06 (m, 0.7 H), 3.67 (br s, 0.3 H), 2.85-3.28 (m, 2 H), 2.50-2.61 (m, 3 H) | 467.2 [M+H]⁺ | 100% |
| 872 | (CD₃OD) δ 8.74-9.06 (m, 1 H), 8.25-8.49 (m, 1 H), 7.57-7.82 (m, 2 H), 7.42-7.55 (m, 1.3 H), 7.10-7.34 (m, 2 H), 6.93 (s, 0.7 H), 5.33 (dd, 0.7 H), 5.12 (br dd, 0.3 H), 3.92-4.10 (m, 0.7 H), 3.53-3.80 (m, 0.3 H), 2.88-3.28 (m, 2 H), 2.50-2.61 (m, 3 H) | 467.2 [M+H]⁺ | 95.1% |
| 873 | (CD₃OD) δ 8.34 (br s, 1 H), 7.64-7.73 (m, 3 H), 7.42 (qd, 2 H), 6.78 (br s, 1 H), 6.02-6.45 (m, 1 H), 4.49-4.61 (m, 0.4 H), 3.89 (br s, 0.6 H), 3.35-3.48 (m, 2.5 H), 2.93-3.18 (m, 1.5 H), 2.86 (br d, 1 H) | 400.1 [M+H]⁺ | 97.8% |
| 874 | (CD₃OD) δ 8.34 (br s, 1 H), 7.64-7.73 (m, 3 H), 7.42 (qd, 2 H), 6.79 (br s, 1 H), 6.03-6.44 (m, 1 H), 4.51-4.60 (m, 0.3 H), 3.91 (br s, 0.7 H), 3.35-3.49 (m, 2.4 H), 2.93-3.20 (m, 1.6 H), 2.86 (br d, 1 H) | 400.2 [M+H]⁺ | 100% |
| 875 | (CD₃OD) δ 8.94 (s, 1 H), 8.22-8.51 (m, 1 H), 7.69-7.83 (m, 1.3 H), 7.57-7.69 (m, 0.7 H), 7.49-7.56 (m, 1.3 H), 7.33-7.44 (m, 1 H), 7.16-7.30 (m, 1 H), 6.94 (s, 0.7 H), 5.35 (dd, 0.7 H), 5.14 (dd, 0.3 H), 4.01 (br t, 0.7 H), 3.59-3.79 (m, 0.3 H), 2.84-3.29 (m, 2 H) | 471.1 [M+H]⁺ | 100% |
| 876 | (CD₃OD) δ 8.63-8.99 (m, 1 H), 8.11-8.35 (m, 1 H), 7.57-7.78 (m, 1.3 H), 7.45-7.56 (m, 0.8 H), 7.37-7.43 (m, 1.3 H), 7.22-7.33 (m, 1 H), 7.07-7.19 (m, 1 H), 6.82 (s, 0.7 H), 5.23 (dd, 0.7 H), 5.02 (dd, 0.3 H), 3.89 (br t, 0.7 H), 3.56 (br d, 0.3 H), 2.76-3.14 (m, 2 H) | 471.1 [M+H]⁺ | 97.3% |
| 877 | (CD₃OD) δ 8.62 (s, 1 H), 8.42 (br s, 1 H), 7.93 (d, 1 H), 7.67 (s, 1 H), 7.47-7.53 (m, 2 H), 7.43 (dd, 1 H), 7.21-7.26 (m, 1 H), 6.76 (br s, 1 H), 4.71 (br d, 1 H), 4.52 (s, 2 H), 3.87 (br s, 1 H), 3.43 (s, 3 H), 3.04-3.17 (m, 1 H), 2.83 (br d, 1 H), 2.45 (s, 3 H) | 443.0 [M+H]⁺ | 95.2% |
| 878 | (CD₃OD) δ 8.63 (s, 1 H), 8.43 (br s, 1 H), 7.94 (d, 1 H), 7.67 (s, 1 H), 7.48-7.54 (m, 2 H), 7.44 (dd, 1 H), 7.24 (dd, 1 H), 6.76 (br s, 1 H), 4.72 (br d, 1 H), 4.53 (s, 2 H), 3.87 (br s, 1 H), 3.43 (s, 3 H), 3.04-3.17 (m, 1 H), 2.83 (br d, 1 H), 2.46 (s, 3 H) | 443.0 [M+H]⁺ | 73.8% |
| 879 | (CD₃OD) δ 9.05 (br s, 1 H), 8.50 (br d, 1 H), 7.88-8.32 (m, 3 H), 7.62-7.75 (m, 2 H), 7.33-7.52 (m, 2 H), 7.10-7.29 (m, 1 H), 6.83 (br s, 1 H), 4.64 (s, 1 H), 3.72-4.18 (m, 1 H), 2.68-3.25 (m, 2 H) | 430.1 [M+H]⁺ | 99.8% |
| 880 | (CD₃OD) δ 8.92 (br s, 1 H), 8.37 (br d, 1 H), 7.76-8.12 (m, 3 H), 7.54-7.71 (m, 2 H), 7.21-7.47 (m, 2 H), 7.10 (br t, 1 H), 6.71 (br s, 1 H), 4.20-4.56 (m, 1 H), 3.89 (br s, 1 H), 2.60-3.07 (m, 2 H) | 430.0 [M+H]⁺ | 99.2% |
| 881 | (CD₃OD) δ 8.23-8.31 (m, 1 H), 7.55-7.73 (m, 2.4 H), 7.37-7.45 (m, 1 H), 7.14-7.25 (m, 1 H), 6.87 (br s, 0.6 H), 5.33 (dd, 0.7 H), 5.10 (dd, 0.3 H), 3.94-4.06 (m, 0.6 H), 3.87-3.93 (m, 3 H), 3.62 (br t, 0.3 H), 3.14-3.24 (m, 0.7 H), 2.94-3.05 (m, 0.4 H), 2.82-2.93 (m, 1 H), 2.47-2.57 (m, 3 H) | 449.2 [M+H]⁺ | 92.7% |
| 882 | (CD₃OD) δ 8.23-8.31 (m, 1 H), 7.55-7.75 (m, 2.4 H), 7.37-7.45 (m, 1 H), 7.14-7.24 (m, 1 H), 6.87 (br s, 0.6 H), 5.33 (dd, 0.7 H), 5.10 (dd, 0.3 H), 3.94-3.99 (m, 0.5 H), 3.89-3.92 (m, 3 H), 3.56-3.83 (m, 0.5 H), 3.11-3.22 (m, 0.6 H), 2.84-3.05 (m, 1.4 H), 2.48-2.56 (m, 3 H) | 449.2 [M+H]⁺ | 88.3% |
| 883 | (CD₃OD) δ 8.45 (br s, 1 H), 7.69 (s, 1 H), 7.54 (d, 1 H), 7.46 (s, 1 H), 6.94-7.31 (m, 2 H), 6.54-6.86 (m, 1 H), 4.53 (br d, 1 H), 3.90 (br s, 1 H), 3.11 (br d, 1 H), 2.84 (br d, 1 H), 2.48 (s, 3 H) | 400.20 [M+H]⁺ | 98.2% |
| 884 | (CD₃OD) δ 8.44 (br s, 1 H), 7.69 (s, 1 H), 7.54 (d, 1 H), 7.46 (s, 1 H), 6.97-7.28 (m, 2 H), 6.76 (br s, 1 H), 4.53 (br d, 1 H), 3.90 (br s, 1 H), 3.11 (br d, 1 H), 2.84 (br d, 1 H), 2.48 (s, 3 H) | 400.20 [M+H]⁺ | 94.7% |
| 885 | (CD₃OD) δ 8.62-8.73 (m, 1 H), 7.55-7.74 (m, 2 H), 7.35-7.44 (m, 1 H), 7.12-7.25 (m, 1 H), 6.85 (br s, 1 H), 5.10 (dd, 0.3 H), 4.75 (dd, 0.7 H), 4.24-4.37 (m, 2 H), 3.80-3.96 (m, 0.7 H), 3.53 (br s, 0.3 H), 3.12-3.26 (m, 0.8 H), 2.93-3.05 (m, 0.3 H), 2.74-2.90 (m, 1 H), 1.37-1.49 (m, 3 H) | 382.2 [M+H]⁺ | 99.0% |
| 886 | (CD₃OD) δ 8.63-8.75 (m, 1 H), 7.56-7.73 (m, 2 H), 7.36-7.43 (m, 1 H), 7.12-7.24 (m, 1 H), 6.85 (br s, 1 H), 5.11 (br dd, 0.2 H), 4.75 (br dd, 0.9 H), 4.24-4.37 (m, 2 H), 3.88 (br s, 0.7 H), 3.47-3.60 (m, 0.3 H), 3.11-3.26 (m, 0.8 H), 2.97 (br d, 0.3 H), 2.75-2.90 (m, 1 H), 1.37-1.53 (m, 3 H) | 382.2 [M+H]⁺ | 97.9% |
| 887 | (CD₃OD) δ 7.62-7.80 (m, 2 H), 7.35-7.50 (m, 2 H), 7.08-7.19 (m, 1 H), 6.93-7.06 (m, 1 H), 6.41-6.85 (m, 1 H), 4.91-4.97 (m, 0.4 H), 4.37 (dd, 0.8 H), 3.83 (br s, 0.8 H), 3.12-3.27 (m, 4 H), 2.74-3.06 (m, 2 H) | 382.3 [M+H]⁺ | 100% |
| 888 | (CD₃OD) δ 7.62-7.80 (m, 2 H), 7.35-7.51 (m, 2 H), 7.09-7.18 (m, 1 H), 6.93-7.06 (m, 1 H), 6.42-6.84 (m, 1 H), 4.91-4.97 (m, 0.4 H), 4.37 (dd, 0.8 H), 3.83 (br s, 0.8 H), 3.12-3.27 (m, 4 H), 2.74-3.06 (m, 2 H) | 382.3 [M+H]⁺ | 99.6% |
| 889 | (CD₃OD) δ 8.44 (s, 1 H), 7.68 (s, 1 H), 6.97-7.29 (m, 3 H), 6.78 (br s, 1 H), 4.49-4.63 (m, 1 H), 4.02 (br s, 1 H), 2.79-3.15 (m, 2 H), 2.45-2.49 (m, 6 H) | 414.1 [M+H]⁺ | 99.3% |
| 890 | (CD₃OD) δ 8.45 (s, 1 H), 7.68 (s, 1 H), 6.71-7.30 (m, 4 H), 4.45-4.66 (m, 1 H), 3.50-4.14 (m, 1 H), 2.74-3.18 (m, 2 H), 2.42-2.52 (m, 7 H) | 414.2 [M+H]⁺ | 99.3% |
| 891 | (CD₃OD) δ 7.63-8.12 (m, 2.8 H), 7.54 (br d, 1 H), 7.34-7.46 (m, 1 H), 7.00-7.34 (m, 2.2 H), 6.81 (br s, 1 H), 4.81 (br s, 1 H), 3.61-4.19 (m, 4 H), 3.17-3.29 (m, 1 H), 2.91 (br d, 1 H) | 484.1 [M+H]⁺ | 98.6% |
| 892 | (CD₃OD) δ 7.65-8.14 (m, 3 H), 7.54 (br d, 1 H), 7.39 (td, 1 H), 7.00-7.34 (m, 2 H), 6.84 (br s, 1 H), 4.81 (br s, 1 H), 3.73-4.14 (m, 4 H), 3.25 (br s, 1 H), 2.91 (br d, 1 H) | 484.2 [M+H]⁺ | 98.5% |
| 893 | (CD₃OD) δ 7.61-7.88 (m, 2 H), 7.48-7.55 (m, 1 H), 7.36-7.48 (m, 1 H), 7.16 (br t, 1 H), 6.77 (s, 1 H), 4.61-4.86 (m, 1 H), 3.44-4.17 (m, 1 H), 2.82-3.27 (m, 2 H), 1.66 (br s, 6 H) | 412.2 [M+H]⁺ | 99.7% |
| 894 | (CD₃OD) δ 7.61-7.88 (m, 2 H), 7.49-7.57 (m, 1 H), 7.43 (td, 1 H), 7.16 (t, 1 H), 6.77 (br s, 1 H), 4.59-4.85 (m, 1 H), 3.58-4.20 (m, 1 H), 2.79-3.22 (m, 2 H), 1.66 (br s, 6 H) | 412.2 [M+H]⁺ | 99.3% |
| 895 | (CD₃OD) δ 8.30 (s, 1 H), 8.10 (s, 1 H), 7.80-7.97 (m, 2 H), 7.45 (dd, 1 H), 7.09-7.30 (m, 2 H), 6.87 (s, 1 H), 4.76 (br dd, 1 H), 3.73-4.04 (m, 2 H), 3.44-3.59 (m, 2 H), 3.08-3.24 (m, 1 H), 2.82-2.99 (m, 3 H), 1.93-2.12 (m, 2 H), 1.59-1.84 (m, 2 H) | 520.2 [M+H]⁺ | 98.9% |
| 896 | (CD₃OD) δ 8.52 (s, 1 H), 8.33 (s, 1 H), 8.13 (s, 1 H), 7.88 (d, 1 H), 7.49 (dd, 1 H), 7.14-7.34 (m, 2 H), 7.06 (s, 1 H), 4.80 (br dd, 1 H), 3.71-3.91 (m, 2 H), 3.46-3.64 (m, 2 H), 3.13-3.28 (m, 1 H), 2.88-3.01 (m, 3 H), 1.95-2.11 (m, 2 H), 1.63-1.81 (m, 2 H) | 520.2 [M+H]⁺ | 97.5% |
| 897 | (DMSO-d₆) δ 12.21 (br s, 1 H), 8.75 (br s, 1 H), 7.60-7.66 (m, 1 H), 7.17-7.33 (m, 3 H), 6.57 (s, 1 H), 3.61-4.95 (m, 2 H), 2.78-3.01 (m, 2 H), 2.46 (s, 3 H) | 418.2 [M+H]⁺ | 99.5% |
| 898 | (DMSO-d₆) δ 12.20 (br s, 1 H), 8.75 (br s, 1 H), 7.59-7.66 (m, 1 H), 7.13-7.33 (m, 3 H), 6.57 (s, 1 H), 3.52-4.93 (m, 2 H), 2.80-2.98 (m, 2 H), 2.46 (s, 3 H) | 418.1 [M+H]+ | 98.7% |
| 899 | (CD₃OD) δ 8.47 (br s, 1 H), 7.71 (s, 1 H), 7.53 (d, 1 H), 7.38 (td, 1 H), 6.99-7.31 (m, 2 H), 4.58 (br d, 1 H), 3.58-4.12 (m, 1 H), 3.13 (br s, 1 H), 2.88 (br d, 1 H) | 405.1 [M+H]⁺ | 99.1% |
| 900 | (CD₃OD) δ 8.47 (br s, 1 H), 7.71 (s, 1 H), 7.53 (d, 1 H), 7.39 (td, 1 H), 6.94-7.30 (m, 2 H), 4.58 (br d, 1 H), 3.58-4.06 (m, 1 H), 3.12 (br s, 1 H), 2.88 (br d, 1 H) | 405.1 [M+H]⁺ | 97.9% |
| 901 | (CD₃OD) δ 7.92-8.07 (m, 1 H), 7.68 (s, 1 H), 7.10-7.47 (m, 3.4 H), 6.87 (br s, 0.6 H), 5.07 (dd, 0.3 H), 4.67 (dd, 0.7 H), 4.06 (s, 3 H), 3.89 (br d, 0.7 H), 3.49 (br d, 0.3 H), 3.12-3.23 (m, 0.7 H), 2.74-3.04 (m, 1.3 H), 2.48-2.60 (m, 3 H) | 364.2 [M+H]⁺ | 100% |
| 902 | (CD₃OD) δ 7.94-8.06 (m, 1 H), 7.68 (s, 1 H), 7.10-7.47 (m, 3.4 H), 6.88 (s, 0.6 H), 5.07 (dd, 0.3 H), 4.67 (dd, 0.7 H), 4.06 (s, 3 H), 3.85-3.96 (m, 0.7 H), 3.50 (td, 0.3 H), 3.13-3.22 (m, 0.7 H), 2.76-3.02 (m, 1.3 H), 2.50-2.60 (m, 3 H) | 364.2 [M+H]⁺ | 97.2% |
| 903 | (CD₃OD) δ 7.92-8.08 (m, 1 H), 7.70 (s, 1 H), 7.30-7.42 (m, 1 H), 7.15-7.28 (m, 1.3 H), 6.83-6.96 (m, 1.7 H), 5.08 (br dd, 0.3 H), 4.69 (dd, 0.7 H), 4.06-4.10 (m, 3 H), 3.98-4.02 (m, 3 H), 3.81-3.93 (m, 0.7 H), 3.43-3.53 (m, 0.3 H), 3.15-3.24 (m, 0.8 H), 2.76-3.03 (m, 1.2 H) | 380.2 [M+H]⁺ | 100% |
| 904 | (CD₃OD) δ 7.93-8.09 (m, 1 H), 7.70 (s, 1 H), 7.30-7.40 (m, 1 H), 7.11-7.28 (m, 1.3 H), 6.80-6.97 (m, 1.7 H), 5.08 (dd, 0.3 H), 4.69 (dd, 0.7 H), 4.05-4.13 (m, 3 H), 3.97-4.03 (m, 3 H), 3.87 (br t, 0.7 H), 3.48 (br s, 0.3 H), 3.15-3.25 (m, 0.7 H), 2.76-3.04 (m, 1.3 H) | 380.2 [M+H]⁺ | 99.7% |
| 905 | (CD₃OD) δ 7.61-7.75 (m, 2 H), 7.43 (br d, 1 H), 6.94-7.32 (m, 2 H), 4.57 (br s, 1 H), 3.60-4.09 (m, 1 H), 3.16 (br s, 1 H), 2.88 (br d, 1 H), 1.51-1.77 (m, 6 H) | 463.2 [M+H]⁺ | 98.2% |
| 906 | (CD₃OD) δ 7.58-7.77 (m, 2 H), 7.43 (dd, 1 H), 6.96-7.31 (m, 2 H), 4.47-4.60 (m, 1 H), 3.59-4.07 (m, 1 H), 2.98-3.24 (m, 1 H), 2.88 (br d, 1 H), 1.46-1.81 (m, 6 H) | 463.2 [M+H]⁺ | 96.1% |
| 907 | (CD₃OD) δ 7.71 (s, 1 H), 7.46-7.56 (m, 1 H), 6.84-7.36 (m, 3 H), 5.08 (dd, 0.3 H), 4.68 (dd, 0.7 H), 3.98 (s, 3 H), 3.40-3.94 (m, 1 H), 2.92-3.25 (m, 1 H), 2.77-2.91 (m, 1 H), 2.30-2.63 (m, 6 H) | 378.2 [M+H]⁺ | 93.2% |
| 908 | (CD₃OD) δ 7.69-7.84 (m, 1 H), 7.45-7.58 (m, 1 H), 6.86-7.33 (m, 3 H), 5.08 (br dd, 0.3 H), 4.69 (dd, 0.7 H), 3.99 (s, 3 H), 3.36-3.95 (m, 1 H), 2.95-3.24 (m, 1 H), 2.76-2.92 (m, 1 H), 2.30-2.59 (m, 6 H) | 378.2 [M+H]⁺ | 85.0% |
| 909 | (CD₃OD) δ 7.65-7.79 (m, 1 H), 7.22-7.40 (m, 2 H), 6.86-7.17 (m, 2 H), 5.04 (br dd, 0.4 H), 4.66 (dd, 0.6 H), 3.76-4.10 (m, 7 H), 2.96-3.25 (m, 1 H), 2.77-2.94 (m, 1 H), 2.30-2.47 (m, 3 H) | 394.2 [M+H]⁺ | 100% |
| 910 | (CD₃OD) δ 7.66-7.79 (m, 1 H), 7.22-7.37 (m, 2 H), 6.86-7.19 (m, 2 H), 5.04 (br dd, 0.4 H), 4.66 (dd, 0.6 H), 3.84-4.07 (m, 7 H), 2.94-3.22 (m, 1 H), 2.76-2.92 (m, 1 H), 2.29-2.42 (m, 3 H) | 394.2 [M+H]⁺ | 99.6% |
| 911 | (CD₃OD) δ 8.44 (s, 1 H), 7.63 (br s, 1 H), 7.53 (d, 1 H), 7.38 (td, 1 H), 7.19-7.30 (m, 1.4 H), 6.96-7.17 (m, 1 H), 6.87 (br s, 0.6 H), 3.83-4.40 (m, 2 H), 0.95-1.34 (m, 4 H) | 430.2 [M+H]⁺ | 92.6% |
| 912 | (CD₃OD) δ 8.44 (s, 1 H), 7.63 (br s, 1 H), 7.53 (d, 1 H), 7.38 (td, 1 H), 7.17-7.29 (m, 1.4 H), 6.96-7.17 (m, 1 H), 6.88 (br s, 0.6 H), 3.79-4.43 (m, 2 H), 0.95-1.34 (m, 4 H) | 430.1 [M+H]⁺ | 89.3% |
| 913 | (CD₃OD) δ 7.51-7.79 (m, 4 H), 7.32-7.48 (m, 3 H), 6.85 (br s, 0.7 H), 6.46 (br s, 0.3 H), 4.94-4.98 (m, 0.4 H), 4.36 (dd, 0.6 H), 3.78 (br s, 0.7 H), 3.36-3.50 (m, 2.3 H), 3.08-3.26 (m, 2 H), 2.67-3.04 (m, 2 H) | 380.1 [M+H]⁺ | 99.7% |
| 914 | (CD₃OD) δ 7.51-7.75 (m, 4 H), 7.32-7.48 (m, 3 H), 6.85 (br s, 0.7 H), 6.46 (br s, 0.3 H), 4.93-4.99 (m, 0.4 H), 4.36 (dd, 0.6 H), 3.77 (br t, 0.7 H), 3.37-3.53 (m, 2.3 H), 3.10-3.27 (m, 2 H), 2.66-3.03 (m, 2 H) | 380.1 [M+H]⁺ | 99.2% |
| 915 | (CD₃OD) δ 8.72 (d, 1 H), 7.38-8.02 (m, 5 H), 7.19 (br t, 2 H), 6.99 (br s, 1 H), 3.44-4.21 (m, 2 H), 2.56-3.00 (m, 2 H) | 471.0 [M+H]⁺ | 97.6% |
| 916 | (CD₃OD) δ 8.73 (br d, 1 H), 7.39-7.95 (m, 5 H), 7.20 (br t, 2 H), 7.00 (br s, 1 H), 3.58-4.19 (m, 2 H), 2.61-2.99 (m, 2 H) | 471.0 [M+H]⁺ | 96.9% |
| 917 | (CD₃OD) δ 8.11 (br s, 1 H), 7.55-7.68 (m, 2 H), 7.34-7.44 (m, 1 H), 7.17 (td, 2 H), 6.65 (br s, 1 H), 4.65 (br s, 1 H), 3.79 (br s, 1 H), 2.44-2.74 (m, 2 H), 1.31-1.68 (m, 4 H) | 394.2 [M+H]⁺ | 97.3% |
| 918 | (CD₃OD) δ 8.11 (br d, 1 H), 7.58-7.67 (m, 2 H), 7.35-7.43 (m, 1 H), 7.17 (td, 2 H), 6.65 (br s, 1 H), 4.63 (br s, 1 H), 3.78 (br s, 1 H), 2.46-2.76 (m, 2 H), 1.32-1.68 (m, 4 H) | 394.2 [M+H]⁺ | 93.4% |
| 919 | (CD₃OD) δ 7.85 (s, 1 H), 7.62-7.82 (m, 1.8 H), 7.51 (br d, 1.2 H), 7.21-7.33 (m, 2 H), 6.88 (br s, 1.8 H), 6.33 (br s, 0.2 H), 4.22-4.35 (m, 0.8 H), 3.46-4.04 (m, 1.2 H), 2.99-3.11 (m, 1 H), 2.69-2.87 (m, 1 H), 2.57 (br s, 3 H) | 399.0 [M+H]⁺ | 95.3% |
| 920 | (CD₃OD) δ 7.73 (s, 1 H), 7.46-7.69 (m, 1.8 H), 7.39 (br d, 1.2 H), 7.09-7.21 (m, 2 H), 6.77 (br s, 1.8 H), 6.20 (br s, 0.2 H), 4.18 (br d, 0.8 H), 3.31-3.91 (m, 1.2 H), 2.87-2.98 (m, 1 H), 2.71 (br d, 1 H), 2.46 (br s, 3 H) | 399.0 [M+H]⁺ | 99.3% |
| 921 | (CD₃OD) δ 8.52 (s, 1H), 7.71 (br s, 1H), 7.66-7.57 (m, 2H), 7.42-7.35 (m, 2H), 7.22 (s, 1H), 6.48 (s, 1H), 5.42-5.40 (d, 1H), 5.27-5.24 (d, 1H). | 372.1 [M+H]⁺ | 98.7% |
| 922 | (CD₃OD) δ 8.52 (s, 1H), 7.71 (br s, 1H), 7.66-7.57 (m, 2H), 7.41-7.36 (m, 2H), 7.22 (s, 1H), 6.45 (s, 1H), 5.41 (s, 1H), 5.27 (s, 1H). | 372.1 [M+H]⁺ | 96.5% |
| 923 | (CD₃OD) δ 7.69-7.66 (m, 2H), 7.63-7.61 (d, 1H), 7.56 (s, 1H), 7.41-7.31 (m, 2H), 6.91 (s, 1H), 6.51 (s, 1H), 5.16-5.12 (d, 1H), 4.92-4.89 (d, 1H), 3.97 (s, 3H). | 335.1 [M+H]⁺ | 99.1% |
| 924 | (CD₃OD) δ 7.70-7.66 (m, 2H), 7.63-7.61 (d, 1H), 7.56 (s, 1H), 7.41-7.31 (m, 2H), 6.91 (s, 1H), 6.51 (s, 1H), 5.15-5.11 (d, 1H), 4.92-4.88 (d, 1H), 3.97 (s, 3H). | 335.1 [M+H]⁺ | 96.0% |
| 925 | Data Provided Above | | |
| 926 | Data Provided Above | | |
| 927 | (CD₃OD) δ 8.88 (s, 1H), 8.72-8.70 (d, 1H), 8.46 (s, 1H), 8.18-8.16 (d, 1H), 7.69 (s, 1H), 7.66-7.64 (m, 1H), 7.60-7.57 (m, 1H), 7.43-7.39 (m, 2H), 6.52 (s, 1H), 5.06-5.03 (d, 1H), 4.70-4.68 (d, 1H). | 332.1 [M+H]⁺ | 99.5% |
| 928 | (CD₃OD) δ 8.88 (s, 1H), 8.72-8.70 (d, 1H), 8.46 (s, 1H), 8.18-8.16 (d, 1H), 7.69 (s, 1H), 7.66-7.64 (m,1H), 7.60-7.57 (m, 1H), 7.43-7.39 (m, 2H), 6.54 (s, 1H), 5.06-5.02 (dd, 1H), 4.71-4.68 (d, 1H). | 332.1 [M+H]⁺ | 98.2% |
| 929 | (CD₃OD) δ 8.63-8.58 (d, 2H), 8.15 (s, 1H), 7.71 (s, 1H), 7.62-7.57 (m, 2H), 7.45-7.41 (t, 1H), 7.38-7.31 (m, 2H), 7.06 (br s, 1H), 6.58 (s, 1H), 5.38 (s, 1H), 5.12-5.09 (d, 1H). | 371.1 [M+H]⁺ | >99% |
| 930 | (CD₃OD) δ 8.63-8.58 (d, 2H), 8.15 (s, 1H), 7.71 (s, 1H), 7.63-7.57 (m, 2H), 7.45-7.41 (t, 1H), 7.39-7.34 (m, 2H), 7.07 (br s, 1H), 6.58 (s, 1H), 5.39 (s, 1H), 5.12-5.09 (d, 1H). | 371.1 [M+H]⁺ | >99% |
| 931 | Data Provided Above | | |
| 932 | Data Provided Above | | |
| 933 | (CD₃OD) δ 8.74-8.73 (m, 1H), 8.38-8.37 (m, 1H), 7.70-7.68 (m, 3H), 7.66-7.64 (m, 1H), 7.48-7.33 (m, 2H), 7.27-7.26 (m, 1H), 6.51-6.50 (d, 1H), 4.98-4.94 (m, 1H), 4.65-4.62 (d, 1H). | 333.1 [M+H]⁺ | >99% |
| 934 | (CD₃OD) δ 8.74-8.73 (m, 1H), 8.38-8.37 (m, 1H), 7.70-6.67 (m, 3H), 7.66-7.63 (m, 1H), 7.48-7.33 (m, 2H), 7.27-7.26 (m, 1H), 6.50-6.51 (d, 1H), 4.98-4.93 (m, 1H), 4.65-4.62 (d, 1H). | 332.2 [M+H]⁺ | 86.9% |
| 935 | (CD₃OD) δ 8.38 (s, 1H), 8.09 (s, 1H), 7.72 (s, 1H), 7.67-7.65 (m, 1H), 7.62-7.59 (m, 1H), 7.41-7.37 (m, 2H), 6.52 (d, 1H), 5.54-5.44 (m, 2H), 3.99 (s, 3H). | 403.1 [M+H]⁺ | 99.5% |
| 936 | (CD₃OD) δ 8.38 (s, 1H), 8.09 (s, 1H), 7.72 (s, 1H), 7.67-7.65 (m, 1H), 7.62-7.60 (m, 1H), 7.41-7.37 (m, 2H), 6.52 (d, 1H), 5.54-5.44 (m, 2H), 3.99 (s, 3H). | 403.1 [M+H]⁺ | 98.6% |
| 937 | (CD₃OD) δ 8.77-8.76 (dt, 1H), 8.31-8.29 (dt, 1H), 8.09-8.05 (td, 1H), 7.74-7.72 (d, 1H), 7.68-7.61 (m, 3H), 7.41-7.35 (m, 2H), 6.55 (s, 1H), 5.59-5.48 (m, 2H). | 400.1 [M+H]⁺ | 95.1% |
| 938 | (CD₃OD) δ 8.78-8.76 (dt, 1H), 8.31-8.29 (m, 1H), 8.09-8.05 (td, 1H), 7.74-7.72 (d, 1H), 7.67-7.59 (m, 3H), 7.41-7.35 (m, 2H), 6.55 (s, 1H), 5.59-5.48 (q, 2H). | 400.1 [M+H]⁺ | 96.9% |
| 939 | (CD₃OD) δ 8.03 (s, 1H), 7.67-7.65 (m, 1H), 7.61-7.59 (m, 1H), 7.57-7.53 (m, 1H), 7.41-7.32 (m, 2H), 6.48 (s, 1H), 5.44-5.42 (d, 1H), 5.30-5.26 (d, 1H), 4.08 (s, 3H). | 336.1 [M+H]⁺ | 96.7% |
| 940 | (CD₃OD) δ 8.03 (s, 1H), 7.67-7.65 (m, 1H), 7.61-7.59 (m, 1H), 7.57-7.53 (m, 1H), 7.40-7.30 (m, 3H), 6.48 (s, 1H), 5.45-5.41 (d, 1H), 5.29-5.26 (d, 1H), 4.08 (s, 3H). | 336.1 [M+H]⁺ | 97.8% |
| 941 | (CD₃OD) δ 8.31 (s, 1H), 7.65-7.59 (m, 3H), 7.39-7.34 (m, 2H), 6.45 (s, 1H), 5.37 (s, 1H), 5.19 (s, 1H), 2.41 (s, 3H). | 336.1 [M+H]⁺ | >99% |
| 942 | (CD₃OD) δ 8.31 (s, 1H), 7.65-7.59 (m, 3H), 7.39-7.36 (m, 3H), 6.47 (s, 1H), 5.39-5.35 (d, 1H), 5.20-5.17 (d, 1H), 2.41 (s, 3H). | 336.1 [M+H]⁺ | 96.3% |
| 943 | (CD₃OD) δ 8.52-8.36 (2 s, 1H), 7.72 (br s, 1H), 7.68-7.50 (m, 2H), 7.42-7.34 (m, 2H), 6.53-6.49 (d, 1H), 5.22-5.19 (d, 1H), 5.03-5.00 (d, 1H). | 390.1 [M+H]⁺ | >99% |
| 944 | (CD₃OD) δ 8.52-8.36 (2 s, 1H), 7.72 (br s, 1H), 7.68-7.50 (m, 2H), 7.43-7.32 (m, 2H), 6.53-6.49 (d, 1H), 5.23-5.20 (d, 1H), 5.03-5.01 (d, 1H). | 390.1 [M+H]⁺ | 94.6% |
| 945 | (CD₃OD) δ 7.74 (s, 1H), 7.70-7.67 (m, 1H), 7.63-7.61 (m, 1H), 7.44-7.37 (m, 2H), 6.39 (s, 1H), 5.06-5.03 (d, 1H), 4.95-4.92 (d, 1H). | 363.0 [M+H]⁺ | 95.8% |
| 946 | (CD₃OD) δ 7.74 (s, 1H), 7.70-7.67 (m, 1H), 7.63-7.59 (m, 1H), 7.45-7.37 (m, 2H), 6.39 (s, 1H), 5.05-5.02 (d, 1H), 4.95-4.92 (d, 1H). | 363.1 [M+H]⁺ | 95.9% |
| 947 | (CD₃OD) δ 7.71 (s, 1H), 7.66-7.57 (m, 2H), 7.41-7.35 (m, 2H), 7.19 (s, 1H), 6.48 (s, 1H), 5.45-5.42 (d, 1H), 5.28-5.25 (d, 1H), 1.72 (s, 6H). | 430.1 [M+H]⁺ | >99% |
| 948 | (CD₃OD) δ 7.71 (s, 1H), 7.66-7.57 (m, 2H), 7.40-7.35 (m, 2H), 7.19 (s, 1H), 6.48 (s, 1H), 5.45-5.42 (d, 1H), 5.28-5.25 (d, 1H), 1.72 (s, 6H). | 430.1 [M+H]⁺ | 96.2% |
| 949 | (CD₃OD) δ 8.71 (s, 1H), 8.48 (s, 1H), 7.69 (s, 1H), 7.62-7.56 (m, 2H), 7.39-7.34 (m, 2H), 6.43 (s, 1H), 5.02-5.00 (d, 2H). | 373.1 [M+H]⁺ | 89.8% |
| 950 | (CD₃OD) δ 8.71 (s, 1H), 8.48 (s, 1H), 7.69 (s, 1H), 7.62-7.56 (m, 2H), 7.39-7.34 (m, 2H), 6.43 (s, 1H), 5.03-5.00 (m, 2H). | 373.1 [M+H]⁺ | 86.9% |
| 951 | (CD₃OD) δ 7.92-7.87 (m, 1H), 7.78 (s, 1H), 7.68-7.61 (dd, 2H), 7.57-7.49 (dd, 1H), 7.43-7.38 (m, 2H), 7.14 (s, 1H), 6.53 (s, 1H), 5.21-5.18 (d, 1H), 4.94-4.91 (d, 1H). | 371.0 [M+H]⁺ | 95.8% |
| 952 | (CD₃OD) δ 7.92-7.87 (m, 1H), 7.78 (s, 1H), 7.68-7.61 (dd, 2H), 7.57-7.49 (dd, 1H), 7.43-7.38 (m, 2H), 7.14 (s, 1H), 6.53 (s, 1H), 5.21-5.18 (d, 1H), 4.94-4.91 (d, 1H). | 371.1 [M+H]⁺ | 95.4% |
| 953 | (CD₃OD) δ 7.90 (s, 1H), 7.70-7.68 (d, 1H), 7.62-7.57 (m, 2H), 7.44-7.35 (m, 2H), 7.04 (s, 1H), 6.46 (s, 1H), 4.96-4.92 (d, 2H). | 389.0 [M+H]⁺ | 90.2% |
| 954 | (CD₃OD) δ 7.90 (s, 1H), 7.70-7.68 (d, 1H), 7.62-7.57 (m, 2H), 7.43-7.35 (m, 2H), 7.04 (s, 1H), 6.46 (s, 1H), 4.97-4.92 (d, 2H). | 389.1 [M+H]⁺ | 85.7% |

### Cell Based Phenylalanine Flux Assay

Cells expressing R408W PAH were made by transducing A375 cells with lentivirus encoding human PAH with the R408W mutation in pLVX-Puro, then selecting with puromycin until stable cell lines were generated. A375 R408W cells were seeded into 96 well plates in DMEM + 10% FBS at a density of 40,000 cells/well one hour prior to compound addition. Compounds were resuspended in DMSO, and 2-fold serial dilutions were performed to generate a 10-point dose curve. Compounds were added to plated cells in a total volume of 100 µl, and a final DMSO concentration of 0.5%. Each compound was tested in duplicate. Following compound addition, cells were placed in a 5% CO₂, 37°C tissue culture incubator for 24 hrs. After the incubation period, 20 µM sepiapterin and 800 µM 13C9,15N-Phenylalanine were added. After 4 hours, cell media was removed. An aliquot of 10 µl of cell media was combined with 200 µl of extraction buffer (80% acetonitrile/20% H₂O) for each well. Determination of ¹³C-Tyrosine concentration was assessed by liquid chromatography mass spectrometry.

Specific compounds disclosed herein were tested in the foregoing assay and they were determined to have an AC₅₀ according to the following scores: (A) less than or equal to 0.500 µM, (B) greater than 0.500 and less than 1.000 µM, (C) greater than or equal to 1.000 and less than 5.000 µM, (D) greater than or equal to 5.000 µM, (E) no fit, and (NT) not tested, as shown below. Where a compound was tested multiple times, the average value of the tests is reported.

| **Ex. #** | **AC₅₀** |
|---|---|
| 1 | B |
| 2 | D |
| 3 | A |
| 4 | D |
| 5 | A |
| 6 | C |
| 7 | B |
| 8 | C |
| 9 | A |
| 10 | C |
| 11 | B |
| 12 | C |
| 13 | A |
| 14 | C |
| 15 | B |
| 16 | C |
| 17 | A |
| 18 | C |
| 19 | C |
| 20 | D |
| 21 | A |
| 22 | C |
| 23 | A |
| 24 | C |
| 25 | A |
| 26 | B |
| 27 | A |
| 28 | C |
| 29 | C |
| 30 | D |
| 31 | B |
| 32 | C |
| 33 | B |
| 34 | C |
| 35 | B |
| 36 | C |
| 37 | C |
| 38 | C |
| 39 | B |
| 40 | C |
| 41 | A |
| 42 | C |
| 43 | A |
| 44 | C |
| 45 | A |
| 46 | A |
| 47 | C |
| 48 | D |
| 49 | A |
| 50 | A |
| 51 | A |
| 52 | B |
| 53 | A |
| 54 | B |
| 55 | B |
| 56 | C |
| 57 | A |
| 58 | A |
| 59 | B |
| 60 | C |
| 61 | B |
| 62 | C |
| 63 | A |
| 64 | A |
| 65 | C |
| 66 | D |
| 67 | C |
| 68 | C |
| 69 | B |
| 70 | C |
| 71 | B |
| 72 | D |
| 73 | B |
| 74 | D |
| 75 | B |
| 76 | C |
| 77 | B |
| 78 | C |
| 79 | B |
| 80 | C |
| 81 | B |
| 82 | D |
| 83 | C |
| 84 | A |
| 85 | A |
| 86 | C |
| 87 | C |
| 88 | B |
| 89 | C |
| 90 | B |
| 91 | B |
| 92 | D |
| 93 | B |
| 94 | C |
| 95 | B |
| 96 | C |
| 97 | A |
| 98 | C |
| 99 | B |
| 100 | C |
| 101 | B |
| 102 | C |
| 103 | C |
| 104 | D |
| 105 | C |
| 106 | C |
| 107 | A |
| 108 | C |
| 109 | C |
| 110 | B |
| 111 | B |
| 112 | C |
| 113 | C |
| 114 | C |
| 115 | B |
| 116 | C |
| 117 | C |
| 118 | A |
| 119 | B |
| 120 | C |
| 121 | C |
| 122 | A |
| 123 | C |
| 124 | B |
| 125 | C |
| 126 | B |
| 127 | B |
| 128 | C |
| 129 | B |
| 130 | D |
| 131 | B |
| 132 | C |
| 133 | B |
| 134 | D |
| 135 | C |
| 136 | D |
| 137 | A |
| 138 | A |
| 139 | B |
| 140 | D |
| 141 | B |
| 142 | C |
| 143 | A |
| 144 | A |
| 145 | A |
| 146 | B |
| 147 | A |
| 148 | A |
| 149 | A |
| 150 | A |
| 151 | A |
| 152 | C |
| 153 | B |
| 154 | C |
| 155 | A |
| 156 | C |
| 157 | B |
| 158 | C |
| 159 | A |
| 160 | B |
| 161 | A |
| 162 | C |
| 163 | A |
| 164 | C |
| 165 | A |
| 166 | D |
| 167 | A |
| 168 | C |
| 169 | A |
| 170 | B |
| 171 | A |
| 172 | A |
| 173 | A |
| 174 | A |
| 175 | B |
| 176 | C |
| 177 | A |
| 178 | D |
| 179 | B |
| 180 | C |
| 181 | A |
| 182 | C |
| 183 | B |
| 184 | C |
| 185 | B |
| 186 | C |
| 187 | A |
| 188 | C |
| 189 | B |
| 190 | C |
| 191 | B |
| 192 | C |
| 193 | A |
| 194 | A |
| 195 | A |
| 196 | C |
| 197 | B |
| 198 | C |
| 199 | A |
| 200 | C |
| 201 | B |
| 202 | C |
| 203 | B |
| 204 | C |
| 205 | A |
| 206 | C |
| 207 | A |
| 208 | C |
| 209 | D |
| 210 | B |
| 211 | B |
| 212 | C |
| 213 | B |
| 214 | A |
| 215 | B |
| 216 | C |
| 217 | A |
| 218 | C |
| 219 | A |
| 220 | D |
| 221 | B |
| 222 | C |
| 223 | C |
| 224 | C |
| 225 | C |
| 226 | A |
| 227 | A |
| 228 | C |
| 229 | A |
| 230 | C |
| 231 | B |
| 232 | C |
| 233 | C |
| 234 | C |
| 235 | C |
| 236 | B |
| 237 | C |
| 238 | C |
| 239 | B |
| 240 | C |
| 241 | C |
| 242 | D |
| 243 | A |
| 244 | B |
| 245 | A |
| 246 | A |
| 247 | B |
| 248 | C |
| 249 | B |
| 250 | D |
| 251 | C |
| 252 | A |
| 253 | A |
| 254 | A |
| 255 | A |
| 256 | C |
| 257 | A |
| 258 | C |
| 259 | B |
| 260 | D |
| 261 | B |
| 262 | B |
| 263 | A |
| 264 | B |
| 265 | B |
| 266 | D |
| 267 | C |
| 268 | B |
| 269 | B |
| 270 | D |
| 271 | C |
| 272 | A |
| 273 | C |
| 274 | B |
| 275 | B |
| 276 | D |
| 277 | B |
| 278 | C |
| 279 | B |
| 280 | C |
| 281 | B |
| 282 | C |
| 283 | A |
| 284 | C |
| 285 | B |
| 286 | C |
| 287 | B |
| 288 | C |
| 289 | B |
| 290 | C |
| 291 | A |
| 292 | A |
| 293 | A |
| 294 | C |
| 295 | B |
| 296 | C |
| 297 | A |
| 298 | C |
| 299 | A |
| 300 | C |
| 301 | A |
| 302 | C |
| 303 | A |
| 304 | C |
| 305 | A |
| 306 | C |
| 307 | A |
| 308 | A |
| 309 | A |
| 310 | B |
| 311 | A |
| 312 | B |
| 313 | A |
| 314 | B |
| 315 | A |
| 316 | C |
| 317 | A |
| 318 | B |
| 319 | B |
| 320 | D |
| 321 | B |
| 322 | C |
| 323 | A |
| 324 | C |
| 325 | A |
| 326 | C |
| 327 | A |
| 328 | A |
| 329 | A |
| 330 | A |
| 331 | B |
| 332 | C |
| 333 | A |
| 334 | C |
| 335 | A |
| 336 | B |
| 337 | A |
| 338 | B |
| 339 | B |
| 340 | C |
| 341 | A |
| 342 | B |
| 343 | C |
| 344 | C |
| 345 | A |
| 346 | C |
| 347 | B |
| 348 | C |
| 349 | B |
| 350 | A |
| 351 | A |
| 352 | B |
| 353 | A |
| 354 | A |
| 355 | B |
| 356 | C |
| 357 | B |
| 358 | C |
| 359 | B |
| 360 | C |
| 361 | A |
| 362 | C |
| 363 | A |
| 364 | C |
| 365 | B |
| 366 | C |
| 367 | C |
| 368 | B |
| 369 | A |
| 370 | C |
| 371 | B |
| 372 | C |
| 373 | A |
| 374 | A |
| 375 | B |
| 376 | C |
| 377 | B |
| 378 | A |
| 379 | A |
| 380 | B |
| 381 | C |
| 382 | A |
| 383 | A |
| 384 | C |
| 385 | A |
| 386 | D |
| 387 | B |
| 388 | D |
| 389 | A |
| 390 | C |
| 391 | C |
| 392 | A |
| 393 | A |
| 394 | B |
| 395 | B |
| 396 | C |
| 397 | B |
| 398 | D |
| 399 | B |
| 400 | C |
| 401 | A |
| 402 | C |
| 403 | B |
| 404 | C |
| 405 | B |
| 406 | A |
| 407 | A |
| 408 | B |
| 409 | B |
| 410 | C |
| 411 | A |
| 412 | B |
| 413 | B |
| 414 | C |
| 415 | B |
| 416 | B |
| 417 | A |
| 418 | A |
| 419 | B |
| 420 | A |
| 421 | A |
| 422 | B |
| 423 | A |
| 424 | A |
| 425 | A |
| 426 | A |
| 427 | A |
| 428 | C |
| 429 | A |
| 430 | B |
| 431 | A |
| 432 | A |
| 433 | A |
| 434 | A |
| 435 | A |
| 436 | B |
| 437 | A |
| 438 | A |
| 439 | A |
| 440 | C |
| 441 | C |
| 442 | B |
| 443 | B |
| 444 | A |
| 445 | A |
| 446 | A |
| 447 | A |
| 448 | C |
| 449 | A |
| 450 | C |
| 451 | A |
| 452 | C |
| 453 | A |
| 454 | C |
| 455 | A |
| 456 | C |
| 457 | A |
| 458 | A |
| 459 | A |
| 460 | A |
| 461 | A |
| 462 | A |
| 463 | A |
| 464 | B |
| 465 | A |
| 466 | B |
| 467 | A |
| 468 | B |
| 469 | C |
| 470 | A |
| 471 | B |
| 472 | C |
| 473 | B |
| 474 | C |
| 475 | A |
| 476 | C |
| 477 | A |
| 478 | D |
| 479 | B |
| 480 | A |
| 481 | B |
| 482 | A |
| 483 | B |
| 484 | C |
| 485 | C |
| 486 | B |
| 487 | A |
| 488 | C |
| 489 | B |
| 490 | C |
| 491 | B |
| 492 | C |
| 493 | A |
| 494 | A |
| 495 | C |
| 496 | C |
| 497 | C |
| 498 | C |
| 499 | A |
| 500 | A |
| 501 | A |
| 502 | A |
| 503 | D |
| 504 | B |
| 505 | B |
| 506 | C |
| 507 | B |
| 508 | D |
| 509 | A |
| 510 | C |
| 511 | B |
| 512 | C |
| 513 | B |
| 514 | C |
| 515 | B |
| 516 | C |
| 517 | C |
| 518 | A |
| 519 | B |
| 520 | A |
| 521 | C |
| 522 | B |
| 523 | B |
| 524 | C |
| 525 | C |
| 526 | A |
| 527 | A |
| 528 | C |
| 529 | A |
| 530 | B |
| 531 | A |
| 532 | A |
| 533 | A |
| 534 | A |
| 535 | A |
| 536 | A |
| 537 | A |
| 538 | C |
| 539 | A |
| 540 | C |
| 541 | A |
| 542 | C |
| 543 | A |
| 544 | C |
| 545 | C |
| 546 | B |
| 547 | B |
| 548 | C |
| 549 | B |
| 550 | C |
| 551 | B |
| 552 | C |
| 553 | B |
| 554 | C |
| 555 | B |
| 556 | C |
| 557 | A |
| 558 | C |
| 559 | A |
| 560 | A |
| 561 | B |
| 562 | C |
| 563 | A |
| 564 | C |
| 565 | B |
| 566 | C |
| 567 | A |
| 568 | C |
| 569 | A |
| 570 | C |
| 571 | A |
| 572 | A |
| 573 | A |
| 574 | A |
| 575 | C |
| 576 | C |
| 577 | B |
| 578 | C |
| 579 | A |
| 580 | B |
| 581 | A |
| 582 | C |
| 583 | A |
| 584 | C |
| 585 | A |
| 586 | C |
| 587 | A |
| 588 | A |
| 589 | A |
| 590 | A |
| 591 | A |
| 592 | B |
| 593 | C |
| 594 | A |
| 595 | C |
| 596 | A |
| 597 | A |
| 598 | C |
| 599 | A |
| 600 | C |
| 601 | B |
| 602 | B |
| 603 | A |
| 604 | C |
| 605 | A |
| 606 | A |
| 607 | A |
| 608 | A |
| 609 | B |
| 610 | C |
| 611 | B |
| 612 | C |
| 613 | A |
| 614 | C |
| 615 | A |
| 616 | A |
| 617 | A |
| 618 | B |
| 619 | C |
| 620 | A |
| 621 | B |
| 622 | C |
| 623 | C |
| 624 | A |
| 625 | C |
| 626 | A |
| 627 | C |
| 628 | C |
| 629 | B |
| 630 | C |
| 631 | A |
| 632 | A |
| 633 | A |
| 634 | A |
| 635 | B |
| 636 | A |
| 637 | A |
| 638 | A |
| 639 | A |
| 640 | C |
| 641 | A |
| 642 | C |
| 643 | A |
| 644 | A |
| 645 | C |
| 646 | C |
| 647 | B |
| 648 | A |
| 649 | A |
| 650 | B |
| 651 | A |
| 652 | C |
| 653 | A |
| 654 | C |
| 655 | A |
| 656 | C |
| 657 | A |
| 658 | A |
| 659 | A |
| 660 | C |
| 661 | C |
| 662 | C |
| 663 | A |
| 664 | A |
| 665 | A |
| 666 | A |
| 667 | A |
| 668 | A |
| 669 | A |
| 670 | A |
| 671 | C |
| 672 | C |
| 673 | A |
| 674 | C |
| 675 | A |
| 676 | A |
| 677 | A |
| 678 | C |
| 679 | B |
| 680 | C |
| 681 | B |
| 682 | D |
| 683 | C |
| 684 | C |
| 685 | B |
| 686 | C |
| 687 | B |
| 688 | C |
| 689 | A |
| 690 | C |
| 691 | B |
| 692 | A |
| 693 | A |
| 694 | A |
| 695 | A |
| 696 | C |
| 697 | B |
| 698 | C |
| 699 | C |
| 700 | D |
| 701 | A |
| 702 | D |
| 703 | B |
| 704 | C |
| 705 | A |
| 706 | B |
| 707 | A |
| 708 | B |
| 709 | E |
| 710 | A |
| 711 | C |
| 712 | C |
| 713 | B |
| 714 | C |
| 715 | A |
| 716 | C |
| 717 | B |
| 718 | A |
| 719 | A |
| 720 | B |
| 721 | C |
| 722 | A |
| 723 | A |
| 724 | C |
| 725 | A |
| 726 | E |
| 727 | B |
| 728 | D |
| 729 | B |
| 730 | E |
| 731 | A |
| 732 | D |
| 733 | A |
| 734 | C |
| 735 | A |
| 736 | C |
| 737 | A |
| 738 | C |
| 739 | B |
| 740 | A |
| 741 | A |
| 742 | A |
| 743 | A |
| 744 | A |
| 745 | B |
| 746 | C |
| 747 | A |
| 748 | C |
| 749 | A |
| 750 | C |
| 751 | C |
| 752 | A |
| 753 | A |
| 754 | C |
| 755 | A |
| 756 | C |
| 757 | C |
| 758 | A |
| 759 | C |
| 760 | A |
| 761 | A |
| 762 | C |
| 763 | C |
| 764 | A |
| 765 | A |
| 766 | E |
| 767 | A |
| 768 | E |
| 769 | C |
| 770 | C |
| 771 | A |
| 772 | B |
| 773 | A |
| 774 | A |
| 775 | B |
| 776 | C |
| 777 | A |
| 778 | B |
| 779 | C |
| 780 | C |
| 781 | A |
| 782 | B |
| 783 | A |
| 784 | A |
| 785 | D |
| 786 | B |
| 787 | A |
| 788 | C |
| 789 | C |
| 790 | B |
| 791 | B |
| 792 | C |
| 793 | A |
| 794 | C |
| 795 | A |
| 796 | B |
| 797 | A |
| 798 | B |
| 799 | B |
| 800 | A |
| 801 | A |
| 802 | B |
| 803 | A |
| 804 | A |
| 805 | C |
| 806 | C |
| 807 | A |
| 808 | C |
| 809 | A |
| 810 | A |
| 811 | A |
| 812 | B |
| 813 | A |
| 814 | A |
| 815 | B |
| 816 | A |
| 817 | B |
| 818 | C |
| 819 | B |
| 820 | D |
| 821 | A |
| 822 | C |
| 823 | C |
| 824 | A |
| 825 | C |
| 826 | D |
| 827 | C |
| 828 | D |
| 829 | C |
| 830 | B |
| 831 | B |
| 832 | A |
| 833 | A |
| 834 | B |
| 835 | A |
| 836 | B |
| 837 | B |
| 838 | A |
| 839 | B |
| 840 | A |
| 841 | A |
| 842 | C |
| 843 | A |
| 844 | B |
| 845 | C |
| 846 | A |
| 847 | A |
| 848 | B |
| 849 | A |
| 850 | C |
| 851 | B |
| 852 | A |
| 853 | B |
| 854 | A |
| 855 | A |
| 856 | B |
| 857 | C |
| 858 | A |
| 859 | C |
| 860 | B |
| 861 | B |
| 862 | A |
| 863 | A |
| 864 | A |
| 865 | A |
| 866 | B |
| 867 | B |
| 868 | A |
| 869 | A |
| 870 | C |
| 871 | C |
| 872 | B |
| 873 | A |
| 874 | B |
| 875 | A |
| 876 | A |
| 877 | C |
| 878 | D |
| 879 | C |
| 880 | C |
| 881 | C |
| 882 | C |
| 883 | B |
| 884 | A |
| 885 | C |
| 886 | C |
| 887 | B |
| 888 | E |
| 889 | E |
| 890 | A |
| 891 | C |
| 892 | A |
| 893 | C |
| 894 | C |
| 895 | C |
| 896 | D |
| 897 | E |
| 898 | A |
| 899 | A |
| 900 | B |
| 901 | B |
| 902 | C |
| 903 | C |
| 904 | D |
| 905 | A |
| 906 | D |
| 907 | C |
| 908 | C |
| 909 | C |
| 910 | D |
| 911 | A |
| 912 | B |
| 913 | B |
| 914 | E |
| 915 | A |
| 916 | E |
| 917 | C |
| 918 | E |
| 919 | A |
| 920 | E |
| 921 | C |
| 922 | C |
| 923 | E |
| 924 | C |
| 925 | D |
| 926 | E |
| 927 | D |
| 928 | E |
| 929 | C |
| 930 | E |
| 931 | C |
| 932 | C |
| 933 | E |
| 934 | E |
| 935 | D |
| 936 | E |
| 937 | C |
| 938 | C |
| 939 | C |
| 940 | B |
| 941 | C |
| 942 | C |
| 943 | C |
| 944 | C |
| 945 | D |
| 946 | C |
| 947 | D |
| 948 | E |
| 949 | C |
| 950 | E |
| 951 | C |
| 952 | E |
| 953 | C |
| 954 | E |

Additionally, the following compounds were tested with an AC₅₀ of C, D, or E:

| **Compound(s)** | **AC₅₀** |
|---|---|
| 4S and 4R enantiomers of 2-{3-[4-(5-fluoro-1,3-benzoxazol-2-yl)-1H,4H,5H,6H,7H-imidazo[4,5-c]pyridine-5-carbonyl]-1,2-oxazol-5-yl}propan-2-ol | D, D |
| 4S and 4R enantiomers of 1-[4-(6-bromo-1,3-benzoxazol-2-yl)-1H,4H,5H,6H,7H-imidazo[4,5-c]pyridin-5-yl]-3-(1,3-thiazol-2-yl)propan-1-one | C, D |
| 4S and 4R enantiomers of 2-[5-(5-chloro-1,3-thiazole-2-carbonyl)-1H,4H,5H,6H,7H-imidazo[4,5-c]pyridin-4-yl]-5-fluoro-1,3-benzoxazole | C, C |
| 4S and 4R enantiomers of 6-bromo-2-[5-(1,3-thiazole-2-carbonyl)-1H,4H,5H,6H,7H-imidazo[4,5-c]pyridin-4-yl]-1,3-benzoxazole | C, D |
| 4S and 4R enantiomers of 6-methyl-2-[5-(1,3-thiazole-5-carbonyl)-1H,4H,5H,6H,7H-imidazo[4,5-c]pyridin-4-yl]-1,3-benzoxazole | C, D |
| 4S and 4R enantiomers of 6-bromo-2-[5-(1,3-thiazole-5-carbonyl)-1H,4H,5H,6H,7H-imidazo[4,5-c]pyridin-4-yl]-1,3-benzoxazole | D, D |
| 4S and 4R enantiomers of 6-bromo-2-[5-[2-(1-methyl-1H-pyrazol-4-yl)-1,3-thiazole-5-carbonyl]-1H,4H,5H,6H,7H-imidazo[4,5-c]pyridin-4-yl]-1,3-benzoxazole | C, C |
| 4S and 4R enantiomers of 6-bromo-2-[5-(1,3-oxazole-5-carbonyl)-1H,4H,5H,6H,7H-imidazo[4,5-c]pyridin-4-yl]-1,3-benzoxazole | D, D |
| 4S and 4R enantiomers of 6-bromo-2-[5-[2-(pyridin-2-yl)-1,3-thiazole-5-carbonyl]-1H,4H,5H,6H,7H-imidazo[4,5-c]pyridin-4-yl]-1,3-benzoxazole | C, C |
| 4S and 4R enantiomers of 5-fluoro-2-[5-(1,3-thiazole-5-carbonyl)-1H,4H,5H,6H,7H-imidazo[4,5-c]pyridin-4-yl]-1,3-benzoxazole | C, D |
| 4S and 4R enantiomers of 5-fluoro-2-[5-(1,3-oxazole-5-carbonyl)-1H,4H,5H,6H,7H-imidazo[4,5-c]pyridin-4-yl]-1,3-benzoxazole | C, D |
| 4S and 4R enantiomers of 5-fluoro-2-[5-[2-(1-methyl-1H-pyrazol-4-yl)-1,3-oxazole-5-carbonyl]-1H,4H,5H,6H,7H-imidazo[4,5-c]pyridin-4-yl]-1,3-benzoxazole | C, D |
| 4S and 4R enantiomers of 5-fluoro-2-[5-(4-methyl-1,3-oxazole-5-carbonyl)-1H,4H,5H,6H,7H-imidazo[4,5-c]pyridin-4-yl]-1,3-benzoxazole | C, D |
| 4S and 4R enantiomers of 2-{5-[4-(5-fluoro-1,3-benzoxazol-2-yl)-1H,4H,5H,6H,7H-imidazo[4,5-c]pyridine-5-carbonyl]-1,3-oxazol-2-yl}propan-2-ol | C, C |
| 4S and 4R enantiomers of 2-{5-[4-(5-fluoro-1,3-benzoxazol-2-yl)-1H,4H,5H,6H,7H-imidazo[4,5-c]pyridine-5-carbonyl]-1,3-thiazol-2-yl}propan-2-ol | C, C |
| 4S and 4R enantiomers of 5-fluoro-2-[5-[4-methyl-2-(pyridin-2-yl)-1,3-thiazole-5-carbonyl]-1H,4H,5H,6H,7H-imidazo[4,5-c]pyridin-4-yl]-1,3-benzoxazole | D, D |
| 4S and 4R enantiomers of 5-fluoro-2-[5-[4-methyl-2-(1-methyl-1H-pyrazol-4-yl)-1,3-thiazole-5-carbonyl]-1H,4H,5H,6H,7H-imidazo[4,5-c]pyridin-4-yl]-1,3-benzoxazole | C, D |
| 4S and 4R enantiomers of 5-fluoro-2-[5-[4-methyl-2-(1-methyl-1H-pyrazol-4-yl)-1,3-oxazole-5-carbonyl]-1H,4H,5H,6H,7H-imidazo[4,5-c]pyridin-4-yl]-1,3-benzoxazole | C, D |
| 4S and 4R enantiomers of 2-{5-[4-(5-fluoro-1,3-benzoxazol-2-yl)-1H,4H,5H,6H,7H-imidazo[4,5-c]pyridine-5-carbonyl]-4-methyl-1,3-oxazol-2-yl}propan-2-ol | C, D |
| 4S and 4R enantiomers of 5-fluoro-2-[5-(5-methyl-1,3,4-oxadiazole-2-carbonyl)-1H,4H,5H,6H,7H-imidazo[4,5-c]pyridin-4-yl]-1,3-benzoxazole | C, D |
| 4S and 4R enantiomers of ({3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}methyl)dimethylamine | C, D |
| 4S and 4R enantiomers of 1-[4-(5-fluoro-1,3-benzoxazol-2-yl)-1H,4H,5H,6H,7H-imidazo[4,5-c]pyridin-5-yl]-2-{pyrazolo[1,5-a]pyridin-3-yl}ethan-1-one | C, D |
| 4S and 4R enantiomers of 2-[5-cyclopropanecarbonyl-1H,4H,5H,6H,7H-imidazo[4,5-c]pyridin-4-yl]-5-fluoro-1,3-benzoxazole | C, D |
| 4S and 4R enantiomers of 5-fluoro-2-[5-[4-(trifluoromethyl)-1,3-oxazole-2-carbonyl]-3H,4H,5H,6H,7H-imidazo[4,5-c]pyridin-4-yl]-1,3-benzoxazole | C, C |
| 4S and 4R enantiomers of 6-methyl-2-[5-(5-methyl-1,3,4-oxadiazole-2-carbonyl)-1H,4H,5H,6H,7H-imidazo[4,5-c]pyridin-4-yl]-1,3-benzoxazole | D, D |
| 4S and 4R enantiomers of 6-methyl-2-[5-(1,3-oxazole-5-carbonyl)-1H,4H,5H,6H,7H-imidazo[4,5-c]pyridin-4-yl]-1,3-benzoxazole | C, D |
| 4S and 4R enantiomers of dimethyl({3-[4-(6-methyl-1,3-benzoxazol-2-yl)-1H,4H,5H,6H,7H-imidazo[4,5-c]pyridine-5-carbonyl]pyrazolo[1,5-a]pyridin-6-yl}methyl)amine | C, D |
| 4S and 4R enantiomers of 5-methyl-2-[5-(5-methyl-1,3,4-oxadiazole-2-carbonyl)-1H,4H,5H,6H,7H-imidazo[4,5-c]pyridin-4-yl]-1,3-benzoxazole | C, D |
| 4S and 4R enantiomers of 5-methyl-2-[5-(1,3-oxazole-5-carbonyl)-1H,4H,5H,6H,7H-imidazo[4,5-c]pyridin-4-yl]-1,3-benzoxazole | C, D |
| 4S and 4R enantiomers of 1-[4-(6-methyl-1,3-benzoxazol-2-yl)-1H,4H,5H,6H,7H-imidazo[4,5-c]pyridin-5-yl]-2-(1,3,4-thiadiazol-2-yl)ethan-1-one | C, E |
| 4S and 4R enantiomers of dimethyl({3-[4-(5-methyl-1,3-benzoxazol-2-yl)-1H,4H,5H,6H,7H-imidazo[4,5-c]pyridine-5-carbonyl]pyrazolo[1,5-a]pyridin-6-yl}methyl)amine | C, C |
| 4S and 4R enantiomers of 2-{5-[4-(5-fluoro-1,3-benzoxazol-2-yl)-1H,4H,5H,6H,7H-imidazo[4,5-c]pyridine-5-carbonyl]-1,3,4-oxadiazol-2-yl}propan-2-ol | C, D |
| 4'S and 4'R enantiomers of 4'-(5-fluoro-1,3-benzoxazol-2-yl)-5'-(4-methyl-1,3-oxazole-5-carbonyl)-1',4',5',6'-tetrahydrospiro[cyclopropane-1,7'-imidazo[4,5-c]pyridine] | C, D |
| 4S and 4R enantiomers of 2-[5-[(1R)-2,2-difluorocyclopropanecarbonyl]-1H,4H,5H,6H,7H-imidazo[4,5-c]pyridin-4-yl]-5-fluoro-1,3-benzoxazole | C, D |
| 4S and 4R enantiomers of 5-fluoro-2-[5-[5-(morpholin-4-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 5-fluoro-2-[5-[5-(4-fluoropiperidin-1-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of [2-({3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}oxy)ethyl]dimethylamine | C, D |
| 4S and 4R enantiomers of 4-(2-{3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}ethyl)piperazin-2-one | C, E |
| 4S and 4R enantiomers of (4-(6-methylbenzo[d]oxazol-2-yl)-6,7-dihydro-1H-imidazo[4,5-c]pyridin-5(4H)-yl)(2-(pyridin-2-yl)-4-(trifluoromethyl)oxazol-5-yl)methanone | E, E |
| 4S and 4R enantiomers of 2-[5-[6-(methoxymethyl)pyrazolo[1,5-a]pyridine-3-carbonyl]-1H,4H,5H,6H,7H-imidazo[4,5-c]pyridin-4-yl]-6-methyl-1,3-benzoxazole | C, D |
| 4S and 4R enantiomers of 3-[4-(5-fluoro-1,3-benzoxazol-2-yl)-1H,4H,5H,6H,7H-imidazo[4,5-c]pyridine-5-carbonyl]-N-(2-methoxyethyl)pyrazolo[1,5-a]pyridin-6-amine | C, D |
| 4S and 4R enantiomers of N-cyclopropyl-5-[4-(5-fluoro-1,3-benzoxazol-2-yl)-1H,4H,5H,6H,7H-imidazo[4,5-c]pyridine-5-carbonyl]-1,3,4-oxadiazol-2-amine | C, C |
| 4S and 4R enantiomers of (2-(2-hydroxypropan-2-yl)-4-(trifluoromethyl)oxazol-5-yl)(4-(6-methylbenzo[d]oxazol-2-yl)-6,7-dihydro-1H-imidazo[4,5-c]pyridin-5(4H)-yl)methanone | E, E |
| 4S and 4R enantiomers of 5-fluoro-2-[5-{6-[(morpholin-4-yl)methyl]pyrazolo[1,5-a]pyridine-3-carbonyl}-1H,4H,5H,6H,7H-imidazo[4,5-c]pyridin-4-yl]-1,3-benzoxazole | C, D |
| 4S and 4R enantiomers of 5-fluoro-2-[5-{6-[2-(1H-pyrazol-1-yl)ethoxy]pyrazolo[1,5-a]pyridine-3-carbonyl}-1H,4H,5H,6H,7H-imidazo[4,5-c]pyridin-4-yl]-1,3-benzoxazole | C, D |
| 4S and 4R enantiomers of (3-{3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}propyl)dimethylamine | D, D |
| 4S and 4R enantiomers of 5-fluoro-2-[5-[5-(3-fluoroazetidin-1-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 5-fluoro-2-[5-[2-(4-fluoropiperidin-1-yl)-1,3-oxazole-5-carbonyl]-1H,4H,5H,6H,7H-imidazo[4,5-c]pyridin-4-yl]-1,3-benzoxazole | C, D |
| 4S and 4R enantiomers of 5-[4-(5-fluoro-1,3-benzoxazol-2-yl)-1H,4H,5H,6H,7H-imidazo[4,5-c]pyridine-5-carbonyl]-2-(4-fluoropiperidin-1-yl)-1,3-oxazole-4-carbonitrile | D, E |
| 4S and 4R enantiomers of (4-(5-methylbenzo[d]oxazol-2-yl)-6,7-dihydro-1H-imidazo[4,5-c]pyridin-5(4H)-yl)(2-(pyridin-2-yl)-4-(trifluoromethyl)oxazol-5-yl)methanone | E, E |
| 4S and 4R enantiomers of dimethyl(2-{3-[4-(6-methyl-1,3-benzoxazol-2-yl)-1H,4H,5H,6H,7H-imidazo[4,5-c]pyridine-5-carbonyl]pyrazolo[1,5-a]pyridin-6-yl}ethyl)amine | C, D |
| 4S and 4R enantiomers of N-{3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}methanesulfonamide | E, E |
| 4S and 4R enantiomers of 5-fluoro-2-[5-{6-[2-(3-fluoroazetidin-1-yl)ethyl]pyrazolo[1,5-a]pyridine-3-carbonyl}-1H,4H,5H,6H,7H-imidazo[4,5-c]pyridin-4-yl]-1,3-benzoxazole | C, D |
| 4S and 4R enantiomers of 2,2-difluoro-1-[4-(5-fluoro-1,3-benzoxazol-2-yl)-1H,4H,5H,6H,7H-imidazo[4,5-c]pyridin-5-yl]-2-(1-hydroxycyclobutyl)ethan-1-one | C, C |
| 4S and 4R enantiomers of 5-[4-(5-fluoro-1,3-benzoxazol-2-yl)-1H,4H,5H,6H,7H-imidazo[4,5-c]pyridine-5-carbonyl]-1,3-oxazole-4-carbonitrile | D, D |
| 4S and 4R enantiomers of 5-[4-(5-fluoro-1,3-benzoxazol-2-yl)-1H,4H,5H,6H,7H-imidazo[4,5-c]pyridine-5-carbonyl]-N,N-dimethyl-1,3,4-oxadiazol-2-amine | C, C |
| 4S and 4R enantiomers of 5-fluoro-2-[5-[5-(4-methylpiperazin-1-yl)-1,3,4-oxadiazole-2-carbonyl]-1H,4H,5H,6H,7H-imidazo[4,5-c]pyridin-4-yl]-1,3-benzoxazole | C, D |
| 4S and 4R enantiomers of N-[2-(dimethylamino)ethyl]-3-[4-(5-fluoro-1,3-benzoxazol-2-yl)-1H,4H,5H,6H,7H-imidazo[4,5-c]pyridine-5-carbonyl]pyrazolo[1,5-a]pyridin-6-amine | D, E |
| 4S and 4R enantiomers of methyl N-{3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}carbamate | C, D |
| 4S and 4R enantiomers of 5-fluoro-2-[5-{6-[(3-fluoroazetidin-1-yl)methyl]pyrazolo[1,5-a]pyridine-3-carbonyl}-1H,4H,5H,6H,7H-imidazo[4,5-c]pyridin-4-yl]-1,3-benzoxazole | C, D |
| 4S and 4R enantiomers of 5-fluoro-2-[5-[6-(3-methoxypropyl)pyrazolo[1,5-a]pyridine-3-carbonyl]-1H,4H,5H,6H,7H-imidazo[4,5-c]pyridin-4-yl]-1,3-benzoxazole | C, D |
| 4S and 4R enantiomers of 5-[4-(6-methyl-1,3-benzoxazol-2-yl)-1H,4H,5H,6H,7H-imidazo[4,5-c]pyridine-5-carbonyl]-1,3-oxazole-4-carbonitrile | C, D |
| 4S and 4R enantiomers of 5-fluoro-2-[5-[2-(4-fluoropiperidin-1-yl)-4-methyl-1,3-oxazole-5-carbonyl]-1H,4H,5H,6H,7H-imidazo[4,5-c]pyridin-4-yl]-1,3-benzoxazole | C, D |
| 4S and 4R enantiomers of ({5-[4-(5-fluoro-1,3-benzoxazol-2-yl)-1H,4H,5H,6H,7H-imidazo[4,5-c]pyridine-5-carbonyl]-4-methyl-1,3-oxazol-2-yl}methyl)dimethylamine | C, D |
| 4S and 4R enantiomers of 5-fluoro-2-[5-[6-(2-methoxyethoxy)pyrazolo[1,5-a]pyridine-3-carbonyl]-1H,4H,5H,6H,7H-imidazo[4,5-c]pyridin-4-yl]-1,3-benzoxazole | C, D |
| 4S and 4R enantiomers of [3-({3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}oxy)propyl]dimethylamine | C, D |
| 4S and 4R enantiomers of 5-fluoro-2-[5-[2-(methoxymethyl)-1,3-thiazole-5-carbonyl]-1H,4H,5H,6H,7H-imidazo[4,5-c]pyridin-4-yl]-1,3-benzoxazole | C, C |
| 4S and 4R enantiomers of 2-[5-{6-[(3,3-difluoropyrrolidin-1-yl)methyl]pyrazolo[1,5-a]pyridine-3-carbonyl}-1H,4H,5H,6H,7H-imidazo[4,5-c]pyridin-4-yl]-5-fluoro-1 ,3-benzoxazole | C, D |
| 4S and 4R enantiomers of 1-[2-({3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}oxy)ethyl]pyrrolidin-2-one | D, E |
| 4S and 4R enantiomers of 4-({3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}methyl)piperazin-2-one | D, E |
| 4S and 4R enantiomers of 5-fluoro-2-[5-{6-[2-(morpholin-4-yl)ethyl]pyrazolo[1,5-a]pyridine-3-carbonyl}-1H,4H,5H,6H,7H-imidazo[4,5-c]pyridin-4-yl]-1,3-benzoxazole | C, D |
| 4S and 4R enantiomers of 5-fluoro-2-[5-[2-(methoxymethyl)-1,3-oxazole-5-carbonyl]-1H,4H,5H,6H,7H-imidazo[4,5-c]pyridin-4-yl]-1,3-benzoxazole | C, D |
| 4S and 4R enantiomers of ({5-[4-(5-fluoro-1,3-benzoxazol-2-yl)-1H,4H,5H,6H,7H-imidazo[4,5-c]pyridine-5-carbonyl]-1,3-oxazol-2-yl}methyl)dimethylamine | D, D |
| 4S and 4R enantiomers of 5-fluoro-2-[5-[6-(2-methoxyethyl)pyrazolo[1,5-a]pyridine-3-carbonyl]-1H,4H,5H,6H,7H-imidazo[4,5-c]pyridin-4-yl]-1,3-benzoxazole | C, C |
| 4S and 4R enantiomers of 5-fluoro-2-[5-{6-fluoropyrazolo[1,5-a]pyridine-3-carbonyl}-1H,4H,5H,6H,7H-imidazo[4,5-c]pyridin-4-yl]-1,3-benzoxazole | D, D |
| 4S and 4R enantiomers of 2-[5-{6-chloropyrazolo[1,5-a]pyridine-3-carbonyl}-1H,4H,5H,6H,7H-imidazo[4,5-c]pyridin-4-yl]-5-fluoro-1,3-benzoxazole | C, C |
| 4S and 4R enantiomers of 5-[4-(5-fluoro-1,3-benzoxazol-2-yl)-1H,4H,5H,6H,7H-imidazo[4,5-c]pyridine-5-carbonyl]-N-(2-methoxyethyl)-1,3,4-oxadiazol-2-amine | D, D |
| 4S and 4R enantiomers of 2-[5-{5-[1-(difluoromethyl)-1H-pyrazol-4-yl]-1,3,4-oxadiazole-2-carbonyl}-1H,4H,5H,6H,7H-imidazo[4,5-c]pyridin-4-yl]-5-fluoro-1,3-benzoxazole | C, C |
| 4S and 4R enantiomers of 5-fluoro-2-[5-{5-[1-(2,2,2-trifluoroethyl)-1H-pyrazol-4-yl]-1,3,4-oxadiazole-2-carbonyl}-1H,4H,5H,6H,7H-imidazo[4,5-c]pyridin-4-yl]-1,3-benzoxazole | C, C |
| 4S and 4R enantiomers of 2-[5-[3-(difluoromethyl)-1-methyl-1H-pyrazole-5-carbonyl]-1H,4H,5H,6H,7H-imidazo[4,5-c]pyridin-4-yl]-5-fluoro-1,3-benzoxazole | C, C |
| 4S and 4R enantiomers of (4-(5-fluorobenzo[d]oxazol-2-yl)-6,7-dihydro-1H-imidazo[4,5-c]pyridin-5(4H)-yl)(6-(2-methoxyethyl)-2-(trifluoromethyl)pyrazolo[1,5-a]pyridin-3-yl)methanone | E, E |
| 4S and 4R enantiomers of 2-[5-[5-(difluoromethyl)-1-methyl-1H-pyrazole-4-carbonyl]-1H,4H,5H,6H,7H-imidazo[4,5-c]pyridin-4-yl]-5-fluoro-1,3-benzoxazole | C, D |
| 4S and 4R enantiomers of 2-[5-[5-(5-fluoropyridin-2-yl)-1,3,4-oxadiazole-2-carbonyl]-1H,4H,5H,6H,7H-imidazo[4,5-c]pyridin-4-yl]-6-methyl-1,3-benzoxazole | C, C |
| 4S and 4R enantiomers of 5-fluoro-2-[5-{5-[5-(trifluoromethyl)pyridin-2-yl]-1,3,4-oxadiazole-2-carbonyl}-1H,4H,5H,6H,7H-imidazo[4,5-c]pyridin-4-yl]-1,3-benzoxazole | C, C |
| 4S and 4R enantiomers of 5-fluoro-2-[5-(1-methyl-1H-1,2,4-triazole-5-carbonyl)-1H,4H,5H,6H,7H-imidazo[4,5-c]pyridin-4-yl]-1,3-benzoxazole | C, C |
| 4S and 4R enantiomers of 5-fluoro-2-[5-[1-(pyridin-2-yl)azetidine-3-carbonyl]-1H,4H,5H,6H,7H-imidazo[4,5-c]pyridin-4-yl]-1,3-benzoxazole | C, D |
| 4S and 4R enantiomers of 5-fluoro-2-[5-(1-methyl-1H-pyrazole-5-carbonyl)-1H,4H,5H,6H,7H-imidazo[4,5-c]pyridin-4-yl]-1,3-benzoxazole | C, C |
| 4S and 4R enantiomers of ({5-[4-(5-fluoro-1,3-benzoxazol-2-yl)-1H,4H,5H,6H,7H-imidazo[4,5-c]pyridine-5-carbonyl]-1,3,4-oxadiazol-2-yl}methyl)dimethylamine | C, D |
| 4S and 4R enantiomers of ({3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}methyl)(methyl)amine | D, D |
| 4S and 4R enantiomers of 3-[4-(5-fluoro-1,3-benzoxazol-2-yl)-1H,4H,5H,6H,7H-imidazo[4,5-c]pyridine-5-carbonyl]pyrazolo[1,5-a]pyridin-6-amine | C, D |
| 4S and 4R enantiomers of 5-fluoro-2-[5-[5-(methoxymethyl)-1,3,4-oxadiazole-2-carbonyl]-1H,4H,5H,6H,7H-imidazo[4,5-c]pyridin-4-yl]-1,3-benzoxazole | C, D |
| 4S and 4R enantiomers of 5-fluoro-2-[5-[5-(2-fluoropropan-2-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of methyl 3-[4-(5-fluoro-1,3-benzoxazol-2-yl)-1H,4H,5H,6H,7H-imidazo[4,5-c]pyridine-5-carbonyl]azetidine-1-carboxylate | C, E |
| 4S and 4R enantiomers of 5-fluoro-2-[5-[1-(pyridin-2-yl)-1H-pyrazole-5-carbonyl]-1H,4H,5H,6H,7H-imidazo[4,5-c]pyridin-4-yl]-1,3-benzoxazole | C, E |
| 4S and 4R enantiomers of 2-[5-[5-(difluoromethyl)-1H-pyrazole-4-carbonyl]-1H,4H,5H,6H,7H-imidazo[4,5-c]pyridin-4-yl]-5-fluoro-1,3-benzoxazole | C, D |
| 4S and 4R enantiomers of 2-[5-{5-[1-(cyclopropanesulfonyl)-1H-pyrazol-4-yl]-1,3,4-oxadiazole-2-carbonyl}-1H,4H,5H,6H,7H-imidazo[4,5-c]pyridin-4-yl]-5-fluoro-1,3-benzoxazole | C, D |
| 4S and 4R enantiomers of 5-fluoro-2-[5-[5-(3-fluoropyridin-2-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 2-{3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}ethan-1-amine | D, E |
| 4S and 4R enantiomers of 3-[4-(5-fluoro-1,3-benzoxazol-2-yl)-1H,4H,5H,6H,7H-imidazo[4,5-c]pyridine-5-carbonyl]-N-methylpyrazolo[1,5-a]pyridin-6-amine | C, D |
| 4S and 4R enantiomers of 2-[5-[5-(3,3-difluorocyclobutyl)-1,3,4-oxadiazole-2-carbonyl]-1H,4H,5H,6H,7H-imidazo[4,5-c]pyridin-4-yl]-5-fluoro-1,3-benzoxazole | C, C |
| 4S and 4R enantiomers of 2-[5-(3-chloro-1-methyl-1H-pyrazole-4-carbonyl)-1H,4H,5H,6H,7H-imidazo[4,5-c]pyridin-4-yl]-5-fluoro-1,3-benzoxazole | C, C |
| 4S and 4R enantiomers of 2-[5-{5-[1-(2,2-difluoroethyl)-1H-pyrazol-4-yl]-1,3,4-oxadiazole-2-carbonyl}-1H,4H,5H,6H,7H-imidazo[4,5-c]pyridin-4-yl]-5-fluoro-1,3-benzoxazole | C, C |
| 4S and 4R enantiomers of (2-{3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}ethyl)(methyl)amine | D, E |
| 4S and 4R enantiomers of 5-fluoro-2-[5-[1-(2,2,2-trifluoroethyl)azetidine-3-carbonyl]-1H,4H,5H,6H,7H-imidazo[4,5-c]pyridin-4-yl]-1,3-benzoxazole | C, C |
| 4S and 4R enantiomers of tert-butyl 3-{5-[4-(5-fluoro-1,3-benzoxazol-2-yl)-1H,4H,5H,6H,7H-imidazo[4,5-c]pyridine-5-carbonyl]-1,3,4-oxadiazol-2-yl}azetidine-1-carboxylate | C, C |
| 4S and 4R enantiomers of 5-fluoro-2-[5-[5-(1H-pyrazol-4-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 5-fluoro-2-[5-[5-(1H-pyrazol-3-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 2-[5-[5-chloro-1-(pyridin-2-yl)-1H-pyrazole-4-carbonyl]-1H,4H,5H,6H,7H-imidazo[4,5-c]pyridin-4-yl]-5-fluoro-1,3-benzoxazole | C, D |
| 4S and 4R enantiomers of 2-[5-[3-chloro-1-(pyridin-2-yl)-1H-pyrazole-4-carbonyl]-1H,4H,5H,6H,7H-imidazo[4,5-c]pyridin-4-yl]-5-fluoro-1,3-benzoxazole | C, D |
| 4S and 4R enantiomers of (2-(2-hydroxypropan-2-yl)-4-(trifluoromethyl)oxazol-5-yl)(4-(5-methylbenzo[d]oxazol-2-yl)-6,7-dihydro-1H-imidazo[4,5-c]pyridin-5(4H)-yl)methanone | E, E |
| 4S and 4R enantiomers of 5-fluoro-2-[5-[5-(3-methyl-1H-pyrazol-4-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 2-[5-[5-(1,5-dimethyl-1H-pyrazol-3-yl)-1,3,4-oxadiazole-2-carbonyl]-1H,4H,5H,6H,7H-imidazo[4,5-c]pyridin-4-yl]-5-fluoro-1,3-benzoxazole | C, C |
| 4S and 4R enantiomers of 5-fluoro-2-[5-[6-(morpholin-4-yl)pyrazolo[1,5-a]pyridine-3-carbonyl]-1H,4H,5H,6H,7H-imidazo[4,5-c]pyridin-4-yl]-1,3-benzoxazole | C, D |
| 4S and 4R enantiomers of (15)-1-{5-[4-(5-fluoro-1,3-benzoxazol-2-yl)-1H,4H,5H,6H,7H-imidazo[4,5-c]pyridine-5-carbonyl]-4-methyl-1,3-oxazol-2-yl}ethan-1-ol | C, D |
| 4S and 4R enantiomers of 5-fluoro-2-[5-[2-(2-methoxypropan-2-yl)-4-methyl-1,3-oxazole-5-carbonyl]-1H,4H,5H,6H,7H-imidazo[4,5-c]pyridin-4-yl]-1,3-benzoxazole | C, D |
| 4S and 4R enantiomers of 1-{5-[4-(5-fluoro-1,3-benzoxazol-2-yl)-1H,4H,5H,6H,7H-imidazo[4,5-c]pyridine-5-carbonyl]-4-methyl-1,3-oxazol-2-yl}cyclobutan-1-ol | C, D |
| 4S and 4R enantiomers of 2-{5-[4-(5-fluoro-1,3-benzoxazol-2-yl)-1H,4H,5H,6H,7H-imidazo[4,5-c]pyridine-5-carbonyl]-1-methyl-1H-pyrazol-3-yl}propan-2-ol | D, D |
| 4S and 4R enantiomers of tert-butyl 4-{3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}piperazine-1-carboxylate | C, D |
| 4S and 4R enantiomers of 5-fluoro-2-[5-[6-(piperazin-1-yl)pyrazolo[1,5-a]pyridine-3-carbonyl]-1H,4H,5H,6H,7H-imidazo[4,5-c]pyridin-4-yl]-1,3-benzoxazole | D, E |
| 4S and 4R enantiomers of 2-[5-(6-{6-azaspiro[2.5]octan-6-yl}pyrazolo[1,5-a]pyridine-3-carbonyl)-1H,4H,5H,6H,7H-imidazo[4,5-c]pyridin-4-yl]-5-fluoro-1,3-benzoxazole | C, D |
| 4S and 4R enantiomers of 1-{3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}methanamine | C, D |
| 4S and 4R enantiomers of 5-fluoro-2-[5-{6-[4-(2,2,2-trifluoroethyl)piperazin-1-yl]pyrazolo[1,5-a]pyridine-3-carbonyl}-1H,4H,5H,6H,7H-imidazo[4,5-c]pyridin-4-yl]-1,3-benzoxazole | C, D |
| 4S and 4R enantiomers of ethyl 4-{3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}piperazine-1-carboxylate | C, D |
| 4S and 4R enantiomers of 2-(1-{3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}piperidin-4-yl)propan-2-ol | C, D |
| 4S and 4R enantiomers of 1-{4-[4-(5-fluoro-1,3-benzoxazol-2-yl)-1H,4H,5H,6H,7H-imidazo[4,5-c]pyridine-5-carbonyl]-1H-pyrazol-1-yl}propan-2-one | D, E |
| 4S and 4R enantiomers of 1-{5-[4-(5-fluoro-1,3-benzoxazol-2-yl)-1H,4H,5H,6H,7H-imidazo[4,5-c]pyridine-5-carbonyl]-4-methyl-1,3-oxazol-2-yl}cyclopropan-1-ol | C, D |
| 4S and 4R enantiomers of dimethyl(2-{3-[4-(5-methyl-1,3-benzoxazol-2-yl)-1H,4H,5H,6H,7H-imidazo[4,5-c]pyridine-5-carbonyl]pyrazolo[1,5-a]pyridin-6-yl}ethyl)amine | C, D |
| 4S and 4R enantiomers of 5-fluoro-2-[5-{5-[3-(trifluoromethyl)-1H-pyrazol-4-yl]-1,3,4-oxadiazole-2-carbonyl}-1H,4H,5H,6H,7H-imidazo[4,5-c]pyridin-4-yl]-1,3-benzoxazole | C, D |
| 4S and 4R enantiomers of 5-fluoro-2-[5-[6-(4-fluoropiperidin-1-yl)pyrazolo[1,5-a]pyridine-3-carbonyl]-1H,4H,SH,6H,7H-imidazo[4,5-c]pyridin-4-yl]-1,3-benzoxazole | C, D |
| 4S and 4R enantiomers of 1-{3-[4-(5-fluoro-1,3-benzoxazol-2-yl)-1H,4H,5H,6H,7H-imidazo[4,5-c]pyridine-5-carbonyl]pyrazolo[1,5-a]pyridin-6-yl}piperidin-4-ol | C, E |
| 4S and 4R enantiomers of 4-{3-[4-(5-fluoro-1,3-benzoxazol-2-yl)-1H,4H,5H,6H,7H-imidazo[4,5-c]pyridine-5-carbonyl\|pyrazolo[1,5-a]pyridin-6-yl}-1lambda6-thiomorpholine-1,1-dione | D, E |
| 4S and 4R enantiomers of 1-{4-[4-(5-fluoro-1,3-benzoxazol-2-yl)-1H,4H,5H,6H,7H-imidazo[4,5-c]pyridine-5-carbonyl]-1H-pyrazol-1-yl}-2-methylpropan-2-ol | D, E |
| 4S and 4R enantiomers of 2-[5-[4-(difluoromethyl)-2-(2-methanesulfonylpropan-2-yl)-1,3-oxazole-5-carbonyl]-1H,4H,5H,6H,7H-imidazo[4,5-c]pyridin-4-yl]-5-fluoro-1,3-benzoxazole | C, C |
| 4S and 4R enantiomers of 6-bromo-2-[5-[2-(1-methyl-1H-pyrazol-4-yl)-1,3-oxazole-5-carbonyl]-1H,4H,5H,6H,7H-imidazo[4,5-c]pyridin-4-yl]-1,3-benzoxazole | C, D |
| 4S and 4R enantiomers of 5-fluoro-2-[5-(6-{8-methyl-3,8-diazabicyclo[3.2.1]octan-3-yl}pyrazolo[1,5-a]pyridine-3-carbonyl)-1H,4H,5H,6H,7H-imidazo[4,5-c]pyridin-4-yl]-1,3-benzoxazole | C, D |
| 4S and 4R enantiomers of 5-fluoro-2-[5-(1H-pyrazole-4-carbonyl)-1H,4H,5H,6H,7H-imidazo[4,5-c]pyridin-4-yl]-1,3-benzoxazole | D, E |
| 4S and 4R enantiomers of 5-fluoro-2-[5-(1H-pyrazole-5-carbonyl)-1H,4H,5H,6H,7H-imidazo[4,5-c]pyridin-4-yl]-1,3-benzoxazole | D, E |
| 4S and 4R enantiomers of 2-[5-(3-chloro-1-methyl-1H-1,2,4-triazole-5-carbonyl)-1H,4H,5H,6H,7H-imidazo[4,5-c]pyridin-4-yl]-5-fluoro-1,3-benzoxazole | C, C |
| 4S and 4R enantiomers of 2-[5-{2-[(2S)-azetidin-2-yl]-4-(difluoromethyl)-1,3-oxazole-5-carbonyl}-1H,4H,5H,6H,7H-imidazo[4,5-c]pyridin-4-yl]-5-fluoro-1,3-benzoxazole | C, C |
| 4S and 4R enantiomers of 2-[5-(5-chloro-1H-pyrazole-4-carbonyl)-1H,4H,5H,6H,7H-imidazo[4,5-c]pyridin-4-yl]-5-fluoro-1,3-benzoxazole | C, C |
| 4S and 4R enantiomers of 5-fluoro-2-[5-(1-methyl-1H-pyrazole-3-carbonyl)-1H,4H,5H,6H,7H-imidazo[4,5-c]pyridin-4-yl]-1,3-benzoxazole | C, D |
| 4S and 4R enantiomers of 5-[4-(5-fluoro-1,3-benzoxazol-2-yl)-1H,4H,5H,6H,7H-imidazo[4,5-c]pyridine-5-carbonyl]-1-methyl-1H-pyrazol-3-amine | D, E |
| 4S and 4R enantiomers of (2-{5-[4-(5-fluoro-1,3-benzoxazol-2-yl)-1H,4H,5H,6H,7H-imidazo[4,5-c]pyridine-5-carbonyl]-4-methyl-1,3-oxazol-2-yl}propan-2-yl)dimethylamine | C, D |
| 4S and 4R enantiomers of 6-fluoro-2-[5-[5-(1-methyl-1H-pyrazol-4-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of (2-{5-[4-(5-fluoro-1,3-benzoxazol-2-yl)-1H,4H,5H,6H,7H-imidazo[4,5-c]pyridine-5-carbonyl]-4-methyl-1,3-oxazol-2-yl}propan-2-yl)(methyl)amine | C, D |
| 4S and 4R enantiomers of 1-(difluoromethyl)-5-[4-(5-fluoro-1,3-benzoxazol-2-yl)-1H,4H,5H,6H,7H-imidazo[4,5-c]pyridine-5-carbonyl]-1H-pyrazol-3-amine | C, D |
| 4S and 4R enantiomers of 5-fluoro-2-[5-[1-methyl-3-(trifluoromethyl)-1H-1,2,4-triazole-5-carbonyl]-1H,4H,5H,6H,7H-imidazo[4,5-c]pyridin-4-yl]-1,3-benzoxazole | C, C |
| 4S and 4R enantiomers of 6-fluoro-3-[4-(5-fluoro-1,3-benzoxazol-2-yl)-1H,4H,5H,6H,7H-imidazo[4,5-c]pyridine-5-carbonyl]pyrazolo[1,5-a]pyridin-5-amine | C, D |
| 4S and 4R enantiomers of (15)-1-{5-[4-(4-fluoro-1,3-benzoxazol-2-yl)-1H,4H,5H,6H,7H-imidazo[4,5-c]pyridine-5-carbonyl]-4-methyl-1,3-oxazol-2-yl}ethan-1-ol | C, D |
| 4S and 4R enantiomers of 3-[4-(5-fluoro-1,3-benzoxazol-2-yl)-1H,4H,5H,6H,7H-imidazo[4,5-c]pyridine-5-carbonyl]pyrazolo[1,5-a]pyridine-6-carbonitrile | C, D |
| 4S and 4R enantiomers of 2-{5-[4-(6-fluoro-1,3-benzoxazol-2-yl)-1H,4H,5H,6H,7H-imidazo[4,5-c]pyridine-5-carbonyl]-4-methyl-1,3-oxazol-2-yl}propan-2-ol | C, D |
| 4S and 4R enantiomers of (1R)-1-{5-[4-(6-fluoro-1,3-benzoxazol-2-yl)-1H,4H,5H,6H,7H-imidazo[4,5-c]pyridine-5-carbonyl]-4-methyl-1,3-oxazol-2-yl}ethan-1-ol | C, D |
| 4S and 4R enantiomers of 6-fluoro-2-[5-[6-(methoxymethyl)pyrazolo[1,5-a]pyridine-3-carbonyl]-1H,4H,5H,6H,7H-imidazo[4,5-c]pyridin-4-yl]-1,3-benzoxazole | C, D |
| 4S and 4R enantiomers of 2-{5-[4-(5-fluoro-1,3-benzoxazol-2-yl)-1H,4H,5H,6H,7H-imidazo[4,5-c]pyridine-5-carbonyl]-1,2-thiazol-3-yl}propan-2-ol | D, D |
| 4S and 4R enantiomers of 2-[4-(difluoromethyl)-5-[4-(5-fluoro-1,3-benzoxazol-2-yl)-1H,4H,5H,6H,7H-imidazo[4,5-c]pyridine-5-carbonyl]-1,3-thiazol-2-yl]propan-2-ol | C, C |
| 4S and 4R enantiomers of 2-{4-bromo-5-[4-(5-fluoro-1,3-benzoxazol-2-yl)-1H,4H,5H,6H,7H-imidazo[4,5-cpyridine-5-carbonyl]-1,3-thiazol-2-yl}propan-2-ol | C, D |
| 4S and 4R enantiomers of 6-fluoro-2-[5-[1-methyl-3-(trifluoromethyl)-1H-1,2,4-triazole-5-carbonyl]-1H,4H,5H,6H,7H-imidazo[4,5-c]pyridin-4-yl]-1,3-benzoxazole | C, D |
| 4S and 4R enantiomers of 4-fluoro-2-[5-[1-methyl-3-(trifluoromethyl)-1H-1,2,4-triazole-5-carbonyl]-1H,4H,5H,6H,7H-imidazo[4,5-c]pyridin-4-yl]-1,3-benzoxazole | C, C |
| 4S and 4R enantiomers of 7-fluoro-2-[5-(1-methyl-1H-1,2,4-triazole-5-carbonyl)-1H,4H,5H,6H,7H-imidazo[4,5-c]pyridin-4-yl]-1,3-benzoxazole | C, C |
| 4S and 4R enantiomers of 4-fluoro-2-[5-(1-methyl-1H-1,2,4-triazole-5-carbonyl)-1H,4H,5H,6H,7H-imidazo[4,5-c]pyridin-4-yl]-1,3-benzoxazole | C, C |
| 4S and 4R enantiomers of 6-fluoro-2-[5-(1,3-oxazole-5-carbonyl)-1H,4H,5H,6H,7H-imidazo[4,5-c]pyridin-4-yl]-1,3-benzoxazole | C, D |
| 4S and 4R enantiomers of 2-{5-[4-(6-fluoro-1,3-benzoxazol-2-yl)-1H,4H,5H,6H,7H-imidazo[4,5-c]pyridine-5-carbonyl]-1,2-oxazol-3-yl}propan-2-ol | C, D |
| 4S and 4R enantiomers of 2-{5-[4-(7-fluoro-1,3-benzoxazol-2-yl)-1H,4H,5H,6H,7H-imidazo[4,5-c]pyridine-5-carbonyl]-1,2-oxazol-3-yl}propan-2-ol | C, C |
| 4S and 4R enantiomers of 2-{5-[4-(4-fluoro-1,3-benzoxazol-2-yl)-1H,4H,5H,6H,7H-imidazo[4,5-c]pyridine-5-carbonyl]-1,2-oxazol-3-yl}propan-2-ol | C, C |
| 4S and 4R enantiomers of 2-{3-[4-(6-fluoro-1,3-benzoxazol-2-yl)-1H,4H,5H,6H,7H-imidazo[4,5-c]pyridine-5-carbonyl]-1,2-oxazol-5-yl}propan-2-ol | C, D |
| 4S and 4R enantiomers of 2-{3-[4-(7-fluoro-1,3-benzoxazol-2-yl)-1H,4H,5H,6H,7H-imidazo[4,5-c]pyridine-5-carbonyl]-1,2-oxazol-5-yl}propan-2-ol | C, C |
| 4S and 4R enantiomers of 2-{5-[4-(5-fluoro-1,3-benzoxazol-2-yl)-1H,4H,5H,6H,7H-imidazo[4,5-c]pyridine-5-carbonyl]-1,2,4-oxadiazol-3-yl}propan-2-ol | D, D |
| 4S and 4R enantiomers of 2-{5-[4-(6-fluoro-1,3-benzoxazol-2-yl)-1H,4H,5H,6H,7H-imidazo[4,5-c]pyridine-5-carbonyl]-1,2,4-oxadiazol-3-yl}propan-2-ol | D, D |
| 4S and 4R enantiomers of 2-{5-[4-(4-fluoro-1,3-benzoxazol-2-yl)-1H,4H,5H,6H,7H-imidazo[4,5-c]pyridine-5-carbonyl]-1,2,4-oxadiazol-3-yl}propan-2-ol | D, D |
| 4S and 4R enantiomers of 6-fluoro-2-[5-(1-methyl-1H-1,2,4-triazole-5-carbonyl)-1H,4H,5H,6H,7H-imidazo[4,5-c]pyridin-4-yl]-1,3-benzoxazole | C, D |
| 4S and 4R enantiomers of 6-fluoro-2-[5-(4-methyl-4H-1,2,4-triazole-3-carbonyl)-1H,4H,5H,6H,7H-imidazo[4,5-c]pyridin-4-yl]-1,3-benzoxazole | C, D |
| 4S and 4R enantiomers of 2-[5-(4-cyclopropyl-4H-1,2,4-triazole-3-carbonyl)-1H,4H,5H,6H,7H-imidazo[4,5-c]pyridin-4-yl]-7-fluoro-1,3-benzoxazole | C, C |
| 4S and 4R enantiomers of 2-[5-(4-cyclopropyl-4H-1,2,4-triazole-3-carbonyl)-1H,4H,5H,6H,7H-imidazo[4,5-c]pyridin-4-yl]-4-fluoro-1,3-benzoxazole | C, C |
| 4S and 4R enantiomers of 4-fluoro-2-[5-(1,3-oxazole-5-carbonyl)-1H,4H,5H,6H,7H-imidazo[4,5-c]pyridin-4-yl]-1,3-benzoxazole | C, D |
| 4S and 4R enantiomers of 2-{3-[4-(4-fluoro-1,3-benzoxazol-2-yl)-1H,4H,5H,6H,7H-imidazo[4,5-c]pyridine-5-carbonyl]-1,2-oxazol-5-yl}propan-2-ol | C, D |
| 4S and 4R enantiomers of 2-{5-[4-(7-fluoro-1,3-benzoxazol-2-yl)-1H,4H,5H,6H,7H-imidazo[4,5-c]pyridine-5-carbonyl]-1,2,4-oxadiazol-3-yl}propan-2-ol | C, C |
| 4S and 4R enantiomers of 2-[5-[3-(difluoromethyl)-1-methyl-1H-1,2,4-triazole-5-carbonyl]-1H,4H,5H,6H,7H-imidazo[4,5-c]pyridin-4-yl]-6-fluoro-1,3-benzoxazole | C, C |
| 4S and 4R enantiomers of 2-[5-[3-(difluoromethyl)-1-methyl-1H-1,2,4-triazole-5-carbonyl]-1H,4H,5H,6H,7H-imidazo[4,5-c]pyridin-4-yl]-4-fluoro-1,3-benzoxazole | C, C |
| 4S and 4R enantiomers of 2-[5-[3-(difluoromethyl)-1-methyl-1H-1,2,4-triazole-5-carbonyl]-1H,4H,5H,6H,7H-imidazo[4,5-c]pyridin-4-yl]-5-fluoro-1,3-benzoxazole | C, C |
| 4S and 4R enantiomers of 7-fluoro-2-[5-(4-methyl-4H-1,2,4-triazole-3-carbonyl)-1H,4H,5H,6H,7H-imidazo[4,5-c]pyridin-4-yl]-1,3-benzoxazole | C, C |
| 4S and 4R enantiomers of 4-fluoro-2-[5-(4-methyl-4H-1,2,4-triazole-3-carbonyl)-1H,4H,5H,6H,7H-imidazo[4,5-c]pyridin-4-yl]-1,3-benzoxazole | C, C |
| 4S and 4R enantiomers of 5-fluoro-2-[5-(4-methyl-4H-1,2,4-triazole-3-carbonyl)-1H,4H,5H,6H,7H-imidazo[4,5-c]pyridin-4-yl]-1,3-benzoxazole | C, C |
| 4S and 4R enantiomers of 2-[5-(4-cyclopropyl-4H-1,2,4-triazole-3-carbonyl)-1H,4H,5H,6H,7H-imidazo[4,5-c]pyridin-4-yl]-6-fluoro-1,3-benzoxazole | C, C |
| 4S and 4R enantiomers of 2-[5-(4-cyclopropyl-4H-1,2,4-triazole-3-carbonyl)-1H,4H,5H,6H,7H-imidazo[4,5-c]pyridin-4-yl]-5-fluoro-1,3-benzoxazole | C, D |
| 4S and 4R enantiomers of 2-{5-[4-(5,6-difluoro-1,3-benzoxazol-2-yl)-1H,4H,5H,6H,7H-imidazo[4,5-c]pyridine-5-carbonyl]-4-(difluoromethyl)-1,3-oxazol-2-yl}propan-2-ol | C, C |
| 4S and 4R enantiomers of 2-{3-[4-(5-fluoro-1,3-benzoxazol-2-yl)-1H,4H,5H,6H,7H-imidazo[4,5-c]pyridine-5-carbonyl]-1,2,4-oxadiazol-5-yl}propan-2-ol | D, D |
| 4S and 4R enantiomers of 2-{3-[4-(6-fluoro-1,3-benzoxazol-2-yl)-1H,4H,5H,6H,7H-imidazo[4,5-c]pyridine-5-carbonyl]-1,2,4-oxadiazol-5-yl}propan-2-ol | D, D |
| 4S and 4R enantiomers of 2-{3-[4-(4-fluoro-1,3-benzoxazol-2-yl)-1H,4H,5H,6H,7H-imidazo[4,5-c]pyridine-5-carbonyl]-1,2,4-oxadiazol-5-yl}propan-2-ol | C, D |
| 4S and 4R enantiomers of 6-fluoro-2-[5-[5-(1H-pyrazol-4-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 6-fluoro-2-[5-[5-(1H-pyrazol-5-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 2-{5-[4-(5-tert-butyl-1,3-benzoxazol-2-yl)-1H,4H,5H,6H,7H-imidazo[4,5-c]pyridine-5-carbonyl]-4-(difluoromethyl)-1,3-oxazol-2-yl}propan-2-ol | E, E |
| 4'S and 4'R enantiomers of 2-[4-(difluoromethyl)-5-{ [4'-(5-fluoro-1,3-benzoxazol-2-yl)-1',4',5',6'-tetrahydrospiro[cyclopropane-1,7'-imidazo[4,5-c]pyridin]-5'-yl]carbonyl}-1,3-oxazol-2-yl]propan-2-ol | C, C |
| 4S and 4R enantiomers of 4-fluoro-2-[5-[5-(1H-pyrazol-4-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 2-[4-(difluoromethyl)-5-[4-[5-(propan-2-yl)-1,3-benzoxazol-2-yl]-1H,4H,5H,6H,7H-imidazo[4,5-c]pyridine-5-carbonyl]-1,3-oxazol-2-yl]propan-2-ol | E, E |
| 4S and 4R enantiomers of 5-tert-butyl-2-[5-[4-(difluoromethyl)-1,3-oxazole-5-carbonyl]-1H,4H,5H,6H,7H-imidazo[4,5-c]pyridin-4-yl]-1,3-benzoxazole | E, E |
| 4S and 4R enantiomers of 1-[4-(6-fluoro-1,3-benzoxazol-2-yl)-1H,4H,5H,6H,7H-imidazo[4,5-c]pyridin-5-yl]-3-(1,3-thiazol-2-yl)propan-1-one | C, D |
| 4S and 4R enantiomers of 1-[4-(6-fluoro-1,3-benzoxazol-2-yl)-1H,4H,5H,6H,7H-imidazo[4,5-c]pyridin-5-yl]-3-(1,3-oxazol-2-yl)propan-1-one | C, E |
| 4S and 4R enantiomers of 1-[4-(7-fluoro-1,3-benzoxazol-2-yl)-1H,4H,5H,6H,7H-imidazo[4,5-c]pyridin-5-yl]-3-(1,3-oxazol-5-yl)propan-1-one | C, E |
| 4S and 4R enantiomers of 1-[4-(6-fluoro-1,3-benzoxazol-2-yl)-1H,4H,5H,6H,7H-imidazo[4,5-c]pyridin-5-yl]-3-(1,3-oxazol-5-yl)propan-1-one | C, E |
| 4S and 4R enantiomers of 2-[5-[4-(difluoromethyl)-1,3-oxazole-5-carbonyl]-1H,4H,5H,6H,7H-imidazo[4,5-c]pyridin-4-yl]-5-(propan-2-yl)-1,3-benzoxazole | E, E |
| 4S and 4R enantiomers of 1-[4-(5-fluoro-1,3-benzoxazol-2-yl)-1H,4H,5H,6H,7H-imidazo[4,5-c]pyridin-5-yl]-3-(1,3-thiazol-2-yl)propan-1-one | C, E |
| 4S and 4R enantiomers of 1-[4-(5-fluoro-1,3-benzoxazol-2-yl)-1H,4H,5H,6H,7H-imidazo[4,5-c]pyridin-5-yl]-3-(1,3-oxazol-2-yl)propan-1-one | C, E |
| 4S and 4R enantiomers of 1-[4-(5-fluoro-1,3-benzoxazol-2-yl)-1H,4H,5H,6H,7H-imidazo[4,5-c]pyridin-5-yl]-3-(1,3-oxazol-5-yl)propan-1-one | C, E |
| 4S and 4R enantiomers of 1-[4-(4-fluoro-1,3-benzoxazol-2-yl)-1H,4H,5H,6H,7H-imidazo[4,5-c]pyridin-5-yl]-3-(1,3-oxazol-5-yl)propan-1-one | C, E |
| 4S and 4R enantiomers of 1-[4-(6-fluoro-1,3-benzoxazol-2-yl)-1H,4H,5H,6H,7H-imidazo[4,5-c]pyridin-5-yl]-3-(1,3-thiazol-5-yl)propan-1-one | C, E |
| 4S and 4R enantiomers of 1-[4-(5-fluoro-1,3-benzoxazol-2-yl)-1H,4H,5H,6H,7H-imidazo[4,5-c]pyridin-5-yl]-3-(1,3-thiazol-5-yl)propan-1-one | C, E |
| 4S and 4R enantiomers of 5-fluoro-2-[5-[5-(pyridin-4-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 1-[4-(4-fluoro-1,3-benzoxazol-2-yl)-1H,4H,5H,6H,7H-imidazo[4,5-c]pyridin-5-yl]-3-(1,3-thiazol-5-yl)propan-1-one | C, D |
| 4S and 4R enantiomers of 1-[4-(5-fluoro-1,3-benzoxazol-2-yl)-1H,4H,5H,6H,7H-imidazo[4,5-c]pyridin-5-yl]-2-(1,3-oxazol-5-yl)ethan-1-one | C, E |
| 4S and 4R enantiomers of 6-methyl-2-[5-[5-(1H-pyrazol-4-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 5-fluoro-2-[5-[(1,3-oxazol-5-yl)methyl]-1H,4H,5H,6H,7H-imidazo[4,5-c]pyridin-4-yl]-1,3-benzoxazole | D, E |
| 4S and 4R enantiomers of 2-{5-[4-(4,7-difluoro-1,3-benzoxazol-2-yl)-1H,4H,5H,6H,7H-imidazo[4,5-c]pyridine-5-carbonyl]-1,3-oxazol-2-yl}propan-2-ol | C, C |
| 4S and 4R enantiomers of 4,6-difluoro-2-[5-(1,3-oxazole-5-carbonyl)-1H,4H,5H,6H,7H-imidazo[4,5-c]pyridin-4-yl]-1,3-benzoxazole | C, D |
| 4S and 4R enantiomers of 2-{5-[4-(4,6-difluoro-1,3-benzoxazol-2-yl)-1H,4H,5H,6H,7H-imidazo[4,5-c]pyridine-5-carbonyl]-1,3-oxazol-2-yl}propan-2-ol | C, C |
| 4S and 4R enantiomers of 2-[5-[3-(difluoromethyl)-1-methyl-1H-1,2,4-triazole-5-carbonyl]-1H,4H,5H,6H,7H-imidazo[4,5-c]pyridin-4-yl]-4,6-difluoro-1,3-benzoxazole | C, C |
| 4S and 4R enantiomers of 5-fluoro-2-[5-[5-(pyrimidin-2-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 1-{3-[4-(4-fluoro-1,3-benzoxazol-2-yl)-1H,4H,5H,6H,7H-imidazo[4,5-c]pyridine-5-carbonyl]pyrazolo[1,5-a]pyridin-6-yl}piperidin-4-ol | C, D |
| 4S and 4R enantiomers of 6,7-difluoro-2-[5-(1,3-oxazole-5-carbonyl)-1H,4H,5H,6H,7H-imidazo[4,5-c]pyridin-4-yl]-1,3-benzoxazole | C, C |
| 4S and 4R enantiomers of 2-{5-[4-(6,7-difluoro-1,3-benzoxazol-2-yl)-1H,4H,5H,6H,7H-imidazo[4,5-c]pyridine-5-carbonyl]-1,3-oxazol-2-yl}propan-2-ol | C, C |
| 4S and 4R enantiomers of 2-[5-[3-(difluoromethyl)-1-methyl-1H-1,2,4-triazole-5-carbonyl]-1H,4H,5H,6H,7H-imidazo[4,5-c]pyridin-4-yl]-6,7-difluoro-1,3-benzoxazole | C, C |
| 4S and 4R enantiomers of 2-{5-[4-(1,3-benzoxazol-2-yl)-1H,4H,5H,6H,7H-imidazo[4,5-c]pyridine-5-carbonyl]-1,3-oxazol-2-yl}propan-2-ol | C, C |
| 4S and 4R enantiomers of 4,5-difluoro-2-[5-(1,3-oxazole-5-carbonyl)-1H,4H,5H,6H,7H-imidazo[4,5-c]pyridin-4-yl]-1,3-benzoxazole | C, D |
| 4S and 4R enantiomers of 2-{5-[4-(4,5-difluoro-1,3-benzoxazol-2-yl)-1H,4H,5H,6H,7H-imidazo[4,5-c]pyridine-5-carbonyl]-1,3-oxazol-2-yl}propan-2-ol | C, C |
| 4S and 4R enantiomers of 2-{5-[4-(5,7-difluoro-1,3-benzoxazol-2-yl)-1H,4H,5H,6H,7H-imidazo[4,5-c]pyridine-5-carbonyl]-1,3-oxazol-2-yl}propan-2-ol | C, C |
| 4S and 4R enantiomers of 2-[5-[3-(difluoromethyl)-1-methyl-1H-1,2,4-triazole-5-carbonyl]-1H,4H,5H,6H,7H-imidazo[4,5-c]pyridin-4-yl]-4,5-difluoro-1,3-benzoxazole | C, C |
| 4S and 4R enantiomers of 2-[5-[1-(difluoromethyl)-1H-pyrazole-5-carbonyl]-1H,4H,5H,6H,7H-imidazo[4,5-c]pyridin-4-yl]-4,5-difluoro-1,3-benzoxazole | C, C |
| 4S and 4R enantiomers of 5-methoxy-2-[5-(1,3-oxazole-5-carbonyl)-1H,4H,5H,6H,7H-imidazo[4,5-c]pyridin-4-yl]-1,3-benzoxazole | C, D |
| 4S and 4R enantiomers of 2-{5-[4-(5-methoxy-1,3-benzoxazol-2-yl)-1H,4H,5H,6H,7H-imidazo[4,5-c]pyridine-5-carbonyl]-1,3-oxazol-2-yl}propan-2-ol | C, D |
| 4S and 4R enantiomers of 2-{5-[4-(7-methyl-1,3-benzoxazol-2-yl)-1H,4H,5H,6H,7H-imidazo[4,5-c]pyridine-5-carbonyl]-1,3-oxazol-2-yl}propan-2-ol | C, D |
| 4S and 4R enantiomers of 2-{5-[4-(7-fluoro-1,3-benzoxazol-2-yl)-1H,4H,5H,6H,7H-imidazo[4,5-c]pyridine-5-carbonyl]-1-methyl-1H-1,2,4-triazol-3-yl}propan-2-ol | C, D |
| 4S and 4R enantiomers of 2-{5-[4-(4-methoxy-1,3-benzoxazol-2-yl)-1H,4H,5H,6H,7H-imidazo[4,5-c]pyridine-5-carbonyl]-1,3-oxazol-2-yl}propan-2-ol | C, C |
| 4S and 4R enantiomers of 7-fluoro-2-[5-[1-methyl-3-(pyridin-2-yl)-1H-1,2,4-triazole-5-carbonyl]-1H,4H,5H,6H,7H-imidazo[4,5-c]pyridin-4-yl]-1,3-benzoxazole | C, D |
| 4S and 4R enantiomers of 2-[5-[5-(pyridin-2-yl)-1,3,4-oxadiazole-2-carbonyl]-1H,4H,5H,6H,7H-imidazo[4,5-c]pyridin-4-yl]-5-(trifluoromethyl)-1,3-benzoxazole | C, C |
| 4S and 4R enantiomers of 5-fluoro-2-[5-[5-(1-methyl-1H-imidazol-4-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 2-[5-[5-(1-methyl-1H-pyrazol-4-yl)-1,3,4-oxadiazole-2-carbonyl]-1H,4H,5H,6H,7H-imidazo[4,5-c]pyridin-4-yl]-5-(trifluoromethyl)-1,3-benzoxazole | C, C |
| 4S and 4R enantiomers of 2-[5-[5-(3-fluoropyridin-2-yl)-1,3,4-oxadiazole-2-carbonyl]-1H,4H,5H,6H,7H-imidazo[4,5-c]pyridin-4-yl]-5-(trifluoromethyl)-1,3-benzoxazole | C, C |
| 4S and 4R enantiomers of 5-fluoro-2-[5-[5-(1-methyl-1H-imidazol-5-yl)-1,3,4-oxadiazole-2-carbonyl]-1H,4H,5H,6H,7H-imidazo[4,5-c]pyridin-4-yl]-1,3-benzoxazole | C, D |
| 4S and 4R enantiomers of 1-{3-[4-(6,7-difluoro-1,3-benzoxazol-2-yl)-1H,4H,5H,6H,7H-imidazo[4,5-c]pyridine-5-carbonyl]pyrazolo[1,5-a]pyridin-6-yl}piperidin-4-ol | C, C |
| 4S and 4R enantiomers of 1-{3-[4-(1,3-benzoxazol-2-yl)-1H,4H,5H,6H,7H-imidazo[4,5-c]pyridine-5-carbonyl]pyrazolo[1,5-a]pyridin-6-yl}piperidin-4-ol | C, C |
| 4S and 4R enantiomers of 2-[5-[5-(1-methyl-1H-pyrazol-3-yl)-1,3,4-oxadiazole-2-carbonyl]-1H,4H,5H,6H,7H-imidazo[4,5-c]pyridin-4-yl]-5-(trifluoromethyl)-1,3-benzoxazole | C, C |
| 4S and 4R enantiomers of 2-[5-[5-(5-methoxypyridin-2-yl)-1,3,4-oxadiazole-2-carbonyl]-1H,4H,5H,6H,7H-imidazo[4,5-c]pyridin-4-yl]-5-(trifluoromethyl)-1,3-benzoxazole | C, C |
| 4S and 4R enantiomers of 2-[4-(difluoromethyl)-5-[4-(5-fluoro-1,3-benzoxazol-2-yl)-7,7-dimethyl-1H,4H,5H,6H,7H-imidazo[4,5-c]pyridine-5-carbonyl]-1,3-oxazol-2-yl]propan-2-ol | D, D |
| 4S and 4R enantiomers of 1-{3-[4-(5-methoxy-1,3-benzoxazol-2-yl)-1H,4H,5H,6H,7H-imidazo[4,5-c]pyridine-5-carbonyl]pyrazolo[1,5-a]pyridin-6-yl}piperidin-4-ol | D, E |
| 4S and 4R enantiomers of 2-[5-{5-[1-(difluoromethyl)-1H-pyrazol-4-yl]-1,3,4-oxadiazole-2-carbonyl}-1H,4H,5H,6H,7H-imidazo[4,5-c]pyridin-4-yl]-5-(trifluoromethyl)-1,3-benzoxazole | D, D |
| 4S and 4R enantiomers of (1-isopropyl-1H-1,2,4-triazol-5-yl)(4-(7-methylbenzo[d]oxazol-2-yl)-6,7-dihydro-1H-imidazo[4,5-c]pyridin-5(4H)-yl)methanone | E, E |
| 4S and 4R enantiomers of (1-isopropyl-1H-1,2,4-triazol-5-yl)(4-(7-methoxybenzo[d]oxazol-2-yl)-6,7-dihydro-1H-imidazo[4,5-c]pyridin-5(4H)-yl)methanone | E, E |
| 4S and 4R enantiomers of 7-methoxy-2-[5-[1-(trifluoromethyl)-1H-pyrazole-5-carbonyl]-1H,4H,5H,6H,7H-imidazo[4,5-c]pyridin-4-yl]-1,3-benzoxazole | E, E |
| 4S and 4R enantiomers of 7-fluoro-2-[5-(1-methyl-1H-pyrazole-5-carbonyl)-1H,4H,5H,6H,7H-imidazo[4,5-c]pyridin-4-yl]-1,3-benzoxazole | C, C |
| 4S and 4R enantiomers of 2-{5-[4-(6-fluoro-1,3-benzoxazol-2-yl)-1H,4H,5H,6H,7H-imidazo[4,5-c]pyridine-5-carbonyl]-1,3-oxazol-2-yl}propan-2-ol | C, C |
| 4S and 4R enantiomers of 1-{3-[4-(7-methyl-1,3-benzoxazol-2-yl)-1H,4H,5H,6H,7H-imidazo[4,5-c]pyridine-5-carbonyl]pyrazolo[1,5-a]pyridin-6-yl}piperidin-4-ol | C, D |
| 4S and 4R enantiomers of 2-[5-{5-[1-(difluoromethyl)-1H-pyrazol-3-yl]-1,3,4-oxadiazole-2-carbonyl}-1H,4H,5H,6H,7H-imidazo[4,5-c]pyridin-4-yl]-5-(trifluoromethyl)-1,3-benzoxazole | C, C |
| 4S and 4R enantiomers of 2-[5-(1-methyl-1H-1,2,4-triazole-5-carbonyl)-1H,4H,5H,6H,7H-imidazo[4,5-c]pyridin-4-yl]-1,3-benzoxazole | C, C |
| 4S and 4R enantiomers of 2-[5-(3-tert-butyl-1-methyl-1H-1,2,4-triazole-5-carbonyl)-1H,4H,5H,6H,7H-imidazo[4,5-c]pyridin-4-yl]-1,3-benzoxazole | C, D |
| 4S and 4R enantiomers of 2-[5-(3-tert-butyl-1-methyl-1H-1,2,4-triazole-5-carbonyl)-1H,4H,5H,6H,7H-imidazo[4,5-c]pyridin-4-yl]-7-fluoro-1,3-benzoxazole | C, C |
| 4S and 4R enantiomers of 2-[5-(3-tert-butyl-1-methyl-1H-1,2,4-triazole-5-carbonyl)-1H,4H,5H,6H,7H-imidazo[4,5-c]pyridin-4-yl]-7-methyl-1,3-benzoxazole | C, E |
| 4S and 4R enantiomers of 2-[5-(3-tert-butyl-1-methyl-1H-1,2,4-triazole-5-carbonyl)-1H,4H,5H,6H,7H-imidazo[4,5-c]pyridin-4-yl]-7-methoxy-1,3-benzoxazole | C, E |
| 4S and 4R enantiomers of 7-fluoro-2-[5-[1-(propan-2-yl)-1H-pyrazole-5-carbonyl]-1H,4H,5H,6H,7H-imidazo[4,5-c]pyridin-4-yl]-1,3-benzoxazole | C, E |
| 4S and 4R enantiomers of 7-methyl-2-[5-[1-(propan-2-yl)-1H-pyrazole-5-carbonyl]-1H,4H,5H,6H,7H-imidazo[4,5-c]pyridin-4-yl]-1,3-benzoxazole | D, E |
| 4S and 4R enantiomers of 2-[5-[1-(pyridin-2-yl)-1H-pyrazole-5-carbonyl]-1H,4H,5H,6H,7H-imidazo[4,5-c]pyridin-4-yl]-1,3-benzoxazole | C, E |
| 4S and 4R enantiomers of 7-methyl-2-[5-[1-(pyridin-2-yl)-1H-pyrazole-5-carbonyl]-1H,4H,5H,6H,7H-imidazo[4,5-c]pyridin-4-yl]-1,3-benzoxazole | C, E |
| 4S and 4R enantiomers of 7-methoxy-2-[5-[1-(pyridin-2-yl)-1H-pyrazole-5-carbonyl]-1H,4H,5H,6H,7H-imidazo[4,5-c]pyridin-4-yl]-1,3-benzoxazole | C, E |
| 4S and 4R enantiomers of 2-[4-(difluoromethyl)-5-[4-(5-fluoro-1,3-benzoxazol-2-yl)-1H,4H,5H,6H,7H,8H-imidazo[4,5-c]azepine-5-carbonyl]-1,3-oxazol-2-yl]propan-2-ol | C, C |
| 4S and 4R enantiomers of 5-fluoro-2-[5-[5-(pyrimidin-4-yl)-1,3,4-oxadiazole-2-carbonyl]-1H,4H,5H,6H,7H-imidazo[4,5-c]pyridin-4-yl]-1,3-benzoxazole | C, C |
| 4S and 4R enantiomers of 1-{3-[4-(4,6-difluoro-1,3-benzoxazol-2-yl)-1H,4H,5H,6H,7H-imidazo[4,5-c]pyridine-5-carbonyl]pyrazolo[1,5-a]pyridin-6-yl}piperidin-4-ol | C, D |
| 4S and 4R enantiomers of 2-[5-(1-cyclopropyl-1H-pyrazole-5-carbonyl)-1H,4H,5H,6H,7H-imidazo[4,5-c]pyridin-4-yl]-7-methyl-1,3-benzoxazole | C, E |
| 4S and 4R enantiomers of 5-fluoro-2-[5-[2-(1-methyl-1H-pyrazol-4-yl)-4-(trifluoromethyl)-1,3-oxazole-5-carbonyl]-1H,4H,5H,6H,7H-imidazo[4,5-c]pyridin-4-yl]-1,3-benzoxazole | E, E |
| 4S and 4R enantiomers of 2-[5-[4-(difluoromethyl)-1,3-oxazole-5-carbonyl]-7,7-dimethyl-1H,4H,5H,6H,7H-imidazo[4,5-c]pyridin-4-yl]-5-fluoro-1,3-benzoxazole | D, D |
| 4S and 4R enantiomers of 2-[5-{ [4-(difluoromethyl)-1,3-oxazol-5-yl]methyl}-1H,4H,5H,6H,7H-imidazo[4,5-c]pyridin-4-yl]-5-fluoro-1,3-benzoxazole | D, E |
| 4S and 4R enantiomers of 2-[4-(difluoromethyl)-5-{ [4-(5-fluoro-1,3-benzoxazol-2-yl)-1H,4H,5H,6H,7H-imidazo[4,5-c]pyridin-5-yl]methyl}-1,3-oxazol-2-yl]propan-2-ol | D, E |
| 4S and 4R enantiomers of 1-{3-[4-(4,5-difluoro-1,3-benzoxazol-2-yl)-1H,4H,5H,6H,7H-imidazo[4,5-c]pyridine-5-carbonyl]pyrazolo[1,5-a]pyridin-6-yl}piperidin-4-ol | D, E |
| 4S and 4R enantiomers of 2-{5-[4-(5-methyl-1,3-benzoxazol-2-yl)-1H,4H,5H,6H,7H-imidazo[4,5-c]pyridine-5-carbonyl]-1,3,4-oxadiazol-2-yl}propan-2-ol | C, D |
| 4S and 4R enantiomers of 2-{5-[4-[5-(trifluoromethyl)-1,3-benzoxazol-2-yl]-1H,4H,5H,6H,7H-imidazo[4,5-c]pyridine-5-carbonyl]-1,3,4-oxadiazol-2-yl}propan-2-ol | E, E |
| 4S and 4R enantiomers of 5-methoxy-2-[5-(1-methyl-1H-1,2,4-triazole-5-carbonyl)-1H,4H,5H,6H,7H-imidazo[4,5-c]pyridin-4-yl]-1,3-benzoxazole | E, E |
| 4S and 4R enantiomers of 7-methyl-2-[5-(1-methyl-1H-1,2,4-triazole-5-carbonyl)-1H,4H,5H,6H,7H-imidazo[4,5-c]pyridin-4-yl]-1,3-benzoxazole | C, C |
| 4S and 4R enantiomers of 2-[5-(1,3-dimethyl-1H-1,2,4-triazole-5-carbonyl)-1H,4H,5H,6H,7H-imidazo[4,5-c]pyridin-4-yl]-1,3-benzoxazole | C, C |
| 4S and 4R enantiomers of 2-[5-(1,3-dimethyl-1H-1,2,4-triazole-5-carbonyl)-1H,4H,5H,6H,7H-imidazo[4,5-c]pyridin-4-yl]-4,7-difluoro-1,3-benzoxazole | C, C |
| 4S and 4R enantiomers of 2-[5-(1,3-dimethyl-1H-1,2,4-triazole-5-carbonyl)-1H,4H,5H,6H,7H-imidazo[4,5-c]pyridin-4-yl]-4-fluoro-1,3-benzoxazole | C, C |
| 4S and 4R enantiomers of 2-[5-(3-tert-butyl-1-methyl-1H-pyrazole-5-carbonyl)-1H,4H,5H,6H,7H-imidazo[4,5-c]pyridin-4-yl]-1,3-benzoxazole | C, D |
| 4S and 4R enantiomers of 2-[5-(3-tert-butyl-1-methyl-1H-pyrazole-5-carbonyl)-1H,4H,5H,6H,7H-imidazo[4,5-c]pyridin-4-yl]-7-fluoro-1,3-benzoxazole | C, D |
| 4S and 4R enantiomers of 2-[5-(3-tert-butyl-1-methyl-1H-pyrazole-5-carbonyl)-1H,4H,5H,6H,7H-imidazo[4,5-c]pyridin-4-yl]-7-methyl-1,3-benzoxazole | C, C |
| 4S and 4R enantiomers of 4,7-difluoro-2-[5-(1-methyl-1H-1,2,4-triazole-5-carbonyl)-1H,4H,5H,6H,7H-imidazo[4,5-c]pyridin-4-yl]-1,3-benzoxazole | C, C |
| 4S and 4R enantiomers of 2-[5-[1-(pyridin-2-yl)-1H-pyrazole-3-carbonyl]-1H,4H,5H,6H,7H-imidazo[4,5-c]pyridin-4-yl]-1,3-benzoxazole | C, D |
| 4S and 4R enantiomers of 7-fluoro-2-[5-[1-(pyridin-2-yl)-1H-pyrazole-3-carbonyl]-1H,4H,5H,6H,7H-imidazo[4,5-c]pyridin-4-yl]-1,3-benzoxazole | C, D |
| 4S and 4R enantiomers of 7-methyl-2-[5-[1-(pyridin-2-yl)-1H-pyrazole-3-carbonyl]-1H,4H,5H,6H,7H-imidazo[4,5-c]pyridin-4-yl]-1,3-benzoxazole | C, D |
| 4S and 4R enantiomers of 2-[5-(3-tert-butyl-1-methyl-1H-pyrazole-5-carbonyl)-1H,4H,5H,6H,7H-imidazo[4,5-c]pyridin-4-yl]-7-methoxy-1,3-benzoxazole | C, D |
| 4S and 4R enantiomers of 2-[5-(1,3-dimethyl-1H-1,2,4-triazole-5-carbonyl)-1H,4H,5H,6H,7H-imidazo[4,5-c]pyridin-4-yl]-5-methyl-1,3-benzoxazole | C, D |
| 4S and 4R enantiomers of 4,7-difluoro-2-[5-[5-(1-methyl-1H-imidazol-5-yl)-1,3,4-oxadiazole-2-carbonyl]-1H,4H,5H,6H,7H-imidazo[4,5-c]pyridin-4-yl]-1,3-benzoxazole | C, D |
| 4S and 4R enantiomers of 2-{5-[4-(6-methyl-1,3-benzoxazol-2-yl)-1H,4H,5H,6H,7H-imidazo[4,5-c]pyridine-5-carbonyl]-1,3,4-oxadiazol-2-yl}propan-2-ol | C, D |
| 4S and 4R enantiomers of 2-[5-(1,3-dimethyl-1H-1,2,4-triazole-5-carbonyl)-1H,4H,5H,6H,7H-imidazo[4,5-c]pyridin-4-yl]-5-methoxy-1,3-benzoxazole | C, C |
| 4S and 4R enantiomers of 2-[5-[5-(1-methyl-1H-imidazol-4-yl)-1,3,4-oxadiazole-2-carbonyl]-1H,4H,5H,6H,7H-imidazo[4,5-c]pyridin-4-yl]-7-(trifluoromethyl)-1,3-benzoxazole | C, C |
| 4S and 4R enantiomers of 2-[5-[5-(1-methyl-1H-imidazol-5-yl)-1,3,4-oxadiazole-2-carbonyl]-1H,4H,5H,6H,7H-imidazo[4,5-c]pyridin-4-yl]-7-(trifluoromethyl)-1,3-benzoxazole | C, C |
| 4S and 4R enantiomers of 2-[5-[1-(2,2-difluoroethyl)-1H-pyrazole-3-carbonyl]-1H,4H,5H,6H,7H-imidazo[4,5-c]pyridin-4-yl]-1,3-benzoxazole | C, E |
| 4S and 4R enantiomers of 2-{5-[4-(4,7-difluoro-1,3-benzoxazol-2-yl)-1H,4H,5H,6H,7H-imidazo[4,5-c]pyridine-5-carbonyl]-1,3,4-oxadiazol-2-yl}propan-2-ol | C, C |
| 4S and 4R enantiomers of 2-[5-{5-[1-(difluoromethyl)-1H-pyrazol-4-yl]-1,3,4-oxadiazole-2-carbonyl}-1H,4H,5H,6H,7H-imidazo[4,5-c]pyridin-4-yl]-7-(trifluoromethyl)-1,3-benzoxazole | C, C |
| 4S and 4R enantiomers of 2-[5-[1-(2,2-difluoroethyl)-1H-pyrazole-3-carbonyl]-1H,4H,5H,6H,7H-imidazo[4,5-c]pyridin-4-yl]-7-fluoro-1,3-benzoxazole | C, C |
| 4S and 4R enantiomers of 2-[5-[1-(2,2-difluoroethyl)-1H-pyrazole-3-carbonyl]-1H,4H,5H,6H,7H-imidazo[4,5-c]pyridin-4-yl]-4-fluoro-1,3-benzoxazole | C, C |
| 4S and 4R enantiomers of 4,7-difluoro-2-[5-(5-methyl-1,3,4-oxadiazole-2-carbonyl)-1H,4H,5H,6H,7H-imidazo[4,5-c]pyridin-4-yl]-1,3-benzoxazole | C, C |
| 4S and 4R enantiomers of 2-{5-[4-(5-fluoro-1,3-benzoxazol-2-yl)-1H,4H,5H,6H,7H-imidazo[4,5-c]pyridine-5-carbonyl]-1,2-oxazol-3-yl}propan-2-ol | C, C |
| (4S, 7S), (4S, 7R), (4R, 7S) and (4R, 7R) diastereomers of 2-[4-(difluoromethyl)-5-[(4R,7S)-4-(5-fluoro-1,3-benzoxazol-2-yl)-7-methyl-1H,4H,5H,6H,7H-imidazo[4,5-c]pyridine-5-carbonyl]-1,3-oxazol-2-yl]propan-2-ol | C, C, C, D |
| 5-fluoro-2-(5-{5-[2-(3-fluoroazetidin-1-yl)ethyl]-1,3,4-oxadiazole-2-carbonyl}-1H,4H,5H,6H,7H-imidazo[4,5-c]pyridin-4-yl)-1,3-benzoxazole | D |
| 2-{5-[4-(difluoromethyl)-2-(2-hydroxypropan-2-yl)-1,3-oxazole-5-carbonyl]-1H,4H,5H,6H,7H-imidazo[4,5-c]pyridin-4-yl}-1,3-benzoxazole-5-carbonitrile | C |

## Claims

1. A compound of Formula I: 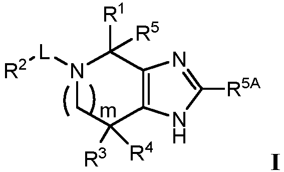 or a pharmaceutically acceptable salt thereof, wherein:
m is 0, 1 or 2;
R¹ is 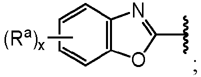
x is 0 to 4;
each R^{a} independently is halo, C₁₋₆alkyl, C₃₋₆cycloalkyl, C₁₋₆haloalkyl, C₁₋₆alkoxy or C₁₋₆haloalkoxy;
R³ is H or C₁₋₆alkyl;
R⁴ is H or C₁₋₆alkyl;
or R³ and R⁴, together with the atom to which they are attached, form a C₃₋₆cycloalkyl;
R⁵ is H or D;
R^{5A} is H or D;
L is a bond, -C(O)-, -C(O)CH₂-, -C(O)CH₂CH₂-, -C(O)CH₂CH₂CH₂-, -C(O)CF₂-, -C(O)CHF-, -C(O)C(CH₃)₂-, -C(O)CH=CH-, 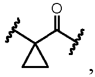 -C(O)NHCH₂-, -CH₂-, or -CH₂CH₂-; and
R² is:
(a) 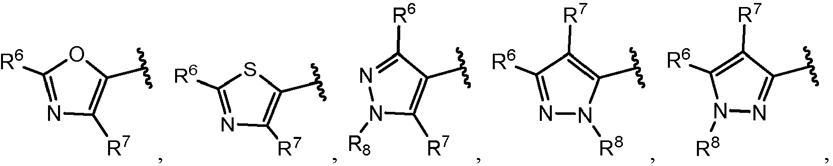 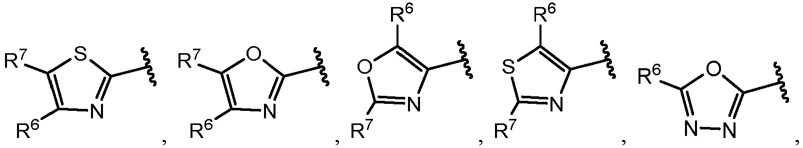 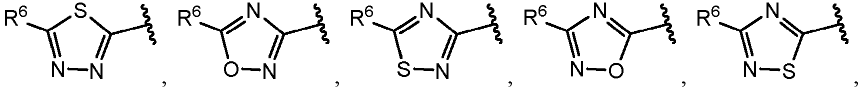 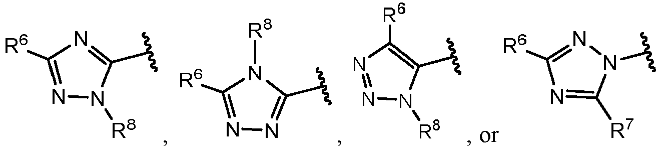 wherein:
R⁶ is H; CN; C₁₋₆alkyl;C₁₋₆haloalkyl; C₁₋₆cyanoalkyl; C₁₋₆hydroxyalkyl; C₁₋₆alkoxy; C₁₋₆alkoxy(alkylene); C₁₋₆haloalkoxy; C₁₋₆haloalkoxy(alkylene); C₁₋₆deuteratedalkoxy(alkylene); halo; (CR^{v}R^{x})ₚNR^{y}R^{z}; C(O)NR^{y2}R^{z2}; C₁₋₆alkylcarbonyl; C₃₋₆cycloalkyl which is optionally substituted with one or more of halo, OH, C₁₋₆haloalkyl, C₁₋₆alkyl, or C₁₋₆alkoxy; heterocyclyl which is optionally substituted with one or more of halo, OH, C₁₋₆haloalkyl, C₁₋₆alkyl, C₁₋₆hydroxyalkyl, C₁₋₆alkoxy, C(O)O(C₁₋₆alkyl), or C₃₋₆cycloalkyl; aryl which is optionally substituted with one or more of halo, C₁₋₆haloalkyl, C₁₋₆alkyl, or C₃₋₆cycloalkyl; heteroaryl which is optionally substituted with one or more of halo, C₁₋₆haloalkyl, C₁₋₆alkyl, C₁₋₆alkoxy, C₁₋₆haloalkoxy, C₃₋₆cycloalkyl, or C₃₋₆cycloalkylsulfonyl; C₂₋₆alkenyl; C₃₋₈cycloalkenyl; C₃₋₈cycloalkyl(alkylene); aryl(alkylene); heterocyclyl(alkylene) which is optionally substituted with one or more of halo, OH, C₁₋₆haloalkyl, C₁₋₆alkyl, C₁₋₆hydroxyalkyl, C₁₋₆alkoxy, C(O)O(C₁₋₆alkyl), or C₃₋₆cycloalkyl; or C₁₋₆alkylsulfonyl,
R⁷ is H, CN, C₁₋₆alkyl, C₁₋₆haloalkyl, C₁₋₆cyanoalkyl, C₁₋₆hydroxyalkyl, C₁₋₆alkoxy, C₁₋₆alkoxy(alkylene), C₁₋₆haloalkoxy, C₁₋₆haloalkoxy(alkylene), C₁₋₆deuteratedalkoxy(alkylene), halo, (CR^{v}R^{x})ₚNR^{y}R^{z}, C(O)NR^{y2}R^{z2}, C₁₋₆alkylcarbonyl, C₃₋₆cycloalkyl, heterocyclyl, aryl, heteroaryl, C₂₋₆alkenyl; C₃₋₈cycloalkenyl; C₃₋₈cycloalkyl(alkylene); aryl(alkylene); heterocyclyl(alkylene); or C₁₋₆alkylsulfonyl,
R⁸ is H, C₁₋₆alkyl, C₁₋₆haloalkyl, C₁₋₆alkoxy(alkylene), C₁₋₆alkylcarbonyl, C₁₋₆hydroxyalkyl, (CR^{v}R^{x})ₚNR^{y}R^{z}, C₃₋₈cycloalkyl, aryl, heteroaryl, or C₃₋₈cycloalkyl(alkylene);
R^{v} and R^{x} are, independently, H or C₁₋₆alkyl;
R^{y} and R^{z} are, independently, H, C₁₋₆alkyl, C₁₋₆alkoxy(alkylene), or C₃₋₆cycloalkyl;
R^{y2} and R^{z2} are, independently, H, C₁₋₆alkyl, or C₃₋₆cycloalkyl; and
p is 0, 1, 2, or 3; or
(b) 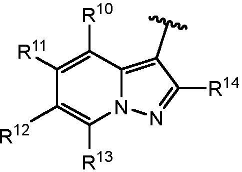 wherein:
R¹⁰, R¹¹, R¹², R¹³ and R¹⁴ are, independently, H, C₁₋₆alkyl, C₁₋₆haloalkyl, C₁₋₆alkoxy, C₁₋₆alkoxy(alkylene), halo, optionally substituted heterocyclyl, optionally substituted heterocyclyl(alkylene), optionally substituted heteroaryl, or (CR^{v}R^{x})ₚNR^{y}R^{z}, wherein the optional substitution on the heterocyclyl and heterocyclyl(alkylene) groups is one or more of halo, C₁₋₆haloalkyl, C₁₋₆alkyl, OH, C₁₋₆hydroxyalkyl, C₁₋₆alkoxy, or C₃₋₆cycloalkyl and the optional substitution on the heteroaryl group is one or more C₁₋₆alkyl;
R^{v} and R^{x} are, independently, H or C₁₋₆alkyl;
R^{y} and R^{z} are, independently, H, C₁₋₆alkyl, C₃₋₆cycloalkyl, or C(O)OC₁₋₆alkyl; and
p is 0, 1, 2, or 3.

2. The compound of claim 1, wherein the compound is of:
(i) Formula I-A-1, 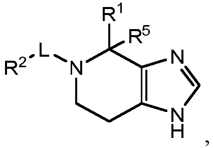 or a pharmaceutically acceptable salt thereof; or
(ii) Formula I-A-3, 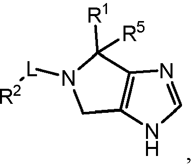 or a pharmaceutically acceptable salt thereof; or
(iii) Formula I-B-1, 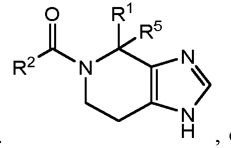 or a pharmaceutically acceptable salt thereof; or
(iv) Formula I-B-3, 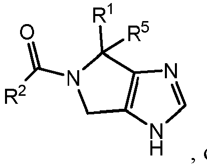 or a pharmaceutically acceptable salt thereof; or
(v) Formula I-C-1, 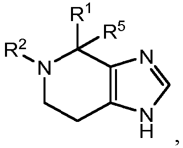 or a pharmaceutically acceptable salt thereof; or
(vi) Formula I-C-3, 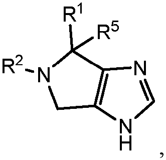 or a pharmaceutically acceptable salt thereof; or
(vii) Formula I-D-1, 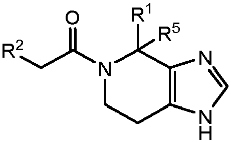 , or a pharmaceutically acceptable salt thereof; or
(viii) Formula I-G-1, 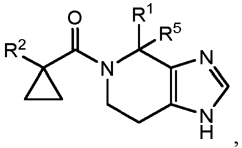 or a pharmaceutically acceptable salt thereof; or
(ix) Formula I-I-1, 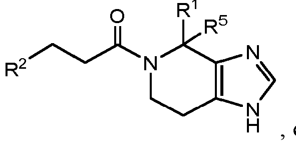 or a pharmaceutically acceptable salt thereof; or
(x) Formula I-L-1, 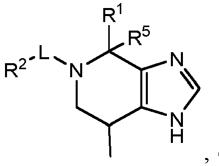 or a pharmaceutically acceptable salt thereof; or
(xi) Formula I-M-1, 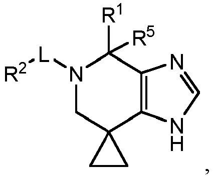 or a pharmaceutically acceptable salt thereof.

3. The compound of claim 1 or claim 2, or a pharmaceutically acceptable salt thereof, wherein:
(i) R¹ is 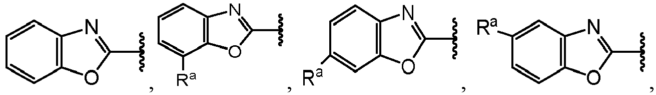 or 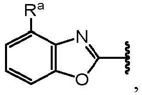 optionally wherein
a. R^{a} is halo such as F, Br, or Cl;
b. R^{a} is C₁₋₆alkyl such as methyl;
c. R^{a} is C₁₋₆alkoxy such as methoxy;
d. R^{a} is C₁₋₆haloalkyl such as CF₃; or
e. R^{a} is C₁₋₆haloalkoxy such as OCF₃; or
(ii) R¹ is 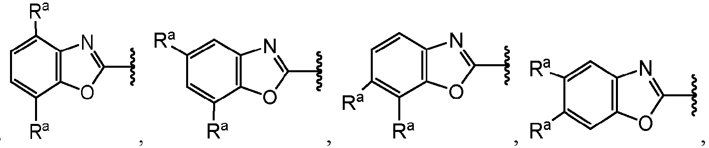 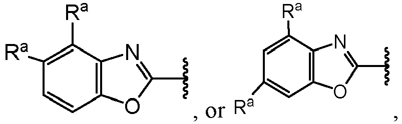 optionally wherein one R^{a} is halo such as Br, Cl, or F or C₁₋₆alkyl such as methyl, and the second R^{a} is halo such as Br, Cl, or F, C₁₋₆alkoxy, such as methoxy, or C₁₋₆alkyl, such as methyl.

4. The compound of any one of claims 1 to 3, or a pharmaceutically acceptable salt thereof, wherein:
(i) R² is (a), and R⁶, R⁷, R⁸ are each H; or
(ii) R² is (b), and R¹⁰, R¹³, and R¹⁴ are each H; or
(iii) R² is (b), and R¹⁰, R¹¹, R¹³, and R¹⁴ are each H; or
(iv) R² is (a), and R⁶ is C₁₋₆alkyl such as methyl, ethyl, isopropyl, or tert-butyl, C₁₋₆haloalkyl such as CF₃, CHF₂, CH₂F, CH₂CF₃, CH(CH₃)F, C(CH₃)F₂, or C(CH₃)₂F, C₁₋₆cyanoalkyl such as C(CH₃)₂CN, or C₁₋₆hydroxyalkyl such as C(CH₃)₂OH, CH(CH₃)OH, CH(CH₂CH₃)OH, CH₂C(CH₃)₂OH, or C(CH₃)₂CH₂OH; or
(v) R² is (a), and R⁶ is 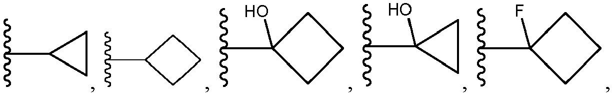 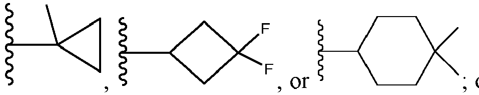 or
(vi) R² is (a), and R⁶ is phenyl, optionally substituted with one or more of halo such as F, Cl, or Br; C₁₋₆haloalkyl such as CF₃, CH₂CF₃, or CHF₂; C₁₋₆alkyl such as methyl, ethyl, or propyl; or C₃₋₆cycloalkyl such as cyclopropyl, cyclobutyl, or cyclopentyl, optionally wherein R⁶ is 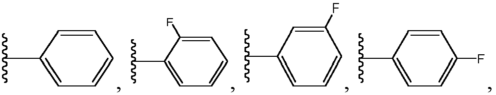 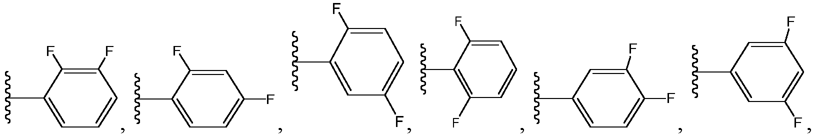 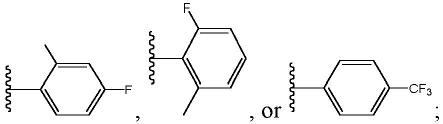 or
(vii) R² is (a), and R⁶ is pyridinyl, pyrazolyl, pyrazinyl, pyridazinyl, imidazolyl, or pyrimidinyl, each optionally substituted with one or more of halo such as F, Cl, or Br; C₁₋₆haloalkyl such as CF₃, CH₂CF₃, CH₂CHF₂, or CHF₂; C₁₋₆alkyl such as methyl, ethyl, propyl, or isopropyl; C₁₋₆alkoxy such as methoxy, ethoxy, or propoxy; C₁₋₆haloalkoxy such as OCF₃; C₃₋₆cycloalkyl such as cyclopropyl, cyclobutyl, or cyclopentyl; or C₃₋₆cycloalkylsulfonyl such as cyclopropylsulfonyl, cyclobutylsulfonyl, or cyclopentylsulfonyl;
optionally wherein (a) R⁶ is 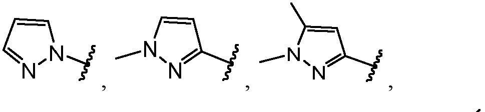 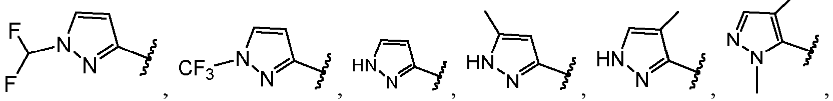 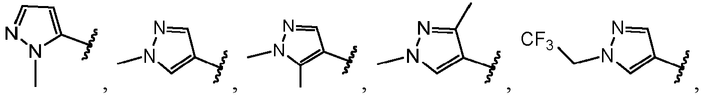 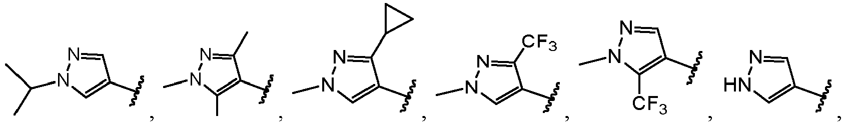 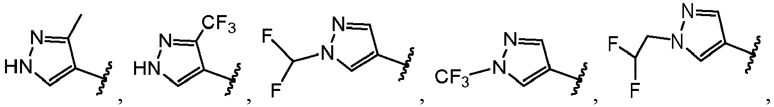 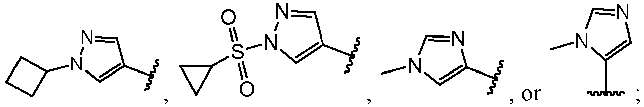 or
(b) R⁶ is 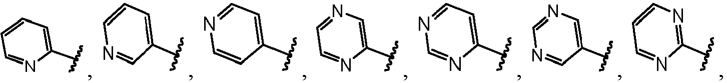 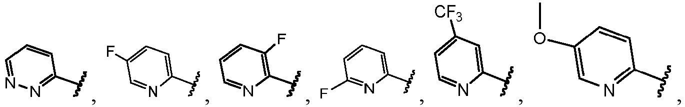 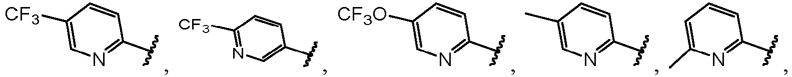 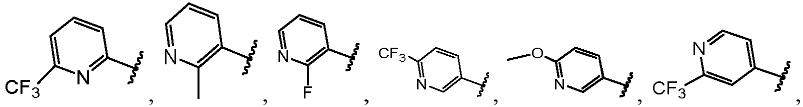 or 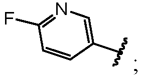 or
(viii) R² is (a), and R⁶ is azetidinyl, pyrrolidinyl, piperidinyl, piperazinyl, or morpholinyl, each optionally substituted with one or more of halo such as F, Cl, or Br; OH; C₁₋₆haloalkyl such as CF₃, CH₂CF₃, or CHF₂; C₁₋₆alkyl such as methyl, ethyl, or propyl; C₁₋₆hydroxyalkyl such as C(CH₃)₂OH; C₁₋₆alkoxy such as methoxy, ethoxy, or propoxy; C(O)O(C₁₋₆alkyl) such as C(O)Omethyl, C(O)Oethyl, C(O)Opropyl, or C(O)Otert-butyl; or C₃₋₆cycloalkyl such as cyclopropyl, cyclobutyl, or cyclopentyl; optionally wherein R⁶ is 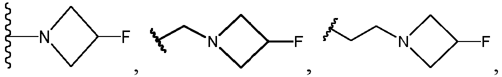 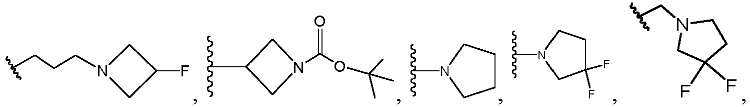 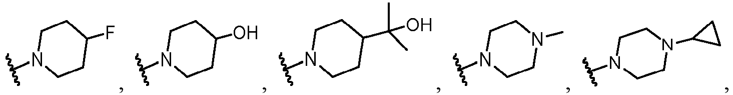 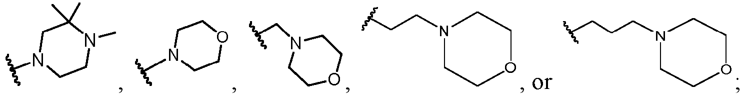 and/or
(ix) R² is (a), and R⁷ is H, CN, C₁₋₆alkyl such as methyl, C₁₋₆haloalkyl such as CF₃, CH₂F, CHF₂, CF₂(CH₃), or CH₂CHF₂, halo such as Br or Cl, C₃₋₈cycloalkyl such as cyclopropyl, phenyl, or pyridinyl.

5. The compound of any one of claims 1 to 3, or a pharmaceutically acceptable salt thereof, wherein R² is: 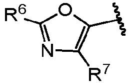 wherein:
R⁶ is H, C₁₋₆alkyl such as methyl, isopropyl, or tert-butyl, C₁₋₆haloalkyl such as C(CH₃)₂F, CH(CH₃)F, or CF₃, C₁₋₆cyanoalkyl such as C(CH₃)₂CN, C₁₋₆hydroxyalkyl such as C(CH₃)₂OH, CH₂C(CH₃)₂OH, CH(CH₂CH₃)OH, C(CH₃)₂(CH₂OH) or CH(CH₃)OH, C₁₋₆alkoxy such as methoxy, C₁₋₆alkoxy(alkylene) such as CH₂OCH₃, C(CH₃)₂OCH₃, or (CH₂)₂OCH₃, C₁₋₆haloalkoxy such as O(CH₂)₂CF₃, C₁₋₆haloalkoxy(alkylene) such CH₂OCF₃, C₁₋₆deuteratedalkoxy(alkylene) such as CH₂OCD₃, (CR^{v}R^{x})ₚNR^{y}R^{z} such as NH₂, CH₂N(CH₃)₂, (CH₂)₂N(CH₃)₂, C(CH₃)₂NHCH₃, C(CH₃)₂N(CH₃)₂, or NHcyclopropyl, C₃₋₆cycloalkyl which is optionally substituted with one or more OH such as 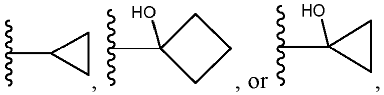 heterocyclyl which is optionally substituted with one or more of halo or C₁₋₆alkyl such as 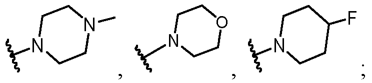 or heteroaryl which is optionally substituted with one or more of C₁₋₆alkyl, C₁₋₆haloalkyl, halo, or C₁₋₆alkoxy such as 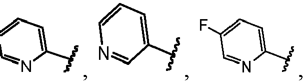 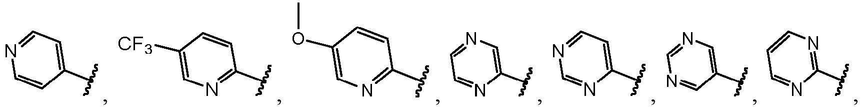 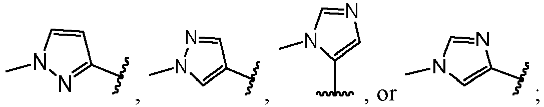
R⁷ is H, C₁₋₆alkyl such as methyl, C₁₋₆haloalkyl such as CHF₂, CH₂F, C(CH₃)F₂, CH₂CHF₂, or CF₃, halo such as Br or Cl, or C₃₋₆cycloalkyl such as cyclopropyl;
R^{v} and R^{x} are, independently, H or C₁₋₆alkyl such as methyl;
R^{y} and R^{z} are, independently, H, C₁₋₆alkyl such as methyl, or C₃₋₆cycloalkyl such as cyclopropyl; and
p is 0, 1, 2, or 3.

6. The compound of any one of claims 1 to 3, or a pharmaceutically acceptable salt thereof, wherein R² is: 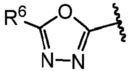 wherein:
R⁶ is H, C₁₋₆alkyl such as methyl or tert-butyl, C₁₋₆haloalkyl such as C(CH₃)₂F or CF₃, C₁₋₆hydroxyalkyl such as C(CH₃)₂OH, C₃₋₆cycloalkyl such as 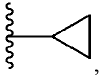 aryl which is optionally substituted with one or more of halo, C₁₋₆alkyl, or C₁₋₆haloalkyl such as 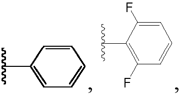 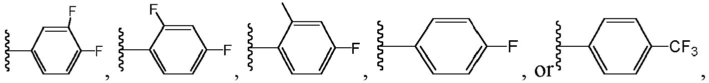 heteroaryl which is optionally substituted with one or more of C₁₋₆alkyl, C₁₋₆haloalkyl, halo, C₁₋₆alkoxy, C₃₋₆cycloalkyl, or C₃₋₆cycloalkylsulfonyl such as

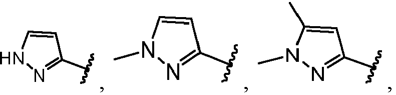

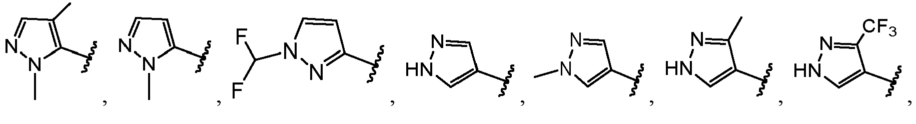

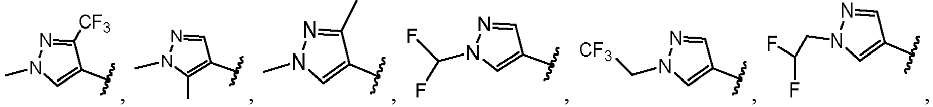

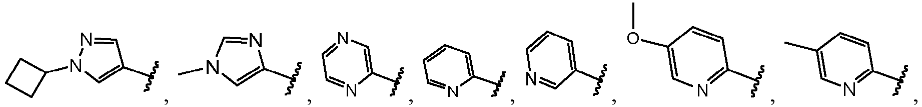

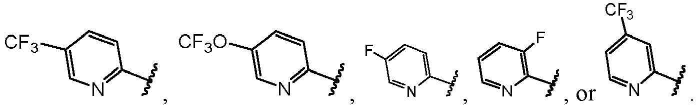

7. The compound of any one of claims 1 to 3 , or a pharmaceutically acceptable salt thereof, wherein R² is:
(a) 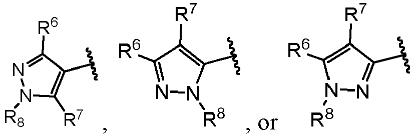 wherein:
R⁶ and R⁷ are, independently, H, halo such as Cl or Br, C₁₋₆alkyl such as methyl, or C₁₋₆haloalkyl such as CHF₂, CFH₂, or CF₃; optionally wherein at least one of R⁶ and R⁷ is H; and
R⁸ is H, C₁₋₆alkyl such as methyl, isopropyl, or tert-butyl, C₁₋₆haloalkyl such as CHF₂, CH₂CF₃, CH₂CHF₂, or CF₃, C₃₋₆cycloalkyl such as cyclopropyl, or heteroaryl such as pyridinyl; or
(b) 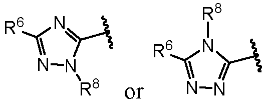 wherein:
R⁶ is H, C₁₋₆alkyl such as methyl, halo such as Cl or Br, C₁₋₆haloalkyl such as CHF₂ or CF₃, or C₃₋₆cycloalkyl such as cyclopropyl; and
R⁸ is H or C₁₋₆alkyl such as methyl, ethyl, or isopropyl.

8. The compound of any one of claims 1 to 7 that is an S-enantiomer, or a pharmaceutically acceptable salt thereof.

9. A compound, or a pharmaceutically acceptable salt thereof, wherein the compound is selected from the following:

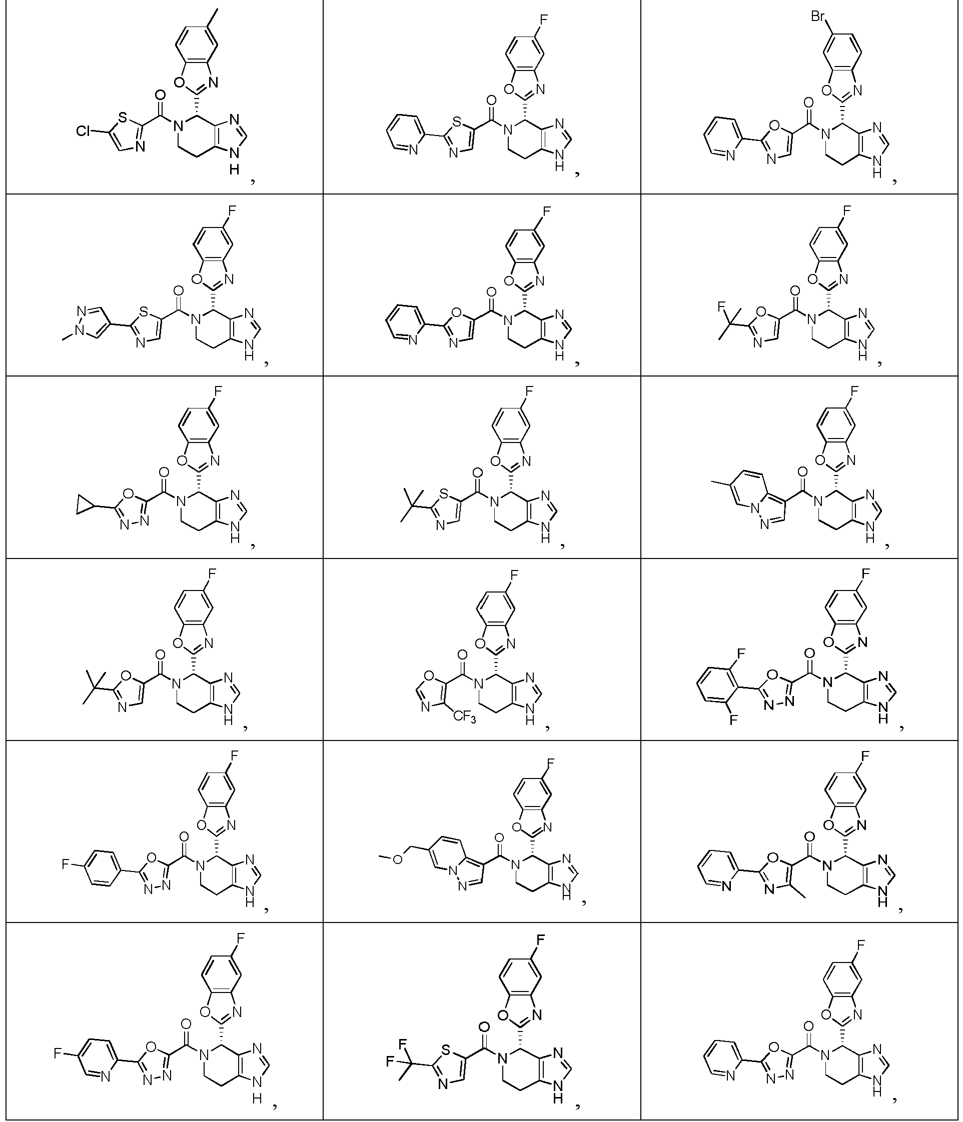

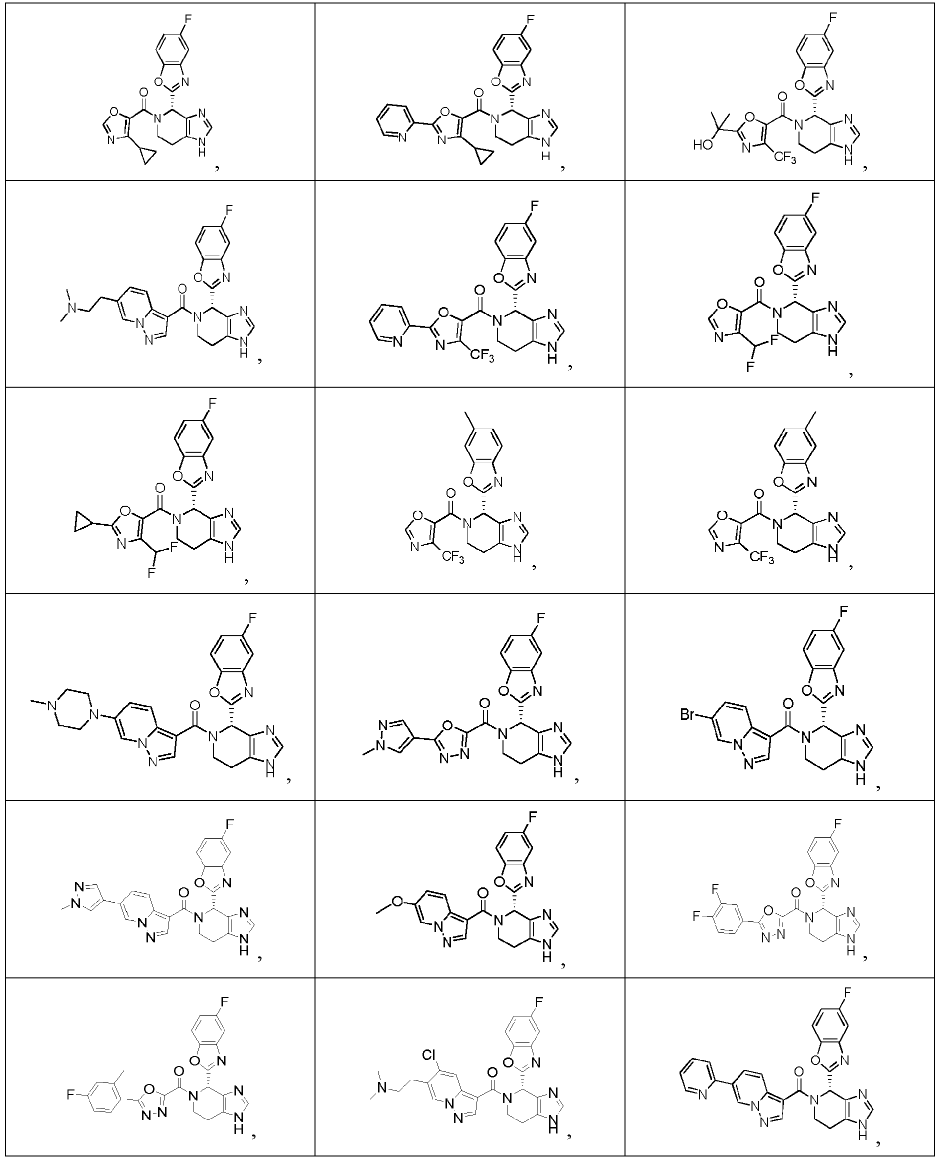

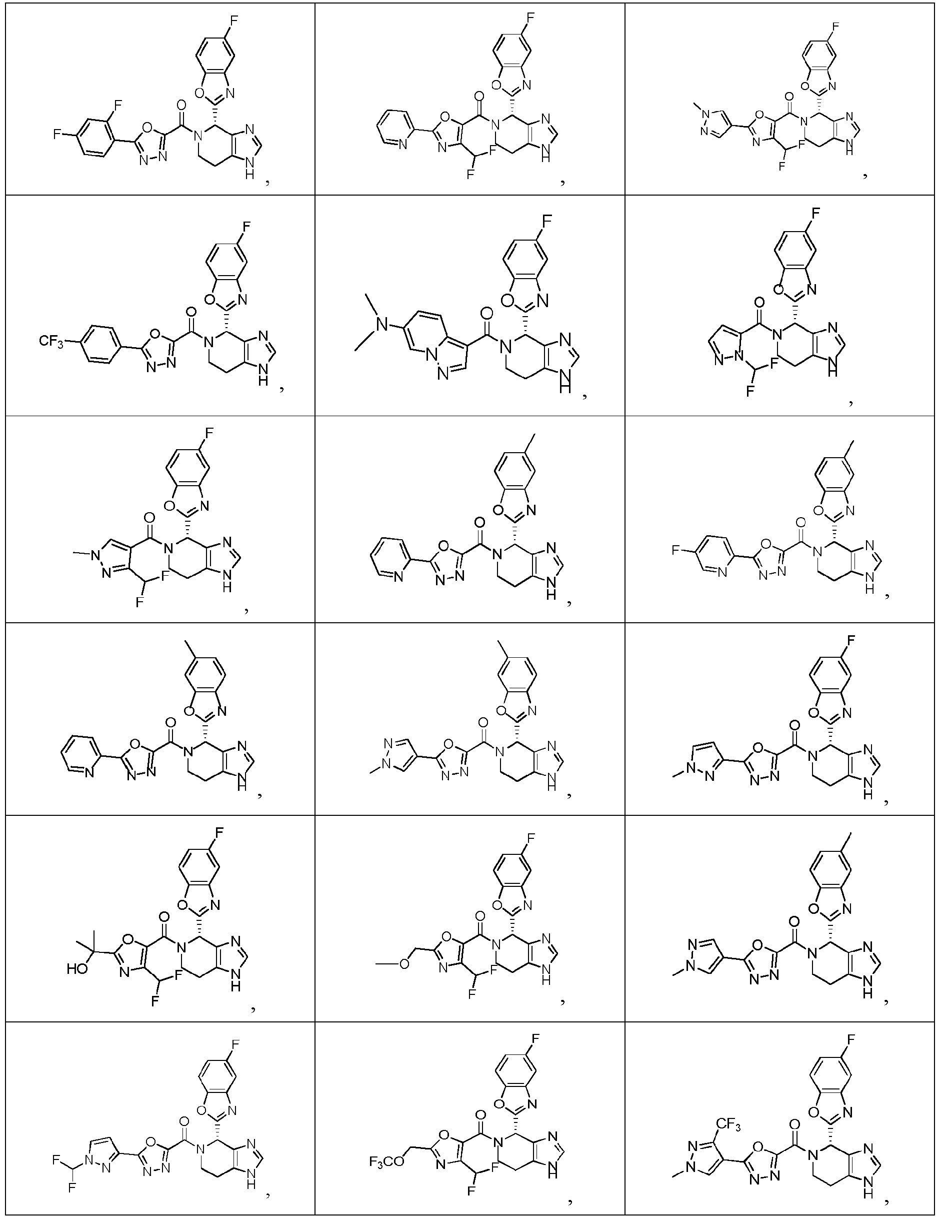

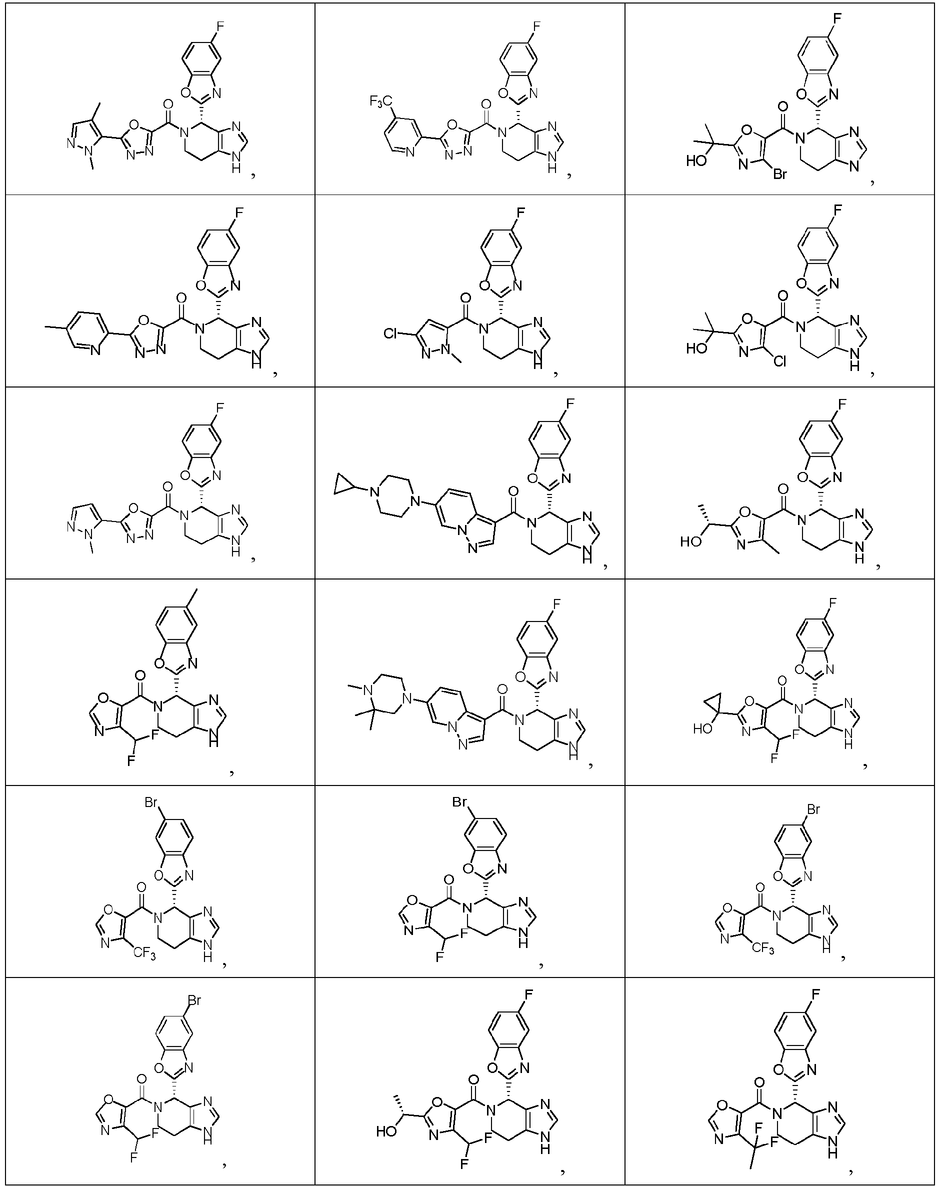

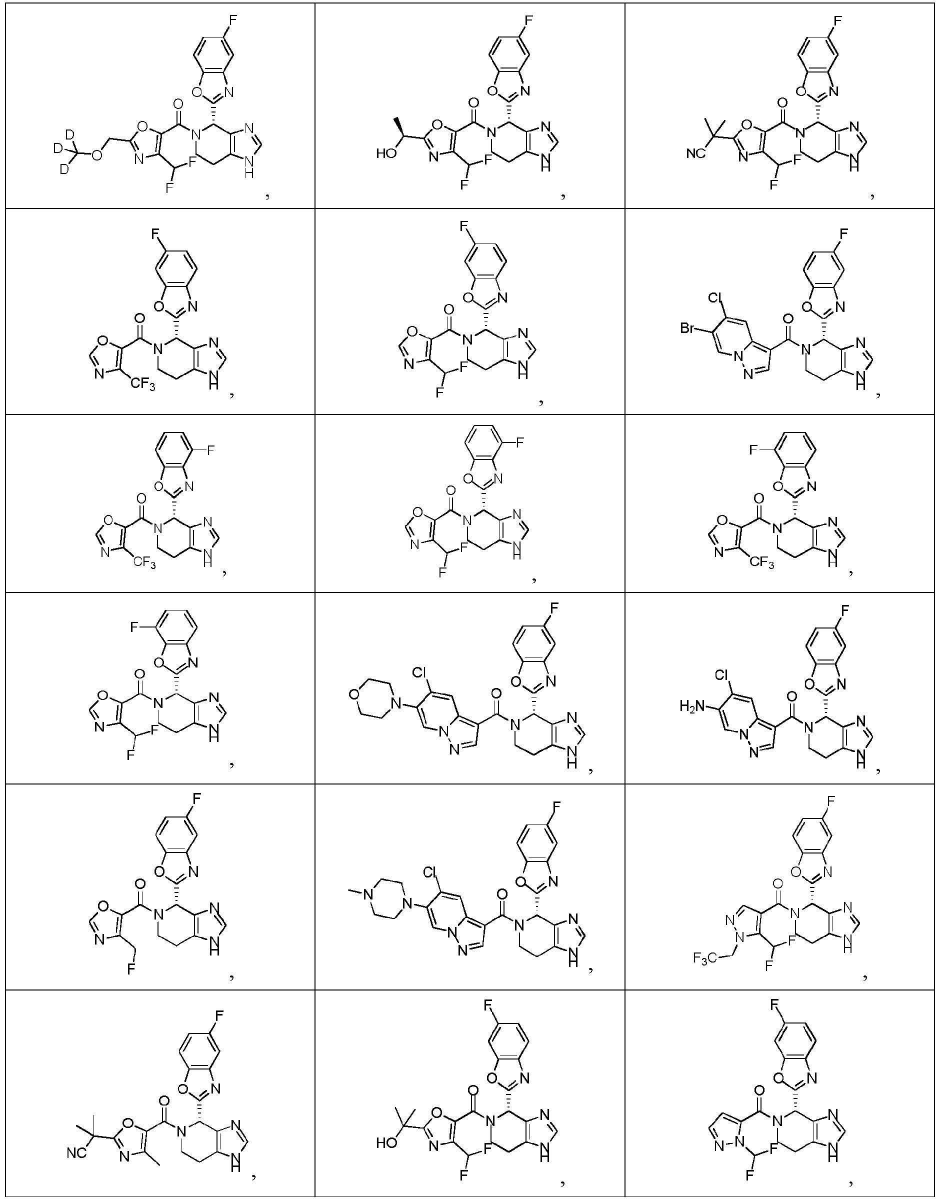

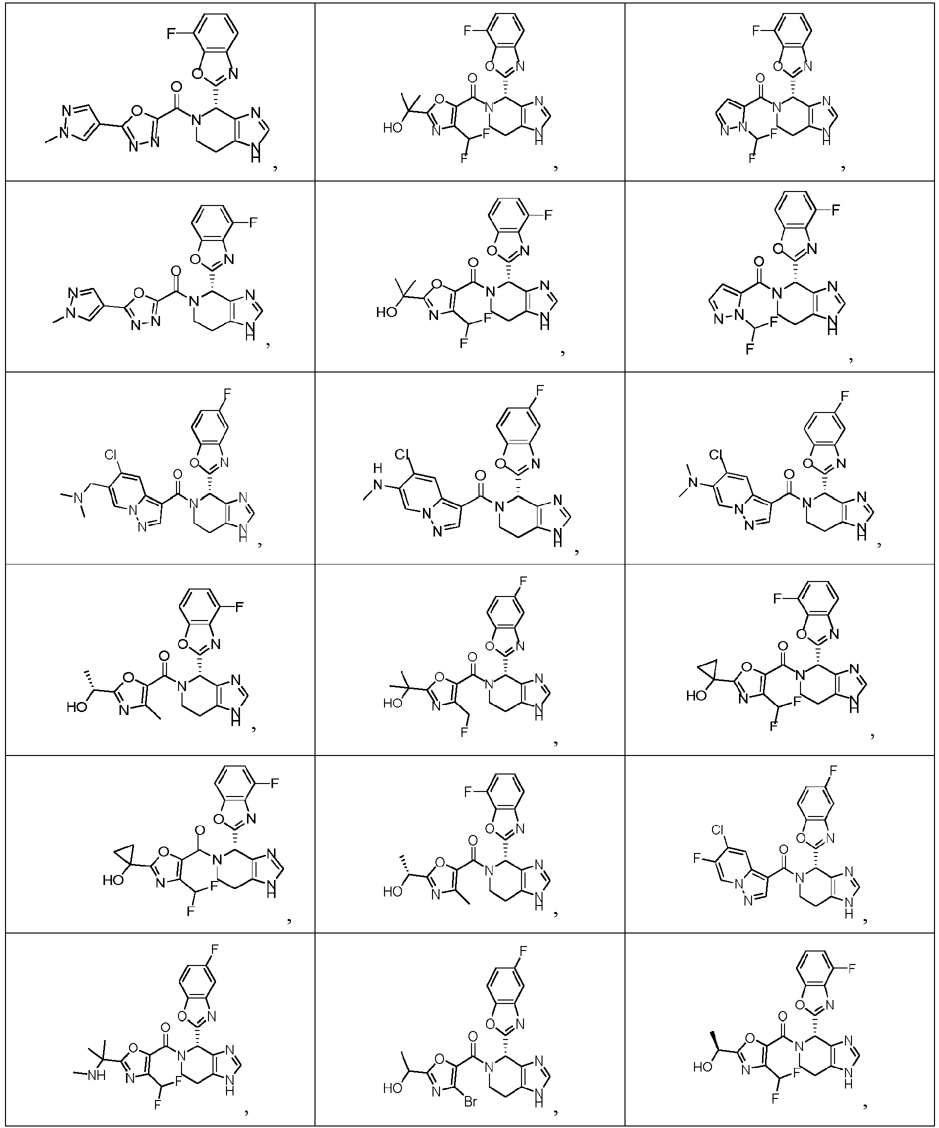

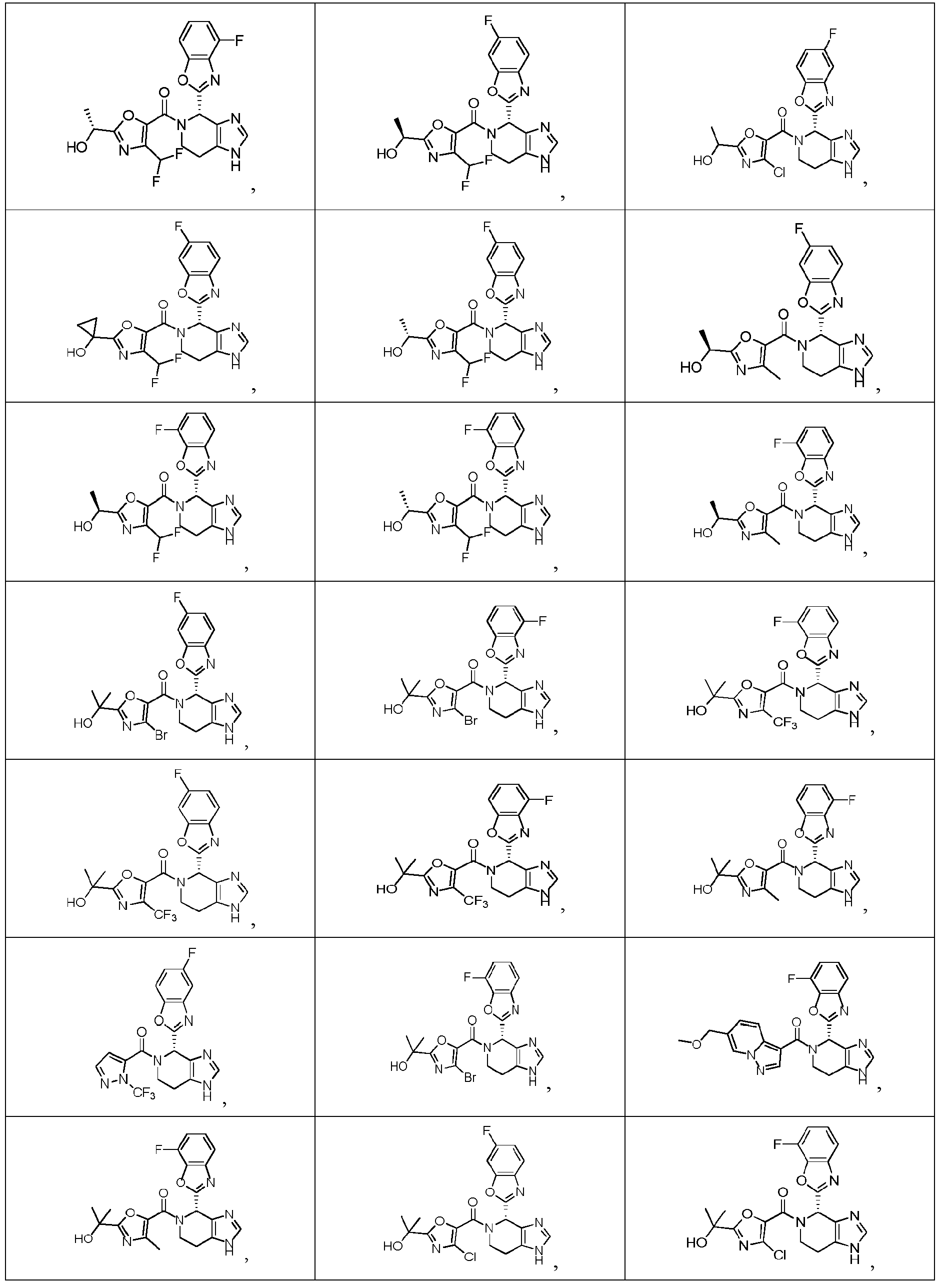

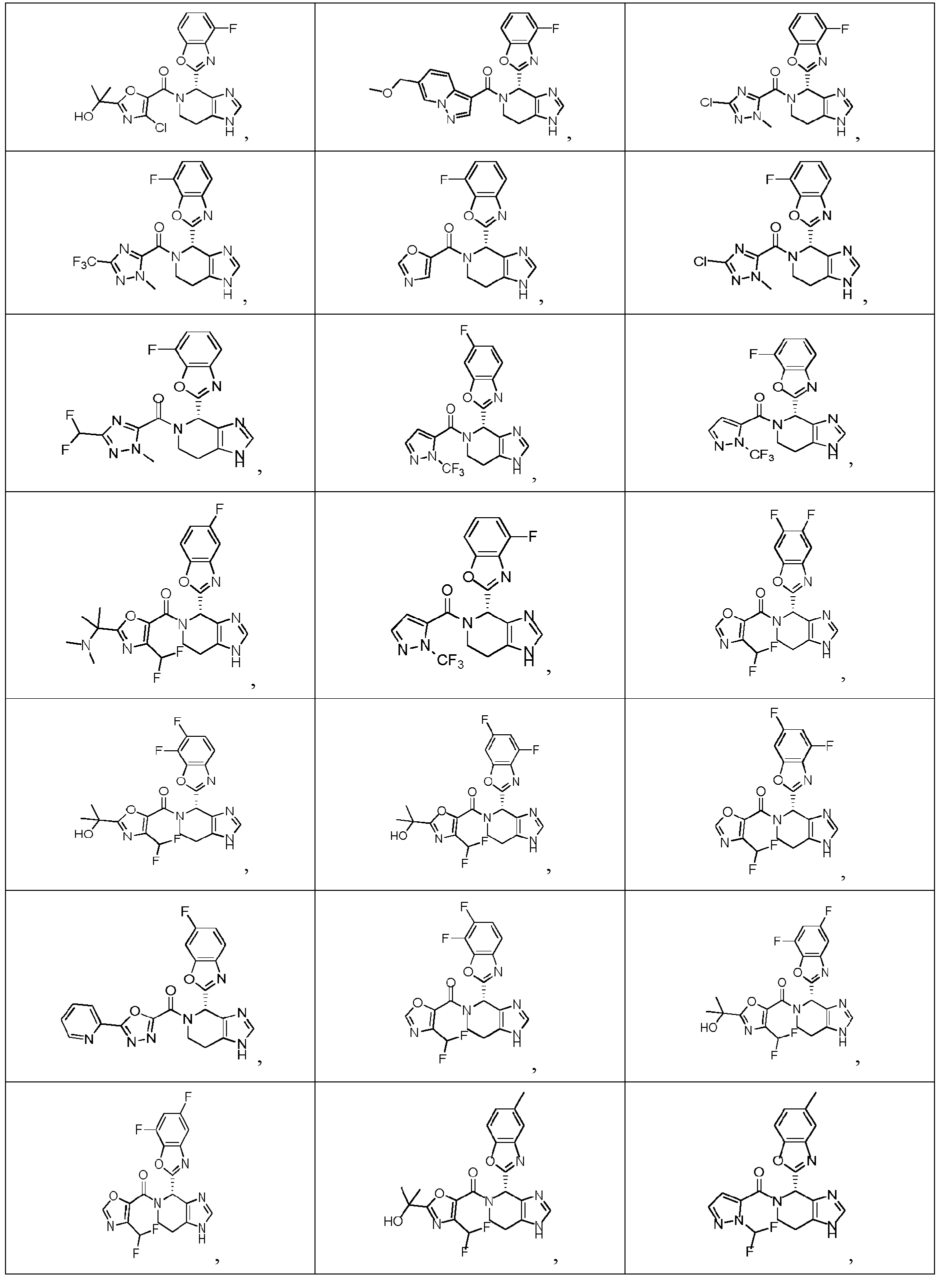

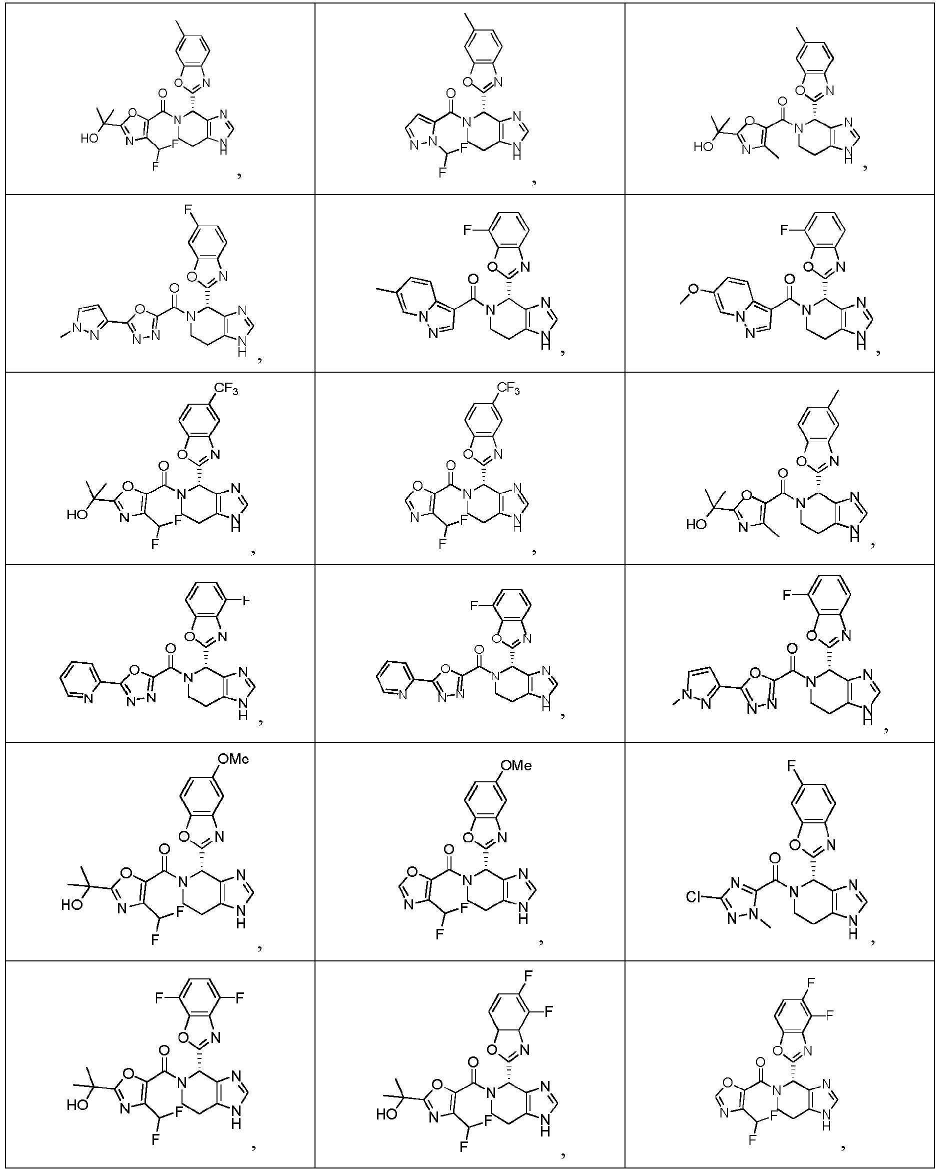

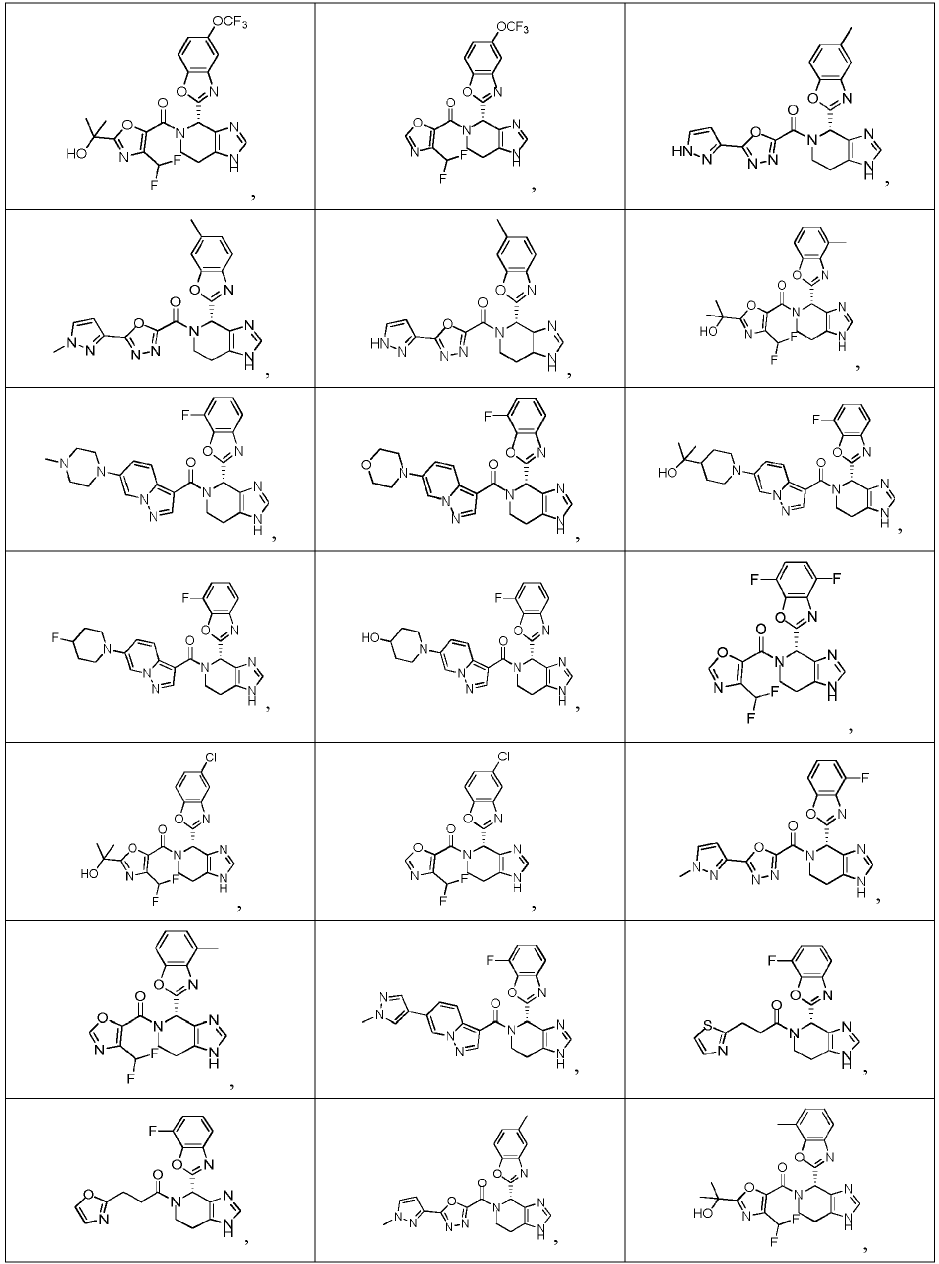

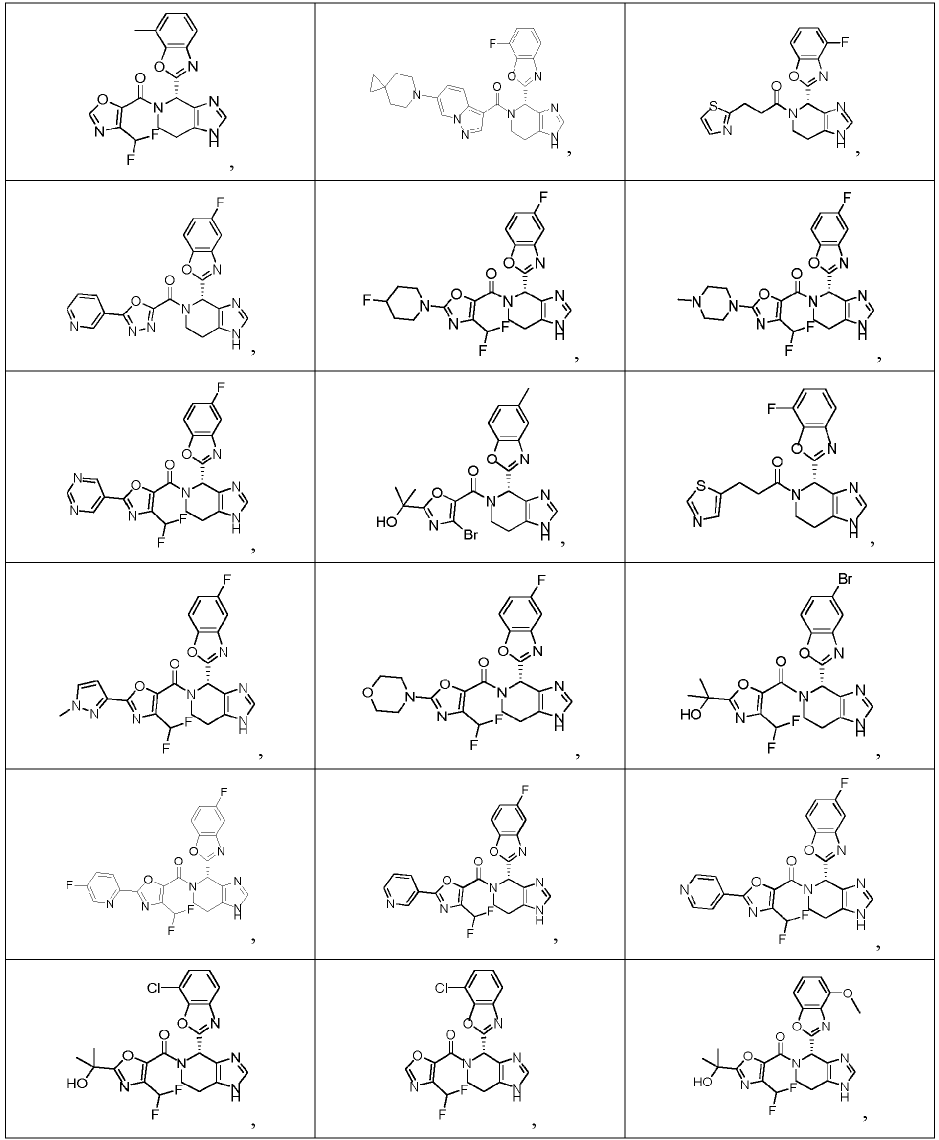

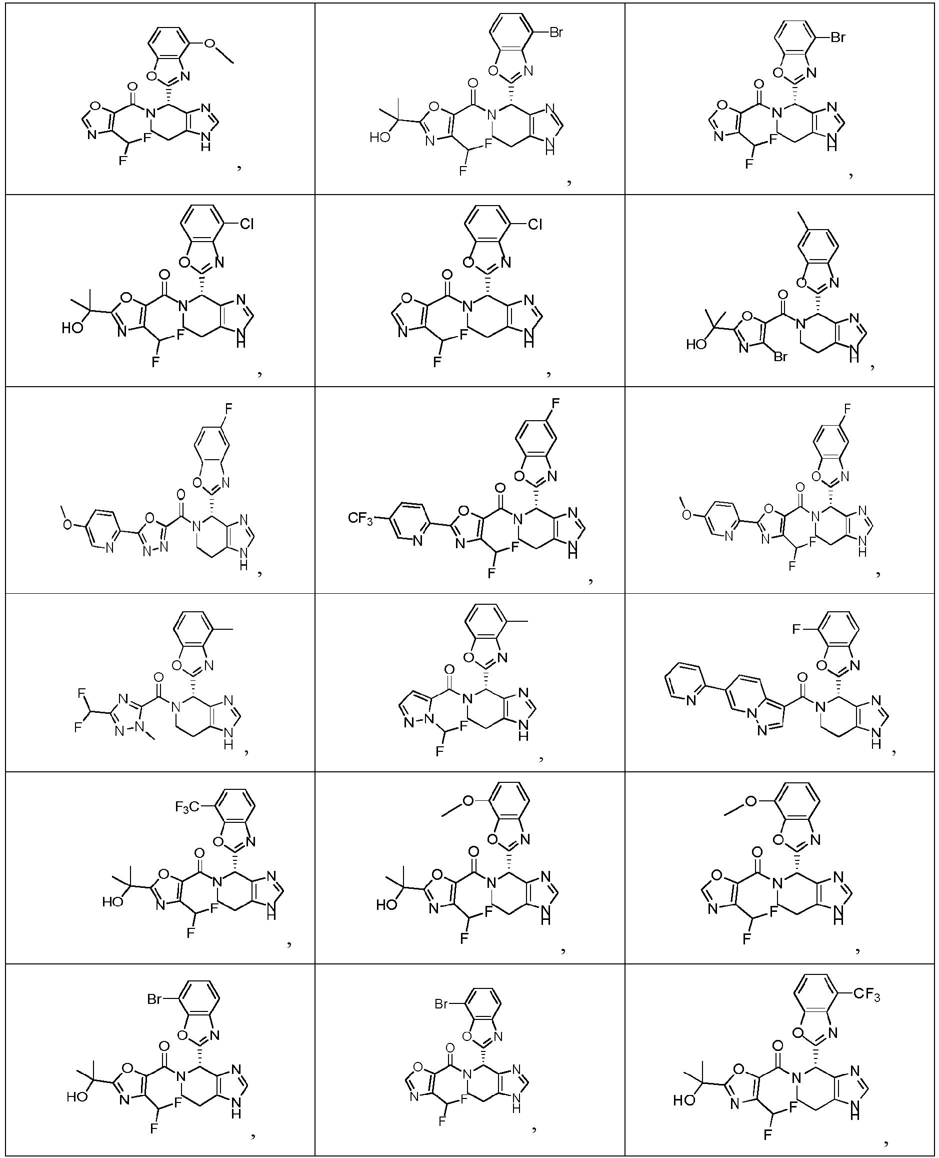

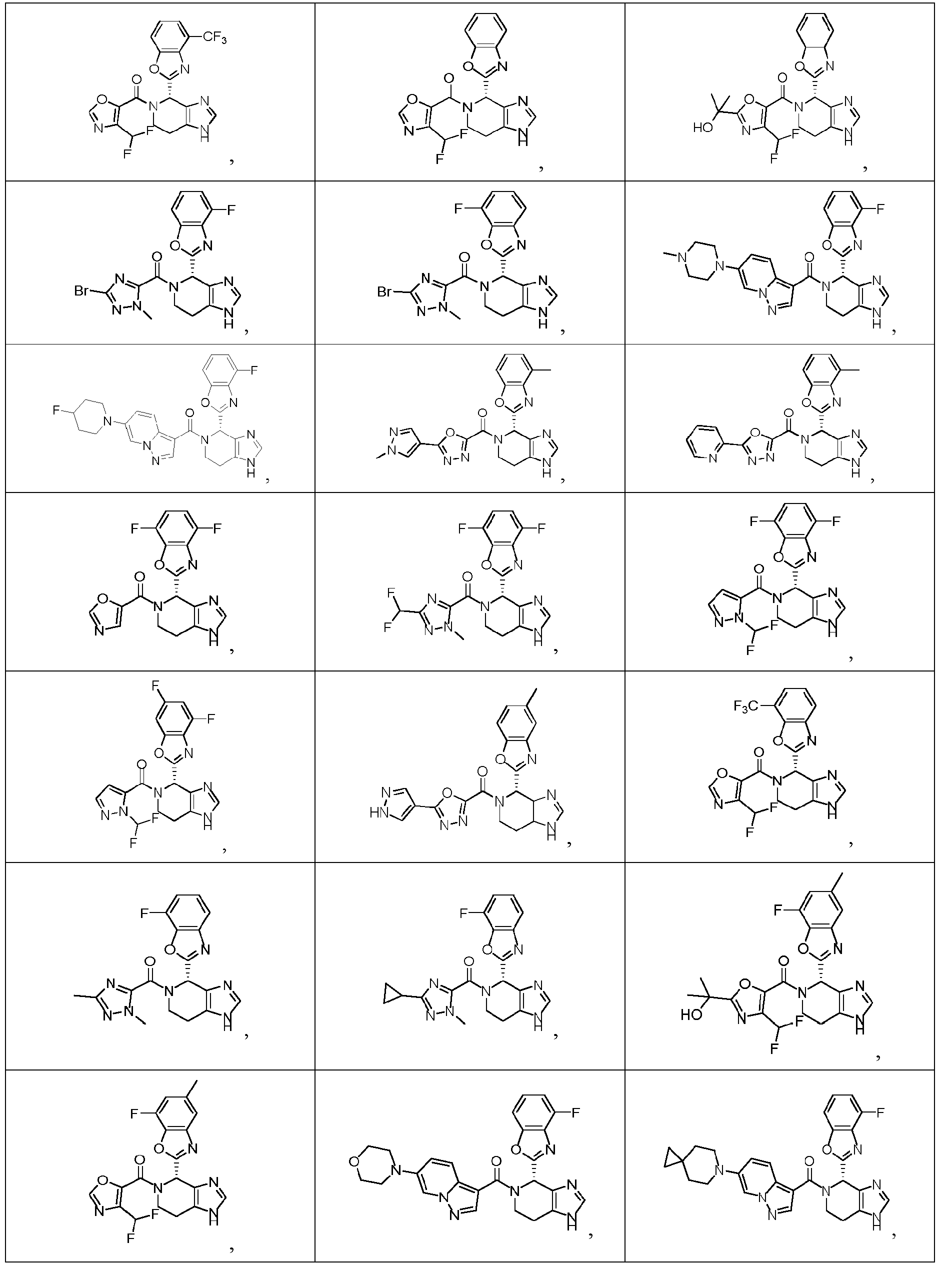

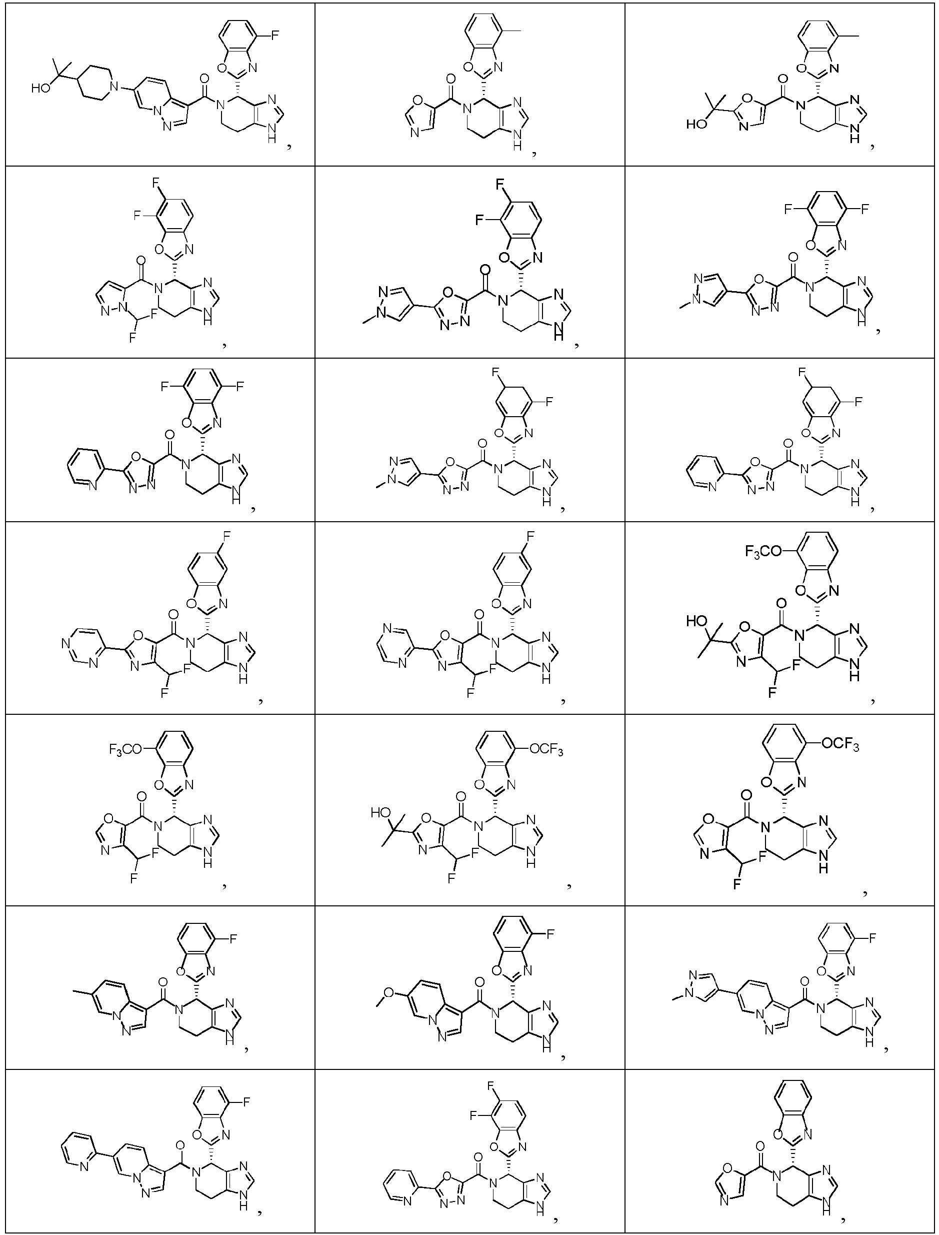

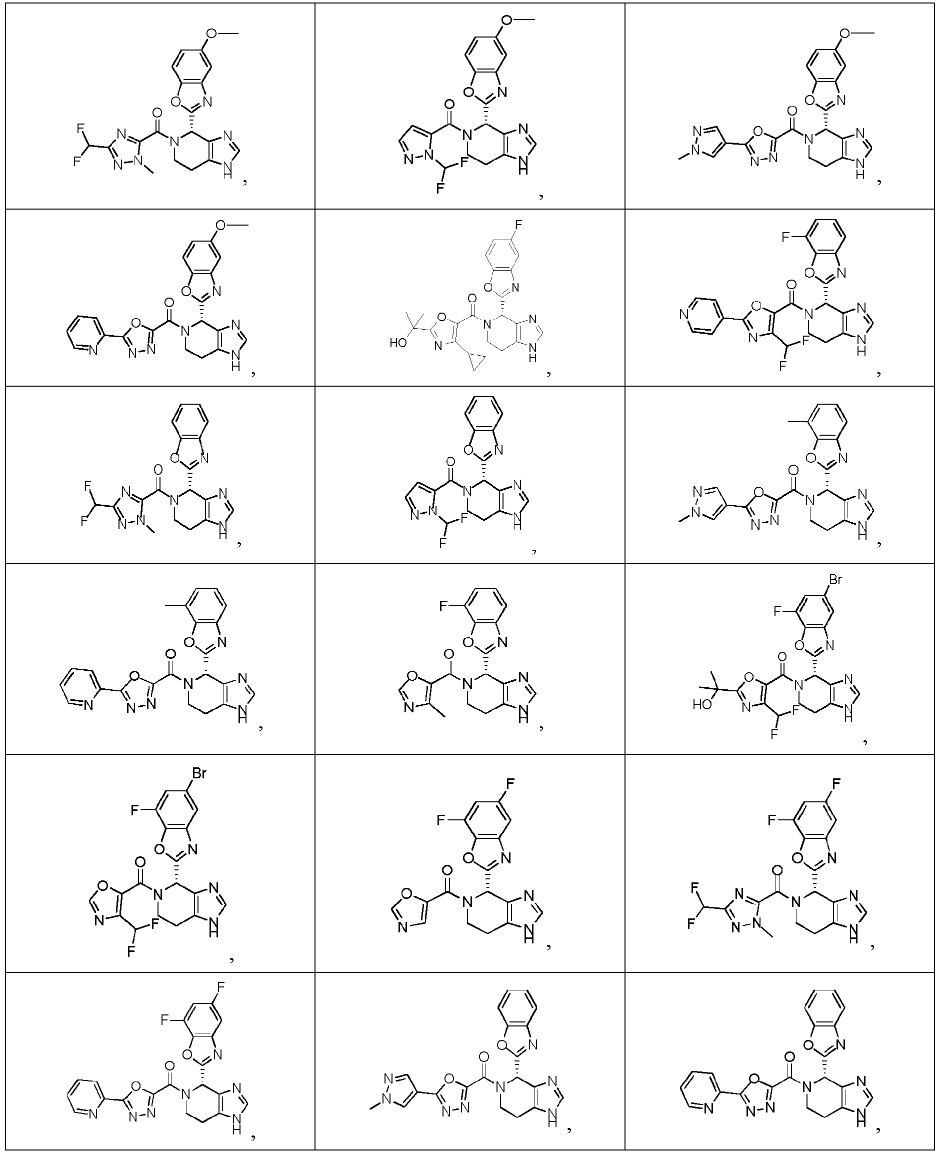

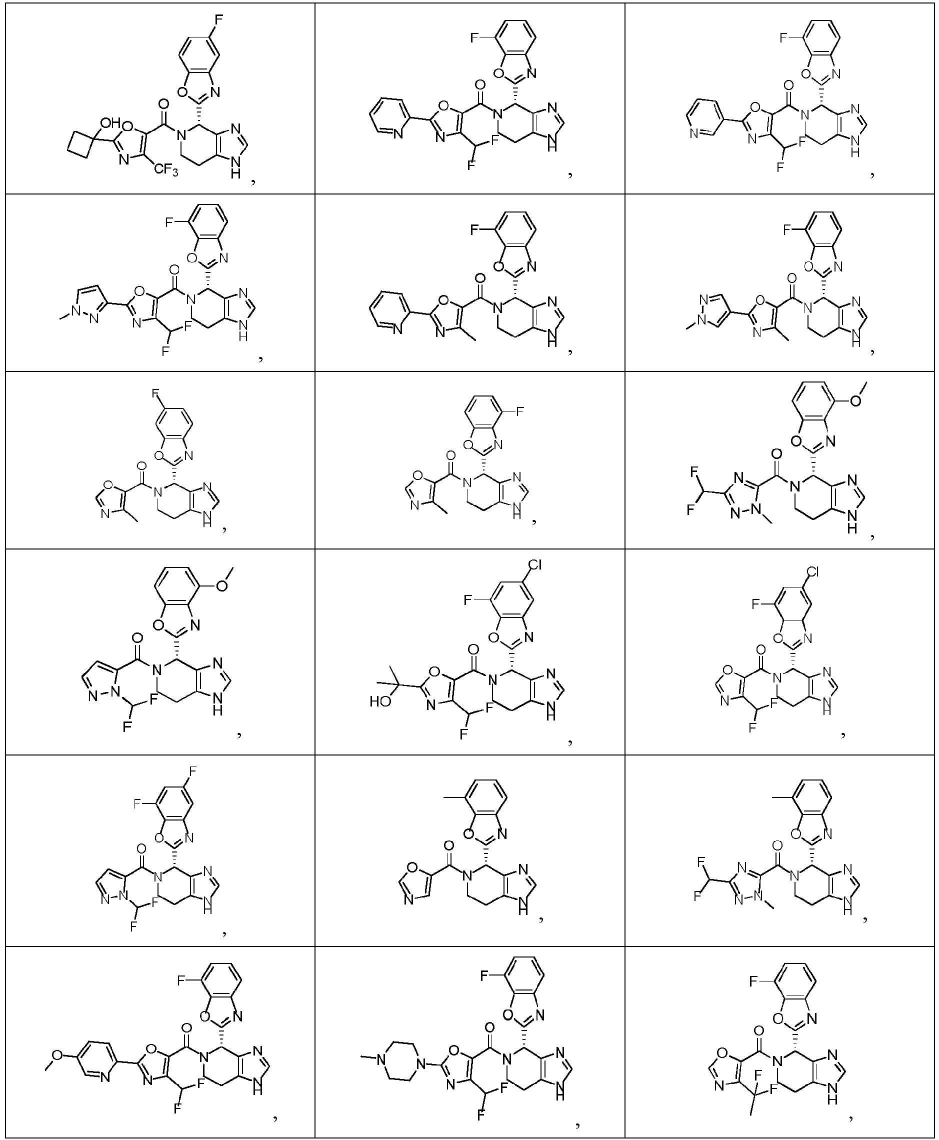

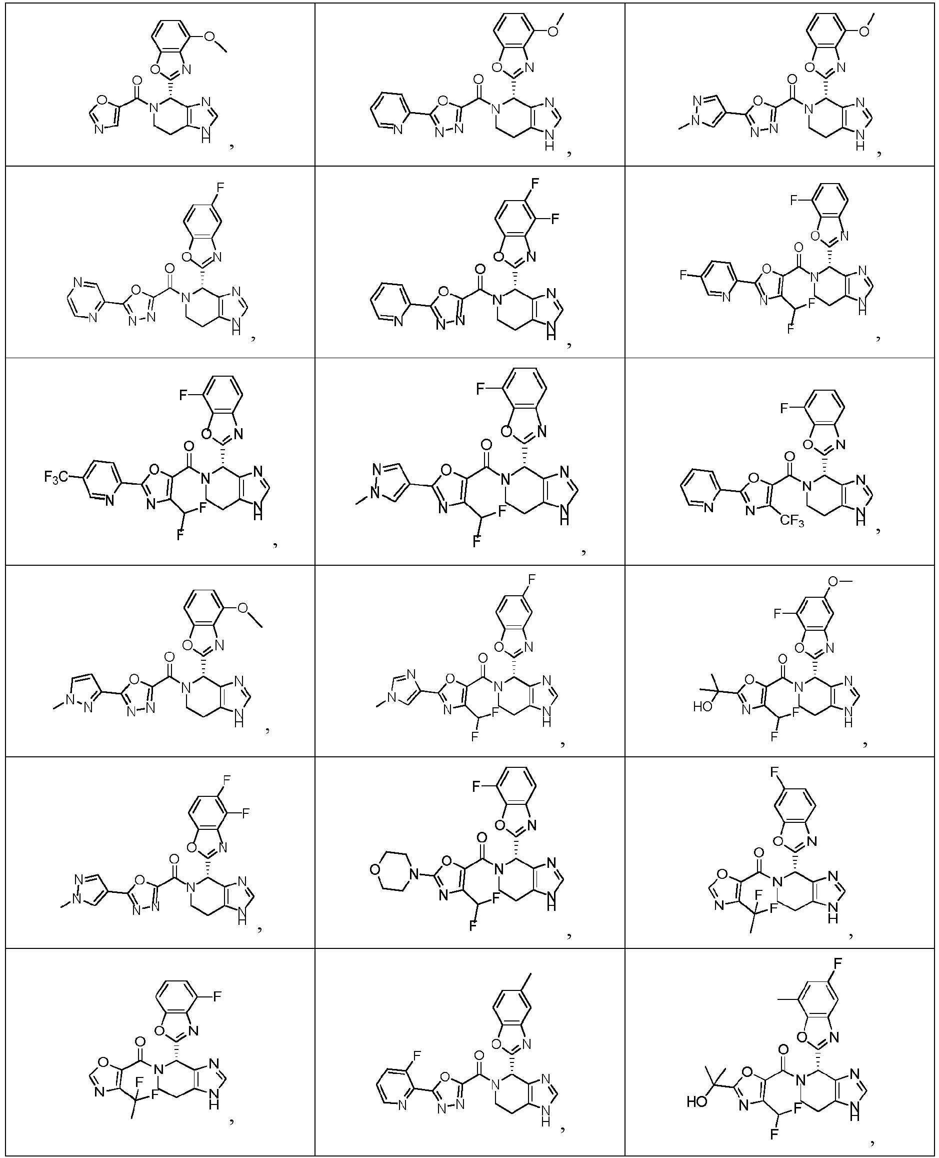

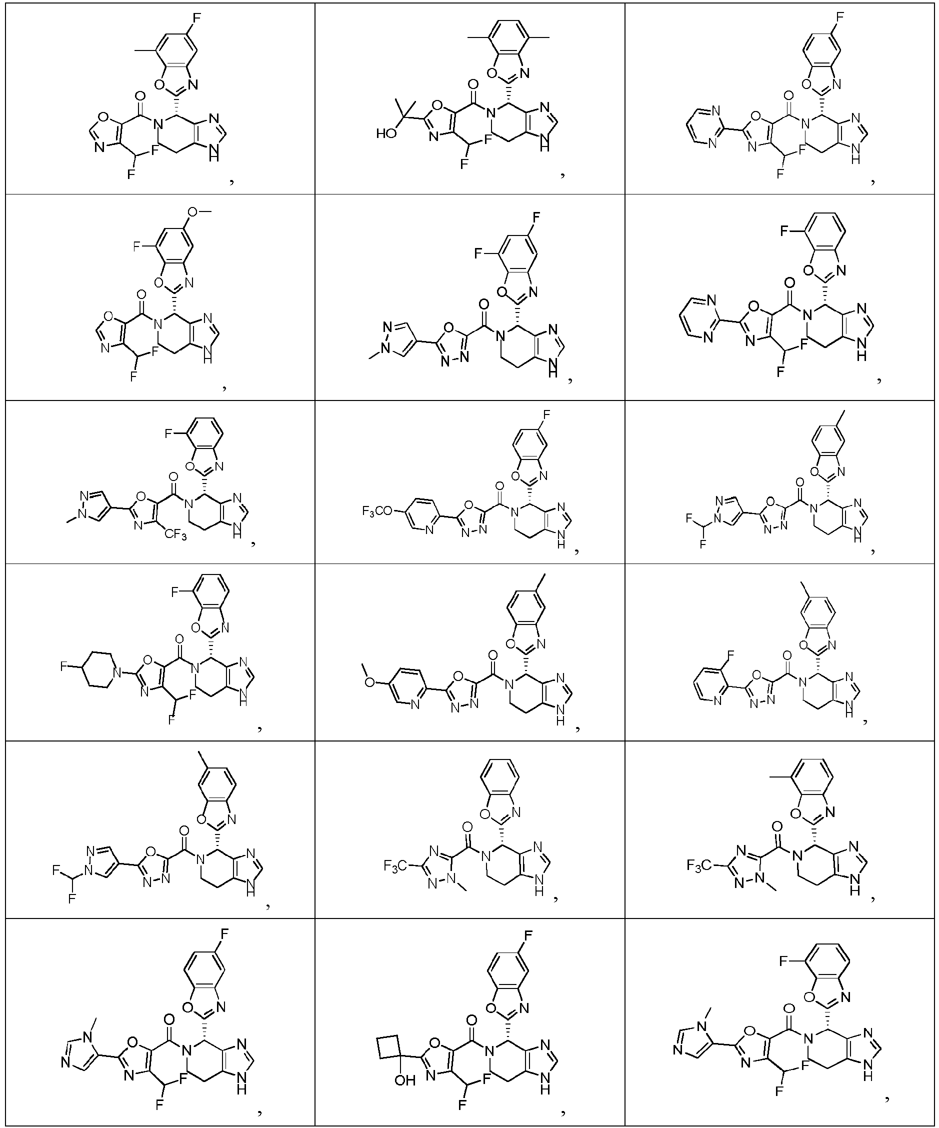

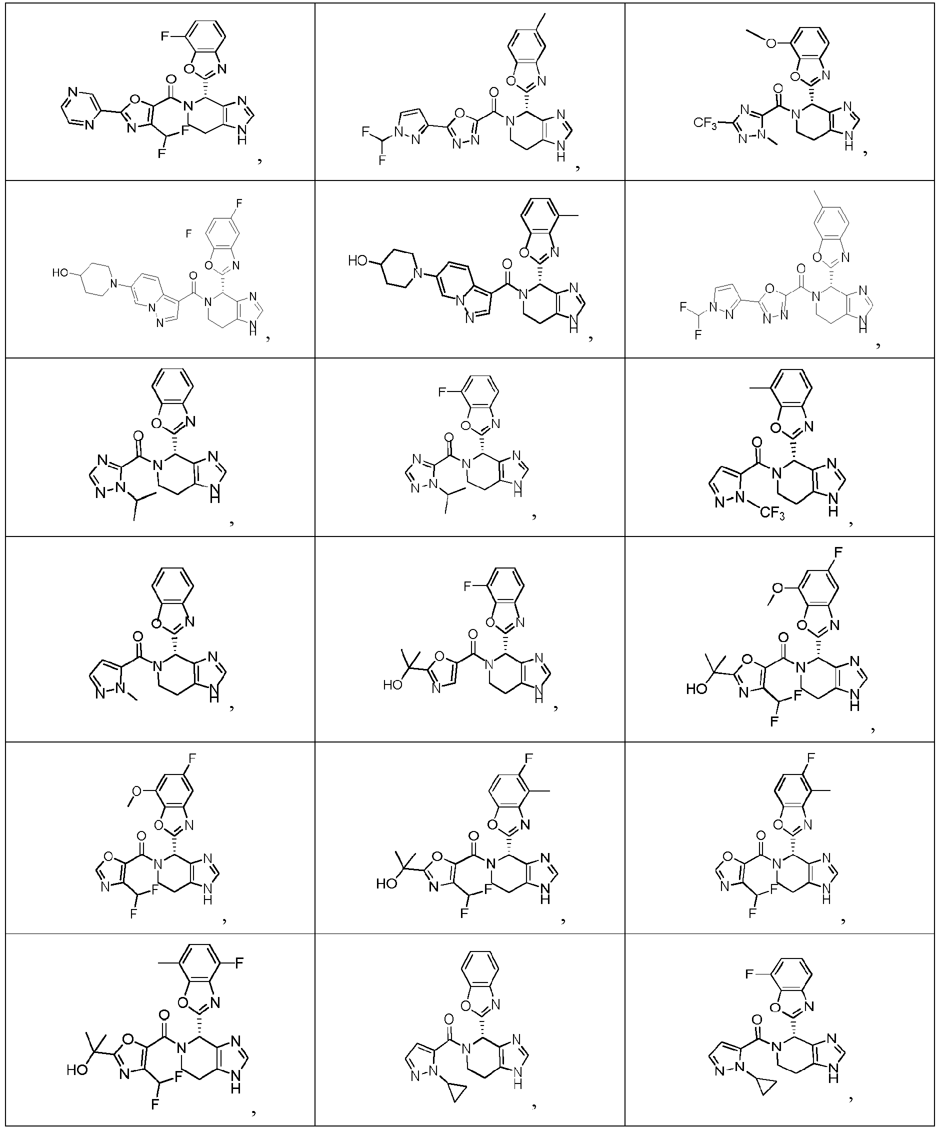

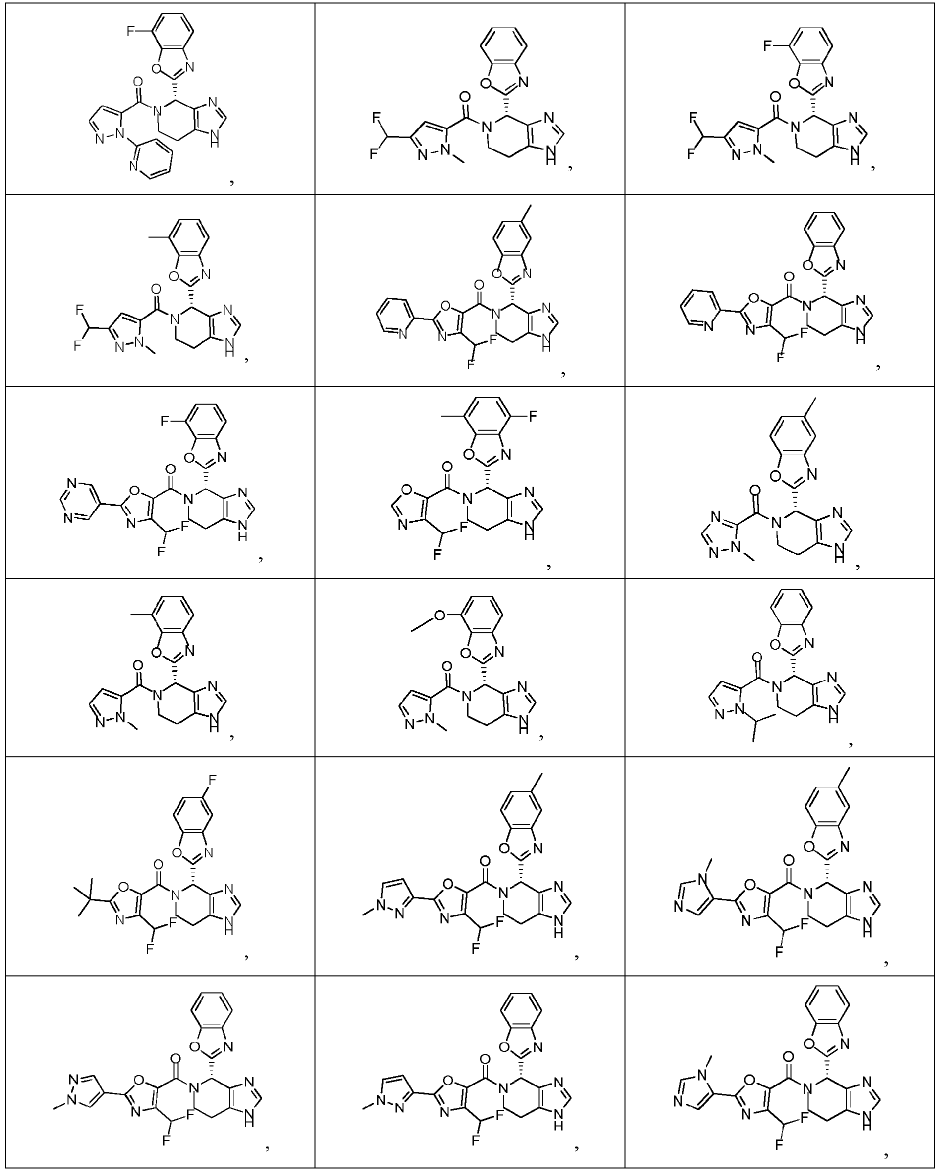

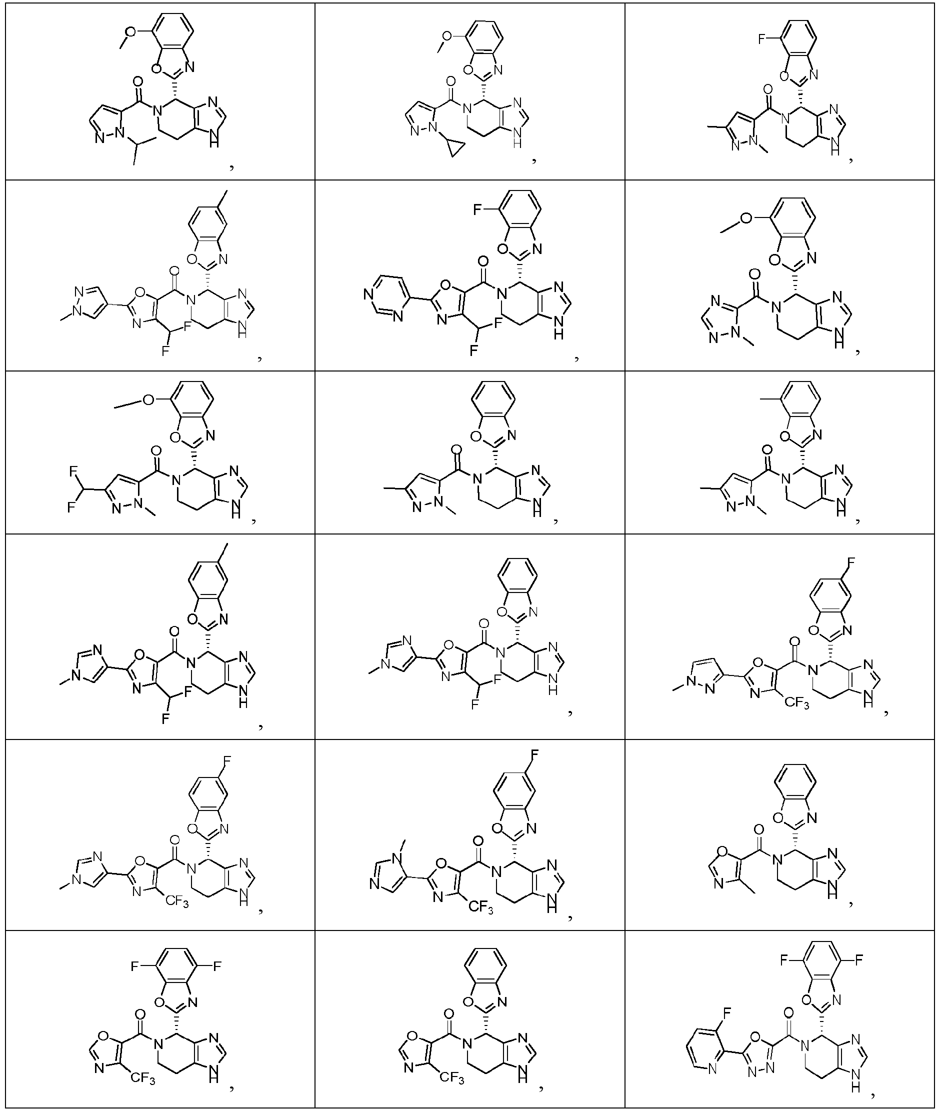

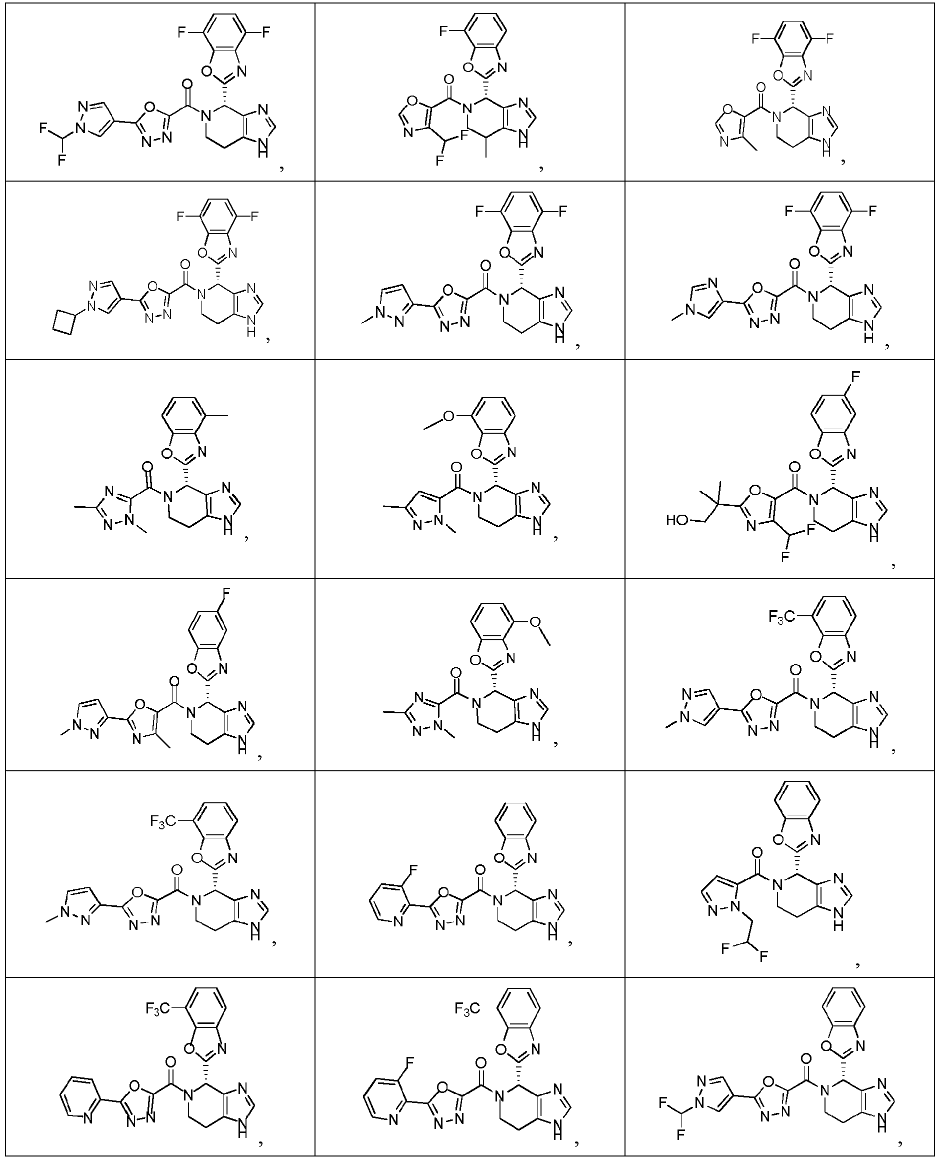

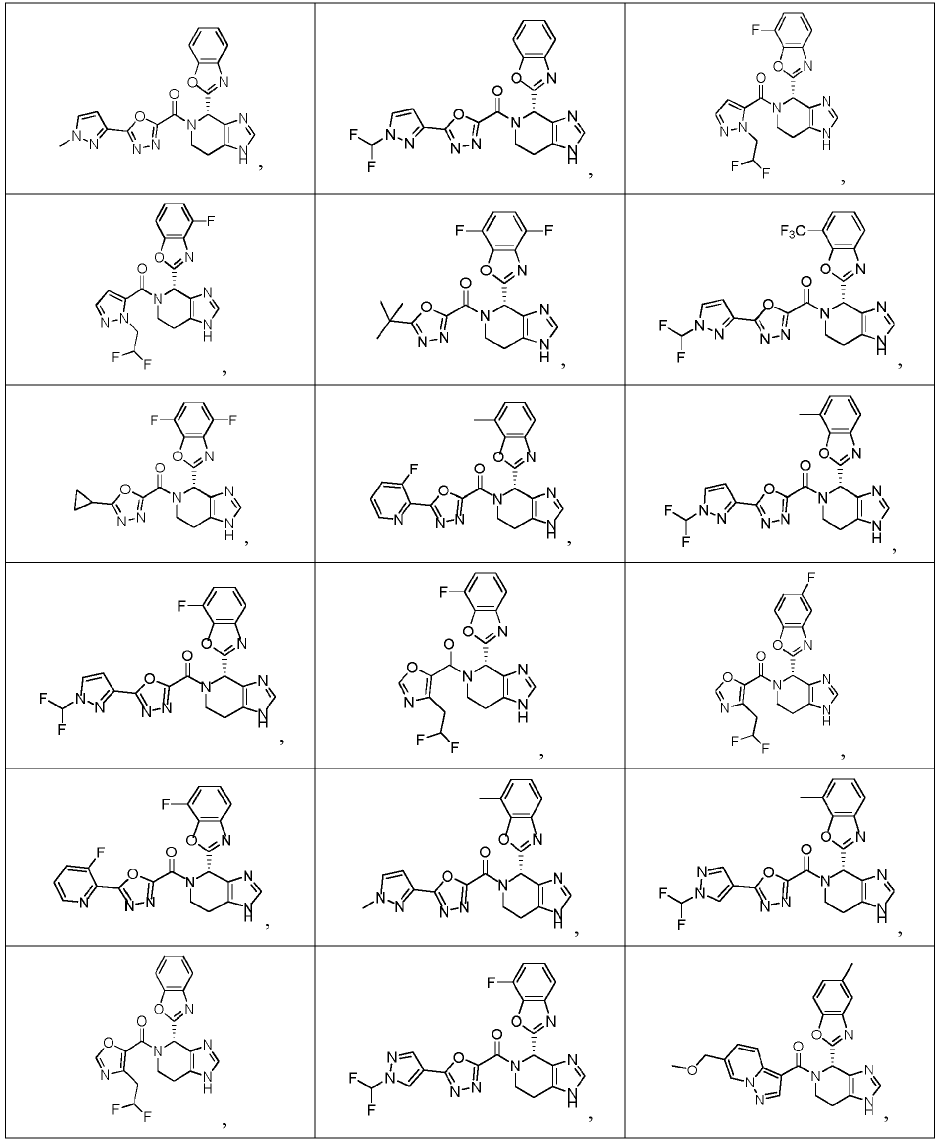

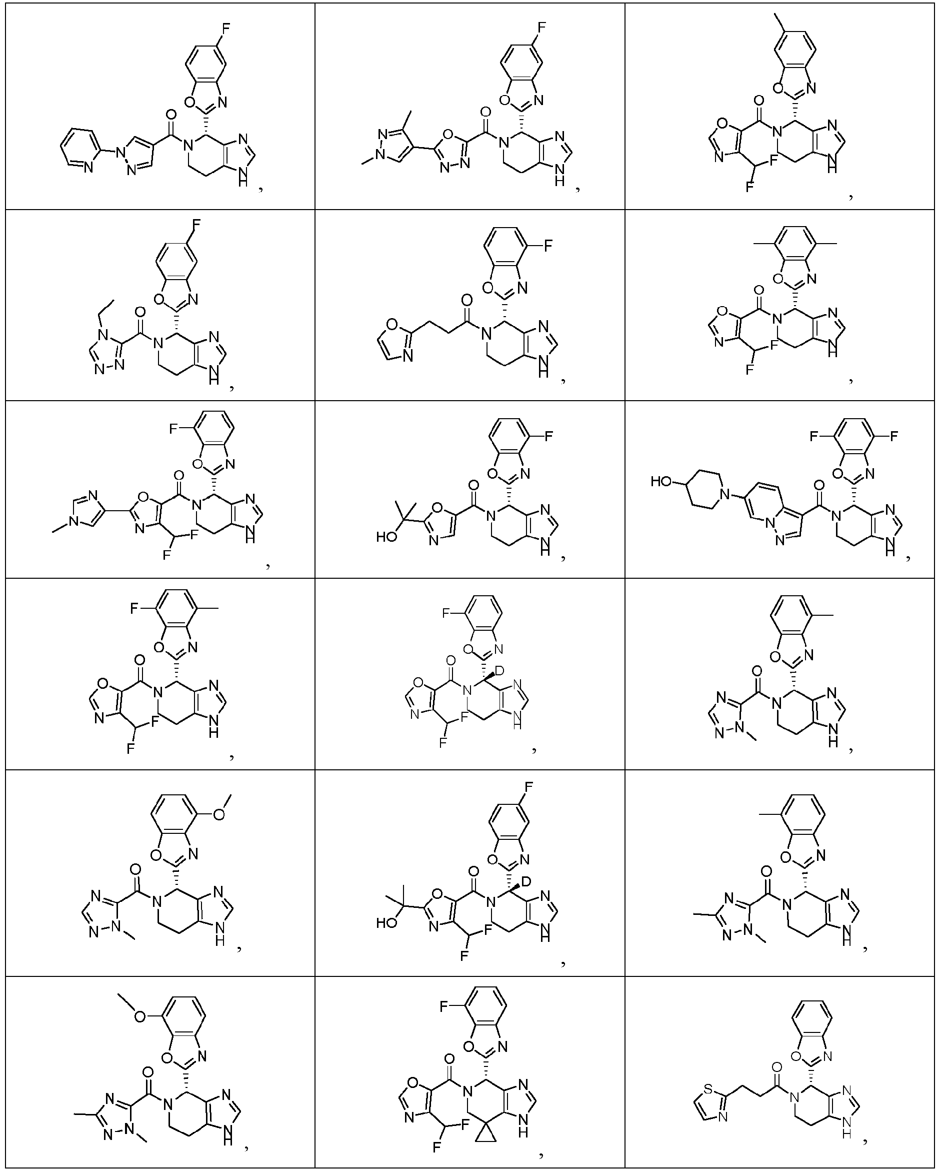

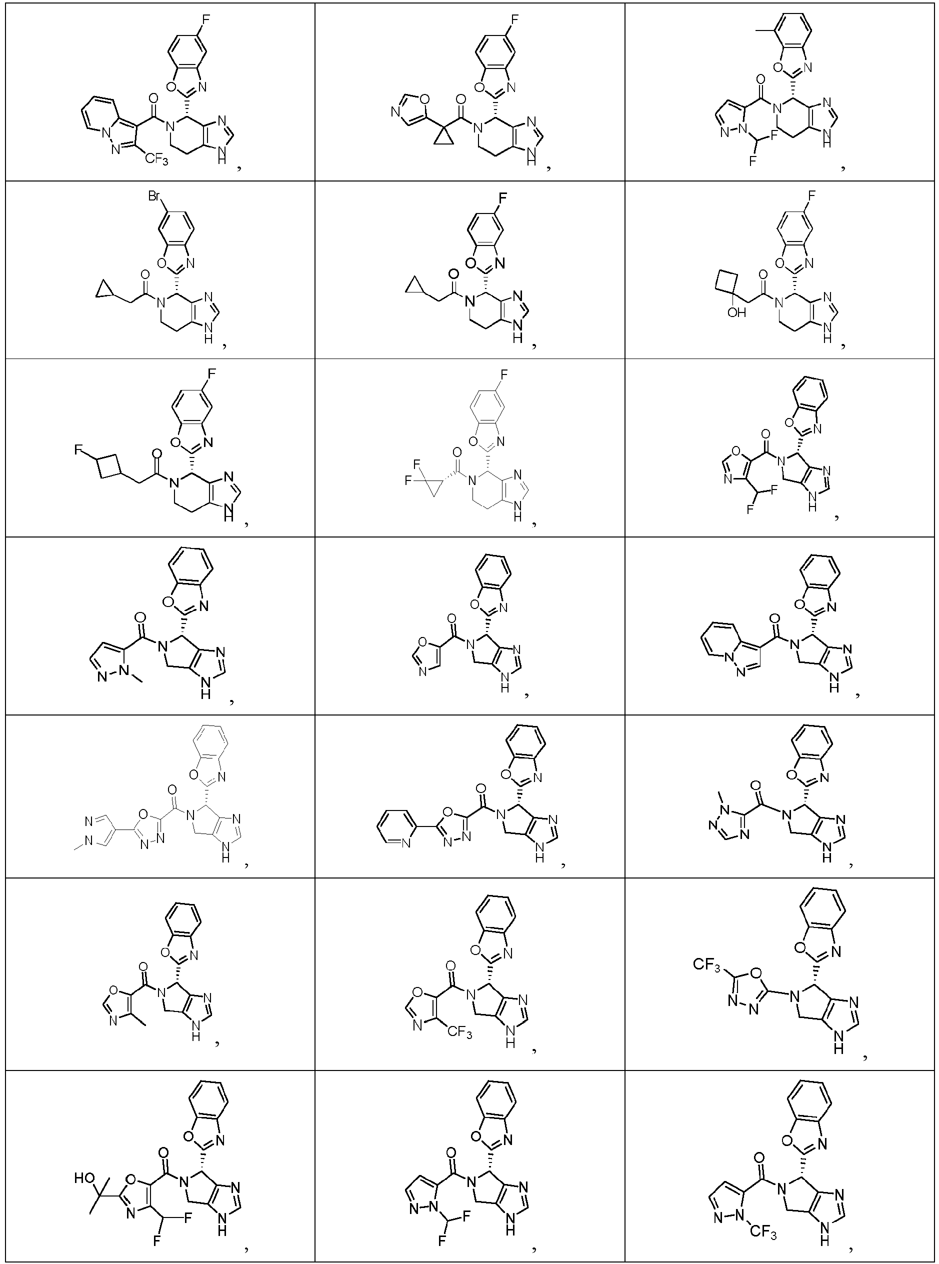

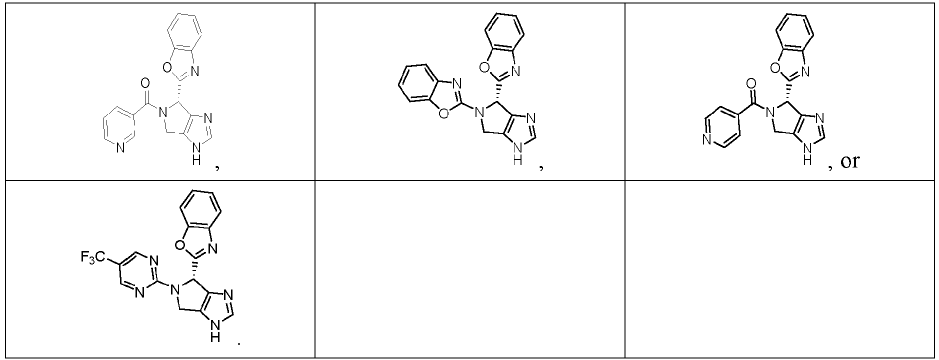

10. A pharmaceutical composition comprising a compound of any one of claims 1 to 9, or a pharmaceutically acceptable salt thereof, and a pharmaceutically acceptable excipient.

11. A compound of any one of claims 1 to 9, or a pharmaceutically acceptable salt thereof, or the pharmaceutical composition of claim 10, for use in stabilizing a mutant PAH protein in a subject suffering from phenylketonuria, comprising contacting the protein with the compound of any one of claims 1 to 9, or a pharmaceutically acceptable salt thereof, or the pharmaceutical composition of claim 10.

12. The compound or a pharmaceutically acceptable salt thereof, or the pharmaceutical composition for use according to claim 11, wherein:
(i) the mutant PAH protein contains at least one R408W, R261Q, R243Q, Y414C, L48S, A403V, 165T, R241C, L348V, R408Q, or V388M mutation;
(ii) the mutant PAH protein contains at least one R408W, Y414C, 165T, F39L, R408Q, L348V, R261Q, A300S, or L48S mutation;
(iii) the mutant PAH protein contains at least one R408W mutation; or
(iv) the mutant PAH protein contains two R408W mutations.

13. A compound of any one of claims 1 to 9, or a pharmaceutically acceptable salt thereof, or the pharmaceutical composition of claim 10, for use in reducing blood phenylalanine concentration in a subject suffering from phenylketonuria, optionally wherein:
(i) the blood phenylalanine concentration is reduced to a concentration less than or equal to 600 µM; or
(ii) the blood phenylalanine concentration is reduced to a concentration less than or equal to 360 µM.

14. The compound, or a pharmaceutically acceptable salt thereof, or the pharmaceutical composition for use of claim 13, wherein:
(i) the subject has a blood phenylalanine concentration greater than 600 µM prior to administration of the compound; or
(ii) the subject has a blood phenylalanine concentration greater than 1200 µM prior to administration of the compound.

15. A compound of any one of claims 1 to 9, or a pharmaceutically acceptable salt thereof, or the pharmaceutical composition of claim 10, for use in treating phenylketonuria in a subject.

## Patentansprüche

1. Verbindung von Formel I: 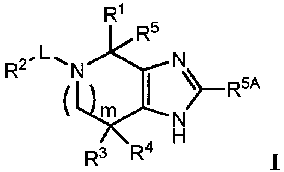 oder ein pharmazeutisch verträgliches Salz davon, wobei:
m 0, 1 oder 2 ist;
R¹ ist 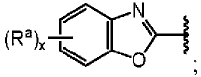
x 0 bis 4 ist;
jedes R^{a} unabhängig Halogen, C₁₋₆-Alkyl, C₃₋₆-Cycloalkyl, C₁₋₆-Halogenalkyl, C₁₋₆-Alkoxy oder C₁₋₆-Halogenalkoxy ist;
R³ H oder C₁₋₆-Alkyl ist;
R⁴ H oder C₁₋₆-Alkyl ist;
oder R³ und R⁴, zusammen mit dem Atom, an das sie gebunden sind, ein C₃₋₆-Cycloalkyl ausbilden;
R⁵ H oder D ist;
R^{5A} H oder D ist;
L eine Bindung, -C(O)-, -C(O)CH₂-, -C(O)CH₂CH₂-, -C(O)CH₂CH₂CH₂-, - C(O)CF₂-, -C(O)CHF-,-C(O)C(CH₃)₂-, -C(O)CH=CH-, 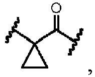 -C(O)NHCH₂-, - CH₂- oder -CH₂CH₂- ist; und
R² ist:
(a) 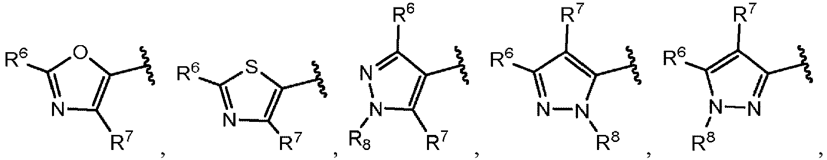 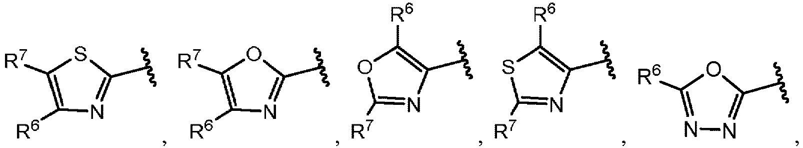 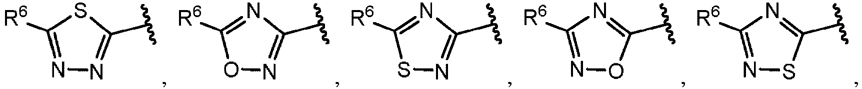 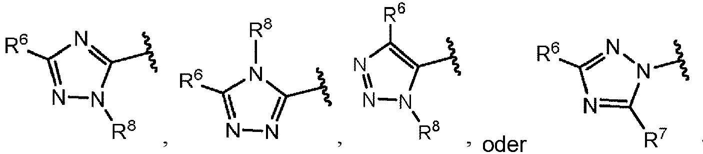 wobei:
R⁶ H; CN; C₁₋₆-Alkyl; C₁₋₆-Halogenalkyl; C₁₋₆-Cyanoalkyl; C₁₋₆-Hydroxyalkyl; C₁₋₆-Alkoxy; C₁₋₆-Alkoxy(alkylen); C₁₋₆-Halogenalkoxy; C₁₋₆-Halogenalkoxy(alkylen); deuteriertes C₁₋₆-Alkoxy(alkylen); Halogen; (CR^{v}R^{x})_{P}NR^{y}R^{z}; C(O)NR^{y2}R^{z2}; C₁₋₆-Alkylcarbonyl; C₃₋₆-Cycloalkyl, das optional mit einem oder mehreren von Halogen, OH, C₁₋₆-Halogenalkyl, C₁₋₆-Alkyl oder C₁₋₆-Alkoxy substituiert ist; Heterocyclyl, das optional mit einem oder mehreren von Halogen, OH, C₁₋₆-Halogenalkyl, C₁₋₆-Alkyl, C₁₋₆-Hydroxyalkyl, C₁₋₆-Alkoxy, C(O)O(C₁₋₆-Alkyl) oder C₃₋₆-Cycloalkyl substituiert ist; Aryl, das optional mit einem oder mehreren von Halogen, C₁₋₆-Halogenalkyl, C₁₋₆-Alkyl oder C₃₋₆-Cycloalkyl substituiert ist; Heteroaryl, das optional mit einem oder mehreren von Halogen, C₁₋₆-Halogenalkyl, C₁₋₆-Alkyl, C₁₋₆-Alkoxy, C₁₋₆-Halogenalkoxy, C₃₋₆-Cycloalkyl oder C₃₋₆-Cycloalkylsulfonyl substituiert ist; C₂₋₆-Alkenyl; C₃₋₈-Cycloalkenyl; C₃₋₈-Cycloalkyl(alkylen); Aryl(alkylen); Heterocyclyl(alkylen), das optional mit einem oder mehreren von Halogen, OH, C₁₋₆-Halogenalkyl, C₁₋₆-Alkyl, C₁₋₆-Hydroxyalkyl, C₁₋₆-Alkoxy, C(O)O(C₁₋₆-Alkyl) oder C₃₋₆-Cycloalkyl substituiert ist; oder C₁₋₆-Alkylsulfonyl ist,
R⁷ H, CN, C₁₋₆-Alkyl, C₁₋₆-Halogenalkyl, C₁₋₆-Cyanoalkyl, C₁₋₆-Hydroxyalkyl, C₁₋₆-Alkoxy, C₁₋₆-Alkoxy(alkylen), C₁₋₆-Halogenalkoxy, C₁₋₆-Halogenalkoxy(alkylen), deuteriertes C₁₋₆-Alkoxy(alkylen), Halogen, (CR^{v}R^{x})ₚNR^{y}R^{z}, C(O)NR^{y2}R^{z2}, C₁₋₆-Alkylcarbonyl, C₃₋₆-Cycloalkyl, Heterocyclyl, Aryl, Heteroaryl, C₂₋₆-Alkenyl; C₃₋₈-Cycloalkenyl; C₃₋₈-Cycloalkyl(alkylen); Aryl(alkylen); Heterocyclyl(alkylen); oder C₁₋₆-Alkylsulfonyl ist,
R⁸ H, C₁₋₆-Alkyl, C₁₋₆-Halogenalkyl, C₁₋₆-Alkoxy(alkylen), C₁₋₆-Alkylcarbonyl, C₁₋₆-Hydroxyalkyl, (CR^{v}R^{x})ₚNR^{y}R^{z}, C₃₋₈-Cycloalkyl, Aryl, Heteroaryl oder C₃₋₈-Cycloalkyl(alkylen) ist;
R^{v} und R^{x} unabhängig H oder C₁₋₆-Alkyl sind;
R^{y} und R^{z} unabhängig H, C₁₋₆-Alkyl, C₁₋₆-Alkoxy(alkylen) oder C₃₋₆-Cycloalkyl sind;
R^{y2} und R^{z2} unabhängig H, C₁₋₆-Alkyl oder C₃₋₆-Cycloalkyl sind; und
p 0, 1, 2 oder 3 ist; oder
(b) 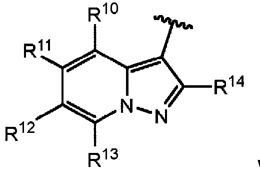 wobei:
R¹⁰, R¹¹, R¹², R¹¹ und R¹⁴ unabhängig H, C₁₋₆-Alkyl, C₁₋₆-Halogenalkyl, C₁₋₆-Alkoxy, C₁₋₆-Alkoxy(alkylen), Halogen, optional substituiertes Heterocyclyl, optional substituiertes Heterocyclyl(alkylen), optional substituiertes Heteroaryl oder (CR^{v}R^{x})ₚNR^{y}R^{z} sind, wobei die optionale Substitution an den Heterocyclyl- und Heterocyclyl(alkylen)gruppen eines oder mehrere von Halogen, C₁₋₆-Halogenalkyl, C₁₋₆-Alkyl, OH, C₁₋₆-Hydroxyalkyl, C₁₋₆-Alkoxy, oder C₃₋₆-Cycloalkyl ist und die optionale Substitution an der Heteroarylgruppe ein oder mehrere C₁₋₆-Alkyle ist;
R^{v} und R^{x} unabhängig H oder C₁₋₆-Alkyl sind;
R^{y} und R^{z} unabhängig H, C₁₋₆-Alkyl, C₃₋₆-Cycloalkyl oder C(O)OC₁₋₆-Alkyl sind; und p 0, 1, 2 oder 3 ist.

2. Verbindung nach Anspruch 1, wobei die Verbindung gehört zu:
(i) Formel I-A-1, 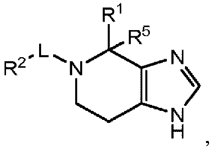 oder einem pharmazeutisch verträglichen Salz davon; oder
(ii) Formel I-A-3, 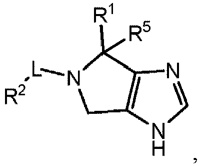 oder einem pharmazeutisch verträglichen Salz davon; oder
(iii) Formel I-B-1, 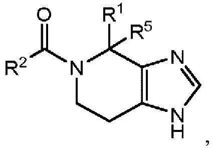 oder einem pharmazeutisch verträglichen Salz davon; oder
(iv) Formel I-B-3, 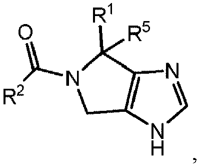 oder einem pharmazeutisch verträglichen Salz davon; oder
(v) Formel I-C-1, 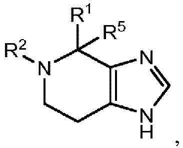 oder einem pharmazeutisch verträglichen Salz davon; oder
(vi) Formel I-C-3, 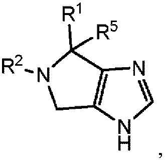 oder einem pharmazeutisch verträglichen Salz davon; oder
(vii) Formel I-D-1, 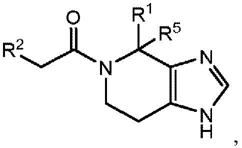 oder einem pharmazeutisch verträglichen Salz davon; oder
(viii) Formel I-G-1, 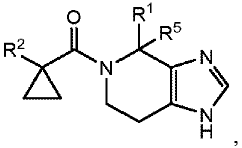 oder einem pharmazeutisch verträglichen Salz davon; oder
(ix) Formel I-I-1, 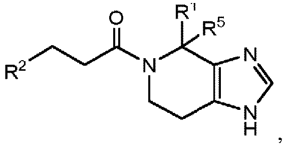 oder einem pharmazeutisch verträglichen Salz davon; oder
(x) Formel I-L-1, 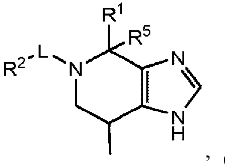 oder einem pharmazeutisch verträglichen Salz davon; oder
(xi) Formel I-M-1, 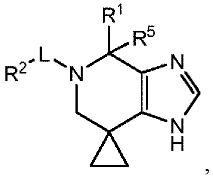 oder einem pharmazeutisch verträglichen Salz davon.

3. Verbindung nach Anspruch 1 oder 2 oder ein pharmazeutisch verträgliches Salz davon, wobei:
(i) R¹ ist 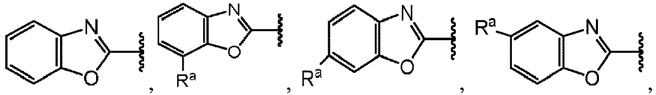 oder 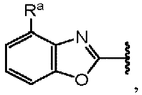 optional wobei
a. R^{a} Halogen wie F, Br oder Cl ist;
b. R^{a} C₁₋₆-Alkyl wie Methyl ist;
c. R^{a} C₁₋₆-Alkoxy wie Methoxy ist;
d. R^{a} C₁₋₆-Halogenalkyl wie CF₃ ist; oder
e. R^{a} C₁₋₆-Halogenalkoxy wie OCF₃; oder
(ii) R¹ ist 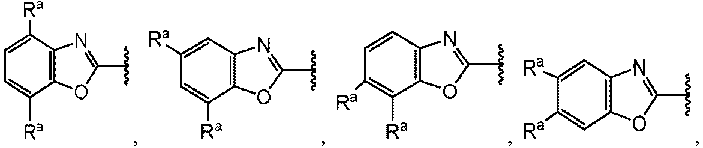 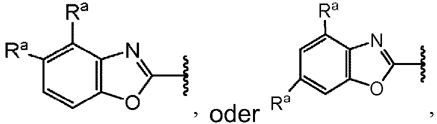 optional wobei ein R^{a} Halogen ist, wie Br, Cl oder F oder C₁₋₆-Alkyl wie Methyl ist, und das zweite R^{a} Halogen wie Br, Cl oder F, C₁₋₆-Alkoxy, wie Methoxy, oder C₁₋₆-Alkyl, wie Methyl ist.

4. Verbindung nach einem der Ansprüche 1 bis 3 oder ein pharmazeutisch verträgliches Salz davon, wobei:
(i) R² (a) ist und R⁶, R⁷, R⁸ jeweils H sind; oder
(ii) R² (b) ist und R¹⁰, R¹³ und R¹⁴ jeweils H sind; oder
(iii) R² (b) ist und R¹⁰, R¹¹, R¹³ und R¹⁴ jeweils H sind; oder
(iv) R² (a) ist und R⁶ C₁₋₆-Alkyl, wie Methyl, Ethyl, Isopropyl oder tert-Butyl, C₁₋₆-Halogenalkyl, wie CF₃. CHF₂, CH₂F, CH₂CF₃, CH(CH₃)F, C(CH₃)F₂ oder C(CH₃)₂F, C₁₋₆-Cyanoalkyl wie C(CH₃)₂CN oder C₁₋₆-Hydroxyalkyl wie C(CH₃)₂OH, CH(CH₃)OH, CH(CH₂CH₃)OH, CH₂C(CH₃)₂OH oder C(CH₃)₂CH₂OH ist; oder
(v) R² (a) ist und R⁶ ist 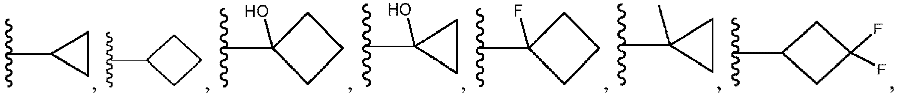 oder 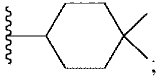 oder
(vi) R² (a) ist, und R⁶ Phenyl ist, optional substituiert mit einem oder mehreren Halogenen wie F, Cl oder Br; C₁₋₆-Halogenalkyl wie CF₃, CH₂CF₃ oder CHF₂; C₁₋₆-Alkyl, wie Methyl, Ethyl oder Propyl; oder C₃₋₆-Cycloalkyl wie Cyclopropyl, Cyclobutyl oder Cyclopentyl,
optional wobei R⁶ ist 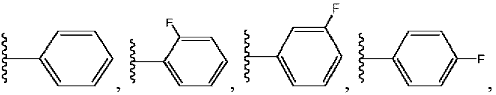 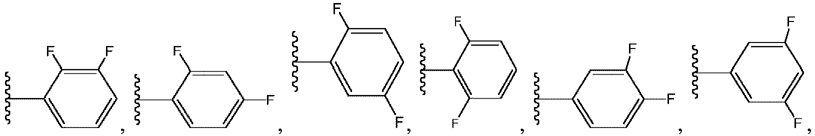 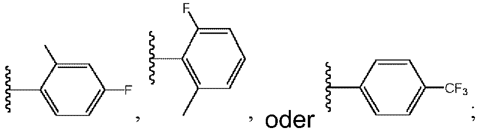 oder
(vii) R² (a) ist und R⁶ Pyridinyl, Pyrazolyl, Pyrazinyl, Pyridazinyl, Imidazolyl oder Pyrimidinyl ist, jeweils optional substituiert mit einem oder mehreren Halogenen wie F, Cl oder Br; C₁₋₆-Halogenalkyl wie CF₃, CH₂CF₃, CH₂CHF₂ oder CHF₂; C₁₋₆-Alkyl wie Methyl, Ethyl, Propyl oder Isopropyl; C₁₋₆-Alkoxy wie Methoxy, Ethoxy oder Propoxy; C₁₋₆-Halogenalkoxy wie OCF₃; C₃₋₆-Cycloalkyl, wie Cyclopropyl, Cyclobutyl oder Cyclopentyl; oder C₃₋₆-Cycloalkylsulfonyl wie Cyclopropylsulfonyl,
Cyclobutylsulfonyl oder Cyclopentylsulfonyl;
optional wobei (a) R⁶ ist 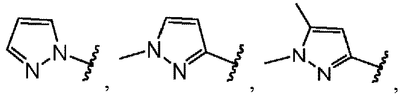 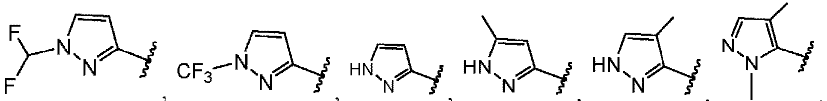 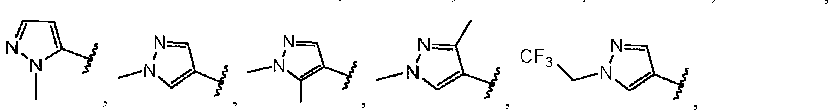 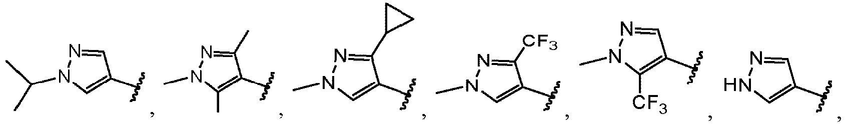 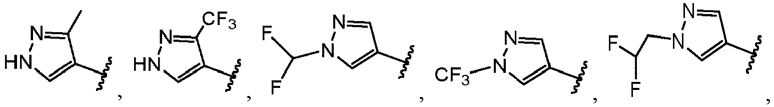 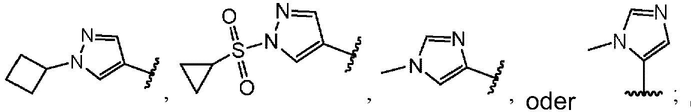 oder
(b) R6 ist 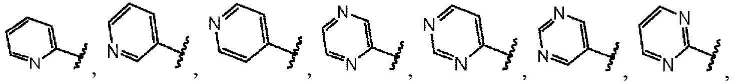 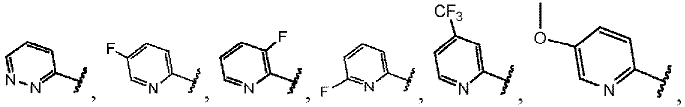 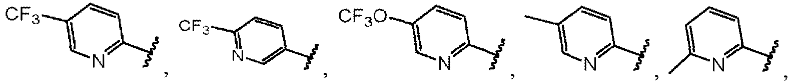 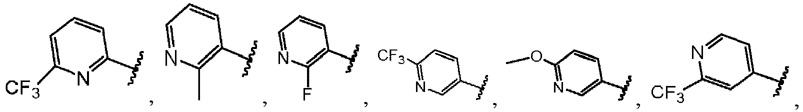 oder 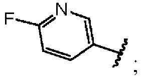 oder
(viii) R² (a) ist und R⁶ Azetidinyl, Pyrrolidinyl, Piperidinyl, Piperazinyl oder Morpholinyl ist, jeweils optional substituiert mit einem oder mehreren Halogenen wie F, Cl oder Br; OH; C₁₋₆-Halogenalkyl wie CF₃, CH₂CF₃ oder CHF₂; C₁₋₆-Alkyl, wie Methyl, Ethyl oder Propyl; C₁₋₆-Hydroxyalkyl wie C(CH₃)₂OH; C₁₋₆-Alkoxy wie Methoxy, Ethoxy oder Propoxy; C(O)O(C₁₋₆-Alkyl) wie C(O)OMethyl, C(O)OEthyl, C(O)OPropyl oder C(O)Otert-butyl; oder C₃₋₆-Cycloalkyl wie Cyclopropyl, Cyclobutyl oder Cyclopentyl;
optional wobei R⁶ ist 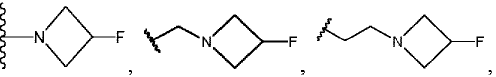 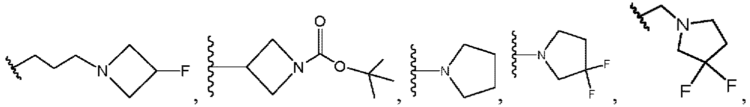 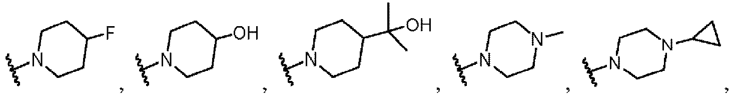 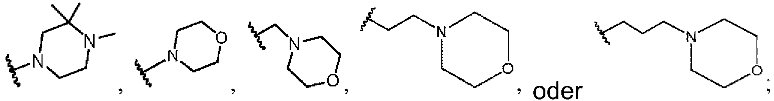 und/oder
(ix) R² (a) ist und R⁷ H, CN, C₁₋₆-Alkyl wie Methyl, C₁₋₆-Halogenalkyl wie CF₃, CH₂F, CHF₂, CF₂(CH₃) oder CH₂CHF₂, Halogen wie Br oder Cl, C₃₋₈-Cycloalkyl wie Cyclopropyl, Phenyl oder Pyridinyl ist.

5. Verbindung nach einem der Ansprüche 1 bis 3 oder ein pharmazeutisch verträgliches Salz davon, wobei R² ist: 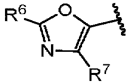 wobei:
R⁶ H, C₁₋₆-Alkyl wie Methyl, Isopropyl oder tert-Butyl, C₁₋₆-Halogenalkyl wie C(CH₃)₂F, CH(CH₃)F, oder CF₃, C₁₋₆-Cyanoalkyl wie C(CH₃)₂CN, C₁₋₆-Hydroxyalkyl wie C(CH₃)₂OH, CH₂C(CH₃)₂OH, CH(CH₂CH₃)OH, C(CH₃)₂(CH₂OH) oder CH(CH₃)OH, C₁₋₆-Alkoxy wie Methoxy, C₁₋₆-Alkoxy(alkylen) wie CH₂OCH₃, C(CH₃)₂OCH₃, oder (CH₂)₂OCH₃, C₁₋₆-Halogenalkoxy wie O(CH₂)₂CF₃, C₁₋₆-Halogenalkoxy(alkylen) wie CH₂OCF₃, deuteriertes C₁₋₆-Alkoxy(alkylen) wie CH₂OCD₃, (CR^{v}R^{x})ₚNR^{y}R^{z} wie NH₂, CH₂N(CH₃)₂, (CH₂)₂N(CH₃)₂, C(CH₃)₂NHCH₃, C(CH₃)₂N(CH₃)₂, oder NHcyclopropyl, C₃₋₆-Cycloalkyl ist, das optional substituiert ist mit einem oder mehreren OH wie 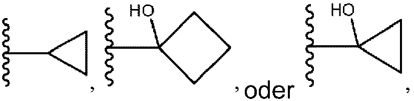 Heterocyclyl, das optional substituiert ist mit einem oder mehreren von Halogen oder C₁₋₆-Alkyl, wie 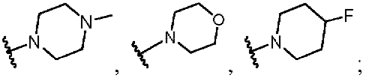 oder Heteroaryl, das optional substituiert ist mit einem oder mehreren von C₁₋₆-Alkyl, C₁₋₆-Halogenalkyl, Halogen oder C₁₋₆-Alkoxy, wie 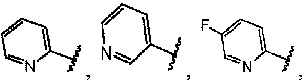 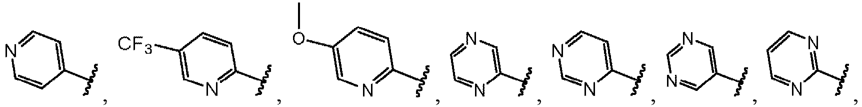 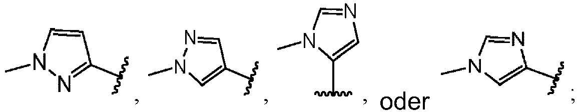
R⁷ H, C₁₋₆-Alkyl wie Methyl, C₁₋₆-Halogenalkyl wie CHF₂, CH₂F, C(CH₃)F₂, CH₂CHF₂ oder
CF₃, Halogen wie Br oder Cl, oder C₃₋₆-Cycloalkyl wie Cyclopropyl ist;
R^{v} und R^{x} unabhängig H oder C₁₋₆-Alkyl 1 wie Methyl sind;
R^{y} und R^{z} unabhängig H, C₁₋₆-Alkyl wie Methyl oder C₃₋₆-Cycloalkyl wie Cyclopropyl sind; und
p 0, 1,2 oder 3 ist.

6. Verbindung nach einem der Ansprüche 1 bis 3 oder ein pharmazeutisch verträgliches Salz davon, wobei R² ist: 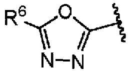 wobei
R⁶ H, C₁₋₆-Alkyl wie Methyl oder tert-Butyl, C₁₋₆-Halogenalkyl wie C(CH₃)₂F oder CF₃, C₁₋₆-Hydroxyalkyl wie C(CH₃)₂OH, C₃₋₆-Cycloalkyl wie 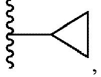 Aryl, das optional substituiert ist mit einem oder mehreren von Halogen, C₁₋₆.Alkyl oder C₁₋₆-Halogenalkyl wie 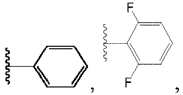 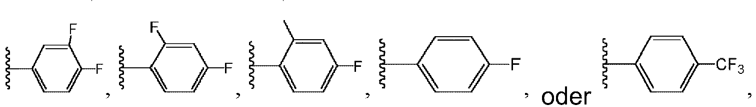 Heteroaryl, das optional substituiert ist mit einem oder mehreren von C₁₋₆-Alkyl, C₁₋₆-Halogenalkyl, Halogen, C₁₋₆-Alkoxy, C₃₋₆-Cycloalkyl oder C₃₋₆-Cycloalkylsulfonyl wie

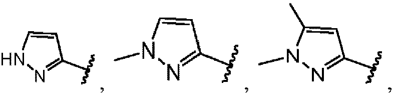

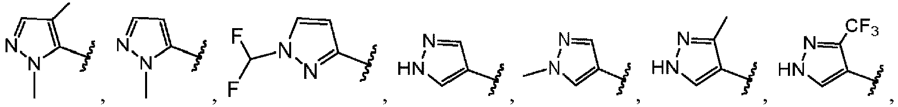

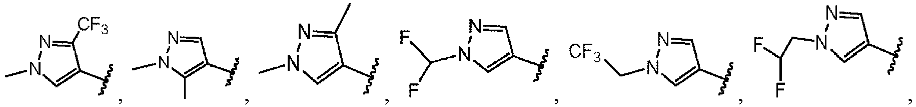

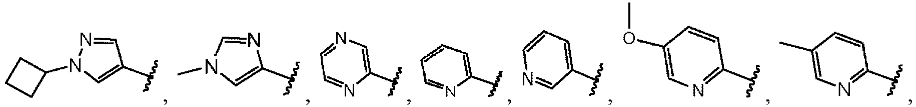

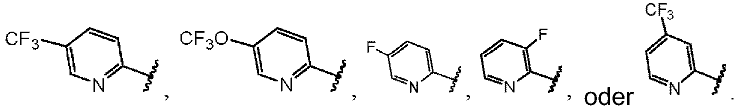

7. Verbindung nach einem der Ansprüche 1 bis 3 oder ein pharmazeutisch verträgliches Salz davon, wobei R² ist:
(a) 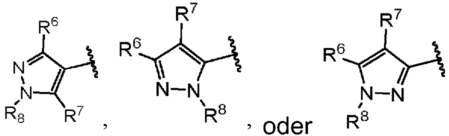 wobei:
R⁶ und R⁷ unabhängig H, Halogen wie Cl oder Br, C₁₋₆-Alkyl wie Methyl oder C₁₋₆-Halogenalkyl wie CHF₂, CFH₂ oder CF₃ sind; optional wobei mindestens eines von R⁶ und R⁷ H ist; und
R⁸ H, C₁₋₆-Alkyl wie Methyl, Isopropyl oder tert-Butyl, C₁₋₆-Halogenalkyl wie CHF₂, CH₂CF₃, CH₂CHF₂ oder CF₃, C₃₋₆-Cycloalkyl wie Cyclopropyl oder Heteroaryl wie Pyridinyl ist; oder
(b) 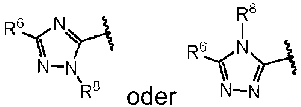 wobei:
R⁶ H, C₁₋₆-Alkyl wie Methyl, Halogen wie Cl oder Br, C₁₋₆-Halogenalkyl wie CHF₂ oder CF₃ oder C₃₋₆-Cycloalkyl wie Cyclopropyl ist; und
R⁸ H oder C₁₋₆-Alkyl wie Methyl, Ethyl oder Isopropyl ist.

8. Verbindung nach einem der Ansprüche 1 bis 7, die ein S-Enantiomer, oder ein pharmazeutisch verträgliches Salz davon ist.

9. Verbindung oder ein pharmazeutisch verträgliches Salz davon, wobei die Verbindung aus dem Folgenden ausgewählt ist:

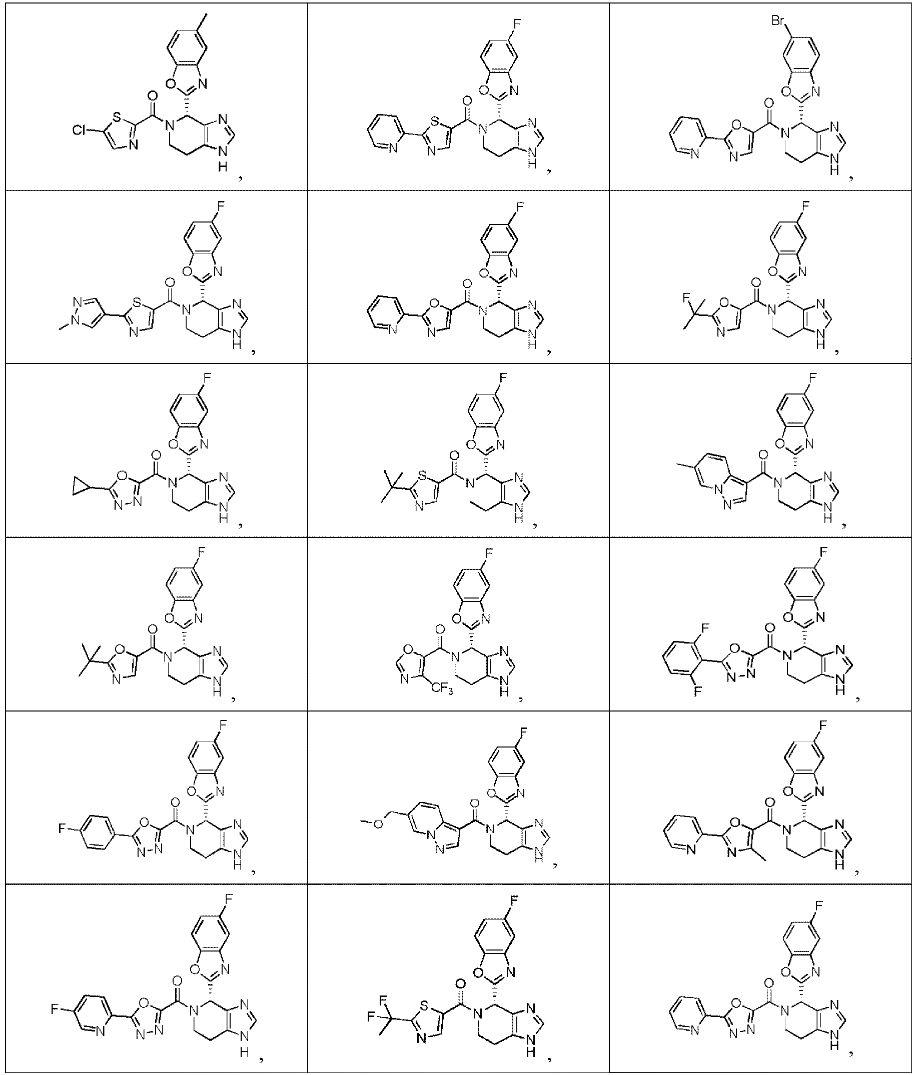

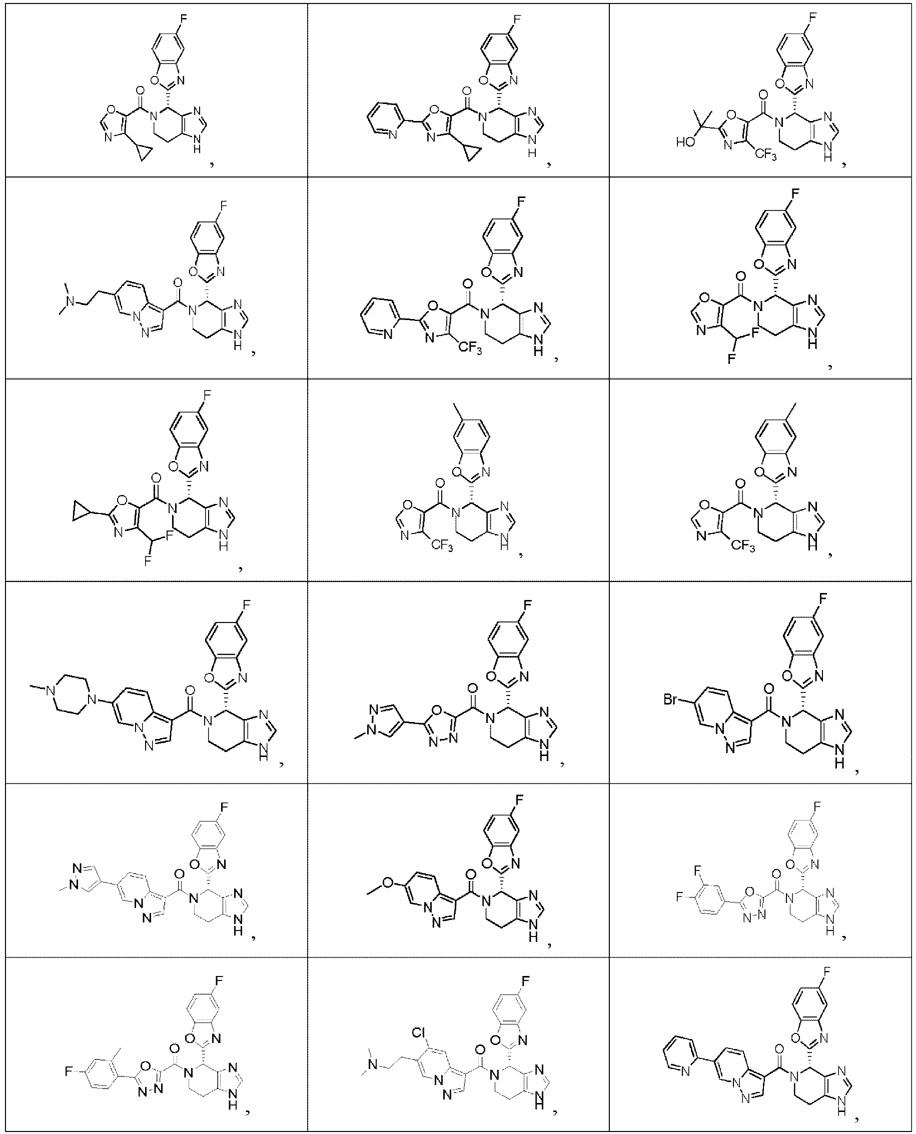

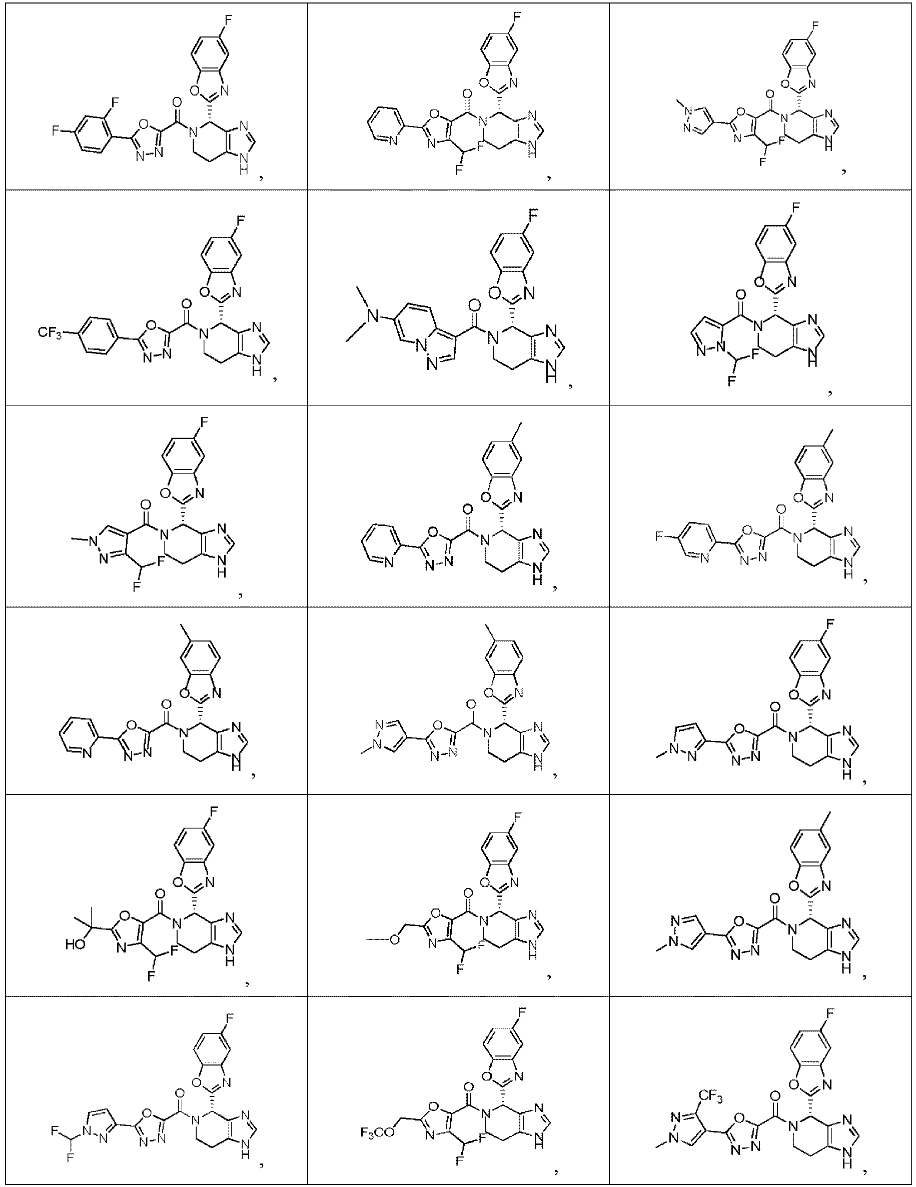

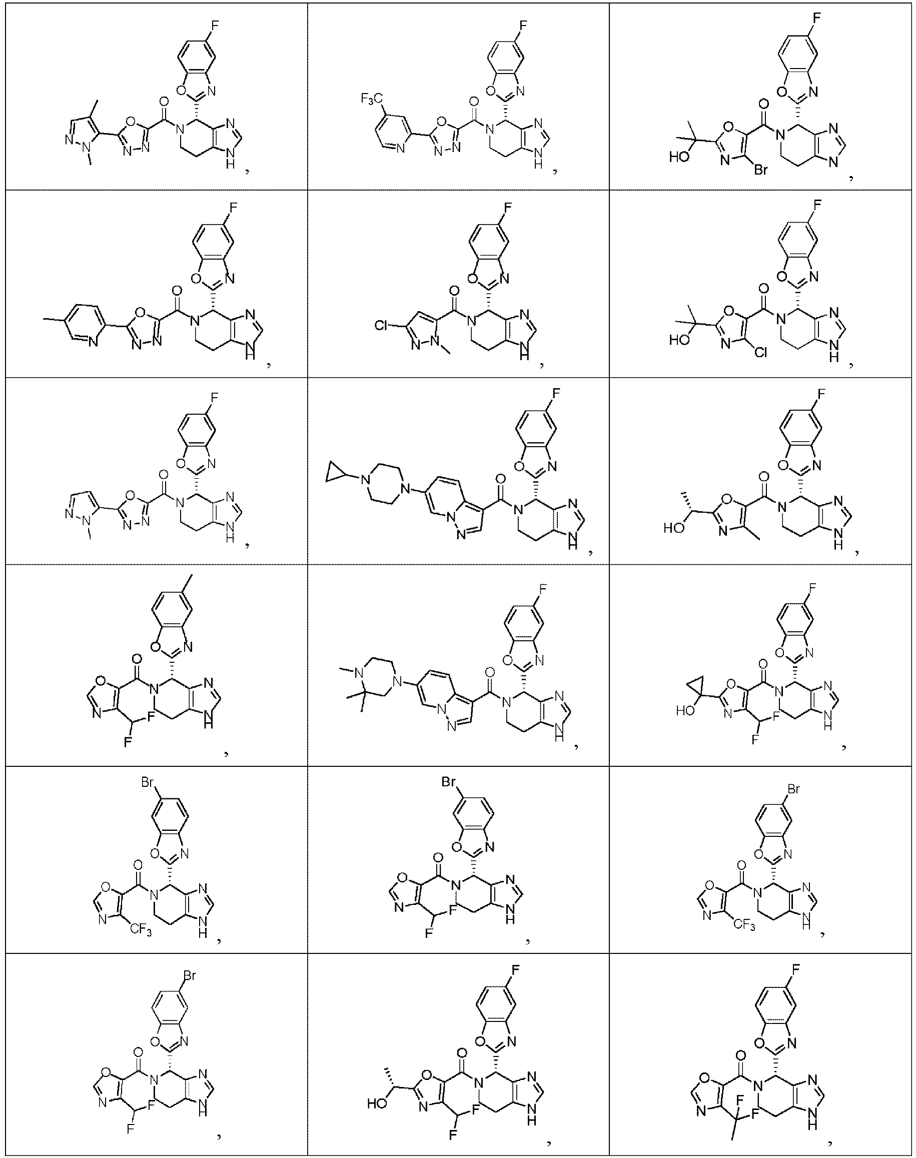

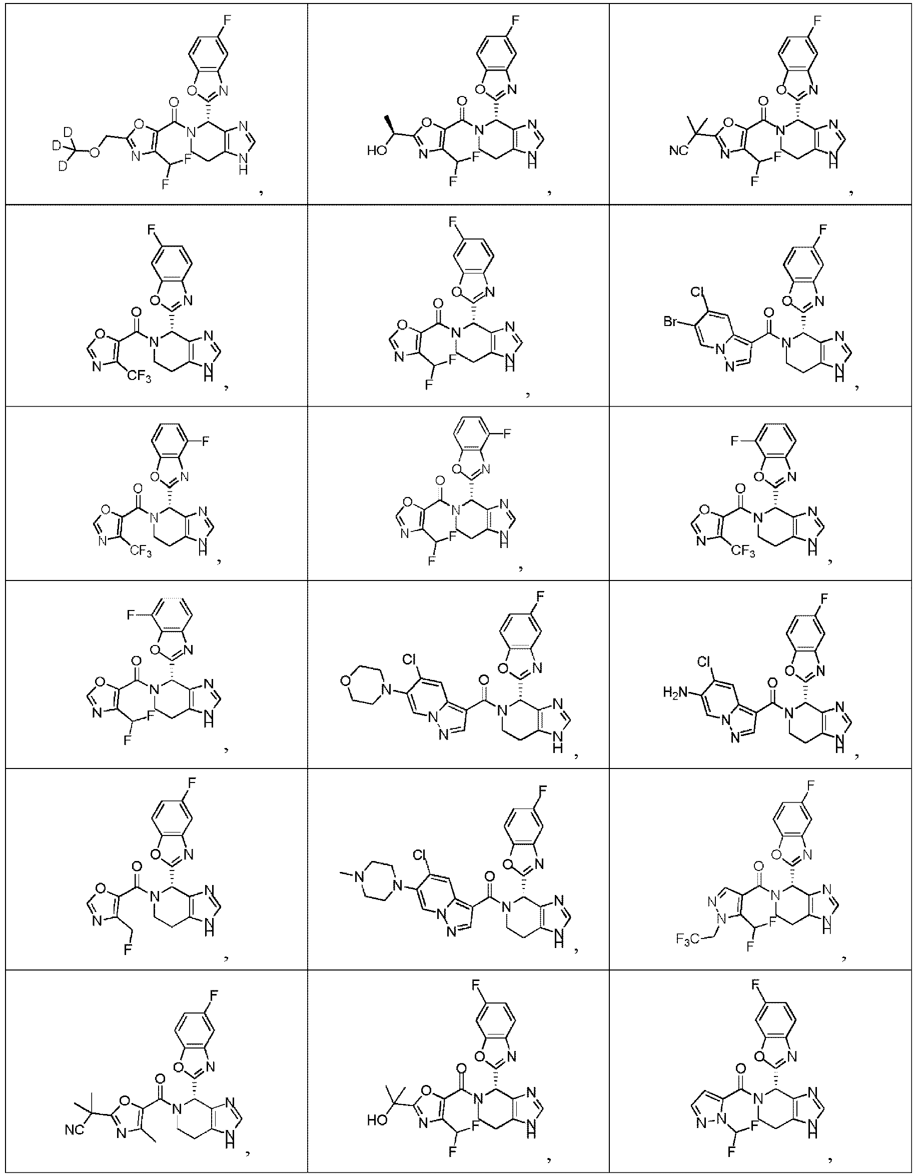

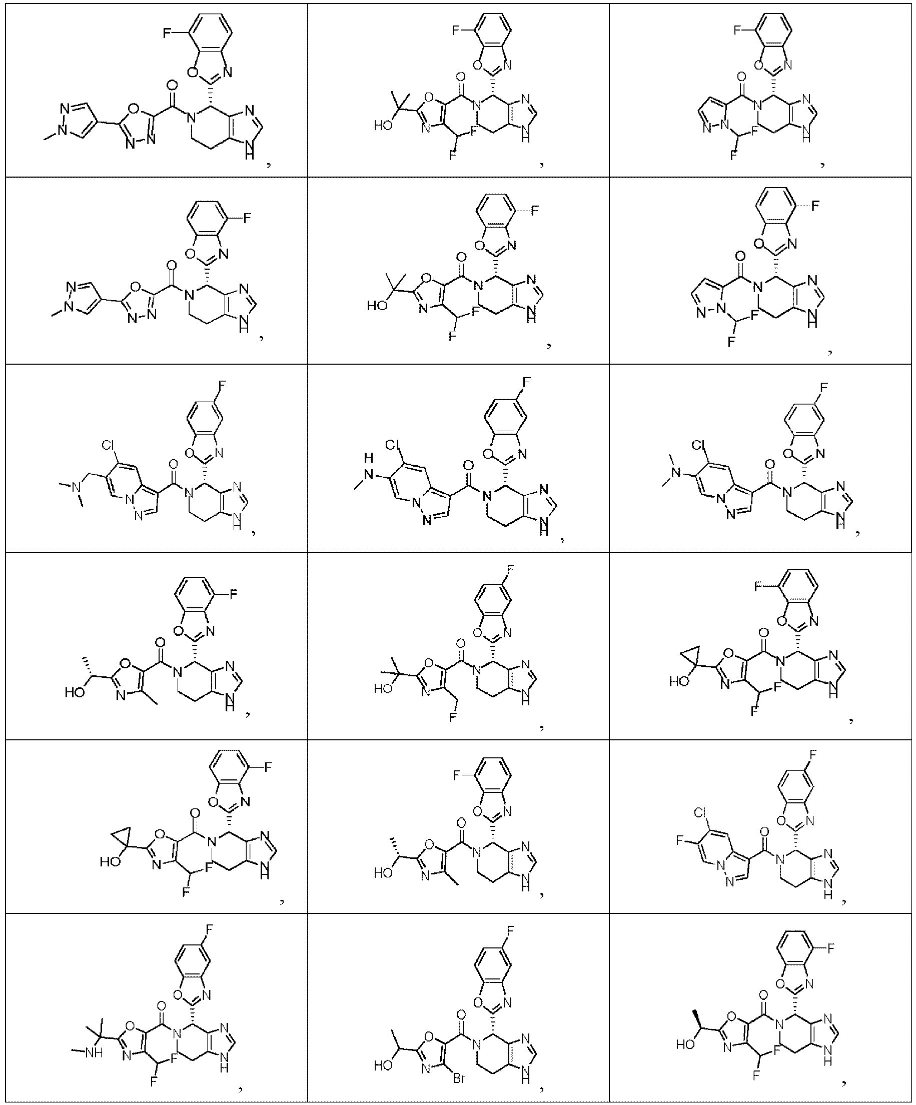

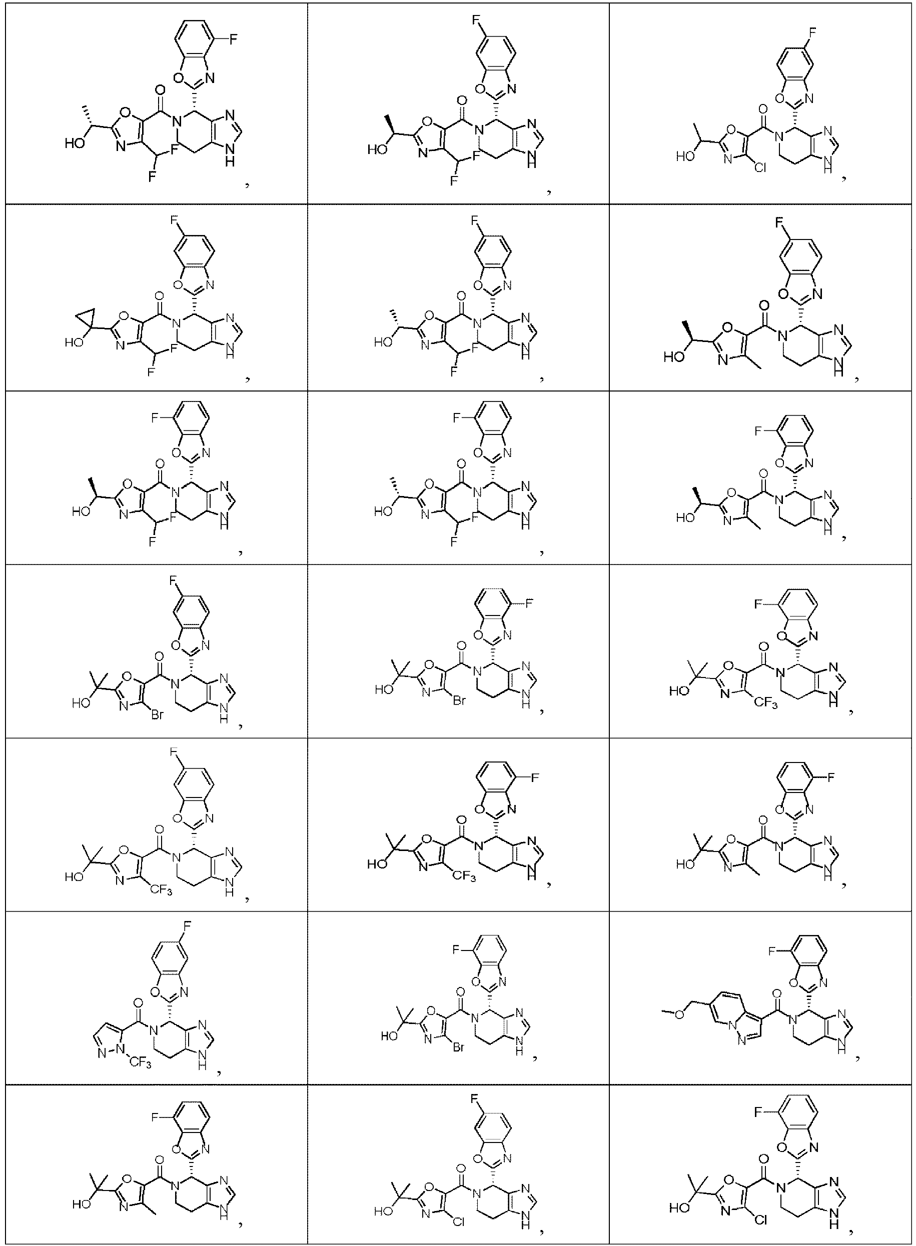

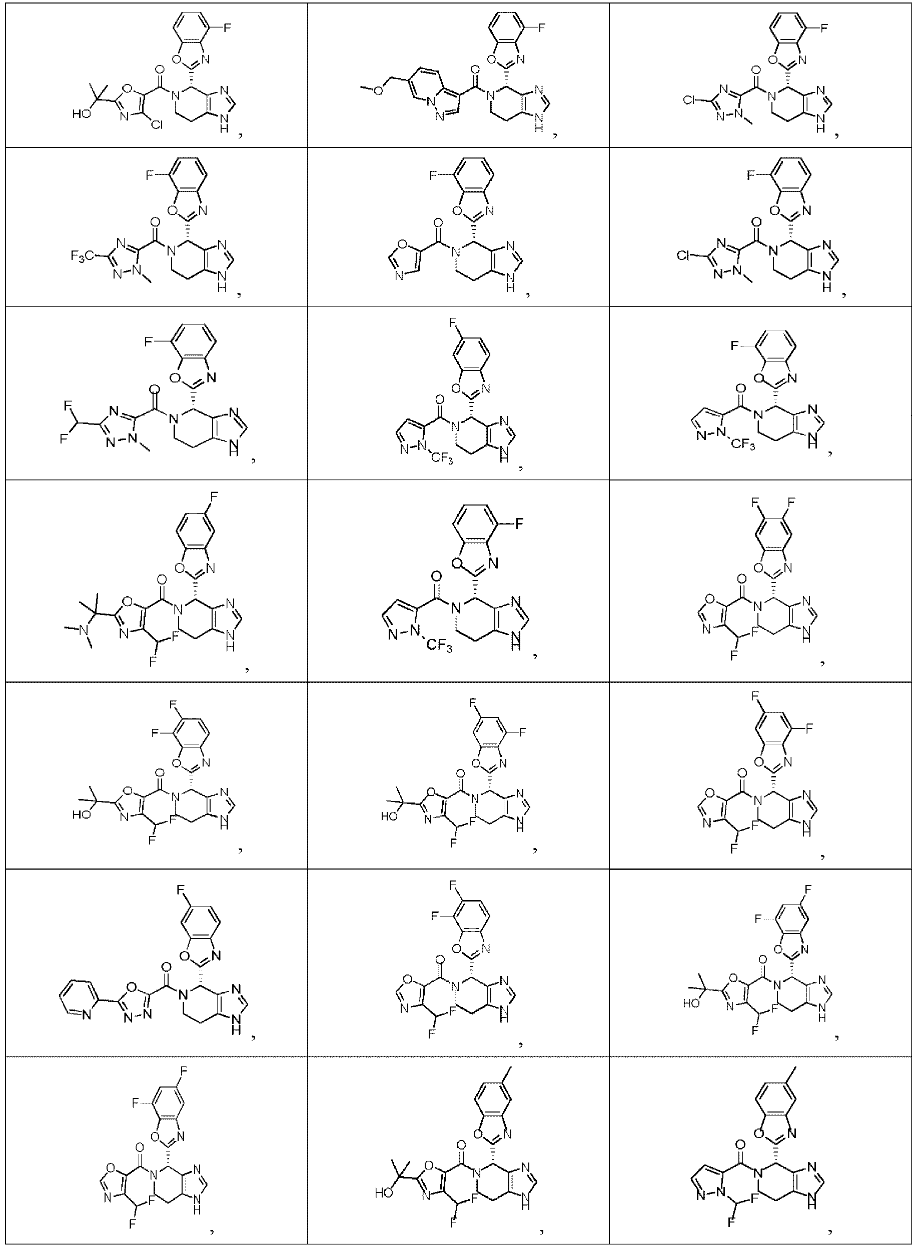

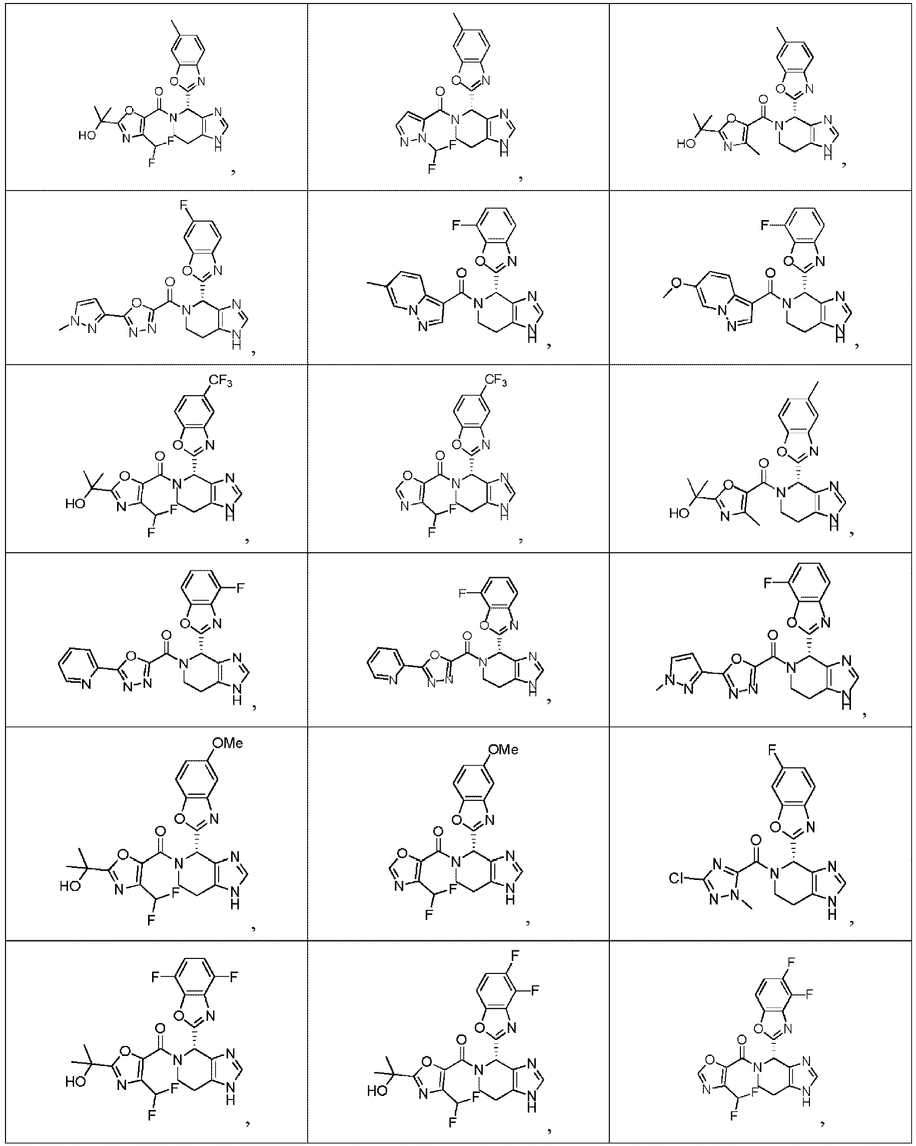

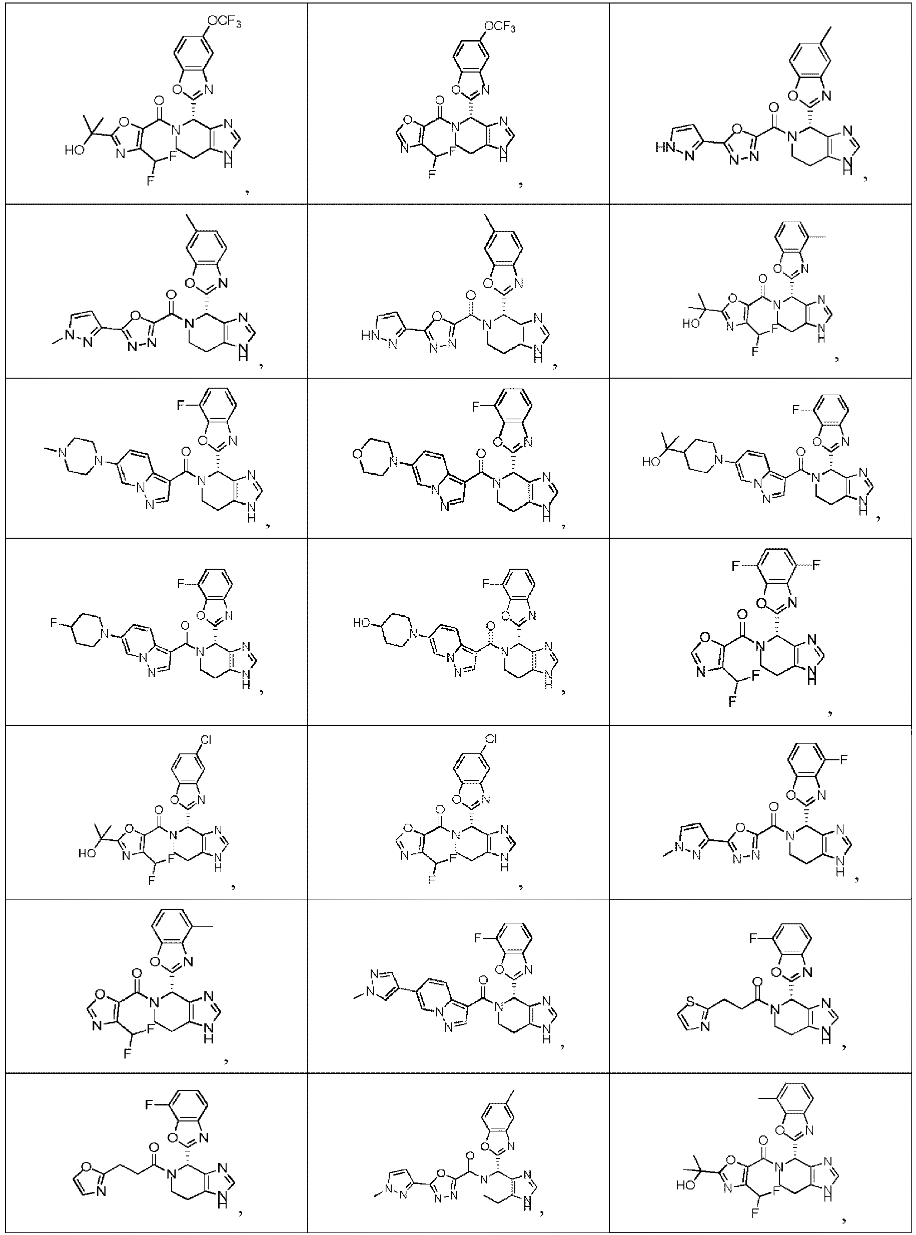

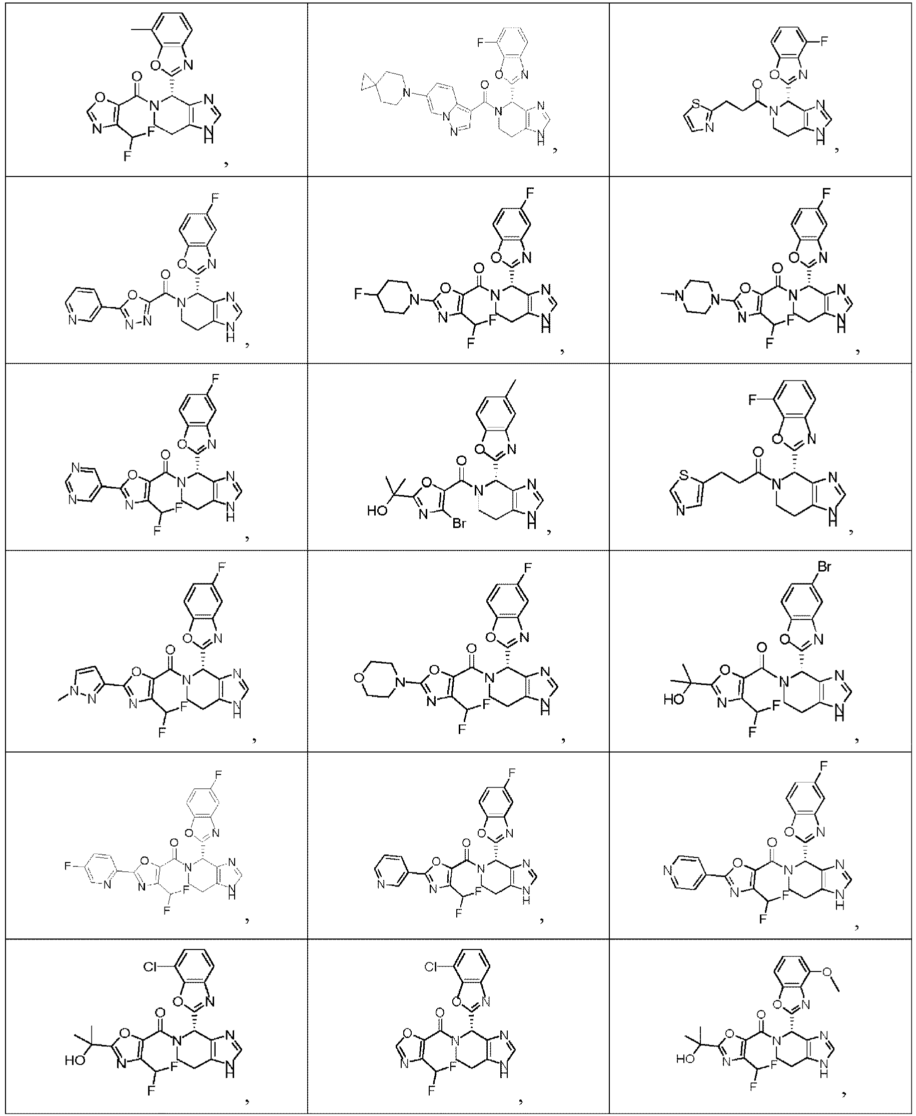

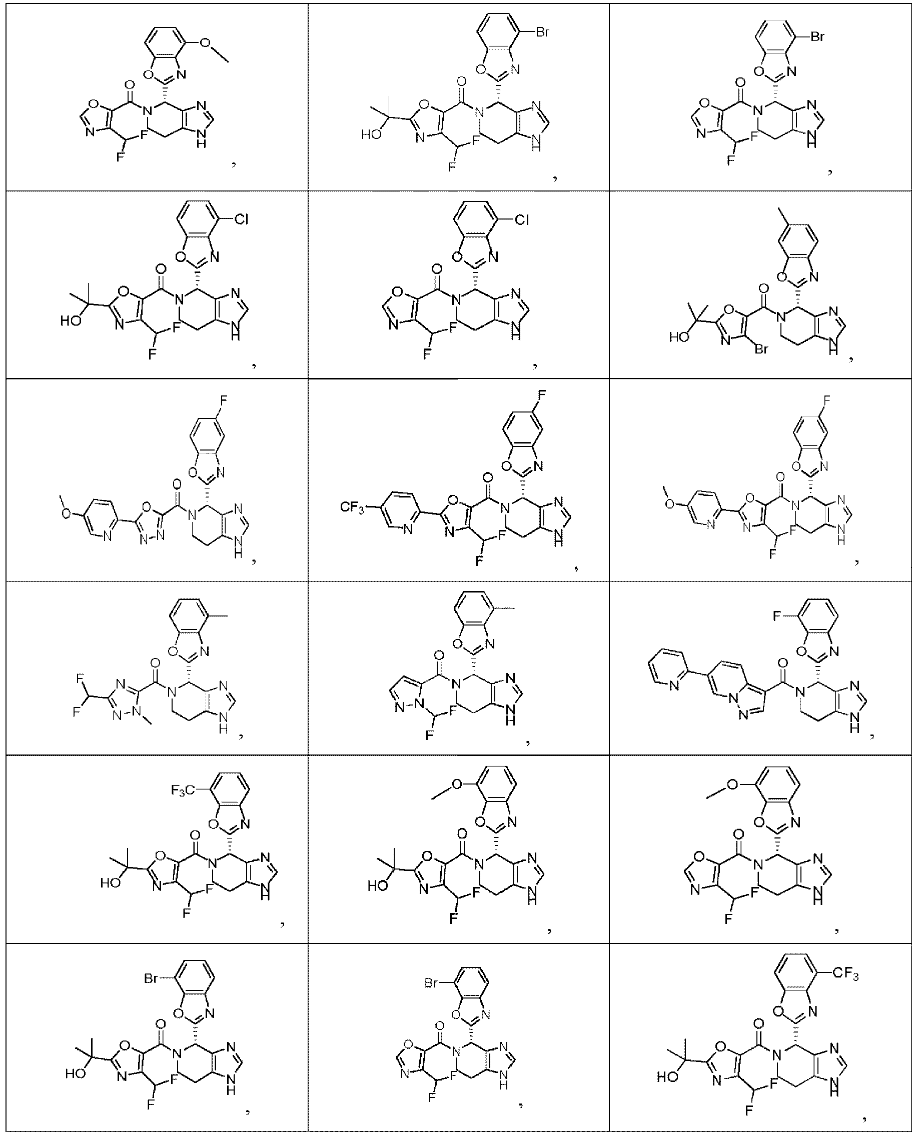

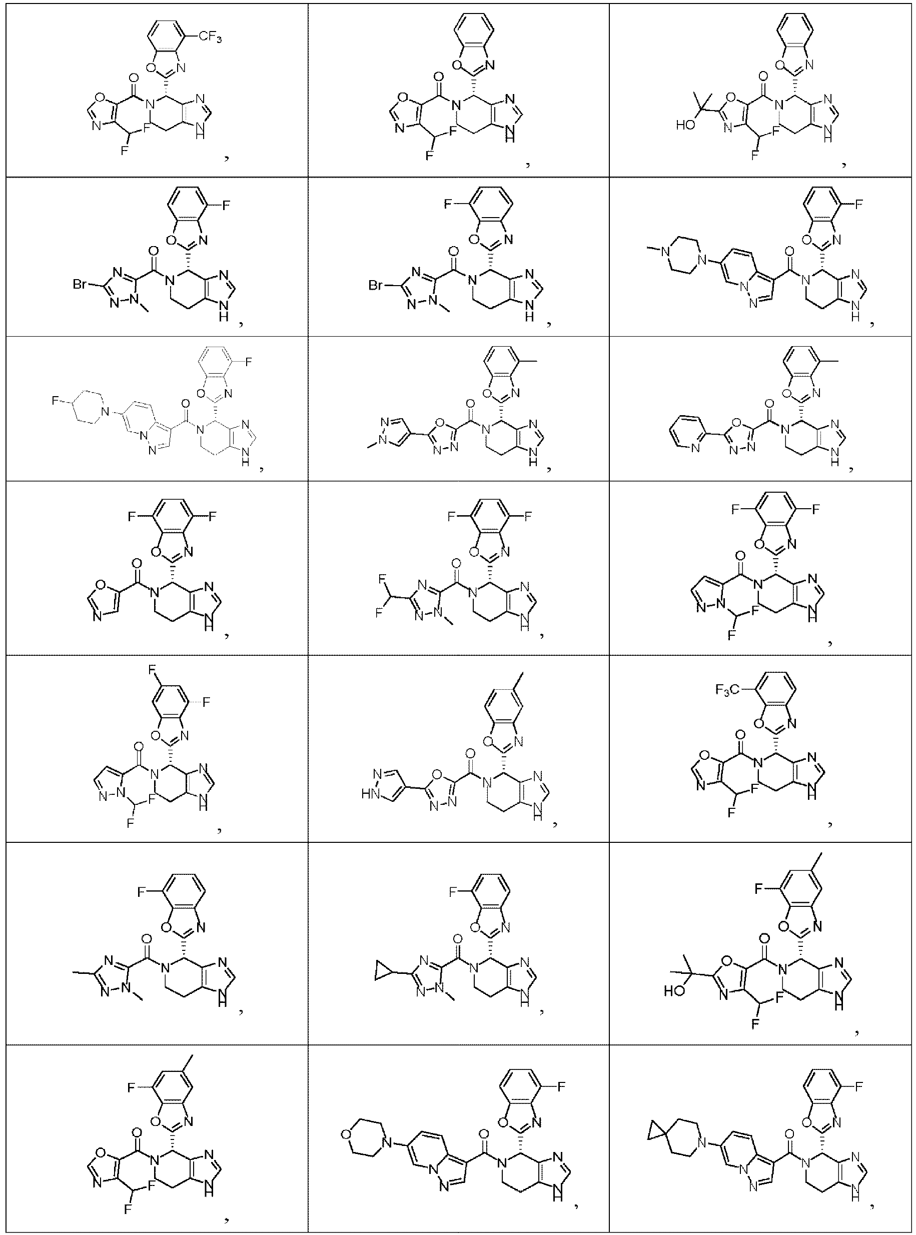

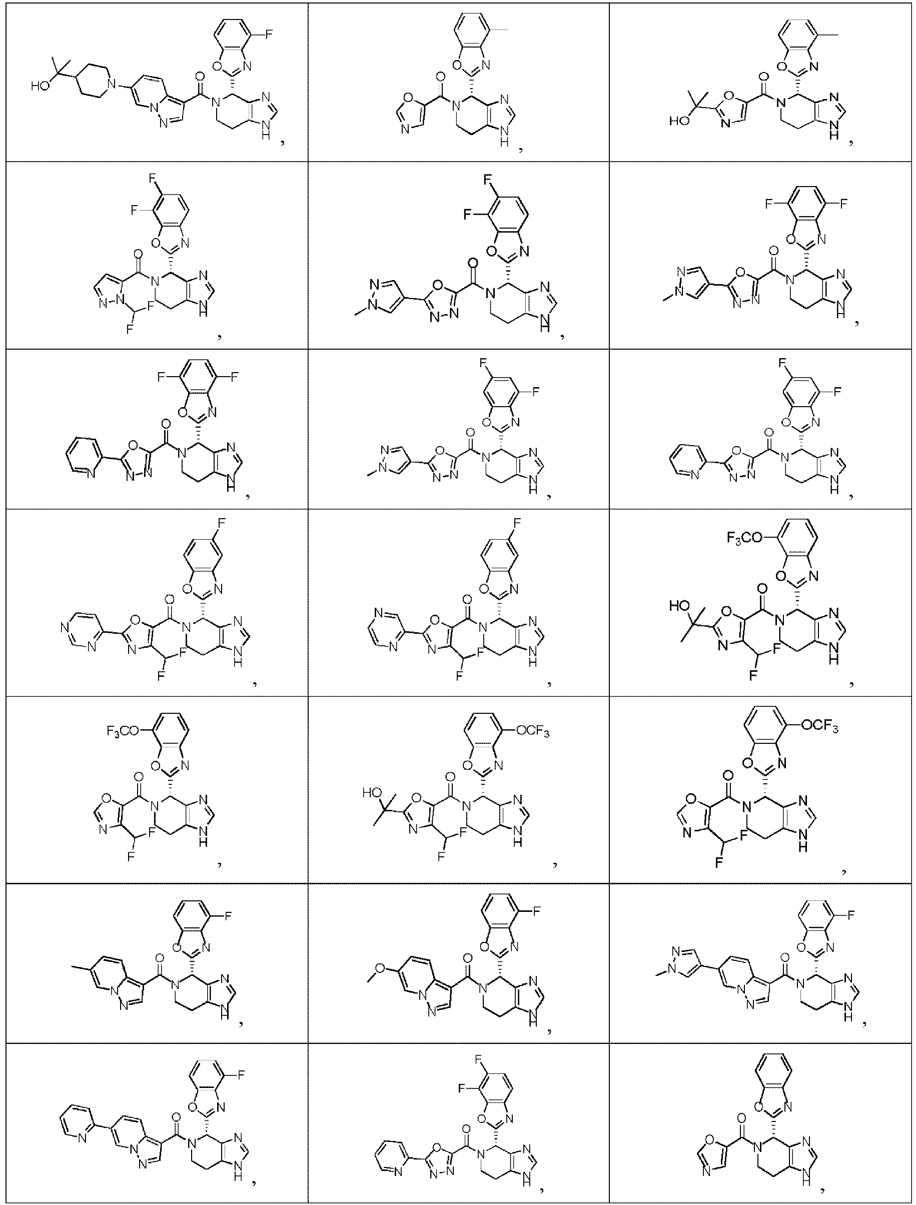

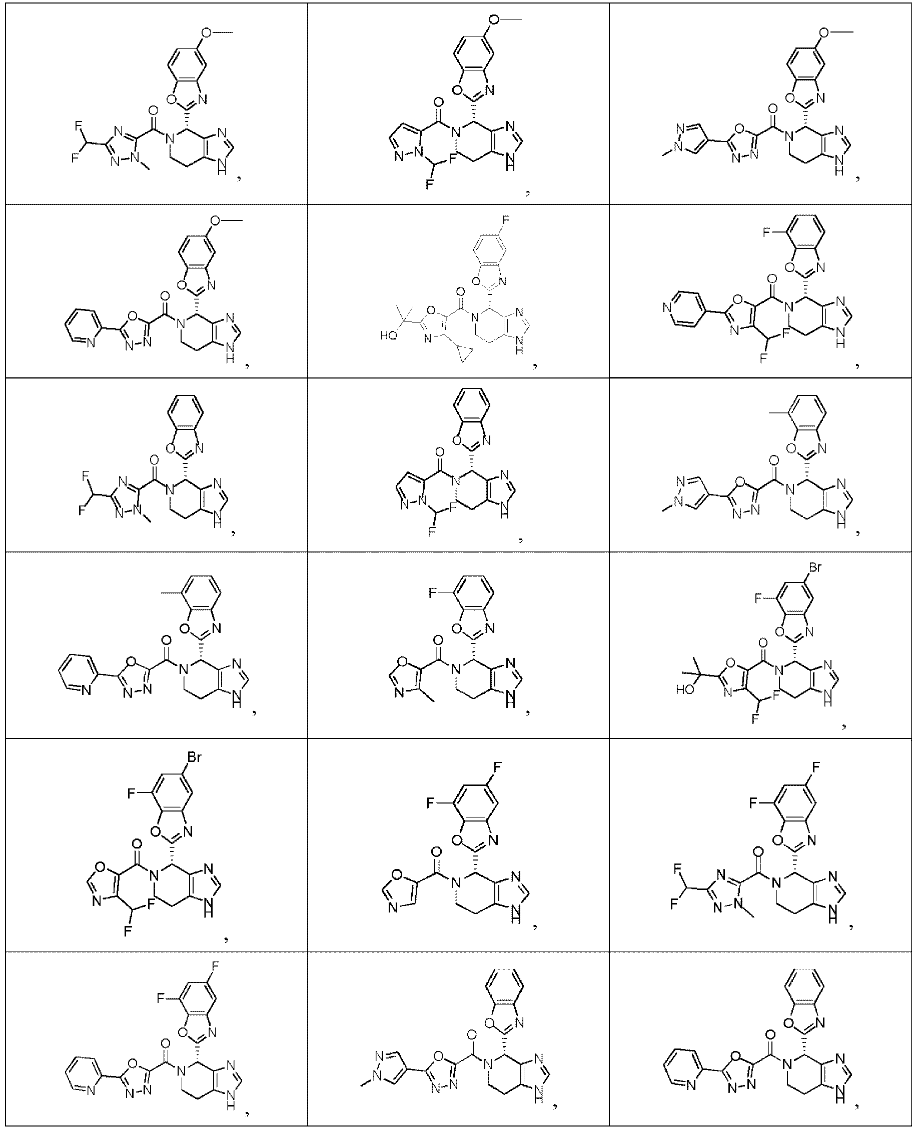

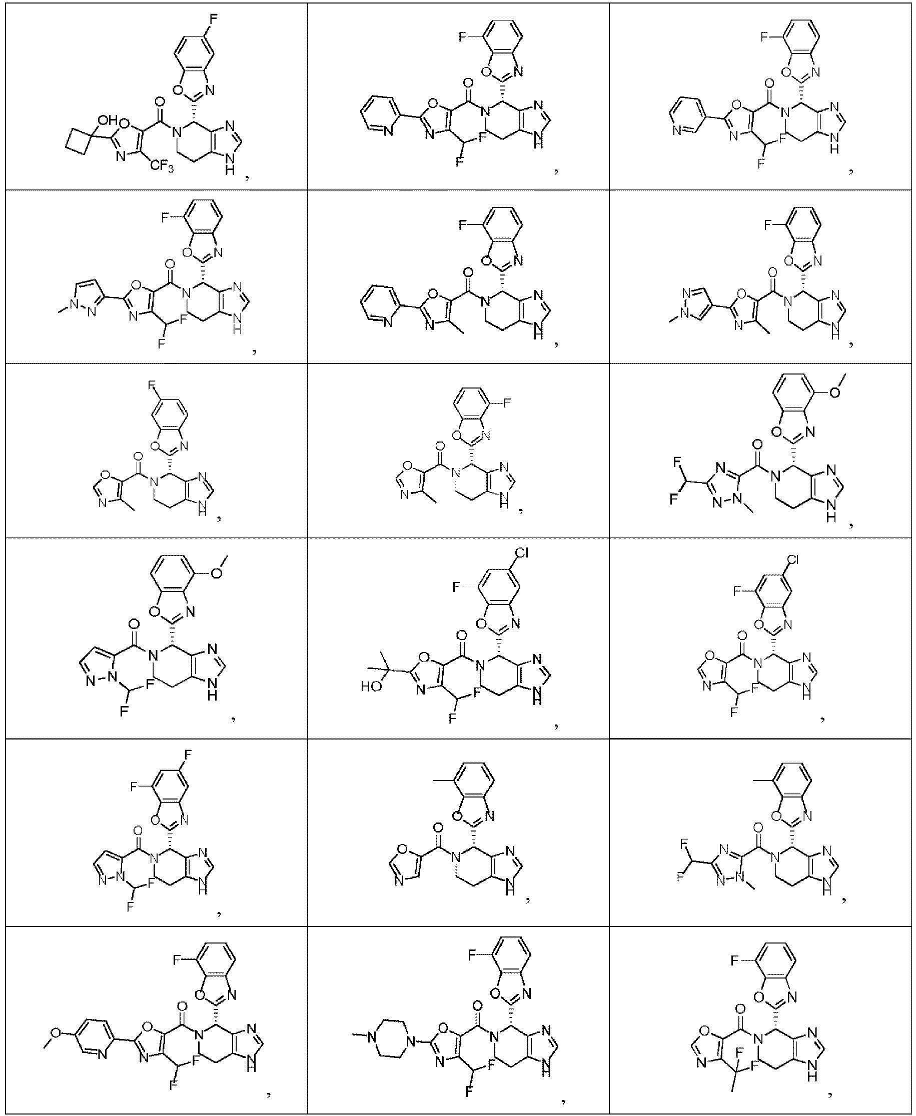

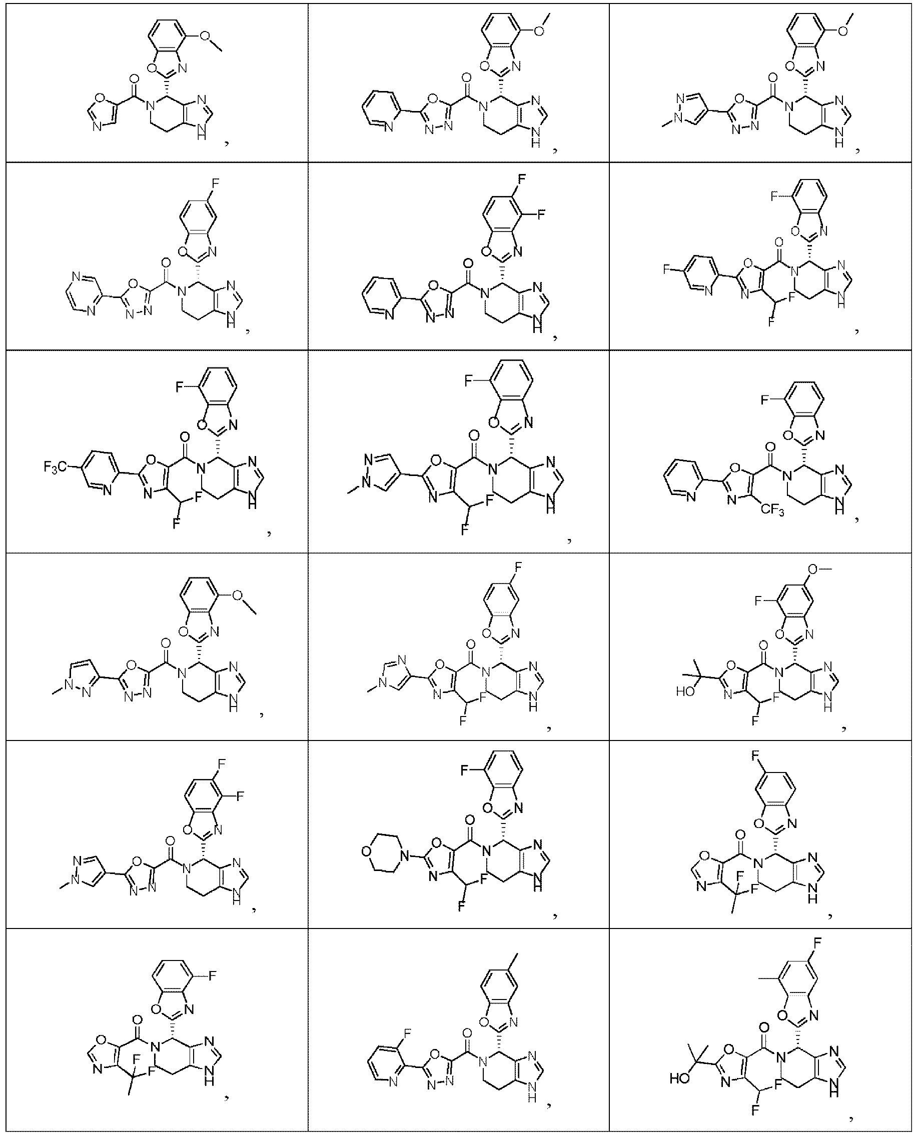

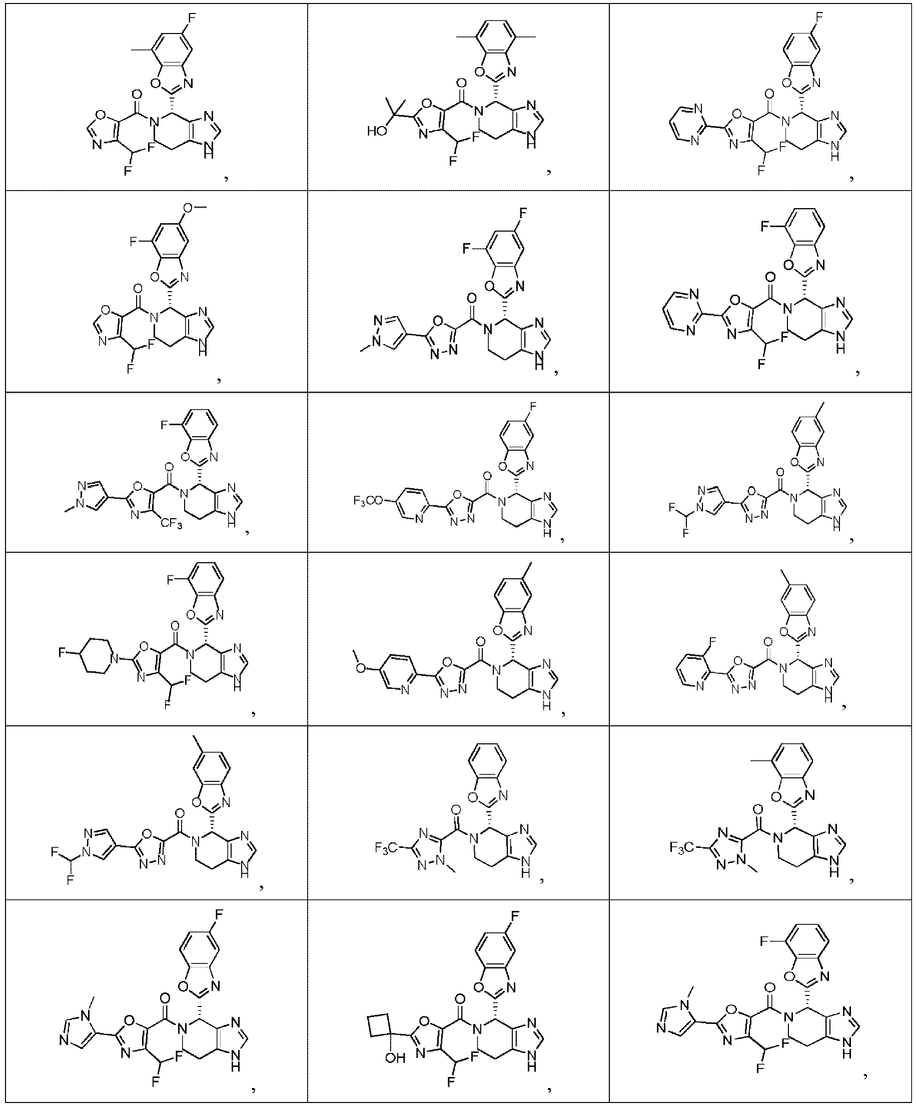

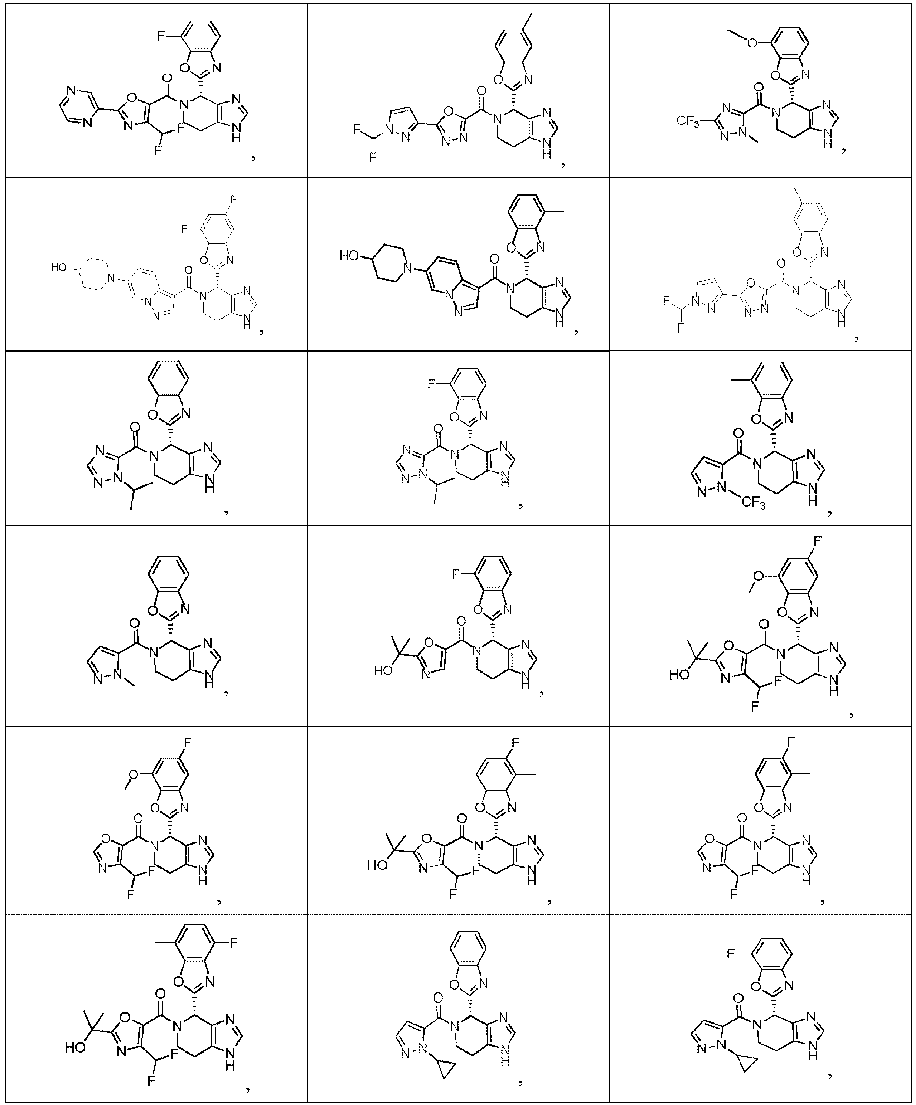

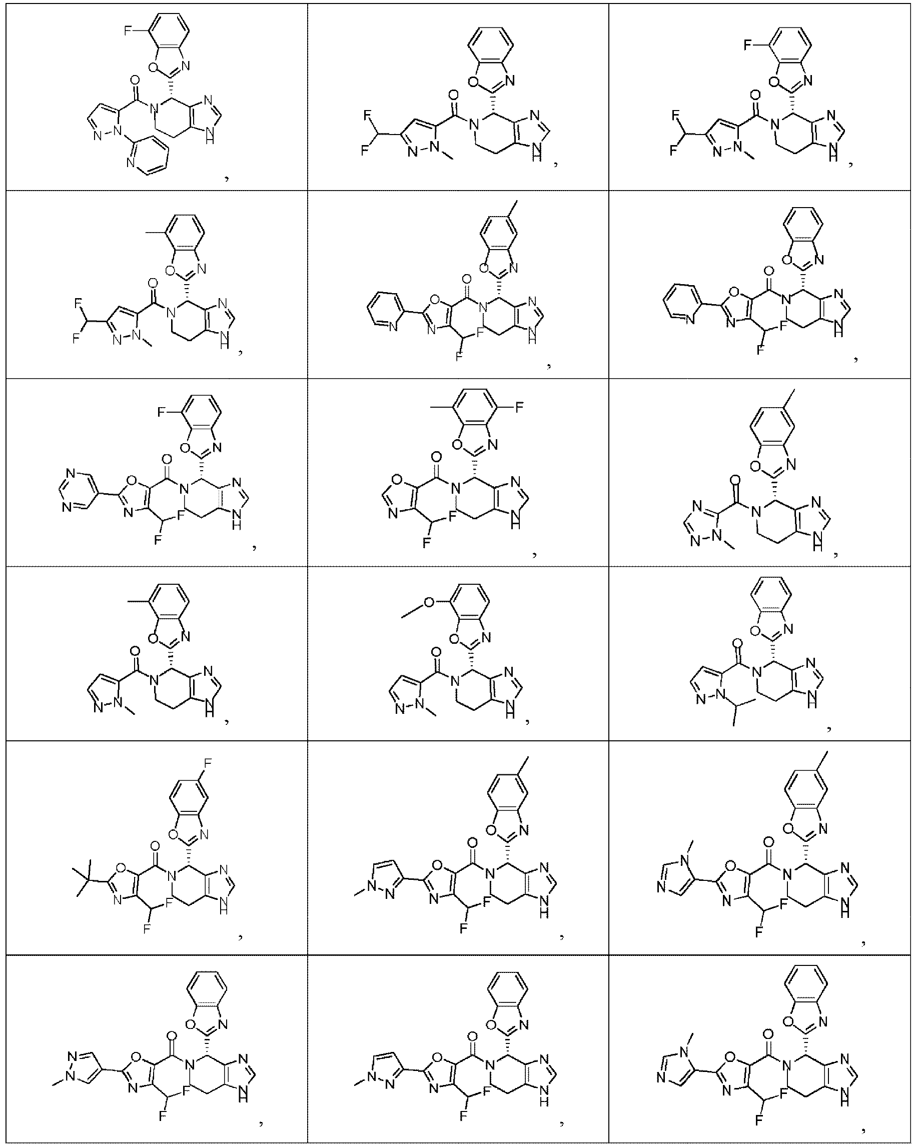

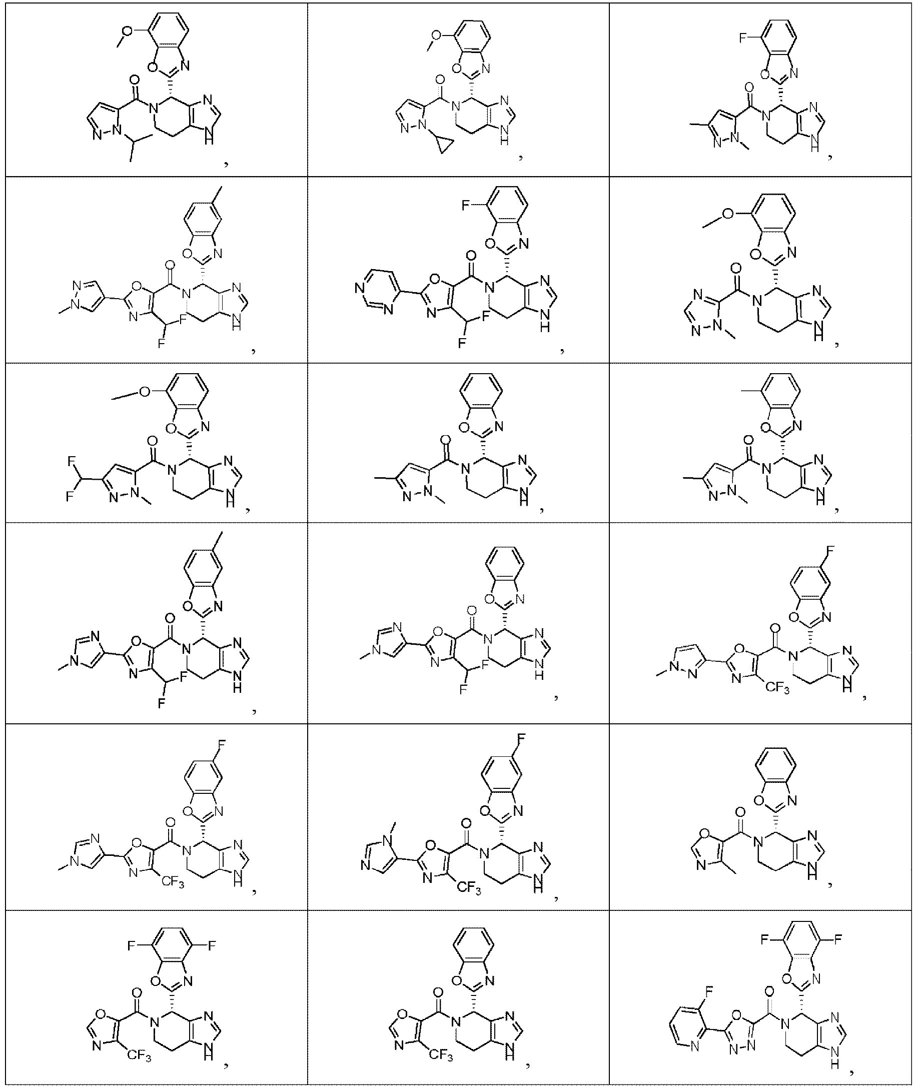

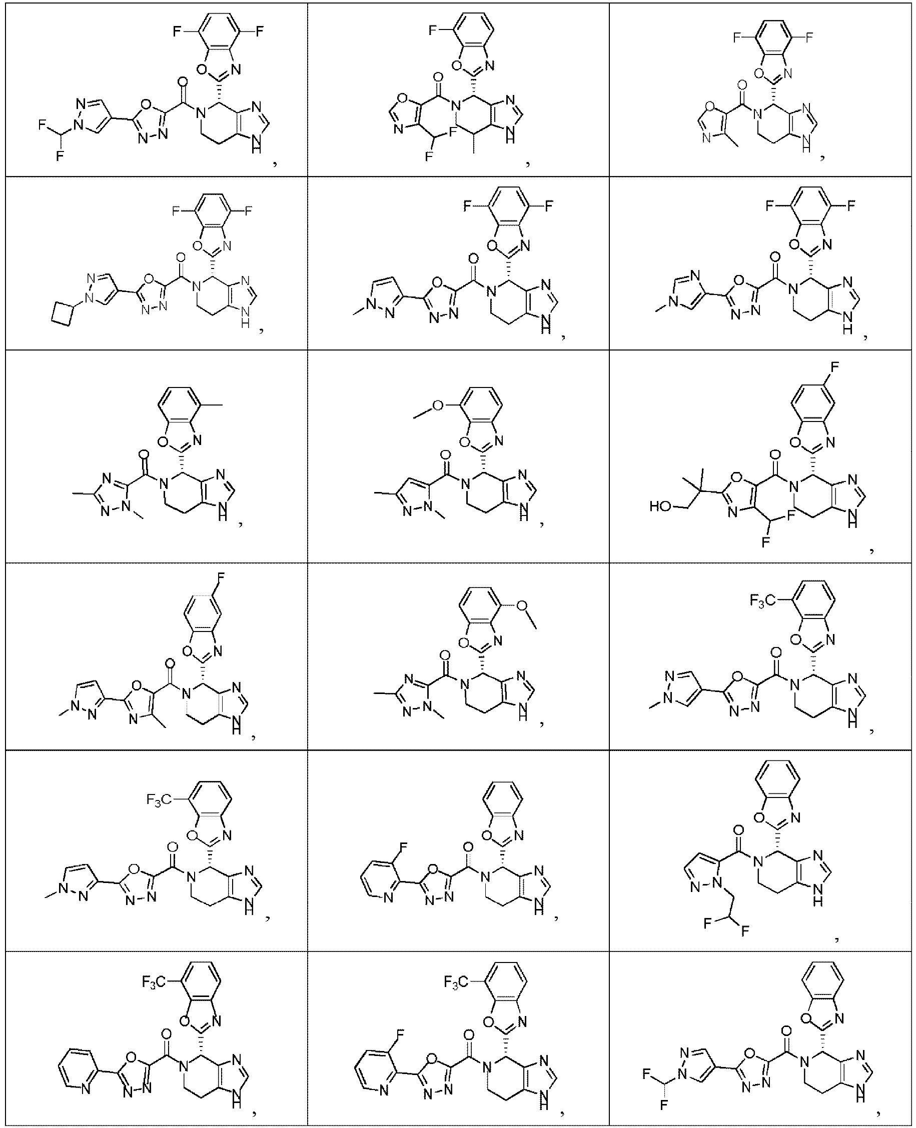

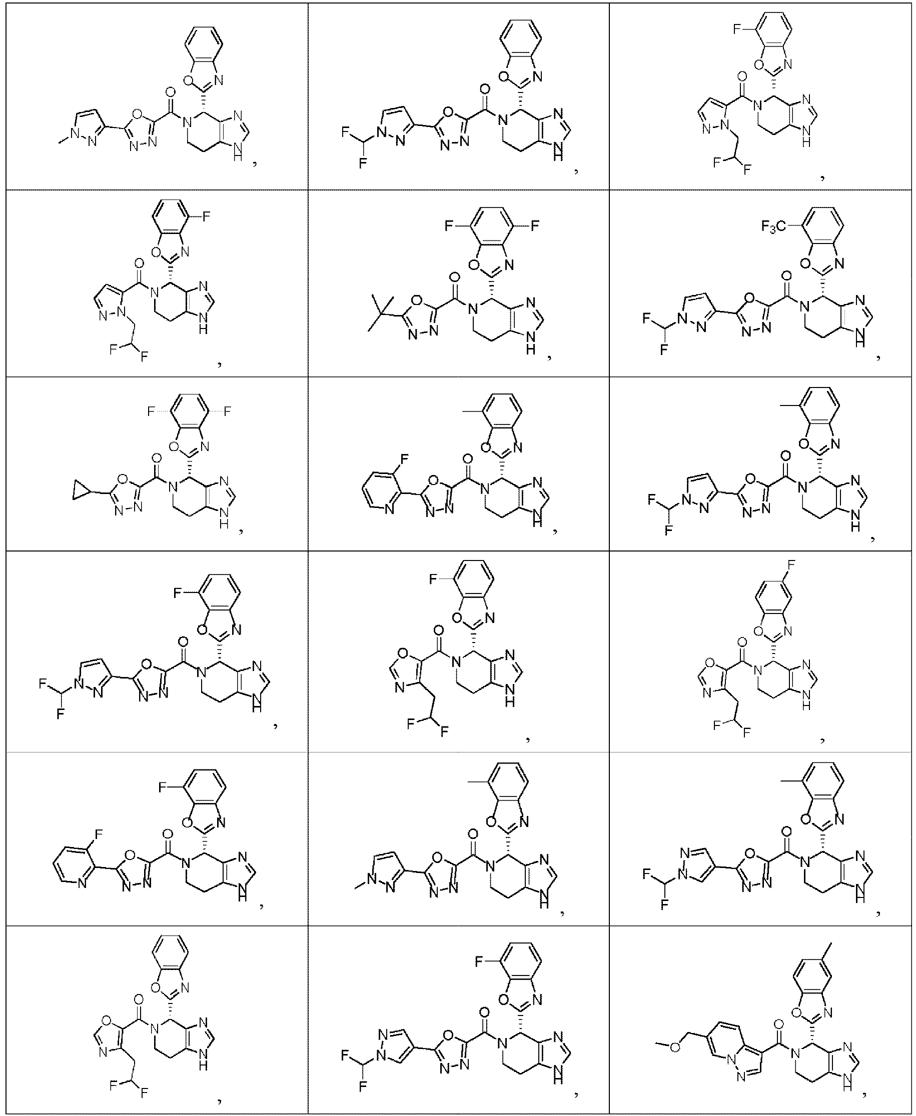

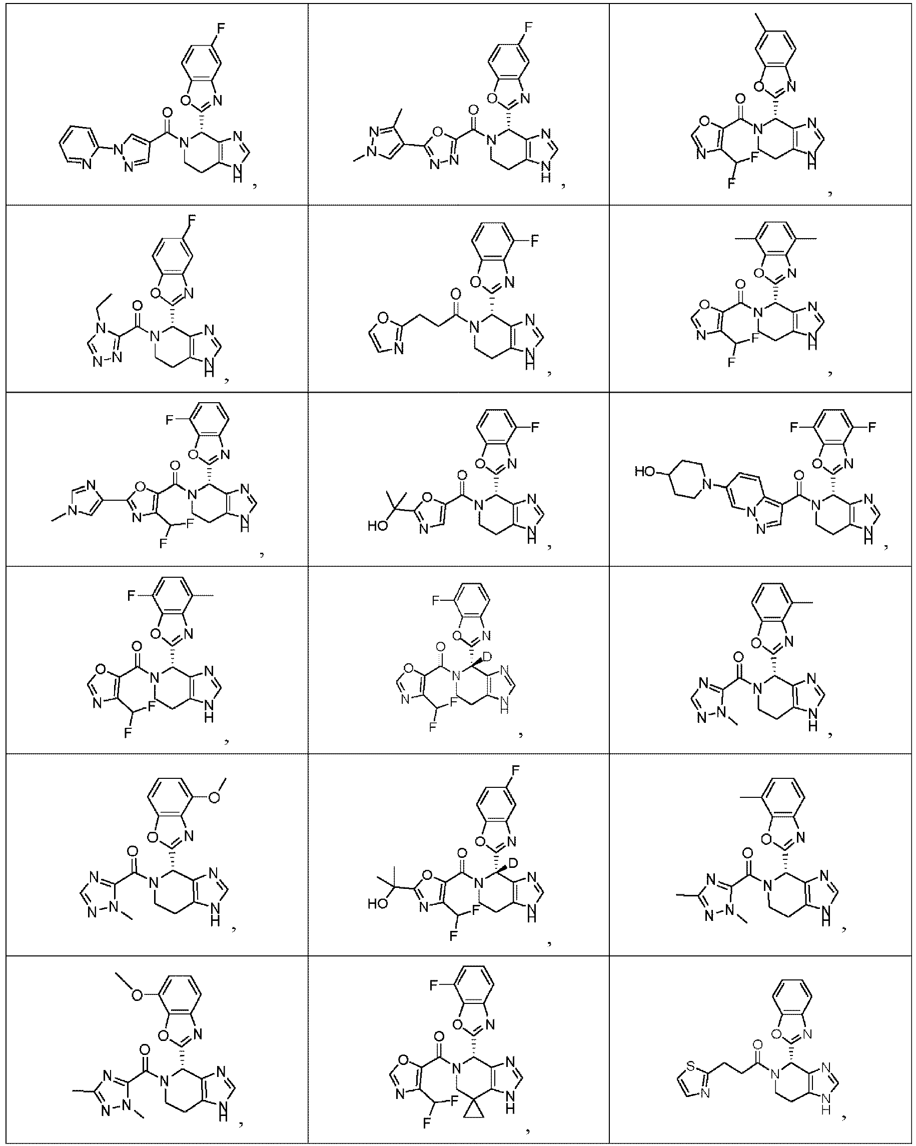

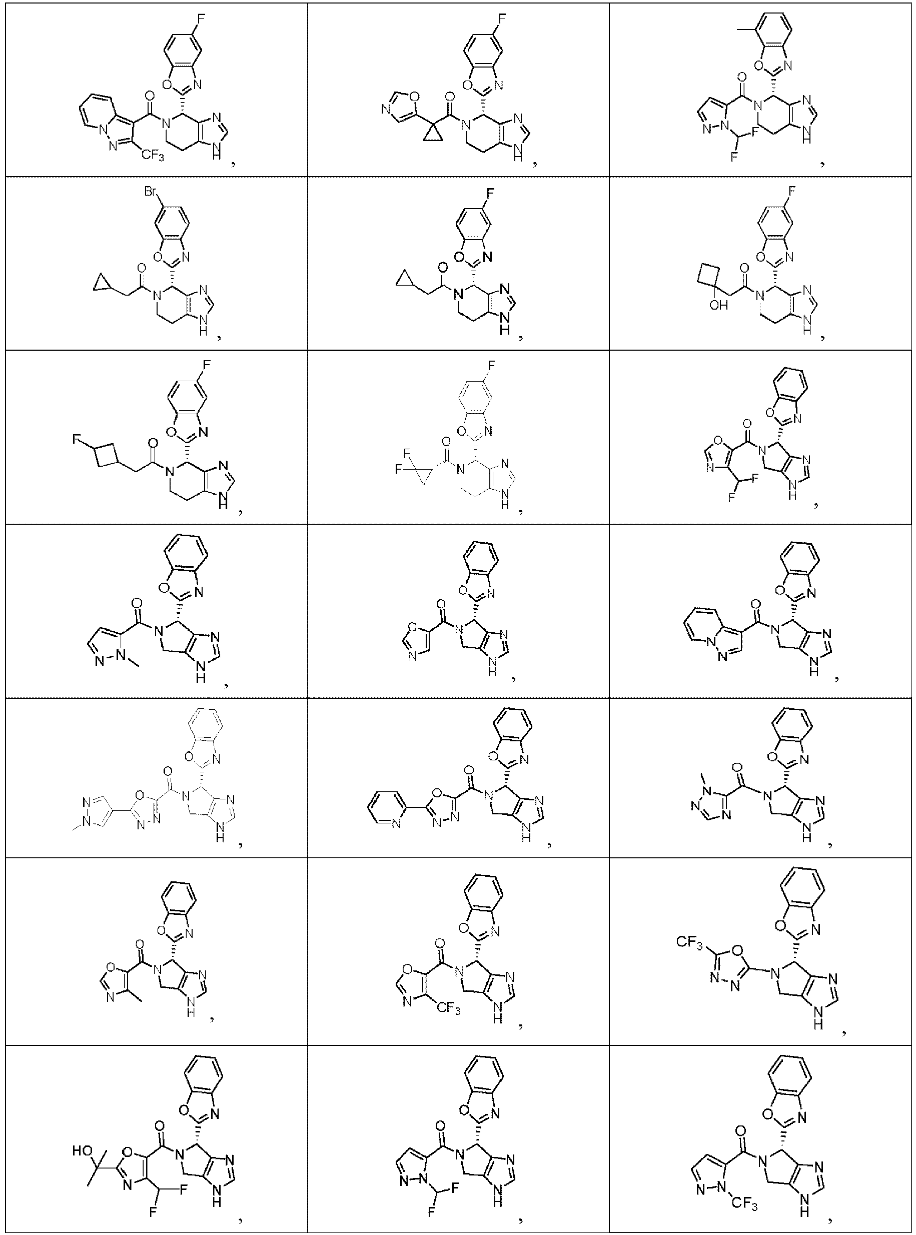

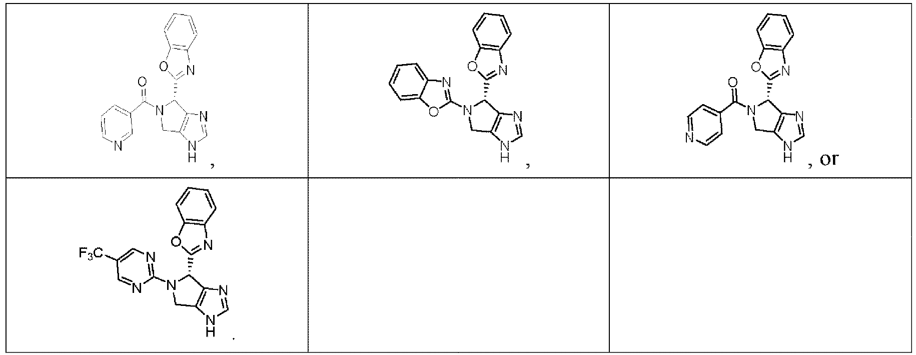

10. Pharmazeutische Zusammensetzung, umfassend: eine Verbindung nach einem der Ansprüche 1 bis 9 oder ein pharmazeutisch verträgliches Salz davon; und einen pharmazeutisch verträglichen Arzneistoffträger.

11. Verbindung nach einem der Ansprüche 1 bis 9 oder ein pharmazeutisch verträgliches Salz davon oder pharmazeutische Zusammensetzung nach Anspruch 10 zur Verwendung bei einem Stabilisieren eines mutierten PAH-Proteins in einem Subjekt, das an Phenylketonurie leidet, umfassend ein Kontaktieren des Proteins mit der Verbindung nach einem der Ansprüche 1 bis 9 oder einem pharmazeutisch verträglichen Salz davon oder der pharmazeutischen Zusammensetzung nach Anspruch 10.

12. Verbindung oder ein pharmazeutisch verträgliches Salz davon oder Zusammensetzung zur Verwendung nach Anspruch 11, wobei:
(i) das mutierte PAH-Protein mindestens eine R408W-, R261Q-, R243Q-, Y414C-, L48S-, A403V-, I65T-, R241C-, L348V-, R408Q- oder V388M-Mutation enthält;
(ii) das mutierte PAH-Protein mindestens eine R408W-, Y414C-, I65T-, F39L-, R408Q-, L348V-, R261Q-, A300S- oder L48S-Mutation enthält;
(iii) das mutierte PAH-Protein mindestens eine R408W-Mutation enthält; oder
(iv) das mutierte PAH-Protein zwei R408W-Mutationen enthält.

13. Verbindung nach einem der Ansprüche 1 bis 9, oder ein pharmazeutisch verträgliches Salz davon, oder pharmazeutische Zusammensetzung nach Anspruch 10 zur Verwendung bei einem Verringern der Phenylalaninkonzentration im Blut bei einem Subjekt, das an Phenylketonurie leidet, optional wobei:
(i) die Phenylalaninkonzentration im Blut auf eine Konzentration von weniger als oder gleich 600 µM reduziert wird; oder
(ii) die Blutphenylalaninkonzentration auf eine Konzentration von weniger als oder gleich 360 µM reduziert wird.

14. Verbindung oder ein pharmazeutisch verträgliches Salz davon oder pharmazeutische Zusammensetzung zur Verwendung nach Anspruch 13, wobei:
(i) das Subjekt vor einer Verabreichung der Verbindung eine Phenylalaninkonzentration im Blut von mehr als 600 µM aufweist; oder
(ii) das Subjekt vor der Verabreichung der Verbindung eine Blutphenylalaninkonzentration von mehr als 1200 µM aufweist.

15. Verbindung nach einem der Ansprüche 1 bis 9 oder ein pharmazeutisch verträgliches Salz davon oder pharmazeutische Zusammensetzung nach Anspruch 10 zur Verwendung bei einem Behandeln von Phenylketonurie bei einem Subjekt.

## Revendications

1. Composé de formule I : 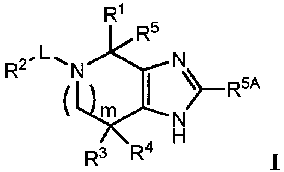 ou un sel pharmaceutiquement acceptable de celui-ci, où :
m vaut 0, 1 ou 2 ;
R¹ est 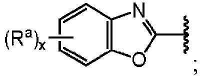
x vaut 0 à 4 ;
chaque R^{a} est indépendamment halogène, alkyle en C₁₋₆, cycloalkyle en C₃₋₆, haloalkyle en C₁₋₆, alcoxy en C₁₋₆ ou haloalcoxy en C₁₋₆ ;
R³ est H ou alkyle en C₁₋₆ ;
R⁴ est H ou alkyle en C₁₋₆ ;
ou R³ et R⁴, conjointement avec l'atome auquel ils sont attachés, forment un cycloalkyle en C₃₋₆ ;
R⁵ est H ou D ;
R^{5A} est H ou D ;
L est une liaison, -C(O)-, -C(O)CH₂-, -C(O)CH₂CH₂-, -C(O)CH₂CH₂CH₂-, - C(O)CF₂-, -C(O)CHF-,-C(O)C(CH₃)₂-, -C(O)CH=CH-, 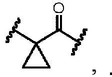 -C(O)NHCH₂-, - CH₂-, ou -CH₂CH₂- ; et
R² est :
(a) 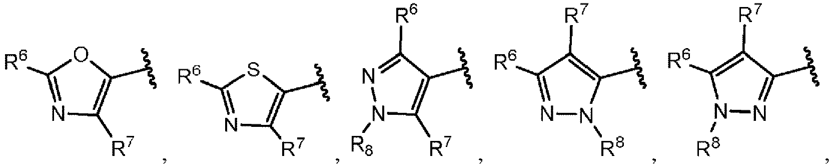 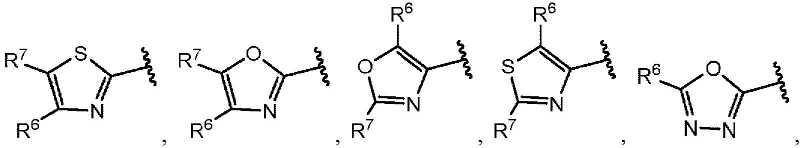 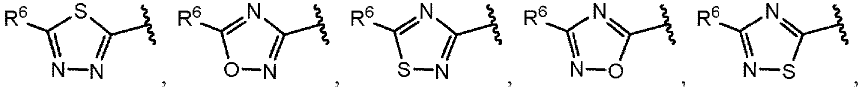 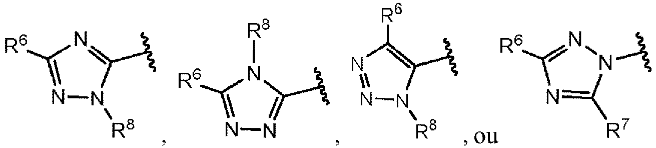 dans lequel :
R⁶ est H ; CN ; alkyle en C₁₋₆ ; haloalkyle en C₁₋₆ ; cyanoalkyle en C₁₋₆ ; hydroxyalkyle en C₁₋₆ ; alcoxy en C₁₋₆ ; alcoxy en C₁₋₆(alkylène) ; haloalcoxy en C₁₋₆ ; haloalcoxy en C₁₋₆(alkylène) ; alcoxy deutéré en C₁₋₆(alkylène) ; halogène ; (CR^{v}R^{x})ₚNR^{y}R^{z} ; C(O)NR^{y2}R^{z2} ; alkylcarbonyle en C₁₋₆ ; cycloalkyle en C₃₋₆ qui est éventuellement substitué par un ou plusieurs parmi halogène, OH, haloalkyle en C₁₋₆, alkyle en C₁₋₆, ou alcoxy en C₁₋₆ ; hétérocyclyle qui est éventuellement substitué par un ou plusieurs parmi halogène, OH, haloalkyle en C₁₋₆, alkyle en C₁₋₆, hydroxyalkyle en C₁₋₆, alcoxy en C₁₋₆, C(O)O(alkyle en C₁₋₆), ou cycloalkyle en C₃₋₆ ; aryle qui est éventuellement substitué par un ou plusieurs parmi halogène, haloalkyle en C₁₋₆, alkyle en C₁₋₆, ou cycloalkyle en C₃₋₆ ; hétéroaryle qui est éventuellement substitué par un ou plusieurs parmi halogène, haloalkyle en C₁₋₆, alkyle en C₁₋₆, alcoxy en C₁₋₆, haloalcoxy en C₁₋₆, cycloalkyle en C₃₋₆, ou cycloalkylsulfonyle en C₃₋₆ ; alcényle en C₂₋₆ ; cycloalcényle en C₃₋₈ ; cycloalkyl en C₃₋₈(alkylène) ; aryl(alkylène) ; hétérocyclyl(alkylène) qui est éventuellement substitué par un ou plusieurs parmi halogène, OH, haloalkyle en C₁₋₆, alkyle en C₁₋₆, hydroxyalkyle en C₁₋₆, alcoxy en C₁₋₆, C(O)O(alkyle en C₁₋₆), ou cycloalkyle en C₃₋₆ ; ou alkylsulfonyle en C₁₋₆,
R⁷ est H, CN, alkyle en C₁₋₆, haloalkyle en C₁₋₆, cyanoalkyle en C₁₋₆, hydroxyalkyle en C₁₋₆, alcoxy en C₁₋₆, alcoxy en C₁₋₆(alkylène), haloalcoxy en C₁₋₆, haloalcoxy en C₁₋₆(alkylène), alcoxy deutéré en C₁₋₆(alkylène), halogène, (CR^{v}R^{x})ₚNR^{y}R^{z}, C(O)NR^{y2}R^{z2}, alkylcarbonyle en C₁₋₆, cycloalkyle en C₃₋₆, hétérocyclyle, aryle, hétéroaryle, alcényle en C₂₋₆ ; cycloalcényle en C₃₋₈ ; cycloalkyl en C₃₋₈(alkylène) ; aryl(alkylène) ; hétérocyclyl(alkylène) ; ou alkylsulfonyle en C₁₋₆,
R⁸ est H, alkyle en C₁₋₆, haloalkyle en C₁₋₆, alcoxy en C₁₋₆(alkylène), alkylcarbonyle en C₁₋₆, hydroxyalkyle en C₁₋₆, (CR^{v}R^{x})ₚNR^{y}R^{z}, cycloalkyle en C₃₋₈, aryle, hétéroaryle, ou cycloalkyl en C₃₋₈(alkylène) ;
R^{v} et R^{x} sont, indépendamment, H ou alkyle en C₁₋₆ ;
R^{y} et R^{z} sont, indépendamment, H, alkyle en C₁₋₆, alcoxy en C₁₋₆(alkylène), ou cycloalkyle en C₃₋₆ ;
R^{y2} et R^{z2} sont, indépendamment, H, alkyle en C₁₋₆, ou cycloalkyle en C₃₋₆ ; et
p vaut 0, 1, 2, ou 3 ; ou
(b) 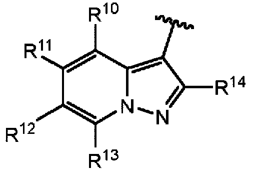 où :
R¹⁰, R¹¹, R¹², R¹³ et R¹⁴ sont, indépendamment, H, alkyle en C₁₋₆, haloalkyle en C₁₋₆, alcoxy en C₁₋₆, alcoxy en C₁₋₆(alkylène), halogène, hétérocyclyle éventuellement substitué, hétérocyclyl(alkylène) éventuellement substitué, hétéroaryle éventuellement substitué, ou (CR^{v}R^{x})ₚNR^{y}R^{z}, où la substitution facultative sur les groupes hétérocyclyle et hétérocyclyl(alkylène) est un ou plusieurs parmi halogène, haloalkyle en C₁₋₆, alkyle en C₁₋₆, OH, hydroxyalkyle en C₁₋₆, alcoxy en C₁₋₆, ou cycloalkyle en C₃₋₆ et la substitution facultative sur le groupe hétéroaryle est un ou plusieurs alkyle en C₁₋₆ ;
R^{v} et R^{x} sont, indépendamment, H ou alkyle en C₁₋₆ ;
R^{y} et R^{z} sont, indépendamment, H, alkyle en C₁₋₆, cycloalkyle en C₃₋₆, ou C(O)Oalkyle en C₁₋₆ ; et p vaut 0, 1, 2, ou 3.

2. Composé selon la revendication 1, dans lequel le composé est de :
(i) formule I-A-1, 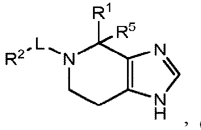 ou un sel pharmaceutiquement acceptable de celui-ci ; ou
(ii) Formule I-A-3, 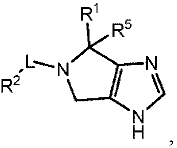 ou un sel pharmaceutiquement acceptable de celui-ci ; ou
(iii) Formule I-B-1, 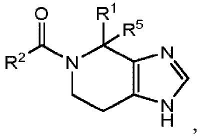 ou un sel pharmaceutiquement acceptable de celui-ci ; ou
(iv) Formule I-B-3, 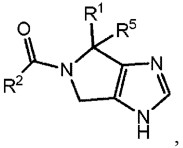 ou un sel pharmaceutiquement acceptable de celui-ci ; ou
(v) Formule I-C-1, 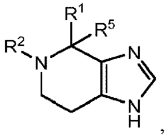 ou un sel pharmaceutiquement acceptable de celui-ci ; ou
(vi) Formule I-C-3, 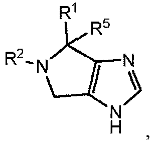 ou un sel pharmaceutiquement acceptable de celui-ci ; ou
(vii) Formule I-D-1, 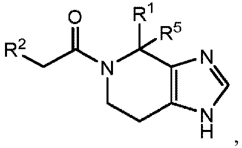 ou un sel pharmaceutiquement acceptable de celui-ci ; ou
(viii) Formule I-G-1, 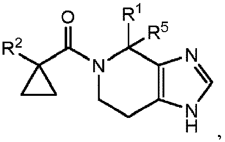 ou un sel pharmaceutiquement acceptable de celui-ci ; ou
(ix) Formule I-I-1, 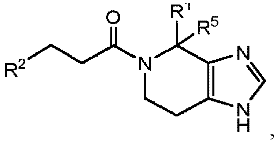 ou un sel pharmaceutiquement acceptable de celui-ci ; ou
(x) Formule I-L-1, 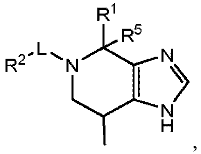 ou un sel pharmaceutiquement acceptable de celui-ci ; ou
(xi) Formule I-M-1, 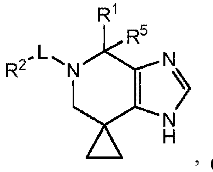 ou un sel pharmaceutiquement acceptable de celui-ci.

3. Composé selon la revendication 1 ou la revendication 2, ou un sel pharmaceutiquement acceptable de celui-ci, dans lequel :
(i) R¹ est 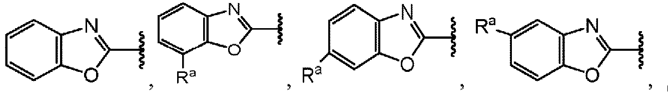 ou 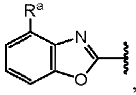 éventuellement dans lequel
a. R^{a} est halogène tel que F, Br ou CI ;
b. R^{a} est alkyle en C₁₋₆ tel que méthyle ;
c. R^{a} est alcoxy en C₁₋₆ tel que méthoxy ;
d. R^{a} est haloalkyle en C₁₋₆ tel que CF₃ ; ou
e. R^{a} est haloalcoxy en C₁₋₆ tel que OCF₃ ; ou
(ii) R¹ is 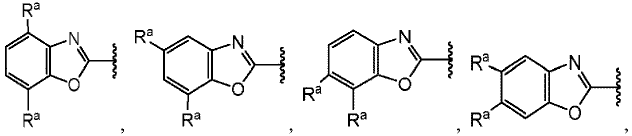 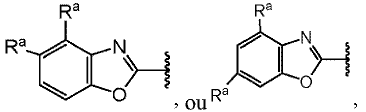 éventuellement dans lequel un R^{a} est halogène tel que
Br, CI, ou F ou alkyle en C₁₋₆ tel que méthyle, et le deuxième R^{a} est halogène tel que Br, CI, ou F, alcoxy en C₁₋₆, tel que méthoxy, ou alkyle en C₁₋₆, tel que méthyle.

4. Composé selon l'une quelconque des revendications 1 à 3, ou un sel pharmaceutiquement acceptable de celui-ci, dans lequel :
(i) R² est (a), et R⁶, R⁷, R⁸ sont chacun H ; ou
(ii) R² est (b), et R¹⁰, R¹³, et R¹⁴ sont chacun H ; ou
(iii) R² est (b), et R¹⁰, R¹¹, R¹³, et R¹⁴ sont chacun H ; ou
(iv) R² est (a), et R⁶ est alkyle en C₁₋₆ tel que méthyle, éthyle, isopropyle ou tert-butyle, haloalkyle en C₁₋₆ tel que CF₃, CHF₂, CH₂F, CH₂CF₃, CH(CH₃)F, C(CH₃)F₂, ou C(CH₃)₂F, cyanoalkyle en C₁₋₆ tel que C(CH₃)₂CN, ou hydroxyalkyle en C₁₋₆ tel que C(CH₃)₂OH, CH(CH₃)OH, CH(CH₂CH₃)OH, CH₂C(CH₃)₂OH, ou C(CH₃)₂CH₂OH ; ou
(v) R² est (a) et R⁶ est 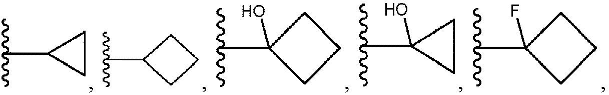 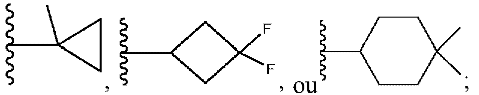 ou
(vi) R² est (a), et R⁶ est phényle, éventuellement substitué par un ou plusieurs parmi halogène tel que F, CI, ou Br ; haloalkyle en C₁₋₆ tel que CF₃, CH₂CF₃, ou CHF₂ ; alkyle en C₁₋₆ tel que méthyle, éthyle, ou propyle ; ou cycloalkyle en C₃₋₆ tel que cyclopropyle, cyclobutyle, ou cyclopentyle,
éventuellement dans lequel R⁶ est 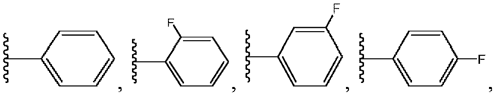 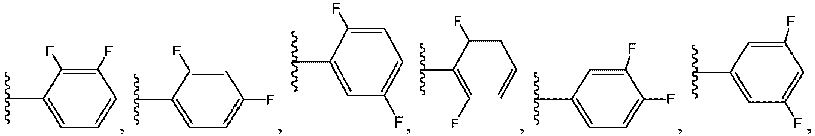 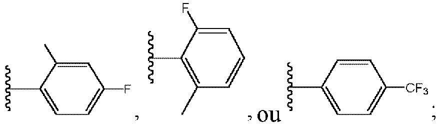 ou
(vii) R² est (a), et R⁶ est pyridinyle, pyrazolyle, pyrazinyle, pyridazinyle, imidazolyle, ou pyrimidinyle, chacun étant éventuellement substitué par un ou plusieurs parmi halogène tel que F, CI, ou Br ; haloalkyle en C₁₋₆ tel que CF₃, CH₂CF₃, CH₂CHF₂, ou CHF₂ ; alkyle en C₁₋₆ tel que méthyle, éthyle, propyle, ou isopropyle ; alcoxy en C₁₋₆ tel que méthoxy, éthoxy, ou propoxy ; haloalcoxy en C₁₋₆ tel que OCF₃ ; cycloalkyle en C₃₋₆ tel que cyclopropyle, cyclobutyle, ou cyclopentyle ; ou cycloalkylsulfonyle en C₃₋₆ tel que cyclopropylsulfonyle,
cyclobutylsulfonyle, ou cyclopentylsulfonyle ;
éventuellement dans lequel (a) R⁶ est 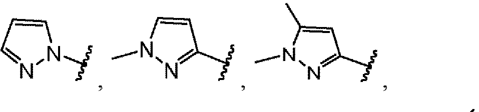 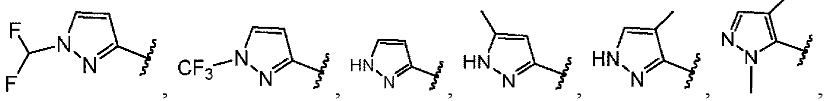 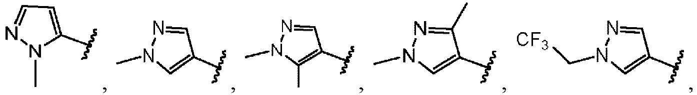 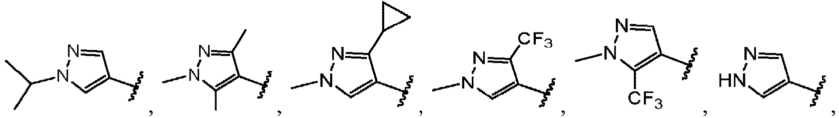 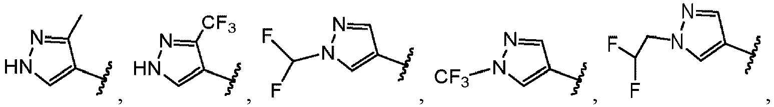 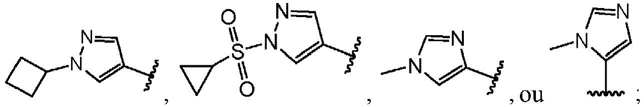 ou
(b) R⁶ est 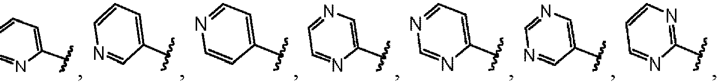 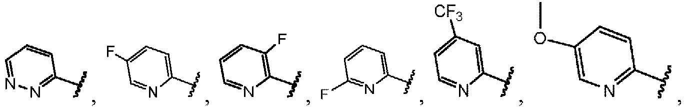 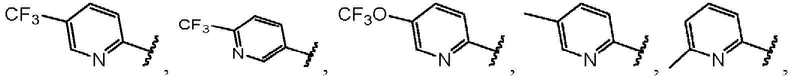 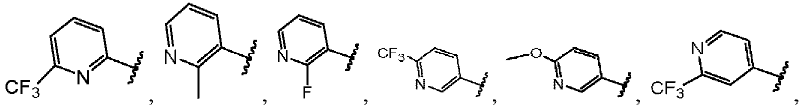 ou 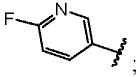 ou
(viii) R² est (a), et R⁶ est azétidinyle, pyrrolidinyle, pipéridinyle, pipérazinyle, ou morpholinyle, chacun étant éventuellement substitué par un ou plusieurs parmi halogène tel que F, CI, ou Br ; OH, haloalkyle en C₁₋₆ tel que CF₃, CH₂CF₃, ou CHF₂ ; alkyle en C₁₋₆ tel que méthyle, éthyle, ou propyle ; hydroxyalkyle en C₁₋₆ tel que C(CH₃)₂OH ; alcoxy en C₁₋₆ tel que méthoxy, éthoxy, ou propoxy ; C(O)O(alkyle en C₁₋₆) tel que C(O)Ométhyle, C(O)Oéthyle, C(O)Opropyle, ou C(O)Otert-butyle ; ou cycloalkyle en C₃₋₆ tel que cyclopropyle, cyclobutyle, ou cyclopentyle ;
éventuellement dans lequel R⁶ est 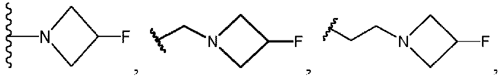 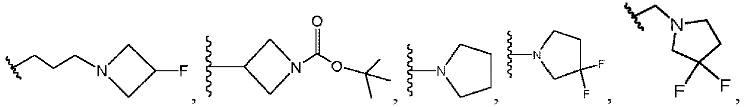 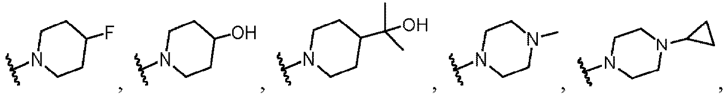 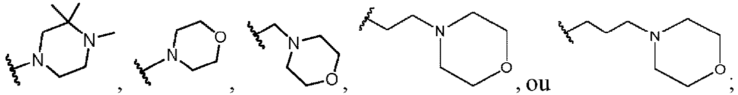 et ou
(ix) R² est (a), et R⁷ est H, CN, alkyle en C₁₋₆ tel que méthyle, haloalkyle en C₁₋₆ tel que CF₃, CH₂F, CHF₂, CF₂(CH₃), ou CH₂CHF₂, halogène tel que Br ou CI, cycloalkyle en C₃₋₈ tel que cyclopropyle, phényle, ou pyridinyle.

5. Composé selon l'une quelconque des revendications 1 à 3, ou un sel pharmaceutiquement acceptable de celui-ci, où R² est : 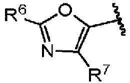 dans lequel :
R⁶ est H, alkyle en C₁₋₆ tel que méthyle, isopropyle, ou tert-butyle, haloalkyle en C₁₋₆ tel que C(CH₃)₂F, CH(CH₃)F, ou CF₃, cyanoalkyle en C₁₋₆ tel que C(CH₃)₂CN, hydroxyalkyle en C₁₋₆ tel que C(CH₃)₂OH, CH₂C(CH₃)₂OH, CH(CH₂CH₃)OH, C(CH₃)₂(CH₂OH) ou CH(CH₃)OH, alcoxy en C₁₋₆ tel que méthoxy, alcoxy en C₁₋₆(alkylène) tel que CH₂OCH₃, C(CH₃)₂OCH₃, ou (CH₂)₂OCH₃, haloalcoxy en C₁₋₆ tel que O(CH₂)₂CF₃, haloalcoxy en C₁₋₆(alkylène) tel que CH₂OCF₃, alcoxy deutéré en C₁₋₆(alkylène) tel que CH₂OCD₃, (CR^{v}R^{x})ₚNR^{y}R^{z} tel que NH₂, CH₂N(CH₃)₂, (CH₂)₂N(CH₃)₂, C(CH₃)₂NHCH₃, C(CH₃)₂N(CH₃)₂, ou NHcyclopropyle, cycloalkyle en C₃₋₆ qui est éventuellement substitué par un ou plusieurs OH tel que 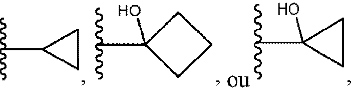 hétérocyclyle qui est éventuellement substitué par un ou plusieurs parmi halogène ou alkyle en C₁₋₆ tel que 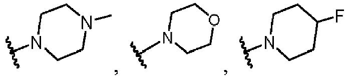 pu hétéroaryle qui est éventuellement substitué par un ou plusieurs parmi alkyle en C₁₋₆, haloalkyle en C₁₋₆, halogène, ou alcoxy en C₁₋₆ tel que 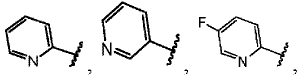 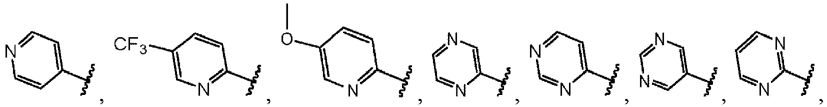 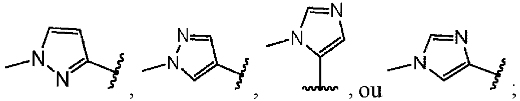
R⁷ est H, alkyle en C₁₋₆ tel que méthyle, haloalkyle en C₁₋₆ tel que CHF₂, CH₂F, C(CH₃)F₂, CH₂CHF₂, ou
CF₃, halogène tel que Br ou CI, ou cycloalkyle en C₃₋₆ tel que cyclopropyle ;
R^{v} et R^{x} sont, indépendamment, H ou alkyle en C₁₋₆ tel que méthyle ;
R^{y} et R^{z} sont, indépendamment, H, alkyle en C₁₋₆ tel que méthyle, ou cycloalkyle en C₃₋₆ tel que cyclopropyle ; et
p vaut 0, 1, 2, ou 3.

6. Composé selon l'une quelconque des revendications 1 à 3, ou un sel pharmaceutiquement acceptable de celui-ci, où R² est : 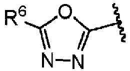 dans laquelle
R⁶ est H, alkyle en C₁₋₆ tel que méthyle ou tert-butyle, haloalkyle en C₁₋₆ tel que C(CH₃)₂F ou CF₃, hydroxyalkyle en C₁₋₆ tel que C(CH₃)₂OH, cycloalkyle en C₃₋₆ tel que 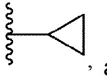 aryle qui est éventuellement substitué par un ou plusieurs parmi halogène, alkyle en C₁₋₆, ou haloalkyle en C₁₋₆ tel que 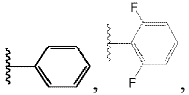 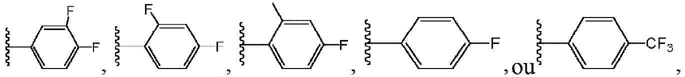 hétéroaryle qui est éventuellement substitué par un ou plusieurs parmi alkyle en C₁₋₆, haloalkyle en C₁₋₆, halogène, alcoxy en C₁₋₆, cycloalkyle en C₃₋₆, ou cycloalkylsulfonyle en C₃₋₆ tel que

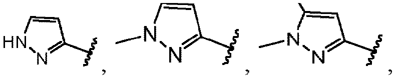

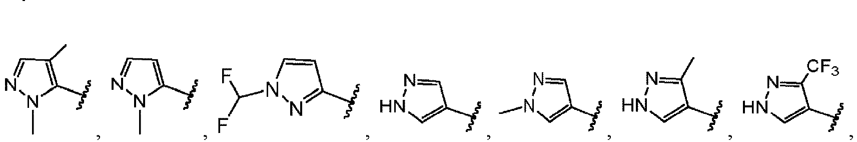

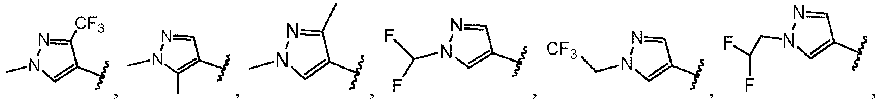

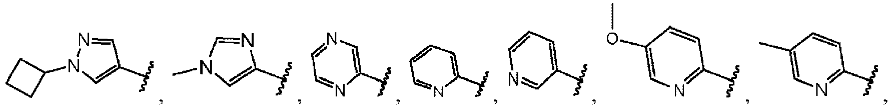

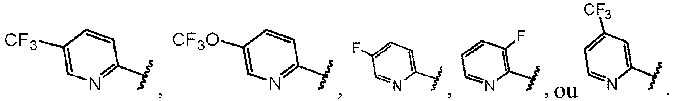

7. Composé selon l'une quelconque des revendications 1 à 3, ou un sel pharmaceutiquement acceptable de celui-ci, où R² est :
(a) 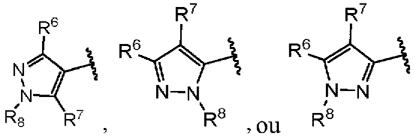 dans lequel :
R⁶ et R⁷ sont, indépendamment, H, halogène tel que CI ou Br, alkyle en C₁₋₆ tel que méthyle, ou haloalkyle en C₁₋₆ tel que CHF₂, CFH₂, ou CF₃ ; éventuellement dans lequel au moins l'un de R⁶ et R⁷ est H ; et
R⁸ est H, alkyle en C₁₋₆ tel que méthyle, isopropyle, ou tert-butyle, haloalkyle en C₁₋₆ tel que CHF₂, CH₂CF₃, CH₂CHF₂, ou CF₃, cycloalkyle en C₃₋₆ tel que cyclopropyle, ou hétéroaryle tel que pyridinyle ; ou
(b) 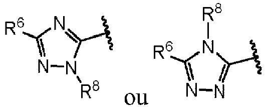 dans lequel :
R⁶ est H, alkyle en C₁₋₆ tel que méthyle, halogène tel que CI ou Br, haloalkyle en C₁₋₆ tel que CHF₂ ou CF₃, ou cycloalkyle en C₃₋₆ tel que cyclopropyle ; et
R⁸ est H ou alkyle en C₁₋₆ tel que méthyle, éthyle, ou isopropyle.

8. Composé selon l'une quelconque des revendications 1 à 7 qui est un énantiomère S, ou un sel pharmaceutiquement acceptable de celui-ci.

9. Composé, ou un sel pharmaceutiquement acceptable de celui-ci, dans lequel le composé est choisi parmi les suivants :

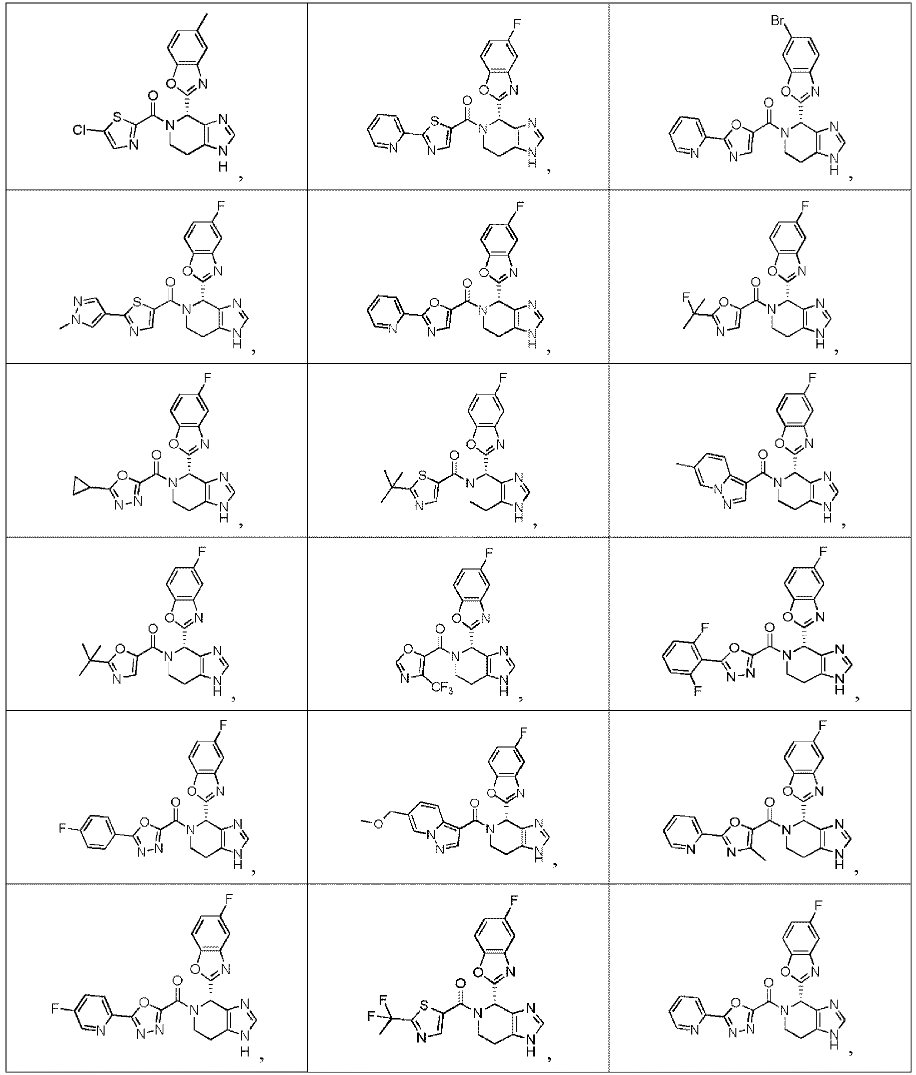

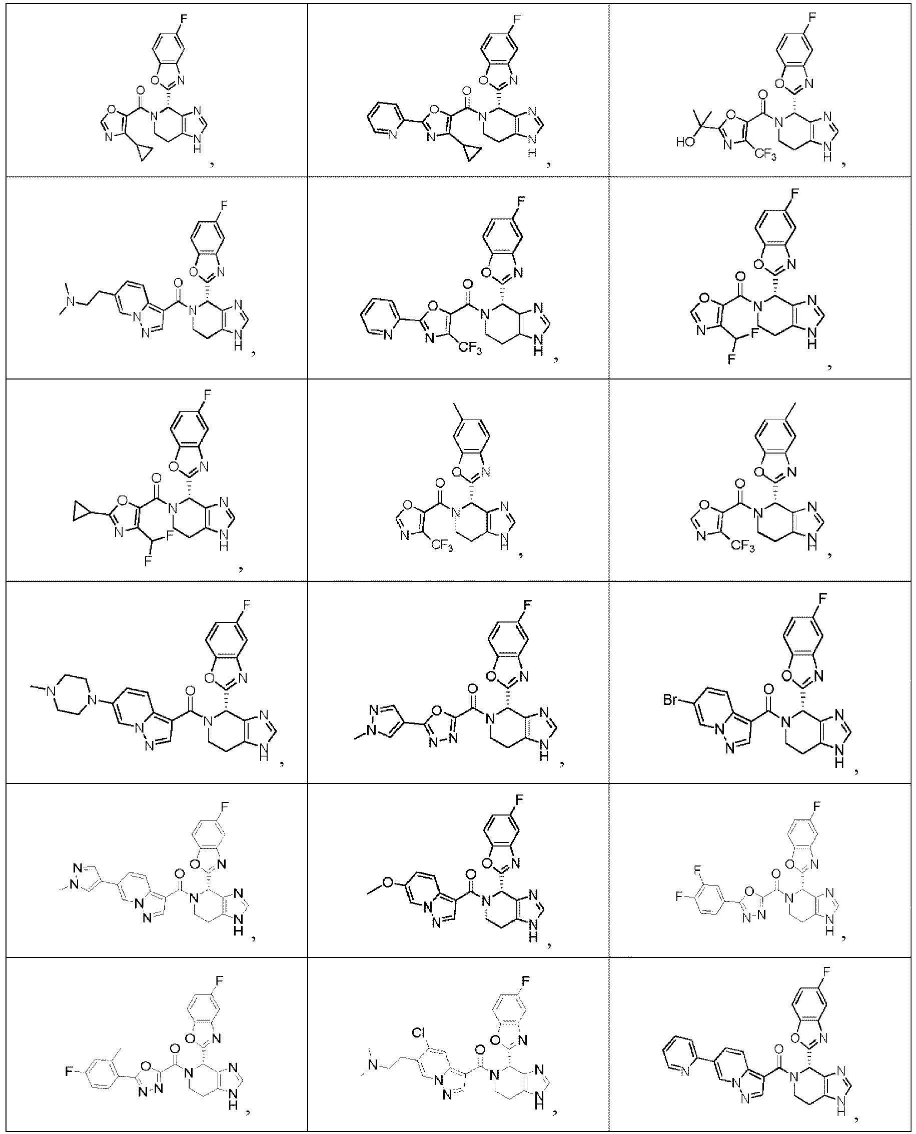

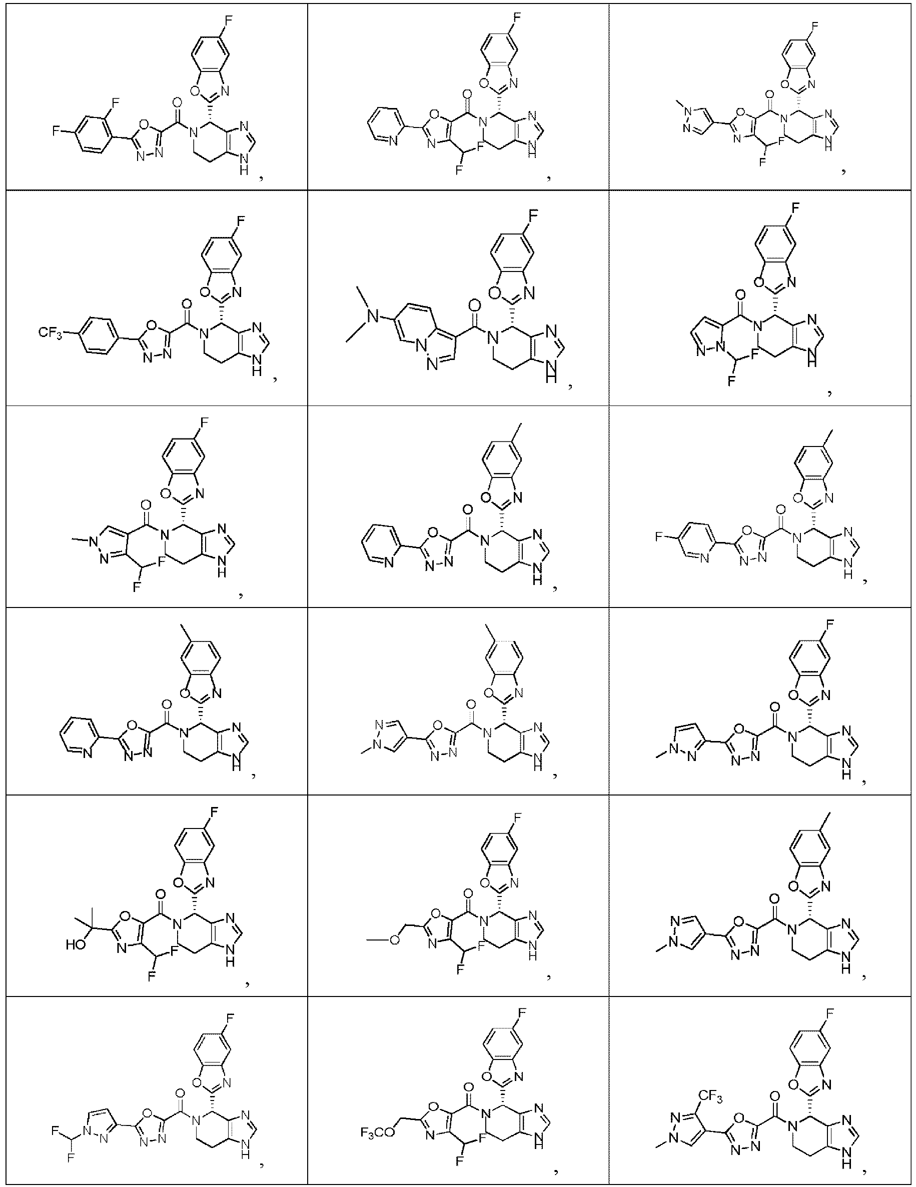

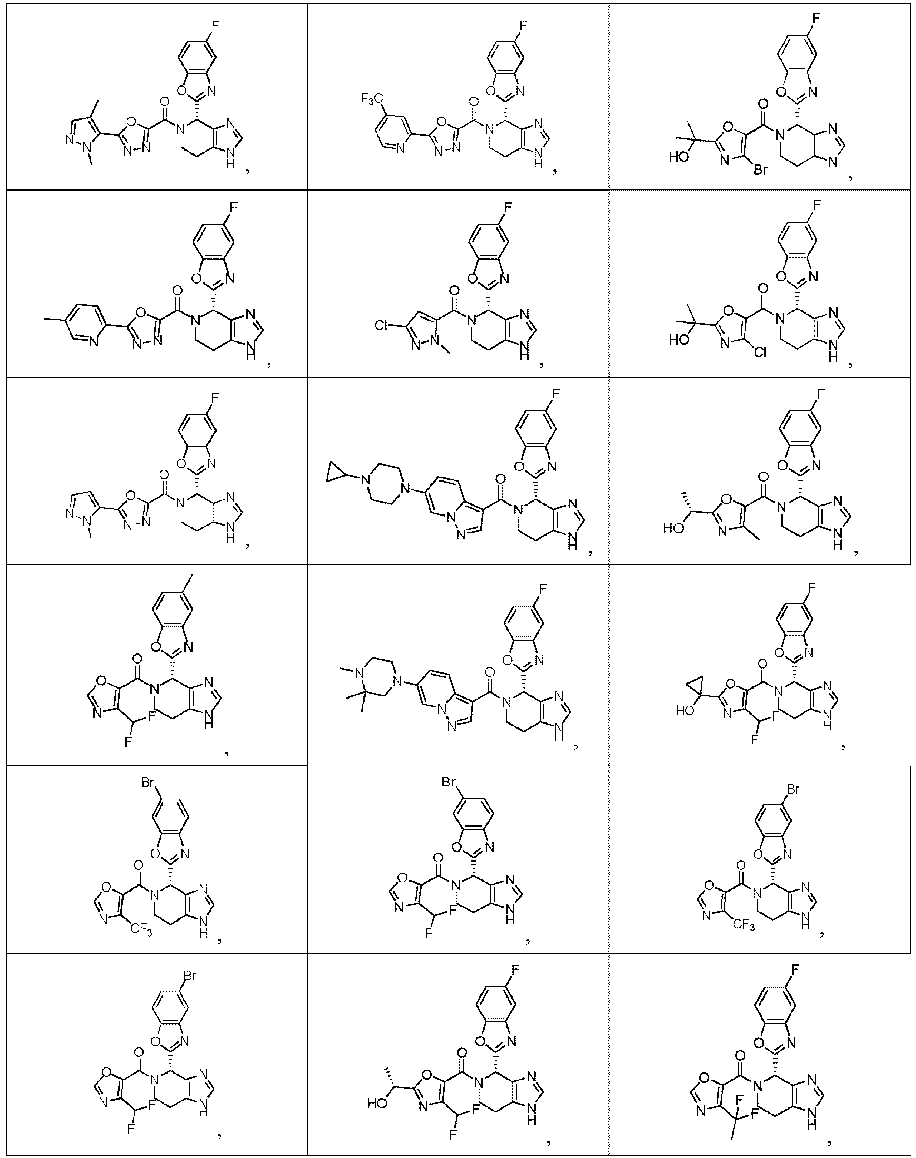

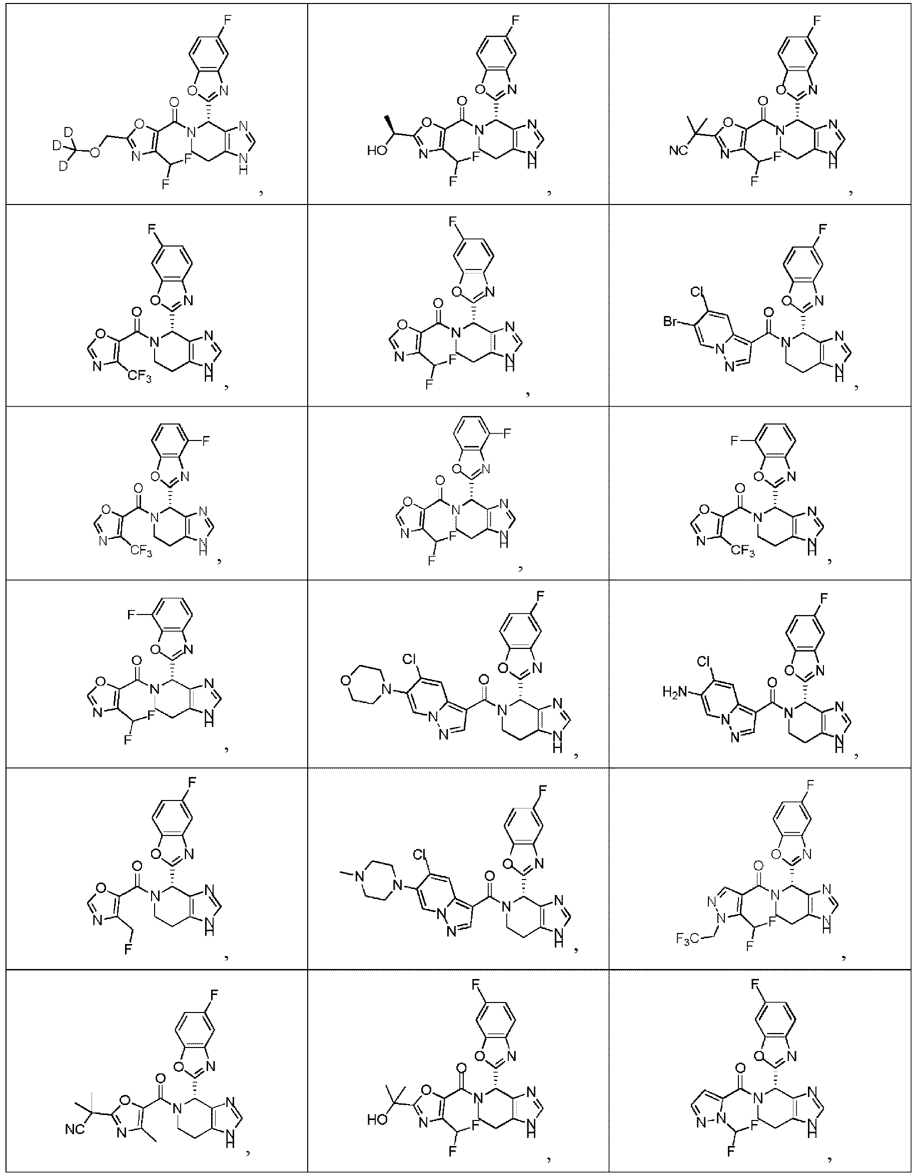

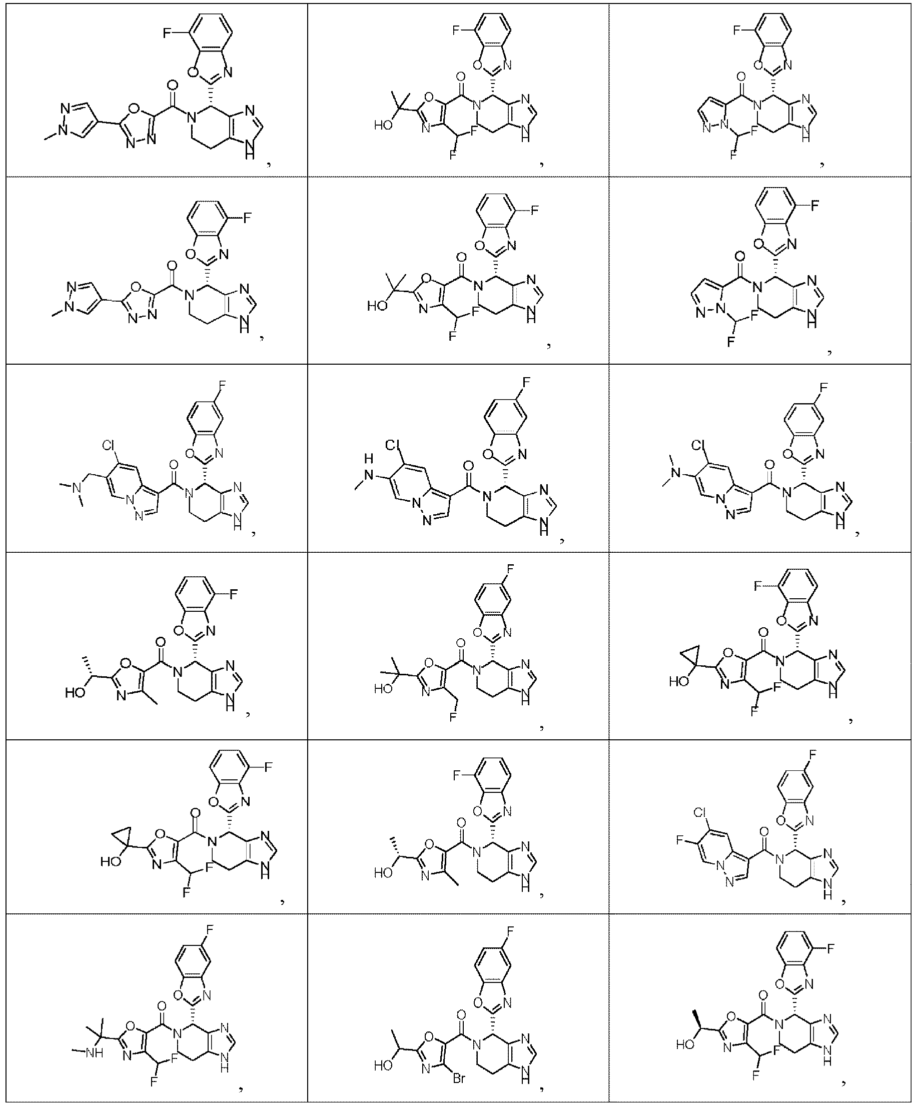

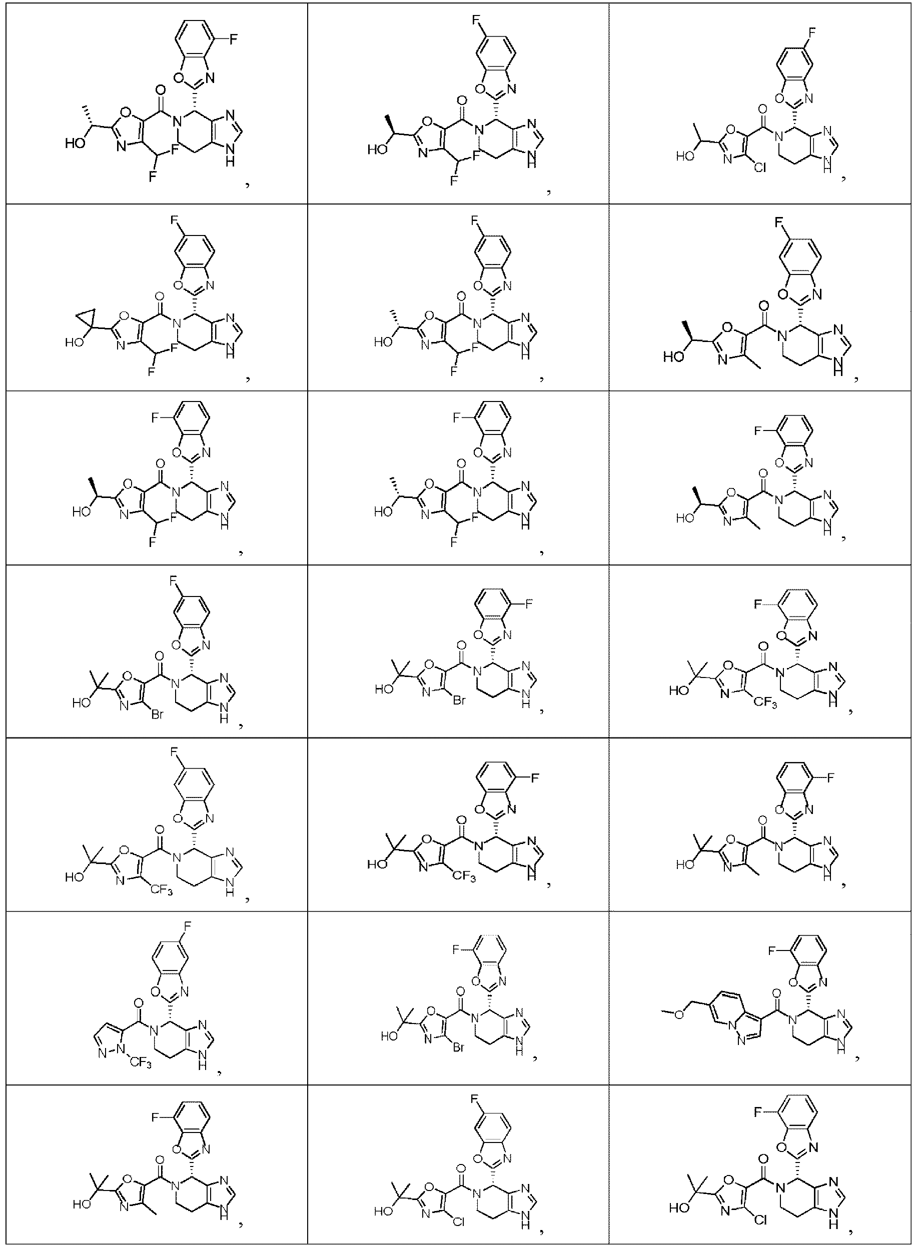

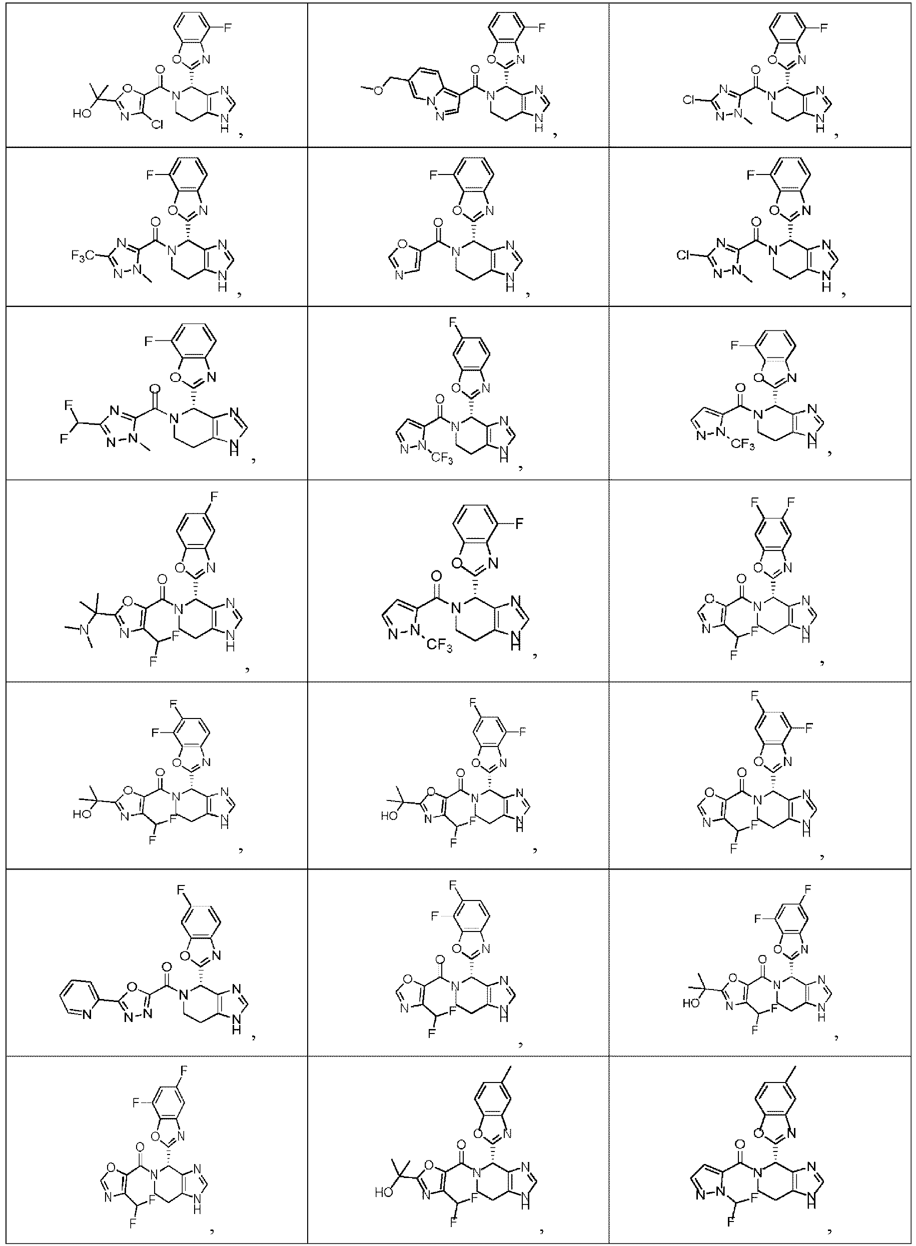

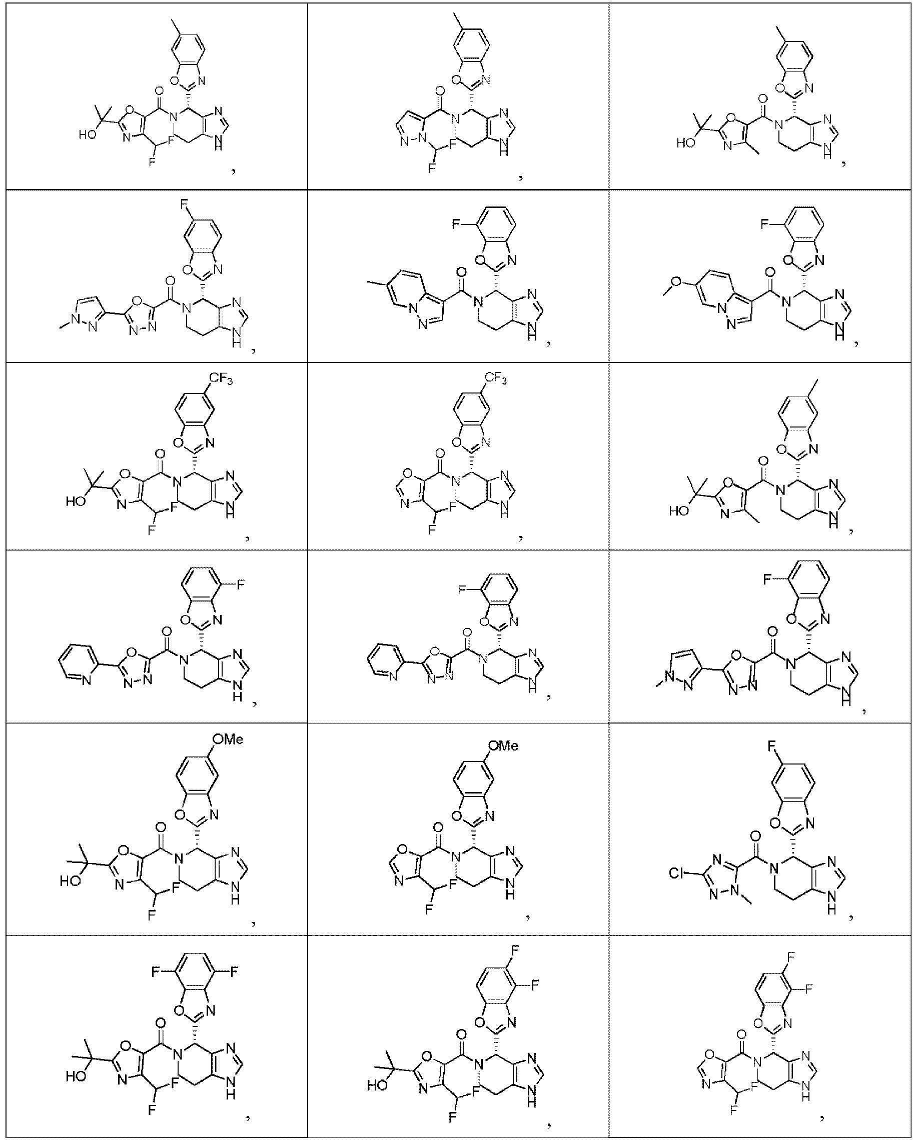

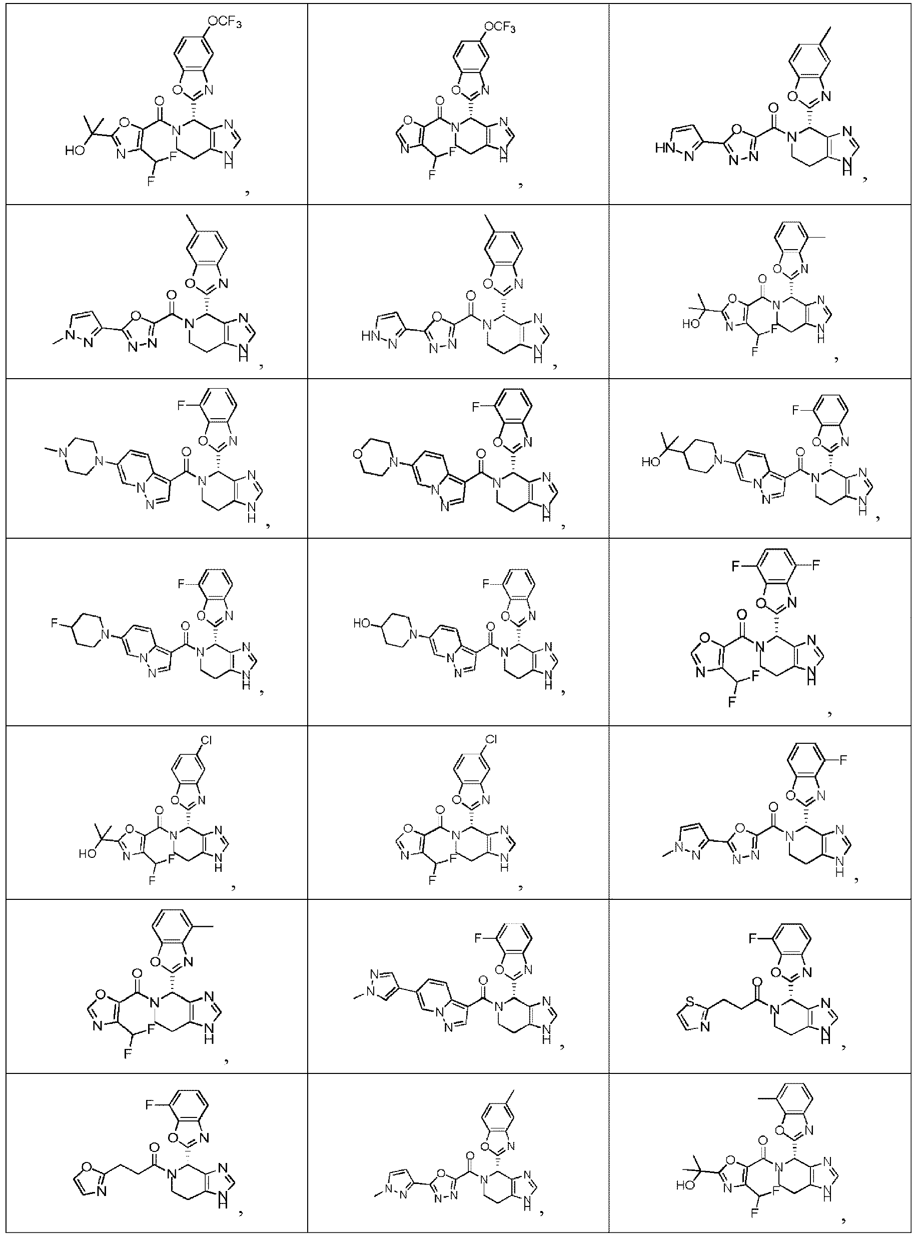

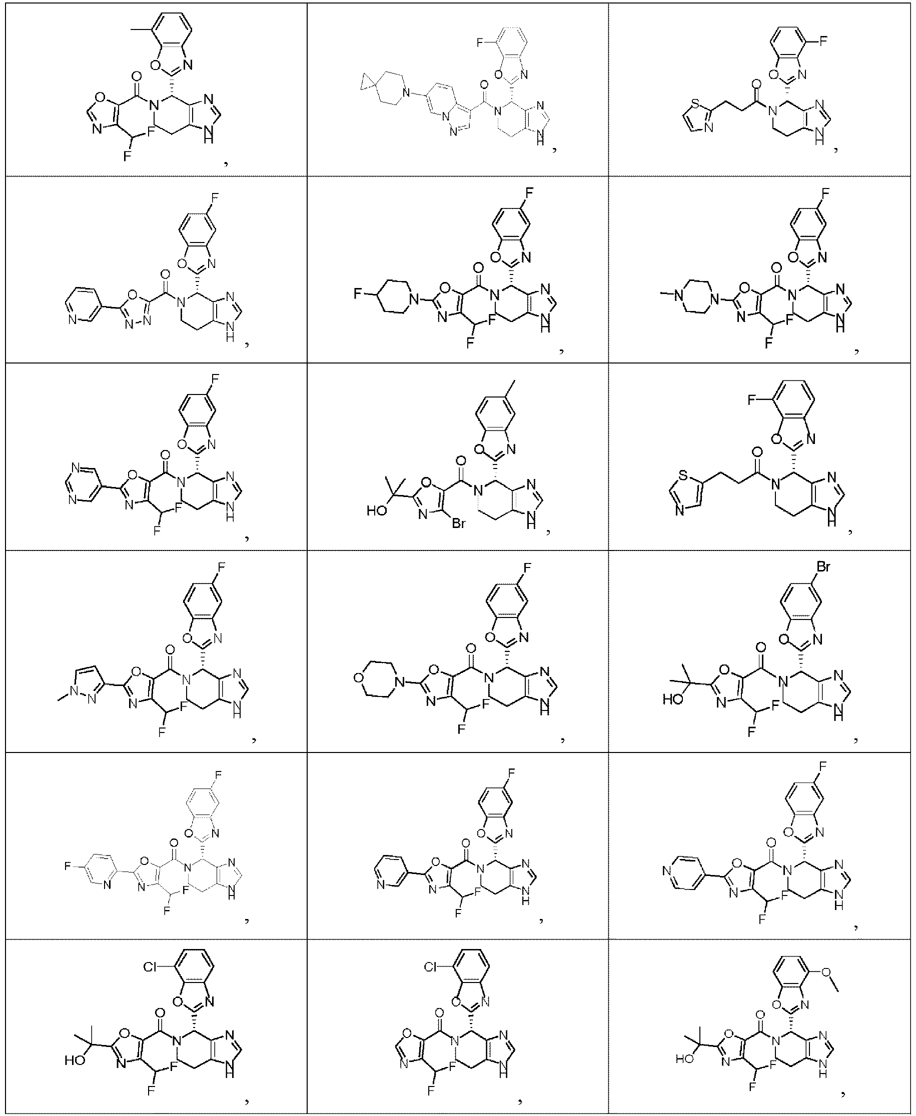

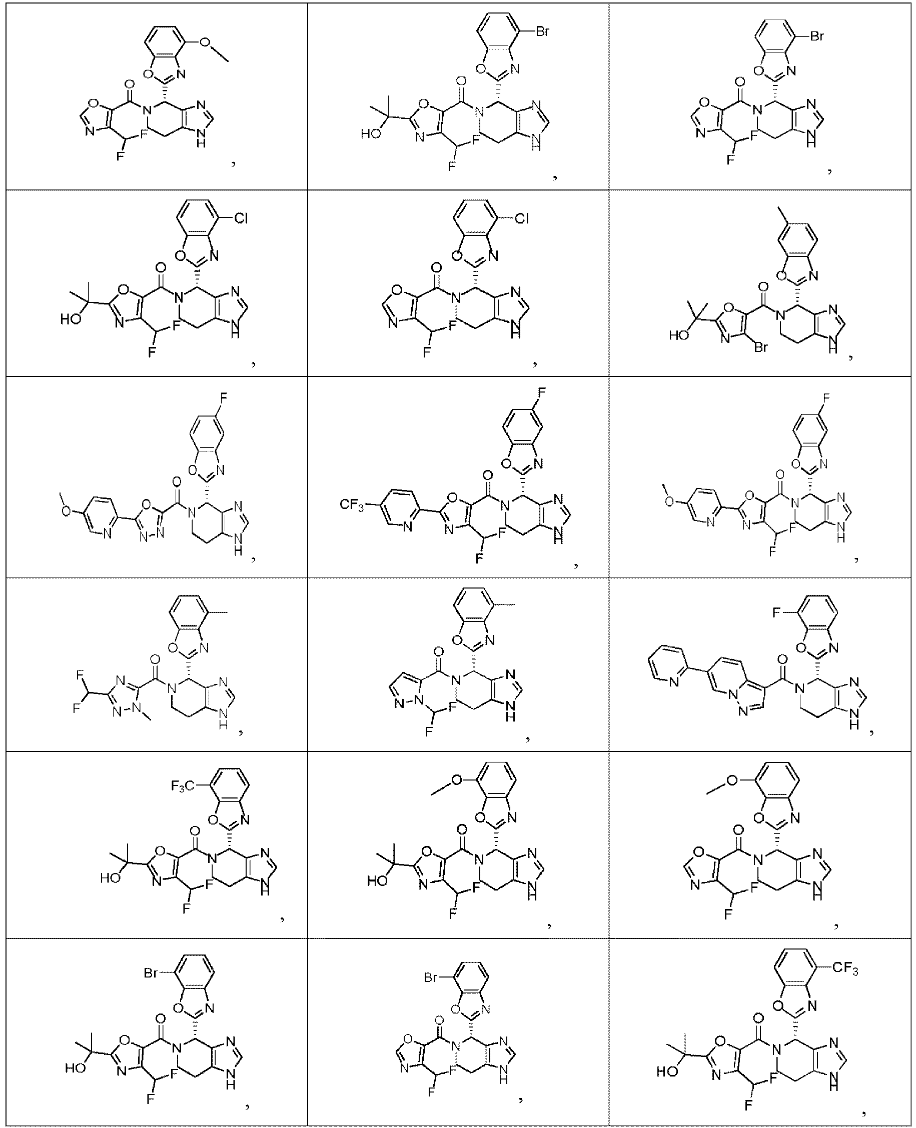

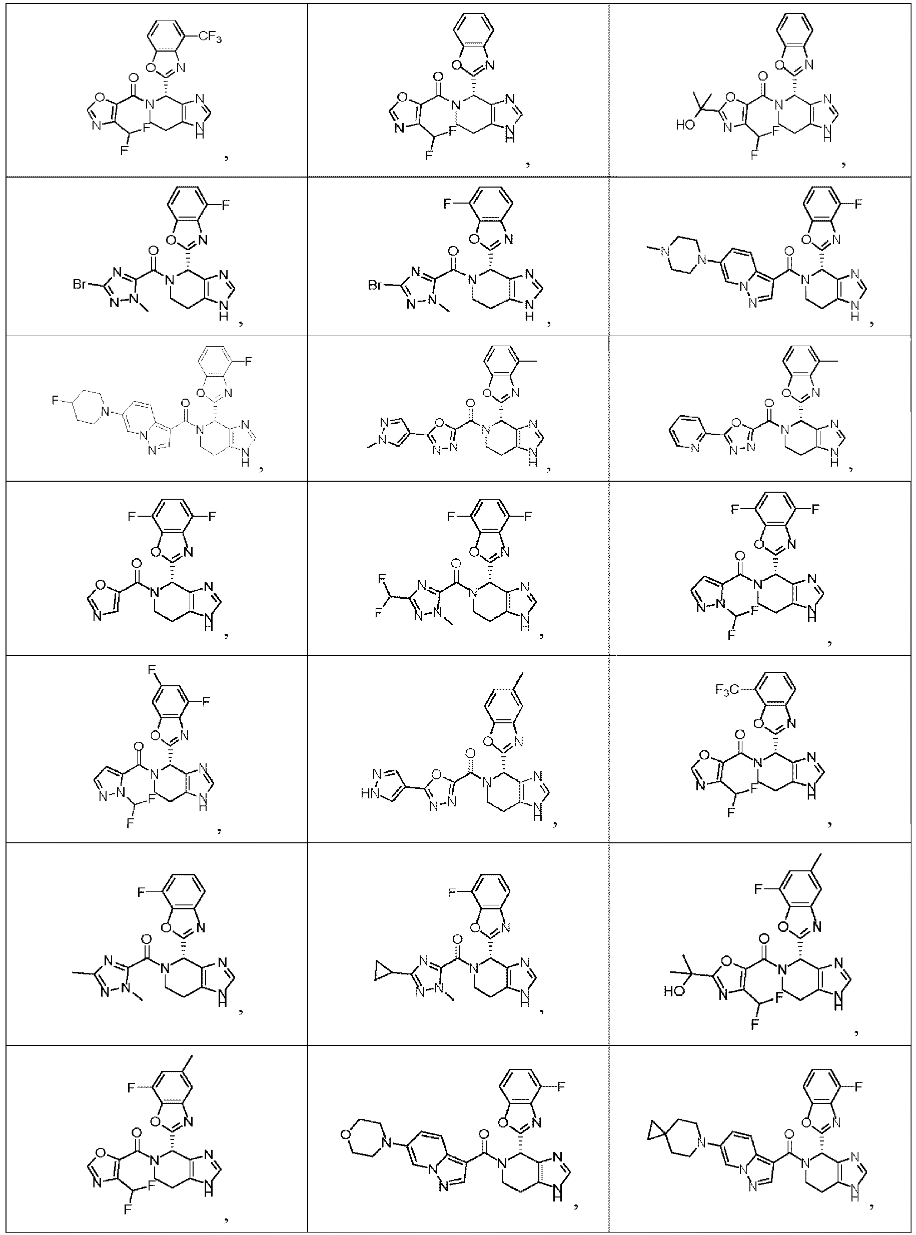

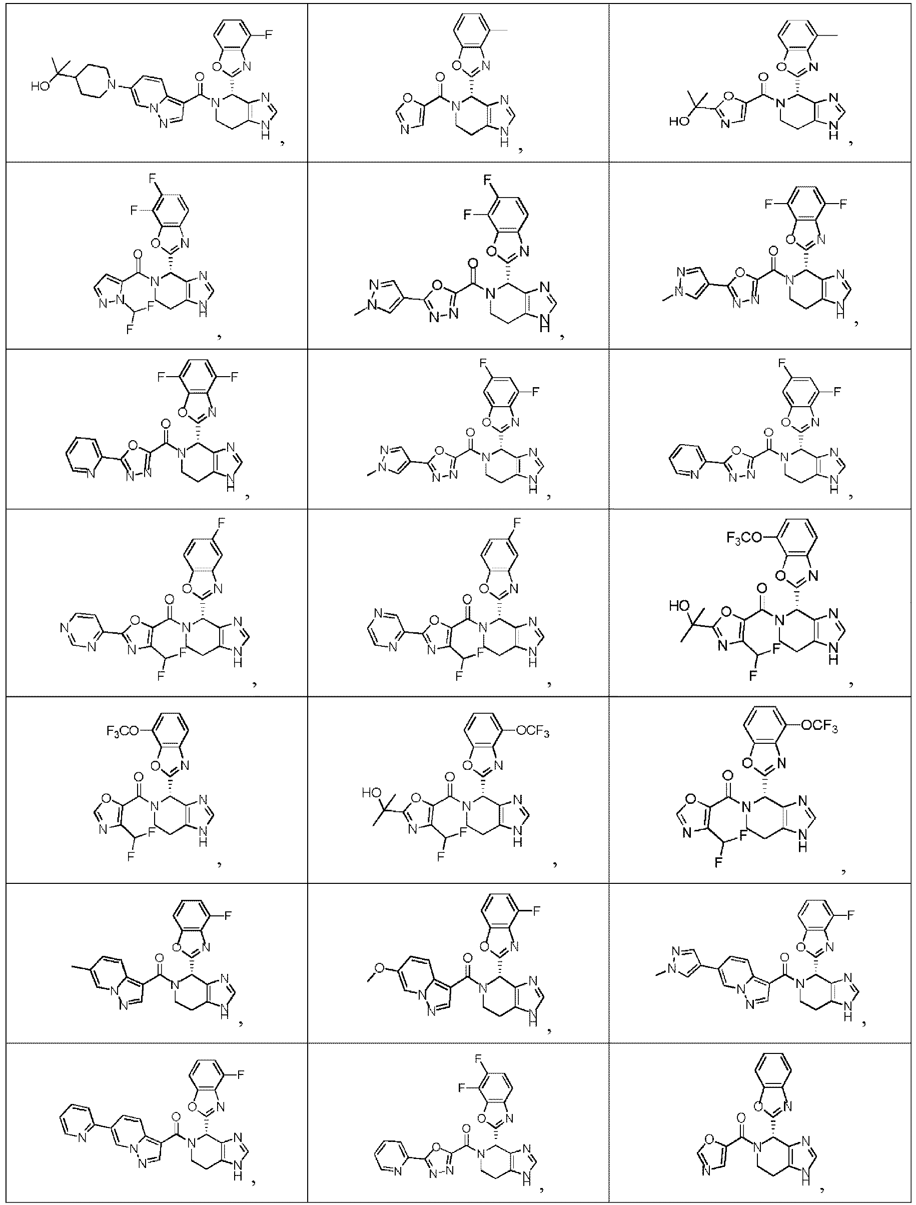

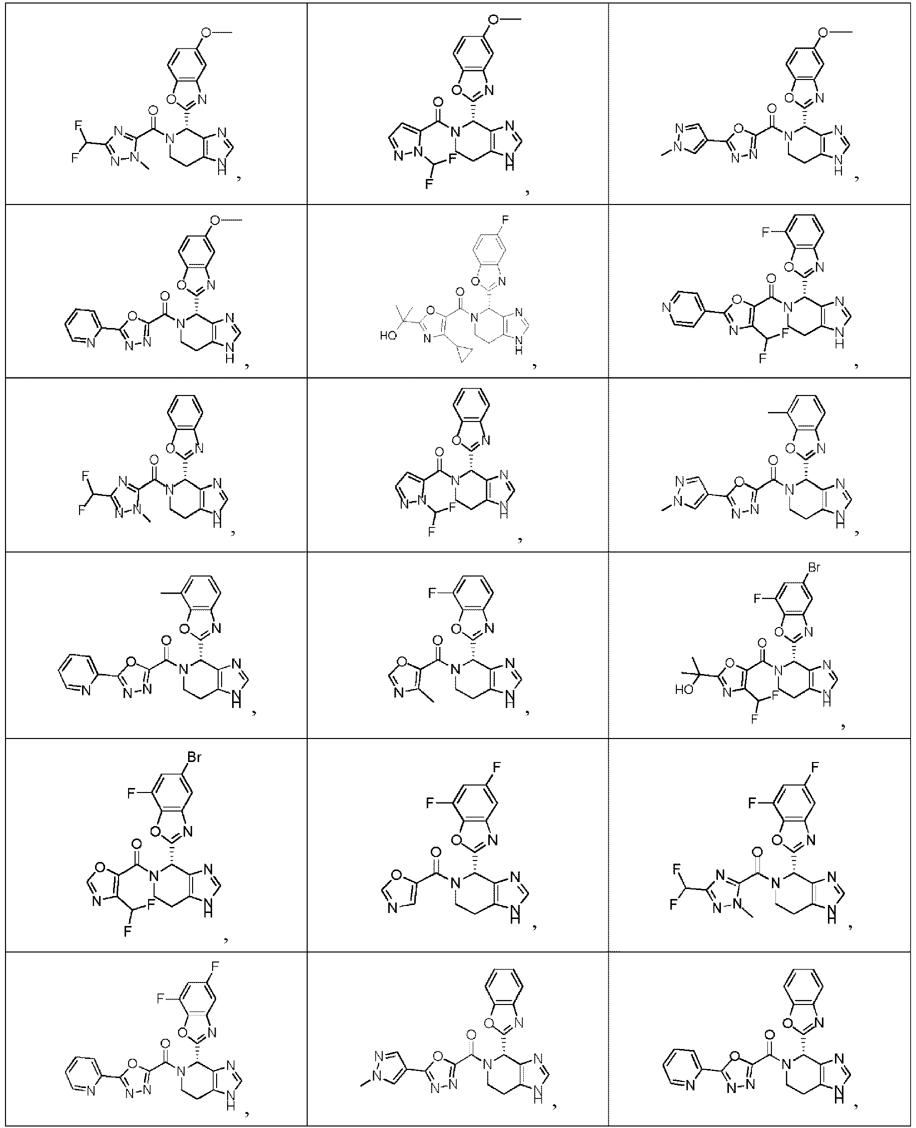

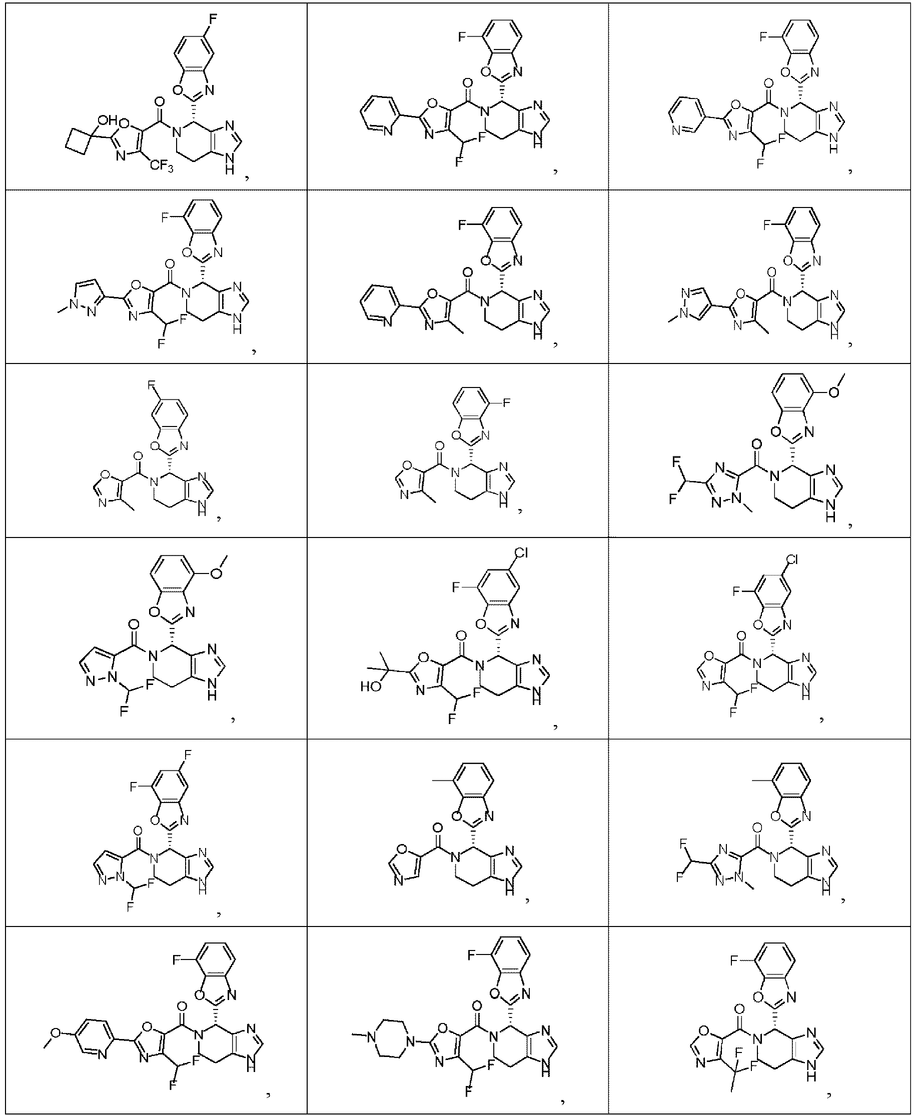

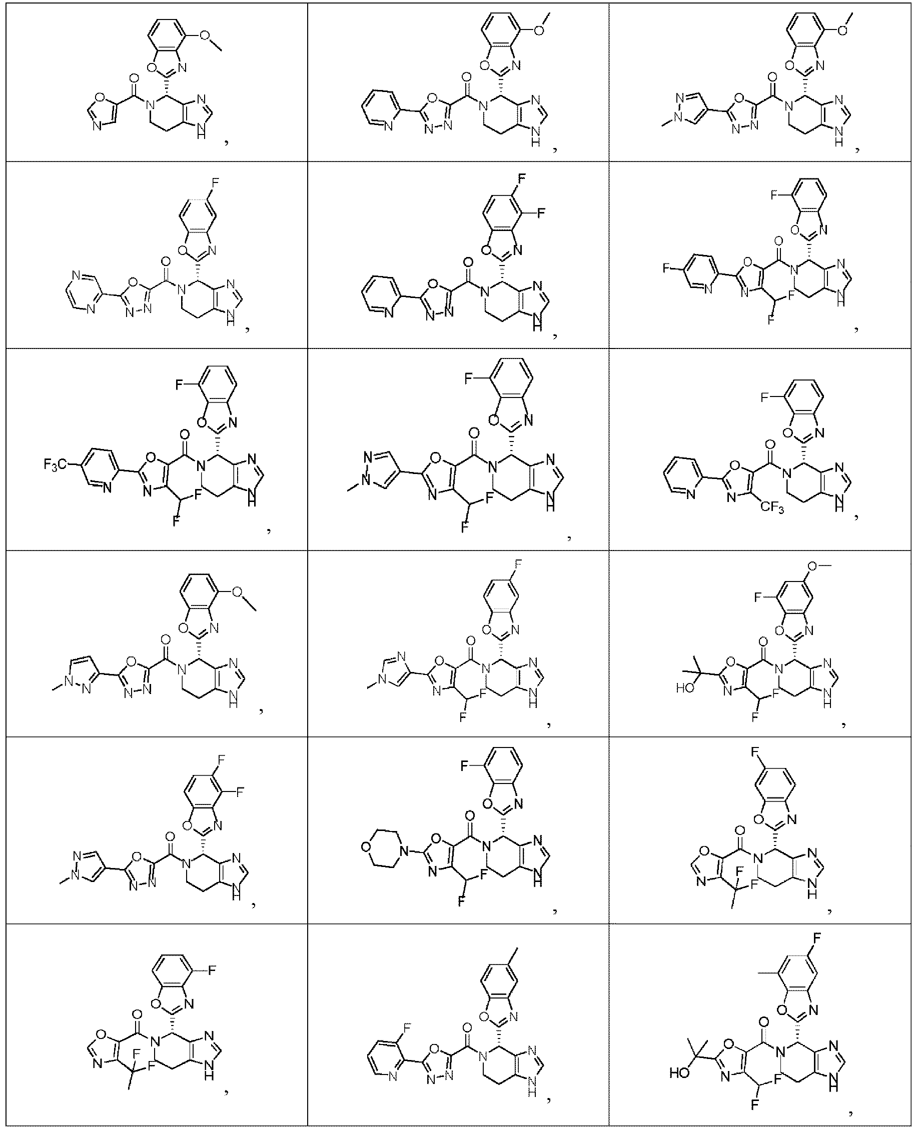

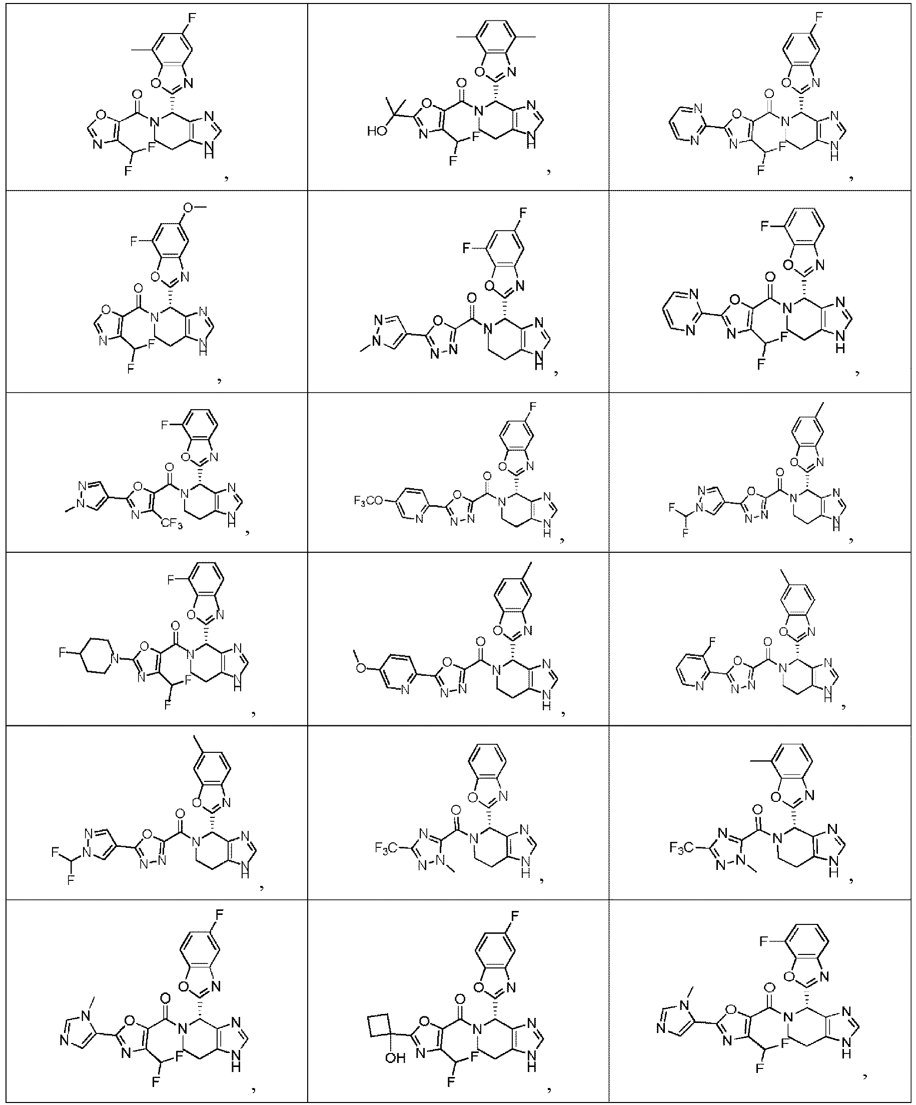

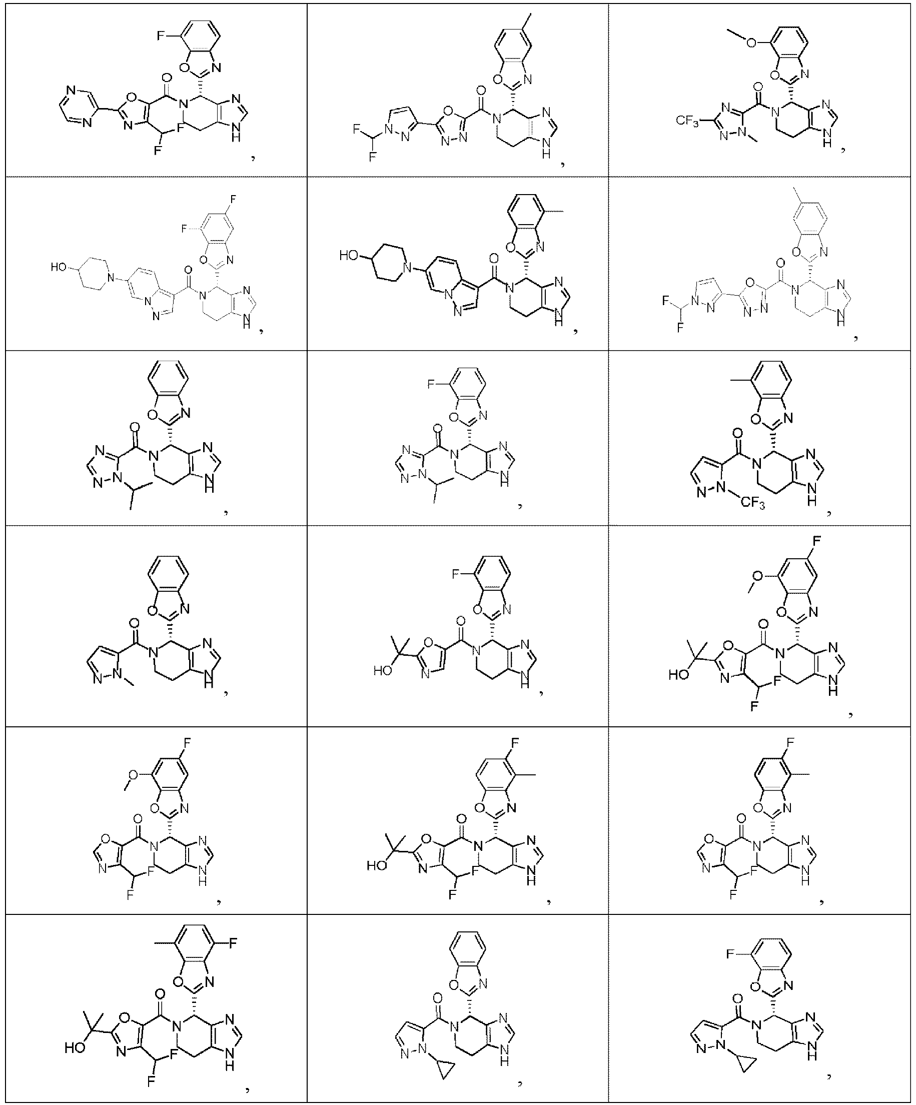

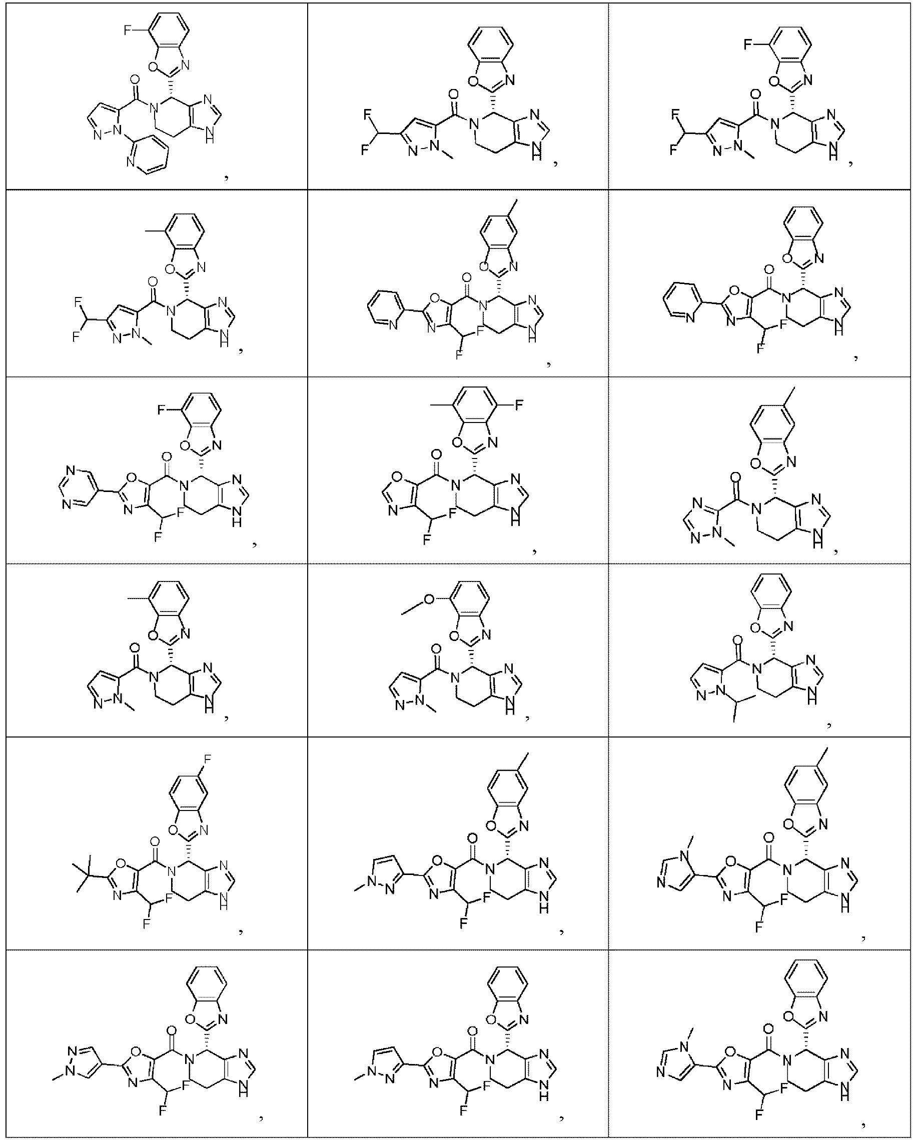

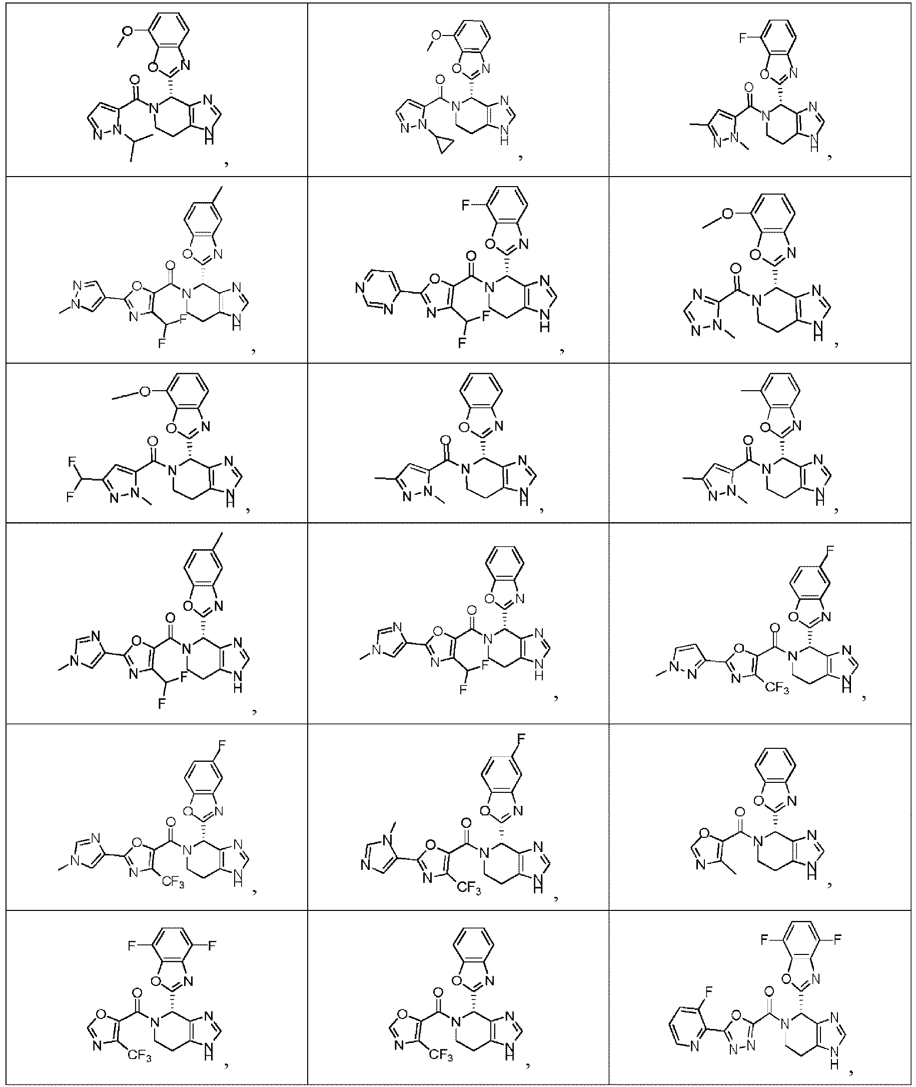

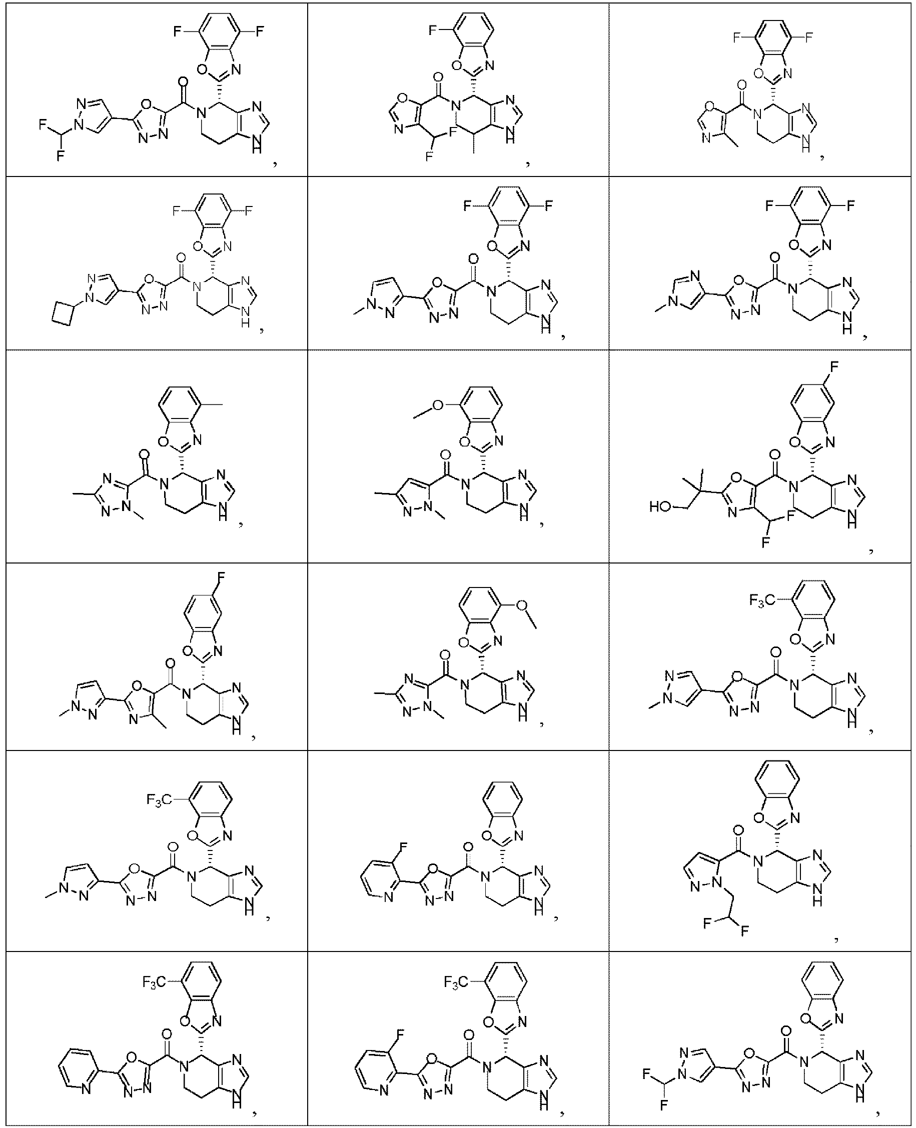

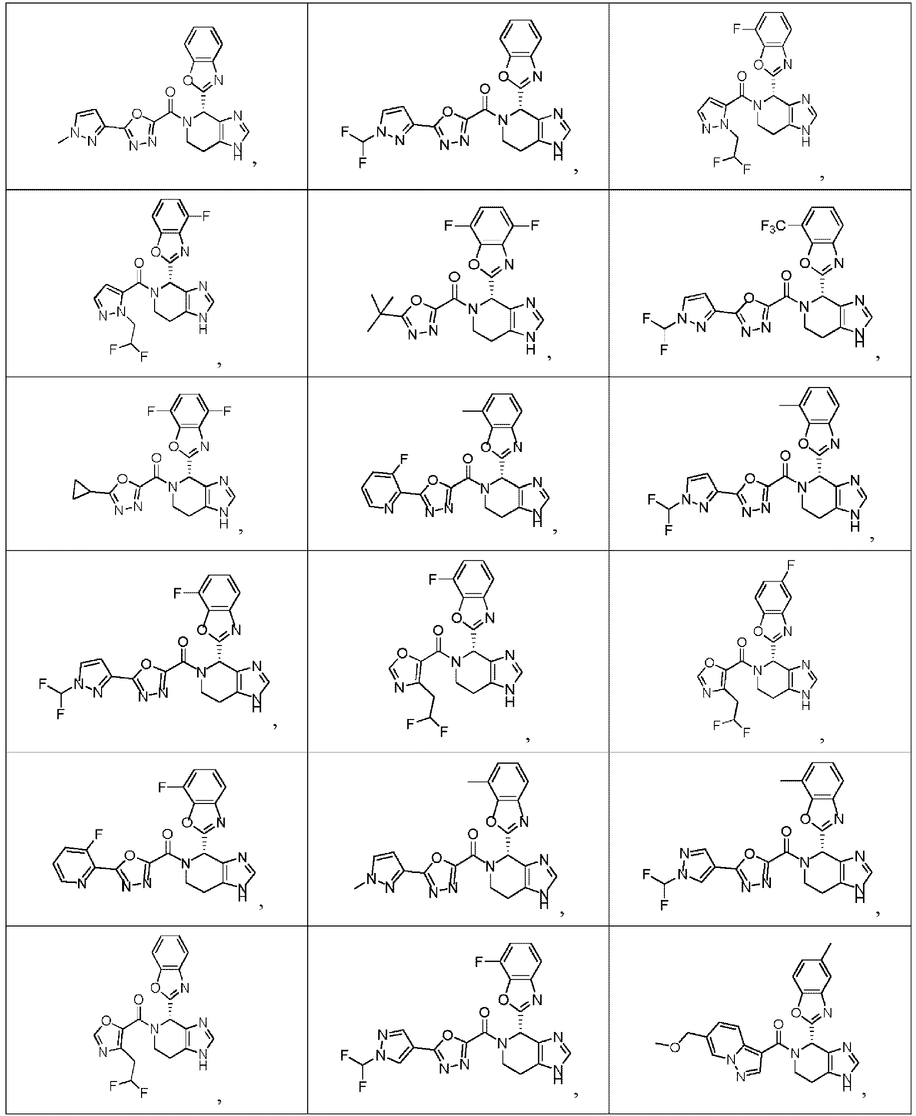

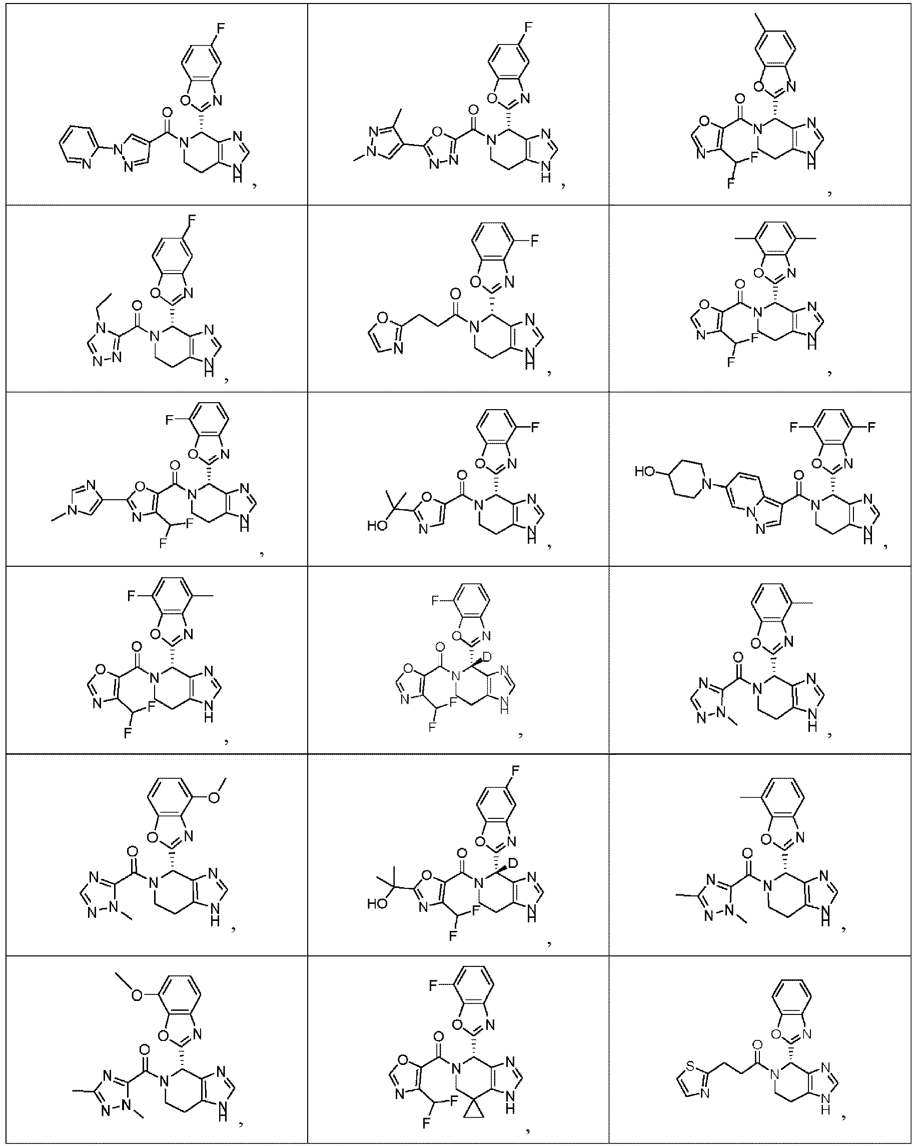

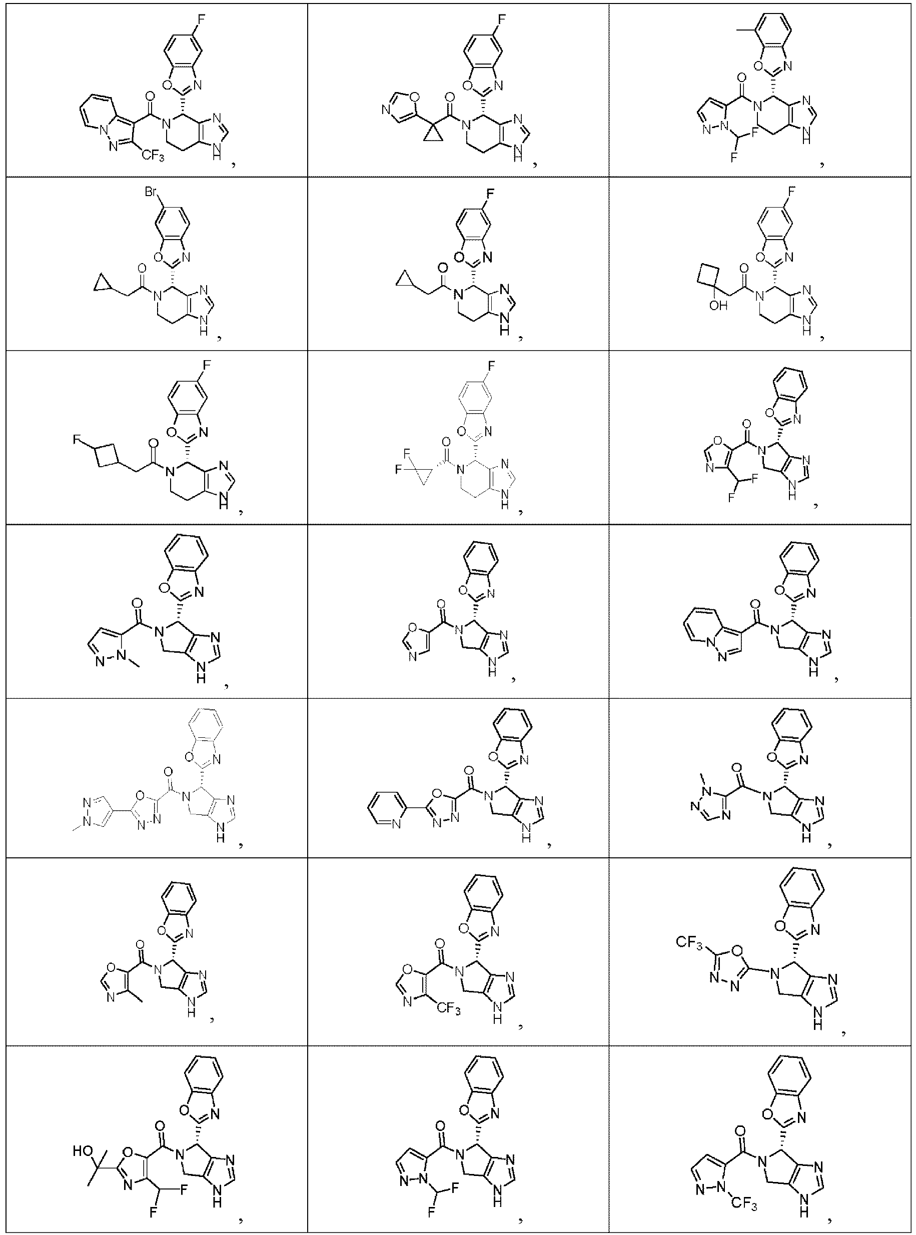

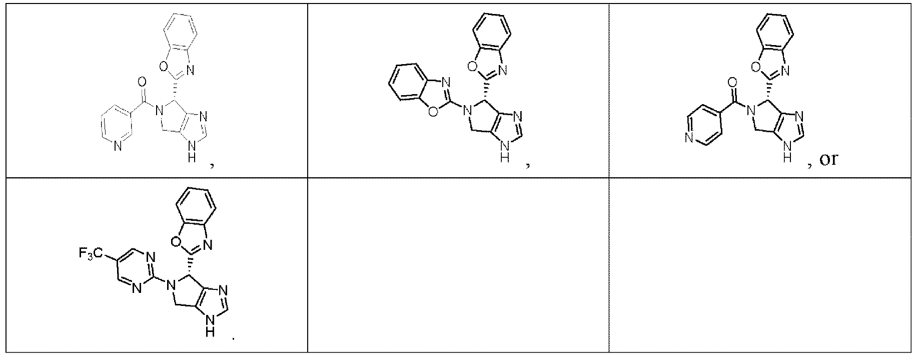

10. Composition pharmaceutique comprenant un composé de l'une quelconque des revendications 1 à 9, ou un sel pharmaceutiquement acceptable de celui-ci, et un excipient pharmaceutiquement acceptable.

11. Composé selon l'une quelconque des revendications 1 à 9, ou un sel pharmaceutiquement acceptable de celui-ci, ou composition pharmaceutique selon la revendication 10, pour une utilisation dans la stabilisation d'une protéine PAH mutante chez un sujet souffrant de phénylcétonurie, comprenant la mise en contact de la protéine avec le composé de l'une quelconque des revendications 1 à 9, ou un sel pharmaceutiquement acceptable de celui-ci, ou la composition pharmaceutique de la revendication 10.

12. Composé ou un sel pharmaceutiquement acceptable de celui-ci, ou composition pour une utilisation selon la revendication 11, dans lequel :
(i) la protéine PAH mutante contient au moins une mutation R408W, R261Q, R243Q, Y414C, L48S, A403V, I65T, R241C, L348V, R408Q, ou V388M ;
(ii) la protéine PAH mutante contient au moins une mutation R408W, Y414C, I65T, F39L, R408Q, L348V, R261Q, A300S, ou L48S ;
(iii) la protéine PAH mutante contient au moins une mutation R408W ; ou
(iv) la protéine PAH mutante contient deux mutations R408W.

13. Composé selon l'une quelconque des revendications 1 à 9, ou un sel pharmaceutiquement acceptable de celui-ci, ou composition pharmaceutique selon la revendication 10, pour une utilisation dans la réduction de la concentration sanguine en phénylalanine chez un sujet souffrant de phénylcétonurie, éventuellement dans lequel :
(i) la concentration sanguine en phénylalanine est réduite jusqu'à une concentration inférieure ou égale à 600 µM ; ou
(ii) la concentration sanguine en phénylalanine est réduite jusqu'à une concentration inférieure ou égale à 360 µM.

14. Composé, ou un sel pharmaceutiquement acceptable de celui-ci, ou composition pour une utilisation selon la revendication 13, dans lequel :
(i) le sujet présente une concentration sanguine en phénylalanine supérieure à 600 µM avant l'administration du composé ; ou
(ii) le sujet présente une concentration sanguine en phénylalanine supérieure à 1 200 µM avant l'administration du composé.

15. Composé selon l'une quelconque des revendications 1 à 9, ou un sel pharmaceutiquement acceptable de celui-ci, ou composition selon la revendication 10, pour une utilisation dans le traitement de la phénylcétonurie chez un sujet.
